# EUROPEAN PATENT SPECIFICATION

(11) **EP 4 522 278 B1**
(45) Date of publication and mention of the grant of the patent: **21.10.2026**
(21) Application number: 23729583.7
(22) Date of filing: 10.05.2023
(51) Int. Cl.: A61P 35/00, C07D 413/14, C07D 417/14, C07D 471/10, C07D 487/10, C07D 498/10, A61K 31/4965

(54) **CINNOLINES AND THEIR USES**
CINNOLINE UND IHRE VERWENDUNGEN
CINNOLINES ET LEURS UTILISATIONS

(30) Priority: 10.05.2022 US 202263340257 P
(43) Date of publication of application: 19.03.2025
(73) Proprietor: Foghorn Therapeutics Inc., Cambridge, MA 02139 (US)
(72) Inventor: NETHERTON, Matthew, Cambridge, Massachusetts 02139 (US); BRUCELLE, Francois, Cambridge, Massachusetts 02139 (US); DENG, Jing, Cambridge, Massachusetts 02139 (US); GAO, Ying-Dou, Cambridge, Massachusetts 02139 (US); ZHAO, Yingzhao, Cambridge, Massachusetts 02139 (US)
(74) Representative: J A Kemp LLP
(86) International application number: PCT/US2023/021666
(87) International publication number: WO 2023/220129

(56) References cited:
- WO-A1-2021/133917
- WO-A1-2021/207291
- WO-A1-2022/109396
- WO-A1-2022/109426
- FARNABY WILLIAM ET AL: "BAF complex vulnerabilities in cancer demonstrated via structure-based PROTAC design", NATURE CHEMICAL BIOLOGY, NATURE PUBLISHING GROUP US, NEW YORK, vol. 15, no. 7, 10 June 2019 (2019-06-10), pages 672 - 680, XP036824718, ISSN: 1552-4450, [retrieved on 20190610], DOI: 10.1038/S41589-019-0294-6

## Description

### Background

The invention relates to compounds useful for modulating BRG1- or BRM-associated factors (BAF) complexes. In particular, the invention relates to compounds useful for treatment of disorders associated with BAF complex function.

Chromatin regulation is essential for gene expression, and ATP-dependent chromatin remodeling is a mechanism by which such gene expression occurs. The human Switch/Sucrose Non-Fermentable (SWI/SNF) chromatin remodeling complex, also known as BAF complex, has two SWI2-like ATPases known as BRG1 (Brahma-related gene-1) and BRM (Brahma). The transcription activator BRG1, also known as ATP-dependent chromatin remodeler SMARCA4, is encoded by the SMARCA4 gene on chromosome 19. BRG1 is overexpressed in some cancer tumors and is needed for cancer cell proliferation. BRM, also known as probable global transcription activator SNF2L2 and/or ATP-dependent chromatin remodeler SMARCA2, is encoded by the SMARCA2 gene on chromosome 9 and has been shown to be essential for tumor cell growth in cells characterized by loss of BRG1 function mutations. Deactivation of BRG and/or BRM results in downstream effects in cells, including cell cycle arrest and tumor suppression.

WO 2022/109426, WO 2022/109396 and WO 2021/207291 are concerned with various compounds useful for the treatment of BAF complex-related disorders.

Farnaby et al. (Nature Chemical Biology 15, 672-680 (2019)) describes a study in which proteolysis targeting chimera (PROTAC) degraders of the BAF ATPase subunits SMARCA2 and SMARCA4 were developed using a bromodomain ligand and recruitment of the E3 ubiquitin ligase VHL.

WO 2021/133917 is concerned with a class of compounds said to have activity as modulators of SMARCA and/or PB1 proteins.

### Summary

The present invention features compounds useful for modulating a BAF complex, as defined in the appended set of claims. In some embodiments, the compounds are useful for the treatment of disorders associated with an alteration in a BAF complex, e.g., a disorder associated with an alteration in one or both of the BRG1 and BRM proteins. The compounds of the invention, alone or in combination with other pharmaceutically active agents, can be for use for treating such disorders.

In an aspect, the invention features a compound, or a pharmaceutically acceptable salt thereof, having the structure of **Formula I:** 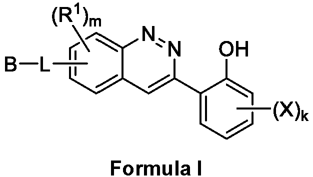 where
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl, optionally substituted C₃-C₈ cycloalkoxy, optionally substituted C₂-C₆ alkynyl, optionally substituted amino, or cyano;
each X is, independently, halo or optionally substituted C₁-C₆ heteroalkyl;
L is a linker of **Formula II:**

   A¹-(B¹)_{f}-(C¹)_{g}-(B²)ₕ-(D)-(B³)ᵢ-(C²)ⱼ-(B⁴)ₖ-A², **Formula II**
or a pharmaceutically acceptable salt thereof,
wherein
A¹ is a bond between the linker and the ring system A;
A² is a bond between the degradation moiety and the linker;
each of B¹, B², B³, and B⁴ is, independently, optionally substituted C₁-C₄ alkyl, optionally substituted C₆-C₁₀ aryl, optionally substituted C₆-C₁₀ aryl C₁₋₄ alkyl, optionally substituted C₁-C₄ heteroalkyl, optionally substituted C₃-C₁₀ cycloalkyl, optionally substituted C₃-C₁₀ carbocyclyl, optionally substituted C₂-C₁₀ heterocyclyl, optionally substituted C₂-C₆ heteroaryl, optionally substituted C₆₋₁₂ aryl, O, S, S(O)₂, or NR^{N};
each R^{N} is, independently, H, optionally substituted C₁₋₄ alkyl, optionally substituted C₂₋₄ alkenyl, optionally substituted C₂₋₄ alkynyl, optionally substituted C₂₋₁₀ heterocyclyl, optionally substituted C₂₋₆ heteroaryl, or optionally substituted C₁₋₇ heteroalkyl;
each of C¹ and C² is, independently, carbonyl, thiocarbonyl, sulphonyl, or phosphoryl;
each of f, g, h, i, j, and k is, independently, 0 or 1; and
D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₂₋₆ heteroaryl, optionally substituted C₆₋₁₂ aryl, optionally substituted C₂-C₁₀ polyethylene glycol, optionally substituted C₃-C₁₀ cycloalkyl, optionally substituted C₃-C₁₀ carbocyclyl, or optionally substituted C₁₋₁₀ heteroalkyl; or D is absent, and the linker is A¹-(B¹)_{f}-(C¹)_{g}-(B²)ₕ-(B³)ᵢ-(C²)ⱼ-(B⁴)ₖ-A²; and
   B is a degradation moiety of **Formula C:** 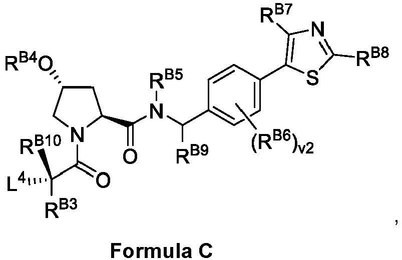
wherein
L⁴ is -N(R^{B1})(R^{B2}), 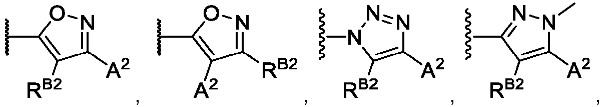 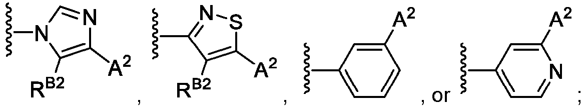
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

In another aspect, the invention features a compound, or a pharmaceutically acceptable salt thereof, having the structure of **Formula I:** 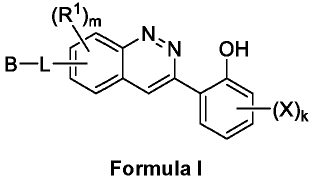 where
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, or optionally substituted C₃-C₈ cycloalkyl;
each X is, independently, halo;
L is a linker of the Formula II; and
B is a degradation moiety of the Formula C.

In some embodiments, the compound has the structure of **Formula I-A:**

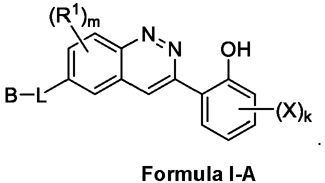

In some embodiments, the compound has the structure of **Formula I-B**

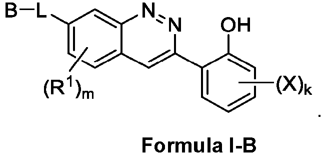

In some embodiments, m is 0. In some embodiments, m is 1. In some embodiments, m is 2. In some embodiments, m is 3.

In some embodiments, R¹ is optionally substituted C₁-C₆ heteroalkyl. In some embodiments, R¹ is alkoxy. In some embodiments, R¹ is methoxy. In some embodiments, R¹ is halo. In some embodiments, R¹ is F or Cl. In some embodiments, R¹ is optionally substituted C₁-C₆ alkyl. In some embodiments, R¹ is methyl. In some embodiments, R¹ is difluoromethoxy. In some embodiments, R¹ is difluoromethyl. In some embodiments, R¹ is optionally substituted C₂-C₆ alkynyl. In some embodiments, R¹ is methyne. In some embodiments, R¹ is optionally substituted C₃-C₈ cycloalkyl. In some embodiments, R¹ is cyclopropane. In some embodiments, R¹ is cyclopropoxy. In some embodiments, R¹ is optionally substituted C₂-C₉ heterocyclyl. In some embodiments, R¹ is optionally substituted amino. In some embodiments, R¹ is cyano.

In some embodiments, k is 0. In some embodiments, k is 1. In some embodiments, k is 2. In some embodiments, X is optionally substituted C₁-C₆ heteroalkyl. In some embodiments, X is methoxy. In some embodiments, X is halo. In some embodiments, X is F.

In some embodiments, the degradation moiety has the structure of **Formula C:** 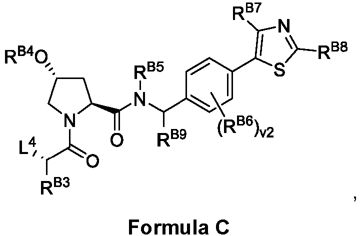 where
L⁴ is -N(R^{B1})(R^{B2}), 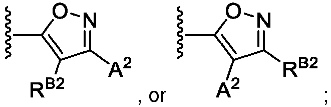
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
where one and only one of R^{B1}, R^{B3}, and R^{B6} is A²,
or a pharmaceutically acceptable salt thereof.

In some embodiments, the degradation moiety has the structure of **Formula C3.**

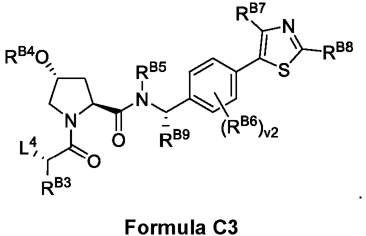

In some embodiments, the degradation moiety has the structure of **Formula C4.**

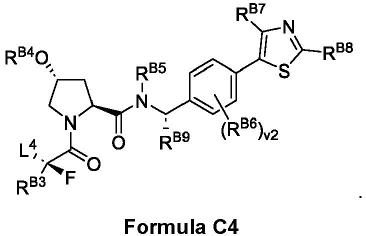

In some embodiments, the degradation moiety has the structure of **Formula C1:**

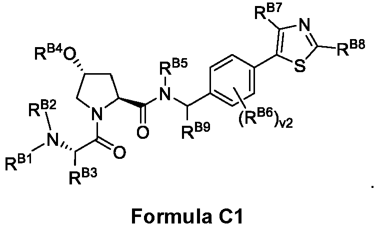

In some embodiments, the degradation moiety is

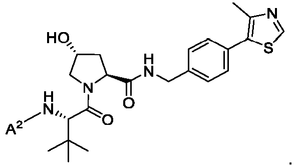

In some embodiments, the degradation moiety is

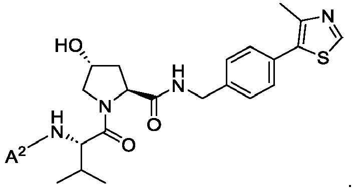

In some embodiments, the degradation moiety is

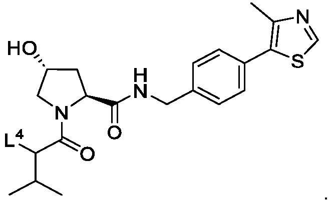

In some embodiments, the degradation moiety is

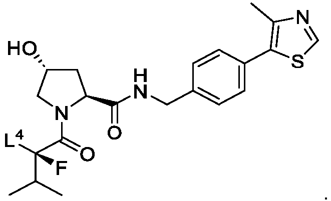

In some embodiments, the degradation moiety is

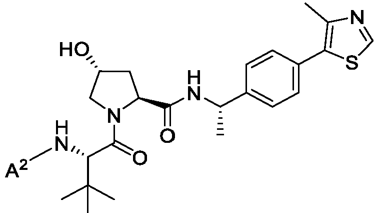

In some embodiments, the degradation moiety is

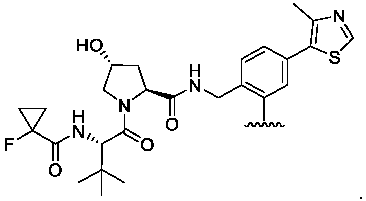

In some embodiments, the degradation moiety has the structure of **Formula C2:**

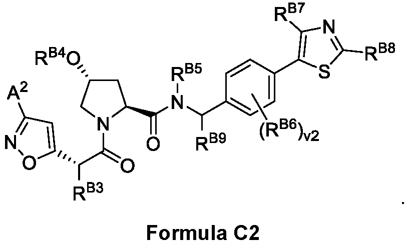

In some embodiments, R^{B9} is optionally substituted C₁-C₆ alkyl. In some embodiments, R^{B9} is methyl.

In some embodiments, R^{B9} is bonded to (S)-stereogenic center.

In some embodiments, v2 is 0. In some embodiments, R^{B4} is H. In some embodiments, R^{B5} is H. In some embodiments, R^{B7} is optionally substituted C₁-C₆ alkyl. In some embodiments, R^{B7} is methyl. In some embodiments, R^{B3} is optionally substituted C₁-C₆ alkyl. In some embodiments, R^{B3} is isopropyl. In some embodiments, R^{B8} is H. In some embodiments, R^{B2} is H.

In some embodiments, the degradation moiety is

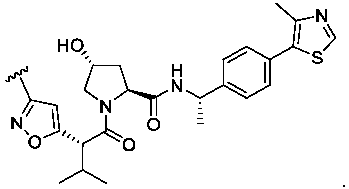

In some embodiments, the degradation moiety has the structure of **Formula Ca2:**

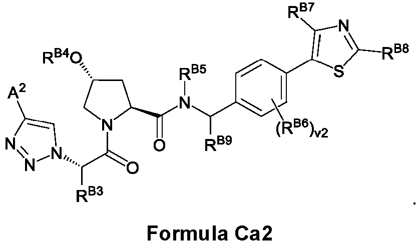

In some embodiments, the degradation moiety has the structure of **Formula Cb2:**

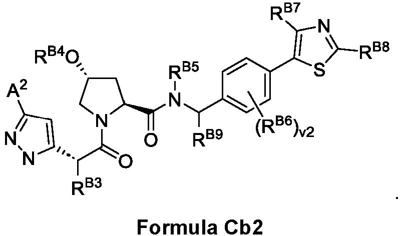

In some embodiments, the degradation moiety has the structure of **Formula Cc2:**

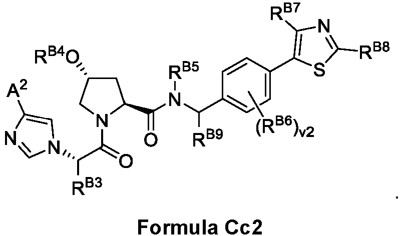

In some embodiments, the degradation moiety has the structure of **Formula Cd2:**

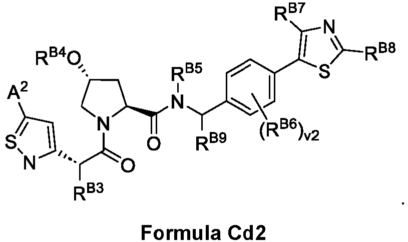

In some embodiments, the degradation moiety has the structure of **Formula Ce2:**

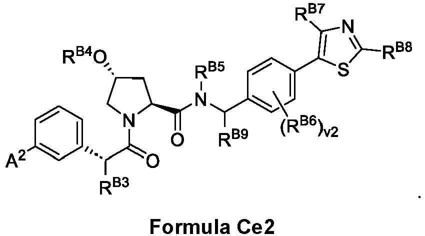

In some embodiments, the degradation moiety has the structure of **Formula Cf2:**

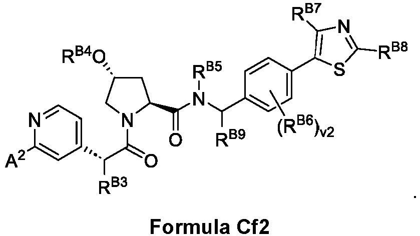

**In** some embodiments, R^{B9} is optionally substituted C₁-C₆ alkyl. In some embodiments, R^{B9} is methyl.

In some embodiments, R^{B9} is bonded to (S)-stereogenic center.

In some embodiments, v2 is 0. In some embodiments, R^{B4} is H. In some embodiments, R^{B5} is H. In some embodiments, R^{B7} is optionally substituted C₁-C₆ alkyl. In some embodiments, R^{B7} is methyl. In some embodiments, R^{B3} is optionally substituted C₁-C₆ alkyl. In some embodiments, R^{B3} is isopropyl. In some embodiments, R^{B3} is optionally substituted C₃-C₁₀ carbocyclyl. In some embodiments, R^{B3} is cyclopropane. In some embodiments, R^{B3} is cyclobutane. In some embodiments, R^{B3} is fluoro-2-methylpropane. In some embodiments, R^{B8} is H. In some embodiments, R^{B2} is H.

In some embodiments, the degradation moiety is

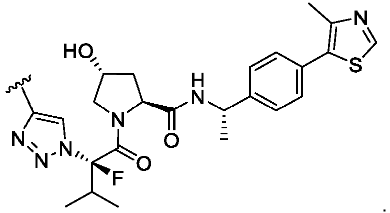

In some embodiments, the degradation moiety is

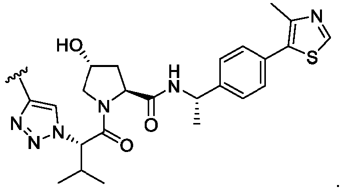

In some embodiments, the degradation moiety is

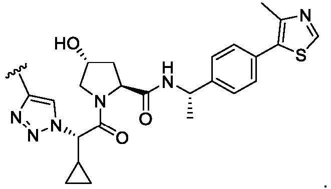

In some embodiments, the degradation moiety is

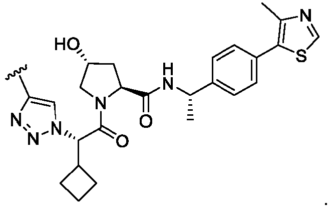

In some embodiments, the degradation moiety is

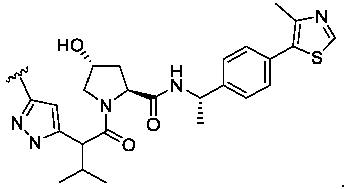

In some embodiments, the degradation moiety is

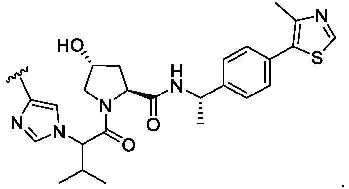

In some embodiments, the degradation moiety is

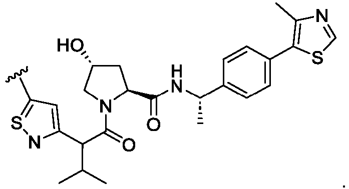

In some embodiments, the degradation moiety is

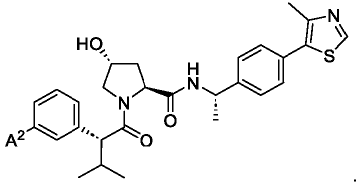

In some embodiments, the degradation moiety is

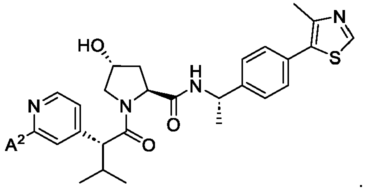

In some embodiments, the linker has the structure of **Formula II:**

A¹-(B¹)_{f}-(C¹)_{g}-(B²)ₕ-(D)-(B³)ᵢ-(C²)ⱼ-(B⁴)ₖ-A², **Formula II**

or a pharmaceutically acceptable salt thereof,
where
A¹ is a bond between the linker and ring system A;
A² is a bond between the degradation moiety and the linker;
each of B¹, B², B³, and B⁴ is, independently, optionally substituted C₁-C₄ alkyl, optionally substituted C₆-C₁₀ aryl, optionally substituted C₆-C₁₀ aryl C₁₋₄ alkyl, optionally substituted C₁-C₄ heteroalkyl, optionally substituted C₃-C₁₀ cycloalkyl, optionally substituted C₃-C₁₀ carbocyclyl, optionally substituted C₂-C₈ heterocyclyl, optionally substituted C₂-C₆ heteroaryl, optionally substituted C₆₋₁₂ aryl, O, S, S(O)₂, or NR^{N};
each R^{N} is, independently, H, optionally substituted C₁₋₄ alkyl, optionally substituted C₂₋₄ alkenyl, optionally substituted C₂₋₄ alkynyl, optionally substituted C₂₋₁₀ heterocyclyl, optionally substituted C₂₋₆ heteroaryl, or optionally substituted C₁₋₇ heteroalkyl;
each of C¹ and C² is, independently, carbonyl, thiocarbonyl, sulphonyl, or phosphoryl;
each of f, g, h, i, j, and k is, independently, 0 or 1; and
D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₂₋₆ heteroaryl, optionally substituted C₆₋₁₂ aryl, optionally substituted C₂-C₁₀ polyethylene glycol, optionally substituted C₃-C₁₀ cycloalkyl, optionally substituted C₃-C₁₀ carbocyclyl, or optionally substituted C₁₋₁₀ heteroalkyl; or D is absent, and the linker is A¹-(B¹)_{f}-(C¹)_{g}-(B²)ₕ-(B³)ᵢ-(C²)ⱼ-(B⁴)ₖ-A².

In some embodiments, each of B¹, B², B³, and B⁴ is, independently, optionally substituted C₁-C₂ alkyl, optionally substituted C₁-C₃ heteroalkyl, optionally substituted C₂-C₁₀ heterocyclyl, optionally substituted C₂₋₆ heteroaryl, O, or NR^{N}; and D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₆₋₁₂ aryl, optionally substituted C₂-C₁₀ polyethylene glycol, or optionally substituted C₁₋₁₀ heteroalkyl, or a chemical bond linking A¹-(B¹)_{f}-(C¹)_{g}-(B²)ₕ- to -(B³)ᵢ-(C²)ⱼ-(B⁴)ₖ-A².

In some embodiments, each of B¹, B², B³, and B⁴ is, independently, optionally substituted C₁-C₂ alkyl, optionally substituted C₁-C₃ heteroalkyl, optionally substituted C₂-C₁₀ heterocyclyl, optionally substituted C₂₋₆ heteroaryl, optionally substituted C₃-C₁₀ cycloalkyl, optionally substituted C₃-C₁₀ carbocyclyl, O, or NR^{N}.

In some embodiments, each of B¹ and B⁴ is, independently,

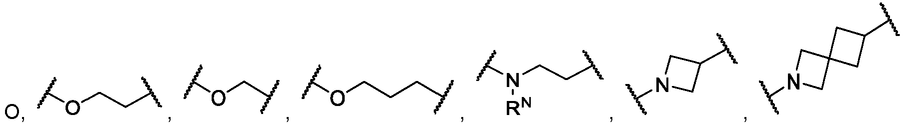

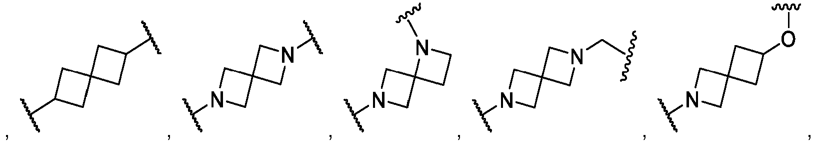

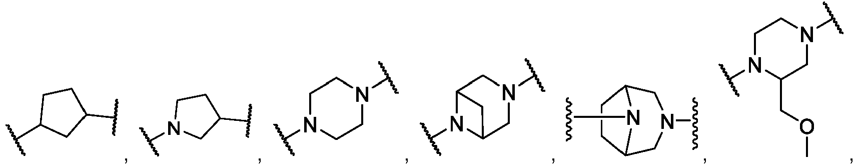

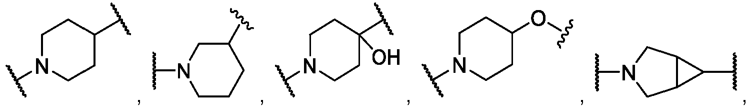

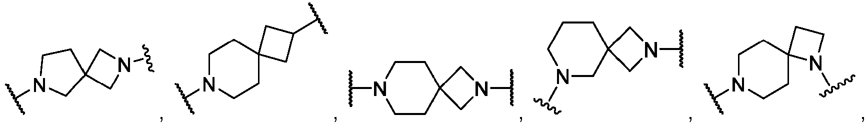

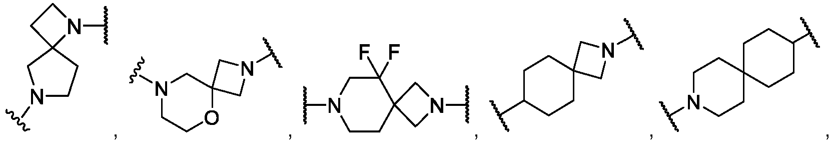

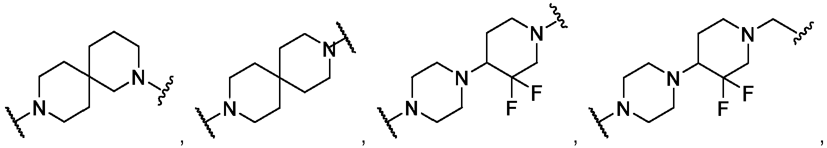

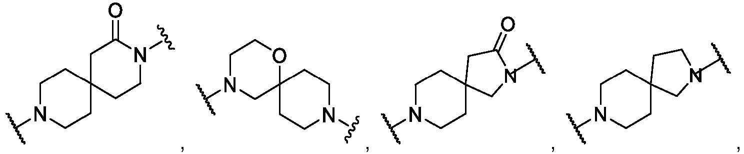

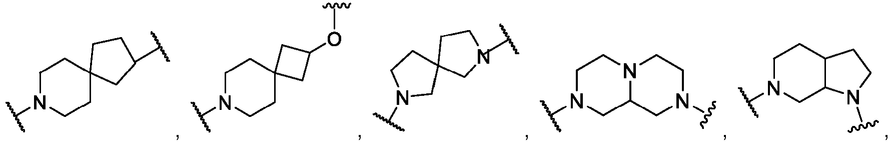

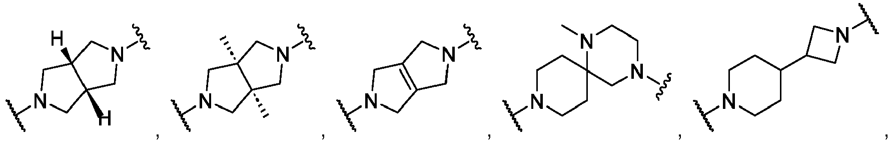

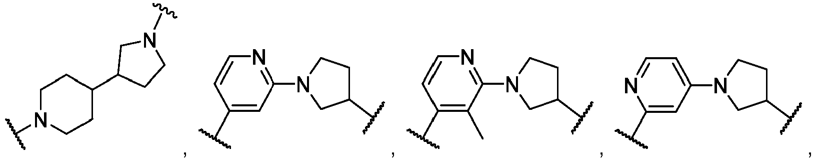

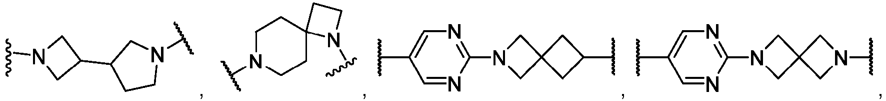

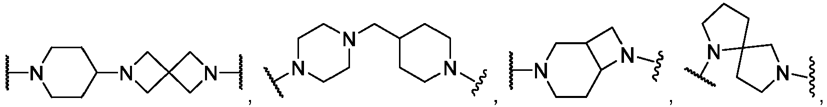

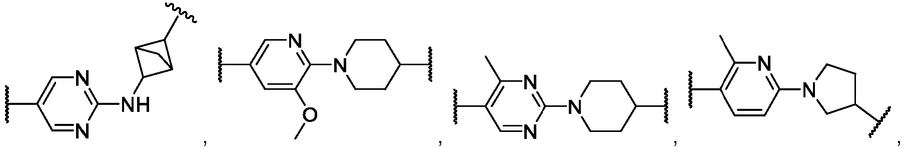

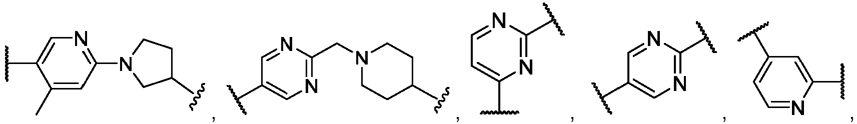

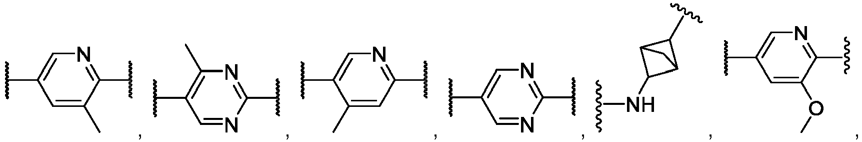

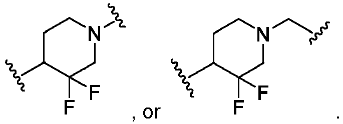

In some embodiments, B¹ is

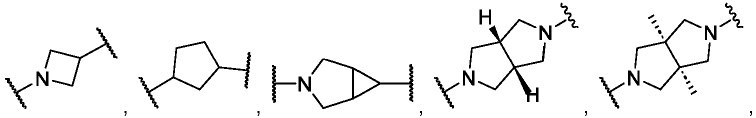

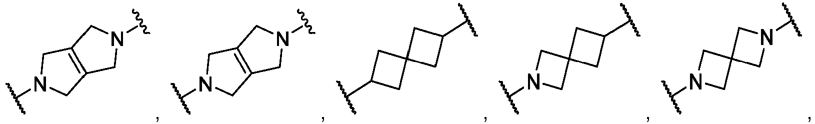

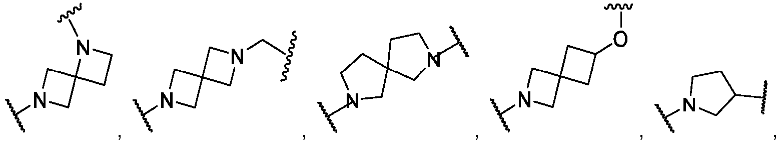

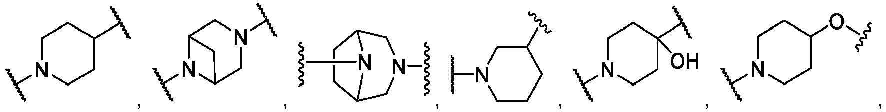

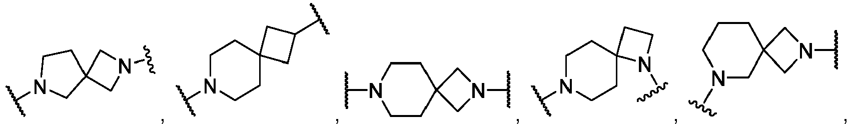

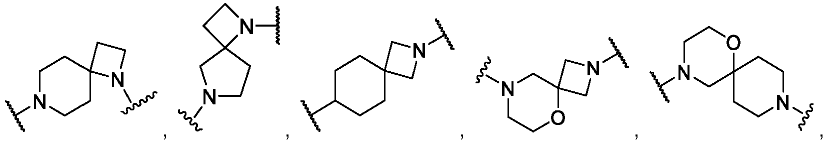

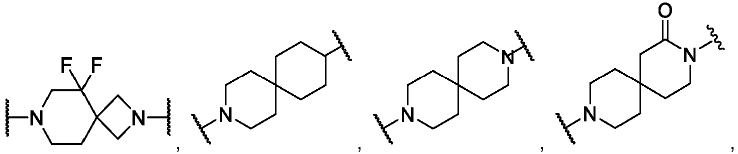

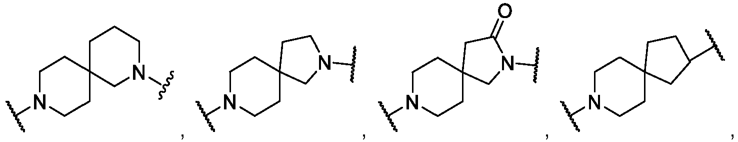

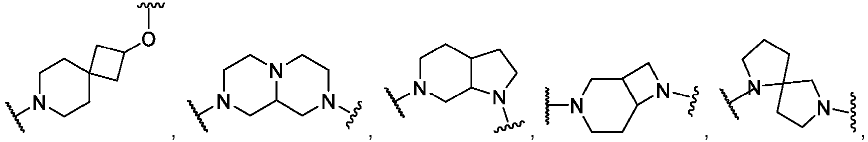

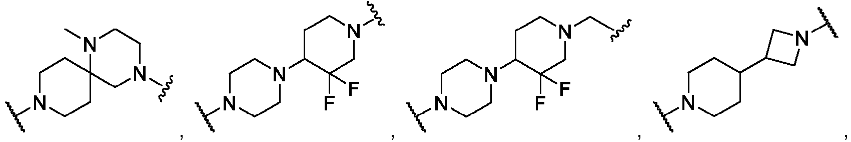

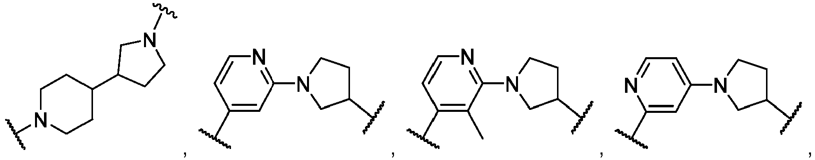

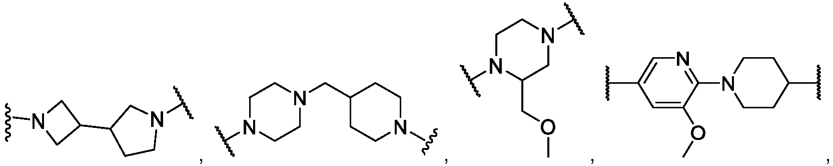

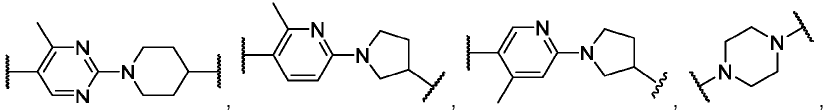

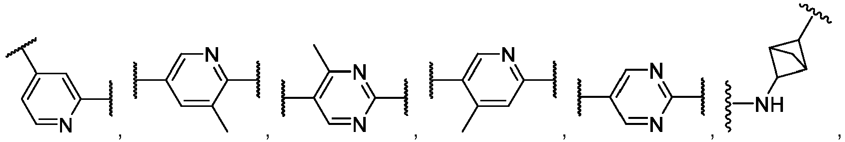

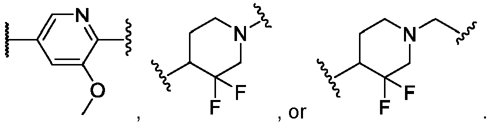

In some embodiments, B⁴ is O,

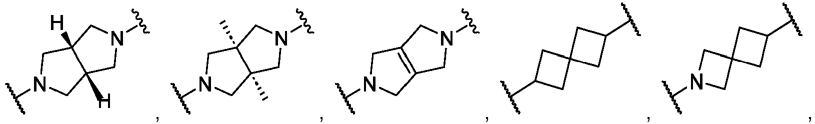

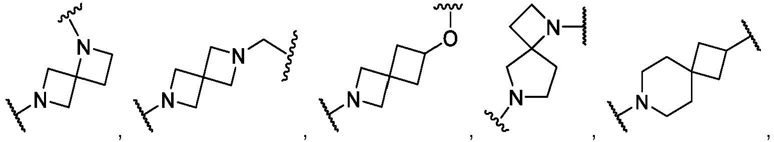

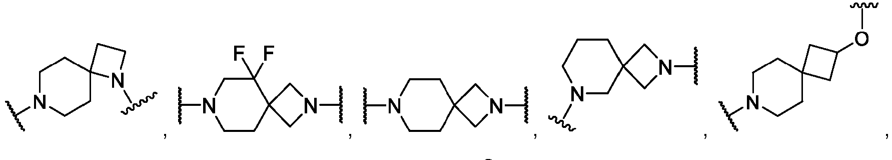

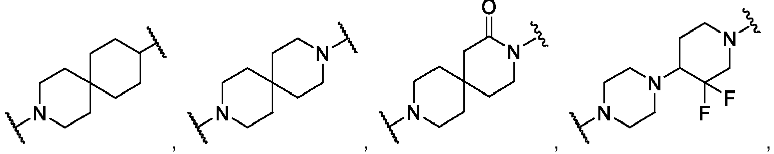

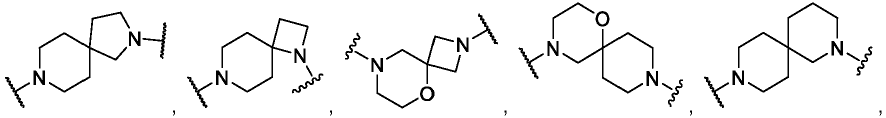

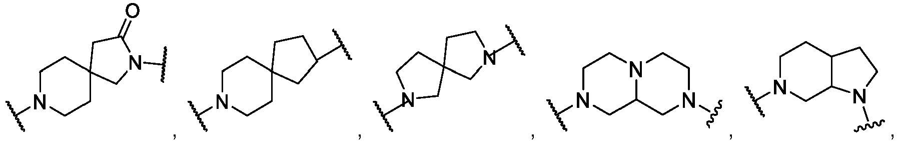

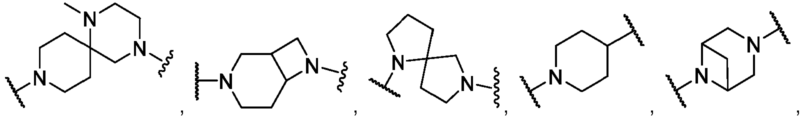

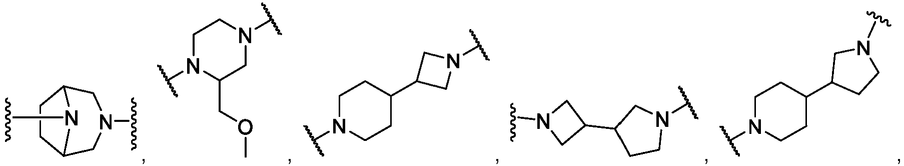

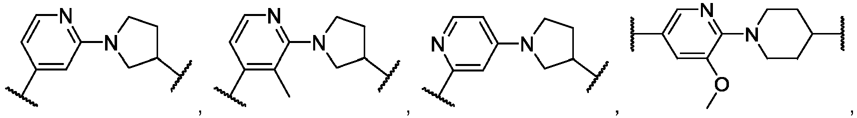

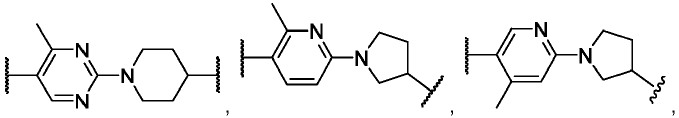

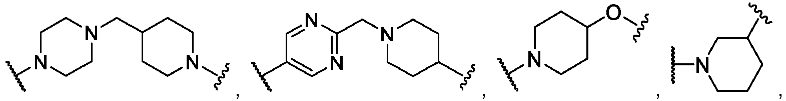

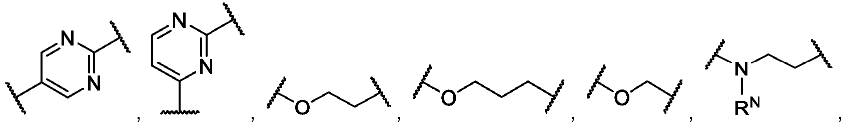

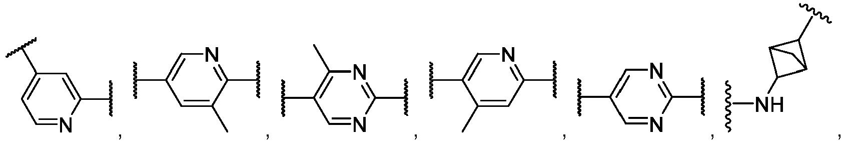

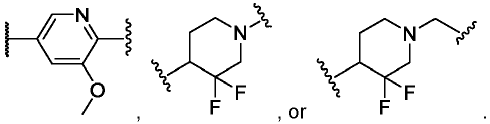

In some embodiments, C¹ is

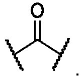

In some embodiments, B² is optionally substituted C₁-C₄ alkyl.

In some embodiments, D is optionally substituted C₁-C₁₀ alkyl.

In some embodiments, f is 1. In some embodiments, g is 0. In some embodiments, g is 1. In some embodiments, h is 0. In some embodiments, h is 1. In some embodiments, i is 0. In some embodiments, i is 1. In some embodiments, j is 0. In some embodiments, j is 1. In some embodiments, k is 0. In some embodiments, k is 1.

In some embodiments, D is absent, and the linker is A¹-(B¹)_{f}-(C¹)_{g}-(B²)ₕ-(B³)ᵢ-(C²)ⱼ-(B⁴)ₖ-A².

In some embodiments, the linker is D. In some embodiments, D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₂₋₆ heteroaryl, optionally substituted C₆₋₁₂ aryl, optionally substituted C₂-C₁₀ polyethylene glycol, or optionally substituted C₁₋₁₀ heteroalkyl. In some embodiments, D is optionally substituted C₃-C₁₀ cycloalkyl, f is 1, g is 0, h is 0, i is 0, j is 0, and, k is 1. In some embodiments, D is optionally substituted C₃-C₁₀ cycloalkyl, f is 1, g is 0, h is 0, i is 0, j is 0, and, k is 0. In some embodiments, D is optionally substituted C₃-C₁₀ cycloalkyl, f is 0, g is 0, h is 0, i is 0, j is 0, and, k is 1. In some embodiments, D is optionally substituted C₃-C₁₀ cycloalkyl, f is 0, g is 0, h is 0, i is 0, j is 0, and, k is 0. In some embodiments, D is optionally substituted C₃-C₁₀ carbocyclyl, f is 1, g is 0, h is 0, i is 0, j is 0, and, k is 1. In some embodiments, D is optionally substituted C₃-C₁₀ carbocyclyl, f is 1, g is 0, h is 0, i is 0, j is 0, and, k is 0. In some embodiments, D is optionally substituted C₃-C₁₀ carbocyclyl, f is 0, g is 0, h is 0, i is 0, j is 0, and, k is 1. In some embodiments, D is optionally substituted C₃-C₁₀ carbocyclyl, f is 0, g is 0, h is 0, i is 0, j is 0, and, k is 0. In some embodiments, D is:

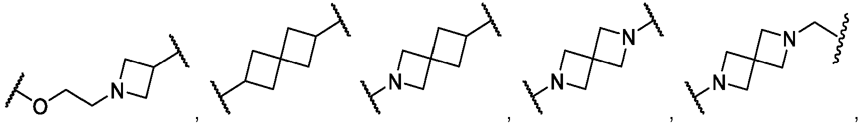

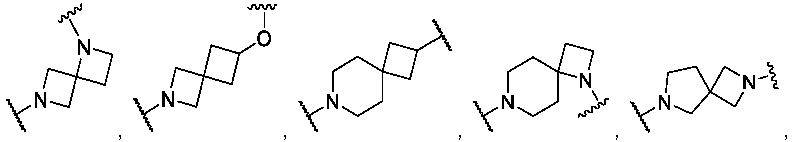

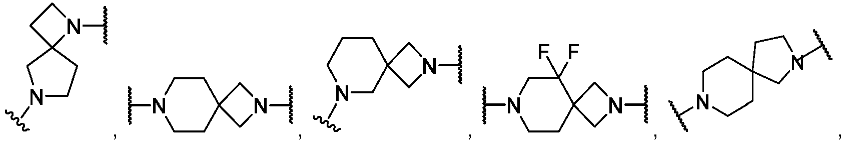

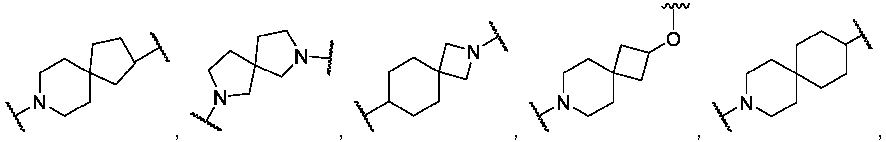

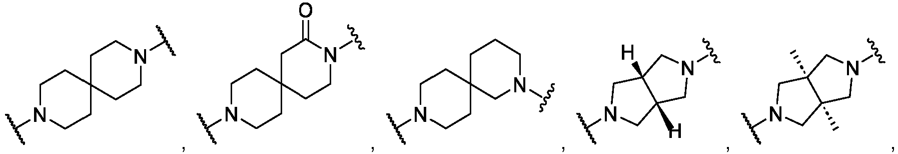

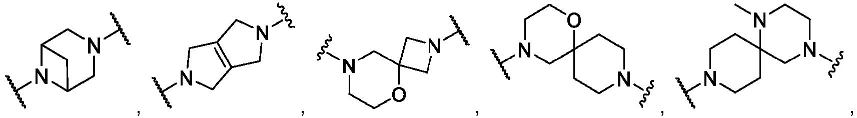

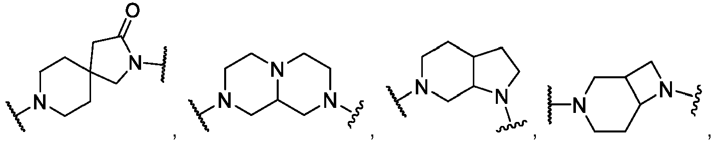

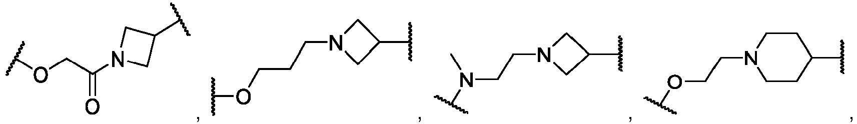

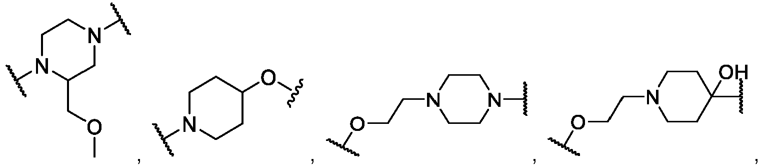

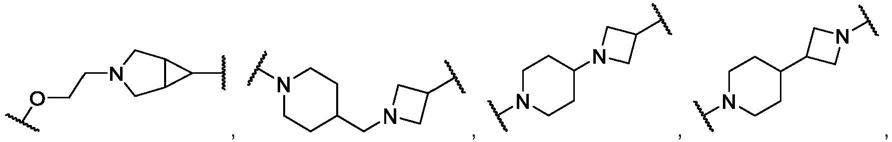

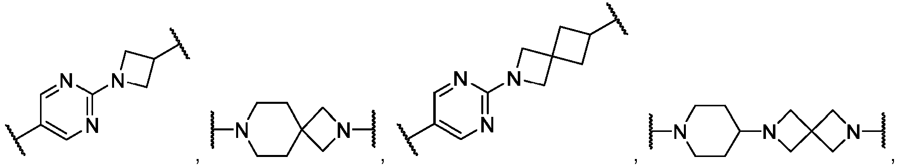

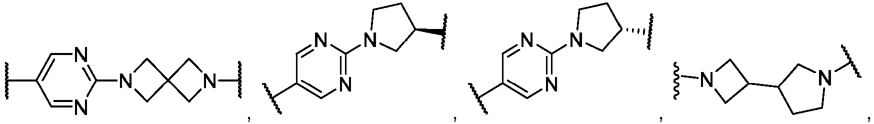

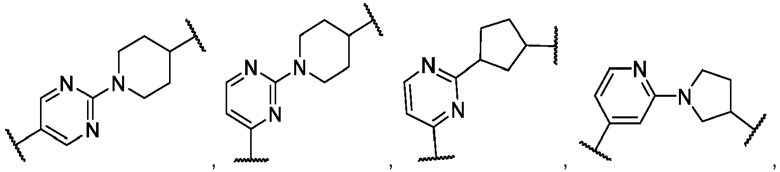

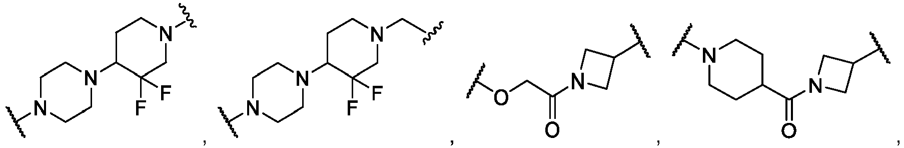

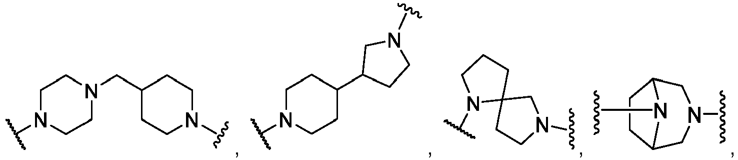

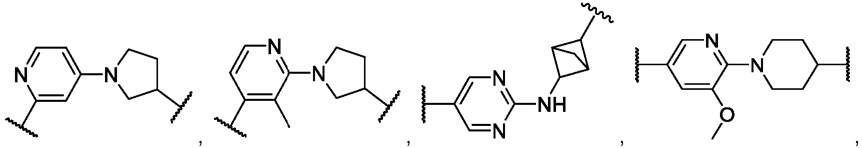

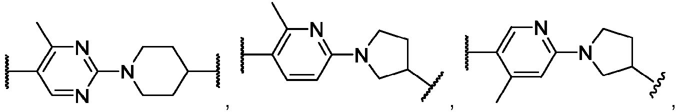

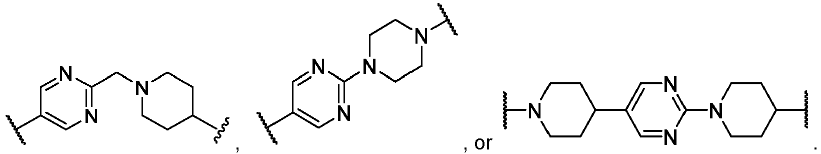

In some embodiments, the linker has the structure of

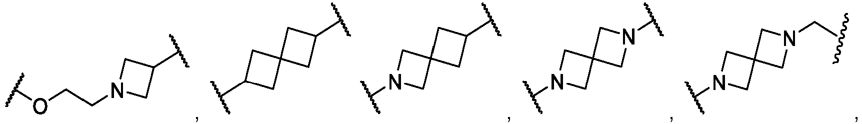

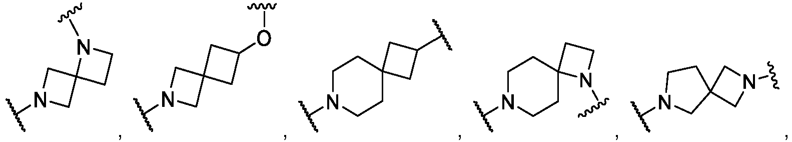

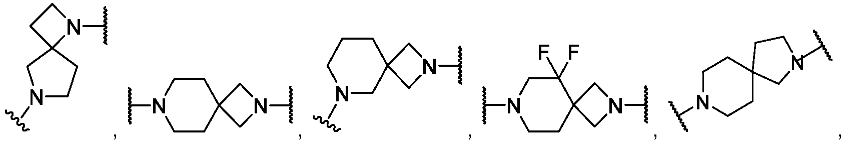

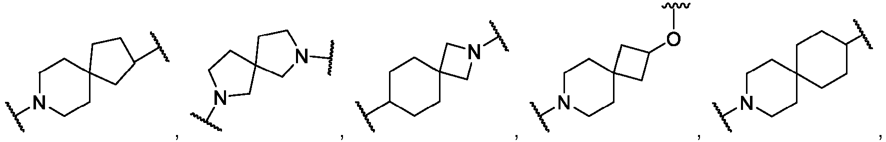

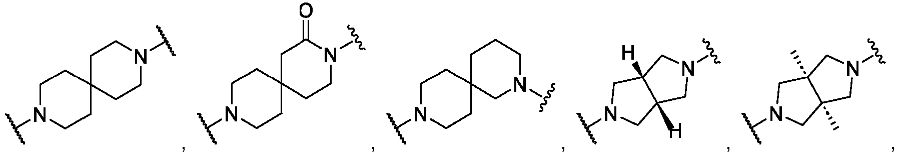

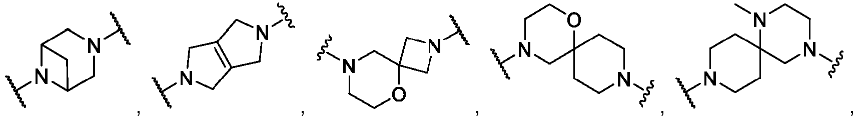

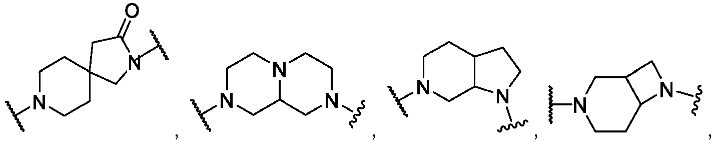

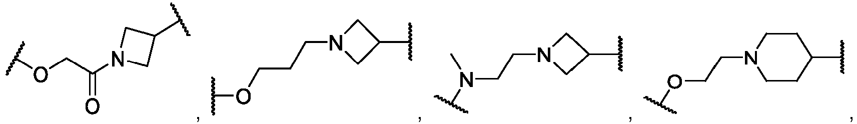

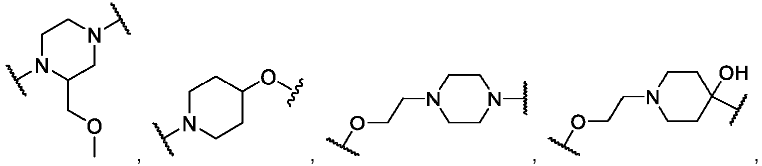

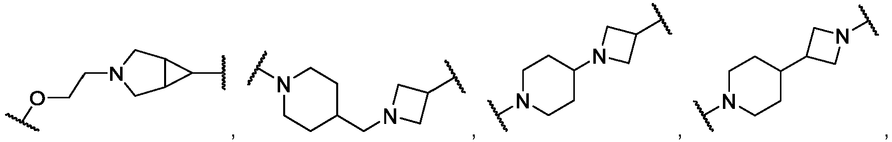

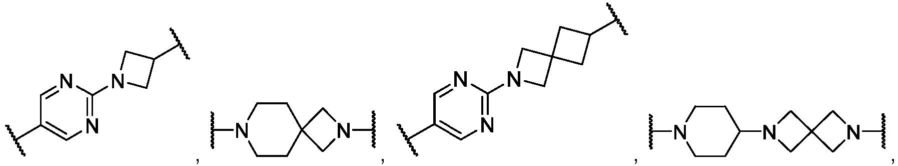

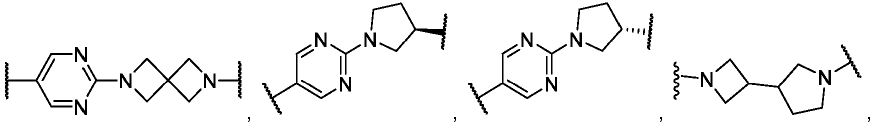

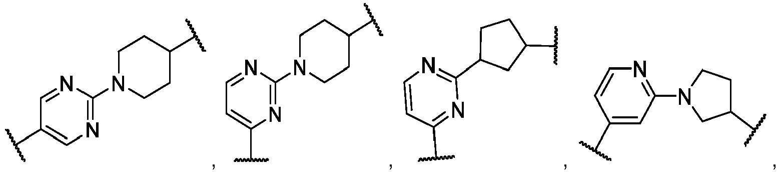

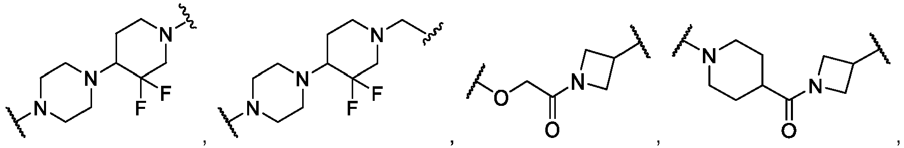

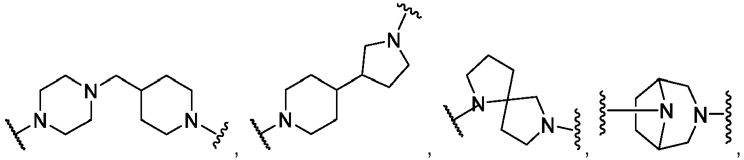

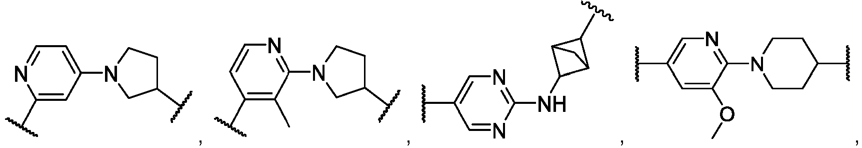

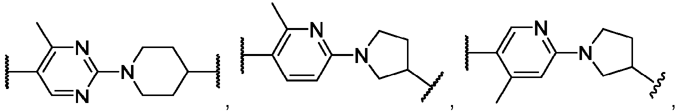

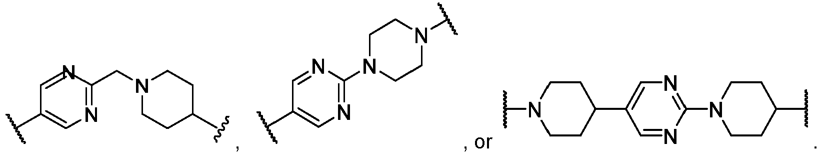

In some embodiments, the shortest chain of atoms connecting two valencies of the linker is 2 to 10 atoms long. In some embodiments, the shortest chain of atoms connecting two valencies of the linker is 6 atoms long.

In some embodiments, the linker has a structure of the linker in any one of compounds 1-291 in Table 1 (e.g., of any of the compounds with a ratio of BRG1 IC₅₀ to BRM IC₅₀ of at least 5 (e.g., at least 7, 10, 15, 20, 25, or 30)). In some embodiments, the linker has a structure of the linker in any one of compounds 1-291 in Table 1 (e.g., of any of the compounds with a BRM IC₅₀ of ++ or better (e.g., +++ or ++++ (e.g., ++++))). In some embodiments, the linker has a structure of the linker in any one of compounds 1-291 in Table 1 (e.g., of any of the compounds with a BRM IC₅₀ of ++ or better (e.g., +++ or ++++ (e.g., ++++)) and with a ratio of BRG1 IC₅₀ to BRM IC₅₀ of at least 5 (e.g., at least 7, 10, 15, 20, 25, or 30)).

In an aspect, the invention features a compound selected from the group consisting of 1-291 in Table 1 and pharmaceutically acceptable salts thereof. In some embodiments, the compound is any one of compounds 1-291 in Table 1 with a ratio of BRG1 IC₅₀ to BRM IC₅₀ of at least 5 (e.g., at least 7, 10, 15, 20, 25, or 30) or a pharmaceutically acceptable salt thereof. In some embodiments, the compound is any one of compounds 1-291 in Table 1 with a BRM IC₅₀ of ++ or better as found in Table 21 (e.g., +++ or ++++ (e.g., ++++)) or a pharmaceutically acceptable salt thereof. In some embodiments, the compound is any one of compounds 1-291 in Table 1 a BRM IC₅₀ of ++ or better as found in Table 21 (e.g., +++ or ++++ (e.g., ++++)) and with a ratio of BRG1 IC₅₀ to BRM IC₅₀ of at least 5 (e.g., at least 7, 10, 15, 20, 25, or 30) or a pharmaceutically acceptable salt thereof.

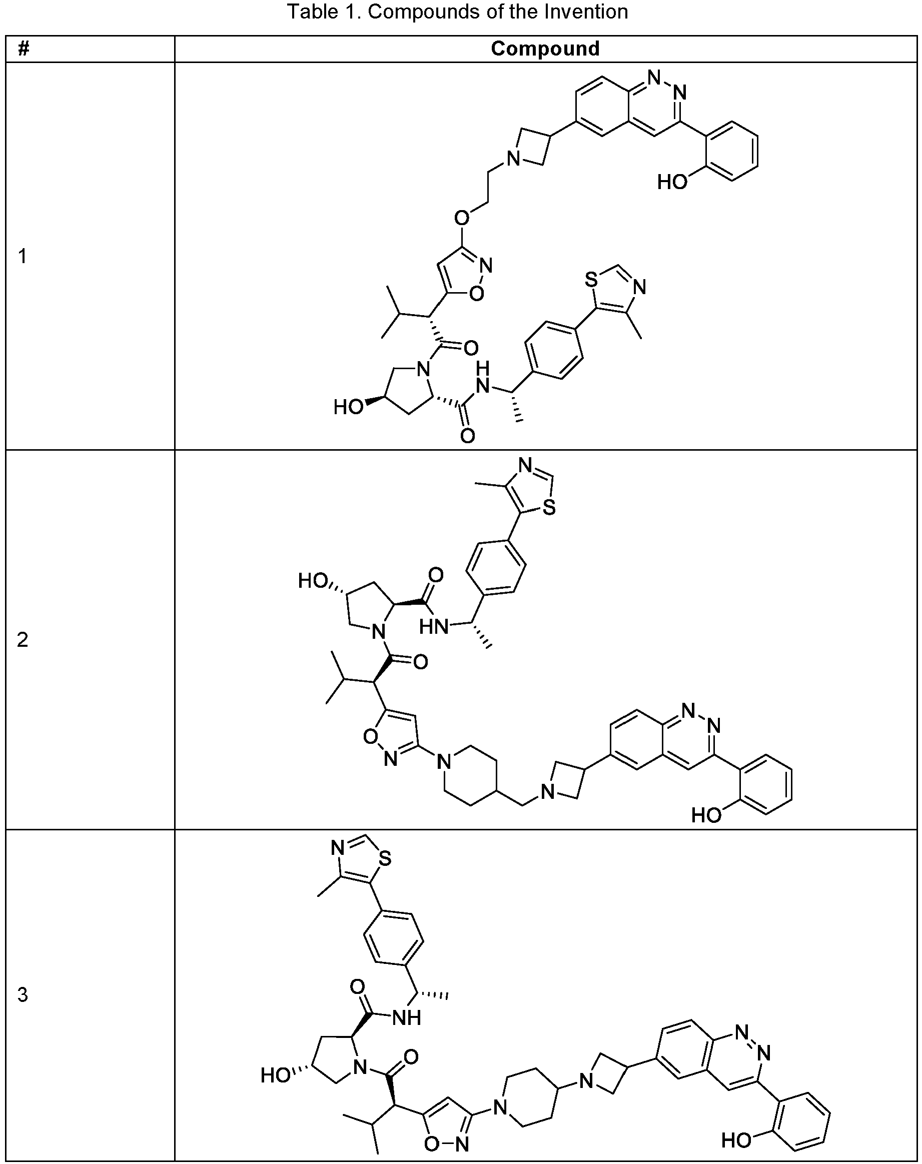

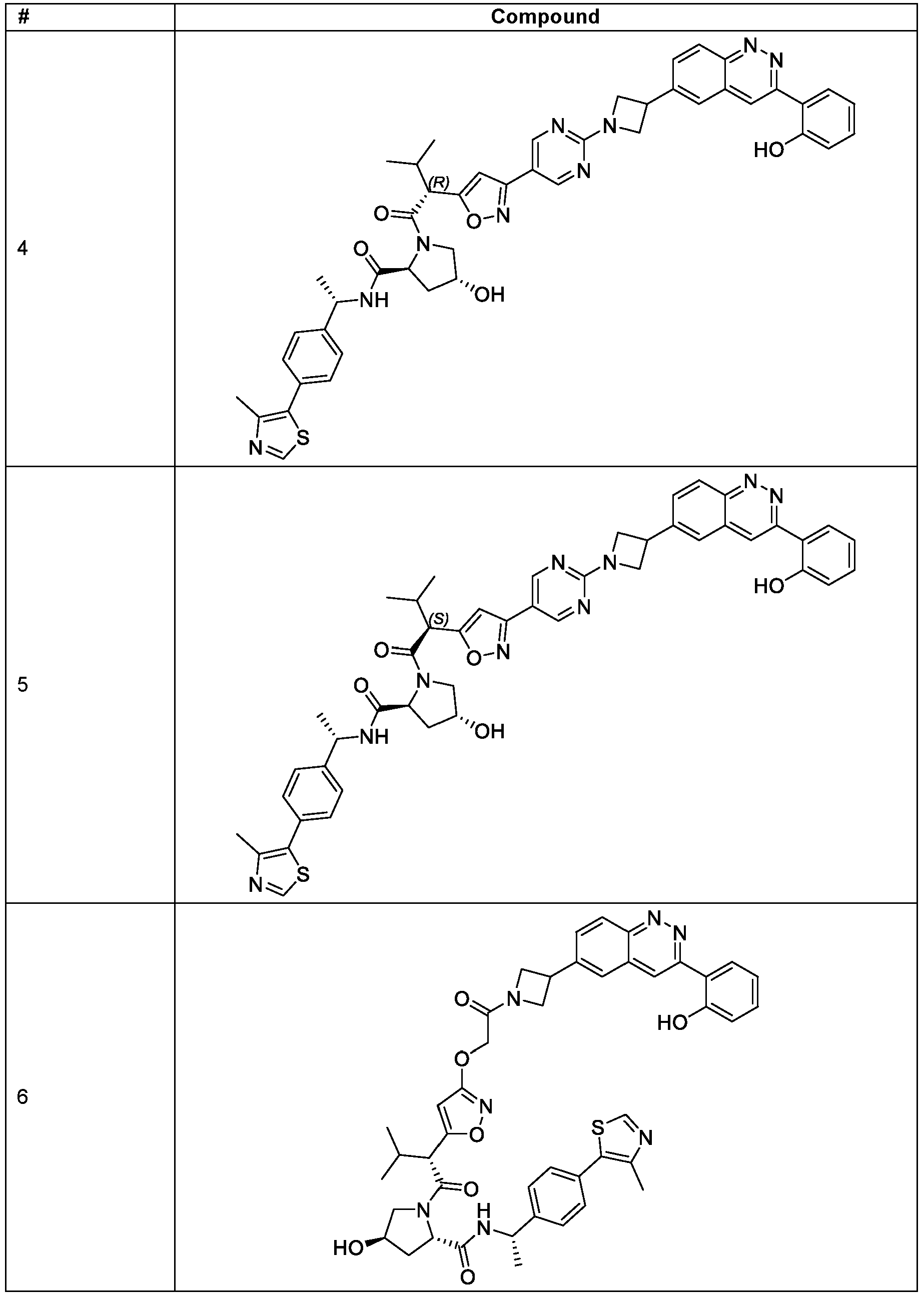

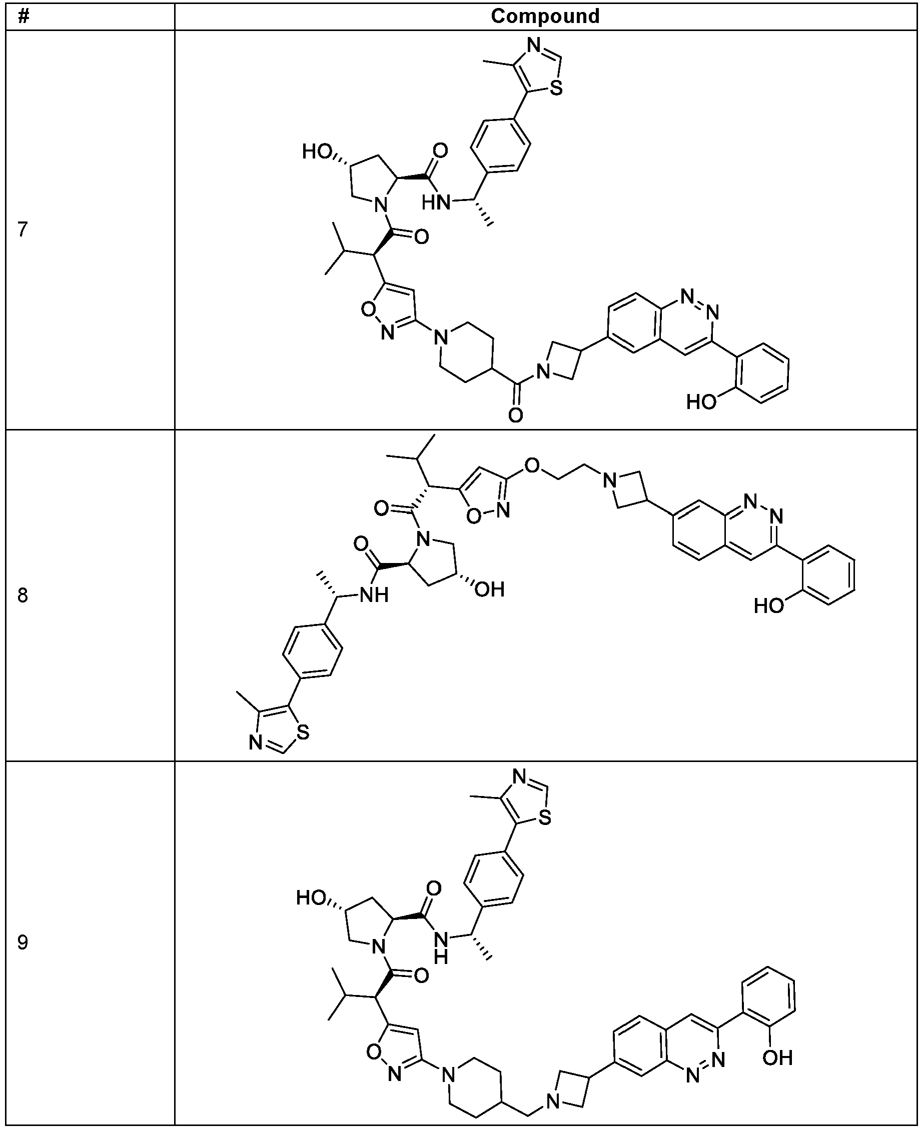

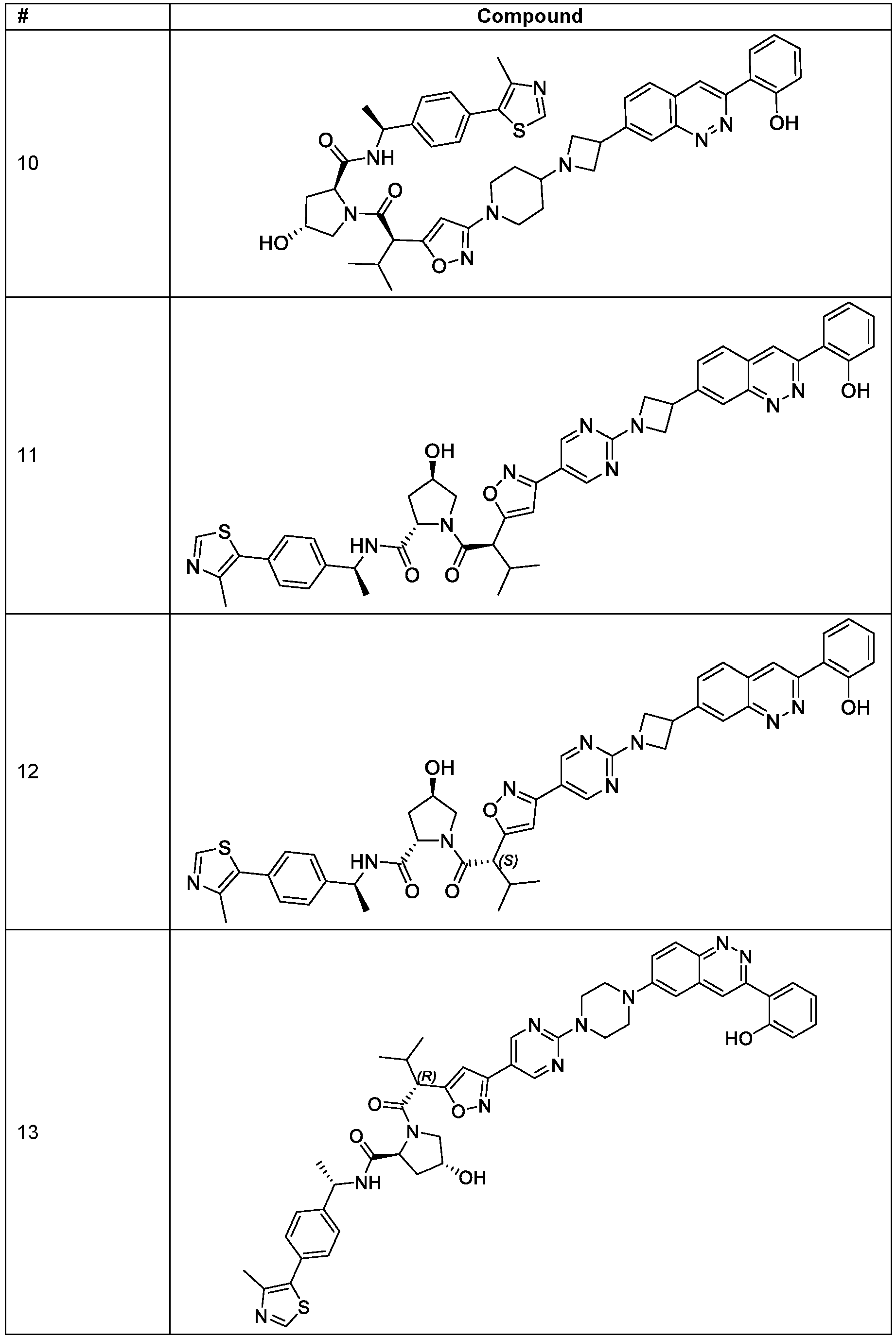

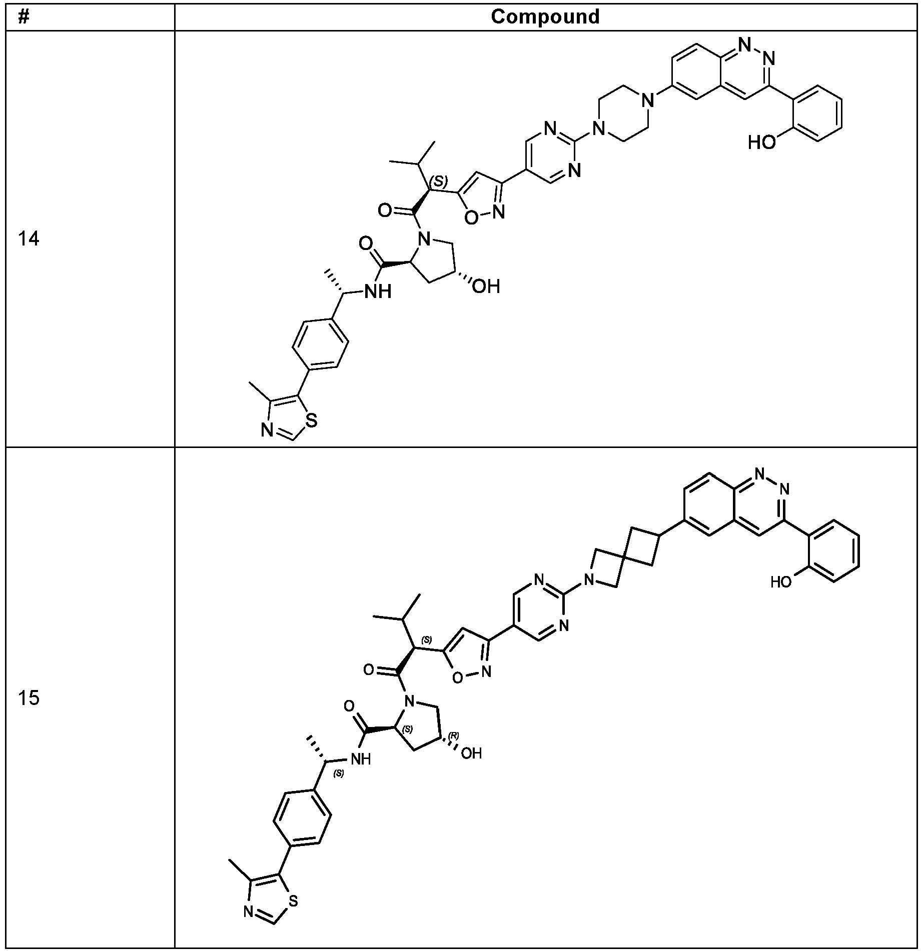

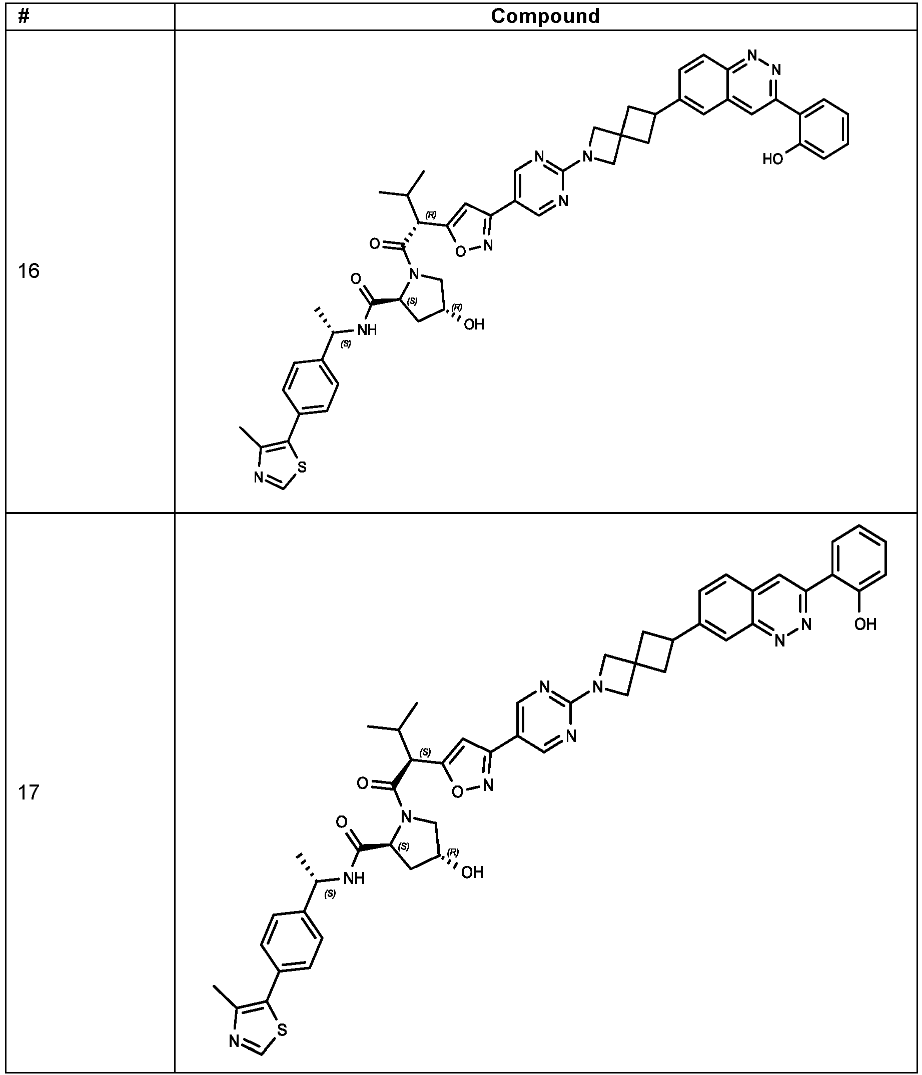

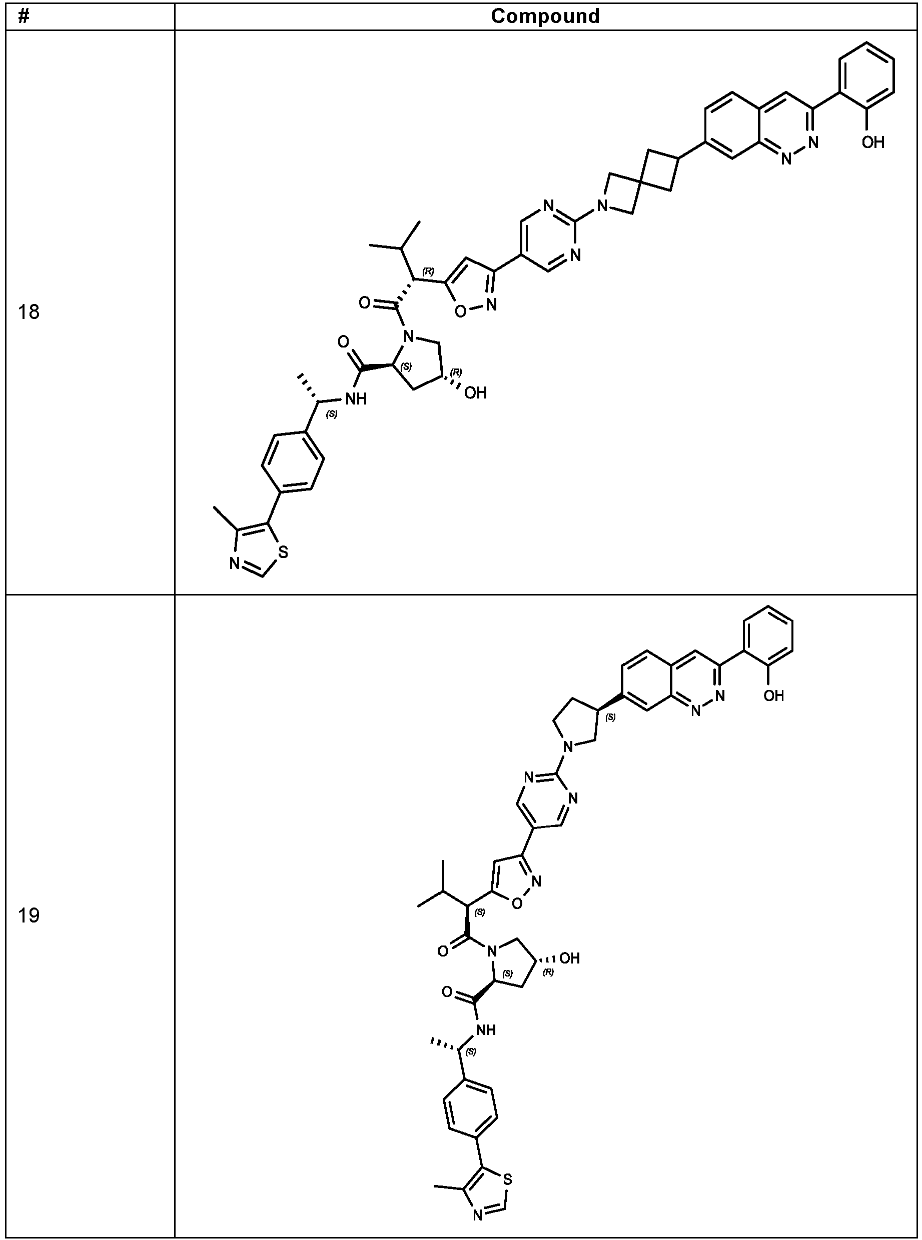

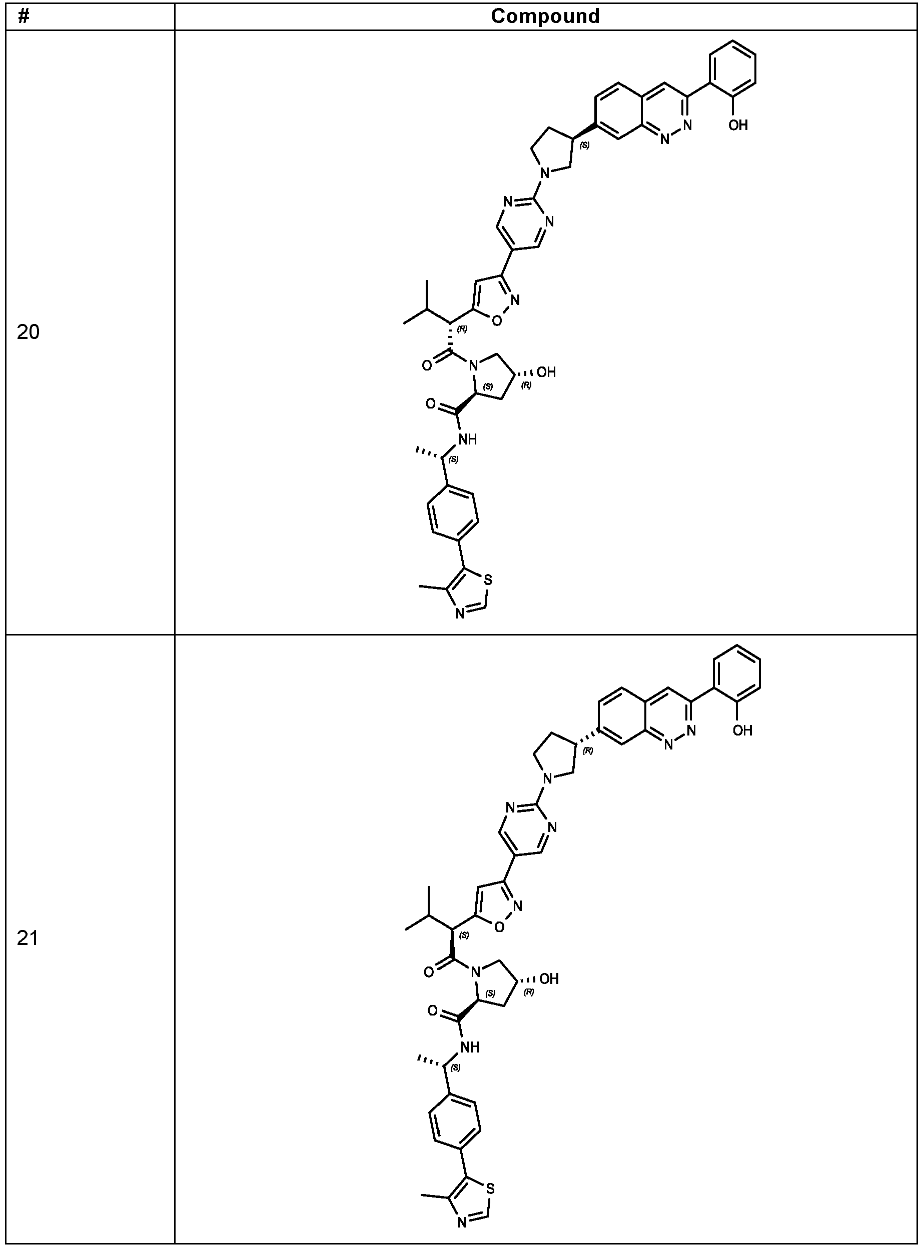

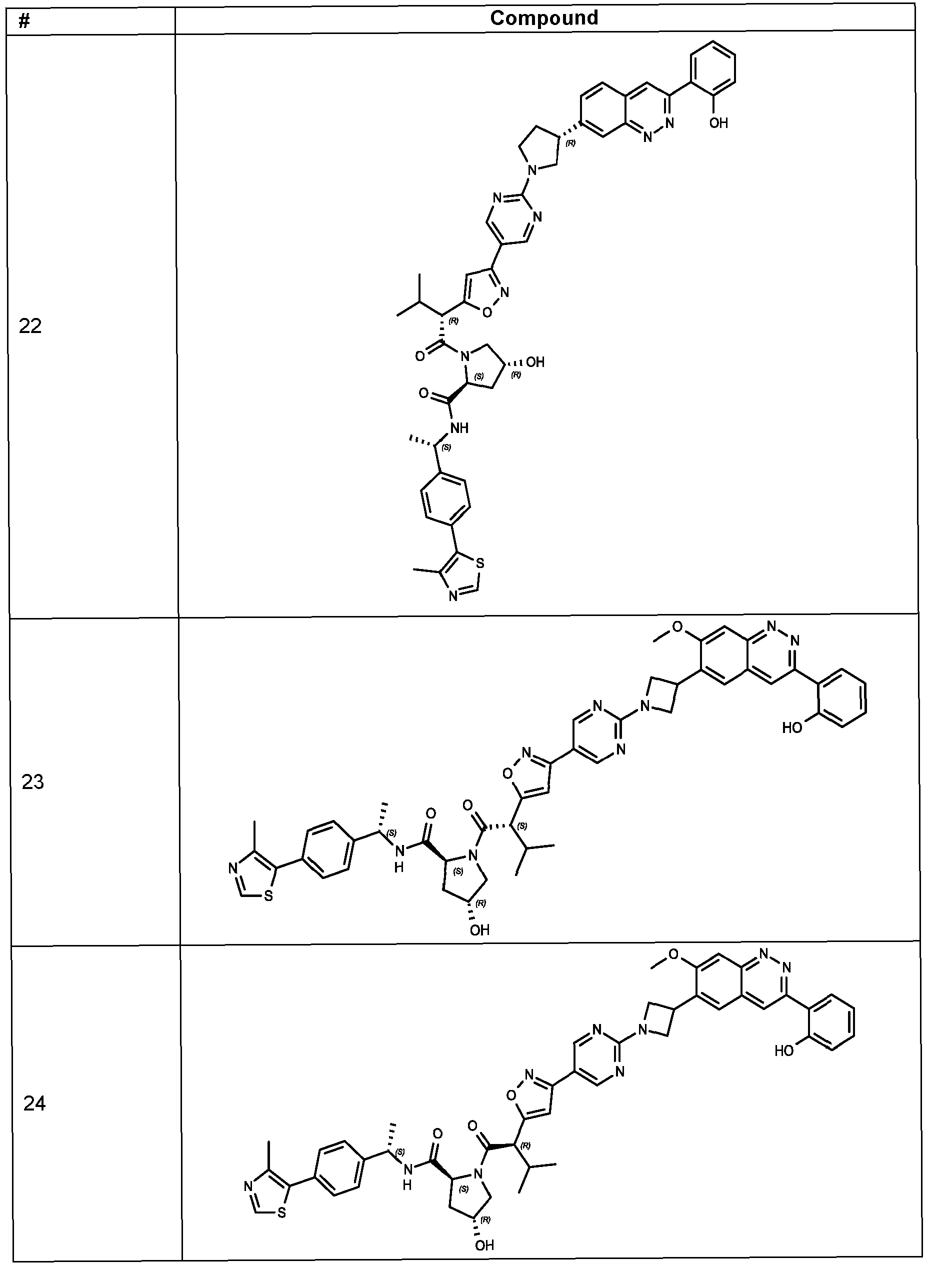

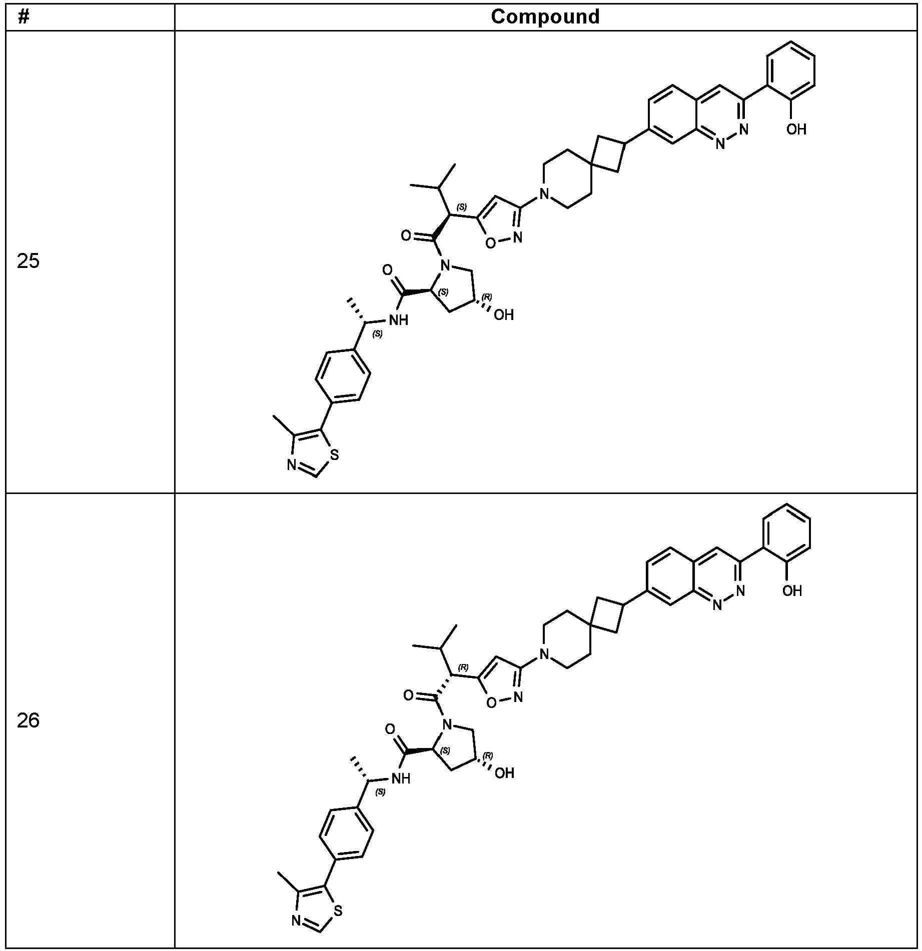

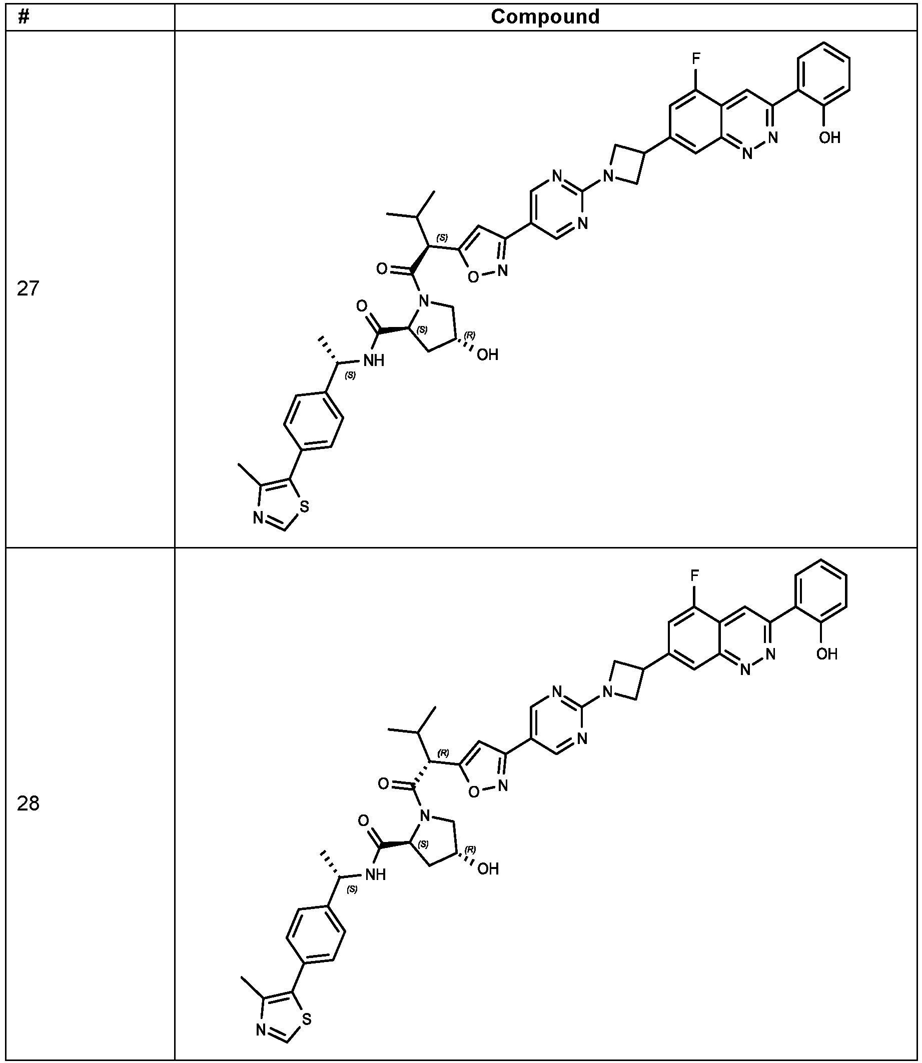

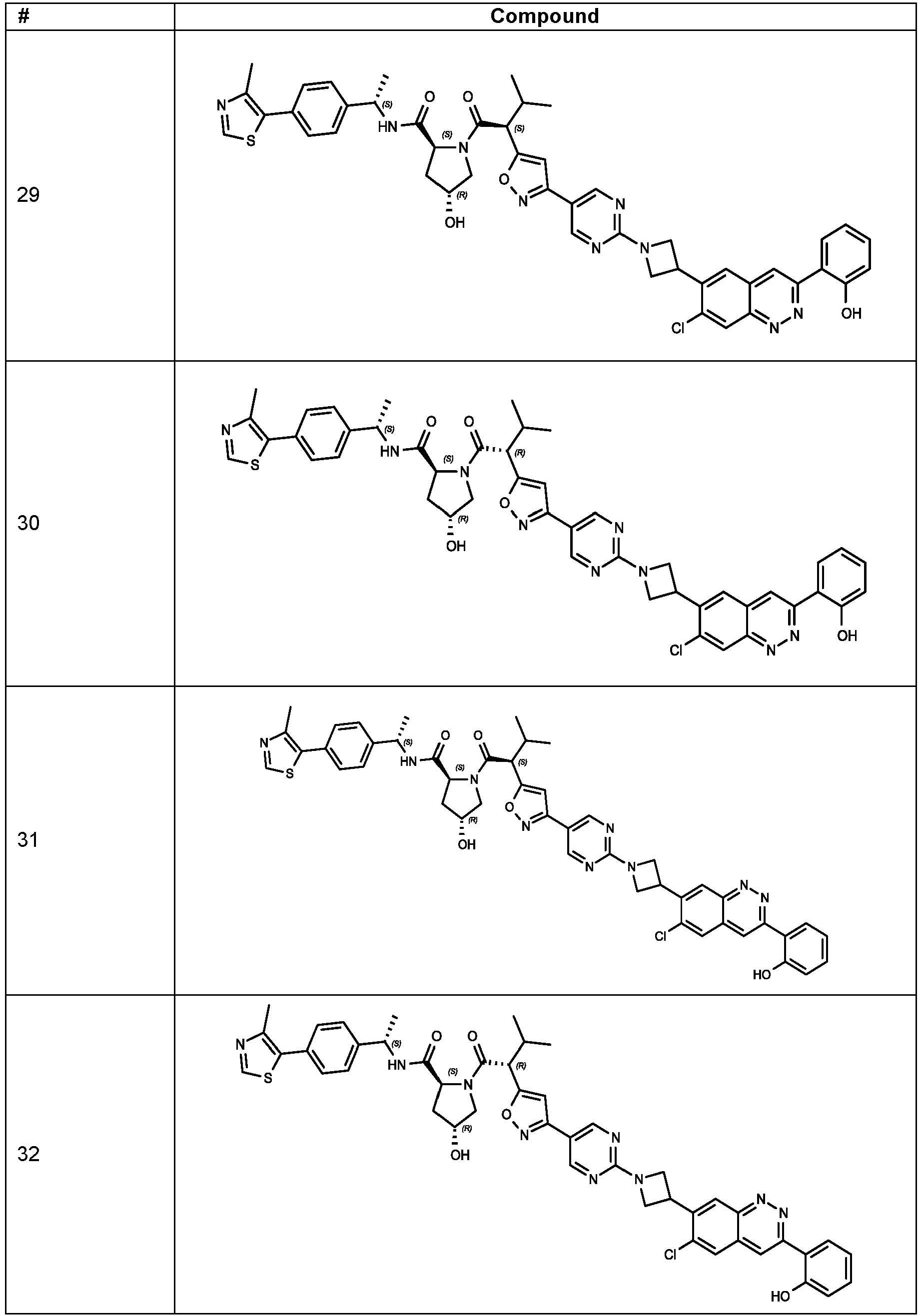

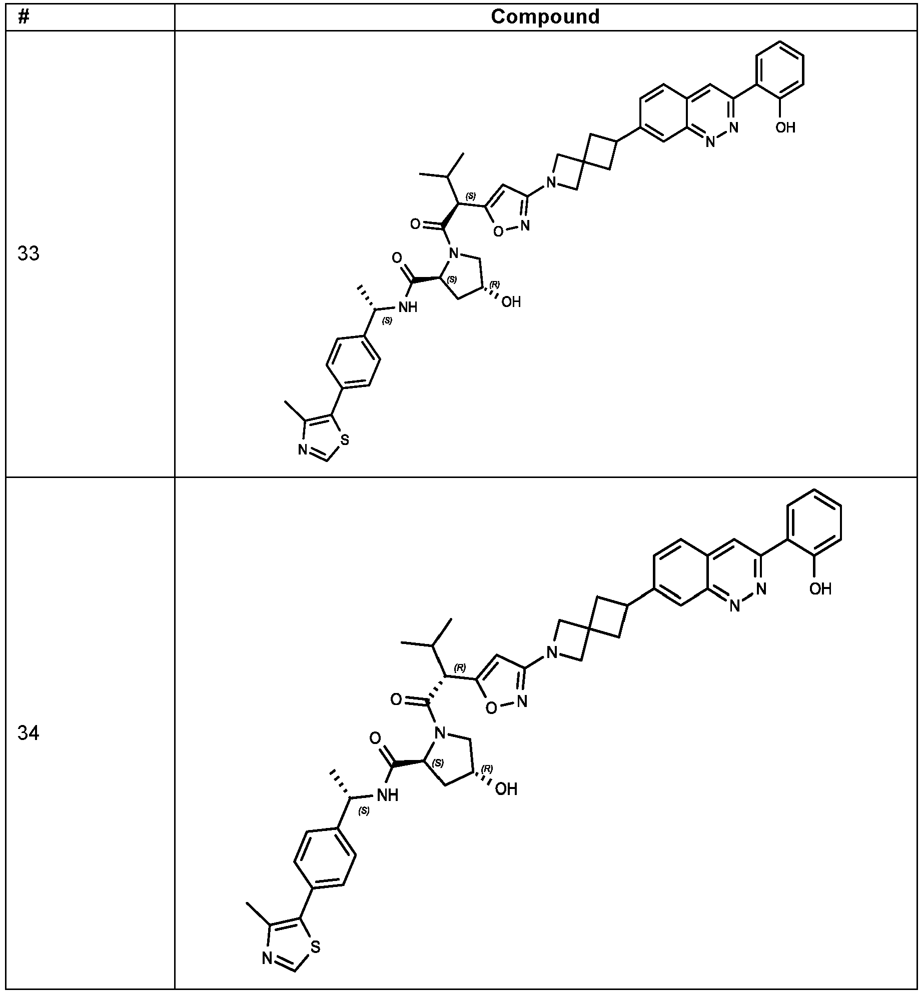

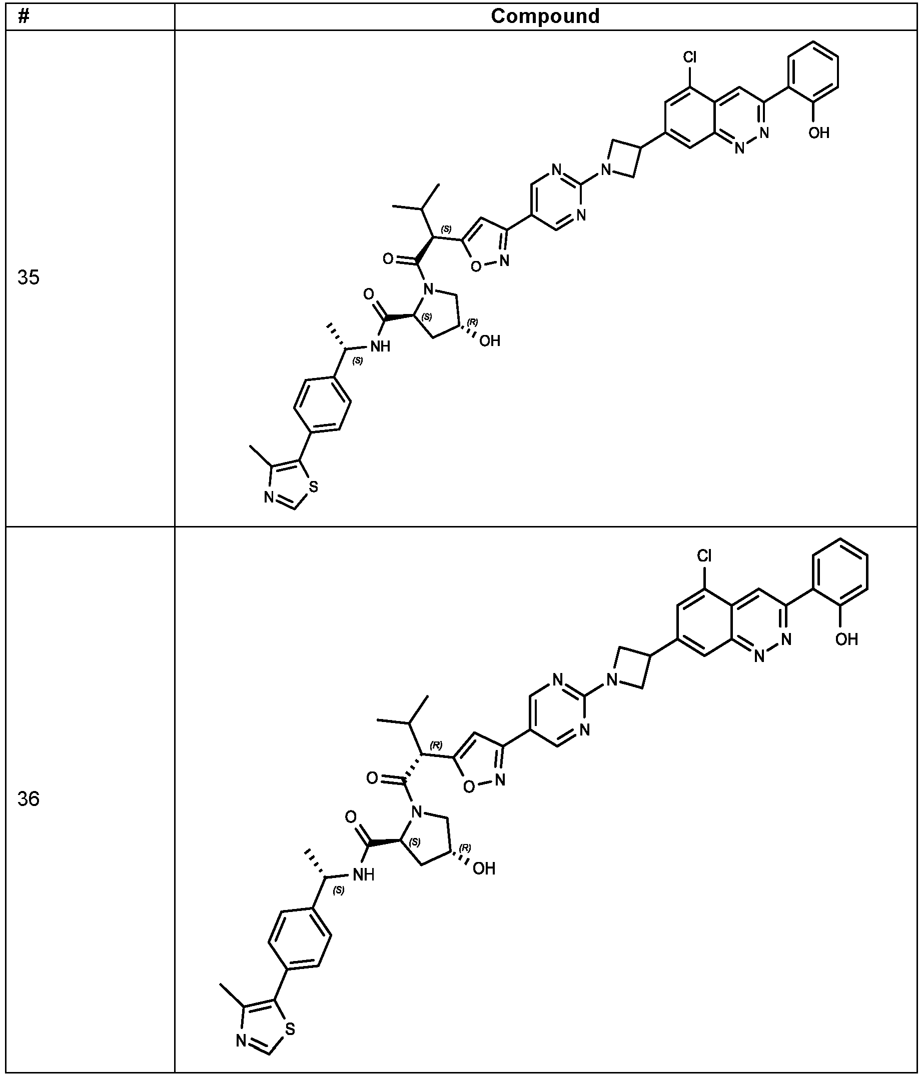

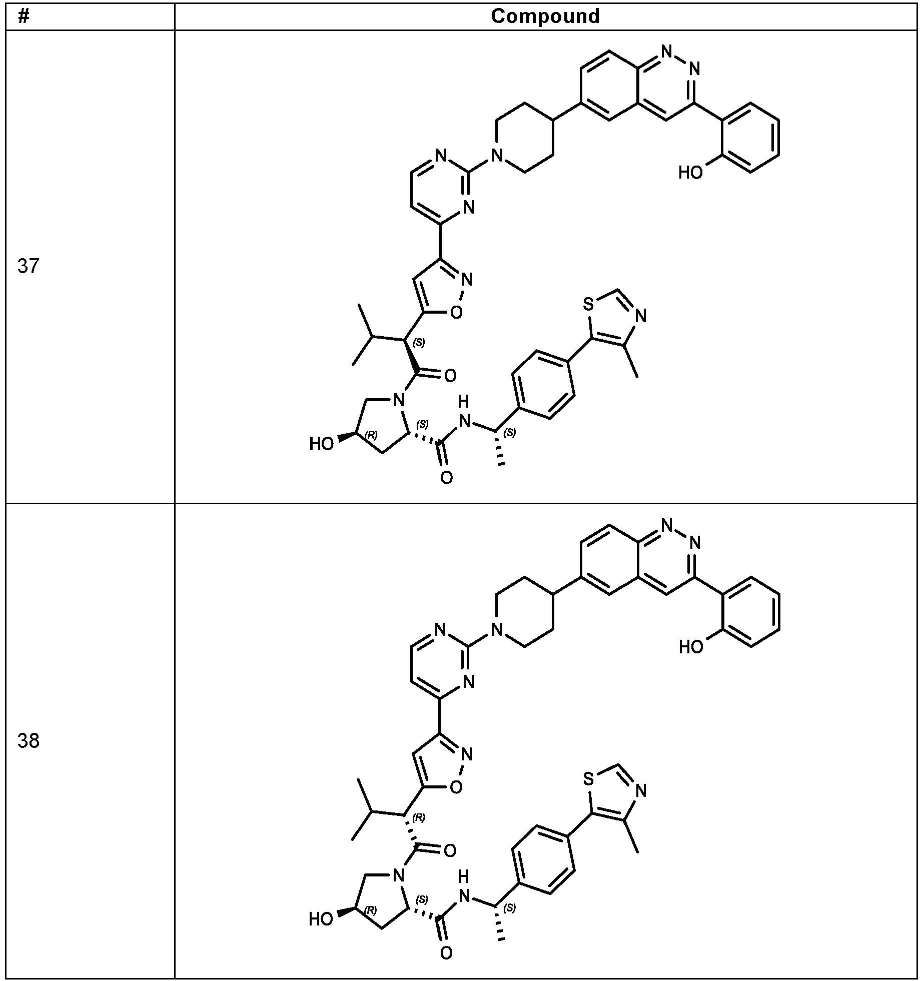

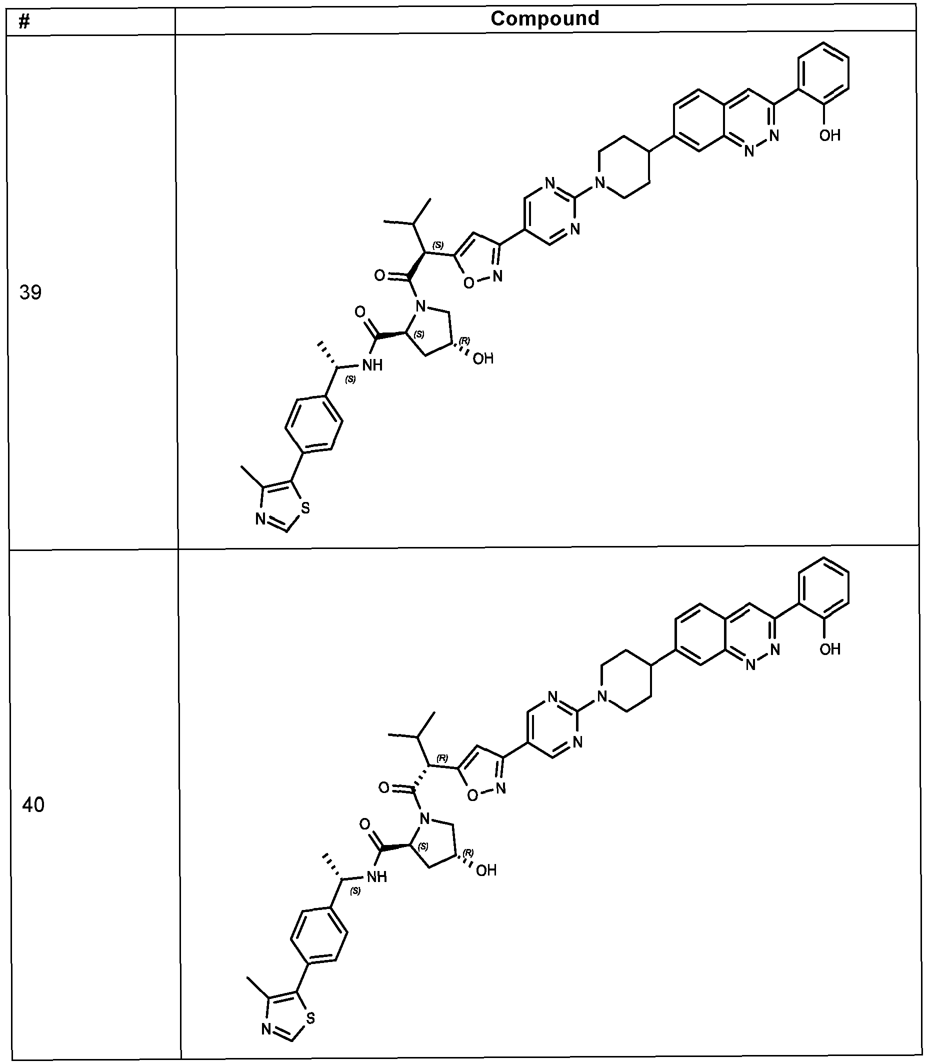

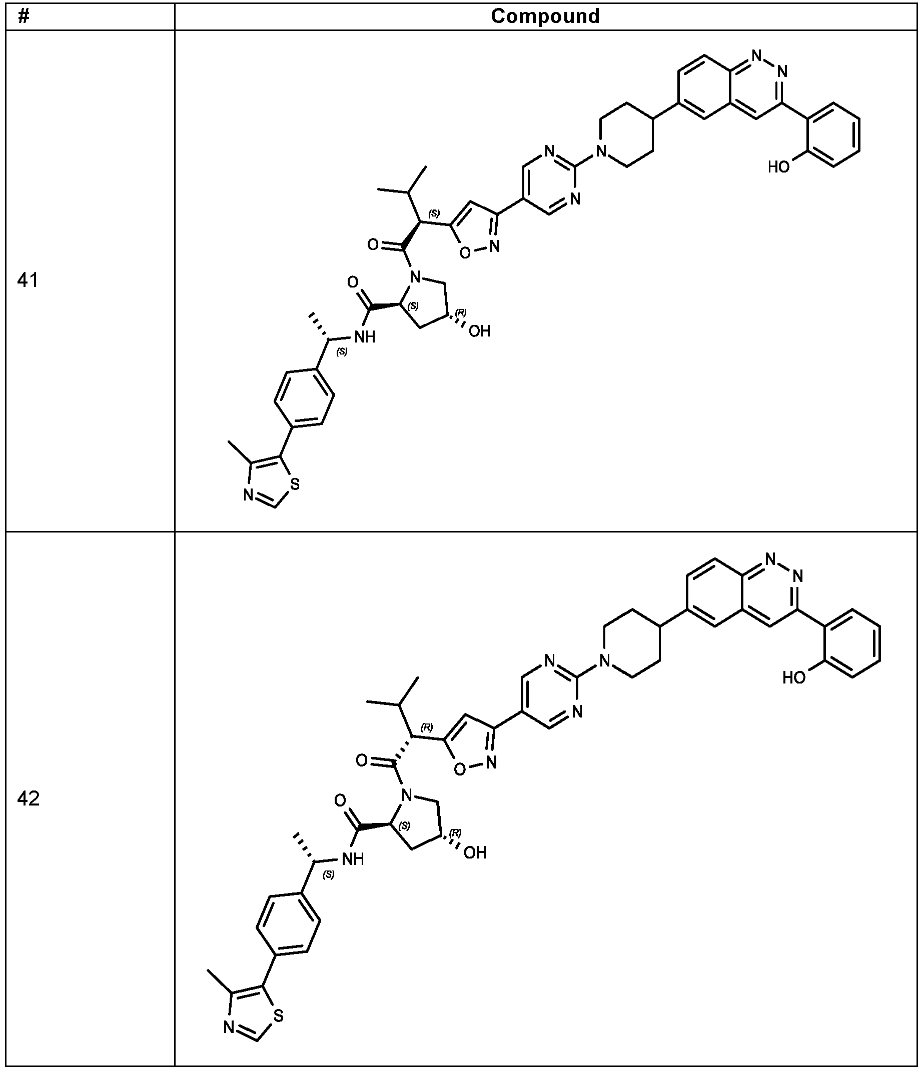

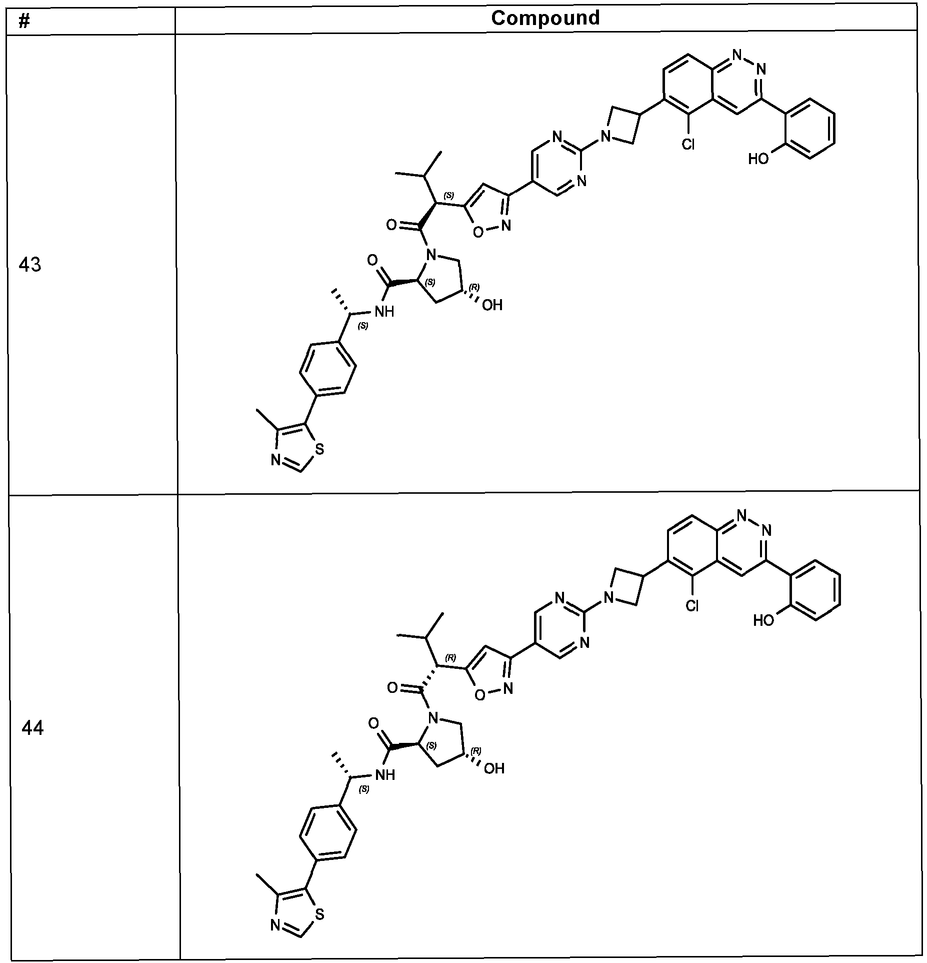

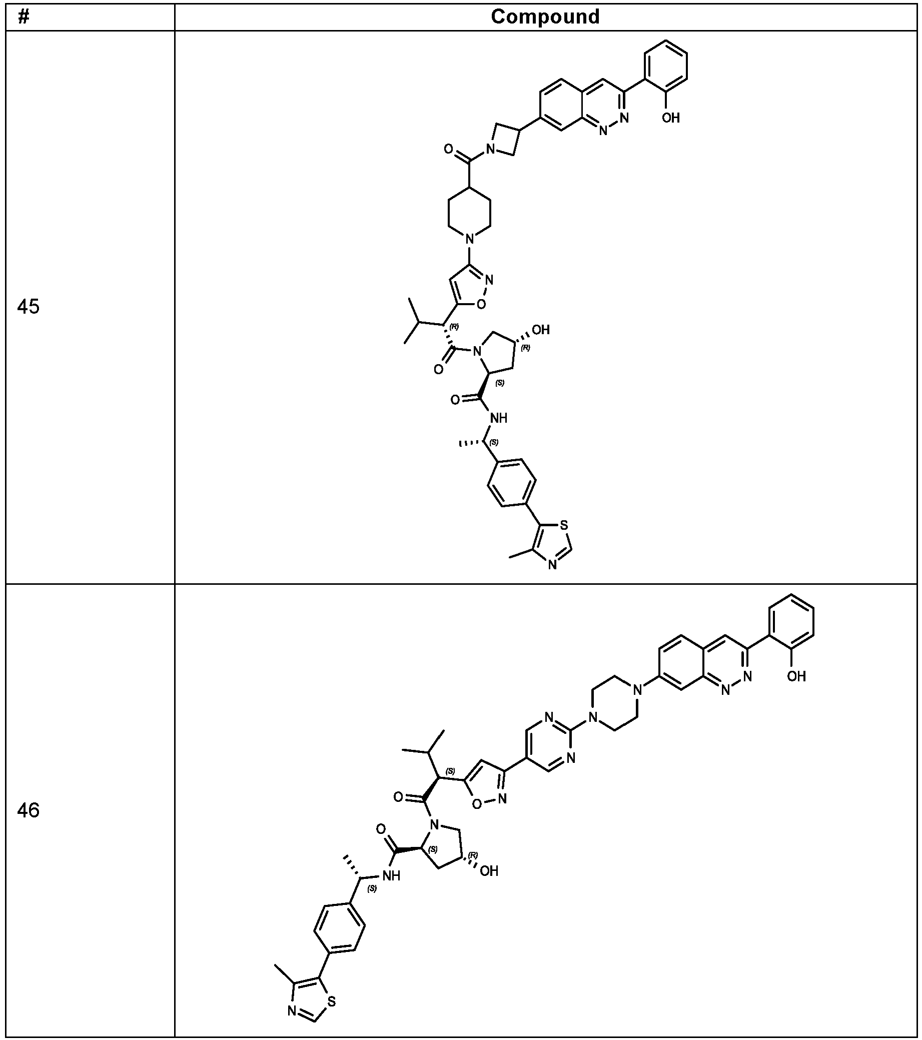

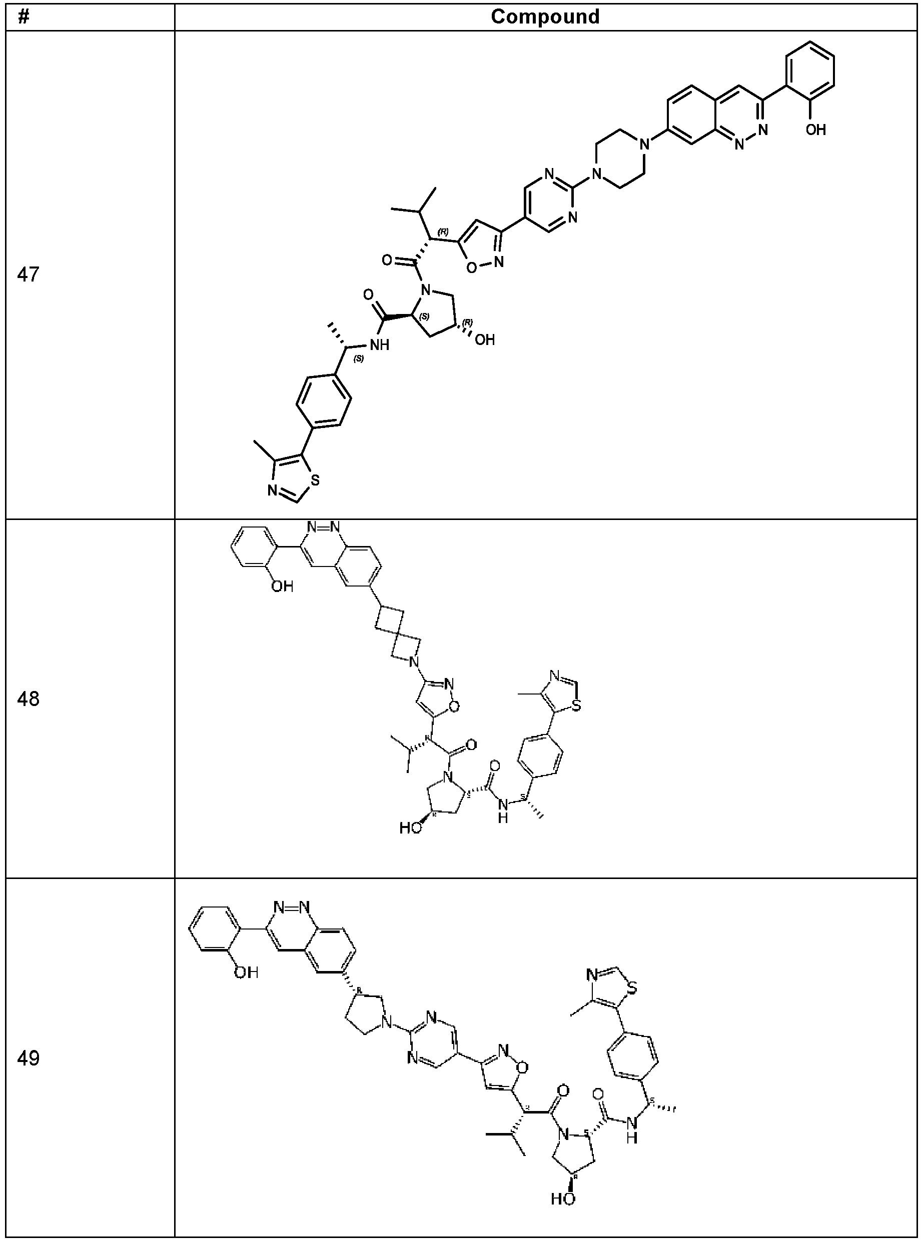

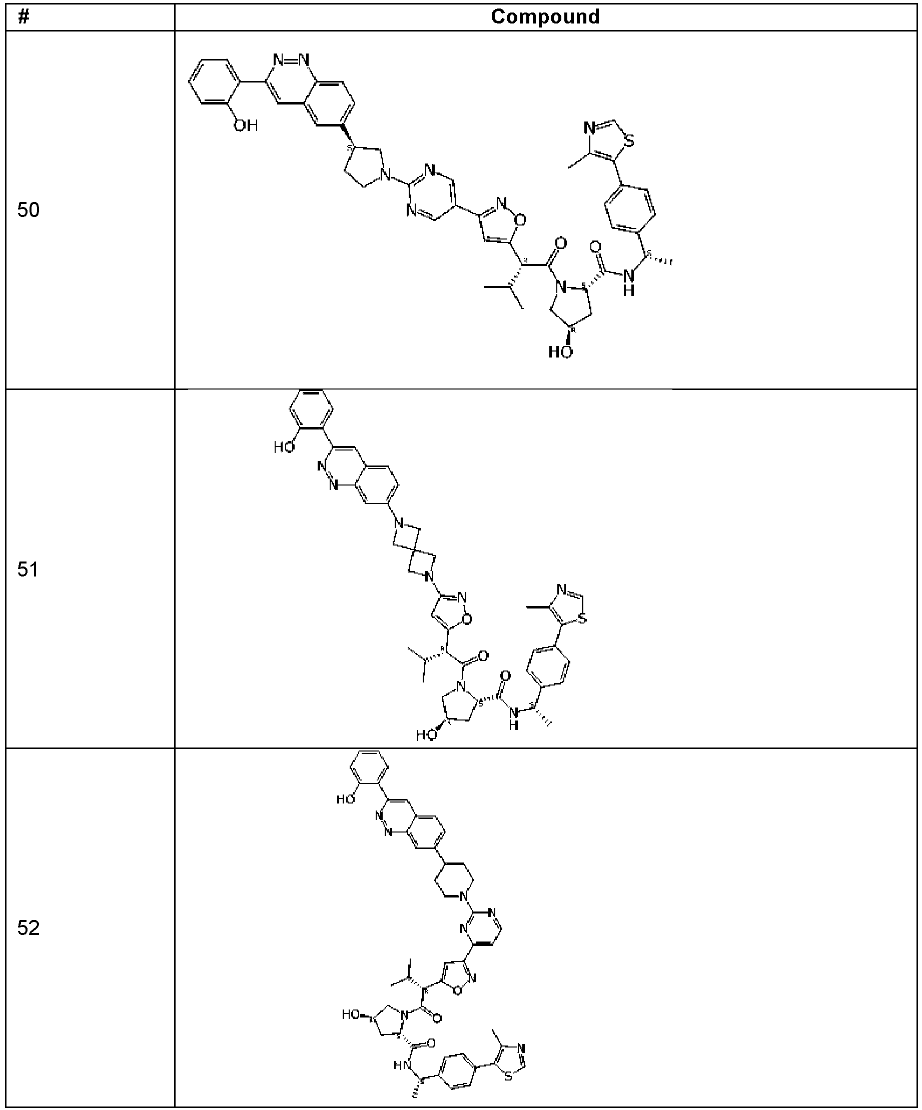

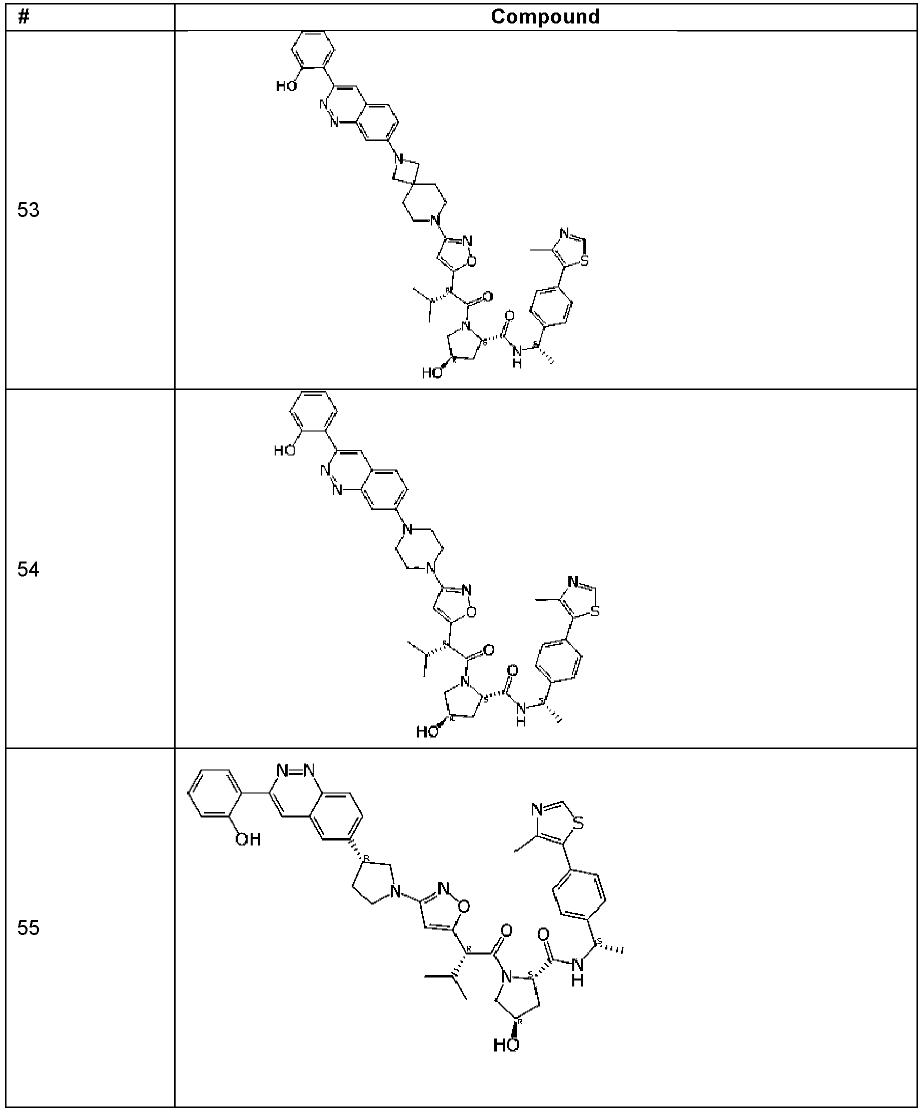

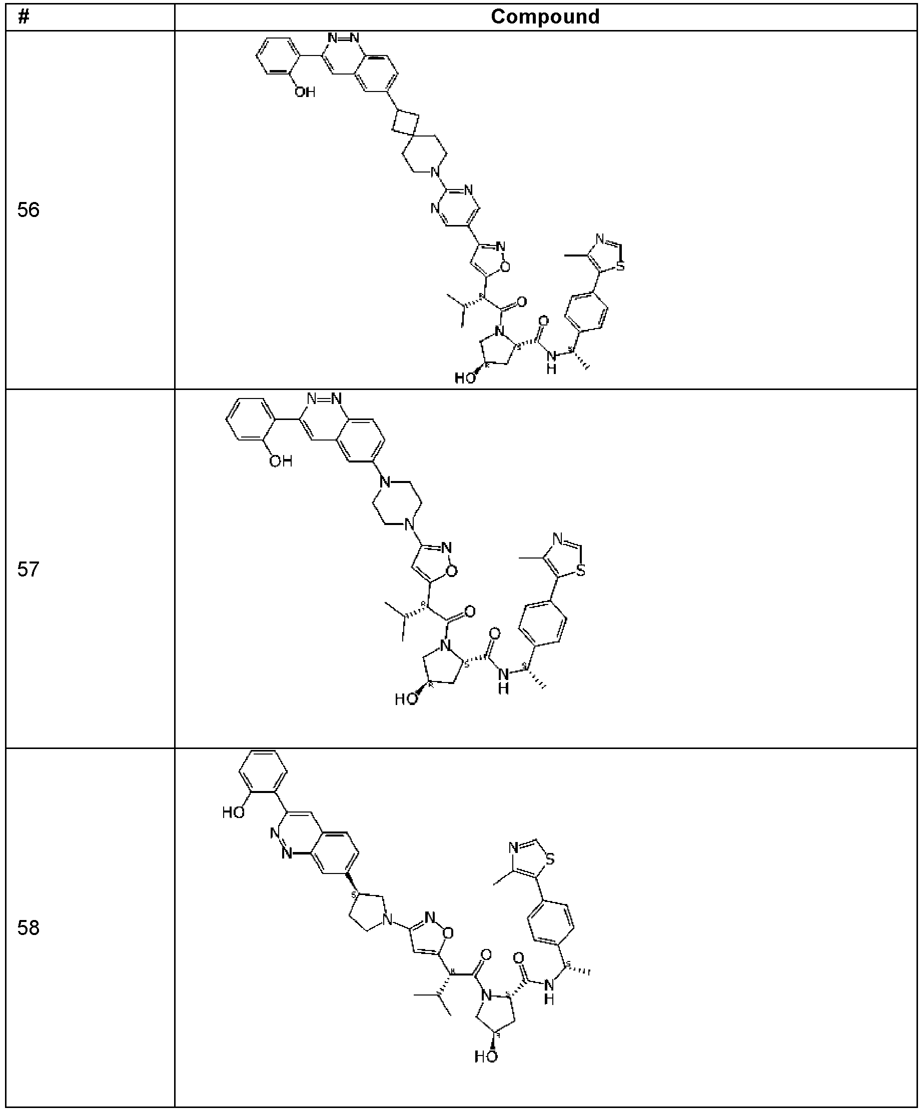

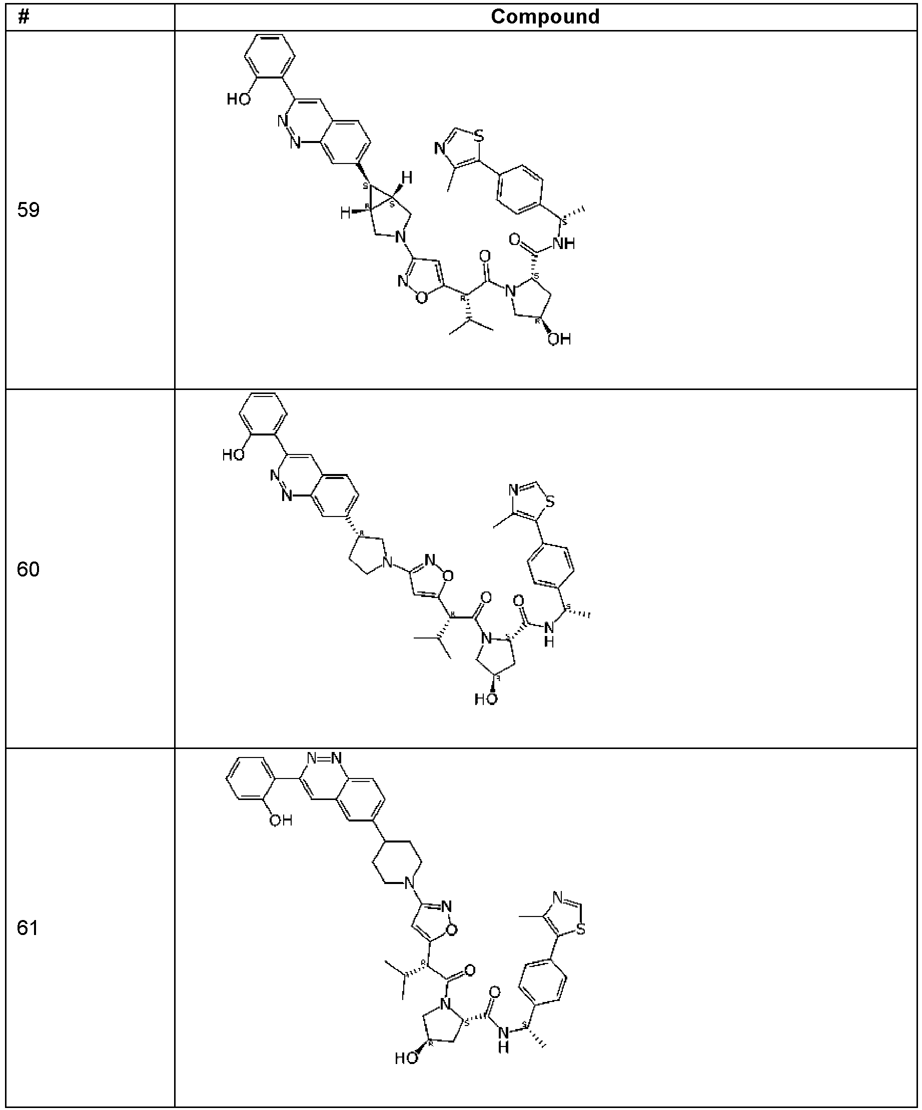

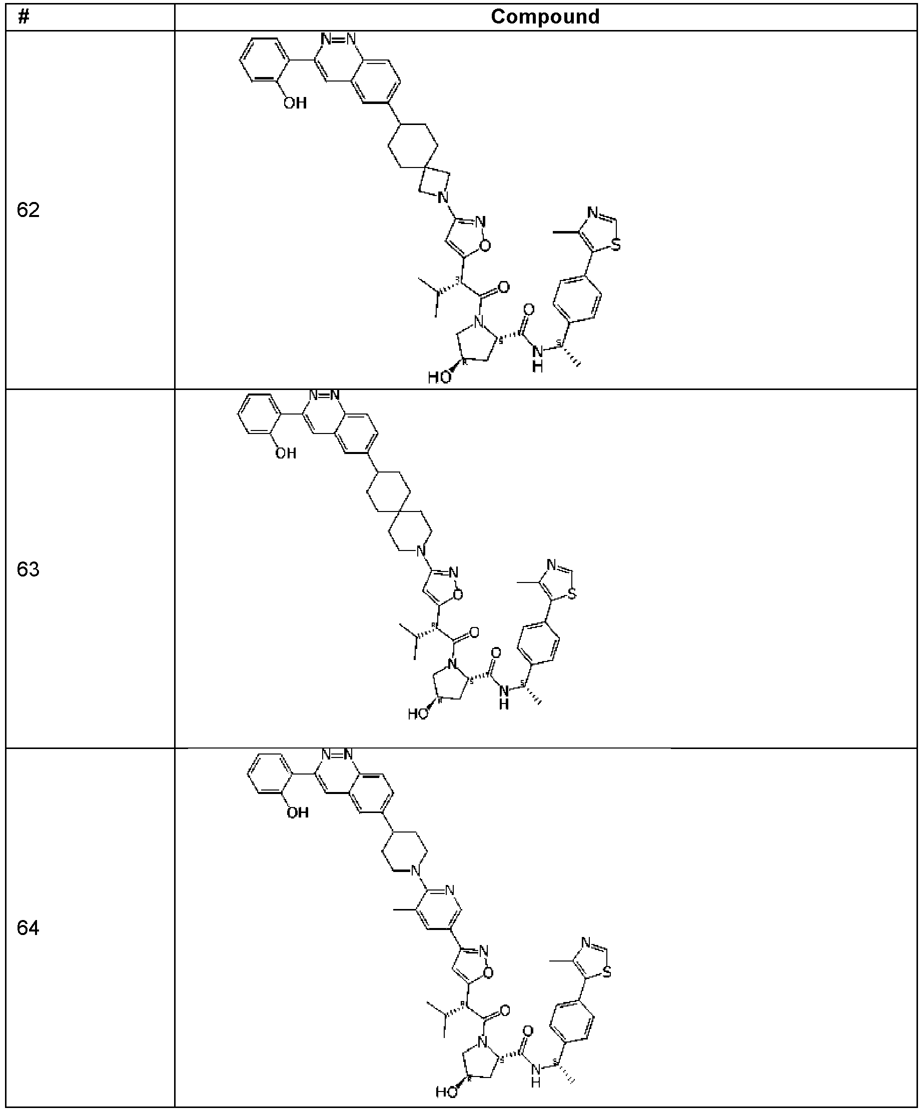

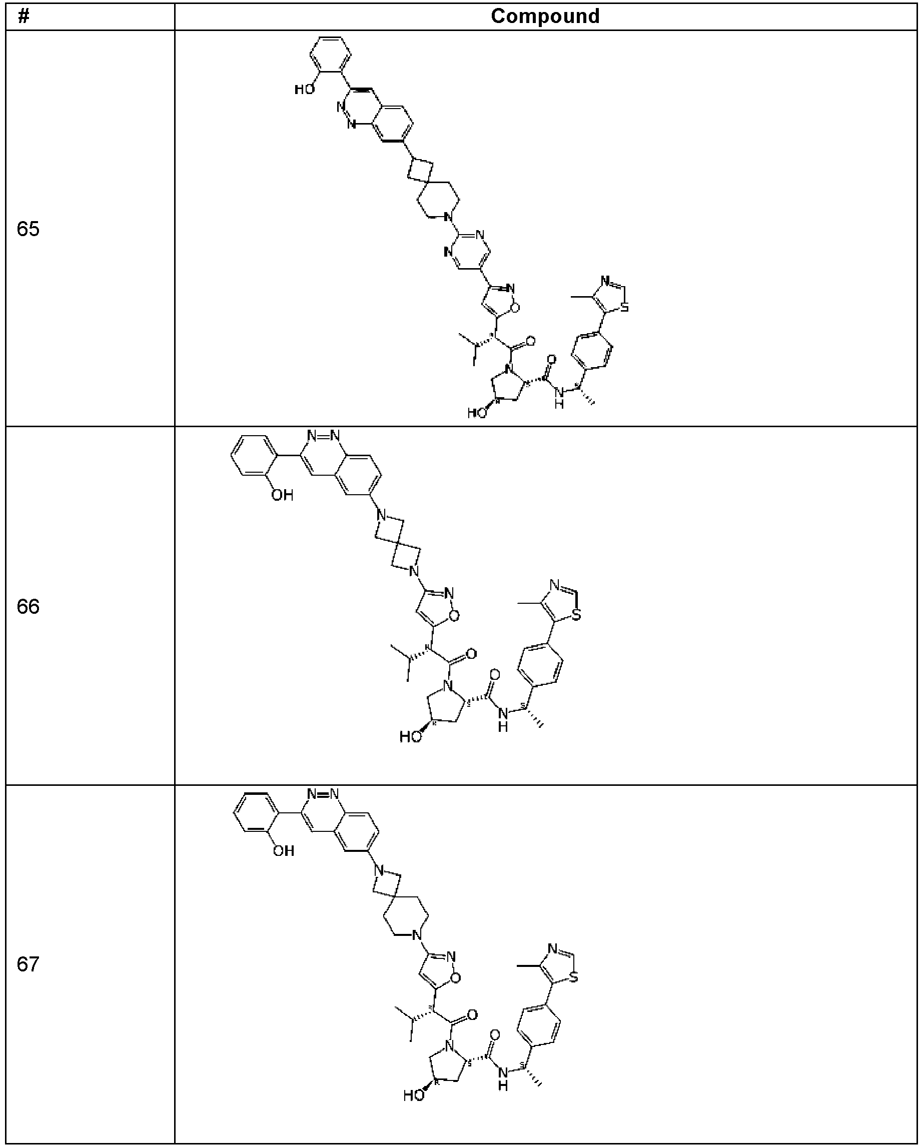

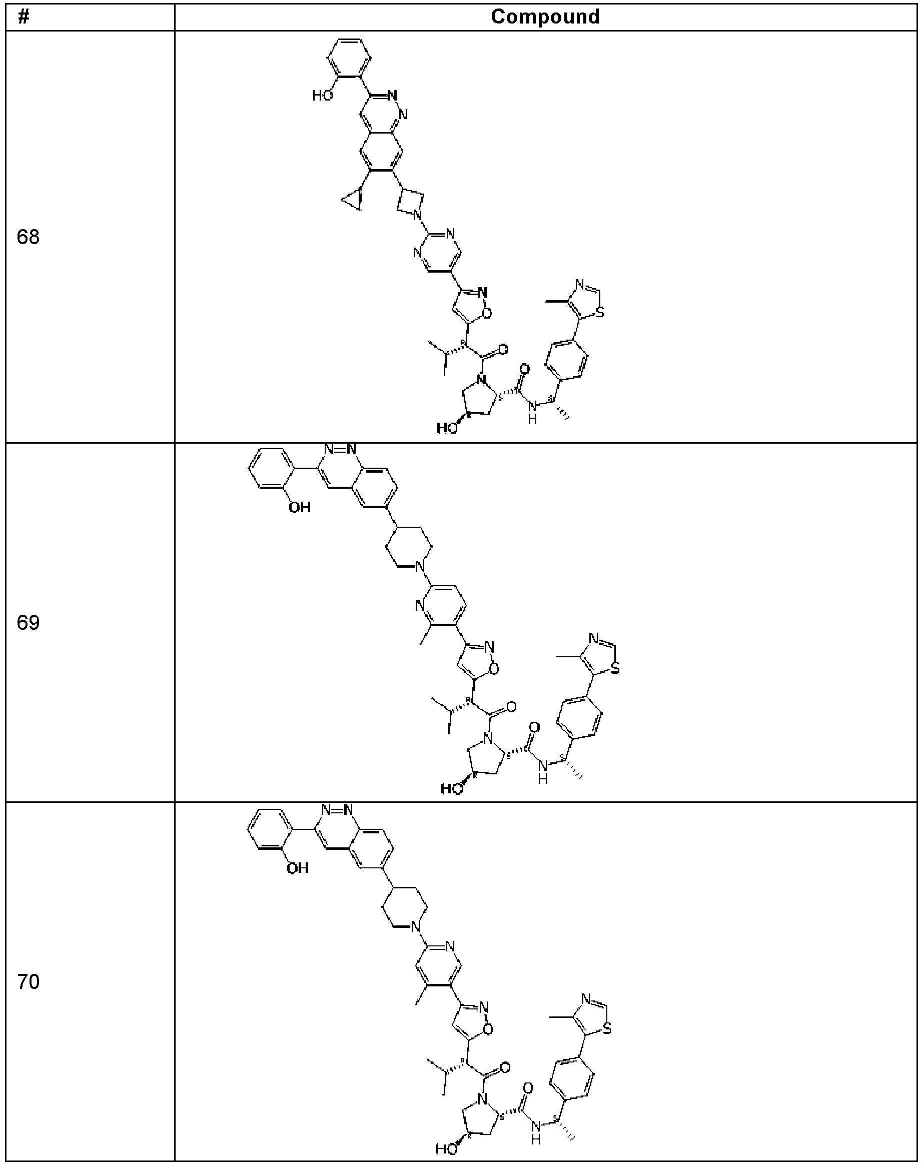

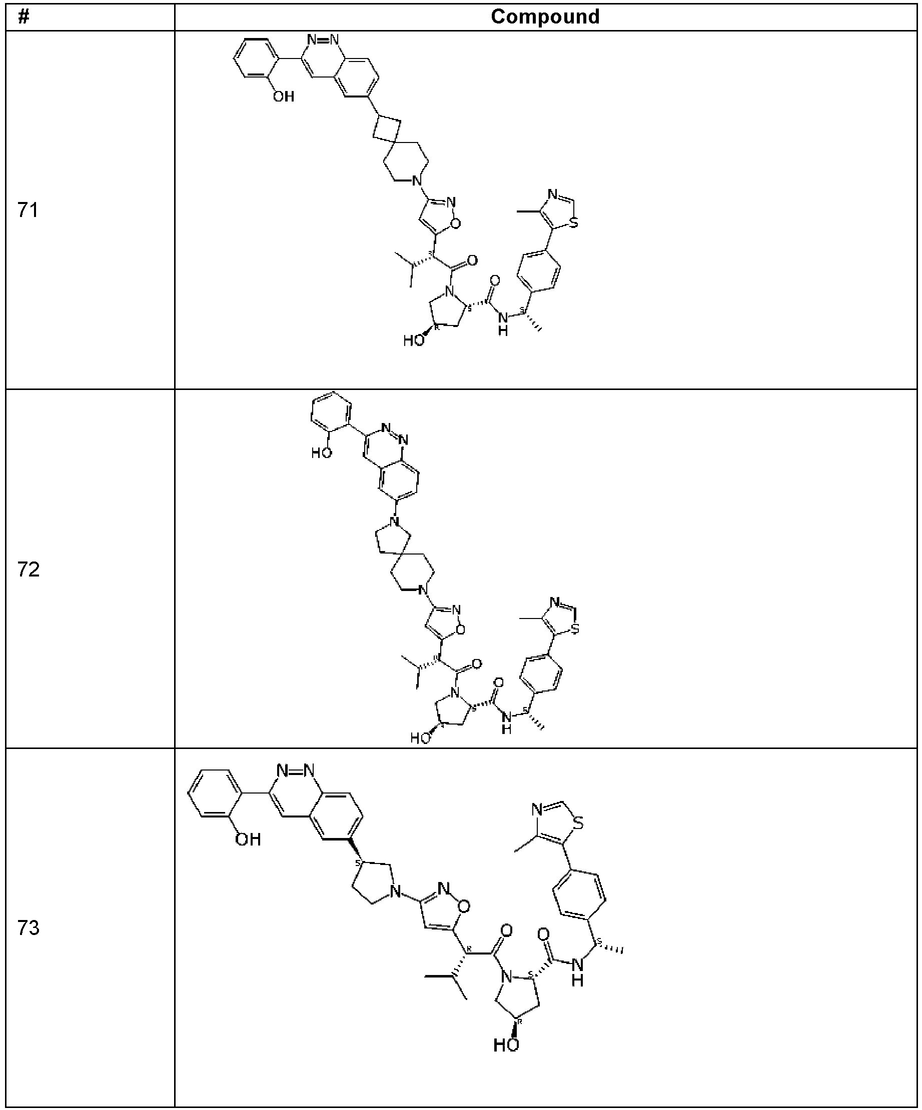

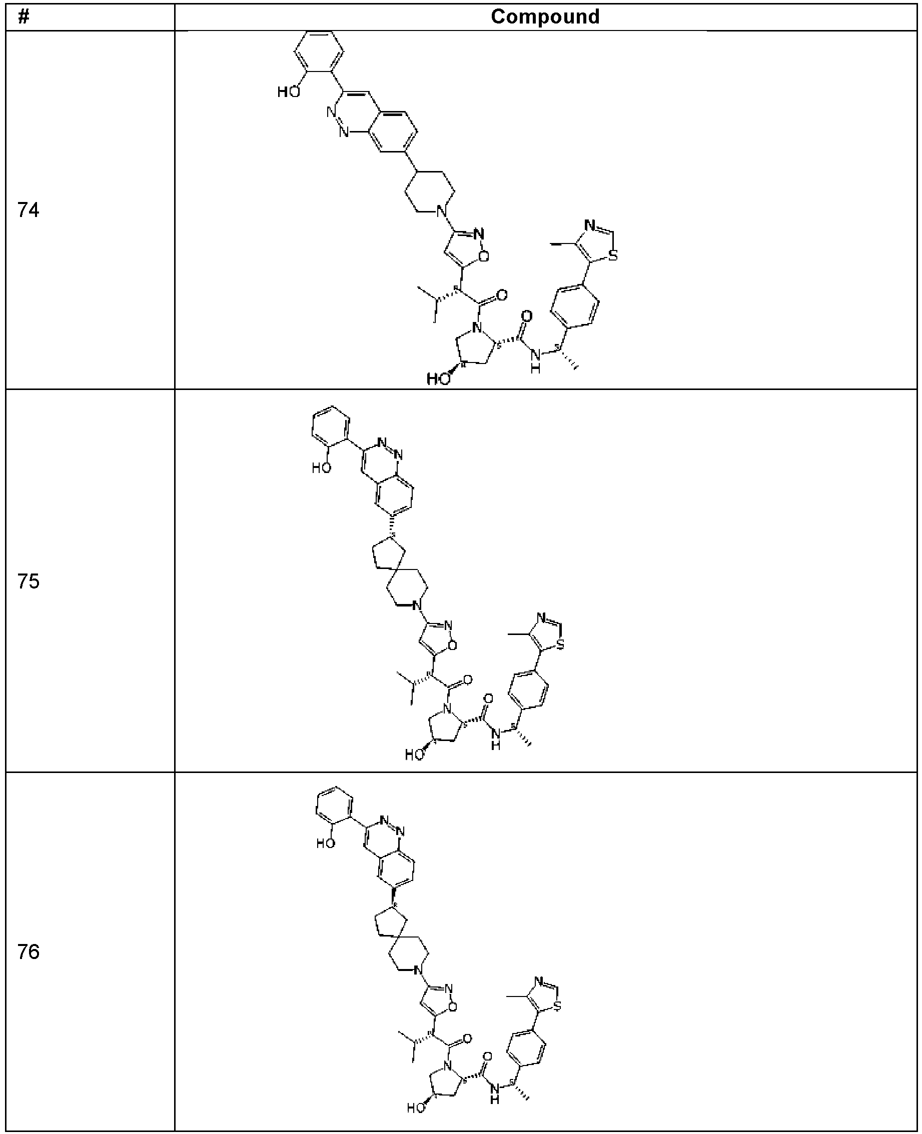

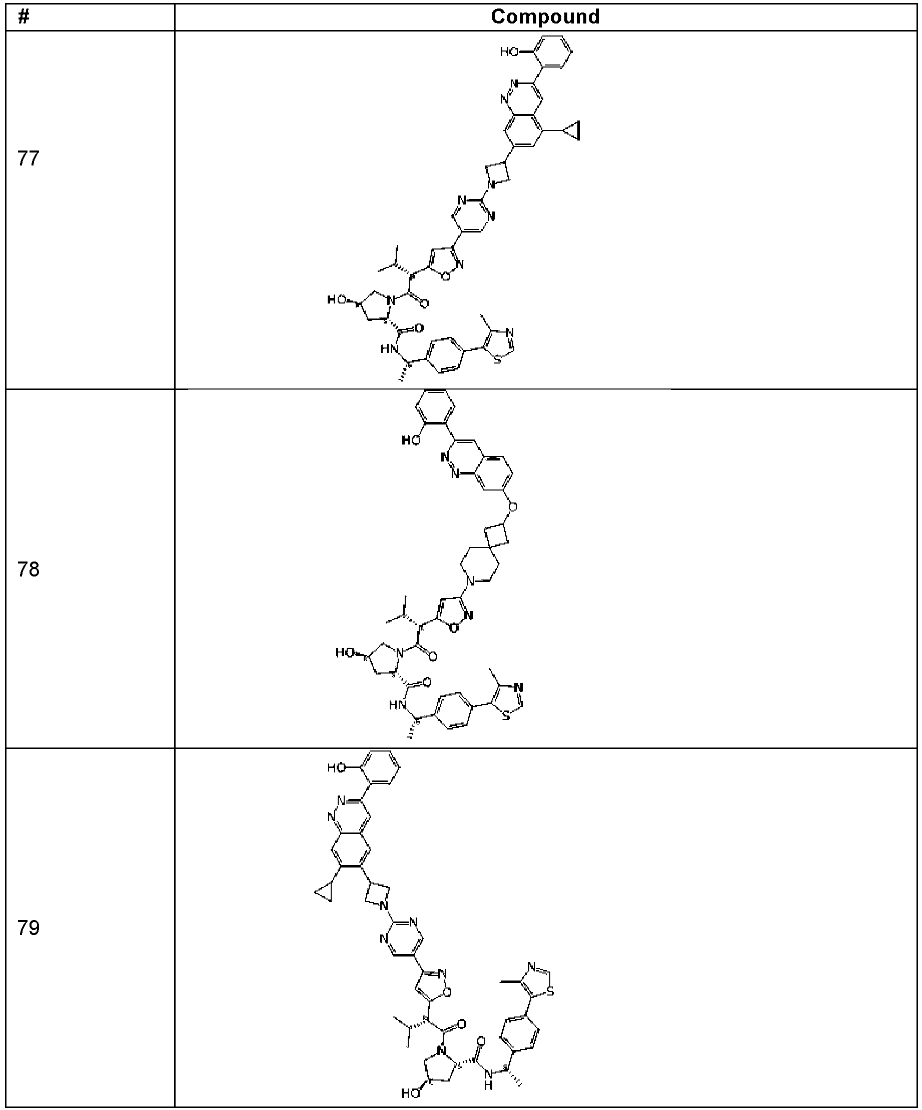

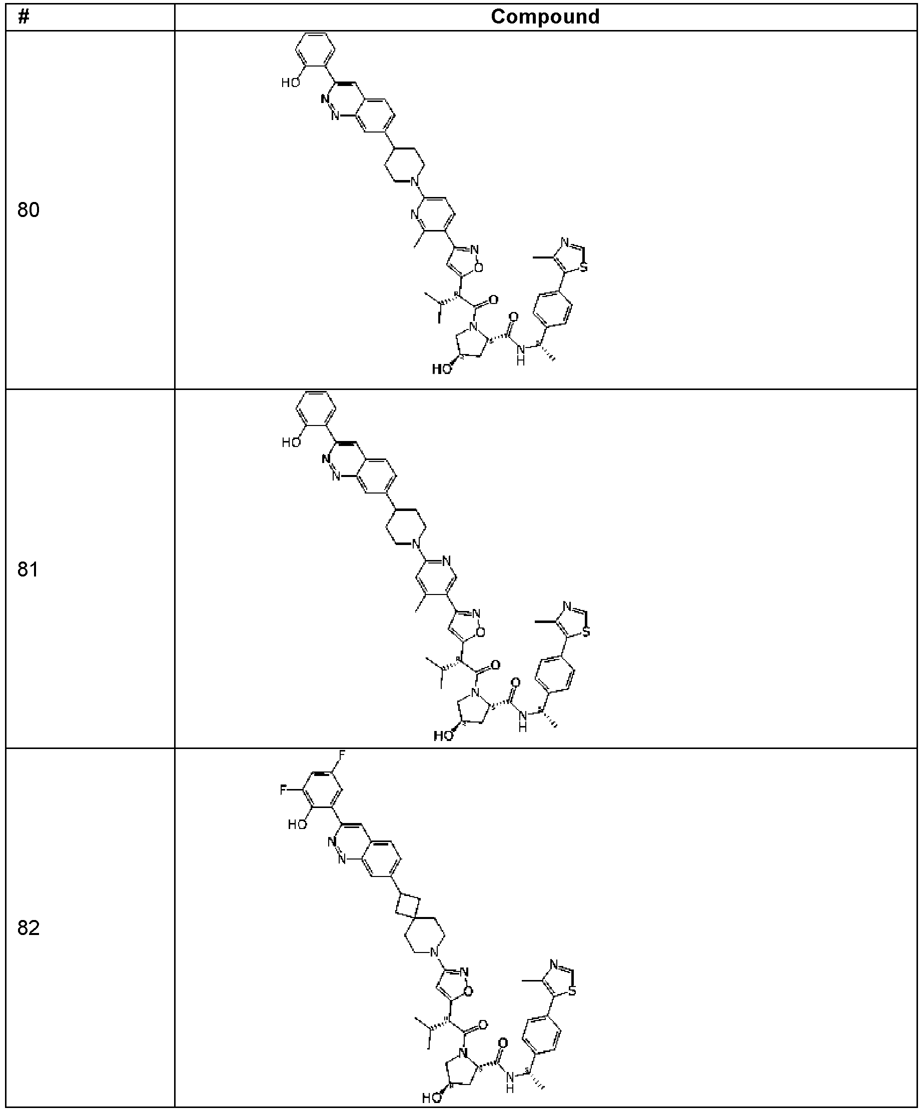

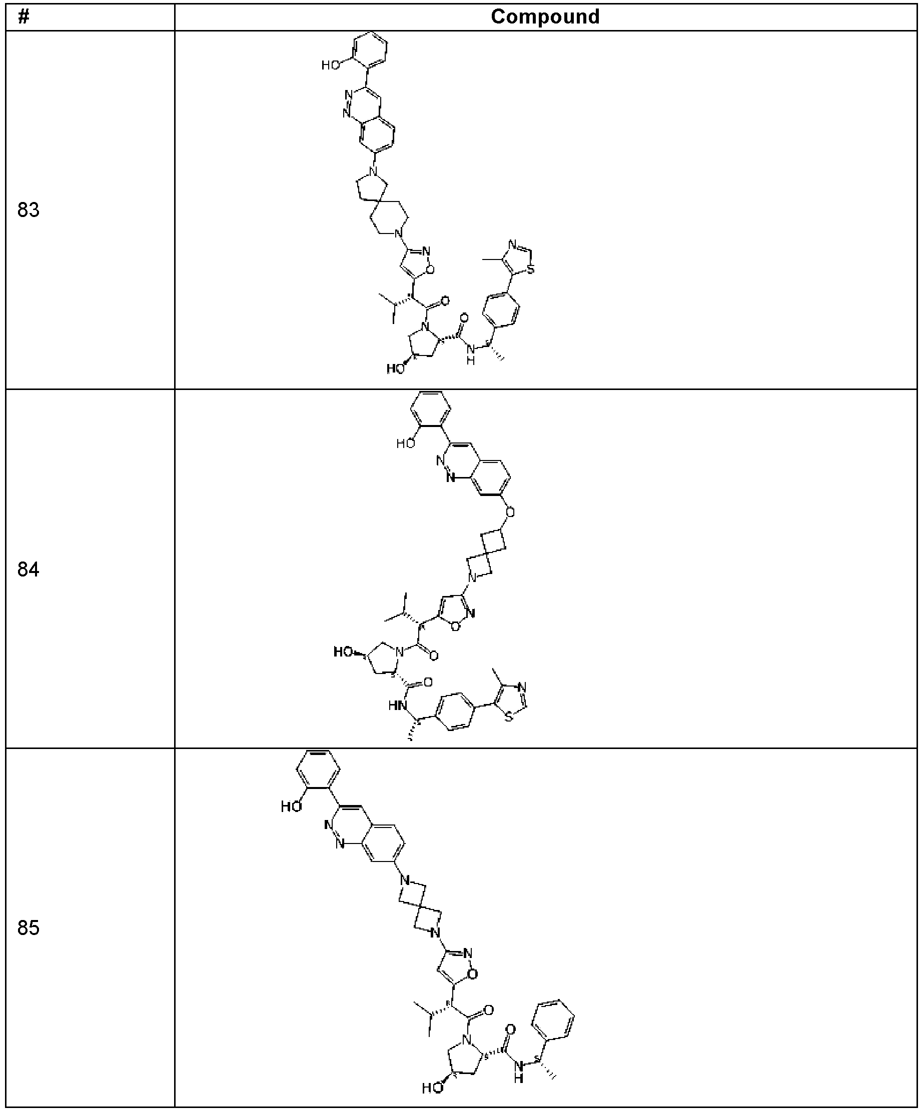

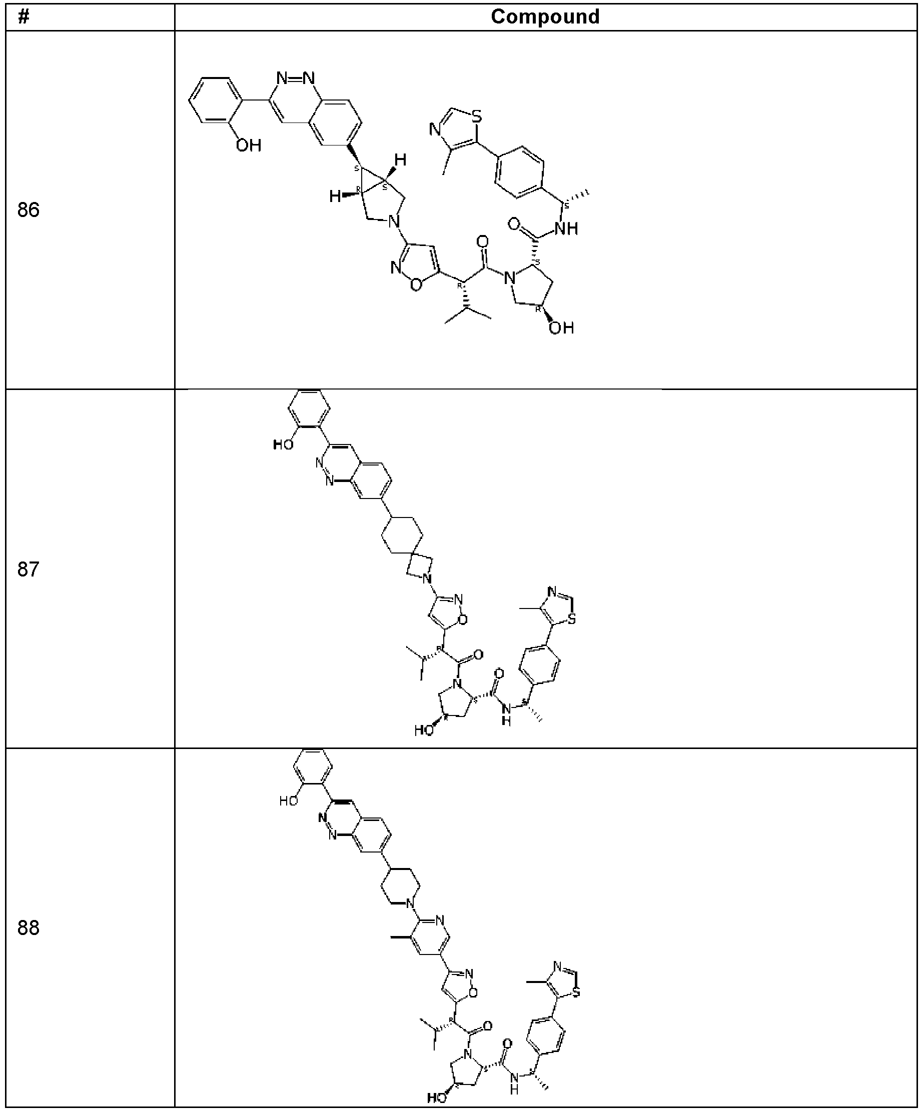

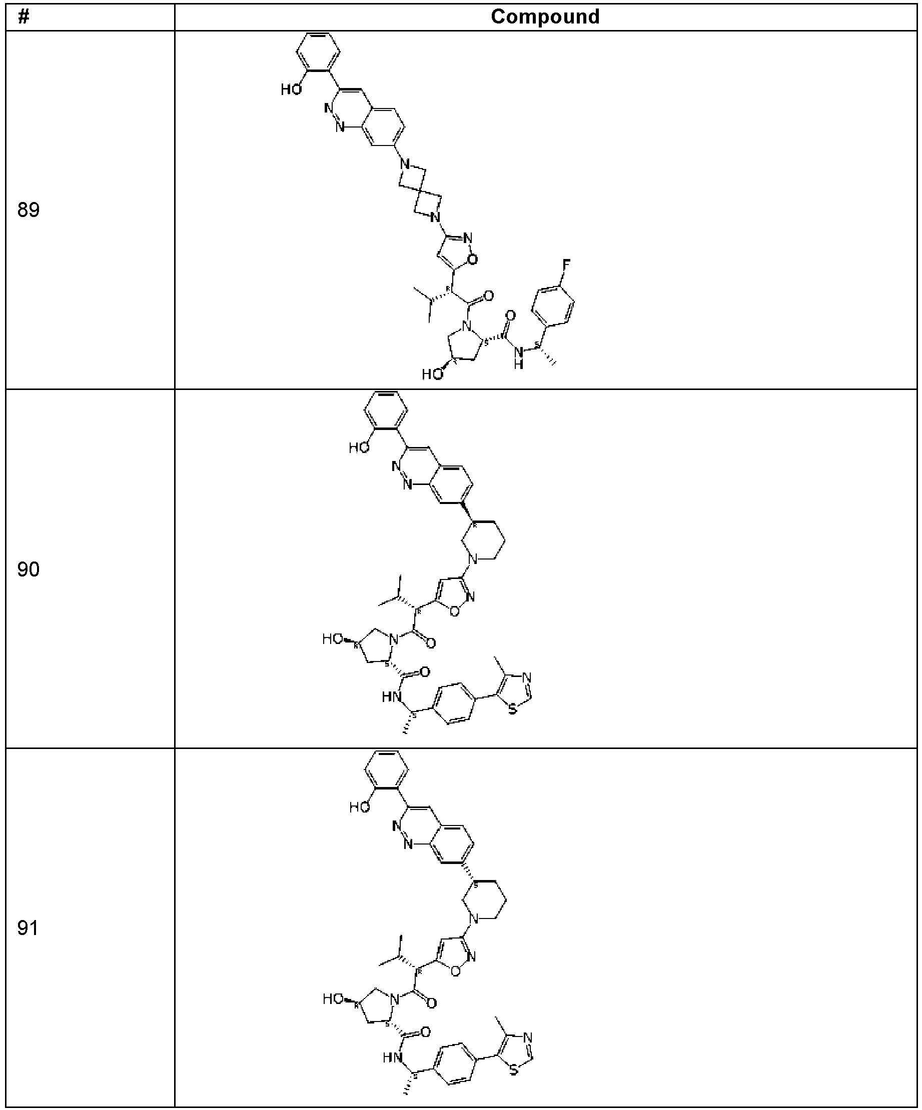

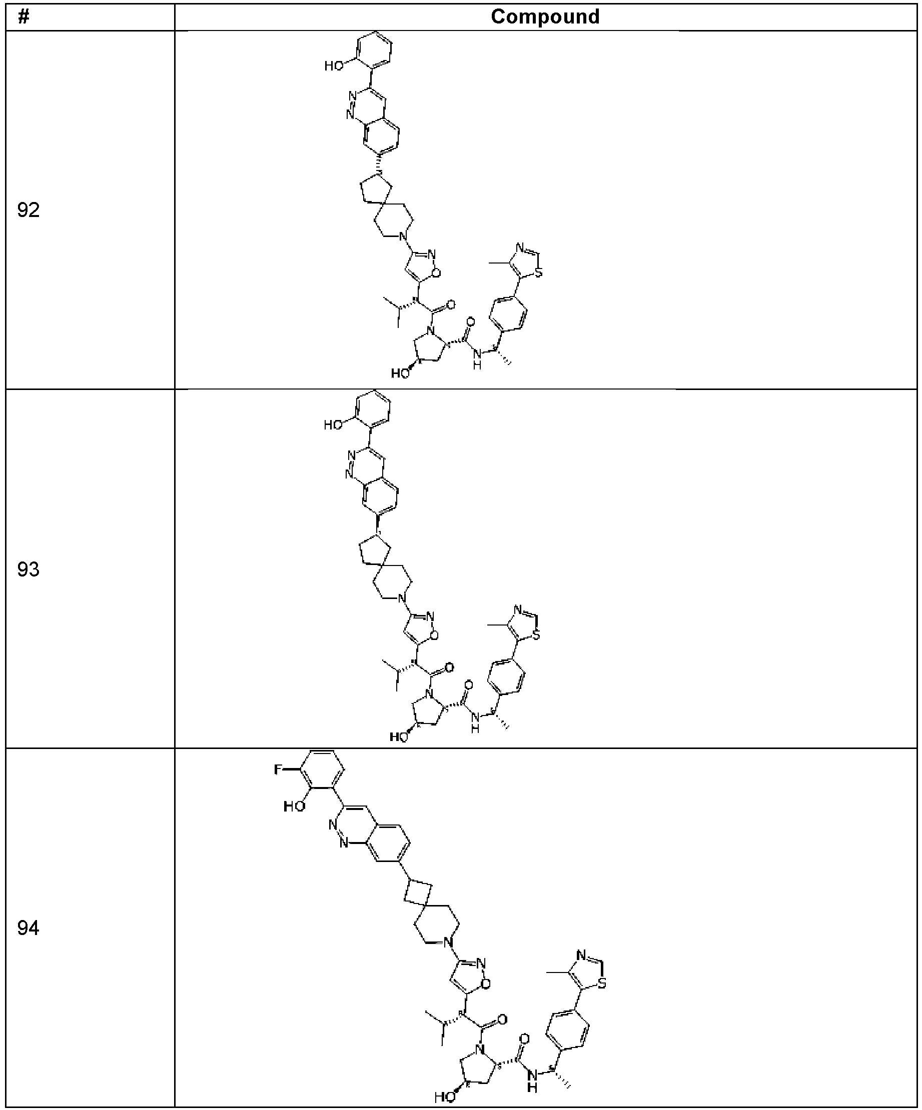

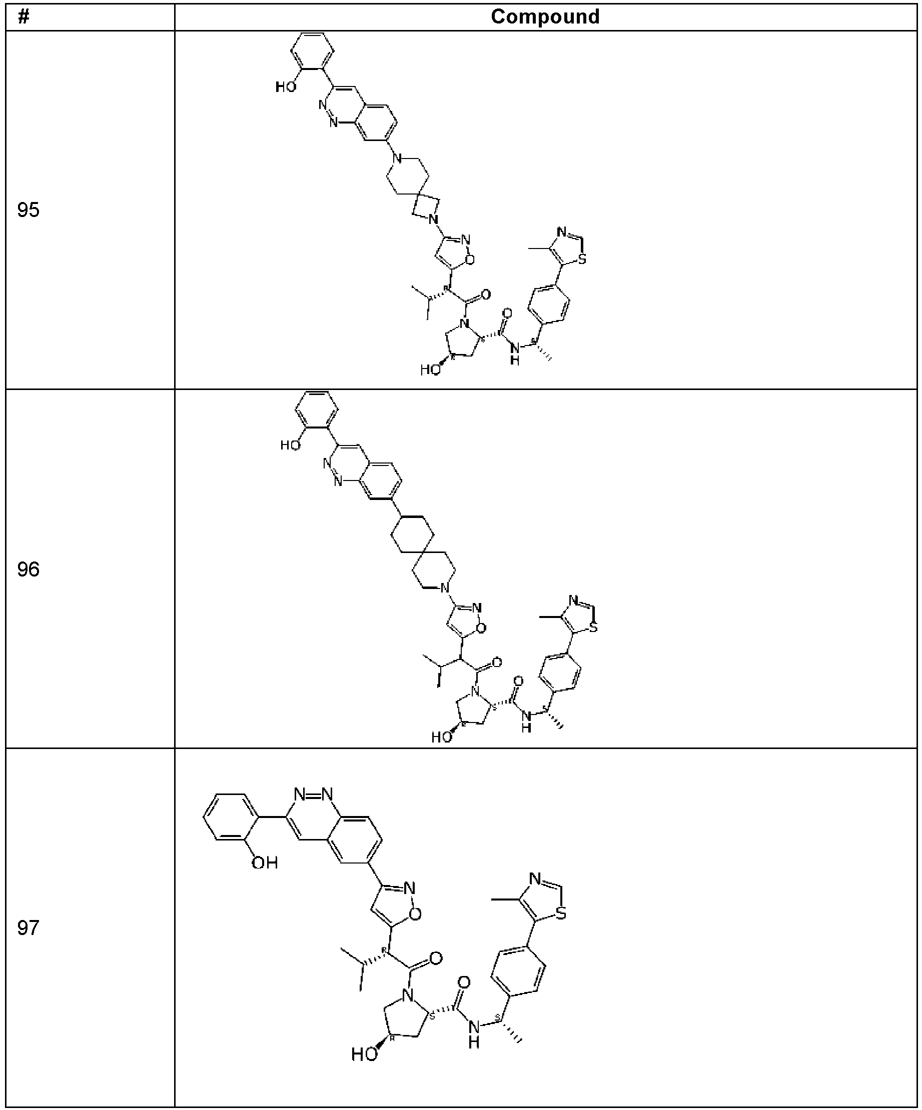

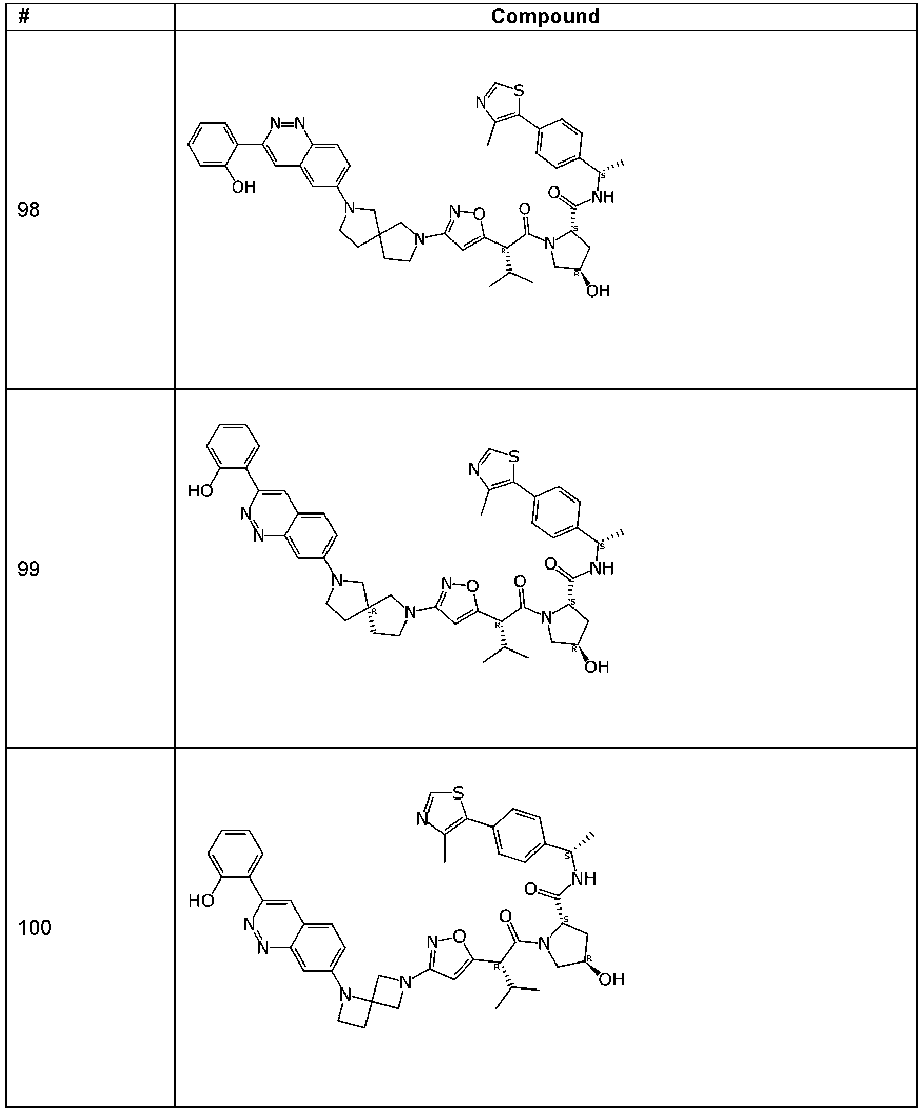

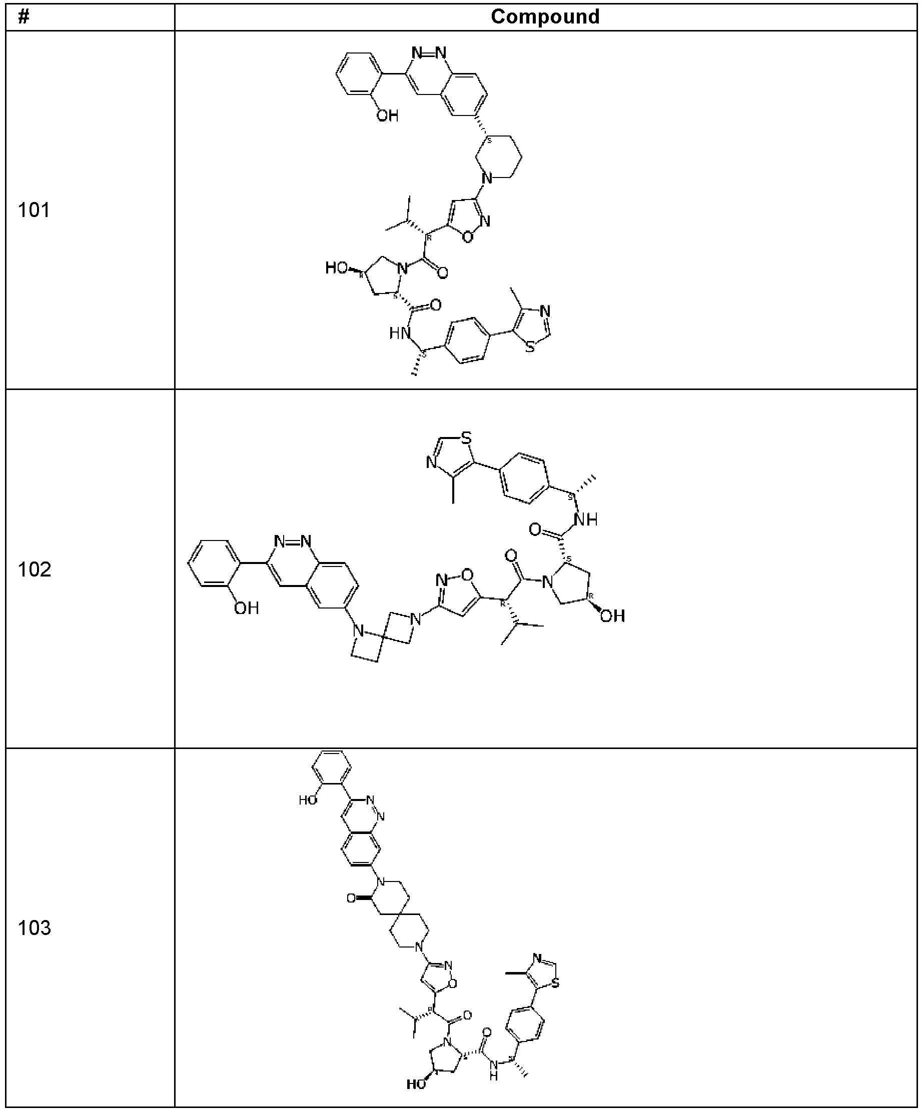

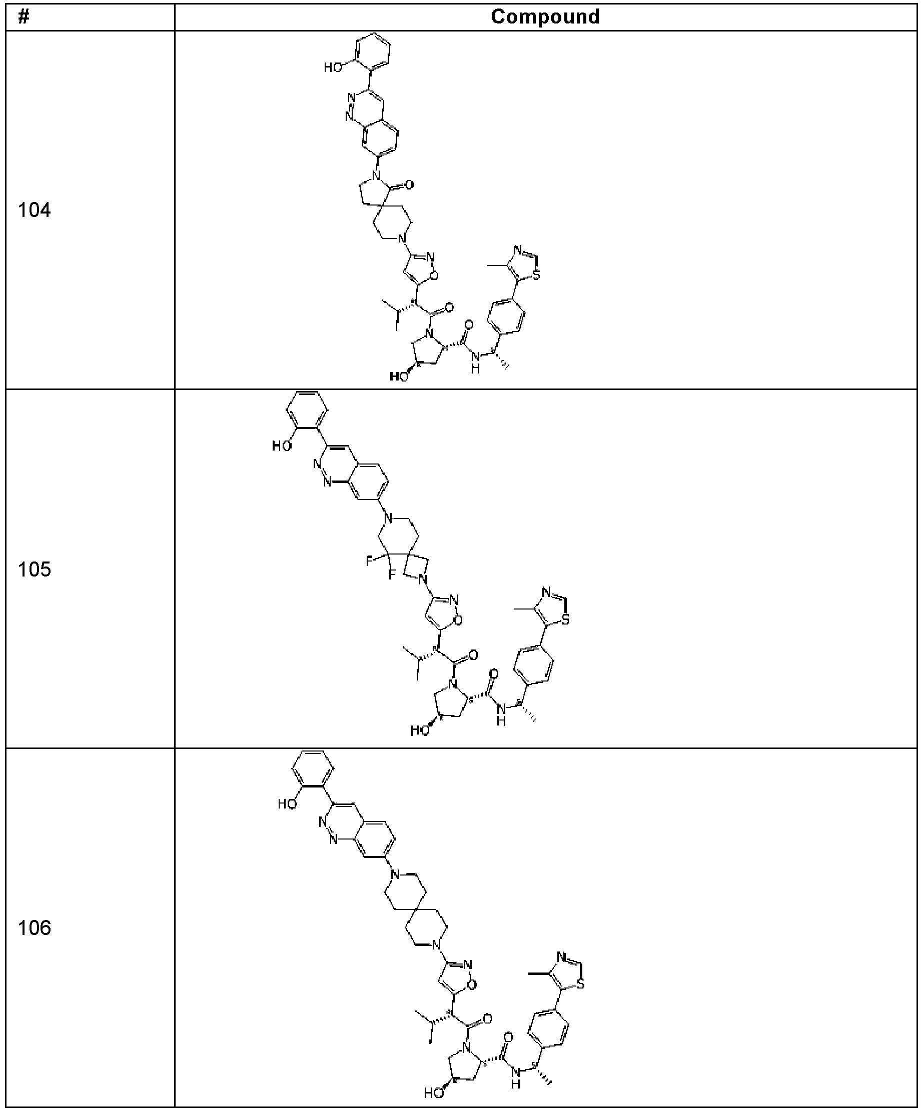

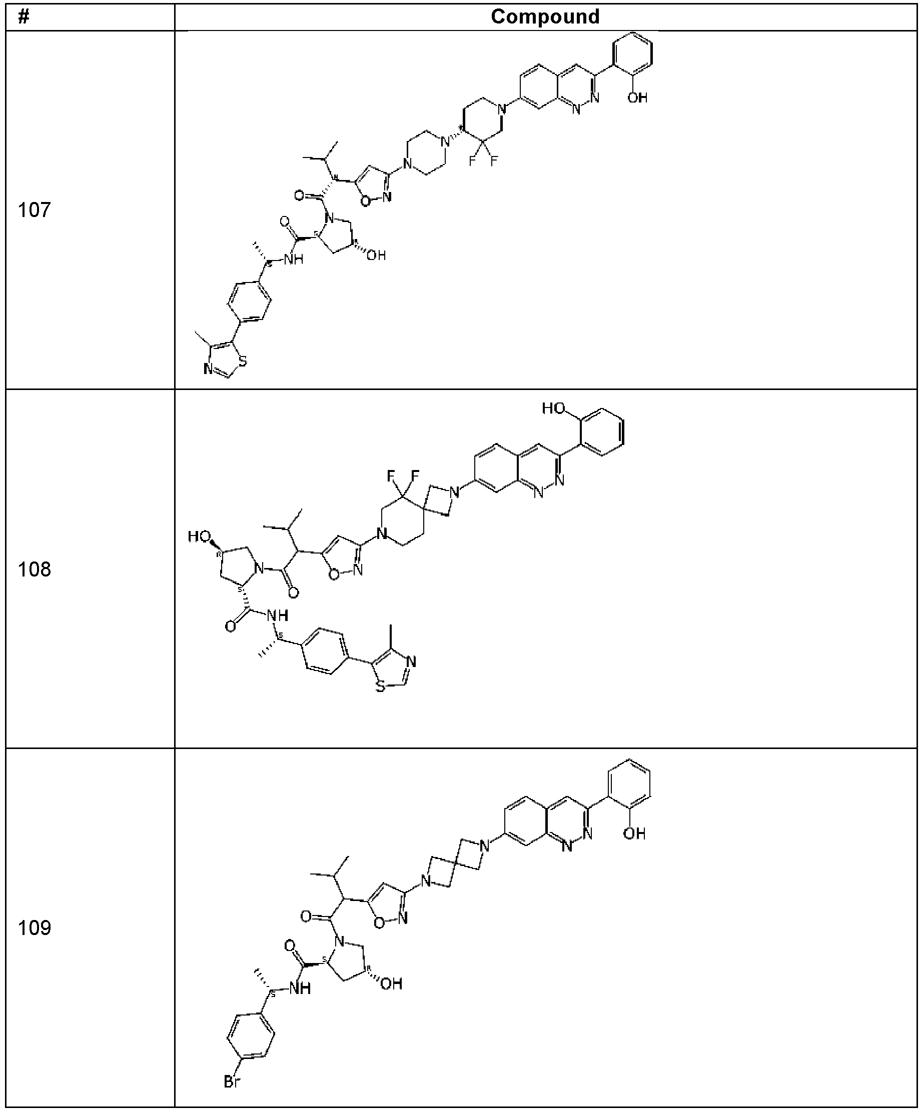

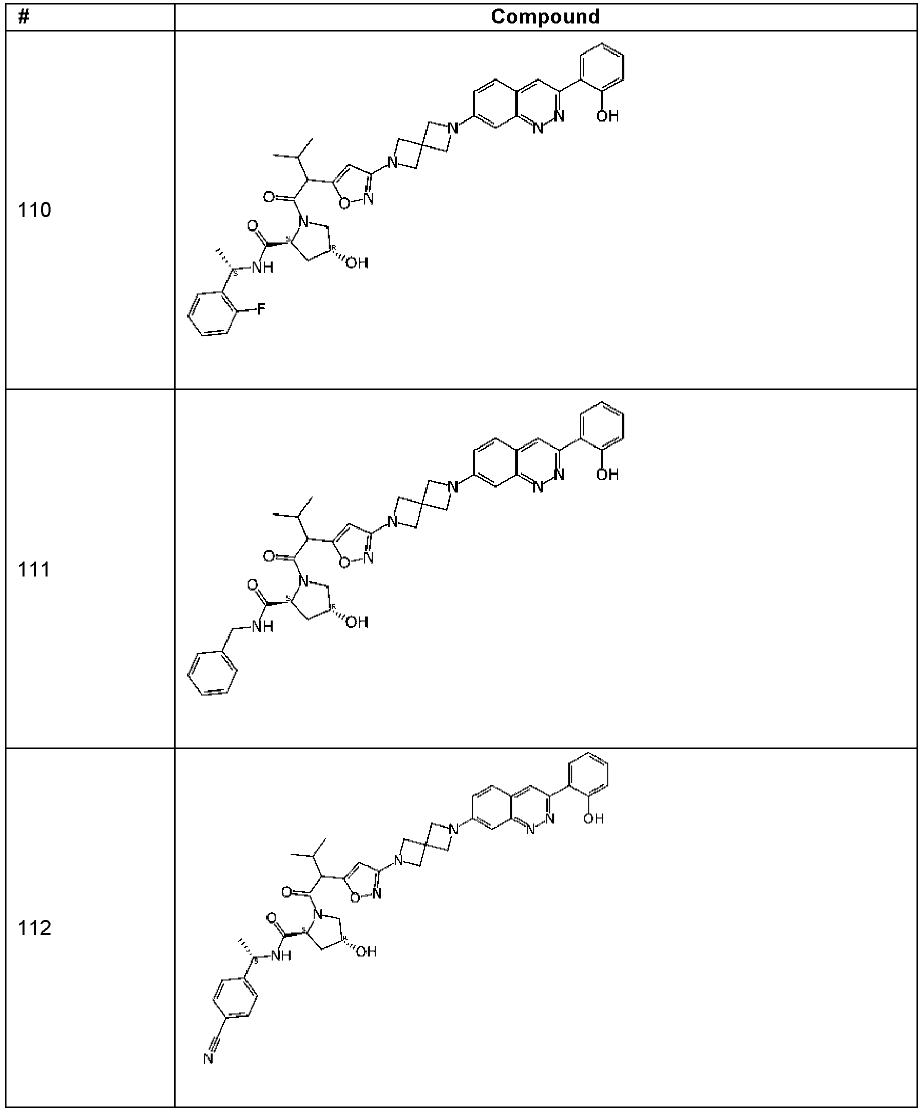

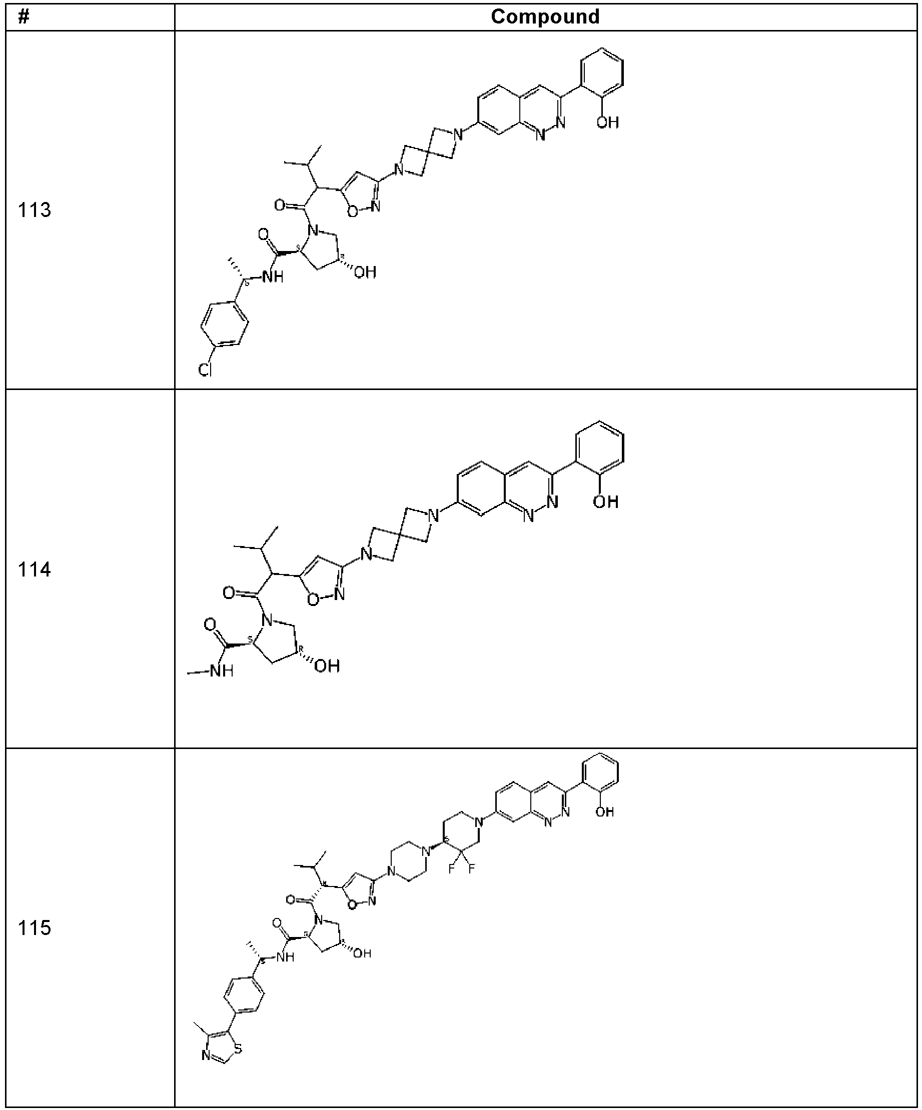

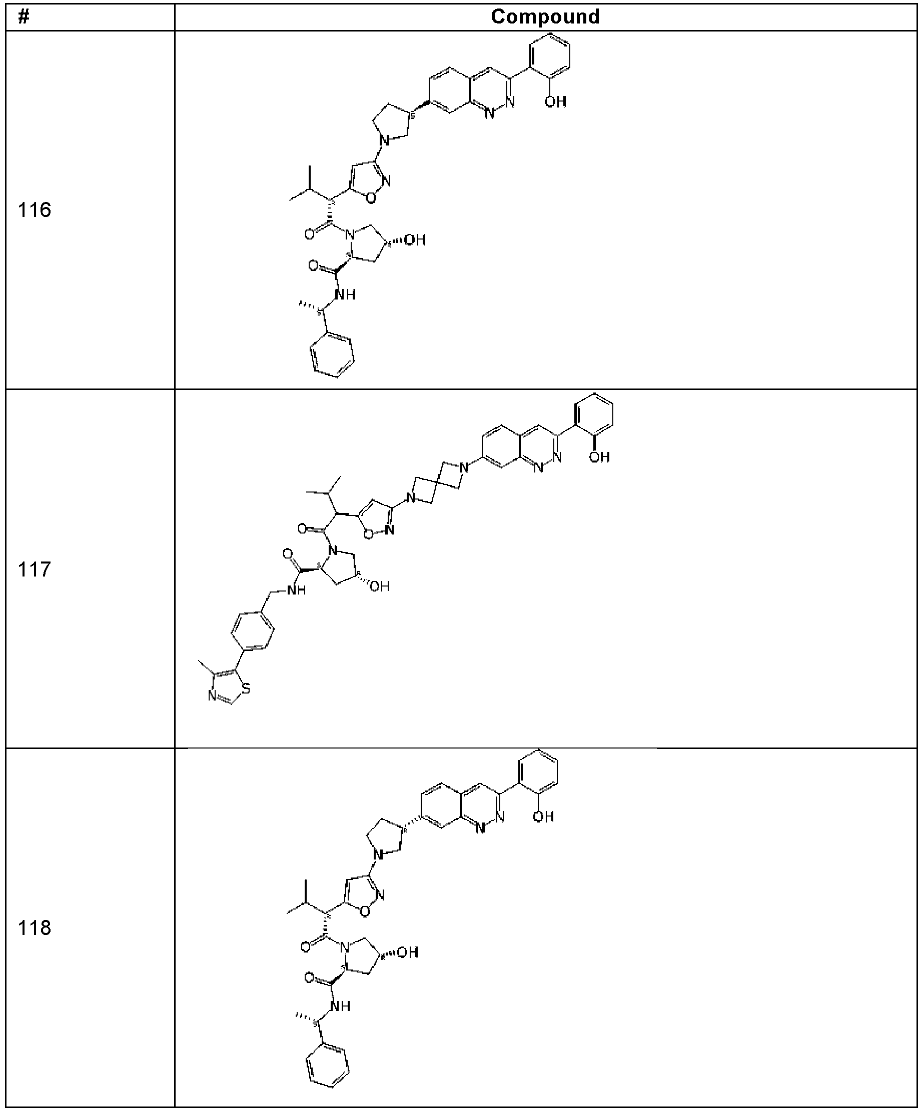

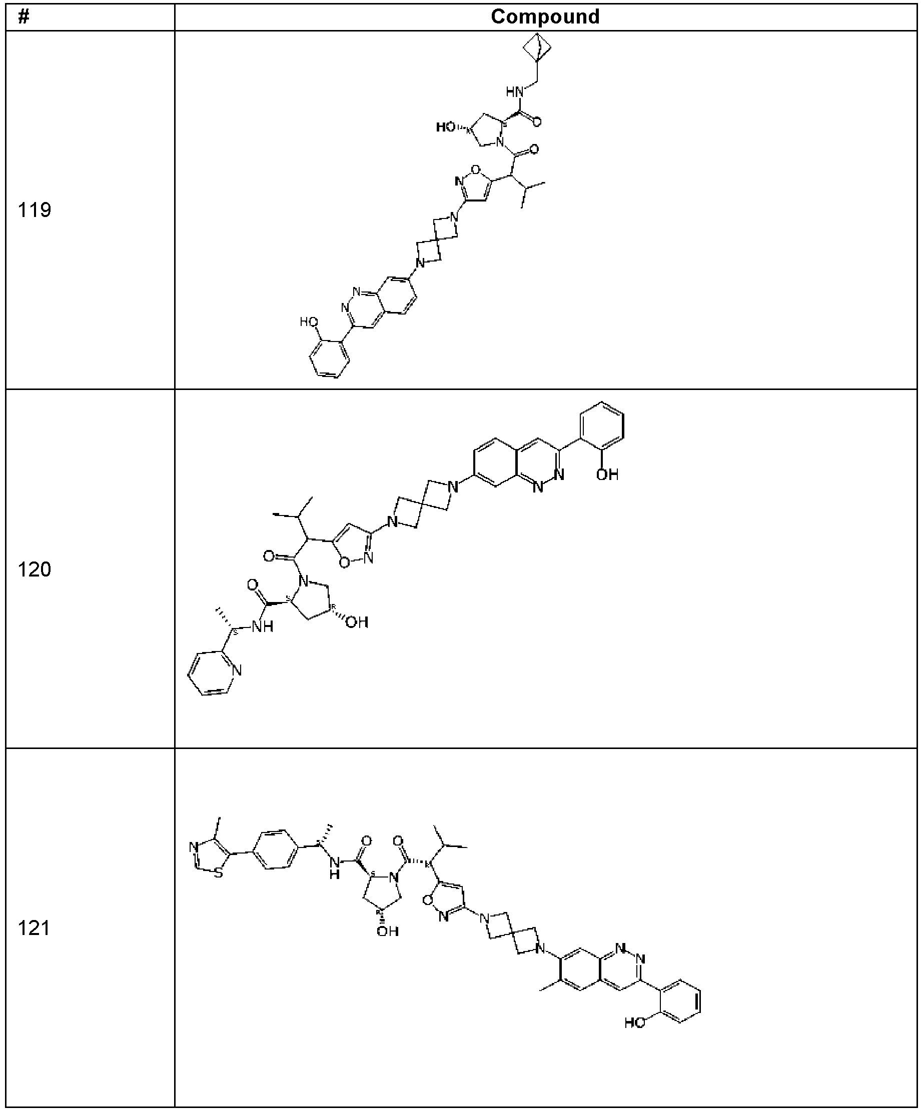

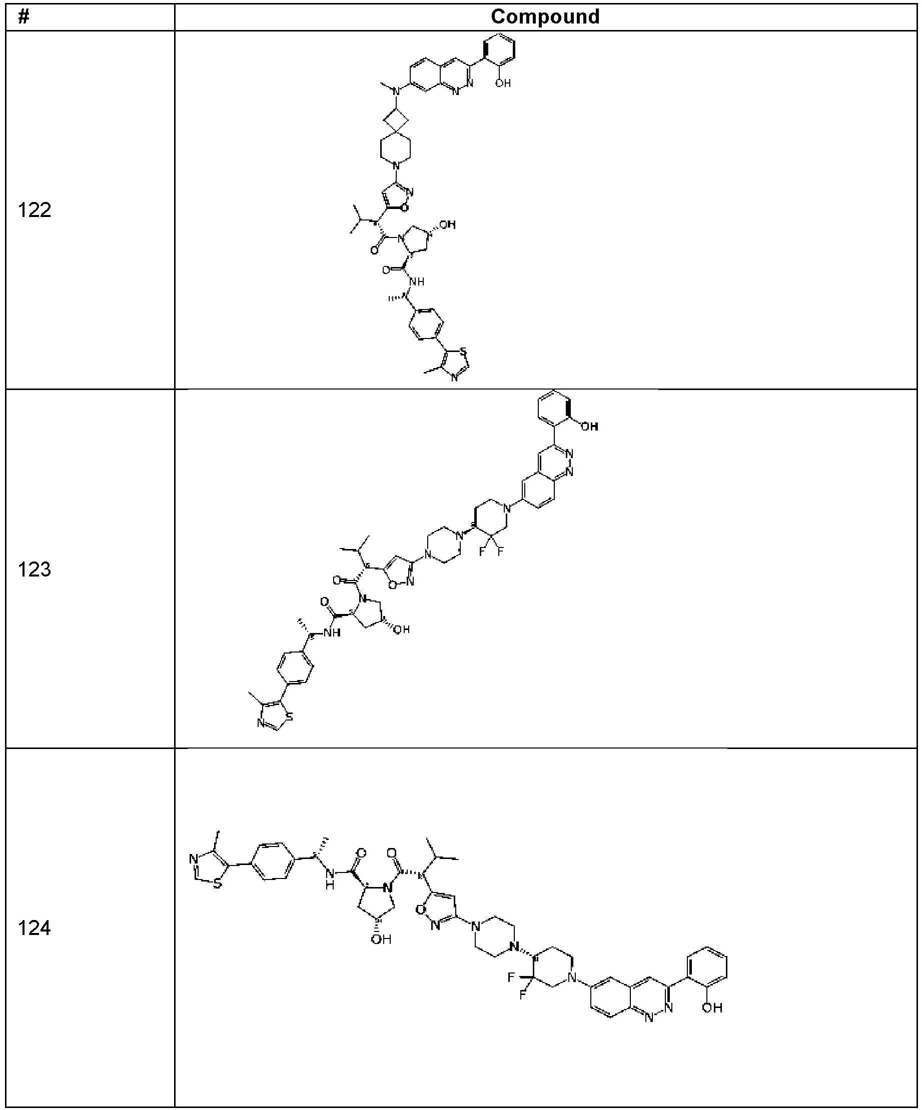

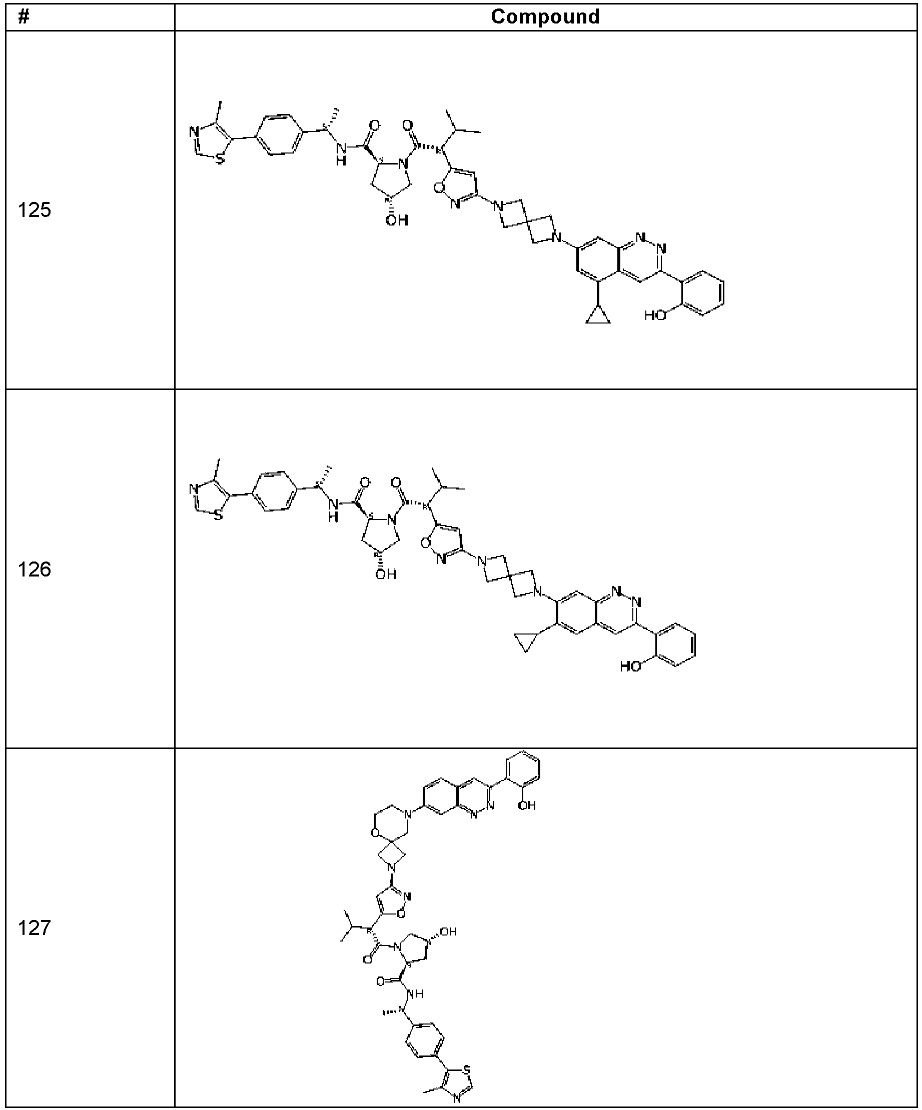

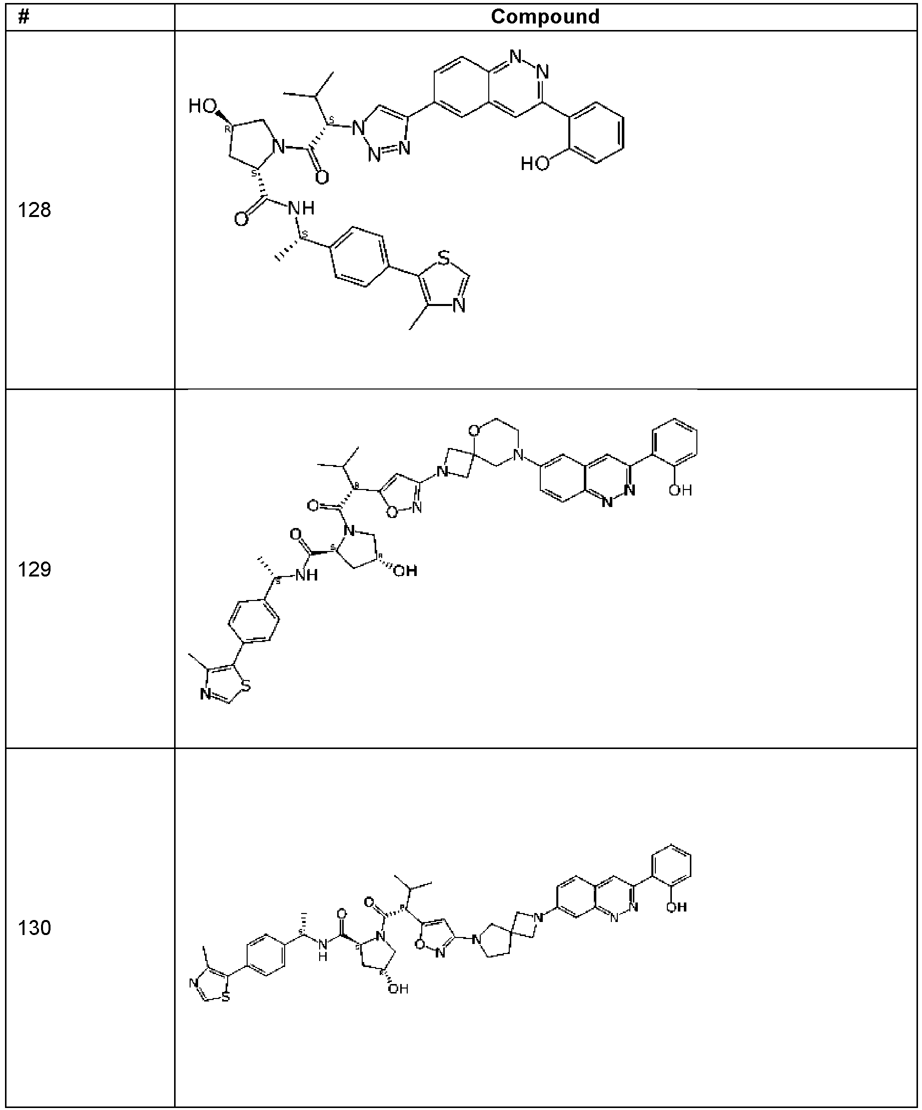

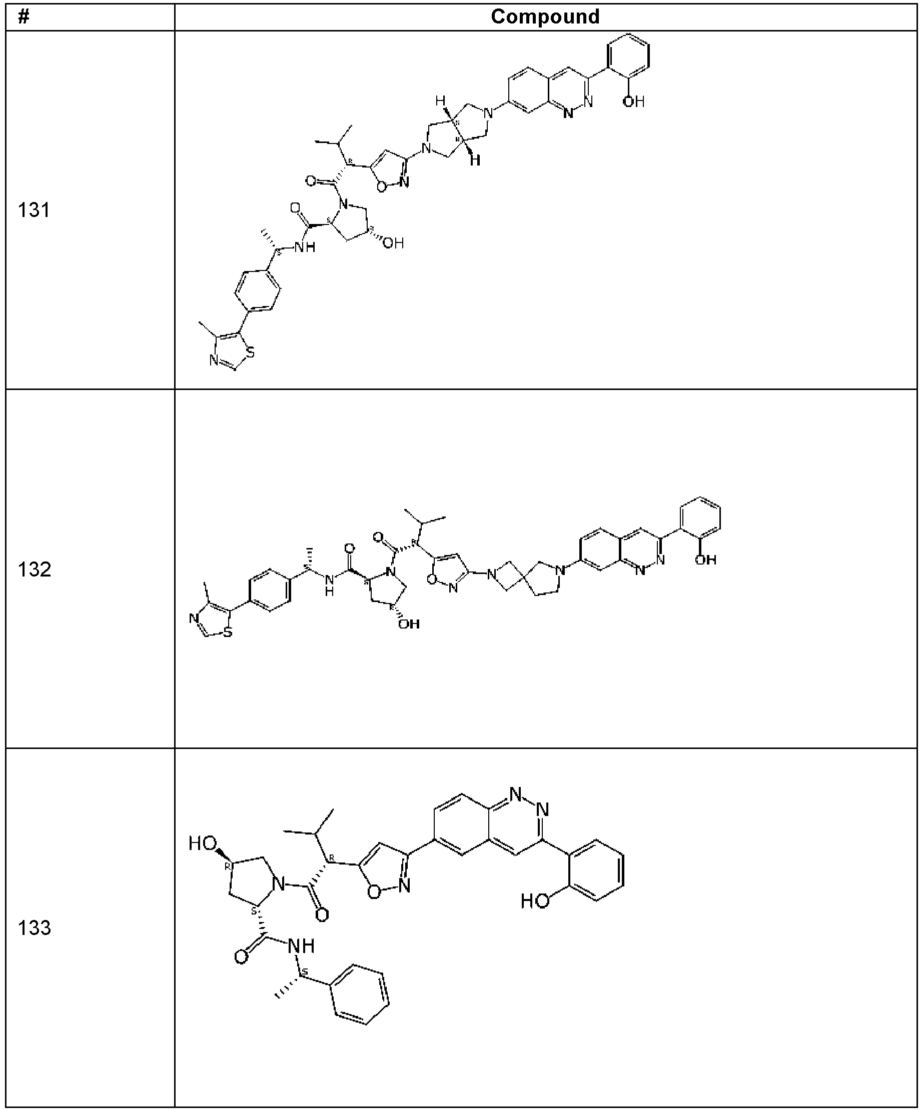

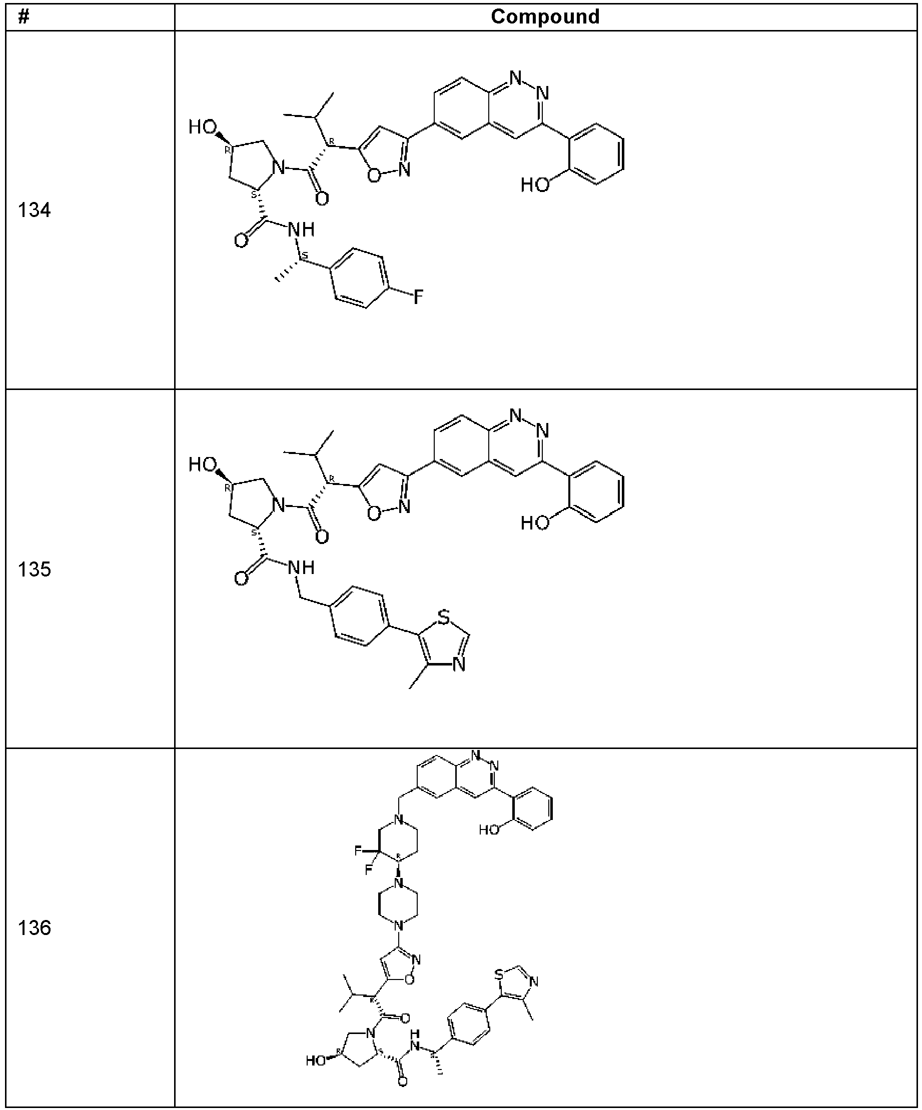

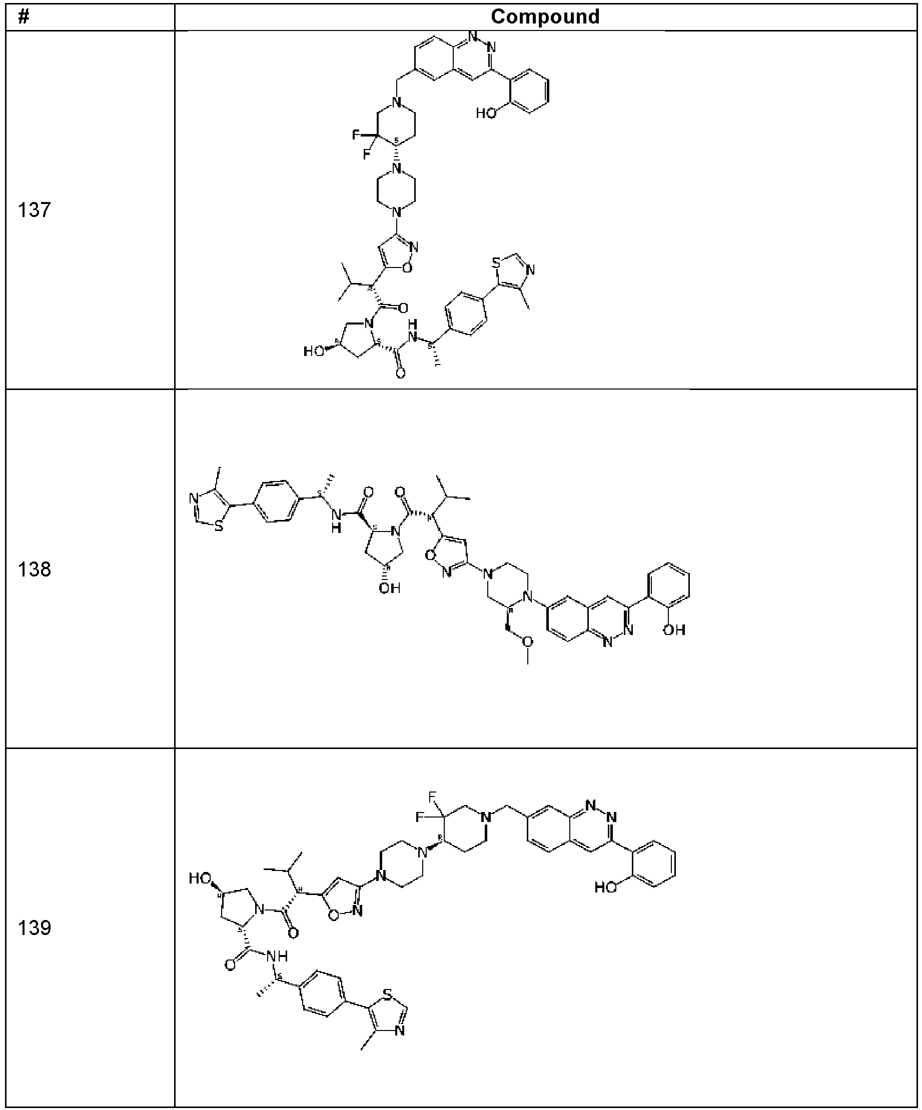

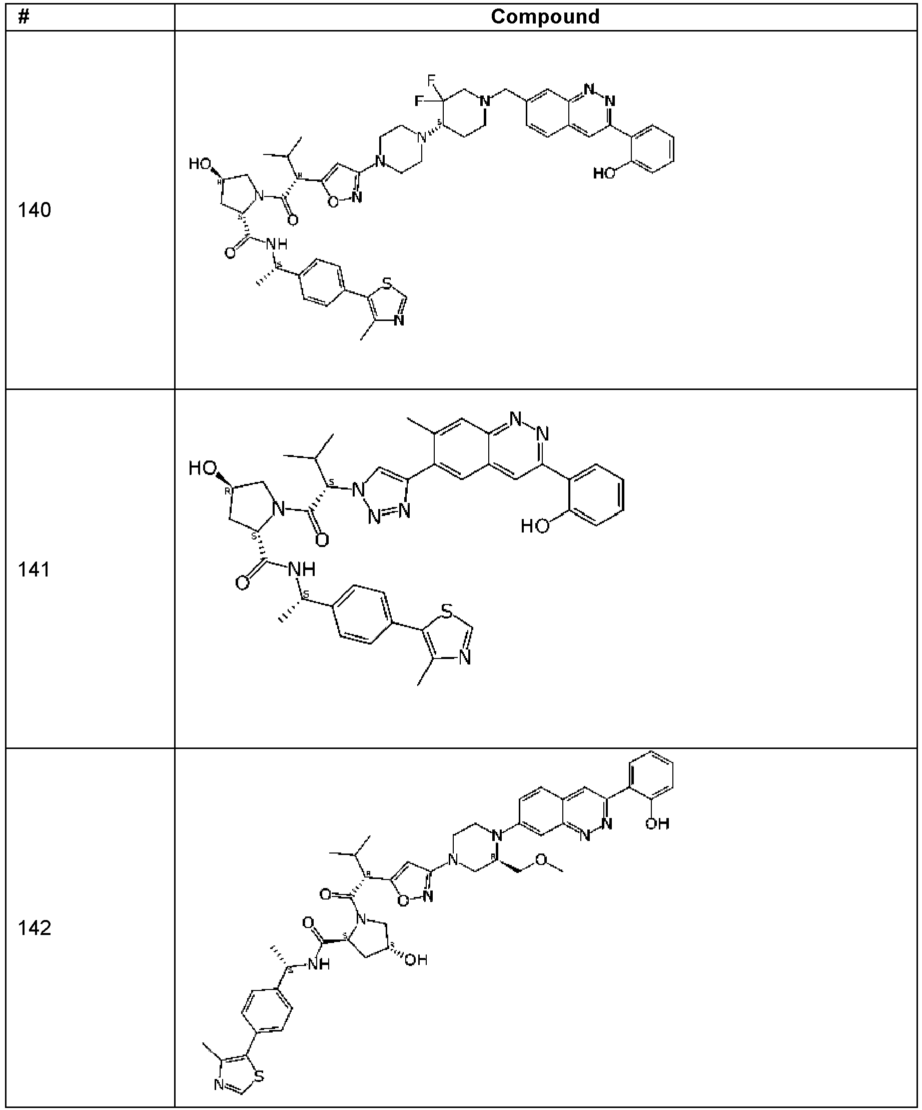

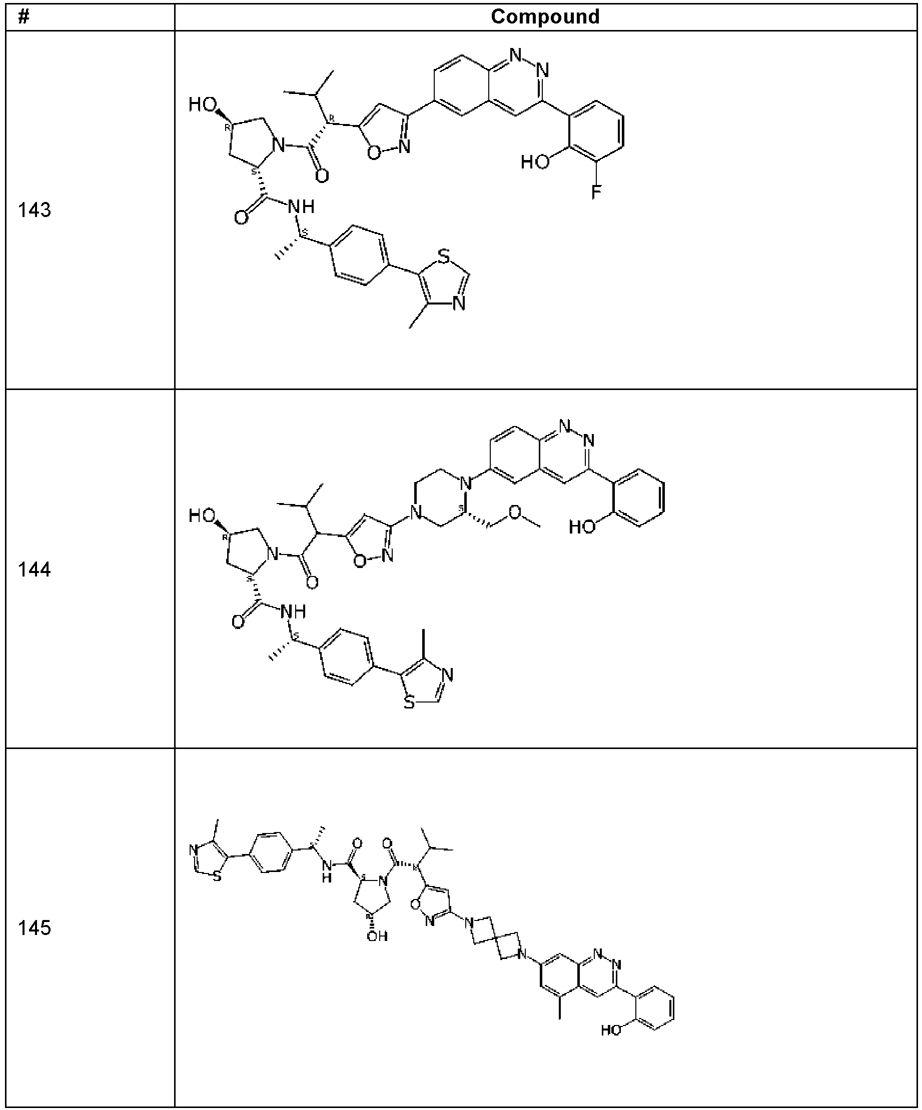

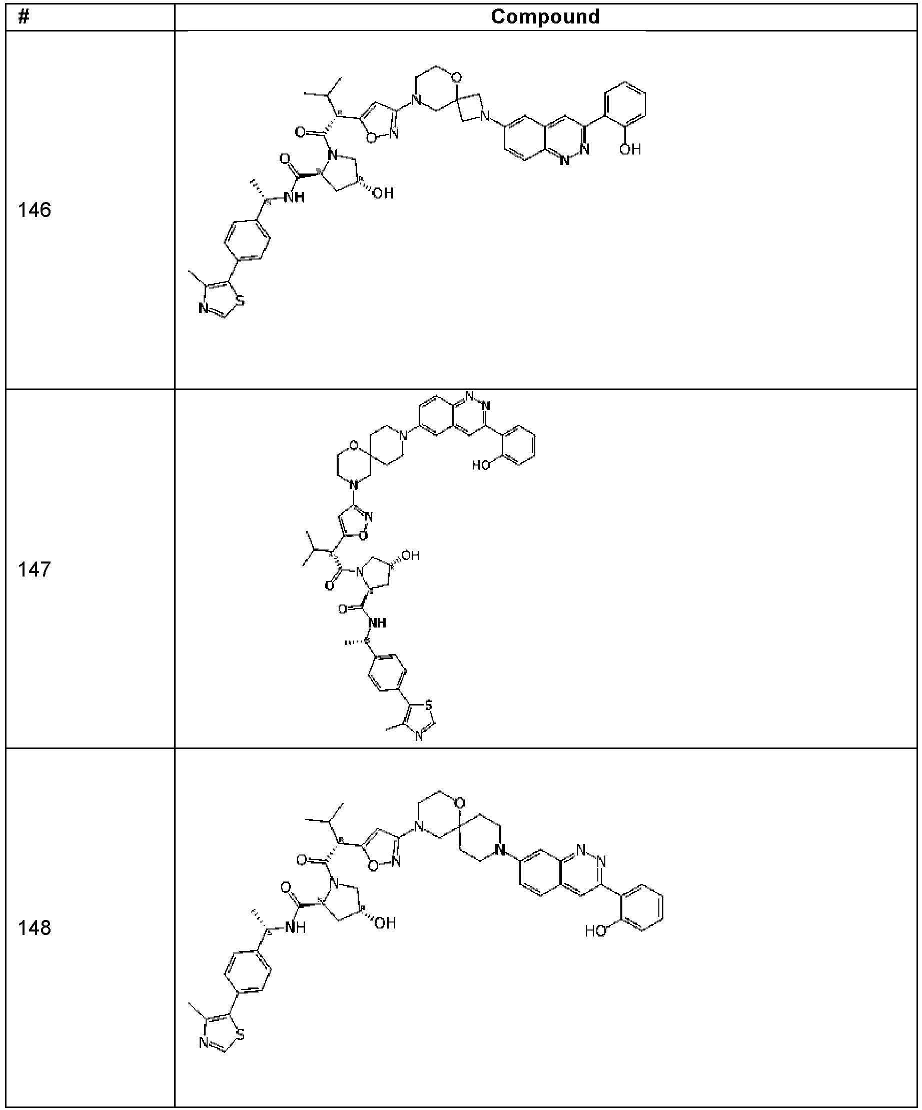

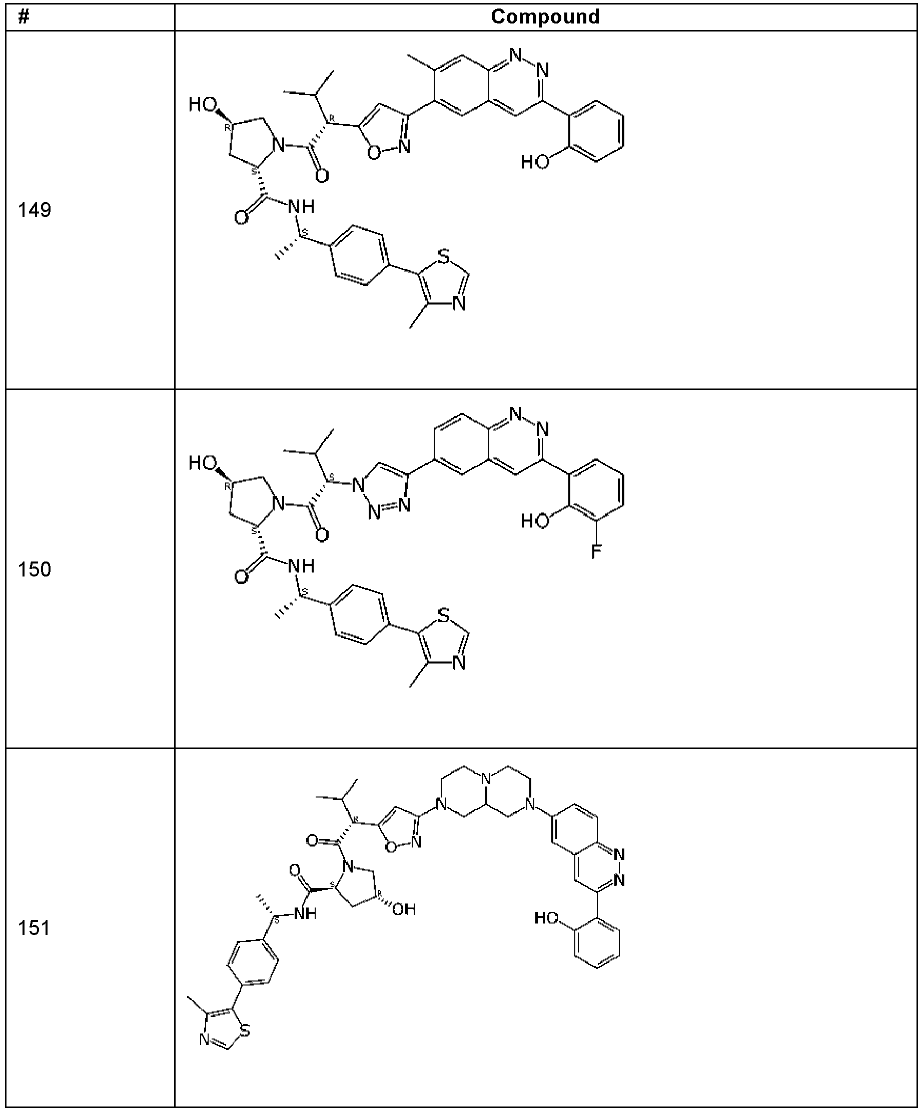

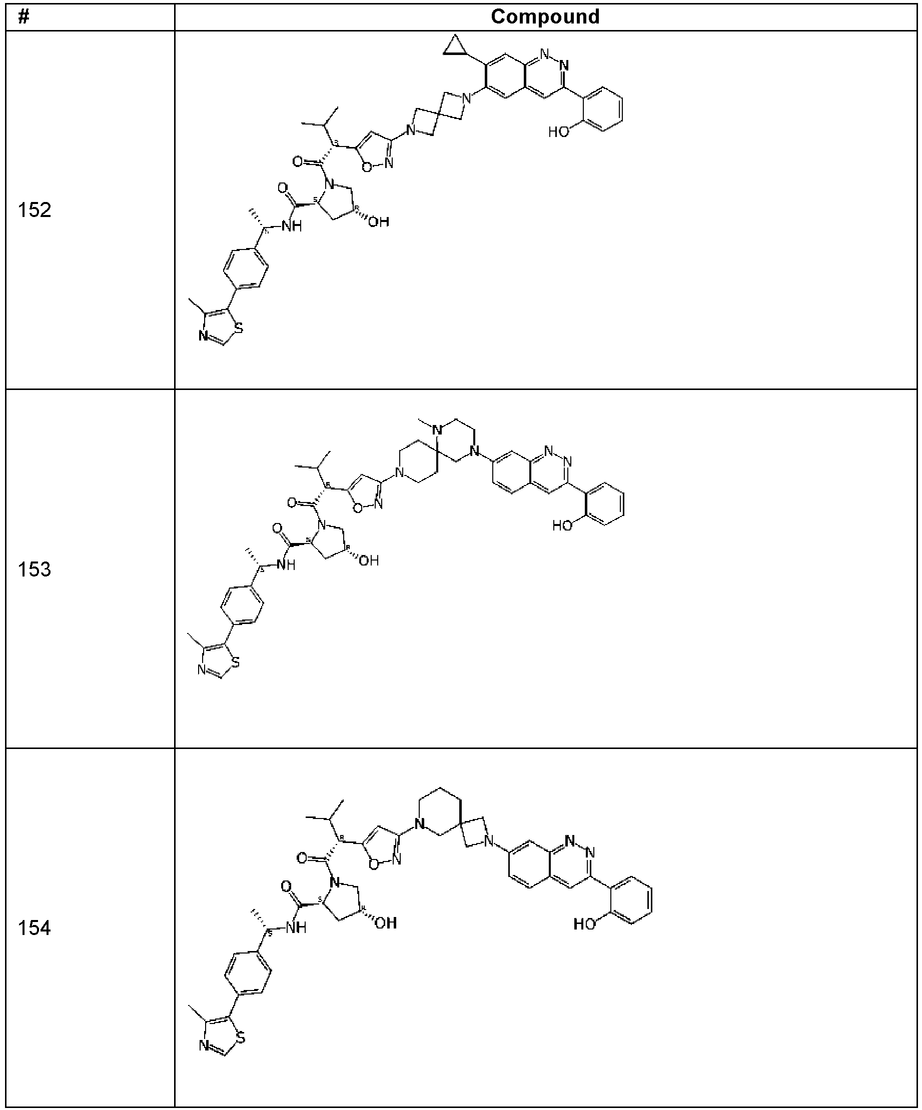

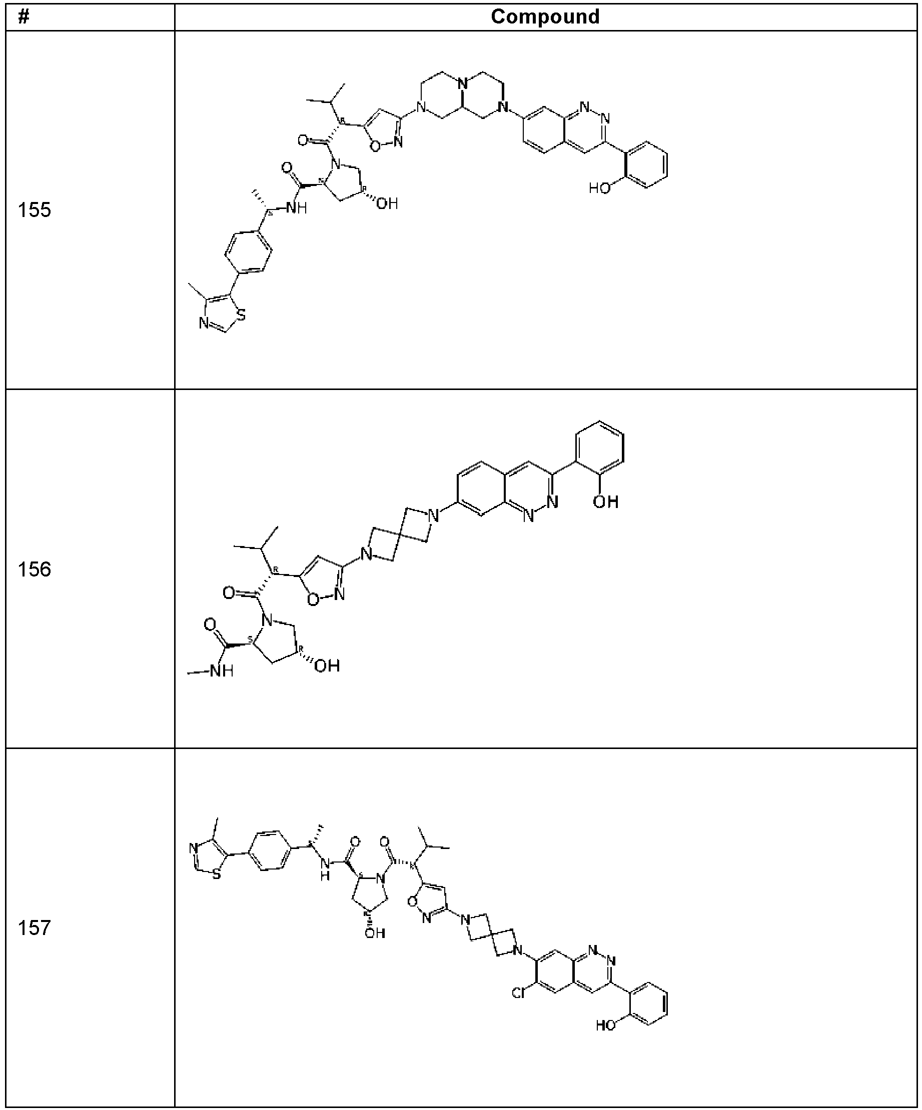

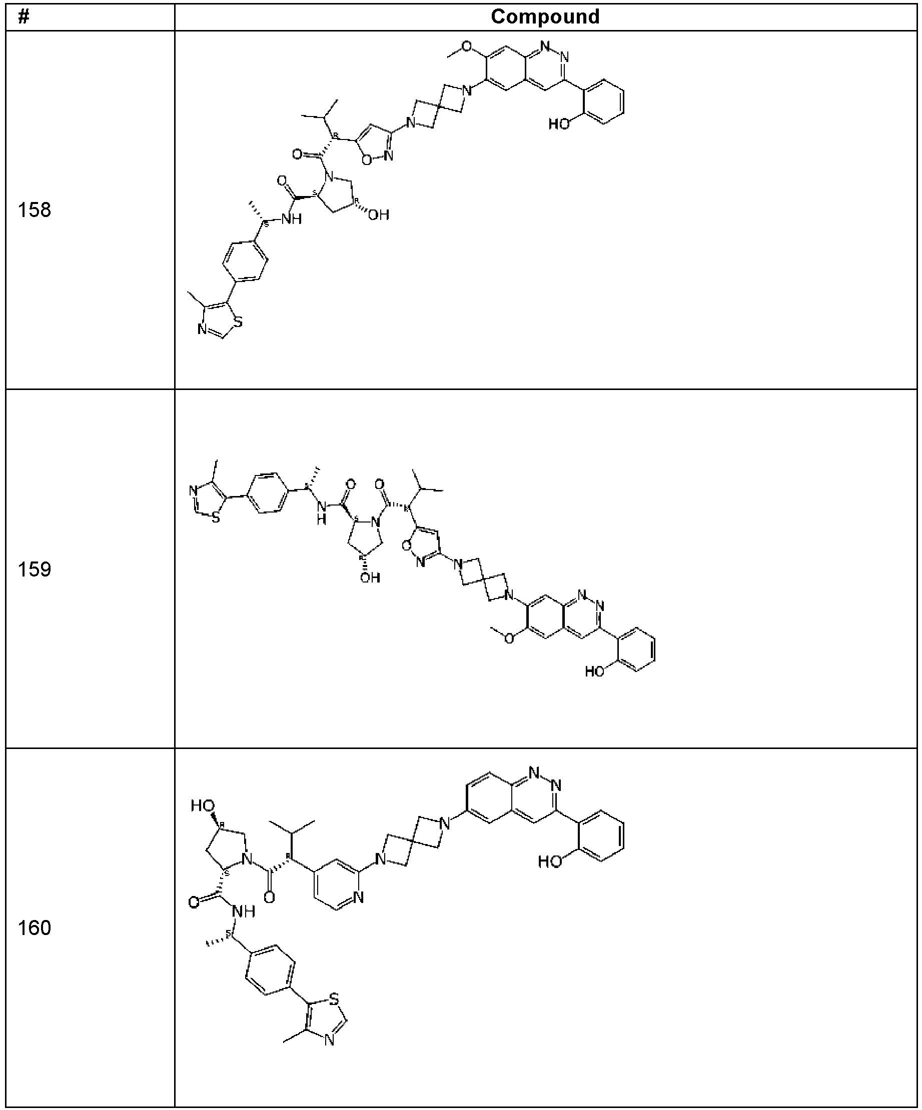

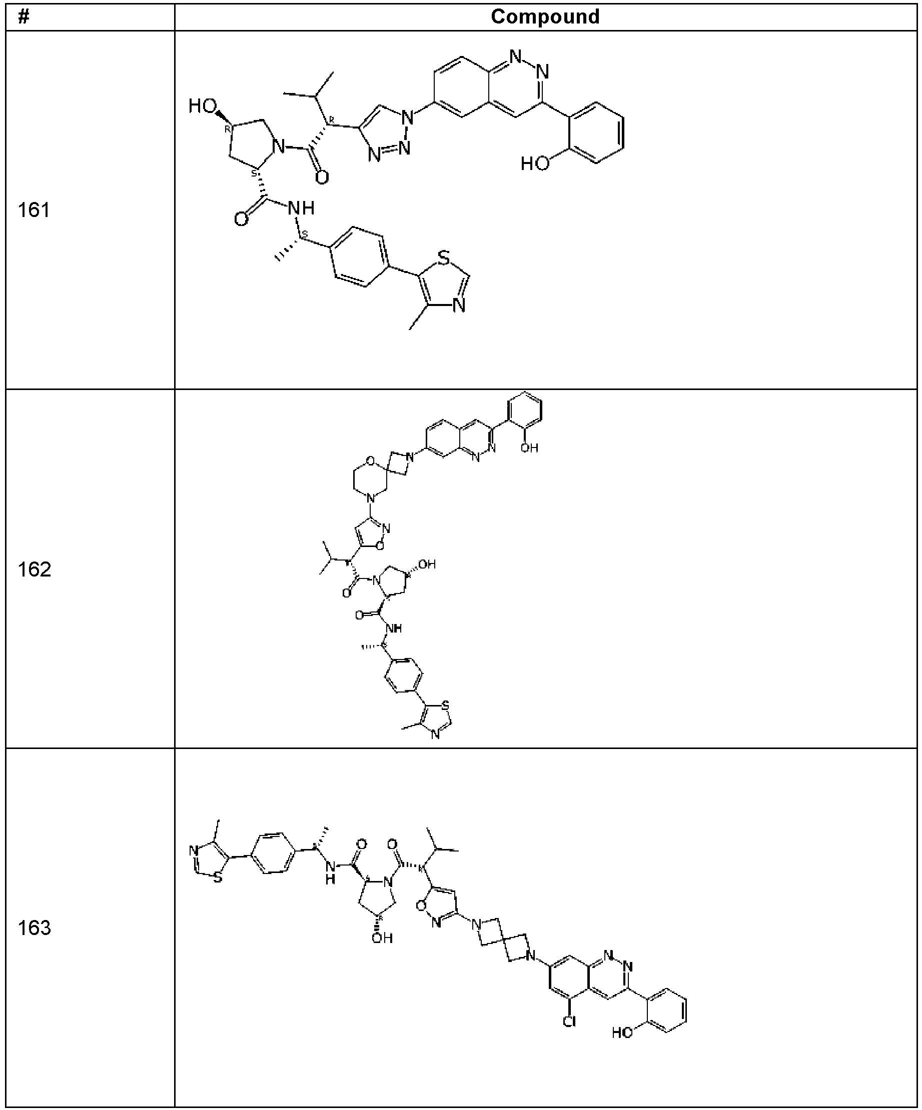

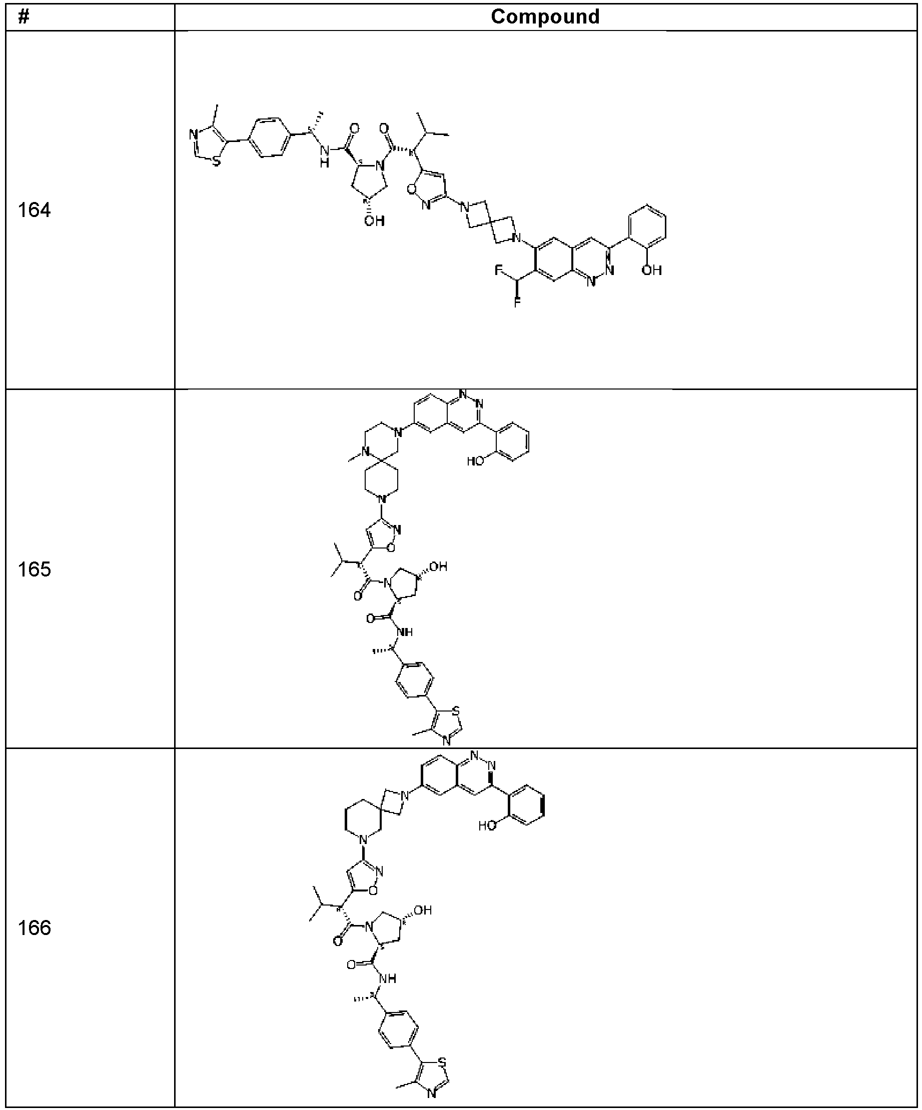

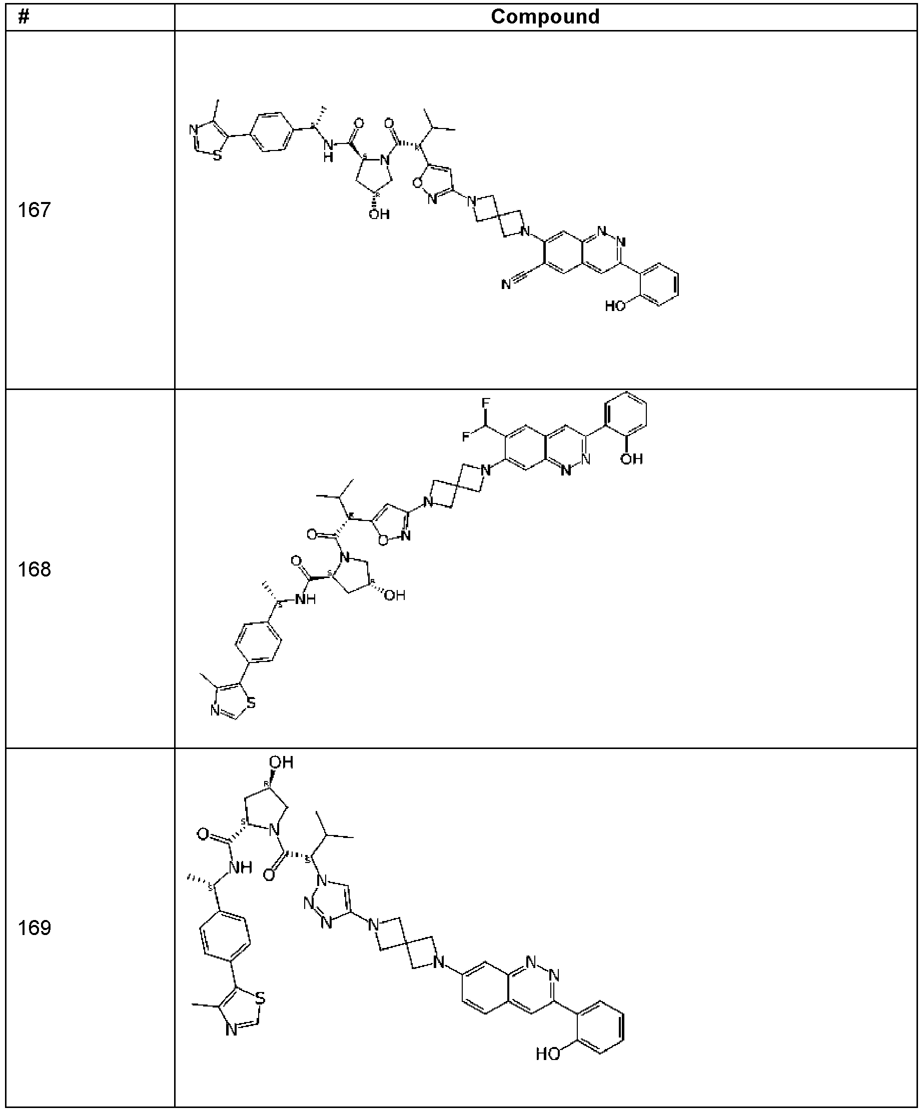

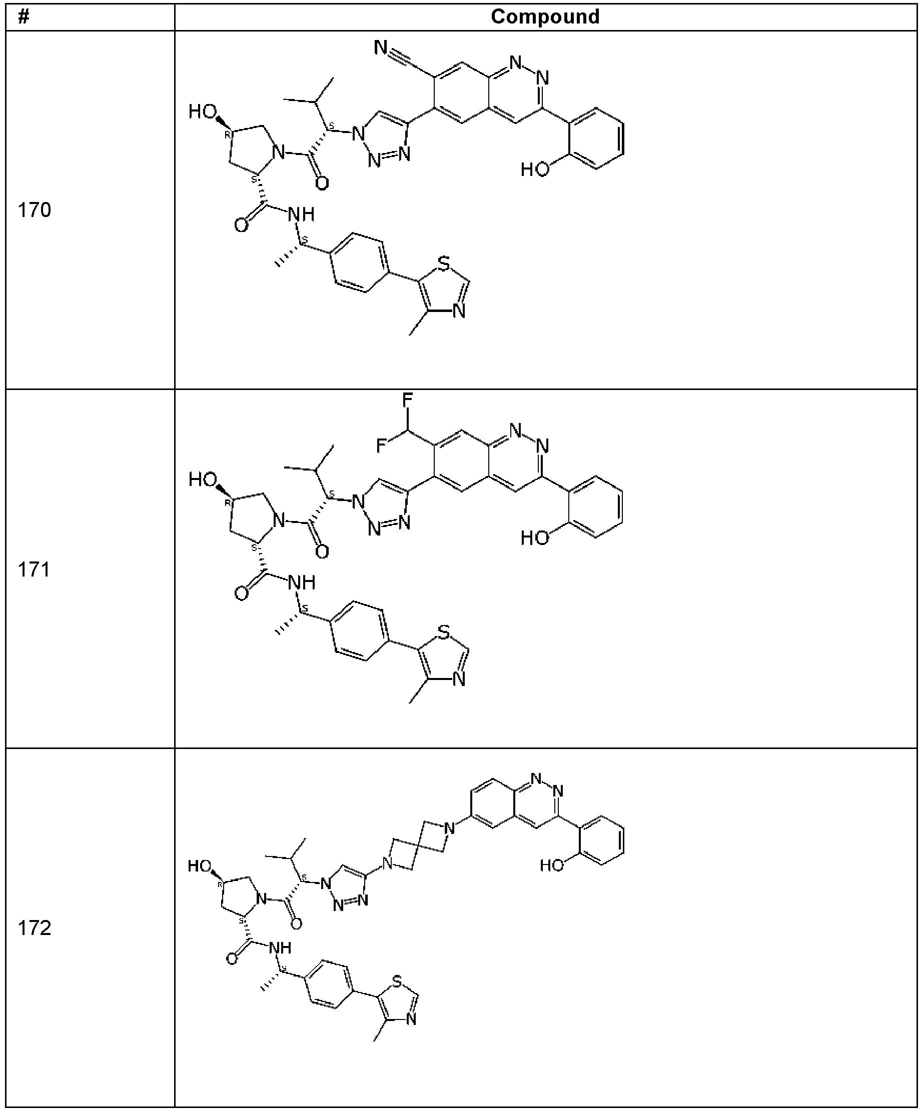

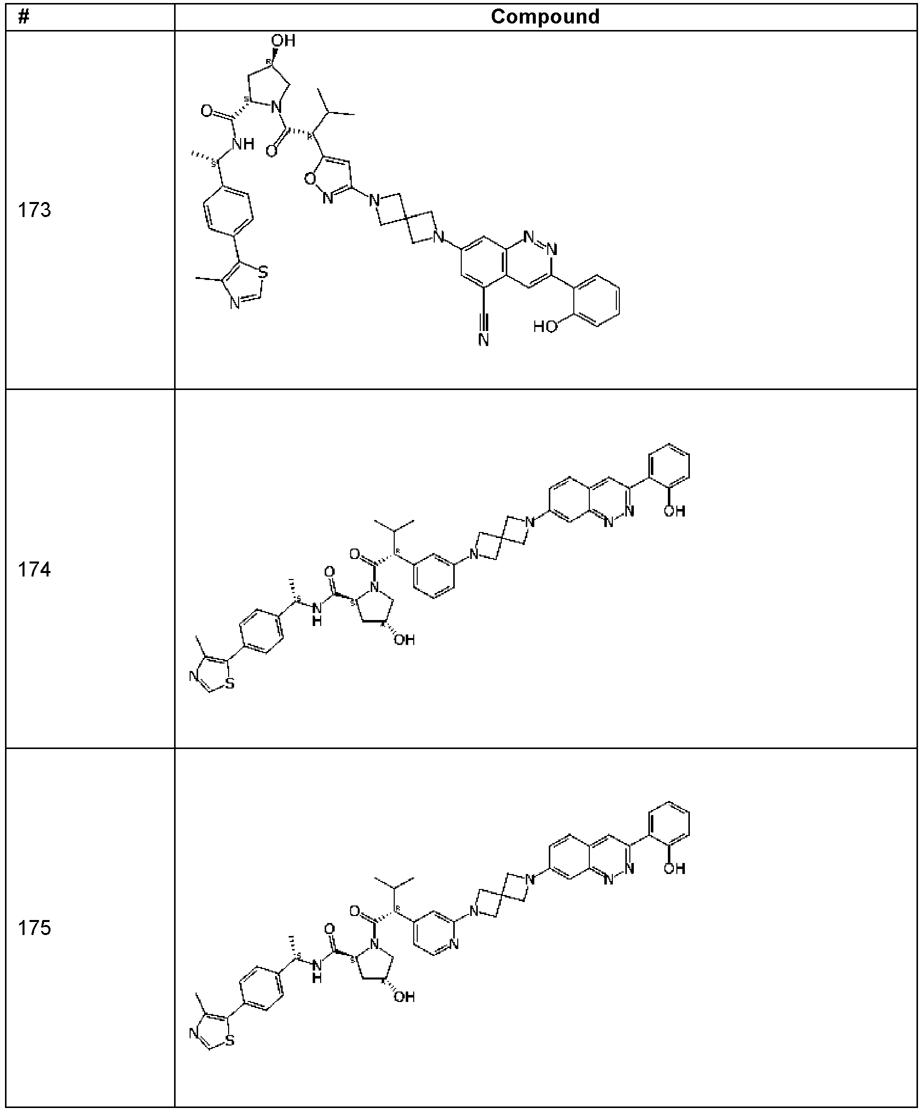

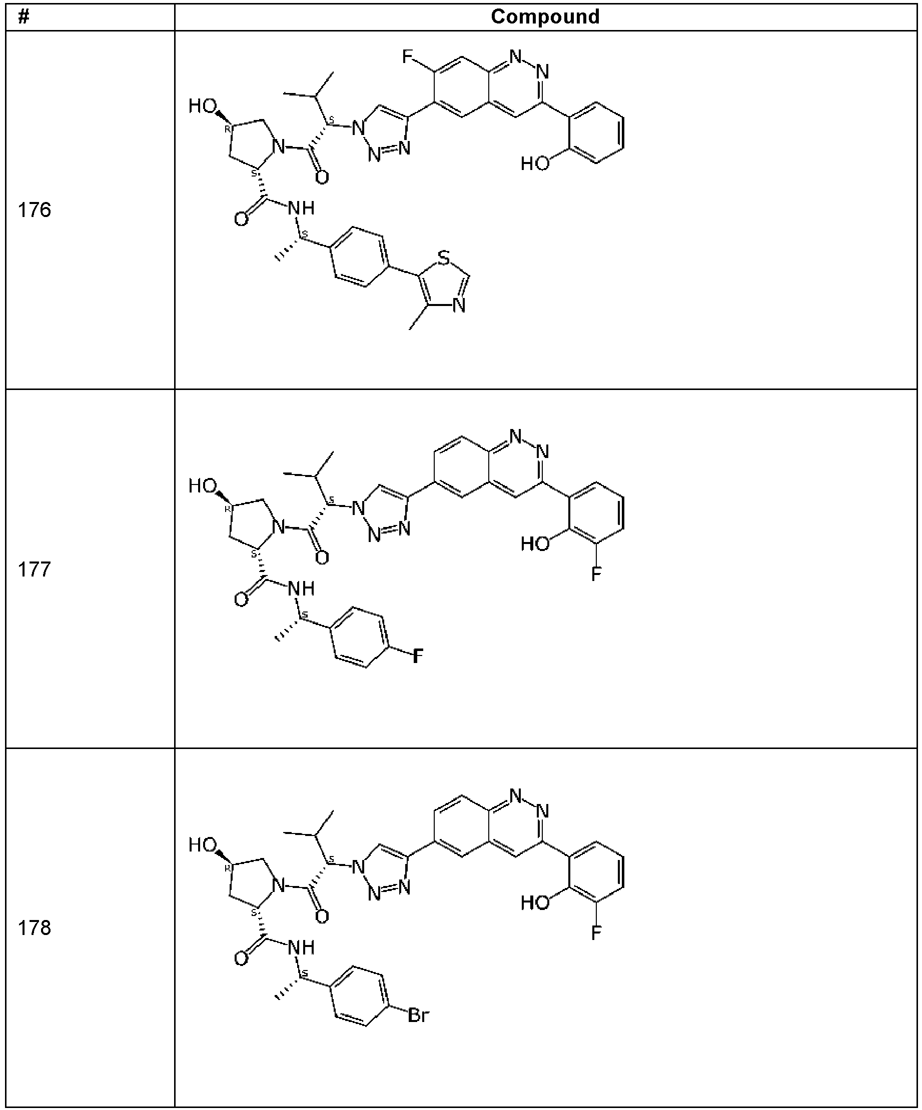

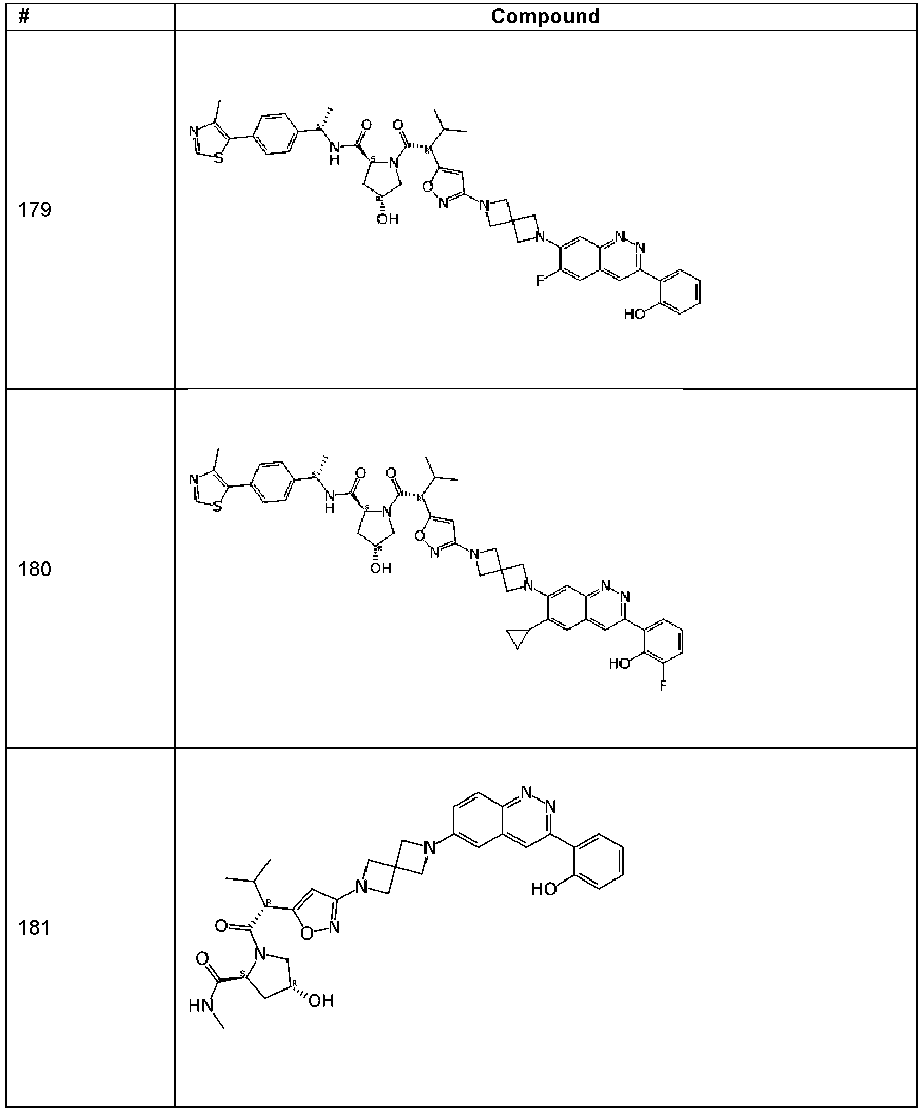

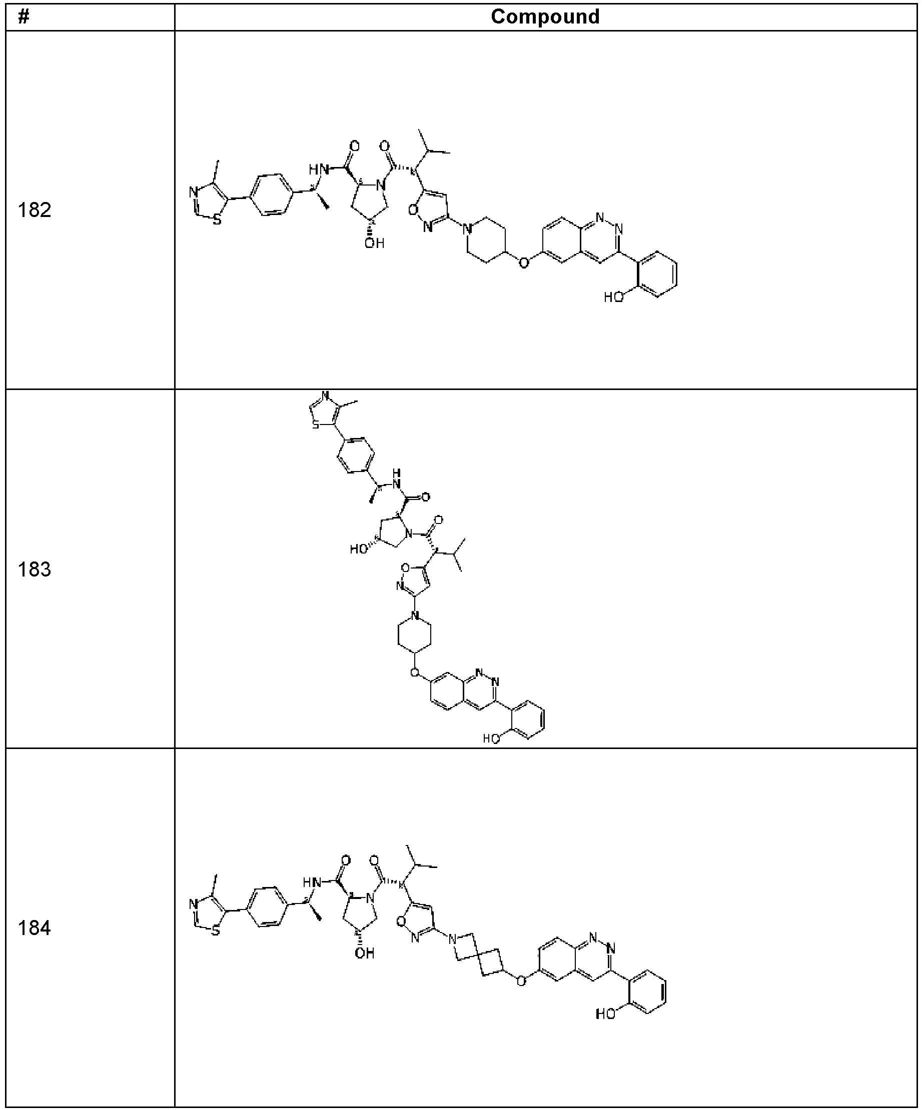

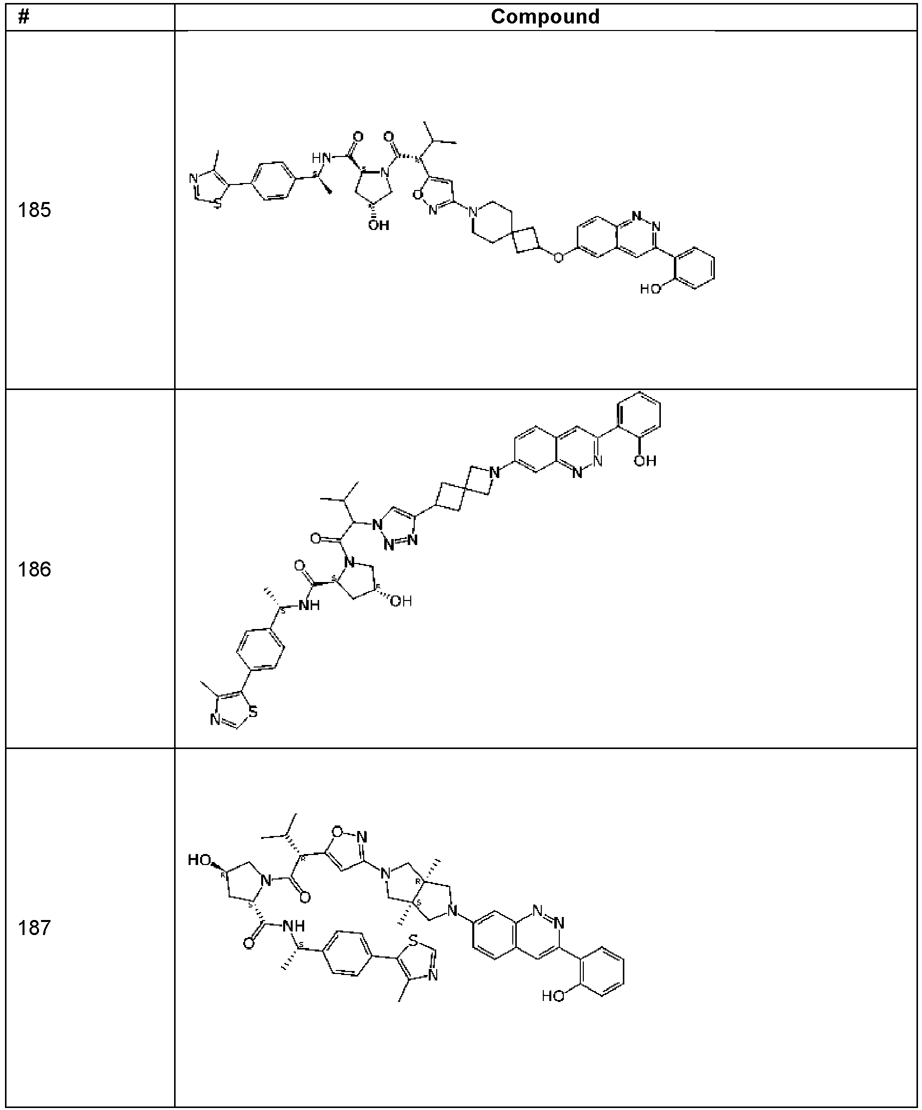

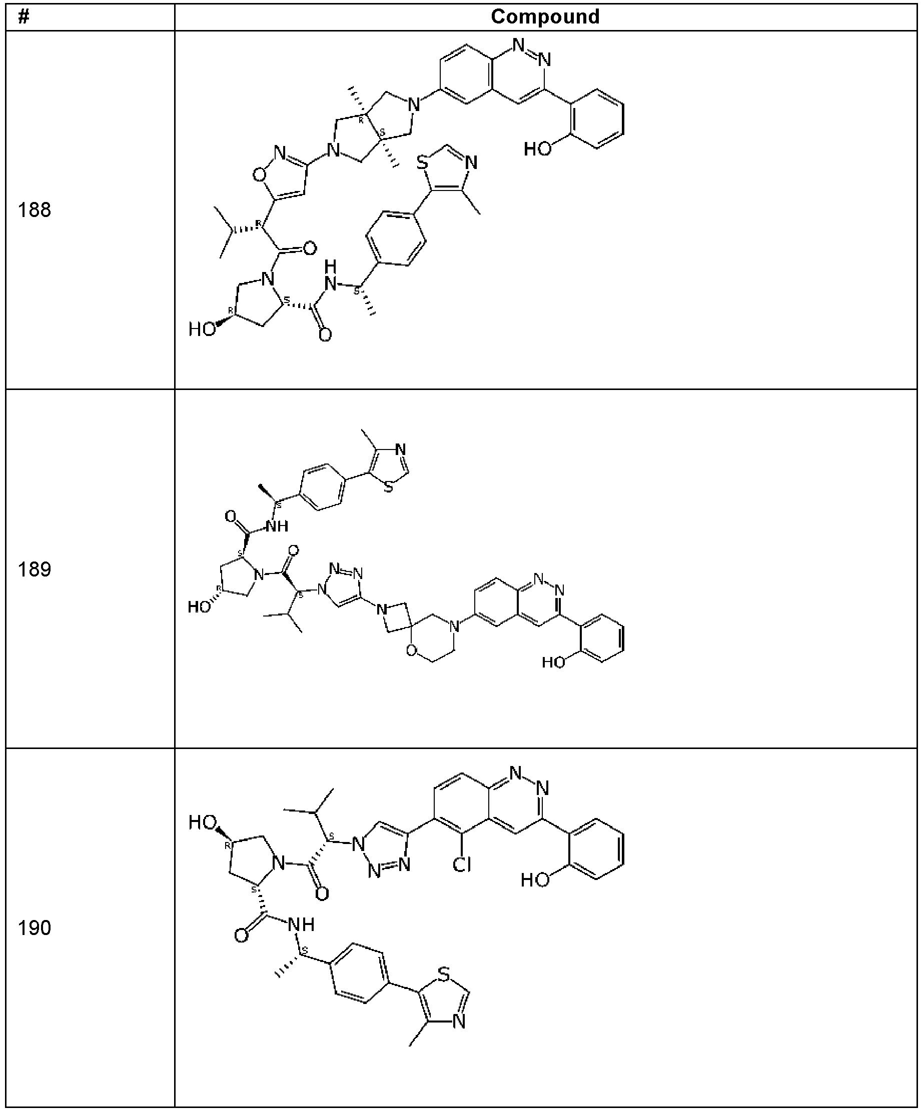

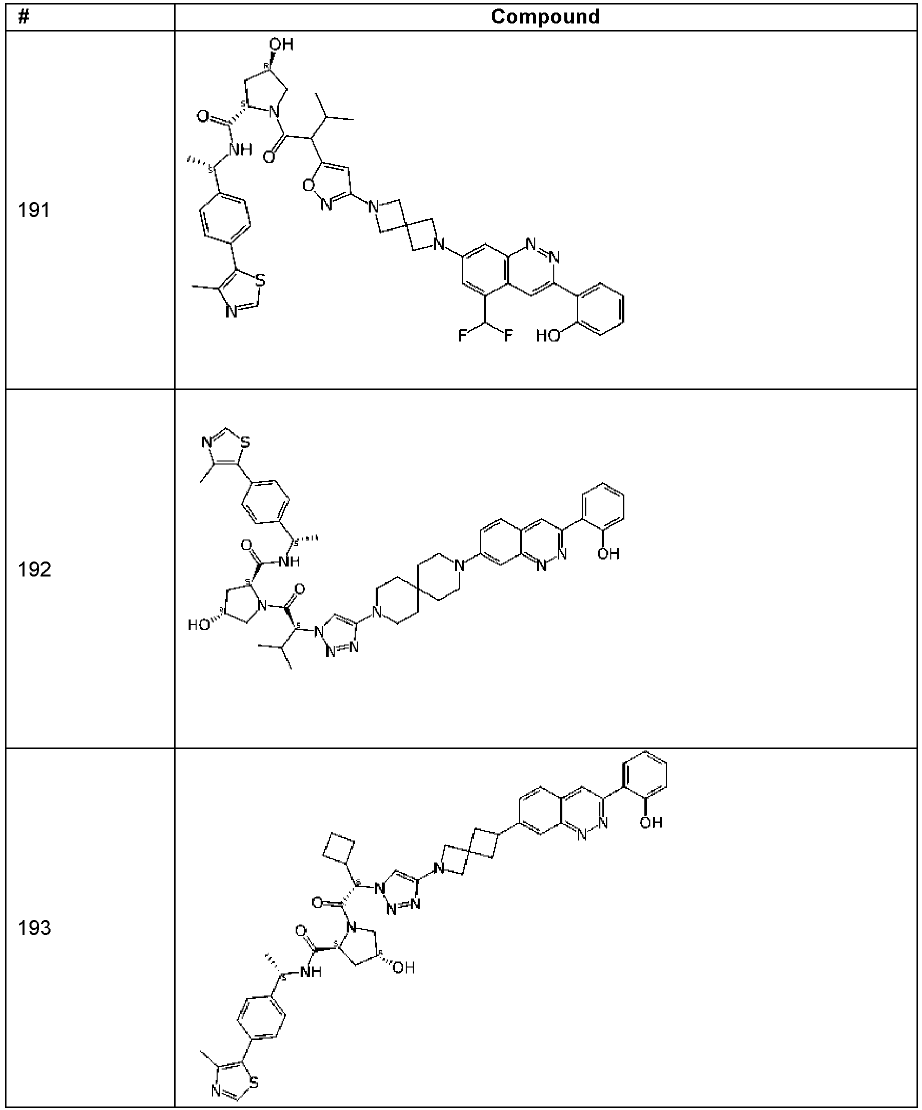

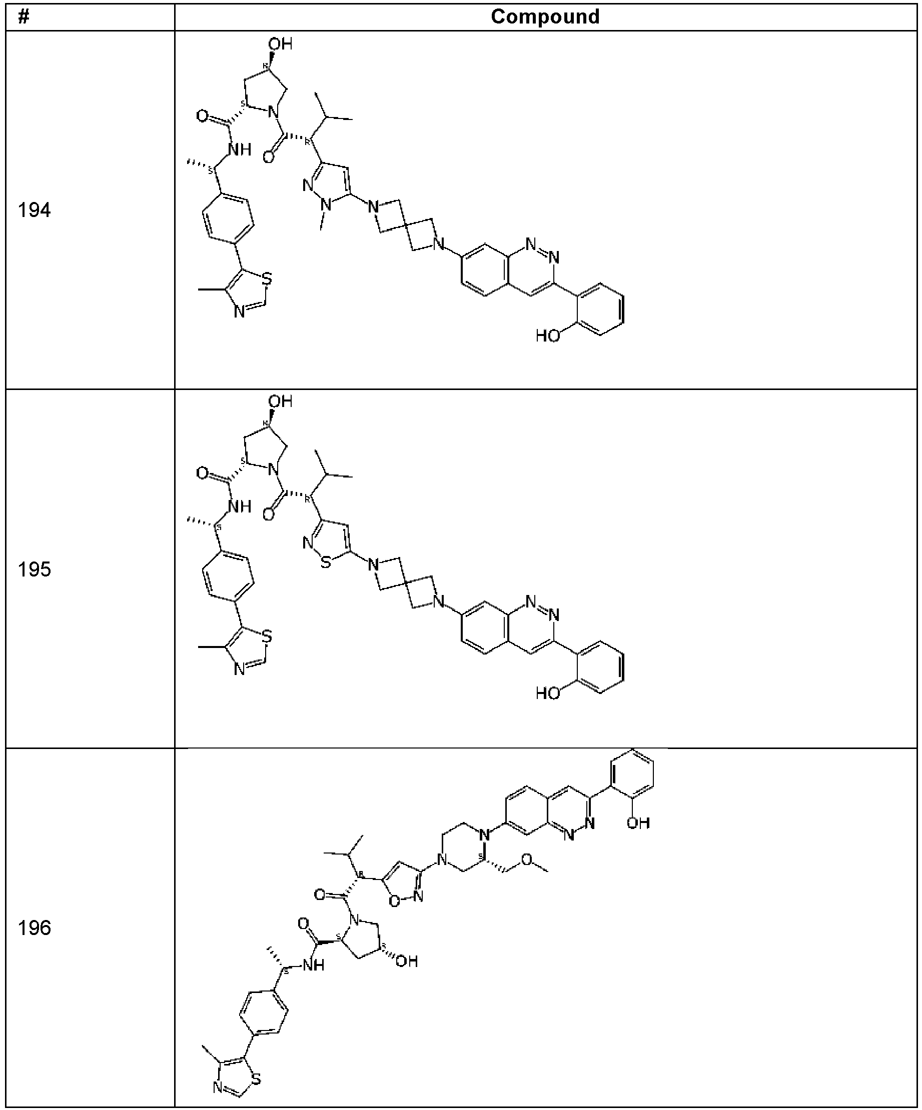

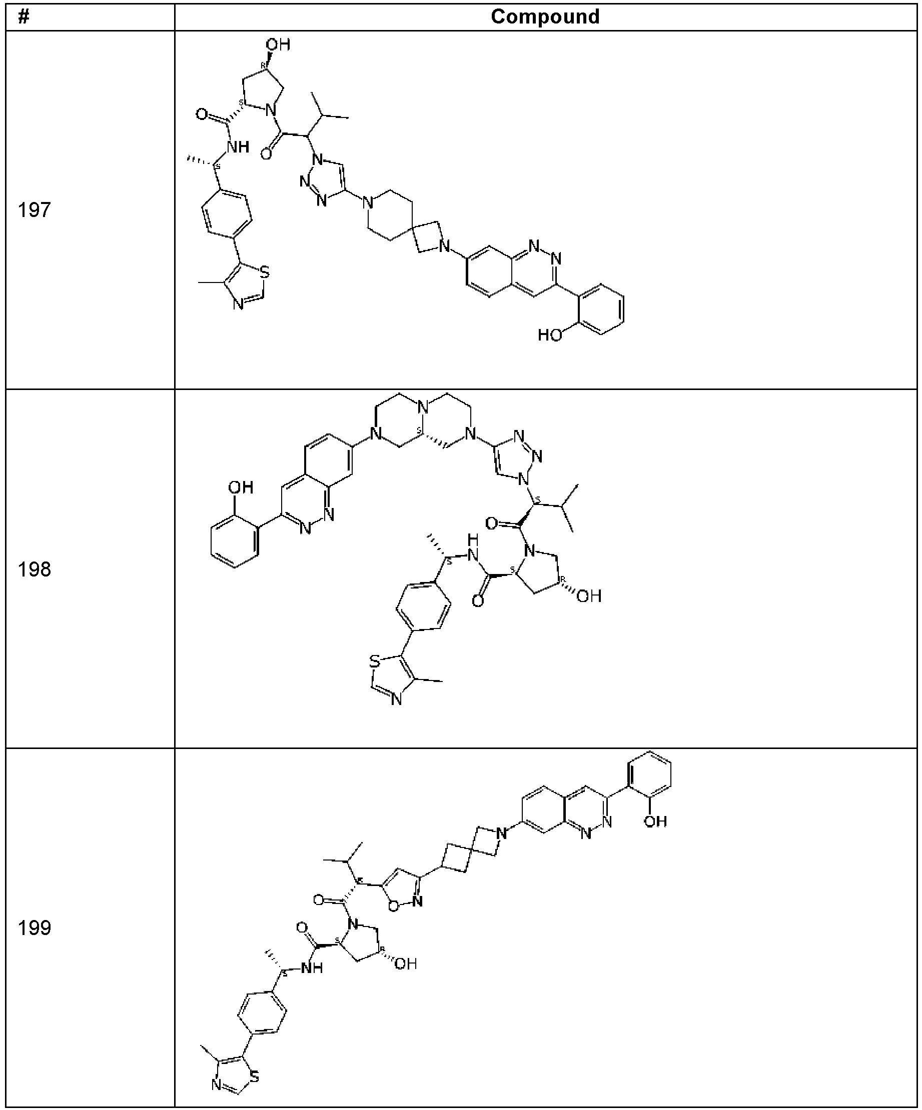

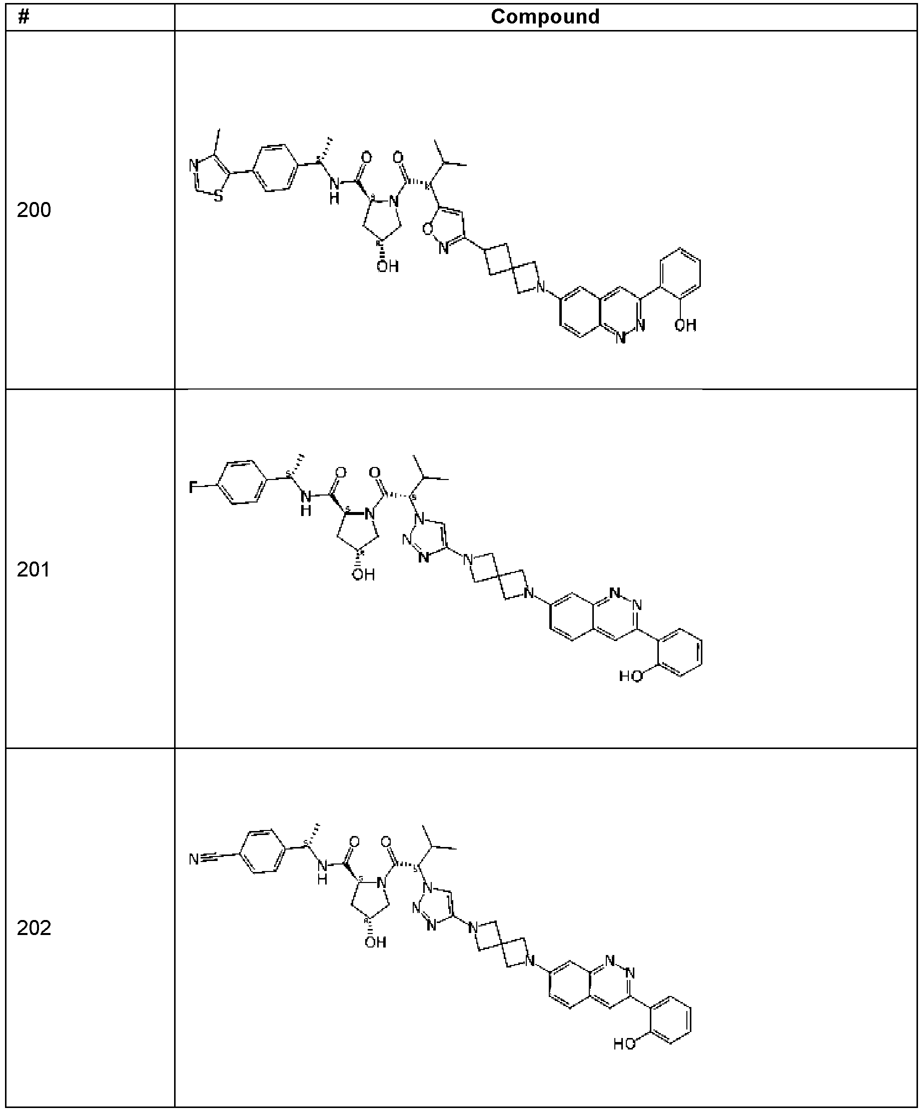

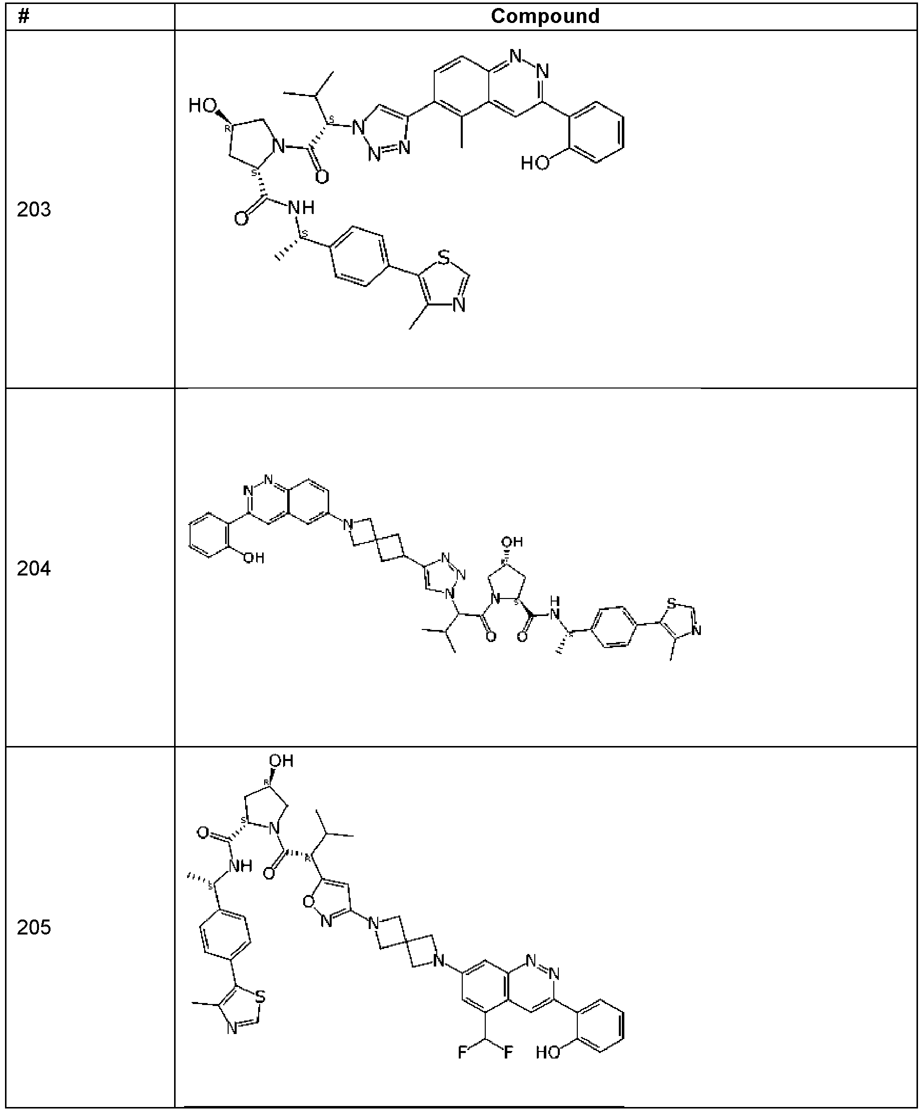

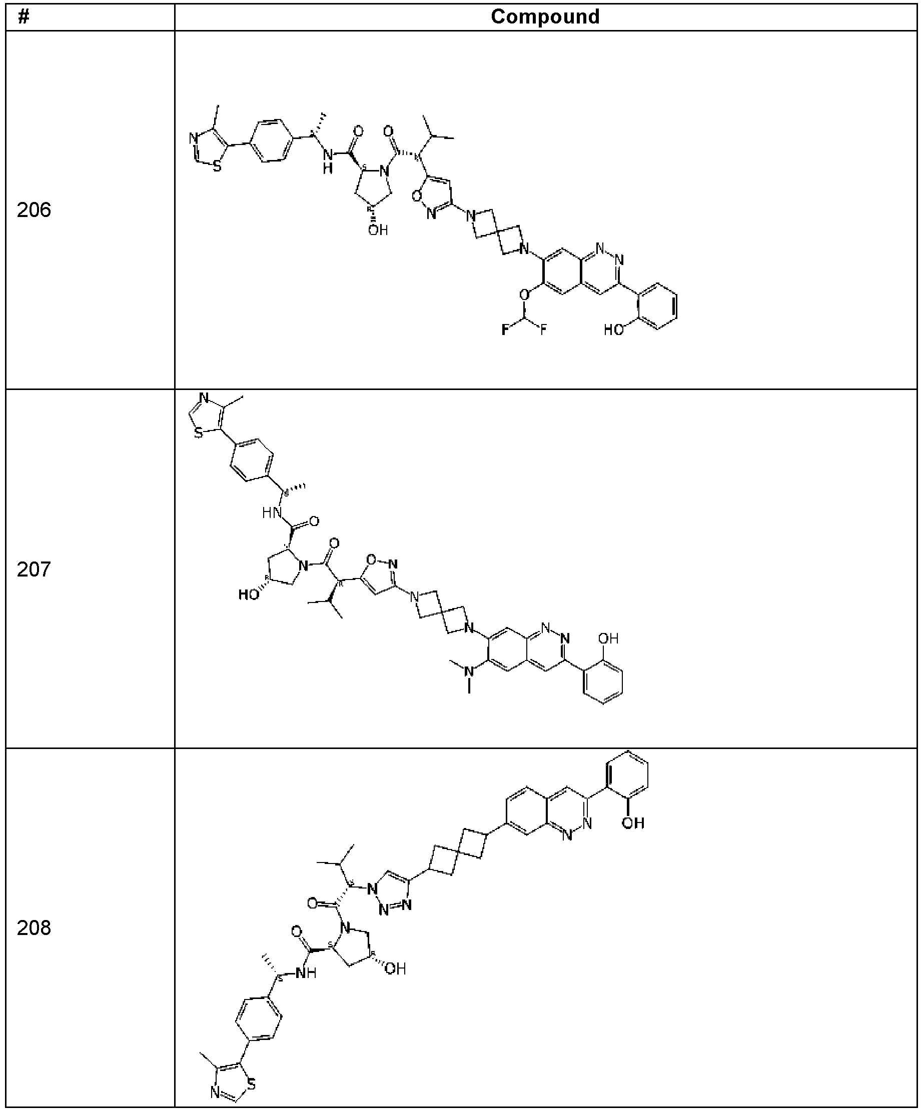

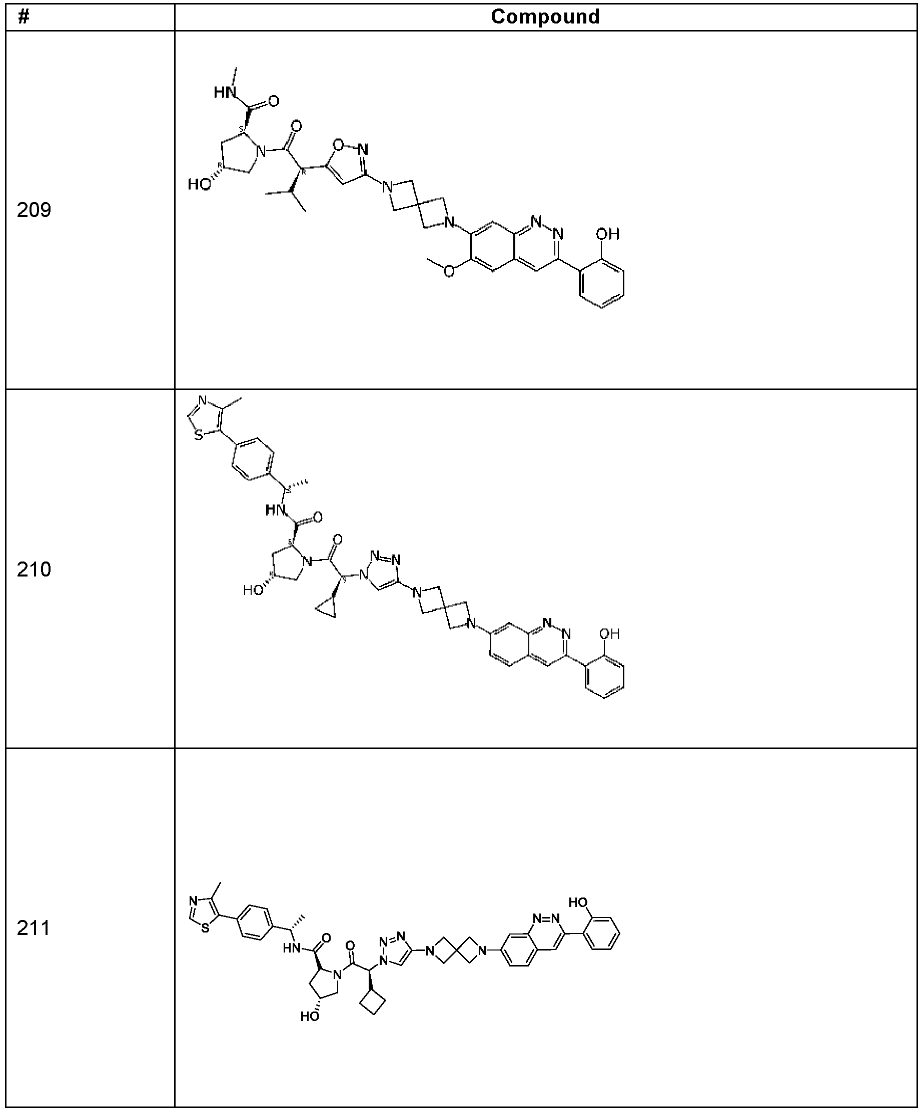

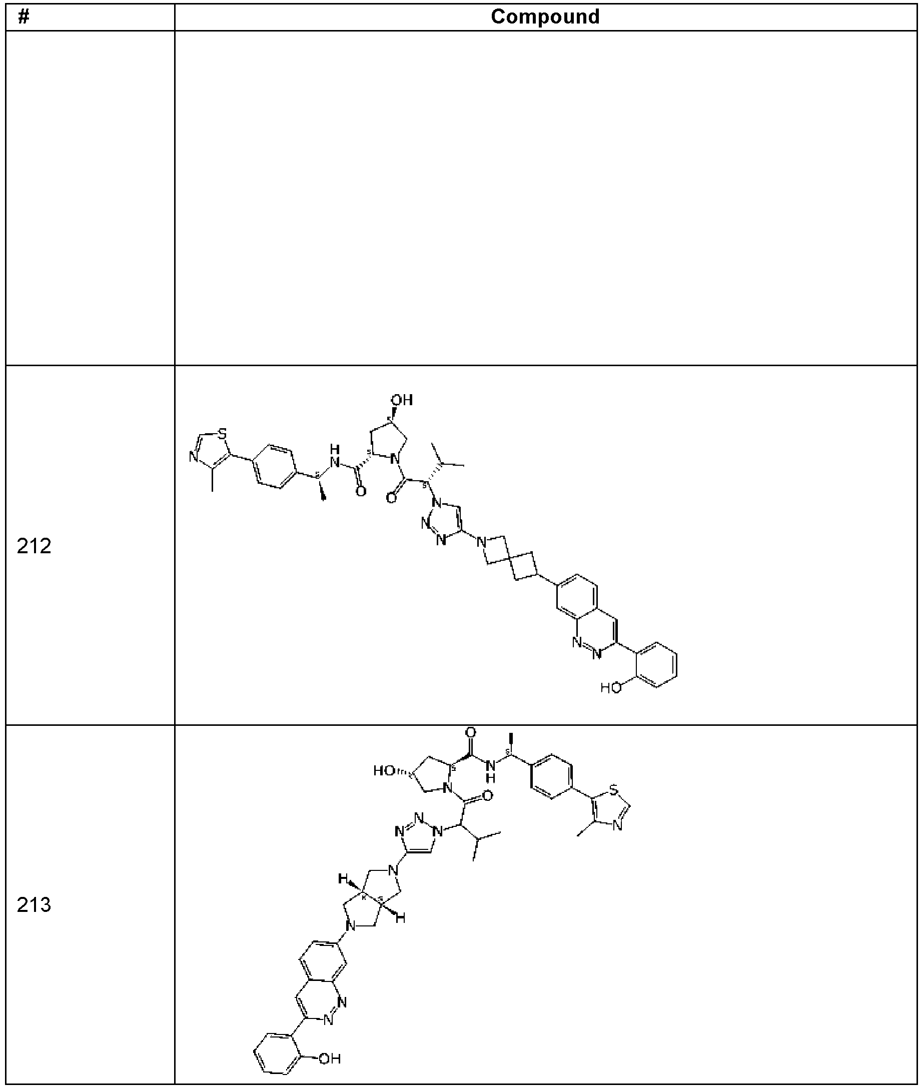

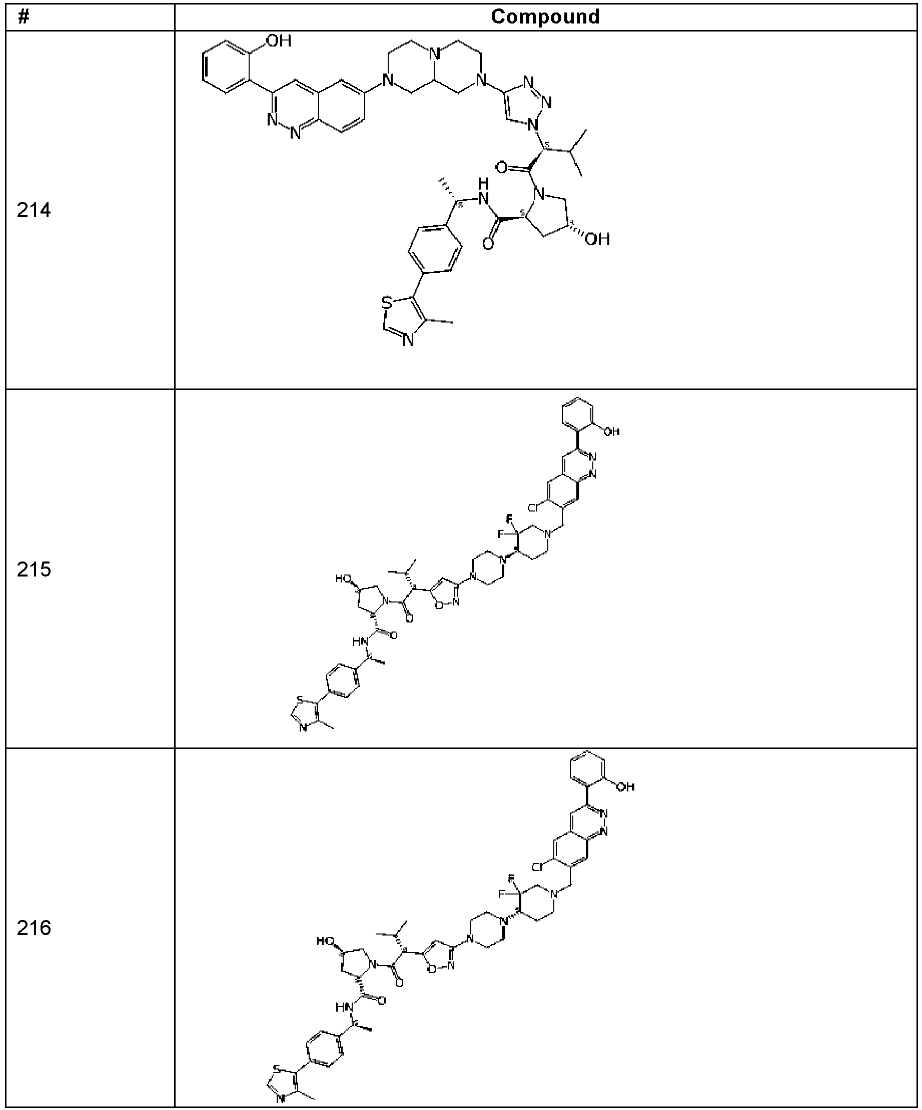

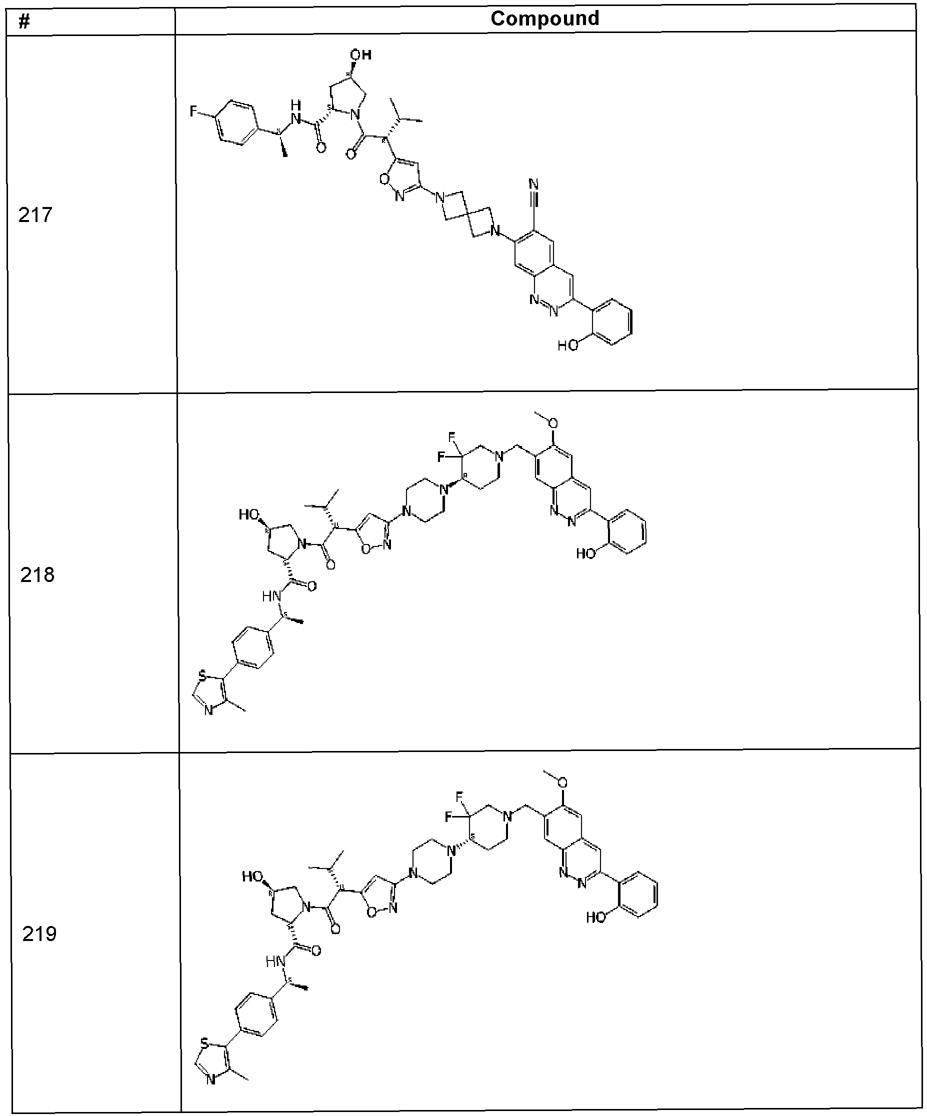

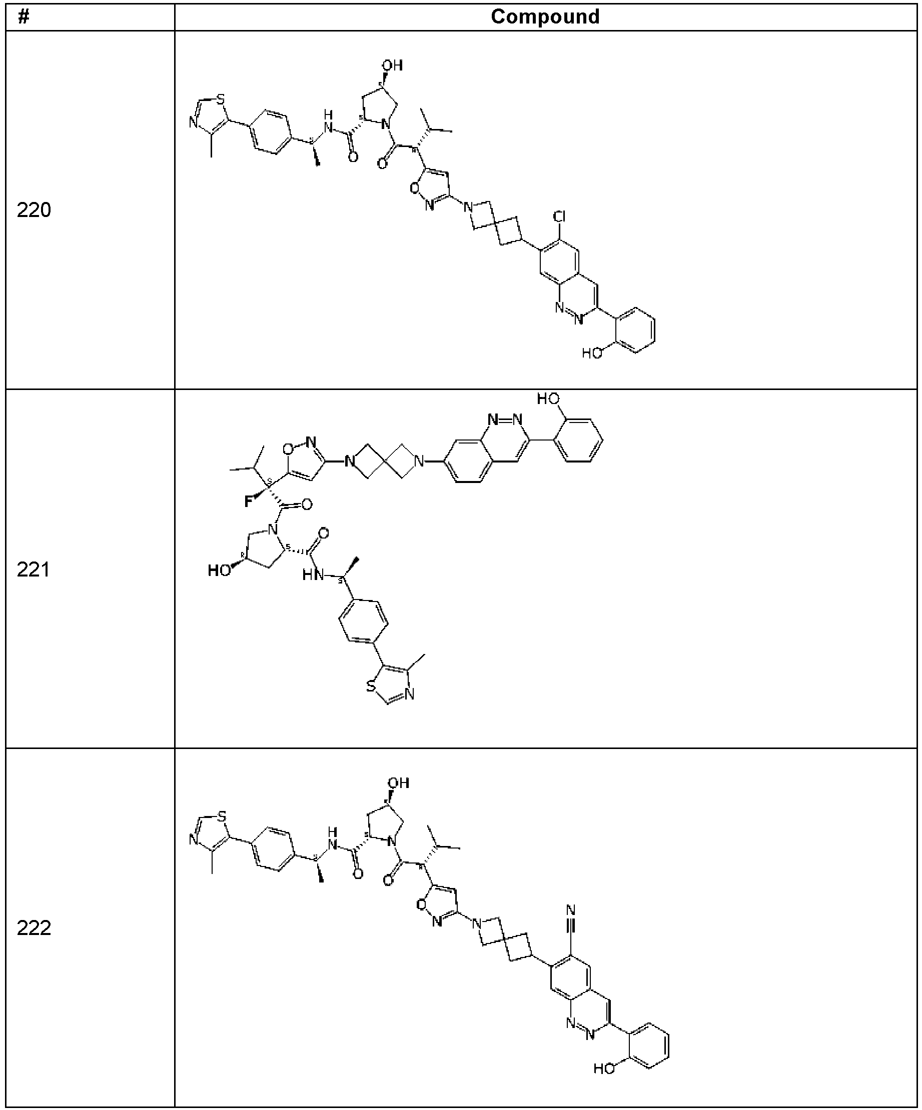

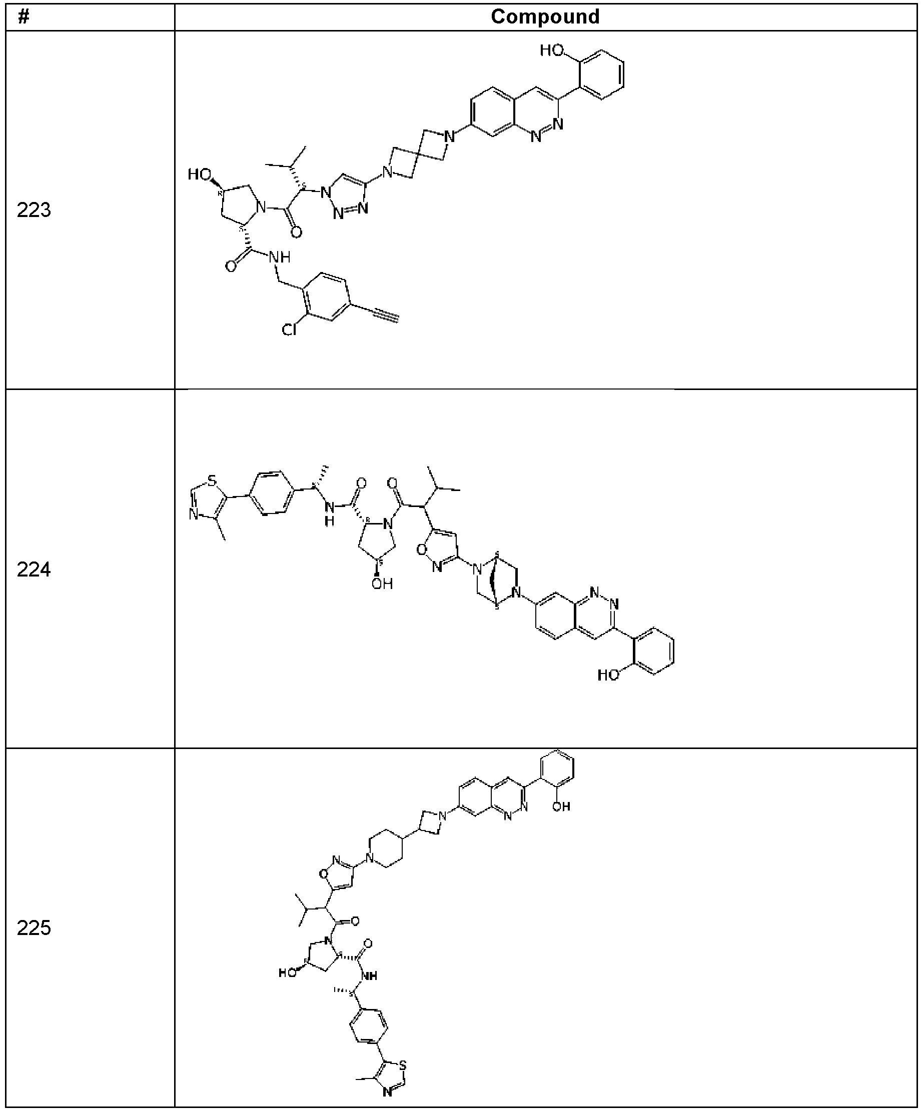

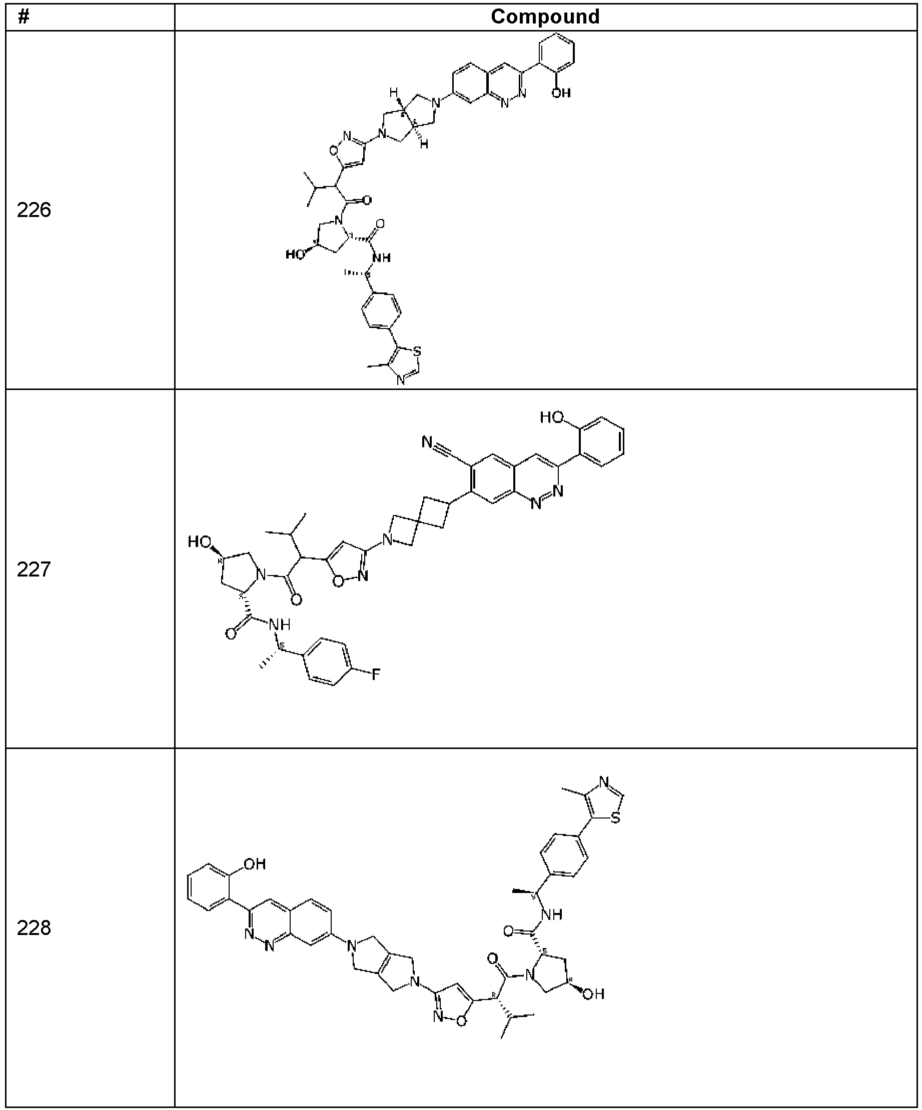

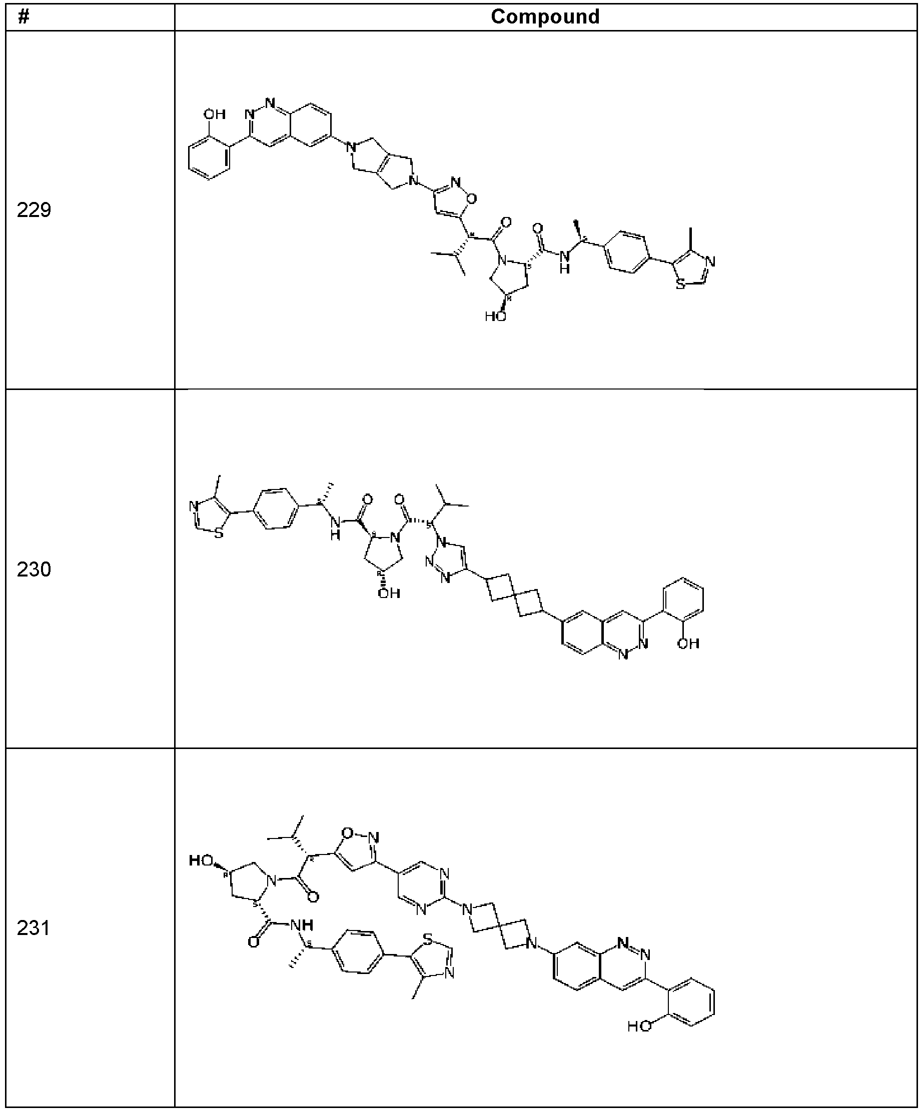

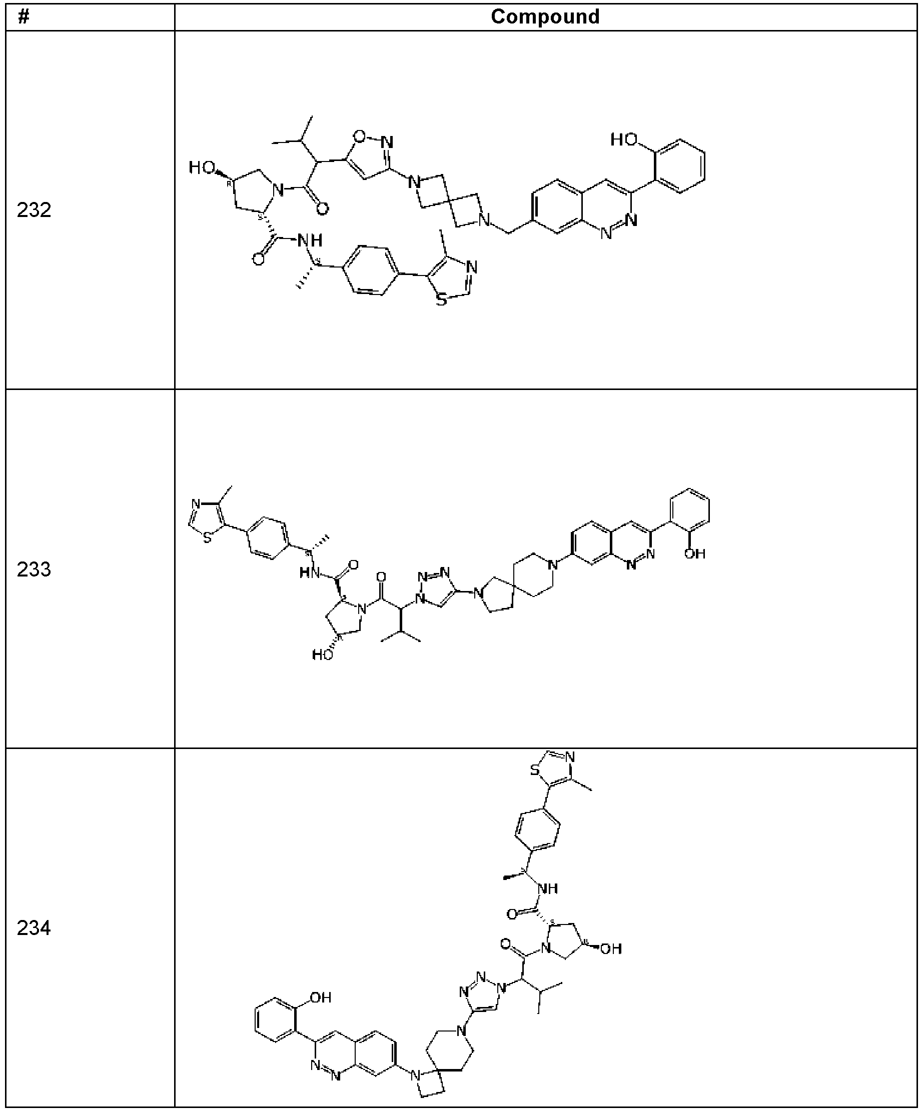

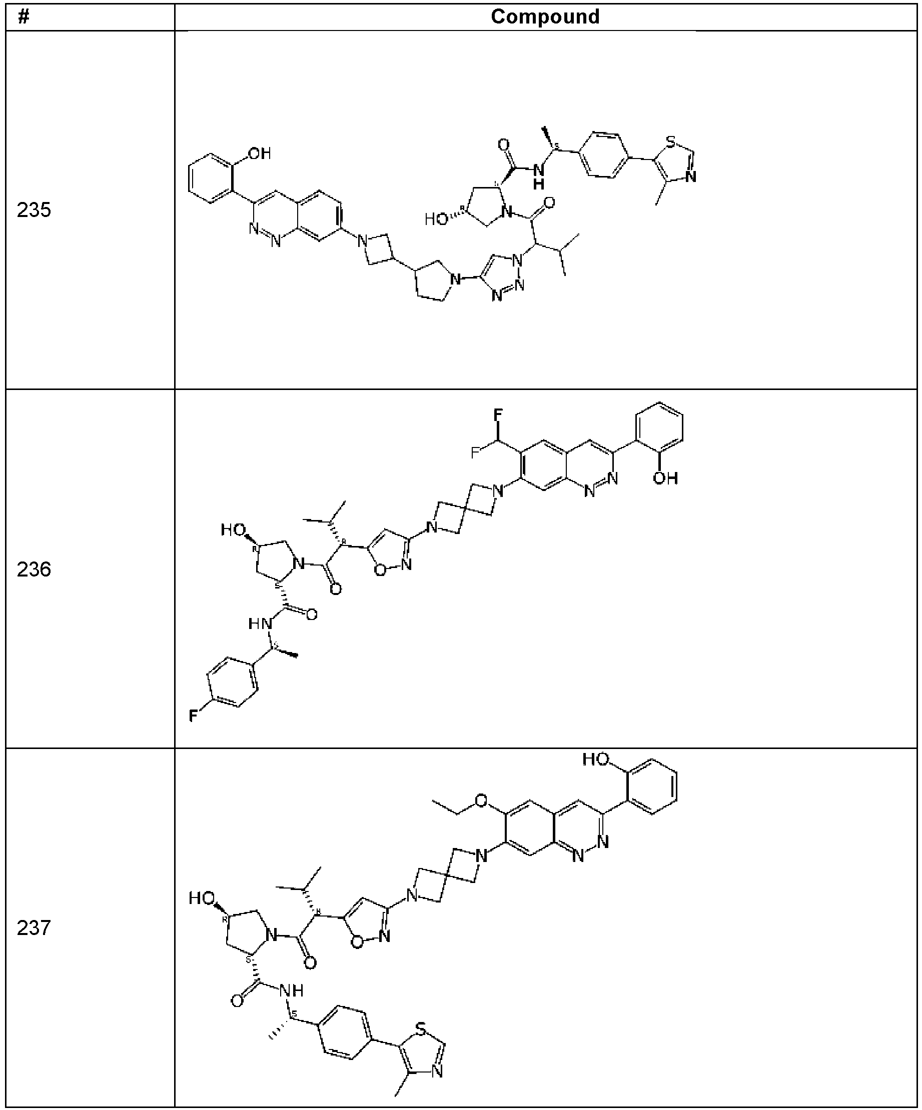

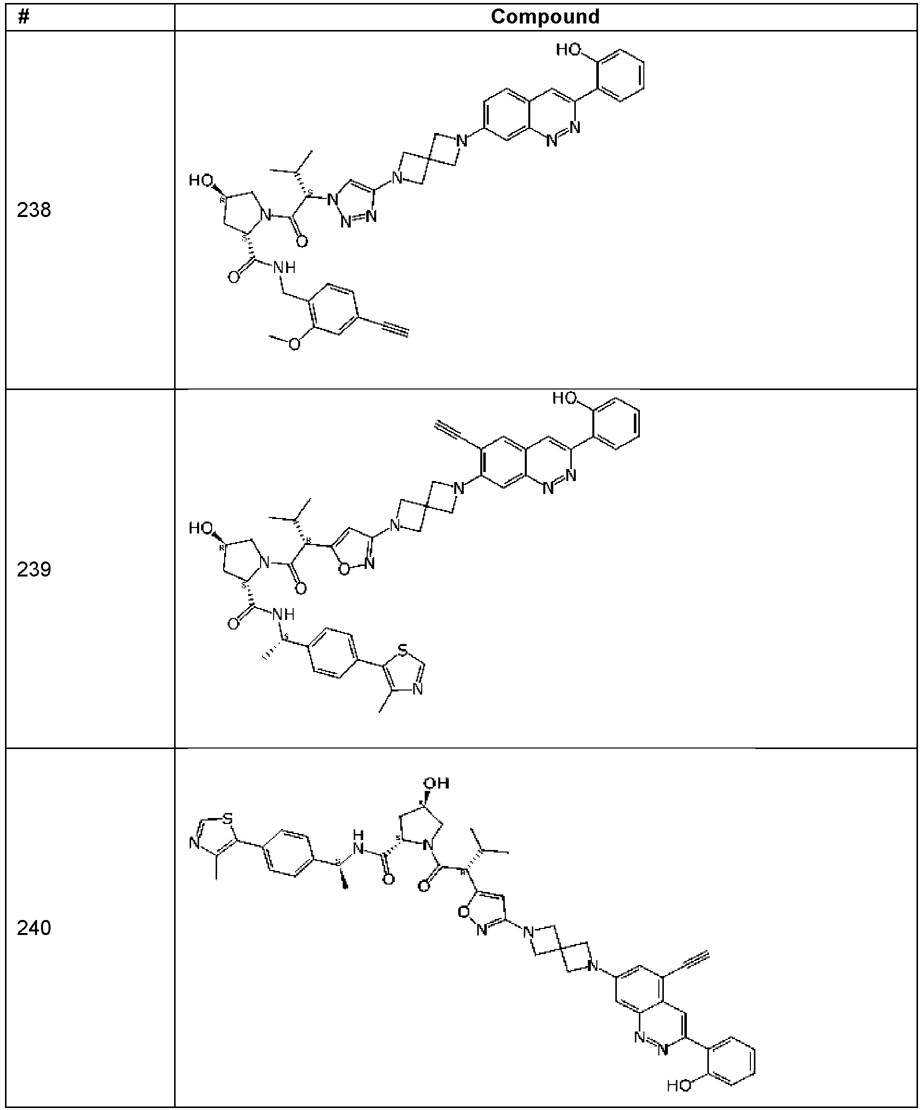

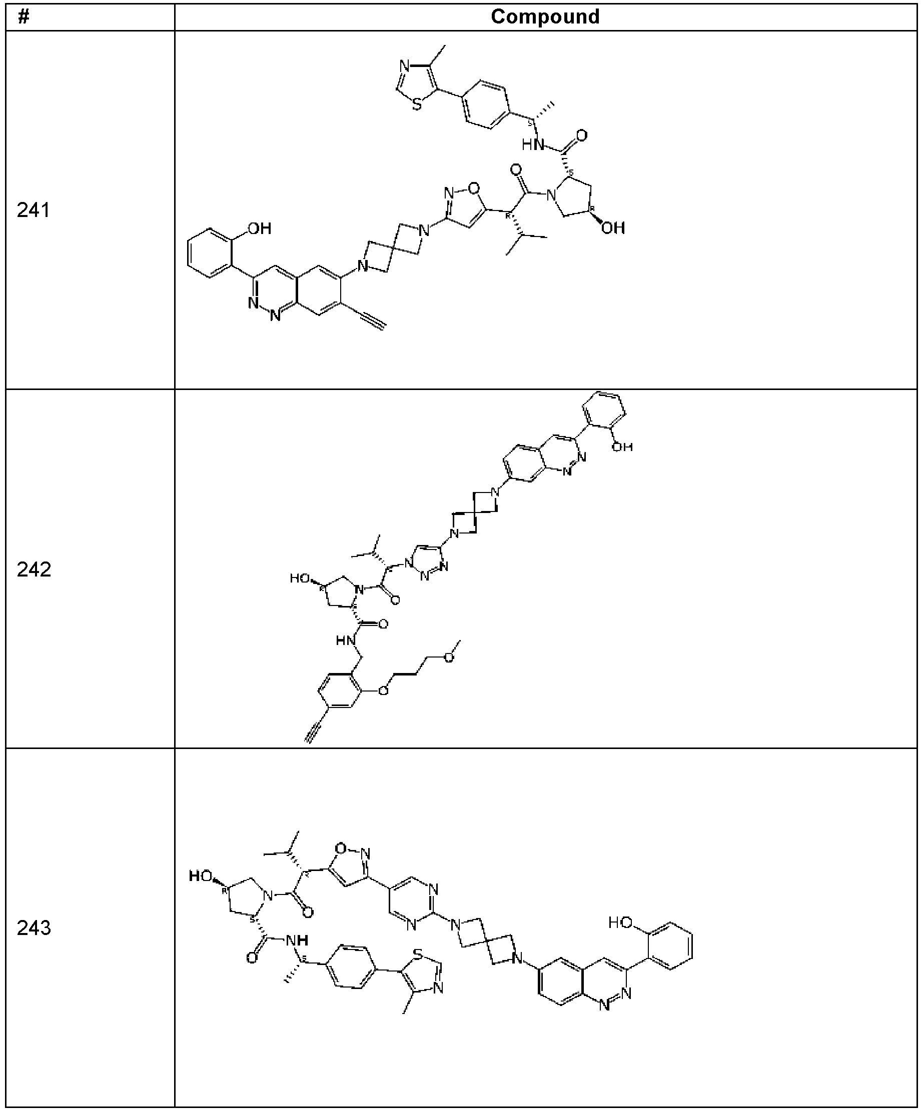

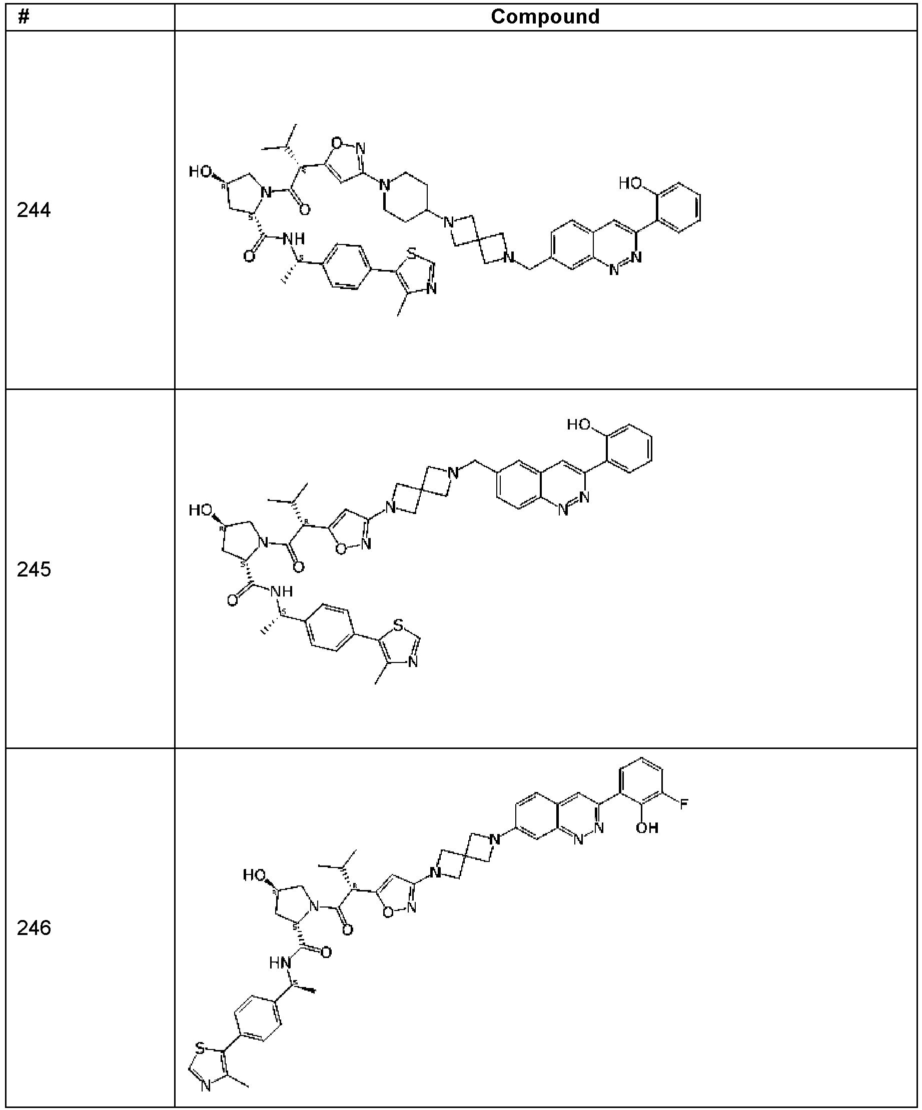

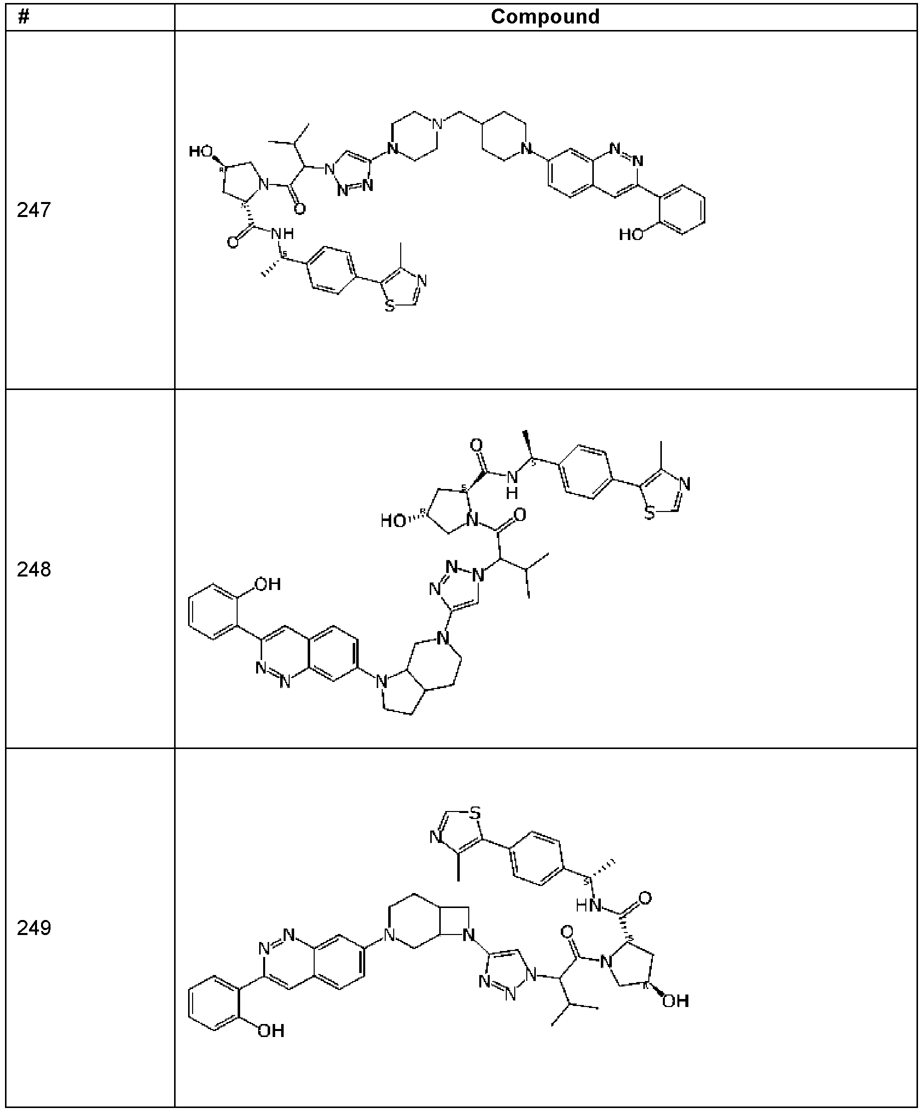

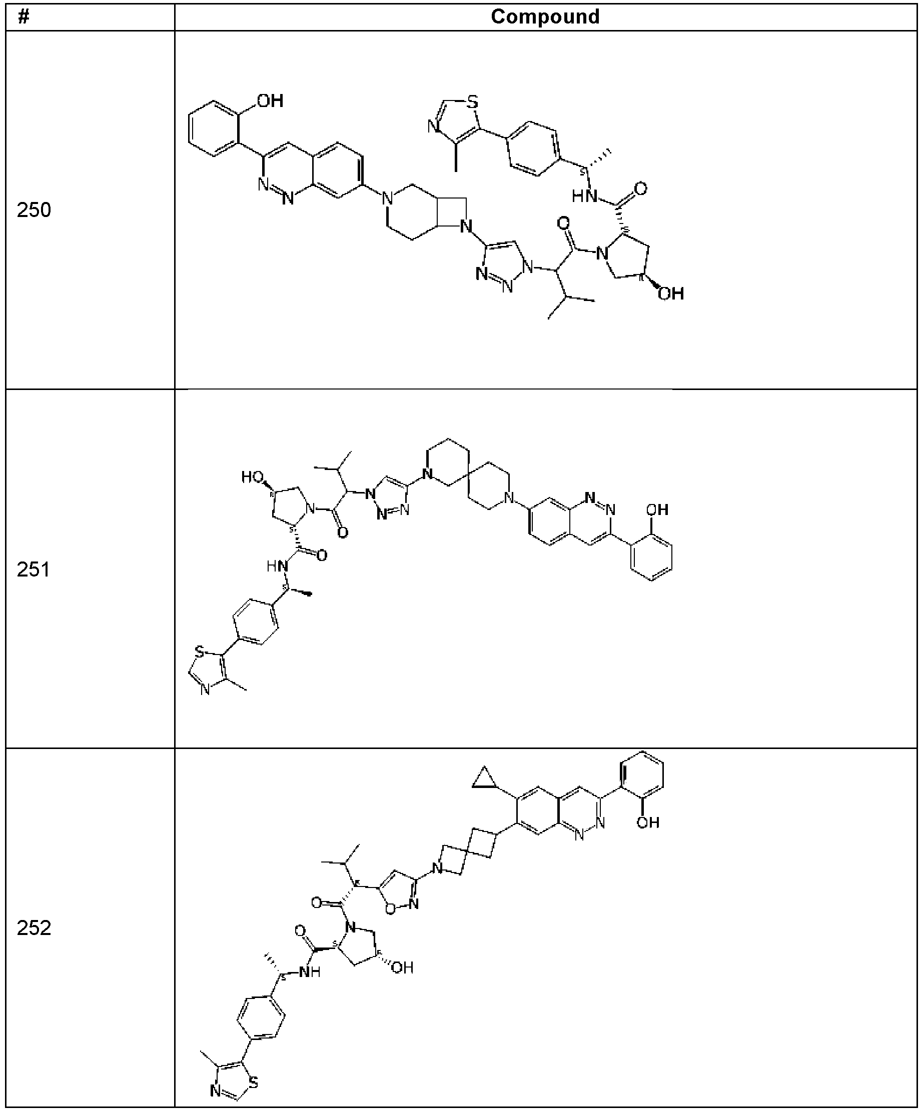

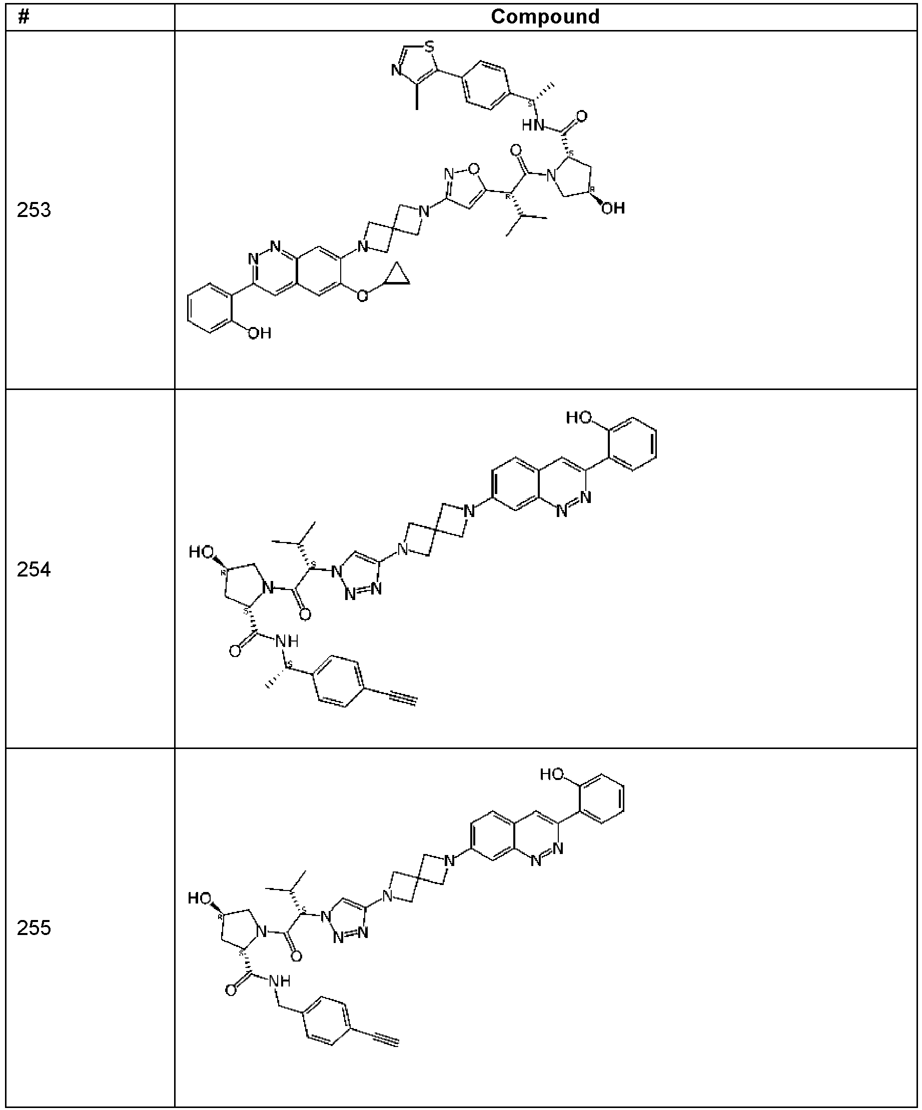

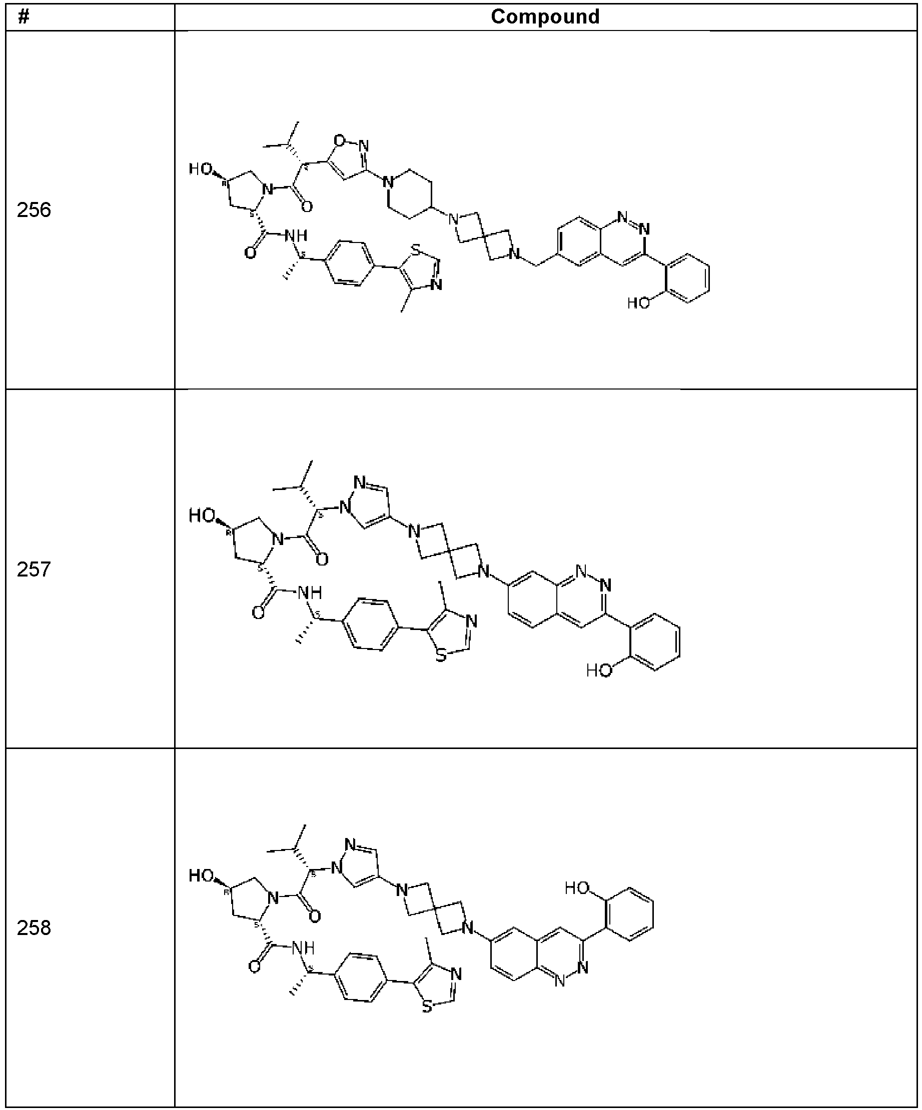

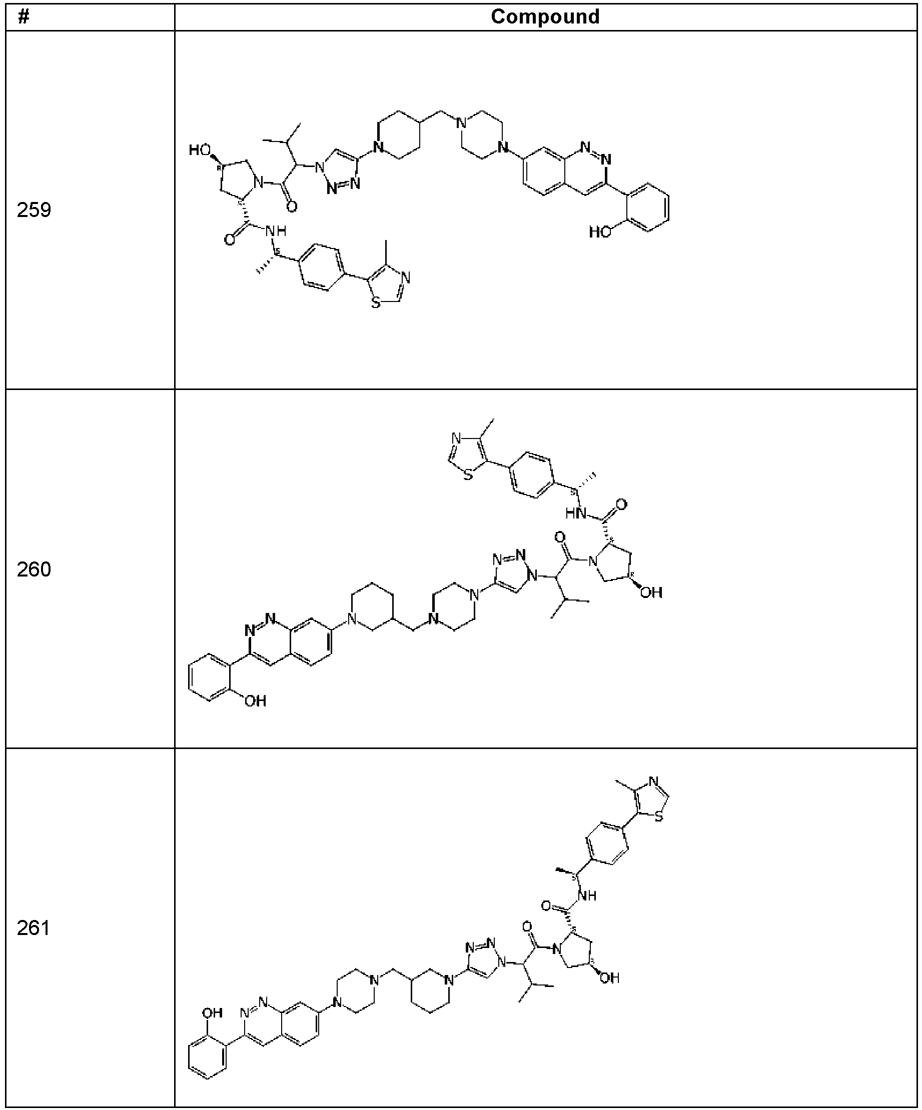

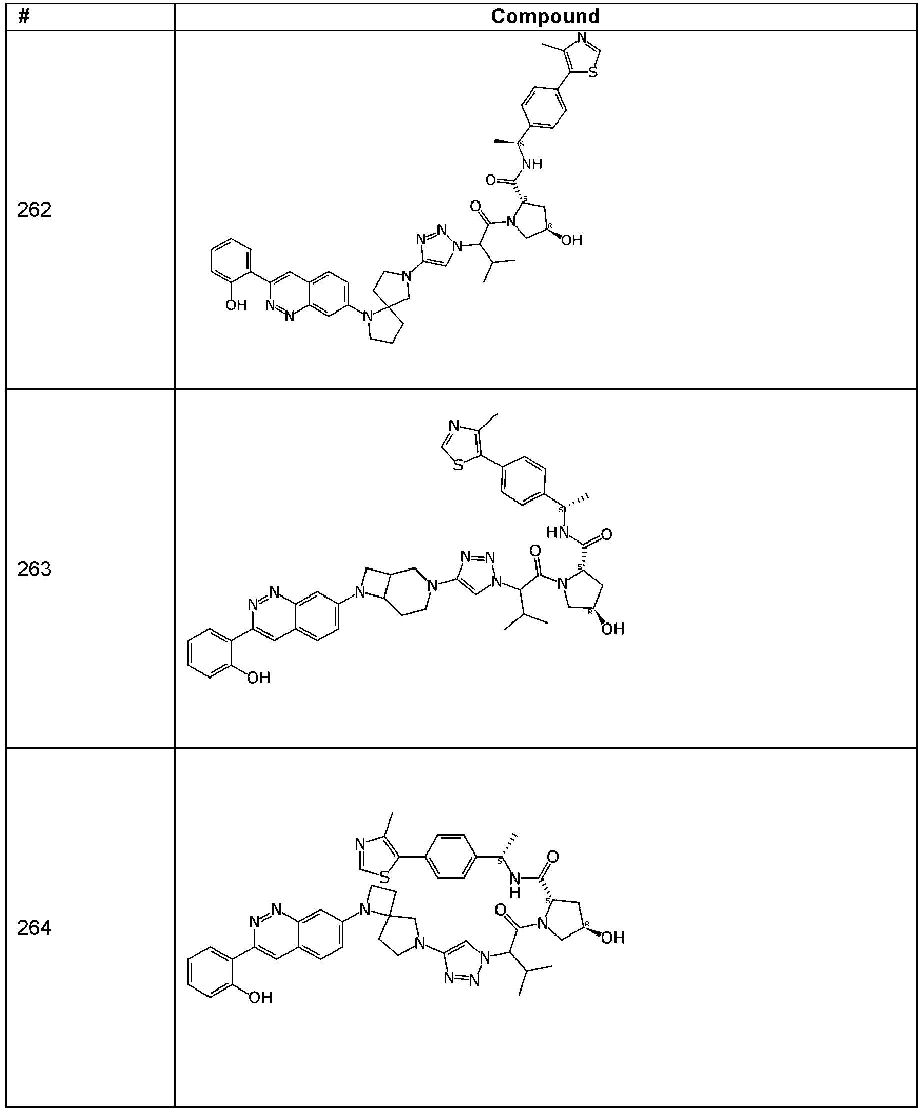

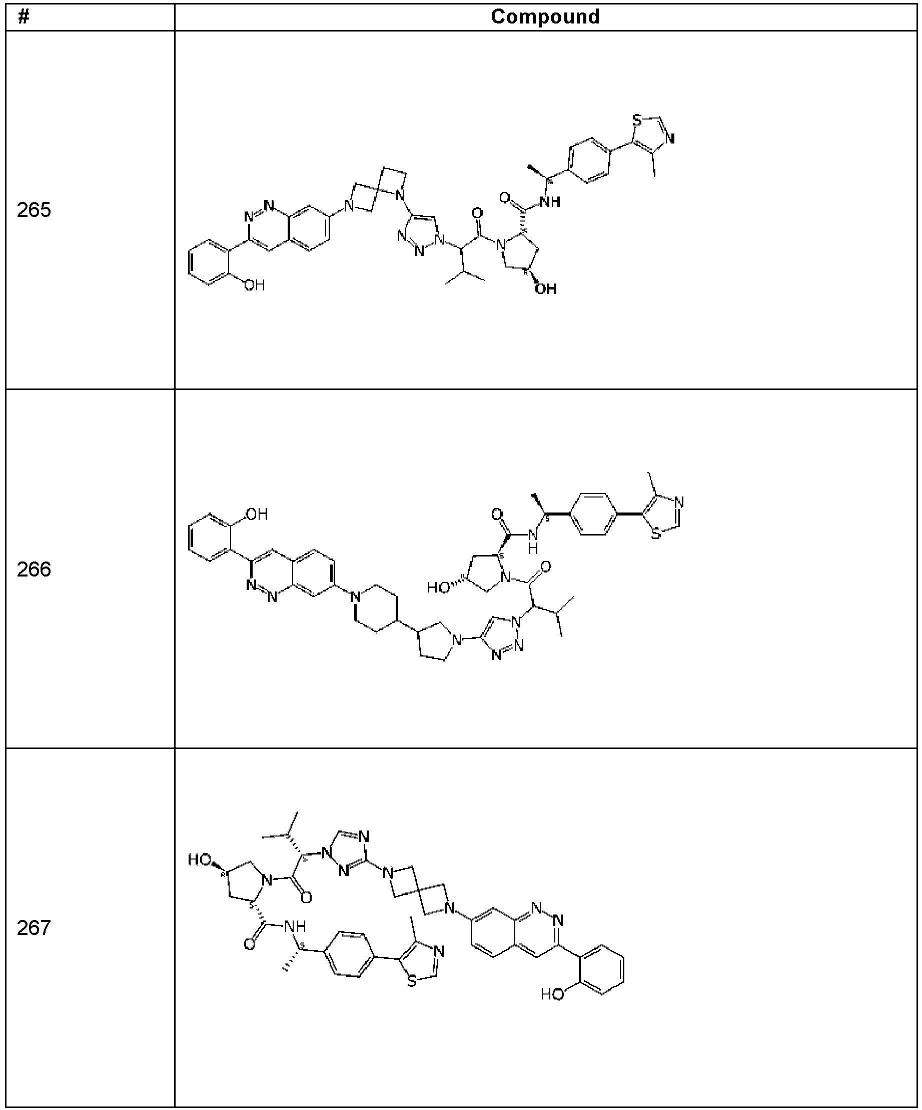

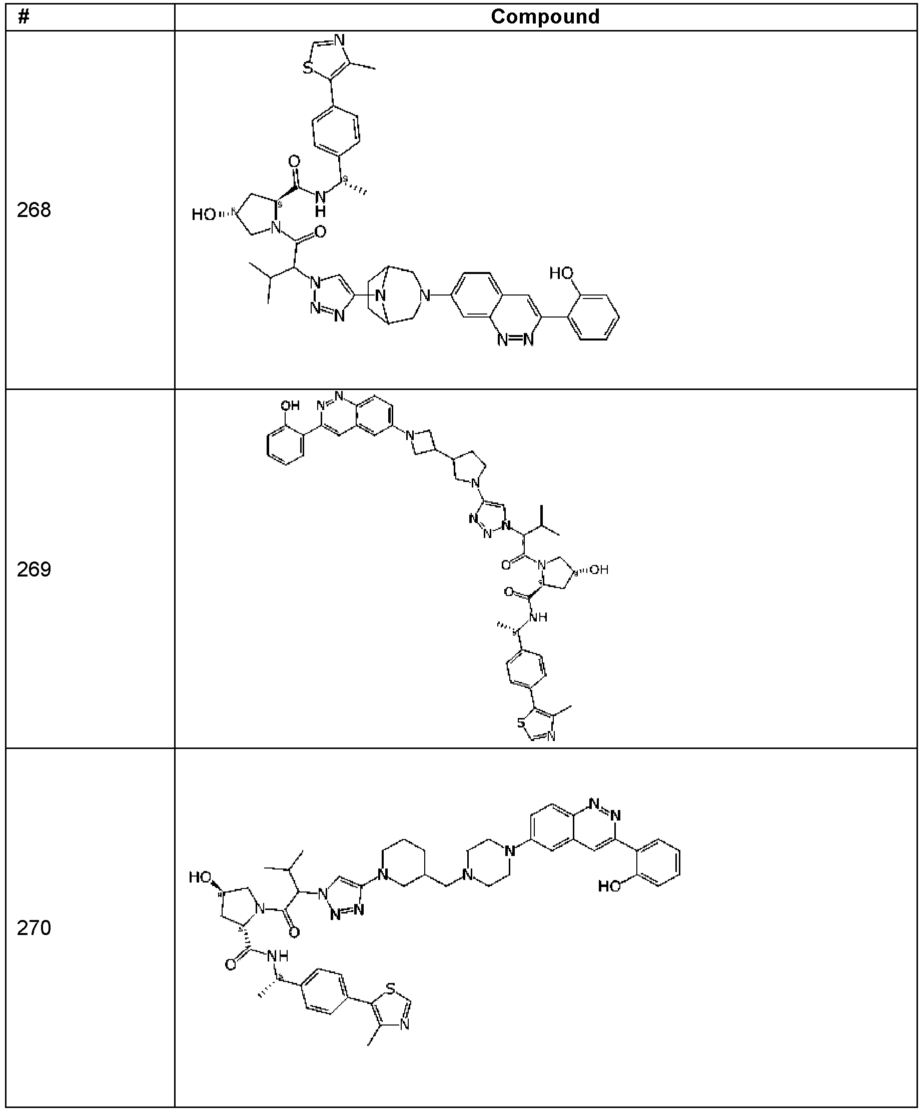

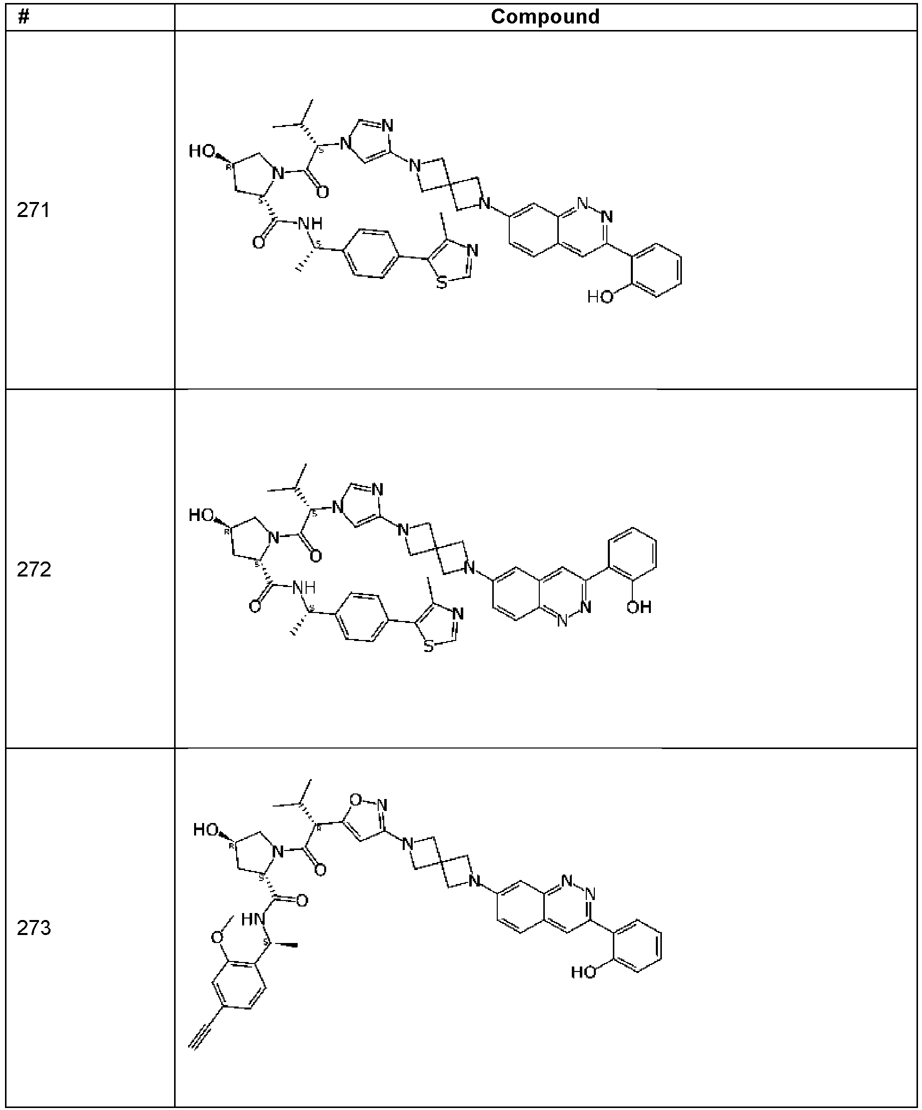

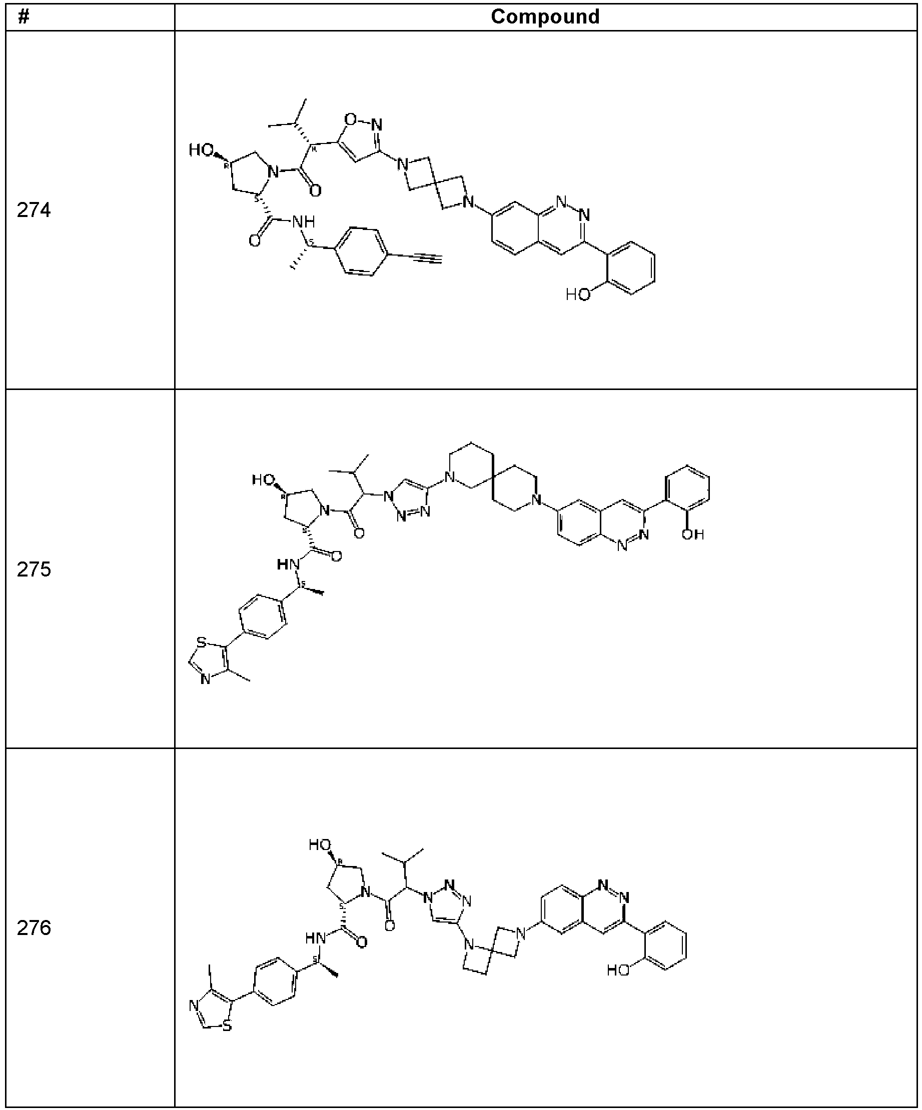

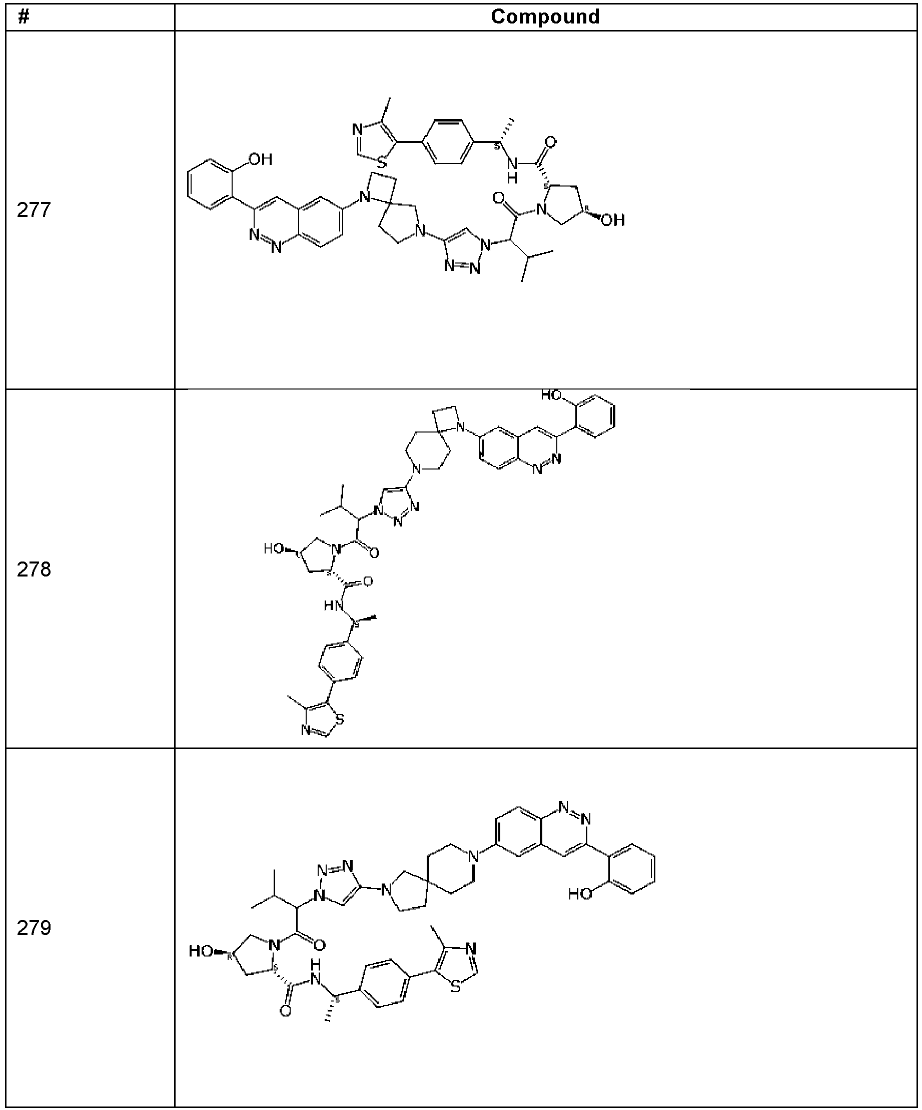

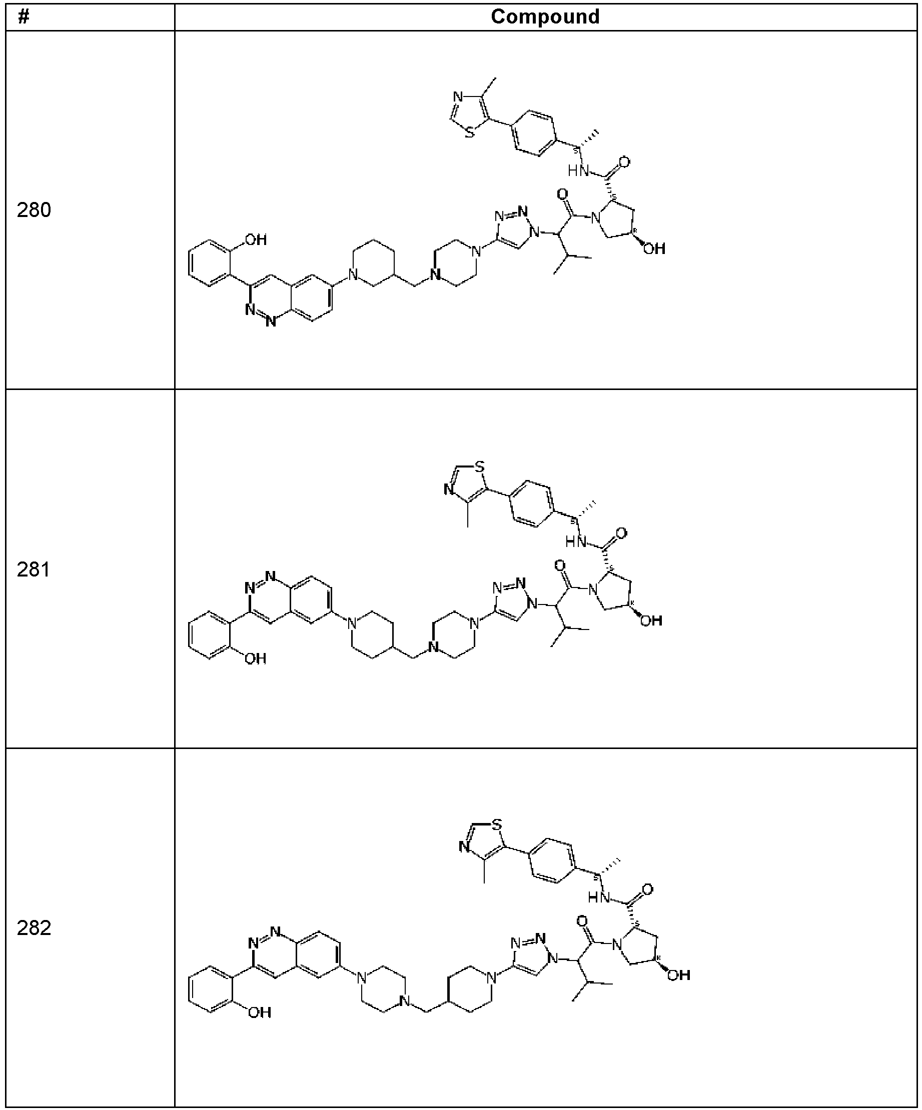

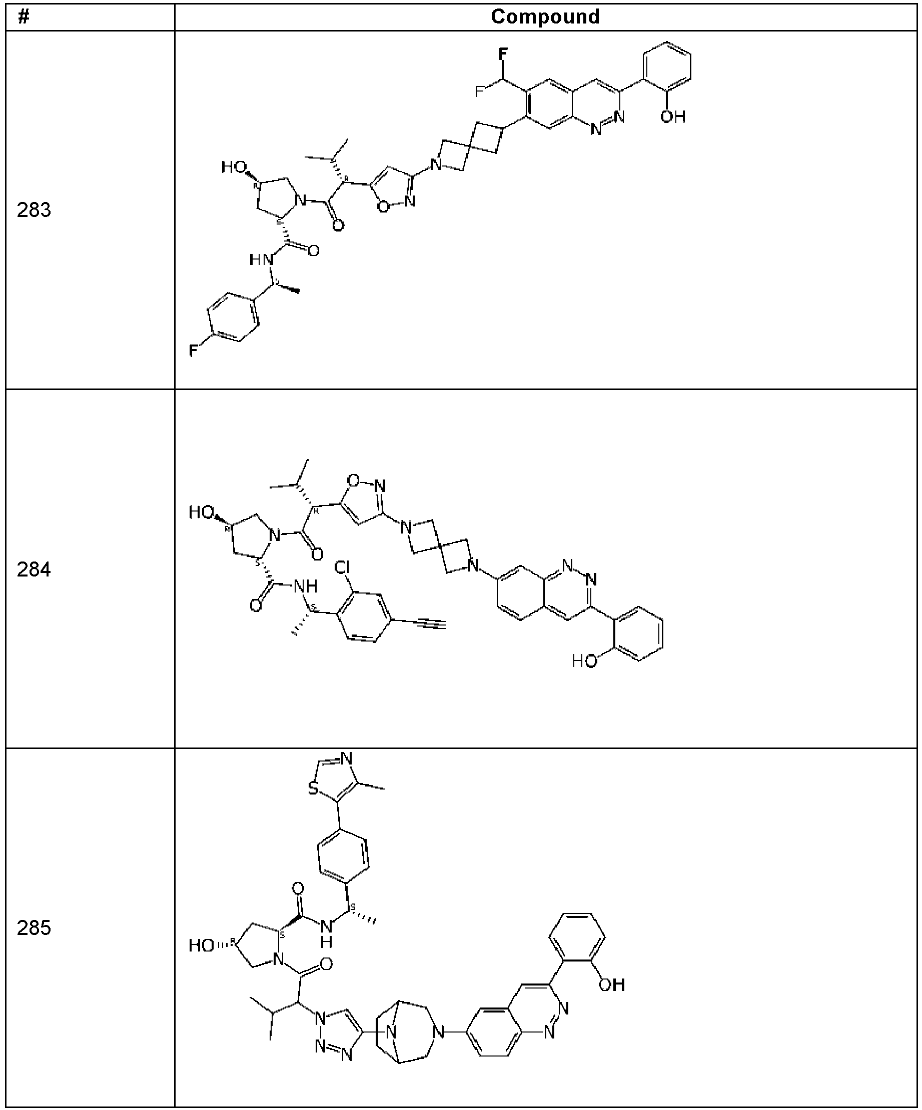

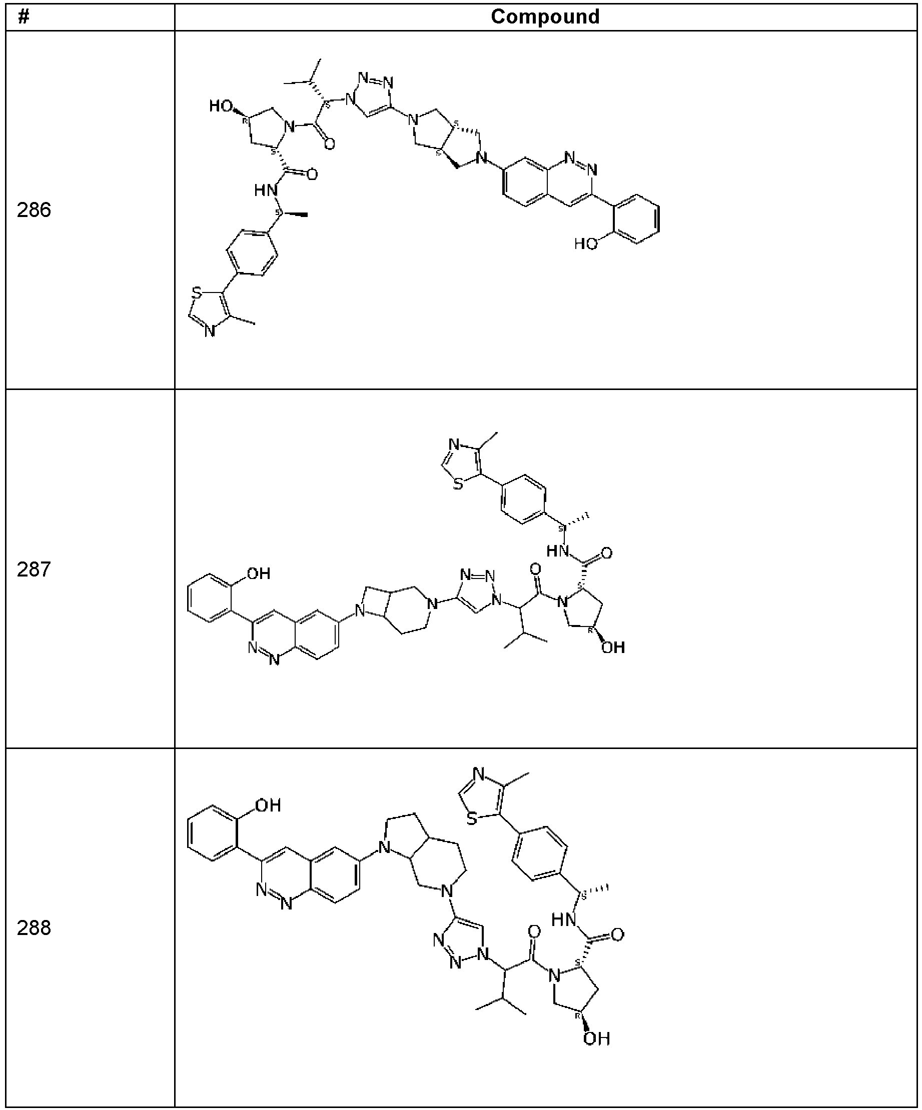

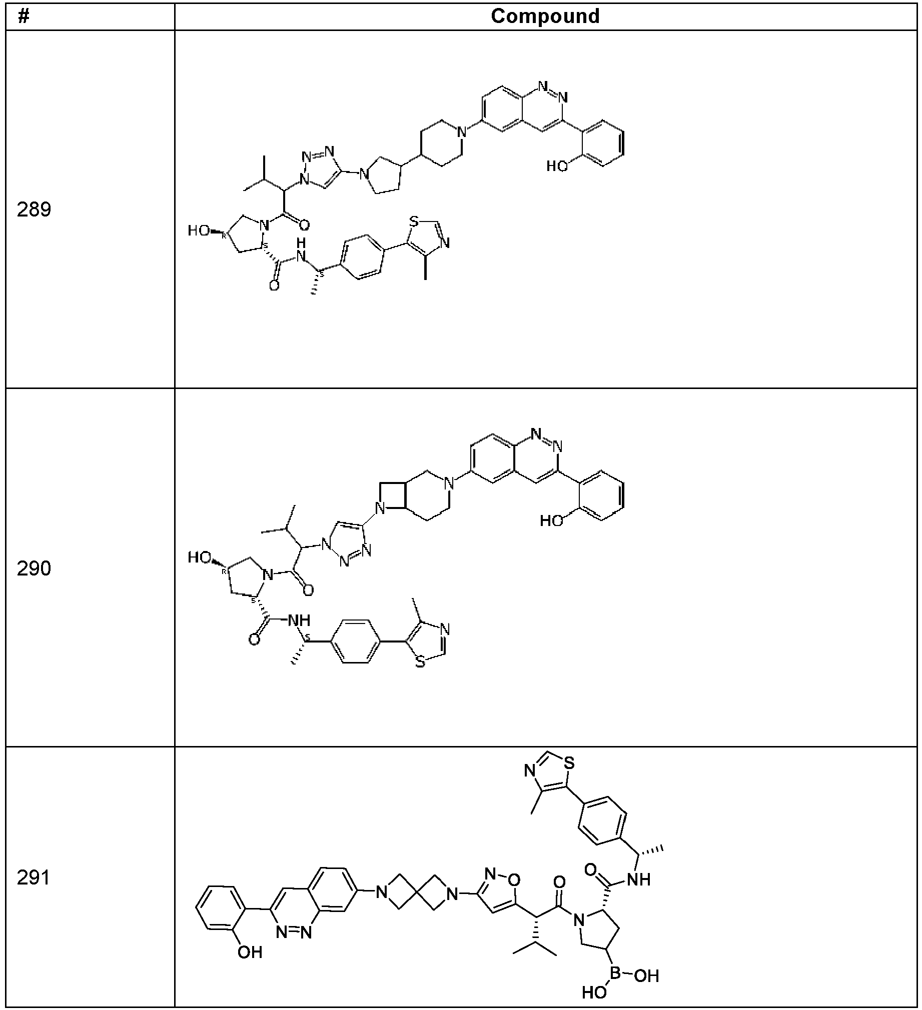

In some embodiments, the compound has a ratio of BRG1 IC₅₀ to BRM IC₅₀ of at least 5. In some embodiments, the compound has a ratio of BRG1 IC₅₀ to BRM IC₅₀ of at least 7. In some embodiments, the compound has a ratio of BRG1 IC₅₀ to BRM IC₅₀ of at least 10. In some embodiments, the compound has a ratio of BRG1 IC₅₀ to BRM IC₅₀ of at least 15. In some embodiments, the compound has a ratio of BRG1 IC₅₀ to BRM IC₅₀ of at least 20. In some embodiments, the compound has a ratio of BRG1 IC₅₀ to BRM IC₅₀ of at least 25. In some embodiments, the compound has a ratio of BRG1 IC₅₀ to BRM IC₅₀ of at least 30.

In an aspect, the invention features a pharmaceutical composition comprising any of the foregoing compounds and a pharmaceutically acceptable excipient.

For clarity, in the present disclosure all references to methods of medical treatment comprising administration of compounds or compositions are to be interpreted as being directed to the compounds or compositions for use in such methods of medical treatment.

The compounds and pharmaceutical compositions can be used in a method of decreasing the activity of a BAF complex in a cell, the method involving contacting the cell with an effective amount of any of the foregoing compounds or a pharmaceutical composition thereof.

In some instances, the cell is a cancer cell.

The compounds and pharmaceutical compositions can be used in a method of treating a BAF complex-related disorder in a subject in need thereof, the method involving administering to the subject an effective amount of any of the foregoing compounds (e.g., a BRM/BRG1 dual inhibitor compound or a BRM-selective compound) or a pharmaceutical composition thereof.

In some instances, the BAF complex-related disorder is cancer or a viral infection.

In an aspect, the present invention features any of the foregoing compounds or pharmaceutical compositions for use in therapy. In one particular such aspect, the present invention features any of the foregoing compounds or pharmaceutical compositions for use in treating cancer.

The compounds and pharmaceutical compositions can be used in a method of inhibiting BRM, the method involving contacting a cell with an effective amount of any of the foregoing compounds (e.g., a BRM/BRG1 dual inhibitor compound or a BRM-selective compound) or a pharmaceutical composition thereof.

In some instances, the cell is a cancer cell.

The compounds and pharmaceutical compositions can be used in a method of inhibiting BRG1, the method involving contacting the cell with an effective amount of any of the foregoing compounds or a pharmaceutical composition thereof.

In some instances, the cell is a cancer cell.

The compounds and pharmaceutical compositions can be used in a method of inhibiting BRM and BRG1, the method involving contacting the cell with an effective amount of any of the foregoing compounds or a pharmaceutical composition thereof.

In some instances, the cell is a cancer cell.

The compounds and pharmaceutical compositions can be used in a method of treating a disorder related to a BRG1 loss of function mutation in a subject in need thereof, the method involving administering to the subject an effective amount of any of the foregoing compounds (e.g., a BRM/BRG1 dual inhibitor compound or a BRM-selective compound) or a pharmaceutical composition thereof.

In some instances, the disorder related to a BRG1 loss of function mutation is cancer. In other instances, the subject is determined to have a BRG1 loss of function disorder, for example, is determined to have a BRG1 loss of function cancer (for example, the cancer has been determined to include cancer cells with loss of BRG1 function).

The compounds and pharmaceutical compositions can be used in a method of inducing apoptosis in a cell, the method involving contacting the cell with an effective amount of any of the foregoing compounds (e.g., a BRM/BRG1 dual inhibitor compound or a BRM-selective compound) or a pharmaceutical composition thereof.

In some instances, the cell is a cancer cell.

A method of treating cancer in a subject in need thereof can include administering to the subject an effective amount of any of the foregoing compounds (e.g., a BRM/BRG1 dual inhibitor compound or a BRM-selective compound) or a pharmaceutical composition thereof.

In some instances of any of the foregoing methods, and in some embodiments of the aspect of the invention featuring any of the foregoing compounds or a pharmaceutical composition thereof for use in treating cancer, the cancer is non-small cell lung cancer, colorectal cancer, bladder cancer, cancer of unknown primary, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, small-cell lung cancer, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer, or penile cancer.

In some instances of any of the foregoing methods, and in some embodiments of the aspect of the invention featuring any of the foregoing compounds or a pharmaceutical composition thereof for use in treating cancer, the cancer is non-small cell lung cancer, colorectal cancer, bladder cancer, cancer of unknown primary, glioma, breast cancer, melanoma, non-melanoma skin cancer, endometrial cancer, or penile cancer.

In some instances, and in some embodiments of the aspect of the invention featuring any of the foregoing compounds or a pharmaceutical composition thereof for use in treating cancer, the cancer is non-small cell lung cancer. In some instances, and in some embodiments of the aspect of the invention featuring any of the foregoing compounds or a pharmaceutical composition thereof for use in treating cancer, the cancer is soft tissue sarcoma.

In some instances of any of the foregoing methods, and in some embodiments of the aspect of the invention featuring any of the foregoing compounds or a pharmaceutical composition thereof for use in treating cancer, the cancer is a drug resistant cancer or has failed to respond to a prior therapy (e.g., vemurafenib, dacarbazine, a CTLA4 inhibitor, a PD1 inhibitor, interferon therapy, a BRAF inhibitor, a MEK inhibitor, radiotherapy, temozolomide, irinotecan, a CAR-T therapy, Herceptin^{®}, Perjeta^{®}, tamoxifen, Xeloda^{®}, docetaxol, platinum agents such as carboplatin, taxanes such as paclitaxel and docetaxel, ALK inhibitors, MET inhibitors, Alimta^{®}, Abraxane^{®}, Adriamycin^{®}, gemcitabine, Avastin^{®}, Halaven^{®}, neratinib, a PARP inhibitor, ARN810, an mTOR inhibitor, topotecan, Gemzar^{®}, a VEGFR2 inhibitor, a folate receptor antagonist, demcizumab, fosbretabulin, or a PDL1 inhibitor).

In some instances of any of the foregoing methods, and in some embodiments of the aspect of the invention featuring any of the foregoing compounds or a pharmaceutical composition thereof for use in treating cancer, the cancer has or has been determined to have BRG1 mutations. In some such instances and embodiments, the BRG1 mutations are homozygous. In some such instances and embodiments, the cancer does not have, or has been determined not to have, an epidermal growth factor receptor (EGFR) mutation. In some such instances and embodiments, the cancer does not have, or has been determined not to have, an anaplastic lymphoma kinase (ALK) driver mutation. In some such instances and embodiments, the cancer has, or has been determined to have, a KRAS mutation. In some such instances and embodiments, the BRG1 mutation is in the ATPase catalytic domain of the protein. In some such instances and embodiments, the BRG1 mutation is a deletion at the C-terminus of BRG1.

In another aspect, the disclosure provides a method treating a disorder related to BAF (e.g., cancer or viral infections) in a subject in need thereof. This method includes contacting a cell with an effective amount of any of the foregoing compounds (e.g., a BRM/BRG1 dual inhibitor compound or a BRM-selective compound), or pharmaceutically acceptable salts thereof, or any of the foregoing pharmaceutical compositions. In some instances, the disorder is a viral infection is an infection with a virus of the Retroviridae family such as the lentiviruses (e.g., Human immunodeficiency virus (HIV) and deltaretroviruses (e.g., human T cell leukemia virus I (HTLV-I), human T cell leukemia virus II (HTLV-II)), Hepadnaviridae family (e.g., hepatitis B virus (HBV)), Flaviviridae family (e.g., hepatitis C virus (HCV)), Adenoviridae family (e.g., Human Adenovirus), Herpesviridae family (e.g., Human cytomegalovirus (HCMV), Epstein-Barr virus, herpes simplex virus 1 (HSV-1), herpes simplex virus 2 (HSV-2), human herpesvirus 6 (HHV-6), Herpesvirus K*, CMV, varicella-zoster virus), Papillomaviridae family (e.g., Human Papillomavirus (HPV, HPV E1)), Parvoviridae family (e.g., Parvovirus B19), Polyomaviridae family (e.g., JC virus and BK virus), Paramyxoviridae family (e.g., Measles virus), Togaviridae family (e.g., Rubella virus). In some embodiments, the disorder is Coffin Siris, Neurofibromatosis (e.g., NF-1, NF-2, or Schwannomatosis), or Multiple Meningioma.

In another aspect, the disclosure provides a method for treating a viral infection in a subject in need thereof. This method includes administering to the subject an effective amount of any of the foregoing compounds (e.g., a BRM/BRG1 dual inhibitor compound or a BRM-selective compound), or pharmaceutically acceptable salts thereof, or any of the foregoing pharmaceutical compositions. In some instances, the viral infection is an infection with a virus of the Retroviridae family such as the lentiviruses (e.g., Human immunodeficiency virus (HIV) and deltaretroviruses (e.g., human T cell leukemia virus I (HTLV-I), human T cell leukemia virus II (HTLV-II)), Hepadnaviridae family (e.g., hepatitis B virus (HBV)), Flaviviridae family (e.g., hepatitis C virus (HCV)), Adenoviridae family (e.g., Human Adenovirus), Herpesviridae family (e.g., Human cytomegalovirus (HCMV), Epstein-Barr virus, herpes simplex virus 1 (HSV-1), herpes simplex virus 2 (HSV-2), human herpesvirus 6 (HHV-6), Herpesvirus K*, CMV, varicella-zoster virus), Papillomaviridae family (e.g., Human Papillomavirus (HPV, HPV E1)), Parvoviridae family (e.g., Parvovirus B19), Polyomaviridae family (e.g., JC virus and BK virus), Paramyxoviridae family (e.g., Measles virus), or Togaviridae family (e.g., Rubella virus).

In some instancesof any of the foregoing aspects, the compound is a BRM-selective compound. In some instances, the BRM-selective compound inhibits the level and/or activity of BRM at least 10-fold greater than the compound inhibits the level and/or activity of BRG1 and/or the compound binds to BRM at least 10-fold greater than the compound binds to BRG1. For example, in some instances, a BRM-selective compound has an IC₅₀ or IP₅₀ that is at least 10-fold lower than the IC₅₀ or IP₅₀ against BRG1. In some instancesof any of the foregoing aspects, the compound is a BRM/BRG1 dual inhibitor compound. In some instances, the BRM/BRG1 dual inhibitor compound has similar activity against both BRM and BRG1 (e.g., the activity of the compound against BRM and BRG1 with within 10-fold (e.g., less than 5-fold, less than 2-fold). In some instances, the activity of the BRM/BRG1 dual inhibitor compound is greater against BRM.

In some embodiments, the activity of the BRM/BRG1 dual inhibitor compound is greater against BRG1. For example, in some instances, a BRM/BRG1 dual inhibitor compound has an IC₅₀ or IP₅₀ against BRM that is within 10-fold of the IC₅₀ or IP₅₀ against BRG1.

The compounds and pharmaceutical compositions can be used in a method of treating melanoma, prostate cancer, breast cancer, bone cancer, renal cell carcinoma, or a hematologic cancer in a subject in need thereof, the method including administering to the subject an effective amount of any of the foregoing compounds or pharmaceutical compositions thereof.

The compounds and pharmaceutical compositions can be used in a method of reducing tumor growth of melanoma, prostate cancer, breast cancer, bone cancer, renal cell carcinoma, or a hematologic cancer in a subject in need thereof, the method including administering to the subject an effective amount of any of the foregoing compounds or pharmaceutical compositions thereof.

The compounds and pharmaceutical compositions can be used in a method of suppressing metastatic progression of melanoma, prostate cancer, breast cancer, bone cancer, renal cell carcinoma, or a hematologic cancer in a subject, the method including administering an effective amount of any of the foregoing compounds or pharmaceutical compositions thereof.

The compounds and pharmaceutical compositions can be used in a method of suppressing metastatic colonization of melanoma, prostate cancer, breast cancer, bone cancer, renal cell carcinoma, or a hematologic cancer in a subject, the method including administering an effective amount of any of the foregoing compounds or pharmaceutical compositions thereof.

The compounds and pharmaceutical compositions can be used in a method of reducing the level and/or activity of BRG1 and/or BRM in a melanoma, prostate cancer, breast cancer, bone cancer, renal cell carcinoma, or hematologic cancer cell, the method including contacting the cell with an effective amount of any of the foregoing compounds or pharmaceutical compositions thereof.

In some instancesof any of the above aspects, the melanoma, prostate cancer, breast cancer, bone cancer, renal cell carcinoma, or hematologic cell is in a subject.

In some instancesof any of the above aspects, the effective amount of the compound reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference. In some instances, the effective amount of the compound that reduces the level and/or activity of BRG1 by at least 50% (e.g., 55%, 60%, 65%, 70%, 75%, 80%, 85%, 90%, or 95%) as compared to a reference. In some instances, the effective amount of the compound that reduces the level and/or activity of BRG1 by at least 90% (e.g., 91%, 92%, 93%, 94%, 95%, 96%, 97%, 98%, or 99%).

In some instances, the effective amount of the compound reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 12 hours (e.g., 14 hours, 16 hours, 18 hours, 20 hours, 22 hours, 24 hours, 30 hours, 36 hours, 48 hours, 72 hours, or more). In some instances, the effective amount of the compound that reduces the level and/or activity of BRG1 by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 4 days (e.g., 5 days, 6 days, 7 days, 14 days, 28 days, or more).

In some instancesof any of the above aspects, the effective amount of the compound reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference. In some instances, the effective amount of the compound that reduces the level and/or activity of BRM by at least 50% (e.g., 55%, 60%, 65%, 70%, 75%, 80%, 85%, 90%, or 95%) as compared to a reference. In some instances, the effective amount of the compound that reduces the level and/or activity of BRM by at least 90% (e.g., 91%, 92%, 93%, 94%, 95%, 96%, 97%, 98%, or 99%).

In some instances, the effective amount of the compound reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 12 hours (e.g., 14 hours, 16 hours, 18 hours, 20 hours, 22 hours, 24 hours, 30 hours, 36 hours, 48 hours, 72 hours, or more). In some instances, the effective amount of the compound that reduces the level and/or activity of BRM by at least 5% (e.g., 6%, 7%, 8%, 9%, 10%, 15%, 20%, 25%, 30%, 35%, 40%, 45%, 50%, 55%, 60%, 65%, 70%, 75%, 80%, 85%, 90%, or 95%) as compared to a reference for at least 4 days (e.g., 5 days, 6 days, 7 days, 14 days, 28 days, or more).

In some instances, the subject has cancer. In some instances, the cancer expresses BRG1 and/or BRM protein and/or the cell or subject has been identified as expressing BRG1 and/or BRM. In some instances, the cancer expresses BRG1 protein and/or the cell or subject has been identified as expressing BRG1. In some instances, the cancer expresses BRM protein and/or the cell or subject has been identified as expressing BRM. In some instances, the cancer is melanoma (e.g., uveal melanoma, mucosal melanoma, or cutaneous melanoma). In some instances, the cancer is prostate cancer. In some instances, the cancer is a hematologic cancer, e.g., multiple myeloma, large cell lymphoma, acute T-cell leukemia, acute myeloid leukemia, myelodysplastic syndrome, immunoglobulin A lambda myeloma, diffuse mixed histiocytic and lymphocytic lymphoma, B-cell lymphoma, acute lymphoblastic leukemia (e.g., T-cell acute lymphoblastic leukemia or B-cell acute lymphoblastic leukemia), diffuse large cell lymphoma, or non-Hodgkin's lymphoma. In some instances, the cancer is breast cancer (e.g., an ER positive breast cancer, an ER negative breast cancer, triple positive breast cancer, or triple negative breast cancer). In some instances, the cancer is a bone cancer (e.g., Ewing's sarcoma). In some instances, the cancer is a renal cell carcinoma (e.g., a Microphthalmia Transcription Factor (MITF) family translocation renal cell carcinoma (tRCC)). In some instances, the cancer is metastatic (e.g., the cancer has spread to the liver). The metastatic cancer can include cells exhibiting migration and/or invasion of migrating cells and/or include cells exhibiting endothelial recruitment and/or angiogenesis. In other instances, the migrating cancer is a cell migration cancer. In still other instances, the cell migration cancer is a non-metastatic cell migration cancer. The metastatic cancer can be a cancer spread via seeding the surface of the peritoneal, pleural, pericardial, or subarachnoid spaces. Alternatively, the metastatic cancer can be a cancer spread via the lymphatic system, or a cancer spread hematogenously. In some instances, the effective amount of an agent that reduces the level and/or activity of BRG1 and/or BRM is an amount effective to inhibit metastatic colonization of the cancer to the liver.

In some instances the cancer harbors a mutation in GNAQ. In some instances, the cancer harbors a mutation in GNA11. In some instances, the cancer harbors a mutation in PLCB4. In some instances, the cancer harbors a mutation in CYSLTR2. In some instances the cancer harbors a mutation in BAP1. In some instances the cancer harbors a mutation in SF3B1. In some instances, the cancer harbors a mutation in EIF1AX. In some instances the cancer harbors a TFE3 translocation. In some instances the cancer harbors a TFEB translocation. In some instances, the cancer harbors a MITF translocation. In some instances, the cancer harbors an EZH2 mutation. In some instances the cancer harbors a SUZ12 mutation. In some instances, the cancer harbors an EED mutation.

In some instances, the method further includes administering to the subject or contacting the cell with an anticancer therapy, e.g., a chemotherapeutic or cytotoxic agent, immunotherapy, surgery, radiotherapy, thermotherapy, or photocoagulation. In some instances, the anticancer therapy is a chemotherapeutic or cytotoxic agent, e.g., an antimetabolite, antimitotic, antitumor antibiotic, asparagine-specific enzyme, bisphosphonates, antineoplastic, alkylating agent, DNA-Repair enzyme inhibitor, histone deacetylase inhibitor, corticosteroid, demethylating agent, immunomodulatory, janus-associated kinase inhibitor, phosphinositide 3-kinase inhibitor, proteasome inhibitor, or tyrosine kinase inhibitor.

In some instances, the compound of the invention is used in combination with another anti-cancer therapy used for the treatment of uveal melanoma such as surgery, a MEK inhibitor, and/or a PKC inhibitor. For example, in some instances, the method further comprises performing surgery prior to, subsequent to, or at the same time as administration of the compound of the invention. In some instances, the method further comprises administration of a MEK inhibitor and/or a PKC inhibitor prior to, subsequent to, or at the same time as administration of the compound of the invention.

In some instances, the anticancer therapy and the compound of the invention are administered within 28 days of each other and each in an amount that together are effective to treat the subject.

In some instances, the subject or cancer has and/or has been identified as having a BRG1 loss of function mutation.

In some instances, the cancer is resistant to one or more chemotherapeutic or cytotoxic agents (e.g., the cancer has been determined to be resistant to chemotherapeutic or cytotoxic agents such as by genetic markers, or is likely to be resistant, to chemotherapeutic or cytotoxic agents such as a cancer that has failed to respond to a chemotherapeutic or cytotoxic agent). In some instances, the cancer has failed to respond to one or more chemotherapeutic agents. In some instances, the cancer is resistant or has failed to respond to dacarbazine, temozolomide, cisplatin, treosulfan, fotemustine, IMCgp100, a CTLA-4 inhibitor (e.g., ipilimumab), a PD-1 inhibitor (e.g., Nivolumab or pembrolizumab), a PD-L1 inhibitor (e.g., atezolizumab, avelumab, or durvalumab), a mitogen-activated protein kinase (MEK) inhibitor (e.g., selumetinib, binimetinib, or tametinib), and/or a protein kinase C (PKC) inhibitor (e.g., sotrastaurin or IDE196).

In some instances, the cancer is resistant to or failed to respond to a previously administered therapeutic used for the treatment of uveal melanoma such as a MEK inhibitor or PKC inhibitor. For example, in some embodiments, the cancer is resistant to or failed to respond to a mitogen-activated protein kinase (MEK) inhibitor (e.g., selumetinib, binimetinib, or tametinib), and/or a protein kinase C (PKC) inhibitor (e.g., sotrastaurin or IDE196).

### Chemical Terms

The terminology employed herein is for the purpose of describing particular embodiments and is not intended to be limiting.

For any of the following chemical definitions, a number following an atomic symbol indicates that total number of atoms of that element that are present in a particular chemical moiety. As will be understood, other atoms, such as H atoms, or substituent groups, as described herein, may be present, as necessary, to satisfy the valences of the atoms. For example, an unsubstituted C₂ alkyl group has the formula -CH₂CH₃. When used with the groups defined herein, a reference to the number of carbon atoms includes the divalent carbon in acetal and ketal groups but does not include the carbonyl carbon in acyl, ester, carbonate, or carbamate groups. A reference to the number of oxygen, nitrogen, or sulfur atoms in a heteroaryl group only includes those atoms that form a part of a heterocyclic ring.

The term "acyl," as used herein, represents a H or an alkyl group that is attached to a parent molecular group through a carbonyl group, as defined herein, and is exemplified by formyl (i.e., a carboxaldehyde group), acetyl, trifluoroacetyl, propionyl, and butanoyl. Exemplary unsubstituted acyl groups include from 1 to 6, from 1 to 11, or from 1 to 21 carbons.

The term "alkyl," as used herein, refers to a branched or straight-chain monovalent saturated aliphatic hydrocarbon radical of 1 to 20 carbon atoms (e.g., 1 to 16 carbon atoms, 1 to 10 carbon atoms, 1 to 6 carbon atoms, or 1 to 3 carbon atoms).

An alkylene is a divalent alkyl group. The term "alkenyl," as used herein, alone or in combination with other groups, refers to a straight chain or branched hydrocarbon residue having a carbon-carbon double bond and having 2 to 20 carbon atoms (e.g., 2 to 16 carbon atoms, 2 to 10 carbon atoms, 2 to 6 carbon atoms, or 2 carbon atoms).

The term "alkynyl," as used herein, alone or in combination with other groups, refers to a straight chain or branched hydrocarbon residue having a carbon-carbon triple bond and having 2 to 20 carbon atoms (e.g., 2 to 16 carbon atoms, 2 to 10 carbon atoms, 2 to 6 carbon atoms, or 2 carbon atoms).

The term "amino," as used herein, represents -N(R^{N1})₂, wherein each R^{N1} is, independently, H, OH, NO₂, N(R^{N2})₂, SO₂OR^{N2}, SO₂R^{N2}, SOR^{N2}, an N-protecting group, alkyl, alkoxy, aryl, arylalkyl, cycloalkyl, acyl (e.g., acetyl, trifluoroacetyl, or others described herein), wherein each of these recited R^{N1} groups can be optionally substituted; or two R^{N1} combine to form an alkylene or heteroalkylene, and wherein each R^{N2} is, independently, H, alkyl, or aryl. The amino groups of the invention can be an unsubstituted amino (i.e., -NH₂) or a substituted amino (i.e., -N(R^{N1})₂).

The term "aryl," as used herein, refers to an aromatic mono- or polycarbocyclic radical of 6 to 12 carbon atoms having at least one aromatic ring. When polycyclic, the aryl group contains 2 or 3 rings. Examples of such groups include, but are not limited to, phenyl, naphthyl, 1,2,3,4-tetrahydronaphthyl, 1,2-dihydronaphthyl, indanyl, and 1H-indenyl.

The term "arylalkyl," as used herein, represents an alkyl group substituted with an aryl group. Unsubstituted arylalkyl groups contain from 7 to 30 carbons (e.g., from 7 to 16 or from 7 to 20 carbons, such as C₁-C₆ alkyl C₆-C₁₀ aryl, C₁-C₁₀ alkyl C₆-C₁₀ aryl, or C₁-C₂₀ alkyl C₆-C₁₀ aryl), such as, benzyl and phenethyl. In some embodiments, the alkyl and the aryl each are further substituted with 1, 2, 3, or 4 substituent groups, valency permitting, as defined herein for the respective groups.

The term "azido," as used herein, represents a -N₃ group.

The term "bridged polycycloalkyl," as used herein, refers to a bridged polycyclic group of 5 to 20 carbons, containing from 1 to 3 bridges. A bridged polycycloalkyl group may be unsubstituted or substituted as defined herein for cycloalkyl.

The term "cyano," as used herein, represents a -CN group.

The term "carbocyclyl," as used herein, refers to a non-aromatic C₃-C₁₂ monocyclic, bicyclic, or tricyclic structure in which the rings are formed by carbon atoms. Carbocyclyl structures include cycloalkyl groups and unsaturated carbocyclyl radicals.

The term "cycloalkyl," as used herein, refers to a saturated, non-aromatic, and monovalent mono- di-, or tricyclic radical of 3 to 10, preferably 3 to 6 carbon atoms. The cycloalkyl group may be fully saturated or contain 1 or more double or triple bonds, provided that no ring is aromatic. This term is further exemplified by radicals such as cyclopropyl, cyclobutyl, cyclopentyl, cyclohexyl, cycloheptyl, norbornyl, and adamantyl. The term "cycloalkoxy" as used herein, refers to cycloalkyl-O- groups (e.g., cyclopropoxy and cyclobutoxy).

The term "halo," as used herein, means a fluorine (fluoro), chlorine (chloro), bromine (bromo), or iodine (iodo) radical.

The term "heteroalkyl," as used herein, refers to an alkyl group, as defined herein, in which one or more of the constituent carbon atoms have been replaced by nitrogen, oxygen, or sulfur. In some embodiments, the heteroalkyl group is further substituted with 1, 2, 3, or 4 substituent groups as described herein for alkyl groups. Examples of heteroalkyl groups are an "alkoxy" which, as used herein, refers alkyl-O- (e.g., methoxy and ethoxy). A heteroalkylene is a divalent heteroalkyl group.

The term "heteroalkenyl," as used herein, refers to an alkenyl group, as defined herein, in which one or more of the constituent carbon atoms have been replaced by nitrogen, oxygen, or sulfur. In some embodiments, the heteroalkenyl group is further substituted with 1, 2, 3, or 4 substituent groups, valency permitting, as described herein for alkenyl groups. Examples of heteroalkenyl groups are an "alkenoxy" which, as used herein, refers alkenyl-O-. A heteroalkenylene is a divalent heteroalkenyl group.

The term "heteroalkynyl," as used herein, refers to an alkynyl group, as defined herein, in which one or more of the constituent carbon atoms have been replaced by nitrogen, oxygen, or sulfur. In some embodiments, the heteroalkynyl group is further substituted with 1, 2, 3, or 4 substituent groups, valency permitting, as described herein for alkynyl groups. Examples of heteroalkynyl groups are an "alkynoxy" which, as used herein, refers alkynyl-O-. A heteroalkynylene is a divalent heteroalkynyl group.

The term "heteroaryl," as used herein, refers to a monocyclic, bicyclic, or tricyclic radical of 5 to 12 atoms having at least one aromatic ring and containing 1, 2, or 3 ring atoms selected from nitrogen, oxygen, and sulfur, with the remaining ring atoms being carbon. One or two ring carbon atoms of the heteroaryl group may be replaced with a carbonyl group. Examples of heteroaryl groups are pyridyl, pyrazoyl, benzooxazolyl, benzoimidazolyl, benzothiazolyl, imidazolyl, oxazolyl, and thiazolyl.

The term "heteroarylalkyl," as used herein, represents an alkyl group substituted with a heteroaryl group. Unsubstituted heteroarylalkyl groups contain from 7 to 30 carbons (e.g., from 7 to 16 or from 7 to 20 carbons, such as C₁-C₆ alkyl C₂-C₉ heteroaryl, C₁-C₁₀ alkyl C₂-C₉ heteroaryl, or C₁-C₂₀ alkyl C₂-C₉ heteroaryl). In some embodiments, the alkyl and the heteroaryl each are further substituted with 1, 2, 3, or 4 substituent groups, valency permitting, as defined herein for the respective groups.

The term "heterocyclyl," as used herein, refers a monocyclic, bicyclic, or tricyclic radical having 3 to 12 atoms having at least one ring containing 1, 2, 3, or 4 ring atoms selected from N, O or S, wherein no ring is aromatic. Examples of heterocyclyl groups include, but are not limited to, morpholinyl, thiomorpholinyl, furyl, piperazinyl, piperidinyl, pyranyl, pyrrolidinyl, tetrahydropyranyl, tetrahydrofuranyl, and 1,3-dioxanyl.

The term "heterocyclylalkyl," as used herein, represents an alkyl group substituted with a heterocyclyl group. Unsubstituted heterocyclylalkyl groups contain from 7 to 30 carbons (e.g., from 7 to 16 or from 7 to 20 carbons, such as C₁-C₆ alkyl C₂-C₉ heterocyclyl, C₁-C₁₀ alkyl C₂-C₉ heterocyclyl, or C₁-C₂₀ alkyl C₂-C₉ heterocyclyl). In some embodiments, the alkyl and the heterocyclyl each are further substituted with 1, 2, 3, or 4 substituent groups as defined herein for the respective groups.

The term "hydroxyalkyl," as used herein, represents an alkyl group substituted with an - OH group.

The term "hydroxyl," as used herein, represents an -OH group.

The term "N-protecting group," as used herein, represents those groups intended to protect an amino group against undesirable reactions during synthetic procedures. Commonly used N-protecting groups are disclosed in Greene, "Protective Groups in Organic Synthesis," 3rd Edition (John Wiley & Sons, New York, 1999). N-protecting groups include, but are not limited to, acyl, aryloyl, or carbamyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and chiral auxiliaries such as protected or unprotected D, L, or D, L-amino acids such as alanine, leucine, and phenylalanine; sulfonyl-containing groups such as benzenesulfonyl, and p-toluenesulfonyl; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4- 20 dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, and phenylthiocarbonyl, arylalkyl groups such as benzyl, triphenylmethyl, and benzyloxymethyl, and silyl groups, such as trimethylsilyl. Preferred N-protecting groups are alloc, formyl, acetyl, benzoyl, pivaloyl, t-butylacetyl, alanyl, phenylsulfonyl, benzyl, t-butyloxycarbonyl (Boc), and benzyloxycarbonyl (Cbz).

The term "nitro," as used herein, represents an -NO₂ group.

The term "oxo," as used herein, represents a divalent oxygen atom (e.g., the structure of oxo may be shown as =O). For example, a carbonyl group is a carbon (e.g., alkyl carbon, alkenyl carbon, alkynyl carbon, heteroalkyl carbon, heteroalkenyl carbon, heteroalkynyl carbon, carbocyclyl carbon, etc.) substituted with oxo. Alternatively, sulfur may be substituted with one or two oxo groups (e.g., -SO- or -SO₂- within a substituted heteroalkyl, heteroalkenyl, heteroalkynyl, or heterocyclyl group).

The term "thiol," as used herein, represents an -SH group.

The alkyl, alkenyl, alkynyl, heteroalkyl, heteroalkenyl, heteroalkynyl, carbocyclyl (e.g., cycloalkyl), aryl, heteroaryl, and heterocyclyl groups may be substituted or unsubstituted. When substituted, there will be 1, 2, 3, 4, or 5 substituents present, valency permitting, unless otherwise specified. The 1 to 5 substituents are each, independently, selected from the group consisting of acyl, alkyl (e.g., unsubstituted and substituted, where the substituents include any group described herein, e.g., aryl, halo, hydroxy), alkenyl, alkynyl, aryl (e.g., substituted and unsubstituted phenyl), carbocyclyl (e.g., substituted and unsubstituted cycloalkyl), halo (e.g., fluoro), hydroxyl, heteroalkyl (e.g., substituted and unsubstituted methoxy, ethoxy, or thioalkoxy), heteroalkenyl, heteroalkynyl, heteroaryl, heterocyclyl, amino (e.g., NH₂ or mono- or dialkyl amino), azido, cyano, nitro, thiol, and oxo. Each of the substituents is unsubstituted or substituted with unsubstituted substituent(s) as defined herein for each respective group. In some embodiments, alkyl, alkenyl, alkynyl, heteroalkyl, heteroalkenyl, and heteroalkynyl are optionally substituted with 1, 2, 3, 4, or 5 substituents independently selected from the group consisting of aryl (e.g., substituted and unsubstituted phenyl), carbocyclyl (e.g., substituted and unsubstituted cycloalkyl), halo (e.g., fluoro), hydroxyl, heteroaryl, heterocyclyl, amino (e.g., NH₂ or mono- or dialkyl amino), azido, cyano, nitro, thiol, and oxo. Each of the substituents is unsubstituted or substituted with unsubstituted substituent(s) as defined herein for each respective group. In some embodiments, the substituents are themselves unsubstituted.

Compounds of the invention can have one or more asymmetric carbon atoms and can exist in the form of optically pure enantiomers, mixtures of enantiomers such as, for example, racemates, optically pure diastereoisomers, mixtures of diastereoisomers, diastereoisomeric racemates, or mixtures of diastereoisomeric racemates. The optically active forms can be obtained for example by resolution of the racemates, by asymmetric synthesis or asymmetric chromatography (chromatography with a chiral adsorbents or eluant). That is, certain of the disclosed compounds may exist in various stereoisomeric forms. Stereoisomers are compounds that differ only in their spatial arrangement. Enantiomers are pairs of stereoisomers whose mirror images are not superimposable, most commonly because they contain an asymmetrically substituted carbon atom that acts as a chiral center. "Enantiomer" means one of a pair of molecules that are mirror images of each other and are not superimposable. Diastereomers are stereoisomers that are not related as mirror images, most commonly because they contain two or more asymmetrically substituted carbon atoms and represent the configuration of substituents around one or more chiral carbon atoms. Enantiomers of a compound can be prepared, for example, by separating an enantiomer from a racemate using one or more well-known techniques and methods, such as, for example, chiral chromatography and separation methods based thereon. The appropriate technique and/or method for separating an enantiomer of a compound described herein from a racemic mixture can be readily determined by those of skill in the art. "Racemate" or "racemic mixture" means a compound containing two enantiomers, wherein such mixtures exhibit no optical activity; i.e., they do not rotate the plane of polarized light. "Geometric isomer" means isomers that differ in the orientation of substituent atoms in relationship to a carbon-carbon double bond, to a cycloalkyl ring, or to a bridged bicyclic system. Atoms (other than H) on each side of a carbon- carbon double bond may be in an E (substituents are on opposite sides of the carbon- carbon double bond) or Z (substituents are oriented on the same side) configuration. "R," "S," "S*," "R*," "E," "Z," "cis," and "trans," indicate configurations relative to the core molecule. Certain of the disclosed compounds may exist in atropisomeric forms. Atropisomers are stereoisomers resulting from hindered rotation about single bonds where the steric strain barrier to rotation is high enough to allow for the isolation of the conformers. The compounds of the invention may be prepared as individual isomers by either isomer-specific synthesis or resolved from an isomeric mixture. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods. When the stereochemistry of a disclosed compound is named or depicted by structure, the named or depicted stereoisomer is at least 60%, 70%, 80%, 90%, 99%, or 99.9% by weight relative to the other stereoisomers. When a single enantiomer is named or depicted by structure, the depicted or named enantiomer is at least 60%, 70%, 80%, 90%, 99%, or 99.9% by weight optically pure. When a single diastereomer is named or depicted by structure, the depicted or named diastereomer is at least 60%, 70%, 80%, 90%, 99%, or 99.9% by weight pure. Percent optical purity is the ratio of the weight of the enantiomer or over the weight of the enantiomer plus the weight of its optical isomer. Diastereomeric purity by weight is the ratio of the weight of one diastereomer or over the weight of all the diastereomers. When the stereochemistry of a disclosed compound is named or depicted by structure, the named or depicted stereoisomer is at least 60%, 70%, 80%, 90%, 99%, or 99.9% by mole fraction pure relative to the other stereoisomers. When a single enantiomer is named or depicted by structure, the depicted or named enantiomer is at least 60%, 70%, 80%, 90%, 99%, or 99.9% by mole fraction pure. When a single diastereomer is named or depicted by structure, the depicted or named diastereomer is at least 60%, 70%, 80%, 90%, 99%, or 99.9% by mole fraction pure. Percent purity by mole fraction is the ratio of the moles of the enantiomer or over the moles of the enantiomer plus the moles of its optical isomer. Similarly, percent purity by moles fraction is the ratio of the moles of the diastereomer or over the moles of the diastereomer plus the moles of its isomer. When a disclosed compound is named or depicted by structure without indicating the stereochemistry, and the compound has at least one chiral center, it is to be understood that the name or structure encompasses either enantiomer of the compound free from the corresponding optical isomer, a racemic mixture of the compound, or mixtures enriched in one enantiomer relative to its corresponding optical isomer. When a disclosed compound is named or depicted by structure without indicating the stereochemistry and has two or more chiral centers, it is to be understood that the name or structure encompasses a diastereomer free of other diastereomers, a number of diastereomers free from other diastereomeric pairs, mixtures of diastereomers, mixtures of diastereomeric pairs, mixtures of diastereomers in which one diastereomer is enriched relative to the other diastereomer(s), or mixtures of diastereomers in which one or more diastereomer is enriched relative to the other diastereomers. The invention embraces all of these forms.

Compounds of the present disclosure also include all of the isotopes of the atoms occurring in the intermediate or final compounds. "Isotopes" refers to atoms having the same atomic number but different mass numbers resulting from a different number of neutrons in the nuclei. For example, isotopes of hydrogen include tritium and deuterium.

Unless otherwise stated, structures depicted herein are also meant to include compounds that differ only in the presence of one or more isotopically enriched atoms. Exemplary isotopes that can be incorporated into compounds of the present invention include isotopes of hydrogen, carbon, nitrogen, oxygen, phosphorus, sulfur, fluorine, chlorine, and iodine, such as ²H, ³H, ¹¹C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. Isotopically-labeled compounds (e.g., those labeled with ³H and ¹⁴C) can be useful in compound or substrate tissue distribution assays. Tritiated (i.e., ³H) and carbon-14 (i.e., ¹⁴C) isotopes can be useful for their ease of preparation and detectability. Further, substitution with heavier isotopes such as deuterium (i.e., ²H) may afford certain therapeutic advantages resulting from greater metabolic stability (e.g., increased in vivo half-life or reduced dosage requirements). In some embodiments, one or more hydrogen atoms are replaced by ²H or ³H, or one or more carbon atoms are replaced by ¹³C- or ¹⁴C-enriched carbon. Positron emitting isotopes such as ¹⁵O, ¹³N, ¹¹C, and ¹⁸F are useful for positron emission tomography (PET) studies to examine substrate receptor occupancy. Preparations of isotopically labelled compounds are known to those of skill in the art. For example, isotopically labeled compounds can generally be prepared by following procedures analogous to those disclosed for compounds of the present invention described herein, by substituting an isotopically labeled reagent for a non-isotopically labeled reagent. Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used. The materials, methods, and examples are illustrative only and not intended to be limiting. In case of conflict, the present specification, including definitions, will control.

### Definitions

In this application, unless otherwise clear from context, the term "a" may be understood to mean "at least one", and the terms "comprising" and "including" may be understood to encompass itemized components or steps whether presented by themselves or together with one or more additional components or steps.

As used herein, the terms "about" and "approximately" refer to a value that is within 10% above or below the value being described. For example, the term "about 5 nM" indicates a range of from 4.5 to 5.5 nM.

As used herein, the term "administration" refers to the administration of a composition (e.g., a compound or a preparation that includes a compound as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intratumoral, intravenous, intraventricular, mucosal, nasal, oral, rectal, subcutaneous, sublingual, topical, tracheal (including by intratracheal instillation), transdermal, vaginal, and vitreal.

As used herein, the term "BAF complex" refers to the BRG1- or HRBM-associated factors complex in a human cell.

As used herein, the term "BAF complex-related disorder" refers to a disorder that is caused or affected by the level of activity of a BAF complex.

As used herein, the term "BRG1 loss of function mutation" refers to a mutation in BRG1 that leads to the protein having diminished activity (e.g., at least 1% reduction in BRG1 activity, for example 2%, 5%, 10%, 25%, 50%, or 100% reduction in BRG1 activity). Exemplary BRG1 loss of function mutations include, but are not limited to, a homozygous BRG1 mutation and a deletion at the C-terminus of BRG1.

As used herein, the term "BRG1 loss of function disorder" refers to a disorder (e.g., cancer) that exhibits a reduction in BRG1 activity (e.g., at least 1% reduction in BRG1 activity, for example 2%, 5%, 10%, 25%, 50%, or 100% reduction in BRG1 activity).

The term "cancer" refers to a condition caused by the proliferation of malignant neoplastic cells, such as tumors, neoplasms, carcinomas, sarcomas, leukemias, and lymphomas.

As used herein, a "combination therapy" or "administered in combination" means that two (or more) different agents or treatments are administered to a subject as part of a defined treatment regimen for a particular disease or condition. The treatment regimen defines the doses and periodicity of administration of each agent such that the effects of the separate agents on the subject overlap. In some embodiments, the delivery of the two or more agents is simultaneous or concurrent and the agents may be co-formulated. In some embodiments, the two or more agents are not co-formulated and are administered in a sequential manner as part of a prescribed regimen. In some embodiments, administration of two or more agents or treatments in combination is such that the reduction in a symptom, or other parameter related to the disorder is greater than what would be observed with one agent or treatment delivered alone or in the absence of the other. The effect of the two treatments can be partially additive, wholly additive, or greater than additive (e.g., synergistic). Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination may be administered by intravenous injection while a second therapeutic agent of the combination may be administered orally.

By "determining the level" of a protein or RNA is meant the detection of a protein or an RNA, by methods known in the art, either directly or indirectly. "Directly determining" means performing a process (e.g., performing an assay or test on a sample or "analyzing a sample" as that term is defined herein) to obtain the physical entity or value. "Indirectly determining" refers to receiving the physical entity or value from another party or source (e.g., a third-party laboratory that directly acquired the physical entity or value). Methods to measure protein level generally include, but are not limited to, western blotting, immunoblotting, enzyme-linked immunosorbent assay (ELISA), radioimmunoassay (RIA), immunoprecipitation, immunofluorescence, surface plasmon resonance, chemiluminescence, fluorescent polarization, phosphorescence, immunohistochemical analysis, matrix-assisted laser desorption/ionization time-of-flight (MALDI-TOF) mass spectrometry, liquid chromatography (LC)-mass spectrometry, microcytometry, microscopy, fluorescence activated cell sorting (FACS), and flow cytometry, as well as assays based on a property of a protein including, but not limited to, enzymatic activity or interaction with other protein partners. Methods to measure RNA levels are known in the art and include, but are not limited to, quantitative polymerase chain reaction (qPCR) and Northern blot analyses.

By "decreasing the activity of a BAF complex" is meant decreasing the level of an activity related to a BAF complex, or a related downstream effect. A non-limiting example of decreasing an activity of a BAF complex is Sox2 activation. The activity level of a BAF complex may be measured using any method known in the art, e.g., the methods described in Kadoch et al. Cell, 2013, 153, 71-85.

As used herein, the term "degrader" refers to a small molecule compound including a degradation moiety, wherein the compound interacts with a protein (e.g., BRG1 and/or BRM) in a way which results in degradation of the protein, e.g., binding of the compound results in at least 5% reduction of the level of the protein, e.g., in a cell or subject.

As used herein, the term "degradation moiety" refers to a moiety whose binding results in degradation of a protein, e.g., BRG1 and/or BRM. In one example, the moiety binds to a protease or a ubiquitin ligase that metabolizes the protein, e.g., BRG1 and/or BRM.

By "modulating the activity of a BAF complex," is meant altering the level of an activity related to a BAF complex (e.g., GBAF), or a related downstream effect. The activity level of a BAF complex may be measured using any method known in the art, e.g., the methods described in Kadoch et al, Cell 153:71-85 (2013).

By "reducing the activity of BRG1 and/or BRM," is meant decreasing the level of an activity related to an BRG1 and/or BRM, or a related downstream effect. A non-limiting example of inhibition of an activity of BRG1 and/or BRM is decreasing the level of a BAF complex in a cell. The activity level of BRG1 and/or BRM may be measured using any method known in the art. In some embodiments, an agent which reduces the activity of BRG1 and/or BRM is a small molecule BRG1 and/or BRM degrader.

By "reducing the level of BRG1 and/or BRM," is meant decreasing the level of BRG1 and/or BRM in a cell or subject. The level of BRG1 and/or BRM may be measured using any method known in the art.

By "level" is meant a level of a protein, or mRNA encoding the protein, as compared to a reference. The reference can be any useful reference, as defined herein. By a "decreased level" or an "increased level" of a protein is meant a decrease or increase in protein level, as compared to a reference (e.g., a decrease or an increase by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 300%, about 400%, about 500%, or more; a decrease or an increase of more than about 10%, about 15%, about 20%, about 50%, about 75%, about 100%, or about 200%, as compared to a reference; a decrease or an increase by less than about 0.01-fold, about 0.02-fold, about 0.1-fold, about 0.3-fold, about 0.5-fold, about 0.8-fold, or less; or an increase by more than about 1.2-fold, about 1.4-fold, about 1.5-fold, about 1.8-fold, about 2.0-fold, about 3.0-fold, about 3.5-fold, about 4.5-fold, about 5.0-fold, about 10-fold, about 15-fold, about 20-fold, about 30-fold, about 40-fold, about 50-fold, about 100-fold, about 1000-fold, or more). A level of a protein may be expressed in mass/vol (e.g., g/dL, mg/mL, µg/mL, ng/mL) or percentage relative to total protein or mRNA in a sample.

As used herein, the term "inhibiting BRM" refers to blocking or reducing the level or activity of the ATPase catalytic binding domain or the bromodomain of the protein. BRM inhibition may be determined using methods known in the art, e.g., a BRM ATPase assay, a Nano DSF assay, or a BRM Luciferase cell assay.

The term "pharmaceutical composition," as used herein, represents a composition containing a compound described herein formulated with a pharmaceutically acceptable excipient and appropriate for administration to a mammal, for example a human. Typically, a pharmaceutical composition is manufactured or sold with the approval of a governmental regulatory agency as part of a therapeutic regimen for the treatment of disease in a mammal. Pharmaceutical compositions can be formulated, for example, for oral administration in unit dosage form (e.g., a tablet, capsule, caplet, gel cap, or syrup); for topical administration (e.g., as a cream, gel, lotion, or ointment); for intravenous administration (e.g., as a sterile solution free of particulate emboli and in a solvent system suitable for intravenous use); or in any other pharmaceutically acceptable formulation.

A "pharmaceutically acceptable excipient," as used herein, refers to any ingredient other than the compounds described herein (for example, a vehicle capable of suspending or dissolving the active compound) and having the properties of being substantially nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration.

As used herein, the term "pharmaceutically acceptable salt" means any pharmaceutically acceptable salt of a compound, for example, any compound of **Formula I.** Pharmaceutically acceptable salts of any of the compounds described herein may include those that are within the scope of sound medical judgment, suitable for use in contact with the tissues of humans and animals without undue toxicity, irritation, allergic response and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting a free base group with a suitable organic acid.

The compounds of the invention may have ionizable groups so as to be capable of preparation as pharmaceutically acceptable salts. These salts may be acid addition salts involving inorganic or organic acids or the salts may, in the case of acidic forms of the compounds of the invention be prepared from inorganic or organic bases. Frequently, the compounds are prepared or used as pharmaceutically acceptable salts prepared as addition products of pharmaceutically acceptable acids or bases. Suitable pharmaceutically acceptable acids and bases and methods for preparation of the appropriate salts are well-known in the art. Salts may be prepared from pharmaceutically acceptable non-toxic acids and bases including inorganic and organic acids and bases.

By a "reference" is meant any useful reference used to compare protein or RNA levels. The reference can be any sample, standard, standard curve, or level that is used for comparison purposes. The reference can be a normal reference sample or a reference standard or level. A "reference sample" can be, for example, a control, e.g., a predetermined negative control value such as a "normal control" or a prior sample taken from the same subject; a sample from a normal healthy subject, such as a normal cell or normal tissue; a sample (e.g., a cell or tissue) from a subject not having a disease; a sample from a subject that is diagnosed with a disease, but not yet treated with a compound of the invention; a sample from a subject that has been treated by a compound of the invention; or a sample of a purified protein or RNA (e.g., any described herein) at a known normal concentration. By "reference standard or level" is meant a value or number derived from a reference sample. A "normal control value" is a pre-determined value indicative of non-disease state, e.g., a value expected in a healthy control subject. Typically, a normal control value is expressed as a range ("between X and Y"), a high threshold ("no higher than X"), or a low threshold ("no lower than X"). A subject having a measured value within the normal control value for a particular biomarker is typically referred to as "within normal limits" for that biomarker. A normal reference standard or level can be a value or number derived from a normal subject not having a disease or disorder (e.g., cancer); a subject that has been treated with a compound of the invention. In preferred embodiments, the reference sample, standard, or level is matched to the sample subject sample by at least one of the following criteria: age, weight, sex, disease stage, and overall health. A standard curve of levels of a purified protein or RNA, e.g., any described herein, within the normal reference range can also be used as a reference.

As used herein, the term "subject" refers to any organism to which a composition in accordance with the invention may be administered, e.g., for experimental, diagnostic, prophylactic, and/or therapeutic purposes. Typical subjects include any animal (e.g., mammals such as mice, rats, rabbits, non-human primates, and humans). A subject may seek or be in need of treatment, require treatment, be receiving treatment, be receiving treatment in the future, or be a human or animal who is under care by a trained professional for a particular disease or condition.

As used herein, the terms "treat," "treated," or "treating" mean therapeutic treatment or any measures whose object is to slow down (lessen) an undesired physiological condition, disorder, or disease, or obtain beneficial or desired clinical results. Beneficial or desired clinical results include, but are not limited to, alleviation of symptoms; diminishment of the extent of a condition, disorder, or disease; stabilized (i.e., not worsening) state of condition, disorder, or disease; delay in onset or slowing of condition, disorder, or disease progression; amelioration of the condition, disorder, or disease state or remission (whether partial or total); an amelioration of at least one measurable physical parameter, not necessarily discernible by the patient; or enhancement or improvement of condition, disorder, or disease. Treatment includes eliciting a clinically significant response without excessive levels of side effects. Treatment also includes prolonging survival as compared to expected survival if not receiving treatment. Compounds of the invention may also be used to "prophylactically treat" or "prevent" a disorder, for example, in a subject at increased risk of developing the disorder.

The details of one or more embodiments of the invention are set forth in the description below. Other features, objects, and advantages of the invention will be apparent from the description and from the claims.

### Detailed Description

The present disclosure features compounds useful for the inhibition of BRG1 and optionally BRM. These compounds may be used to modulate the activity of a BAF complex, for example, for the treatment of a BAF-related disorder, such as cancer (e.g., BRG1-loss of function disorders). Exemplary compounds described herein include compounds having a structure according to **Formula I,** or a pharmaceutically acceptable salt thereof.
In an aspect, the invention features a compound, or a pharmaceutically acceptable salt thereof, having the structure of **Formula I:** 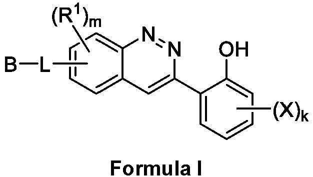 where
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl, optionally substituted C₃-C₈ cycloalkoxy, optionally substituted C₂-C₆ alkynyl, optionally substituted amino, or cyano;
each X is, independently, halo or optionally substituted C₁-C₆ heteroalkyl;
L is a linker of **Formula II:**

   A¹-(B¹)_{f}-(C¹)_{g}-(B²)ₕ-(D)-(B³)ᵢ-(C²)ⱼ-(B⁴)ₖ-A², **Formula II**
or a pharmaceutically acceptable salt thereof,
wherein
A¹ is a bond between the linker and the ring system A;
A² is a bond between the degradation moiety and the linker;
each of B¹, B², B³, and B⁴ is, independently, optionally substituted C₁-C₄ alkyl, optionally substituted C₆-C₁₀ aryl, optionally substituted C₆-C₁₀ aryl C₁₋₄ alkyl, optionally substituted C₁-C₄ heteroalkyl, optionally substituted C₃-C₁₀ cycloalkyl, optionally substituted C₃-C₁₀ carbocyclyl, optionally substituted C₂-C₁₀ heterocyclyl, optionally substituted C₂-C₆ heteroaryl, optionally substituted C₆₋₁₂ aryl, O, S, S(O)₂, or NR^{N};
each R^{N} is, independently, H, optionally substituted C₁₋₄ alkyl, optionally substituted C₂₋₄ alkenyl, optionally substituted C₂₋₄ alkynyl, optionally substituted C₂₋₁₀ heterocyclyl, optionally substituted C₂₋₆ heteroaryl, or optionally substituted C₁₋₇ heteroalkyl;
each of C¹ and C² is, independently, carbonyl, thiocarbonyl, sulphonyl, or phosphoryl;
each of f, g, h, i, j, and k is, independently, 0 or 1; and
D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₂₋₆ heteroaryl, optionally substituted C₆₋₁₂ aryl, optionally substituted C₂-C₁₀ polyethylene glycol, optionally substituted C₃-C₁₀ cycloalkyl, optionally substituted C₃-C₁₀ carbocyclyl, or optionally substituted C₁₋₁₀ heteroalkyl; or D is absent, and the linker is A¹-(B¹)_{f}-(C¹)_{g}-(B²)ₕ-(B³)ᵢ-(C²)ⱼ-(B⁴)ₖ-A²; and
   B is a degradation moiety of **Formula C:** 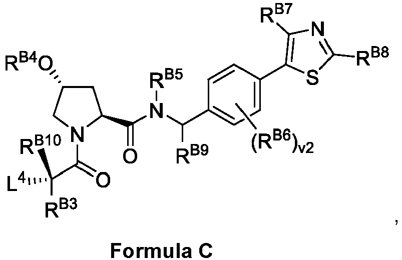
wherein
L⁴ is -N(R^{B1})(R^{B2}), 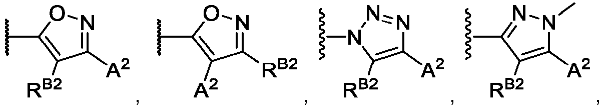 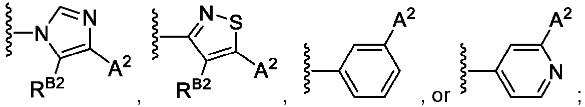
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

In some embodiments, the compound has the structure of any one of compounds 1-47 in Table 1, or pharmaceutically acceptable salt thereof.

Other embodiments, as well as exemplary methods for the synthesis of production of these compounds, are described herein.

### Pharmaceutical Uses

As already indicated, in the present disclosure all references to methods of medical treatment comprising administration of compounds or compositions are to be interpreted as being directed to the compounds or compositions for use in such methods of medical treatment.

The compounds described herein are useful in the methods of the disclosure and, while not bound by theory, are believed to exert their ability to modulate the level, status, and/or activity of a BAF complex, i.e., by inhibiting the activity of the BRG1 and/or BRM proteins within the BAF complex in a mammal. BAF complex-related disorders include, but are not limited to, BRG1 loss of function mutation-related disorders.

An aspect of the present invention relates to methods of treating disorders related to BRG1 loss of function mutations such as cancer (e.g., non-small cell lung cancer, colorectal cancer, bladder cancer, cancer of unknown primary, glioma, breast cancer, melanoma, non-melanoma skin cancer, endometrial cancer, or penile cancer) in a subject in need thereof. In some embodiments, the compound is administered in an amount and for a time effective to result in one or more (e.g., two or more, three or more, four or more) of: (a) reduced tumor size, (b) reduced rate of tumor growth, (c) increased tumor cell death (d) reduced tumor progression, (e) reduced number of metastases, (f) reduced rate of metastasis, (g) decreased tumor recurrence (h) increased survival of subject, (i) increased progression free survival of subject.

Treating cancer can result in a reduction in size or volume of a tumor. For example, after treatment, tumor size is reduced by 5% or greater (e.g., 10%, 20%, 30%, 40%, 50%, 60%, 70%, 80%, 90%, or greater) relative to its size prior to treatment. Size of a tumor may be measured by any reproducible means of measurement. For example, the size of a tumor may be measured as a diameter of the tumor.

Treating cancer may further result in a decrease in number of tumors. For example, after treatment, tumor number is reduced by 5% or greater (e.g., 10%, 20%, 30%, 40%, 50%, 60%, 70%, 80%, 90%, or greater) relative to number prior to treatment. Number of tumors may be measured by any reproducible means of measurement, e.g., the number of tumors may be measured by counting tumors visible to the naked eye or at a specified magnification (e.g., 2x, 3x, 4x, 5x, 10x, or 50x).

Treating cancer can result in a decrease in number of metastatic nodules in other tissues or organs distant from the primary tumor site. For example, after treatment, the number of metastatic nodules is reduced by 5% or greater (e.g., 10%, 20%, 30%, 40%, 50%, 60%, 70%, 80%, 90% or greater) relative to number prior to treatment. The number of metastatic nodules may be measured by any reproducible means of measurement. For example, the number of metastatic nodules may be measured by counting metastatic nodules visible to the naked eye or at a specified magnification (e.g., 2x, 10x, or 50x).

Treating cancer can result in an increase in average survival time of a population of subjects treated according to the present invention in comparison to a population of untreated subjects. For example, the average survival time is increased by more than 30 days (more than 60 days, 90 days, or 120 days). An increase in average survival time of a population may be measured by any reproducible means. An increase in average survival time of a population may be measured, for example, by calculating for a population the average length of survival following initiation of treatment with the compound of the invention. An increase in average survival time of a population may also be measured, for example, by calculating for a population the average length of survival following completion of a first round of treatment with a pharmaceutically acceptable salt of the invention.

Treating cancer can also result in a decrease in the mortality rate of a population of treated subjects in comparison to an untreated population. For example, the mortality rate is decreased by more than 2% (e.g., more than 5%, 10%, or 25%). A decrease in the mortality rate of a population of treated subjects may be measured by any reproducible means, for example, by calculating for a population the average number of disease-related deaths per unit time following initiation of treatment with a pharmaceutically acceptable salt of the invention. A decrease in the mortality rate of a population may also be measured, for example, by calculating for a population the average number of disease-related deaths per unit time following completion of a first round of treatment with a pharmaceutically acceptable salt of the invention.

Exemplary cancers that may be treated by the invention include, but are not limited to, non-small cell lung cancer, small-cell lung cancer, colorectal cancer, bladder cancer, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer and penile cancer.

### Combination Formulations and Uses Thereof

The compounds of the invention can be combined with one or more therapeutic agents. In particular, the therapeutic agent can be one that treats or prophylactically treats any cancer described herein.

### Combination Therapies

A compound of the invention can be used alone or in combination with an additional therapeutic agent, e.g., other agents that treat cancer or symptoms associated therewith, or in combination with other types of treatment to treat cancer. In combination treatments, the dosages of one or more of the therapeutic compounds may be reduced from standard dosages when administered alone. For example, doses may be determined empirically from drug combinations and permutations or may be deduced by isobolographic analysis (e.g., Black et al., Neurology 65:S3-S6, 2005). In this case, dosages of the compounds when combined should provide a therapeutic effect.

In some embodiments, the second therapeutic agent is a chemotherapeutic agent (e.g., a cytotoxic agent or other chemical compound useful in the treatment of cancer). These include alkylating agents, antimetabolites, folic acid analogs, pyrimidine analogs, purine analogs and related inhibitors, vinca alkaloids, epipodopyyllotoxins, antibiotics, L-Asparaginase, topoisomerase inhibitors, interferons, platinum coordination complexes, anthracenedione substituted urea, methyl hydrazine derivatives, adrenocortical suppressant, adrenocorticosteroides, progestins, estrogens, antiestrogen, androgens, antiandrogen, and gonadotropin-releasing hormone analog. Also included is 5-fluorouracil (5-FU), leucovorin (LV), irenotecan, oxaliplatin, capecitabine, paclitaxel and doxetaxel. Non-limiting examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammall and calicheamicin omegall (see, e.g., Agnew, Chem. Intl. Ed Engl. 33:183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo- 5-oxo-L-norleucine, Adriamycin^{®} (doxorubicin, including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5- FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK^{®} polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T- 2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., Taxol^{®} paclitaxel (Bristol-Myers Squibb Oncology, Princeton, N.J.), ABraxane^{®}, cremophor-free, albumin-engineered nanoparticle formulation of paclitaxel (American Pharmaceutical Partners, Schaumberg, III.), and Taxotere^{®} doxetaxel (Rhone-Poulenc Rorer, Antony, France); chloranbucil; Gemzar^{®} gemcitabine; 6-thioguanine; mercaptopurine; methotrexate; platinum coordination complexes such as cisplatin, oxaliplatin and carboplatin; vinblastine; platinum; etoposide (VP-16); ifosfamide; mitoxantrone; vincristine; Navelbine^{®} vinorelbine; novantrone; teniposide; edatrexate; daunomycin; aminopterin; xeloda; ibandronate; irinotecan (e.g., CPT-11); topoisomerase inhibitor RFS 2000; difluoromethylornithine (DMFO); retinoids such as retinoic acid; capecitabine; and pharmaceutically acceptable salts, acids or derivatives of any of the above. Two or more chemotherapeutic agents can be used in a cocktail to be administered in combination with the first therapeutic agent described herein. Suitable dosing regimens of combination chemotherapies are known in the art and described in, for example, Saltz et al. (1999) Proc ASCO 18:233a and Douillard et al. (2000) Lancet 355:1041-7.

In some embodiments, the second therapeutic agent is a therapeutic agent which is a biologic such a cytokine (e.g., interferon or an interleukin (e.g., IL-2)) used in cancer treatment. In some embodiments the biologic is an anti-angiogenic agent, such as an anti-VEGF agent, e.g., bevacizumab (Avastin^{®}). In some embodiments the biologic is an immunoglobulin-based biologic, e.g., a monoclonal antibody (e.g., a humanized antibody, a fully human antibody, an Fc fusion protein or a functional fragment thereof) that agonizes a target to stimulate an anti-cancer response or antagonizes an antigen important for cancer. Such agents include Rituxan (Rituximab); Zenapax (Daclizumab); Simulect (Basiliximab); Synagis (Palivizumab); Remicade (Infliximab); Herceptin (Trastuzumab); Mylotarg (Gemtuzumab ozogamicin); Campath (Alemtuzumab); Zevalin (Ibritumomab tiuxetan); Humira (Adalimumab); Xolair (Omalizumab); Bexxar (Tositumomab-I-131); Raptiva (Efalizumab); Erbitux (Cetuximab); Avastin (Bevacizumab); Tysabri (Natalizumab); Actemra (Tocilizumab); Vectibix (Panitumumab); Lucentis (Ranibizumab); Soliris (Eculizumab); Cimzia (Certolizumab pegol); Simponi (Golimumab); Ilaris (Canakinumab); Stelara (Ustekinumab); Arzerra (Ofatumumab); Prolia (Denosumab); Numax (Motavizumab); ABThrax (Raxibacumab); Benlysta (Belimumab); Yervoy (Ipilimumab); Adcetris (Brentuximab Vedotin); Perjeta (Pertuzumab); Kadcyla (Ado-trastuzumab emtansine); and Gazyva (Obinutuzumab). Also included are antibody-drug conjugates.

The second agent may be a therapeutic agent which is a non-drug treatment. For example, the second therapeutic agent is radiation therapy, cryotherapy, hyperthermia and/or surgical excision of tumor tissue.

The second agent may be a checkpoint inhibitor. In one embodiment, the inhibitor of checkpoint is an inhibitory antibody (e.g., a monospecific antibody such as a monoclonal antibody). The antibody may be, e.g., humanized or fully human. In some embodiments, the inhibitor of checkpoint is a fusion protein, e.g., an Fc-receptor fusion protein. In some embodiments, the inhibitor of checkpoint is an agent, such as an antibody, that interacts with a checkpoint protein. In some embodiments, the inhibitor of checkpoint is an agent, such as an antibody, that interacts with the ligand of a checkpoint protein. In some embodiments, the inhibitor of checkpoint is an inhibitor (e.g., an inhibitory antibody or small molecule inhibitor) of CTLA-4 (e.g., an anti-CTLA4 antibody such as ipilimumab/Yervoy or tremelimumab). In some embodiments, the inhibitor of checkpoint is an inhibitor (e.g., an inhibitory antibody or small molecule inhibitor) of PD-1 (e.g., nivolumab/Opdivo^{®}; pembrolizumab/Keytruda^{®}; pidilizumab/CT-011). In some embodiments, the inhibitor of checkpoint is an inhibitor (e.g., an inhibitory antibody or small molecule inhibitor) of PDL1 (e.g., MPDL3280A/RG7446; MEDI4736; MSB0010718C; BMS 936559). In some embodiments, the inhibitor of checkpoint is an inhibitor (e.g., an inhibitory antibody or Fc fusion or small molecule inhibitor) of PDL2 (e.g., a PDL2/Ig fusion protein such as AMP 224). In some embodiments, the inhibitor of checkpoint is an inhibitor (e.g., an inhibitory antibody or small molecule inhibitor) of B7-H3 (e.g., MGA271), B7-H4, BTLA, HVEM, TIM3, GAL9, LAG3, VISTA, KIR, 2B4, CD160, CGEN-15049, CHK 1, CHK2, A2aR, B-7 family ligands, or a combination thereof.

In any of the combination embodiments described herein, the first and second therapeutic agents are administered simultaneously or sequentially, in either order. The first therapeutic agent may be administered immediately, up to 1 hour, up to 2 hours, up to 3 hours, up to 4 hours, up to 5 hours, up to 6 hours, up to 7 hours, up to, 8 hours, up to 9 hours, up to 10 hours, up to 11 hours, up to 12 hours, up to 13 hours, 14 hours, up to hours 16, up to 17 hours, up 18 hours, up to 19 hours up to 20 hours, up to 21 hours, up to 22 hours, up to 23 hours up to 24 hours or up to 1-7, 1-14, 1-21 or 1-30 days before or after the second therapeutic agent.

### Pharmaceutical Compositions

The compounds of the invention are preferably formulated into pharmaceutical compositions for administration to a mammal, preferably, a human, in a biologically compatible form suitable for administration in vivo. Accordingly, in an aspect, the present invention provides a pharmaceutical composition comprising a compound of the invention in admixture with a suitable diluent, carrier, or excipient.

The compounds of the invention may be used in the form of the free base, in the form of salts, solvates, and as prodrugs. All forms are within the scope of the invention. In accordance with the methods of the invention, the described compounds or salts, solvates, or prodrugs thereof may be administered to a patient in a variety of forms depending on the selected route of administration, as will be understood by those skilled in the art. The compounds of the invention may be administered, for example, by oral, parenteral, buccal, sublingual, nasal, rectal, patch, pump, or transdermal administration and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

A compound of the invention may be orally administered, for example, with an inert diluent or with an assimilable edible carrier, or it may be enclosed in hard- or soft-shell gelatin capsules, or it may be compressed into tablets, or it may be incorporated directly with the food of the diet. For oral therapeutic administration, a compound of the invention may be incorporated with an excipient and used in the form of ingestible tablets, buccal tablets, troches, capsules, elixirs, suspensions, syrups, and wafers. A compound of the invention may also be administered parenterally. Solutions of a compound of the invention can be prepared in water suitably mixed with a surfactant, such as hydroxypropylcellulose. Dispersions can also be prepared in glycerol, liquid polyethylene glycols, DMSO, and mixtures thereof with or without alcohol, and in oils. Under ordinary conditions of storage and use, these preparations may contain a preservative to prevent the growth of microorganisms. Conventional procedures and ingredients for the selection and preparation of suitable formulations are described, for example, in Remington's Pharmaceutical Sciences (2003, 20th ed.) and in The United States Pharmacopeia: The National Formulary (USP 24 NF19), published in 1999. The pharmaceutical forms suitable for injectable use include sterile aqueous solutions or dispersions and sterile powders for the extemporaneous preparation of sterile injectable solutions or dispersions. In all cases the form must be sterile and must be fluid to the extent that may be easily administered via syringe. Compositions for nasal administration may conveniently be formulated as aerosols, drops, gels, and powders. Aerosol formulations typically include a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal after use. Where the dosage form comprises an aerosol dispenser, it will contain a propellant, which can be a compressed gas, such as compressed air or an organic propellant, such as fluorochlorohydrocarbon. The aerosol dosage forms can also take the form of a pump-atomizer. Compositions suitable for buccal or sublingual administration include tablets, lozenges, and pastilles, where the active ingredient is formulated with a carrier, such as sugar, acacia, tragacanth, gelatin, and glycerine. Compositions for rectal administration are conveniently in the form of suppositories containing a conventional suppository base, such as cocoa butter. A compound described herein may be administered intratumorally, for example, as an intratumoral injection. Intratumoral injection is injection directly into the tumor vasculature and is specifically contemplated for discrete, solid, accessible tumors. Local, regional, or systemic administration also may be appropriate. A compound described herein may advantageously be contacted by administering an injection or multiple injections to the tumor, spaced for example, at approximately, 1 cm intervals. In the case of surgical intervention, the present invention may be used preoperatively, such as to render an inoperable tumor subject to resection. Continuous administration also may be applied where appropriate, for example, by implanting a catheter into a tumor or into tumor vasculature.

The compounds of the invention may be administered to an animal, e.g., a human, alone or in combination with pharmaceutically acceptable carriers, as noted herein, the proportion of which is determined by the solubility and chemical nature of the compound, chosen route of administration, and standard pharmaceutical practice.

### Dosages

The dosage of the compounds of the invention, and/or compositions comprising a compound of the invention, can vary depending on many factors, such as the pharmacodynamic properties of the compound; the mode of administration; the age, health, and weight of the recipient; the nature and extent of the symptoms; the frequency of the treatment, and the type of concurrent treatment, if any; and the clearance rate of the compound in the animal to be treated. One of skill in the art can determine the appropriate dosage based on the above factors. The compounds of the invention may be administered initially in a suitable dosage that may be adjusted as required, depending on the clinical response. In general, satisfactory results may be obtained when the compounds of the invention are administered to a human at a daily dosage of, for example, between 0.05 mg and 3000 mg (measured as the solid form). Dose ranges include, for example, between 10-1000 mg (e.g., 50-800 mg). In some embodiments, 50, 100, 150, 200, 250, 300, 350, 400, 450, 500, 550, 600, 650, 700, 750, 800, 850, 900, 950, or 1000 mg of the compound is administered.

Alternatively, the dosage amount can be calculated using the body weight of the patient. For example, the dose of a compound, or pharmaceutical composition thereof, administered to a patient may range from 0.1-100 mg/kg (e.g., 0.25-25 mg/kg). In exemplary, non-limiting embodiments, the dose may range from 0.5-5.0 mg/kg (e.g., 0.5, 1.0, 1.5, 2.0, 2.5, 3.0, 3.5, 4.0, 4.5, or 5.0 mg/kg) or from 5.0-20 mg/kg (e.g., 5.5, 6.0, 6.5, 7.0, 7.5, 8.0, 8.5, 9.0, 9.5, 10, 11, 12, 13, 14, 15, 16, 17, 18, 19, or 20 mg/kg).

### EXAMPLES

The following abbreviations are used throughout the Examples below.
- Ac: acetyl
- ACN or MeCN: acetonitrile
- AcOH: acetic acid
- Ac₂O: acetic anhydride
- aq.: aqueous
- Boc: tert-butoxycarbonyl
- Bu or n-Bu: butyl
- CDI: 1,1'-carbonyldiimidazole
- DCE or 1,2-DCE: 1,2-dichloroethane
- DCM: dichloromethane
- DIAD: diisopropyl azodicarboxylate
- DIPEA or DIEA: N.N-diisopropylethylamine
- DMAP: 4-(dimethylamino)pyridine
- DMB: 2,4-dimethoxybenzyl
- DME: 1,2-dimethoxyethane
- DMF: N.N-dimethylformamide
- DMSO: dimethyl sulfoxide
- EA or EtOAc: ethyl acetate
- EDCl: N-(3-dimethylaminopropyl)-N'-ethylcarbodiimide hydrochloride
- equiv: equivalents
- Et₃N or TEA: triethylamine
- EtOH: ethyl alcohol
- FA: formic acid
- h or hr: hour
- HATU: 1-[bis(dimethylamino)methylene]-1H-1,2,3-triazolo[4,5-b]pyridinium 3-oxid hexafluorophosphate
- HOAt: 1-hydroxy-7-azabenzotriazole
- HOBt or HOBT: 1-hydroxybenzotriazole hydrate
- iPr: Isopropyl
- MeOH: methyl alcohol
- Me₄t-BuXphos: ditert-butyl-[2,3,4,5-tetramethyl-6-(2,4,6-triisopropylphenyl)phenyl]phosphane
- min: minute
- MTBE: tert-butyl methyl ether
- n-BuLi: n-butylithium
- NMP: 1-methyl-2-pyrrolidinone
- OAc: acetate
- Pd/C: palladium on carbon
- PDC: pyridinium dichromate
- PdCl₂(dtbpf) or Pd(dtbpf)Cl₂: dichloro[1,1'-bis(di-t-butylphosphino)ferrocene]palladium(II)
- PdCl₂(dppf) or Pd(dppf)Cl₂: [1,1'-bis(diphenylphosphino)ferrocene]dichloropalladium(II)
- Pd₂(dba)₃: tris(dibenzylideneacetone)dipalladium(0)
- Pd(PPh₃)₄: tetrakis(triphenylphosphine)palladium(0
- Pd(PPh₃)₂Cl₂: dichlorobis(triphenylphosphine)palladium(II)
- PE: petroleum ether
- PPh₃: triphenylphosphine
- Pr: n-propyl
- Py: pyridine
- rac: racemic
- Rf: retention factor
- r.t. or rt: room temperature
- sat.: saturated
- SFC: supercritical fluid chromatography
- t-Bu: tert-butyl
- tBuXphos-Pd-G3 or tBuXphos Pd G₃ or t-BuXphos-Pd (gen 3): [2-(2-aminophenyl)phenyl]-methylsulfonyloxypalladium;ditert- butyl-[2-(2,4,6-triisopropylphenyl)phenyl]phosphane
- TFA: trifluoroacetic acid
- Tf₂O: trifluoromethanesulfonic anhydride
- THF: tetrahydrofuran
- TLC: thin layer chromatography
- Xantphos-Pd-G3: [2-(2- aminophenyl)phenyl]-methylsulfonyloxy-palladium;(5-diphenylphosphanyl-9,9-dimethyl-xanthen-4-yl)-diphenyl-phosphane
- XPhos Pd G3: (2-Dicyclohexylphosphino-2',4',6'-triisopropyl-1,1'-biphenyl)[2-(2'-amino-1,1'-biphenyl)]palladium(II) methanesulfonate

### Example 1. Preparation of Compounds

### Preparation of (2S,4R)-4-hydroxy-1-((R)-3-methyl-2-(3-(2-oxoethoxy)isoxazol-5-yl)butanoyl)-N-((S)-1-(4-(4-methylthiazol-5-yl)phenyl)ethyl)pyrrolidine-2-carboxamide (1-2)

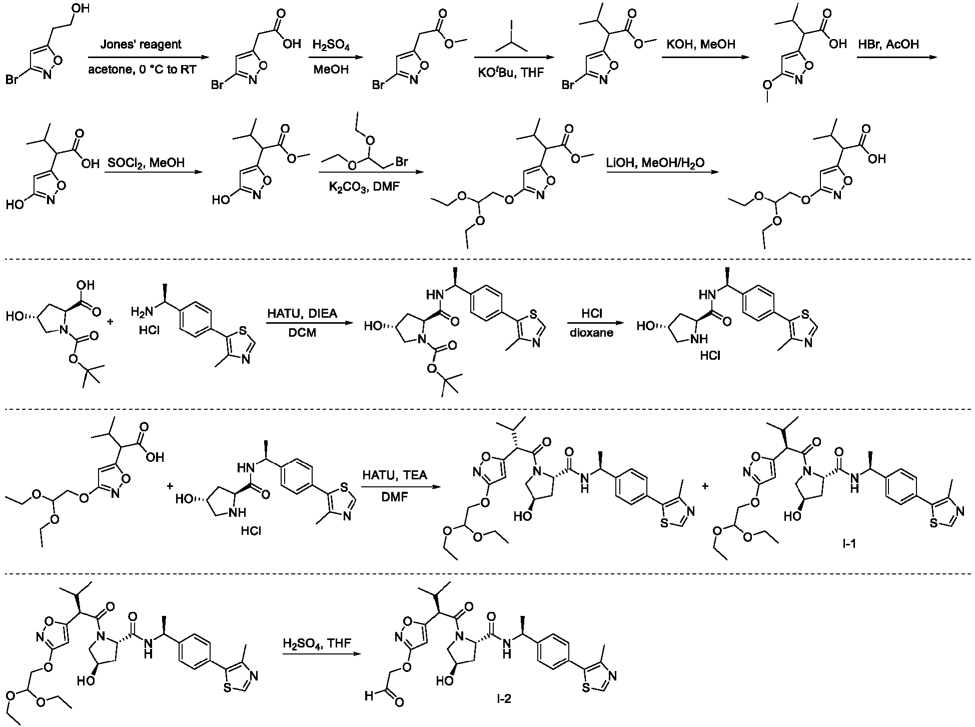

### Step 1: Preparation of 2-(3-bromoisoxazol-5-yl)acetic acid.

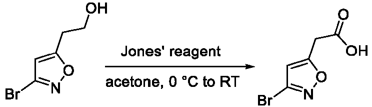

To a stirring solution of 2-(3-bromo-1 ,2-oxazol-5-yl)ethan-1-ol (30 g, 156 mmol) in acetone (389 mL) was added Jones' reagent (2 M in acetone, 156 mL, 312 mmol) dropwise at 0 °C. The resulting solution was stirred at 25 °C overnight. The mixture was diluted with water and extracted with EtOAc. The organic layer was washed with brine, and dried over anhydrous Na₂SO₄ and concentrated under reduced pressure to afford 2-(3-bromoisoxazol-5-yl)acetic acid as a brown solid (28 g, 86.5%). LCMS (ESI) m/z: [M+H]⁺ = 206.08 and 208.08.

### Step 2: Preparation of methyl 2-(3-bromoisoxazol-5-yl)acetate.

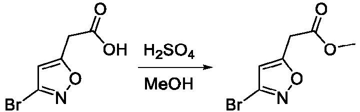

A solution of 2-(3-bromoisoxazol-5-yl)acetic acid (28 g, 135 mmol) and concentrated H₂SO₄ (3 mL, 72 mmol) in methanol (250 mL) was stirred at 70 °C for 2 h. The resulting solution was concentrated under reduced pressure. The residue was diluted with water and extracted with EtOAc. The organic layer was washed with brine, and dried over anhydrous MgSO₄ and concentrated under reduced pressure. The residue was purified by silica gel flash chromatography (EtOAc/petroleum ether) to afford methyl 2-(3-bromoisoxazol-5-yl)acetate as a white solid (23.4 g, 79%). LCMS (ESI) m/z: [M+H]⁺ = 219.90 and 221.86.

### Step 3: Preparation of methyl 2-(3-bromoisoxazol-5-yl)-3-methylbutanoate.

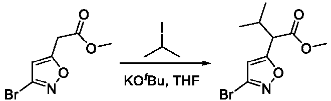

To a stirring solution of methyl 2-(3-bromoisoxazol-5-yl)acetate (23.4 g, 106 mmol) and KO^{t}Bu (17.8 g, 159 mmol) in THF (210 mL) was added 2-iodopropane (13.8 mL, 137 mmol) dropwise at 0 °C. The reaction mixture was stirred at room temperature for 16 h and then quenched with water/ice. The resulting solution was extracted several times with EtOAc. The combined organic layers were washed with brine, and dried over anhydrous Na₂SO₄ and concentrated under reduced pressure. The residue was purified by silica gel flash chromatography (EtOAc/petroleum ether) to afford methyl 2-(3-bromoisoxazol-5-yl)-3-methylbutanoate as a clear oil (16.7 g, 60%).

### Step 4: Preparation of 2-(3-methoxyisoxazol-5-yl)-3-methylbutanoic acid.

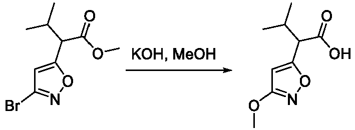

To a solution of methyl 2-(3-bromo-1,2-oxazol-5-yl)-3-methylbutanoate (16.7 g, 63.7 mmol) in methanol (130 mL) was added potassium hydroxide (35.7 g, 637 mmol). The mixture was stirred for 4 h at 100 °C. The mixture was concentrated under vacuum and then diluted with water. The resulting solution was washed with EtOAc and pH of the aqueous layer was adjusted to pH 5 with 1 N HCl. This mixture was extracted several times with EtOAc. The combined organic layers were washed with brine and dried over anhydrous MgSO₄. The residue was purified by silica gel flash chromatography (EtOAc/petroleum ether) to afford 2-(3-methoxyisoxazol-5-yl)-3-methylbutanoic acid as a yellow oil (8.8 g, 70%). LCMS (ESI) m/z: [M+H]⁺ = 200.15.

### Step 5: Preparation of 2-(3-hydroxyisoxazol-5-yl)-3-methylbutanoic acid.

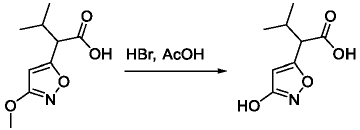

A solution of 2-(3-methoxyisoxazol-5-yl)-3-methylbutanoic acid (8.8 g, 44.1 mmol) in HOAc (80 mL) and HBr (80 mL) was stirred at 60 °C for 16 h. The resulting mixture was concentrated under reduced pressure to afford crude 2-(3-hydroxyisoxazol-5-yl)-3-methylbutanoic acid (8.16 g, quant.).

### Step 6: Preparation of methyl 2-(3-hydroxyisoxazol-5-yl)-3-methylbutanoate.

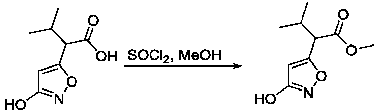

To a solution of 2-(3-hydroxy-1 ,2-oxazol-5-yl)-3-methylbutanoic acid (8.16 g, 44.0 mmol) in methanol (30 mL) was slowly added SOCl₂ (14.2 mL, 197 mmol). The mixture was stirred at room temperature for 3 h. The solvent was removed under reduced pressure. The residue was diluted with water and extracted with EtOAc. The organic layer was washed with brine, and dried over anhydrous Na₂SO₄ and concentrated under reduced pressure. The residue was purified by silica gel flash chromatography (MeOH/DCM) to afford methyl 2-(3-hydroxyisoxazol-5-yl)-3-methylbutanoate as a clear oil (7.79 g, 89%). LCMS (ESI) m/z: [M+H]⁺ = 200.15.

### Step 7: Preparation of methyl 2-(3-(2,2-diethoxyethoxy)isoxazol-5-yl)-3-methylbutanoate.

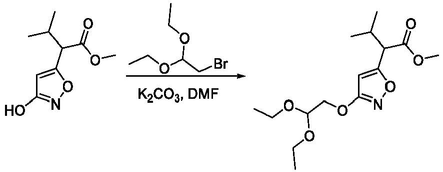

To a solution of methyl 2-(3-hydroxy-1,2-oxazol-5-yl)-3-methylbutanoate (7.79 g, 39.1 mmol) in DMF (90 mL) were added 2-bromo-1,1-diethoxyethane (8.77 mL, 58.6 mmol) and potassium carbonate (10.8 g, 78.2 mmol). The reaction was stirred at 70 °C overnight. The reaction mixture was cooled and then water was added to the mixture. The resulting mixture was extracted with EtOAc several times. The combined organic layers were washed with brine and dried over anhydrous MgSO₄. Solvent was removed under reduced pressure and the resulting residue was purified by silica gel flash chromatography (EtOAc/heptane) to afford methyl 2-(3-(2,2-diethoxyethoxy)isoxazol-5-yl)-3-methylbutanoate as a colorless oil (7.8 g, 63%). LCMS (ESI) m/z: [M-C₂H₅O]⁺ = 270.30.

### Step 8: Preparation of 2-(3-(2,2-diethoxyethoxy)isoxazol-5-yl)-3-methylbutanoic acid.

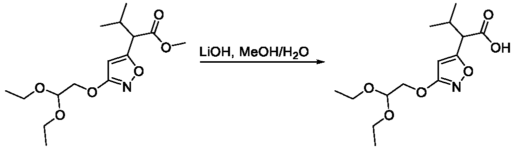

To a solution of methyl 2-[3-(2,2-diethoxyethoxy)-1,2-oxazol-5-yl]-3-methylbutanoate (7.8 g, 24.7 mmol) in methanol (50 mL) and water (25 mL) was added lithium hydroxide mono hydrate (4.14 g, 98.8 mmol). The reaction was stirred at 40 °C for 2 h. The pH was adjusted to 4-5 with 1 N HCl. The mixture was extracted with ethyl acetate several times and combined organic layers were dried over MgSO₄. Solvents were removed under reduced pressure and the residue was purified by silica gel flash chromatography (DCM/MeOH) to afford 2-(3-(2,2-diethoxyethoxy)isoxazol-5-yl)-3-methylbutanoic acid as a colorless oil (6.1 g, 89%). LCMS (ESI) m/z: [M-H]⁻ = 300.21.

### Step 9: Preparation of tert-butyl (2S,4R)-4-hydroxy-2-(((S)-1-(4-(4-methylthiazol-5-yl)phenyl)ethyl)carbamoyl)pyrrolidine-1-carboxylate.

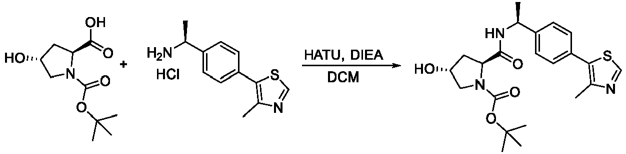

To a solution of (S)-1-(4-(4-methylthiazol-5-yl)phenyl)ethan-1-amine hydrochloride (5.0 g, 19.6 mmol) and (2S,4R)-1-[(tert-butoxy)carbonyl]-4-hydroxypyrrolidine-2-carboxylic acid (4.47 g, 20.5 mmol) in DCM (70 mL) at 0 °C was added HATU (8.98 g, 23.5 mmol) followed by dropwise addition of DIEA (16.4 mL, 98.0 mmol). After stirring for 16 h at room temperature, the reaction mixture was poured into ice water. The resulting mixture was extracted several times with DCM. The combined organic layers were washed with water, brine, and dried over anhydrous Na₂SO₄ and concentrated under vacuum. The resulting residue was purified by silica gel flash chromatography (MeOH/DCM) to afford tert-butyl (2S,4R)-4-hydroxy-2-(((S)-1-(4-(4-methylthiazol-5-yl)phenyl)ethyl)carbamoyl)pyrrolidine-1-carboxylate (8.33 g, 98%). LCMS (ESI) m/z: [M+H]⁺ = 432.38.

### Step 10: Preparation of (2S,4R)-4-hydroxy-N-((S)-1-(4-(4-methylthiazol-5-yl)phenyl)ethyl)pyrrolidine-2-carboxamide hydrochloride.

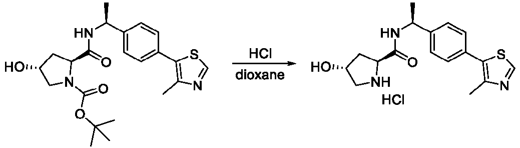

To tert-butyl (2S,4R)-4-hydroxy-2-(((S)-1-(4-(4-methylthiazol-5-yl)phenyl)ethyl)carbamoyl)pyrrolidine-1-carboxylate (8.33 g, 19.3 mmol) at 0 °C was added a solution of HCl in 1,4-dioxane (4 N, 50 mL, 200 mmol) resulting in a sticky yellow gum. 15 mL of MeOH were added to the mixture and the mixture was stirred at room temperature for 2 h. The solvents were removed under reduced pressure and the residue was washed with diethyl ether to afford (2S,4R)-4-hydroxy-N-((S)-1-(4-(4-methylthiazol-5-yl)phenyl)ethyl)pyrrolidine-2-carboxamide hydrochloride which was used in the next step without further purification.

### Step 11: Preparation of (2S,4R)-1-((R)-2-(3-(2,2-diethoxyethoxy)isoxazol-5-yl)-3-methylbutanoyl)-4-hydroxy-N-((S)-1-(4-(4-methylthiazol-5-yl)phenyl)ethyl)pyrrolidine-2-carboxamide (I-1).

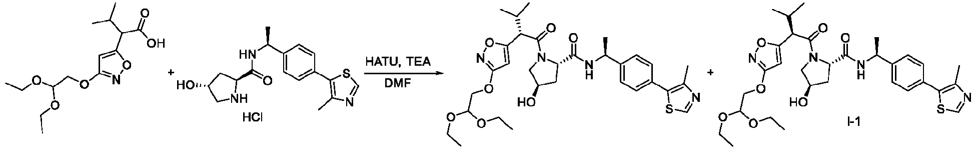

To a solution of 2-[3-(2,2-diethoxyethoxy)isoxazol-5-yl]-3-methyl-butanoic acid (5.75 g, 19.0 mmol) in DMF (30 mL) was added HATU (8.6 g, 22.7 mmol). After stirring at 20 °C for 0.5 h, a solution of (2S,4R)-4-hydroxy-N-[(1S)-1-[4-(4-methylthiazol-5-yl)phenyl]ethyl]pyrrolidine-2-carboxamide hydrochloride (6.97 g, 19.0 mmol) and triethylamine (7.92 mL, 56.9 mmol) in DMF (20 mL) was added to the mixture and the resulting mixture was stirred at 20 °C. The reaction mixture was quenched by addition of water and extracted several times with EtOAc. The combined organic layers were washed with brine, and dried over anhydrous MgSO₄, filtered and concentrated under reduced pressure. The residue was purified by silica gel flash chromatography (DCM/MeOH) to afford (2S,4R)-1-[2-[3-(2,2-diethoxyethoxy)isoxazol-5-yl]-3- methylbutanoyl]-4-hydroxy-N-[(1S)-1-[4-(4-methylthiazol-5-yl)phenyl]ethyl]pyrrolidine-2-carboxamide (10 g, 16.2 mmol) as a white solid. The mixture of diastereomers was separated by chiral SFC chromatography to afford (2S,4R)-1-((S)-2-(3-(2,2-diethoxyethoxy)isoxazol-5-yl)-3-methylbutanoyl)-4-hydroxy-N-((S)-1-(4-(4-methylthiazol-5-yl)phenyl)ethyl)pyrrolidine-2-carboxamide and (2S,4R)-1-((R)-2-(3-(2,2-diethoxyethoxy)isoxazol-5-yl)-3-methylbutanoyl)-4-hydroxy-N-((S)-1-(4-(4-methylthiazol-5-yl)phenyl)ethyl)pyrrolidine-2-carboxamide.
(2S,4R)-1-((S)-2-(3-(2,2-diethoxyethoxy)isoxazol-5-yl)-3-methylbutanoyl)-4-hydroxy-N-((S)-1-(4-(4-methylthiazol-5-yl)phenyl)ethyl)pyrrolidine-2-carboxamide peak 1: (2.2 g, 19%). LCMS (ESI) m/z [M+H]⁺ = 615.4.
(2S,4R)-1-((R)-2-(3-(2,2-diethoxyethoxy)isoxazol-5-yl)-3-methylbutanoyl)-4-hydroxy-N-((S)-1-(4-(4-methylthiazol-5-yl)phenyl)ethyl)pyrrolidine-2-carboxamide (**I-1**) peak 2: (2.5 g, 21%). LCMS (ESI) m/z [M+H]⁺ = 615.4.

### Step 12: Preparation of (2S,4R)-4-hydroxy-1-((R)-3-methyl-2-(3-(2-oxoethoxy)isoxazol-5-yl)butanoyl)-N-((S)-1-(4-(4-methylthiazol-5-yl)phenyl)ethyl)pyrrolidine-2-carboxamide (I-2).

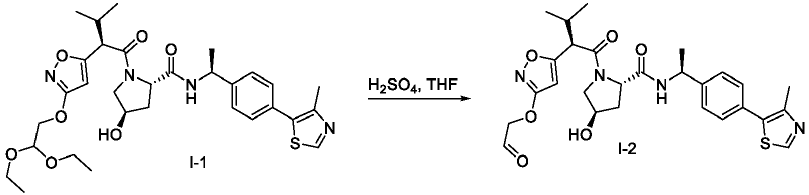

To a stirred solution of H₂SO₄ (1 N, 6.00 mL) and THF (6.00 mL) was added (2S,4R)-1-[(2R)-2-[3-(2-ethoxy-2-methoxyethoxy)-1,2-oxazol-5-yl]-3-methylbutanoyl]-4-hydroxy-N-[(1S)-1-[4-(4-methyl-1,3-thiazol-5-yl)phenyl]ethyl]pyrrolidine-2-carboxamide (I-1, 300 mg, 0.499 mmol) in portions at room temperature. The resulting mixture was stirred for 8 h at 50 °C. The resulting mixture was diluted with water, then neutralized to pH ~7 with saturated aqueous NaHCO₃. The resulting mixture was extracted three times with EtOAc. The combined organic layers were washed twice with brine and dried over anhydrous Na₂SO₄. After filtration, the filtrate was concentrated under reduced pressure to afford (2S,4R)-4-hydroxy-1-((R)-3-methyl-2-(3-(2-oxoethoxy)isoxazol-5-yl)butanoyl)-N-((S)-1-(4-(4-methylthiazol-5-yl)phenyl)ethyl)pyrrolidine-2-carboxamide (**I-2**, 256 mg, 97.3%) as a white solid. LCMS (ESI) m/z: [M+H]⁺ = 541.

### Preparation of (2S,4R)-4-hydroxy-N-[(1S)-1-[4-(4-methyl-1,3-thiazol-5-yl)phenyl]ethyl]-1-[(2R)-3-methyl-2-[3-(piperazin-1-yl)-1,2-oxazol-5-yl]butanoyl]pyrrolidine-2-carboxamide (I-3) and (2S,4R)-4-hydroxy-N-[(1S)-1-[4-(4-methyl-1,3-thiazol-5-yl)phenyl]ethyl]-1-[(2S)-3-methyl-2-[3-(piperazin-1-yl)-1,2-oxazol-5-yl]butanoyl]pyrrolidine-2-carboxamide (1-4).

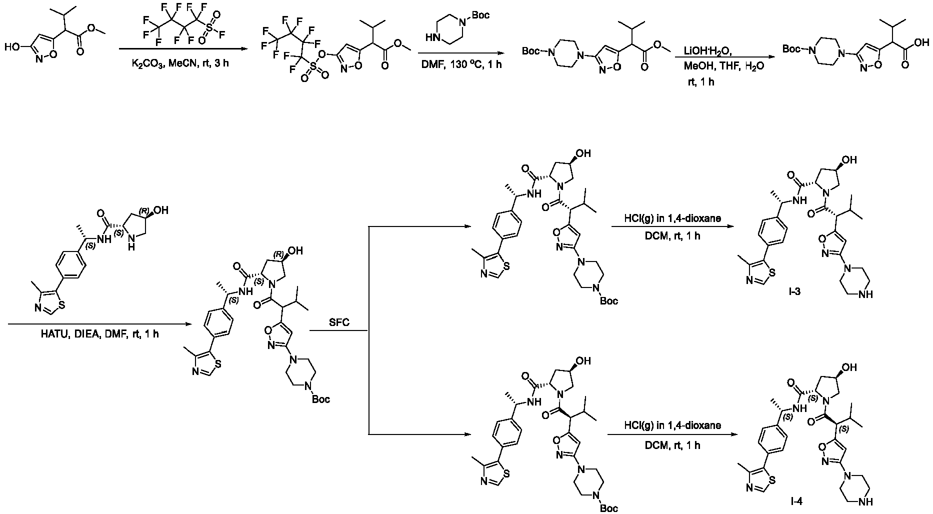

### Step 1: Preparation of methyl 3-methyl-2-[3-[(1,1,2,2,3,3,4,4,4-nonafluorobutanesulfonyl)oxy]-1,2-oxazol-5-yl]butanoate.

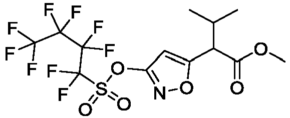

To a stirred solution of methyl 2-(3-hydroxy-1,2-oxazol-5-yl)-3-methylbutanoate (100.00 mg, 0.502 mmol, 1.00 equiv) in MeCN (0.50 mL) were added perfluorobutanesulfonyl fluoride (303.29 mg, 1.004 mmol, 2.00 equiv) and K₂CO₃ (208.13 mg, 1.506 mmol, 3.00 equiv) at room temperature. The resulting mixture was stirred for 3 h, then carefully quenched with water at 0 degrees C. The resulting mixture was extracted with EA (2 x 50 mL). The combined organic layers were washed with brine (50 mL) and dried over anhydrous Na₂SO₄. After filtration, the filtrate was concentrated under reduced pressure. The residue was purified by silica gel column chromatography, eluting with PE/EA (2/1) to afford methyl 3-methyl-2-[3-[(1,1,2,2,3,3,4,4,4-nonafluorobutanesulfonyl)oxy]-1,2-oxazol-5-yl]butanoate (217 mg) as a white solid. LCMS (ESI) m/z: [M+H]⁺ = 482.

### Step 2: Preparation of tert-butyl 4-[5-(1-methoxy-3-methyl-1-oxobutan-2-yl)-1,2-oxazol-3-yl]piperazine-1-carboxylate.

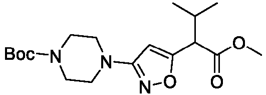

To a stirred solution of methyl 3-methyl-2-[3-[(1,1,2,2,3,3,4,4,4-nonafluorobutanesulfonyl)oxy]-1,2-oxazol-5-yl]butanoate (217.00 mg, 0.451 mmol, 1.00 equiv) in DMF (3.00 mL) was added tert-butyl piperazine-1-carboxylate (83.98 mg, 0.451 mmol, 1.00 equiv) at room temperature. The resulting mixture was stirred for 1 h at 130 °C. The mixture was allowed to cool down to room temperature. The residue was purified by reverse phase flash chromatography with the following conditions: column, C18 silica gel; mobile phase, MeCN in water (0.1% FA), 0 to 100% gradient in 30 min. This provided tert-butyl 4-[5-(1-methoxy-3-methyl-1-oxobutan-2-yl)-1,2-oxazol-3-yl]piperazine-1-carboxylate (54 mg, 32.59%) as a yellow oil. LCMS (ESI) m/z: [M+H]⁺ = 368.

### Step 3: Preparation of 2-[3-[4-(tert-butoxycarbonyl)piperazin-1-yl]-1,2-oxazol-5-yl]-3-methylbutanoic acid.

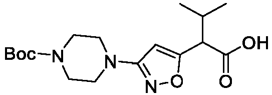

To a stirred solution of tert-butyl 4-[5-(1-methoxy-3-methyl-1-oxobutan-2-yl)-1,2-oxazol-3-yl]piperazine-1-carboxylate (54.00 mg, 0.147 mmol, 1.00 equiv) in MeOH (0.80 mL) were added THF (0.80 mL) and H₂O (0.80 mL) at room temperature, followed by addition of LiOH·H₂O (18.50 mg, 0.441 mmol, 3.00 equiv). The resulting mixture was stirred for 1 h at room temperature. The mixture was acidified to pH 6 with HCl (1 M, aq.), then extracted with EA (2 x 50 mL). The combined organic layers were washed with brine (50 mL), and dried over anhydrous Na₂SO₄, and filtered. The filtrate was concentrated under reduced pressure. This provided 2-[3-[4-(tert-butoxycarbonyl)piperazin-1-yl]-1,2-oxazol-5-yl]-3-methylbutanoic acid (52 mg, crude product) as a yellow solid. LCMS (ESI) m/z: [M+H]⁺ = 354.

### Step 4: Preparation of tert-butyl 4-(5-[1-[(2S,4R)-4-hydroxy-2-[[(1S)-1-[4-(4-methyl-1 ,3-thiazol-5-yl)phenyl]ethyl]carbamoyl]pyrrolidin-1-yl]-3-methyl-1-oxobutan-2-yl]-1,2-oxazol-3-yl)piperazine-1-carboxylate.

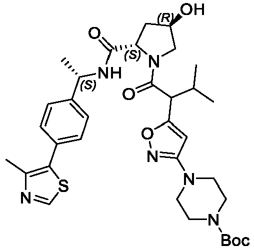

To a stirred solution of 2-[3-[4-(tert-butoxycarbonyl)piperazin-1-yl]-1,2-oxazol-5-yl]-3-methylbutanoic acid (52.00 mg, 0.119 mmol, 1.00 equiv) in DMF (2.00 mL) were added HATU (135.56 mg, 0.357 mmol, 3.00 equiv) and DIEA (76.80 mg, 0.595 mmol, 5.00 equiv) at room temperature. To the above mixture was added (2S,4R)-4-hydroxy-N-[(1S)-1-[4-(4-methyl-1,3-thiazol-5-yl)phenyl]ethyl]pyrrolidine-2-carboxamide (70.90 mg, 0.214 mmol, 1.80 equiv) at room temperature. The resulting mixture was stirred for 1 h. The mixture was purified directly by reverse phase flash chromatography with the following conditions: column, C18 silica gel; mobile phase, MeCN in water (0.1% FA), 0 to 100% gradient in 30 min. This provided tert-butyl 4-(5-[1-[(2S,4R)-4-hydroxy-2-[[(1S)-1-[4-(4-methyl-1,3-thiazol-5-yl)phenyl]ethyl]carbamoyl]pyrrolidin-1-yl]-3-methyl-1-oxobutan-2-yl]-1,2-oxazol-3-yl)piperazine-1-carboxylate (73 mg, 92.12%) as a white solid. LCMS (ESI) m/z: [M+H]⁺ = 667.

### Step 5: Preparation of tert-butyl 4-(5-((R)-1-((2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)piperazine-1-carboxylate and tert-butyl 4-(5-((S)-1-((2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)piperazine-1-carboxylate.

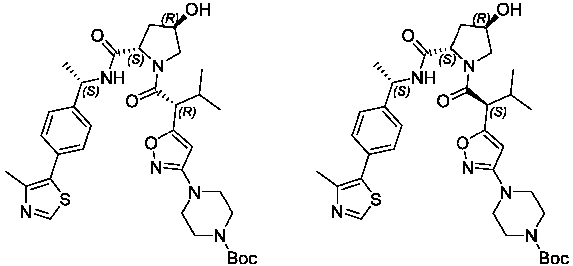

tert-butyl 4-(5-[1-[(2S,4R)-4-hydroxy-2-[[(1S)-1-[4-(4-methyl-1 ,3-thiazol-5-yl)phenyl]ethyl]carbamoyl]pyrrolidin-1-yl]-3-methyl-1-oxobutan-2-yl]-1,2-oxazol-3-yl)piperazine-1-carboxylate (73 mg) was purified by SFC with the following conditions: Column, CHIRAL ART Amylose-C NEO, 3*25 cm, 5 mm; mobile phase, MeOH. This provided:
tert-butyl 4-(5-((R)-1-((2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)piperazine-1-carboxylate (37 mg, second peak). LCMS (ESI) m/z: [M+H]⁺ = 667.
tert-butyl 4-(5-((S)-1-((2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)piperazine-1-carboxylate (34 mg, first peak). LCMS (ESI) m/z: [M+H]⁺ = 667.

### Step 6: Preparation of (2S,4R)-4-hydroxy-N-[(1S)-1-[4-(4-methyl-1,3-thiazol-5-yl)phenyl]ethyl]-1-[(2R)-3-methyl-2-[3-(piperazin-1-yl)-1,2-oxazol-5-yl]butanoyl]pyrrolidine-2-carboxamide (I-3) and (2S,4R)-4-hydroxy-N-[(1S)-1-[4-(4-methyl-1 ,3-thiazol-5-yl)phenyl]ethyl]-1-[(2S)-3-methyl-2-[3-(piperazin-1-yl)-1,2-oxazol-5-yl]butanoyl]pyrrolidine-2-carboxamide (I-4).

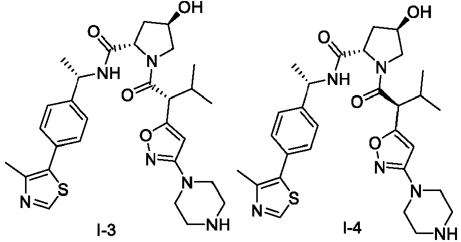

To a stirred solution of tert-butyl 4-(5-((R)-1-((2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)piperazine-1-carboxylate (37.00 mg, 0.055 mmol, 1.00 equiv) in DCM (1.50 mL) was added HCl in 1,4-dioxane (1.50 mL, 26.276 mmol, 473.57 equiv) at 0 °C. The resulting mixture was stirred for 1 h at room temperature, then was concentrated under reduced pressure. This provided **I-3** (45 mg, crude product) as a yellow oil. LCMS (ESI) m/z: [M+H]⁺ = 567.

**I-4** was prepared following the same protocol as **I-3** and was obtained as a yellow oil. LCMS (ESI) m/z: [M+H]⁺ = 567.

The following intermediates in Table 2 were prepared in a similar manner as described in the preparation of intermediate **I-3** starting with methyl 3-methyl-2-[3-[(1,1,2,2,3,3,4,4,4-nonafluorobutanesulfonyl)oxy]-1,2-oxazol-5-yl]butanoate and the appropriate amines.

**Table 2.**

| **Structure** | **Intermediate No.** | **Name** | **LCMS (ESI) m/z** |
|---|---|---|---|
| 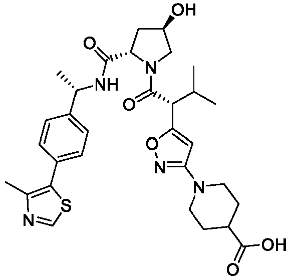 | **I-5** | 1-{5-[(2R)-1-[(2S,4R)-4-hydroxy-2-{[(1S)-1-[4-(4-methyl-1,3-thiazol-5-yl)phenyl]ethyl]carbamoyl}pyrr olidin-1-yl]-3-methyl-1-oxobutan-2-yl]-1,2-oxazol-3-yl}piperidine-4-carboxylic acid | 610 |
| 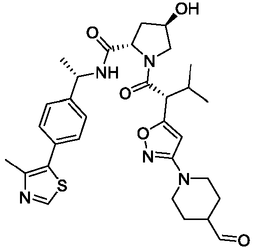 | **I-6** | (2S,4R)-1-[(2R)-2-[3-(4-formylpiperidin-1-yl)-1,2-oxazol-5-yl]-3-methylbutanoyl]-4-hydroxy-N-[(1S)-1-[4-(4-methyl-1,3-thiazol-5-yl)phenyl]ethyl]pyrrolidine-2-carboxamide | 594 |
| 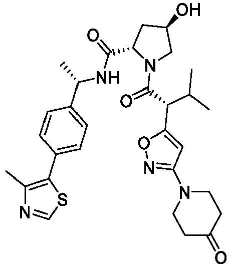 | **I-7** | (2S,4R)-4-hydroxy-1-((R)-3-methyl-2-(3-(4-oxopiperidin-1-yl)isoxazol-5-yl)butanoyl)-N-((S)-1-(4-(4-methylthiazol-5-yl)phenyl)ethyl)pyrrolidine-2-carboxamide | 580 |

### Preparation of methyl 2-[3-(2-chloropyrimidin-5-yl)-1,2-oxazol-5-yl]-3-methylbutanoate.

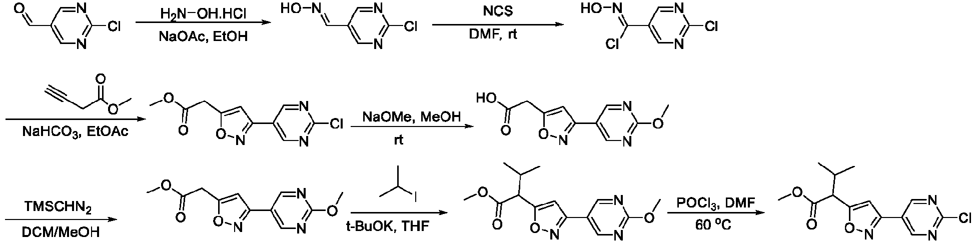

### Step 1: Preparation of (E)-N-[(2-chloropyrimidin-5-yl)methylidene]hydroxylamine.

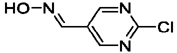

To a stirred solution of 2-chloropyrimidine-5-carbaldehyde (5 g, 35.078 mmol, 1 equiv) and NH₂OH HCl (4.93 g, 70.945 mmol, 2.02 equiv) in EtOH (250 mL) was added NaOAc (14.48 g, 176.512 mmol, 5.03 equiv) at room temperature. The resulting mixture was stirred for 2 h at room temperature. The solvent was removed under reduced pressure. The residue was dissolved in EtOAc (500 mL), washed with brine (500 mL), and dried over anhydrous Na₂SO₄. After filtration, the filtrate was concentrated under reduced pressure to afford (E)-N-[(2-chloropyrimidin-5-yl)methylidene]hydroxylamine (4.6 g, crude product) as a light yellow solid which was used in the next step directly without further purification. LCMS (ESI) m/z: [M+H]⁺ = 158.

### Step 2: Preparation of (Z)-2-chloro-N-hydroxypyrimidine-5-carbonimidoyl chloride.

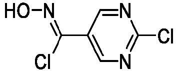

A solution of (E)-N-[(2-chloropyrimidin-5-yl)methylidene]hydroxylamine (4.6 g, 29.195 mmol, 1 equiv) and NCS (4.4 g, 32.951 mmol, 1.13 equiv) in DMF (150 mL) was stirred for 2 h at room temperature. The mixture was diluted with EtOAc (500 mL). The resulting mixture was washed with water (3 x 300 mL), brine (1 x 300 mL) and the organic phase was dried over anhydrous Na₂SO₄. After filtration, the filtrate was concentrated under reduced pressure to afford (Z)-2-chloro-N-hydroxypyrimidine-5-carbonimidoyl chloride (4.8 g, crude product) as a yellow solid. LCMS (ESI) m/z: [M+H]⁺ = 192.

### Step 3: Preparation of methyl 2-[3-(2-chloropyrimidin-5-yl)-1,2-oxazol-5-yl]acetate.

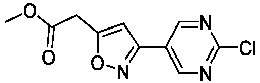

A solution of (Z)-2-chloro-N-hydroxypyrimidine-5-carbonimidoyl chloride (4.8 g, 25.00 mmol, 1 equiv) in EtOAc (80 mL) was treated with NaHCO₃ (3 g, 35.712 mmol, 1.43 equiv) for 30 min at 0 °C under an atmosphere of dry nitrogen followed by the addition of methyl but-3-ynoate (2.02 g, 20.591 mmol, 0.82 equiv) in portions at 0 °C. The resulting mixture was stirred for 12 h at room temperature. The resulting mixture was diluted with water (150 mL) and extracted with EtOAc (2 x 400 mL). The combined organic layers were washed with brine (1 x 400 mL) and dried over anhydrous Na₂SO₄. After filtration, the filtrate was concentrated under reduced pressure. The residue was purified by silica gel column chromatography, eluted with PE/EA (3:1) to afford methyl 2-[3-(2-chloropyrimidin-5-yl)-1,2-oxazol-5-yl]acetate (2.5 g, 38.64%) as a light yellow solid. LCMS (ESI) m/z: [M+H]⁺ = 254.

### Step 4: Preparation of [3-(2-methoxypyrimidin-5-yl)-1,2-oxazol-5-yl]acetic acid.

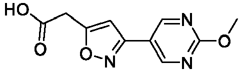

A solution of methyl 2-[3-(2-chloropyrimidin-5-yl)-1,2-oxazol-5-yl]acetate (3 g, 11.828 mmol, 1 equiv) and NaOMe (1.92 g, 35.484 mmol, 3.00 equiv) in MeOH (50 mL) was stirred for 1 h at room temperature under an atmosphere of dry nitrogen. The mixture was acidified to pH 6 with HCl (aq.). The residue was dissolved in EtOAc (300 mL). The resulting mixture was washed with water (2 x 300 mL). The combined organic layers were washed with brine (1 x 300 mL) and dried over anhydrous Na₂SO₄. After filtration, the filtrate was concentrated under reduced pressure to afford [3-(2-methoxypyrimidin-5-yl)-1,2-oxazol-5-yl]acetic acid (2.5 g, crude product) as a light yellow solid which was used in the next step directly without further purification. LCMS (ESI) m/z: [M+H]⁺ = 236.

### Step 5: Preparation of methyl 2-[3-(2-methoxypyrimidin-5-yl)-1,2-oxazol-5-yl]acetate

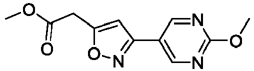

A solution of [3-(2-methoxypyrimidin-5-yl)-1,2-oxazol-5-yl]acetic acid (2.4 g, 10.204 mmol, 1 equiv) and (trimethylsilyl)diazomethane (2.33 g, 20.408 mmol, 2 equiv) in DCM (20 mL) and MeOH (5 mL) was stirred for 30 min at room temperature. The resulting mixture was concentrated under reduced pressure. The residue was purified by silica gel column chromatography, eluted with PE/EA (3:1) to afford methyl 2-[3-(2-methoxypyrimidin-5-yl)-1,2-oxazol-5-yl]acetate (1.2 g, 45.77%) as a white solid. LCMS (ESI) m/z: [M+H]⁺ = 250.

### Step 6: Preparation of methyl 2-[3-(2-methoxypyrimidin-5-yl)-1,2-oxazol-5-yl]-3-methylbutanoate.

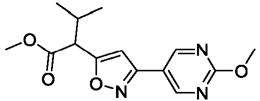

A solution of methyl 2-[3-(2-methoxypyrimidin-5-yl)-1,2-oxazol-5-yl]acetate (2.5 g, 10.031 mmol, 1 equiv) in THF (20 mL) was treated with t-BuOK (1.2 g, 10.694 mmol, 1.07 equiv) for 30 min at 0 °C under an atmosphere of dry nitrogen followed by the addition of 2-iodopropane (1.5 g, 8.824 mmol, 0.88 equiv) dropwise at 0 °C. The resulting mixture was stirred for 12 h at room temperature. The mixture was acidified to pH 6 with HCl (aq.). The resulting mixture was extracted with EtOAc (2 x 200 mL). The combined organic layers were washed with brine (2 x 200 mL) and dried over anhydrous Na₂SO₄. After filtration, the filtrate was concentrated under reduced pressure. The residue was purified by silica gel column chromatography, eluted with PE/EA (3:1) to afford methyl 2-[3-(2-methoxypyrimidin-5-yl)-1,2-oxazol-5-yl]-3-methylbutanoate (310 mg, 10.08%) as a yellow oil. LCMS (ESI) m/z: [M+H]⁺ = 292.

### Step 7: Preparation of methyl 2-[3-(2-chloropyrimidin-5-yl)-1,2-oxazol-5-yl]-3-methylbutanoate.

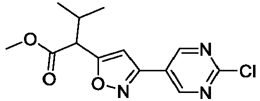

A solution of methyl 2-[3-(2-methoxypyrimidin-5-yl)-1,2-oxazol-5-yl]-3-methylbutanoate (200 mg, 0.687 mmol, 1 equiv) and POCl₃ (1.9 mL, 20.61 mmol, 30 equiv) in DMF (1.5 mL) was stirred for 3 h at 60 °C under an atmosphere of dry nitrogen. The residue was dissolved in EtOAc (100 mL). The resulting mixture was washed with brine (2 x 100 mL) and dried over anhydrous Na₂SO₄. After filtration, the filtrate was concentrated under reduced pressure to afford methyl 2-[3-(2-chloropyrimidin-5-yl)-1,2-oxazol-5-yl]-3-methylbutanoate (160 mg, crude product) as a brown oil which was used in the next step directly without further purification. LCMS (ESI) m/z: [M+H]⁺ = 296.

### Preparation of 2-((5-((R)-1-((2S,4R)-4-hydroxy-2-(((S)-1-(4-(2-methylthiazol-5-yl)phenyl)ethyl)carbamoyl)pyrrolidin-1-yl)-3-methyl-1-oxobutan-2-yl)isoxazol-3-yl)oxy)acetic acid (1-8)

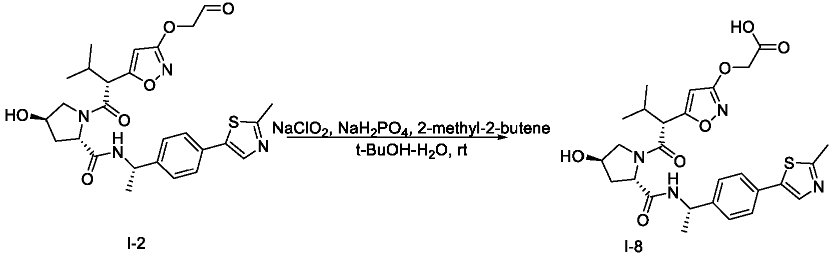

To a stirred solution of (2S,4R)-4-hydroxy-N-[(1S)-1-[4-(2-methyl-1,3-thiazol-5-yl)phenyl]ethyl]-1-[(2R)-3-methyl-2-[3-(2-oxoethoxy)-1,2-oxazol-5-yl]butanoyl]pyrrolidine-2-carboxamide (30.00 mg, 0.055 mmol, 1.00 equiv) and 2-methyl-2-butene (0.78 mg, 0.011 mmol, 0.20 equiv) in tert-butanol (2 mL) was added dropwise a solution of NaClO₂ (50.19 mg, 0.550 mmol, 10.00 equiv) and NaH₂PO₄ (78.77 mg, 0.550 mmol, 10.00 equiv) in water (2.00 mL) at 0 °C. The mixture was stirred at 0 °C for 0.5 h, then warmed up to room temperature and stirred for 1.5 h. The reaction was quenched by addition of a mixture of saturated Na₂S₂O₃ solution and brine, extracted with CHCl₃ (20 mL x 3). The combined organic extracts were dried over Na₂SO₄, filtered, concentrated in vacuo and purified by silica gel chromatography (PE/EtOAc = 1:1 to 1:3). This provided intermediate **I-8** (15.80 mg, 49.93%) as a colorless oil. LCMS (ESI) m/z: [M+H]⁺ = 557.

### Preparation of (2S,4R)-4-hydroxy-1-[(2R)-2-[3-(2-{3-[3-(2-hydroxyphenyl)cinnolin-6-yl]azetidin-1-yl}ethoxy)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 1).

### Preparation of 2-[6-(azetidin-3-yl)cinnolin-3-yl]phenol (Intermediate 1-9).

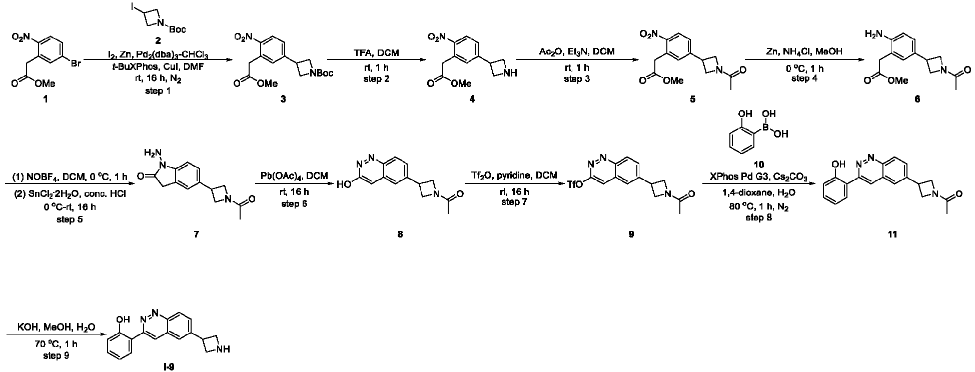

### Step 1: Preparation of tert-butyl 3-[3-(2-methoxy-2-oxoethyl)-4-nitrophenyl]azetidine-1-carboxylate (Intermediate 3).

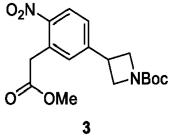

To a stirred solution of tert-butyl 3-iodoazetidine-1-carboxylate (10.33 g, 36.488 mmol, 2.00 equiv) in DMF (10.00 mL) were added I₂ (2.32 g, 9.122 mmol, 0.50 equiv) and Zn (3.58 g, 54.732 mmol, 3.00 equiv) at 0 °C (= Solution A). The resulting mixture was stirred for 1 h at 0 °C under nitrogen atmosphere. To a stirred solution of methyl 2-(5-bromo-2-nitrophenyl)acetate (5.00 g, 18.244 mmol, 1.00 equiv) in DMF (10.00 mL) were added Pd₂(dba)₃-CHCl₃ (1.89 g, 1.824 mmol, 0.10 equiv), *t*-BuXPhos (0.77 g, 1.824 mmol, 0.10 equiv) and Cul (0.35 g, 1.824 mmol, 0.10 equiv) at room temperature (= Solution B). To Solution A was added Solution B at 0 °C under nitrogen atmosphere. The resulting mixture was stirred for 16 h at room temperature under nitrogen atmosphere. The reaction was quenched with water at 0 °C. The resulting mixture was extracted with EtOAc (3 x 200 mL). The combined organic layers were washed with brine (200 mL) then dried over anhydrous Na₂SO₄. After filtration, the filtrate was concentrated under reduced pressure. The residue was purified by silica gel column chromatography, eluted with PE/EtOAc (2/1) to afford Intermediate **3** (4.1 g, 64.14%) as a brown oil. LCMS (ESI) m/z: [M+H]⁺ = 351.

### Step 2: Preparation of methyl 2-[5-(azetidin-3-yl)-2-nitrophenyl]acetate (Intermediate 4).

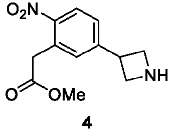

To a stirred solution of Intermediate **3** (4.10 g, 11.416 mmol, 1.00 equiv) in DCM (32.00 mL) was added TFA (8.00 mL) at room temperature. The resulting mixture was stirred for 1 h at room temperature. The resulting mixture was concentrated under reduced pressure. This resulted in Intermediate **4** (3.0 g, crude) as a brown oil. LCMS (ESI) m/z: [M+H]⁺ = 251.

### Step 3: Preparation of methyl 2-[5-(1-acetylazetidin-3-yl)-2-nitrophenyl]acetate (Intermediate 5).

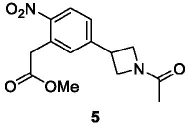

To a stirred solution of Intermediate **4** (3.00 g, 11.988 mmol, 1.00 equiv) in DCM (30.00 mL) were added Ac₂O (3.67 g, 35.964 mmol, 3.00 equiv) and Et₃N (3.64 g, 35.964 mmol, 3.00 equiv) at room temperature. The resulting mixture was stirred for 1 h at room temperature. The resulting mixture was concentrated under reduced pressure. The residue was purified by silica gel column chromatography, eluted with DCM/MeOH (10/1) to afford Intermediate **5** (4.5 g, >100%) as a yellow oil. LCMS (ESI) m/z: [M+H]⁺ = 293.

### Step 4: Preparation of methyl 2-[5-(1-acetylazetidin-3-yl)-2-aminophenyl]acetate (Intermediate 6).

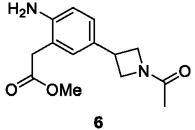

To a stirred solution of Intermediate **5** (4.50 g, 15.396 mmol, 1.00 equiv) in MeOH (30.00 mL) were added NH₄Cl (8.24 g, 153.960 mmol, 10.00 equiv) and Zn (10.07 g, 153.960 mmol, 10.00 equiv) at 0 °C. The resulting mixture was stirred for 1 h at 0 °C. The resulting mixture was filtered, the filter cake was washed with MeOH and the filtrate was concentrated under reduced pressure. To the residue was added water (100 mL) and the product was extracted with DCM (2 x 100 mL). The combined organic layers were dried over anhydrous Na₂SO₄. After filtration, the filtrate was concentrated under reduced pressure. This resulted in Intermediate **6** (2.1 g, 52.00%) as a yellow oil. LCMS (ESI) m/z: [M+H]⁺ = 263.

### Step 5: Preparation of 5-(1-acetylazetidin-3-yl)-1-amino-3H-indol-2-one (Intermediate 7).

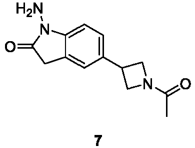

To a stirred solution of Intermediate **6** (2.10 g, 8.006 mmol, 1.00 equiv) in DCM (34.00 mL) was added NOBF₄ (1.87 g, 16.012 mmol, 2.00 equiv) at 0 °C. The resulting mixture was stirred for 1 h at 0 °C. To the above mixture was added SnCl₂·2H₂O (18.23 g, 80.060 mmol, 10.00 equiv) and conc. HCl (68.00 mL) at 0 °C. The resulting mixture was stirred for 16 h at room temperature. The resulting mixture was concentrated under reduced pressure. The residue was purified by reverse phase flash chromatography with the following conditions: column, C18 silica gel; mobile phase, MeCN in water, 0 to 100% gradient in 30 min; detector, UV 254/220 nm. This resulted in Intermediate **7** (1.6 g, 81.48%) as a brown oil. LCMS (ESI) m/z: [M+H]⁺ = 246.

### Step 6: Preparation of 1-[3-(3-hydroxycinnolin-6-yl)azetidin-1-yl]ethanone (Intermediate 8).

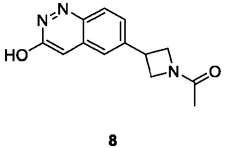

To a stirred solution of Intermediate **7** (1.60 g, 5.708 mmol, 1.00 equiv) in DCM (10.00 mL) was added Pb(OAc)₄ (3.80 g, 8.562 mmol, 1.50 equiv) at 0 °C. The resulting mixture was stirred for 16 h at room temperature. The reaction was quenched with MeOH at 0 °C. The resulting mixture was concentrated under reduced pressure. The residue was purified by reverse phase flash chromatography with the following conditions: column, C18 silica gel; mobile phase, MeCN in water, 0 to 100% gradient in 30 min; detector, UV 254/220 nm. This resulted in Intermediate **8** (880 mg, 63.38%) as a green oil. LCMS (ESI) m/z: [M+H]⁺ = 244.

### Step 7: Preparation of 6-(1-acetylazetidin-3-yl)cinnolin-3-yl trifluoromethanesulfonate (Intermediate 9).

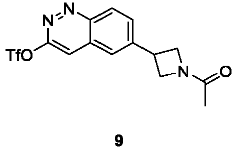

To a stirred solution of Intermediate **8** (880.00 mg, 3.617 mmol, 1.00 equiv) in DCM (20.00 mL) were added Tf₂O (10.21 g, 36.170 mmol, 10.00 equiv) and pyridine (2.86 g, 36.170 mmol, 10.00 equiv) at 0 °C. The resulting mixture was stirred for 16 h at room temperature. The resulting mixture was concentrated under reduced pressure. The residue was purified by reverse phase flash chromatography with the following conditions: column, C18 silica gel; mobile phase, MeCN in water (0.1% FA), 0 to 100% gradient in 30 min; detector, UV 254/220 nm. This resulted in Intermediate **9** (415 mg, 30.57%) as a brown oil. LCMS (ESI) m/z: [M+H]⁺ = 376.

### Step 8: Preparation of 1-{3-[3-(2-hydroxyphenyl)cinnolin-6-yl]azetidin-1-yl}ethanone (Intermediate 11).

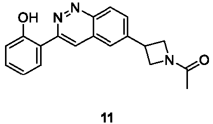

To a stirred solution of Intermediate **9** (415.00 mg, 1.106 mmol, 1.00 equiv) and 2-hydroxyphenylboronic acid (458.16 mg, 3.320 mmol, 3.00 equiv) in 1,4-dioxane (8.00 mL) and H₂O (2.00 mL) were added XPhos Pd G3 (187.13 mg, 0.221 mmol, 0.20 equiv) and Cs₂CO₃ (1.082 g, 3.320 mmol, 3.00 equiv) at room temperature. The resulting mixture was stirred for 1 h at 80 °C under nitrogen atmosphere. The mixture was allowed to cool down to room temperature and was concentrated under reduced pressure. The residue was purified by reverse phase flash chromatography with the following conditions: column, C18 silica gel; mobile phase, MeCN in water (0.1% FA), 0 to 100% gradient in 30 min; detector, UV 254/220 nm. This resulted in Intermediate **11** (254 mg, 71.95%) as a yellow oil. LCMS (ESI) m/z: [M+H]⁺ = 320.

### Step 9: Preparation of 2-[6-(azetidin-3-yl)cinnolin-3-yl]phenol (I-9).

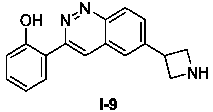

To a stirred solution of Intermediate **11** (254.00 mg, 0.794 mmol, 1.00 equiv) in MeOH (5.00 mL) and H₂O (5.00 mL) was added KOH (133.39 mg, 2.382 mmol, 3.00 equiv) at room temperature. The resulting mixture was stirred for 1 h at 70 °C. The mixture was allowed to cool down to room temperature and was concentrated under reduced pressure. The residue was purified by reverse phase flash chromatography with the following conditions: column, C18 silica gel; mobile phase, MeCN in water (10 mmol/L NH₄HCO₃), 0 to 100% gradient in 30 min; detector, UV 254/220 nm. This resulted in I-9 (134 mg, 60.91%) as a white solid. LCMS (ESI) m/z: [M+H]⁺ = 278.

### Preparation of (2S,4R)-4-hydroxy-1-[(2R)-2-[3-(2-{3-[3-(2-hydroxyphenyl)cinnolin-6-yl]azetidin-1-yl}ethoxy)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 1).

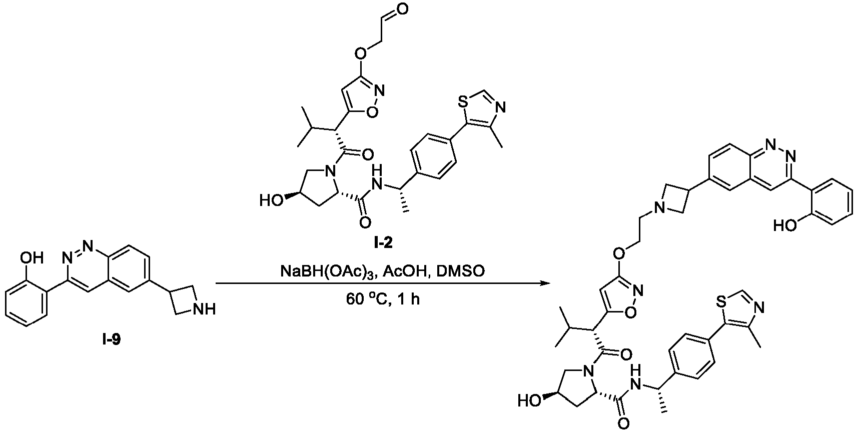

To a stirred solution of (2S,4R)-4-hydroxy-N-[(1S)-1-[4-(4-methyl-1 ,3-thiazol-5-yl)phenyl]ethyl]-1-[(2R)-3-methyl-2-[3-(2-oxoethoxy)-1,2-oxazol-5-yl]butanoyl]pyrrolidine-2-carboxamide (29.24 mg, 0.054 mmol, 1.00 equiv) in DMSO (1.00 mL) was added IO-9 (15.00 mg, 0.054 mmol, 1.00 equiv) at room temperature. The resulting mixture was stirred for 30 min at room temperature. To the above mixture were added NaBH(OAc)₃ (34.39 mg, 0.162 mmol, 3.00 equiv) and AcOH (cat.) at room temperature. The resulting mixture was stirred for 1 h at 60 °C. The mixture was allowed to cool down to room temperature. The reaction was quenched with water at 0 °C and then purified by Chiral-Prep-HPLC with the following conditions: Column, Xselect CSH F-Phenyl OBD column, 19*250 mm, 5 µm; mobile phase, water (0.05% FA) and MeOH (43% MeOH up to 67% in 7 min); Detector, UV 254/220 nm. This resulted in **Compound 1** (6.8 mg, 15.50%) as a white solid. ¹H NMR (400 MHz, DMSO-d6) *δ* 12.16 - 11.87 (m, 1H), 8.98 (d, *J* = 2.1 Hz, 1H), 8.87 (d, *J* = 8.2 Hz, 1H), 8.47 - 8.39 (m, 2H), 8.30 - 8.15 (m, 1H, FA), 8.14 - 8.06 (m, 1H), 8.04 - 7.94 (m, 2H), 7.47 - 7.41 (m, 2H), 7.41 - 7.31 (m, 3H), 7.09 - 7.00 (m, 2H), 6.10 (s, 1H), 5.25 - 4.97 (m, 1H), 4.97 - 4.85 (m, 1H), 4.37 (t, *J* = 7.9 Hz, 1H), 4.31 - 4.23 (m, 1H), 4.18 (t, *J* = 5.4 Hz, 2H), 3.97 - 3.86 (m, 1H), 3.79 (t, *J* = 7.3 Hz, 2H), 3.73 - 3.62 (m, 2H), 3.59 - 3.42 (m, 3H), 2.86 (t, *J* = 5.4 Hz, 2H), 2.45 (d, *J* = 6.6 Hz, 3H), 2.30 - 2.13 (m, 1H), 2.08 - 1.98 (m, 1H), 1.82 - 1.72 (m, 1H), 1.37 (d, *J* = 7.0 Hz, 3H), 0.96 (d, *J* = 6.6 Hz, 3H), 0.81 (d, *J* = 6.6 Hz, 3H). LCMS (ESI) m/z: [M+H]⁺ = 802.40.

The compounds in Table 3 were prepared using procedures similar to those used above for the preparation of **Compound 1** using the appropriate amine and aldehyde (or ketone).

**Table 3.**

| **No.** | **Name** | **LCMS (ESI) m/z** | **¹H NMR** |
|---|---|---|---|
| **Compound 2** | (2S,4R)-4-hydroxy-1-((R)-2-(3-(4-((3-(3-(2-hydroxyphenyl)cinnolin-6-yl)azetidin-1-yl)methyl)piperidin-1-yl)isoxazol-5-yl)-3-methylbutanoyl)-N-((S)-1-(4-(4-methylthiazol-5-yl)phenyl)ethyl)pyrrolidine-2-carboxamide | 855.80 | 1H NMR (400 MHz, DMSO-d6) δ 12.10 - 11.94 (m, 1H), 8.99 (s, 1H), 8.88 (s, 1H), 8.48 - 8.26 (m, 2H), 8.11 (dd, J = 7.9, 1.7 Hz, 1H), 8.04 - 7.99 (m, 2H), 7.49 - 7.32 (m, 5H), 7.10 - 7.00 (m, 2H), 6.12 (s, 1H), 5.11 (d, J = 3.6 Hz, 1H), 4.97 - 4.86 (m, 1H), 4.36 (t, J = 7.9 Hz, 1H), 4.32 - 4.24 (m, 1H), 3.93 - 3.83 (m, 1H), 3.75 - 3.67 (m, 3H), 3.67 - 3.49 (m, 4H), 3.46 - 3.39 (m, 1H), 3.28 - 3.22 (m, 3H), 2.80 - 2.69 (m, 2H), 2.46 (s, 3H), 2.39 (d, J = 6.8 Hz, 2H), 2.28 - 2.12 (m, 1H), 2.07 - 1.95 (m, 1H), 1.84 - 1.70 (m, 3H), 1.56 - 1.47 (m, 1H), 1.38 (d, J = 7.0 Hz, 3H), 0.96 (t, J = 6.6 Hz, 3H), 0.81 (d, J = 6.6 Hz, 3H). |
| **Compound 3** | (2S,4R)-4-hydroxy-1-((R)-2-(3-(4-(3-(3-(2-hydroxyphenyl)cinnolin-6-yl)azetidin-1-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 841.80 | 1H NMR (400 MHz, DMSO-d6) δ 12.04 - 11.95 (m, 1H), 8.99 (d, J = 2.1 Hz, 1H), 8.88 (s, 1H), 8.43 (dd, J = 18.1, 8.3 Hz, 2H), 8.11 (d, J = 7.9 Hz, 1H), 8.02 (d, J = 8.5 Hz, 2H), 7.48 - 7.41 (m, 2H), 7.41 - 7.33 (m, 3H), 7.09 - 7.01 (m, 2H), 6.15 (s, 1H), 5.11 (d, J = 3.7 Hz, 1H), 4.96 - 4.86 (m, 1H), 4.37 (t, J = 7.8 Hz, 1H), 4.32 - 4.24 (m, 1H), 3.92 - 3.82 (m, 1H), 3.76 - 3.67 (m, 3H), 3.61 - 3.48 (m, 4H), 3.47 - 3.39 (m, 1H), 3.30 - 3.23 (m, 3H), 2.95 - 2.82 (m, 2H), 2.46 (s, 3H), 2.38 - 2.34 (m, 1H), 2.28 - 2.11 (m, 1H), 2.07 - 1.98 (m, 1H), 1.84 - 1.67 (m, 3H), 1.38 (d, J = 7.1 Hz, 3H), 0.96 (t, J = 6.6 Hz, 3H), 0.82 (d, J = 6.6 Hz, 3H). |

### Preparation of (2S,4R)-4-hydroxy-1-[(2R)-2-[3-(2-{3-[3-(2-hydroxyphenyl)cinnolin-6-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 4) and (2S,4R)-4-hydroxy-1-[(2S)-2-[3-(2-{3-[3-(2-hydroxyphenyl)cinnolin-6-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 5).

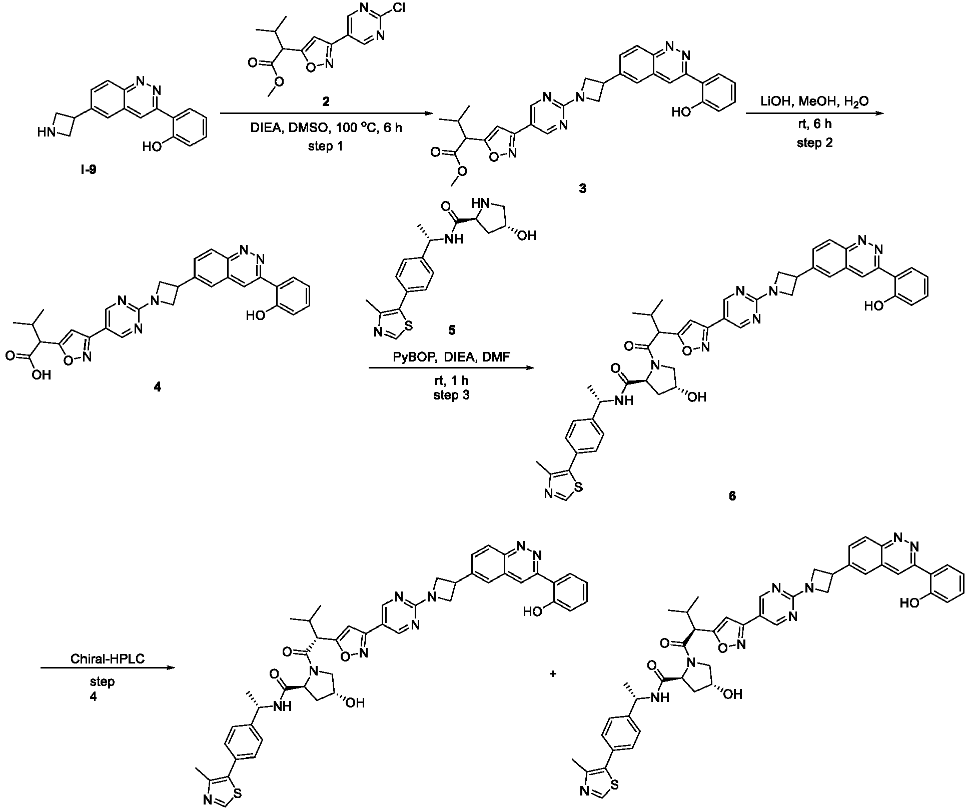

### Step 1: Preparation of methyl 2-[3-(2-{3-[3-(2-hydroxyphenyl)cinnolin-6-yl]azetidin-1-yl}pyrimidin-5-yl)-1,2-oxazol-5-yl]-3-methylbutanoate (Intermediate 3).

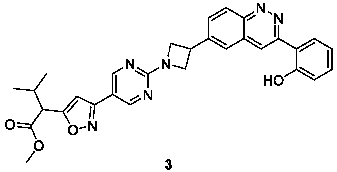

To a stirred solution of I- (50.00 mg, 0.180 mmol, 1.00 equiv) in DMSO (2.00 mL) were added methyl 2-[3-(2-chloropyrimidin-5-yl)-1,2-oxazol-5-yl]-3-methylbutanoate (53.32 mg, 0.180 mmol, 1.00 equiv) and DIEA (69.91 mg, 0.540 mmol, 3.00 equiv) at room temperature. The resulting mixture was stirred for 6 h at 100 °C. The mixture was allowed to cool down to room temperature and was purified by reverse phase flash chromatography with the following conditions: column, C18 silica gel; mobile phase, MeCN in water (0.1% FA), 0 to 100% gradient in 30 min; detector, UV 254/220 nm. This resulted in Intermediate **3** (20 mg, 20.67%) as a yellow solid. LCMS (ESI) m/z: [M+H]⁺ = 537.

### Step 2: Preparation of 2-[3-(2-{3-[3-(2-hydroxyphenyl)cinnolin-6-yl]azetidin-1-yl}pyrimidin-5-yl)-1,2-oxazol-5-yl]-3-methylbutanoic acid (Intermediate 4).

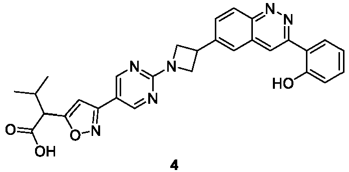

To a stirred solution of Intermediate **3** (20.00 mg, 0.037 mmol, 1.00 equiv) in MeOH (1.00 mL) and H₂O (1.00 mL) was added LiOH·H₂O (4.69 mg, 0.111 mmol, 3.00 equiv) at room temperature. The resulting mixture was stirred for 6 h at room temperature. The mixture was acidified to pH 3 with aqueous HCl (1 M). The resulting mixture was concentrated under reduced pressure. This resulted in Intermediate **4** (20 mg, crude) as a yellow solid. LCMS (ESI) m/z: [M+H]⁺ = 523.

### Step 3: Preparation of (2S,4R)-4-hydroxy-1-{2-[3-(2-{3-[3-(2-hydroxyphenyl)cinnolin-6-yl]azetidin-1-yl}pyrimidin-5-yl)-1,2-oxazol-5-yl]-3-methylbutanoyl}-N-[(1S)-1-[4-(4-methyl-1,3-thiazol-5-yl)phenyl]ethyl]pyrrolidine-2-carboxamide (Intermediate 6).

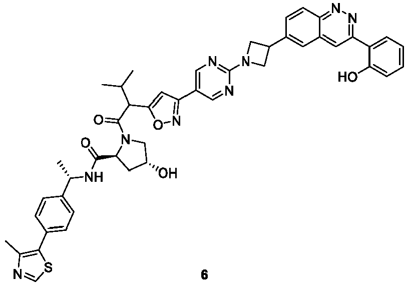

To a stirred solution of Intermediate **4** (20.00 mg, 0.038 mmol, 1.00 equiv) in DMF (1.00 mL) were added PyBOP (59.28 mg, 0.114 mmol, 3 equiv) and DIEA (24.51 mg, 0.190 mmol, 5 equiv) at room temperature. To the above mixture was added (2S,4R)-4-hydroxy-N-[(1S)-1-[4-(4-methyl-1,3-thiazol-5-yl)phenyl]ethyl]pyrrolidine-2-carboxamide (12.62 mg, 0.038 mmol, 1 equiv) at room temperature. The resulting mixture was stirred for 1 h at room temperature. The mixture was purified by reverse phase flash chromatography with the following conditions: column, C18 silica gel; mobile phase, MeCN in H₂O (10 mmol/L NH₄HCO₃), 0 to 100% gradient in 30 min; detector, UV 254/220 nm. This resulted in Intermediate **6** (5 mg, 15.62%) as an off-white solid. LCMS (ESI) m/z: [M+H]⁺ = 836.

### Step 4: Preparation of (2S,4R)-4-hydroxy-1-[(2R)-2-[3-(2-{3-[3-(2-hydroxyphenyl)cinnolin-6-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 4) and (2S,4R)-4-hydroxy-1-[(2S)-2-[3-(2-{3-[3-(2-hydroxyphenyl)cinnolin-6-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 5).

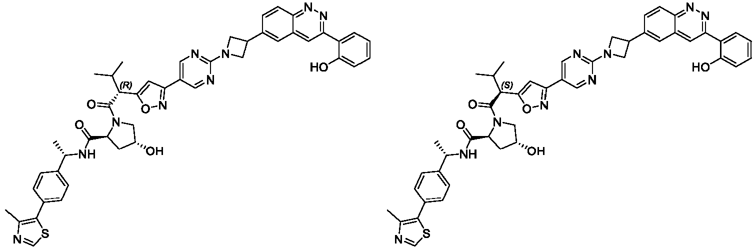

Intermediate **6** (5.00 mg) was purified by Chiral-Prep-HPLC with the following conditions: Column, CHIRALPAK ID, 2*25 cm, 5 mm; mobile phase, MtBE (10 mM NH₃-MeOH) and MeOH (hold 50% MeOH in 29 min); Detector, UV 254/220 nm. This resulted in:
**Compound 4** (1.0 mg, 20.00%) as a white solid. ¹H NMR (400 MHz, Methanol-d4) δ 8.89 - 8.77 (m, 4H), 8.48 (d, *J* = 8.9 Hz, 1H), 8.12 - 8.02 (m, 3H), 7.48 - 7.34 (m, 5H), 7.07 - 7.01 (m, 2H), 6.80 (s, 1H), 5.08 - 5.00 (m, 1H), 4.74 (d, *J* = 8.6 Hz, 2H), 4.63 - 4.49 (m, 1H), 4.49 - 4.26 (m, 3H), 3.92 - 3.85 (m, 1H), 3.71 - 3.59 (m, 3H), 2.47 (d, *J* = 7.8 Hz, 3H), 2.23 - 2.14 (m, 1H), 2.09 - 1.87 (m, 2H),1.53 (d, *J* = 7.0 Hz, 3H), 1.10 (d, *J* = 6.6 Hz, 3H), 0.93 (d, *J* = 6.6 Hz, 3H). LCMS (ESI) m/z: [M+H]⁺ = 836.40.

**Compound 5** (0.6 mg, 12.00%) as a white solid. ¹H NMR (400 MHz, Methanol-d4) δ 8.89 - 8.82 (m, 2H), 8.82 - 8.75 (m, 2H), 8.48 (d, *J* = 8.9 Hz, 1H), 8.13 - 8.02 (m, 3H), 7.41 - 7.32 (m, 5H), 7.07 - 7.00 (m, 2H), 6.78 (s, 1H), 5.04 - 4.96 (m, 1H), 4.78 - 4.70 (m, 2H), 4.59 (d, *J* = 8.0 Hz, 1H), 4.47 - 4.28 (m, 3H), 4.00 - 3.86 (m, 1H), 3.79 - 3.68 (m, 1H), 3.63 (s, 2H), 2.44 (s, 3H), 2.29 - 2.11 (m, 1H), 2.06 - 1.86 (m, 2H), 1.49 (d, *J* = 7.0 Hz, 3H), 1.10 (d, *J* = 6.7 Hz, 3H), 0.95 (d, *J* = 6.7 Hz, 3H). LCMS (ESI) m/z: [M+H]⁺ = 836.50.

### Preparation of (2S,4R)-4-hydroxy-1-[(2R)-2-[3-(2-{3-[3-(2-hydroxyphenyl)cinnolin-6-yl]azetidin-1-yl}-2-oxoethoxy)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 6).

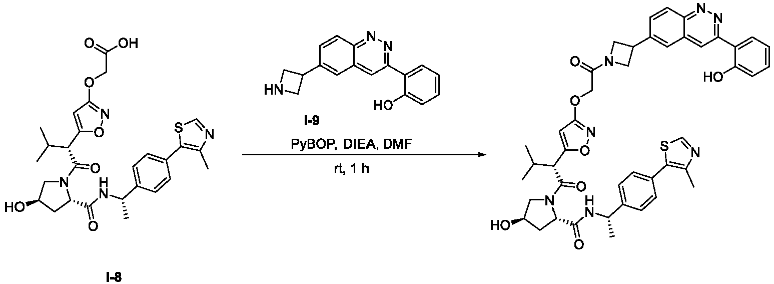

To a stirred solution of ({5-[(2R)-1-[(2S,4R)-4-hydroxy-2-{[(1S)-1-[4-(4-methyl-1,3-thiazol-5-yl)phenyl]ethyl]carbamoyl}pyrrolidin-1-yl]-3-methyl-1-oxobutan-2-yl]-1,2-oxazol-3-yl}oxy)acetic acid (30.11 mg, 0.054 mmol, 1.00 equiv) in DMF (1.00 mL) were added PyBOP (84.44 mg, 0.162 mmol, 3.00 equiv) and DIEA (34.95 mg, 0.270 mmol, 5.00 equiv) at room temperature. To the above mixture was added I-9 (15.00 mg, 0.054 mmol, 1.00 equiv) at room temperature. The resulting mixture was stirred for 1 h at room temperature. The mixture was purified by Prep-HPLC with the following conditions: Column, XBridge Shield RP18 OBD Column, 19*150 mm, 5 µm; mobile phase, water (10 mmol/L NH₄HCO₃) and CH₃CN (42% CH₃CN up to 55% in 7 min); Detector, UV 254/220 nm. This resulted in **Compound 6** (15.5 mg, 33.86%) as a white solid. ¹H NMR (400 MHz, DMSO-d6) *δ* 11.93 - 11.69 (m, 1H), 8.98 (s, 1H), 8.88 (s, 1H), 8.50 (d, *J =* 8.8 Hz, 1H), 8.43 (d, *J* = 7.7 Hz, 1H), 8.15 - 8.07 (m, 2H), 8.02 - 7.97 (m, 1H), 7.47 - 7.41 (m, 2H), 7.41 - 7.33 (m, 3H), 7.10 - 7.01 (m, 2H), 6.19 (d, *J =* 1.6 Hz, 1H), 5.11 (d, *J =* 3.7 Hz, 1H), 4.97 - 4.86 (m, 1H), 4.80 (s, 2H), 4.77 - 4.65 (m, 1H), 4.48 - 4.34 (m, 3H), 4.32 - 4.18 (m, 2H), 4.15 - 4.05 (m, 1H), 3.74 - 3.65 (m, 2H), 3.51 - 3.42 (m, 1H), 2.47 - 2.43 (m, 3H), 2.31 - 2.15 (m, 1H), 2.07 - 1.96 (m, 1H), 1.83 - 1.69 (m, 1H), 1.37 (d, *J* = 6.9 Hz, 3H), 0.96 (d, *J* = 6.6 Hz, 3H), 0.80 (d, *J* = 6.6 Hz, 3H). LCMS (ESI) m/z: [M+H]⁺ = 816.70.

The compound in Table 4 was prepared using a procedure similar to the one used above for the preparation of **Compound 6** using the appropriate amine and carboxylic acid.

Table 4.

| **No.** | **Name** | **LCMS (ESI) m/z** | **¹H NMR** |
|---|---|---|---|
| **Compound 7** | (2S,4R)-4-hydroxy-1-((R)-2-(3-(4-(3-(3-(2-hydroxyphenyl)cinnolin-6-yl)azetidine-1-carbonyl)piperidin-1-yl)isoxazol-5-yl)-3-methylbutanoyl)-N-((S)-1-(4-(4-methylthiazol-5-yl)phenyl)ethyl)pyrrolidine-2-carboxamide | 869.80 | 1H NMR (400 MHz, DMSO-d6) δ 11.83 (d, J = 4.4 Hz, 1H), 8.99 (d, J = 2.3 Hz, 1H), 8.88 (d, J = 1.0 Hz, 1H), 8.49 (d, J = 8.9 Hz, 1H), 8.41 (d, J = 7.7 Hz, 1H), 8.15 - 8.08 (m, 2H), 7.98 (dd, J = 8.9, 1.9 Hz, 1H), 7.49 - 7.41 (m, 2H), 7.41 - 7.34 (m, 3H), 7.10 - 7.02 (m, 2H), 6.16 (s, 1H), 5.11 (d, J = 3.8 Hz, 1H), 4.96 - 4.87 (m, 1H), 4.76 - 4.64 (m, 1H), 4.42 - 4.33 (m, 3H), 4.32 - 4.25 (m, 1H), 4.22 - 4.12 (m, 1H), 4.06 - 3.97 (m, 1H), 3.76 - 3.63 (m, 3H), 3.58 (d, J = 9.9 Hz, 1H), 3.47 - 3.40 (m, 1H), 2.90 - 2.76 (m, 2H), 2.57 - 2.52 (m, 1H), 2.46 (s, 3H), 2.30 - 2.13 (m, 1H), 2.07 - 1.98 (m, 1H), 1.84 - 1.68 (m, 3H), 1.66 - 1.51 (m, 2H), 1.38 (d, J = 7.0 Hz, 3H), 0.96 (d, J = 6.7 Hz, 3H), 0.82 (d, J = 6.7 Hz, 3H). |

### Preparation of (2S,4R)-4-hydroxy-1-[(2R)-2-[3-(2-{3-[3-(2-hydroxyphenyl)cinnolin-7-yl]azetidin-1-yl}ethoxy)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 8).

### Preparation of 2-[7-(azetidin-3-yl)cinnolin-3-yl]phenol (I-10).

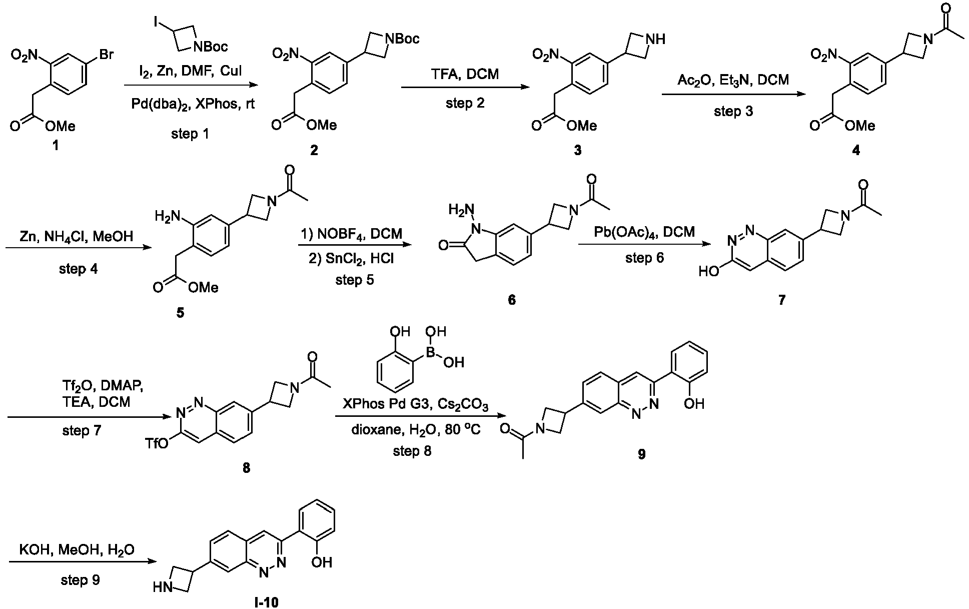

### Step 1: Preparation of tert-butyl 3-[4-(2-methoxy-2-oxoethyl)-3-nitrophenyl]azetidine-1-carboxylate (Intermediate 2).

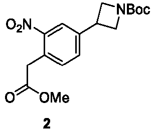

A mixture of tert-butyl 3-iodoazetidine-1-carboxylate (12.40 g, 43.784 mmol, 1.2 equiv) and I₂ (4.63 g, 18.244 mmol, 0.5 equiv) in DMF (100 mL) was allowed to cool down to 0 °C. Zn (7.16 g, 109.461 mmol, 3 equiv) was then added in portions at this temperature. The resulting mixture was stirred for 1 h at room temperature under nitrogen atmosphere. Then Cul (1.39 g, 7.297 mmol, 0.2 equiv), Pd(dba)₂ (4.20 g, 7.297 mmol, 0.2 equiv), XPhos (3.478 g, 7.297 mmol, 0.2 equiv) and methyl 2-(4-bromo-2-nitrophenyl)acetate (10 g, 36.487 mmol, 1 equiv) were added. The mixture was stirred for 5 h at room temperature under nitrogen atmosphere. The resulting mixture was filtered through a pad of Celite and the filter cake was washed with EtOAc (3 x 200 mL). The filtrate was washed with water (3 x 300 mL). The organic layer was dried over anhydrous Na₂SO₄. After filtration, the filtrate was concentrated under reduced pressure. The residue was purified by silica gel column chromatography, eluted with PE/EA (3:1), to afford Intermediate **2** (5 g, 35.20%) as a yellow oil. LCMS (ESI) m/z: [M+H]⁺ = 351.

### Step 2: Preparation of methyl 2-[4-(azetidin-3-yl)-2-nitrophenyl]acetate (Intermediate 3).

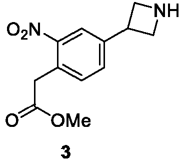

To a stirred mixture of Intermediate **2** (5 g, 14.271 mmol, 1 equiv) in DCM (15 mL) was added TFA (5 mL) dropwise. The resulting mixture was stirred for 1 h at room temperature and then was concentrated under reduced pressure. This resulted in Intermediate 3 (5.5 g, 95.22%) as a yellow oil. The crude product was used in the next step directly without further purification. LCMS (ESI) m/z: [M+H]⁺ = 251.

### Step 3: Preparation of methyl 2-[4-(1-acetylazetidin-3-yl)-2-nitrophenyl]acetate (Intermediate 4).

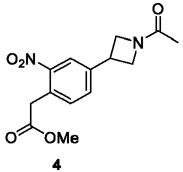

To a stirred mixture of Intermediate **3** (5 g, 13.726 mmol, 1.00 equiv) and Et₃N (6.94 g, 68.630 mmol, 5 equiv) in DCM (20 mL) was added Ac₂O (2.10 g, 20.589 mmol, 1.5 equiv) dropwise. The resulting mixture was stirred for 1 h at room temperature and then was concentrated under reduced pressure. The residue was purified by silica gel column chromatography, eluted with CH₂Cl₂/MeOH (14:1), to afford Intermediate **4** (3.5 g, 78.52%) as a yellow oil. LCMS (ESI) m/z: [M+H]⁺ = 293.

### Step 4: Preparation of methyl 2-[4-(1-acetylazetidin-3-yl)-2-aminophenyl]acetate (Intermediate 5).

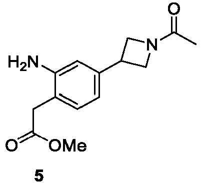

A mixture of Intermediate **4** (3.5 g, 11.974 mmol, 1 equiv) and NH₄Cl (12.81 g, 239.480 mmol, 20 equiv) in MeOH (50 mL) was allowed to cool down to 0 °C followed by the addition of Zn (7.83 g, 119.740 mmol, 10 equiv) in portions. The resulting mixture was stirred for 1 h at 0 °C. The resulting mixture was filtered through a pad of Celite and the filtrate was concentrated under reduced pressure. The residue was diluted with water (50 mL) and extracted with EtOAc (3 x 100 mL). The combined organic layers were dried over anhydrous Na₂SO₄. After filtration, the filtrate was concentrated under reduced pressure. This resulted in Intermediate **5** (2 g, 57.31%) as a yellow oil. The crude product was used in the next step directly without further purification. LCMS (ESI) m/z: [M+H]⁺ = 263.

### Step 5: Preparation of 6-(1-acetylazetidin-3-yl)-1-amino-3H-indol-2-one (Intermediate 6).

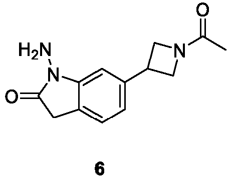

A mixture of Intermediate **5** (2 g, 7.625 mmol, 1 equiv) in DCM (40 mL) was allowed to cool down to 0 °C. Then NOBF₄ (1.34 g, 11.438 mmol, 1.5 equiv) was added in one portion. The resulting mixture was stirred for 1 h at 0 °C. To the above mixture was added SnCl₂ (11.69 g, 61.000 mmol, 8 equiv) in HCl (60 mL) dropwise at 0 °C. The resulting mixture was stirred overnight at room temperature and then was concentrated under reduced pressure. The residue was purified by reverse phase flash chromatography with the following conditions: column, C18 silica gel; mobile phase, MeCN in Water, 0% to 100% gradient in 30 min; detector, UV 254 nm. This resulted in Intermediate **6** (600 mg, 28.87%) as a yellow green solid. LCMS (ESI) m/z: [M+H]⁺ = 246.

### Step 6: Preparation of 1-[3-(3-hydroxycinnolin-7-yl)azetidin-1-yl]ethanone (Intermediate 7).

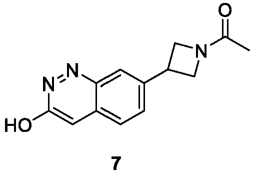

A mixture of Intermediate **6** (600 mg, 2.446 mmol, 1 equiv) in DCM (10 mL) was allowed to cool down to 0 °C. Then Pb(OAc)₄ (1301.55 mg, 2.935 mmol, 1.2 equiv) was added in portions. The resulting mixture was stirred for 1 h at 0 °C and then was concentrated under reduced pressure. The residue was purified by reverse phase flash chromatography with the following conditions: column, C18 silica gel; mobile phase, MeCN in Water, 0% to 100% gradient in 30 min; detector, UV 254 nm. This resulted in Intermediate 7 (400 mg, 60.50%) as a yellow solid. LCMS (ESI) m/z: [M+H]⁺ = 244.

### Step 7: Preparation of 7-(1-acetylazetidin-3-yl)cinnolin-3-yl trifluoromethanesulfonate (Intermediate 8).

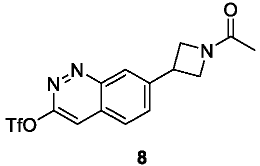

To a stirred mixture of Intermediate **7** (400 mg, 1.644 mmol, 1 equiv) and DMAP (40.18 mg, 0.329 mmol, 0.2 equiv) in DCM (10 mL) was added TEA (499.17 mg, 4.932 mmol, 3 equiv). The mixture was allowed to cool down to 0 °C. To the above mixture was added Tf₂O (695.86 mg, 2.466 mmol, 1.5 equiv) dropwise at 0 °C. The resulting mixture was stirred for an additional 1 h at 0 °C. The resulting mixture was diluted with water (50 mL) and was extracted with CH₂Cl₂ (3 x 100 mL). The combined organic layers were dried over anhydrous Na₂SO₄. After filtration, the filtrate was concentrated under reduced pressure. This resulted in Intermediate **8** (400 mg, 58.33%) as a yellow solid. The crude product was used in the next step directly without further purification. LCMS (ESI) m/z: [M+H]⁺ = 376.

### Step 8: Preparation of 1-{3-[3-(2-hydroxyphenyl)cinnolin-7-yl]azetidin-1-yl}ethanone (Intermediate 9).

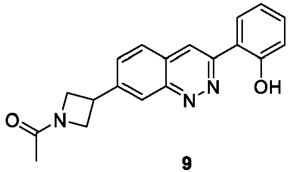

To a solution of Intermediate **8** (310 mg, 0.826 mmol, 1 equiv) and 2-hydroxyphenylboronic acid (341.77 mg, 2.478 mmol, 3 equiv) in dioxane (5 mL) and H₂O (1 mL) were added Cs₂CO₃ (807.34 mg, 2.478 mmol, 3 equiv) and XPhos Pd G3 (139.83 mg, 0.165 mmol, 0.2 equiv). After stirring for 1 h at 80 °C under nitrogen atmosphere, the resulting mixture was concentrated under reduced pressure. The residue was diluted with water (20 mL) and was extracted with EtOAc (3 x 20 mL). The combined organic layers were dried over anhydrous Na₂SO₄. After filtration, the filtrate was concentrated under reduced pressure. The residue was purified by reverse phase flash chromatography with the following conditions: column, C18 silica gel; mobile phase, MeCN in Water (0.1% FA), 0% to 100% gradient in 30 min; detector, UV 254 nm. This resulted in Intermediate **9** (100 mg, 34.12%) as a yellow solid. LCMS (ESI) m/z: [M+H]⁺ = 320.

### Step 9: Preparation of 2-[7-(azetidin-3-yl)cinnolin-3-yl]phenol (I-10).

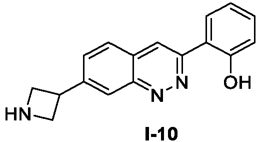

To a stirred mixture of Intermediate **9** (100 mg, 0.313 mmol, 1 equiv) in MeOH (3 mL) and H₂O (3 mL) was added KOH (175.68 mg, 3.130 mmol, 10 equiv) in portions at room temperature. The resulting mixture was stirred for 2 h at 70 °C and then was concentrated under reduced pressure. The residue was purified by reverse phase flash chromatography with the following conditions: column, C18 silica gel; mobile phase, MeCN in Water (0.1% FA), 0% to 100% gradient in 30 min; detector, UV 254 nm. This resulted in I-10 (92 mg, 95.35%) as a yellow solid. LCMS (ESI) m/z: [M+H]⁺ = 278.

### Preparation of (2S,4R)-4-hydroxy-1-[(2R)-2-[3-(2-{3-[3-(2-hydroxyphenyl)cinnolin-7-yl]azetidin-1-yl}ethoxy)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 8).

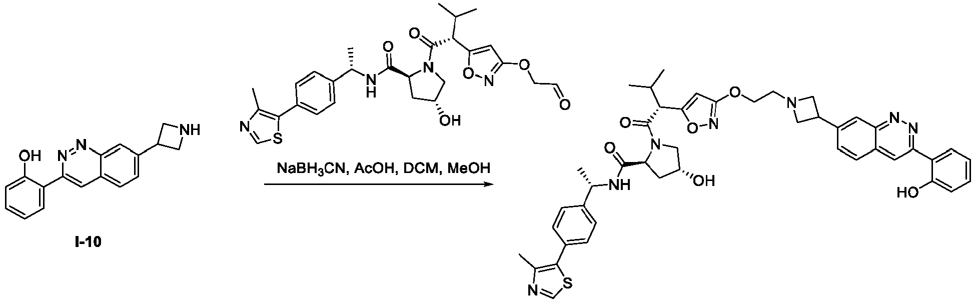

To a stirred solution of I-10 (16 mg, 0.058 mmol, 1 equiv) and (2S,4R)-4-hydroxy-N-[(1S)-1-[4-(4-methyl-1,3-thiazol-5-yl)phenyl]ethyl]-1-[(2R)-3-methyl-2-[3-(2-oxoethoxy)-1,2-oxazol-5-yl]butanoyl]pyrrolidine-2-carboxamide (31.19 mg, 0.058 mmol, 1 equiv) in DCM (1 mL) and MeOH (1 mL) were added AcOH (3.46 mg, 0.058 mmol, 1 equiv) and NaBH₃CN (10.88 mg, 0.174 mmol, 3 equiv). The resulting mixture was stirred overnight at room temperature under nitrogen atmosphere. The resulting mixture was concentrated under reduced pressure. The crude product was purified by Prep-HPLC with the following conditions: Column, Kinetex EVO C18 Column, 21.2*150 mm, 5 mm; Mobile Phase A: water (10 mmol/L NH₄HCO₃), Mobile Phase B: CH₃CN; Flow rate: 25 mL/min; Gradient: 35% B to 62% B in 7 min, then 62% B; Detector, UV 254/220 nm. This resulted in **Compound 8** (18.1 mg, 38.77%) as a light yellow solid. ¹H NMR (300 MHz, DMSO-d6) *δ* 12.03 (d, J = 6.3 Hz, 1H), 8.96 (d, J = 22.1 Hz, 2H), 8.48 - 8.32 (m, 2H), 8.13 (d, J = 8.5 Hz, 2H), 7.99 (d, J = 8.7 Hz, 1H), 7.50 - 7.30 (m, 5H), 7.13 - 6.99 (m, 2H), 6.03 (d, J = 50.8 Hz, 1H), 5.11 (d, J = 3.7 Hz, 1H), 4.92 (t, J = 7.1 Hz, 1H), 4.38 (t, J = 7.8 Hz, 1H), 4.29 (s, 1H), 4.19 (t, J = 5.4 Hz, 2H), 3.98 (t, J = 7.1 Hz, 1H), 3.83 (d, J = 7.2 Hz, 2H), 3.75 - 3.62 (m, 2H), 3.46 (d, J = 11.2 Hz, 3H), 2.89 (s, 2H), 2.46 (d, J = 2.7 Hz, 3H), 2.24 (s, 1H), 2.04 (t, J = 10.3 Hz, 1H), 1.86 - 1.69 (m, 1H), 1.42 (dd, J = 22.1, 7.0 Hz, 3H), 0.96 (d, J = 6.5 Hz, 3H), 0.89 - 0.73 (m, 3H). LCMS (ESI) m/z: [M+H]⁺ = 802.30.

The compounds in Table 5 were prepared using procedures similar to those used above for the preparation of **Compound 8** using the appropriate amine and aldehyde (or ketone).

**Table 5.**

| **No.** | **Name** | **LCMS (ESI) m/z** | **¹H NMR** |
|---|---|---|---|
| **Compound 9** | (2S,4R)-4-hydroxy-1-((R)-2-(3-(4-((3-(3-(2-hydroxyphenyl)cinnolin-7-yl)azetidin-1-yl)methyl)piperidin-1-yl)isoxazol-5-yl)-3-methylbutanoyl)-N-((S)-1-(4-(4-methylthiazol-5-yl)phenyl)ethyl)pyrrolidine-2-carboxamide | 855.25 | 1H NMR (400 MHz, DMSO-d6) δ 12.03 (s, 1H), 8.98 (s, 1H), 8.91 (s, 1H), 8.42 - 8.34 (m, 2H), 8.16 - 8.09 (m, 2H), 8.00 (dd, J = 8.6, 1.8 Hz, 1H), 7.49 - 7.41 (m, 2H), 7.41 - 7.33 (m, 3H), 7.10 - 7.00 (m, 2H), 6.12 (s, 1H), 5.09 (d, J = 3.7 Hz, 1H), 4.96 - 4.88 (m, 1H), 4.37 (t, J = 7.9 Hz, 1H), 4.29 (s, 1H), 3.98 - 3.87 (m, 1H), 3.72 (t, J = 7.4 Hz, 3H), 3.63 (d, J = 12.1 Hz, 2H), 3.56 (d, J = 9.8 Hz, 1H), 3.48 - 3.31 (m, 2H), 3.29 - 3.25 (m, 1H), 2.75 (t, J = 11.8 Hz, 2H), 2.46 (s, 3H), 2.41 (d, J = 6.7 Hz, 2H), 2.30 - 2.16 (m, 1H), 2.07 - 1.95 (m, 1H), 1.85 - 1.74 (m, 3H), 1.53 - 1.49 (m, 1H), 1.47 - 1.35 (m, 3H), 1.26 - 1.16 (m, 2H), 1.00 - 0.92 (m, 3H), 0.86 - 0.76 (m, 3H). |
| **Compound 10** | (2S,4R)-4-hydroxy-1-((R)-2-(3-(4-(3-(3-(2-hydroxyphenyl)cinnolin-7-yl)azetidin-1-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 841.25 | 1H NMR (400 MHz, DMSO-d6) δ 12.02 (s, 1H), 8.98 (s, 1H), 8.90 (s, 1H), 8.42 - 8.33 (m, 2H), 8.12 (dd, J = 8.2, 2.1 Hz, 2H), 7.99 (dd, J = 8.6, 1.7 Hz, 1H), 7.49 - 7.41 (m, 2H), 7.41 - 7.33 (m, 3H), 7.09 - 7.00 (m, 2H), 6.14 (s, 1H), 5.10 (d, J = 3.7 Hz, 1H), 4.96 - 4.88 (m, 1H), 4.38 (t, J = 7.8 Hz, 1H), 4.29 (s, 1H), 3.95 - 3.89 (m, 1H), 3.76 - 3.69 (m, 3H), 3.61 - 3.50 (m, 3H), 3.49 - 3.32 (m, 3H), 2.90 (t, J = 10.4 Hz, 2H), 2.46 (s, 3H), 2.38 - 2.33 (m, 1H), 2.25 - 2.20 (m, 1H), 2.04 - 2.00 (m, 1H), 1.83 - 1.69 (m, 3H), 1.49 - 1.34 (m, 3H), 1.34 - 1.24 (m, 2H), 1.00 - 0.93 (m, 3H), 0.87 - 0.77 (m, 3H). |

### Preparation of (2S,4R)-4-hydroxy-1-[(2R)-2-[3-(2-{3-[3-(2-hydroxyphenyl)cinnolin-7-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 11) and (2S,4R)-4-hydroxy-1-[(2S)-2-[3-(2-{3-[3-(2-hydroxyphenyl)cinnolin-7-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 12).

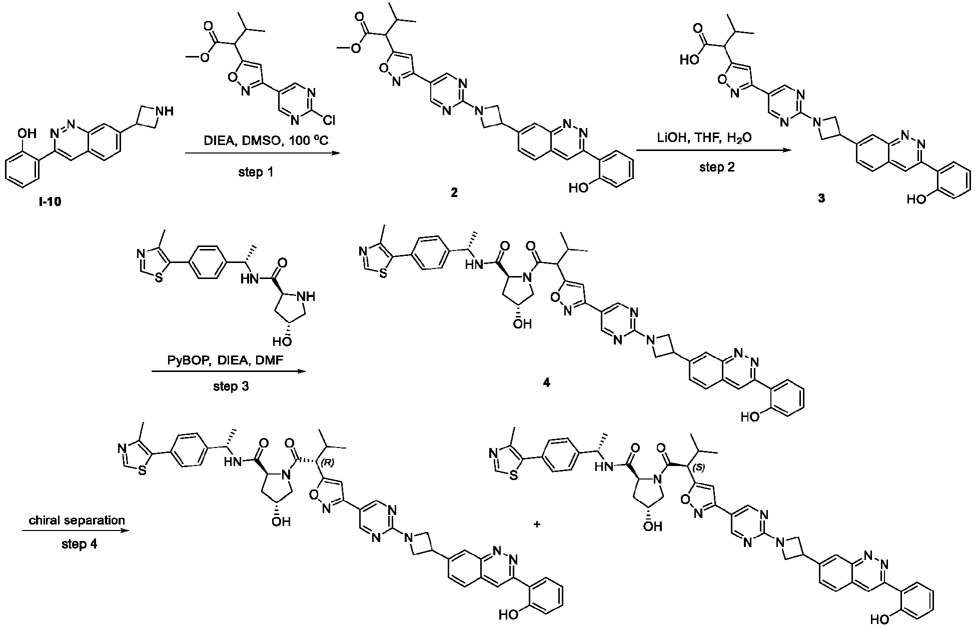

### Step 1: Preparation of methyl 2-[3-(2-{3-[3-(2-hydroxyphenyl)cinnolin-7-yl]azetidin-1-yl}pyrimidin-5-yl)-1,2-oxazol-5-yl]-3-methylbutanoate (Intermediate 2).

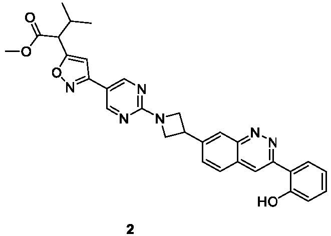

To a stirred solution of I-10 (80 mg, 0.288 mmol, 1 equiv) and methyl 2-[3-(2-chloropyrimidin-5-yl)-1,2-oxazol-5-yl]-3-methylbutanoate (106.63 mg, 0.288 mmol, 1 equiv) in DMSO (3.00 mL) was added DIEA (111.85 mg, 0.864 mmol, 3 equiv). The resulting mixture was stirred for 3 h at 100 °C under nitrogen atmosphere. The mixture was cooled down to room temperature and was then purified by reverse phase flash chromatography with the following conditions: Column, C18 silica gel; Mobile Phase A: water (0.1% FA), Mobile Phase B: CH₃CN; Flow rate: 35 mL/min; Gradient: 0% B to 100% B in 40 min; Detector, UV 254/220 nm. This resulted in Intermediate **2** (110 mg, 67.51%) as a light yellow solid. LCMS (ESI) m/z: [M+H]⁺ = 537.

### Step 2: Preparation of 2-[3-(2-{3-[3-(2-hydroxyphenyl)cinnolin-7-yl]azetidin-1-yl}pyrimidin-5-yl)-1,2-oxazol-5-yl]-3-methylbutanoic acid (Intermediate 3).

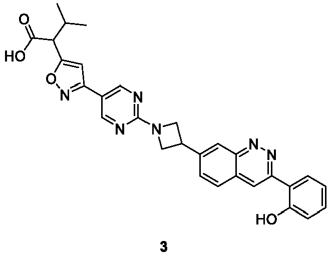

A solution of LiOH (49.10 mg, 2.050 mmol, 10 equiv) in THF (3.00 mL) and H₂O (0.60 mL) was stirred for 10 min at room temperature under nitrogen atmosphere. To the above mixture was added Intermediate **2** (110 mg, 0.205 mmol, 1 equiv). The resulting mixture was stirred overnight at room temperature and then was concentrated under reduced pressure. The crude product was purified by reverse phase flash chromatography with the following conditions: Column, C18 silica gel; Mobile Phase A: Water (0.1% FA), Mobile Phase B: CH₃CN; Flow rate: 35 mL/min; Gradient: 0% B to 100% B in 40 min; Detector, UV 254/220 nm. This resulted in Intermediate 3 (98 mg, 86.91%) as a light yellow solid. LCMS (ESI) m/z: [M+H]⁺ = 523.

### Step 3: Preparation of (2S,4R)-4-hydroxy-1-{2-[3-(2-{3-[3-(2-hydroxyphenyl)cinnolin-7-yl]azetidin-1-yl}pyrimidin-5-yl)-1,2-oxazol-5-yl]-3-methylbutanoyl}-N-[(1S)-1-[4-(4-methyl-1,3-thiazol-5-yl)phenyl]ethyl]pyrrolidine-2-carboxamide (Intermediate 4).

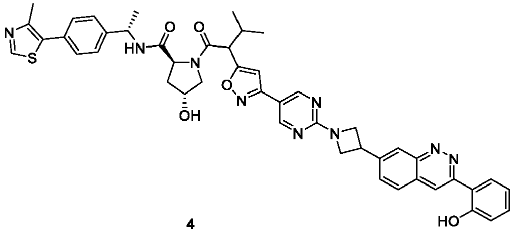

To a stirred solution of Intermediate **3** (98 mg, 0.188 mmol, 1 equiv) and (2S,4R)-4-hydroxy-N-[(1S)-1-[4-(4-methyl-1,3-thiazol-5-yl)phenyl]ethyl]pyrrolidine-2-carboxamide (62.16 mg, 0.188 mmol, 1 equiv) in DMF (3.00 mL) were added PyBOP (195.19 mg, 0.376 mmol, 2 equiv) and DIEA (72.72 mg, 0.564 mmol, 3 equiv). The resulting mixture was stirred for 2 h at room temperature under nitrogen atmosphere. The mixture was then purified by reverse phase flash chromatography with the following conditions: Column, C18 silica gel; Mobile Phase A: Water (0.1% FA), Mobile Phase B: CH₃CN; Flow rate: 35 mL/min; Gradient: 0% B to 100% B in 40 min; Detector, UV 254/220 nm. This resulted in Intermediate **4** (90 mg, 54.54%) as a light yellow solid. LCMS (ESI) m/z: [M+H]⁺ = 836.

### Step 4: Preparation of (2S,4R)-4-hydroxy-1-[(2R)-2-[3-(2-{3-[3-(2-hydroxyphenyl)cinnolin-7-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 11) and (2S,4R)-4-hydroxy-1-[(2S)-2-[3-(2-{3-[3-(2-hydroxyphenyl)cinnolin-7-yl]azetidin-1-yl}pyrimidin-5-yl)-1,2-oxazol-5-yl]-3-methylbutanoyl]-N-[(1S)-1-[4-(4-methyl-1,3-thiazol-5-yl)phenyl]ethyl]pyrrolidine-2-carboxamide (Compound 12).

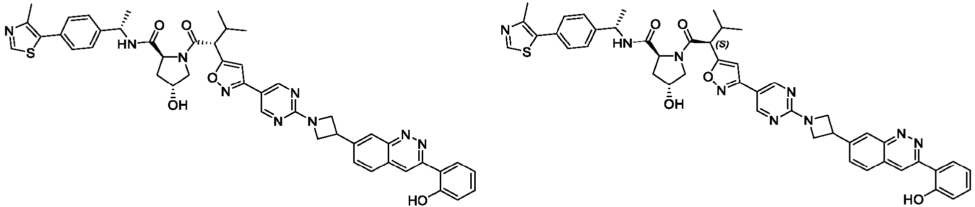

Intermediate **4** (90 mg) was purified by Chiral-Prep-HPLC with the following conditions: Column, CHIRALPAK ID, 2*25 cm, 5 µm; Mobile Phase A: MtBE (10 mM NH₃-MeOH), Mobile Phase B: MeOH; Flow rate: 20 mL/min; Gradient: 10% B to 50% B in 65 min; Detector, UV 254/220 nm. This resulted in:
**Compound 11** (30.1 mg, 34.64%) as a light-yellow solid. ¹H NMR (300 MHz, DMSO-d6) δ 11.96 (s, 1H), 8.97 (d, J = 16.6 Hz, 2H), 8.87 (d, J = 2.0 Hz, 2H), 8.45 (d, J = 11.1 Hz, 2H), 8.21 - 8.11 (m, 2H), 8.05 (dd, J = 8.6, 1.7 Hz, 1H), 7.45 (d, J = 8.2 Hz, 2H), 7.41 - 7.33 (m, 3H), 7.11 - 7.01 (m, 2H), 6.90 (d, J = 33.8 Hz, 1H), 5.12 (d, J = 3.6 Hz, 1H), 4.94 (t, J = 7.3 Hz, 1H), 4.76 - 4.63 (m, 2H), 4.46 - 4.21 (m, 5H), 3.87 (d, J = 9.7 Hz, 1H), 3.82 - 3.70 (m, 1H), 3.51 (t, J = 5.3 Hz, 1H), 2.46 (d, J = 4.2 Hz, 3H), 2.34 (d, J = 10.2 Hz, 1H), 2.05 (t, J = 10.5 Hz, 1H), 1.87 - 1.69 (m, 1H), 1.45 (dd, J = 31.1, 7.0 Hz, 3H), 1.02 (d, J = 6.4 Hz, 3H), 0.86 (t, J = 6.2 Hz, 3H). LCMS (ESI) m/z: [M+H]⁺ = 836.35.

**Compound 12** (21.0 mg, 24.32%) as a light yellow solid. ¹H NMR (300 MHz, DMSO-d₆) *δ* 11.97 (s, 1H), 9.04 - 8.91 (m, 2H), 8.85 (d, J = 14.4 Hz, 2H), 8.48 (s, 1H), 8.28 (d, J = 7.9 Hz, 1H), 8.19 (d, J = 8.6 Hz, 1H), 8.14 (d, J = 7.7 Hz, 1H), 8.07 (d, J = 6.9 Hz, 1H), 7.48 (d, J = 7.2 Hz, 1H), 7.42 - 7.22 (m, 4H), 7.11 - 7.01 (m, 2H), 6.96 (s, 1H), 5.15 (d, J = 3.6 Hz, 1H), 4.93 - 4.84 (m, 1H), 4.68 (t, J = 8.3 Hz, 2H), 4.49 - 4.25 (m, 5H), 3.96 (d, J = 8.9 Hz, 1H), 3.63 (s, 1H), 3.51 (s, 1H), 2.41 (s, 3H), 2.27 (s, 1H), 2.08 (s, 1H), 1.49 (d, J = 7.0 Hz, 1H), 1.38 - 1.20 (m, 3H), 1.02 (d, J = 6.5 Hz, 3H), 0.91 - 0.81 (m, 3H). LCMS (ESI) m/z: [M+H]⁺ = 836.30.

The compounds in Table 6 were prepared using procedures similar to those used above for the preparation of **Compound 11** using the appropriate amines and heteroaryl halides.

**Table 6.**

| **No.** | **Name** | **LCMS (ESI) m/z** | **¹H NMR** |
|---|---|---|---|
| 243 | (2S,4R)-4-hydroxy-1-((R)-2-(3-(2-(6-(3-(2-hydroxyphenyl)cinnolin-6-yl)-2,6-diazaspiro[3.3]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 877.25 | 1H NMR (400 MHz, DMSO-d6) δ 13.33 (d, J = 3.5 Hz, 1H), 8.99 (d, J = 2.2 Hz, 1H), 8.82 (d, J = 2.9 Hz, 2H), 8.56 (s, 1H), 8.42 (d, J = 7.7 Hz, 1H), 8.21 (d, J = 9.2 Hz, 1H), 8.05 (dd, J = 8.3, 1.7 Hz, 1H), 7.48 -7.44 (m, 2H), 7.39 - 7.24 (m, 4H), 7.04 - 6.95 (m, 3H), 6.59 (d, J = 2.4 Hz, 1H), 5.10 (d, J = 3.6 Hz, 1H), 4.93 (p, J = 7.4 Hz, 1H), 4.38 (d, J = 10.3 Hz, 9H), 3.85 (d, J = 9.7 Hz, 1H), 3.76 (dd, J = 10.5, 4.3 Hz, 1H), 3.50 (d, J = 10.6 Hz, 1H), 2.45 (d, J = 5.7 Hz, 3H), 2.33 - 2.33(m, 2H), 2.04 (t, J = 10.2 Hz, 1H), 1.83 - 1.75 (m, 1H), 1.39 (d, J = 7.0 Hz, 3H), 1.02 (dd, J = 6.6, 3.7 Hz, 3H), 0.85 (t, J = 7.2 Hz, 3H). |
| 231 | (2S,4R)-4-hydroxy-1-((R)-2-(3-(2-(6-(3-(2-hydroxyphenyl)cinnolin-7-yl)-2,6-diazaspiro[3.3]heptan-2-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 877.35 | 1H NMR (400 MHz, DMSO-d6) δ 12.82 (s, 1H), 8.99 (d, J = 2.4 Hz, 1H), 8.82 (d, J = 3.2 Hz, 2H), 8.77 (s, 1H), 8.43 (d, J = 7.7 Hz, 1H), 8.07 - 8.00 (m, 1H), 7.97 (d, J = 9.0 Hz, 1H), 7.49 - 7.42 (m, 2H), 7.38 - 7.26 (m, 4H), 7.08 - 6.91 (m, 4H), 5.11 (d, J = 3.6 Hz, 1H), 5.05 - 4.90 (m, 1H), 4.37 (d, J = 17.9 Hz, 10H), 3.85 (d, J = 9.7 Hz, 1H), 3.78 - 3.65 (m, 1H), 3.64 - 3.48 (m, 1H), 2.45 (d, J = 5.5 Hz, 3H), 2.33 (d, J = 8.1 Hz, 1H), 2.04 (t, J = 10.6 Hz, 1H), 1.84 - 1.76 (m, 1H), 1.48 - 1.37 (m, 3H), 1.02 - 1.00 (m, 2H), 0.88 - 0.81 (m, 4H). |
| 88 | (2S,4R)-4-hydroxy-1-((R)-2-(3-(6-(4-(3-(2-hydroxyphenyl)cinnolin-7-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.3 | 1H NMR (300 MHz, DMSO-d6) δ 12.08 (s, 1H), 9.00 (s, 1H), 8.93 (s, 1H), 8.61 (d, J = 2.2 Hz, 1H), 8.44 (d, J = 7.7 Hz, 1H), 8.34 (s, 1H), 8.12 (d, J = 9.2 Hz, 2H), 7.97 (d, J = 10.1 Hz, 2H), 7.46 (d, J = 8.2 Hz, 2H), 7.38 (dt, J = 6.8, 3.5 Hz, 3H), 7.11 - 6.90 (m, 3H), 5.12 (d, J = 3.6 Hz, 1H), 4.95 (q, J = 7.1 Hz, 1H), 4.41 (t, J = 7.9 Hz, 1H), 4.31 (s, 1H), 3.87 (d, J = 9.7 Hz, 1H), 3.78 (d, J = 11.8 Hz, 3H), 3.52 (d, J = 10.7 Hz, 1H), 3.12 (s, 1H), 3.00 (t, J = 12.1 Hz, 2H), 2.46 (d, J = 7.5 Hz, 3H), 2.39 (s, 4H), 2.15 - 1.95 (m, 5H), 1.87 - 1.79 (m, 1H), 1.45 (dd, J = 30.2, 7.0 Hz, 3H), 1.24 (s, 1H), 1.03 (d, J = 6.5 Hz, 3H), 0.86 (t, J = 6.8 Hz, 3H). |
| 81 | (2S,4R)-4-hydroxy-1-((R)-2-(3-(6-(4-(3-(2-hydroxyphenyl)cinnolin-7-yl)piperidin-1-yl)-4-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.25 | 1H NMR (300 MHz, DMSO-d6) δ 8.96 (d, J = 15.2 Hz, 2H), 8.44 (d, J = 7.6 Hz, 1H), 8.31 (d, J = 20.6 Hz, 2H), 8.19 - 8.03 (m, 2H), 7.90 (dd, J = 8.7, 1.7 Hz, 1H), 7.45 (d, J = 8.2 Hz, 2H), 7.41 - 7.25 (m, 3H), 7.10 - 6.96 (m, 2H), 6.92 (s, 1H), 6.67 (d, J = 52.6 Hz, 1H), 5.12 (s, 1H), 4.92 (q, J = 7.1 Hz, 1H), 4.64 (d, J = 12.8 Hz, 2H), 4.48 - 4.20 (m, 2H), 3.84 (d, J = 9.8 Hz, 1H), 3.76 (dd, J = 10.5, 4.2 Hz, 1H), 3.59 - 3.48 (m, 1H), 3.17 (d, J = 11.8 Hz, 3H), 3.10 - 2.93 (m, 2H), 2.51 (d, J = 2.1 Hz, 3H), 2.47 - 2.41 (m, 3H), 2.06 (d, J = 12.6 Hz, 3H), 1.91 - 1.72 (m, 3H), 1.53 - 1.33 (m, 3H), 1.02 (d, J = 6.4 Hz, 3H), 0.86 (dd, J = 9.2, 6.7 Hz, 3H). |
| 80 | (2S,4R)-4-hydroxy-1-((R)-2-(3-(6-(4-(3-(2-hydroxyphenyl)cinnolin-7-yl)piperidin-1-yl)-2-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.2 | 1H NMR (300 MHz, DMSO-d6) δ 12.08 (s, 1H), 9.00 (s, 1H), 8.92 (s, 1H), 8.44 (d, J = 7.7 Hz, 1H), 8.30 (s, 1H), 8.17 - 8.06 (m, 2H), 7.93 (d, J = 8.7 Hz, 1H), 7.75 (t, J = 9.1 Hz, 1H), 7.49 - 7.28 (m, 5H), 7.05 (t, J = 8.2 Hz, 2H), 6.88 (d, J = 8.9 Hz, 1H), 6.72 (s, 1H), 5.12 (d, J = 3.6 Hz, 1H), 4.95 (t, J = 7.0 Hz, 1H), 4.67 (d, J = 12.9 Hz, 2H), 4.41 (t, J = 7.9 Hz, 1H), 4.31 (s, 1H), 3.84 (d, J = 9.7 Hz, 1H), 3.80 - 3.72 (m, 1H), 3.54 (d, J = 10.9 Hz, 1H), 3.19 (t, J = 11.9 Hz, 1H), 3.03 (t, J = 12.5 Hz, 2H), 2.55 (s, 3H), 2.46 (d, J = 5.2 Hz, 3H), 2.40 - 2.30 (m, 1H), 2.07 (d, J = 12.5 Hz, 3H), 1.97 - 1.72 (m, 3H), 1.42 (dd, J = 7.0, 7.0 Hz, 3H), 1.02 (d, J = 6.5 Hz, 3H), 0.86 (d, J = 6.8 Hz, 3H). |
| 79 | (2S,4R)-1-((R)-2-(3-(2-(3-(7-cyclopropyl-3-(2-hydroxyphenyl)cinnolin-6-yl)azetidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 876.35 | 1H NMR (300 MHz, DMSO-d6) δ 8.98 (d, J = 3.5 Hz, 1H), 8.94 (s, 1H), 8.86 (d, J = 1.4 Hz, 2H), 8.43 (d, J = 7.7 Hz, 1H), 8.21 (d, J = 4.0 Hz, 1H), 8.10 - 8.01 (m, 2H), 7.47 - 7.41 (m, 2H), 7.40 - 7.28 (m, 3H), 7.06 - 6.97 (m, 2H), 6.95 (d, J = 4.7 Hz, 1H), 5.17 - 5.02 (br s, 1H), 4.99 - 4.85 (m, 2H), 4.71 (d, J = 3.6 Hz, 3H), 4.48 - 4.35 (m, 3H), 4.30 (s, 1H), 3.85 (d, J = 9.7 Hz, 1H), 3.75 (dd, J = 10.7, 4.4 Hz, 1H), 3.50 (d, J = 10.6 Hz, 1H), 2.45 (d, J = 6.1 Hz, 3H), 2.37 - 2.24 (m, 1H), 2.18 - 1.98 (m, 2H), 1.85 - 1.73 (m, 1H), 1.44 (dd, J = 30.2, 7.0 Hz, 3H), 1.18 - 1.09 (m, 2H), 1.07 - 0.89 (m, 5H), 0.84 (t, J = 6.3 Hz, 3H). |
| 77 | (2S,4R)-1-((R)-2-(3-(2-(3-(5-cyclopropyl-3-(2-hydroxyphenyl)cinnolin-7-yl)azetidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 876.3 | 1H NMR (300 MHz, DMSO-d6) δ 11.92 (s, 1H), 9.13 (s, 1H), 8.99 (s, 1H), 8.89 - 8.78 (m, 2H), 8.43 (d, J = 7.7 Hz, 1H), 8.30 (s, 1H), 8.22 (d, J = 8.1 Hz, 1H), 7.64 - 7.58 (m, 1H), 7.49 - 7.40 (m, 2H), 7.44 - 7.32 (m, 3H), 7.11 - 7.00 (m, 2H), 6.95 (s, 1H), 5.10 (d, J = 3.7 Hz, 1H), 5.07 - 4.88 (m, 1H), 4.67 - 4.61 (m, 2H), 4.45 - 4.28 (m, 5H), 3.90 - 3.71 (m, 2H), 3.51 (d, J = 10.8 Hz, 1H), 2.70 - 2.57 (m, 1H), 2.45 (s, 3H), 2.35 - 2.29 (m, 1H), 2.15 - 1.98 (m, 1H), 1.87 - 1.73 (m, 1H), 1.45 - 1.31 (m, 3H), 1.21 - 1.09 (m, 2H), 1.02 (d, J = 6.5 Hz, 3H), 0.95 - 0.80 (m, 5H). |
| 70 | (2S,4R)-4-hydroxy-1-((R)-2-(3-(6-(4-(3-(2-hydroxyphenyl)cinnolin-6-yl)piperidin-1-yl)-4-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.35 | 1H NMR (400 MHz, Methanol-d4) δ 8.87 (d, J = 5.5 Hz, 1H), 8.78 (s, 1H), 8.38 (d, J = 9.3 Hz, 1H), 8.25 (s, 1H), 8.04 (dd, J = 8.2, 1.6 Hz, 1H), 7.97 - 7.89 (m, 2H), 7.51 - 7.26 (m, 5H), 7.11 - 6.98 (m, 2H), 6.85 (s, 1H), 6.63 (s, 1H), 5.06 (d, J = 7.1 Hz, 1H), 4.69 - 4.51 (m, 3H), 4.45 (s, 1H), 3.89 (dd, J = 10.5, 4.5 Hz, 2H), 3.70 (d, J = 11.1 Hz, 1H), 3.63 (s, 1H), 3.20 - 3.04 (m, 3H), 2.47 (d, J = 10.0 Hz, 6H), 2.20 (s, 1H), 2.09 (d, J = 13.0 Hz, 2H), 2.02 - 1.95 (m, 1H), 1.93 - 1.83 (m, 2H), 1.60 (dd, J = 7.0, 7.1 Hz, 3H), 1.11 (d, J = 6.6 Hz, 3H), 0.99 - 0.91 (m, 3H). |
| 69 | (2S,4R)-4-hydroxy-1-((R)-2-(3-(6-(4-(3-(2-hydroxyphenyl)cinnolin-6-yl)piperidin-1-yl)-2-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.25 | 1H NMR (400 MHz, DMSO-d6) δ 12.00 (s, 1H), 8.98 (s, 1H), 8.86 (s, 1H), 8.42 (d, J = 8.3 Hz, 2H), 8.09 (d, J = 8.1 Hz, 1H), 7.95 (d, J = 8.8 Hz, 2H), 7.76 (d, J = 8.9 Hz, 1H), 7.44 (d, J = 8.2 Hz, 2H), 7.41 - 7.33 (m, 3H), 7.09 - 7.00 (m, 2H), 6.85 (d, J = 8.8 Hz, 1H), 6.71 (s, 1H), 5.10 (d, J = 3.6 Hz, 1H), 4.93 (t, J = 7.2 Hz, 1H), 4.64 (d, J = 13.0 Hz, 2H), 4.40 (t, J = 7.8 Hz, 2H), 3.84 (d, J = 9.8 Hz, 1H), 3.79 - 3.72 (m, 1H), 3.53 (d, J = 13.1 Hz, 1H), 3.17 - 3.13 (m, 1H), 3.03 (t, J = 12.5 Hz, 2H), 2.56 - 2.47 (m, 3H), 2.57 - 2.25 (m, 4H), 2.03 (d, J = 12.3 Hz, 3H), 1.83 - 1.73 (m, 3H), 1.47 - 1.33 (m, 3H), 1.01 (d, J = 6.5 Hz, 3H), 0.91 - 0.82 (m, 3H). |
| 68 | (2S,4R)-1-((R)-2-(3-(2-(3-(6-cyclopropyl-3-(2-hydroxyphenyl)cinnolin-7-yl)azetidin-1-yl)pyrimidin-5-yl)isoxazol-5-yl)-3-methylbutanoyl)-4-hydroxy-N-((S)-1-(4-(4-methylthiazol-5-yl)phenyl)ethyl)pyrrolidine-2-carboxamide | 876.3 | 1H NMR (400 MHz, DMSO-d6) δ 12.12 (s, 1H), 8.98 (d, J = 5.2 Hz, 1H), 8.83 (d, J = 21.8 Hz, 3H), 8.49 - 8.35 (m, 2H), 8.08 (dd, J = 7.9, 1.7 Hz, 1H), 7.76 (s, 1H), 7.47 - 7.41 (m, 2H), 7.41 - 7.31 (m, 3H), 7.12 - 6.99 (m, 2H), 6.88 (d, J = 46.0 Hz, 1H), 5.09 (d, J = 3.7 Hz, 1H), 4.99 - 4.89 (m, 1H), 4.73 (d, J = 4.4 Hz, 3H), 4.46 (d, J = 4.7 Hz, 2H), 4.39 (t, J = 7.9 Hz, 1H), 4.31 (s, 1H), 3.85 (d, J = 9.7 Hz, 1H), 3.76 (dd, J = 10.6, 4.4 Hz, 1H), 3.50 (d, J = 9.9 Hz, 1H), 2.45 (d, J = 7.6 Hz, 3H), 2.39 - 2.26 (m, 1H), 2.18 - 2.08 (1, 1H), 2.08 - 1.97 (m, 1H), 1.87 - 1.71 (m, 1H), 1.44 (dd, J = 39.2, 6.9 Hz, 3H), 1.02 (dd, J = 6.5, 3.7 Hz, 3H), 0.95 - 0.89 (m, 2H), 0.85 (t, J = 7.4 Hz, 5H). |
| 65 | (2S,4R)-4-hydroxy-1-(2-(3-(2-(2-(3-(2-hydroxyphenyl)cinnolin-7-yl)-7-azaspiro[3.5]nonan-7-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 904.25 | 1H NMR (400 MHz, DMSO-d6) δ 12.17 (s, 1H), 9.01 - 8.96 (m, 1H), 8.92 (s, 1H), 8.83 - 8.77 (m, 2H), 8.42 (d, J = 7.7 Hz, 1H), 8.26 (s, 1H), 8.16 - 8.07 (m, 2H), 7.90 - 7.83 (m, 1H), 7.48 - 7.41 (m, 2H), 7.41 - 7.32 (m, 3H), 7.09 - 6.99 (m, 2H), 6.90 (s, 1H), 5.10 (s, 1H), 4.97 - 4.88 (m, 1H), 4.39 (t, J = 7.9 Hz, 1H), 4.30 (s, 1H), 3.95 - 3.90 (m, 3H), 3.84 (d, J = 9.7 Hz, 1H), 3.80 - 3.58 (m, 3H), 3.53 - 3.46 (m, 1H), 2.49 - 2.42 (m, 4H), 2.41 - 2.21 (m, 2H), 2.16 - 2.06 (m, 2H), 2.06 - 2.01 (m, 1H), 1.86 - 1.76 (m, 3H), 1.68 - 1.61 (m, 2H), 1.52 - 1.36 (m, 3H), 1.05 - 0.98 (m, 3H), 0.88 - 0.82 (m, 3H). |
| 64 | (2S,4R)-4-hydroxy-1-((R)-2-(3-(6-(4-(3-(2-hydroxyphenyl)cinnolin-6-yl)piperidin-1-yl)-5-methylpyridin-3-yl)isoxazol-5-yl)-3-methylbutanoyl)-N-((S)-1-(4-(4-methylthiazol-5-yl)phenyl)ethyl)pyrrolidine-2-carboxamide | 877.2 | 1H NMR (400 MHz, DMSO-d6) δ 9.03 - 8.79 (m, 2H), 8.62 - 8.56 (m, 1H), 8.46 - 8.40 (m, 2H), 8.15 - 8.08 (m, 1H), 8.02 - 7.94 (m, 3H), 7.49 - 7.29 (m, 5H), 7.09 - 6.97 (m, 2H), 6.87 (d, J = 52.0 Hz, 1H), 5.14 - 4.69 (m, 2H), 4.48 - 4.25 (m, 2H), 3.89 - 3.82 (m, 1H), 3.80 - 3.72 (m, 3H), 3.69 - 3.59 (m, 1H), 3.11 - 2.94 (m, 3H), 2.46 (s, 3H), 2.39 - 2.32 (m, 4H), 2.11 - 1.89 (m, 5H), 1.86 - 1.75 (m, 1H), 1.53 - 1.34 (m, 3H), 1.09 - 0.99 (m, 3H), 0.89 - 0.79 (m, 3H). |
| 56 | (2S,4R)-4-hydroxy-1-((R)-2-(3-(2-(2-(3-(2-hydroxyphenyl)cinnolin-6-yl)-7-azaspiro[3.5]nonan-7-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 904.4 | 1H NMR (400 MHz, DMSO-d6) δ 12.12 - 12.07 (m, 1H), 9.01 - 8.96 (m, 1H), 8.89 (s, 1H), 8.85 - 8.75 (m, 2H), 8.47 - 8.39 (m, 2H), 8.15 - 8.08 (m, 1H), 7.95 (s, 1H), 7.92 - 7.85 (m, 1H), 7.48 - 7.42 (m, 2H), 7.42 - 7.33 (m, 3H), 7.10 - 7.01 (m, 2H), 6.91 (s, 1H), 5.07 (dd, J = 29.1, 3.3 Hz, 1H), 4.98 - 4.69 (m, 1H), 4.39 (t, J = 7.9 Hz, 1H), 4.30 (s, 1H), 3.94 - 3.81 (m, 4H), 3.80 - 3.72 (m, 3H), 3.53 - 3.46 (m, 1H), 2.47 - 2.43 (m, 5H), 2.37 - 2.28 (m, 1H), 2.13 - 1.99 (m, 3H), 1.85 - 1.81 (m, 3H), 1.65 - 1.61 (m, 2H), 1.44 (dd, J = 40.0, 7.0 Hz, 3H), 1.04 - 0.98 (m, 3H), 0.88 - 0.80 (m, 3H). |
| 52 | (2S,4R)-4-hydroxy-1-((R)-2-(3-(2-(4-(3-(2-hydroxyphenyl)cinnolin-7-yl)piperidin-1-yl)pyrimidin-4-yl)isoxazol-5-yl)-3-methylbutanoyl)-N-((S)-1-(4-(4-methylthiazol-5-yl)phenyl)ethyl)pyrrolidine-2-carboxamide | 864.4 | 1H NMR (300 MHz, DMSO-d6) δ 12.07 (s, 1H), 9.01 - 8.81 (m, 2H), 8.58 - 8.50 (m, 1H), 8.43 (d, J = 7.7 Hz, 1H), 8.32 (s, 1H), 8.16 - 8.05 (m, 2H),7.94 (d, J = 8.6 Hz, 1H), 7.48 - 7.31 (m, 5H), 7.16 (d, J = 5.0 Hz, 1H), 7.10 - 6.99 (m, 2H), 6.96 (s, 1H), 5.13 - 4.86 (m, 4H), 4.45 - 4.34 (m, 1H), 4.29 (s, 1H), 3.95 (d, J = 9.6 Hz, 1H), 3.79 - 3.66 (m, 1H), 3.53 (d, J = 11.1 Hz, 1H), 3.19 - 3.05 (m, 3H), 2.48 - 2.38 (m, 3H), 2.34 (s, 1H), 2.14 - 2.04 (m, 3H), 1.86 (d, J = 13.1 Hz, 1H), 1.78 (d, J = 11.9 Hz, 2H),1.53 - 1.45 (m, 1H), 1.38 (d, J = 7.0 Hz, 2H), 1.01 (d, J = 6.5 Hz, 3H), 0.89 - 0.78 (m, 3H). |
| 50 | (2S,4R)-4-hydroxy-1-((R)-2-(3-(2-((S)-3-(3-(2-hydroxyphenyl)cinnolin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 850.2 | 1H NMR (400 MHz, DMSO-d6) δ 11.97 (s, 1H),8.99 (d, J = 1.2 Hz, 1H), 8.92 - 8.83 (m, 3H), 8.45 (dd, J = 15.3, 8.3 Hz, 2H), 8.14- 7.99 (m, 3H), 7.48 - 7.41 (m, 2H), 7.41 - 7.33 (m, 3H), 7.10 - 7.00 (m, 2H), 6.93 (s, 1H),5.11 (d, J = 3.7 Hz, 1H), 4.99 - 4.89 (m, 1H), 4.43 - 4.35 (m, 1H), 4.33 - 4.19(m, 2H), 3.95 - 3.81 (m, 3H), 3.80 - 3.58 (m, 3H), 3.51 (d, J = 10.6 Hz, 1H), 2.49 - 2.42 (m, 4H), 2.40 - 2.20 (m, 2H), 2.09 - 1.99 (m, 1H), 1.86 - 1.75 (m, 1H), 1.49 (d, J = 7.0 Hz, 1H), 1.39 (d, J = 7.0 Hz, 2H), 1.06 - 0.98 (m, 3H), 0.89 - 0.81 (m, 3H). |
| 49 | (2S,4R)-4-hydroxy-1-((R)-2-(3-(2-((R)-3-(3-(2-hydroxyphenyl)cinnolin-6-yl)pyrrolidin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide | 850.2 | 1H NMR (400 MHz, Methanol-d4) δ 8.89 - 8.84 (m, 1H), 8.81 - 8.73 (m, 3H), 8.42 (d, J = 8.9 Hz, 1H), 8.08 - 7.94 (m, 3H), 7.49 - 7.31 (m, 5H), 7.07 - 6.99 (m, 2H), 6.78 (s, 1H), 5.10 - 5.02 (m, 1H), 4.61 - 4.49 (m, 1H), 4.46 (s, 1H), 4.31 - 4.22 (m, 1H), 4.03 - 3.93 (m, 1H), 3.91 - 3.84 (m, 3H), 3.82 - 3.71 (m, 2H), 3.71 - 3.61 (m, 1H), 2.60 (s, 1H), 2.52 - 2.43 (m, 4H), 2.42 - 2.30 (m, 1H), 2.24 - 2.14 (m, 1H), 2.03 - 1.92 (m, 1H), 1.64 - 1.50 (m, 3H), 1.10 (d, J = 6.5 Hz, 3H), 0.97 - 0.89 (m, 3H). |

### Preparation of (2S,4R)-4-hydroxy-1-((R)-2-(3-(2-(4-(3-(2-hydroxyphenyl)cinnolin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 13) and (2S,4R)-4-hydroxy-1-((S)-2-(3-(2-(4-(3-(2-hydroxyphenyl)cinnolin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 14).

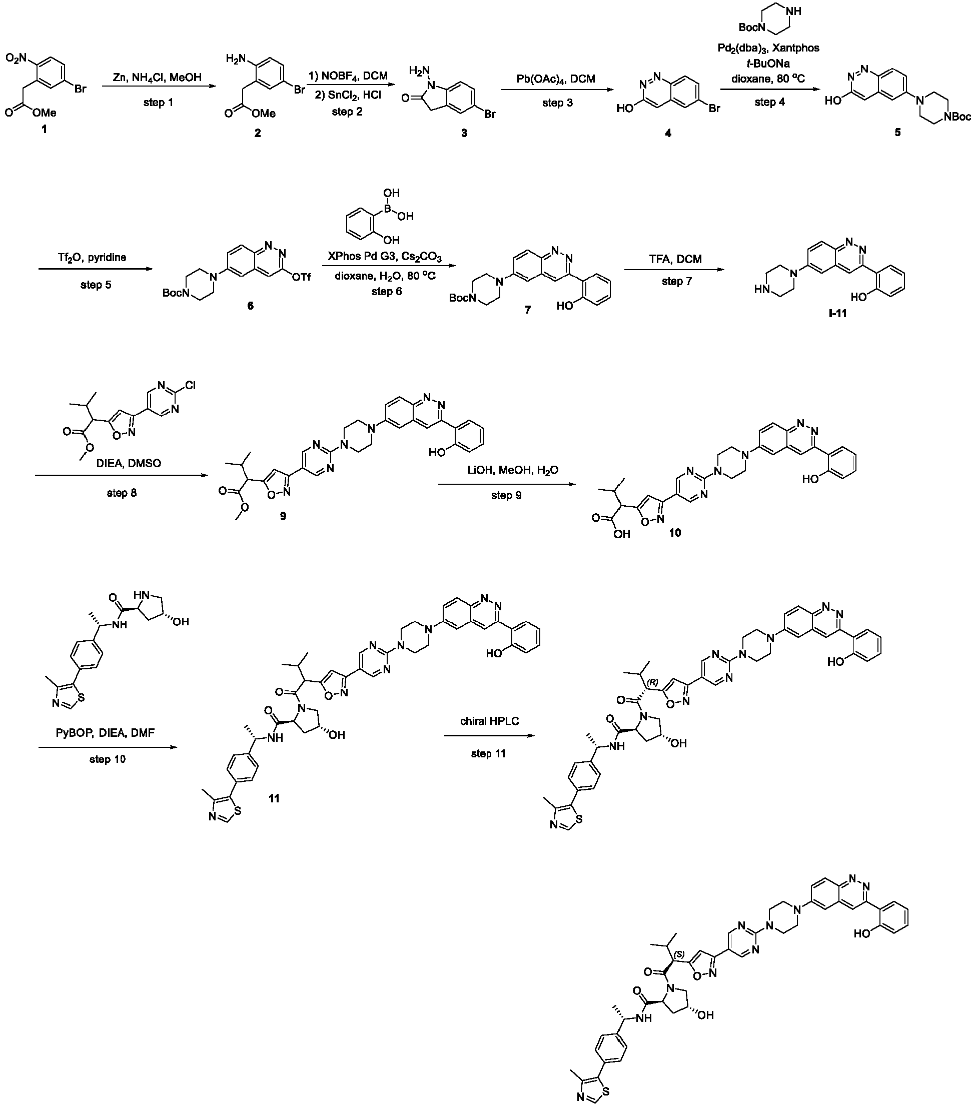

### Step 1: Preparation of methyl 2-(2-amino-5-bromophenyl)acetate (intermediate 2)

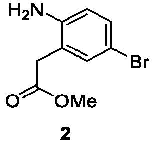

To a mixture of methyl 2-(5-bromo-2-nitrophenyl)acetate (10.00 g, 36.663 mmol, 1.00 equiv) and NH₄Cl (38.80 g, 733.26 mmol, 20.00 equiv) in MeOH (150 mL) was added Zn (47.60 g, 733.26 mmol, 20.00 equiv) at 0 °C. The resulting mixture was stirred for 1 h at 0 °C. The resulting mixture was filtered through a pad of Celite and the filtrate was concentrated under reduced pressure. The residue was diluted with water (50 mL) and extracted with EtOAc (3 x 100 mL). The combined organic layers were dried over anhydrous Na₂SO₄. After filtration, the filtrate was concentrated under reduced pressure. The residue was purified by reverse phase flash chromatography with the following conditions: column, C18 silica gel; mobile phase, ACN in water, 10% to 50% gradient in 10 min; detector, UV 254 nm. This resulted in intermediate 2 (8.00 g, 89.8%) as a yellow solid. LCMS (ESI) m/z: [M+H]⁺ = 244.

### Step 2: Preparation of 1-amino-5-bromoindolin-2-one (intermediate 3).

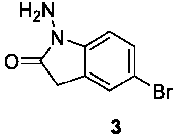

To a mixture of intermediate **2** (8.00 g, 32.921 mmol, 1.00 equiv) in DCM (80 mL) was added NOBF₄ (5.72 g, 49.381 mmol, 1.50 equiv) at 0 °C. The mixture was stirred for 1 h then SnCl₂.2H₂O (44.4 g, 197.526 mmol, 6.00 equiv) in HCl (50 mL) was added at 0 °C. The resulting mixture was stirred overnight at room temperature and then was concentrated under reduced pressure. The residue was purified by reverse phase flash chromatography with the following conditions: column, C18 silica gel; mobile phase, ACN in water, 0% to 30% gradient in 10 min; detector, UV 254 nm. This resulted in intermediate 3 (3.71 g, 50.0%) as a yellow solid. LCMS (ESI) m/z: [M+H]⁺ = 227.

### Step 3: Preparation of 6-bromocinnolin-3-ol (intermediate 4).

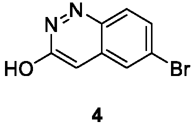

To a mixture of intermediate **3** (2.00 g, 8.849 mmol, 1.00 equiv) in DCM (30 mL) was added Pb(OAc)₄ (5.88 g, 13.273 mmol, 1.50 equiv) at 0 °C. The mixture was stirred for 20 min and then was concentrated under reduced pressure. The residue was purified by reverse phase flash chromatography with the following conditions: column, C18 silica gel; mobile phase, ACN in water, 0% to 30% gradient in 10 min; detector, UV 254 nm. This resulted in intermediate **4** (1.50 g, 75.7%) as a yellow solid. LCMS (ESI) m/z: [M+H]⁺ = 225.

### Step 4: Preparation of tert-butyl 4-(3-hydroxycinnolin-6-yl)piperazine-1-carboxylate (intermediate 5).

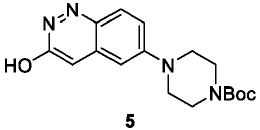

To a mixture of intermediate **4** (800.0 mg, 3.571 mmol, 1.00 equiv), tert-butyl piperazine-1-carboxylate (2.65 g, 14.284 mmol, 4.00 equiv), Xantphos (412.6 mg, 0.714 mmol, 0.20 equiv) and *t*-BuONa (1.028 g, 10.713 mmol, 3.00 equiv) in dioxane (20 mL) was added Pd₂(dba)₃ (654 mg, 0.714 mmol, 0.20 equiv) under nitrogen atmosphere. The mixture was stirred overnight at 80 °C. The resulting mixture was concentrated under reduced pressure and the residue was purified by reverse phase flash chromatography with the following conditions: column, C18 silica gel; mobile phase, ACN in water, 10% to 50% gradient in 10 min; detector, UV 254 nm. This resulted in intermediate **5** (205.0 mg, 17.4%) as a yellow solid. LCMS (ESI) m/z: [M+H]⁺ = 331.

### Step 5: Preparation of tert-butyl 4-(3-(((trifluoromethyl)sulfonyl)oxy)cinnolin-6-yl)piperazine-1-carboxylate (intermediate 6)

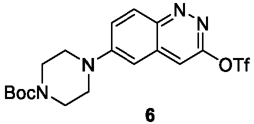

To a mixture of intermediate **5** (205.0 mg, 0.621 mmol, 1.00 equiv) and pyridine (496.8 mg, 6.210 mmol, 10.00 equiv) in DCM (5 mL) was added Tf₂O (350.2 mg, 1.242 mmol, 2.00 equiv) at 0 °C. The mixture was stirred for 1 h and then was concentrated under reduced pressure. The residue was diluted with water (50 mL) and extracted with DCM (3 x 50 mL). The combined organic layers were washed with brine (50 mL) and dried over anhydrous Na₂SO₄. After filtration, the filtrate was concentrated under reduced pressure to afford intermediate **6** (210.0 mg, crude product). LCMS (ESI) m/z: [M+H]⁺ = 463.

### Step 6: Preparation of tert-butyl 4-(3-(2-hydroxyphenyl)cinnolin-6-yl)piperazine-1-carboxylate (intermediate 7)

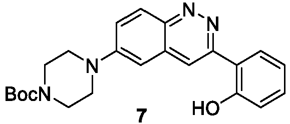

To a mixture of intermediate **6** (210.0 mg, 0.453 mmol, 1.00 equiv), (2-hydroxyphenyl)boronic acid (125.0 mg, 0.906 mmol, 2.00 equiv) and Cs₂CO₃ (441.6 mg, 1.359 mmol, 3.00 equiv) in dioxane (5 mL) and water (1 mL) was added XPhos Pd G3 (76.6 mg, 0.090 mmol, 0.20 equiv) under nitrogen atmosphere. The mixture was stirred for 1 h at 80°C and then was concentrated under reduced pressure. The residue was purified by reverse phase flash chromatography with the following conditions: column, C18 silica gel; mobile phase, ACN in water, 10% to 50% gradient in 10 min; detector, UV 254 nm. This resulted in intermediate **7** (110.0 mg, 59.7%) as a yellow solid. LCMS (ESI) m/z: [M+H]⁺ = 407.

### Step 7: Preparation of 2-(6-(piperazin-1-yl)cinnolin-3-yl)phenol (I-11)

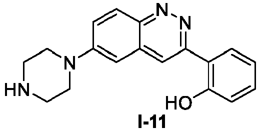

To a mixture of intermediate **7** (110.0 mg, 0.270 mmol, 1.00 equiv) in DCM (3 mL) was added TFA (1 mL). The resulting mixture was stirred for 1 h at room temperature and then was concentrated under reduced pressure to afford intermediate **8** (160.0 mg, crude product). LCMS (ESI) m/z: [M+H]⁺ = 307.

### Step 8: Preparation of methyl 2-(3-(2-(4-(3-(2-hydroxyphenyl)cinnolin-6-yl)piperazin-1-yl)pyrimidin-5-yl)isoxazol-5-yl)-3-methylbutanoate (intermediate 9).

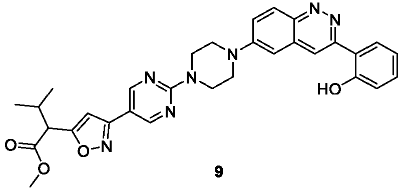

To a stirred mixture of I-11 (160.0 mg, 0.521 mmol, 1.00 equiv) and methyl 2-[3-(2-chloropyrimidin-5-yl)-1,2-oxazol-5-yl]-3-methylbutanoate (153.6 mg, 0.521 mmol, 1 equiv) in DMSO (3 mL) was added DIEA (336.0 mg, 2.605 mmol, 5.00 equiv) dropwise. The resulting mixture was stirred for 1 h at 100 °C. The mixture was allowed to cool down to room temperature and the product was precipitated by the addition of water. The precipitated solids were collected by filtration and washed with water (3 x 10 mL). This resulted in Intermediate **9** (130.0 mg, 44.1%) as a yellow solid. LCMS (ESI) m/z: [M+H]⁺ = 566.

### Step 9: Preparation of 2-(3-(2-(4-(3-(2-hydroxyphenyl)cinnolin-6-yl)piperazin-1-yl)pyrimidin-5-yl)isoxazol-5-yl)-3-methylbutanoic acid (intermediate 10).

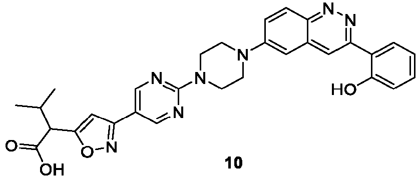

To a stirred mixture of Intermediate **9** (130.0 mg, 0.229 mmol, 1.00 equiv) in MeOH (2 mL) and H₂O (1 mL) was added LiOH·H₂O (93.8 mg, 2.290 mmol, 10 equiv). After stirring for 2 h at 60 °C, the mixture was allowed to cool down to room temperature. The mixture was acidified to pH 6 with 4 M aq. HCl. The precipitated solids were collected by filtration and washed with water (3 x 10 mL). This resulted in Intermediate **10** (150.0 mg, crude product) as a yellow solid. LCMS (ESI) m/z: [M+H]⁺ = 552.

### Step 10: Preparation of (2S,4R)-4-hydroxy-1-(2-(3-(2-(4-(3-(2-hydroxyphenyl)cinnolin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (intermediate 11).

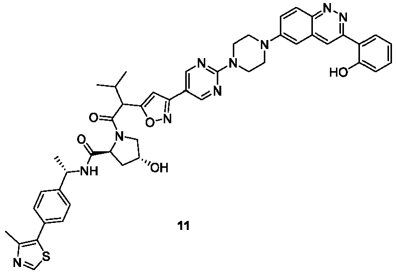

To a stirred mixture of Intermediate **10** (150 mg, 0.264 mmol, 1.00 equiv) and (2S,4R)-4-hydroxy-N-[(1S)-1-[4-(4-methyl-1,3-thiazol-5-yl)phenyl]ethyl]pyrrolidine-2-carboxamide (87.58 mg, 0.264 mmol, 1 equiv) in DMF (4 mL) were added DIEA (102.4 mg, 0.792 mmol, 3.00 equiv) and PyBOP (274.5 mg, 0.528 mmol, 2.00 equiv). The resulting mixture was stirred for 2 h at room temperature. The mixture was then directly purified by reverse phase flash chromatography with the following conditions: column, C18 silica gel; mobile phase, MeCN in Water (10 mmol/L FA), 0% to 100% gradient in 30 min; detector, UV 254 nm. This resulted in Intermediate **11** (130.0 mg, 57.4%) as a yellow solid. LCMS (ESI) m/z: [M+H]⁺ = 865.

### Step 11: Preparation of (2S,4R)-4-hydroxy-1-((R)-2-(3-(2-(4-(3-(2-hydroxyphenyl)cinnolin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 13) and (2S,4R)-4-hydroxy-1-((S)-2-(3-(2-(4-(3-(2-hydroxyphenyl)cinnolin-6-yl)piperazin-1-yl)pyrimidin-5-yl)isoxazol-5-yl)-3-methylbutanoyl)-N-((S)-1-(4-(4-methylthiazol-5-yl)phenyl)ethyl)pyrrolidine-2-carboxamide (Compound 14).

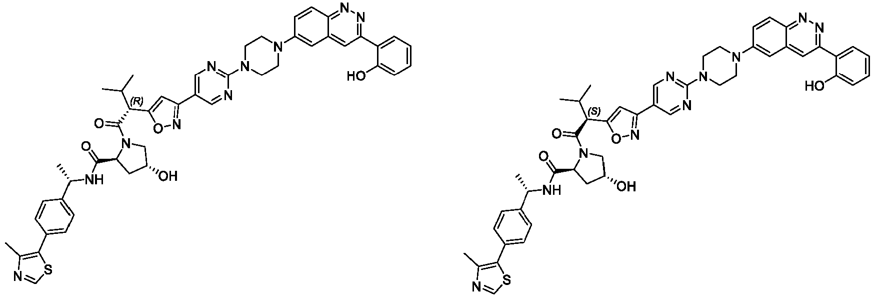

Intermediate **11** (130 mg) was separated by chiral HPLC with the following conditions: Column, CHIRALPAK IA, 2*25 cm, 20 µm; Mobile Phase A: MtBE (10 mM NH₃-MeOH), Mobile Phase B: MeOH; Flow rate: 20 mL/min; Gradient: 10% B to 50% B in 30 min; Detector, UV 254/220 nm. This resulted in:
**Compound 13** (53.6 mg, 40.82%) as a yellow solid. ¹H NMR (400 MHz, DMSO-d6) δ 13.05 (s, 1H), 9.00 - 8.96 (m, 1H), 8.91 - 8.86 (m, 2H), 8.61 (s, 1H), 8.41 (d, J = 7.6 Hz, 1H), 8.26 (d, J = 9.6 Hz, 1H), 8.05 (dd, J = 8.3, 1.7 Hz, 1H), 7.90 (dd, J = 9.7, 2.5 Hz, 1H), 7.48 - 7.41 (m, 2H), 7.41 - 7.31 (m, 3H), 7.16 (d, J = 2.6 Hz, 1H), 7.05 - 6.97 (m, 2H), 6.95 (s, 1H), 5.10 (d, J = 3.7 Hz, 1H), 4.97 - 4.89 (m, 1H), 4.39 (t, J = 7.9 Hz, 1H), 4.31 (s, 1H), 4.05 (t, J = 5.4 Hz, 4H), 3.86 (d, J = 9.7 Hz, 1H), 3.76 (dd, J = 10.8, 4.3 Hz, 1H), 3.68 (t, J = 5.4 Hz, 4H), 3.51 (d, J = 10.6 Hz, 1H), 2.47 - 2.43 (m, 3H), 2.38 - 2.28 (m, 1H), 2.08 - 1.99 (m, 1H), 1.84 - 1.76 (m, 1H), 1.52 - 1.36 (m, 3H), 1.05 - 0.99 (m, 3H), 0.89 - 0.81 (m, 3H). LCMS (ESI) m/z: [M+H]⁺ = 865.30.

**Compound 14** (28.4 mg, 21.8%) as a yellow solid. ¹H NMR (400 MHz, DMSO-d6) δ 13.05 (s, 1H), 9.00 - 8.96 (m, 1H), 8.91 - 8.86 (m, 2H), 8.61 (s, 1H), 8.29 - 8.21 (m, 2H), 8.05 (dd, J = 8.3, 1.7 Hz, 1H), 7.90 (dd, J = 9.7, 2.5 Hz, 1H), 7.52 - 7.41 (m, 1H), 7.39 - 7.26 (m, 4H), 7.16 (d, J = 2.6 Hz, 1H), 7.05 - 6.97 (m, 2H), 6.95 (s, 1H), 5.10 (d, J = 3.7 Hz, 1H), 4.97 - 4.89 (m, 1H), 4.39 (t, J = 7.9 Hz, 1H), 4.33 - 4.24 (m, 1H), 4.05 (t, J = 5.4 Hz, 4H), 3.86 (d, J = 9.7 Hz, 1H), 3.68 (t, J = 5.4 Hz, 4H), 3.64 - 3.60 (m, 1H), 3.29 - 3.27 (m,1H), 2.47 - 2.43 (m, 3H), 2.38 - 2.28 (m, 1H), 2.08 - 1.99 (m, 1H), 1.84 - 1.76 (m, 1H), 1.52 - 1.36 (m, 3H), 1.05 - 0.99 (m, 3H), 0.89 - 0.81 (m, 3H). LCMS (ESI) m/z: [M+H]⁺ = 865.30.

### Preparation of 7-chloro-3-(2-(methoxymethoxy)phenyl)cinnoline (I-12)

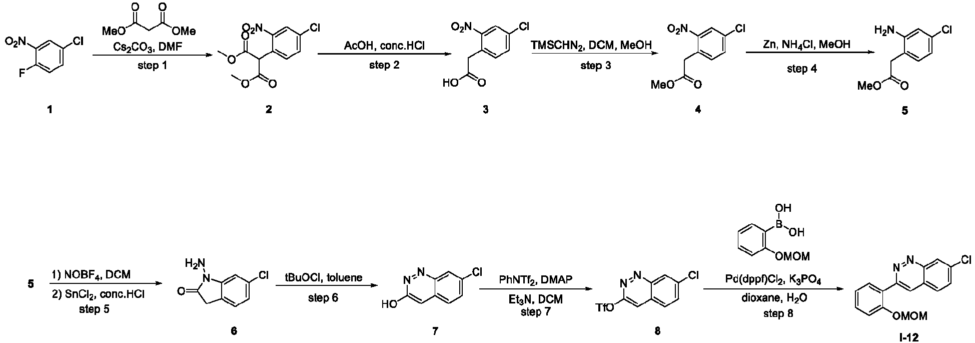

### Step 1: Preparation of dimethyl 2-(4-chloro-2-nitrophenyl)malonate (Intermediate 2).

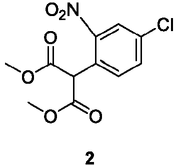

4-Chloro-1-fluoro-2-nitrobenzene (11 g, 63 mmol), dimethyl malonate (12.5 g, 95 mmol), Cs₂CO₃ (41.1 g, 126 mmol), and DMF (63 mL) were stirred at rt for 6 h. The reaction mixture was partitioned between aqueous 1 M HCl and EtOAc. The organic layer was washed with brine, dried over Na₂SO₄ and concentrated to afford intermediate 2 (18 g, 99%) as a yellow oil. The crude product was used in the next step directly without further purification.

### Step 2: Preparation of 2-(4-chloro-2-nitrophenyl)acetic acid (Intermediate 3).

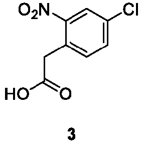

The intermediate **2** was combined with AcOH (30 mL) and cone. HCl (30 mL) and heated at 95 °C for 16 h. The mixture was diluted with H₂O to form a precipitate. The solid was collected by vacuum filtration, washed with H₂O, hexane/ether (1:1) and dried to afford intermediate **3** (11.2 g, 83%) as a white solid.

### Step 3: Preparation of methyl 2-(4-chloro-2-nitrophenyl)acetate (Intermediate 4).

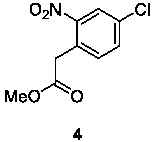

Intermediate **3** (11.2 g, 52 mmol) was suspended in CH₂Cl₂ (250 mL). Oxalyl chloride (7 mL, 79 mmol) was added to the mixture followed by DMF (0.1 mL, 1 mmol). The mixture was stirred at room temperature for 1 h, and then added dropwise to MeOH at 0 °C. The solvent was removed under vacuum to yield intermediate **4** (12 g, 98%) as a white solid. The crude product was used in the next step directly without further purification.

### Step 4: Preparation of methyl 2-(2-amino-4-chlorophenyl)acetate (Intermediate 5).

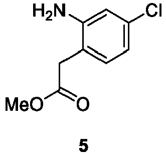

The intermediate **4** (12 g, 51 mmol) was suspended in a mixture of MeOH (200 mL) and NH₄Cl (55 g, 1.03 mol) at 0 °C. Zinc powder (16.8 g, 257 mmol) was added in one portion. The mixture was stirred at room temperature for 2 h, and then was filtered through Celite. The filtrate was concentrated and then partitioned between EtOAc and H₂O. The organic layer was washed with brine, dried over Na₂SO₄, filtered and concentrated to yield intermediate **5** (9.5 g, 94%) as a white solid. The crude product was used in the next step directly without further purification. LCMS (ESI) m/z: [M+H]⁺ = 200.

### Step 5: Preparation of 1-amino-6-chloroindolin-2-one (Intermediate 6).

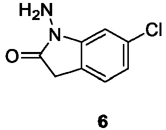

The intermediate **5** (9.5 g, 48 mmol) was suspended in CH₂Cl₂ (150 mL) at 0 °C. Nitrosonium tetrafluoroborate (8.4 g, 72 mmol) was added in one portion to the mixture. The mixture was stirred at 0 °C for 1 h. The mixture was added directly to a vigorously stirred mixture of SnCl₂ dihydrate (43.8 g, 194 mmol) in cone. HCl (200 mL) at 0 °C. The mixture was allowed to slowly warm to room temperature with stirring. After 24 h, the mixture was filtered. The solid was washed with H₂O and ether, and then dried to yield intermediate **6** (6.6 g, 76%) as a yellow solid. LCMS (ESI) m/z: [M+H]⁺ = 183.

### Step 6: Preparation of 7-chlorocinnolin-3-ol (Intermediate 7).

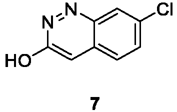

The intermediate **6** (6.6 g, 37 mmol) was suspended in toluene (500 mL) at 0 °C. *tert*-butyl hypochlorite (4 g, 37 mmol) was added to the mixture in one portion. The mixture was stirred at 0 °C for 20 min. The solid was collected by vacuum filtration, washed with H₂O, hexane/ether (1:1) and dried to afford intermediate **7** (3 g, 45%) as a yellow solid. LCMS (ESI) m/z: [M+H]⁺ = 181.

### Step 7: Preparation of 7-chlorocinnolin-3-yl trifluoromethanesulfonate (Intermediate 8).

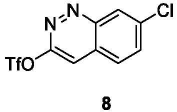

To intermediate **7** (3 g, 16.7 mmol) in CH₂Cl₂ (20 mL) at 0 °C was added triethylamine (4.7 mL, 33 mmol), 4-(dimethylamino)pyridine ( 0.2 g, 0.16 mmol,) and *N-*Phenylbis(trifluoromethanesulfonimide) (9.0 g, 25 mmol). The resulting mixture was stirred at rt for 1 h, and then concentrated in *vacuo.* The residue was purified by chromatography on silica gel, eluting with PE/EtOAc to afford intermediate **8** (4.2 g, 82%) as a yellow solid. LCMS (ESI) m/z: [M+H]⁺ = 313.

### Step 8: Preparation of 7-chloro-3-(2-(methoxymethoxy)phenyl)cinnoline (I-12).

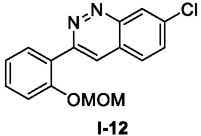

A mixture of intermediate **8** (4.2 g, 13.4 mmol), Pd(dppf)Cl₂ (1 g, 1.3 mmol), K₃PO₄ (5.6 g, 26.8 mmol) and (2-(methoxymethoxy)phenyl)boronic acid (3 g, 16 mmol) in dioxane (20 mL) / H₂O (2 mL) was stirred for 1 h at 40 °C under nitrogen atmosphere. The resulting mixture was filtered, the filter cake was washed with EtOAc (3x10 mL). The filtrate was concentrated under reduced pressure. The residue was purified by silica gel column chromatography, eluted with PE / EA (8:1) to afford **1-12** (2.8 g, 70%) as a yellow solid. LCMS (ESI) m/z: [M+H]⁺ = 301.

### Preparation of 6-Chloro-3-(2-(methoxymethoxy)phenyl)cinnoline (1-13)

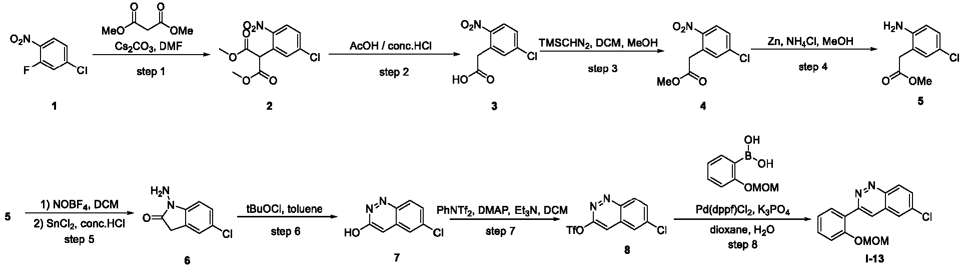

### Step 1: Preparation of dimethyl 2-(5-chloro-2-nitrophenyl)malonate (Intermediate 2).

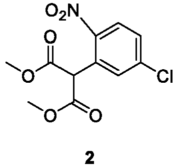

4-Chloro-2-fluoro-1-nitrobenzene (16.5 g, 94.5 mmol), dimethyl malonate (18.8 g, 143 mmol), Cs₂CO₃ (61.6 g, 189 mmol), and DMF (100 mL) were stirred at rt for 6 h. The reaction mixture was partitioned between aqueous 1 M HCl and EtOAc. The organic layer was washed with brine, dried over Na₂SO₄ and concentrated to afford intermediate **2** (28 g, 99%) as a yellow oil. The crude product was used in the next step directly without further purification.

### Step 2: Preparation of 2-(5-chloro-2-nitrophenyl)acetic acid (Intermediate 3).

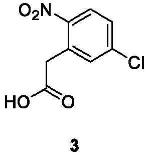

The intermediate **2** was combined with AcOH (30 mL) and cone. HCl (30 mL) and heated at 95 °C for 16 h. The mixture was allowed to cool down to 0 °C and then was diluted with H₂O to form a precipitate. The solid was collected by vacuum filtration, washed with H₂O, hexane/ether (1:1) and dried to afford intermediate **3** (17 g, 83%) as a white solid.

### Step 3: Preparation of methyl 2-(5-chloro-2-nitrophenyl)acetate (Intermediate 4).

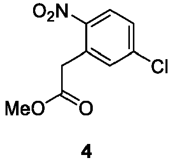

Intermediate **3** (17 g, 78 mmol) was suspended in CH₂Cl₂ (300 mL). Oxalyl chloride (10.5 mL, 119 mmol) was added to the mixture followed by DMF (0.1 mL, 1 mmol). The mixture was stirred at room temperature for 1 h, and then added dropwise to MeOH at 0 °C. The solvent was removed under *vacuum* to yield intermediate **4** (18 g, 98%) as a white solid. The crude product was used in the next step directly without further purification.

### Step 4: Preparation of methyl 2-(2-amino-5-chlorophenyl)acetate (Intermediate 5).

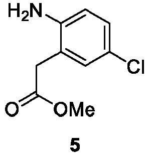

The intermediate **4** (18 g, 77 mmol) was suspended in a mixture of MeOH (300 mL) and NH₄Cl (83 g, 1.53 mol) at 0 °C. Zinc powder (25 g, 386 mmol) was added in one portion. The mixture was stirred at room temperature for 1 h, and then was filtered through Celite. The filtrate was concentrated and then partitioned between EtOAc and H₂O. The organic layer was washed with brine, dried over Na₂SO₄, filtered and concentrated to yield intermediate **5** (14.3 g, 94%) as a white solid. The crude product was used in the next step directly without further purification. LCMS (ESI) m/z: [M+H]⁺ = 200.

### Step 5: Preparation of 1-amino-5-chloroindolin-2-one (Intermediate 6).

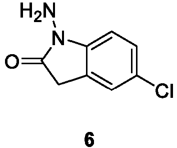

The intermediate **5** (14.3 g, 72 mmol) was suspended in CH₂Cl₂ (200 mL) at 0 °C. Nitrosonium tetrafluoroborate (12.6 g, 108 mmol) was added in one portion to the mixture. The mixture was stirred at 0 °C for 1 h. The mixture was added directly to a vigorously stirred mixture of SnCl₂ dihydrate (66 g, 291 mmol) in cone. HCl (300 mL) at 0 °C. The mixture was allowed to slowly warm to room temperature with stirring. After 24 h, the mixture was filtered. The solid was washed with H₂O and ether, and then dried to yield intermediate **6** (10 g, 76%) as a yellow solid. LCMS (ESI) m/z: [M+H]⁺ = 183.

### Step 6: Preparation of 6-chlorocinnolin-3-ol (Intermediate 7).

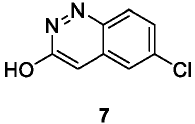

The intermediate **6** (10 g, 56 mmol) was suspended in toluene (500 mL) at 0 °C. *tert*-butyl hypochlorite (6 g, 56 mmol) was added to the mixture in one portion. The mixture was stirred at 0 °C for 20 min. The solid was collected by vacuum filtration, washed with H₂O, hexane/ether (1:1) and dried to afford intermediate **7** (4.5 g, 45%) as a yellow solid. LCMS (ESI) m/z: [M+H]⁺ = 181.

### Step 7: Preparation of 6-chlorocinnolin-3-yl trifluoromethanesulfonate (Intermediate 8).

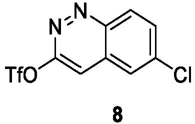

To intermediate **7** (4.5 g, 25 mmol) in CH₂Cl₂ (40 mL) at 0 °C was added triethylamine (7 mL, 50 mmol), 4-(dimethylamino)pyridine ( 0.3 g, 0.25 mmol,) and *N-*Phenylbis(trifluoromethanesulfonimide) (13.5 g, 37.5 mmol). The resulting mixture was stirred at rt for 1 h, and then concentrated in *vacuo.* The residue was purified by chromatography on silica gel, eluting with PE/EtOAc to afford intermediate **8** (6.3 g, 82%) as a yellow solid. LCMS (ESI) m/z: [M+H]⁺ = 313.

### Step 8: Preparation of 6-chloro-3-(2-(methoxymethoxy)phenyl)cinnoline (I-13)

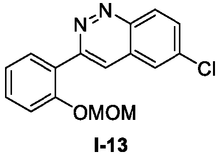

A mixture of intermediate **8** (6.3 g, 20.1 mmol), Pd(dppf)Cl₂ (1.5 g, 1.95 mmol), K₃PO₄ (8.4 g, 40.2 mmol) and (2-(methoxymethoxy)phenyl)boronic acid (4.5 g, 24 mmol) in dioxane (40 mL) / H₂O (6 mL) was stirred for 2 h at r.t under nitrogen atmosphere. The resulting mixture was filtered, the filter cake was washed with EtOAc (3x20 mL). The filtrate was concentrated under reduced pressure. The residue was purified by silica gel column chromatography, eluted with PE / EA (8:1) to afford I**-13** (4.1 g, 69%) as a yellow solid. LCMS (ESI) m/z: [M+H]⁺ = 301.

### Preparation of 2-(7-bromo-6-chlorocinnolin-3-yl)phenol; methyl ether (I-14)

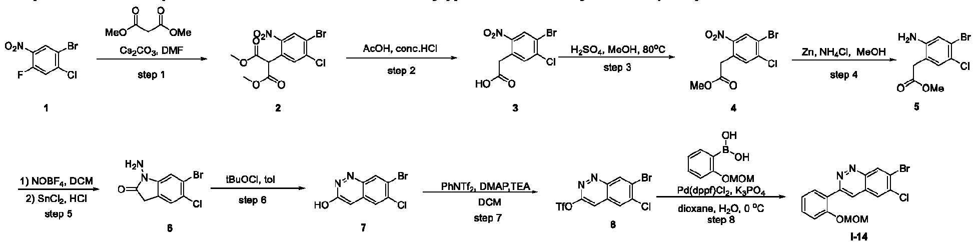

### Step 1: Preparation of 1,3-dimethyl 2-(4-bromo-5-chloro-2-nitrophenyl)propanedioate (Intermediate 2)

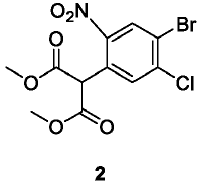

A solution of 1-bromo-2-chloro-4-fluoro-5-nitrobenzene (100 g, 393.020 mmol, 1 equiv) and dimethyl malonate (57.12 g, 432.322 mmol, 1.1 equiv) in DMF (500 mL) was stirred for 12 h at room temperature. The resulting mixture was diluted with water (300 mL). The mixture was acidified to pH 6 with HCl (aq.). The resulting mixture was extracted with EtOAc (1 x 1000 mL). The combined organic layers were washed with brine (3x1000 mL), dried over anhydrous Na2SO4. After filtration, the filtrate was concentrated under reduced pressure. The resulting mixture was concentrated under reduced pressure. This resulted in Intermediate **2** (140 g, crude) as a yellow solid. The crude was used in the next step directly without further purification. LCMS (ESI) m/z [M+H]⁺ =366.

### Step 2: Preparation of (4-bromo-5-chloro-2-nitrophenyl)acetic acid (Intermediate 3)

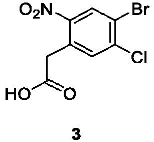

A solution of Intermediate **2** (70 g, 190.970 mmol, 1 equiv) and AcOH (500 mL) in conc.HCl (500 mL) was stirred for 12 h at 100 °C. The mixture was allowed to cool down to 0 °C. The precipitated solids were collected by filtration and washed with water (3 x 300 mL). The resulting mixture was concentrated under reduced pressure. This resulted in Intermediate **3** (50 g, crude) as a yellow solid. The crude product was used in the next step directly without further purification. LCMS (ESI) m/z [M+H]⁺ =294.

### Step 3: Preparation of methyl 2-(4-bromo-5-chloro-2-nitrophenyl)acetate (Intermediate 4)

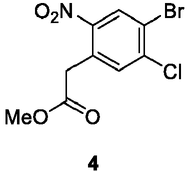

A solution of Intermediate **3** (50 g, 169.785 mmol, 1 equiv) and H₂SO₄ (4 mL) in MeOH (400 mL) was stirred for 12 h at 60°C. The resulting mixture was concentrated under reduced pressure. The resulting mixture was diluted with water (400 mL). The mixture was neutralized to pH 6 with saturated NaHCO3 (aq.). The resulting mixture was extracted with EtOAc (1 x 1000 mL). The combined organic layers were washed with water (3 x 500 mL), dried over anhydrous sodium sulfate. After filtration, the filtrate was concentrated under reduced pressure. The crude product/ resulting mixture was used in the next step directly without further purification. This resulted in Intermediate **4** (46 g, crude) as a yellow solid. LCMS (ESI) m/z [M+H]⁺ =308.

### Step 4: Preparation of methyl 2-(4-bromo-5-chloro-2-nitrophenyl)acetate (Intermediate 5)

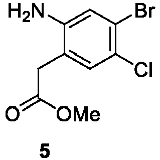

A solution of Intermediate **4** (50 g, 169.785 mmol, 1 equiv) and H₂SO₄ (4 mL) in MeOH (400 mL) was stirred for 12 h at 60°C. The resulting mixture was concentrated under reduced pressure. The resulting mixture was diluted with water (400 mL). The mixture was neutralized to pH 6 with saturated NaHCO₃ (aq.). The resulting mixture was extracted with EtOAc (1 x 1000 mL). The combined organic layers were washed with water (3 x 500 mL), dried over anhydrous sodium sulfate. After filtration, the filtrate was concentrated under reduced pressure. This resulted in Intermediate **5** (46 g, crude) as a yellow solid. The crude product was used in the next step directly without further purification. LCMS (ESI) m/z [M+H]⁺ =278.

### Step 5: Preparation of 1-amino-6-bromo-5-chloro-3H-indol-2-one (Intermediate 6)

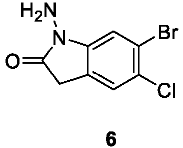

A solution of Intermediate **5** (16 g, 57.444 mmol, 1 equiv) and NOBF4 (10.07 g, 86.166 mmol, 1.5 equiv) in DCM (300 mL) was stirred for 2 h at 0 °C. To the above mixture was added SnCl2 (88.06 g, 459.552 mmol, 8.0 equiv) in HCl (300 mL) dropwise at 0 °C. The resulting mixture was stirred for additional 24 h at room temperature. The precipitated solids were collected by filtration and washed with water (3 x 100 mL). The resulting mixture was concentrated under reduced pressure. This resulted in Intermediate **6** (10.4 g, crude) as a white solid. The crude product was used in the next step directly without further purification. LCMS (ESI) m/z [M+H]⁺ =261.

### Step 6: Preparation of 7-bromo-6-chlorocinnolin-3-ol (Intermediate 7)

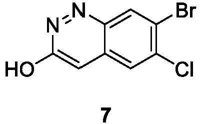

To a stirred solution of Intermediate **6** (10.4 g, 39.771 mmol, 1 equiv) in toluene (100 mL) was added tert-butyl hypochlorite (4.32 g, 39.771 mmol, 1.0 equiv) dropwise at room temperature. The resulting mixture was stirred for 25 min at room temperature. The resulting mixture was filtered, the filter cake was washed with PE (3 x 50 mL). The filtrate was concentrated under reduced pressure. This resulted in Intermediate **7** (9.5 g ,crude) as a yellow solid. The crude product was used in the next step directly without further purification. LCMS (ESI) m/z [M+H]⁺ =259.

### Step 7: Preparation of 7-bromo-6-chlorocinnolin-3-yl trifluoromethanesulfonate (Intermediate 8)

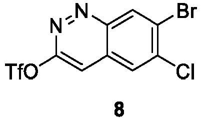

A solution of Intermediate **7** (9.5 g, 36.610 mmol, 1 equiv) and DMAP (447.27 mg, 3.661 mmol, 0.1 equiv) in DCM (2 mL) was stirred for 2 h at room temperature. The mixture was concentrated under vacuum. The residue was purified by silica gel column chromatography, eluted with PE / EtOAc (12:1) to afford Intermediate **8** (9.4 g, 65.58%) as a white solid. LCMS (ESI) m/z [M+H]⁺ =391.

### Step 8: Preparation of 2-(7-bromo-6-chlorocinnolin-3-yl)phenol; methyl ether (I-14)

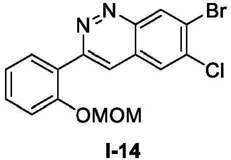

To a solution of Intermediate **8** (3.00 g, 7.662 mmol, 1 equiv) and 2-(methoxymethoxy)phenylboronic acid (1.39 g, 7.662 mmol, 1.0 equiv) in dioxane (100 mL) and H₂O (25 mL) were added K₃PO₄ (4.88 g, 22.986 mmol, 3.0 equiv) and Pd(dppf)Cl₂ (1.12 g, 1.532 mmol, 0.2 equiv). After stirring for 1 h at room temperature under a nitrogen atmosphere, the mixture was concentrated under vacuum, the residue was purified by silica gel column chromatography, eluted with PE / EtOAc (10:1) to afford **I-14** (960 mg, 32.83%) as a yellow solid. LCMS (ESI) m/z [M+H]⁺ =827.

The following intermediates in Table 7 were prepared in a similar manner as described in the preparation of intermediate **I-14.**

**Table 7.**

| **Structure** | **Intermediate No.** | **Name** | **LCMS (ESI) m/z** |
|---|---|---|---|
| 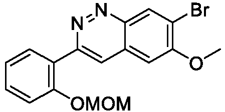 | **I-15** | *7-bromo-6-methoxy-3-[2-(methoxymethoxy)phenyl]cinnoline* | 375 |
| 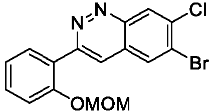 | **I-16** | *6-bromo-7-chloro-3-(2-(methoxymethoxy)phenyl)cinnoline* | 379 |
| 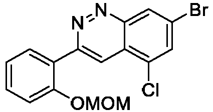 | **I-17** | *7-bromo-5-chloro-3-[2-(methoxymethoxy)phenyl]cinnoline* | 381 |
| 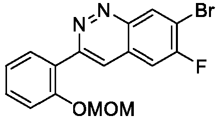 | **I-18** | *7-bromo-6-fluoro-3-[2-(methoxymethoxy)phenyl]cinnoline* | 363 |
| 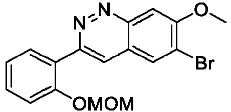 | **I-19** | *6-bromo-7-methoxy-3-(2-(methoxymethoxy)phenyl)cinnoline* | 375 |

### Preparation of 6-chloro-7-cyclopropyl-3-[2-(methoxymethoxy)phenyl]cinnoline (I-20)

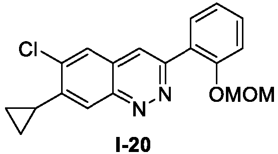

To a solution of **I-14** (1 g, 2.634 mmol, 1 equiv) and cyclopropylboronic acid (678.80 mg, 7.902 mmol, 3.0 equiv) in dioxane (10 mL) and H₂O (2.5 mL) were added K₃PO₄ (1677.37 mg, 7.902 mmol, 3.0 equiv) and Pd(dppf)Cl₂ (385.4 mg, 0.527 mmol, 0.2 equiv). After stirring for 2 h at 60 °C under a nitrogen atmosphere, the resulting mixture was concentrated under reduced pressure. The residue was purified by silica gel column chromatography, eluted with PE / EA (8:1) to afford I**-20** (380.0 mg, 42.3%) as a yellow solid. LCMS (ESI) m/z [M+H]⁺ =341.

### Preparation of tert-butyl 6-{6-chloro-3-[2-(methoxymethoxy)phenyl]cinnolin-7-yl}-2-azaspiro[3.3]heptane-2-carboxylate (I-21)

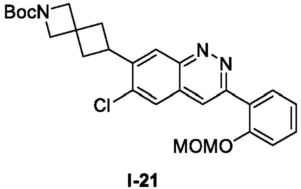

A solution of Zn (413.32 mg, 6.324 mmol, 6 equiv) and I₂ (133.71 mg, 0.527 mmol, 0.5 equiv) and tert-butyl 6-iodo-2-azaspiro[3.3]heptane-2-carboxylate (681.01 mg, 2.108 mmol, 2 equiv) in DMF was stirred for 2 h at 30 °C under nitrogen atmosphere. To the above mixture was added **I-14** (400 mg, 1.054 mmol, 1 equiv) and XPhos Pd G3 (178.37 mg, 0.211 mmol, 0.2 equiv) and XPhos (100.46 mg, 0.211 mmol, 0.2 equiv) in portions over 5 min at room temperature under nitrogen atmosphere. The resulting mixture was stirred overnight at 60 °C under nitrogen atmosphere. The resulting mixture was diluted with water (30 mL). The resulting mixture was extracted with EtOAc (30 x mL). The combined organic layers were washed with brine (3x20 mL), dried over anhydrous Na₂SO₄. After filtration, the filtrate was concentrated under reduced pressure. The residue was purified by silica gel column chromatography, eluted with PE / EA (3:1) to afford **I-21** (190 mg, 36.36%) as a yellow solid. LCMS (ESI) m/z: [M+H]⁺ = 496.

### Preparation of tert-butyl 6-(6-cyano-3-(2-(methoxymethoxy)phenyl)cinnolin-7-yl)-2-azaspiro[3.3]heptane-2-carboxylate (I-22)

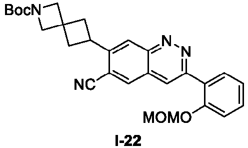

To a stirred solution of **I-21** (220 mg, 0.444 mmol, 1 equiv) and Zn(CN)₂ (208.33 mg, 1.776 mmol, 4 equiv) in DMF (3 mL) was added XPhos Pd G3 (75.09 mg, 0.089 mmol, 0.2 equiv) in portions at room temperature. The resulting mixture was stirred for 2 h at 100°
C under nitrogen atmosphere. The resulting mixture was diluted with EtOAc (50 mL). The combined organic layers were washed with brine (2 x 25 mL), dried over anhydrous Na₂SO₄. After filtration, the filtrate was concentrated under reduced pressure. The residue was purified by silica gel column chromatography, eluted with PE/EA (5:1) to afford **I-22** (117 mg, 54.21%) as a yellow oil. LCMS (ESI) m/z: [M+H]+ =487.

### Preparation of tert-butyl 6-(6-chloro-3-(2-(methoxymethoxy) phenyl) cinnolin-7-yl)-2,6-diazaspiro[3.3] heptane-2-carboxylate (I-23).

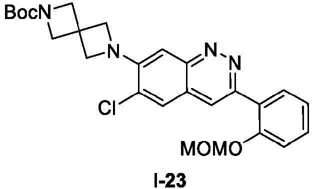

To a solution of **I-14** (340.0 mg, 0.90 mmol, 1.00 equiv) and tert-butyl 2,6-diazaspiro[3.3] heptane-2-carboxylate hemioxalate (435.8 mg, 0.90 mmol, 1.00 equiv) in Toluene (10 mL) were added BINAP (111.5 mg, 0.18 mmol, 0.20 equiv), Pd₂(dba)₃ (164.0 mg, 0.18 mmol, 0.20 equiv) and t-BuONa (172.1 mg, 1.79 mmol, 2.00 equiv). After stirring for 2 hrs at 100 °C under a nitrogen atmosphere. The resulting mixture was concentrated under reduced pressure. The residue was purified by silica gel column chromatography, eluted with PE : EtOAc =10 : 1 ~ 2 : 1 to afford **I-23** (350.0 mg, 78.6%) as an orange solid. LCMS (ESI) m/z: [M+H] ⁺ = 497.

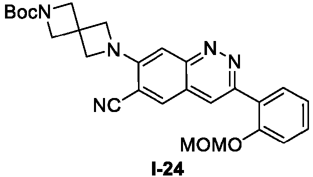

tert-butyl 6-(6-cyano-3-(2-(methoxymethoxy)phenyl)cinnolin-7-yl)-2,6-diazaspiro[3.3]heptane-2-carboxylate (**I-24**) was prepared using a similar procedure as **I-22** using **I-23.**

### Preparation of tert-butyl 6-(6-ethoxy-3-(2-(methoxymethoxy)phenyl)cinnolin-7-yl)-2,6-diazaspiro[3.3]heptane-2-carboxylate (I-25)

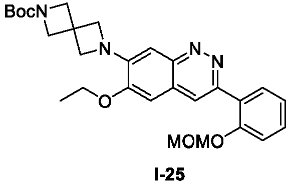

To a stirred solution of **I-23** (300.0 mg, 0.604 mmol, 1 equiv) and AcONa (149.0 mg, 1.812 mmol, 3 equiv) in 1,4-dioxane (2 ml) and EtOH (2 mL) were added di-tert-butyl[2',4',6'-tris(propan-2-yl)-[1,1'-biphenyl]-2-yl]phosphane (51.2 mg, 0.121 mmol, 0.2 equiv) and Pd₂(dba)₃ (110.5 mg, 0.121 mmol, 0.2 equiv). The resulting mixture was stirred for 2 h at 80°C under nitrogen atmosphere. The resulting mixture was concentrated under reduced pressure. The residue was purified by silica gel column chromatography, eluted with PE / EtOAc (1:1) to afford **I-25** (210.0 mg, 68.6%) as a yellow solid. LCMS (ESI) m/z [M+H]⁺ = 507.

### Preparation of tert-butyl 6-{6-cyclopropoxy-3-[2-(methoxymethoxy)phenyl]cinnolin-7-yl}-2,6-diazaspiro[3.3]heptane-2-carboxylate (I-26).

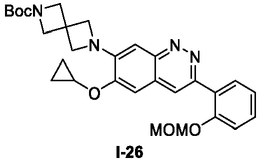

To a solution of **I-23** (200 mg, 0.402 mmol, 1 equiv) and Pd₂(dba)₃ (36.85 mg, 0.040 mmol, 0.1 equiv) in dioxane (1.2 mL, 14.165 mmol) and cyclopropanol (1.2 mL) were added CH₃COONa (99.03 mg, 1.206 mmol, 3 equiv) and t-BuXPhos (34.18 mg, 0.080 mmol, 0.2 equiv). The resulting solution was stirred at 80 degrees C for 16 hours (under N₂ atmosphere). The mixture was diluted with EtOAc (150 mL) and washed with water (150 mL x 3). The organic layer was dried over anhydrous sodium sulfate, filtered, and concentrated to give a crude product. The crude product was purified by silica gel column chromatography, eluted with 0 to 50% EtOAc in petroleum ether to afford **I-26** (61 mg, 29.23%) as a yellow solid. LCMS (ESI) m/z: [M+H]⁺ =519.

### Preparation of 2-(7-{2,6-diazaspiro [3.3] heptan-2-yl}-6-(dimethylamino)cinnolin-3-yl)phenol (1-27).

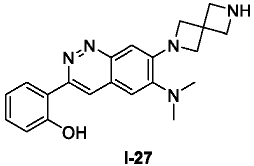

To a stirred solution of **I-23** (66 mg, 0.131 mmol, 1 equiv) in DCM (2 mL, 31.461 mmol, 241.02 equiv) and TFA (2 mL, 26.926 mmol, 206.28 equiv) was stirred for 2 h at room temperature under air atmosphere. The resulting mixture was concentrated under reduced pressure. This resulted in **I-27** (48 mg, 91.56%) as a red solid. LCMS (ESI) m/z: [M+H] ⁺ = 362.

### Preparation of tert-butyl 6-(6-cyano-3-(2-(methoxymethoxy) phenyl) cin nolin-7-yl)-2,6-diazaspiro[3.3] heptane-2-carboxylate (I-28).

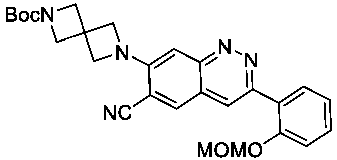

To a solution of **I-23** (350.0 mg, 0.70 mmol, 1.00 equiv) in DMF (7 mL) was added XPhos (67.1 mg, 0.14 mmol, 0.20 equiv), XPhos Pd G3 (119.2 mg, 0.14 mmol, 0.20 equiv) and Zn(CN)₂ (248.1 mg, 2.11 mmol, 3.00 equiv) at room temperature. After stirring for 2 h at 100 °C. The mixture was diluted with water (20 mL) and extracted with EtOAc (3x 20 mL). The combined organic layers were washed with H₂0 (3 x 20 mL), dried over anhydrous Na₂SO₄. After filtration, the filtrate was concentrated under reduced pressure. The residue was purified by silica gel column chromatography, eluted with PE: EtOAc = 10 : 1 ~ 1 : 1 to afford **I-28** (150.0 mg, 43.7%) as an orange solid. LCMS (ESI) m/z: [M+H] ⁺ = 488.

### Preparation of tert-butyl 6-[6-(difluoromethoxy)-3-[2-(methoxymethoxy)phenyl]cinnolin-7-yl]-2,6-diazaspiro[3.3]heptane-2-carboxylate (I-30).

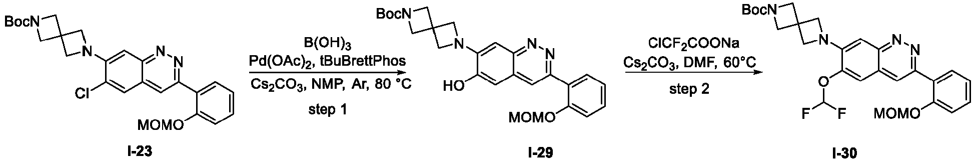

### Step 1: Preparation of tert-butyl 6-{6-hydroxy-3-[2-(methoxymethoxy)phenyl]cinnolin-7-yl}-2,6-diazaspiro[3.3]heptane-2-carboxylate (I-29).

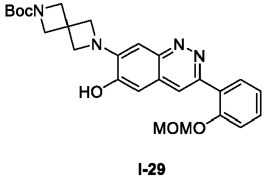

To a stirred solution of **I-23** (923.0 mg, 1.857 mmol, 1 equiv) and KOH (0.31 g, 5.571 mmol, 3 equiv) in 1,4-dioxane (5 mL) and H₂O (5 mL) were added Pd₂(dba)₃ (0.34 g, 0.371 mmol, 0.2 equiv) and di-tert-butyl[2',4',6'-tris(propan-2-yl)-[1,1'-biphenyl]-2-yl]phosphane (0.16 g, 0.371 mmol, 0.2 equiv). The resulting mixture was stirred for 2h at 80°C under nitrogen atmosphere. The resulting mixture was concentrated under vacuum, the residue was purified by silica gel column chromatography, eluted with CH₂Cl₂ / MeOH (12:1) to afford **I-29** (780.0 mg, 87.7%) as a brown solid. LCMS (ESI) m/z: [M+H]⁺ =479.

### Step 2: Preparation of tert-butyl 6-[6-(difluoromethoxy)-3-[2-(methoxymethoxy)phenyl]cinnolin-7-yl]-2,6-diazaspiro[3.3]heptane-2-carboxylate (I-30).

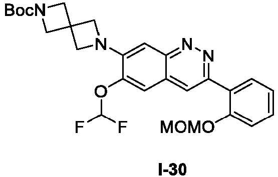

To a stirred mixture of **I-29** (300.0 mg, 0.627 mmol, 1 equiv) and Cs₂CO₃ (612.76 mg, 1.881 mmol, 3 equiv) in DMF (6 mL) was added sodium 2-chloro-2,2-difluoroacetate (286.7 mg, 1.881 mmol, 3 equiv). The resulting mixture was stirred for 2 h at 60°C. The resulting mixture was extracted with EtOAc (2 x 100 mL). The combined organic layers were washed with brine (3 x 100 mL), dried over anhydrous sodium sulfate. After filtration, the filtrate was concentrated under reduced pressure. The residue was purified by silica gel column chromatography, eluted with PE / EtOAc (6:1) to afford **I-30** (140.0 mg, 42.2%) as a yellow solid. LCMS (ESI) m/z: [M+H]⁺ = 529.

### Preparation of tert-butyl 6-{6-ethynyl-3-[2-(methoxymethoxy)phenyl]cinnolin-7-yl}-2,6-diazaspiro[3.3]heptane-2-carboxylate (I-31).

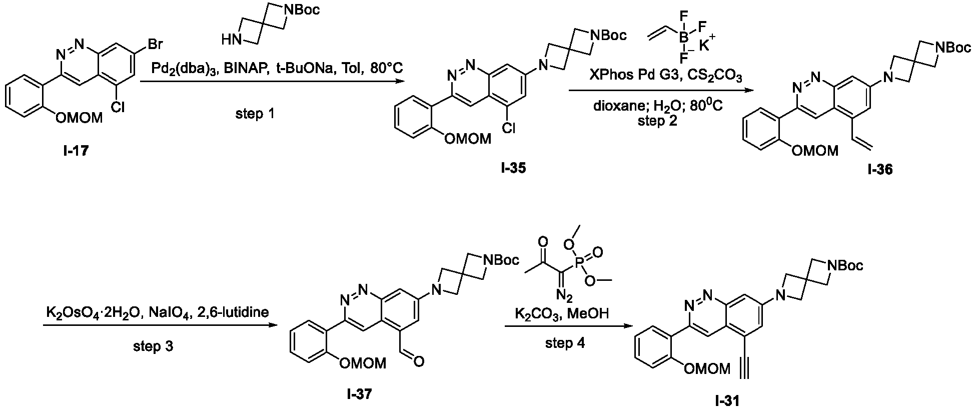

### Step 1: Preparation of tert-butyl 6-{5-chloro-3-[2-(methoxymethoxy)phenyl]cinnolin-7-yl}-2,6-diazaspiro[3.3]heptane-2-carboxylate (I-35)

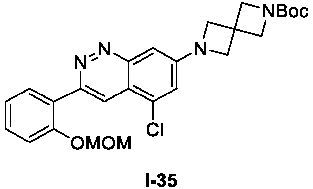

To a solution of **I-17** (1.00 g, 2.634 mmol, 1 equiv), tert-butyl 2,6-diazaspiro[3.3]heptane-2-carboxylate (0.52 g, 2.634 mmol, 1 equiv) and BINAP (0.33 g, 0.527 mmol, 0.2 equiv) in toluene (12 mL) was added Pd₂(dba)₃ (0.48 g, 0.527 mmol, 0.2 equiv) and t-BuONa (0.51 g, 5.268 mmol, 2 equiv), the mixture was stirred for 2 h at 80 °C under nitrogen atmosphere. The resulting mixture was concentrated under reduced pressure. The residue was purified by silica gel column chromatography, eluted with PE / EtOAc (5:1) to afford **I-35** (1.00 g, 76.9%) as a yellow solid. LCMS (ESI) m/z: [M+H]⁺ =497.

### Step 2: Preparation of tert-butyl 6-{5-ethenyl-3-[2-(methoxymethoxy)phenyljcinnolin-7-yl}-2,6-diazaspiro[3.3]heptane-2-carboxylate (I-36).

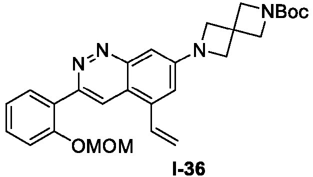

To a solution of **I-35** (1.00 g, 2.012 mmol, 1 equiv) and ethenyldifluoro-lambda4-boranyl)-lambda2-fluoranide (0.19 g, 2.012 mmol, 1 equiv) in H₂O (2 mL) and dioxane (10 mL) was added XPhos Pd G3 (0.34 g, 0.402 mmol, 0.2 equiv) and Cs₂CO₃ (1.97 g, 6.036 mmol, 3 equiv), the mixtue was stirred for 2 h at 80°C under nitrogen atmosphere. The resulting mixture was concentrated under reduced pressure. The residue was purified by silica gel column chromatography, eluted with MeOH / H₂O (5:1) to afford **I-36** (900.0 mg, 91.8%) as a yellow solid. LCMS (ESI) m/z: [M+H]⁺ =489.

### Step 3: Preparation of tert-butyl 6-{6-formyl-3-[2-(methoxymethoxy)phenyl]cinnolin-7-yl}-2,6-diazaspiro[3.3]heptane-2-carboxylate (I-37).

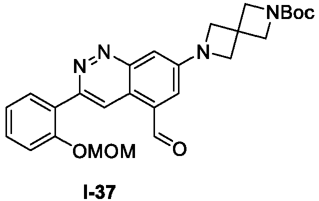

To a solution of **I-36** (840.0 mg, 1.719 mmol, 1 equiv) and 2,6-lutidine (368.5 mg, 3.438 mmol, 2 equiv) in dioxane (60 mL) and H₂O (30 mL) was added NaIO₄ (1470.9 mg, 6.876 mmol, 4 equiv) and K₂OsO₄.2H₂O (50.9 mg, 0.138 mmol, 0.08 equiv), the mixture was stirred for an hour at room temperature. The mixture was diluted with water (50 mL), extracted with EtOAc (3 x100 mL). The combined organic layers were washed with brine (3 x 100 mL), dried over anhydrous sodium sulfate. After filtration, the filtrate was concentrated under reduced pressure. The residue was purified by silica gel column chromatography, eluted with PE / EtOAc (5:1) to afford **I-37** (400.0 mg, 47.4%) as a yellow solid. LCMS (ESI) m/z: [M+H]⁺ =491.

### Step 4: Preparation of tert-butyl 6-{6-ethynyl-3-[2-(methoxymethoxy)phenyl]cinnolin-7-yl}-2,6-diazaspiro[3.3]heptane-2-carboxylate (I-31).

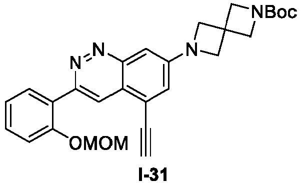

To a solution of intermediate **4** (400.0 mg, 0.815 mmol, 1 equiv) and dimethyl (1-diazo-2-oxopropyl)phosphonate (234.9 mg, 1.222 mmol, 1.5 equiv) in MeOH (6 mL) was added K₂CO₃ (338.1 mg, 2.445 mmol, 3 equiv), the mixture was stirred for 3 h at room temperature. The residue was purified by silica gel column chromatography, eluted with PE / EtOAc (5:1) to afford **I-31** (220.0 mg, 55.4%) as a yellow solid. LCMS (ESI) m/z: [M+H]⁺ =487.

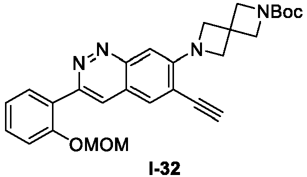

### tert-butyl 6-{6-ethynyl-3-[2-(methoxymethoxy)phenyl]cinnolin-7-yl}-2,6-diazaspiro[3.3]heptane-2-carboxylate (I-32) was prepared in a similar manner as described in the preparation of I-31. LCMS (ESI) m/z: [M+H]⁺ =487.

### Preparation of 7-chloro-6-(difluoromethyl)-3-[2-(methoxymethoxy)phenyl]cinnoline (I-33).

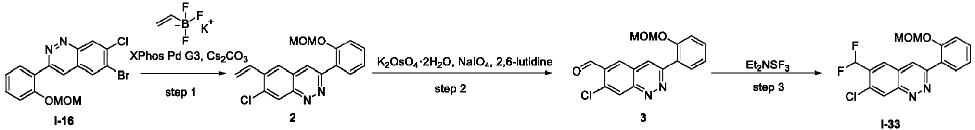

### Step 1: Preparation of 7-chloro-6-ethenyl-3-[2-(methoxymethoxy)phenyl]cinnoline (Intermediate 2).

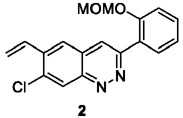

To a stirred solution of I-21 (500.0 mg, 1.317 mmol, 1 equiv) and Potassium vinyltrifluoroborate (352.9 mg, 2.634 mmol, 2 equiv) in dioxane (7.5 mL) and H₂O (1.5 mL) were added XPhos Pd G3 (222.9 mg, 0.263 mmol, 0.2 equiv) and Cs₂CO₃ (1287.3 mg, 3.951 mmol, 3 equiv). The resulting mixture was stirred overnight at 80 °C under nitrogen atmosphere. The mixture was concentrated under vacuum. The residue was purified by silica gel column chromatography, eluted with PE / EA (9:1) to afford intermediate 2 (350.0 mg, 81.2%) as a light yellow solid. LCMS (ESI) m/z: [M+H]⁺ = 327.

### Step 2: Preparation of 7-chloro-3-[2-(methoxymethoxy)phenyl]cinnoline-6-carbaldehyde (Intermediate 3).

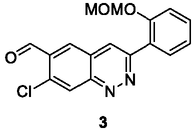

To a stirred solution of intermediate **2** (350 mg, 1.070 mmol, 1 equiv) and NaIO₄ (924.6mg, 4.281 mmol, 4 equiv) in dioxane (3 mL) and H₂O (3 mL) were added K₂OsO₄.2H₂O (18.1 mg, 0.053 mmol, 0.05 equiv) and 2,6-lutidine (228.9 mg, 2.140 mmol, 2 equiv) at 0 °C. The resulting mixture was stirred for 30 mins at room temperature. The reaction was quenched with sat. sodium hyposulfite (aq.) at 0 °C. The resulting mixture was extracted with EtOAc (3 x 50 mL). The combined organic layers were washed with brine (2 x 20 mL), dried over anhydrous sodium sulfate. After filtration, the filtrate was concentrated under reduced pressure. The crude product was purified by Prep-TLC (PE / EtOAc 1:1) to afford intermediate **3** (300 mg, 85.2%) as a light yellow solid. LCMS (ESI) m/z: [M+H]⁺ =329.

### Step 3: Preparation of 7-chloro-6-(difluoromethyl)-3-[2-(methoxymethoxy)phenyl]cinnoline (I-33).

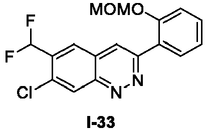

A solution of intermediate **3** (300 mg, 0.911 mmol, 1 equiv) in DCM (5 mL) was added BAST (1 mL) at 0 °C. The resulting mixture was stirred for 1 h at 0 °C. The reaction was quenched with sat. NH₄Cl (aq.) at 0 °C. The resulting mixture was extracted with EtOAc (3 x 20 mL). The combined organic layers were washed with brine (2 x 20 mL), dried over anhydrous sodium sulfate. After filtration, the filtrate was concentrated under reduced pressure. The residue was purified by Prep-TLC (PE / EtOAc 1:1) to afford **I-33** (250.0 mg, 78.1%) as a light yellow solid. LCMS (ESI) m/z: [M+H]⁺ = 351.

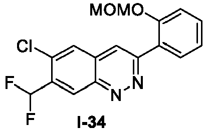

6-chloro-7-(difluoromethyl)-3-(2-(methoxymethoxy)phenyl)cinnoline (**I-34**) was prepared using a similar procedure as above from **I-33.**

### Preparation of tert-butyl 6-[5-(difluoromethyl)-3-[2-(methoxymethoxy)phenyl]cinnolin-7-yl]-2,6-diazaspiro[3.3]heptane-2-carboxylate (I-38)

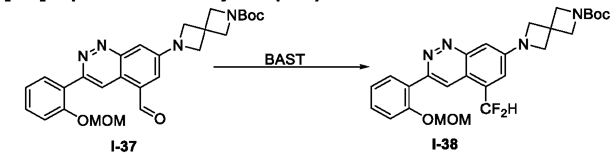

To a stirred solution of **I-37** (440.0 mg, 0.897 mmol, 1 equiv) in DCM (5 ml) was added BAST (1 mL) at 0°C. The resulting mixture was stirred for 30 mins at 0°C. The reaction was quenched with sat. NH₄Cl (aq.) at 0°C. The aqueous layer was extracted with EtOAc (2 x 200 mL), dried over anhydrous sodium sulfate. After filtration, the filtrate was concentrated under reduced pressure. The residue was purified by silica gel column chromatography, eluted with PE / EA (1:1) to afford **I-38** (160.0 mg, 34.8%) as a yellow solid. LCMS (ESI) m/z: [M+H]⁺ = 513.

### Preparation of 2-(6-cyclopropyl-7-{2,6-diazaspiro[3.3]heptan-2-yl}cinnolin-3-yl)phenol (I-39)

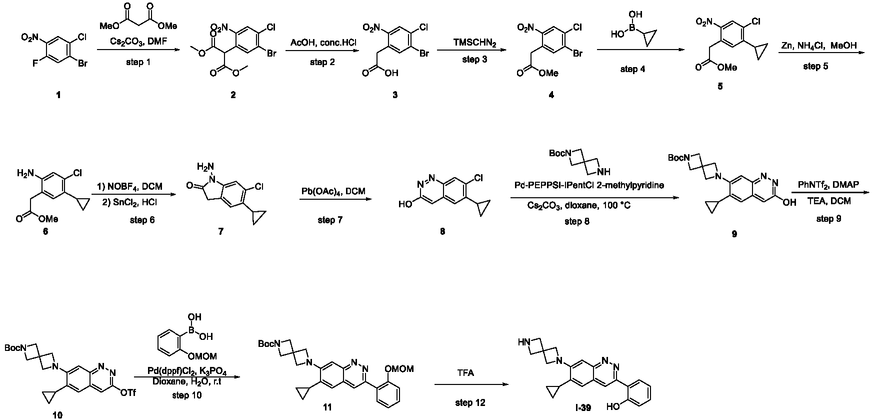

### Step 1: Preparation of 1,3-dimethyl 2-(5-bromo-4-chloro-2-nitrophenyl)propanedioate (Intermediate 2)

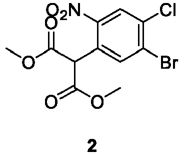

A solution of 1-bromo-2-chloro-5-fluoro-4-nitrobenzene (20 g, 78.604 mmol, 1 equiv) and dimethyl malonate (11.42 g, 86.464 mmol, 1.1 equiv), Cs₂CO₃ (51.22 g, 157.208 mmol, 2.0 equiv) in DMF (50 mL) was stirred for 12 h at room temperature. The mixture was acidified to pH 6 with 1 N HCl (aq.). The resulting mixture was diluted with water (200 mL). The resulting mixture was extracted with EtOAc (3 x 200 mL). The combined organic layers were washed with brine (3 x 300 mL), dried over anhydrous sodium sulfate. After filtration, the filtrate was concentrated under reduced pressure. This resulted in Intermediate **2** (20 g, 69.4%) as a yellow solid. The crude product was used in the next step directly without further purification. LCMS (ESI) m/z [M+H]⁺ =366.

### Step 2: Preparation of (5-bromo-4-chloro-2-nitrophenyl)acetic acid (Intermediate 3)

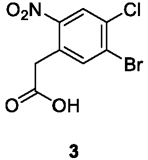

A solution of Intermediate **2** (20 g, 54.563 mmol, 1 equiv) and AcOH (250 mL) in HCl (250 mL) was stirred for 12 h at 100 °C. The resulting mixture was diluted with water (200 mL). The precipitated solids were collected by filtration and washed with water (3 x 200 mL). The resulting mixture was concentrated under reduced pressure. This resulted in Intermediate **3** (15 g, crude) as a yellow solid. The crude product was used in the next step directly without further purification. LCMS (ESI) m/z [M+H]⁺ =294.

### Step 3: Preparation of methyl 2-(5-bromo-4-chloro-2-nitrophenyl)acetate (Intermediate 4)

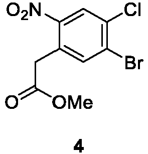

To a stirred solution of Intermediate **3** (15 g, 50.936 mmol, 1 equiv) in MeOH (100 mL) and DCM (400 mL) was added TMSCHN₂ (34.91 g, 152.807 mmol, 3 equiv) dropwise at 0 °C. The resulting mixture was stirred for 2h at room temperature. The resulting mixture was concentrated under reduced pressure. This resulted in Intermediate **4** (16 g, crude) as a light yellow solid. The crude product was used in the next step directly without further purification. LCMS (ESI) m/z [M+H]⁺ =308.

### Step 4: Preparation of methyl 2-(4-chloro-5-cyclopropyl-2-nitrophenyl)acetate (Intermediate 5)

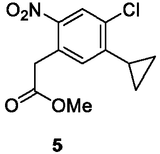

To a solution of Intermediate **4** (3.0 g, 9.724 mmol, 1 equiv) and cyclopropylboronic acid (1.67 g, 19.448 mmol, 2.0 equiv) in dioxane (20 mL) and H₂O (5 mL) were added K₃PO₄ (6.19 g, 29.172 mmol, 3.0 equiv) and Pd(dppf)Cl₂ (1.42 g, 1.945 mmol, 0.2 equiv). After stirring for 2 h at 60 °C under a nitrogen atmosphere, the mixture was concentrated under vacuum, the residue was purified by silica gel column chromatography, eluted with PE / EtOAc (5:1) to afford Intermediate **5** (1.8 g, 68.6%) as a yellow solid. LCMS (ESI) m/z [M+H]⁺ =270.

### Step 5: Preparation of methyl 2-(2-amino-4-chloro-5-cyclopropylphenyl)acetate (Intermediate 6)

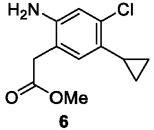

To a stirred solution of Intermediate **5** (1.8 g, 6.675 mmol, 1 equiv) and NH₄Cl (7.14 g, 133.500 mmol, 20 equiv) in MeOH (20 mL) was added Zn (8.73 g, 133.500 mmol, 20 equiv) in portions at 0 °C. The resulting mixture was stirred for 1 h at 0 °C. The resulting mixture was filtered, the filter cake was washed with MeOH (3 x 50 mL). The filtrate was concentrated under reduced pressure. The resulting mixture was diluted with water (100 mL). The resulting mixture was extracted with EtOAc (3 x 100 mL). The combined organic layers were washed with brine (3 x 100 mL), dried over anhydrous sodium sulfate. After filtration, the filtrate was concentrated under reduced pressure to afford Intermediate **6** (1.4 g, crude) as yellow oil. The crude product was used in the next step directly without further purification. LCMS (ESI) m/z [M+H]⁺ =240.

### Step 6: Preparation of 1-amino-6-chloro-5-cyclopropyl-3H-indol-2-one (Intermediate 7)

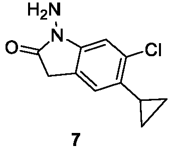

A solution of Intermediate **6** (1.8 g, 7.509 mmol, 1 equiv) and NOBF₄ (1.32 g, 11.264 mmol, 1.5 equiv) in DCM (25 mL) was stirred for 1 h at 0°C. To the above mixture was added SnCl₂ (8.63 g, 45.054 mmol, 6.0 equiv) in HCl (30mL) dropwise at 0°C. The resulting mixture was stirred for additional 12 h at room temperature. The resulting mixture was concentrated under reduced pressure. The residue was purified by reversed-phase flash chromatography with the following conditions: column, C₁₈ silica gel; mobile phase, MeCN in Water (0.1% FA), 0% to 50% gradient in 50 min; detector, UV 254 nm. This resulted in Intermediate **7** (700.0 mg, 41.8%) as a yellow solid. LCMS (ESI) m/z [M+H]⁺ =223.

### Step 7: Preparation of tert-butyl 6-(6-cyclopropyl-3-hydroxycinnolin-7-yl)-2,6-diazaspiro[3.3]heptane-2-carboxylate (Intermediate 8)

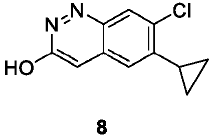

To a solution of Intermediate **7** (300.0 mg, 1.360 mmol, 1 equiv) and tert-butyl 2,6-diazaspiro[3.3]heptane-2-carboxylate (539.1 mg, 2.720 mmol, 2.0 equiv) in dioxane (5 mL) were added Cs₂CO₃ (1328.9 mg, 4.080 mmol, 3.0 equiv) and Pd-PEPPSI-IPentCl 2-methylpyridine (215.5 mg, 0.272 mmol, 0.2 equiv) . After stirring for 2 h at 100 °C under a nitrogen atmosphere, the resulting mixture was concentrated under reduced pressure. The residue was purified by reversed-phase flash chromatography with the following conditions: column, C₁₈ silica gel; mobile phase, MeCN in Water (0.1% FA), 0% to 50% gradient in 50 min; detector, UV 254 nm. This resulted in Intermediate **8** (250.0 mg, 48.0%) as a yellow solid. LCMS (ESI) m/z [M+H]⁺ =221.

### Step 8: Preparation of tert-butyl 6-(6-cyclopropyl-3-hydroxycinnolin-7-yl)-2,6-diazaspiro[3.3]heptane-2-carboxylate (Intermediate 9)

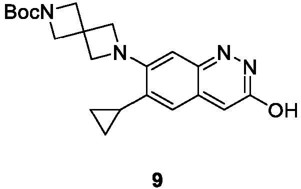

To a solution of Intermediate **8** (300.0 mg, 1.360 mmol, 1 equiv) and tert-butyl 2,6-diazaspiro[3.3]heptane-2-carboxylate (539.1 mg, 2.720 mmol, 2.0 equiv) in dioxane (5 mL) were added Cs₂CO₃ (1328.9 mg, 4.080 mmol, 3.0 equiv) and Pd-PEPPSI-IPentCl 2-methylpyridine (215.5 mg, 0.272 mmol, 0.2 equiv) . After stirring for 2 h at 100 °C under a nitrogen atmosphere, the resulting mixture was concentrated under reduced pressure. The residue was purified by reversed-phase flash chromatography with the following conditions: column, C₁₈ silica gel; mobile phase, MeCN in Water (0.1% FA), 0% to 50% gradient in 50 min; detector, UV 254 nm. This resulted in Intermediate **9** (250.0 mg, 48.0%) as a yellow solid. LCMS (ESI) m/z [M+H]⁺ =383.

### Step 9: Preparation of tert-butyl 6-[6-cyclopropyl-3-(trifluoromethanesulfonyloxy)cinnolin-7-yl]-2,6-diazaspiro[3.3]heptane-2-carboxylate (Intermediate 10)

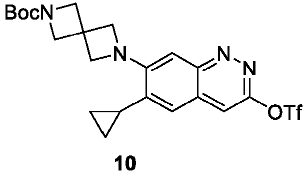

To a solution of Intermediate **9** (250.0 mg, 0.654 mmol, 1 equiv) and 1,1,1-trifluoro-N-phenyl-N-trifluoromethanesulfonylmethanesulfonamide (467.0 mg, 1.308 mmol, 2.0 equiv) in DCM (5 mL) was added TEA (198.4 mg, 1.962 mmol, 3.0 equiv) and DMAP (7.9 mg, 0.065 mmol, 0.1 equiv), the mixture was stirred for 1 h at room temperature. The mixture was concentrated under vacuum, the residue was purified by silica gel column chromatography, eluted with PE / EA (23:67) to afford Intermediate 10 (270.0 mg, 80.2%) as yellow oil. LCMS (ESI) m/z [M+H]⁺ =515.

### Step 10: Preparation of tert-butyl 6-[6-cyclopropyl-3-(2-hydroxyphenyl)cinnolin-7-yl]-2,6-diazaspiro[3.3]heptane-2-carboxylate (Intermediate 11)

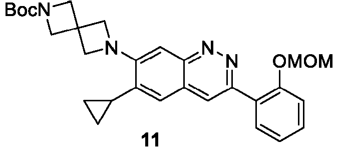

To a solution of Intermediate **10** (210.0 mg, 0.408 mmol, 1 equiv) and 2-hydroxyphenylboronic acid (168.8 mg, 1.224 mmol, 3.0 equiv) in dioxane (4 mL) and H₂O (1 mL) were added K₃PO₄ (259.4 mg, 1.224 mmol, 3.0 equiv) and Pd(dppf)Cl₂ (59.4 mg, 0.082 mmol, 0.2 equiv). After stirring for 1 h at 60 °C under a nitrogen atmosphere, the resulting mixture was concentrated under reduced pressure. The residue was purified by reversed-phase flash chromatography with the following conditions: column, C₁₈ silica gel; mobile phase, MeCN in Water (0.1% FA), 0% to 60% gradient in 40 min; detector, UV 254 nm. This resulted in Intermediate 11 (100.0 mg, 53.4%) as yellow oil. LCMS (ESI) m/z [M+H]⁺ =503.

### Step 11: Preparation of 2-(6-cyclopropyl-7-{2,6-diazaspiro[3.3]heptan-2-yl}cinnolin-3-yl)phenol (I-39)

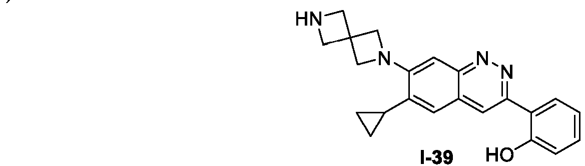

A solution of Intermediate **11** (70.0 mg, 0.153 mmol, 1 equiv) and TFA (0.5 mL) in DCM (2 mL) was stirred for 1 h at room temperature. The resulting mixture was concentrated under reduced pressure. This resulted in **I-39** (90 mg, crude) as a red solid. The crude product was used in the next step directly without further purification. LCMS (ESI) m/z [M+H]⁺ =359.

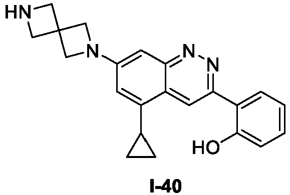

2-(5-cyclopropyl-7- {2,6-diazaspiro [3.3] heptan-2-yl} cinnolin-3-yl} phenol (**I-40**) was prepared in a similar manner as described in the preparation of intermediate **I-39.** LCMS (ESI) m/z [M+H]⁺ =359.

### Preparation of 2-(7-{2,6-diazaspiro[3.3]heptan-2-yl}-5-methylcinnolin-3-yl)phenol (I-41).

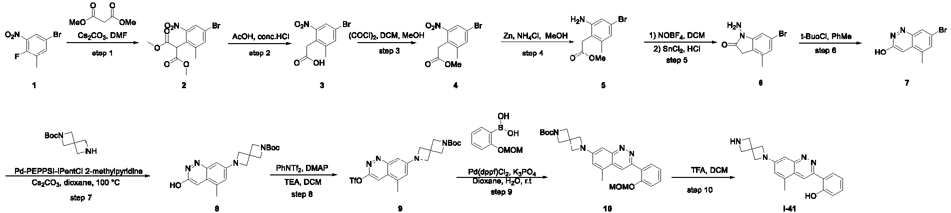

### Step1: Preparation of 1,3-dimethyl 2-(4-bromo-2-methyl-6-nitrophenyl)propanedioate (Intermediate 2)

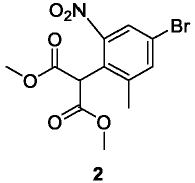

A solution of 5-bromo-2-fluoro-1-methyl-3-nitrobenzene (30.00 g, 128.192 mmol, 1.00 equiv) and dimethyl malonate (16.94 g, 128.192 mmol, 1.00 equiv) in DMF (300 mL) was stirred overnight at room temperature. The resulting mixture was diluted with water (2 L). The resulting mixture was extracted with EtOAc (3 x 1 L). The combined organic layers were washed with brine (3 x 1 L), dried over anhydrous Na₂SO₄. After filtration, the filtrate was concentrated under reduced pressure to afford intermediate **2** (44.00 g, 96.1%) as a light yellow solid. The crude product was used in the next step directly without further purification. LCMS (ESI) m/z: [M+H]⁺ =246.

### Step 2: Preparation of (4-bromo-2-methyl-6-nitrophenyl)acetic acid (Intermediate 3).

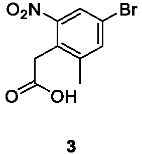

A solution of intermediate **2** (44.00 g, 127.119 mmol, 1.00 equiv) and AcOH (300 mL) in conc.HCl (300 mL) was stirred overnight at 100°C. The precipitated solids were collected by filtration and washed with water (3 x 300 mL). This resulted in intermediate **3** (33.00 g, 92.8%) as a white solid. The crude product was used in the next step directly without further purification. LCMS (ESI) m/z: [M+H]⁺ =274.

### Step 3: Preparation of methyl 2-(4-bromo-2-methyl-6-nitrophenyl)acetate (Intermediate 4).

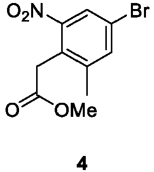

To a stirred solution of intermediate **3** (33.00 g, 120.407 mmol, 1.00 equiv) in DCM (300 mL) was added (COCl)₂ (30.56 g, 240.814 mmol, 2.00 equiv) dropwise at 0°C under nitrogen atmosphere. The resulting mixture was stirred for 1.5 h at room temperature under nitrogen atmosphere. To the above mixture was added MeOH (165 mL) dropwise over 10 min at 0°C. The resulting mixture was stirred for an additional 1 h at room temperature. The resulting mixture was concentrated under reduced pressure. The resulting mixture was diluted with water (1 L). The resulting mixture was extracted with EtOAc (3 x 700 mL). The combined organic layers were washed with sodium bicarbonate solution (3 x 1 L), dried over anhydrous Na₂SO₄. After filtration, the filtrate was concentrated under reduced pressure to afford intermediate **4** (35.00 g, 98.88%) as light-yellow oil. The crude product was used in the next step directly without further purification. LCMS (ESI) m/z: [M+H]⁺ =288.

### Step 4: Preparation of methyl 2-(2-amino-4-bromo-6-methylphenyl)acetate (Intermediate 5).

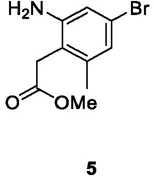

To a stirred solution of intermediate **4** (35.00 g, 121.487 mmol, 1.00 equiv) and NH₄Cl (129.97 g, 2429.740 mmol, 20.00 equiv) in MeOH (400 mL) was added Zn (119.14 g, 1822.305 mmol, 15.00 equiv) at 0°C. The resulting mixture was stirred for 30 mins at 0°C. The resulting mixture was filtered, the filter cake was washed with MeOH (3 x 300 mL). The filtrate was concentrated under reduced pressure. The resulting mixture was diluted with water (1 L). The resulting mixture was extracted with EtOAc (3 x 600 mL). The combined organic layers were washed with brine (3 x 500 mL), dried over anhydrous Na₂SO₄. After filtration, the filtrate was concentrated under reduced pressure to afford intermediate **5** (32.00 g, 63.27%) as a light-yellow solid. The crude product was used in the next step directly without further purification. LCMS(ESI) m/z: [M+H]⁺ =258.

### Step 5: Preparation of 1-amino-6-bromo-4-methyl-3H-indol-2-one (Intermediate 6).

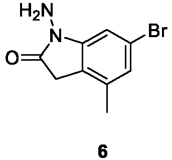

To a stirred solution of intermediate **5** (32.00 g, 123.976 mmol, 1.00 equiv) in DCM (320 mL) was added NOBF₄ (21.72 g, 185.964 mmol, 1.50 equiv) at 0°C. The resulting mixture was stirred for 2 h at 0°C. To the above mixture was added SnCl₂ (118.79 g, 619.880 mmol, 5.00 equiv) and HCl (320 mL) in portions over 10 min at 0°C. The resulting mixture was stirred overnight at room temperature. The precipitated solids were collected by filtration and washed with water (3 x 300 mL). This resulted in intermediate **6** (13.00 g, 43.06%) as a white solid. The crude product was used in the next step directly without further purification. LCMS (ESI) m/z: [M+H]⁺ =241.

### Step 6: Preparation of 7-bromo-5-methylcinnolin-3-ol (Intermediate 7).

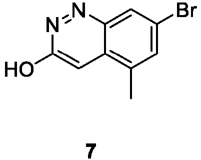

To a stirred solution of intermediate **6** (11.00 g, 45.626 mmol, 1.00 equiv) in toluene (200 mL) was added tert-butyl hypochlorite (3.96 g, 36.501 mmol, 0.80 equiv) dropwise at 0°C. The resulting mixture was stirred for 30 mins at 0°C. The precipitated solids were collected by filtration and washed with PE (3 x 300 mL). This resulted in intermediate **7** (11.70 g, 92.2%) as a yellow solid. The crude product was used in the next step directly without further purification. LCMS (ESI) m/z: [M+H]⁺ =239.

### Step 7: Preparation of tert-butyl 6-(3-hydroxy-5-methylcinnolin-7-yl)-2,6-diazaspiro[3.3]heptane-2-carboxylate (Intermediate 8).

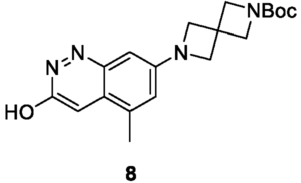

To a solution of intermediate **7** (2.00 g, 8.366 mmol, 1.00 equiv), tert-butyl 2,6-diazaspiro[3.3]heptane-2-carboxylate (3.32 g, 16.732 mmol, 2.00 equiv) and Cs₂CO₃ (8.18 g, 25.098 mmol, 3.00 equiv) in dioxane (25 mL) was added {1,3-bis[2,6-bis(pentan-3-yl)phenyl]-4,5-dichloro-2,3-dihydro-1H-imidazol-2-yl}dichloro(2-methyl-1lambda4-pyridin-1-yl)palladium (351.8 mg, 0.418 mmol, 0.05 equiv), the mixture was stirred overnight at 100°C under nitrogen atmosphere. The resulting mixture was diluted with water (500 mL). The resulting mixture was extracted with EtOAc (3 x 300 mL). The combined organic layers were washed with brine (3 x 400 mL), dried over anhydrous Na₂SO₄. After filtration, the filtrate was concentrated under reduced pressure. The residue was purified by silica gel column chromatography, eluted with CH₂Cl₂ / MeOH (12:1) to afford intermediate **8** (3.40 g, 62.71%) as a black solid. LCMS (ESI) m/z: [M+H]⁺ =257.

### Step 8: Preparation of tert-butyl 6-[5-methyl-3-(trifluoromethanesulfonyloxy)cinnolin-7-yl]-2,6-diazaspiro[3.3]heptane-2-carboxylate (Intermediate 9).

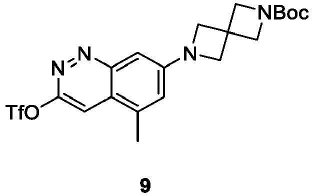

To a stirred solution of intermediate **8** (3.40 g, 9.539 mmol, 1.00 equiv) and TEA (2.90 g, 28.617 mmol, 3.00 equiv) in DCM (40 mL) was added 1,1,1-trifluoro-N-phenyl-N-(trifluoromethane)sulfonylmethanesulfonamide (5.11 g, 14.308 mmol, 1.50 equiv) at 0°C. The resulting mixture was stirred for 1 h at 0°C. The resulting mixture was concentrated under reduced pressure. The residue was diluted with water (1 L). The resulting mixture was extracted with EtOAc (3 x 500 mL). The combined organic layers were washed with brine (3 x 500 mL), dried over anhydrous Na₂SO₄. After filtration, the filtrate was concentrated under reduced pressure. The residue was purified by silica gel column chromatography, eluted with PE / EA (7:3) to afford intermediate **9** (1.9 g, 36.7%) as an orange solid. LCMS (ESI) m/z: [M+H]⁺ =489.

### Step 9: Preparation of tert-butyl 6-{3-[2-(methoxymethoxy)phenyl]-5-methylcinnolin-7-yl}-2,6-diazaspiro[3.3]heptane-2-carboxylate (Intermediate 10).

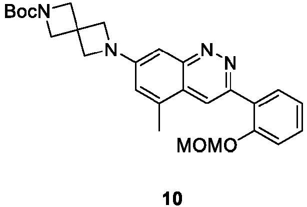

To a stirred solution of intermediate **9** (1.90 g, 3.890 mmol, 1.00 equiv) and 2-(methoxymethoxy)phenylboronic acid (707.8 mg, 3.890 mmol, 1.00 equiv) in dioxane (20 mL) and H₂O (4 mL) were added Pd(dppf)Cl₂ (569.2 mg, 0.778 mmol, 0.20 equiv) and K₃PO₄ (2.48 g, 11.670 mmol, 3.00 equiv). The resulting mixture was stirred for 1 h at room temperature under nitrogen atmosphere. The resulting mixture was diluted with water (500 mL). The aqueous layer was extracted with EtOAc (3 x 400 mL). The combined organic layers were washed with brine (3 x 400 mL), dried over anhydrous Na₂SO₄. After filtration, the filtrate was concentrated under reduced pressure. The residue was purified by silica gel column chromatography, eluted with PE / EA (2:8) to afford intermediate **10** (780.0 mg, 42.1%) as a yellow solid. LCMS (ESI) m/z: [M+H]⁺ =477.

### Step 10: Preparation of 2-(7-{2,6-diazaspiro[3.3]heptan-2-yl}-5-methylcinnolin-3-yl)phenol (I-41).

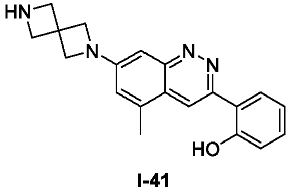

A solution of intermediate **10** (770.0 mg, 1.616 mmol, 1.00 equiv) in DCM (8 mL) and TFA (2 mL) was stirred for 1 h at room temperature. The resulting mixture was concentrated under reduced pressure. The residue was purified by reversed-phase flash chromatography with the following conditions: column, C₁₈ silica gel; mobile phase, MeOH in Water (0.05% TFA), 0% to 100% gradient in 30 min; detector, UV 254/220 nm. The resulting mixture was concentrated under vacuum. This resulted in **I-41** (374.0 mg, 62.6%) as a red solid. LCMS (ESI) m/z: [M+H]⁺ =333.

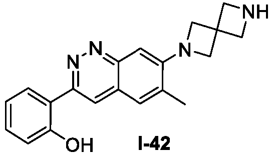

2-(6-methyl-7-(2,6-diazaspiro[3.3] heptan-2-yl) cinnolin-3-yl) phenol **(I-42)** was prepared in a similar manner as described in the preparation of intermediate **I-41.** LCMS (ESI) m/z: [M+H]⁺ =333.

### Preparation of 2-(7- {2-azaspiro [3.3] heptan-6-yl} cinnolin-3-yl) phenol (I-43)

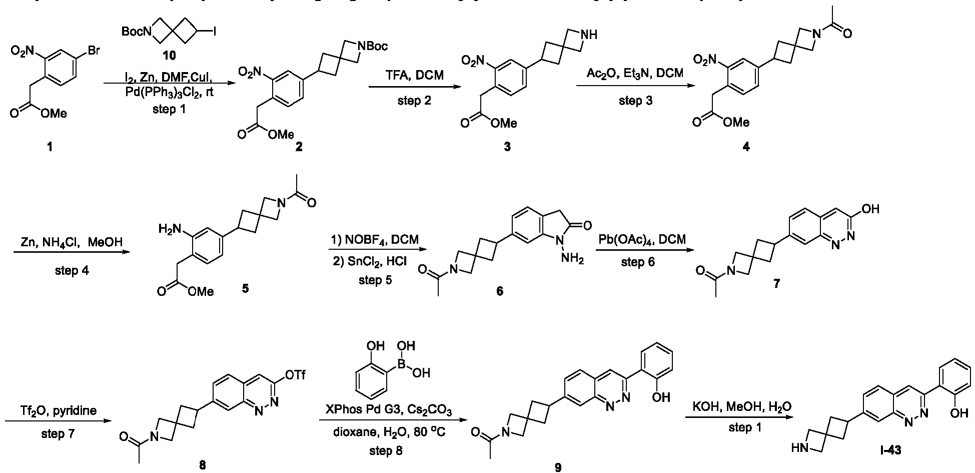

### Step 1: ethyl (2E)-3-(4-amino-6-chloropyridazin-3-yl) prop-2-enoate (Intermediate 2)

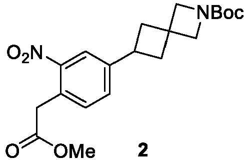

A solution of tert-butyl 6-iodo-2-azaspiro [3.3] heptane-2-carboxylate (4.99 g, 15.433 mmol, 1.5 equiv), I₂ (1.31 g, 5.144 mmol, 0.5 equiv) and Zn (71.57 mg, 1.095 mmol, 3 equiv) in DMF (20 mL) was stirred for 1.5 h at room temperature under nitrogen atmosphere. To the resulting solution were added methyl 2-(4-bromo-2-nitrophenyl) acetate (2.82 g, 10.289 mmol, 1 equiv), Cul (0.98 g, 5.144 mmol, 0.5 equiv) and Pd(PPh₃)₂Cl₂ (1.44 g, 2.058 mmol, 0.2 equiv) at room temperature under nitrogen atmosphere. The final reaction mixture was stirred overnight at room temperature under nitrogen atmosphere. Desired product could be detected by LCMS. The residue was purified by silica gel column chromatography, eluted with PE / EA (2:1) to afford Intermediate **2** (1.87 g, 46.55%) as a yellow oil. LCMS (ESI) m/z [M+H] ⁺ =390.

### Step 2: Preparation of methyl 2-(4- {2-azaspiro [3.3] heptan-6-yl}-2-nitrophenyl) acetate (Intermediate 3)

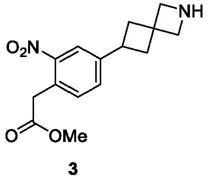

A mixture of Intermediate **2** (1.87 g, 4.790 mmol, 1 equiv) and TFA (5 mL) in DCM (15 mL) was stirred for 1 h at room temperature. Desired product could be detected by LCMS. The resulting mixture was concentrated under reduced pressure. The crude product was used in the next step directly without further purification. LCMS (ESI) m/z [M+H] ⁺ =290.

### Step 3: Preparation of methyl 2-(4- {2-acetyl-2-azaspiro [3.3] heptan-6-yl}-2-nitrophenyl) acetate (Intermediate 4)

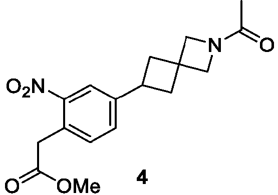

A mixture of Intermediate **3** (1.37 g, 4.719 mmol, 1 equiv) and acetic anhydride (0.96 g, 9.438 mmol, 2 equiv) in DCM (13 mL) was stirred for 50 min at room temperature. The residue was purified by silica gel column chromatography, eluted with CH₂Cl₂ / MeOH (9:1) to afford Intermediate **4** (1.44 g, 91.82%) as a yellow oil. LCMS (ESI) m/z: [M+H] ⁺ =332.

### Step 4: Preparation of methyl 2-(4-(2-acetyl-2-azaspiro [3.3] heptan-6-yl)-2-aminophenyl) acetate (Intermediate 5)

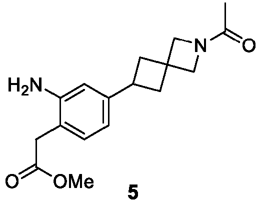

To a stirred mixture of Intermediate **4** (1.44 g, 4.333 mmol, 1 equiv) and NH₄Cl (4.64 g, 86.660 mmol, 20 equiv) in MeOH (15 mL) was added Zn (5.67 g, 86.660 mmol, 20 equiv) in portions at 0 °C. The resulting mixture was stirred for 1 h at room temperature. Desired product could be detected by LCMS. The residue was dissolved in EtOAc (100 mL). The resulting mixture was filtered the filter cake was washed with EtOAc (3x5 mL). The resulting mixture was washed with water (2x50 mL), dried over anhydrous Na₂SO₄. After filtration, the filtrate was concentrated under reduced pressure. The crude product was used in the next step directly without further purification. LCMS (ESI) m/z: [M+H] ⁺ =302.

### Step 5: Preparation of 6- {2-acetyl-2-azaspiro [3.3] heptan-6-yl}-1-amino-3H-indol-2-one (Intermediate 6)

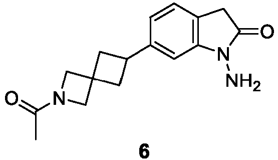

To a stirred mixture of Intermediate **5** (1.127 g, 3.727 mmol, 1 equiv) was suspended in DCM (12 mL) at 0 °C. NOBF₄ (0.65 g, 5.590 mmol, 1.5 equiv) was added in one portion to the mixture. The mixture was stirred for 1 h at 0°C. The mixture was added directly to a vigorously stirred mixture of SnCl₂ (5.71 g, 29.816 mmol, 8 equiv) in HCl (6 mL, 197.477 mmol, 181.49 equiv) at 0°C. The resulting mixture was stirred for overnight at 30 °C. Desired product could be detected by LCMS. The resulting mixture was concentrated under reduced pressure. The residue was purified by reverse flash chromatography with the following conditions: column, C18 silica gel; mobile phase, MeCN in Water, 0% to 40% gradient in 30 min; detector, UV 254 nm. This resulted in Intermediate **6** (520 mg, 48.89%) as a light yellow solid. LCMS (ESI) m/z: [M+H] ⁺ =285.

### Step 6: Preparation of 1-[6-(3-hydroxycinnolin-7-yl)-2-azaspiro [3.3] heptan-2-yl] ethanone (Intermediate 7)

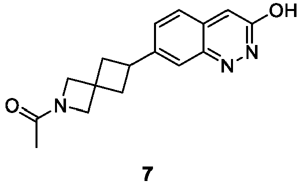

A solution of Intermediate **6** (500 mg, 1.752 mmol, 1 equiv) and Pb(OAc)₄ (932.34 mg, 2.102 mmol, 1.2 equiv) in DCM (6 mL) was stirred for 20 min at room temperature. Desired product could be detected by LCMS. The resulting mixture was dried in an oven under reduced pressure. The residue was purified by reverse flash chromatography with the following conditions: column, C18 silica gel; mobile phase, MeCN in Water, 0% to 40% gradient in 20 min; detector, UV 254 nm. This resulted in Intermediate 7 (232 mg, 46.73%) as a yellow solid. LCMS (ESI) m/z: [M+H] ⁺ =283.

### Step 7: Preparation of 7-(2-acetyl-2-azaspiro [3.3] heptan-6-yl) cinnolin-3-yl trifluoromethanesulfonate (Intermediate 8)

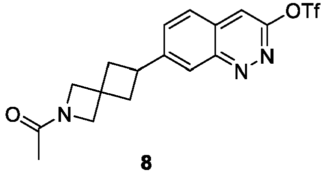

A solution of Intermediate **7** (250 mg, 0.882 mmol, 1 equiv) and Tf₂O (1249.68 mg, 4.429 mmol, 5.02 equiv) in pyridine (6 mL) was stirred for 1 h at room temperature. Desired product could be detected by LCMS. The residue was dissolved in EtOAc (50 mL). The combined organic layers were washed with water (2x30 mL), dried over anhydrous Na₂SO₄. After filtration, the filtrate was concentrated under reduced pressure. The crude product was used in the next step directly without further purification. LCMS (ESI) m/z: [M+H] ⁺ =415.

### Step 8: Preparation of 1-{6-[3-(2-hydroxyphenyl) cinnolin-7-yl]-2-azaspiro [3.3] heptan-2-yl} ethanone (Intermediate 9)

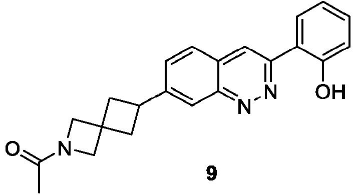

To a stirred mixture of Intermediate **8** (60 mg, 0.072 mmol, 1 equiv, 50%) and 2-hydroxyphenylboronic acid (19.92 mg, 0.144 mmol, 2 equiv) in dioxane (2.5 mL) and H₂O (0.5 mL) were added XPhos Pd G3 (12.23 mg, 0.014 mmol, 0.2 equiv) and Cs₂CO₃ (70.59 mg, 0.216 mmol, 3 equiv). The resulting mixture was stirred for 2 h at 60 °C under nitrogen atmosphere. Desired product could be detected by LCMS. The crude product (60 mg was purified by Chiral-Prep-HPLC with the following conditions (NB-Prep-HPLC-01): Column, Xselect CSH C18 OBD Column 30*150mm 5um; mobile phase, Water (0.1%FA) and ACN (35% ACN up to 58% in 7 min) to afford *Intermediate* **9** (8.9 mg, 34.08%) as a light-yellow solid. ¹H NMR (400 MHz, DMSO-d6) *δ* 12.05 (s, 1H), 8.90 (s, 1H), 8.24 (s, 1H), 8.15 - 8.06 (m, 2H), 7.86 - 7.78 (m, 1H), 7.41 - 7.33 (m, 1H), 7.09 - 7.00 (m, 2H), 4.31 (s, 1H), 4.09 (s, 1H), 4.03 (s, 1H), 3.81 (s, 1H), 3.72 (p, *J* = 8.7 Hz, 1H), 2.75 - 2.64 (m, 2H), 2.50 - 2.42 (m, 2H), 1.76 (d, *J* = 13.2 Hz, 3H). LCMS (ESI) m/z: [M+H] ⁺ =359.16.

### Step 9: Preparation of 2-(7- {2-azaspiro [3.3] heptan-6-yl} cinnolin-3-yl) phenol (I-43)

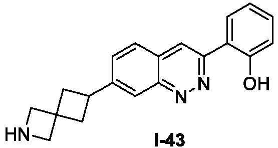

A solution of *Intermediate* ***9*** (122 mg, 0.339 mmol, 1 equiv) and KOH (190.44 mg, 3.390 mmol, 10 equiv) in MeOH (1.5 mL) and H₂O (1.5 mL) was stirred overnight at 70 °C. Desired product could be detected by LCMS. The residue was purified by reverse flash chromatography with the following conditions: column, C18 silica gel; mobile phase, MeCN in Water (0.1% FA), 10% to 60% gradient in 40 min; detector, UV 254 nm. This resulted in **I-43** (102 mg, 94.68%) as an off-white solid. LCMS (ESI) m/z [M+H] ⁺ =317.

### Preparation of 1-{3-[7-cyclopropyl-3-(2-hydroxyphenyl)cinnolin-6-yl]azetidin-1-yl}ethanone (I-44).

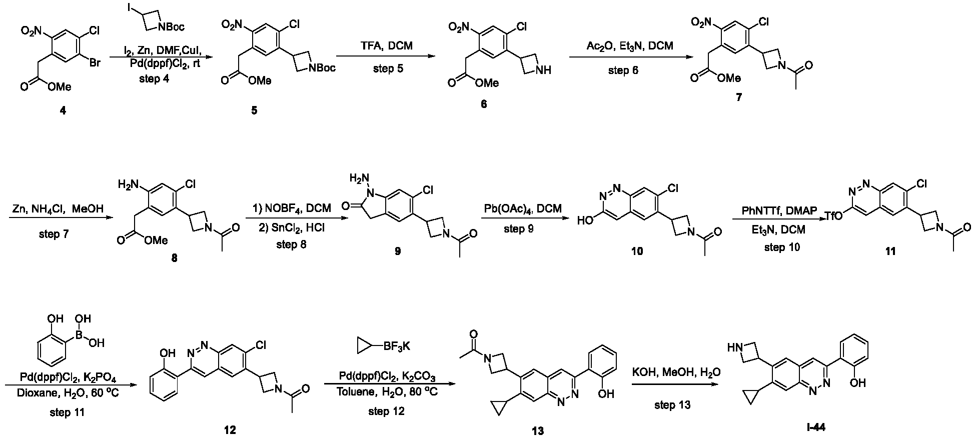

### Step 4: Preparation of tert-butyl 3-[2-chloro-5-(2-methoxy-2-oxoethyl)-4-nitrophenyl]azetidine-1-carboxylate (Intermediate 5).

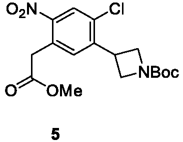

A solution of tert-butyl 3-iodoazetidine-1-carboxylate (22.94 g, 81.035 mmol, 1 equiv) and I₂ (10.28 g, 40.517 mmol, 0.5 equiv), Zn (15.89 g, 243.105 mmol, 3 equiv) in DMF (250 mL) was stirred for 1 h at room temperature under nitrogen atmosphere. To the above mixture was added Cul (3.09 g, 16.207 mmol, 0.2 equiv), Pd(dppf)Cl₂ (11.86 g, 16.207 mmol, 0.2 equiv) and Intermediate **4** (25 g, 81.035 mmol, 1 equiv) at room temperature. The mixture solution was stirred for an additional 3 h at room temperature. The resulting mixture was filtered, the filter cake was washed with EtOAc (3x50 mL). The filtrate was diluted with water (500 mL), extracted with EtOAc (3 x 500 mL). The combined organic layers were washed with brine (2 x 500 mL), dried over anhydrous sodium sulfate. After filtration, the filtrate was concentrated under reduced pressure. The crude product was purified by silica gel column chromatography, eluted with PE / EtOAc (2:1) to afford Intermediate **5** (32 g, 97.4%) as light yellow oil. LCMS (ESI) m/z: [M+H]⁺ = 385.

### Step 5: Preparation of methyl 2-[5-(azetidin-3-yl)-4-chloro-2-nitrophenyl]acetate (Intermediate 6).

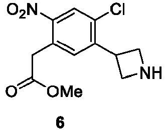

A solution of Intermediate **5** (31 g, 80.559 mmol, 1 equiv) in TFA (30 mL) and DCM (90 mL) was stirred for 1 h at room temperature under nitrogen atmosphere. The resulting mixture was concentrated under reduced pressure. This resulted in Intermediate **6** (40 g, crude) as a Brown yellow oil. The crude product was used in the next step directly without further purification. LCMS (ESI) m/z: [M+H]⁺ =285.

### Step 6: Preparation of methyl 2-[5-(1-acetylazetidin-3-yl)-4-chloro-2-nitrophenyl]acetate (Intermediate 7).

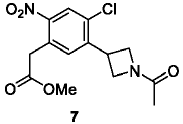

To a stirred solution of Intermediate **6** (40 g, crude) and Et₃N (21.33 g, 210.747 mmol, 3 equiv) in DCM (250 mL) was added Ac₂O (7.17 g, 70.249 mmol, 1 equiv) dropwise at 0 °C. The resulting mixture was stirred for 1h at room temperature under nitrogen atmosphere and concentrated under reduced pressure. The crude product was purified by reverse phase flash with the following conditions (Mobile Phase A: Water (0.1% FA), Mobile Phase B: ACN; Flow rate: 60 mL/min; Gradient: 0% B to 100% B in 40 min; 254/220 nm) to afford Intermediate **7** (21 g, 86.9%) as a light-yellow solid. LCMS (ESI) m/z: [M+H]⁺ = 327.

### Step 7: Preparation of methyl 2-[5-(1-acetylazetidin-3-yl)-2-amino-4-chlorophenyl]acetate (Intermediate 8).

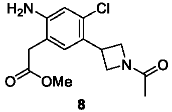

To a stirred solution of Intermediate **7** (10 g, 30.606 mmol, 1 equiv) and NH₄Cl (16.37 g, 306.060 mmol, 10 equiv) in MeOH (100 mL) was added Zn (20.01 g, 306.060 mmol, 10 equiv) at 0 °C. The resulting mixture was stirred for 1h at 0 °C. The mixture solution was filtered, the filter cake was washed with EtOAc (3 x 100 mL). The filtrate was concentrated under vacuum, the residue was diluted with water (500 mL), extracted with EtOAc (3 x 200 mL). The combined organic layers were washed with brine (2 x 400 mL), dried over anhydrous sodium sulfate. After filtration, the filtrate was concentrated under reduced pressure. This resulted in Intermediate **8** (9.5 g, crude) as a light-yellow solid. The crude product was used in the next step directly without further purification. LCMS (ESI) m/z: [M+H]⁺ =297.

### Step 8: Preparation of 5-(1-acetylazetidin-3-yl)-1-amino-6-chloro-3H-indol-2-one (Intermediate 9).

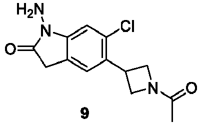

A solution of Intermediate **8** (9.5 g, crude) and NOBF₄ (5.61 g, 48.019 mmol, 1.5 equiv) in DCM (100 mL) was stirred for 1 h at 0 °C. To the above mixture was added SnCl₂ (36.81 g, 192.078 mmol, 6 equiv) in HCl (100 mL) dropwise at 0 °C. The resulting mixture was stirred for additional overnight at room temperature. The mixture solution was concentrated under reduced pressure. The crude product was purified by reverse phase flash with the following conditions (Mobile Phase A: Water (0.1% FA), Mobile Phase B: ACN; Flow rate: 45 mL/min; Gradient: 0% B to 50% B in 40 min; 254/220 nm) to afford Intermediate **9** (5.2 g, 34.8%) as a light-yellow solid. LCMS (ESI) m/z: [M+H]⁺ =280.

### Step 9: Preparation of 1-[3-(7-chloro-3-hydroxycinnolin-6-yl)azetidin-1-yl]ethanone (Intermediate 10).

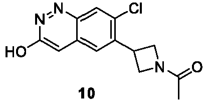

To a stirred solution of Intermediate **9** (1.5 g, 4.558 mmol, 1 equiv, 85%) in DCM (20 mL) was added Pb(OAc)₄ (3.03 g, 6.837 mmol, 1.5 equiv) at 0 °C. The resulting mixture was stirred for 1 h at 0 °C. The mixture solution was concentrated under reduced pressure. The crude product was purified by reverse phase flash with the following conditions (Mobile Phase A: Water (0.1% FA), Mobile Phase B: ACN; Flow rate: 60 mL/min; Gradient: 0% B to 50% B in 40 min; 254/220 nm) to afford Intermediate **10** (1.5 g, 94.8%) as a yellow solid. LCMS (ESI) m/z: [M+H]⁺ =278.

### Step 10: Preparation of 6-(1-acetylazetidin-3-yl)-7-chlorocinnolin-3-yl trifluoromethanesulfonate (Intermediate 11).

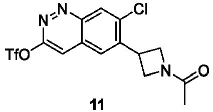

To a stirred solution of Intermediate **10** (1.5 g, 4.321 mmol, 1 equiv, 80%) and Et₃N (1.31 g, 12.963 mmol, 3 equiv) in DCM (2 mL) were added DMAP (0.26 g, 2.160 mmol, 0.5 equiv) and 1,1,1-trifluoro-N-phenyl-N-trifluoromethanesulfonylmethanesulfonamide (1.54 g, 4.321 mmol, 1 equiv) at 0 °C. The resulting mixture was stirred for 1 h at room temperature under nitrogen atmosphere. The residue was purified by silica gel column chromatography, eluted with (PE / EtOAc 1:1) to afford Intermediate **11** (1.5 g, 72.0%) as a light-yellow solid. LCMS (ESI) m/z: [M+H]⁺ =410.

### Step 11: Preparation of 1-{3-[7-chloro-3-(2-hydroxyphenyl)cinnolin-6-yl]azetidin-1-yl}ethanone (Intermediate 12).

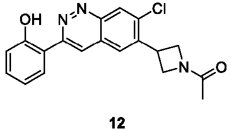

To a stirred solution of Intermediate **11** (700.0 mg, 1.708 mmol, 1 equiv) and 2-hydroxyphenylboronic acid (353.44 mg, 2.562 mmol, 1.5 equiv) in dioxane (70 mL) and H₂O (14 mL) were added Pd(dppf)Cl₂ (250.00 mg, 0.342 mmol, 0.2 equiv) and K₃PO₄ (1087.85 mg, 5.124 mmol, 3 equiv). The resulting mixture was stirred for 1 h at 60 °C under nitrogen atmosphere. The mixture solution was diluted with water (50 mL), extracted with EtOAc (3 x 100 mL). The combined organic layers were washed with brine (1x300 mL), dried over anhydrous sodium sulfate. After filtration, the filtrate was concentrated under reduced pressure. The crude product was purified by reverse phase flash with the following conditions (Mobile Phase A: Water (0.1% FA), Mobile Phase B: ACN; Flow rate: 60 mL/min; Gradient: 0% B to 100% B in 40 min; 254/220 nm) to afford Intermediate **12** (436.0 mg, 68.5%) as a light yellow solid. LCMS (ESI) m/z: [M+H]⁺ =354.

### Step 12: Preparation of 1-{3-[7-cyclopropyl-3-(2-hydroxyphenyl)cinnolin-6-yl]azetidin-1-yl}ethanone (Intermediate 13).

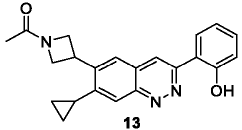

To a stirred solution of Intermediate **12** (25.0 mg, 0.071 mmol, 1 equiv) and cyclopropyltrifluorolambda4-borane potassium (52.28 mg, 0.355 mmol, 5 equiv) in toluene (2 mL) and H₂O (0.4 mL) were added Pd(dppf)Cl₂ (10.34 mg, 0.014 mmol, 0.2 equiv) and K₂CO₃ (29.30 mg, 0.213 mmol, 3 equiv). The resulting mixture was stirred for 1 h at 60 °C under nitrogen atmosphere. The mixture was concentrated under reduced pressure. The residue was purified by Prep-HPLC with the following conditions (Column: XBridge Shield RP₁₈ OBD Column, 30*150 mm, 5µm; Mobile Phase A: Water(0.1%FA), Mobile Phase B: ACN; Flow rate: 60 mL/min; Gradient: 35% B to 50% B in 8 min, 50% B; Wave Length: 254/220 nm; RT1 (min): 8.86; Number of Runs: 0) to afford Intermediate **13** (2.8 mg, 11.0%) as a light yellow solid. LCMS (ESI) m/z: [M+H]⁺ =741.30. ¹H NMR (400 MHz, Methanol-d4) δ 8.80 (s, 1H), 8.17 - 7.95 (m, 3H), 7.40 - 7.32 (m, 1H), 7.10 - 6.96 (m, 2H), 4.79 (t, J = 8.5 Hz, 1H), 4.68 - 4.48 (m, 3H), 4.30 (dd, J = 9.4, 6.3 Hz, 1H), 2.08 - 2.00 (m, 1H), 1.96 (s, 3H), 1.23 - 1.13 (m, 2H), 0.97 - 0.89 (m, 2H).

### Step 13: Preparation of 2-[6-(azetidin-3-yl)-7-cyclopropylcinnolin-3-yl]phenol (I-44).

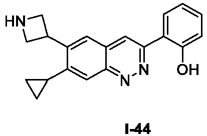

A solution of Intermediate **13** (150.0 mg, 0.417 mmol, 1 equiv) and KOH (234.1 mg, 4.170 mmol, 10 equiv) in MeOH (2 mL) and H₂O (2 mL) was stirred overnight at 60 °C. The residue was purified by reverse phase flash with the following conditions (Mobile Phase A: Water (0.1% FA), Mobile Phase B: ACN; Flow rate: 35 mL/min; Gradient: 0% B to 100% B in 40 min; 254/220 nm) to afford **I-44** (61.0 mg, 44.2%) as a light yellow solid. LCMS (ESI) m/z: [M+H]⁺ = 318.

### Preparation of 2-fluoro-2-(3-hydroxy-1,2-oxazol-5-yl)-3-methylbutanoic acid (I-58).

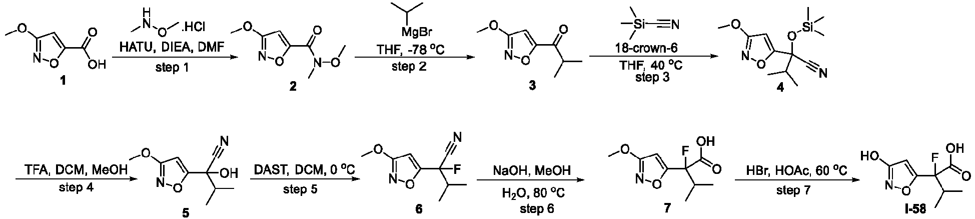

### Step 1: Preparation of N,3-dimethoxy-N-methyl-1,2-oxazole-5-carboxamide (Intermediate 2).

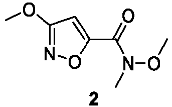

To a solution of 3-methoxy-1,2-oxazole-5-carboxylic acid (15 g, 104.823 mmol, 1 equiv), N,O-dimethylhydroxylamine (7.68 g, 125.788 mmol, 1.2 equiv) and HATU (47.83 g, 125.788 mmol, 1.2 equiv) in DMF (150 mL) was added DIEA (67.74 g, 524.115 mmol, 5 equiv). The solution was stirred at room temperature for 2 h. Desired product could be detected by LCMS. The resulting mixture was diluted with EtOAc (800mL) and washed with H₂O (800 mLx3). The organic layer was dried over anhydrous sodium sulfate, filtered and concentrated. The residue was purified by silica gel column chromatography, elution gradient 0 to 39% ethyl acetate in petroleum ether to afford intermediate **2** (17.6 g, 90.19%) as a yellow oil. LCMS (ESI) m/z: [M+H]⁺ = 187.

### Step 2: Preparation of 1-(3-methoxy-1,2-oxazol-5-yl)-2-methylpropan-1-one (Intermediate 3).

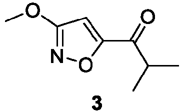

To a solution of intermediate **2** (16 g, 85.944 mmol, 1 equiv) in THF (150 mL) was added bromo(isopropyl)magnesium (25.32 g, 171.888 mmol, 2 equiv) at -78 degrees C under N₂ atmosphere. The resulting solution was stirred at -78 degrees C for 2 hours. The reaction solution was quenched with MeOH (30 mL) and concentrated. The residue was diluted with EtOAc (600 mL) and washed with water (3 x 600 mL). The organic layer was dried over anhydrous sodium sulfate, filtered and concentrated to give a crude product. The crude product was purified by silica gel column chromatography, elution gradient 0 to15% ethyl acetate in petroleum ether to afford intermediate 3 (9.8 g, 67.40%) as a colorless oil. LCMS (ESI) m/z: [M+H]⁺ = 170.

### Step 3: Preparation of 2-(3-methoxy-1,2-oxazol-5-yl)-3-methyl-2-[(trimethylsilyl)oxy]butanenitrile (Intermediate 4).

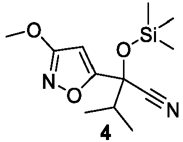

To a solution of intermediate **3** (9.8 g, 57.926 mmol, 1 equiv) in THF (100 mL) were added trimethylsilyl cyanide (22.99 g, 231.704 mmol, 4 equiv) and 18-crown-6 (1.53 g, 5.793 mmol, 0.1 equiv). The resulting solution was stirred at 40 degrees C for 24 hours. The reaction was quenched with saturated sodium bicarbonate aqueous solution (100 mL). The mixture was extracted with EtOAc (2 x 300 mL) and the combined organic layers were washed with saturated sodium chloride aqueous solution (3 x 500 mL). The organic layer was dried over anhydrous sodium sulfate, filtered and concentrated. The residue was purified by silica gel column chromatography, elution gradient 0 to 30% dichloromethane in petroleum ether to afford intermediate **4** (11.4 g, 73.33%) as a colorless oil. LCMS (ESI) m/z: [M+H]⁺ = 269.

### Step 4: Preparation of 2-hydroxy-2-(3-methoxy-1,2-oxazol-5-yl)-3-methylbutanenitrile (Intermediate 5).

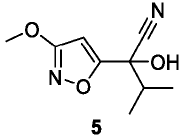

To a solution of intermediate **4** (11.4 g, 42.476 mmol, 1 equiv) in DCM (50 mL) and MeOH (50 mL) was added TFA (5 mL). The resulting solution was stirred at room temperature for 5 hours. The resulting mixture was concentrated under reduced pressure to give a crude product. The crude product was purified by flash C18 chromatography, elution gradient 0 to 33% acetonitrile in water (0.1% TFA) to afford intermediate **5** (7.8 g, 93.59%) as a yellow oil. LCMS (ESI) m/z: [M+H]⁺ = 197.

### Step 5: Preparation of 2-fluoro-2-(3-methoxy-1,2-oxazol-5-yl)-3-methylbutanenitrile (Intermediate 5).

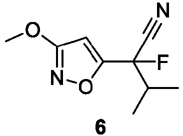

To a solution of intermediate **5** (7.8 g, 39.754 mmol, 1 equiv) in DCM (60 mL) was added DAST (7.69 g, 47.708 mmol, 1.20 equiv) at 0 degrees C. The resulting solution was stirred at 0 degrees C for 15 min. The reaction was quenched with saturated sodium bicarbonate aqueous solution (30 mL). The mixture was extracted with DCM (2 x 200 mL) and the combined organic layers were washed with saturated sodium chloride aqueous solution (3 x 500 mL). The layer was dried over anhydrous sodium sulfate, filtered and concentrated. The residue was purified by silica gel column chromatography, elution gradient 0 to 50% dichloromethane in petroleum ether to afford intermediate **6** (4.01 g, 50.89%) as a colorless oil. LCMS (ESI) m/z: [M+H]⁺ = 199.

### Step 6: Preparation of 2-fluoro-2-(3-methoxy-1,2-oxazol-5-yl)-3-methylbutanoic acid (Intermediate 7).

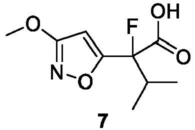

To a solution of intermediate **6** (200 mg, 1.009 mmol, 1 equiv) in MeOH (3 mL) and H₂O (3 mL) was added NaOH (403.61 mg, 10.090 mmol, 10 equiv). The resulting solution was stirred at 80 degrees C for 1 hour. The mixture was acidified to pH 2 with 1 M HCl (aq). The solution was diluted with water (100 mL) and extracted with EtOAc (3 x 100 mL). The combined organic layers were dried over anhydrous Na₂SO₄. After filtration, the filtrate was concentrated under reduced pressure to give intermediate **7** (220 mg, crude) as a yellow oil that was used in the next step directly without further purification. LCMS (ESI) m/z: [M+H]⁺ = 218.

### Step 7: Preparation of 2-fluoro-2-(3-hydroxy-1,2-oxazol-5-yl)-3-methylbutanoic acid (I-58).

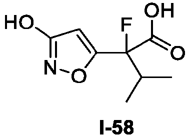

To a solution of intermediate **7** (220 mg, 1.013 mmol, 1 equiv) in HOAc (2.5 mL) was added HBr (48% in water, 2.5 mL). The resulting solution was stirred at 60 degrees C for 16 hours. The resulting mixture was concentrated under reduced pressure. The residue was purified by flash C18 chromatography, elution gradient 0 to 25% acetonitrile in water (0.1% FA) to afford **I-58** (136 mg, 66.09%) as a white solid. ¹H NMR (300 MHz, DMSO-d6) δ 14.12 (s, 1H), 11.55 (s, 1H), 6.16 (s, 1H), 2.72 - 2.52 (m, 1H), 0.97 (d, J = 6.9 Hz, 3H), 0.88 (d, J = 6.8 Hz, 3H). LCMS (ESI) m/z: [M+H]⁺ = 204.

### Preparation of (2S,4R)-4-hydroxy-1-[(2R)-2-(3-{6-[3-(2-hydroxyphenyl)cinnolin-7-yl]-2,6-diazaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 51) .

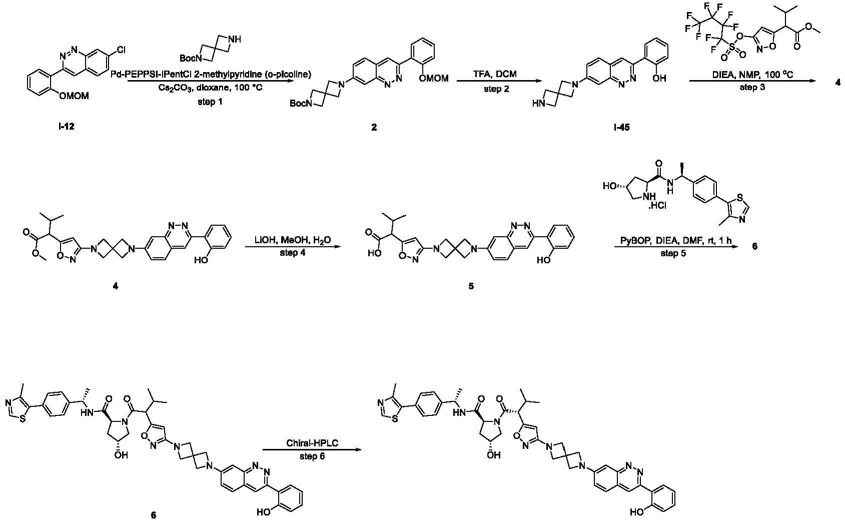

### Step 1: Preparation of tert-butyl 6-{3-[2-(methoxymethoxy)phenyl]cinnolin-7-yl}-2,6-diazaspiro[3.3]heptane-2-carboxylate (Intermediate 2).

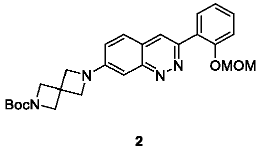

To a stirred mixture of 7-chloro-3-[2-(methoxymethoxy)phenyl]cinnoline (6 g, 19.951 mmol, 1 equiv) and tert-butyl 2,6-diazaspiro[3.3]heptane-2-carboxylate (1.58 g, 7.980 mmol, 1.2 equiv) in dioxane (50 mL) were added Cs₂CO₃ (19.50 g, 59.853 mmol, 3 equiv) and Pd-PEPPSI-IPentCl 2-methylpyridine (o-picoline) (1.68 g, 1.995 mmol, 0.1 equiv) in portions at room temperature under nitrogen atmosphere. The resulting mixture was stirred for additional 1 h at 100°C. The resulting mixture was concentrated under reduced pressure. The residue was purified by silica gel column chromatography, eluted with PE / EA (1:1) to afford intermediate **2** (8 g, 86.69%) as a yellow solid. LCMS (ESI) m/z: [M+H]⁺ = 463.

### Step 2: Preparation of 2-(7-{2,6-diazaspiro[3.3]heptan-2-yl}cinnolin-3-yl)phenol (I-45).

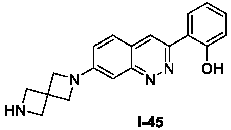

To a stirred mixture intermediate **2** (8 g, 17.295 mmol, 1 equiv) in TFA (10 mL) and DCM (30 mL) was stirred for 1 h at room temperature under nitrogen atmosphere. The resulting mixture was concentrated under reduced pressure. The residue was purified by reversed-phase flash chromatography with the following conditions: column, C18 silica gel; mobile phase, MeCN in Water (0.1% FA), 10% to 60% gradient in 30 min; detector, UV 254 nm. This resulted in **I-45** (4 g, 72.64%) as a red solid. LCMS (ESI) m/z: [M+H]⁺ = 319.

### Step 3: Preparation of methyl 2-(3-{6-[3-(2-hydroxyphenyl)cinnolin-7-yl]-2,6-diazaspiro[3.3]heptan-2-yl}-1,2-oxazol-5-yl)-3-methylbutanoate (Intermediate 4).

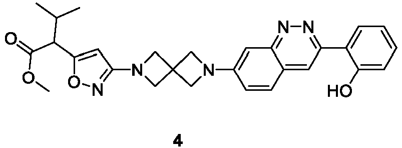

To a stirred mixture of intermediate **3** and methyl 3-methyl-2-{3-[(1,1,2,2,3,3,4,4,4-nonafluorobutanesulfonyl)oxy]-1,2-oxazol-5-yl}butanoate (9.07 g, 18.846 mmol, 2 equiv) in NMP (20 mL) was added DIEA (3.65 g, 28.269 mmol, 3 equiv) dropwise at room temperature under nitrogen atmosphere. The resulting mixture was stirred for an additional 1 h at 100 °C. The residue was purified by reversed-phase flash chromatography with the following conditions: column, C18 silica gel; mobile phase, MeCN in Water (10mmol/L NH₄HCO₃), 10% to 90% gradient in 40 min; detector, UV 254 nm. This resulted in intermediate **4** (700 mg, 14.87%) as a red solid. LCMS (ESI) m/z: [M+H]⁺ = 500.

### Step 4: Preparation of [3-(2-methoxypyrimidin-5-yl)-1,2-oxazol-5-yl]acetic acid (Intermediate 5).

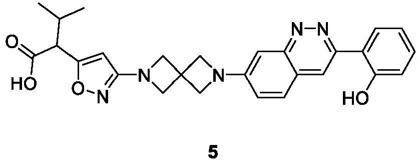

A mixture of intermediate **4** (700 mg, 1.401 mmol, 1 equiv) and LiOH.H₂O (587.94 mg, 14.010 mmol, 10 equiv) in MeOH (8 mL) and H₂O (2 mL) was stirred for 2 h at room temperature under nitrogen atmosphere. Desired product could be detected by LCMS. The mixture was acidified to pH 5 with HCl (aq.). The resulting mixture was diluted with CH₂Cl₂ / MeOH (9:1) (100 mL). The resulting mixture was washed with 3x100 mL of brine, dried over anhydrous Na₂SO₄. After filtration, the filtrate was concentrated under reduced pressure. This resulted in intermediate **5** (500 mg, 73.49%) as a red solid. LCMS (ESI) m/z: [M+H]⁺ =486.

### Step 5: Preparation of (2R,4S)-4-hydroxy-1-[2-(3-{6-[3-(2-hydroxyphenyl)cinnolin-7-yl]-2,6-diazaspiro[3.3]heptan-2-yl}-1,2-oxazol-5-yl)-3-methylbutanoyl]-N-[(1R)-1-[4-(4-methyl-1,3-thiazol-5-yl)phenyl]ethyl]pyrrolidine-2-carboxamide (Intermediate 6).

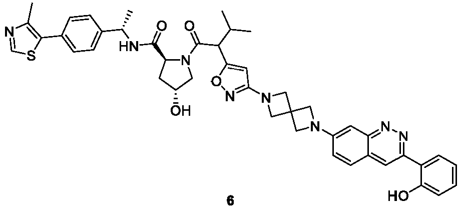

To a stirred mixture of intermediate **5** (210 mg, 0.433 mmol, 1 equiv) and (2*S*,4*R*)-4-hydroxy-N-[(1S)-1-[4-(4-methyl-1,3-thiazol-5-yl)phenyl]ethyl]pyrrolidine-2-carboxamide (186.35 mg, 0.563 mmol, 1.3 equiv) in DMF (2 mL) were added PyBOP (450.15 mg, 0.866 mmol, 2 equiv) and DIEA (226.01 uL, 1.299 mmol, 3 equiv) dropwise at room temperature. The resulting mixture was stirred for an additional 1 h at room temperature. The residue was purified by reverse flash chromatography with the following conditions: column, C18 silica gel; mobile phase, MeCN in Water (10mmol/L NH₄HCO₃), 10% to 90% gradient in 30 min; detector, UV 254 nm. This resulted in Intermediate **6** (102 mg, 29.52%) as a yellow solid. LCMS (ESI) m/z: [M+H]⁺ = 799.

### Step 6: Preparation of (2S,4R)-4-hydroxy-1-[(2R)-2-(3-{6-[3-(2-hydroxyphenyl)cinnolin-7-yl]-2,6-diazaspiro[3.3]heptan-2-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 51).

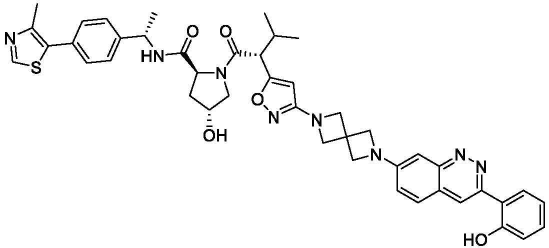

The Intermediate **6** was purified by Chiral-Prep-HPLC with the following conditions: Column, CHIRALPAK ID, 2*25 cm, 5 um; mobile phase, MtBE(10mM NH3-MeOH) and EtOH- (hold 50% EtOH- in 30 min); Detector, UV 254 nm. This resulted in **51** (second peak) (47.8 mg, 45.74%) as a red solid. ¹H NMR (400 MHz, DMSO-d6) *δ* 12.82 (d, *J =* 4.8 Hz, 1H), 9.00 (d, *J =* 6.4 Hz, 1H), 8.76 (s, 1H), 8.42 (d, *J =* 7.7 Hz, 1H), 8.07 - 8.01 (m, 1H), 7.96 (d, *J =* 9.0 Hz, 1H), 7.50 - 7.42 (m, 2H), 7.37 (d, *J* = 8.1 Hz, 2H), 7.35 - 7.25 (m, 2H), 7.01 (d, *J* = 8.0 Hz, 3H), 5.87 (d, *J* = 42.8 Hz, 1H), 5.12 (d, *J* = 3.6 Hz, 1H), 5.05 - 4.85 (m, 1H), 4.37 (t, *J* = 7.7 Hz, 1H), 4.29 (s, 5H), 4.13 (s, 4H), 3.71 (dd, *J =* 10.6, 4.4 Hz, 1H), 3.60 (t, *J =* 10.6 Hz, 1H), 3.45 (dd, *J =* 14.0, 10.8 Hz, 1H), 2.47 (d, *J* = 4.9 Hz, 3H), 2.34 - 2.13 (m, 1H), 2.03 (t, *J =* 10.0 Hz, 1H), 1.79 (ddd, J = 12.8, 8.2, 4.9 Hz, 1H), 1.43 (dd, *J =* 32.8, 7.0 Hz, 3H), 0.96 (d, *J* = 6.5 Hz, 3H), 0.82 (dd, *J* = 14.9, 6.7 Hz, 3H). LCMS (ESI) m/z: [M+H]⁺ = 799.15.

The compounds in Table 8 were prepared using procedures similar to those used above for the preparation of **Compound 51** using the appropriate Boc-diamines and chloro-cinnolines.

**Table 8.**

| **No.** | **Name** | **LCMS (ESI) m/z** | **¹H NMR** |
|---|---|---|---|
| 106 | (2S,4R)-4-hydroxy-1-((R)-2-(3-(9-(3-(2-hydroxyphenyl)cinnolin-7-yl)-3,9-diazaspiro[5.5]undecan-3-yl)isoxazol-5-yl)-3-methylbutanoyl)-N-((S)-1-(4-(4-methylthiazol-5-yl)phenyl)ethyl)pyrrolidine-2-carboxamide | 855.4 | ¹H NMR (400 MHz, DMSO-d6) δ 12.87 (s, 1H), 9.00 (d, J = 6.4 Hz, 1H), 8.76 (s, 1H), 8.42 (d, J = 7.7 Hz, 1H), 8.08 - 8.01 (m, 1H), 7.94 (d, J = 9.4 Hz, 2H), 7.65 (d, J = 1.9 Hz, 3H), 7.50 - 7.40 (m, 3H), 7.05 - 6.96 (m, 2H), 6.16 (s, 1H), 5.12 (d, J = 3.6 Hz, 1H), 4.92 (d, J = 7.1 Hz, 1H), 4.37 (t, J = 7.8 Hz, 1H), 4.29 (s, 1H), 3.72 (dd, J = 10.5, 4.4 Hz, 1H), 3.57 (d, J = 9.9 Hz, 1H), 3.52 (s, 4H), 3.43 (d, J = 10.2 Hz, 1H), 3.23 (s, 4H), 2.46 (d, J = 2.6 Hz, 3H), 1.96 (dd, J = 8.1, 4.8 Hz, 1H), 1.93 - 1.86 (m, 1H), 1.83 - 1.77 (m, 1H), 1.66 (s, 4H), 1.59 (s, 4H), 1.46 (d, J = 7.0 Hz, 3H), 0.97 (t, J = 7.1 Hz, 3H), 0.82 (dd, J = 16.0, 6.8 Hz, 3H). |
| 53 | (2S,4R)-4-hydroxy-1-((R)-2-(3-(2-(3-(2-hydroxyphenyl)cinnolin-7-yl)-2,7-diazaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 827.35 | ¹H NMR (300 MHz, DMSO-d6) δ 12.90 (s, 1H), 9.01 - 8.97 (m, 1H), 8.76 (s, 1H), 8.41 (d, J = 7.6 Hz, 1H), 8.03 (dd, J = 8.4, 1.7 Hz, 1H), 7.95 (d, J = 9.0 Hz, 1H), 7.52 - 6.94 (m, 8H), 6.20 (s, 1H), 5.12 (d, J = 3.6 Hz, 1H), 4.92 (t, J = 7.1 Hz, 1H), 4.38 (t, J = 7.8 Hz, 1H), 4.29 (s, 1H), 3.89 (s, 4H), 3.78 - 3.68 (m, 1H), 3.59 (d, J = 9.9 Hz, 1H), 3.54 - 3.39 (m, 1H), 3.28 - 3.18 (m, 4H), 2.47 - 2.43 (m, 3H), 2.31 - 2.13 (m, 1H), 2.09 - 1.96 (m, 1H), 1.93 - 1.72 (m, 5H), 1.51 - 1.34 (m, 3H), 1.00 - 0.92 (m, 3H), 0.83 - 0.76 (m, 3H). |
| 54 | (2S,4R)-4-hydroxy-1-((R)-2-(3-(4-(3-(2-hydroxyphenyl)cinnolin-7-yl)piperazin-1-yl)isoxazol-5-yl)-3-methylbutanoyl)-N-((S)-1-(4-(4-methylthiazol-5-yl)phenyl)ethyl)pyrrolidine-2-carboxamide | 787.45 | ¹H NMR (300 MHz, DMSO-d6) δ 12.69 (d, J = 4.7 Hz, 1H), 8.98 (d, J = 6.7 Hz, 1H), 8.80 (s, 1H), 8.42 (d, J = 7.7 Hz, 1H), 8.11 - 7.87 (m, 3H), 7.59 (d, J = 2.1 Hz, 1H), 7.47 (d, J = 7.3 Hz, 2H), 7.44 - 7.28 (m, 3H), 7.10 - 6.95 (m, 2H), 6.30 (s, 1H), 5.12 (d, J = 3.6 Hz, 1H), 4.93 (t, J = 7.3 Hz, 1H), 4.39 (t, J = 7.8 Hz, 1H), 4.30 (s, 1H), 3.74 (dd, J = 10.7, 4.6 Hz, 1H), 3.67 - 3.58 (m, 5H), 3.51 - 3.39 (m, 5H), 2.46 (d, J = 4.5 Hz, 3H), 2.30 - 2.14 (m, 1H), 2.04 (t, J = 10.9 Hz, 1H), 1.87 - 1.73 (m, 1H), 1.41 (dd, J = 7.1, 7.0 Hz, 3H), 0.98 (d, J = 6.4 Hz, 3H), 0.83 (d, J = 6.7 Hz, 3H). |
| 57 | (2S,4R)-4-hydroxy-1-((R)-2-(3-(4-(3-(2-hydroxyphenyl)cinnolin-6-yl)piperazin-1-yl)isoxazol-5-yl)-3-methylbutanoyl)-N-((S)-1-(4-(4-methylthiazol-5-yl)phenyl)ethyl)pyrrolidine-2-carboxamide | 787.2 | ¹H NMR (400 MHz, DMSO-d6) δ 12.97 (s, 1H), 9.01 - 8.95 (m, 1H), 8.61 (s, 1H), 8.40 (d, J = 7.7 Hz, 1 H),8.25 (d, J = 9.5 Hz, 1H), 8.04 (d, J = 7.2 Hz, 1H), 7.95 - 7.88 (m, 1H), 7.47 - 7.41 (m, 2H), 7.43 - 7.33 (m, 2H), 7.36 - 7.26 (m, 1H), 7.23 - 7.17 (m, 1H), 7.05 - 6.98 (m, 2H), 6.29 (s, 1H),5.11 (d, J = 3.7 Hz, 1H), 4.96 - 4.88 (m, 1H), 4.42 - 4.33 (m, 1H), 4.29 (s, 1H), 3.76 - 3.70 (m, 1H), 3.65 - 3.55 (m, 4H), 3.48 - 3.43 (m, 6H),2.48 - 2.42 (m, 3H), 2.34 - 1.95 (m, 2H), 1.83 - 1.75(m, 1H),1.39 (d, J = 7.0 Hz, 3H),0.97 (d, J = 6.4 Hz, 3H), 0.88 - 0.78 (m, 3H). |
| 66 | (2S,4R)-4-hydroxy-1-((R)-2-(3-(6-(3-(2-hydroxyphenyl)cinnolin-6-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 799.3 | ¹H NMR (400 MHz, DMSO-d6) δ 13.31 (s, 1H), 8.99 (d, J = 6.9 Hz, 1H), 8.55 (d, J = 0.9 Hz, 1H), 8.39 (d, J = 7.7 Hz, 1H), 8.20 (d, J = 9.3 Hz, 1H), 8.10 - 8.00 (m, 1H), 7.50 - 7.41 (m, 2H), 7.41 - 7.30 (m, 3H), 7.26 (dd, J = 9.3, 2.4 Hz, 1H), 7.04 - 6.96 (m, 2H), 6.58 (d, J = 2.4 Hz, 1H), 5.86 (d, J = 42.7 Hz, 1H), 5.10 (d, J = 3.6 Hz, 1H), 4.98 - 4.87 (m, 1H), 4.34 (d, J = 23.0 Hz, 6H), 4.12 (s, 4H), 3.71 (dd, J = 10.6, 4.5 Hz, 1H), 3.59 (t, J = 11.1 Hz, 1H), 3.51 - 3.41 (m, 1H), 2.46 (d, J = 4.5 Hz, 3H), 2.27 - 2.16 (m, 1H), 2.07 - 1.99 (m, 1H), 1.80 (ddd, J = 12.8, 8.0, 4.8 Hz, 1H), 1.49 - 1.36 (m, 3H), 1.24 (s, 1H), 0.97 (t, J = 6.4 Hz, 3H), 0.85 (d, J = 2.7 Hz, 1H), 0.81 (t, J = 7.3 Hz, 3H). |
| 67 | (2S,4R)-4-hydroxy-1-((R)-2-(3-(2-(3-(2-hydroxyphenyl)cinnolin-6-yl)-2,7-diazaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 827.3 | 1H NMR (400 MHz, DMSO-d6) δ 13.41 (s, 1H), 8.99 (d, J = 6.6 Hz, 1H), 8.52 (d, J = 1.0 Hz, 1H), 8.39 (d, J = 7.7 Hz, 1H), 8.19 (d, J = 9.2 Hz, 1H), 8.04 (dd, J = 8.5, 1.7 Hz, 1H), 7.43 (s, 2H), 7.40 - 7.30 (m, 3H), 7.29 - 7.17 (m, 1H), 7.04 - 6.90 (m, 2H), 6.54 (d, J = 2.4 Hz, 1H), 6.20 (s, 1H), 5.11 (d, J = 3.7 Hz, 1H), 5.02 - 4.84 (m, 1H), 4.38 (t, J = 7.8 Hz, 1H), 4.30 (s, 1H), 3.90 (d, J = 3.4 Hz, 4H), 3.73 (dd, J = 10.5, 4.5 Hz, 1H), 3.59 (d, J = 9.9 Hz, 1H), 3.44 (d, J = 11.5 Hz, 1H), 3.23 (d, J = 6.0 Hz, 4H), 2.46 (d, J = 3.7 Hz, 3H), 2.35 - 2.14 (m, 1H), 2.03 (ddd, J = 11.8, 7.9, 3.2 Hz, 1H), 1.96 - 1.75 (m, 5H), 1.43 (dd, J = 32.8, 7.0 Hz, 3H), 0.97 (t, J = 6.7 Hz, 3H), 0.81 (d, J = 6.6 Hz, 3H). |
| 72 | (2S,4R)-4-hydroxy-1-((R)-2-(3-(2-(3-(2-hydroxyphenyl)cinnolin-6-yl)-2,8-diazaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 841.3 | ¹H NMR (300 MHz, Methanol-d4) δ 8.87 (s, 1H), 8.36 (s, 1H), 8.13 (d, J = 9.4 Hz, 1H), 8.08 - 7.80 (m, 1H), 7.64 - 7.45 (m, 1H), 7.48 - 7.27 (m, 3H), 7.40 - 7.15 (m, 2H), 7.04 - 6.94 (m, 2H), 6.70 (d, J = 2.5 Hz, 1H), 6.14 (s, 1H), 5.17 - 4.91 (m, 2H), 4.52 (t, J = 8.2 Hz, 2H), 3.90 - 3.70 (m, 1H), 3.68 - 3.57 (m, 5H), 3.50 (m, 2H), 3.47 (s, 2H), 2.47 (d, J = 2.0 Hz, 3H),2.34 - 2.46(s,1H), 2.34 - 2.16 (m, 1H), 2.15 - 1.91 (m, 3H), 1.77 (s, 4H), 1.56 (dd, J = 21.7, 7.0 Hz, 3H), 1.06 (d, J = 6.5 Hz, 3H), 1.00 - 0.72 (m, 3H). |
| 83 | (2S,4R)-4-hydroxy-1-((R)-2-(3-(2-(3-(2-hydroxyphenyl)cinnolin-7-yl)-2,8-diazaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 841.4 | ¹H NMR (400 MHz, DMSO-d6) δ 13.09 (s, 1H), 8.99 (d, J = 3.0 Hz, 1H), 8.84 - 8.71 (m, 1H), 8.55 - 8.39 (m, 1H), 8.03 (d, J = 7.8 Hz, 1H), 7.94 (d, J = 9.1 Hz, 1H), 7.52 (d, J = 9.3, 2.4 Hz, 1H), 7.44 (d, J = 8.0 Hz, 2H), 7.37 (d, J = 8.1 Hz, 2H), 7.34 - 7.26 (m, 1H), 7.11 (d, J = 2.3 Hz, 1H), 6.99 (t, J = 7.9 Hz, 2H), 6.12 (d, J = 51.7 Hz, 1H), 5.16 - 5.00 (m, 1H), 4.98 - 4.64 (m, 1H), 4.37 (t, J = 7.9 Hz, 1H), 4.30 (s, 1H), 3.77 - 3.68 (m, 1H), 3.63 - 3.55 (m, 3H), 3.52 - 3.37 (m, 4H), 3.32 - 3.24 (m, 3H), 2.46 (s, 3H), 2.28 - 2.23 (m, 1H), 2.07 - 1.96 (m, 3H), 1.85 - 1.74 (m, 1H), 1.69 - 1.62 (m, 4H), 1.43 (dd, J = 34.6, 7.0 Hz, 3H), 0.96 (d, J = 6.6 Hz, 3H), 0.86 - 0.76 (m, 3H). |
| 85 | (2S,4R)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)-N-((S)-1-phenylethyl)pyrrolidine-2-carboxamide | 702.3 | ¹H NMR (400 MHz, DMSO-d6) δ 12.82 (s, 1H), 8.76 (s, 1H), 8.39 - 8.20 (m, 1H), 8.03 (d, J = 7.9 Hz, 1H), 7.95 (d, J = 9.0 Hz, 1H), 7.39 - 7.16 (m, 7H), 7.01 (d, J = 8.1 Hz, 3H), 5.86 (d, J = 44.7 Hz, 1H), 5.14 - 4.99 (m, 1H), 4.93 - 4.62 (m, 1H), 4.37 (t, J = 7.8 Hz, 1H), 4.29 (s, 5H), 4.12 (s, 4H), 3.75 - 3.67 (m, 1H), 3.61 (d, J = 9.9 Hz, 1H), 3.50 - 3.39 (m, 1H), 2.25 - 2.17 (m, 1H), 2.05 - 1.95 (m, 1H), 1.82 - 1.71 (m, 1H), 1.43 - 1.34 (m, 3H), 0.96 (d, J = 6.3 Hz, 3H), 0.80 (d, J = 6.7 Hz, 3H). |
| 89 | (2S,4R)-N-((S)-1-(4-fluorophenyl)ethyl)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 720.3 | ¹H NMR (400 MHz, Methanol-d4) δ13.0 -12.6 (s, 1H), 8.81 (s, 1H), 8.51 (d, 1H), 8.23 - 8.0 (m, 1H), 8.0- 7.91 (m, 1H),7.75 - 7.35 (m, 4H), 7.36 - 7.22 (m, 2H), 7.10 - 6.64 (m, 3H), 6.08 (s, 1H), 5.02 - 4.94 (m, 1H), 4.63 - 4.54 (m, 1H), 4.44 (m, 6H), 4.25 - 4.12 (m, 4H), 3.85 - 3.73 (m, 1H), 3.73 - 3.65 (m, 1H), 3.65 - 3.61 (m, 1H), 2.26 - 2.13 (m, 1H), 2.02 - 1.91 (m, 1H), 1.60 (d, J = 7.0 Hz, 1H), 1.50 - 1.37 (m, 3H), 0.97 - 0.83 (m, 6H). |
| 95 | (2S,4R)-4-hydroxy-1-((R)-2-(3-(7-(3-(2-hydroxyphenyl)cinnolin-7-yl)-2,7-diazaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 827.3 | ¹H NMR (400 MHz, DMSO-d6) δ 12.85 - 12.79 (m, 1H), 9.02 - 8.96 (m, 1H), 8.82 - 8.74 (m, 1H), 8.42 (d, J = 7.7 Hz, 1H), 8.04 (d, J = 7.9 Hz, 1H), 7.94 (dd, J = 9.3, 2.1 Hz, 1H), 7.88 (d, J = 9.6 Hz, 1H), 7.52 (s, 1H), 7.50 - 7.40 (m, 2H), 7.40 - 7.28 (m, 3H), 7.05 - 6.96 (m, 2H), 5.88 (d, J = 1.9 Hz, 1H), 5.15 - 5.09 (m, 1H), 4.96 - 4.87 (m, 1H), 4.41 - 4.33 (m, 1H), 4.29 (s, 1H), 3.70 (s, 4H), 3.63 - 3.56 (m, 1H), 3.50 (s, 4H), 3.33 (s, 2H), 2.49 - 2.43 (s, 3H), 2.22 - 2.18 (m, 1H), 2.05 - 2.00 (m, 1H), 1.92 (d, J = 7.0 Hz, 4H), 1.79 (s, 1H), 1.41 - 1.32 (m, 3H), 1.00 - 0.92 (m, 3H), 0.89 - 0.77 (m, 3H). |
| 98 | (2S,4R)-4-hydroxy-1-((2R)-2-(3-(7-(3-(2-hydroxyphenyl)cinnolin-6-yl)-2,7-diazaspiro[4.4]nonan-2-yl)isoxazol-5-yl)-3-methylbutanoyl)-N-((S)-1-(4-(4-methylthiazol-5-yl)phenyl)ethyl)pyrrolidine-2-carboxamide | 827.3 | ¹H NMR (400 MHz, DMSO-d6) δ 13.51 (s, 1H), 8.99 (s, 1H), 8.51 (s, 1H), 8.40 (t, J = 7.5 Hz, 1H), 8.19 (d, J = 9.5 Hz, 1H), 8.08 - 7.93 (m, 1H), 7.51 (dd, J = 9.4, 2.5 Hz, 1H), 7.44 (dd, J = 8.4, 2.2 Hz, 2H), 7.35 (ddd, J = 12.9, 7.9, 2.4 Hz, 3H), 7.05 - 6.93 (m, 2H), 6.70 (d, J = 2.5 Hz, 1H), 5.95 (s, 1H), 5.11 (t, J = 3.6 Hz, 1H), 4.99 - 4.63 (m, 1H), 4.41 - 4.32 (m, 1H), 4.28 (s, 1H), 3.71 (dd, J = 10.5, 4.4 Hz, 1H), 3.68 - 3.57 (m, 2H), 3.52 (dd, J = 16.7, 9.8 Hz, 3H), 3.42 (d, J = 9.9 Hz, 3H), 3.31 - 3.22 (m, 1H), 2.45 (d, J = 1.3 Hz, 3H), 2.35 - 2.13 (m, 1H), 2.12 - 2.05 (m, 2H), 2.03 (s, 3H), 1.94 - 1.68 (m, 2H), 1.50 - 1.32 (m, 3H), 0.95 (d, J = 6.5 Hz, 3H), 0.80 (d, J = 6.6 Hz, 3H). |
| 99 | (2S,4R)-4-hydroxy-1-((R)-2-(3-((R)-7-(3-(2-hydroxyphenyl)cinnolin-7-yl)-2,7-diazaspiro[4.4]nonan-2-yl)isoxazol-5-yl)-3-methylbutanoyl)-N-((S)-1-(4-(4-methylthiazol-5-yl)phenyl)ethyl)pyrrolidine-2-carboxamide | 827.3 | ¹H NMR (300 MHz, DMSO-d6) δ 13.05 (s, 1H), 9.02-8.99 (d, J = 2.7 Hz, 1H), 8.74 (s, 1H), 8.44-8.36(d, J = 7.6 Hz, 1H), 8.06-7.99 (d, J = 7.4 Hz, 1H), 7.97-7.91 (d, J = 9.2 Hz, 1H), 7.55 - 7.26 (m, 6H), 7.13-6.96 (m, 3H), 5.96 (s, 1H), 5.15-5.08 (d, J = 3.6 Hz, 1H), 5.04 - 4.61 (m, 1H), 4.43-4.24 (m, 2H), 3.78- 3.50 (m, 11H), 2.48-2.44 (d, J = 2.2 Hz, 3H), 2.31-2.16 (m, 1H), 2.15-1.96 (m, 5H), 1.84-1.72 (m, 1H), 1.54-1.30 (m, 3H), 1.01-0.89 (t, J = 6.5 Hz, 3H), 0.88-0.73 (m, 3H). |
| 100 | (2S,4R)-4-hydroxy-1-((R)-2-(3-(1-(3-(2-hydroxyphenyl)cinnolin-7-yl)-1,6-diazaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide | 799.3 | ¹H NMR (400 MHz, DMSO-d6) δ 12.85 (s, 1H), 8.99 (s, 1H), 8.77 (s, 1H), 8.44 (d, J = 7.7 Hz, 1H), 8.03 (dd, J = 15.6, 8.4 Hz, 2H), 7.53 (s, 1H), 7.45 (d, J = 8.1 Hz, 2H), 7.38 (d, J = 8.0 Hz, 2H), 7.34 - 7.29 (m, 2H), 7.05 - 6.96 (m, 2H), 6.03 (s, 1H), 5.11 (d, J = 51.9 Hz, 1H), 4.93 (h, J = 6.9 Hz, 1H), 4.58 (d, J = 9.7 Hz, 2H), 4.40 (t, J = 7.9 Hz, 1H), 4.31 (s, 1H), 4.15 (dd, J = 9.9, 5.8 Hz, 2H), 3.97 (t, J = 7.0 Hz, 2H), 3.74 (dd, J = 10.6, 4.4 Hz, 1H), 3.68 - 3.57 (m, 1H), 3.53 - 3.44 (m, 1H), 2.64 (t, J = 7.1 Hz, 2H), 2.46 (s, 3H), 2.31 - 2.11 (m, 1H), 2.09 - 1.94 (m, 1H), 1.81 (ddd, J = 12.8, 8.0, 4.8 Hz, 1H), 1.48 - 1.33 (m, 3H), 0.98 (d, J = 6.6 Hz, 3H), 0.85 (d, J = 6.6 Hz, 3H). |
| 102 | (2S,4R)-4-hydroxy-1-((R)-2-(3-(1-(3-(2-hydroxyphenyl)cinnolin-6-yl)-1,6-diazaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide | 799.3 | ¹H NMR (400 MHz, DMSO-d6) δ 13.31 (s, 1H), 8.98 (d, J = 4.3 Hz, 1H), 8.55 (d, J = 11.8 Hz, 1H), 8.43 (d, J = 7.7 Hz, 1H), 8.29 (d, J = 9.2 Hz, 1H), 8.15 - 8.03 (m, 1H), 7.60 (s, 1H), 7.45 (d, J = 8.0 Hz, 2H), 7.35 (dd, J = 18.1, 8.1 Hz, 3H), 7.06 - 6.93 (m, 2H), 6.82 (s, 1H), 5.97 (d, J = 42.0 Hz, 1H), 5.15 (d, J = 3.5 Hz, 1H), 4.92 (t, J = 7.3 Hz, 1H), 4.57 (d, J = 9.7 Hz, 2H), 4.38 (t, J = 7.9 Hz, 1H), 4.31 (s, 1H), 4.16 (dd, J = 9.9, 4.4 Hz, 2H), 3.97 (s, 2H), 3.69 (dd, J = 31.9, 8.2 Hz, 2H), 3.47 (d, J = 11.0 Hz, 1H), 2.64 (d, J = 7.3 Hz, 2H), 2.45 (d, J = 9.5 Hz, 3H), 2.25 (d, J = 10.2 Hz, 1H), 2.05 (t, J = 10.8 Hz, 1H), 1.84 - 1.74 (m, 1H), 1.43 (dd, J = 39.5, 6.9 Hz, 3H), 1.07 - 0.76 (m, 6H). |
| 105 | (2S,4R)-1-((R)-2-(3-(5,5-difluoro-7-(3-(2-hydroxyphenyl)cinnolin-7-yl)-2,7-diazaspiro[3.5]nonan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 863.3 | ¹H NMR (400 MHz, DMSO-d6) δ 12.66 - 12.60 (m, 1H), 9.02 - 8.97 (m, 1H), 8.78 (s, 1H), 8.43 (d, J = 7.7 Hz, 1H), 8.09 - 7.90 (m, 3H), 7.67 - 7.62 (m, 1H), 7.51 - 7.29 (m, 5H), 7.06 - 6.97 (m, 2H), 5.95 (s, 1H), 5.12 (d, J = 3.6 Hz, 1H), 4.98 - 4.86 (m, 1H), 4.42-4.27 (m, 2H), 4.07 - 3.99 (m, 2H), 3.98 - 3.88 (m, 2H), 3.84 - 3.77 (m, 2H), 3.75 - 3.67 (m, 1H), 3.63 - 3.59 (m, 3H), 3.51 - 3.41 (m, 1H), 2.49 - 2.44 (m, 3H), 2.26 - 2.20 (m, 3H), 2.10 - 1.99 (m, 1H), 1.84 - 1.74 (m, 1H), 1.38 (d, J = 7.0 Hz, 3H), 1.01 - 0.77 (m, 6H). |
| 107 | (2S,4R)-1-((R)-2-(3-(4-((R)-3,3-difluoro-1-(3-(2-hydroxyphenyl)cinnolin-7-yl)piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 906.35 | ¹H NMR (400 MHz, Methanol-d4) δ 8.87 (s, 1H), 8.67 (s, 1H), 8.03 - 7.92 (m, 2H), 7.80 (dd, J = 9.4, 2.4 Hz, 1H), 7.55 (s, 1H), 7.48 - 7.28 (m, 5H), 7.05 - 6.97 (m, 2H), 6.10 - 6.07 (m, 1H), 5.09 - 5.06 (m, 1H), 4.60 (s, 1H), 4.51 (t, J = 8.2 Hz, 1H), 4.31 - 4.19 (m, 2H), 3.84 (dd, J = 10.8, 4.2 Hz, 1H), 3.62 (t, J = 10.4 Hz, 2H), 3.34 (s, 2H), 3.24 - 3.19 (m, 5H), 2.97 (d, J = 5.1 Hz, 4H), 2.48 (s, 3H), 2.43 - 2.29 (m, 1H), 2.18 (t, J = 11.5 Hz, 2H), 2.07 (s, 1H), 2.05 - 1.87 (m, 1H), 1.52 (d, J = 7.0 Hz, 3H), 1.05 (d, J = 6.5 Hz, 3H), 0.90 (dd, J = 11.7, 6.7 Hz, 3H). |
| 108 | (2S,4R)-1-(2-(3-(5,5-difluoro-2-(3-(2-hydroxyphenyl)cinnolin-7-yl)-2,7-diazaspiro[3.5]nonan-7-yl)isoxazol-5-yl)-3-methylbutanoyl)-4-hydroxy-N-((S)-1-(4-(4-methylthiazol-5-yl)phenyl)ethyl)pyrrolidine-2-carboxamide | 863.3 | ¹H NMR (400 MHz, Methanol-d4) δ 8.91 - 8.84 (m, 1H), 8.65 (s, 1H), 8.01 - 7.88 (m, 2H), 7.51 - 7.36 (m, 4H), 7.35 - 7.20 (m, 2H), 7.06 - 6.96 (m, 3H), 6.29 - 6.11 (m, 1H), 5.22 - 4.96 (m, 1H), 4.44 (s, 1H), 4.29 (t, J = 9.3 Hz, 2H), 3.98 (dd, J = 13.5, 8.3 Hz, 2H), 3.85 (dd, J = 10.8, 4.2 Hz, 1H), 3.80 - 3.55 (m, 4H), 3.52 - 3.39 (m, 2H), 3.34 (s, 2H), 2.48 (q, J = 1.8 Hz, 3H), 2.46 - 2.33 (m, 1H), 2.31 - 2.13 (m, 3H), 2.03 - 1.90 (m, 1H), 1.64 - 1.47 (m, 3H), 1.06 (dd, J = 6.7, 1.8 Hz, 3H), 0.96 - 0.82 (m, 3H). |
| 109 | (2S,4R)-N-((S)-1-(4-bromophenyl)ethyl)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 782.1 | 1H NMR (400 MHz, DMSO-d6) δ 9.14 - 8.68 (m, 1H), 8.32 (dd, J = 51.4, 7.7 Hz, 1H), 8.12 - 7.80 (m, 2H), 7.61 - 7.42 (m, 2H), 7.39 - 7.13 (m, 4H), 7.11 - 6.95 (m, 2H), 6.86 (s, 1H), 5.99 - 5.88 (m, 1H), 5.04 - 4.68 (m, 1H), 4.56 - 4.29 (m, 5H), 4.27 (d, J = 5.1 Hz, 1H), 4.20 - 4.07 (m, 4H), 3.78 - 3.66 (m, 1H), 3.64 - 3.55 (m, 2H), 2.31 - 2.09 (m, 1H), 2.07 - 1.91 (m, 1H), 1.85 - 1.62 (m, 1H), 1.48 - 1.27 (m, 3H), 1.04 - 0.89 (m, 3H), 0.87 - 0.71 (m, 3H). |
| 110 | (2S,4R)-N-((S)-1-(2-fluorophenyl)ethyl)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 720.35 | 1H NMR (400 MHz, DMSO-d6) δ 12.13 (s, 1H), 9.00 - 8.78 (m, 1H), 8.41 (dd, J = 33.0, 7.6 Hz, 1H), 8.15 - 7.98 (m, 1H), 7.94 (dt, J = 8.0, 2.3 Hz, 1H), 7.43 - 7.24 (m, 4H), 7.22 - 7.08 (m, 2H), 7.07 - 6.93 (m, 2H), 6.87 (s, 1H), 6.00 - 5.78 (m, 1H), 5.22 - 4.88 (m, 2H), 4.49 - 4.31 (m, 5H), 4.26 (dd, J = 8.0, 4.1 Hz, 1H), 4.13 (d, J = 3.4 Hz, 4H), 3.75 - 3.64 (m, 1H), 3.61 (d, J = 9.9 Hz, 1H), 3.57 - 3.44 (m, 1H), 2.30 - 2.15 (m, 1H), 2.07 - 1.88 (m, 1H), 1.85 - 1.55 (m, 1H), 1.46 - 1.31 (m, 3H), 0.96 (dd, J = 6.6, 1.9 Hz, 3H), 0.80 (td, J = 13.7, 12.4, 6.8 Hz, 3H). |
| 111 | (2S,4R)-N-benzyl-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 688.2 | ¹H NMR (400 MHz, DMSO-d6) δ 12.56 (s, 1H), 8.80 (s, 1H), 8.43 (dt, J = 25.1, 6.0 Hz, 1H), 7.99 (dd, J = 8.7, 3.5 Hz, 2H), 7.40 - 7.15 (m, 7H), 7.08 - 6.83 (m, 3H), 5.99 - 5.59 (m, 1H), 5.13 (s, 1H), 4.71 - 4.18 (m, 8H), 4.17 - 3.96 (m, 4H), 3.85 - 3.70 (m, 1H), 3.67 - 3.54 (m, 1H), 2.30 - 2.11 (m, 1H), 2.02 (td, J = 8.7, 7.9, 4.1 Hz, 1H), 1.90 (ddd, J = 12.7, 7.8, 4.7 Hz, 1H), 1.26 (td, J = 7.2, 4.9 Hz, 1H), 0.96 (dd, J = 6.8, 4.4 Hz, 3H), 0.83 - 0.61 (m, 3H). |
| 112 | (2S,4R)-N-((S)-1-(4-cyanophenyl)ethyl)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 727.25 | ¹H NMR (400 MHz, DMSO-d6) δ 12.82 (s, 1H), 8.76 (s, 1H), 8.43 (dd, J = 59.9, 7.4 Hz, 1H), 8.03 (dd, J = 8.1, 1.6 Hz, 1H), 7.96 (d, J = 9.1 Hz, 1H), 7.86 - 7.73 (m, 2H), 7.55 - 7.39 (m, 2H), 7.35 - 7.23 (m, 2H), 7.06 - 6.95 (m, 3H), 5.96 - 5.78 (m, 1H), 5.13 (d, J = 3.5 Hz, 1H), 5.05 - 4.86 (m, 1H), 4.38 (dt, J = 20.8, 7.9 Hz, 1H), 4.29 (s, 5H), 4.12 (d, J = 5.6 Hz, 4H), 3.74 - 3.66 (m, 1H), 3.63 - 3.48 (m, 1H), 3.43 (d, J = 11.0 Hz, 1H), 2.29 - 2.13 (m, 1H), 2.01 (d, J = 11.4 Hz, 1H), 1.74 (td, J = 11.2, 9.7, 6.4 Hz, 1H), 1.39 (ddd, J = 35.7, 6.9, 4.6 Hz, 3H), 0.95 (t, J = 7.1 Hz, 3H), 0.80 (td, J = 12.9, 6.6 Hz, 3H). |
| 113 | (2S,4R)-N-((S)-1-(4-chlorophenyl)ethyl)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 736.3 | 1H NMR (300 MHz, DMSO-d6) δ 8.84 (s, 1H), 8.44 - 8.24 (m, 1H), 8.06 - 7.91 (m, 2H), 7.47 - 7.21 (m, 6H), 7.08 - 6.95 (m, 2H), 6.90 (s, 1H), 5.97 - 5.79 (m, 1H), 4.94 - 4.78 (m, 1H), 4.45 - 4.22 (m, 6H), 4.13 (d, J = 2.8 Hz, 4H), 3.80 - 3.55 (m, 3H), 2.33 - 2.13 (m, 1H), 2.06 - 1.92 (m, 1H), 1.88 - 1.61 (m, 1H), 1.49 - 1.25 (m, 3H), 1.03 - 0.91 (m, 3H), 0.87 - 0.71 (m, 4H). |
| 114 | (2S,4R)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)-N-methylpyrrolidine-2-carboxamide | 612.3 | 1H NMR (400 MHz, DMSO-d6) δ 12.82 (s, 1H), 8.76 (s, 1H), 8.03 (dd, J = 8.2, 1.6 Hz, 1H), 7.95 (d, J = 9.0 Hz, 1H), 7.83 (dd, J = 19.1, 4.7 Hz, 1H), 7.38 - 7.20 (m, 2H), 7.12 - 6.92 (m, 3H), 5.98 - 5.67 (m, 1H), 5.11 (d, J = 3.5 Hz, 1H), 4.37 - 4.18 (m, 6H), 4.12 (t, J = 4.0 Hz, 4H), 3.72 (dd, J = 20.0, 9.5 Hz, 1H), 3.63 - 3.49 (m, 1H), 3.42 (q, J = 13.6, 12.6 Hz, 1H), 2.69 - 2.53 (m, 3H), 2.36 - 2.05 (m, 1H), 1.97 (dd, J = 8.9, 4.2 Hz, 1H), 1.88 - 1.72 (m, 1H), 1.02 - 0.68 (m, 6H). |
| 115 | (25,4R)-1-((R)-2-(3-(4-((S)-3,3-difluoro-1-(3-(2-hydroxyphenyl)cinnolin-7-yl)piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 906.55 | 1H NMR (400 MHz, DMSO-d6) δ 12.67 (d, J = 4.6 Hz, 1H), 8.99 (d, J = 2.9 Hz, 1H), 8.78 (s, 1H), 8.41 (d, J = 7.7 Hz, 1H), 8.05 (d, J = 7.8 Hz, 1H), 7.99 - 7.85 (m, 2H), 7.62 (s, 1H), 7.44 (d, J = 8.2 Hz, 2H), 7.40 - 7.23 (m, 3H), 7.02 (t, J = 7.8 Hz, 2H), 6.14 (s, 1H), 5.11 (d, J = 3.7 Hz, 1H), 5.01 - 4.89 (m, 1H), 4.36 (t, J = 7.9 Hz, 2H), 4.32 - 4.19 (m, 2H), 3.71 (d, J = 6.5 Hz, 1H), 3.58 (d, J = 10.0 Hz, 1H), 3.45 (m, 2H), 3.16 (s, 6H), 2.84 (s, 4H), 2.46 (d, J = 1.7 Hz, 3H), 2.21 (s, 1H), 2.01 (d, J = 12.8 Hz, 3H), 1.83 - 1.74 (m, 1H), 1.51 - 1.33 (m, 3H), 0.96 (t, J = 6.7 Hz, 3H), 0.89 - 0.76 (m, 3H). |
| 117 | (2S,4R)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)-N-(4-(4-methylthiazol-5-yl)benzyl)pyrrolidine-2-carboxamide | 785.3 | 1H NMR (400 MHz, DMSO-d6) δ 12.82 (d, J = 4.5 Hz, 1H), 9.00 (d, J = 8.1 Hz, 1H), 8.76 (s, 1H), 8.49 (t, J = 27.7, 6.0 Hz, 1H), 8.03 (d, J = 7.8 Hz, 2H), 7.50 - 7.35 (m, 6H), 7.30 - 7.22 (m, 3H), 5.95 - 5.86 (m, 1H), 5.14 (d, J = 3.6 Hz, 1H), 4.49 - 4.27 (m, 8H), 4.25 - 4.17 (m, 4H), 4.09 - 3.99 (m, 1H), 3.82 - 3.70 (m, 1H), 3.67 - 3.56 (m, 1H), 2.46 (s, 3H), 2.10 - 2.00 (m, 1H), 1.91 (dt, J = 12.6, 6.6 Hz, 2H), 1.13 - 0.76 (m, 6H). |
| 119 | (2S,4R)-N-(bicyclo[1.1.1]pentan-1-ylmethyl)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 678.4 | 1H NMR (400 MHz, DMSO-d6) δ 12.82 (s, 1H), 8.76 (s, 1H), 8.48 - 7.58 (m, 3H), 7.39 - 7.19 (m, 2H), 7.12 - 6.86 (m, 3H), 6.09 - 5.63 (m, 1H), 5.05 (dd, J = 48.3, 3.5 Hz, 1H), 4.68 - 4.20 (m, 6H), 4.18 - 4.00 (m, 4H), 3.88 - 3.55 (m, 2H), 3.49 - 3.36 (m, 1H), 3.28 - 3.04 (m, 1H), 3.02 - 2.87 (m, 1H), 2.44 (s, 1H), 2.37 - 2.09 (m, 1H), 2.04 - 1.79 (m, 2H), 1.77 - 1.48 (m, 6H), 1.06 - 0.61 (m, 6H). |
| 120 | (2S,4R)-4-hydroxy-1-(2-(3-(6-(3-(2-hydroxyphenyl)cinnolin-7-yl)-2,6-diazaspiro[3.3]heptan-2-yl)isoxazol-5-yl)-3-methylbutanoyl)-N-((S)-1-(pyridin-2-yl)ethyl)pyrrolidine-2-carboxamide | 703.3 | 1H NMR (400 MHz, DMSO-d6) δ 12.82 (s, 1H), 8.76 (s, 1H), 8.60 - 8.45 (m, 1H), 8.31 (dd, J = 48.3, 7.7 Hz, 1H), 8.06 - 8.00 (m, 1H), 7.95 (d, J = 9.0 Hz, 1H), 7.80 - 7.70 (m, 1H), 7.37 - 7.20 (m, 4H), 7.01 (d, J = 7.9 Hz, 3H), 6.02 - 5.75 (m, 1H), 5.12 (d, J = 3.6 Hz, 1H), 5.05 - 4.83 (m, 1H), 4.43 (dt, J = 23.8, 7.6 Hz, 1H), 4.29 (t, J = 3.4 Hz, 5H), 4.12 (q, J = 6.1 Hz, 4H), 3.73 (dd, J = 10.0, 4.9 Hz, 1H), 3.62 (d, J = 9.8 Hz, 1H), 3.58 - 3.45 (m, 1H), 2.34 - 2.13 (m, 1H), 2.02 (t, J = 6.4 Hz, 1H), 1.83 (q, J = 5.4 Hz, 1H), 1.48 - 1.33 (m, 3H), 0.96 (t, J = 6.5 Hz, 3H), 0.88 - 0.72 (m, 3H). |
| 122 | (2S,4R)-4-hydroxy-1-((R)-2-(3-(2-((3-(2-hydroxyphenyl)cinnolin-7-yl)(methyl)amino)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 855.35 | 1H NMR (400 MHz, DMSO-d6) δ 12.95 (d, J = 4.4 Hz, 1H), 8.99 (s, 1H), 8.75 (s, 1H), 8.41 (d, J = 7.7 Hz, 1H), 8.07 - 8.00 (m, 1H), 7.94 (d, J = 9.4 Hz, 1H), 7.76 (dd, J = 9.4, 2.5 Hz, 1H), 7.50 - 7.41 (m, 2H), 7.40 - 7.27 (m, 4H), 7.00 (dd, J = 8.0, 6.8 Hz, 2H), 6.17 (s, 1H), 5.12 (d, J = 3.6 Hz, 1H), 4.92 (t, J = 7.1 Hz, 1H), 4.51 (t, J = 8.2 Hz, 1H), 4.40 - 4.26 (m, 2H), 3.72 (dd, J = 10.4, 4.4 Hz, 1H), 3.57 (d, J = 9.9 Hz, 1H), 3.24 (s, 2H), 3.10 (d, J = 19.3 Hz, 5H), 2.46 (s, 3H), 2.38 (d, J = 10.3 Hz, 2H), 2.29 - 2.18 (m, 1H), 2.10 - 1.92 (m, 3H), 1.79 (s, 3H), 1.62 (s, 2H), 1.42 (dd, J = 31.6, 7.0 Hz, 3H), 1.23 (s, 2H), 0.96 (t, J = 6.9 Hz, 3H), 0.82 (dd, J = 19.0, 6.8 Hz, 3H). |
| 123 | (2S,4R)-1-((R)-2-(3-(4-((S)-3,3-difluoro-1-(3-(2-hydroxyphenyl)cinnolin-6-yl)piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 906.45 | 1H NMR (300 MHz, DMSO-d6) δ 12.6-13.1(m, 1H), 8.99 (d, J = 1.8 Hz, 1H), 8.58 (s, 1H), 8.40 (d, J = 7.4 Hz, 1H), 8.22 (d, J = 9.5 Hz, 1H), 8.02 (d, J = 8.3 Hz, 1H), 7.91 (d, J = 9.7 Hz, 1H), 7.50 - 7.31 (m, 5H), 7.25 (s, 1H), 7.08 - 6.97 (m, 2H), 6.13 (s, 1H), 4.10 (s, 1H), 4.91 (s, 1H),4.50 - 4.16 (m, 4H), 3.70 (s, 1H), 3.57 (d, J = 10.0 Hz, 1H), 3.50 (s, 2H),3.15 (s, 5H), 2.78 (d, J = 31.6 Hz, 4H), 2.45 (s, 3H), 2.13 (d, J = 87.6 Hz, 5H), 1.37 (d, J = 7.0 Hz, 3H), 1.24 (s, 1H), 0.95 (d, J = 6.3 Hz, 3H), 0.87 - 0.73 (m, 3H). |
| 124 | (2S,4R)-1-((R)-2-(3-(4-((R)-3,3-difluoro-1-(3-(2-hydroxyphenyl)cinnolin-6-yl)piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 906.35 | 1H NMR (400 MHz, Methanol-d4) δ 8.87 (s, 1H), 8.51 (s, 1H), 8.20 (d, J = 9.6 Hz, 1H), 8.03 - 7.96 (m, 1H), 7.86 - 7.78 (m, 1H), 7.48 - 7.30 (m, 5H), 7.19 (d, J = 2.7 Hz, 1H), 7.05 - 6.97 (m, 2H), 6.10 (s, 1H), 5.06-5.01 (m, 1H), 4.55 - 4.41 (m, 1H), 4.37 - 4.24 (m, 3H), 3.88 - 3.80 (m, 1H), 3.66 - 3.57 (m, 2H), 3.42 - 3.34 (m, 1H), 3.26 - 3.14 (m, 6H), 2.99- 2.93 (m, 4H), 2.48 (s, 3H), 2.41 -2.30(m, 1H), 2.23 - 2.09 (m, 2H), 2.07- 1.83 (m, 2H), 1.52 (d, J = 7.0 Hz, 3H), 1.05 (d, J = 6.5 Hz, 3H), 0.94 - 0.85 (m, 3H). |
| 127 | (2S,4R)-4-hydroxy-1-((R)-2-(3-(8-(3-(2-hydroxyphenyl)cinnolin-7-yl)-5-oxa-2,8-diazaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 829.4 | 1H NMR (400 MHz, DMSO-d6) δ 12.62 (s, 1H), 8.99 (s, 1H), 8.80 (s, 1H), 8.42 (d, J = 7.7 Hz, 1H), 8.06 (dd, J = 7.9, 1.6 Hz, 1H), 8.02 - 7.92 (m, 2H), 7.67 - 7.60 (m, 1H), 7.48 - 7.41 (m, 2H), 7.39 - 7.30 (m, 3H), 7.02 (t, J = 8.4 Hz, 2H), 5.90 (d, J = 40.3 Hz, 1H), 5.12 (d, J = 3.6 Hz, 1H), 5.03 - 4.82 (m, 1H), 4.37 (t, J = 7.9 Hz, 1H), 4.31 - 4.26 (m, 1H), 3.97 (dd, J = 8.4, 3.3 Hz, 2H), 3.90 - 3.77 (m, 4H), 3.75 - 3.64 (m, 3H), 3.62 (d, J = 9.8 Hz, 1H), 3.44 (q, J = 6.1, 3.6 Hz, 3H), 2.46 (s, 3H), 2.36 - 2.12 (m, 1H), 2.07 - 1.97 (m, 1H), 1.79 (ddd, J = 13.1, 8.2, 4.9 Hz, 1H), 1.42 (dd, J = 32.8, 7.0 Hz, 3H), 0.96 (d, J = 6.5 Hz, 3H), 0.83 (dd, J = 14.8, 6.6 Hz, 3H). |
| 129 | (2S,4R)-4-hydroxy-1-((R)-2-(3-(8-(3-(2-hydroxyphenyl)cinnolin-6-yl)-5-oxa-2,8-diazaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 829.25 | 1H NMR (400 MHz, DMSO-d6) δ 12.86 (d, J = 8.3 Hz, 1H), 8.99 (s, 1H), 8.61 (d, J = 3.0 Hz, 1H), 8.41 (d, J = 7.7 Hz, 1H), 8.25 (dd, J = 9.6, 3.8 Hz, 1H), 8.04 (dd, J = 8.0, 1.6 Hz, 1H), 7.95 (dd, J = 9.6, 2.6 Hz, 1H), 7.50 - 7.41 (m, 2H), 7.40 - 7.30 (m, 3H), 7.25 (d, J = 2.6 Hz, 1H), 7.01 (dd, J = 8.7, 6.8 Hz, 2H), 6.00 - 5.78 (m, 1H), 5.12 (d, J = 3.5 Hz, 1H), 5.05 - 4.64 (m, 1H), 4.37 (t, J = 7.9 Hz, 1H), 4.29 (s, 1H), 3.93 (dd, J = 8.4, 3.6 Hz, 2H), 3.89 - 3.78 (m, 4H), 3.72 (s, 2H), 3.65 - 3.54 (m, 1H), 3.45 (d, J = 5.8 Hz, 3H), 3.32 (s, 1H), 2.46 (s, 3H), 2.22 (dp, J = 20.2, 6.6 Hz, 1H), 2.09 - 1.97 (m, 1H), 1.96 - 1.73 (m, 1H), 1.48 - 1.33 (m, 3H), 0.96 (d, J = 6.5 Hz, 3H), 0.82 (dd, J = 14.7, 6.7 Hz, 3H). |
| 130 | (2S,4R)-4-hydroxy-1-((R)-2-(3-(2-(3-(2-hydroxyphenyl)cinnolin-7-yl)-2,6-diazaspiro[3.4]octan-6-yl)isoxazol-5-yl)-3-methylbutanoyl)-N-((S)-1-(4-(4-methylthiazol-5-yl)phenyl)ethyl)pyrrolidine-2-carboxamide | 813.3 | 1H NMR (300 MHz, DMSO-d6) δ 12.87 (d, J = 4.5 Hz, 1H), 9.00 (d, J = 2.5 Hz, 1H), 8.78 (s, 1H), 8.42 (d, J = 7.6 Hz, 1H), 8.04 (d, J = 7.6 Hz, 1H), 7.97 (d, J = 9.0 Hz, 1H), 7.51 - 7.27 (m, 6H), 7.02 (d, J = 8.0 Hz, 3H), 5.92 (d, J = 28.3 Hz, 1H), 5.16 - 4.86 (m, 2H), 4.45 - 4.22 (m, 2H), 4.15 - 4.06 (m, 4H), 3.73 (d, J = 6.5 Hz, 1H), 3.64 - 3.49 (m, 4H), 3.45 (m, 2H), 2.46 (s, 4H), 2.28 (s, 2H), 2.06 (d, J = 15.9 Hz, 1H), 1.81 (d, J = 6.8 Hz, 1H), 1.39 (d, J = 7.1 Hz, 3H), 0.96 (d, J = 6.3 Hz, 3H), 0.82 (d, J = 6.7 Hz, 3H). |
| 131 | (2S,4R)-4-hydroxy-1-((R)-2-(3-((3aR,6aS)-5-(3-(2-hydroxyphenyl)cinnolin-7-yl)hexahydropyrrolo[3,4-c]pyrrol-2(1H)-yl)isoxazol-5-yl)-3-methylbutanoyl)-N-((S)-1-(4-(4-methylthiazol-5-yl)phenyl)ethyl)pyrrolidine-2-carboxamide | 813.3 | 1H NMR (400 MHz, DMSO-d6) δ 13.01 (d, J = 6.8 Hz, 1H), 8.97 (d, J = 9.9 Hz, 1H), 8.75 (s, 1H), 8.41 (d, J = 7.7 Hz, 1H), 8.03 (d, J = 7.9 Hz, 1H), 7.95 (d, J = 9.2 Hz, 1H), 7.57 - 7.50 (m, 1H), 7.44 (d, J = 8.1 Hz, 2H), 7.36 (d, J = 8.3 Hz, 2H), 7.34 - 7.27 (m, 1H), 7.14 (d, J = 2.3 Hz, 1H), 7.01 (d, J = 7.8 Hz, 2H), 5.98 (s, 1H), 5.10 (d, J = 3.5 Hz, 1H), 4.91 (t, J = 7.3 Hz, 1H), 4.35 (t, J = 7.8 Hz, 1H), 4.28 (s, 1H), 3.78 (d, J = 3.6 Hz, 2H), 3.75 - 3.67 (m, 1H), 3.56 (t, J = 9.5 Hz, 3H), 3.48 (d, J = 10.5 Hz, 2H), 3.41 (d, J = 10.9 Hz, 2H), 3.25 (d, J = 28.9 Hz, 3H), 2.45 (d, J = 6.2 Hz, 3H), 2.21 (dd, J = 15.0, 7.6 Hz, 1H), 2.01 (s, 1H), 1.81 - 1.73 (m, 1H), 1.37 (d, J = 7.0 Hz, 3H), 0.95 (t, J = 6.9 Hz, 3H), 0.81 (dd, J = 15.6, 6.6 Hz, 3H), |
| 132 | (2S,4R)-4-hydroxy-1-((R)-2-(3-(6-(3-(2-hydroxyphenyl)cinnolin-7-yl)-2,6-diazaspiro[3.4]octan-2-yl)isoxazol-5-yl)-3-methylbutanoyl)-N-((S)-1-(4-(4-methylthiazol-5-yl)phenyl)ethyl)pyrrolidine-2-carboxamide | 813.3 | 1H NMR (400 MHz, DMSO-d6) δ 13.03 (d, J = 5.2 Hz, 1H), 8.99 (d, J = 3.1 Hz, 1H), 8.75 (s, 1H), 8.43 (d, J = 7.7 Hz, 1H), 8.07 - 8.00 (m, 1H), 7.95 (d, J = 9.2 Hz, 1H), 7.51 (dd, J = 9.1, 2.4 Hz, 1H), 7.50 - 7.41 (m, 2H), 7.41 - 7.35 (m, 2H), 7.31 (td, J = 7.7, 1.7 Hz, 1H), 7.11 (s, 1H), 7.00 (dd, J = 8.1, 6.9 Hz, 2H), 5.92 (s, 1H), 5.13 (d, J = 3.6 Hz, 1H), 4.92 (p, J = 7.2 Hz, 1H), 4.37 (t, J = 7.8 Hz, 1H), 4.29 (s, 1H), 3.95 (dd, J = 7.7, 3.3 Hz, 2H), 3.90 (dd, J = 7.6, 3.7 Hz, 2H), 3.73 (d, J = 19.1 Hz, 3H), 3.64 - 3.53 (m, 3H), 3.45 (t, J = 11.8 Hz, 1H), 2.46 (d, J = 3.4 Hz, 3H), 2.34 (t, J = 6.7 Hz, 2H), 2.26 - 2.17 (m, 1H), 2.02 (d, J = 10.0 Hz, 1H), 1.84 - 1.73 (m, 1H), 1.38 (d, J = 7.0 Hz, 3H), 0.97 (t, J = 6.8 Hz, 3H), 0.84 (d, J = 6.9 Hz, 1H), 0.80 (d, J = 6.7 Hz, 3H). |
| 138 | (2S,4R)-4-hydroxy-1-((R)-2-(3-((R)-4-(3-(2-hydroxyphenyl)cinnolin-6-yl)-3-(methoxymethyl)piperazin-1-yl)isoxazol-5-yl)-3-methylbutanoyl)-N-((S)-1-(4-(4-methylthiazol-5-yl)phenyl)ethyl)pyrrolidine-2-carboxamide | 831.3 | 1H NMR (300 MHz, DMSO-d6) δ 13.11 (s, 1H), 8.99 (s, 1H), 8.61 (s, 1H), 8.42 (d, J = 7.6 Hz, 1H), 8.24 (d, J = 9.5 Hz, 1H), 8.05 (d, J = 7.5 Hz, 1H), 7.92 (d, J = 9.5 Hz, 1H), 7.45 (d, J = 8.3 Hz, 2H), 7.41 - 7.32 (m, 3H), 7.15 (s, 1H), 7.06 - 6.99 (m, 2H), 6.20 (d, J = 38.4 Hz, 1H), 5.12 (d, J = 3.6 Hz, 1H), 5.04 - 4.88 (m, 1H), 4.54 (s, 1H), 4.43 - 4.22 (m, 2H), 3.97 - 3.70 (m, 4H), 3.63 (d, J = 9.5 Hz, 2H), 3.53 - 3.38 (m, 3H), 3.23 (s, 3H), 3.17 (d, J = 10.3 Hz, 2H), 2.47 (s, 3H), 2.28 (d, J = 2.3 Hz, 1H), 2.03 (s, 1H), 1.80 (s, 1H), 1.52 - 1.34 (m, 3H), 0.98 (d, J = 6.5 Hz, 3H), 0.83 (d, J = 6.7 Hz, 3H). |
| 142 | (2S,4R)-4-hydroxy-1-((R)-2-(3-((R)-4-(3-(2-hydroxyphenyl)cinnolin-7-yl)-3-(methoxymethyl)piperazin-1-yl)isoxazol-5-yl)-3-methylbutanoyl)-N-((S)-1-(4-(4-methylthiazol-5-yl)phenyl)ethyl)pyrrolidine-2-carboxamide | 831.3 | 1H NMR (400 MHz, DMSO-d6) δ 12.74 (d, J = 5.5 Hz, 1H), 8.99 (s, 1H), 8.77 (s, 1H), 8.42 (d, J = 7.7 Hz, 1H), 8.05 (d, J = 7.8 Hz, 1H), 8.01 - 7.89 (m, 2H), 7.54 - 7.29 (m, 6H), 7.05 - 6.97 (m, 2H), 6.26 (s, 1H), 5.12 (d, J = 3.6 Hz, 1H), 4.98 - 4.88 (m, 1H), 4.54 - 4.49 (m, 1H), 4.42 - 4.28 (m, 2H), 3.90 - 3.69 (m, 4H), 3.65 - 3.59 (m, 2H), 3.51 - 3.40 (m, 2H),3.21 (s, 4H),3.10 - 2.98 (m, 1H), 2.46 (s, 3H), 2.36 - 2.15 (m, 2H),2.08 - 1.98 (m, 1H), 1.85 - 1.74 (m, 1H), 1.45 - 1.36 (m, 3H), 1.02 - 0.94 (m, 3H), 0.89 - 0.79 (m, 3H). |
| 144 | (2S,4R)-4-hydroxy-1-(2-(3-((S)-4-(3-(2-hydroxyphenyl)cinnolin-6-yl)-3-(methoxymethyl)piperazin-1-yl)isoxazol-5-yl)-3-methylbutanoyl)-N-((S)-1-(4-(4-methylthiazol-5-yl)phenyl)ethyl)pyrrolidine-2-carboxamide | 831.2 | 1H NMR (300 MHz, DMSO-d6) δ 13.12 (s, 1H), 9.03 - 8.93 (m, 1H), 8.61 (s, 1H), 8.29 (d, J = 9.5 Hz, 1H), 8.26 - 8.19 (m, 1H), 8.10 - 8.00 (m, 1H), 7.92 (d, J = 8.4 Hz, 1H), 7.56 - 7.42 (m, 2H), 7.41 - 7.24 (m, 3H), 7.13 (d, J = 7.1 Hz, 1H), 7.06 - 6.95 (m, 2H), 6.26 (d, J = 10.2 Hz, 1H), 5.26 - 4.75 (m, 2H), 4.66 - 4.21 (m, 3H), 4.01 - 3.67 (m, 4H), 3.66 - 3.50 (m, 2H), 3.50 - 3.40 (m, 3H), 3.20 (d, J = 10.6 Hz, 4H), 3.06 (t, J = 14.3 Hz, 1H), 2.50 - 2.41 (m, 3H), 2.03 (s, 1H), 1.87 - 1.72 (m, 1H), 1.48 (d, J = 7.0 Hz, 1H), 1.44 - 1.28 (m, 3H), 0.98 (d, J = 6.5 Hz, 2H), 0.89 - 0.74 (m, 4H). |
| 146 | (2S,4R)-4-hydroxy-1-((R)-2-(3-(2-(3-(2-hydroxyphenyl)cinnolin-6-yl)-5-oxa-2,8-diazaspiro[3.5]nonan-8-yl)isoxazol-5-yl)-3-methylbutanoyl)-N-((S)-1-(4-(4-methylthiazol-5-yl)phenyl)ethyl)pyrrolidine-2-carboxamide | 829.35 | 1H NMR (400 MHz, DMSO-d6) δ 13.30 (d, J = 8.6 Hz, 1H), 8.98 (s, 1H), 8.53 (d, J = 4.6 Hz, 1H), 8.39 (d, J = 7.8 Hz, 1H), 8.21 (dd, J = 9.3, 4.7 Hz, 1H), 8.09 - 8.02 (m, 1H), 7.48 - 7.40 (m, 2H), 7.39 - 7.31 (m, 3H), 7.35 - 7.27 (m, 1H), 7.04 - 6.96 (m, 2H), 6.63 (d, J = 2.5 Hz, 1H), 6.31 (s, 1H), 5.12 (d, J = 3.6 Hz, 1H), 4.91 (q, J = 6.8 Hz, 1H), 4.36 (t, J = 7.9 Hz, 1H), 4.29 (s, 1H), 4.13 (d, J = 9.1 Hz, 2H), 4.03 (d, J = 9.0 Hz, 2H), 3.81 (s, 2H), 3.72 (dd, J = 10.6, 4.4 Hz, 1H), 3.61 (d, J = 9.9 Hz, 1H), 3.48 (s, 2H), 3.43 (d, J = 10.8 Hz, 1H), 3.21 (s, 2H), 2.45 (d, J = 2.5 Hz, 3H), 2.03 (t, J = 10.0 Hz, 1H), 1.79 (ddd, J = 12.8, 8.1, 4.7 Hz, 1H), 1.49 - 1.40 (m, 1H), 1.36 (d, J = 7.0 Hz, 3H), 0.96 (t, J = 6.2 Hz, 3H), 0.83 (dd, J = 13.4, 6.7 Hz, 3H). |
| 147 | (2S,4R)-4-hydroxy-1-((R)-2-(3-(9-(3-(2-hydroxyphenyl)cinnolin-6-yl)-1-oxa-4,9-diazaspiro[5.5] undecan-4-yl)isoxazol-5-yl)-3-methylbutanoyl)-N-((S)-1-(4-(4-methylthiazol-5-yl)phenyl)ethyl)pyrrolidine-2-carboxamide | 857.2 | 1H NMR (400 MHz, DMSO-d6) δ 13.20 (s, 1H), 8.98 (d, J = 2.1 Hz, 1H), 8.57 (s, 1H), 8.38 (d, J = 7.7 Hz, 1H), 8.20 (d, J = 9.6 Hz, 1H), 8.07 - 7.99 (m, 1H), 7.92 - 7.81 (m, 1H), 7.71 - 7.24 (m, 5H), 7.13 (d, J = 2.6 Hz, 1H), 7.07 - 6.94 (m, 2H), 6.20 (d, J = 42.1 Hz, 1H), 5.12 (d, J = 3.6 Hz, 1H), 4.90 (t, J = 7.1 Hz, 1H), 4.41 - 4.24 (m, 2H), 3.75 (d, J = 32.2 Hz, 4H), 3.64 (s, 1H), 3.58 (d, J = 9.9 Hz, 1H), 3.42 (d, J = 11.5 Hz, 3H), 3.20 (s, 2H), 3.14 (s, 2H), 2.45 (s, 3H), 2.27 - 2.13 (m, 1H), 2.10 - 1.92 (m, 3H), 1.85 - 1.66 (m, 3H), 1.50 - 1.32 (m, 3H), 0.96 (t, J = 6.4 Hz, 3H), 0.89 - 0.72 (m, 3H). |
| 148 | (2S,4R)-4-hydroxy-1-((R)-2-(3-(9-(3-(2-hydroxyphenyl)cinnolin-7-yl)-1-oxa-4,9-diazaspiro[5.5] undecan-4-yl)isoxazol-5-yl)-3-methylbutanoyl)-N-((S)-1-(4-(4-methylthiazol-5-yl)phenyl)ethyl)pyrrolidine-2-carboxamide | 857.35 | 1H NMR (400 MHz, DMSO-d6) δ 12.83 (s, 1H), 8.99 (s, 1H), 8.77 (s, 1H), 8.40 (d, J = 7.7 Hz, 1H), 8.20 (s, 1H), 8.04 (d, J = 7.8 Hz, 1H), 7.95 (d, J = 9.3 Hz, 1H), 7.88 (dd, J = 9.4, 2.4 Hz, 1H), 7.52 (d, J = 2.3 Hz, 1H), 7.48 - 7.40 (m, 2H), 7.36 (d, J = 8.2 Hz, 2H), 7.01 (d, J = 8.1 Hz, 2H), 6.27 (s, 1H), 5.13 (d, J = 3.6 Hz, 1H), 4.97 - 4.87 (m, 1H), 4.41 - 4.33 (m, 1H), 4.29 (s, 1H), 3.83 - 3.77 (m, 3H), 3.75 (s, 2H), 3.72 (dd, J = 10.4, 4.3 Hz, 1H), 3.58 (d, J = 9.9 Hz, 1H), 3.49 - 3.39 (m, 1H), 3.24 - 3.11 (m, 5H), 2.45 (s, 3H), 2.27 - 2.15 (m, 1H), 1.98 (d, J = 14.0 Hz, 3H), 1.84 - 1.77 (m, 1H), 1.76 (s, 1H), 1.74 (d, J = 11.0 Hz, 1H), 1.36 (d, J = 7.0 Hz, 3H), 0.95 (d, J = 6.6 Hz, 3H), 0.80 (d, J = 6.6 Hz, 3H). |
| 151 | (2S,4R)-4-hydroxy-1-((2R)-2-(3-(8-(3-(2-hydroxyphenyl)cinnolin-6-yl)octahydro-2H-pyrazino[1,2-a]pyrazin-2-yl)isoxazol-5-yl)-3-methylbutanoyl)-N-((S)-1-(4-(4-methylthiazol-5-yl)phenyl)ethyl)pyrrolidine-2-carboxamide | 842.35 | 1H NMR (400 MHz, Methanol-d4) δ 8.87 (s, 1H), 8.46 (s, 1H), 8.17 (d, J = 9.6 Hz, 1H), 7.96 (dd, J = 8.2, 1.6 Hz, 1H), 7.81 (dd, J = 9.7, 2.7 Hz, 1H), 7.48 - 7.41 (m, 1H), 7.45 - 7.33 (m, 5H), 7.33 (d, J = 8.9 Hz, 1H), 7.11 (d, J = 2.6 Hz, 1H), 7.05 - 6.96 (m, 2H), 6.18 (s, 1H), 5.09 - 4.95 (m, 1H), 4.56 - 4.47 (m, 1H), 4.44 (s, 1H), 4.06 (t, J = 14.9 Hz, 2H), 3.85 (dd, J = 10.9, 4.2 Hz, 1H), 3.79 - 3.57 (m, 4H), 3.23 - 3.09 (m, 1H), 3.04 (d, J = 11.7 Hz, 1H), 2.96 (d, J = 11.2 Hz, 1H), 2.81 (d, J = 11.4 Hz, 1H), 2.77 (s, 1H), 2.50 (s, 2H), 2.47 (s, 3H), 2.44 (dd, J = 9.4, 3.3 Hz, 1H), 2.18 (t, J = 10.6 Hz, 1H), 2.02 - 1.91 (m, 1H), 1.52 (dd, J = 7.0, 2.3 Hz, 3H), 1.06 (d, J = 6.5 Hz, 3H), 0.96 - 0.86 (m, 3H). |
| 153 | (2S,4R)-4-hydroxy-1-((R)-2-(3-(4-(3-(2-hydroxyphenyl)cinnolin-7-yl)-1-methyl-1,4,9-triazaspiro[5.5]undecan-9-yl)isoxazol-5-yl)-3-methylbutanoyl)-N-((S)-1-(4-(4-methylthiazol-5-yl)phenyl)ethyl)pyrrolidine-2-carboxamide | 870.35 | 1H NMR (300 MHz, Methanol-d4) δ 8.86 (d, J = 7.2 Hz, 1H), 8.63 (s, 1H), 8.04 - 7.85 (m, 2H), 7.77 (dd, J = 9.5, 2.4 Hz, 1H), 7.53 (s, 1H), 7.47 - 7.36 (m, 4H), 7.32 (ddd, J = 8.8, 7.3, 1.6 Hz, 1H), 7.05 - 6.96 (m, 2H), 6.11 (d, J = 18.5 Hz, 1H), 5.03 (q, J = 7.1 Hz, 1H), 4.52 (t, J = 8.2 Hz, 2H), 3.85 (dd, J = 10.8, 4.1 Hz, 1H), 3.71 - 3.44 (m, 8H), 3.20 (d, J = 12.0 Hz, 2H), 3.04 (s, 2H), 2.47 (d, J = 5.6 Hz, 6H), 2.38 (dd, J = 10.0, 6.4 Hz, 1H), 2.24 - 1.90 (m, 4H), 1.81 (d, J = 13.5 Hz, 2H), 1.52 (d, J = 7.0 Hz, 3H), 1.06 (d, J = 6.5 Hz, 3H), 0.90 (d, J = 6.8 Hz, 3H). |
| 154 | (2S,4R)-4-hydroxy-1-((R)-2-(3-(2-(3-(2-hydroxyphenyl)cinnolin-7-yl)-2,6-diazaspiro[3.5]nonan-6-yl)isoxazol-5-yl)-3-methylbutanoyl)-N-((S)-1-(4-(4-methylthiazol-5-yl)phenyl)ethyl)pyrrolidine-2-carboxamide | 827.35 | 1H NMR (300 MHz, DMSO-d6) δ 12.93 (s, 1H), 8.97 (d, J = 3.8 Hz, 1H), 8.76 (s, 1H), 8.39 (d, J = 7.7 Hz, 1H), 8.03 (d, J = 7.7 Hz, 1H), 7.99 - 7.89 (m, 1H), 7.47 - 7.23 (m, 6H), 7.05 - 6.94 (m, 3H), 6.20 (d, J = 45.2 Hz, 1H), 5.13 (d, J = 3.5 Hz, 1H), 5.04 - 4.85 (m, 1H), 4.36 (t, J = 7.9 Hz, 1H), 4.29 (s, 1H), 3.93 - 3.77 (m, 4H), 3.77 - 3.67 (m, 1H), 3.62 - 3.48 (m, 1H), 3.48 - 3.38 (m, 3H), 3.17 (s, 2H), 2.44 (d, J = 4.7 Hz, 3H), 2.24 - 2.18 (m, 1H), 2.05 - 1.96 (m, 1H), 1.85 (s, 3H), 1.65 (s, 2H), 1.48 - 1.31 (m, 3H), 0.95 (d, J = 6.4 Hz, 3H), 0.88 - 0.73 (m, 3H). |
| 155 | (2S,4R)-4-hydroxy-1-((2R)-2-(3-(8-(3-(2-hydroxyphenyl)cinnolin-7-yl)octahydro-2H-pyrazino[1,2-a]pyrazin-2-yl)isoxazol-5-yl)-3-methylbutanoyl)-N-((S)-1-(4-(4-methylthiazol-5-yl)phenyl)ethyl)pyrrolidine-2-carboxamide | 842.35 | 1H NMR (400 MHz, DMSO-d6) δ 12.73 (s, 1H), 8.99 (s, 1H), 8.78 (s, 1H), 8.51 - 8.36 (m, 1H), 8.05 (dd, J = 7.9, 1.7 Hz, 1H), 7.97 (d, J = 9.3 Hz, 1H), 7.93 - 7.81 (m, 1H), 7.55 (d, J = 2.4 Hz, 1H), 7.49 - 7.29 (m, 5H), 7.01 (t, J = 8.2 Hz, 2H), 6.23 - 6.06 (m, 1H), 5.13 (d, J = 3.6 Hz, 1H), 5.05 - 4.82 (m, 1H), 4.38 (t, J = 7.9 Hz, 1H), 4.29 (s, 1H), 4.06 (d, J = 11.7 Hz, 2H), 3.73 (d, J = 11.6 Hz, 2H), 3.61 (d, J = 10.0 Hz, 2H), 3.46 (t, J = 11.0 Hz, 1H), 3.05 (t, J = 11.7 Hz, 1H), 3.00 - 2.81 (m, 3H), 2.66 (dt, J = 23.1, 11.3 Hz, 2H), 2.46 (s, 3H), 2.40 - 2.13 (m, 4H), 2.03 (t, J = 9.8 Hz, 1H), 1.79 (ddd, J = 12.7, 8.0, 4.8 Hz, 1H), 1.53 - 1.36 (m, 3H), 0.98 (t, J = 6.2 Hz, 3H), 0.81 (d, J = 6.6 Hz, 3H). |
| 156 | (2S,4R)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)-N-methylpyrrolidine-2-carboxamide | 612.25 | 1H NMR (400 MHz, DMSO-d6) δ 12.82 (d, J = 2.1 Hz, 1H), 8.76 (s, 1H), 8.03 (dd, J = 8.2, 1.6 Hz, 1H), 7.95 (d, J = 9.1 Hz, 1H), 7.86 (q, J = 4.5 Hz, 1H), 7.38 - 7.23 (m, 2H), 7.01 (d, J = 7.9 Hz, 3H), 5.82 (d, J = 61.5 Hz, 1H), 5.06 (dd, J = 44.3, 3.3 Hz, 1H), 4.29 (s, 5H), 4.22 (s, 1H), 4.11 (d, J = 4.4 Hz, 4H), 3.74 (dd, J = 10.5, 4.4 Hz, 1H), 3.58 (t, J = 10.3 Hz, 1H), 3.40 (dd, J = 14.0, 10.1 Hz, 1H), 2.57 (d, J = 4.7 Hz, 3H), 2.21 (dq, J = 16.5, 6.8 Hz, 1H), 1.96 (t, J = 10.1 Hz, 1H), 1.83 (ddd, J = 12.8, 8.2, 4.7 Hz, 1H), 0.94 (dd, J = 10.6, 6.5 Hz, 3H), 0.79 (dd, J = 6.6, 3.7 Hz, 3H). |
| 162 | (2S,4R)-4-hydroxy-1-((R)-2-(3-(2-(3-(2-hydroxyphenyl)cinnolin-7-yl)-5-oxa-2,8-diazaspiro[3.5]nonan-8-yl)isoxazol-5-yl)-3-methylbutanoyl)-N-((S)-1-(4-(4-methylthiazol-5-yl)phenyl)ethyl)pyrrolidine-2-carboxamide | 829.3 | 1H NMR (400 MHz, DMSO-d6) δ 12.84 (d, J = 10.7 Hz, 1H), 8.98 (s, 1H), 8.77 (s, 1H), 8.39 (d, J = 7.7 Hz, 1H), 8.04 (d, J = 8.1 Hz, 1H), 7.97 (dd, J = 9.0, 3.7 Hz, 1H), 7.48 - 7.39 (m, 2H), 7.39 - 7.27 (m, 4H), 7.08 (d, J = 1.9 Hz, 1H), 7.04 - 6.96 (m, 2H), 6.32 (s, 1H), 5.12 (d, J = 3.6 Hz, 1H), 4.91 (q, J = 7.2 Hz, 1H), 4.37 (t, J = 7.9 Hz, 1H), 4.29 (s, 1H), 4.13 (d, J = 9.1 Hz, 2H), 4.01 (d, J = 8.8 Hz, 2H), 3.81 (s, 2H), 3.72 (dd, J = 10.5, 4.3 Hz, 1H), 3.61 (d, J = 9.9 Hz, 1H), 3.48 (s, 3H), 3.22 (s, 2H), 3.08 (s, 0H), 2.45 (d, J = 1.4 Hz, 3H), 2.28 - 2.18 (m, 1H), 2.08 - 1.98 (m, 1H), 1.79 (ddd, J = 12.7, 8.3, 4.9 Hz, 1H), 1.39 - 1.32 (m, 3H), 0.97 (t, J = 6.5 Hz, 3H), 0.83 (dd, J = 13.4, 6.4 Hz, 3H). |
| 165 | (2S,4R)-4-hydroxy-1-((R)-2-(3-(4-(3-(2-hydroxyphenyl)cinnolin-6-yl)-1-methyl-1,4,9-triazaspiro[5.5]undecan-9-yl)isoxazol-5-yl)-3-methylbutanoyl)-N-((S)-1-(4-(4-methylthiazol-5-yl)phenyl)ethyl)pyrrolidine-2-carboxamide | 870.4 | 1H NMR (400 MHz, DMSO-d6) δ 13.17 (d, J = 9.9 Hz, 1H), 8.99 (s, 1H), 8.56 (s, 1H), 8.39 (d, J = 7.7 Hz, 1H), 8.18 (d, J = 9.5 Hz, 1H), 8.02 (d, J = 8.0 Hz, 1H), 7.93 (d, J = 9.8 Hz, 1H), 7.54 - 7.28 (m, 5H), 7.19 (d, J = 2.6 Hz, 1H), 7.00 (t, J = 7.7 Hz, 2H), 6.16 (s, 1H), 5.12 (d, J = 3.5 Hz, 1H), 5.01 - 4.87 (m, 1H), 4.36 (t, J = 7.9 Hz, 1H), 4.29 (s, 1H), 3.76 - 3.69 (m, 1H), 3.52 (m, J = 26.1, 24.8, 8.0 Hz, 8H), 3.18 (t, J = 11.5 Hz, 2H), 2.85 (s, 2H), 2.46 (d, J = 1.1 Hz, 3H), 2.31 (s, 3H), 2.24 (d, J = 10.0 Hz, 1H), 2.03 (d, J = 11.3 Hz, 1H), 1.91 (t, J = 10.3 Hz, 2H), 1.84 - 1.76 (m, 1H), 1.56 (d, J = 13.3 Hz, 2H), 1.42 (m, J = 34.3, 6.9 Hz, 3H), 0.96 (t, J = 7.3 Hz, 3H), 0.82 (m, J = 15.9, 6.7 Hz, 3H). |
| 166 | (2S,4R)-4-hydroxy-1-((R)-2-(3-(2-(3-(2-hydroxyphenyl)cinnolin-6-yl)-2,6-diazaspiro[3.5]nonan-6-yl)isoxazol-5-yl)-3-methylbutanoyl)-N-((S)-1-(4-(4-methylthiazol-5-yl)phenyl)ethyl)pyrrolidine-2-carboxamide | 827.4 | 1H NMR (400 MHz, DMSO-d6) δ 13.41 (d, J = 5.3 Hz, 1H),8.98 (d, J = 2.9 Hz, 1H), 8.50 (d, J = 6.1 Hz, 1H), 8.37 (d, J = 7.7 Hz, 1H), 8.18 (dd, J = 9.2, 5.6 Hz, 1H), 8.08 - 8.01 (m, 1H), 7.46 - 7.41 (m, 2H), 7.39- 7.24 (m, 4H), 7.03 - 6.95 (m, 2H), 6.58 - 6.50 (m, 1H), 6.27 - 6.01 (m, 1H), 5.11 (d, J = 3.6 Hz, 1H), 4.90 (t, J = 7.2 Hz, 1H), 4.36 - 4.29 (m, 2H),3.91 - 3.80 (m, 4H), 3.71 (dd, J = 10.5, 4.5 Hz, 1H), 3.57 (d, J = 9.8 Hz, 1H), 3.49 - 3.42 (m, 2H), 3.16 (s, 2H), 2.44 (d, J = 7.4 Hz, 3H), 2.22 (dt, J = 14.0, 7.2 Hz, 2H), 2.02 (t, J = 10.1 Hz, 1H), 1.85 - 1.73 (m, 3H), 1.64 (s, 2H), 1.36 (d, J = 7.0 Hz, 3H), 0.95 (t, J = 6.3 Hz, 3H), 0.82 (dd, J = 15.2, 7.1 Hz, 3H). |
| 181 | (2S,4R)-4-hydroxy-1-((R)-2-(3-(6-(3-(2-hydroxyphenyl)cinnolin-6-yl)-2,6-diazaspiro[3.3]heptan-2-yl)isoxazol-5-yl)-3-methylbutanoyl)-N-methylpyrrolidine-2-carboxamide | 612.25 | 1H NMR (400 MHz, Methanol-d4) δ 8.35 (s, 1H), 8.13 (d, J = 9.3 Hz, 1H), 7.95 (d, J = 7.9 Hz, 1H), 7.33 (td, J = 7.7, 1.6 Hz, 1H), 7.23 (dd, J = 9.3, 2.4 Hz, 1H), 7.04 - 6.89 (m, 2H), 6.55 (d, J = 2.3 Hz, 1H), 5.87 (d, J = 22.6 Hz, 1H), 4.68 - 4.36 (m, 2H), 4.31 (d, J = 2.2 Hz, 4H), 4.18 (s, 4H), 3.91 - 3.53 (m, 3H), 2.74 (d, J = 9.4 Hz, 3H), 2.43 - 2.25 (m, 1H), 2.15 (q, J = 11.4, 9.6 Hz, 1H), 2.03 (ddd, J = 13.2, 8.7, 4.5 Hz, 1H), 1.04 (dd, J = 14.1, 6.5 Hz, 3H), 0.89 (d, J = 6.7 Hz, 3H). |
| 187 | (2S,4R)-4-hydroxy-1-((R)-2-(3-((3aR,6aS)-5-(3-(2-hydroxyphenyl)cinnolin-7-yl)-3a,6a-dimethylhexahydropyrrolo[3,4-c]pyrrol-2(1 H)-yl)isoxazol-5-yl)-3-methylbutanoyl)-N-((S)-1-(4-(4-methylthiazol-5-yl)phenyl)ethyl)pyrrolidine-2-carboxamide | 841.3 | 1H NMR (400 MHz, Methanol-d4) δ 8.86 (s, 1H), 8.55 (d, J = 7.4 Hz, 1H), 7.97 - 7.90 (m, 1H), 7.87 (dd, J = 9.2, 5.6 Hz, 1H), 7.43 (dd, J = 9.6, 3.2 Hz, 4H), 7.42 - 7.25 (m, 2H), 7.06 - 6.94 (m, 3H), 5.96 (s, 1H), 5.07 - 4.92 (m, 1H), 4.50 (t, J = 8.2 Hz, 1H), 4.46 - 4.36 (m, 1H), 3.90 - 3.79 (m, 1H), 3.77 - 3.55 (m, 4H), 3.59 - 3.49 (m, 4H), 3.48 - 3.35 (m, 2H), 2.47 (s, 3H), 2.44 - 2.31 (m, 1H), 2.21 - 2.11 (m, 1H), 2.02 - 1.89 (m, 1H), 1.53 (dd, J = 17.5, 7.0 Hz, 3H), 1.28 (d, J = 3.0 Hz, 6H), 1.04 (d, J = 6.5 Hz, 3H), 0.91 (dd, J = 11.5, 6.7 Hz, 3H). |
| 188 | (2S,4R)-4-hydroxy-1-((R)-2-(3-((3aR,6aS)-5-(3-(2-hydroxyphenyl)cinnolin-6-yl)-3a,6a-dimethylhexahydropyrrolo[3,4-c]pyrrol-2(1 H)-yl)isoxazol-5-yl)-3-methylbutanoyl)-N-((S)-1-(4-(4-methylthiazol-5-yl)phenyl)ethyl)pyrrolidine-2-carboxamide | 841.35 | ¹H NMR (300 MHz, DMSO-d6) δ 13.43 (s, 1H), 8.98 (s, 1H), 8.48 (s, 1H), 8.38 (d, J = 7.5 Hz, 1H), 8.18 (d, J = 9.5 Hz, 1H), 8.02 (d, J = 8.0 Hz, 1H), 7.52 - 7.28 (m, 6H), 6.99 (d, J = 8.1 Hz, 2H), 6.66 (s, 1H), 5.91 (s, 1H), 5.09 (s, 1H), 5.01 - 4.83 (m, 1H), 4.44 - 4.25 (m, 2H), 3.66 (d, J = 11.2 Hz, 3H), 3.63 - 3.38 (m, 6H), 3.29 - 3.21 (m, 2H),2.40 (s, 3H), 2.21 (s, 1H), 2.05 (s, 1H), 1.79 (s, 1H), 1.48 - 1.32 (m, 3H), 1.19 (s, 6H), 0.94 (d, J = 6.4 Hz, 3H), 0.79 (d, J = 6.7 Hz, 3H). |
| 196 | (2S,4R)-4-hydroxy-1-((R)-2-(3-((S)-4-(3-(2-hydroxyphenyl)cinnolin-7-yl)-3-(methoxymethyl)piperazin-1-yl)isoxazol-5-yl)-3-methylbutanoyl)-N-((S)-1-(4-(4-methylthiazol-5-yl)phenyl)ethyl)pyrrolidine-2-carboxamide | 831.3 | 1H NMR (400 MHz, DMSO-d6) δ 12.75 (d, J = 5.3 Hz, 1H), 8.99 (d, J = 1.4 Hz, 1H), 8.77 (s, 1H), 8.41 (d, J = 7.8 Hz, 1H), 8.09 - 8.01 (m, 1H), 7.95 (q, J = 9.6 Hz, 2H), 7.52 (s, 1H), 7.49 - 7.40 (m, 2H), 7.40 - 7.27 (m, 3H), 7.06 - 6.96 (m, 2H), 6.18 (d, J = 49.3 Hz, 1H), 5.12 (d, J = 3.7 Hz, 1H), 4.93 (q, J = 7.2 Hz, 1H), 4.52 (s, 1H), 4.39 (t, J = 7.8 Hz, 1H), 4.30 (s, 1H), 3.91 - 3.76 (m, 3H), 3.73 (dd, J = 10.5, 4.4 Hz, 1H), 3.64 (t, J = 9.4 Hz, 2H), 3.50 - 3.43 (m, 2H), 3.43 (s, 1H), 3.39 (s, 1H), 3.21 (d, J = 2.5 Hz, 3H), 3.17 - 3.10 (m, 1H), 3.06 (d, J = 8.9 Hz, 1H), 2.46 (s, 3H), 2.28 (s, 1H), 2.10 - 2.00 (m, 1H), 1.87 - 1.73 (m, 1H), 1.44 (dd, J = 36.5, 7.0 Hz, 3H), 1.24 (s, 2H), 0.98 (t, J = 6.2 Hz, 3H), 0.84 (dd, J = 13.9, 6.5 Hz, 3H). |
| 221 | (2S,4R)-1-((R)-2-fluoro-2-(3-(6-(3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 817.4 | 1H NMR (400 MHz, DMSO-d6) δ 12.80 (s, 1H), 8.99 (s, 1H), 8.76 (s, 1H), 8.48 (d, J = 7.7 Hz, 1H), 8.04 - 7.95 (m, 2H), 7.44 (d, J = 7.8 Hz, 2H), 7.43 - 7.36 (m, 2H), 7.34 - 7.32 (m, 2H), 7.30 - 7.00 (m, 3H), 6.20 (s, 1H), 5.07 - 4.89 (m, 2H), 4.50 (t, J = 8.4 Hz, 1H), 4.30 (s, 4H), 4.16 (s, 4H), 3.66 (dd, J = 15.9, 10.2 Hz, 2H), 2.54 (s, 1H), 2.46 (s, 4H), 2.03 (t, J = 10.2 Hz, 1H), 1.68 (s, 1H), 1.39 (d, J = 7.1 Hz, 3H), 0.98 (d, J = 6.7 Hz, 3H), 0.82 (d, J = 6.9 Hz, 3H). |
| 224 | (2R,4S)-4-hydroxy-1-(2-(3-((1S,4S)-5-(3-(2-hydroxyphenyl)cinnolin-7-yl)-2,5-diazabicyclo[2.2.1]heptan-2-yl)isoxazol-5-yl)-3-methylbutanoyl)-N-((S)-1-(4-(4-methylthiazol-5-yl)phenyl)ethyl)pyrrolidine-2-carboxamide | 799.6 | 1H NMR (400 MHz, DMSO-d6) δ 12.94 - 12.89 (m, 1H), 8.97 - 8.93 (m, 1H), 8.69 (d, J = 6.8 Hz, 1H), 8.03 - 7.83 (m, 2H), 7.57 - 7.48 (m, 1H), 7.44 - 7.38 (m, 1H), 7.36 - 7.32 (m, 2H), 7.30 - 7.25 (m, 1H), 7.25 - 7.20 (m, 1H), 7.20 - 7.11 (m, 1H), 7.00 - 6.93 (m, 2H), 6.09 - 6.04 (m, 1H), 5.07 - 4.90 (m, 2H), 4.55 - 4.48 (m, 2H), 4.43 - 4.31 (m, 1H), 4.26 - 4.15 (m, 1H), 3.77 - 3.62 (m, 2H), 3.55 - 3.36 (m, 4H), 2.43 (d, J = 8.0 Hz, 3H), 2.33 - 2.16 (m, 2H), 2.11 - 2.03 (m, 2H), 1.80 - 1.71 (m, 1H), 1.39 (dd, J = 18.1, 6.9 Hz, 2H), 1.26 - 1.16 (m, 2H), 0.92 (d, J = 6.6 Hz, 3H), 0.82 - 0.68 (m, 3H). |
| 225 | (2S,4R)-4-hydroxy-1-(2-(3-(4-(1-(3-(2-hydroxyphenyl)cinnolin-7-yl)azetidin-3-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 841.6 | 1H NMR (400 MHz, DMSO-d₆) δ 12.88 (s, 1H), 8.97 - 8.94 (m, 1H), 8.72 (s, 1H), 8.34 (d, J = 6.7 Hz, 1H), 8.03 - 7.98 (m, 1H), 7.92 (d, J = 9.0 Hz, 1H), 7.48 - 7.22 (m, 6H), 7.00 - 6.94 (m, 3H), 6.13 (d, J = 7.7 Hz, 1H), 5.05 (s, 1H), 4.89 (d, J = 15.3 Hz, 1H), 4.39 (d, J = 22.1 Hz, 1H), 4.27 (s, 1H), 4.15 (q, J = 8.3 Hz, 2H), 3.87 - 3.77 (m, 2H), 3.73 - 3.58 (m, 3H), 3.57 - 3.50 (m, 1H), 3.42 (s, 1H), 2.76 (s, 2H), 2.45 - 2.42 (m, 3H), 2.02- (m, 1H), 1.87 - 1.59 (m, 5H), 1.35 (dd, J = 11.0, 7.0 Hz, 3H), 1.19 (d, J = 22.0 Hz, 3H), 0.94 (dd, J = 6.6, 4.0 Hz, 3H), 0.85 - 0.71 (m, 3H). |
| 226 | (2S,4R)-4-hydroxy-1-(2-(3-((3aS,6aS)-5-(3-(2-hydroxyphenyl)cinnolin-7-yl)hexahydropyrrolo[3,4-c]pyrrol-2(1 H)-yl)isoxazol-5-yl)-3-methylbutanoyl)-N-((S)-1-(4-(4-methylthiazol-5-yl)phenyl)ethyl)pyrrolidine-2-carboxamide | 813.8 | ¹H NMR (400 MHz, DMSO-d₆) δ 13.00 (d, *J* = 4.9 Hz, 1H), 8.98 - 8.92 (m, 1H), 8.71 (s, 1H), 8.17 (d, *J* = 7.9 Hz, 1H), 8.01 (d, *J* = 7.6 Hz, 1H), 7.94 (d, *J* = 9.2 Hz, 1H), 7.52 - 7.26 (m, 6H), 7.13 - 7.08 (m, 1H), 7.01 - 6.91 (m, 2H), 5.99 - 5.91 (m, 1H), 5.06 (d, *J* = 3.7 Hz, 1H), 4.95 - 4.85 (m, 1H), 4.46 - 4.36 (m, 1H), 4.30 - 4.21 (m, 1H), 3.79 - 3.68 (m, 4H), 3.60 - 3.41 (m, 4H), 3.36 - 3.28 (m, 3H), 3.20 - 3.08 (m, 3H), 2.46 - 2.37 (m, 3H), 2.06 - 1.97 (m, 1H), 1.84 - 1.73 (m, 1H), 1.46 (d, *J* = 7.0 Hz, 1H), 1.40 - 1.33 (m, 2H), 0.96 (t, *J* = 6.2 Hz, 3H), 0.87 - 0.73 (m, 3H). |
| 228 | (2S,4R)-4-hydroxy-1-((R)-2-(3-(5-(3-(2-hydroxyphenyl)cinnolin-7-yl)-3,4,5,6-tetrahydropyrrolo[3,4-c]pyrrol-2(1 H)-yl)isoxazol-5-yl)-3-methylbutanoyl)-N-((S)-1-(4-(4-methylthiazol-5-yl)phenyl)ethyl)pyrrolidine-2-carboxamide | 811.3 | 1H NMR (400 MHz, DMSO-d6) δ 13.44 (s, 1H), 8.98 (d, J = 10.0 Hz, 1H), 8.54 (d, J = 5.5 Hz, 1H), 8.43 (d, J = 7.7 Hz, 1H), 8.25 (d, J = 8.9 Hz, 1H), 8.06 (d, J = 8.0 Hz, 1H), 7.84 - 7.22 (m, 7H), 7.11 - 6.92 (m, 2H), 6.75 - 6.56 (m, 1H), 5.97 (d, J = 40.9 Hz, 1H), 5.20 - 4.87 (m, 2H), 4.47 - 4.29 (m, 5H), 4.31 - 4.04 (m, 5H),3.71 - 3.91 (m, 1H),3.62 - 3.7(m, 2H) ,2.47 (s, 3H), 2.26 (s, 1H), 2.04 (s, 1H), 1.86 - 1.74 (m, 1H), 1.52 - 1.35 (m, 3H), 0.98 (d, J = 6.5 Hz, 3H), 0.87 - 0.77 (m, 3H). |
| 229 | (2S,4R)-4-hydroxy-1-((R)-2-(3-(5-(3-(2-hydroxyphenyl)cinnolin-6-yl)-3,4,5,6-tetrahydropyrrolo[3,4-c]pyrrol-2(1H)-yl)isoxazol-5-yl)-3-methylbutanoyl)-N-((S)-1-(4-(4-methylthiazol-5-yl)phenyl)ethyl)pyrrolidine-2-carboxamide | 811.25 | 1H NMR (400 MHz, DMSO-d6) δ 13.41 (s, 1H), 9.13 - 8.91 (m, 1H), 8.55 (s, 1H), 8.47 - 7.79 (m, 2H), 7.69 - 7.17 (m, 6H), 7.09 - 6.91 (m, 2H), 6.78 - 6.58 (m, 1H), 6.27 - 5.80 (m, 1H), 5.12 (s, 1H), 4.98 - 4.86 (m, 1H), 4.68 - 3.99 (m, 10H), 3.81 - 3.62 (m, 3H), 2.48 - 2.43 (m, 3H), 2.35 - 2.17 (m, 2H), 2.09 - 1.93 (m, 1H), 1.88 - 1.70 (m, 1H), 1.60 - 1.37 (m, 3H), 1.06 - 0.95 (m, 2H), 0.90 - 0.72 (m, 4H). |
| 246 | (2S,4R)-1-((R)-2-(3-(6-(3-(3-fluoro-2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 817.25 | 1H NMR (400 MHz, DMSO-d6) δ 13.56 (s, 1H), 9.00 (d, J = 6.3 Hz, 1H), 8.83 (s, 1H), 8.44 (d, J = 7.6 Hz, 1H), 7.90 (dd, J = 36.4, 8.5 Hz, 2H), 7.53 - 7.42 (m, 2H), 7.38 (d, J = 7.9 Hz, 2H), 7.28 (ddd, J = 10.5, 6.1, 1.8 Hz, 2H), 7.06 - 6.92 (m, 2H), 5.88 (d, J = 42.0 Hz, 1H), 5.15 (s, 1H), 4.99 - 4.66 (m, 1H), 4.38 (t, J = 7.9 Hz, 1H), 4.30 (s, 5H), 4.13 (s, 4H), 3.72 (dd, J = 10.6, 4.4 Hz, 1H), 3.62 (d, J = 9.6 Hz, 1H), 3.46 (dd, J = 14.7, 10.0 Hz, 1H), 2.47 (d, J = 4.7 Hz, 3H), 2.29- 2.15 (m, 1H), 2.10 - 1.89 (m, 1H), 1.85- 1.76 (m, 1H), 1.43 (dd, J = 33.6, 7.0 Hz, 3H), 0.97 (d, J = 6.4 Hz, 3H), 0.83 (dd, J = 15.0, 6.6 Hz, 3H). |
| 104 | (2S,4R)-4-hydroxy-1-((R)-2-(3-(2-(3-(2-hydroxyphenyl)cinnolin-7-yl)-1-oxo-2,8-diazaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 855.4 | 1H NMR (400 MHz, DMSO-d6) δ 11.96 (s, 1H), 8.99 (d, J = 2.3 Hz, 1H), 8.89 (s, 1H), 8.62 - 8.30 (m, 3H), 8.19 - 8.08 (m, 2H), 7.63 - 7.22 (m, 5H), 7.10 - 7.00 (m, 2H), 6.15 (d, J = 53.0 Hz, 1H), 5.13 (d, J = 3.7 Hz, 1H), 4.98 - 4.87 (m, 1H), 4.39 (t, J = 7.8 Hz, 1H), 4.30 (s, 1H), 4.09 (t, J = 6.9 Hz, 2H), 3.79 - 3.70 (m, 1H), 3.70 - 3.55 (m, 3H), 3.45 (d, J = 10.4 Hz, 1H), 3.04 (t, J = 12.1 Hz, 2H), 2.46 (d, J = 2.4 Hz, 3H), 2.23 (s, 3H), 2.04 (t, J = 10.1 Hz, 1H), 1.95 - 1.74 (m, 3H), 1.66 (d, J = 12.8 Hz, 2H), 1.43 (dd, J = 34.4, 6.9 Hz, 3H), 0.98 (t, J = 6.9 Hz, 3H), 0.84 (dd, J = 16.0, 6.7 Hz, 3H). |
| 103 | (2S,4R)-4-hydroxy-1-((R)-2-(3-(9-(3-(2-hydroxyphenyl)cinnolin-7-yl)-8-oxo-3,9-diazaspiro[5.5]undecan-3-yl)isoxazol-5-yl)-3-methylbutanoyl)-N-((S)-1-(4-(4-methylthiazol-5-yl)phenyl)ethyl)pyrrolidine-2-carboxamide | 869.35 | 1H NMR (400 MHz, DMSO-d6) δ 11.91 (d, J = 3.7 Hz, 1H), 9.13 - 8.70 (m, 2H), 8.51 - 8.31 (m, 2H), 8.25 - 8.02 (m, 2H), 7.94 (dd, J = 8.9, 2.0 Hz, 1H), 7.52 - 7.42 (m, 2H), 7.41 - 7.34 (m, 3H), 7.12 - 6.97 (m, 2H), 6.12 (d, J = 52.4 Hz, 1H), 5.12 (d, J = 3.6 Hz, 1H), 5.03 - 4.85 (m, 1H), 4.37 (t, J = 7.9 Hz, 1H), 4.29 (s, 1H), 3.94 (t, J = 6.2 Hz, 2H), 3.72 (dd, J = 10.5, 4.5 Hz, 1H), 3.58 (d, J = 9.9 Hz, 1H), 3.45 (t, J = 9.2 Hz, 1H), 3.28 (s, 4H), 2.46 (s, 4H), 2.35 - 2.14 (m, 2H), 2.08 - 1.87 (m, 3H), 1.79 (ddd, J = 12.8, 8.0, 4.7 Hz, 1H), 1.64 (d, J = 6.4 Hz, 4H), 1.43 (dd, J = 34.8, 7.0 Hz, 3H), 1.02 - 0.77 (m, 6H). |
| 253 | (2S,4R)-1-((R)-2-(3-(6-(6-cyclopropoxy-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 855.35 | 1H NMR (400 MHz, Methanol-d4) δ 8.89 (d, J = 4.6 Hz, 1H), 8.49 (s, 1H), 7.94 (dd, J = 8.3, 1.6 Hz, 1H), 7.51 (s, 1H), 7.49 - 7.36 (m, 4H), 7.35 - 7.24 (m, 1H), 7.09 - 6.95 (m, 2H), 6.84 (s, 1H), 5.91 (s, 1H), 5.04 (q, J = 7.2 Hz, 1H), 4.52 (t, J = 8.2 Hz, 1H), 4.43 (s, 1H), 4.33 (s, 4H), 4.15 (d, J = 4.8 Hz, 4H), 4.10 - 3.99 (m, 1H), 3.91 - 3.80 (m, 1H), 3.69 - 3.55 (m, 2H), 2.48 (d, J = 3.5 Hz, 3H), 2.46 - 2.28 (m, 1H), 2.24 - 2.13 (m, 1H), 2.04 - 1.88 (m, 1H), 1.55 (dd, J = 7.0, 7.0 Hz, 3H), 1.06 (d, J = 6.5 Hz, 3H), 0.99 - 0.84 (m, 7H). |
| 241 | (2S,4R)-1-((R)-2-(3-(6-(7-ethynyl-3-(2-hydroxyphenyl)cinnolin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 823.3 | 1H NMR (300 MHz, DMSO-d6) δ 12.80 (s, 1H), 8.99 (s, 1H), 8.55 (s, 1H), 8.41 (d, J = 7.6 Hz, 1H), 8.33 (s, 1H), 8.04 (d, J = 7.8 Hz, 1H), 7.87 - 7.21 (m, 5H), 7.02 (d, J = 7.9 Hz, 2H), 6.65 (s, 1H), 5.91 (s, 1H), 5.12 (s, 1H), 5.05 - 4.75 (m, 1H), 4.72 (s, 1H), 4.50 (s, 4H), 4.43 - 4.33 (m, 1H), 4.30 (s, 1H), 4.11 (s, 4H), 3.80 - 3.66 (m, 1H), 3.61 (d, J = 9.7 Hz, 1H), 3.53 - 3.38 (m, 1H), 2.49 - 2.39 (m, 3H), 2.30 - 2.13 (m, 1H), 2.09 - 1.94 (m, 1H), 1.88 - 1.62 (m, 1H), 1.52 - 1.33 (m, 3H), 1.05 - 0.89 (m, 3H), 0.90 - 0.61 (m, 3H). |
| 240 | (2S,4R)-1-((R)-2-(3-(6-(5-ethynyl-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 823.25 | 1H NMR (300 MHz, DMSO-d6) δ 11.60 (s, 1H), 9.09 - 8.87 (m, 1H), 8.78 - 8.62 (m, 1H), 8.49 - 8.31 (m, 1H), 8.12 - 7.97 (m, 1H), 7.54 - 7.43 (m, 3H), 7.41 - 7.24 (m, 4H), 7.20 - 6.96 (m, 3H), 6.08 - 5.76 (m, 1H), 5.21 - 4.78 (m, 3H), 4.48 - 4.23 (m, 6H), 4.12 (s, 4H), 3.83 - 3.48 (m, 2H), 2.48 - 2.44 (m, 3H), 2.30 - 2.13 (m, 1H), 2.11 - 1.92 (m, 1H), 1.90 - 1.61 (m, 1H), 1.56 - 1.29 (m, 3H), 1.04 - 0.91 (m, 3H), 0.88 - 0.75 (m, 3H). |
| 239 | (2S,4R)-1-((R)-2-(3-(6-(6-ethynyl-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 823.3 | 1H NMR (300 MHz, DMSO-d6) δ 12.29 (s, 1H), 8.99 (s, 1H), 8.68 (s, 1H), 8.42 (d, J = 7.6 Hz, 1H), 8.14 (s, 1H), 7.99 (d, J = 7.6 Hz, 1H), 7.52 - 7.27 (m, 5H), 7.11 (s, 1H), 7.02 (d, J = 7.8 Hz, 2H), 5.91 (s, 1H), 5.12 (s, 1H), 4.92 (t, J = 7.1 Hz, 1H), 4.77 (s, 1H), 4.48 (s, 4H), 4.38 (t, J = 7.7 Hz, 1H), 4.30 (s, 1H), 4.12 (s, 4H), 3.72 (d, J = 6.1 Hz, 1H), 3.61 (d, J = 9.8 Hz, 1H), 3.43 (d, J = 11.0 Hz, 1H), 2.47 (d, J = 3.4 Hz, 3H), 2.22 (s, 1H), 2.03 (s, 1H), 1.79 (s, 1H), 1.43 (dd, J = 24.2, 7.0 Hz, 3H), 0.96 (d, J = 6.5 Hz, 3H), 0.88 - 0.76 (m, 3H). |
| 237 | (2S,4R)-1-((R)-2-(3-(6-(6-ethoxy-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 843.25 | 1H NMR (300 MHz, DMSO-d6) δ 13.39 (s, 1H), 9.03 - 8.96 (m, 1H), 8.61 (s, 1H), 8.41 (d, J = 7.5 Hz, 1H), 7.99 (d, J = 8.0 Hz, 1H), 7.49 - 7.21 (m, 6H), 7.04 - 6.95 (m, 2H), 6.93 (s, 1H), 5.91 (s, 1H), 5.11 (d, J = 3.6 Hz, 1H), 4.95 - 4.89 (m, 1H), 4.42 - 4.19 (m, 9H), 4.10 (s, 4H), 3.61 (d, J = 9.9 Hz, 2H), 2.50 - 2.43 (m, 3H), 2.30 - 1.71 (m, 3H), 1.53 - 1.43 (m, 4H), 1.38 (d, J = 6.9 Hz, 2H), 1.00 - 0.92 (m, 3H), 0.88 - 0.76 (m, 3H). |
| 236 | (2S,4R)-1-((R)-2-(3-(6-(6-(difluoromethyl)-3-(2-hydroxyphenyl)cinnolin-7-yl)-2,6-diazaspiro[3.3]heptan-2-yl)isoxazol-5-yl)-3-methylbutanoyl)-N-((S)-1-(4-fluorophenyl)ethyl)-4-hydroxypyrrolidine-2-carboxamide | 770.3 | 1H NMR (300 MHz, DMSO-d6) δ 12.61 (d, J = 6.2 Hz, 1H), 8.62 (s, 1H), 8.48 (s, 1H), 8.36 (d, J = 7.8 Hz, 1H), 8.14 - 8.02 (m, 1H), 7.45 - 7.25 (m, 4H), 7.15 (q, J = 8.8 Hz, 2H), 7.02 (dd, J = 8.2, 7.0 Hz, 2H), 6.74 (s, 1H), 5.87 (d, J = 33.3 Hz, 1H), 5.06 (dd, J = 31.8, 3.2 Hz, 1H), 4.88 (t, J = 7.2 Hz, 1H), 4.44 (s, 4H), 4.33 (dd, J = 17.1, 9.1 Hz, 2H), 4.13 (s, 4H), 3.71 (dd, J = 10.4, 4.3 Hz, 1H), 3.61 (d, J = 9.8 Hz, 1H), 3.44 (t, J = 8.8 Hz, 1H), 2.31 - 2.15 (m, 1H), 2.02 (d, J = 12.2 Hz, 1H), 1.75 (dt, J = 12.8, 6.0 Hz, 1H), 1.39 (dd, J = 24.8, 7.0 Hz, 3H), 0.96 (d, J = 6.5 Hz, 3H), 0.82 (t, J = 7.0 Hz, 3H). |
| 217 | (2S,4R)-1-((R)-2-(3-(6-(6-cyano-3-(2-hydroxyphenyl)cinnolin-7-yl)-2,6-diazaspiro[3.3]heptan-2-yl)isoxazol-5-yl)-3-methylbutanoyl)-N-((S)-1-(4-fluorophenyl)ethyl)-4-hydroxypyrrolidine-2-carboxamide | 745.3 | 1H NMR (400 MHz, DMSO-d6) δ 11.39 (s, 1H), 8.70 (s, 1H), 8.65 (s, 1H), 8.35 (d, J = 7.7 Hz, 1H), 8.02 (dd, J = 7.8, 1.7 Hz, 1H), 7.38 - 7.27 (m, 4H), 7.20 - 7.08 (m, 2H), 7.08 - 6.98 (m, 2H), 5.91 (s, 1H), 5.10 (d, J = 3.6 Hz, 1H), 4.92 - 4.82 (m, 1H), 4.52 (d, J = 3.0 Hz, 4H), 4.39 - 4.30 (m, 1H), 4.28 (s, 1H), 4.13 (s, 4H), 3.71 (dd, J = 10.7, 4.5 Hz, 1H), 3.60 (d, J = 9.8 Hz, 1H), 3.48 - 3.38 (m, 1H), 2.29 - 2.13 (m, 1H), 2.04 - 1.94 (m, 1H), 1.80 - 1.69 (m, 1H), 1.34 (d, J = 7.0 Hz, 3H), 1.00 - 0.92 (m, 3H), 0.82 (dd, J = 12.6, 6.6 Hz, 3H). |
| 209 | (2S,4R)-4-hydroxy-1-((R)-2-(3-(6-(3-(2-hydroxyphenyl)-6-methoxycinnolin-7-yl)-2,6-diazaspiro[3.3]heptan-2-yl)isoxazol-5-yl)-3-methylbutanoyl)-N-methylpyrrolidine-2-carboxamide | 642.2 | 1H NMR (300 MHz, DMSO-d6) δ 13.35 (s, 1H), 8.63 (s, 1H), 8.19 - 7.75 (m, 2H), 7.43 - 7.15 (m, 2H), 7.07 - 6.81 (m, 3H), 5.95 - 5.68 (m, 1H), 5.13 - 4.93 (m, 1H), 4.47 - 4.19 (m, 6H), 4.16 - 4.04 (m, 4H), 3.99 (s, 3H), 3.83 - 3.69 (m, 1H), 3.65 - 3.51 (m, 1H), 3.49 - 3.35 (m, 1H), 2.64 - 2.55 (m, 3H), 2.32 - 2.11 (m, 1H), 2.05 - 1.75 (m, 2H), 1.03 - 0.90 (m, 3H), 0.88 - 0.77 (m, 3H). |
| 207 | (2S,4R)-1-((R)-2-(3-(6-(6-(dimethylamino)-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 842.3 | 1H NMR (400 MHz, Methanol-d4) δ 8.88 (d, J = 4.7 Hz, 1H), 8.50 (s, 1H), 7.96 (dd, J = 8.4, 1.6 Hz, 1H), 7.47 - 7.37 (m, 4H), 7.34 - 7.26 (m, 2H), 7.12 (s, 1H), 7.04 - 6.92 (m, 2H), 5.89 (d, J = 21.3 Hz, 1H), 5.04 (q, J = 7.1 Hz, 1H), 4.59 - 4.49 (m, 1H), 4.43 (d, J = 14.4 Hz, 1H), 4.23 (s, 4H), 4.17 (d, J = 3.5 Hz, 4H), 3.96 - 3.81 (m, 1H), 3.70 - 3.51 (m, 2H), 2.90 (s, 6H), 2.48 (d, J = 3.2 Hz, 3H), 2.44 - 2.29 (m, 1H), 2.24 - 2.12 (m, 1H), 1.96 (ddd, J = 13.3, 8.7, 4.6 Hz, 1H), 1.56 (dd, J = 28.7, 7.0 Hz, 3H), 1.05 (d, J = 6.5 Hz, 3H), 0.91 (dd, J = 9.6, 6.7 Hz, 3H). |
| 206 | (2S,4R)-1-((R)-2-(3-(6-(6-(difluoromethoxy)-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 865.3 | 1H NMR (400 MHz, DMSO-d6) δ 12.62 - 12.56 (m, 1H), 9.02 - 8.96 (m, 1H), 8.76 (s, 1H), 8.41 (d, J = 7.7 Hz, 1H), 8.02 (d, J = 7.5 Hz, 1H), 7.73 - 7.61 (m, 1H), 7.51 - 7.27 (m, 6H), 7.15 (s, 1H), 7.05 - 6.97 (m, 2H), 5.91 (s, 1H), 5.11 (d, J = 3.6 Hz, 1H), 4.96 - 4.88 (m, 1H), 4.40 (s, 4H), 4.39 - 4.27 (m, 2H), 4.12 (s, 4H), 3.74 - 3.57 (m, 3H), 2.49 - 2.44 (m, 2H), 2.26 - 2.17 (m, 2H), 2.05 - 2.00 (m, 1H), 1.85 - 1.74 (m, 1H), 1.38 (d, J = 7.0 Hz, 3H), 1.01 - 0.93 (m, 3H), 0.87 - 0.77 (m, 3H). |
| 205 | (2S,4R)-1-((R)-2-(3-(6-(5-(difluoromethyl)-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 849.3 | 1H NMR (400 MHz, DMSO-d6) δ 11.77 - 11.72 (m, 1H), 9.03 - 8.97 (m, 1H), 8.74 (s, 1H), 8.42 (d, J = 7.7 Hz, 1H), 8.10 - 8.03 (m, 1H), 7.64 (s, 1H), 7.55 - 7.29 (m, 6H), 7.23 - 7.18 (m, 1H), 7.06 - 6.98 (m, 2H), 5.93 (s, 1H), 5.12 (d, J = 3.6 Hz, 1H), 4.96 - 4.88 (m, 1H), 4.38 (d, J = 7.8 Hz, 1H), 4.33 - 4.27 (m, 5H), 4.13 (s, 4H), 3.74 - 3.50 (m, 3H), 2.49 - 2.44 (m, 3H), 2.24 - 2.19 (m, 1H), 2.10 - 1.98 (m, 1H), 1.84 - 1.73 (m, 1H), 1.47 (d, J = 6.9 Hz, 3H), 1.01 - 0.93 (m, 3H), 0.87 - 0.77 (m, 3H). |
| 191 | (2S,4R)-1-(2-(3-(6-(5-(difluoromethyl)-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 849.35 | 1H NMR (400 MHz, DMSO-d6) δ 11.75 (s, 1H), 9.01 - 8.96 (m, 1H), 8.74 (s, 1H), 8.41- 8.27 (m, 1H), 8.10 - 8.03 (m, 1H), 7.78 (s, 1H), 7.64 (s, 1H), 7.54 - 7.29 (m, 5H), 7.22 - 7.18 (m, 2H), 7.06 - 6.98 (m, 1H), 5.98 - 5.90 (m, 1H), 5.14 - 5.08 (m, 1H), 5.03 - 4.91 (m, 1H), 4.47 - 4.28 (m, 5H), 4.16 - 4.05 (m, 4H), 3.76 - 3.69 (m, 1H), 3.65-3.52 (m, 2H), 2.49 - 2.44 (m, 3H), 2.28 - 2.20 (m, 1H), 2.03 (s, 2H), 1.83 - 1.73 (m, 1H), 1.47 (d, J = 7.0 Hz, 1H), 1.42 - 1.34 (m, 2H), 1.00 - 0.93 (m, 3H), 0.87 - 0.73 (m, 3H). |
| 180 | (2S,4R)-1-((R)-2-(3-(6-(6-cyclopropyl-3-(3-fluoro-2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 857.3 | 1H NMR (400 MHz, DMSO-d6) δ 13.66 (s, 1H), 8.99 (d, J = 4.8 Hz, 1H), 8.72 (s, 1H), 8.41 (d, J = 7.7 Hz, 1H), 7.86 - 7.74 (m, 1H), 7.63 (s, 1H), 7.52 - 7.33 (m, 4H), 7.29 (ddd, J = 11.1, 8.0, 1.4 Hz, 1H), 7.04 (s, 1H), 6.98 (td, J = 8.0, 5.1 Hz, 1H), 5.87 (d, J = 43.5 Hz, 1H), 5.11 (d, J = 3.6 Hz, 1H), 5.02 - 4.88 (m, 1H), 4.50 (s, 4H), 4.38 (t, J = 7.9 Hz, 1H), 4.30 (s, 1H), 4.13 (s, 4H), 3.72 (dd, J = 10.5, 4.5 Hz, 1H), 3.61 (d, J = 9.8 Hz, 1H), 3.45 (dd, J = 14.8, 10.0 Hz, 1H), 2.46 (d, J = 4.6 Hz, 3H), 2.28 - 1.98 (m, 3H), 1.79 (ddd, J = 16.2, 8.2, 4.2 Hz, 1H), 1.43 (dd, J = 33.1, 7.0 Hz, 3H), 1.15 - 1.07 (m, 2H), 0.97 (t, J = 6.4 Hz, 3H), 0.91 (td, J = 6.0, 4.1 Hz, 2H), 0.82 (dd, J = 14.5, 6.7 Hz, 3H). |
| 179 | (2S,4R)-1-((R)-2-(3-(6-(6-fluoro-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 817.25 | 1H NMR (400 MHz, DMSO-d6) δ 12.43 (s, 1H), 8.99 (dd, J = 4.9, 2.3 Hz, 1H), 8.84 - 8.71 (m, 1H), 8.41 (d, J = 7.5 Hz, 1H), 8.01 (dd, J = 7.8, 2.7 Hz, 1H), 7.78 (dt, J = 12.6, 2.4 Hz, 1H), 7.51 - 7.42 (m, 2H), 7.41 - 7.28 (m, 3H), 7.20 (d, J = 8.5 Hz, 1H), 7.01 (dd, J = 9.1, 7.0 Hz, 2H), 5.95 - 5.79 (m, 1H), 5.12 (s, 1H), 5.03 - 4.88 (m, 1H), 4.39 (d, J = 14.9 Hz, 5H), 4.30 (s, 1H), 4.12 (d, J = 2.4 Hz, 4H), 3.71 (dd, J = 10.6, 4.6 Hz, 1H), 3.64 - 3.56 (m, 1H), 3.43 (d, J = 11.1 Hz, 1H), 2.48 - 2.45 (m, 3H), 2.26 - 2.17 (m, 1H), 2.03 (s, 1H), 1.84 - 1.76 (m, 1H), 1.49 - 1.35 (m, 3H), 1.04 - 0.92 (m, 3H), 0.87 - 0.77 (m, 3H). |
| 173 | (2S,4R)-1-((R)-2-(3-(6-(5-cyano-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 824.25 | 1H NMR (300 MHz, DMSO-d6) δ 11.14 (brs, 1H), 9.00 (d, J = 4.6 Hz, 1H), 8.66 (s, 1H), 8.41 (d, J = 7.6 Hz, 1H), 8.16 (d, J = 7.5 Hz, 1H), 8.00 (d, J = 2.3 Hz, 1H), 7.52 - 7.41 (m, 2H), 7.41 - 7.29 (m, 4H), 7.09 - 6.98 (m, 2H), 5.93 (s, 1H), 5.12 (d, J = 3.6 Hz, 1H), 5.04 - 4.84 (m, 1H), 4.48 - 4.25 (m, 6H), 4.12 (s, 4H), 3.77 - 3.54 (m, 1H), 3.54 - 3.39 (m, 2H), 2.47 (d, J = 3.6 Hz, 3H), 2.30 - 2.13 (m, 1H), 2.09 - 1.95 (m, 1H), 1.87 - 1.73 (m, 1H), 1.51 - 1.34 (m, 3H), 0.96 (d, J = 6.4 Hz, 3H), 0.80 (d, J = 6.7 Hz, 3H). |
| 168 | (2S,4R)-1-((R)-2-(3-(6-(6-(difluoromethyl)-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 849.35 | 1H NMR (300 MHz, DMSO-d6) δ 12.15 (d, J = 3.9 Hz, 1H), 8.99 (d, J = 3.5 Hz, 1H), 8.86 (s, 1H), 8.41 (d, J = 7.7 Hz, 1H), 8.30 (s, 1H), 8.02 (d, J = 7.6 Hz, 1H), 7.59 - 7.28 (m, 6H), 7.18 (d, J = 12.0 Hz, 1H), 7.03 (d, J = 7.7 Hz, 2H), 5.88 (d, J = 32.2 Hz, 1H), 5.11 (d, J = 3.6 Hz, 1H), 4.92 (t, J = 7.1 Hz, 1H), 4.40 (d, J = 12.7 Hz, 5H), 4.30 (s, 1H), 4.13 (s, 4H), 3.72 (dd, J = 10.6, 4.4 Hz, 1H), 3.61 (d, J = 9.7 Hz, 1H), 3.45 (t, J = 11.0 Hz, 1H), 2.47 (d, J = 3.6 Hz, 3H), 2.22 (t, J = 8.2 Hz, 1H), 2.03 (t, J = 10.1 Hz, 1H), 1.86 - 1.73 (m, 1H), 1.43 (dd, J = 24.7, 6.9 Hz, 3H), 0.96 (d, J = 6.5 Hz, 3H), 0.88 - 0.76 (m, 3H). |
| 167 | (2S,4R)-1-((R)-2-(3-(6-(6-cyano-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 824.3 | 1H NMR (400 MHz, DMSO-d6) δ 11.42 (s, 1H), 8.99 (s, 1H), 8.70 (s, 1H), 8.66 (s, 1H), 8.42 (d, J = 7.6 Hz, 1H), 8.05 - 7.99 (m, 1H), 7.51 - 7.41 (m, 2H), 7.4 - 7.30 (m, 3H), 7.28 (s, 1H), 7.06 - 6.99 (m, 2H), 5.92 (s, 1H), 5.15 - 5.10 (m, 1H), 4.95 - 4.88 (m, 1H), 4.52 (s, 4H), 4.38 (t, J = 7.9 Hz, 1H), 4.33 - 4.26 (m, 1H), 4.14 (s, 4H), 3.76 - 3.68 (m, 1H), 3.63 - 3.55 (m, 1H), 3.47 - 3.40 (m, 1H), 2.46 (s, 3H), 2.28 - 2.15 (m, 1H), 2.08 - 1.96 (m. 1H), 1.85 - 1.74 (m, 1H), 1.47 (d, J = 7.0 Hz, 1H), 1.39 (d, J = 7.0 Hz, 2H), 0.96 (d, J = 6.5 Hz, 3H), 0.82 (dd, J = 15.4, 6.9 Hz, 3H). |
| 164 | (2S,4R)-1-((R)-2-(3-(6-(7-(difluoromethyl)-3-(2-hydroxyphenyl)cinnolin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 849.3 | 1H NMR (300 MHz, DMSO-d6) δ 12.60 (s, 1H), 8.99 (d, J = 3.7 Hz, 1H), 8.62 (s, 1H), 8.48 (s, 1H), 8.41 (d, J = 7.7 Hz, 1H), 8.07 (d, J = 7.9 Hz, 1H), 7.50 - 7.41 (m, 2H), 7.41 - 7.31 (m, 4H), 7.02 (t, J = 7.6 Hz, 2H), 6.74 (s, 1H), 5.92 (s, 1H), 5.11 (d, J = 3.6 Hz, 1H), 4.92 (t, 1H), 4.44 (s, 4H), 4.36 (t, 1H), 4.30 (s, 1H), 4.12 (s, 4H), 3.72 (d, J = 6.9 Hz, 1H), 3.61 (d, J = 10.0 Hz, 1H), 3.43 (d, J = 11.2 Hz, 1H), 2.46 (d, J = 3.4 Hz, 3H), 2.32 - 2.12 (m, 1H), 2.10 - 1.96 (m, 1H), 1.85 - 1.71 (m, 1H), 1.42 (dd, J = 24.4, 7.0 Hz, 3H), 0.96 (d, J = 6.4 Hz, 3H), 0.80 (d, J = 6.7 Hz, 3H). |
| 163 | (2S,4R)-1-((R)-2-(3-(6-(5-chloro-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 833.25 | 1H NMR (300 MHz, DMSO-d6) δ 11.53 (s, 1H), 9.05 - 8.89 (m, 1H), 8.67 (s, 1H), 8.42 (d, J = 7.6 Hz, 1H), 8.18 - 8.07 (m, 1H), 7.54 - 7.21 (m, 6H), 7.15 - 6.83 (m, 3H), 5.98 - 5.80 (m, 1H), 5.12 (s, 1H), 4.99 - 4.81 (m, 1H), 4.42 - 4.22 (m, 6H), 4.12 (s, 4H), 3.85 - 3.66 (m, 1H), 3.61 (d, J = 9.7 Hz, 1H), 3.52 - 3.39 (m, 1H), 2.48 - 2.45 (m, 3H), 2.33 - 2.13 (m, 1H), 2.10 - 1.93 (m, 1H), 1.89 - 1.67 (m, 1H), 1.53 - 1.31 (m, 3H), 1.00 - 0.92 (m, 3H), 0.89 - 0.71 (m, 3H). |
| 159 | (2S,4R)-4-hydroxy-1-((R)-2-(3-(6-(3-(2-hydroxyphenyl)-6-methoxycinnolin-7-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 829.25 | 1H NMR (400 MHz, DMSO-d6) δ 13.35 (s, 1H), 9.03 - 8.97 (m, 1H), 8.65 (s, 1H), 8.43 (d, J = 7.6 Hz, 1H), 8.04 - 7.97 (m, 1H), 7.51 - 7.25 (m, 6H), 7.03 - 6.96 (m, 2H), 6.95 (s, 1H), 5.91 (s, 1H), 5.12 (d, J = 3.6 Hz, 1H), 4.96 - 4.88 (m, 1H), 4.37 (s, 5H), 4.29 (s, 1H), 4.10 (s, 4H), 3.99 (s, 3H), 3.75 - 3.68 (m, 1H), 3.61 (d, J = 9.7 Hz, 1H), 3.47 - 3.41 (m, 1H), 2.46 (s, 3H), 2.28 - 2.16 (m, 1H), 2.05 - 1.73 (m, 2H), 1.38 (d, J = 7.0 Hz, 3H), 1.00 - 0.93 (m, 3H), 0.87 - 0.77 (m, 3H). |
| 158 | (2S,4R)-4-hydroxy-1-((R)-2-(3-(6-(3-(2-hydroxyphenyl)-7-methoxycinnolin-6-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 829.25 | 1H NMR (400 MHz, DMSO-d6) δ 13.71 (s, 1H), 9.00 (d, J = 6.1 Hz, 1H), 8.54 (s, 1H), 8.42 (d, J = 7.7 Hz, 1H), 8.02 (dd, J = 8.4, 1.6 Hz, 1H), 7.53 (s, 1H), 7.49 - 7.42 (m, 2H), 7.40 - 7.29 (m, 3H), 7.02 - 6.94 (m, 2H), 6.54 (s, 1H), 5.85 (d, J = 44.1 Hz, 1H), 5.07 (dd, J = 42.5, 3.2 Hz, 1H), 4.97 - 4.63 (m, 1H), 4.38 (d, J = 13.1 Hz, 5H), 4.29 (s, 1H), 4.10 (s, 4H), 4.01 (s, 3H), 3.71 (dd, J = 10.6, 4.4 Hz, 1H), 3.61 (d, J = 9.7 Hz, 1H), 3.45 (dd, J = 12.5, 10.2 Hz, 1H), 2.47 (d, J = 4.5 Hz, 3H), 2.30 - 2.08 (m, 1H), 2.08 - 1.86 (m, 1H), 1.79 (ddd, J = 12.8, 8.1, 4.8 Hz, 1H), 1.46 (d, J = 6.9 Hz, 1H), 1.38 (d, J = 7.0 Hz, 2H), 0.96 (d, J = 6.5 Hz, 3H), 0.82 (dd, J = 14.6, 6.5 Hz, 3H). |
| 157 | (2S,4R)-1-((R)-2-(3-(6-(6-chloro-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 833.35 | 1H NMR (300 MHz, DMSO-d6) δ 12.16 (d, J = 3.7 Hz, 1H), 8.99 (d, J = 3.8 Hz, 1H), 8.70 (s, 1H), 8.42 (d, J = 7.6 Hz, 1H), 8.15 (s, 1H), 8.00 (d, J = 7.7 Hz, 1H), 7.44 (d, J = 8.5 Hz, 2H), 7.41 - 7.25 (m, 4H), 7.03 (d, J = 7.8 Hz, 2H), 5.87 (d, J = 33.1 Hz, 1H), 5.12 (d, J = 3.6 Hz, 1H), 4.92 (t, J = 7.1 Hz, 1H), 4.48 (s, 4H), 4.37 (t, J = 7.9 Hz, 1H), 4.29 (s, 1H), 4.12 (s, 4H), 3.61 (d, J = 9.7 Hz, 1H), 3.51 - 3.32 (m, 2H), 2.46 (d, J = 3.5 Hz, 3H), 2.24 (s, 1H), 2.03 (s, 1H), 1.85 - 1.72 (m, 1H), 1.43 (dd, J = 24.6, 7.0 Hz, 3H), 0.96 (d, J = 6.5 Hz, 3H), 0.84 - 0.75 (m, 3H). |
| 152 | (2S,4R)-1-((R)-2-(3-(6-(7-cyclopropyl-3-(2-hydroxyphenyl)cinnolin-6-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 839.3 | 1H NMR (300 MHz, DMSO-d6) δ 13.44 (s, 1H), 8.99 (d, J = 4.0 Hz, 1H), 8.56 (s, 1H), 8.41 (d, J = 7.7 Hz, 1H), 8.04 (dd, J = 8.4, 1.6 Hz, 1H), 7.84 (s, 1H), 7.52 - 7.28 (m, 5H), 7.04 - 6.93 (m, 2H), 6.60 (s, 1H), 5.93 (s, 1H), 5.12 (d, J = 3.6 Hz, 1H), 4.97 - 4.87 (m, 1H), 4.52 (s, 4H), 4.37 (t, J = 7.9 Hz, 1H), 4.32 - 4.26 (m, 1H), 4.13 (s, 4H), 3.77 - 3.66 (m, 1H), 3.61 (d, J = 9.7 Hz, 1H), 3.48 - 3.39 (m, 1H), 2.46 (d, J = 3.3 Hz, 3H), 2.31 - 1.97 (m, 1H), 1.87 - 1.72 (m, 1H), 1.51 - 1.34 (m, 3H), 1.13 - 1.04 (m, 2H), 1.00 - 0.92 (m, 5H), 0.88 - 0.76 (m, 3H). |
| 145 | (2S,4R)-4-hydroxy-1-((R)-2-(3-(6-(3-(2-hydroxyphenyl)-5-methylcinnolin-7-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 813.4 | 1H NMR (300 MHz, DMSO-d6) δ 12.92 (s, 1H), 8.99 (d, J = 4.8 Hz, 1H), 8.63 (s, 1H), 8.41 (d, J = 7.8 Hz, 1H), 8.16 (d, J = 8.2 Hz, 1H), 7.50 - 7.41 (m, 2H), 7.40 - 7.26 (m, 3H), 7.13 (s, 1H), 7.05 - 6.95 (m, 2H), 6.88 (s, 1H), 5.92 (s, 1H), 5.11 (d, J = 3.5 Hz, 1H), 4.97 - 4.88 (m, 1H), 4.37 (t, J = 8.0 Hz, 1H), 4.28 (s, 4H), 4.12 (s, 4H), 3.72 (d, J = 10.8 Hz, 1H), 3.61 (d, J = 9.7 Hz, 1H), 3.43 (d, J = 11.0 Hz, 2H), 2.70 (s, 3H), 2.47 (d, J = 3.7 Hz, 3H), 2.33 - 2.15 (m, 1H), 2.30 - 1.98 (m, 1H), 1.89 - 1.72 (m, 1H), 1.38 (d, J = 6.9 Hz, 3H), 0.96 (d, J = 6.5 Hz, 3H), 0.86 - 0.78 (m, 3H) |
| 126 | (2S,4R)-1-((R)-2-(3-(6-(6-cyclopropyl-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 839.3 | 1H NMR (300 MHz, DMSO-d6) δ 12.93 (s, 1H), 9.00 (d, J = 3.5 Hz, 1H), 8.67 (s, 1H), 8.43 (d, J = 7.6 Hz, 1H), 7.99 (d, J = 8.1 Hz, 1H), 7.62 (s, 1H), 7.53 - 7.26 (m, 5H), 7.08 - 6.95 (m, 3H), 5.93 (s, 1H), 5.13 (d, J = 3.6 Hz, 1H), 4.93 (t, J = 7.2 Hz, 1H), 4.48 (s, 4H), 4.38 (t, J = 7.9 Hz, 1H), 4.33 - 4.27 (m, 1H), 4.14 (s, 4H), 3.76 - 3.68 (m, 1H), 3.62 (d, J = 9.7 Hz, 1H), 3.49 - 3.43 (m, 1H), 2.47 (d, J = 3.4 Hz, 3H), 2.30 - 1.98 (m, 3H), 1.87 - 1.73 (m, 1H), 1.51 - 1.35 (m, 3H), 1.15 - 1.06 (m, 2H), 1.03 - 0.86 (m, 5H), 0.81 (d, J = 6.7 Hz, 3H). |
| 125 | (2S,4R)-1-((R)-2-(3-(6-(5-cyclopropyl-3-(2-hydroxyphenyl)cinnolin-7-yl)-2,6-diazaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 839.35 | 1H NMR (400 MHz, DMSO-d6) δ 12.71 (s, 1H), 9.09 - 8.76 (m, 2H), 8.42 (d, J = 7.7 Hz, 1H), 8.18 - 8.11 (m, 1H), 7.60 - 7.23 (m, 5H), 7.01 (d, J = 7.8 Hz, 2H), 6.85 (dd, J = 19.5, 2.2 Hz, 2H), 5.87 (d, J = 43.0 Hz, 1H), 5.22 - 5.02 (m, 1H), 4.99 - 4.61 (m, 1H), 4.45 - 4.21 (m, 6H), 4.11 (s, 4H), 3.75 - 3.67 (m, 1H), 3.60 (t, J = 10.6 Hz, 1H), 3.43 (d, J = 10.8 Hz, 1H), 2.66 - 2.56 (m, 1H), 2.47 (d, J = 5.0 Hz, 3H), 2.23 - 2.19 (m, 1H), 2.03 (t, J = 10.1 Hz, 1H), 1.85 - 1.74 (m, 1H), 1.42 (dd, J = 32.4, 7.0 Hz, 3H), 1.17 - 1.08 (m, 2H), 1.02 - 0.70 (m, 8H). |
| 121 | (2S,4R)-4-hydroxy-1-((R)-2-(3-(6-(3-(2-hydroxyphenyl)-6-methylcinnolin-7-yl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 813.3 | 1H NMR (300 MHz, DMSO-d6) δ 12.95 (s, 1H), 9.03-8.96 (d, J = 3.9 Hz, 1H), 8.67 (s, 1H), 8.47-8.39 (d, J = 7.7 Hz, 1H), 8.08-7.99 (d, J = 8.1 Hz, 1H), 7.71 (s, 1H), 7.53 - 7.27 (m, 5H), 7.09 - 6.95 (m, 3H), 5.93 (s, 1H), 5.15-5.11 (d, J = 3.6 Hz, 1H), 5.04-4.86 (m, 1H), 4.44-4.25 (m, 6H), 4.12 (s, 4H), 3.78 - 3.39 (m, 3H), 2.48-2.43 (d, J = 3.4 Hz, 6H), 2.31-2.13 (m, 1H), 2.08-1.95 (m,1H), 1.85-1.74 (m,1H), 1.52-1.33 (m, 3H), 1.01-0.91 (d, J = 6.4 Hz, 3H), 0.89 - 0.77 (m, 3H). |
| 274 | (2S,4R)-N-((S)-1-(4-ethynylphenyl)ethyl)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 726.25 | 1H NMR (300 MHz, DMSO-d6) δ 12.82 (s, 1H), 8.77 (s, 1H), 8.41 (d, J = 7.7 Hz, 1H), 8.00 (dd, J = 22.9, 8.4 Hz, 2H), 7.43 (d, J = 8.2 Hz, 2H), 7.30 (t, J = 7.6 Hz, 4H), 7.02 (d, J = 7.6 Hz, 3H), 5.92 (s, 1H), 5.12 (d, J = 3.4 Hz, 1H), 4.93 - 4.83 (m, 1H), 4.30 (s, 6H), 4.13 (d, J = 3.6 Hz, 5H), 3.66 (dd, J = 28.6, 9.6 Hz, 2H), 3.43 (d, J = 10.3 Hz, 1H), 2.24 (d, J = 20.1 Hz, 1H), 2.01 (s, 1H), 1.75 (s, 1H), 1.39 (dd, J = 25.0, 6.9 Hz, 3H), 0.95 (d, J = 6.7 Hz, 3H),0.80 (d, J = 6.8 Hz, 3H). |
| 273 | (2S,4R)-N-((S)-1-(4-ethynyl-2-methoxyphenyl)ethyl)-4-hydroxy-1-((R)-2-(3-(6-(3-(2-hydroxyphenyl)cinnolin-7-yl)-2,6-diazaspiro[3.3]heptan-2-yl)isoxazol-5-yl)-3-methylbutanoyl)pyrrolidine-2-carboxamide | 756.35 | 1H NMR (400 MHz, DMSO-d6) δ 12.83 (d, J = 6.0 Hz, 1H), 8.76 (s, 1H), 8.36 (d, J = 7.8 Hz, 1H), 8.03 (d, J = 7.8 Hz, 1H), 7.95 (d, J = 9.0 Hz, 1H), 7.36 - 7.24 (m, 2H), 7.19 (dd, J = 7.7, 4.3 Hz, 1H), 7.12 - 6.96 (m, 5H), 5.92 (s, 1H), 5.11 (dd, J = 10.3, 5.2 Hz, 2H), 4.42 - 4.24 (m, 6H), 4.14 (d, J = 12.1 Hz, 5H), 3.83 (d, J = 5.1 Hz, 3H), 3.69 (dd, J = 10.6, 4.4 Hz, 1H), 3.61 (d, J = 9.8 Hz, 1H), 3.42 (d, J = 9.8 Hz, 1H), 2.29 - 2.20 (m, 1H), 2.06 - 1.97 (m, 1H), 1.81 - 1.70 (m, 1H), 1.26 (d, J = 6.9 Hz, 3H), 1.00 - 0.92 (m, 3H), 0.81 (dd, J = 10.9, 6.6 Hz, 3H). |

### Preparation of (2S,4R)-1-[(2S)-2-cyclopropyl-2-(4-{6-[3-(2-hydroxyphenyl) cinnolin-7-yl]-2,6-diazaspiro [3.3] heptan-2-yl}-1,2,3-triazol-1-yl) acetyl]-4-hydroxy-N-[(1S)-1-[4-(4-methyl-1,3-thiazol-5-yl) phenyl] ethyl] pyrrolidine-2-carboxamide (Compound 210-001).

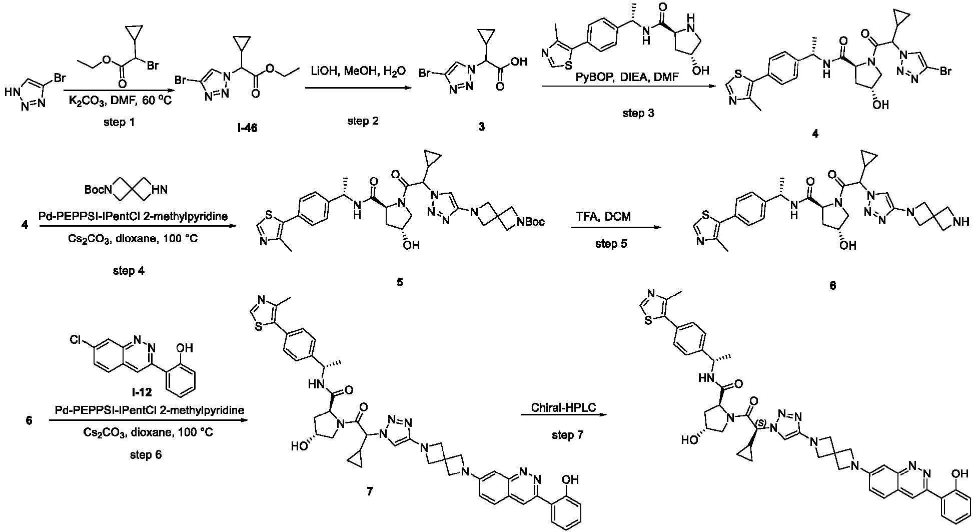

### Step 1: preparation of ethyl 2-(4-bromo-1,2,3-triazol-1-yl)-2-cyclopropylacetate (1-46)

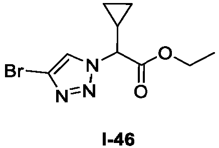

To a solution of 4-bromo-1H-1,2,3-triazole (1 g, 6.758 mmol, 1 equiv) and ethyl 2-bromo-2-cyclopropylacetate (2.80 g, 13.516 mmol, 2 equiv) in DMF (5 mL) was added K₂CO₃ (1.87 g, 13.516 mmol, 2 equiv). The resulting solution was stirred for 4 h at 60 °C. The resulting mixture was diluted with water (300 mL) and extracted with ethyl acetate (3 x 200 mL). The organic layer was dried over anhydrous sodium sulfate, filtered and concentrated to get the residue. The residue was purified by reversed-phase flash chromatography with the following conditions: column, C18 silica gel; mobile phase, MeCN in Water (0.1% TFA), 0% to 100% gradient in 30 min; detector, UV 254/220 nm. to afford **I-46** (611 mg, 32.98%) as a yellow oil. LCMS (ESI) m/z: [M+H] ⁺ = 274.

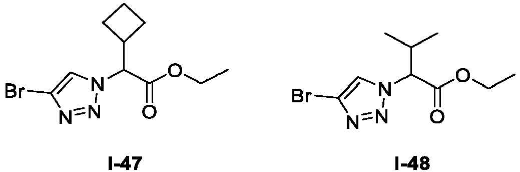

Ethyl 2-(4-bromo-1,2,3-triazol-1-yl)-2-cyclobutylacetate (**I-47**) and ethyl 2-(4-bromo-1H-1,2,3-triazol-1-yl)-3-methylbutanoate (**I-48**) were prepared using similar procedures as **I-46** from 4-bromo-1H-1,2,3-triazole.

### Step 2: preparation of (4-bromo-1,2,3-triazol-1-yl) (cyclopropyl)acetic acid (Intermediate 3)

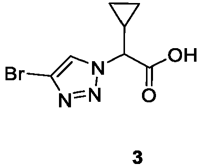

A solution of intermediate 2 (489 mg, 1.784 mmol, 1 equiv) and LiOH (213.62 mg, 8.920 mmol, 5 equiv) in MeOH (4 mL) in H2O (1 mL) was stirred for 3 h at room temperature. The resulting mixture was diluted with water (200 mL) and extracted with ethyl acetate (3 x 200 mL). The combined organic layers were dried over anhydrous sodium sulfate, filtered and concentrated to get the intermediate **3** (481 mg, crude) as a yellow solid. LCMS (ESI) m/z: [M+H] + = 245.

### Step 3: preparation of (2S,4R)-1-[2-(4-bromo-1,2,3-triazol-1-yl)-2-cyclopropylacetyl]-4-hydroxy-N-[(1S)-1-[4-(4-methyl-1,3-thiazol-5-yl) phenyl] ethyl] pyrrolidine-2-carboxamide (Intermediate 4)

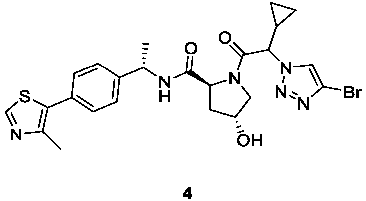

To a stirred solution of intermediate **3** (481 mg, 1.955 mmol, 1 equiv) and (2S,4R)-4-hydroxy-N-[(1S)-1-[4-(4-methyl-1,3-thiazol-5-yl) phenyl] ethyl] pyrrolidine-2-carboxamide (971.81 mg, 2.933 mmol, 1.5 equiv), HOBT (528.28 mg, 3.910 mmol, 2 equiv), EDCI (749.46 mg, 3.910 mmol, 2 equiv) in DMF (2 mL) was added DIEA (1263.24 mg, 9.775 mmol, 5 equiv). The resulting mixture was stirred for 1 h at room temperature. The reaction solution was purified by reversed-phase flash chromatography with the following conditions: column, C18 silica gel; mobile phase, MeCN in Water (0.1% NH₄HCO₃), 0% to 100% gradient in 30 min; detector, UV 254/220 nm. to afford intermediate **4** (291 mg, 26.61%) as a yellow solid. LCMS (ESI) m/z: [M+H] ⁺ = 559.

### Step 4: preparation of tert-butyl 6-(1-{1-cyclopropyl-2-[(2S,4R)-4-hydroxy-2-{[(1S)-1-[4-(4-methyl-1,3-thiazol-5-yl) phenyl] ethyl] carbamoyl} pyrrolidin-1-yl]-2-oxoethyl}-1,2,3-triazol-4-yl)-2,6-diazaspiro [3.3] heptane-2-carboxylate (Intermediate 5)

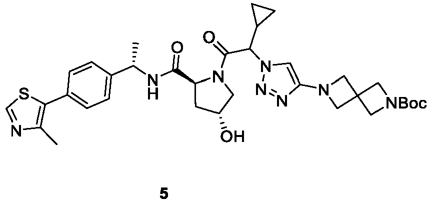

A solution of intermediate **4** (281 mg, 0.502 mmol, 1 equiv) and tert-butyl 2,6-diazaspiro [3.3] heptane-2-carboxylate (199.16 mg, 1.004 mmol, 2 equiv), Pd-PEPPSI-IPentCl 2-methylpyridine (o-picoline) (42.25 mg, 0.050 mmol, 0.1 equiv), Cs₂CO₃ (327.29 mg, 1.004 mmol, 2 equiv) in 1,4-dioxane (2 mL) was stirred for 2 h at 100 °C under nitrogen atmosphere. The resulting mixture was diluted with water (200 mL) and extracted with ethyl acetate (3 x 200 mL). The organic layer was dried over anhydrous sodium sulfate, filtered and concentrated to get the residue. The residue was purified by reversed-phase flash chromatography with the following conditions: column, C18 silica gel; mobile phase, MeCN in Water (0.1% NH₄HCO₃), 0% to 100% gradient in 30 min; detector, UV 254/220 nm. to afford Intermediate **5** (116 mg, 34.12%) as a yellow solid. LCMS (ESI) m/z: [M+H] ⁺ = 677.

### Step 5: preparation of (2S,4R)-1-[2-cyclopropyl-2-(4-{2,6-diazaspiro [3.3] heptan-2-yl}-1,2,3-triazol-1-yl)acetyl]-4-hydroxy-N-[(1S)-1-[4-(4-methyl-1,3-thiazol-5-yl)phenyl]ethylpyrrolidine-2-carboxamide (Intermediate 6)

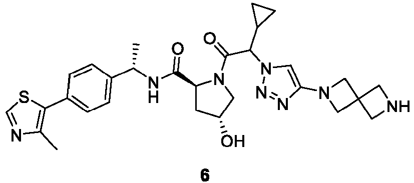

To a solution of intermediate **5** (106 mg, 0.157 mmol, 1 equiv) in DCM (1.5 mL) was added TFA (0.5 mL) was stirred for 1 h at room temperature. The resulting mixture was concentrated under reduced pressure. The residue was purified by reversed-phase flash chromatography with the following conditions: column, C18 silica gel; mobile phase, MeCN in Water (0.1% NH₄HCO₃), 0% to 100% gradient in 30 min; detector, UV 254/220 nm.to afford intermediate **6** (61 mg, 67.54%) as a yellow solid. LCMS (ESI) m/z: [M+H] ⁺ = 577.

### Step 6: preparation of (2S,4R)-1-[2-cyclopropyl-2-(4-{6-[3-(2-hydroxyphenyl) cinnolin-7-yl]-2,6-diazaspiro [3.3] heptan-2-yl}-1, 2,3-triazol-1-yl) acetyl]-4-hydroxy-N-[(1S)-1-[4-(4-methyl-1,3-thiazol-5-yl) phenyl] ethyl] pyrrolidine-2-carboxamide (Intermediate 7)

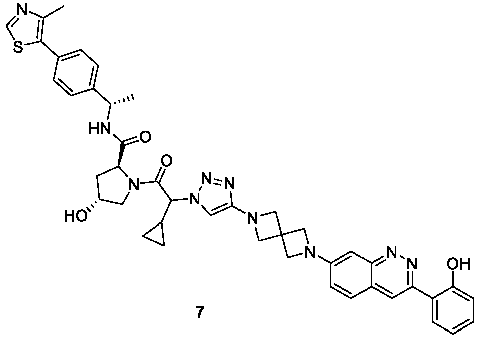

A solution of intermediate **6** (51 mg, 0.088 mmol, 1 equiv) and **I-12** (22.70 mg, 0.088 mmol, 1 equiv), Pd-PEPPSI-IPentCl 2-methylpyridine (9 mg, 0.009 mmol, 0.1 equiv), Cs₂CO₃ (57.63 mg, 0.176 mmol, 2 equiv) in 1,4-dioxane (1 mL) was stirred for 2 h at 100 °C under nitrogen atmosphere. The resulting mixture was diluted with water (200 mL) and extracted with ethyl acetate (3 x 200 mL). The combined organic layers were dried over anhydrous sodium sulfate, filtered and concentrated to get the residue. The residue was purified by reversed-phase flash chromatography with the following conditions: column, C18 silica gel; mobile phase, MeCN in Water (0.1% NH₄HCO₃), 0% to 100% gradient in 30 min; detector, UV 254/220 nm.to afford intermediate **7** (43 mg, 61.01%) as a red solid. LCMS (ESI) m/z: [M+H] ⁺ = 797.

### Step 7: preparation of (2S,4R)-1-[(2S)-2-cyclopropyl-2-(4-{6-[3-(2-hydroxyphenyl) cinnolin-7-yl]-2,6-diazaspiro [3.3] heptan-2-yl}-1,2,3-triazol-1-yl) acetyl]-4-hydroxy-N-[(1S)-1-[4-(4-methyl-1,3-thiazol-5-yl) phenyl] ethyl] pyrrolidine-2-carboxamide (Compound 210-001)

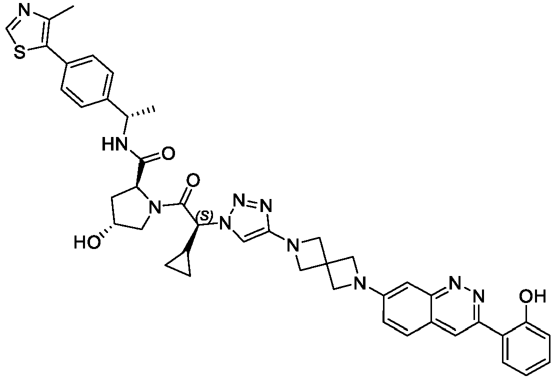

The intermediate **7** (36 mg) was purified by Chiral-Prep-HPLC with the following conditions: Column: CHIRAL ART Cellulose-SB, 2*25 cm, 5 µm; Mobile Phase A: MtBE(10mM NH₃-MeOH), Mobile Phase B: MeOH; Flow rate: 20 mL/min; Gradient: isocratic 50; Wave Length: 272/210 nm; RT1 (min): 6.75; RT2(min): 10; Sample Solvent: MeOH; Injection Volume: 0.7 mL; Number Of Runs: 4 to afford **210-001** (second peak) (9.5 mg, 26.39%) as a yellow solid. 1H NMR (300 MHz, DMSO-d6) δ 12.83 (s, 1H), 8.99 (s, 1H), 8.76 (s, 1H), 8.42 (d, J = 7.6 Hz, 1H), 8.11 - 7.90 (m, 2H), 7.55 (s, 1H), 7.49 - 7.27 (m, 6H), 7.01 (d, J = 8.4 Hz, 3H), 5.16 (s, 1H), 5.04 (d, J = 8.7 Hz, 1H), 4.93 (s, 1H), 4.35 (d, J = 27.2 Hz, 6H), 4.05 (s, 4H), 3.65 (s, 1H), 3.52 (s, 1H), 2.46 (s, 3H), 2.04 (s, 1H), 1.80 (s, 1H), 1.53 (s, 1H), 1.38 (d, J = 7.1 Hz, 3H), 0.73 - 0.44 (m, 4H). LCMS (ESI) m/z: [M+H] ⁺ = 797.30.

The compounds in Table 9 were prepared using procedures similar to those used above for the preparation of **Compound 210-001.**

**Table 9.**

| **No.** | **Name** | **LCMS (ESI) m/z** | **¹H NMR** |
|---|---|---|---|
| 266 | (2S,4R)-4-hydroxy-1-(2-(4-(3-(1-(3-(2-hydroxyphenyl)cinnolin-7-yl)piperidin-4-yl)pyrrolidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 855.4 | 1H NMR (300 MHz, DMSO-d6) δ 12.87 (s, 1H), 9.03 - 8.94 (m, 1H), 8.77 (s, 1H), 8.08 - 8.02 (m, 1H), 7.99 - 7.83 (m, 2H), 7.53 - 7.12 (m, 7H), 7.01 (dd, J = 7.9, 6.6 Hz, 2H), 5.20 - 5.12 (m, 1H), 5.08 - 4.81 (m, 2H), 4.49 - 4.26 (m, 2H), 4.12 (d, J = 12.8 Hz, 2H), 3.72 - 3.45 (m, 2H), 3.22 (d, J = 20.1 Hz, 2H), 2.92 (dd, J = 18.1, 9.5 Hz, 3H), 2.48 - 2.43 (m, 3H), 2.07 (s, 2H), 1.95 - 1.75 (m, 3H), 1.73 - 1.29 (m, 8H), 1.24 (s, 1H), 1.01 (t, J = 7.3 Hz, 3H), 0.68 (t, J = 6.2 Hz, 3H). |
| 265 | (2S,4R)-4-hydroxy-1-(2-(4-(6-(3-(2-hydroxyphenyl)cinnolin-7-yl)-1,6-diazaspiro[3.3]heptan-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 799.25 | 1H NMR (400 MHz, Methanol-d4) δ 8.86 (s, 1H), 8.68 - 7.88 (m, 1H), 8.00 - 7.79 (m, 2H), 7.50 (s, 1H), 7.46 - 7.38 (m, 1H), 7.37 - 7.34 (m, 2H), 7.33 - 7.20 (m, 3H), 7.03 - 6.89 (m, 3H), 5.16 (dd, J = 19.2, 9.8 Hz, 1H), 4.96 (q, J = 7.0 Hz, 1H), 4.61 - 4.45 (m, 3H), 4.37 (s, 1H), 4.28 - 4.09 (m, 2H), 3.92 - 3.81 (m, 2H), 3.79 - 3.61 (m, 1H), 3.43 (dd, J = 11.1, 4.0 Hz, 1H), 2.70 (dt, J = 14.2, 6.6 Hz, 2H), 2.54 - 2.42 (m, 4H), 2.34 - 2.09 (m, 1H), 2.08 - 1.80 (m, 1H), 1.57 - 1.32 (m, 3H), 1.04 (dd, J = 6.7, 3.5 Hz, 3H), 0.74 (dd, J = 6.7, 1.9 Hz, 3H). |
| 264 | (2S,4R)-4-hydroxy-1-(2-(4-(1-(3-(2-hydroxyphenyl)cinnolin-7-yl)-1,6-diazaspiro[3.4]octan-6-yl)-1H-1,2,3-triazol-1-yl)-3-methylbutanoyl)-N-((S)-1-(4-(4-methylthiazol-5-yl)phenyl)ethyl)pyrrolidine-2-carboxamide | 813.35 | 1H NMR (300 MHz, DMSO-d6) δ 12.88 (s, 1H), 9.01 - 8.89 (m, 1H), 8.73 (d, J = 5.5 Hz, 1H), 8.54 - 8.42 (m, 1H), 8.02 (d, J = 7.9 Hz, 1H), 7.91 (t, J = 9.6 Hz, 1H), 7.59 - 7.20 (m, 8H), 7.13 - 6.95 (m, 3H), 5.22 - 5.09 (m, 2H), 4.91 (s, 1H), 4.50 - 4.29 (m, 2H), 4.05 - 3.90 (m, 3H), 3.64 (d, J = 11.9 Hz, 2H), 3.44 (s, 1H), 3.17 (t, J = 7.7 Hz, 2H), 2.48 - 2.33 (m, 5H), 2.07 (s, 1H), 1.77 (d, J = 13.4 Hz, 1H), 1.44 - 1.24 (m, 3H), 1.00 (dt, J = 15.1, 7.2 Hz, 3H), 0.70 (t, J = 5.8 Hz, 3H). |
| 263 | (2S,4R)-4-hydroxy-1-(2-(4-(7-(3-(2-hydroxyphenyl)cinnolin-7-yl)-3,7-diazabicyclo[4.2.0]octan-3-yl)-1H-1,2,3-triazol-1-yl)-3-methylbutanoyl)-N-((S)-1-(4-(4-methylthiazol-5-yl)phenyl)ethyl)pyrrolidine-2-carboxamide | 813.2 | 1H NMR (400 MHz, Methanol-d4) δ 8.91 - 8.84 (m, 1H), 8.61 - 8.50 (m, 1H), 7.98 - 7.79 (m, 2H), 7.50 - 7.34 (m, 2H), 7.37 - 7.24 (m, 2H), 7.31 - 7.26 (m, 2H), 7.22 (d, J = 8.4 Hz, 1H), 7.03 - 6.95 (m, 2H), 6.98 - 6.89 (m, 1H), 5.20 - 5.00 (m, 2H), 4.71 (s, 1H), 4.64 - 4.45 (m, 1H), 4.48 - 4.40 (m, 1H), 4.15 - 4.00 (m, 1H), 4.00 - 3.97 (m, 1H), 3.99 - 3.84 (m, 2H), 3.83 - 3.72 (m, 2H), 3.69 - 3.48 (m, 1H),3.40 - 3.31 (m, 1H),3.20 - 3.08 (m, 1H), 2.64 - 2.51 (m, 1H), 2.51 - 2.42 (m, 3H), 2.40 (s, 1H), 2.31 - 2.14 (m, 2H), 2.05 - 1.88 (m, 1H), 1.55 - 1.45 (m, 2H), 1.34 (d, J = 7.0 Hz, 1H), 1.10 (t, J = 6.4 Hz, 3H), 0.82 - 0.68 (m, 3H). |
| 262 | (2S,4R)-4-hydroxy-1-(2-(4-(1-(3-(2-hydroxyphenyl)cinnolin-7-yl)-1,7-diazaspiro[4.4]nonan-7-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.25 | 1H NMR (400 MHz, DMSO-d6) δ 8.97 (d, J = 3.9 Hz, 1H), 8.73 (s, 1H), 8.66 (dd, J = 12.3, 7.7 Hz, 1H), 8.02 (d, J = 8.1 Hz, 1H), 7.95 (d, J = 9.3 Hz, 1H), 7.70 (d, J = 9.4 Hz, 1H), 7.61 - 7.34 (m, 6H), 7.30 (t, J = 7.6 Hz, 1H), 6.99 (d, J = 7.7 Hz, 2H), 5.20 (d, J = 10.4 Hz, 1H), 4.92 (q, J = 7.8 Hz, 1H), 4.39 (d, J = 8.5 Hz, 1H), 4.26 (s, 1H), 4.01 (d, J = 10.8 Hz, 1H), 3.85 (d, J = 6.0 Hz, 2H), 3.74 - 3.59 (m, 3H), 2.95 (t, J = 10.2 Hz, 1H), 2.74 - 2.62 (m, 1H), 2.44 (d, J = 12.7 Hz, 3H), 2.35 - 2.32 (m, 1H), 2.20 (d, J = 21.5 Hz, 2H), 2.05 (d, J = 18.2 Hz, 3H), 1.78 (d, J = 19.3 Hz, 3H), 1.38 (dd, J = 10.6, 7.0 Hz, 3H), 1.24 (s, 1H), 0.96 (d, J = 6.6 Hz, 3H), 0.85 (d, J = 6.4 Hz, 1H), 0.72 (t, J = 6.2 Hz, 3H). |
| 261 | (2S,4R)-4-hydroxy-1-(2-(4-(3-((4-(3-(2-hydroxyphenyl)cinnolin-7-yl)piperazin-1-yl)methyl)piperidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 884.4 | 1H NMR (400 MHz, DMSO-d6) δ 12.79 (s, 1H), 9.10 - 8.89 (m, 1H), 8.80 - 8.73 (m, 1H), 8.54 - 8.20 (m, 1H), 8.04 (td, J = 5.5, 2.8 Hz, 1H), 7.91 - 7.99 (m, 1H), 7.86 (d, J = 9.1 Hz, 1H), 7.55 - 7.21 (m, 7H), 7.01 (t, J = 8.7 Hz, 2H), 5.23 - 4.96 (m, 2H), 4.91 - 4.62 (m, 1H), 4.44 (t, J = 7.9 Hz, 1H), 4.28 (s, 1H), 3.68 (t, J = 12.4 Hz, 2H), 3.54 - 3.40 (m, 5H), 2.69 - 2.55 (m, 3H), 2.43 (d, J = 8.7 Hz, 3H), 2.38 - 2.25 (m, 2H), 2.20 (dd, J = 12.0, 6.0 Hz, 1H), 2.12 - 1.96 (m, 1H), 1.93 - 1.85 (m, 1H), 1.78 (td, J = 11.9, 11.4, 6.5 Hz, 2H), 1.68 (dq, J = 9.9, 3.3 Hz, 1H), 1.56 (t, J = 12.1 Hz, 1H), 1.47 (d, J = 7.0 Hz, 1H), 1.39 - 1.31 (m, 3H), 1.24 (d, J = 7.3 Hz, 3H), 0.99 (d, J = 6.7 Hz, 3H), 0.79 - 0.87 (m, 1H), 0.71 - 0.56 (m, 3H). |
| 260 | (2S,4R)-4-hydroxy-1-(2-(4-(4-((1-(3-(2-hydroxyphenyl)cinnolin-7-yl)piperidin-3-yl)methyl)piperazin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 884.4 | 1H NMR (400 MHz, DMSO-d6) δ 12.90 (s, 1H), 9.08 - 8.92 (m, 1H), 8.74 (d, J = 3.0 Hz, 1H), 8.39 (dd, J = 103.5, 7.8 Hz, 1H), 8.04 (d, J = 7.9 Hz, 1H), 7.93 (dd, J = 9.4, 5.0 Hz, 1H), 7.85-7.78 (m, 1H), 7.69 - 7.16 (m, 7H), 7.00 (t, J = 8.2 Hz, 2H), 5.22 - 4.96 (m, 2H), 4.95 - 4.67 (m, 1H), 4.48 - 4.21 (m, 2H), 4.05 - 3.82 (m, 2H), 3.65 (dd, J = 18.7, 10.6 Hz, 1H), 3.43 (dd, J = 11.6, 4.1 Hz, 1H), 3.12 (q, J = 13.4, 10.7 Hz, 4H), 2.94-2.83 (m, 1H), 2.62 - 2.54 (m, 1H), 2.48-2.39 (m, 6H), 2.35 - 2.16 (m, 2H), 2.12 - 2.03 (m, 1H), 2.01-1.90 (m, 2H), 1.88 - 1.73 (m, 3H), 1.61 (p, J = 10.6, 10.0 Hz, 1H), 1.53 - 1.43 (m, 1H), 1.39 - 1.29 (m, 3H), 1.02 (dd, J = 14.0, 6.6 Hz, 3H), 0.85 (dd, J = 9.0, 6.7 Hz, 1H), 0.74 - 0.60 (m, 3H). |
| 259 | (2S,4R)-4-hydroxy-1-(2-(4-(4-((4-(3-(2-hydroxyphenyl)cinnolin-7-yl)piperazin-1-yl)methyl)piperidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 884.45 | 1H NMR (400 MHz, DMSO-d6) δ 12.78 (d, J = 2.3 Hz, 1H), 9.18 - 8.95 (m, 1H), 8.78 (s, 1H), 8.64 - 8.15 (m, 1H), 8.11 - 8.00 (m, 1H), 8.00 - 7.92 (m, 1H), 7.91 - 7.80 (m, 1H), 7.59 - 7.22 (m, 7H), 7.12 - 6.97 (m, 2H), 5.29 - 5.11 (m, 1H), 5.09 - 4.53 (m, 2H), 4.49 - 4.35 (m, 1H), 4.35 - 4.18 (m, 1H), 3.85 - 3.58 (m, 3H), 3.56 - 3.38 (m, 5H), 2.68 - 2.53 (m, 6H), 2.48 - 2.43 (m, 3H), 2.31 - 2.17 (m, 2H), 2.13 - 2.01 (m, 1H), 1.88 - 1.71 (m, 3H), 1.59 - 1.43 (m, 1H), 1.43 - 1.31 (m, 3H), 1.30 - 1.25 (m, 1H), 1.20 - 1.15 (m, 1H), 1.10 - 0.94 (m, 3H), 0.91 - 0.74 (m, 1H), 0.72 - 0.59 (m, 3H). |
| 268 | (2S,4R)-4-hydroxy-1-(2-(4-(3-(3-(2-hydroxyphenyl)cinnolin-7-yl)-3,8-diazabicyclo[3.2.1]octan-8-yl)-1H-1,2,3-triazol-1-yl)-3-methylbutanoyl)-N-((S)-1-(4-(4-methylthiazol-5-yl)phenyl)ethyl)pyrrolidine-2-carboxamide | 813.3 | 1H NMR (400 MHz, DMSO-d6) δ 12.80 (s, 1H), 9.07 - 8.90 (m, 1H), 8.77 (d, J = 7.3 Hz, 1H), 8.51 (d, J = 7.7 Hz, 1H), 8.04 (t, J = 7.3 Hz, 1H), 7.94 (dd, J = 13.2, 9.4 Hz, 1H), 7.88 - 7.76 (m, 1H), 7.51 - 7.27 (m, 6H), 7.21 (d, J = 8.1 Hz, 1H), 7.01 (d, J = 8.6 Hz, 2H), 5.18 (d, J = 9.4 Hz, 1H), 5.12 (d, J = 10.3 Hz, 1H), 4.90 - 4.24 (m, 4H), 3.85 - 3.75 (m, 3H), 3.72 (d, J = 12.6 Hz, 1H), 3.68 - 3.59 (m, 1H), 3.23 (d, J = 11.5 Hz, 2H), 2.49 - 2.39 (m, 3H), 2.39 (s, 1H), 2.06 - 2.01 (m, 1H), 1.90 - 1.81 (m, 5H), 1.47 - 1.14 (m, 3H), 1.02 (dd, J = 17.7, 6.7 Hz, 3H), 0.70 - 0.58 (m, 3H). |
| 251 | (2S,4R)-4-hydroxy-1-(2-(4-(9-(3-(2-hydroxyphenyl)cinnolin-7-yl)-2,9-diazaspiro[5.5]undecan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 855.5 | 1H NMR (400 MHz, DMSO-d6) δ 12.88 (brs, 1H), 9.01 - 8.96 (m, 1H), 8.79 - 8.74 (m, 1H), 8.52 - 8.21 (m, 1H), 8.07 - 8.01 (m, 1H), 7.96 - 7.82 (m, 2H), 7.62 - 7.22 (m, 7H), 7.04 - 6.97 (m, 2H), 5.15 (d, J = 9.4 Hz, 1H), 5.06 (d, J = 10.5 Hz, 1H), 4.93 - 4.68 (m, 1H), 4.47 - 4.24 (m, 2H), 3.69 - 3.40 (m, 6H), 3.15 - 2.98 (m, 4H), 2.48 - 2.42 (m, 3H), 2.12 - 1.86 (m, 1H), 1.83 - 1.42 (m, 10H) 1.33 (dd, J = 20.8, 7.0 Hz, 3H), 1.05 - 0.96 (m, 3H), 0.70 - 0.61 (m, 3H). |
| 250 | (2S,4R)-4-hydroxy-1-(2-(4-(3-(3-(2-hydroxyphenyl)cinnolin-7-yl)-3,7-diazabicyclo[4.2.0]octan-7-yl)-1H-1,2,3-triazol-1-yl)-3-methylbutanoyl)-N-((S)-1-(4-(4-methylthiazol-5-yl)phenyl)ethyl)pyrrolidine-2-carboxamide | 813.4 | 1H NMR (300 MHz, DMSO-d6) δ 13.03 (s, 1H), 8.96 (d, J = 12.9 Hz, 1H), 8.75 (s, 1H), 8.49 (t, J = 6.0 Hz, 1H), 8.03 (d, J = 7.9 Hz, 1H), 7.94 (t, J = 8.2 Hz, 1H), 7.83 (d, J = 10.0 Hz, 1H), 7.49 - 7.30 (m, 7H), 7.01 (d, J = 7.8 Hz, 2H), 5.16 (d, J = 4.4 Hz, 1H), 5.08 (d, J = 10.5 Hz, 1H), 4.97 - 4.86 (m, 1H), 4.31 (s, 3H), 4.25 (s, 2H), 3.82 - 3.67 (m, 6H), 3.02 (s, 1H), 2.46 (s, 3H), 2.44 (d, J = 11.4 Hz, 3H), 2.20 (d, J = 14.1 Hz, 1H), 1.51 - 1.44 (m, 1H), 1.31 (d, J = 12.7 Hz, 3H), 1.11 - 1.07 (m, 3H), 0.81 (dd, J = 7.3, 4.3 Hz, 2H), 0.68 (s, 1H). |
| 249 | (2S,4R)-4-hydroxy-1-(2-(4-(3-(3-(2-hydroxyphenyl)cinnolin-7-yl)-3,8-diazabicyclo[4.2.0]octan-8-yl)-1H-1,2,3-triazol-1-yl)-3-methylbutanoyl)-N-((S)-1-(4-(4-methylthiazol-5-yl)phenyl)ethyl)pyrrolidine-2-carboxamide | 835.3 | 1H NMR (400 MHz, Methanol-d4) δ 8.89 - 8.68 (m, 1H), 8.58 - 8.40 (m, 1H), 7.97 - 7.61 (m, 3H), 7.59 - 7.10 (m, 7H), 7.02 - 6.88 (m, 2H), 5.14 - 4.96 (m, 1H), 4.78 - 4.22 (m, 4H), 4.20 - 4.00 (m, 2H), 3.95 - 3.70 (m, 3H), 3.70 - 3.45 (m, 3H), 3.01 - 2.83 (m, 1H), 2.59 - 2.51 (m, 1H), 2.50 - 2.41 (m, 2H), 2.42 - 2.22 (m, 3H), 2.17 - 2.01 (m, 1H), 1.99 - 1.87 (m, 1H), 1.61 - 1.34 (m, 2H), 1.13 (dd, J = 14.9, 7.0 Hz, 1H), 1.08 - 0.87 (m, 3H), 0.76 - 0.53 (m, 3H). |
| 248 | (2S,4R)-4-hydroxy-1-(2-(4-(1-(3-(2-hydroxyphenyl)cinnolin-7-yl)octahydro-6H-pyrrolo[2,3-c]pyridin-6-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.25 | ¹H NMR (300 MHz, DMSO-d6) δ 13.48 - 12.76 (s, 1H), 9.04 - 8.93 (m, 1H), 8.73 (s, 1H), 8.57 - 8.21 (m, 1H), 8.08 - 7.99 (m, 1H), 7.98 - 7.89 (m, 1H), 7.68 - 7.21 (m, 7H), 7.17 - 7.07 (m, 1H), 7.04 - 6.93 (m, 2H), 5.27 - 4.77 (m, 3H), 4.49 - 4.10 (m, 3H), 3.86 - 3.57 (m, 5H), 3.56 - 3.38 (m, 2H), 3.12 - 2.95 (m, 1H), 2.76 - 2.66 (m, 1H), 2.47 - 2.44 (m, 3H), 2.44 - 2.40 (m, 1H), 2.33 - 2.17 (m, 2H), 2.16 - 1.97 (m, 2H), 1.87 - 1.71 (m, 1H), 1.67 - 1.45 (m, 1H), 1.42 - 1.27 (m, 3H), 1.10 - 0.95 (m, 3H), 0.75 - 0.60 ( m, 3H). |
| 247 | (2S,4R)-4-hydroxy-1-(2-(4-(4-((1-(3-(2-hydroxyphenyl)cinnolin-7-yl)piperidin-4-yl)methyl)piperazin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 884.4 | 1H NMR (400 MHz, DMSO-d6) δ 12.87 (s, 1H), 9.09 - 8.95 (m, 1H), 8.76 (s, 1H), 8.57 - 8.20 (m, 1H), 8.04 (d, J = 7.8 Hz, 1H), 7.99 - 7.79 (m, 2H), 7.60 - 7.22 (m, 7H), 7.07 - 6.91 (m, 2H), 5.21 - 4.72 (m, 3H), 4.50 - 4.21 (m, 2H), 4.12 - 3.98 (m, 2H), 3.80 - 3.62 (m, 1H), 3.49 - 3.38 (m, 1H), 3.09 (s, 4H), 3.01 - 2.87 (m, 2H), 2.48 - 2.40 (m, 5H), 2.28 - 2.02 (m, 3H), 1.96 - 1.71 (m, 4H), 1.54 - 1.33 (m, 4H), 1.30 - 1.19 (m, 3H), 1.07 - 0.96 (m, 3H), 0.88 - 0.82 (m, 1H), 0.72 - 0.57 (m, 3H). |
| 235 | (2S,4R)-4-hydroxy-1-(2-(4-(3-(1-(3-(2-hydroxyphenyl)cinnolin-7-yl)azetidin-3-yl)pyrrolidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.35 | 1H NMR (400 MHz, Methanol-d4) δ 8.90 - 8.82 (m, 1H), 8.57 (s, 1H), 7.94 (d, J = 7.8 Hz, 1H), 7.85 (d, J = 8.5 Hz, 1H), 7.60 - 7.49 (m, 1H), 7.49 - 7.35 (m, 3H), 7.35 - 7.07 (m, 3H), 7.03 - 6.95 (m, 2H), 6.88 - 6.82 (m, 1H), 5.16 - 5.07 (m, 1H), 5.07 - 4.95 (m, 1H), 4.69 - 4.43 (m, 1H), 4.42 - 4.33 (m, 1H), 4.30 - 4.04 (m, 2H), 3.98 - 3.71 (m, 4H), 3.69 - 3.37 (m, 2H), 3.20 - 3.00 (m, 1H), 2.98 - 2.83 (m, 1H), 2.71 (s, 1H), 2.58 - 2.54 (m, 1H), 2.53 - 2.44 (m, 3H), 2.44 - 2.40 (m, 1H), 2.24 - 2.18 (m, 1H), 2.11 - 1.91 (m, 1H), 1.83 - 1.57 (m, 1H), 1.58 - 1.47 (m, 2H), 1.41 (t, J = 7.4 Hz, 1H), 1.32 - 1.26 (m, 1H), 1.14 - 0.97 (m, 3H), 0.83 - 0.62 (m, 3H). |
| 234 | (2S,4R)-4-hydroxy-1-(2-(4-(1-(3-(2-hydroxyphenyl)cinnolin-7-yl)-1,7-diazaspiro[3.5]nonan-7-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.45 | 1H NMR (300 MHz, DMSO-d6) δ 12.94 (brs, 1H), 8.97 (d, J = 8.9 Hz, 1H), 8.73 (d, J = 6.2 Hz, 1H), 8.56 - 8.21 (m, 1H), 8.06 - 7.97 (m, 1H), 7.93 - 7.79 (m, 1H), 7.64 - 7.50 (m, 1H), 7.49 - 7.24 (m, 6H), 7.05 - 6.94 (m, 3H), 5.24 - 4.70 (m, 2H), 4.49 - 4.38 (m, 1H), 4.35 - 4.29 (m, 1H), 3.96 (d, J = 7.5 Hz, 2H), 3.88 - 3.58 (m, 4H),3.56 - 3.37(m, 2H), 2.81 - 2.64 (m, 2H), 2.44 (d, J = 11.5 Hz, 3H), 2.38 - 2.25 (m, 3H), 2.26 - 1.69 (m, 4H), 1.42 - 1.31 (m, 3H), 1.09 - 0.97 (m, 3H), 0.74 - 0.66 (m, 3H). |
| 233 | (2S,4R)-4-hydroxy-1-(2-(4-(8-(3-(2-hydroxyphenyl)cinnolin-7-yl)-2,8-diazaspiro[4.5]decan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 841.5 | 1H NMR (400 MHz, DMSO-d6) δ 12.84 (s, 1H), 9.07 - 8.95 (m, 1H), 8.76 (s, 1H), 8.52 - 8.19 (m, 1H), 8.08 - 7.83 (m, 3H), 7.55 - 7.26 (m, 7H), 7.05 - 6.97 (m, 2H), 5.20 - 4.65 (m, 3H), 4.48 - 4.23 (m, 2H), 3.80 - 3.74 (m, 1H), 3.71 - 3.42 (m, 5H), 3.21 - 3.12 (m, 4H), 2.49 - 2.41 (m, 3H), 2.10 - 2.01 (m, 1H), 1.93 - 1.67 (m, 8H), 1.36 (dd, J = 14.3, 7.0 Hz, 3H), 1.05 - 0.97 (m, 3H), 0.72 - 0.60 (m, 3H). |
| 214 | (2S,4R)-4-hydroxy-1-((2S)-2-(4-(8-(3-(2-hydroxyphenyl)cinnolin-6-yl)octahydro-2H-pyrazino[1,2-a]pyrazin-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 842.35 | 1H NMR (400 MHz, DMSO-d6) δ 13.02 (s, 1H), 8.99 (s, 1H), 8.57 (d, J = 2.4 Hz, 1H), 8.51 (d, J = 7.7 Hz, 1H), 8.23 (d, J = 9.5 Hz, 1H), 8.10 - 8.00 (m, 1H), 7.93 - 7.85 (m, 1H), 7.56 (d, J = 3.1 Hz, 1H), 7.49 - 7.41 (m, 2H), 7.42 - 7.30 (m, 3H), 7.16 (t, J = 2.6 Hz, 1H), 7.01 (dd, J = 8.2, 7.0 Hz, 2H), 5.25 - 5.05 (m, 2H), 5.02 - 4.88 (m, 1H), 4.40 (t, J = 8.1 Hz, 1H), 4.33 (s, 1H), 4.07 (d, J = 12.7 Hz, 2H), 3.86 - 3.54 (m, 4H), 3.19 - 3.05 (m, 1H), 2.99 - 2.84 (m, 2H), 2.86 - 2.69 (m, 2H), 2.46 (s, 4H), 2.44 - 2.19 (m, 4H), 2.13 - 1.94 (m, 1H), 1.85 - 1.76 (m, 1H), 1.57 - 1.35 (m, 3H), 1.04 (d, J = 6.5 Hz, 3H), 0.68 (d, J = 6.5 Hz, 3H). |
| 213 | (2S,4R)-4-hydroxy-1-(2-(4-((3aR,6aS)-5-(3-(2-hydroxyphenyl)cinnolin-7-yl)hexahydropyrrolo[3,4-c]pyrrol-2(1H)-yl)-1H-1,2,3-triazol-1-yl)-3-methylbutanoyl)-N-((S)-1-(4-(4-methylthiazol-5-yl)phenyl)ethyl)pyrrolidine-2-carboxamide | 813.6 | NA |
| 198 | (2S,4R)-4-hydroxy-1-((S)-2-(4-((S)-8-(3-(2-hydroxyphenyl)cinnolin-7-yl)octahydro-2H-pyrazino[1,2-a]pyrazin-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 842.4 | 1H NMR (400 MHz, DMSO-d6) δ 12.73 (s, 1H), 8.99 (s, 1H), 8.78 (s, 1H), 8.51 (d, J = 7.7 Hz, 1H), 8.05 (dd, J = 8.2, 1.6 Hz, 1H), 7.98 (d, J = 9.4 Hz, 1H), 7.90 - 7.83 (m, 1H), 7.55 (d, J = 4.8 Hz, 2H), 7.50 - 7.41 (m, 2H), 7.41 - 7.35 (m, 2H), 7.33 - 7.29 (m, 1H), 7.02 (t, J = 7.6 Hz, 2H), 5.17 (d, J = 3.7 Hz, 1H), 5.13 - 5.03 (m, 1H), 4.99 - 4.90 (m, 1H), 4.61 - 4.39 (m, 1H), 4.32 (s, 1H), 4.06 (d, J = 11.9 Hz, 2H), 3.80 - 3.57 (m, 4H), 3.06 (t, J = 11.6 Hz, 1H), 2.93 (dd, J = 28.1, 11.1 Hz, 2H), 2.83 - 2.65 (m, 2H), 2.46 (s, 3H), 2.43 - 2.24 (m, 5H), 2.08 (q, J = 10.9, 9.5 Hz, 1H), 1.85 - 1.76 (m, 1H), 1.39 (d, J = 7.0 Hz, 3H), 1.04 (d, J = 6.5 Hz, 3H), 0.68 (d, J = 6.6 Hz, 3H). |
| 197 | (2S,4R)-4-hydroxy-1-(2-(4-(2-(3-(2-hydroxyphenyl)cinnolin-7-yl)-2,7-diazaspiro[3.5]nonan-7-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.45 | 1H NMR (400 MHz, Methanol-d4) δ 8.87 (d, J = 3.7 Hz, 1H), 8.60 (d, J = 6.4 Hz, 1H), 7.99 - 7.93 (m, 1H), 7.89 (dd, J = 9.1, 4.0 Hz, 1H), 7.55 - 7.21 (m, 7H), 7.09 - 6.92 (m, 3H), 5.23 - 4.94 (m, 2H), 4.63 - 4.40 (m, 2H), 3.91 (d, J = 15.4 Hz, 4H), 3.78 (dd, J = 20.8, 10.6 Hz, 1H), 3.60 - 3.45 (m, 1H), 3.23 (d, J = 5.6 Hz, 4H), 2.64 - 2.49 (m, 1H), 2.48 (d, J = 4.0 Hz, 3H), 2.18 (ddd, J = 20.4, 13.8, 7.4 Hz, 2H), 2.07 - 1.90 (m, 4H), 1.51 (dd, J = 14.2, 7.0 Hz, 3H), 1.12 (t, J = 6.8 Hz, 3H), 0.78 (d, J = 6.6 Hz, 3H). |
| 189 | (2S,4R)-4-hydroxy-1-((S)-2-(4-(8-(3-(2-hydroxyphenyl)cinnolin-6-yl)-5-oxa-2,8-diazaspiro[3.5]nonan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 829.3 | 1H NMR (300 MHz, DMSO-d6) δ 12.90 (s, 1H), 8.99 (s, 1H), 8.63 (d, J = 0.9 Hz, 1H), 8.50 (d, J = 7.6 Hz, 1H), 8.26 (d, J = 9.6 Hz, 1H), 8.09 - 8.00 (m, 1H), 7.95 (dd, J = 9.6, 2.6 Hz, 1H), 7.53 - 7.42 (m, 3H), 7.42 - 7.30 (m, 3H), 7.26 (d, J = 2.6 Hz, 1H), 7.07 - 6.96 (m, 2H), 5.18 (d, J = 3.5 Hz, 1H), 5.11 (d, J = 10.4 Hz, 1H), 4.98 - 4.87 (m, 1H), 4.40 (t, J = 8.1 Hz, 1H), 4.33 (s, 1H), 3.96 - 3.82 (m, 4H), 3.81 - 3.60 (m, 6H), 3.48 (s, 2H), 2.46 (s, 3H), 2.07 (q, J = 8.8, 7.8 Hz, 1H), 1.86 - 1.72 (m, 1H), 1.38 (d, J = 7.0 Hz, 3H), 1.04 (d, J = 6.5 Hz, 3H), 0.68 (d, J = 6.5 Hz, 3H). |
| 270 | (2S,4R)-4-hydroxy-1-(2-(4-(3-((4-(3-(2-hydroxyphenyl)cinnolin-6-yl)piperazin-1-yl)methyl)piperidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 884.4 | 1H NMR (400 MHz, DMSO-d6) δ 13.13 (s, 1H), 8.98 (d, J = 8.0 Hz, 1H), 8.66 - 8.45 (m, 1H), 8.36 - 8.16 (m, 2H), 8.04 (d, J = 7.4 Hz, 1H), 7.84 (t, J = 8.2 Hz, 1H), 7.63 - 7.19 (m, 6H), 7.14 - 6.89 (m, 3H), 5.24 - 5.03 (m, 1H), 5.03 - 4.67 (m, 2H), 4.43 (dt, J = 20.6, 7.9 Hz, 1H), 4.36 - 4.24 (m, 1H), 3.81 (d, J = 14.9 Hz, 2H), 3.77 - 3.61 (m, 8H), 2.68 - 2.56 (m, 3H), 2.49 (d, J = 20.3 Hz, 4H), 2.38 - 2.17 (m, 3H), 2.09 (ddd, J = 14.6, 10.7, 7.1 Hz, 1H), 1.93 (d, J = 17.3 Hz, 1H), 1.83 - 1.65 (m, 3H), 1.64 - 1.46 (m, 1H), 1.43 - 1.27 (m, 3H), 1.09 - 0.80 (m, 4H), 0.79 - 0.60 (m, 3H). |
| 269 | (2S,4R)-4-hydroxy-1-(2-(4-(3-(1-(3-(2-hydroxyphenyl)cinnolin-6-yl)azetidin-3-yl)pyrrolidin-1-yl)-1H-1,2,3-triazol-1-yl)-3-methylbutanoyl)-N-((S)-1-(4-(4-methylthiazol-5-yl)phenyl)ethyl)pyrrolidine-2-carboxamide | 827.3 | 1H NMR (400 MHz, DMSO-d6) δ 13.41 (s, 1H), 9.02 - 8.95 (m, 1H), 8.54 - 8.49 (m, 1H), 8.24 - 8.14 (m, 1H), 8.04 (d, J = 8.0 Hz, 1H), 7.53 - 7.14 (m, 8H), 7.03 - 6.95 (m, 2H), 6.56 - 6.48 (m, 1H), 5.60 - 5.02 (m, 2H), 4.99 - 4.65 (m, 1H), 4.58 - 4.08 (m, 4H), 3.98 - 3.56 (m, 4H), 3.22 - 3.08 (m, 2H), 3.00 - 2.79 (m, 2H), 2.67 - 2.62 (m, 1H), 2.48 - 2.42 (m, 3H), 2.36 - 2.30 (m, 1H), 2.18 - 1.89 (m, 2H), 1.80 - 1.73 (m, 1H), 1.68 - 1.60 (m, 1H), 1.55 - 1.42 (m, 1H), 1.41 - 1.29 (m, 3H), 1.05 - 0.91 (m, 3H), 0.71 - 0.55 (m, 3H). |

### Preparation of 2-(4-(6-(3-(2-hydroxyphenyl)cinnolin-7-yl)-2,6-diazaspiro[3.3]heptan-2-yl)-1H-1,2,3-triazol-1-yl)-3-methylbutanoic acid (I-49)

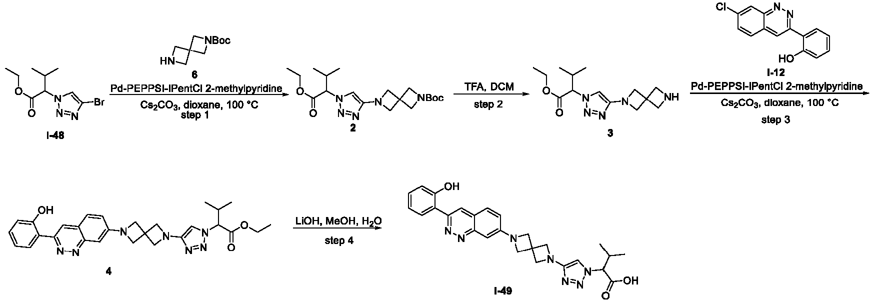

### Step 1: Preparation of tert-butyl 6-(1-(1-ethoxy-3-methyl-1-oxobutan-2-yl)-1H-1,2,3-triazol-4-yl)-2,6-diazaspiro[3.3]heptane-2-carboxylate (Intermediate 2)

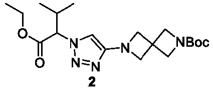

To a stirred solution of **I-48** (1.58 g, 5.722 mmol, **1** equiv) and Intermediate **6** (3.40 g, 17.166 mmol, 3 equiv) in 1,4-dioxane (30 mL) was added Cs₂CO₃ (5.59 g, 17.166 mmol, 3 equiv) and Pd-PEPPSI-IPentCl 2-methylpyridine (0.19 g, 0.229 mmol, 0.04 equiv) at room temperature under nitrogen atmosphere. The resulting mixture was stirred for overnight at 100°C under nitrogen atmosphere. The residue was purified by reversed-phase flash chromatography with the following conditions: column, C18 silica gel; mobile phase, MeCN in Water (0.1% FA), 10% to 50% gradient in 25 min; detector, UV 254 nm. This resulted in Intermediate **2** (2.1g, 92.10%) as a yellow oil. LCMS (ESI) m/z: [M+H]⁺ = 394.

### Step 2: Preparation of ethyl 2-(4-(2,6-diazaspiro[3.3]heptan-2-yl)-1H-1,2,3-triazol-1-yl)-3-methylbutanoate (Intermediate 3)

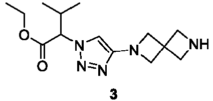

A solution of Intermediate **2** (2.1g, 5.340 mmol, 1 equiv) in DCM (6 mL) was added TFA (3 mL) at room temperature. The resulting mixture was stirred for 1 h at room temperature under nitrogen atmosphere. The resulting mixture was concentrated under reduced pressure. The residue was purified by reversed-phase flash chromatography with the following conditions: column, C18 silica gel; mobile phase, MeCN in Water, 10% to 100% gradient in 30 min; detector, UV 254 nm. This resulted in Intermediate **3** (1.4g, 89.17%) as a colorless oil. LCMS (ESI) m/z [M+H]⁺ = 294.

### Step 3: Preparation of ethyl 2-(4-(6-(3-(2-hydroxyphenyl)cinnolin-7-yl)-2,6-diazaspiro[3.3]heptan-2-yl)-1H-1,2,3-triazol-1-yl)-3-methylbutanoate (Intermediate 4)

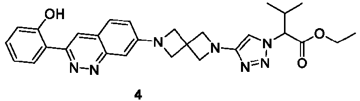

To a stirred solution of Intermediate **3** (1 g, 3.409 mmol, 1 equiv) and I**-12** (1.31 g, 5.114 mmol, 1.5 equiv) in dioxane (10 mL) were added Cs₂CO₃ (3.34 g, 10.227 mmol, 3 equiv) and Pd-PEPPSI-IPentCl 2-methylpyridine (2.87 mg, 0.003 mmol, 0.1 equiv) in portions at room temperature under nitrogen atmosphere. The resulting mixture was stirred for 2 h at 100°C under nitrogen atmosphere. Desired product could be detected by LCMS. The resulting mixture was diluted with EtOAc (100 mL). The combined organic layers were washed with brine (2 x 50 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in Intermediate **4** (985 mg, 56.26%) as a yellow solid. LCMS (ESI) m/z [M+H]⁺ = 514.

### Step 4: Preparation of 2-(4-(6-(3-(2-hydroxyphenyl)cinnolin-7-yl)-2,6-diazaspiro[3.3]heptan-2-yl)-1H-1,2,3-triazol-1-yl)-3-methylbutanoic acid (I-49)

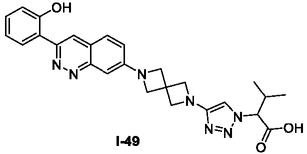

A mixture of Intermediate **4** (985 mg, 1.918 mmol, 1 equiv) and LiOH.H₂O (459.32 mg, 19.180 mmol, 10 equiv) in MeOH (4 mL) and H₂O (2 mL) was stirred for 2 h at room temperature. Desired product could be detected by LCMS. The mixture was acidified to pH 6 with HCl (aq.). The resulting mixture was diluted with CH₂Cl₂ (3 x 50 mL). The combined organic layers were washed with brine (1 x 100 mL), dried over anhydrous Na₂SO₄. After filtration, the filtrate was concentrated under reduced pressure. This resulted in **I-49** (498 mg, 53.48%) as a red solid. LCMS (ESI) m/z [M+H]⁺ = 486.

### Preparation of (2S,4R)-N-[(2-chloro-4-ethynylphenyl)methyl]-4-hydroxypyrrolidine-2-carboxamide (I-50)

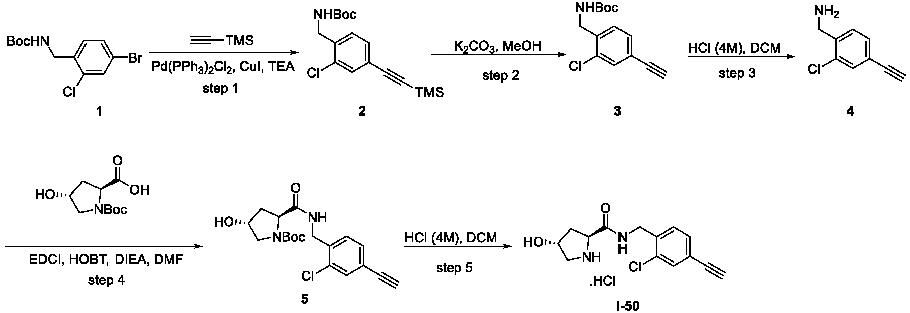

### Step 1: Preparation of tert-butyl N-({2-chloro-4-[2-(trimethylsilyl)ethynyljphenyl}methyl)carbamate (Intermediate 2)

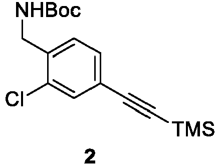

A mixture of tert-butyl N-[(4-bromo-2-chlorophenyl)methyl]carbamate (1 g, 3.119 mmol, 1 equiv), trimethylsilylacetylene (919.06 mg, 9.357 mmol, 3 equiv), Pd(dppf)Cl₂.CH₂Cl₂ (127.04 mg, 0.156 mmol, 0.05 equiv) and Cul (59.40 mg, 0.312 mmol, 0.1 equiv) in TEA (10 mL) was stirred for 4 h at 80 °C under nitrogen atmosphere. Desired product could be detected by LCMS. The resulting mixture was diluted with water (100 mL). The resulting mixture was extracted with EtOAc (2 x 100 mL). The combined organic layers were washed with brine (3x50 mL), dried over anhydrous Na₂SO₄. After filtration, the filtrate was concentrated under reduced pressure. The residue was purified by silica gel column chromatography, eluted with PE / EA (4:1) to afford Intermediate **2** (1.15 g, crude) as a light yellow solid. LCMS (ESI) m/z: [M+H]⁺ = 338.

### Step 2: Preparation of tert-butyl N-[(2-chloro-4-ethynylphenyl)methyl]carbamate (Intermediate 3)

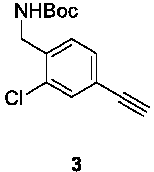

A mixture of Intermediate **2** (1.1 g, 3.255 mmol, 1 equiv) and K₂CO₃ (1349.66 mg, 9.765 mmol, 3 equiv) in MeOH (10 mL) was stirred for 3 h at room temperature. Desired product could be detected by LCMS. The resulting mixture was diluted with water (100 mL). The resulting mixture was extracted with EtOAc (3 x 100 mL). The combined organic layers were washed with brine (3x30 mL), dried over anhydrous Na₂SO₄. After filtration, the filtrate was concentrated under reduced pressure to afford Intermediate **3** (860 mg, 99.42%) as a Brown yellow oil. LCMS (ESI) m/z [M+H]⁺ = 266.

### Step 3: Preparation of 1-(2-chloro-4-ethynylphenyl)methanamine (Intermediate 4)

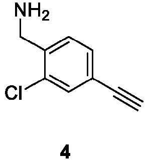

To a stirred solution of Intermediate **3** (850 mg, 3.199 mmol, 1 equiv) in DCM (5 mL) was added HCl(gas) in 1,4-dioxane (4M) (5 mL) dropwise at room temperature. The resulting mixture was stirred for 3 h at room temperature. Desired product could be detected by LCMS. The resulting mixture was concentrated under reduced pressure to afford Intermediate **4** (670 mg, HCl salt) as an off-white solid. LCMS (ESI) m/z: [M+H]⁺ = 166.

### Step 4: Preparation of tert-butyl (2S,4R)-2-{[(2-chloro-4-ethynylphenyl)methyl]carbamoyl}-4-hydroxypyrrolidine-1-carboxylate (Intermediate 5)

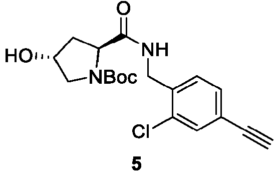

A mixture of (2S,4R)-1-(tert-butoxycarbonyl)-4-hydroxypyrrolidine-2-carboxylic acid (200 mg, 0.865 mmol, 1.00 equiv), EDCI (248.69 mg, 1.297 mmol, 1.5 equiv) and HOBT (175.30 mg, 1.297 mmol, 1.5 equiv) in DMF (3 mL) was stirred for 15 min at room temperature. To the above mixture was added Intermediate **4** (171.89 mg, 1.038 mmol, 1.2 equiv) and DIEA (335.35 mg, 2.595 mmol, 3 equiv) at room temperature. The resulting mixture was stirred for additional 2 h at room temperature. Desired product could be detected by LCMS. The residue was purified by reversed-phase flash chromatography with the following conditions: column, C18 silica gel; mobile phase, MeCN in Water (0.1% FA), 0% to 50% gradient in 15 min; detector, UV 254 nm to afford Intermediate **5** (320 mg, 97.66%) as a yellow solid. LCMS (ESI) m/z: [M+H]⁺ = 379.

### Step 5: Preparation of (2S,4R)-N-[(2-chloro-4-ethynylphenyl)methyl]-4-hydroxypyrrolidine-2-carboxamide (I-50)

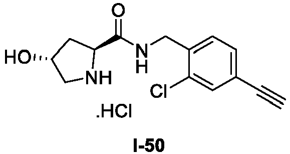

To a stirred solution of Intermediate **5** (310 mg, 0.818 mmol, 1 equiv) in DCM (3 mL) was added HCl(gas)in 1,4-dioxane (4M) (3 mL) dropwise at room temperature. The resulting mixture was stirred for 1 h at room temperature. Desired product could be detected by LCMS. The resulting mixture was concentrated under reduced pressure to afford **I-50** (310 mg, HCl salt) as an off-white solid. LCMS (ESI) m/z: [M+H]⁺ = 279.

### Preparation of 2-(3-{2-[(3S)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoic acid (I-52)

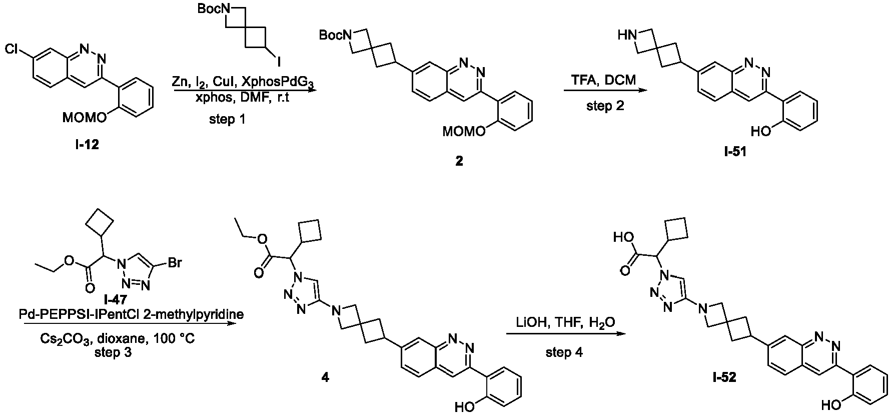

### Step 1: Preparation of tert-butyl 6-{3-[2-(methoxymethoxy)phenyl]cinnolin-7-yl}-2-azaspiro[3.3]heptane-2-carboxylate (Intermediate 2)

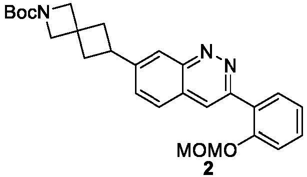

To a stirred mixture of tert-butyl 6-iodo-2-azaspiro[3.3]heptane-2-carboxylate (1.45 g, 4.489 mmol, 1.5 equiv),I₂ (0.38 g, 1.496 mmol, 0.5 equiv) and Zn (0.59 g, 8.979 mmol, 3 equiv) in DMF (9 mL) were added I**-12** (900 mg, 2.993 mmol, 1 equiv), xphos (0.29 g, 0.599 mmol, 0.2 equiv) and XPhos Pd G₃ (0.51 g, 0.599 mmol, 0.2 equiv) , in portions at room temperature under nitrogen atmosphere. The resulting mixture was stirred for overnight at 60°C under nitrogen atmosphere. This resulted in Intermediate 2 (1 g, 72.40%) as a brown solid. LCMS (ESI) m/z [M+H] ⁺ = 462.

### Step 2: Preparation of 2-(7-{2-azaspiro[3.3]heptan-6-yl}cinnolin-3-yl)phenol (I-51)

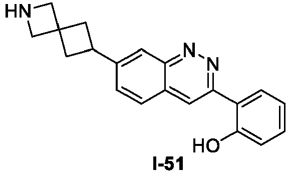

A mixture of Intermediate **2** (1 g, 2.167 mmol, 1 equiv) in TFA (7.5 mL) and DCM (2.5 mL) was stirred for 1h at room temperature. The resulting mixture was concentrated under reduced pressure. This resulted in **I-51** (1.4 g, 91.62%) as a brown oil. The crude product was used in the next step directly without further purification. LCMS (ESI) m/z [M+H] ⁺ = 318.

### Step 3: Preparation of ethyl 2-cyclobutyl-2-(4-{6-[3-(2-hydroxyphenyl)cinnolin-7-yl]-2-azaspiro[3.3]heptan-2-yl}-1,2,3-triazol-1-yl) acetate (Intermediate 4)

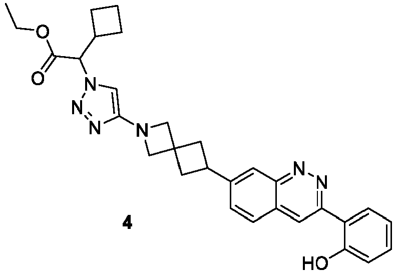

To a stirred mixture of **I-51** (200 mg, 0.630 mmol, 1 equiv) and **I-47** (217.88 mg, 0.756 mmol, 1.2 equiv) in DMSO (3 mL) were added Cul (24.00 mg, 0.126 mmol, 0.2 equiv), K₂CO₃ (261.26 mg, 1.890 mmol, 3 equiv) and L-proline (14.51 mg, 0.126 mmol, 0.2 equiv) in portions at room temperature under nitrogen atmosphere. The residue was purified by reversed-phase flash chromatography with the following conditions: column, C18 silica gel; mobile phase, MeCN in Water (0.1% FA), 0% to 100% gradient in 10 min; detector, UV 254 nm. This resulted in Intermediate **4** (60 mg, 18.1%) as a brown solid. LCMS (ESI) m/z: [M+H] ⁺ = 525.

### Step 4: Preparation of 2-(3-{2-[(3S)-3-[3-(2-hydroxyphenyl)thieno[2,3-c]pyridazin-6-yl]pyrrolidin-1-yl]pyrimidin-5-yl}-1,2-oxazol-5-yl)-3-methylbutanoic acid (I-52)

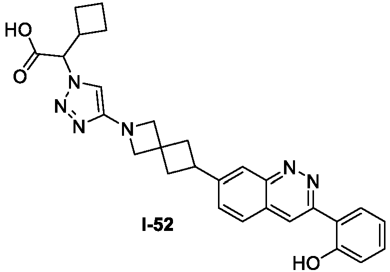

A mixture of Intermediate **4** (60 mg, 0.12 mmol, 1 equiv) and LiOH (24.91 mg, 1.240 mmol, 3 equiv) in MeOH (2 mL) and H₂O (1 mL) was stirred for 2 h at room temperature. Desired product could be detected by LCMS. The mixture was acidified to pH 6 with conc. HCl. The resulting mixture was concentrated under reduced pressure to afford **I-52** (40 mg, crude) as a brown solid. LCMS (ESI) m/z [M+H]⁺ = 497.

### Preparation of 2-(4-{6-[3-(2-hydroxyphenyl)cinnolin-7-yl]-2-azaspiro[3.3]heptan-2-yl}-1,2,3-triazol-1-yl)-3-methylbutanoic acid (I-53)

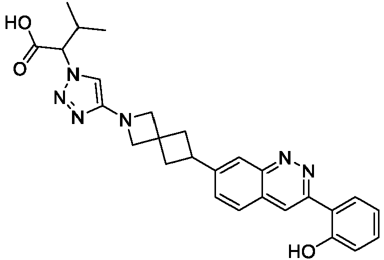

Intermediate **I-53** as a brown solid was prepared using procedures similar to those used above for the preparation of **I-52** using the **I-46** and **I-51.** LCMS (ESI) m/z [M+H]⁺ = 485.

### Preparation of (2S,4R)-N-[(2-chloro-4-ethynylphenyl)methyl]-4-hydroxy- 1-[(2S)-2-(4-{6-[3-(2-hydroxyphenyl)cinnolin-7-yl]-2,6-diazaspiro[3.3]heptan-2-yl}-1,2,3-triazol-1-yl)-3-methylbutanoyl]pyrrolidine-2-carboxamide (Compound 223-001)

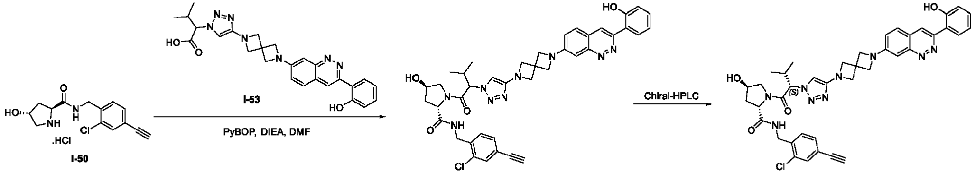

A mixture of I-50 (60.28 mg, 0.216 mmol, 1.50 equiv), **I-53** (70 mg, 0.144 mmol, 1.00 equiv), PyBOP (112.54 mg, 0.216 mmol, 1.5 equiv) and DIEA (55.90 mg, 0.432 mmol, 3 equiv) in DMF (1 mL) was stirred for 1 h at room temperature. Desired product could be detected by LCMS. The crude product was purified by Prep-HPLC with the following conditions (Column: CHIRALPAK IA-3, 4.6*50 mm, 3µm; Mobile Phase A: MtBE (0.1%DEA): EtOH=50: 50; Gradient) to afford the mixture of final compound (60 mg, 55.77%) as a reddish brown solid. LCMS (ESI) m/z: [M+H]⁺ = 746.

The above mixture was purified by Chiral-HPLC with the following conditions (Column: CHIRALPAK IA-3, 4.6*50 mm, 3 µm; Mobile Phase A: MtBE(0.1%DEA): MeOH=70: 30) to afford **223-001** (23.5 mg, 38.70%) as a yellow solid. ¹H NMR (400 MHz, DMSO-d6) δ 12.82 (s, 1H), 8.76 (s, 1H), 8.67 (t, J = 5.9 Hz, 1H), 8.03 (d, J = 8.2, 1.6 Hz, 1H), 7.96 (d, J = 9.1 Hz, 1H), 7.60 - 7.54 (m, 1H), 7.51 - 7.37 (m, 3H), 7.36 - 7.25 (m, 2H), 7.05 - 6.96 (m, 3H), 5.20 (d, J = 3.6 Hz, 1H), 5.11 (d, J = 10.4 Hz, 1H), 4.44 - 4.31 (m, 4H), 4.30 (d, J = 4.2 Hz, 5H), 4.09 - 3.99 (m, 4H), 3.86 - 3.78 (m, 1H), 3.62 (d, J = 10.9 Hz, 1H), 2.34 - 2.28 (m, 1H), 2.13 - 2.03 (m, 1H), 1.97 - 1.86 (m, 1H), 0.99 (d, J = 6.5 Hz, 3H), 0.67 (d, J = 6.6 Hz, 3H). LCMS (ESI) m/z: [M+H]⁺ = 746.25.

The compounds in Table 10 were prepared using procedures similar to those used above for the preparation of **Compound 223-001** using the appropriate amines and acid (**I-53** or analogs).

**Table 10.**

| **No.** | **Name** | **LCMS (ESI) m/z** | **¹H NMR** |
|---|---|---|---|
| 255 | (2S,4R)-N-(4-ethynylbenzyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 712.4 | 1H NMR (400 MHz, DMSO-d6) δ 12.82 (s, 1H), 8.76 (s, 1H), 8.50 (t, J = 6.0 Hz, 1H), 8.07 - 7.92 (m, 2H), 7.48 - 7.37 (m, 3H), 7.37 - 7.19 (m, 4H), 7.04 - 6.96 (m, 3H), 5.23 - 5.16 (m, 2H), 4.49 - 4.18 (m, 8H), 4.15 (d, J = 4.6 Hz, 1H), 4.07 - 3.96 (m, 4H), 3.75 - 3.68 (m, 1H), 3.53 (dd, J = 10.7, 4.4 Hz, 1H), 2.46 - 2.37 (m, 1H), 2.13 - 1.83 (m, 2H), 0.99 (d, J = 6.6 Hz, 3H), 0.71 (dd, J = 20.2, 6.6 Hz, 3H). |
| 254 | (2S,4R)-N-((S)-1-(4-ethynylphenyl)ethyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 726.25 | 1H NMR (300 MHz, DMSO-d6) δ 12.83 (s, 1H), 8.77 (s, 1H), 8.49 (d, J = 7.7 Hz, 1H), 8.04 (d, J = 7.5 Hz, 1H), 7.96 (d, J = 9.1 Hz, 1H), 7.49 - 7.39 (m, 3H), 7.30 (t, J = 7.7 Hz, 4H), 7.02 (dd, J = 7.4, 1.7 Hz, 3H), 5.18 (d, J = 3.6 Hz, 1H), 5.10 (d, J = 10.5 Hz, 1H), 4.89 (t, J = 7.2 Hz, 1H), 4.34 (d, J = 22.3 Hz, 6H), 4.14 (s, 1H), 4.12 - 3.97 (m, 4H), 3.76 (dd, J = 10.7, 4.1 Hz, 1H), 3.61 (d, J = 10.7 Hz, 1H), 2.38 (dd, J = 10.4, 6.3 Hz, 1H), 2.04 (s, 1H), 1.82 - 1.68 (m, 1H), 1.35 (d, J = 7.0 Hz, 3H), 1.03 (d, J = 6.6 Hz, 3H), 0.67 (d, J = 6.5 Hz, 3H). |
| 242 | (2S,4R)-N-(4-ethynyl-2-(3-methoxypropoxy)benzyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 800.35 | 1H NMR (400 MHz, DMSO-d6) δ 12.81 (s, 1H), 8.76 (s, 1H), 8.47 - 8.40 (m, 1H), 7.99 (dd, J = 29.2, 8.4 Hz, 2H), 7.46 (s, 1H), 7.36 - 7.23 (m, 3H), 7.12 - 6.83 (m, 5H), 5.36 - 4.89 (m, 2H), 4.46 - 4.33 (m, 2H), 4.32 - 4.19 (m, 6H), 4.14 (s, 1H), 4.10 - 3.98 (m, 6H), 3.86 - 3.79 (m, 1H), 3.61 (d, J = 10.8 Hz, 1H), 3.54 - 3.46 (m, 2H), 3.26 (s, 3H), 2.33 (s, 2H), 2.12 - 2.02 (m, 1H), 1.99 - 1.88 (m, 2H), 1.00 (d, J = 6.6 Hz, 3H), 0.67 (d, J = 6.6 Hz, 3H). |
| 238 | (2S,4R)-N-(4-ethynyl-2-methoxybenzyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 742.25 | 1H NMR (300 MHz, Methanol-d4) δ 8.60 (s, 1H), 7.95 (d, J = 7.2 Hz, 1H), 7.89 (d, J = 9.1 Hz, 1H), 7.42 (d, J = 13.6 Hz, 1H), 7.36 - 7.19 (m, 3H), 7.08 - 6.93 (m, 5H), 5.14 (d, J = 10.4 Hz, 1H), 4.54 (d, J = 8.4 Hz, 1H), 4.47 (d, J = 16.3 Hz, 1H), 4.38 (d, J = 3.1 Hz, 2H), 4.30 (s, 4H), 4.12 (s, 4H), 3.98 - 3.85 (m, 1H), 3.86 (s, 3H), 3.80 (d, J = 10.9 Hz, 1H), 3.45 (s, 1H), 2.58 - 2.52 (m, 1H), 2.27 - 2.14 (m, 1H), 2.14 - 2.00 (m, 1H), 1.09 (d, J = 6.6 Hz, 3H), 0.77 (d, J = 6.6 Hz, 3H). |
| 202 | (2S,4R)-N-((S)-1-(4-cyanophenyl)ethyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 727.35 | 1H NMR (400 MHz, DMSO-d6) δ 12.77 (s, 1H), 8.78 (s, 1H), 8.59 (d, J = 7.3 Hz, 1H), 8.06 - 7.93 (m, 2H), 7.85 - 7.77 (m, 2H), 7.51 - 7.43 (m, 3H), 7.36 - 7.25 (m, 2H), 7.04 - 6.96 (m, 3H), 5.19 (s, 1H), 5.09 (d, J = 10.4 Hz, 1H), 4.93 (p, J = 6.9 Hz, 1H), 4.37 (t, J = 8.1 Hz, 1H), 4.30 (s, 5H), 4.03 (q, J = 8.1 Hz, 4H), 3.83 - 3.69 (m, 1H), 3.62 (d, J = 10.7 Hz, 1H), 2.38 (dt, J = 11.3, 6.5 Hz, 1H), 2.12 - 2.01 (m, 1H), 1.73 (ddd, J = 13.1, 8.8, 4.6 Hz, 1H), 1.53 - 1.31 (m, 3H), 1.01 (d, J = 6.6 Hz, 3H), 0.66 (d, J = 6.6 Hz, 3H). |
| 201 | (2S,4R)-N-((S)-1-(4-fluorophenyl)ethyl)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)pyrrolidine-2-carboxamide | 720.3 | 1H NMR (400 MHz, DMSO-d6) δ 12.83 (s, 1H), 8.77 (s, 1H), 8.46 (d, J = 7.7 Hz, 1H), 8.03 (dd, J = 8.2, 1.6 Hz, 1H), 7.96 (d, J = 9.1 Hz, 1H), 7.46 (s, 1H), 7.37 - 7.23 (m, 4H), 7.14 (td, J = 9.2, 2.7 Hz, 2H), 7.06 - 6.90 (m, 3H), 5.18 (d, J = 3.6 Hz, 1H), 5.13 - 5.02 (m, 1H), 4.96 - 4.70 (m, 1H), 4.36 (t, J = 8.1 Hz, 1H), 4.29 (s, 5H), 4.03 (q, J = 8.1 Hz, 4H), 3.84 - 3.72 (m, 1H), 3.61 (d, J = 10.8 Hz, 1H), 2.39 (dt, J = 10.5, 6.6 Hz, 1H), 2.03 (t, J = 10.5 Hz, 1H), 1.74 (ddd, J = 12.9, 8.3, 4.5 Hz, 1H), 1.52 - 1.30 (m, 3H), 1.03 (d, J = 6.5 Hz, 3H), 0.67 (d, J = 6.5 Hz, 3H). |
| 169 | (2S,4R)-4-hydroxy-1-((S)-2-(4-(6-(3-(2-hydroxyphenyl)cinnolin-7-yl)-2,6-diazaspiro[3.3]heptan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 799.3 | 1H NMR (400 MHz, DMSO-d6) δ 12.83 (s, 1H), 8.99 (d, J = 4.2 Hz, 1H), 8.76 (s, 1H), 8.50 (d, J = 7.7 Hz, 1H), 8.03 (d, J = 7.2 Hz, 1H), 7.96 (d, J = 9.1 Hz, 1H), 7.64 - 7.43 (m, 3H), 7.42 - 7.35 (m, 2H), 7.34 - 7.19 (m, 2H), 7.13 - 6.89 (m, 3H), 5.17 (d, J = 3.6 Hz, 1H), 5.09 (d, J = 10.3 Hz, 1H), 5.01 - 4.81 (m, 1H), 4.39 (t, J = 8.1 Hz, 1H), 4.31 (s, 5H), 4.03 (q, J = 8.1 Hz, 4H), 3.76 (dd, J = 10.6, 4.1 Hz, 1H), 3.62 (d, J = 10.9 Hz, 1H), 2.47 (d, J = 4.3 Hz, 3H), 2.42 - 2.33 (m, 1H), 2.06 (t, J = 10.4 Hz, 1H), 1.95 - 1.69 (m, 1H), 1.57 - 1.35 (m, 3H), 1.03 (d, J = 6.6 Hz, 3H), 0.69 (d, J = 6.5 Hz, 3H). |
| 211 | (2S,4R)-1-((S)-2-cyclobutyl-2-(4-(6-(3-(2-hydroxyphenyl)cinnolin-7-yl)-2,6-diazaspiro[3.3]heptan-2-yl)-1H-1,2,3-triazol-1-yl)acetyl)-4-hydroxy-N-((S)-1-(4-(4-methylthiazol-5-yl)phenyl)ethyl)pyrrolidine-2-carboxamide | 811.4 | 1H NMR (400 MHz, DMSO-d6) δ 12.83 (d, J = 3.7 Hz, 1H), 8.99 (d, J = 4.4 Hz, 1H), 8.76 (s, 1H), 8.38 (d, J = 7.7 Hz, 1H), 8.03 (d, J = 7.8 Hz, 1H), 7.96 (d, J = 9.0 Hz, 1H), 7.44 (d, J = 8.2 Hz, 3H), 7.39 - 7.23 (m, 4H), 7.10 - 6.87 (m, 3H), 5.49 (d, J = 10.1 Hz, 1H), 5.16 (d, J = 3.8 Hz, 1H), 4.96 - 4.85 (m, 1H), 4.39 (t, J = 7.9 Hz, 1H), 4.29 (s, 5H), 4.10 - 3.93 (m, 4H), 3.79 (dd, J = 10.7, 4.3 Hz, 1H), 3.53 (d, J = 10.6 Hz, 1H), 3.12 - 2.95 (m, 1H), 2.47 (d, J = 4.7 Hz, 3H), 2.27 - 1.95 (m, 2H), 1.92 - 1.66 (m, 6H), 1.38 (d, J = 7.0 Hz, 3H). |
| 193 | (2S,4R)-1-((S)-2-cyclobutyl-2-(4-(6-(3-(2-hydroxyphenyl)cinnolin-7-yl)-2-azaspiro[3.3]heptan-2-yl)-1H-1,2,3-triazol-1-yl)acetyl)-4-hydroxy-N-((S)-1-(4-(4-methylthiazol-5-yl)phenyl)ethyl)pyrrolidine-2-carboxamide | 810.35 | 1H NMR (400 MHz, Methanol-d4) δ 8.84 (d, J = 30.5 Hz, 2H), 8.20 (s, 1H), 8.04 (d, J = 8.6 Hz, 2H), 7.80 (dd, J = 8.6, 1.7 Hz, 1H), 7.63 - 7.28 (m, 6H), 7.04 (ddd, J = 8.5, 3.7, 1.7 Hz, 2H), 5.43 (dd, J = 68.4, 10.0 Hz, 1H), 5.03 (q, J = 7.1 Hz, 1H), 4.64 - 4.41 (m, 2H), 4.09 (s, 2H), 3.95 - 3.62 (m, 5H), 3.24 - 3.05 (m, 1H), 2.80 (td, J = 8.7, 2.9 Hz, 2H), 2.54 (td, J = 9.6, 2.9 Hz, 2H), 2.48 (s, 3H), 2.25 - 2.13 (m, 2H), 2.08 - 1.82 (m, 6H), 1.52 (d, J = 7.0 Hz, 3H). |
| 212 | (2S,4R)-4-hydroxy-1-((S)-2-(4-(6-(3-(2-hydroxyphenyl)cinnolin-7-yl)-2-azaspiro[3.3]heptan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 798.35 | 1H NMR (300 MHz, Methanol-d4) δ 8.89 (s, 1H), 8.80 (s, 1H), 8.23 (s, 1H), 8.06 (d, J = 8.5 Hz, 2H), 7.81 (dd, J = 8.7, 1.8 Hz, 1H), 7.52 - 7.27 (m, 6H), 7.17 - 6.86 (m, 2H), 5.24 - 4.94 (m, 2H), 4.54 (dd, J = 18.3, 10.1 Hz, 2H), 4.12 (s, 2H), 3.97 - 3.66 (m, 5H), 2.83 (t, J = 10.3 Hz, 2H), 2.65 - 2.50 (m, 3H), 2.50 (s, 3H), 2.28 - 2.15 (m, 1H), 2.00 (td, J = 8.8, 4.5 Hz, 1H), 1.56 (d, J = 7.1 Hz, 3H), 1.14 (d, J = 6.4 Hz, 3H), 0.80 (dd, J = 6.5, 4.2 Hz, 3H). |
| 192 | (2S,4R)-4-hydroxy-1-((S)-2-(4-(9-(3-(2-hydroxyphenyl)cinnolin-7-yl)-3,9-diazaspiro[5.5]undecan-3-yl)-1H-1,2,3-triazol-1-yl)-3-methylbutanoyl)-N-((S)-1-(4-(4-methylthiazol-5-yl)phenyl)ethyl)pyrrolidine-2-carboxamide | 855.4 | 1H NMR (400 MHz, DMSO-d6) δ 12.87 (s, 1H), 8.99 (s, 1H), 8.76 (s, 1H), 8.51 (d, J = 7.6 Hz, 1H), 8.09 - 7.99 (m, 1H), 7.94 (d, J = 9.3 Hz, 1H), 7.90 - 7.84 (m, 1H), 7.56 - 7.48 (m, 2H), 7.45 (d, J = 8.2 Hz, 2H), 7.41 - 7.29 (m, 3H), 7.01 (t, J = 7.6 Hz, 2H), 5.18 (d, J = 3.6 Hz, 1H), 5.08 (d, J = 10.3 Hz, 1H), 4.92 (p, J = 7.2 Hz, 1H), 4.39 (t, J = 8.1 Hz, 1H), 4.32 (s, 1H), 3.81 - 3.74 (m, 1H), 3.62 (d, J = 10.7 Hz, 1H), 3.52 (d, J = 6.5 Hz, 4H), 3.31 (s, 1H), 3.22 - 3.10 (m, 4H), 2.55 (m, 3H), 2.06 (t, J = 10.6 Hz, 1H), 1.84 - 1.75 (m, 1H), 1.71 - 1.50 (m, 8H), 1.38 (d, J = 7.0 Hz, 3H), 1.03 (d, J = 6.5 Hz, 3H), 0.67 (d, J = 6.5 Hz, 3H). |
| 172 | (2S,4R)-4-hydroxy-1-((S)-2-(4-(6-(3-(2-hydroxyphenyl)cinnolin-6-yl)-2,6-diazaspiro[3.3]heptan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 799.3 | 1H NMR (400 MHz, DMSO-d6) δ 13.34 (s, 1H), 8.99 (s, 1H), 8.61 - 8.45 (m, 2H), 8.20 (d, J = 9.3 Hz, 1H), 8.08 - 7.92 (m, 1H), 7.53 - 7.43 (m, 3H), 7.42 - 7.30 (m, 3H), 7.27 (dd, J = 9.3, 2.4 Hz, 1H), 7.06 - 6.93 (m, 2H), 6.58 (d, J = 2.4 Hz, 1H), 5.18 (d, J = 3.5 Hz, 1H), 5.09 (d, J = 9.7 Hz, 1H), 4.99 - 4.86 (m, 1H), 4.39 (t, J = 8.1 Hz, 1H), 4.31 (s, 5H), 4.03 (q, J = 8.1 Hz, 4H), 3.82 - 3.70 (m, 1H), 3.62 (d, J = 10.8 Hz, 1H), 2.47 (d, J = 4.1 Hz, 3H), 2.45 - 2.26 (m, 1H), 2.11 - 2.00 (m, 1H), 1.90 - 1.72 (m, 1H), 1.59 - 1.35 (m, 3H), 1.03 (d, J = 6.6 Hz, 3H), 0.68 (d, J = 6.5 Hz, 3H). |

### Preparation of (2S,4R)-4-hydroxy-1-[(2S)-2-(4-{6-[3-(2-hydroxyphenyl)cinnolin-7-yl]spiro[3.3]heptan-2-yl}-1,2,3-triazol-1-yl)-3-methylbutanoyl]-N-[(1S)-1-[4-(4-methyl-1,3-thiazol-5-yl)phenyl]ethyl]pyrrolidine-2-carboxamide (Compound 208-001).

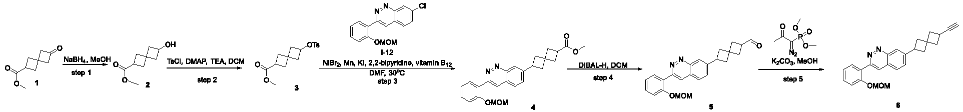

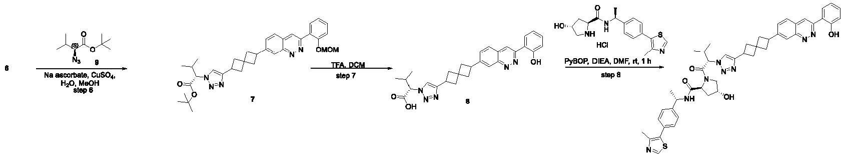

### Step 1: Preparation of methyl 6-hydroxyspiro[3.3]heptane-2-carboxylate (intermediate 2).

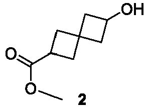

To a stirred solution of methyl 6-oxospiro[3.3]heptane-2-carboxylate (5 g, 29.728 mmol, 1 equiv) in MeOH (60 mL, 1481.930 mmol, 49.85 equiv) was added NaBH4 (1.69 g, 44.592 mmol, 1.5 equiv) in portions at -4 °C under air atmosphere. The resulting mixture was stirred for an additional 10 min at -4 °C. The resulting mixture was concentrated under reduced pressure. The residue was dissolved in water (20 mL). The resulting mixture was extracted with EtOAc (3 x 40 mL). The combined organic layers were washed with brine (3x20 mL), dried over anhydrous Na₂SO₄. After filtration, the filtrate was concentrated under reduced pressure. This resulted in intermediate **2** (4.1 g, 72.93%) as a yellow oil. LCMS (ESI) m/z: [M+H]⁺ = 171.

### Step 2: Preparation methyl 6-[(4-methylbenzenesulfonyl)oxy]spiro[3.3]heptane-2-carboxylate (intermediate 3).

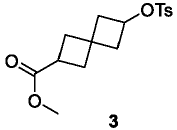

To a stirred solution of methyl 6-hydroxyspiro[3.3]heptane-2-carboxylate (4 g, 23.501 mmol, 1 equiv) and TsCl (5.38 g, 28.201 mmol, 1.2 equiv) in DCM (40 mL, 629.224 mmol, 26.77 equiv) were added DMAP (287.11 mg, 2.350 mmol, 0.1 equiv) and TEA (7.13 g, 70.503 mmol, 3 equiv) in portions at room temperature under air atmosphere. The resulting mixture was stirred for additional 2 h at room temperature. The resulting mixture was concentrated under reduced pressure. The residue was purified by silica gel column chromatography, eluted with PE / EA (1:1) to afford intermediate **3** (7.6 g, 89.72%) as a off-white oil. LCMS (ESI) m/z: [M+H] ⁺ = 325.

### Step 3: Preparation of methyl 6-{3-[2-(methoxymethoxy)phenyl]cinnolin-7-yl}spiro[3.3]heptane-2-carboxylate (intermediate 4).

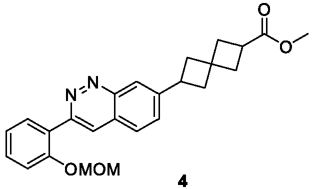

A solution of Mn (338.72 mg, 6.164 mmol, 4 equiv), KI (255.87 mg, 1.541 mmol, 1 equiv), NiBr₂.glyme (47.57 mg, 0.154 mmol, 0.1 equiv) and 2,2-bipyridine (24.07 mg, 0.154 mmol, 0.1 equiv) ,Vitamin B12 (207.07 mg, 0.154 mmol, 0.1 equiv) in DMF (10 mL) was stirred for 1h at room temperature. Then methyl 6-[(4-methylbenzenesulfonyl)oxy]spiro[3.3]heptane-2-carboxylate (500 mg, 1.541 mmol, 1 equiv) and 7-chloro-3-[2-(methoxymethoxy)phenyl]cinnoline (463.55 mg, 1.541 mmol, 1 equiv) was added to the reaction mixture. The resulting mixture was stirred for 16h at 60°C under nitrogen atmosphere. The residue was purified by reversed-phase flash chromatography with the following conditions: column, C18 silica gel; mobile phase, MeCN in Water (0.1% FA), 10% to 80% gradient in 30 min; detector, UV 254 nm.This resulted in intermediate **4** (150 mg, 23.25%) as a brown oil. LCMS (ESI) m/z: [M+H] ⁺ = 419.

### Step 4: Preparation 6-{3-[2-(methoxymethoxy)phenyl]cinnolin-7-yl}spiro[3.3]heptane-2-carbaldehyde (intermediate 5).

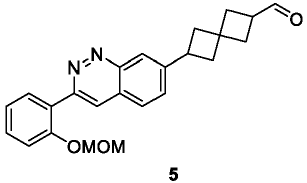

A solution of methyl 6-{3-[2-(methoxymethoxy)phenyl]cinnolin-7-yl}spiro[3.3]heptane-2-carboxylate (110 mg, 0.263 mmol, 1 equiv) in DCM (4 mL) was cooled to -78°C. DIBAI-H (56.07 mg, 0.395 mmol, 1.5 equiv) was added to the reaction mixture at -78°C. The resulting mixture was stirred for 1h at -78°C under nitrogen atmosphere. Desired product could be detected by LCMS. The reaction was quenched with Water at 0°C. The aqueous layer was extracted with EtOAc (3x10 mL). The residue was purified by silica gel column chromatography, eluted with PE / EA (1:1) to afford intermediate **5** (55 mg, 53.86%) as a colorless oil. LCMS (ESI) m/z: [M+H] ⁺ = 389.

### Step 5: Preparation of 7-{6-ethynylspiro[3.3]heptan-2-yl}-3-[2-(methoxymethoxy)phenyl]cinnoline (intermediate 6).

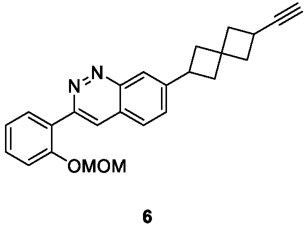

A solution 6-{3-[2-(methoxymethoxy)phenyl]cinnolin-7-yl}spiro[3.3]heptane-2-carbaldehyde (50 mg, 0.129 mmol, 1 equiv) in MeOH (2 mL) was added seyferth-gilbert homologation (37.09 mg, 0.194 mmol, 1.5 equiv) and K₂CO₃ (53.37 mg, 0.387 mmol, 3 equiv). The resulting mixture was stirred for 2h at room temperature under nitrogen atmosphere. Desired product could be detected by LCMS. The resulting mixture was concentrated under vacuum. The residue was purified by silica gel column chromatography, eluted with PE / EA (1:1) to afford intermediate **6** (40 mg, 80.83%) as a yellow oil. LCMS (ESI) m/z: [M+H] ⁺ = 385.

### Step 6: Preparation of tert-butyl (2S)-2-[4-(6-{3-[2-(methoxymethoxy)phenyl]cinnolin-7-yl}spiro[3.3]heptan-2-yl)-1,2,3-triazol-1-yl]-3-methylbutanoate (intermediate 7).

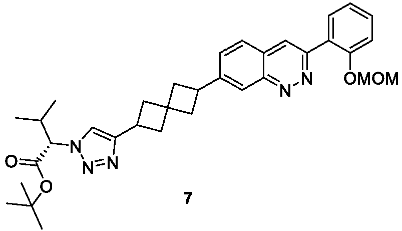

A solution of 7-{6-ethynylspiro[3.3]heptan-2-yl}-3-[2-(methoxymethoxy)phenyl]cinnoline (40 mg, 0.104 mmol, 1 equiv) and tert-butyl (2S)-2-azido-3-methylbutanoate (41.46 mg, 0.208 mmol, 2 equiv) in MeOH (1 mL) and H₂O (0.5 mL) was added sodium ascorbate (10.36 mg, 0.052 mmol, 0.5 equiv) and CuSO4.5H2O (12.99 mg, 0.052 mmol, 0.5 equiv). The resulting mixture was stirred for 2h at room temperature under nitrogen atmosphere. Desired product could be detected by LCMS. The resulting mixture was concentrated under vacuum. The residue was purified by silica gel column chromatography, eluted with PE / EA (1:1) to afford intermediate 7 (28 mg, 46.11%) as a yellow solid. LCMS (ESI) m/z: [M+H] ⁺ = 584.

### Step 7: Preparation of (2S)-2-(4-{6-[3-(2-hydroxyphenyl)cinnolin-7-yl]spiro[3.3]heptan-2-yl}-1,2,3-triazol-1-yl)-3-methylbutanoic acid (intermediate 8).

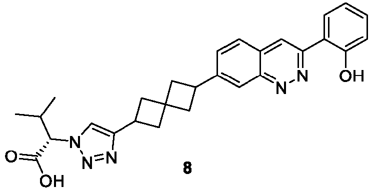

A solution of tert-butyl (2S)-2-[4-(6-{3-[2-(methoxymethoxy)phenyl]cinnolin-7-yl}spiro[3.3]heptan-2-yl)-1,2,3-triazol-1-yl]-3-methylbutanoate (30 mg, 0.051 mmol, 1 equiv) in TFA (1 mL) and DCM (1 mL) was stirre for 3h at room temperature. The resulting mixture was concentrated under vacuum. This resulted in (2S)-2-(4-{6-[3-(2-hydroxyphenyl)cinnolin-7-yl]spiro[3.3]heptan-2-yl}-1,2,3-triazol-1-yl)-3-methylbutanoic acid (20 mg, 80.48%) as a red oil. LCMS (ESI) m/z: [M+H] ⁺ = 484.

### Step 8: Preparation of (2S,4R)-4-hydroxy-1-[(2S)-2-(4-{6-[3-(2-hydroxyphenyl)cinnolin-7-yl]spiro[3.3]heptan-2-yl}-1,2,3-triazol-1-yl)-3-methylbutanoyl]-N-[(1S)-1-[4-(4-methyl-1,3-thiazol-5-yl)phenyljethyljpyrrolidine-2-carboxamide (Compound 208-001).

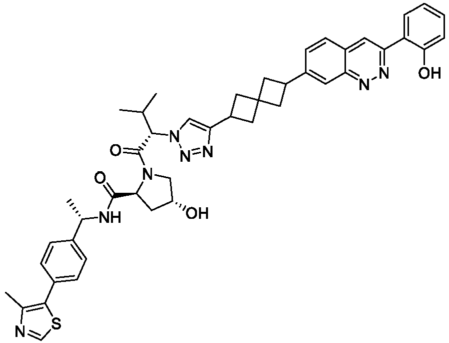

A solution of (2S)-2-(4-{6-[3-(2-hydroxyphenyl)cinnolin-7-yl]spiro[3.3]heptan-2-yl}-1,2,3-triazol-1-yl)-3-methylbutanoic acid (25 mg, 0.052 mmol, 1 equiv) and PyBOP (40.36 mg, 0.078 mmol, 1.5 equiv) in DMF (1.5 mL) was added (2S,4R)-4-hydroxy-N-[(1S)-1-[4-(4-methyl-1,3-thiazol-5-yl)phenyl]ethyl]pyrrolidine-2-carboxamide (22.27 mg, 0.068 mmol, 1.3 equiv) and DIEA (20.05 mg, 0.156 mmol, 3 equiv) .The resulting mixture was stirred for 1h at room temperature under nitrogen atmosphere. The residue was purified by reversed-phase flash chromatography with the following conditions: column, C18 silica gel; mobile phase, MeCN in Water (0.1% FA), 10% to 80% gradient in 30 min; detector, UV 254 nm. This resulted in **208-001** (3 mg, 7.28%) as a yellow solid. 1H NMR (400 MHz, Methanol-d4) δ 8.87 (d, J = 5.1 Hz, 1H), 8.80 (s, 1H), 8.19 (s, 1H), 8.13 - 8.01 (m, 2H), 7.95 (d, J = 5.6 Hz, 1H), 7.79 (dd, J = 8.7, 1.8 Hz, 1H), 7.57 - 7.19 (m, 5H), 7.10 - 6.96 (m, 2H), 5.27 (d, J = 10.3 Hz, 1H), 5.04 (d, J = 7.0 Hz, 1H), 4.57 - 4.42 (m, 2H), 3.90 (dd, J = 10.9, 3.9 Hz, 1H), 3.86 - 3.72 (m, 2H), 3.59 (p, J = 8.5 Hz, 1H), 2.77 (ddd, J = 23.5, 13.6, 6.2 Hz, 2H), 2.62 - 2.52 (m, 2H), 2.47 (d, J = 3.9 Hz, 3H), 2.46 - 2.31 (m, 3H), 2.32 - 2.21 (m, 2H), 2.19 (d, J = 8.3 Hz, 1H), 1.97 (ddd, J = 13.4, 9.1, 4.6 Hz, 1H), 1.59 (dd, J = 46.8, 7.0 Hz, 3H), 1.13 (t, J = 6.5 Hz, 3H), 0.76 (t, J = 6.4 Hz, 3H). LCMS (ESI) m/z: [M+H] ⁺ = 797.62.

The compound in Table 11 was prepared using procedures similar to those used above for the preparation of **Compound 208-001.**

**Table 11.**

| **No.** | **Name** | **LCMS (ESI) m/z** | **¹H NMR** |
|---|---|---|---|
| 230 | (2S,4R)-4-hydroxy-1-((S)-2-(4-(6-(3-(2-hydroxyphenyl)cinnolin-6-yl)spiro[3.3]heptan-2-yl)-1H-1,2,3-triazol-1-yl)-3-methylbutanoyl)-N-((S)-1-(4-(4-methylthiazol-5-yl)phenyl)ethyl)pyrrolidine-2-carboxamide | 797.35 | 1H NMR (400 MHz, DMSO-d6) δ 8.99 (s, 1H), 8.88 (s, 1H), 8.49 (d, J = 7.6 Hz, 1H), 8.40 (d, J = 8.8 Hz, 1H), 8.11 (d, J = 7.1 Hz, 1H), 7.99 (s, 1H), 7.89 - 7.86 (m, 1H), 7.85 (d, J = 8.8 Hz, 1H), 7.55 - 7.42 (m, 2H), 7.41 - 7.25 (m, 3H), 7.11 - 6.95 (m, 2H), 5.35 - 5.04 (m, 2H), 4.92 (t, J = 7.1 Hz, 1H), 4.40 (t, J = 8.1 Hz, 1H), 4.32 (s, 1H), 3.80 - 3.63 (m, 3H), 3.52 - 3.45 (m, 2H), 3.04 (s, 1H), 2.92 - 2.85 (m, 1H), 2.76 - 2.60 (m, 4H), 2.45 (s, 4H), 2.41 - 2.30 (m, 3H), 2.31 - 2.16 (m, 3H), 2.06 (t, J = 10.6 Hz, 1H), 1.90 - 2.00 (m, 2H), 1.40 (d, J = 7.0 Hz, 3H), 1.05 (d, J = 6.6 Hz, 3H), 0.66 (d, J = 6.5 Hz, 3H). |

### Preparation of (2S,4R)-4-hydroxy-1-((R)-2-(3-(2-((3-(2-hydroxyphenyl)cinnolin-6-yl)oxy)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide (Compound 185-001)

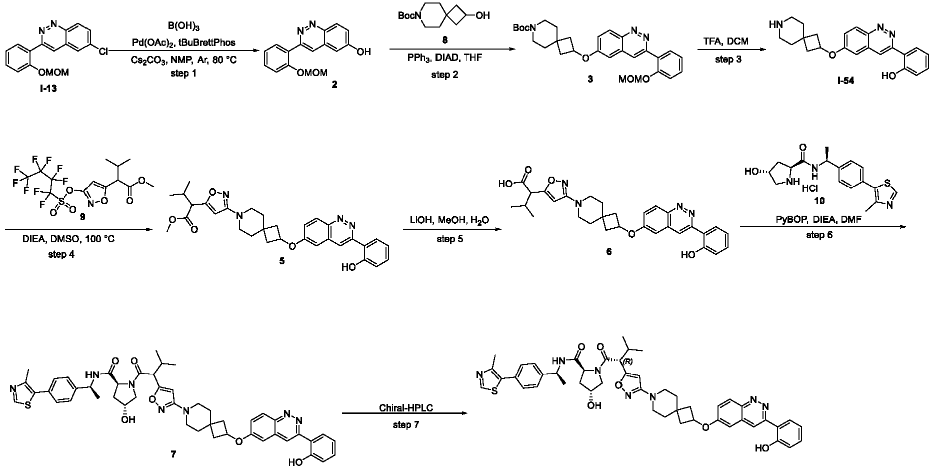

### Step 1: Preparation of 3-(2-(methoxymethoxy)phenyl)cinnolin-6-ol (Intermediate 2)

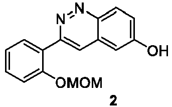

To a stirred mixture of I-26 (3 g, 9.975 mmol, 1 equiv) and boric acid (1.23 g, 19.950 mmol, 2 equiv) in NMP (30 mL) were added Cs₂CO₃ (9.78 g, 29.925 mmol, 3 equiv), t-BuBrettPhos (1.21 g, 2.494 mmol, 0.25 equiv) and Pd(OAc)₂ (223.96 mg, 0.998 mmol, 0.1 equiv) in portions at room temperature. The resulting mixture was stirred for 2 h at 80°C under nitrogen atmosphere. Desired product could be detected by LCMS. The resulting mixture was extracted with EtOAc (3 x 50 mL). The combined organic layers were washed with brine (1 x 100 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% FA), 0% to 100% gradient in 30 min; detector, UV 254 nm. The resulting mixture was concentrated under reduced pressure. This resulted in Intermediate **2** (1.042 g, 37.00%) as a yellow solid. LCMS (ESI) m/z: [M+H]⁺ = 283.

### Step 2: Preparation of tert-butyl 2-((3-(2-(methoxymethoxy)phenyl)cinnolin-6-yl)oxy)-7-azaspiro[3.5]nonane-7-carboxylate (Intermediate 3)

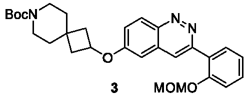

To a stirred mixture of Intermediate **2** (300 mg, 1.063 mmol, 1 equiv) and Intermediate **8** (384.69 mg, 1.595 mmol, 1.5 equiv) in THF (1 mL) were added triphenylphosphine (278.74 mg, 1.063 mmol, 1 equiv) and DIAD (214.89 mg, 1.063 mmol, 1 equiv) in portions at 60°C under nitrogen atmosphere. The resulting mixture was stirred overnight at 40°C under nitrogen atmosphere. Desired product could be detected by LCMS. The resulting mixture was extracted with EtOAc (3 x 50 mL). The combined organic layers were washed with brine (1 x 100 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% FA), 0% to 100% gradient in 30 min; detector, UV 254 nm. The resulting mixture was concentrated under reduced pressure to afford Intermediate **3** (357 mg, 66.44%) as a yellow solid. LCMS (ESI) m/z [M+H]⁺ = 506.

### Step 3: Preparation of 2-(6-((7-azaspiro[3.5]nonan-2-yl)oxy)cinnolin-3-yl)phenol (I-54)

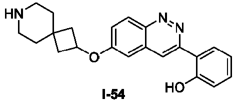

A mixture of Intermediate **3** (357 mg, 0.706 mmol, 1 equiv) and TFA (1 mL) in DCM (3 mL) was stirred for 1.5 h at room temperature. Desired product could be detected by LCMS. The resulting mixture was concentrated under reduced pressure. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in **I-54** (187 mg, 73.27%) as a light-yellow solid. LCMS (ESI) m/z [M+H]⁺ = 362.

### Step 4: Preparation of methyl 2-(3-(2-((3-(2-hydroxyphenyl)cinnolin-6-yl)oxy)-7-azaspiro[3.5]nonan-7-yl)isoxazol-5-yl)-3-methylbutanoate (Intermediate 5)

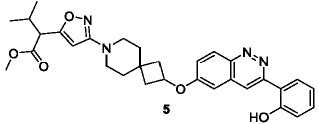

To a stirred mixture of **I-54** (178 mg, 0.492 mmol, 1 equiv) and Intermediate **9** (474.04 mg, 0.984 mmol, 2 equiv) in DMSO (3 mL) was added DIEA (190.95 mg, 1.476 mmol, 3 equiv) at room temperature under nitrogen atmosphere. The resulting mixture was stirred for 2 h at 100°C under nitrogen atmosphere. Desired product could be detected by LCMS. The residue was purified by reversed-phase flash chromatography with the following conditions: column, C18 silica gel; mobile phase, MeCN in Water (0.1% FA), 0% to 100% gradient in 30 min; detector, UV 254 nm. The resulting mixture was concentrated under reduced pressure to afford Intermediate **5** (65 mg, 24.32%) as a light-yellow solid. LCMS (ESI) m/z [M+H]⁺ = 543.

### Step 5: Preparation of 2-(3-(2-((3-(2-hydroxyphenyl)cinnolin-6-yl)oxy)-7-azaspiro[3.5]nonan-7-yl)isoxazol-5-yl)-3-methylbutanoic acid (Intermediate 6)

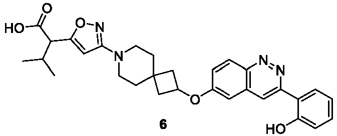

A mixture of Intermediate **5** (65 mg, 0.079 mmol, 1 equiv) and LiOH.H₂O (18.88 mg, 0.790 mmol, 10 equiv) in MeOH (1 mL) and H₂O (0.5 mL) was stirred for 2 h at room temperature. Desired product could be detected by LCMS. The mixture was acidified to pH 4 with HCl (aq.). The precipitated solids were collected by filtration and washed with Water (3 x 10 mL). This resulted in Intermediate **6** (55 mg) as a light-yellow solid. LCMS (ESI) m/z [M+H]⁺ = 529.

### Step 6: Preparation of (2S,4R)-4-hydroxy-1-(2-(3-(2-((3-(2-hydroxyphenyl)cinnolin-6-yl)oxy)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide (Intermediate 7)

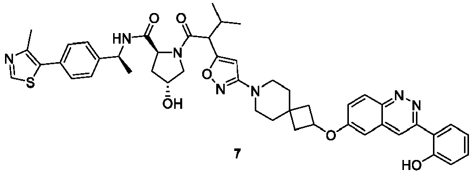

To a stirred mixture of Intermediate **6** (50 mg, 0.095 mmol, 1 equiv) and Intermediate **10** (47.02 mg, 0.143 mmol, 1.5 equiv) in DMF (1 mL) were added PyBOP (98.45 mg, 0.190 mmol, 2 equiv) and DIEA (36.68 mg, 0.285 mmol, 3 equiv) in portions at room temperature. The resulting mixture was stirred for 2 h at room temperature. Desired product could be detected by LCMS. The residue was purified by reversed-phase flash chromatography with the following conditions: column, C18 silica gel; mobile phase, MeCN in Water (0.1% FA), 0% to 100% gradient in 30 min; detector, UV 254 nm. The resulting mixture was concentrated under reduced pressure. This resulted in Intermediate **7** (35 mg, 43.94%) as a light-yellow solid. LCMS (ESI) m/z [M+H]⁺ = 842.

### Step 7: Preparation of (2S,4R)-4-hydroxy-1-((R)-2-(3-(2-((3-(2-hydroxyphenyl)cinnolin-6-yl)oxy)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide (Compound 185-001)

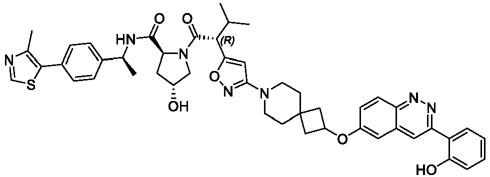

The Intermediate **7** was purified by Chiral-HPLC with the following conditions (Column: CHIRALPAK ID 2*25 cm, 5 µm; Mobile Phase A: MtBE (10mM NH₃-MeOH), Mobile Phase B: EtOH--HPLC; Flow rate: 20 mL/min; Wave Length: 272/212 nm; RT1 (min): 6.575; RT2(min): 10.685; Sample Solvent: MeOH: DCM=1: 1--HPLC; Injection Volume: 0.8 mL; Number of Runs: 3) to afford **185-001** (second peak) (15.6 mg, 43.41%) as a light yellow solid. ¹H NMR (300 MHz, DMSO-d₆) *δ* 12.36 (s, 1H), 8.99 (s, 1H), 8.80 (s, 1H), 8.44 - 8.31 (m, 2H), 8.11 - 8.02 (m, 1H), 7.61 - 7.51 (m, 1H), 7.50 - 7.39 (m, 2H), 7.43 - 7.33 (m, 3H), 7.30 (d, J = 2.6 Hz, 1H), 7.04 (t, J = 7.9 Hz, 2H), 6.09 (d, J = 39.3 Hz, 1H), 5.11 (s, 1H), 5.02 (t, J = 6.7 Hz, 1H), 4.92 (t, J = 7.2 Hz, 1H), 4.37 (t, J = 7.8 Hz, 1H), 4.29 (s, 1H), 3.77 - 3.68 (m, 1H), 3.57 (d, J = 9.9 Hz, 1H), 3.40 (s, 1H), 3.23 (s, 2H), 3.13 (s, 2H), 2.62 (d, J = 10.5 Hz, 2H), 2.46 (s, 3H), 2.35 - 2.11 (m, 1H), 2.09 - 1.90 (m, 3H), 1.69 (d, J = 23.7 Hz, 3H), 1.50 - 1.36 (m, 2H), 0.95 (d, J = 6.5 Hz, 3H), 0.88 - 0.75 (m, 3H). LCMS (ESI) m/z: [M+H]⁺ = 842.30.

The compound in Table 12 were prepared using procedures similar to those used above for the preparation of **Compound 185-001.**

**Table 12.**

| **No.** | **Name** | **LCMS (ESI) m/z** | **¹H NMR** |
|---|---|---|---|
| 184 | (2S,4R)-4-hydroxy-1-((R)-2-(3-(6-((3-(2-hydroxyphenyl)cinnolin-6-yl)oxy)-2-azaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 814.25 | 1H NMR (300 MHz, Methanol-d4) δ 8.87 (s, 1H), 8.73 - 8.58 (m, 1H), 8.30 (d, J = 9.4 Hz, 1H), 8.01 (d, J = 8.0 Hz, 1H), 7.57 - 7.48 (m, 1H), 7.43 (d, J = 2.5 Hz, 5H), 7.24 - 7.17 (m, 1H), 7.07 - 6.99 (m, 2H), 5.86 (d, J = 14.6 Hz, 1H), 5.03 (d, J = 7.1 Hz, 2H), 4.51 (t, J = 8.1 Hz, 1H), 4.44 (s, 1H), 4.08 (s, 2H), 3.98 (s, 2H), 3.91 - 3.78 (m, 1H), 3.69 - 3.55 (m, 2H), 2.99 - 2.93 (m, 2H), 2.47 (d, J = 2.3 Hz, 5H), 2.36 (s, 2H), 2.18 (s, 1H), 1.56 (dd, J = 20.0, 7.0 Hz, 3H), 1.05 (d, J = 6.6 Hz, 3H), 0.95 - 0.84 (m, 3H). |
| 183 | (2S,4R)-4-hydroxy-1-((R)-2-(3-(4-((3-(2-hydroxyphenyl)cinnolin-7-yl)oxy)piperidin-1-yl)isoxazol-5-yl)-3-methylbutanoyl)-N-((S)-1-(4-(4-methylthiazol-5-yl)phenyl)ethyl)pyrrolidine-2-carboxamide | 802.15 | 1H NMR (400 MHz, DMSO-d6) δ 12.26 (d, J = 6.1 Hz, 1H), 8.99 (s, 1H), 8.87 (s, 1H), 8.41 (d, J = 7.6 Hz, 1H), 8.07 (d, J = 9.0 Hz, 2H), 7.91 (s, 1H), 7.63 - 7.55 (m, 1H), 7.51- 7.31 (m, 5H), 7.08- 6.99 (m, 2H), 6.21 (s, 1H), 5.13 (d, J = 3.7 Hz, 1H), 5.06- 4.87 (m, 2H), 4.39 (t, J = 7.9 Hz, 1H), 4.33- 4.27 (m, 1H), 3.77-3.68 (m, 1H), 3.63 - 3.55 (m, 3H), 3.49-3.42 (m, 1H), 3.27- 3.17 (m, 2H), 2.49-2.45 (m, 3H), 2.27- 1.98 (m, 4H), 1.87-1.72 (m, 3H), 1.47 (d, J = 6.9 Hz, 1H), 1.39 (d, J = 7.0 Hz, 2H), 0.97 (d, J = 6.6 Hz, 3H), 0.84 (dd, J = 15.6, 6.4 Hz, 3H). |
| 182 | (2S,4R)-4-hydroxy-1-((R)-2-(3-(4-((3-(2-hydroxyphenyl)cinnolin-6-yl)oxy)piperidin-1-yl)isoxazol-5-yl)-3-methylbutanoyl)-N-((S)-1-(4-(4-methylthiazol-5-yl)phenyl)ethyl)pyrrolidine-2-carboxamide | 802.25 | 1H NMR (300 MHz, DMSO-d6) δ 12.42 - 12.14 (m, 1H), 8.99 (s, 1H), 8.84 - 8.73 (m, 1H), 8.49 - 8.28 (m, 2H), 8.18 - 7.96 (m, 1H), 7.68 - 7.53 (m, 2H), 7.48 - 7.41 (m, 2H), 7.40 - 7.33 (m, 3H), 7.12 - 6.96 (m, 2H), 6.28 - 6.05 (m, 1H), 5.12 (d, J = 3.6 Hz, 1H), 4.96 - 4.65 (m, 2H), 4.45 - 4.20 (m, 2H), 3.80 - 3.55 (m, 4H), 3.51 - 3.39 (m, 1H), 3.27 - 3.09 (m, 2H), 2.48 - 2.43 (m, 3H), 2.28 - 1.92 (m, 4H), 1.89 - 1.63 (m, 3H), 1.50 - 1.29 (m, 3H), 1.05 - 0.90 (m, 3H), 0.88 - 0.72 (m, 3H). |
| 84 | (2S,4R)-4-hydroxy-1-((R)-2-(3-(6-((3-(2-hydroxyphenyl)cinnolin-7-yl)oxy)-2-azaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 814.2 | 1H NMR (300 MHz, DMSO-d6) δ 12.22 (s, 1H), 8.99 (s, 1H), 8.88 (s, 1H), 8.42 (d, J = 7.7 Hz, 1H), 8.07 (dd, J = 9.3, 2.7 Hz, 2H), 7.63 (d, J = 2.4 Hz, 1H), 7.54 (dd, J = 9.1, 2.4 Hz, 1H), 7.49 - 7.40 (m, 2H), 7.41 - 7.29 (m, 3H), 7.13 - 6.94 (m, 2H), 5.88 (s, 1H), 5.12 (s, 1H), 4.95 (dq, J = 28.0, 6.9 Hz, 2H), 4.44 - 4.17 (m, 2H), 4.02 (s, 2H), 3.90 (s, 2H), 3.71 (dd, J = 10.5, 4.4 Hz, 1H), 3.59 (d, J = 9.7 Hz, 1H), 3.53 - 3.38 (m, 1H), 2.95 (dd, J = 13.4, 6.4 Hz, 2H), 2.46 (s, 3H), 2.44 - 2.35 (m, 2H), 2.30 - 2.12 (m, 1H), 2.03 (t, J = 10.4 Hz, 1H), 1.78 (ddd, J = 12.7, 8.0, 4.6 Hz, 1H), 1.51 - 1.32 (m, 3H), 0.95 (d, J = 6.4 Hz, 3H), 0.81 (dd, J = 11.2, 6.6 Hz, 3H). |
| 78 | (2S,4R)-4-hydroxy-1-((R)-2-(3-(2-((3-(2-hydroxyphenyl)cinnolin-7-yl)oxy)-7-azaspiro[3.5]nonan-7-yl)isoxazol-5-yl)-3-methylbutanoyl)-N-((S)-1-(4-(4-methylthiazol-5-yl)phenyl)ethyl)pyrrolidine-2-carboxamide | 842.15 | 1H NMR (400 MHz, Chloroform-d) δ 8.83 (s, 1H), 8.38 (s, 1H), 7.98 - 7.75 (m, 2H), 7.53 (d, J = 2.5 Hz, 1H), 7.49 (d, J = 7.6 Hz, 1H), 7.46 - 7.34 (m, 6H), 7.14 (dd, J = 8.3, 1.2 Hz, 1H), 7.05 - 6.97 (m, 1H), 5.95 (s, 1H), 5.07 (p, J = 7.1 Hz, 1H), 4.95 (p, J = 6.6 Hz, 1H), 4.65 (ddd, J = 20.0, 8.8, 5.2 Hz, 2H), 3.78 (dd, J = 10.7, 5.0 Hz, 1H), 3.64 (dd, J = 10.6, 3.8 Hz, 1H), 3.49 (d, J = 10.0 Hz, 1H), 3.27 (t, J = 5.6 Hz, 2H), 3.20 (t, J = 5.7 Hz, 2H), 2.59 (s, 6H), 2.50 - 2.38 (m, 1H), 2.09 (dd, J = 12.9, 6.5 Hz, 2H), 1.98 (ddd, J = 13.1, 8.2, 4.8 Hz, 1H), 1.82 - 1.73 (m, 6H), 1.50 (d, J = 7.0 Hz, 3H), 1.05 (d, J = 6.5 Hz, 3H), 0.94 (d, J = 6.7 Hz, 3H). |

### Preparation of methyl 2-(3-(2-azaspiro[3.3]heptan-6-yl)isoxazol-5-yl)-3-methylbutanoate (I-55)

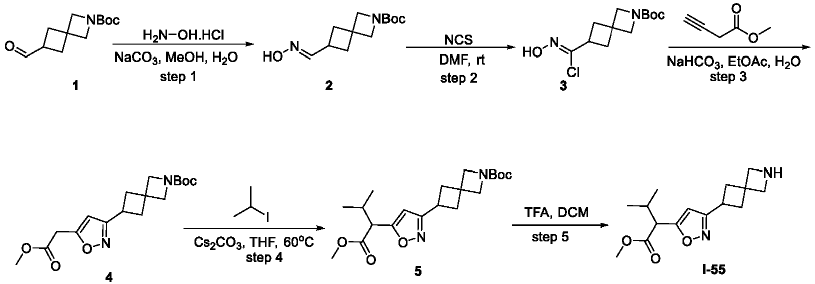

### Step 1: Preparation of tert-butyl (E)-6-((hydroxyimino)methyl)-2-azaspiro[3.3]heptane-2-carboxylate (Intermediate 2)

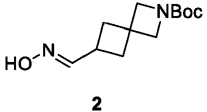

To a stirred mixture of tert-butyl 6-formyl-2-azaspiro[3.3]heptane-2-carboxylate (6 g, 26.633 mmol, 1 equiv) and hydroxylamine hydrochloride (2.78 g, 39.950 mmol, 1.5 equiv) in MeOH (60 mL) and H₂O (6 mL) was added sodium methaneperoxoate sodium (8.55 g, 79.899 mmol, 3 equiv) dropwise at room temperature. The resulting mixture was stirred for 2 h at room temperature. Desired product could be detected by LCMS. The resulting mixture was concentrated under reduced pressure diluted with water (100 mL), The resulting mixture was extracted with EtOAc (3 x 100 mL). The combined organic layers were washed with brine (1 x 250 mL), dried over anhydrous Na₂SO₄. After filtration, the filtrate was concentrated under reduced pressure. This resulted in Intermediate **2** (6.7 g, crude) as a light yellow oil. LCMS (ESI) m/z: [M+H]⁺ = 241.

### Step 2: Preparation of tert-butyl (Z)-6-(chloro(hydroxyimino)methyl)-2-azaspiro[3.3]heptane-2-carboxylate (Intermediate 3)

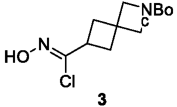

To a stirred mixture of Intermediate **2** (6.5 g, 27.049 mmol, 1 equiv) and NCS (4.33 g, 32.459 mmol, 1.2 equiv) in EA (65 mL) was stirred for 2 h at room temperature. Desired product could be detected by LCMS. The resulting mixture was concentrated under reduced pressure. The resulting mixture was diluted with EtOAc (100 mL). The combined organic layers were washed with brine (1 x 250 mL), dried over anhydrous Na₂SO₄. After filtration, the filtrate was concentrated under reduced pressure. This resulted in Intermediate **3** (9.403 g, crude) as a blue solid. LCMS (ESI) m/z [M+H]⁺ = 275.

### Step 3: Preparation of tert-butyl 6-(5-(2-methoxy-2-oxoethyl)isoxazol-3-yl)-2-azaspiro[3.3]heptane-2-carboxylate (Intermediate 4)

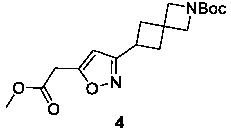

To a stirred mixture of Intermediate **3** (9.403 g, 29.090 mmol, 1 equiv, 85%) and methyl but-3-ynoate (2.85 g, 29.090 mmol, 1 equiv) in EA (90 mL) were added sodium methaneperoxoate (7.33 g, 87.270 mmol, 3 equiv) and H₂O (10 mL) in portions at room temperature. The resulting mixture was stirred for overnight at room temperature. Desired product could be detected by LCMS. The resulting mixture was diluted with water (100 mL) extracted with EtOAc (3 x 100 mL). The combined organic layers were washed with brine (1 x 250 mL), dried over anhydrous Na₂SO₄. After filtration, the filtrate was concentrated under reduced pressure. The residue was purified by silica gel column chromatography, eluted with PE / EA (1:5) to afford Intermediate **4** (4.414 g, 45.11%) as a light yellow oil. LCMS (ESI) m/z [M+H]⁺ = 337.

### Step 4: Preparation of tert-butyl 6-(5-(1-methoxy-3-methyl-1-oxobutan-2-yl)isoxazol-3-yl)-2-azaspiro[3.3]heptane-2-carboxylate Intermediate 5)

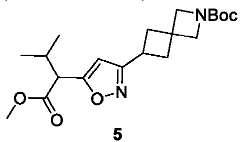

To a stirred mixture of Intermediate **4** (4.4 g, 13.080 mmol, 1 equiv) and caesio methaneperoxoate caesium (12.82 g, 39.240 mmol, 3 equiv) in THF (45 mL) was added 2-iodopropane (6.67 g, 39.240 mmol, 3 equiv) in portions at room temperature. The resulting mixture was stirred for overnight at 60°C under nitrogen atmosphere. Desired product could be detected by LCMS. The resulting mixture was diluted with water (100 mL) extracted with EtOAc (3 x 100 mL). The combined organic layers were washed with brine (1 x 200 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in Intermediate **5** (1.954 g, 39.47%) as a red oil. LCMS (ESI) m/z [M+H]⁺ = 379.

### Step 5: Preparation of methyl 2-(3-(2-azaspiro[3.3]heptan-6-yl)isoxazol-5-yl)-3-methylbutanoate (I-55)

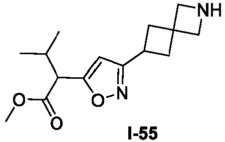

A mixture of Intermediate **5** (1.9 g, 5.020 mmol, 1 equiv) and TFA (7 mL) in DCM (21 mL) was stirred for 2 h at room temperature. Desired product could be detected by LCMS. The resulting mixture was concentrated under reduced pressure. This resulted in **I-55** (2.1 g, TFA salt) as a Brown yellow oil. LCMS (ESI) m/z [M+H]⁺ = 279.

### Preparation of (2S,4R)-4-hydroxy-1-((R)-2-(3-(2-(3-(2-hydroxyphenyl)cinnolin-6-yl)-2-azaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide (Compound 200-001)

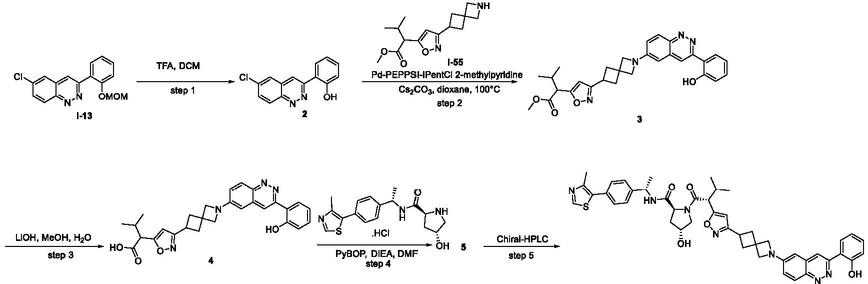

### Step 1: Preparation of 2-(6-chlorocinnolin-3-yl)phenol (Intermediate 2)

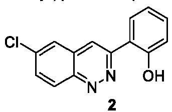

To a stirred solution of **I-13** (500 mg, 1.663 mmol, 1 equiv) and TFA (5 mL) in DCM (5 mL) was stirred for 5h at room temperature. The residue was purified by reversed-phase flash chromatography with the following conditions: column, C18 silica gel; mobile phase, MeCN in Water (0.1% FA), 0% to 70% gradient in 20 min; detector, UV 254 nm. This resulted in Intermediate **2** (219 mg, 51.32%) as a brown oil. LCMS (ESI) m/z: [M+H]⁺ = 257.

### Step 2: Preparation of methyl 2-(3-(2-(3-(2-hydroxyphenyl)cinnolin-6-yl)-2-azaspiro[3.3]heptan-6-yl)isoxazol-5-yl)-3-methylbutanoate (Intermediate 3)

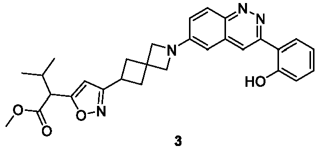

To a stirred solution of 2-(6-chlorocinnolin-3-yl)phenol (209 mg, 0.814 mmol, 1 equiv) and methyl 2-(3-{2-azaspiro[3.3]heptan-6-yl}-1,2-oxazol-5-yl)-3-methylbutanoate (271.96 mg, 0.977 mmol, 1.2 equiv) in dioxane (5 mL) were added Pd-PEPPSI-IPentCl 2-methylpyridine (o-picoline (68.49 mg, 0.081 mmol, 0.1 equiv) and Cs₂CO₃ (530.57 mg, 1.628 mmol, 2 equiv) in portions at room temperature. The resulting mixture was stirred for 2h at 100°C under nitrogen atmosphere. Desired product could be detected by LCMS. The residue was purified by reversed-phase flash chromatography with the following conditions: column, C18 silica gel; mobile phase, MeCN in Water (0.1% FA), 20% to 70% gradient in 20 min; detector, UV 254 nm. This resulted in Intermediate **3** (237 mg, 58.38%) as a red solid. LCMS (ESI) m/z: [M+H]⁺ = 499.

### Step 3: Preparation of methyl 2-(3-(2-(3-(2-hydroxyphenyl)cinnolin-6-yl)-2-azaspiro[3.3]heptan-6-yl)isoxazol-5-yl)-3-methylbutanoate (Intermediate 4)

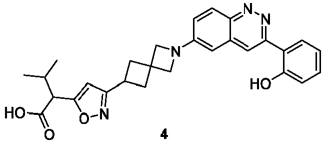

A solution of methyl 2-(3-{2-[3-(2-hydroxyphenyl)cinnolin-6-yl]-2-azaspiro[3.3]heptan-6-yl}-1,2-oxazol-5-yl)-3-methylbutanoate (227 mg, 0.455 mmol, 1 equiv) and LiOH.H₂O (191.04 mg, 4.550 mmol, 10 equiv) in MeOH (4 mL) and H₂O (2 mL) was stirred for 1h at room temperature. Desired product could be detected by LCMS. The mixture was acidified to pH 5 with HCl (aq.). The resulting mixture was extracted with EtOAc (3 x 20 mL). The combined organic layers were washed with brine (1x 25 mL), dried over anhydrous Na₂SO₄. After filtration, the filtrate was concentrated under reduced pressure. This resulted in Intermediate **4** (124 mg, 56.21%) as an orange solid. LCMS (ESI) m/z: [M+H]⁺ = 485.

### Step4: Preparation of (2S,4R)-4-hydroxy-1-(2-(3-(2-(3-(2-hydroxyphenyl)cinnolin-6-yl)-2-azaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide (Intermediate 5)

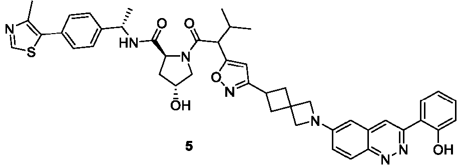

To a stirred solution of 2-(3-{2-[3-(2-hydroxyphenyl)cinnolin-6-yl]-2-azaspiro[3.3]heptan-6-yl}-1,2-oxazol-5-yl)-3-methylbutanoic acid (114 mg, 0.235 mmol, 1 equiv) and (2S,4R)-4-hydroxy-N-[(1S)-1-[4-(4-methyl-1,3-thiazol-5-yl)phenyl]ethyl]pyrrolidine-2-carboxamide (93.57 mg, 0.282 mmol, 1.2 equiv) in DMF (3.0 mL) were added PyBOP (244.87 mg, 0.470 mmol, 2 equiv) and DIEA (91.22 mg, 0.705 mmol, 3 equiv) in portions at room temperature. The resulting mixture was stirred for 2h at room temperature. The residue was purified by reversed-phase flash chromatography with the following conditions: column, C18 silica gel; mobile phase, MeCN in Water (0.1% FA), 0% to 100% gradient in 25 min; detector, UV 254 nm. This resulted in Intermediate **5** (95 mg, 50.60%) as an orange solid. LCMS (ESI) m/z: [M+H]⁺ = 798.

### Step 5: Preparation of(2S,4R)-4-hydroxy-1-((R)-2-(3-(2-(3-(2-hydroxyphenyl)cinnolin-6-yl)-2-azaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide (Compound 200-001.)

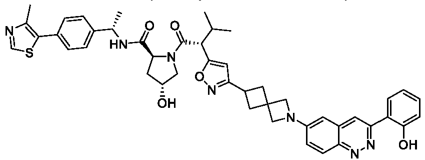

The mixture product Intermediate **5** was purified by Chiral-HPLC with the following conditions (Column: CHIRAL ART Cellulose-SB, 3*25 cm, 5 µm; Mobile Phase A: MtBE(10mM NH3-MeOH), Mobile Phase B: EtOH--HPLC; Flow rate: 40 mL/min; Gradient: isocratic 15; Wave Length: 220/284 nm; RT1(min): 7.94; RT2(min): 11.467; Sample Solvent: MeOH--HPLC; Injection Volume: 1mL) to afford **200-001** (48.4 mg, 50.74%) (second peak) as a yellow solid. ¹H NMR (400 MHz, DMSO-d6) δ 13.39 (s, 1H), 8.99 (s, 1H), 8.53 (s, 1H), 8.41 (d, J = 7.7 Hz, 1H), 8.18 (d, J = 9.2 Hz, 1H), 8.08 - 8.01 (m, 1H), 7.50 - 7.41 (m, 2H), 7.41 - 7.30 (m, 3H), 7.28 - 7.21 (m, 1H), 7.03 - 6.95 (m, 2H), 6.57 - 6.51 (m, 1H), 6.33 (s, 1H), 5.06 (dd, J = 34.3, 3.3 Hz, 1H), 4.93 (p, 1H), 4.38 (t, J = 7.9 Hz, 1H), 4.32 - 4.23 (m, 3H), 4.08 (s, 2H), 3.80 - 3.69 (m, 2H), 3.63 - 3.44 (m, 2H), 2.71 - 2.62 (m, 2H), 2.46 (s, 5H), 2.32 - 2.18 (m, 1H), 2.06 - 1.99 (m, 1H), 1.85 - 1.74 (m, 1H), 1.43 (dd, J = 33.2, 6.9 Hz, 3H), 0.99 (d, J = 6.5 Hz, 3H), 0.81 (dd, J = 11.8, 6.6 Hz, 3H). LCMS (ESI) m/z: [M+H]⁺ = 798.34.

The compound in Table 13 was prepared using procedures similar to those used above for the preparation of **Compound 200-001.**

**Table 13.**

| **No.** | **Name** | **LCMS (ESI) m/z** | **¹H NMR** |
|---|---|---|---|
| 199 | (2S,4R)-4-hydroxy-1-((R)-2-(3-(2-(3-(2-hydroxyphenyl)cinnolin-7-yl)-2-azaspiro[3.3]heptan-6-yl)isoxazol-5-yl)-3-methylbutanoyl)-N-((S)-1-(4-(4-methylthiazol-5-yl)phenyl)ethyl)pyrrolidine-2-carboxamide | 798.25 | 1H NMR (400 MHz, DMSO-d6) δ 12.89 (d, J = 3.8 Hz, 1H), 8.99 (d, J = 3.1 Hz, 1H), 8.75 (s, 1H), 8.42 (d, J = 7.7 Hz, 1H), 8.03 (dd, J = 8.0, 1.7 Hz, 1H), 7.94 (d, J = 9.0 Hz, 1H), 7.51 - 7.42 (m, 2H), 7.42 - 7.35 (m, 2H), 7.35 - 7.22 (m, 2H), 7.04 - 6.95 (m, 3H), 6.39 (s, 1H), 5.11 (d, J = 3.6 Hz, 1H), 4.92 (q, J = 7.2 Hz, 1H), 4.39 (t, J = 7.9 Hz, 1H), 4.30 (s, 1H), 4.23 (s, 2H), 4.06 (s, 2H), 3.84 - 3.69 (m, 2H), 3.64 - 3.44 (m, 2H), 2.72 - 2.62 (m, 2H), 2.50 - 2.41 (m, 4H), 2.33 - 2.19 (m, 2H), 2.10 - 1.99 (m, 1H), 1.86 - 1.73 (m, 1H), 1.39 (d, J = 7.0 Hz, 3H), 1.02 - 0.96 (m, 3H), 0.88 - 0.77 (m, 3H). |

### Preparation of (2S,4R)-4-hydroxy-1-[(2S)-2-(4-{2-[3-(2-hydroxyphenyl)cinnolin-7-yl]-2-azaspiro[3.3]heptan-6-yl}-1,2,3-triazol-1-yl)-3-methylbutanoyl]-N-[(1S)-1-[4-(4-methyl-1,3-thiazol-5-yl)phenyl]ethyl]pyrrolidine-2-carboxamide (Compound 186-001)

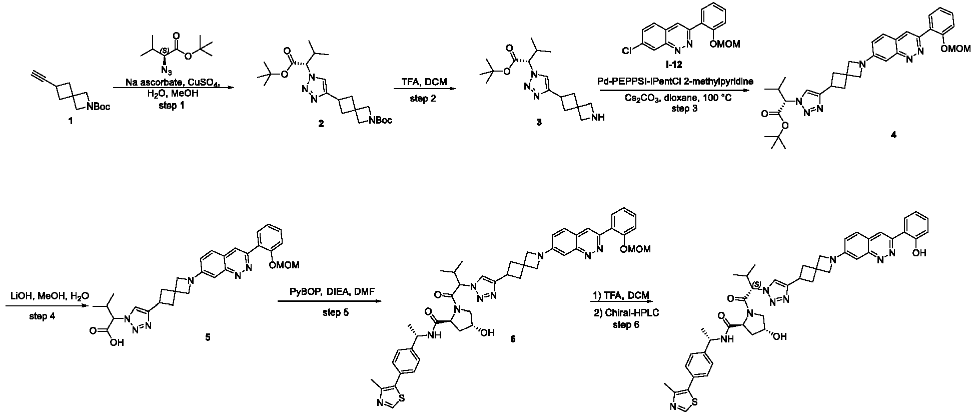

### Step 1: Preparation of tert-butyl 6-{1-[(2S)-1-(tert-butoxy)-3-methyl-1-oxobutan-2-yl]-1,2,3-triazol-4-yl}-2-azaspiro[3.3]heptane-2-carboxylate (Intermediate 2)

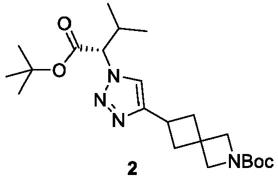

To a stirred mixture of tert-butyl 6-ethynyl-2-azaspiro[3.3]heptane-2-carboxylate (200 mg, 0.904 mmol, 1 equiv) and tert-butyl (2S)-2-azido-3-methylbutanoate (270.11 mg, 1.356 mmol, 1.5 equiv) in MeOH (2 mL, 49.398 mmol) and H₂O (4 mL, 222.037 mmol) were added CuSO₄ (72.12 mg, 0.452 mmol, 0.5 equiv) and sodium (5R)-5-[(1S)-1,2-dihydroxyethyl]-3,4-dihydroxy-2,5-dihydrofuran-2-one (89.97 mg, 0.452 mmol, 0.5 equiv) dropwise/ in portions at room temperature.The residue was purified by silica gel column chromatography, eluted with CH₂Cl₂ / MeOH (10:1) to afford Intermediate **2** (270 mg, 71.04%) as a colorless oil. LCMS (ESI) m/z [M+H]⁺ = 421.

### Step 2: Preparation of (2S)-2-(4-{2-azaspiro[3.3]heptan-6-yl}-1 ,2,3-triazol-1-yl)-3-methylbutanoate (Intermediate 3)

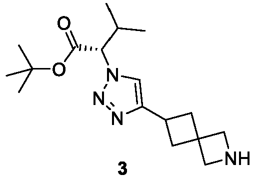

A mixture of Intermediate **2** (90 mg, 0.214 mmol, 1 equiv) in TFA (1.5 mL) and DCM (0.5 mL) was stirred for 1h at room temperature. The resulting mixture was concentrated under reduced pressure. This resulted in Intermediate 3 (78.3 mg, 87.50%) as a brown oil. LCMS (ESI) m/z [M+H] ⁺ = 321.

### Step 3: Preparation of tert-butyl (2S)-2-[4-(2-{3-[2-(methoxymethoxy)phenyl]cinnolin-7-yl}-2-azaspiro[3.3]heptan-6-yl)-1,2,3-triazol-1-yl]-3-methylbutanoate (Intermediate 4)

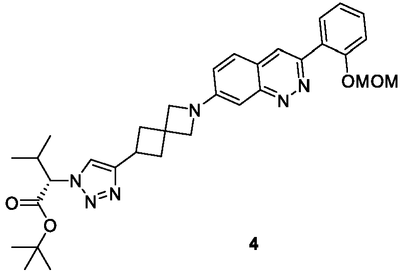

To a stirred mixture of Intermediate **3** (150 mg, 0.468 mmol, 1 equiv) and Cs₂CO₃ (457.56 mg, 1.404 mmol, 3 equiv) in dioxane (3 mL) was added Pd-PEPPSI-IPentCI 2-methylpyridine (o-picoline (39.38 mg, 0.047 mmol, 0.1 equiv) and 7-chloro-3-[2-(methoxymethoxy)phenyl]cinnoline (140.78 mg, 0.468 mmol, 1 equiv) in portions at room temperature under nitrogen atmosphere. The residue was purified by silica gel column chromatography, eluted with PE / EA (1:1) to afford Intermediate **4** (100 mg, 36.53%) as a brown solid. LCMS (ESI) m/z: [M+H]⁺ = 557.

### Step 4: Preparation of 2-[4-(2-{3-[2-(methoxymethoxy)phenyl]cinnolin-7-yl}-2-azaspiro[3.3]heptan-6-yl)-1,2,3-triazol-1-yl]-3-methylbutanoic acid (Intermediate 5)

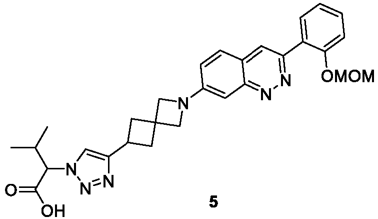

A mixture of Intermediate **4** (100 mg, 0. 17 mmol, 1 equiv) and LiOH (40.96 mg, 1.710 mmol, 10 equiv) in MeOH (2 mL, 148.175 mmol) and H₂O (0.5 mL, 111.005 mmol) was stirred for 1h at room temperature. The residue was acidified to pH 7 with conc. HCl. The precipitated solids were collected by filtration and washed with water (2x10 mL). This resulted in Intermediate **5** (80 mg, 88.49%) as a brown solid. LCMS (ESI) m/z [M+H] ⁺ = 529.

### Step 5: Preparation of (2S,4R)-4-hydroxy-1-{2-[4-(2-{3-[2-(methoxymethoxy)phenyl]cinnolin-7-yl}-2-azaspiro[3.3]heptan-6-yl)-1,2,3-triazol-1-yl]-3-methylbutanoyl}-N-[(1S)-1-[4-(4-methyl-1,3-thiazol-5-yl)phenyl]ethyl]pyrrolidine-2-carboxamide (Intermediate 6)

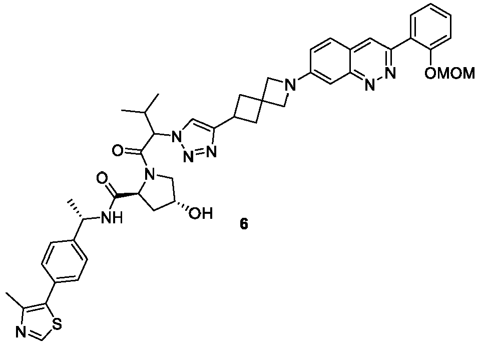

To a stirred mixture of Intermediate **5** (80 mg, 0.151 mmol, 1 equiv) and (2S,4R)-4-hydroxy-N-[(1S)-1-[4-(4-methyl-1,3-thiazol-5-yl)phenyl]ethyl]pyrrolidine-2-carboxamide (50.16 mg, 0.151 mmol, 1 equiv) in DMF (2 mL) were added PyBOP (157.51 mg, 0.302 mmol, 2 equiv) and DIEA (58.68 mg, 0.453 mmol, 3 equiv) in portions at room temperature. The residue was purified by reversed-phase flash chromatography with the following conditions: column, C18 silica gel; mobile phase, MeCN in Water (0.1% NH₃.H₂O), 0% to 100% gradient in 10 min; detector, UV 254 nm. This resulted in Intermediate **6** (40 mg, 31.39%) as a brown solid. LCMS (ESI) m/z: [M+H] ⁺ = 842.

### Step 6: Preparation of (2S,4R)-4-hydroxy-1-[(2S)-2-(4-{2-[3-(2-hydroxyphenyl)cinnolin-7-yl]-2-azaspiro[3.3]heptan-6-yl}-1,2,3-triazol-1-yl)-3-methylbutanoyl]-N-[(1S)-1-[4-(4-methyl-1,3-thiazol-5-yl)phenyl]ethyl]pyrrolidine-2-carboxamide (Compound 186-001)

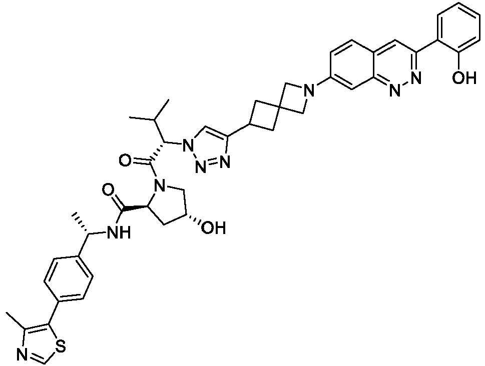

A solution of Intermediate **6** (35 mg, 0.042 mmol, 1 equiv) in TFA (0.3 mL) and DCM (1 mL) was stirred for 1h at room temperature. The crude product was purified by Prep-HPLC with the following conditions to afford **186-001** (13.4 mg, 39.76%) as a yellow solid. 1H NMR (400 MHz, DMSO-d6) δ 12.90 (d, J = 4.7 Hz, 1H), 9.24 - 8.82 (m, 1H), 8.75 (d, J = 5.0 Hz, 1H), 8.41 (dd, J = 80.5, 7.8 Hz, 1H), 8.10 - 7.77 (m, 3H), 7.55 - 7.35 (m, 3H), 7.35 - 7.17 (m, 3H), 7.06 - 6.93 (m, 3H), 5.29 (dd, J = 32.6, 9.7 Hz, 1H), 5.18 (d, J = 4.0 Hz, 1H), 5.02 - 4.82 (m, 1H), 4.43 (dt, J = 27.6, 8.0 Hz, 1H), 4.31 (d, J = 13.5 Hz, 1H), 4.21 (d, J = 18.3 Hz, 2H), 4.10 - 4.00 (m, 2H), 3.84 - 3.36 (m, 3H), 2.73 - 2.58 (m, 2H), 2.45 (d, J = 10.6 Hz, 4H), 2.15 - 2.00 (m, 1H), 1.79 (ddt, J = 13.5, 7.3, 3.2 Hz, 1H), 1.61 - 1.31 (m, 3H), 1.03 (dd, J = 18.6, 6.6 Hz, 3H), 0.74 - 0.59 (m, 3H).LCMS (ESI) m/z: [M+H]⁺ = 797.35.

The compound in Table 14 was prepared using procedures similar to those used above for the preparation of **Compound 186-001.**

**Table 14.**

| **No.** | **Name** | **LCMS (ESI) m/z** | **¹H NMR** |
|---|---|---|---|
| 204 | (2S,4R)-4-hydroxy-1-((S)-2-(4-(2-(3-(2-hydroxyphenyl)cinnolin-6-yl)-2-azaspiro[3.3]heptan-6-yl)-1H-1,2,3-triazol-1-yl)-3-methylbutanoyl)-N-((S)-1-(4-(4-methylthiazol-5-yl)phenyl)ethyl)pyrrolidine-2-carboxamide | 798.2 | 1H NMR (300 MHz, DMSO-d6) δ 13.41 (s, 1H), 9.01 - 8.87 (m, 1H), 8.56 - 8.47 (m, 2H), 8.18 (dd, J = 9.3, 4.8 Hz, 1H), 8.10 - 7.94 (m, 2H), 7.53 - 7.18 (m, 6H), 7.05 - 6.94 (m, 2H), 6.53 (dd, J = 7.2, 2.3 Hz, 1H), 5.38 - 5.15 (m, 2H), 5.13 - 4.84 (m, 1H), 4.52 - 4.19 (m, 4H), 4.13 - 4.03 (m, 2H), 3.84 - 3.63 (m, 2H), 3.60 - 3.45 (m, 1H), 2.75 - 2.61 (m, 2H), 2.50 - 2.42 (m, 5H), 2.14 - 2.01 (m, 2H), 1.87 - 1.73 (m, 1H), 1.45 - 1.32 (m, 3H), 1.11 - 0.84 (m, 3H), 0.68 (d, J = 6.5 Hz, 3H). |

### Preparation of (2S,4R)-1-[(2R)-2-(3-{4-[(4R)-3,3-difluoro-1-{[3-(2-hydroxyphenyl)-6-methoxycinnolin-7-yl]methyl}piperidin-4-yl]piperazin-1-yl}-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide (Compound 218)

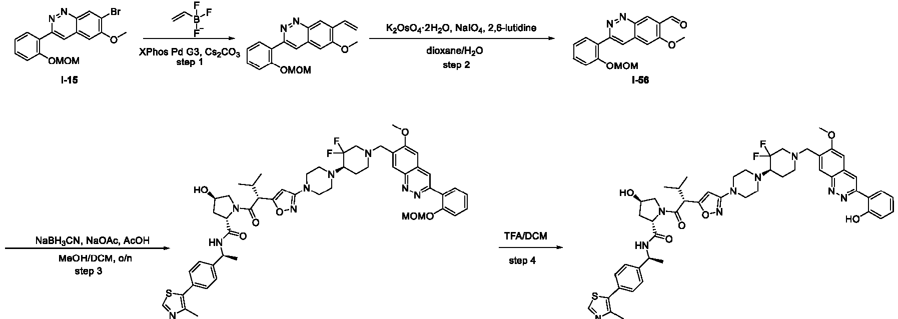

### Step 1: Preparation of 7-ethenyl-6-methoxy-3-[2-(methoxymethoxy)phenyl]cinnoline

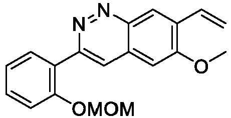

To a solution of **I-15** (500.0 mg, 1.333 mmol, 1 equiv) and (ethenyldifluoro-lambda4-boranyl)-lambda2-fluoranide (379.18 mg, 3.999 mmol, 3.0 equiv) in dioxane (20 mL) and H₂O (5 mL) were added Cs₂CO₃ (1302.50 mg, 3.999 mmol, 3.0 equiv) and XPhos Pd G3 (225.59 mg, 0.267 mmol, 0.2 equiv). After stirring for 2 h at 80 °C under a nitrogen atmosphere. the mixture was concentrated under vacuum, the residue was purified by silica gel column chromatography, eluted with PE / EA (5:1) to afford title compound (400.0 mg, 93.1%) as a yellow solid. LCMS (ESI) m/z [M+H]⁺ =323.

### Step 2: Preparation of 6-methoxy-3-[2-(methoxymethoxy)phenyl]cinnoline-7-carbaldehyde (I-56)

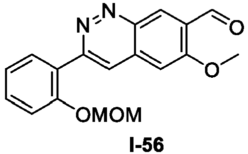

A solution of above intermediate (400.0 mg, 1.241 mmol, 1 equiv) and K₂OsO₄.2H₂O (22.8 mg, 0.062 mmol, 0.05 equiv) in dioxane (10 mL) was stirred for 2 h at room temperature. The resulting mixture was diluted with water (50 mL), extracted with EtOAc (3 x 50 mL). The combined organic layers were washed with brine (3 x 100 mL), dried over anhydrous sodium sulfate. After filtration, the filtrate was concentrated under reduced pressure. The residue was purified by silica gel column chromatography, eluted with PE / EA (5:1) to afford **I-56** (100.0 mg, 24.8%) as a yellow solid. LCMS (ESI) m/z [M+H]⁺ =325.

### Step 3: Preparation of (2S,4R)-1-[(2R)-2-(3-{4-[(4R)-3,3-difluoro-1-({6-methoxy-3-[2-(methoxymethoxy)phenyl]cinnolin-7-yl}methyl)piperidin-4-yl]piperazin-1-yl}-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide

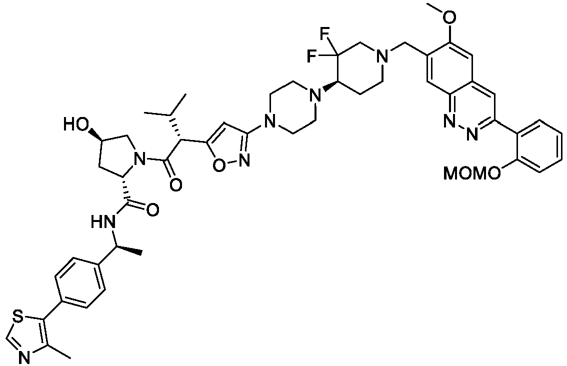

To a solution of **I-56** (18.0 mg, 0.055 mmol, 1 equiv) and (2S,4R)-1-[(2R)-2-(3-{4-[(4R)-3,3-difluoropiperidin-4-yl]piperazin-1-yl}-1,2-oxazol-5-yl)-3-methylbutanoyl]-4-hydroxy-N-[(1S)-1-[4-(4-methyl-1,3-thiazol-5-yl)phenyl]ethyl]pyrrolidine-2-carboxamide (57.1 mg, 0.083 mmol, 1.5 equiv) and AcOH (66.6 mg, 1.100 mmol, 20 equiv) in DMF (0.5 mL) was stirred for 2 h at room temperature. To the above mixture was added NaBH₃CN (10.46 mg, 0.165 mmol, 3.0 equiv) in portions at room temperature. The resulting mixture was stirred for additional 2 h at room temperature. The residue was purified by reversed-phase flash chromatography with the following conditions: column, C₁₈ silica gel; mobile phase, MeCN in Water (0.1% FA), 0% to 50% gradient in 10 min; detector, UV 254 nm to afford the title compound (22.0 mg, 39.8%) as a yellow solid. LCMS (ESI) m/z [M+H]⁺ =994.

### Step 4: Preparation of (2S,4R)-1-[(2R)-2-(3-{4-[(4R)-3,3-difluoro-1-{[3-(2-hydroxyphenyl)-6-methoxycinnolin-7-yl]methyl}piperidin-4-yl]piperazin-1-yl}-1,2-oxazol-5-yl)-3-methylbutanoyl]4-hydroxy-N-[(1S)-1-[4-(4-methyl-1,3-thiazol-5-yl)phenyl]ethyl]pyrrolidine-2-carboxamide (Compound 218)

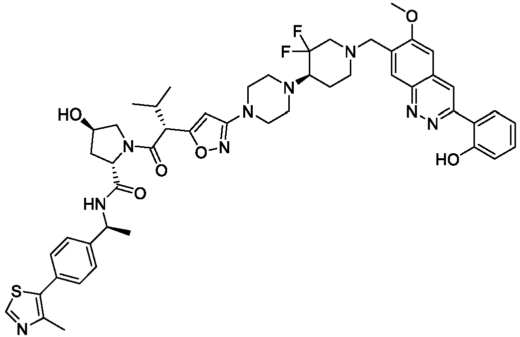

A solution of above intermediate (22 mg, 0.022 mmol, 1 equiv) and HCl (22.00 mg, 0.600 mmol, 27.27 equiv) in CH₃OH (1.0 mL) was stirred for 2 h at 60 °C. The crude product (22 mg) was purified by Prep-HPLC with the following conditions (Column: XBridge Prep OBD RP₁₈ Column, 30*150 mm, 5µm; Mobile Phase A: Water (10mmol/L NH₄HCO₃ ⁺ 0.05% NH₃H₂O), Mobile Phase B: ACN; Flow rate: 60 mL/min mL/min; Gradient: 45% B to 63% B in 8 min; Wave Length: 254nm/220nm nm; RT1(min): 10.05) to afford Compound **218** (9.4 mg, 44.7%) as a white solid. ¹H NMR (400 MHz, DMSO-d6) δ 12.45 (brs, 1H), 8.98 (s, 1H), 8.80 (s, 1H), 8.44 - 8.33 (m, 2H), 8.12 - 8.04 (m, 1H), 7.48 - 7.32 (m, 6H), 7.09 - 6.98 (m, 2H), 6.14 (s, 1H), 5.10 (d, J = 3.8 Hz, 1H), 4.97 - 4.86 (m, 1H), 4.37 (t, J = 7.8 Hz, 1H), 4.31 - 4.25 (m, 1H), 4.02 (s, 3H), 3.83 (s, 2H), 3.76 - 3.67 (m, 1H), 3.58 (d, J = 10.0 Hz, 1H), 3.47 - 3.41 (m, 1H), 3.18 - 3.12 (m, 5H), 3.10 - 3.04 (m, 1H), 3.00 - 2.76 (m, 5H), 2.46 (s, 3H), 2.43 - 2.11 (m, 3H), 2.10 - 1.88 (m, 2H), 1.86 - 1.76 (m, 2H), 1.42 (dd, J = 22.8, 7.1 Hz, 3H), 0.95 (d, J = 6.5 Hz, 3H), 0.80 (d, J = 6.7 Hz, 3H). LCMS (ESI) m/z [M+H]⁺ =950.45.

The compounds in Table 15 were prepared using procedures similar to those used above for the preparation of **Compound 218.**

**Table 15.**

| **No.** | **Name** | **LCMS (ESI) m/z** | **¹H NMR** |
|---|---|---|---|
| 256 | (2S,4R)-4-hydroxy-1-((R)-2-(3-(4-(6-((3-(2-hydroxyphenyl)cinnolin-6-yl)methyl)-2,6-diazaspiro[3.3]heptan-2-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 896.35 | 1H NMR (300 MHz, DMSO-d6) δ 12.06 (s, 1H), 8.99 (s, 1H), 8.91 (s, 1H), 8.85 - 8.27 (m, 2H), 8.23 - 8.03 (m, 1H), 7.96 (s, 1H), 7.87 - 7.77 (m, 1H), 7.65 - 7.19 (m, 5H), 7.11 - 6.99 (m, 2H), 6.06 (d, J = 38.5 Hz, 1H), 5.11 (s, 1H), 4.91 (p, J = 7.0 Hz, 1H), 4.51 (dt, J = 92.6, 7.6 Hz, 1H), 4.28 (s, 1H), 3.80 (s, 2H), 3.71 (dd, J = 10.4, 4.4 Hz, 1H), 3.54 (t, J = 10.7 Hz, 3H), 3.43 - 3.34 (m, 5H), 3.29 (s, 3H), 2.79 (t, J = 10.9 Hz, 2H), 2.46 (d, J = 1.7 Hz, 3H), 2.33 - 1.88 (m, 4H), 1.79 (ddd, J = 12.6, 7.9, 4.8 Hz, 1H), 1.66 (d, J = 12.1 Hz, 2H), 1.41 (dd, J = 20.7, 7.0 Hz, 3H), 1.17 (d, J = 10.9 Hz, 2H), 0.95 (d, J = 6.3 Hz, 3H), 0.80 (dd, J = 12.2, 6.6 Hz, 3H). |
| 245 | (2S,4R)-4-hydroxy-1-((R)-2-(3-(6-((3-(2-hydroxyphenyl)cinnolin-6-yl)methyl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 813.35 | 1H NMR (400 MHz, DMSO-d6) δ 12.06 (d, J = 6.2 Hz, 1H), 8.98 (s, 1H), 8.90 (s, 1H), 8.41 (d, J = 8.4 Hz, 2H), 8.11 (dd, J = 7.9, 1.7 Hz, 1H), 7.97 (s, 1H), 7.84 (dd, J = 8.9, 1.7 Hz, 1H), 7.44 (d, J = 8.2 Hz, 2H), 7.37 (d, J = 7.9 Hz, 3H), 7.10 - 7.00 (m, 2H), 5.81 (d, J = 39.9 Hz, 1H), 5.11 (d, J = 3.6 Hz, 1H), 4.91 (p, J = 7.2 Hz, 1H), 4.36 (t, J = 7.9 Hz, 1H), 4.28 (d, J = 5.5 Hz, 1H), 3.97 (s, 4H), 3.81 (s, 2H), 3.70 (dd, J = 10.6, 4.5 Hz, 1H), 3.58 (d, J = 9.8 Hz, 2H), 3.43 (s, 3H), 2.46 (s, 3H), 2.26 - 1.73 (m, 4H), 1.42 (dd, J = 27.9, 7.0 Hz, 3H), 0.95 (t, J = 6.4 Hz, 3H), 0.80 (dd, J = 14.5, 6.6 Hz, 3H). |
| 244 | (2S,4R)-4-hydroxy-1-((R)-2-(3-(4-(6-((3-(2-hydroxyphenyl)cinnolin-7-yl)methyl)-2,6-diazaspiro[3.3]heptan-2-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 896.4 | 1H NMR (400 MHz, DMSO-d6) δ 12.02 (s, 1H), 8.94 (d, J = 35.4 Hz, 2H), 8.42 - 8.25 (m, 2H), 8.16 - 8.03 (m, 2H), 7.86 - 7.75 (m, 1H), 7.51 - 7.32 (m, 5H), 7.12 - 7.00 (m, 2H), 6.04 (d, J = 53.3 Hz, 1H), 5.10 (s, 1H), 4.91 (p, J = 7.1 Hz, 1H), 4.69 - 4.23 (m, 3H), 3.81 (s, 2H), 3.71 (dd, J = 10.5, 4.6 Hz, 1H), 3.57 - 3.41 (m, 5H), 3.20 (s, 4H), 2.79 (t, J = 10.9 Hz, 2H), 2.45 (s, 3H), 2.32 - 1.87 (m, 5H), 1.78 (ddd, J = 12.7, 7.8, 4.7 Hz, 1H), 1.63 (d, J = 12.0 Hz, 2H), 1.45 - 1.33 (m, 3H), 1.15 (d, J = 10.6 Hz, 2H), 0.97 - 0.74 (m, 6H). |
| 232 | (2S,4R)-4-hydroxy-1-((R)-2-(3-(6-((3-(2-hydroxyphenyl)cinnolin-7-yl)methyl)-2,6-diazaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 813.3 | 1H NMR (400 MHz, DMSO-d6) δ 12.01 (s, 1H), 9.11 - 8.75 (m, 2H), 8.46 - 8.22 (m, 2H), 8.18 - 8.04 (m, 2H), 7.85-7.78 (m, 1H), 7.52 - 7.25 (m, 5H), 7.10 - 7.01 (m, 2H), 5.97 - 5.73 (m, 1H), 5.21 - 4.79 (m, 2H), 4.53 - 4.18 (m, 2H), 4.02 - 3.89 (m, 4H), 3.83 (d, J = 6.2 Hz, 2H), 3.70 (d, J = 8.6 Hz, 1H), 3.58 (d, J = 9.8 Hz, 5H), 2.47-2.42 (m, 3H), 2.31 - 1.87 (m, 3H), 1.84-1.72 (m, 1H), 1.49 - 1.33 (m, 3H), 0.98-0.92 (m, 2H), 0.88 - 0.66 (m, 4H). |
| 219 | (2S,4R)-1-((R)-2-(3-(4-((S)-3,3-difluoro-1-((3-(2-hydroxyphenyl)-6-methoxycinnolin-7-yl)methyl)piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 950.4 | 1H NMR (300 MHz, DMSO-d6) δ 12.45 (brs, 1H), 8.98 (s, 1H), 8.80 (s, 1H), 8.39 (d, J = 16.5 Hz, 2H), 8.08 (d, J = 7.9 Hz, 1H), 7.40 (dd, J = 21.0, 7.5 Hz, 6H), 7.05 (d, J = 7.8 Hz, 2H), 6.14 (s, 1H), 5.10 (d, J = 3.5 Hz, 1H), 4.91 (t, J = 6.9 Hz, 1H), 4.37 (t, J = 7.6 Hz, 1H), 4.31 - 4.25 (m, 1H), 4.02 (s, 3H), 3.86 (d, J = 17.0 Hz, 2H), 3.76 - 3.67 (m, 1H), 3.58 (d, J = 9.8 Hz, 1H), 3.44 (s, 1H), 3.18 - 3.12 (m, 5H), 3.10 - 3.01 (m, 1H), 2.88 - 2.82 (m, 5H), 2.46 (s, 3H), 2.43 - 2.08 (m, 3H), 2.10 - 1.86 (m, 2H), 1.87 - 1.71 (m, 2H), 1.42 (dd, J = 23.1, 6.9 Hz, 3H), 0.95 (d, J = 6.4 Hz, 3H), 0.79 (d, J = 6.6 Hz, 3H). |
| 216 | (2R,4S)-1-((R)-2-(3-(4-((S)-1-((6-chloro-3-(2-hydroxyphenyl)cinnolin-7-yl)methyl)-3,3-difluoropiperidin-4-yl)piperazin-1-yl)isoxazol-5-yl)-3-methylbutanoyl)-4-hydroxy-N-((R)-1-(4-(4-methylthiazol-5-yl)phenyl)ethyl)pyrrolidine-2-carboxamide | 954.45 | 1H NMR (400 MHz, DMSO-d6) δ 11.50 (s, 1H), 8.98 (s, 1H), 8.83 (s, 1H), 8.54 (s, 1H), 8.41 (d, J = 7.7 Hz, 1H), 8.32 (s, 1H), 8.08 (dd, J = 7.9, 1.7 Hz, 1H), 7.53 - 7.29 (m, 5H), 7.12 - 6.98 (m, 2H), 6.08 (d, J = 48.0 Hz, 1H), 5.16 - 4.99 (m, 1H), 4.92 (p, J = 7.0 Hz, 1H), 4.70 - 4.33 (m, 1H), 4.29 (s, 1H), 3.92 (s, 2H), 3.72 (dd, J = 10.6, 4.5 Hz, 1H), 3.58 (d, J = 10.0 Hz, 1H), 3.44 (t, J = 9.3 Hz, 1H), 3.16 (t, J = 5.2 Hz, 5H), 3.08 - 2.77 (m, 6H), 2.68 - 2.53 (m, 1H), 2.48 - 2.32 (m, 4H), 2.30 - 2.13 (m, 1H), 2.09 - 1.88 (m, 2H), 1.86 - 1.73 (m, 2H), 1.42 (dd, J = 31.1, 7.0 Hz, 3H), 0.97 (t, J = 6.7 Hz, 3H), 0.82 (dd, J = 15.4, 6.7 Hz, 3H). |
| 215 | (2R,4S)-1-((R)-2-(3-(4-((R)-1-((6-chloro-3-(2-hydroxyphenyl)cinnolin-7-yl)methyl)-3,3-difluoropiperidin-4-yl)piperazin-1-yl)isoxazol-5-yl)-3-methylbutanoyl)-4-hydroxy-N-((R)-1-(4-(4-methylthiazol-5-yl)phenyl)ethyl)pyrrolidine-2-carboxamide | 954.35 | 1H NMR (400 MHz, DMSO-d6) δ 11.50 (s, 1H), 8.98 (s, 1H), 8.83 (s, 1H), 8.54 (s, 1H), 8.41 (d, J = 7.7 Hz, 1H), 8.32 (s, 1H), 8.08 (dd, J = 7.9, 1.7 Hz, 1H), 7.51 - 7.33 (m, 5H), 7.13 - 7.01 (m, 2H), 6.08 (d, J = 48.1 Hz, 1H), 5.14 - 4.99 (m, 1H), 4.92 (p, J = 6.9 Hz, 1H), 4.52 (dt, J = 119.7, 7.7 Hz, 1H), 4.29 (s, 1H), 3.92 (s, 2H), 3.72 (dd, J = 10.5, 4.5 Hz, 1H), 3.58 (d, J = 9.9 Hz, 1H), 3.43 (d, J = 10.2 Hz, 1H), 3.22 - 3.11 (m, 5H), 3.11 - 2.79 (m, 6H), 2.66 - 2.54 (m, 1H), 2.46 (s, 4H), 2.32 - 2.15 (m, 1H), 2.07 - 1.87 (m, 2H), 1.84 - 1.74 (m, 2H), 1.42 (dd, J = 30.6, 7.0 Hz, 3H), 0.96 (t, J = 6.7 Hz, 3H), 0.82 (dd, J = 15.3, 6.7 Hz, 3H). |
| 140 | (2S,4R)-1-((R)-2-(3-(4-((S)-3,3-difluoro-1-((3-(2-hydroxyphenyl)cinnolin-7-yl)methyl) piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 920.35 | 1H NMR (400 MHz, DMSO-d6) δ 11.96 - 11.91 (m, 1H), 8.99 (s, 1H), 8.91 (s, 1H), 8.41 (d, J = 7.7 Hz, 1H), 8.36 (s, 1H), 8.15 - 8.09 (m, 2H), 7.91 - 7.84 (m, 1H), 7.49 - 7.33 (m, 5H), 7.10 - 7.01 (m, 2H), 6.13 (s, 1H), 5.11 (d, J = 3.7 Hz, 1H), 4.97 - 4.85 (m, 1H), 4.40 - 4.32 (m, 1H), 4.30 - 4.26 (m, 1H), 3.89 (s, 2H), 3.75 - 3.67 (m, 1H), 3.57 (d, J = 10.0 Hz, 1H), 3.46 - 3.41 (m, 2H), 3.16 - 3.11 (m, 5H), 3.05 - 2.94 (m, 1H), 2.84 - 2.80 (m, 4H), 2.46 (s, 3H), 2.32 - 2.15 (m, 2H), 2.06 - 1.74 (m, 5H), 1.48 - 1.34 (m, 3H), 1.00 - 0.92 (m, 3H), 0.86 - 0.76 (m, 3H). |
| 139 | (2S,4R)-1-((R)-2-(3-(4-((R)-3,3-difluoro-1-((3-(2-hydroxyphenyl)cinnolin-7-yl)methyl) piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 920.35 | 1H NMR (300 MHz, DMSO-d6) δ 11.93 (s, 1H), 8.98 (s, 1H), 8.91 (s, 1H), 8.45 - 8.33 (m, 2H), 8.12 (d, J = 8.5 Hz, 2H), 7.87 (d, J = 8.5 Hz, 1H), 7.48 - 7.32 (m, 5H), 7.05 (t, J = 8.2 Hz, 2H), 6.13 (s, 1H), 5.10 (d, J = 3.7 Hz, 1H), 4.91 (t, J = 7.1 Hz, 1H), 4.36 (t, J = 7.7 Hz, 1H), 4.31 - 4.25 (m, 1H), 3.89 (s, 2H), 3.75 - 3.67 (m, 1H), 3.57 (d, J = 9.8 Hz, 1H), 3.53 - 3.31 (m, 3H), 3.16 - 3.10 (m, 5H), 2.99 - 2.93 (m, 1H), 2.85 - 2.79 (m, 4H), 2.45 (s, 3H), 2.41 - 2.11 (m, 2H), 2.10 - 1.71 (m, 5H), 1.49 - 1.33 (m, 3H), 0.95 (d, J = 6.5 Hz, 3H), 0.79 (d, J = 6.7 Hz, 3H). |
| 137 | (2S,4R)-1-((R)-2-(3-(4-((S)-3,3-difluoro-1-((3-(2-hydroxyphenyl)cinnolin-6-yl)methyl) piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 920.4 | 1H NMR (300 MHz, Methanol-d4) δ 8.89 (d, J = 3.6 Hz, 2H), 8.83 (d, J = 0.8 Hz, 1H), 8.13 - 7.95 (m, 3H), 7.51 - 7.33 (m, 5H), 7.12 - 7.01 (m, 2H), 6.09 (d, J = 16.7 Hz, 1H), 5.04 (t, J = 6.9 Hz, 1H), 4.53 (t, J = 8.1 Hz, 1H), 4.45 (s, 1H), 3.86 (dd, J = 11.3, 4.1 Hz, 3H), 3.64 (t, J = 9.0 Hz, 2H), 3.24 (s, 4H), 3.06 (d, J = 12.0 Hz, 2H), 2.95 (s, 5H), 2.49 (d, J = 3.3 Hz, 3H), 2.40 - 2.27 (m, 3H), 2.26 - 1.92 (m, 4H), 1.57 (dd, J = 19.3, 7.0 Hz, 3H), 1.07 (d, J = 6.5 Hz, 3H), 0.91 (d, J = 6.8 Hz, 3H). |
| 136 | (2S,4R)-1-((R)-2-(3-(4-((R)-3,3-difluoro-1-((3-(2-hydroxyphenyl)cinnolin-6-yl)methyl) piperidin-4-yl)piperazin-1-yl)isoxazol-5-yl)-3-methylbutanoyl)-4-hydroxy-N-((S)-1-(4-(4-methylthiazol-5-yl)phenyl)ethyl)pyrrolidine-2-carboxamide | 920.25 | 1H NMR (300 MHz, Methanol-d4) δ 8.86 (d, J = 20.7 Hz, 2H), 8.43 (d, J = 8.8 Hz, 1H), 8.13 - 7.96 (m, 3H), 7.53 - 7.33 (m, 5H), 7.10 - 7.02 (m, 2H), 6.09 (d, J = 16.8 Hz, 1H), 5.05 (d, J = 7.0 Hz, 1H), 4.63 - 4.44 (m, 2H), 3.94 - 3.81 (m, 3H), 3.64 (t, J = 9.0 Hz, 2H), 3.25 (s, 4H), 3.04 (s, 2H), 2.95 (s, 5H), 2.49 (d, J = 4.5 Hz, 3H), 2.44 - 1.84 (m, 7H), 1.64 - 1.50 (m, 3H), 1.07 (d, J = 6.5 Hz, 3H), 0.91 (d, J = 6.8 Hz, 3H). |

### Preparation of Preparation of (2S,4R)-4-hydroxy-1-[(2R)-2-(3-{2-[3-(2-hydroxyphenyl)cinnolin-6-yl]-7-azaspiro[3.5]nonan-7-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 71-001)

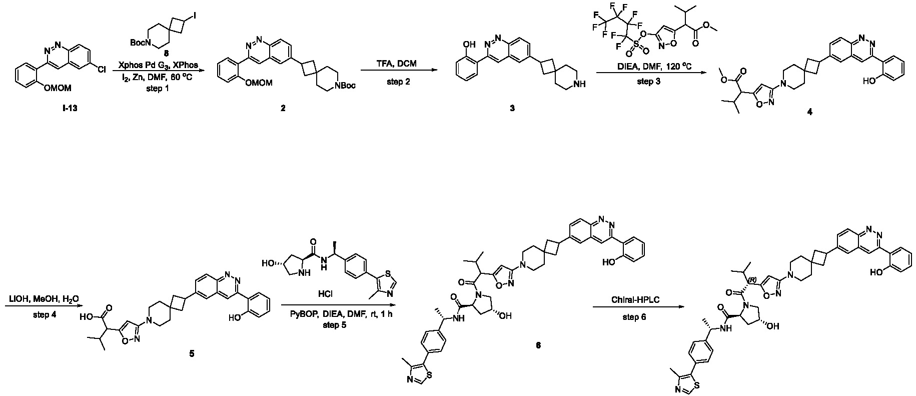

### Step 1: Preparation of tert-butyl 2-{3-[2-(methoxymethoxy)phenyl]cinnolin-6-yl}-7-azaspiro[3.5]nonane-7-carboxylate (Intermediate 2)

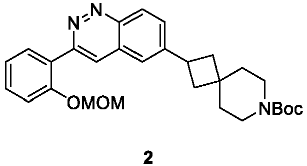

To a stirred mixture of Zn (1.09 g, 16.625 mmol, 5 equiv) and I₂ (843.95 mg, 3.325 mmol, 1 equiv) in DMF (20 mL) was added tert-butyl 2-iodo-7-azaspiro[3.5]nonane-7-carboxylate (2.34 g, 6.650 mmol, 2.00 equiv) in DMF (10 mL) at room temperature under nitrogen atmosphere. The resulting mixture was stirred for 0.5 h at room temperature under nitrogen atmosphere. To the above mixture was added I**-13** (1 g, 3.325 mmol, 1 equiv), XPhos Pd G3 (562.92 mg, 0.665 mmol, 0.2 equiv) and XPhos (634.07 mg, 1.330 mmol, 0.4 equiv) at room temperature. The resulting mixture was stirred for additional 1 h at 60 °C. Desired product could be detected by LCMS. The resulting mixture was diluted with water (300 mL). The resulting mixture was extracted with EtOAc (3 x 300 mL). The combined organic layers were washed with brine (3 x 100 mL), dried over anhydrous Na₂SO₄. After filtration, the filtrate was concentrated under reduced pressure. The residue was purified by silica gel column chromatography, eluted with CH₂Cl₂ / MeOH (9:1) to afford Intermediate **2** (1.01 g, crude) as a yellow oil. LCMS (ESI) m/z: [M+H]⁺ = 490.

### Step 2: Preparation of 2-(6-{7-azaspiro[3.5]nonan-2-yl}cinnolin-3-yl)phenol (Intermediate 3)

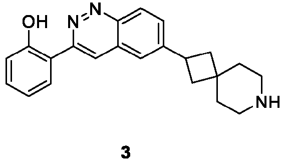

To a stirred solution of Intermediate **2** (1 g, 2.042 mmol, 1 equiv) in DCM (4.5 mL) was added TFA (1.5 mL) dropwise at room temperature. The resulting mixture was stirred for overnight at room temperature. Desired product could be detected by LCMS. The resulting mixture was concentrated under reduced pressure. The residue was purified by reverse flash chromatography with the following conditions: column, C18 silica gel; mobile phase, MeCN in Water, 0% to 100% gradient in 30 min; detector, UV 254 nm to afford Intermediate **3** (190 mg, 26.93%) as a brown solid. LCMS (ESI) m/z [M+H]⁺ = 346.

### Step 3: Preparation of methyl 2-(3-{2-[3-(2-hydroxyphenyl)cinnolin-6-yl]-7-azaspiro[3.5]nonan-7-yl}-1,2-oxazol-5-yl)-3-methylbutanoate (Intermediate 4)

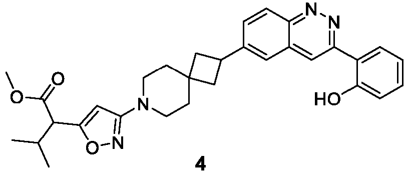

A mixture of Intermediate **3** (250 mg, 0.724 mmol, 1 equiv), methyl 3-methyl-2-{3-[(1,1,2,2,3,3,4,4,4-nonafluorobutanesulfonyl)oxy]-1,2-oxazol-5-yl}butanoate (1044.93 mg, 2.172 mmol, 3 equiv) and DIEA (280.61 mg, 2.172 mmol, 3 equiv) in DMF (5 mL) was stirred for 2 h at 120 °C under nitrogen atmosphere. Desired product could be detected by LCMS. The residue was purified by reversed-phase flash chromatography with the following conditions: column, C18 silica gel; mobile phase, MeCN in Water (0.1% FA), 0% to 100% gradient in 30 min; detector, UV 254 nm to afford Intermediate **4** (80 mg, 20.99%) as a yellow solid. LCMS (ESI) m/z: [M+H]⁺ = 527.

### Step 4: Preparation of 2-(3-{2-[3-(2-hydroxyphenyl)cinnolin-6-yl]-7-azaspiro[3.5]nonan-7-yl}-1,2-oxazol-5-yl)-3-methylbutanoic acid (Intermediate 5)

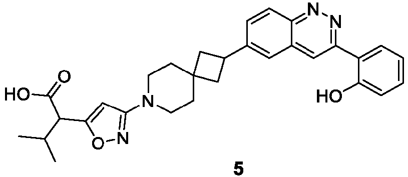

A mixture of Intermediate **4** (70 mg, 0.133 mmol, 1 equiv) and LiOH.H₂O (55.77 mg, 1.330 mmol, 10 equiv) in THF (2 mL), MeOH (2 mL) and H₂O (1 mL) was stirred for 2 h at room temperature. Desired product could be detected by LCMS. The mixture was acidified to pH 6 with conc. HCl. The resulting mixture was extracted with EtOAc (3 x 50 mL). The combined organic layers were washed with brine (3x10 mL), dried over anhydrous Na₂SO₄. After filtration, the filtrate was concentrated under reduced pressure to afford Intermediate **5** (56 mg, 82.19%) as a yellow solid. LCMS (ESI) m/z: [M+H]⁺ = 513.

### Step 5: Preparation of (2S,4R)-4-hydroxy-1-[2-(3-{2-[3-(2-hydroxyphenyl)cinnolin-6-yl]-7-azaspiro[3.5]nonan-7-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Intermediate 6)

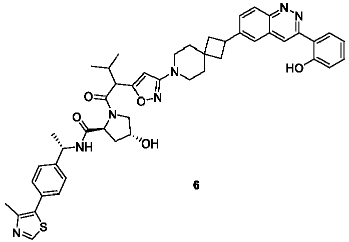

A mixture of Intermediate **5** (51 mg, 0.099 mmol, 1 equiv), (2S,4R)-4-hydroxy-N-[(1S)-1-[4-(4-methyl-1,3-thiazol-5-yl)phenyl]ethyl]pyrrolidine-2-carboxamide (49.46 mg, 0.149 mmol, 1.5 equiv), PyBOP (77.66 mg, 0.149 mmol, 1.5 equiv) and DIEA (51.44 mg, 0.396 mmol, 4 equiv) in DMF (3 mL) was stirred for 2 h at room temperature. Desired product could be detected by LCMS. The crude product was purified by Prep-HPLC with the following conditions (Column: XBridge Shield RP18 OBD Column, 30*150 mm, 5µm; Mobile Phase A: Water (10 mmol/L NH₄HCO₃+0.1%NH₃.H₂O), Mobile Phase B: MeOH--HPLC; Flow rate: 60 mL/min; Gradient: 2% B to 2% B in 1 min, 2% B to 66% B in 1.5 min, 66% B to 83% B in 8.5 min, 83% B; Wave Length: 254/220 nm; RT1(min): 9.33) to afford Intermediate **6** (72 mg, 87.61%) as a yellow solid. LCMS (ESI) m/z: [M+H]⁺ = 826.

### Step 6: Preparation of (2S,4R)-4-hydroxy-1-[(2R)-2-(3-{2-[3-(2-hydroxyphenyl)cinnolin-6-yl]-7-azaspiro[3.5]nonan-7-yl}-1,2-oxazol-5-yl)-3-methylbutanoyl]-N-[(1S)-1-[4-(4-methyl-1,3-thiazol-5-yl)phenyl]ethyl]pyrrolidine-2-carboxamide (Compound 71-001)

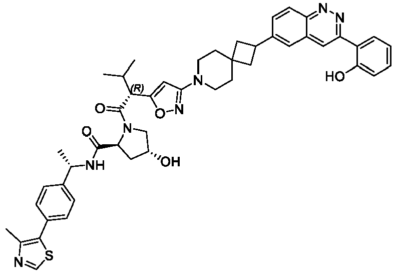

The Intermediate **6** (72 mg) was purified by Chiral-HPLC with the following conditions (Column: CHIRAL ART Cellulose-SB, 2*25 cm, 5 µm; Mobile Phase A: MtBE (10mM NH3-MeOH), Mobile Phase B: MeOH--HPLC; Flow rate: 20 mL/min; Gradient: 15% B to 15% B in 17 min; Wave Length: 268/220 nm; RT1(min): 7.47; RT2(min): 11.695; Sample Solvent: MeOH: DCM=1: 1--HPLC; Injection Volume: 1 mL) to afford **71-001** (14.1 mg, 43.75%) as a light yellow solid. ¹H NMR (400 MHz, DMSO-d6) *δ* 12.14 - 12.08 (m, 1H), 9.01 - 8.96 (m, 1H), 8.88 (s, 1H), 8.40 (t, J = 8.7 Hz, 2H), 8.14 - 8.07 (m, 1H), 7.93 (s, 1H), 7.87 (d, J = 8.8 Hz, 1H), 7.49 - 7.41 (m, 2H), 7.41 - 7.34 (m, 3H), 7.10 - 7.00 (m, 2H), 6.16 (s, 1H), 5.05 (dd, J = 41.5, 3.3 Hz, 1H), 4.98 - 4.63 (m, 1H), 4.37 (t, J = 7.8 Hz, 1H), 4.29 (s, 1H), 3.85 (q, J = 8.9 Hz, 1H), 3.76 - 3.68 (m, 1H), 3.57 (d, J = 9.8 Hz, 1H), 3.46 - 3.39 (m, 1H), 3.28 - 3.23 (m, 2H), 3.14 - 3.10 (m, 2H), 2.46 (s, 3H), 2.42 - 2.36 (m, 2H), 2.30 - 2.19 (m, 1H), 2.09 - 1.99 (m, 3H), 1.85 - 1.81 (m, 3H), 1.63 (s, 2H), 1.42 (dd, J = 31.2, 7.0 Hz, 3H), 1.00 - 0.92 (m, 3H), 0.82 (dd, J = 15.9, 6.8 Hz, 3H). LCMS (ESI) m/z: [M+H]⁺ = 826.35.

The compounds in Table 16 were prepared using procedures similar to those used above for the preparation of **Compound 71-001.**

**Table 16.**

| **No.** | **Name** | **LCMS (ESI) m/z** | **¹H NMR** |
|---|---|---|---|
| 94 | (2S,4R)-1-((R)-2-(3-(2-(3-(3-fluoro-2-hydroxyphenyl)cinnolin-7-yl)-7-azaspiro[3.5]nonan-7-yl)isoxazol-5-yl)-3-methylbutanoyl)-4-hydroxy-N-((S)-1-(4-(4-methylthiazol-5-yl)phenyl)ethyl)pyrrolidine-2-carboxamide | 844.5 | 1H NMR (400 MHz, DMSO-d6) δ 12.76 (s, 1H), 8.99 (s, 2H), 8.41 (d, J = 7.8 Hz, 1H), 8.27 (s, 1H), 8.12 (d, J = 8.7 Hz, 1H), 7.92 (dd, J = 16.1, 8.4 Hz, 2H), 7.44 (d, J = 8.2 Hz, 2H), 7.37 (d, J = 8.2 Hz, 3H), 7.09 - 6.99 (m, 1H), 6.17 (s, 1H), 5.11 (d, J = 3.7 Hz, 1H), 4.96 - 4.88 (m, 1H), 4.41 - 4.33 (m, 1H), 4.29 (s, 1H), 3.94 - 3.85 (m, 1H), 3.71 (s, 1H), 3.57 (d, J = 9.7 Hz, 1H),3.4 (s, 1H), 3.25 (s, 2H), 3.13 (s, 2H), 2.46 (s, 3H), 2.42 (s, 1H), 2.25 (s, 2H), 2.12 - 2.02 (m, 3H), 1.84 (s, 3H), 1.65 (s, 2H), 1.38 (d, J = 7.0 Hz, 3H), 0.96 (d, J = 6.6 Hz, 3H), 0.82 (dd, J = 16.1, 6.8 Hz, 3H). |
| 58 | (2S,4R)-4-hydroxy-1-((R)-2-(3-((S)-3-(3-(2-hydroxyphenyl)cinnolin-7-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 772.3 | 1H NMR (400 MHz, DMSO-d6) δ 11.99 (d, J = 10.8 Hz, 1H), 8.99 (s, 1H), 8.92 (s, 1H), 8.42 (d, J = 7.7 Hz, 1H), 8.36 (s, 1H), 8.12 (d, J = 8.3 Hz, 2H), 7.95 (dd, J = 8.6, 1.8 Hz, 1H), 7.51 - 7.30 (m, 5H), 7.10 - 6.98 (m, 2H), 6.03 (s, 1H), 5.13 (d, J = 3.6 Hz, 1H), 4.91 (t, J = 7.1 Hz, 1H), 4.38 (t, J = 7.9 Hz, 1H), 4.30 (s, 1H), 3.98 - 3.80 (m, 2H), 3.74 (dd, J = 10.5, 4.4 Hz, 1H), 3.65 - 3.51 (m, 2H), 3.50 - 3.39 (m, 3H), 2.46 (s, 3H), 2.31 - 2.07 (m, 3H), 2.06 - 1.89 (m, 1H), 1.88 - 1.70 (m, 1H), 1.41 (d, J = 7.0, 7.1 Hz, 3H), 0.98 (d, J = 7.4 Hz, 3H), 0.85 (dd, J = 14.9, 6.9 Hz, 3H). |
| 252 | (2S,4R)-1-((R)-2-(3-(6-(6-cyclopropyl-3-(2-hydroxyphenyl)cinnolin-7-yl)-2-azaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 838.3 | 1H NMR (300 MHz, Methanol-d4) δ 8.87 (s, 1H), 8.68 (s, 1H), 8.17 (s, 1H), 8.06 - 7.97 (m, 1H), 7.62 (s, 1H), 7.50 - 7.30 (m, 5H), 7.08 - 6.97 (m, 2H), 5.88 (d, J = 14.7 Hz, 1H), 5.10 - 4.99 (m, 1H), 4.58 - 4.39 (m, 2H), 4.19 (s, 2H), 4.10 - 3.98 (m, 1H), 3.93 (s, 2H), 3.90 - 3.79 (m, 1H), 3.69 - 3.53 (m, 2H), 2.88 (t, J = 10.1 Hz, 2H), 2.59 (t, J = 10.8 Hz, 2H), 2.48 (s, 3H), 2.42 - 2.31 (m, 1H), 2.20 - 2.14 (m, 2H), 2.06 - 1.86 (m, 1H), 1.64 - 1.48 (m, 3H), 1.23 - 1.10 (m, 2H), 1.05 (d, J = 6.5 Hz, 3H), 0.92 - 0.90 (m, 5H). |
| 227 | (2S,4R)-1-((R)-2-(3-(6-(6-cyano-3-(2-hydroxyphenyl)cinnolin-7-yl)-2-azaspiro[3.3]heptan-2-yl)isoxazol-5-yl)-3-methylbutanoyl)-N-((S)-1-(4-fluorophenyl)ethyl)-4-hydroxypyrrolidine-2-carboxamide | 744.25 | 1H NMR (400 MHz, Methanol-d4) δ 8.93 (d, J = 2.8 Hz, 1H), 8.62 (s, 1H), 8.44 (s, 1H), 8.07 - 8.01 (m, 1H), 7.70 - 7.29 (m, 2H), 7.36 - 7.25 (m, 2H), 7.11 - 6.98 (m, 4H), 5.93 - 5.79 (m, 1H), 4.97 (dd, J = 12.2, 6.7 Hz, 1H), 4.58 - 4.44 (m, 2H), 4.18 (d, J = 4.0 Hz, 2H), 3.98 (d, J = 8.8 Hz, 1H), 3.94 (s, 2H), 3.84 (dd, J = 10.8, 4.2 Hz,1H), 3.76 - 3.56 (m, 2H), 2.93 (t, J = 10.6 Hz, 2H), 2.65 (t, J = 10.4 Hz, 2H), 2.37 (s, 1H), 2.20 - 2.10 (m, 1H), 1.98 - 1.87 (m, 1H), 1.47 (t, J = 7.6 Hz, 3H), 1.00 (dd, J = 38.6, 6.6 Hz, 3H), 0.94 - 0.82 (m, 3H), |
| 222 | (2S,4R)-1-((R)-2-(3-(6-(6-cyano-3-(2-hydroxyphenyl)cinnolin-7-yl)-2-azaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 823.25 | 1H NMR (400 MHz, DMSO-d6) 11.85 - 10.42 (m,1H), 8.97 (s, 1H), 8.93 (d, J = 8.6 Hz, 1H), 8.82 (s, 1H), 8.49 (s, 1H), 8.41 (d, J = 7.6 Hz, 1H), 8.16 - 8.09 (m, 1H), 7.51 - 7.42 (m, 1H), 7.37 (d, J = 7.9 Hz, 3H), 7.11 - 7.01 (m, 2H), 5.88 (s, 1H), 5.11 (d, J = 3.5 Hz, 1H), 5.02 - 4.88 (m, 1H), 4.87 - 4.70 (m, 1H), 4.38 (t, J = 7.8 Hz, 1H), 4.29 (s, 1H), 4.09 (s, 1H), 3.95 - 3.80 (m, 1H), 3.86 (s, 4H), 3.75 - 3.67 (m, 1H), 3.60 (d, J = 9.7 Hz, 1H), 2.89 - 2.80 (m, 3H), 2.49 - 2.43 (m, 4H), 2.24 - 2.19 (m, 1H), 1.84 - 1.74 (m, 1H), 1.46 (d, J = 6.9 Hz, 1H), 1.39 (d, J = 7.0 Hz, 3H), 1.00 - 0.92 (m, 3H), 0.89 - 0.77 (m, 3H). |
| 220 | (2S,4R)-1-((R)-2-(3-(6-(6-chloro-3-(2-hydroxyphenyl)cinnolin-7-yl)-2-azaspiro[3.3]heptan-2-yl)isoxazol-5-yl)-3-methylbutanoyl)-4-hydroxy-N-((S)-1-(4-(4-methylthiazol-5-yl)phenyl)ethyl)pyrrolidine-2-carboxamide | 832.25 | 1H NMR (300 MHz, DMSO-d6) δ 11.55 (s, 1H), 8.99 (s, 1H), 8.82 (s, 1H), 8.41 (d, J = 9.1 Hz, 2H), 8.29 (s, 1H), 8.18 - 8.00 (m, 1H), 7.54 - 7.31 (m, 5H), 7.07 (d, J = 8.6 Hz, 2H), 5.88 (s, 1H), 5.01 (dd, J = 60.6, 5.6 Hz, 2H), 4.49 - 4.20 (m, 2H), 4.09 (s, 2H), 3.97 - 3.65 (m, 4H), 3.60 (d, J = 9.7 Hz, 1H), 3.43 (d, J = 10.9 Hz, 1H), 2.80 (s, 2H), 2.73 (s, 2H), 2.46 (s, 3H), 2.24 (d, J = 17.3 Hz, 1H), 2.02 (s, 1H), 1.79 (s, 1H), 1.42 (dd, J = 23.5, 7.0 Hz, 3H), 0.95 (d, J = 6.3 Hz, 3H), 0.80 (d, J = 6.8 Hz, 3H). |
| 118 | (2S,4R)-4-hydroxy-1-((R)-2-(3-((R)-3-(3-(2-hydroxyphenyl)cinnolin-7-yl)pyrrolidin-1-yl)isoxazol-5-yl)-3-methylbutanoyl)-N-((S)-1-phenylethyl)pyrrolidine-2-carboxamide | 675.3 | 1H NMR (400 MHz, DMSO-d6) δ 11.99 (s, 1H), 9.02 - 8.67 (m, 1H), 8.39 - 8.33 (m, 2H), 8.16 - 8.09 (m, 2H), 7.98 - 7.91 (m, 1H), 7.41 - 7.15 (m, 6H), 7.10 - 7.00 (m, 2H), 6.08 - 5.88 (m, 1H), 5.16 - 4.97 (m, 1H), 4.94 - 4.63 (m, 1H), 4.41 - 4.33 (m, 1H), 4.28 (s, 1H), 3.86 - 3.81 (m, 2H), 3.77 - 3.69 (m, 1H), 3.63 - 3.48 (m, 2H), 3.45 - 3.40 (m, 3H), 2.49 - 2.46 (m, 1H), 2.34 - 2.19 (m, 2H), 2.05 - 1.95 (m, 1H), 1.82 - 1.71 (m, 1H), 1.47 - 1.31 (m, 3H), 1.02 - 0.79 (m, 6H). |
| 116 | (2S,4R)-4-hydroxy-1-((R)-2-(3-((S)-3-(3-(2-hydroxyphenyl)cinnolin-7-yl)pyrrolidin-1-yl)isoxazol-5-yl)-3-methylbutanoyl)-N-((S)-1-phenylethyl)pyrrolidine-2-carboxamide | 675.4 | 1H NMR (400 MHz, DMSO-d6) δ 12.00 (d, J = 11.6 Hz, 1H), 8.91 (s, 1H), 8.35 (d, J = 7.2 Hz, 2H), 8.16 - 8.09 (m, 2H), 7.99 - 7.91 (m, 1H), 7.42 - 7.33 (m, 1H), 7.36 - 7.23 (m, 4H), 7.27 - 7.16 (m, 1H), 7.10 - 7.00 (m, 2H), 6.03 (s, 1H), 5.12 (d, J = 3.6 Hz, 1H), 4.95 - 4.82 (m, 1H), 4.37 (t, J = 7.8 Hz, 1H), 4.31 - 4.25 (m, 1H), 3.90 - 3.77 (m, 2H), 3.77 - 3.69 (m, 1H), 3.63 - 3.50 (m, 2H), 3.50 - 3.37 (m, 3H), 2.32 - 2.14 (m, 2H), 2.05 - 1.95 (m, 1H), 1.82 - 1.71 (m, 1H), 1.35 (d, J = 7.0 Hz, 3H), 1.23 (s, 1H), 0.97 (t, J = 6.5 Hz, 3H), 0.89 - 0.79 (m, 3H). |
| 101 | (2S,4R)-4-hydroxy-1-((R)-2-(3-((S)-3-(3-(2-hydroxyphenyl)cinnolin-6-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 786.35 | 1H NMR (400 MHz, DMSO-d6) δ 11.93 (s, 1H), 8.98 (d, J = 4.7 Hz, 1H), 8.91 - 8.78 (m, 1H), 8.43 (dd, J = 14.2, 8.2 Hz, 2H), 8.18 - 8.06 (m, 1H), 8.06 - 7.92 (m, 2H), 7.66 - 7.14 (m, 5H), 7.12 - 6.89 (m, 2H), 6.18 (d, J = 64.1 Hz, 1H), 5.21 - 5.03 (m, 1H), 4.91 (p, J = 7.1 Hz, 1H), 4.37 (t, J = 7.9 Hz, 1H), 4.29 (s, 1H), 3.86 (t, J = 11.9 Hz, 1H), 3.82 - 3.66 (m, 2H), 3.58 (d, J = 10.0 Hz, 1H), 3.45 (t, J = 10.5 Hz, 1H), 3.18 - 2.97 (m, 2H), 2.97 - 2.85 (m, 1H), 2.44 (d, J = 12.0 Hz, 3H), 2.29 - 2.19 (m, 1H), 2.04 (dd, J = 24.4, 11.6 Hz, 2H), 1.77 (dq, J = 27.6, 12.5, 11.6 Hz, 4H), 1.40 (dd, J = 21.4, 7.0 Hz, 3H), 0.95 (d, J = 6.4 Hz, 3H), 0.82 (dd, J = 14.3, 6.6 Hz, 3H). |
| 96 | (2S,4R)-4-hydroxy-1-((R)-2-(3-(9-(3-(2-hydroxyphenyl)cinnolin-7-yl)-3-azaspiro[5.5]undecan-3-yl)isoxazol-5-yl)-3-methylbutanoyl)-N-((S)-1-(4-(4-methylthiazol-5-yl)phenyl)ethyl)pyrrolidine-2-carboxamide | 854.35 | 1H NMR (400 MHz, DMSO-d6) δ 12.13 (s, 1H), 9.08 - 8.93 (m, 1H), 8.90 (s, 1H), 8.48 - 8.17 (m, 2H), 8.09 (ddd, J = 19.9, 8.4, 2.1 Hz, 2H), 7.90 (dd, J = 13.6, 8.0 Hz, 1H), 7.57 - 7.22 (m, 5H), 7.14 - 6.95 (m, 2H), 6.16 (dd, J = 9.3, 4.6 Hz, 1H), 5.25 - 4.72 (m, 2H), 4.53 - 4.33 (m, 1H), 4.29 (s, 1H), 3.71 (q, J = 6.9, 4.7 Hz, 1H), 3.57 (dd, J = 9.8, 4.7 Hz, 1H), 3.43 (t, J = 7.9 Hz, 1H), 3.31 - 2.97 (m, 4H), 2.82 (d, J = 7.8 Hz, 1H), 2.45 (dq, J = 6.1, 3.0, 2.1 Hz, 3H), 2.35 - 2.10 (m, 1H), 2.10 - 1.56 (m, 10H), 1.56 - 1.11 (m, 8H), 0.97 (s, 2H), 0.81 (d, J = 6.5 Hz, 3H). |
| 93 | (2S,4R)-4-hydroxy-1-((R)-2-(3-((R)-2-(3-(2-hydroxyphenyl)cinnolin-7-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 840.4 | 1H NMR (400 MHz, DMSO-d6) δ 12.11 (s, 1H), 8.99 (s, 1H), 8.90 (s, 1H), 8.41 (d, J = 7.6 Hz, 1H), 8.29 (s, 1H), 8.10 (dd, J = 13.4, 8.1 Hz, 2H), 7.90 (d, J = 8.6 Hz, 1H), 7.44 (d, J = 8.0 Hz, 2H), 7.37 (d, J = 8.2 Hz, 3H), 7.05 (dd, J = 10.7, 7.7 Hz, 2H), 6.16 (s, 1H), 5.01 - 4.85 (m, 2H), 4.37 (t, J = 7.8 Hz, 1H), 4.29 (s, 1H), 3.72 (dd, J = 10.4, 4.3 Hz, 1H), 3.57 (d, J = 9.8 Hz, 2H), 3.21 (s, 4H), 2.45 (d, J = 2.0 Hz, 3H), 2.27 - 2.16 (m, 3H), 1.89 (t, J = 10.7 Hz, 2H), 1.85 - 1.74 (m, 2H), 1.62 (d, J = 22.3 Hz, 6H), 1.42 (dd, J = 32.0, 6.9 Hz, 3H), 0.96 (t, J = 6.9 Hz, 3H), 0.82 (dd, J = 16.0, 6.6 Hz, 3H). |
| 92 | (2S,4R)-4-hydroxy-1-((R)-2-(3-((S)-2-(3-(2-hydroxyphenyl)cinnolin-7-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 840.35 | 1H NMR (400 MHz, DMSO-d6) δ 12.10 (s, 1H), 9.11 - 8.95 (m, 1H), 8.91 - 8.66 (m, 1H), 8.41 (d, J = 7.7 Hz, 1H), 8.29 (s, 1H), 8.15 - 8.00 (m, 2H), 7.98 - 7.81 (m, 1H), 7.49 - 7.40 (m, 2H), 7.39 - 7.32 (m, 3H), 7.10 - 6.99 (m, 2H), 6.16 (s, 1H), 5.11 (d, J = 3.6 Hz, 1H), 5.02 - 4.86 (m, 1H), 4.37 (t, J = 7.8 Hz, 1H), 4.32 - 4.24 (m, 1H), 3.76 - 3.67 (m, 1H), 3.60 - 3.55 (m, 1H), 3.54 - 3.48 (m, 1H), 3.47 - 3.39 (m, 1H), 3.28 - 3.16 (m, 4H), 2.46 - 2.44 (m, 3H), 2.35 - 2.13 (m, 3H), 2.09 - 1.97 (m, 1H), 1.96 - 1.85 (m, 1H), 1.84 - 1.74 (m, 2H), 1.73 - 1.56 (m, 6H), 1.49 - 1.33 (m 3H), 1.00 - 0.90 (m, 3H), 0.86 - 0.76 (m, 3H). |
| 91 | (2S,4R)-4-hydroxy-1-((R)-2-(3-((S)-3-(3-(2-hydroxyphenyl)cinnolin-7-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 786.35 | 1H NMR (300 MHz, DMSO-d6) δ 12.01 (s, 1H), 8.99 (s, 1H), 8.92 (d, J = 1.0 Hz, 1H), 8.53 - 8.26 (m, 2H), 8.19 - 8.04 (m, 2H), 7.95 (dd, J = 8.7, 1.7 Hz, 1H), 7.49 - 7.29 (m, 5H), 7.17 - 6.84 (m, 2H), 6.19 (d, J = 48.4 Hz, 1H), 5.12 (s, 1H), 4.99 - 4.79 (m, 1H), 4.38 (t, J = 7.8 Hz, 1H), 4.29 (s, 1H), 3.88 (d, J = 9.5 Hz, 1H), 3.79 - 3.63 (m, 2H), 3.59 (d, J = 9.9 Hz, 1H), 3.44 (d, J = 10.6 Hz, 1H), 3.16 - 3.04 (m, 2H), 2.94 (t, J = 11.9 Hz, 1H), 2.45 (d, J = 6.4 Hz, 3H), 2.32 - 2.15 (m, 1H), 2.05 (q, J = 10.8, 8.6 Hz, 2H), 1.94 - 1.66 (m, 4H), 1.38 (d, J = 7.0 Hz, 3H), 0.97 (dd, J = 6.6, 4.5 Hz, 3H), 0.81 (d, J = 6.7 Hz, 3H). |
| 90 | (2S,4R)-4-hydroxy-1-((R)-2-(3-((R)-3-(3-(2-hydroxyphenyl)cinnolin-7-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 786.3 | 1H NMR (400 MHz, Methanol-d4) δ 8.86 (d, J = 5.3 Hz, 1H), 8.82 (s, 1H), 8.33 (s, 1H), 8.06 (t, J = 7.8 Hz, 2H), 7.90 (d, J = 8.7 Hz, 1H), 7.82 - 7.14 (m, 5H), 7.14 - 6.95 (m, 2H), 6.15 (d, J = 30.5 Hz, 1H), 5.03 (d, J = 6.9 Hz, 1H), 4.66 - 4.37 (m, 2H), 3.94 (d, J = 9.4 Hz, 1H), 3.85 (dd, J = 10.7, 4.1 Hz, 1H), 3.77 (d, J = 12.8 Hz, 1H), 3.64 (t, J = 9.4 Hz, 2H), 3.16 (d, J = 9.6 Hz, 2H), 3.06 (t, J = 11.3 Hz, 1H), 2.77 - 2.43 (m, 3H), 2.40 (s, 1H), 2.20 (d, J = 12.1 Hz, 2H), 2.05 - 1.61 (m, 4H), 1.55 (dd, J = 27.9, 7.1 Hz, 3H), 1.06 (d, J = 6.5 Hz, 3H), 0.90 (t, J = 8.5 Hz, 3H). |
| 87 | (2S,4R)-4-hydroxy-1-((R)-2-(3-(7-(3-(2-hydroxyphenyl)cinnolin-7-yl)-2-azaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 826.3 | 1H NMR (400 MHz, DMSO-d6) δ 12.08 (s, 1H), 9.01 - 8.78 (m, 2H), 8.41 (d, J = 7.7 Hz, 1H), 8.26 (s, 1H), 8.14 - 8.03 (m, 2H), 7.88 (dd, J = 8.7, 1.6 Hz, 1H), 7.51 - 7.42 (m, 2H), 7.40 - 7.29 (m, 3H), 7.10 - 7.00 (m, 2H), 5.87 (s, 1H), 5.12 (d, J = 3.7 Hz, 1H), 4.99 - 4.87 (m, 1H), 4.38 (t, J = 7.9 Hz, 1H), 4.29 (s, 1H), 3.73 (d, J = 16.4 Hz, 2H), 3.59 (d, J = 8.7 Hz, 3H), 3.45 (s, 1H), 2.84 (s, 1H), 2.46 (d, J = 1.5 Hz, 3H), 2.25 - 2.01 (m, 4H), 1.91 (d, J = 9.5 Hz, 2H), 1.84 - 1.75 (m, 1H), 1.66 (t, J = 11.4 Hz, 4H), 1.44 - 1.34 (m, 3H), 1.19 (s, 1H), 0.97 (t, J = 6.6 Hz, 3H), 0.82 (dd, J = 15.0, 6.6 Hz, 3H). |
| 86 | (2S,4R)-4-hydroxy-1-((R)-2-(3-((1R,5S,6S)-6-(3-(2-hydroxyphenyl)cinnolin-6-yl)-3-azabicyclo[3.1.0]hexan-3-yl)isoxazol-5-yl)-3-methylbutanoyl)-N-((S)-1-(4-(4-methylthiazol-5-yl)phenyl)ethyl)pyrrolidine-2-carboxamide | 874.1 | 1H NMR (400 MHz, DMSO-d6) δ 11.99 (s, 1H), 9.02 - 8.97 (m, 1H), 8.76 (s, 1H), 8.42 (d, J = 7.7 Hz, 1H), 8.36 (d, J = 8.8 Hz, 1H), 8.14 - 8.07 (m, 1H), 7.82 - 7.78 (m, 1H), 7.78 - 7.72 (m, 1H), 7.51 - 7.32 (m, 5H), 7.09 - 7.00 (m, 2H), 6.01 (s, 1H), 5.13 (d, J = 3.6 Hz, 1H), 4.98 - 4.88 (m, 1H), 4.42 - 4.33 (m, 1H), 4.32 - 4.28 (m, 1H), 3.77 - 3.54 (m, 4H), 3.48 - 3.41 (m, 3H), 2.49 - 2.44 (m, 3H), 2.29 - 2.18 (m, 4H), 2.10 - 1.99 (m, 1H), 1.85 - 1.74 (m, 1H), 1.39 (d, J = 7.0 Hz, 3H), 1.02 - 0.79 (m, 6H). |
| 82 | (2S,4R)-1-((R)-2-(3-(2-(3-(3,5-difluoro-2-hydroxyphenyl)cinnolin-7-yl)-7-azaspiro[3.5]nonan-7-yl)isoxazol-5-yl)-3-methylbutanoyl)-4-hydroxy-N-((S)-1-(4-(4-methylthiazol-5-yl)phenyl)ethyl)pyrrolidine-2-carboxamide | 862.3 | 1H NMR (400 MHz, DMSO-d6) δ 12.44 (s, 1H), 9.03 (s, 1H), 9.01 - 8.97 (m, 1H), 8.41 (d, J = 7.7 Hz, 1H), 8.29 (s, 1H), 8.14 - 8.07 (m, 1H), 7.94 - 7.89 (m, 1H), 7.89 - 7.82 (m, 1H), 7.50 - 7.40 (m, 3H), 7.40 - 7.34 (m, 2H), 6.17 (s, 1H), 5.06 (dd, J = 42.3, 3.3 Hz, 1H), 4.86 (dp, 1H), 4.37 (t, J = 7.9 Hz, 1H), 4.29 (s, 1H), 3.90 (p, J = 9.0 Hz, 1H), 3.76 - 3.68 (m, 1H), 3.57 (d, J = 9.8 Hz, 1H), 3.47 - 3.42 (m, 1H), 3.26 (s, 2H), 3.13 (s, 2H), 2.46 (s, 3H), 2.44 - 2.35 (m, 2H), 2.31 -2.16 (m, 1H), 2.12 - 1.98 (m, 3H), 1.86 - 1.75 (m, 3H), 1.65 (s, 2H), 1.42 (dd, J = 32.2, 6.9 Hz, 3H), 1.01 - 0.93 (m, 3H), 0.89 - 0.77 (m, 3H). |
| 76 | (2S,4R)-4-hydroxy-1-((R)-2-(3-((R)-2-(3-(2-hydroxyphenyl)cinnolin-6-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 840.2 | 1H NMR (400 MHz, DMSO-d6) δ 12.08 (d, J = 6.2 Hz, 1H), 8.98 (d, J = 2.9 Hz, 1H), 8.86 (d, J = 0.9 Hz, 1H), 8.44 - 8.37 (m, 2H), 8.10 (dd, J = 7.8, 1.7 Hz, 1H), 7.98 - 7.90 (m, 2H), 7.49 - 7.41 (m, 2H), 7.37 (dd, J = 8.5, 2.6 Hz, 3H), 7.09 - 7.00 (m, 2H), 6.16 (s, 1H), 5.11 (d, J = 3.6 Hz, 1H), 4.91 (q, J = 7.2 Hz, 1H), 4.37 (t, J = 7.9 Hz, 1H), 4.29 (s, 1H), 3.72 (dd, J = 10.5, 4.5 Hz, 1H), 3.57 (d, J = 9.9 Hz, 1H), 3.48 - 3.39 (m, 2H), 3.22 (s, 3H), 2.45 (d, J = 3.0 Hz, 2H), 2.19 (qt, J = 17.3, 8.3 Hz, 3H), 2.01 (d, J = 9.4 Hz, 1H), 1.85 (s, 3H), 1.83 - 1.73 (m, 6H), 1.58 (d, J = 11.5 Hz, 3H), 1.46 (d, J = 7.0 Hz, 1H), 0.96 (t, J = 6.8 Hz, 3H), 0.82 (dd, J = 17.7, 7.9 Hz, 1H), 0.76 (t, J = 6.8 Hz, 3H). |
| 75 | (2S,4R)-4-hydroxy-1-((R)-2-(3-((S)-2-(3-(2-hydroxyphenyl)cinnolin-6-yl)-8-azaspiro[4.5]decan-8-yl)isoxazol-5-yl)-3-methylbutanoyl)-N-((S)-1-(4-(4-methylthiazol-5-yl)phenyl)ethyl)pyrrolidine-2-carboxamide | 840.35 | 1H NMR (400 MHz, Methanol-d4) δ 8.86 (d, J = 10.3 Hz, 1H), 8.77 (s, 1H), 8.36 (d, J = 9.3 Hz, 1H), 8.05 (m, 1H), 7.92 (d, J = 7.6 Hz, 2H), 7.51 - 7.39 (m, 4H), 7.39 - 7.34 (m, 1H), 7.04 (m, 2H), 6.12 (s, 1H), 5.04 (s, 2H), 4.60 (s, 1H), 4.51 (t, J = 8.2 Hz, 1H), 4.42 (d, J = 16.2 Hz, 1H), 3.85 (m, 1H), 3.65 - 3.58 (m, 2H), 3.54 - 3.45 (m, 2H), 2.47 (d, J = 8.1 Hz, 3H), 2.41 - 2.34 (m, 1H), 2.29 - 2.15 (m, 3H), 2.06 - 1.89 (m, 3H), 1.88 - 1.64 (m, 7H), 1.56 (m, 3H), 1.05 (d, J = 6.6 Hz, 3H), 0.91 (dd, J = 12.1, 6.7 Hz, 3H). |
| 74 | (2S,4R)-4-hydroxy-1-((R)-2-(3-(4-(3-(2-hydroxyphenyl)cinnolin-7-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 786.2 | 1H NMR (400 MHz, DMSO-d6) δ 12.06 (s, 1H), 8.99 (s, 1H), 8.91 (s, 1H), 8.42 (d, J = 7.7 Hz, 1H), 8.29 (s, 1H), 8.17 - 8.07 (m, 2H), 7.92 (dd, J = 8.7, 1.7 Hz, 1H), 7.48 - 7.42 (m, 2H), 7.41 - 7.30 (m, 3H), 7.05 (td, J = 8.1, 2.5 Hz, 2H), 6.16 (d, J = 56.2 Hz, 1H), 5.15 - 5.02 (m, 1H), 4.96 - 4.63 (m, 1H), 4.39 (t, J = 7.8 Hz, 1H), 4.30 (s, 1H), 3.85 (d, J = 12.0 Hz, 2H), 3.73 (dd, J = 10.5, 4.5 Hz, 1H), 3.60 (d, J = 9.9 Hz, 1H), 3.45 (d, J = 10.5 Hz, 1H), 3.07 (t, J = 12.3 Hz, 1H), 2.97 (t, J = 11.9 Hz, 2H), 2.46 (s, 3H), 2.35 - 2.18 (m, 1H), 2.09 - 1.97 (m, 3H), 1.94 - 1.75 (m, 3H), 1.43 (dd, J = 34.8, 7.0 Hz, 3H), 0.98 (t, J = 6.8 Hz, 3H), 0.85 (dd, J = 15.9, 6.7 Hz, 3H). |
| 73 | (2S,4R)-4-hydroxy-1-((R)-2-(3-((S)-3-(3-(2-hydroxyphenyl)cinnolin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 772.15 | 1H NMR (300 MHz, DMSO-d6) δ 11.96 (s, 1H), 8.99 (d, J = 3.4 Hz, 1H), 8.92 - 8.78 (m, 1H), 8.44 (dd, J = 11.4, 8.3 Hz, 2H), 8.12 - 7.94 (m, 3 H), 7.49 - 7.30 (m, 5H), 7.12 - 6.97 (m, 2H), 6.02 (s, 1H), 5.13 (d, J = 3.6 Hz, 1H), 4.93 (t, J = 7.3 Hz, 1H), 4.38 (t, J = 7.8 Hz, 1H), 4.30 (s, 1H),3.91 - 3.67 (m, 3H), 3.58 (t, J = 10.6 Hz, 2H), 3.52 (s, 3H), 2.45 (d, J = 8.2 Hz, 4H), 2.34 - 2.15 (m, 2H), 2.02 (d, J = 9.6 Hz, 1H), 1.47 (d, J = 7.0 Hz, 1H), 1.39 (d, J = 7.0 Hz, 3H), 1.24 (s, 1H), 0.98 (t, J = 6.1 Hz, 3H), 0.85 (dd, J = 11.5, 6.6 Hz, 3H). |
| 63 | (2S,4R)-4-hydroxy-1-((S)-2-(3-(9-(3-(2-hydroxyphenyl)cinnolin-6-yl)-3-azaspiro[5.5]undecan-3-yl)isoxazol-5-yl)-3-methylbutanoyl)-N-((S)-1-(4-(4-methylthiazol-5-yl)phenyl)ethyl)pyrrolidine-2-carboxamide | 854.2 | 1H NMR (300 MHz, DMSO-d6) δ 12.05 (d, J = 9.7 Hz, 1H), 8.99 (s, 1H), 8.86 (s, 1H), 8.40 (d, J = 8.7 Hz, 2H), 8.11 (d, J = 8.1 Hz, 1H), 7.96 (d, J = 9.4 Hz, 2H), 7.45 (d, J = 7.6 Hz, 2H), 7.37 (dd, J = 8.3, 2.1 Hz, 3H), 7.05 (t, J = 8.1 Hz, 2H), 6.15 (d, J = 4.7 Hz, 1H), 5.16 - 5.07 (m, 1H), 4.98 - 4.87 (m, 1H), 4.43 - 4.32 (m, 1H), 4.32 - 4.26 (m, 1H), 3.77 - 3.67 (m, 1H), 3.63 - 3.48 (m, 1H), 3.48 - 3.42 (m, 1H), 3.25 - 3.19 (m, 4H), 3.14 - 2.96 (m, 1H), 2.83 - 2.77 (m, 1H), 2.46 (s, 3H), 2.27 - 2.21 (m, 1H), 2.08 - 1.74 (m, 9H), 1.54 - 1.28 (m, 7H), 0.96 (d, J = 6.5 Hz, 3H), 0.83 (dd, J = 11.8, 6.6 Hz,3H). |
| 62 | (2S,4R)-4-hydroxy-1-((R)-2-(3-(7-(3-(2-hydroxyphenyl)cinnolin-6-yl)-2-azaspiro[3.5]nonan-2-yl)isoxazol-5-yl)-3-methylbutanoyl)-N-((S)-1-(4-(4-methylthiazol-5-yl)phenyl)ethyl)pyrrolidine-2-carboxamide | 826.3 | 1H NMR (300 MHz, DMSO-d6) δ 12.00 (s, 1H), 8.99 (s, 1H), 8.85 (s, 1H), 8.47 - 8.34 (m, 2H), 8.10 (d, J = 7.8 Hz, 1H), 7.91 (d, J = 9.2 Hz, 2H), 7.44 (d, J = 8.4 Hz, 2H), 7.37 (d, J = 8.3 Hz, 3H), 7.06 (d, J = 8.0 Hz, 2H), 5.86 (s, 1H), 5.12 (d, J = 3.6 Hz, 1H), 4.99 - 4.82 (m, 1H), 4.37 (s, 2H), 3.73 (s, 3H), 3.64 - 3.47 (m, 4H), 2.76 (d, J = 21.0 Hz, 1H), 2.46 (s, 3H), 2.27 (s, 1H), 2.08 (s, 3H), 1.95 - 1.58 (m, 7H), 1.38 (d, J = 7.0 Hz, 3H), 0.96 (d, J = 6.4 Hz, 3H), 0.90 - 0.73 (m, 3H). |
| 61 | (2S,4R)-4-hydroxy-1-((R)-2-(3-(4-(3-(2-hydroxyphenyl)cinnolin-6-yl)piperidin-1-yl)isoxazol-5-yl)-3-methylbutanoyl)-N-((S)-1-(4-(4-methylthiazol-5-yl)phenyl)ethyl)pyrrolidine-2-carboxamide | 786.15 | 1H NMR (300 MHz, DMSO-d6) δ 12.03 (d, J = 7.1 Hz, 1H), 8.99 (s, 1H), 8.87 (d, J = 4.4 Hz, 1H), 8.42 (dd, J = 8.4, 4.1 Hz, 2H), 8.11 (d, J = 7.5 Hz, 1H), 7.95 (d, J = 8.6 Hz, 2H), 7.55 - 7.27 (m, 5H), 7.05 (t, J = 8.1 Hz, 2H), 6.23 (s, 1H), 5.13 (d, J = 3.6 Hz, 1H), 4.93 (t, J = 7.1 Hz, 1H), 4.39 (t, J = 7.8 Hz, 1H), 4.31 (s, 1H), 3.85 (d, J = 12.5 Hz, 2H), 3.74 (dd, J = 10.5, 4.5 Hz, 1H), 3.61 (d, J = 9.9 Hz, 1H), 3.47 (s, 1H), 3.09 - 2.89 (m, 3H), 2.46 (d, J = 3.6 Hz, 3H),2.33 (s, 1H), 2.00 (d, J = 12.5 Hz, 3H), 1.83 (d, J = 12.3 Hz, 3H), 1.43 (dd, J = 26.1, 6.9 Hz, 3H),0.98 (d, J = 6.5 Hz, 3H), 0.85 (dd, J = 11.8, 6.4 Hz, 3H). |
| 60 | (2S,4R)-4-hydroxy-1-((R)-2-(3-((R)-3-(3-(2-hydroxyphenyl)cinnolin-7-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 772.25 | 1H NMR (300 MHz, Methanol-d4) δ 8.87 (d, J = 15.7 Hz, 2H), 8.39 - 8.35 (m, 1H), 8.16 - 8.03 (m, 2H), 7.97 - 7.87 (m, 1H), 7.46 (d, J = 2.0 Hz, 4H), 7.46 - 7.33 (m, 1H), 7.07 (ddd, J = 7.2, 3.8, 2.5 Hz, 2H), 6.05 (d, J = 16.4 Hz, 1H), 5.06 - 5.02 (m, 1H), 4.58 (d, J = 16.6 Hz, 2H), 4.48 (s, 1H), 3.91 - 3.84 (m, 2H), 3.71 - 3.64 (m, 3H), 3.73 - 3.55 (m, 2H), 2.61 (s, 1H), 2.50 (d, J = 4.4 Hz, 3H), 2.38 (s, 2H), 2.20 (d, J = 8.4 Hz, 1H), 2.07 - 1.92 (m, 1H), 1.58 (dd, J = 20.4, 7.0 Hz, 3H), 1.09 (d, J = 6.5 Hz, 3H), 0.95 (d, J = 6.8 Hz, 3H). |
| 59 | (2S,4R)-4-hydroxy-1-((R)-2-(3-((1R,5S,6S)-6-(3-(2-hydroxyphenyl)cinnolin-7-yl)-3-azabicyclo[3.1.0]hexan-3-yl)isoxazol-5-yl)-3-methylbutanoyl)-N-((S)-1-(4-(4-methylthiazol-5-yl)phenyl)ethyl)pyrrolidine-2-carboxamide | 784.2 | 1H NMR (400 MHz, DMSO-d6) δ 12.09 (s, 1H), 8.98 (s, 1H), 8.88 (s, 1H), 8.40 (d, J = 7.7 Hz, 1H), 8.18 (s, 1H), 8.10 (dd, J = 8.0, 1.7 Hz, 1H), 8.04 (d, J = 8.7 Hz, 1H), 7.71 (dd, J = 8.6, 1.7 Hz, 1H), 7.50 - 7.41 (m, 2H), 7.42 - 7.30 (m, 3H), 7.09 - 7.00 (m, 2H), 6.01 (s, 1H), 5.12 (d, J = 3.6 Hz, 1H), 5.03 - 4.87 (m, 1H), 4.38 (t, J = 7.9 Hz, 1H), 4.30 (s, 1H), 3.73 (dd, J = 10.6, 4.5 Hz, 1H), 3.68 (dd, J = 9.8, 6.9 Hz, 2H), 3.60 (d, J = 10.0 Hz, 1H), 3.53 - 3.40 (m, 3H), 2.46 (d, J = 2.6 Hz, 3H), 2.35 - 2.20 (m, 4H), 2.10 - 1.96 (m, 1H), 1.80 (ddd, J = 12.8, 8.1, 4.9 Hz, 1H), 1.44 (dd, J = 38.1, 7.0 Hz, 3H), 0.98 (t, J = 7.0 Hz, 3H), 0.84 (dd, J = 14.6, 6.6 Hz, 3H). |
| 55 | (2S,4R)-4-hydroxy-1-((R)-2-(3-((R)-3-(3-(2-hydroxyphenyl)cinnolin-6-yl)pyrrolidin-1-yl)isoxazol-5-yl)-3-methylbutanoyl)-N-((S)-1-(4-(4-methylthiazol-5-yl)phenyl)ethyl)pyrrolidine-2-carboxamide | 772.4 | 1H NMR (400 MHz, Methanol-d4) δ 8.86 (d, J = 11.3 Hz, 1H), 8.79 (s, 1H), 8.41 (dd, J = 8.8, 4.3 Hz, 1H), 8.12 - 7.73 (m, 3H), 7.48 - 7.40 (m, 3H), 7.43 - 7.33 (m, 1H), 7.33 - 7.18 (m, 1H), 7.08 - 6.89 (m, 2H), 6.02 (d, J = 15.6 Hz, 1H), 5.05 (t, J = 7.0 Hz, 1H), 4.60 - 4.48 (m, 1H), 4.45 (s, 1H), 3.91 - 3.80 (m, 3H), 3.69 - 3.59 (m, 3H), 3.57 - 3.48 (m, 2H), 2.57 (s, 1H), 2.48 - 2.40 (m, 3H), 2.39 - 2.22 (m, 2H), 2.20 (dd, J = 22.1, 9.0 Hz, 1H), 1.97 (ddd, J = 13.3, 8.7, 4.6 Hz, 1H), 1.55 (dd, J = 21.7, 7.0 Hz, 3H), 1.07 (dd, J = 6.6, 3.9 Hz, 3H), 0.99 - 0.85 (m, 3H). |
| 48 | (2S,4R)-4-hydroxy-1-((R)-2-(3-(6-(3-(2-hydroxyphenyl)cinnolin-6-yl)-2-azaspiro[3.3]heptan-2-yl)isoxazol-5-yl)-3-methylbutanoyl)-N-((S)-1-(4-(4-methylthiazol-5-yl)phenyl)ethyl)pyrrolidine-2-carboxamide | 798.25 | 1H NMR (400 MHz, DMSO-d6) δ 12.07 - 12.00 (m, 1H), 9.02 - 8.97 (m, 1H), 8.87 (s, 1H), 8.45 - 8.38 (m, 2H), 8.14 - 8.07 (m, 1H), 7.92 (s, 1H), 7.89 - 7.81 (m, 1H), 7.51 - 7.41 (m, 2H), 7.41 - 7.33 (m, 3H), 7.10 - 7.00 (m, 2H), 5.88 (s, 1H), 5.11 (d, J = 3.6 Hz, 1H), 4.92 (p, 1H), 4.37 (t, J = 7.9 Hz, 1H), 4.29 (s, 1H), 4.07 (s, 2H), 3.84 (s, 2H), 3.75 - 3.64 (m, 2H), 3.59 (d, J = 9.8 Hz, 1H), 3.53 - 3.39 (m, 1H), 2.78 - 2.68 (m, 2H), 2.48 - 2.43 (m, 5H), 2.27 - 2.17 (m, 1H), 2.10 - 1.95 (m, 1H), 1.84 - 1.73 (m, 1H), 1.42 (dd, J = 31.2, 7.0 Hz, 3H), 1.00 - 0.92 (m, 3H), 0.81 (dd, J = 14.4, 6.5 Hz, 3H). |

### Preparation of (2S,4R)-4-hydroxy-1-[(2R)-2-(5-{6-[3-(2-hydroxyphenyl)cinnolin-7-yl]-2,6-diazaspiro[3.3]heptan-2-yl}-1,2-thiazol-3-yl)-3-methylbutanoyl]-N-[(1S)-1-[4-(4-methyl-1,3-thiazol-5-yl)phenyl]ethyl]pyrrolidine-2-carboxamide (Compound 195).

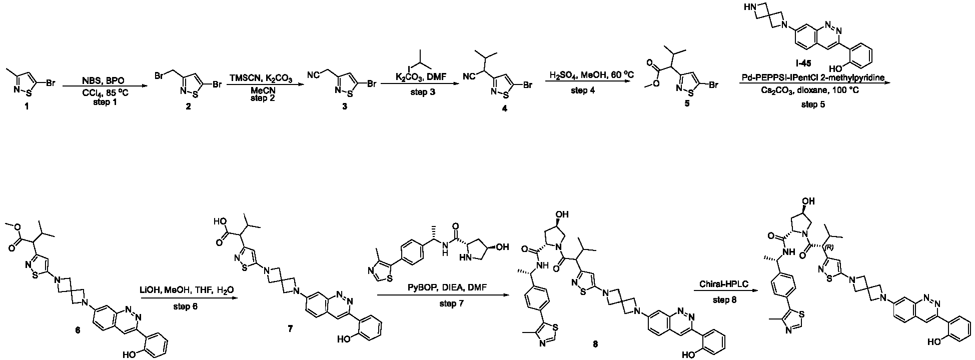

### Step 1: Preparation of 5-bromo-3-(bromomethyl)-1,2-thiazole (Intermediate 2).

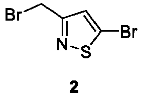

To a solution of 5-bromo-3-methyl-1,2-thiazole (2 g, 11.233 mmol, 1 equiv) and NBS (2.40 g, 13.480 mmol, 1.2 equiv) in CCl₄ (20 mL) was added BPO (287.85 mg, 1.123 mmol, 0.1 equiv). The resulting solution was stirred at 80 degrees C for 16 hours. The mixture was diluted with EtOAc (300 mL) and washed with water (300 mL x 3). The organic layer was dried over anhydrous sodium sulfate, filtered, and concentrated to give a crude product. The crude product was purified by silica gel column chromatography, eluted with 0 to 37% dichloromethane in petroleum ether to afford intermediate **2** (1.06 g, 36.73%) as a yellow oil. LCMS (ESI) m/z: [M+H]⁺ = 256.

### Step 2: Preparation of 2-(5-bromo-1,2-thiazol-3-yl)acetonitrile (Intermediate 3).

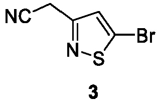

To a solution of intermediate **2** (1.06 g, 4.125 mmol, 1 equiv) and K₂CO₃ (1.14 g, 8.250 mmol, 2 equiv) in acetonitrile (10 mL) was added TMSCN (613.92 mg, 6.188 mmol, 1.5 equiv). The resulting solution was stirred at 60 degrees C for 4 hours. The mixture was diluted with EtOAc (250 mL) and washed with water (250 mL x 3). The organic layer was dried over anhydrous sodium sulfate, filtered and concentrated to give a crude product. The crude product was purified by silica gel column chromatography, eluted with 0 to 52% dichloromethane in petroleum ether to afford intermediate **3** (772 mg, 92.15%) as a colorless oil. LCMS (ESI) m/z: [M+H]⁺ =203.

### Step 3: Preparation of 2-(5-bromo-1,2-thiazol-3-yl)-3-methylbutanenitrile (Intermediate 4).

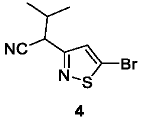

To a solution of intermediate **3** (772 mg, 3.802 mmol, 1 equiv) and K₂CO₃ (1.05 g, 7.604 mmol, 2 equiv) in DMF (6 mL, 77.529 mmol, 20.39 equiv) was added 2-iodopropane (1.29 g, 7.604 mmol, 2 equiv). The resulting solution was stirred at 25 degrees C for 16 hours. The mixture was diluted with EtOAc (200 mL) and washed with water (200 mL x 3). The organic layer was dried over anhydrous sodium sulfate, filtered and concentrated to give a crude product. The crude product was purified by silica gel column chromatography, eluted with 0 to 69% dichloromethane in petroleum ether to afford intermediate **4** (228 mg, 24.46%) as a yellow oil. LCMS (ESI) m/z: [M+H]⁺ = 245.

### Step 4: Preparation of methyl 2-(5-bromo-1,2-thiazol-3-yl)-3-methylbutanoate (Intermediate 5).

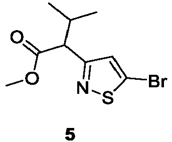

To a solution of intermediate **4** (228 mg, 0.930 mmol, 1 equiv) in methanol (3 mL) was added H₂SO₄ (1 mL). The resulting solution was stirred at 80 degrees C for 16 hours. The reaction was quenched with water/ice at 0°C. The mixture was basified to pH 8 with saturated NaHCO₃ (aq.). The mixture was diluted with EtOAc (120 mL) and washed with water (120 mL x 3). The organic layer was dried over anhydrous sodium sulfate, filtered and concentrated to give a crude product. The crude product was purified by Prep-TLC (dichloromethane / petroleum ether 2:1) to afford intermediate **5** (127 mg, 49.09%) as a colorless oil. LCMS (ESI) m/z: [M+H]⁺ = 278.

### Step 5: Preparation of methyl 2-(5-{6-[3-(2-hydroxyphenyl)cinnolin-7-yl]-2,6-diazaspiro[3.3]heptan-2-yl}-1,2-thiazol-3-yl)-3-methylbutanoate (Intermediate 6).

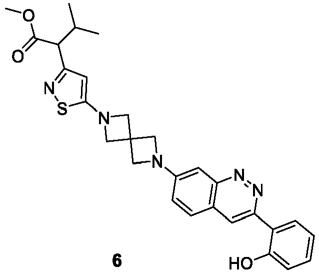

To a solution of intermediate **5** (127 mg, 0.457 mmol, 1 equiv) and **I-45** (159.90 mg, 0.503 mmol, 1.1 equiv) in dioxane (3 mL) were added Pd-PEPPSI-IPentCl 2-methylpyridine (o-picoline) (19.20 mg, 0.023 mmol, 0.05 equiv) and Cs₂CO₃ (297.52 mg, 0.914 mmol, 2 equiv). The resulting solution was stirred at 100 degrees C for 4 hours (under N₂ atmosphere). The mixture was diluted with EtOAc (100 mL) and washed with water (100 mL x 3). The organic layer was dried over anhydrous sodium sulfate, filtered and concentrated to give a crude product. The crude product was purified by flash C18 chromatography, elution gradient 0 to 82% methanol in water to afford intermediate **6** (147 mg, 62.44%) as a red solid. LCMS (ESI) m/z: [M+H]⁺ =516.

### Step 6: Preparation of 2-(5-{6-[3-(2-hydroxyphenyl)cinnolin-7-yl]-2,6-diazaspiro[3.3]heptan-2-yl}-1,2-thiazol-3-yl)-3-methylbutanoic acid (Intermediate 7).

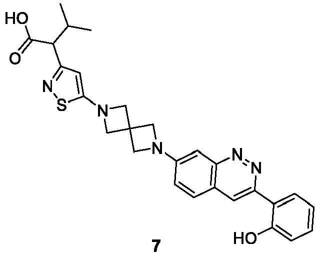

To a solution of intermediate **6** (142 mg, 0.275 mmol, 1 equiv) in methanol (2 mL) and H₂O (1 mL) was added LiOH (52.76 mg, 2.200 mmol, 8 equiv) and THF (2 mL). The resulting solution was stirred at room temperature for 16 hours. The mixture was acidified to pH 5 with 1 M HCl (aq.). The precipitated solids were collected by filtration and washed with water (10 mL) and methanol (3 x 10 mL). This resulted in intermediate **7** (126 mg, 91.21%) as a red solid. LCMS (ESI) m/z: [M+H]⁺ = 502.

### Step 7: Preparation of (2S,4R)-4-hydroxy-1-[2-(5-{6-[3-(2-hydroxyphenyl)cinnolin-7-yl]-2,6-diazaspiro[3.3]heptan-2-yl}-1,2-thiazol-3-yl)-3-methylbutanoyl]-N-[(1S)-1-[4-(4-methyl-1,3-thiazol-5-yl)phenyl]ethyl]pyrrolidine-2-carboxamide (Intermediate 8).

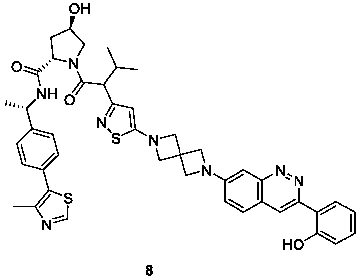

To a solution of intermediate **7** (121 mg, 0.241 mmol, 1 equiv) and (2S,4R)-4-hydroxy-N-[(1S)-1-[4-(4-methyl-1,3-thiazol-5-yl)phenyl]ethyl]pyrrolidine-2-carboxamide (119.92 mg, 0.361 mmol, 1.5 equiv) in DMF (2.5 mL) were added PyBOP (251.07 mg, 0.482 mmol, 2 equiv) and DIEA (155.89 mg, 1.205 mmol, 5 equiv). The resulting solution was stirred at room temperature for 2 hours. The reaction was purified by Prep-HPLC with the following conditions (Column: Xselect CSH C18 OBD Column 30*150mm, 5µm; Mobile Phase A: Water (0.1% FA), Mobile Phase B: ACN; Flow rate: 60 mL/min mL/min; Gradient: 43% B to 58% B in 8 min; Wave Length: 254nm/220nm nm; RT1 (min): 8.36) to afford intermediate **8** (97 mg, 49.34%) as a red solid. LCMS (ESI) m/z: [M+H]⁺ =815.

### Step 8: Preparation of (2S,4R)-4-hydroxy-1-[(2R)-2-(5-{6-[3-(2-hydroxyphenyl)cinnolin-7-yl]-2,6-diazaspiro[3.3]heptan-2-yl}-1,2-thiazol-3-yl)-3-methylbutanoyl]-N-[(1S)-1-[4-(4-methyl-1,3-thiazol-5-yl)phenyl]ethyl]pyrrolidine-2-carboxamide (Compound 195).

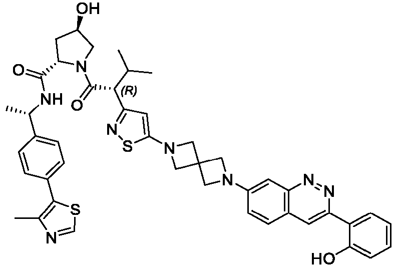

The intermediate **8** (97 mg) was purified by Chiral-Prep-HPLC with the following conditions: Column: CHIRALPAK IC, 2*25 cm, 5 µm; Mobile Phase A: MtBE(10mM NH3-MeOH), Mobile Phase B: MeOH--HPLC; Flow rate: 20 mL/min; Gradient: isocratic 40; Wave Length: 274/212 nm; RT1(min): 7.5; RT2(min): 14.5; Sample Solvent: MeOH--HPLC; Injection Volume: 1 mL; Number Of Runs: 4. Compound **195** (41.2 mg, 42.09%) as a yellow solid.. ¹H NMR (300 MHz, DMSO-d6) δ 12.80 (s, 1H), 8.99 (s, 1H), 8.78 (s, 1H), 8.38 (d, J = 7.7 Hz, 1H), 8.08 - 7.99 (m, 1H), 7.96 (d, J = 9.0 Hz, 1H), 7.55 - 7.21 (m, 6H), 7.10 - 6.91 (m, 3H), 6.14 (s, 1H), 5.06 (d, J = 3.6 Hz, 1H), 4.94 (q, J = 7.2, 6.7 Hz, 1H), 4.46 - 4.25 (m, 6H), 4.21 (s, 4H), 3.72 (dd, J = 10.5, 4.5 Hz, 1H), 3.49 (d, J = 10.5 Hz, 1H), 3.40 (d, J = 10.5 Hz, 1H), 2.46 (s, 3H), 2.31 - 2.17 (m, 1H), 2.08 - 1.89 (m, 1H), 1.85 - 1.70 (m, 1H), 1.39 (d, J = 7.0 Hz, 3H), 0.93 (d, J = 6.4 Hz, 3H), 0.68 (d, J = 6.6 Hz, 3H). LCMS (ESI) m/z: [M+H]⁺ =815.25

### Preparation of (2S,4R)-4-hydroxy- 1-[2-(4-{6-[3-(2-hydroxyphenyl)cinnolin-7-yl]-2,6-diazaspiro[3.3]heptan-2-yl}pyrazol-1-yl)-3-methylbutanoyl]-N-[(1S)-1-[4-(4-methyl-1,3-thiazol-5-yl)phenyl]ethyl]pyrrolidine-2-carboxamide (Compound 258).

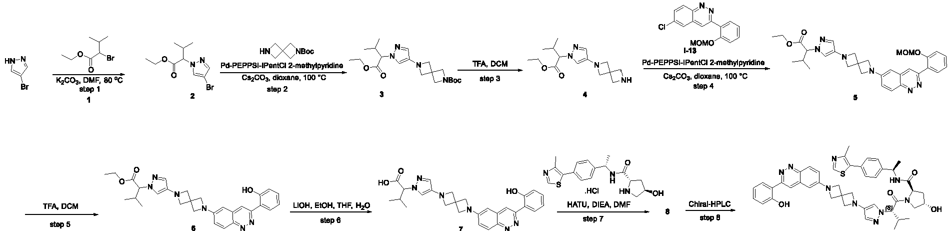

### Step 1: Preparation of ethyl 2-(4-bromopyrazol-1-yl)-3-methylbutanoate (2).

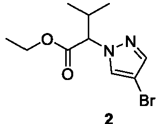

A solution of 4-bromopyrazole (4 g, 27.216 mmol, 1 equiv), ethyl 2-bromo-3-methylbutanoate (6.83 g, 32.659 mmol, 1.2 equiv) and K₂CO₃ (7.52 g, 54.432 mmol, 2 equiv) in DMF (40 mL) was stirred for 16 h at 80°C. The resulting mixture was diluted with water (200 mL). The resulting mixture was extracted with EtOAc (3 x 50 mL). The combined organic layers were washed with water (4 x 100 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (10 mmol/L NH₄HCO₃), 0% to 65% gradient in 35 min; hold 5 min at 65%; detector, UV 254 nm. This resulted in intermediate **2** (2.6 g, 34.72%) as a white solid. LCMS (ESI) m/z: [M+H]⁺ = 275.

### Step 2: Preparation of tert-butyl 6-[1-(1-ethoxy-3-methyl-1-oxobutan-2-yl)pyrazol-4-yl]-2,6-diazaspiro[3.3]heptane-2-carboxylate (3).

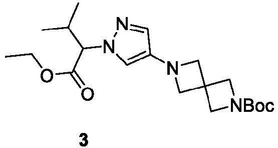

A solution of intermediate **2** (1 g, 3.634 mmol, 1 equiv), tert-butyl 2,6-diazaspiro[3.3]heptane-2-carboxylate (1.15 g, 3.997 mmol, 1.1 equiv) and Cs₂CO₃ (3.55 g, 10.902 mmol, 3 equiv) in dioxane (20 mL) was stirred for 6h at 100°C .The resulting mixture was diluted with water (200 mL).The resulting mixture was extracted with EtOAc (3 x 50 mL). The combined organic layers were washed with water (4 x 100 mL), dried over anhydrous Na₂SO₄. After filtration, the filtrate was concentrated under reduced pressure. The residue was purified by silica gel column chromatography, eluted with PE / EA (1:1) to afford intermediate **3** (0.35 g, 24.54%) as a yellow solid. LCMS (ESI) m/z: [M+H]⁺ = 393.

### Step 3: Preparation of ethyl 2-(4-{2,6-diazaspiro[3.3]heptan-2-yl}pyrazol-1-yl)-3-methylbutanoate (4).

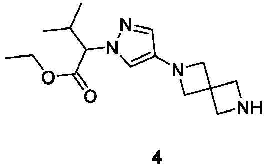

A solution of intermediate **3** (1 g, 2.548 mmol, 1 equiv) and TFA (5 mL, 0.662 mmol, 0.26 equiv) in DCM (50 mL) was stirred for 1h. The resulting mixture was concentrated under reduced pressure, residue was dissolved in CH₂Cl₂ (60mL). The resulting mixture was concentrated under reduced pressure. This resulted in intermediate **4** (0.7 g, 93.97%) as a yellow liquid. LCMS (ESI) m/z: [M+H]⁺ = 293.

### Step 4: Preparation of ethyl 2-[4-(6-{3-[2-(methoxymethoxy)phenyl]cinnolin-6-yl}-2,6-diazaspiro[3.3]heptan-2-yl)pyrazol-1-yl]-3-methylbutanoate (5).

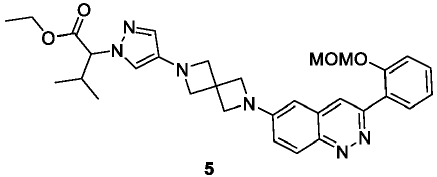

A solution of intermediate **4** (0.9 g, 3.078 mmol, 1 equiv), **I-13** (0.92 g, 3.078 mmol, 1 equiv), Pd-PEPPSI-IPentCl 2-methylpyridine (o-picoline (0.13 g, 0.154 mmol, 0.05 equiv) and Cs₂CO₃ (4.01 g, 12.312 mmol, 4 equiv) in dioxane (20 mL) was stirred for 2h at 100°C under nitrogen atmosphere. The resulting mixture was diluted with water (200 mL). The resulting mixture was extracted with EtOAc (3 x 50 mL). The combined organic layers were washed with water (4 x 100 mL), dried over anhydrous Na₂SO₄. After filtration, the filtrate was concentrated under reduced pressure. The residue was purified by reversed-phase flash chromatography with the following conditions: column, C18 silica gel; mobile phase, MeCN in Water (0.1% FA), 0% to 100% gradient in 35 min; hold 5 min at 41%. detector, UV 254 nm. This resulted in intermediate **5** (0.61 g, 35.60%) as a dark red solid. LCMS (ESI) m/z: [M+H]⁺ = 557.

### Step 5: Preparation of ethyl 2-(4-{6-[3-(2-hydroxyphenyl)cinnolin-6-yl]-2,6-diazaspiro[3.3]heptan-2-yl)pyrazol-1-yl)-3-methylbutanoate (6).

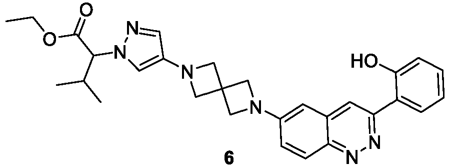

A solution of intermediate **5** (0.3 g, 0.539 mmol, 1 equiv) and TFA (3 mL) in DCM (25 mL) was stirred for 2h at room temperature. The resulting mixture was concentrated under reduced pressure. residue was dissolved in CH₂Cl₂ (60 mL). The resulting mixture was concentrated under reduced pressure. This resulted in intermediate **6** (0.3 g, 108.59%) as a dark red liquid. LCMS (ESI) m/z: [M+H]⁺ = 513.

### Step 6: Preparation of 2-(4-{6-[3-(2-hydroxyphenyl)cinnolin-6-yl]-2,6-diazaspiro[3.3]heptan-2-yl}pyrazol-1-yl)-3-methylbutanoic acid (7).

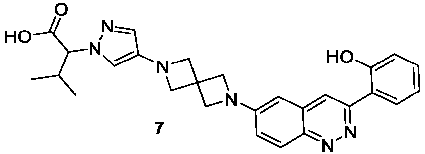

A solution of intermediate **6** (0.3 g, 0.585 mmol, 1 equiv) and LiOH.H₂O (7.5 mL) in THF (11.5 mL) and EtOH (11.5 mL) was stirred for 1h at room temperature. The mixture was purified by reversed-phase flash chromatography with the following conditions: column, C18 silica gel; mobile phase, MeCN in Water (0.1% FA), 0% to 44% gradient in 35 min; hold 5 min at 44%. detector, UV 254 nm. This resulted in intermediate **7** (0.07 g, 24.68%) as a yellow solid. LCMS (ESI) m/z: [M+H]⁺ = 485. *Step 7: Preparation of* (2R,4S)-4-hydroxy-1-[2-(4-{6-[3-(2-hydroxyphenyl)cinnolin-6-yl]-2,6-diazaspiro[3.3]heptan-2-yl}pyrazol-1-yl)-3-methylbutanoyl]-N-[(1R)-1-[4-(4-methyl-1,3-thiazol-5-yl)phenyl]ethyl]pyrrolidine-2-carboxamide *(8)*.

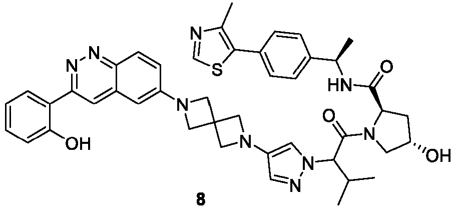

A solution of intermediate **7** (60 mg, 0.124 mmol, 1 equiv), HATU (61.21 mg, 0.161 mmol, 1.3 equiv) and DIEA (107.84 uL, 0.620 mmol, 5 equiv) in DMF (3 mL) was stirred for 0.5h at room temperature under nitrogen atmosphere. To the above mixture was added (2S,4R)-4-hydroxy-N-[(1S)-1-[4-(4-methyl-1,3-thiazol-5-yl)phenyl]ethyl]pyrrolidine-2-carboxamide (45.14 mg, 0.136 mmol, 1.1 equiv). The resulting mixture was stirred for additional 1h at room temperature. The mixture was purified by reversed-phase flash chromatography with the following conditions: column, C18 silica gel; mobile phase, MeCN in Water (0.1% FA), 0% to 41% gradient in 40 min, hold 5 min at 41%; detector, UV 254 nm. The resulting mixture was concentrated under reduced pressure. This resulted in intermediate **8** (70 mg, 70.84%) as a yellow solid. LCMS (ESI) m/z: [M+H]⁺ = 798. *Step 8: Preparation of* (2R,4S)-4-hydroxy-1-[(2S)-2-(4-{6-[3-(2-hydroxyphenyl)cinnolin-6-yl]-2,6-diazaspiro[3.3]heptan-2-yl}pyrazol-1-yl)-3-methylbutanoyl]-N-[(1R)-1-[4-(4-methyl-1,3-thiazol-5-yl)phenyl]ethyl]pyrrolidine-2-carboxamide *(Compound **258**).*

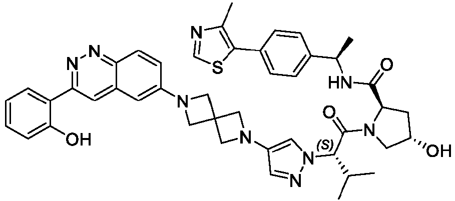

Intermediate **8** (70 mg, 0.088 mmol, 1 equiv) was purified by Prep-Chiral with the following conditions (Column: CHIRALPAK IC, 2*25 cm, 5 µm; Mobile Phase A: MtBE(10mM NH₃-MeOH), Mobile Phase B: MeOH; Flow rate: 20 mL/min; Gradient: isocratic 30; Wave Length: 212/272 nm; RT1(min): 7.25; RT2(min): 15.0; Sample Solvent: MeOH: DCM=1: 2; Injection Volume: 1.1 mL; Number Of Runs: 4) to afford Compound **258** (7.4 mg, 10.51%) as a yellow solid. ¹H NMR (400 MHz, DMSO-d6) δ 8.98 (s, 1H), 8.56 (s, 1H), 8.46 (d, J = 7.7 Hz, 1H), 8.19 (d, J = 9.2 Hz, 1H), 8.05 (dd, J = 8.1, 1.6 Hz, 1H), 7.44 (d, J = 8.3 Hz, 2H), 7.41 - 7.29 (m, 3H), 7.29 - 7.20 (m, 2H), 7.02 - 6.93 (m, 3H), 6.56 (d, J = 2.4 Hz, 1H), 5.34 - 4.88 (m, 2H), 4.63 (d, J = 10.5 Hz, 1H), 4.38 (t, J = 7.9 Hz, 1H), 4.28 (s, 5H), 3.84 (t, J = 5.0 Hz, 4H), 3.75 (dd, J = 10.6, 4.4 Hz, 1H), 3.52 (d, J = 10.6 Hz, 1H), 2.46 (s, 3H), 2.40 - 2.29 (m, 1H), 2.06 - 1.95 (m, 1H), 1.79 (ddd, J = 12.8, 8.1, 4.8 Hz, 1H), 1.39 (d, J = 7.0 Hz, 3H), 0.97 (d, J = 6.5 Hz, 3H), 0.63 (d, J = 6.6 Hz, 3H). LCMS (ESI) m/z: [M+H]⁺ = 798.35.

Compounds in Table 17 were prepared using procedures similar to the one used above for the preparation of **Compound 258** using the appropriate aryl bromides and amines.

**Table 17.**

| **No.** | **Name** | **LCMS (ESI) m/z** | **¹H NMR** |
|---|---|---|---|
| 272 | (2S,4R)-4-hydroxy-1-((S)-2-(4-(6-(3-(2-hydroxyphenyl)cinnolin-6-yl)-2,6-diazaspiro[3.3]heptan-2-yl)-1 H-imidazol-1-yl)-3-methylbutanoyl)-N-((S)-1-(4-(4-methylthiazol-5-yl)phenyl)ethyl)pyrrolidine-2-carboxamide | 798.25 | 1H NMR (400 MHz, DMSO-d6) δ 13.35 (s, 1H), 8.99 (s, 1H), 8.58 - 8.47 (m, 2H), 8.20 (d, J = 9.3 Hz, 1H), 8.09 - 7.99 (m, 1H), 7.45 (d, J = 8.2 Hz, 3H), 7.41 - 7.32 (m, 3H), 7.29 - 7.22 (m, 1H), 7.09 - 6.95 (m, 2H), 6.61 - 6.53 (m, 1H), 6.41 (d, J = 1.4 Hz, 1H), 5.17 (d, J = 3.7 Hz, 1H), 5.08 - 4.88 (m, 1H), 4.58 (d, J = 10.3 Hz, 1H), 4.34 (d, J = 41.7 Hz, 6H), 3.96 - 3.79 (m, 4H), 3.69 - 3.59 (m, 2H), 2.46 (d, J = 3.3 Hz, 3H), 2.29 - 2.19 (m, 1H), 2.06 (t, J = 10.7 Hz, 1H), 1.81 - 1.74 (m, 1H), 1.51 - 1.34 (m, 3H), 0.96 (d, J = 6.5 Hz, 3H), 0.73 - 0.59 (m, 3H). |
| 271 | (2S,4R)-4-hydroxy-1-((S)-2-(4-(6-(3-(2-hydroxyphenyl)cinnolin-7-yl)-2,6-diazaspiro[3.3]heptan-2-yl)-1H-imidazol-1-yl)-3-methylbutanoyl)-N-((S)-1-(4-(4-methylthiazol-5-yl)phenyl)ethyl)pyrrolidine-2-carboxamide | 798.35 | 1H NMR (400 MHz, DMSO-d6) δ 12.84 (s, 1H), 8.99 (s, 1H), 8.76 (s, 1H), 8.51 (d, J = 7.7 Hz, 1H), 7.99 (dd, J = 31.8, 8.2 Hz, 2H), 7.52 - 7.19 (m, 7H), 7.04 - 6.93 (m, 3H), 6.41 (d, J = 1.5 Hz, 1H), 5.17 - 4.83 (m, 2H), 4.58 - 4.40 (m, 2H), 4.27 (s, 5H), 3.93 - 3.81 (m, 4H), 3.73 - 3.56 (m, 2H), 2.46 (s, 3H), 2.30 - 2.19 (m, 1H), 2.06 (t, J = 9.1 Hz, 1H), 1.82 - 1.73 (m, 1H), 1.51 - 1.35 (m, 3H), 0.96 (d, J = 6.5 Hz, 3H), 0.71 - 0.60 (m, 3H). |
| 257 | (2S,4R)-4-hydroxy-1-((R)-2-(4-(6-(3-(2-hydroxyphenyl)cinnolin-7-yl)-2,6-diazaspiro[3.3]heptan-2-yl)-1H-pyrazol-1-yl)-3-methylbutanoyl)-N-((S)-1-(4-(4-methylthiazol-5-yl)phenyl)ethyl)pyrrolidine-2-carboxamide | 798.3 | 1H NMR (400 MHz, DMSO-d6) δ 12.87 (s, 1H), 8.99 (s, 1H), 8.74 (s, 1H), 8.27 - 7.86 (m, 3H), 7.55 - 7.17 (m, 7H), 7.16 - 6.92 (m, 4H), 5.17 - 4.66 (m, 3H), 4.48 - 4.32 (m, 1H), 4.24 (d, J = 9.8 Hz, 4H), 3.85 (d, J = 9.2 Hz, 4H), 3.60 - 3.39 (m, 2H), 3.03 (d, J = 8.7 Hz, 1H), 2.46 (s, 4H), 2.18 - 1.72 (m, 2H), 1.44 (dd, J = 29.5, 6.9 Hz, 3H), 1.24 (d, J = 11.1 Hz, 1H), 0.99 (d, J = 6.6 Hz, 2H), 0.83 (t, J = 8.9 Hz, 1H), 0.64 (dd, J = 34.3, 6.6 Hz, 3H). |

### Preparation of (2S,4R)-4-hydroxy-1-[(2S)-2-(3-{6-[3-(2-hydroxyphenyl)cinnolin-7-yl]-2,6-diazaspiro[3.3]heptan-2-yl}-1,2,4-triazol-1-yl)-3-methylbutanoyl]-N-[(1S)-1-[4-(4-methyl-1,3-thiazol-5-yl)phenyl]ethyl]pyrrolidine-2-carboxamide (Compound 267).

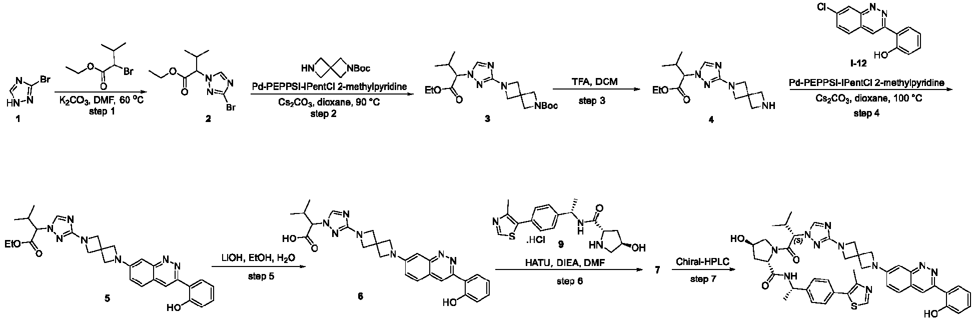

### Step 1: Preparation of ethyl 2-(3-bromo-1,2,4-triazol-1-yl)-3-methylbutanoate (Intermediate 2).

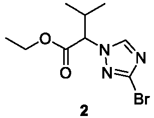

A mixture of 3-bromo-1H-1,2,4-triazole (5 g, 33.792 mmol, 1 equiv), K₂CO₃ (14.01 g, 101.376 mmol, 3 equiv) and ethyl 2-bromo-3-methylbutanoate (10.60 g, 50.688 mmol, 1.5 equiv) in DMF (50 mL) were stirred for overnight at 60°C. The mixture was diluted with water (100 mL), extracted with EtOAc (3 x 100 mL), The combined organic layers were washed with brine (3 x 200 mL), dried over anhydrous Na₂SO₄. After filtration, the filtrate was concentrated under reduced pressure. The residue was purified by silica gel column chromatography, eluted with PE / EA (1:1) to afford intermediate **2** (7.55 g, 80.91%) as a colorless oil. LCMS (ESI) m/z: [M+H]⁺ = 277. ¹H NMR (400 MHz, Chloroform-d) δ 8.28 (s, 1H), 4.79 (d, 1H), 4.34 - 4.18 (m, 2H), 2.60 - 2.44 (m, 1H), 1.31 (t, 3H), 1.03 (d, 3H), 0.93 (d, 3H).

### Step 2: Preparation of tert-butyl 6-[1-(1-ethoxy-3-methyl-1-oxobutan-2-yl)-1,2,4-triazol-3-yl]-2,6-diazaspiro[3.3]heptane-2-carboxylate (Intermediate 3).

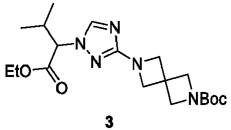

A mixture of intermediate **2** (2 g, 7.243 mmol, 1 equiv), Cs₂CO₃ (7.08 g, 21.729 mmol, 3 equiv), Pd-PEPPSI-IPentCl 2-methylpyridine (o-picoline (121.85 mg, 0.145 mmol, 0.02 equiv) and tert-butyl 2,6-diazaspiro[3.3]heptane-2-carboxylate (1.72 g, 8.692 mmol, 1.20 equiv) in dioxane (20 mL) were stirred for 2 h at 100°C under nitrogen atmosphere. The mixture was diluted with water (100 mL), extracted with EtOAc (3 x 100 mL), The combined organic layers were dried over anhydrous Na₂SO₄. After filtration, the filtrate was concentrated under reduced pressure. The residue was purified by silica gel column chromatography, eluted with PE / EA (5:1) to afford intermediate 3 (2.7 g, 94.74%) as a light yellow oil. LCMS (ESI) m/z: [M+H]⁺ =395.

### Step 3: Preparation of ethyl 2-(3-{2,6-diazaspiro[3.3]heptan-2-yl}-1,2,4-triazol-1-yl)-3-methylbutanoate (Intermediate 4).

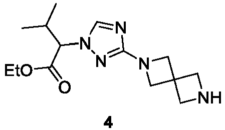

A mixture of intermediate **3** (2.6 g, 6.608 mmol, 1 equiv) and TFA (8 mL) in DCM (24 mL) were stirred overnight at room temperature. The reaction was monitored by LCMS. The resulting crude solid was dried in an oven under reduced pressure. The residue was dissolved in MeCN (5 mL) and water (100 mL). The resulting crude solid was dried by lyophilization. This resulted in intermediate **4** (4.36 g, crude) as an off-white crude solid. LCMS (ESI) m/z: [M+H]⁺ = 294.

### Step 4: Preparation of ethyl 2-(3-{6-[3-(2-hydroxyphenyl)cinnolin-7-yl]-2,6-diazaspiro[3.3]heptan-2-yl}-1,2,4-triazol-1-yl)-3-methylbutanoate (Intermediate 5).

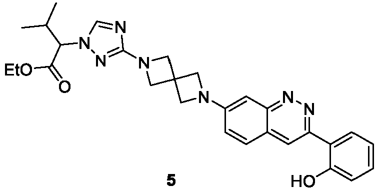

A mixture of intermediate **4** (350 mg, 1.193 mmol, 1.00 equiv), Cs₂CO₃ (2.33 g, 7.158 mmol, 6 equiv), Pd-PEPPSI-IPentCl 2-methylpyridine (o-picoline) (50.18 mg, 0.060 mmol, 0.05 equiv) and **1-12** (358.79 mg, 1.193 mmol, 0.5 equiv) in dioxane (6 mL) were stirred for 2 h at 100°C under nitrogen atmosphere. The mixture was diluted with water (100 mL), extracted with EtOAc (3 x 100 mL), The combined organic layers were dried over anhydrous Na₂SO₄. After filtration, the filtrate was concentrated under reduced pressure. The residue was purified by silica gel column chromatography, eluted with CH₂Cl₂ / MeOH (10:1). This resulted in intermediate **5** (196 mg, 31.99%) as a yellow solid. LCMS (ESI) m/z: [M+H]⁺ = 514.

### Step 5: Preparation of 2-(3-{6-[3-(2-hydroxyphenyl)cinnolin-7-yl]-2,6-diazaspiro[3.3]heptan-2-yl}-1,2,4-triazol-1-yl)-3-methylbutanoic acid (Intermediate 6).

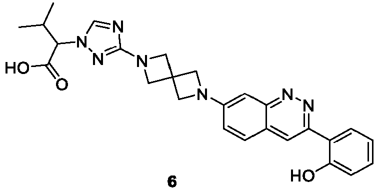

A mixture of intermediate **5** (186 mg, 0.369 mmol, 1 equiv) and LiOH.H₂O (151.96 mg, 3.620 mmol, 10 equiv) in EtOH (4 mL) and H₂O (2 mL) were stirred for 2 h at room temperature. The reaction was monitored by LCMS. The mixture was neutralized to pH 7 with HCl (aq.1 M). The resulting mixture was extracted with EtOAc (3 x 50 mL). The combined organic layers were dried over anhydrous Na₂SO₄. After filtration, the filtrate was concentrated under reduced pressure. This resulted in intermediate **6** (198 mg, crude) as a red solid. LCMS (ESI) m/z: [M+H]⁺ =486.

### Step 6: Preparation of (2S,4R)-4-hydroxy-1-[2-(3-{6-[3-(2-hydroxyphenyl)cinnolin-7-yl]-2,6-diazaspiro[3.3]heptan-2-yl}-1,2,4-triazol-1-yl)-3-methylbutanoyl]-N-[(1S)-1-[4-(4-methyl-1,3-thiazol-5-yl)phenyl]ethyl]pyrrolidine-2-carboxamide (Intermediate 7).

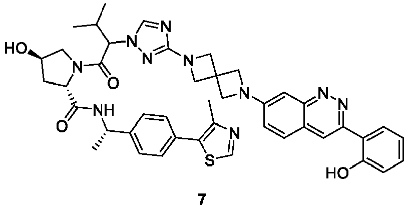

A mixture of intermediate **6** (198 mg, 0.408 mmol, 1 equiv), PyBOP (318.32 mg, 0.612 mmol, 1.5 equiv), DIEA (210.82 mg, 1.632 mmol, 4 equiv) and (2S,4R)-4-hydroxy-N-[(1S)-1-[4-(4-methyl-1,3-thiazol-5-yl)phenyl]ethyl]pyrrolidine-2-carboxamide (148.67 mg, 0.449 mmol, 1.1 equiv) in DMF (3 mL) were stirred for 2 h at room temperature. The reaction was monitored by LCMS. The residue was purified by reversed-phase flash chromatography with the following conditions: column, C18 silica gel; mobile phase, MeCN in Water (10mmol/L NH₄HCO₃), 0 to 70% gradient in 30 min; detector, UV 254 nm. This resulted in intermediate **7** (164.8 mg, 50.58%) as a yellow solid. LCMS (ESI) m/z: [M+H]⁺ = 799.

### Step 7: Preparation of (2S,4R)-4-hydroxy-1-[(2S)-2-(3-{6-[3-(2-hydroxyphenyl)cinnolin-7-yl]-2,6-diazaspiro[3.3]heptan-2-yl}-1,2,4-triazol-1-yl)-3-methylbutanoyl]-N-[(1S)-1-[4-(4-methyl-1,3-thiazol-5-yl)phenyl]ethyl]pyrrolidine-2-carboxamide (Compound 267).

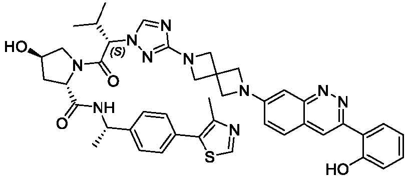

Intermediate 7 (164.8 mg) was purified by PREP_CHIRAL_HPLC with the following conditions (Column: CHIRALPAK ID, 2*25 cm, 5 µm; Mobile Phase A: MtBE(10mM NH3-MeOH), Mobile Phase B: MeOH; Flow rate: 20 mL/min; Gradient: isocratic 30; Wave Length: 212/272 nm; RT1(min): 10.7; RT2(min): 19.5; Sample Solvent: MeOH: DCM=1: 2; Injection Volume: 0.6 mL; Number Of Runs: 4). This resulted in 267 (69.9 mg, 40.97%) as a yellow solid. LCMS (ESI) m/z: [M+H]⁺ = 799.45. ¹H NMR (400 MHz, DMSO-d6) δ 12.82 (s, 1H), 8.98 (s, 1H), 8.75 (s, 1H), 8.48 (d, J = 7.6 Hz, 1H), 8.25 (s, 1H), 8.08 - 7.90 (m, 2H), 7.50 - 7.42 (m, 2H), 7.40 - 7.25 (m, 4H), 7.04 - 6.96 (m, 3H), 5.16 (d, J = 3.8 Hz, 1H), 4.96 - 4.86 (m, 1H), 4.78 (d, J = 10.0 Hz, 1H), 4.41 (t, J = 8.0 Hz, 1H), 4.36 - 4.23 (m, 5H), 4.12 - 4.08 (m, 4H), 3.75 - 3.67 (m, 1H), 3.56 (d, J = 10.9 Hz, 1H), 2.46 (s, 3H), 2.44 - 2.30 (m, 1H), 2.10 - 2.00 (m, 1H), 1.85 - 1.74 (m, 1H), 1.39 (d, J = 7.0 Hz, 3H), 1.00 (d, J = 6.6 Hz, 3H), 0.83 - 0.73 (m, 3H).

### Preparation of (2S,4R)-4-hydroxy- 1-[(2R)-2-(5-{6-[3-(2-hydroxyphenyl)cinnolin-7-yl]-2,6-diazaspiro[3.3]heptan-2-yl}-1-methylpyrazol-3-yl)-3-methylbutanoyl]-N-[(1S)-1-[4-(4-methyl-1,3-thiazol-5-yl)phenyl]ethyl]pyrrolidine-2-carboxamide (Compound 194-001).

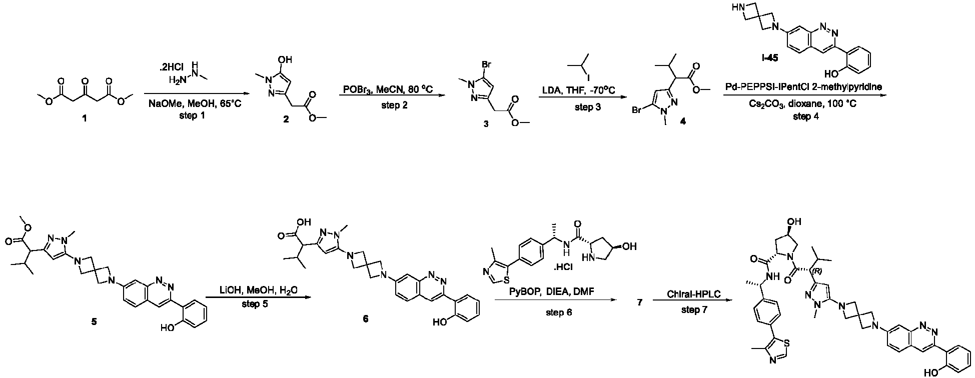

### Step 1: Preparation of methyl 2-(5-hydroxy-1-methylpyrazol-3-yl)acetate (Intermediate 2).

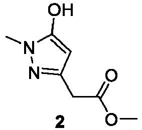

A solution of 1,5-dimethyl 3-oxopentanedioate (5.85 g, 33.615 mmol, 1.00 equiv) in MeOH (60 mL) was treated with methylhydrazine dihydrochloride (4 g, 33.615 mmol, 1.00 equiv) for 1 h at room temperature under nitrogen atmosphere followed by the addition of NaOMe (5.45 g, 100.845 mmol, 3 equiv) in portions at room temperature. The resulting mixture was stirred for 3 h at 65°C. The resulting crude solid was dried in an oven under reduced pressure. The residue was purified by silica gel column chromatography, eluted with CH₂Cl₂ / MeOH (10:1) to afford intermediate **2** (5 g, 87.41%) as a light-yellow solid. LCMS (ESI) m/z: [M+H]⁺ = 171.

### Step 2: Preparation of methyl 2-(5-bromo-1-methylpyrazol-3-yl)acetate (Intermediate 3).

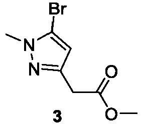

A mixture of intermediate **2** (4 g, 23.506 mmol, 1 equiv) and POBr₃(33.69 g, 117.530 mmol, 5 equiv) in MeCN (40 mL) were stirred for overnight at 80°C. The mixture was neutralized to pH 7 with saturated NaHCO₃ (aq.). The aqueous layer was extracted with EtOAc (3 x 300 mL). The combined organic layers were dried over anhydrous Na₂SO₄. After filtration, the filtrate was concentrated under reduced pressure. The residue was dissolved in DMF (5 mL). The residue was purified by reversed-phase flash chromatography with the following conditions: column, C18 silica gel; mobile phase, MeCN in Water (0.1% FA), 0 to 100% gradient in 30 min; detector, UV 254 nm. to afford intermediate 3 (852 mg, 15.55%) as a light-yellow oil. LCMS (ESI) m/z: [M+H]⁺ =233/235. ¹H NMR (400 MHz, DMSO-d6) δ 6.34 (s, 1H), 3.76 (s, 3H), 3.62 (s, 3H), 3.61 (s, 2H).

### Step 3: Preparation of methyl 2-(5-bromo-1-methylpyrazol-3-yl)-3-methylbutanoate (Intermediate 4).

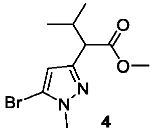

A solution of intermediate 3 (709 mg, 3.042 mmol, 1 equiv) in THF (10 mL) was treated with LDA (in 2M THF) (4.56 mL, 9.126 mmol, 3 equiv) for 30 min at -70°C under nitrogen atmosphere followed by the addition of 2-iodopropane (1.03 g, 6.084 mmol, 2 equiv) dropwise at -70°C. The resulting mixture was stirred for overnight at room temperature under nitrogen atmosphere. The resulting crude oil was dried in an oven under reduced pressure. The residue was dissolved in DMF (5 mL). The residue was purified by reversed-phase flash chromatography with the following conditions: column, C18 silica gel; mobile phase, MeCN in Water (0.1% FA), 0 to 100% gradient in 30 min; detector, UV 220 nm. to afford intermediate **4** (307 mg, 36.68%) as a light yellow oil. LCMS (ESI) m/z: [M+H]⁺ = 275.

### Step 4: Preparation of methyl 2-(5-{6-[3-(2-hydroxyphenyl)cinnolin-7-yl]-2,6-diazaspiro[3.3]heptan-2-yl}-1-methylpyrazol-3-yl)-3-methylbutanoate (Intermediate 5).

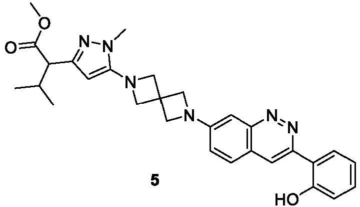

A mixture of intermediate **4** (297 mg, 1.079 mmol, 1 equiv), Cs₂CO₃ (1.41 g, 4.316 mmol, 4 equiv), Pd-PEPPSI-IPentCl 2-methylpyridine (o-picoline) (45.40 mg, 0.054 mmol, 0.05 equiv) and **I-45** (687.34 mg, 2.158 mmol, 2 equiv) in dioxane (5 mL) were stirred for 6 h at 100°C under nitrogen atmosphere. The residue was purified by silica gel column chromatography, eluted with CH₂Cl₂ / MeOH (10:1) to afford intermediate **5** (287 mg, 51.87%) as a yellow solid. LCMS (ESI) m/z: [M+H]⁺ = 513.

### Step 5: Preparation of 2-(5-{6-[3-(2-hydroxyphenyl)cinnolin-7-yl]-2,6-diazaspiro[3.3]heptan-2-yl}-1-methylpyrazol-3-yl)-3-methylbutanoic acid (Intermediate 6).

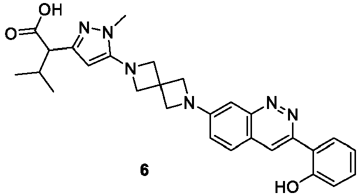

A mixture of intermediate **5** (287 mg, 0.560 mmol, 1 equiv) and LiOH.H₂O (234.92 mg, 5.600 mmol, 10 equiv) in MeOH (2 mL) and H₂O (1 mL) were stirred overnight at room temperature. The mixture was acidified to pH 4 with HCl (aq. 1M). The aqueous layer was extracted with EtOAc (3x100 mL). The combined organic layers were dried over anhydrous Na₂SO₄. After filtration, the filtrate was concentrated under reduced pressure. to afford intermediate **6** (288 mg, crude) as a light-yellow solid. LCMS (ESI) m/z: [M+H]⁺ =499.

### Step 6: Preparation of (2S,4R)-4-hydroxy-1-[2-(5-{6-[3-(2-hydroxyphenyl)cinnolin-7-yl]-2,6-diazaspiro[3.3]heptan-2-yl}-1-methylpyrazol-3-yl)-3-methylbutanoyl]-N-[(1S)-1-[4-(4-methyl-1,3-thiazol-5-yl)phenyl]ethyl]pyrrolidine-2-carboxamide (Intermediate 7).

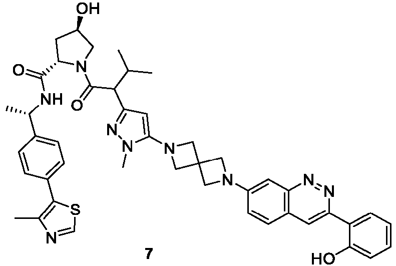

To a stirred mixture of intermediate **6** (278 mg, 0.558 mmol, 1 equiv), PyBOP (435.24 mg, 0.837 mmol, 1.5 equiv) and (2S,4R)-4-hydroxy-N-[(1S)-1-[4-(4-methyl-1 ,3-thiazol-5-yl)phenyl]ethyl]pyrrolidine-2-carboxamide (277.20 mg, 0.837 mmol, 1.5 equiv) in DMF (5 mL) were added DIEA (288.26 mg, 2.232 mmol, 4 equiv) dropwise at room temperature. The resulting mixture was stirred for 3 h at room temperature. The reaction was monitored by LCMS. After completion of reaction, the residue was purified by reversed-phase flash chromatography with the following conditions: column, C18 silica gel; mobile phase, MeCN in water (10mmol/L NH₄HCO₃), 0 to 100% gradient in 30 min; detector, UV 254 nm. to afford intermediate 7 (232 mg, 51.24%) as a light yellow solid. LCMS (ESI) m/z: [M+H]⁺ = 812.

### Step 7: Preparation of (2S,4R)-4-hydroxy-1-[(2R)-2-(5-{6-[3-(2-hydroxyphenyl)cinnolin-7-yl]-2,6-diazaspiro[3.3]heptan-2-yl}-1-methylpyrazol-3-yl)-3-methylbutanoyl]-N-[(1S)-1-[4-(4-methyl-1,3-thiazol-5-yl)phenyl]ethyl]pyrrolidine-2-carboxamide (Compound 194).

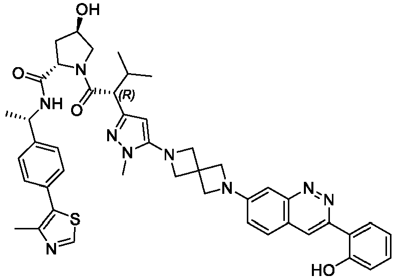

Intermediate 7 (232 mg) was purified by PREP_CHIRAL_HPLC with the following conditions (Column: CHIRALPAK IC-3, 4.6*50 mm, 3 um; Mobile Phase A: MtBE (0.1%DEA): EtOH=50: 50; to afford **194** (101.8 mg, 42.56%) (second peak) as a yellow solid. ¹H NMR (400 MHz, DMSO-d6) δ 12.81 (s, 1H), 8.99 (s, 1H), 8.76 (s, 1H), 8.33 (d, J = 7.7 Hz, 1H), 8.06 - 8.00 (m, 1H), 7.96 (d, J = 9.1 Hz, 1H), 7.48 - 7.41 (m, 2H), 7.40 - 7.35 (m, 2H), 7.35 - 7.27 (m, 2H), 7.06 - 6.96 (m, 3H), 5.46 (s, 1H), 5.05 (d, J = 3.6 Hz, 1H), 4.98 - 4.88 (m, 1H), 4.38 - 4.23 (m, 6H), 4.11 - 4.02 (m, 4H), 3.75 - 3.67 (m, 1H), 3.53 - 3.44 (m, 4H), 3.18 (d, J = 10.4 Hz, 1H), 2.49 - 2.44 (m, 3H), 2.18 - 2.07 (m, 1H), 1.97 - 1.92 (m, 1H), 1.85 - 1.75 (m, 1H), 1.39 (d, J = 7.0 Hz, 3H), 0.92 (d, J = 6.6 Hz, 3H), 0.69 (d, J = 6.7 Hz, 3H). LCMS (ESI) m/z: [M+H]⁺ =812.40.

### Preparation of (2S,4R)-4-hydroxy-1-((R)-2-(3-(6-(3-(2-hydroxyphenyl)cinnolin-7-yl)-2,6-diazaspiro[3.3]heptan-2-yl)phenyl)-3-methylbutanoyl)-N-((S)-1-(4-(4-methylthiazol-5-yl)phenyl)ethyl)pyrrolidine-2-carboxamide (Compound 174)

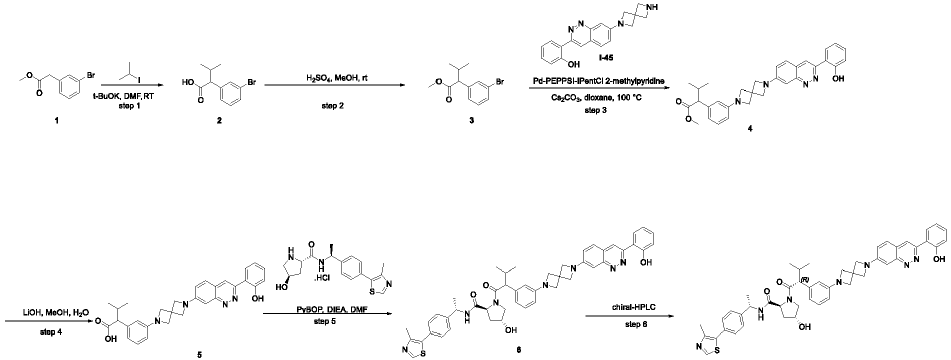

### Step 1: Preparation of 2-(3-bromophenyl)-3-methylbutanoic acid (Intermediate 2)

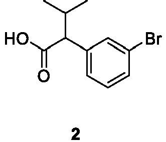

A mixture of methyl 2-(3-bromophenyl)acetate (2 g, 8.731 mmol, 1 equiv), 2-iodopropane (2.97 g, 17.462 mmol, 2 equiv) and t-BuOK (2.94 g, 26.193 mmol, 3 equiv) in DMF (15 mL) was stirred overnight at room temperature. Desired product could be detected by LCMS. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in intermediate **2** (2.0 g, 89.3%) as a colorless oil. LCMS (ESI) m/z: [M+H]⁺ = 257/259

### Step 2: Preparation of methyl 2-(3-bromophenyl)-3-methylbutanoate (Intermediate 3)

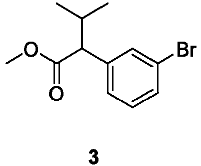

A mixture of intermediate **2** (2 g, 7.778 mmol, 1.00 equiv) and H₂SO₄ (0.41 mL, 7.778 mmol, 1 equiv) in MeOH (20 mL) was stirred for 2h at room temperature. The reaction was quenched with Water/Ice at room temperature. The aqueous layer was extracted with EtOAc (3x100 mL). The combined organic layers were washed with brine (1 x 250 mL), dried over anhydrous Na₂SO₄. After filtration, the filtrate was concentrated under reduced pressure. The residue was purified by silica gel column chromatography, eluted with PE / EA (3:1) to afford intermediate **3** (1.2 g, 47.79%) as a colorless oil. LCMS (ESI) m/z [M+H]⁺ =271/273

### Step 3: Preparation of methyl 2-(3-(6-(3-(2-hydroxyphenyl)cinnolin-7-yl)-2,6-diazaspiro[3.3]heptan-2-yl)phenyl)-3-methylbutanoate (Intermediate 4)

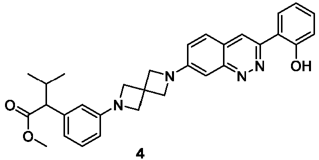

To a stirred mixture of **I-45** (120 mg, 0.377 mmol, 1 equiv) and intermediate **3** (122.64 mg, 0.452 mmol, 1.20 equiv) in NMP (3 mL) were added Pd-PEPPSI-IPentCl 2-methylpyridine (o-picoline (1.32 mg, 0.002 mmol, 0.05 equiv) and Cs₂CO₃ (0.62 g, 1.893 mmol, 5.02 equiv) in portions at room temperature. The resulting mixture was stirred for 2 h at 100°C under nitrogen atmosphere. The residue was purified by reversed-phase flash chromatography with the following conditions: column, C18 silica gel; mobile phase, MeCN in Water (0.1% FA), 0% to 100% gradient in 30 min; detector, UV 254 nm. This resulted in intermediate **4** (60 mg, 31.30%) as a yellow solid. LCMS (ESI) m/z: [M+H]+ = 509.

### Step 4: Preparation of 2-(3-(6-(3-(2-hydroxyphenyl)cinnolin-7-yl)-2,6-diazaspiro[3.3]heptan-2-yl)phenyl)-3-methylbutanoic acid (Intermediate 5)

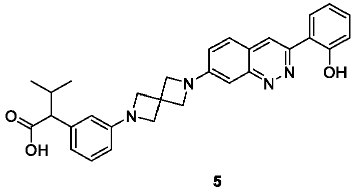

A solution of 4 (85 mg, 0.167 mmol, 1 equiv) and LiOH.H₂O (70.12 mg, 1.670 mmol, 10 equiv) in MeOH (2 mL) and H₂O (1 mL) was stirred for 1h at room temperature. The mixture was acidified to pH 3 with HCl (aq.). The resulting mixture was extracted with EtOAc (3 x 25 mL). The combined organic layers were washed with brine (1 x 25 mL), dried over anhydrous Na₂SO₄. After filtration, the filtrate was concentrated under reduced pressure. This resulted in intermediate **5** (61 mg, 73.80%) as an orange solid. LCMS (ESI) m/z: [M+H]+ = 495.

### Step 5: Preparation of (2S,4R)-4-hydroxy-1-(2-(3-(6-(3-(2-hydroxyphenyl)cinnolin-7-yl)-2,6-diazaspiro[3.3]heptan-2-yl)phenyl)-3-methylbutanoyl)-N-((S)-1-(4-(4-methylthiazol-5-yl)phenyl)ethyl)pyrrolidine-2-carboxamide (Intermediate 6)

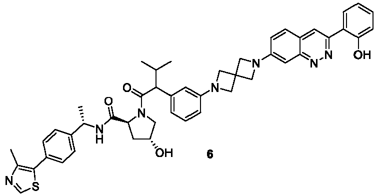

To a stirred mixture of intermediate **5** (56 mg, 0.116 mmol, 1 equiv) and (2S,4R)-4-hydroxy-N-[(1S)-1-[4-(4-methyl-1,3-thiazol-5-yl)phenyl]ethyl]pyrrolidine-2-carboxamide (45.96 mg, 0.139 mmol, 1.2 equiv) in DMF (2 mL) were added PyBOP (120.27 mg, 0.232 mmol, 2 equiv) and DIEA (74.68 mg, 0.580 mmol, 5 equiv) in portions at room temperature. The resulting mixture was stirred for 2 h at room temperature. The residue was purified by reversed-phase flash chromatography with the following conditions: column, C18 silica gel; mobile phase, MeCN in Water (0.1% FA), 0% to 100% gradient in 25 min; detector, UV 254 nm. This resulted in intermediate **6** (29 mg, 31.06%) as an orange solid. LCMS (ESI) m/z: [M+H]⁺ = 808.

### Step 6: Preparation of (2S,4R)-4-hydroxy-1-((R)-2-(3-(6-(3-(2-hydroxyphenyl)cinnolin-7-yl)-2,6-diazaspiro[3.3]heptan-2-yl)phenyl)-3-methylbutanoyl)-N-((S)-1-(4-(4-methylthiazol-5-yl)phenyl)ethyl)pyrrolidine-2-carboxamide (Compound 174)

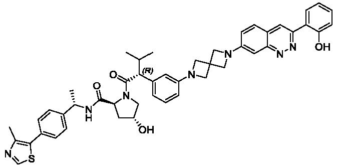

Intermediate **6** was purified by Chiral-Prep-HPLC with the following conditions: Column, CHIRALPAK ID, 2*25 cm, 5 um; mobile phase, MtBE(10mM NH₃-MeOH) and MeOH- (hold 50% MeOH- in 13 min); Detector, UV 254 nm. This resulted in **174** (6.9 mg) as a yellow solid. ¹H NMR (300 MHz, Methanol-d4) δ 8.87 (s, 1H), 8.61 (s, 1H), 7.93 (dd, J = 17.6, 8.4 Hz, 2H), 7.44 (s, 4H), 7.37 - 7.20 (m, 2H), 7.15 (t, J = 7.9 Hz, 1H), 7.06 - 6.94 (m, 3H), 6.76 (d, J = 7.4 Hz, 1H), 6.57 (s, 1H), 6.45 (d, J = 8.0 Hz, 1H), 5.05 (d, J = 7.0 Hz, 1H), 4.46 (t, J = 8.1 Hz, 2H), 4.31 (s, 4H), 4.07 (s, 4H), 3.96 - 3.85 (m, 1H), 3.54 (d, J = 9.3 Hz, 1H), 3.23 (d, J = 10.4 Hz, 1H), 2.48 (s, 3H), 2.33 (s, 1H), 2.12 - 1.92 (s, 1H), 2.0 - 1.85 (s, 1H), 1.54 (d, J = 7.0 Hz, 3H), 1.04 (d, J = 6.4 Hz, 3H), 0.69 (d, J = 6.6 Hz, 3H). LCMS (ESI) m/z: [M+H]⁺ = 808.36.

### Preparation of (2S,4R)-4-hydroxy- 1-[(2R)-2-(2-{6-[3-(2-hydroxyphenyl)cinnolin-7-yl]-2,6-diazaspiro[3.3]heptan-2-yl}pyridin-4-yl)-3-methylbutanoyl]-N-[(1S)-1-[4-(4-methyl-1,3-thiazol-5-yl)phenyl]ethyl]pyrrolidine-2-carboxamide (Compound 175)

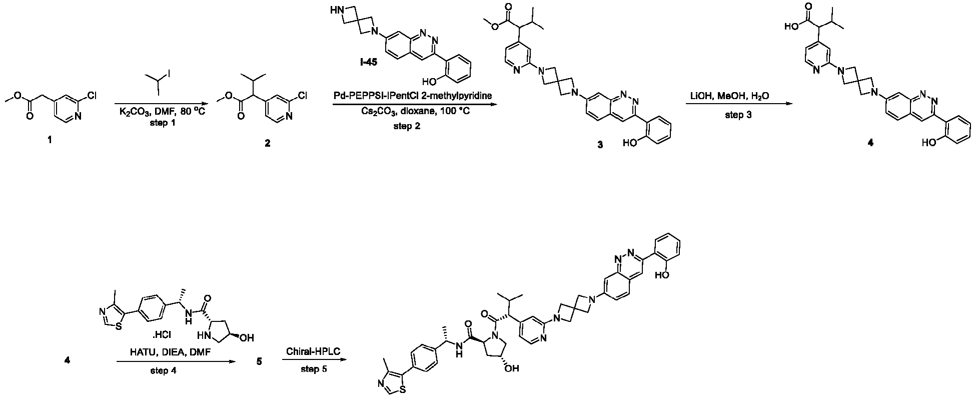

### Step 1: Preparation of methyl 2-(2-chloropyridin-4-yl)-3-methylbutanoate (Intermediate 2)

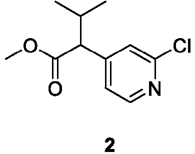

To a stirred mixture of methyl 2-(2-chloropyridin-4-yl)acetate (1 g, 5.388 mmol, 1 equiv) and 2-iodopropane (1.83 g, 10.776 mmol, 2 equiv) in THF was added Cs₂CO₃ (5.27 g, 16.164 mmol, 3 equiv) in portions at room temperature. The resulting mixture was stirred for overnight at 60 °C. The resulting mixture was diluted with water (30 mL). The resulting mixture was extracted with EtOAc (30 x mL). The combined organic layers were washed with water (3x30 mL), dried over anhydrous Na₂SO₄. After filtration, the filtrate was concentrated under reduced pressure. The residue was purified by silica gel column chromatography, eluted with PE / EA (4:1) to afford intermediate **2** (1 g, 81.52%) as a yellow oil. LCMS (ESI) m/z: [M+H]⁺ = 228.

### Step 2: Preparation of methyl 2-(2-{6-[3-(2-hydroxyphenyl)cinnolin-7-yl]-2,6-diazaspiro[3.3]heptan-2-yl}pyridin-4-yl)-3-methylbutanoate (Intermediate 3)

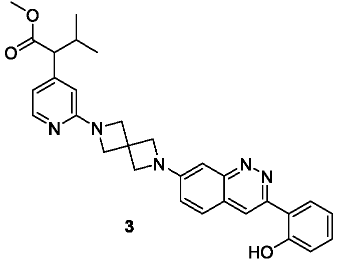

To a stirred solution of intermediate **2** (400 mg, 1.757 mmol, 1 equiv) and **I-45** (559.32 mg, 1.757 mmol, 1 equiv) in dioxane were added Cs₂CO₃ (2.29 g, 7.028 mmol, 4 equiv) and Pd-PEPPSI-IPentCl 2-methylpyridine (o-picoline (73.89 mg, 0.088 mmol, 0.05 equiv) in portions at room temperature under nitrogen atmosphere. The resulting mixture was stirred for 2 h at 100 °C under nitrogen atmosphere. The residue was purified by silica gel column chromatography, eluted with CH₂Cl₂ / MeOH (9:1) to afford intermediate 3 (340 mg, 37.98%) as a red solid. LCMS (ESI) m/z: [M+H]⁺ = 510.

### Step 3: Preparation of 2-(2-{6-[3-(2-hydroxyphenyl)cinnolin-7-yl]-2,6-diazaspiro[3.3]heptan-2-yl}pyridin-4-yl)-3-methylbutanoic acid (Intermediate 4)

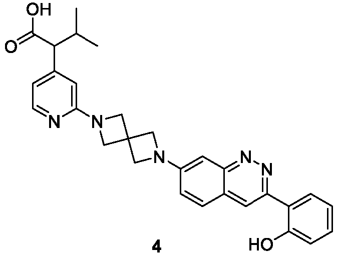

To a stirred solution of intermediate **3** (330 mg, 0.648 mmol, 1 equiv) in MeOH were added LiOH.H₂O (271.71 mg, 6.480 mmol, 10 equiv) and H₂O (1 mL) in portions at room temperature. The resulting mixture was stirred for 1 h at 60 °C. The residue was acidified to pH 4 with HCl (aq.). The precipitated solids were collected by filtration and washed with water (3x10 mL). The resulting mixture was concentrated under reduced pressure. This resulted in intermediate **4** (250 mg, 77.90%) as a red solid. LCMS (ESI) m/z [M+H]⁺ = 496.

### Step 4: Preparation of (2S,4R)-4-hydroxy-1-[2-(2-{6-[3-(2-hydroxyphenyl)cinnolin-7-yl]-2,6-diazaspiro[3.3]heptan-2-yl}pyridin-4-yl)-3-methylbutanoyl]-N-[(1S)-1-[4-(4-methyl-1,3-thiazol-5-yl)phenyl]ethyl]pyrrolidine-2-carboxamide (Intermediate 5)

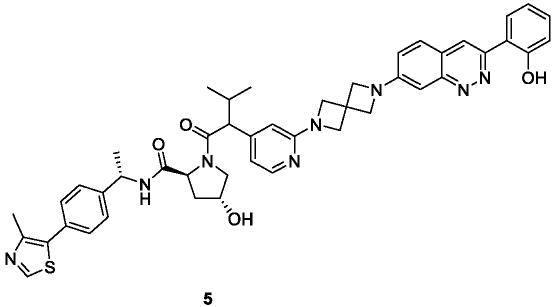

To a stirred mixture of intermediate **4** (240 mg, 0.484 mmol, 1 equiv) and (2S,4R)-4-hydroxy-N-[(1S)-1-[4-(4-methyl-1,3-thiazol-5-yl)phenyl]ethyl]pyrrolidine-2-carboxamide (192.61 mg, 0.581 mmol, 1.2 equiv) in DMF were added PyBOP (378.03 mg, 0.726 mmol, 1.5 equiv) and DIEA (250.37 mg, 1.936 mmol, 4 equiv) in portions at room temperature. The resulting mixture was stirred for 1 h at room temperature. The residue was purified by reversed-phase flash chromatography with the following conditions: column, C18 silica gel; mobile phase, MeCN in Water (10mmol/L NH₄HCO₃), 10% to 80% gradient in 25 min; detector, UV 254 nm. The crude product (120mg) was purified by Prep-HPLC with the following conditions (Column: XBridge Shield RP18 OBD Column, 30*150 mm, 5µm; Mobile Phase A: Water (10 mmol/L NH₄HCO₃+0.05%NH₃.H₂O), Mobile Phase B: ACN; Flow rate: 60 mL/min mL/min; Gradient: 41% B to 55% B in 10 min; Wave Length: 254nm/220nm nm; RT1(min): 9.8) to afford intermediate **5** (85 mg, 21.70%) as a red solid. LCMS (ESI) m/z: [M+H]⁺ = 809.

### Step 5: Preparation of (2S,4R)-4-hydroxy-1-[(2R)-2-(2-{6-[3-(2-hydroxyphenyl)cinnolin-7-yl]-2,6-diazaspiro[3.3]heptan-2-yl}pyridin-4-yl)-3-methylbutanoyl]-N-[(1S)-1-[4-(4-methyl-1,3-thiazol-5-yl)phenyl]ethyl]pyrrolidine-2-carboxamide (Compound 175-001)

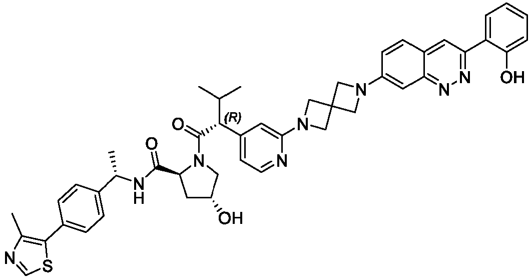

Intermediate **45** was purified by Prep-CHIRAL-HPLC with the following conditions (Column: CHIRAL ART Amylose-SA 2*25 cm, 5 µm; Mobile Phase A: MtBE(10mM NH3-MeOH), Mobile Phase B: EtOH; Flow rate: 20 mL/min; Gradient: isocratic 35; Wave Length: 270/214 nm; RT1(min): 3.105; RT2(min): 14.230; Sample Solvent: DCM: EtOH=9: 1--HPLC; Injection Volume: 2.2 mL; Number Of Runs: 3) to afford **175** (28.5 mg, 33.53%) as a yellow solid. ¹H NMR (300 MHz, DMSO-d6) δ 12.83 (d, J = 3.6 Hz, 1H), 8.99 (s, 1H), 8.77 (s, 1H), 8.40 (d, J = 7.6 Hz, 1H), 8.08 - 7.90 (m, 3H), 7.50 - 7.24 (m, 6H), 7.01 (d, J = 8.0 Hz, 3H), 6.69 (d, J = 5.3 Hz, 1H), 6.42 (s, 1H), 5.05 - 4.86 (m, 2H), 4.32 (s, 6H), 4.17 (s, 4H), 3.82 - 3.70 (m, 1H), 3.38 (s, 1H), 3.26 (d, J = 10.2 Hz, 1H), 2.46 (s, 3H), 2.27 (s, 1H), 1.96 (s, 1H), 1.77 (d, J = 6.7 Hz, 1H), 1.39 (d, J = 7.0 Hz, 3H), 0.96 (d, J = 6.4 Hz, 3H), 0.64 (d, J = 6.7 Hz, 3H). LCMS (ESI) m/z: [M+H]⁺ = 809.30.

Compound in Table 18 was prepared using a procedure similar to the one used above for the preparation of **Compound 175** using the appropriate aryl bromide and amine.

**Table 18.**

| **No.** | **Name** | **LCMS (ESI) m/z** | **¹H NMR** |
|---|---|---|---|
| **160** | (2S,4R)-4-hydroxy-1-[(2R)-2-(2-{6-[3-(2-hydroxyphenyl)cinnolin-6-yl]-2,6-diazaspiro[3.3]heptan-2-yl}pyridin-4-yl)-3-methylbutanoyl]-N-[(1S)-1-[4-(4-methyl-1,3-thiazol-5-yl)phenyl]ethyl]pyrrolidine-2-carboxamide | 809.35 | ¹H NMR (300 MHz, DMSO-d6) δ 13.33 (s, 1H), 8.99 (s, 1H), 8.56 (s, 1H), 8.40 (d, J = 7.7 Hz, 1H), 8.21 (d, J = 9.2 Hz, 1H), 8.09 - 7.93 (m, 2H), 7.44 (d, J = 8.4 Hz, 2H), 7.41 - 7.25 (m, 4H), 7.06 - 6.96 (m, 2H), 6.73 (s, 1H), 6.60 (d, J = 2.4 Hz, 1H), 6.48 (s, 1H), 5.11 - 4.83 (m, 2H), 4.35 (s, 4H), 4.31 (d, J = 8.0 Hz, 2H), 4.21 (s, 4H), 3.75 (dd, J = 10.5, 4.5 Hz, 1H), 3.45 (s, 2H), 2.46 (s, 3H), 2.23 (d, J = 22.5 Hz, 1H), 1.95 (d, J = 9.6 Hz, 1H), 1.79 (q, J = 6.7 Hz, 1H), 1.52 - 1.32 (m, 3H), 0.96 (d, J = 6.4 Hz, 3H), 0.68 (dd, J = 18.2, 6.7 Hz, 3H). |

### Preparation of (2S,4R)-4-hydroxy-1-[(2R)-2-{3-[3-(2-hydroxyphenyl)cinnolin-6-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Compound 97).

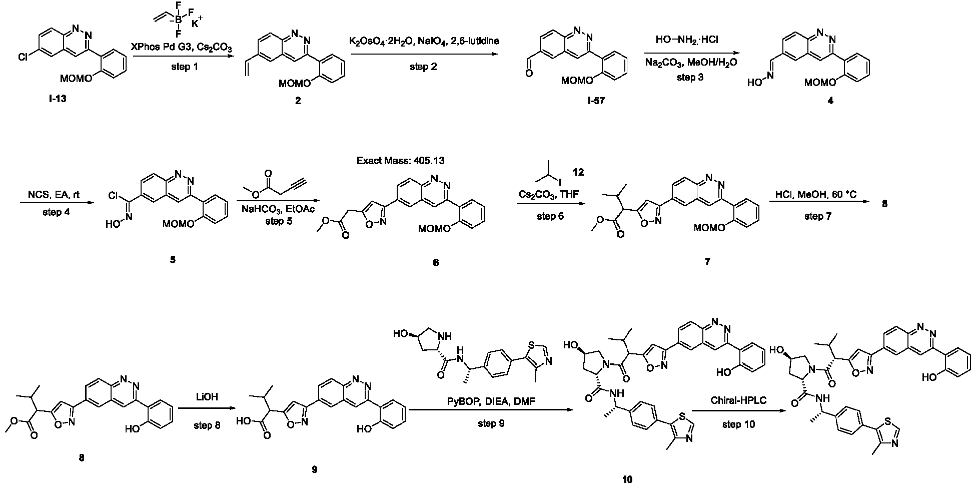

### Step 1: Preparation of 6-ethenyl-3-[2-(methoxymethoxy)phenyl]cinnoline (Intermediate 2).

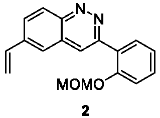

To a stirred solution of **I-13** (1.5 g, 4.988 mmol, 1 equiv) and ethenyltrifluoro-lambda4-borane potassium (3.34 g, 24.940 mmol, 5 equiv) in dioxane (25 mL) and H₂O (5 mL) were added XPhos Pd G3 (0.84 g, 0.998 mmol, 0.2 equiv) and Cs₂CO₃ (4.88 g, 14.964 mmol, 3 equiv). The resulting mixture was stirred overnight at 80 °C under nitrogen atmosphere. The mixture was diluted with water (50 mL), extracted with EtOAc (3 x 100 mL). The combined organic layers were washed with brine (1x 50 mL), dried over anhydrous sodium sulfate. After filtration, the filtrate was concentrated under reduced pressure. The residue was purified by silica gel column chromatography, eluted with PE / EtOAc (1:1) to afford intermediate **2** (1.5 g, 97.7%) as a light yellow oil. LCMS (ESI) m/z: [M+H]⁺ = 293.

### Step 2: Preparation of 3-[2-(methoxymethoxy)phenyl]cinnoline-6-carbaldehyde (I-57).

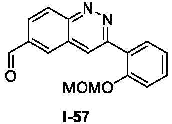

To a stirred solution of intermediate **2** (1.5 g, 5.131 mmol, 1 equiv) and NaIO₄ (4.39 g, 20.524 mmol, 4 equiv) in dioxane (80 mL) and H₂O (40 mL) were added K₂OsO₄.2H₂O (0.09 g, 0.257 mmol, 0.05 equiv) and 2,6-lutidine (1.10 g, 10.262 mmol, 2 equiv) at 0 °C. The resulting mixture was stirred for 1 h at room temperature. The reaction was quenched with sat. sodium hyposulfite (aq.) at 0 °C. The resulting mixture was extracted with EtOAc (3 x 100 mL). The combined organic layers were washed with brine (2 x 100 mL), dried over anhydrous sodium sulfate. After filtration, the filtrate was concentrated under reduced pressure. This resulted in **I-57** (1.4 g, 85.2%) as a yellow solid. The crude product was used in the next step directly without further purification. LCMS (ESI) m/z: [M+H]⁺ =295.

### Step 3: Preparation of (E)-N-({3-[2-(methoxymethoxy)phenyl]cinnolin-6-yl}methylidene)hydroxylamine (Intermediate 4).

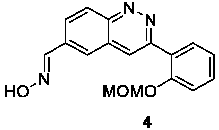

To a stirred solution of **I-57** (1.3 g, 4.417 mmol, 1 equiv) and hydroxylamine hydrochloride (0.61 g, 8.834 mmol, 2 equiv) in MeOH (40 mL) and H₂O (16 mL) was added Na₂CO₃ (1.40 g, 13.251 mmol, 3 equiv). The resulting mixture was stirred for 1 h at room temperature. The mixture was concentrated under vacuum and diluted with water (50 mL), extracted with EtOAc (3 x 100 mL). The combined organic layers were washed with brine (2 x 100 mL), dried over anhydrous sodium sulfate. After filtration, the filtrate was concentrated under reduced pressure. This resulted in intermediate **4** (1.26 g, 84.84%) as a light yellow solid. The crude product was used in the next step directly without further purification. LCMS (ESI) m/z: [M+H]⁺ = 310.

### Step 4: Preparation of (Z)-N-hydroxy-3-[2-(methoxymethoxy)phenyl]cinnoline-6-carbonimidoyl chloride (Intermediate 5).

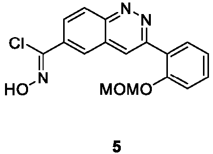

A solution of intermediate **4** (1.21 g, 3.912 mmol, 1 equiv) and NCS (0.78 g, 5.868 mmol, 1.5 equiv) in EtOAc (20 mL) was stirred overnight at room temperature. The resulting mixture was extracted with EtOAc (3 x 150 mL). The combined organic layers were washed with brine (3 x 150 mL), dried over anhydrous sodium sulfate. After filtration, the filtrate was concentrated under reduced pressure. This resulted in intermediate **5** (1.4 g, 95.7%) as a light yellow solid. The crude product was used in the next step directly without further purification. LCMS (ESI) m/z: [M+H]⁺ = 344.

### Step 5: Preparation of methyl 2-(3-{3-[2-(methoxymethoxy)phenyl]cinnolin-6-yl}-1,2-oxazol-5-yl)acetate (Intermediate 6).

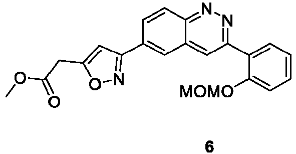

To a stirred solution of intermediate **5** (1.35 g, 3.927 mmol, 1 equiv) and NaHCO₃ (0.99 g, 11.781 mmol, 3 equiv) in EtOAc (20 mL) was added methyl but-3-ynoate (0.77 g, 7.854 mmol, 2 equiv) dropwise at 0 °C. The resulting mixture was stirred overnight at room temperature. The residue was purified by silica gel column chromatography, eluted with PE / EtOAc (1:2) to afford intermediate 6 (576.0 mg, 35.4%) as a light yellow solid. LCMS (ESI) m/z: [M+H]⁺ =406.

### Step 6: Preparation of methyl 2-(3-{3-[2-(methoxymethoxy)phenyl]cinnolin-6-yl}-1,2-oxazol-5-yl)-3-methylbutanoate (Intermediate 7).

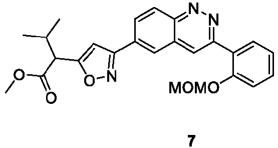

To a stirred solution of intermediate **6** (400.0 mg, 0.987 mmol, 1 equiv) and Cs₂CO₃ (642.9 mg, 1.974 mmol, 2 equiv) in THF (10 mL) was added 2-iodopropane (335.4 mg, 1.974 mmol, 2 equiv). The resulting mixture was stirred overnight at 60 °C. The resulting mixture was concentrated under reduced pressure. The crude product was purified by reverse phase flash with the following conditions (Mobile Phase A: Water (0.1% FA), Mobile Phase B: ACN; Flow rate: 35 mL/min; Gradient: 0% B to 100% B in 40 min; 254/220 nm) to afford intermediate **7** (86.0 mg, 17.5%) as a light yellow solid. LCMS (ESI) m/z: [M+H]⁺ = 448.

### Step 7: Preparation of methyl 2-{3-[3-(2-hydroxyphenyl)cinnolin-6-yl]-1,2-oxazol-5-yl)-3-methylbutanoate (Intermediate 8).

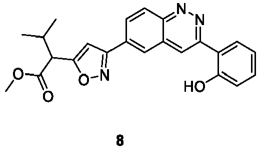

A solution of intermediate **7** (86.0 mg, 0.192 mmol, 1 equiv) in HCl (0.5 mL) and MeOH (3 mL) was stirred for 1 h at 60 °C under nitrogen atmosphere. The resulting mixture was concentrated under reduced pressure. This resulted in methyl intermediate **8** (86.0 mg, crude) as a yellow solid. The crude product was used in the next step directly without further purification. LCMS (ESI) m/z: [M+H]⁺ =404.

### Step 8: Preparation of 2-{3-[3-(2-hydroxyphenyl)cinnolin-6-yl]-1,2-oxazol-5-yl}-3-methylbutanoic acid (Intermediate 9).

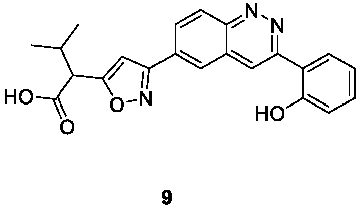

A solution of methyl intermediate **8** (86 mg, 0.213 mmol, 1 equiv) and LiOH (51.05 mg, 2.130 mmol, 10 equiv) in THF (3 mL) and H₂O (1 mL) was stirred for 1 h at 60 °C. The mixture was acidified to pH 6 with HCl (aq.). The resulting mixture was concentrated under reduced pressure. The crude product was purified by reverse phase flash with the following conditions (Mobile Phase A: Water (0.1% FA), Mobile Phase B: ACN; Flow rate: 35 mL/min; Gradient: 0% B to 100% B in 40 min; 254/220 nm) to afford intermediate 9 (51.0 mg, 58.3%) as a light yellow solid. LCMS (ESI) m/z: [M+H]⁺ =390.

### Step 9: Preparation of (2S,4R)-4-hydroxy-1-[(2R)-2-{3-[3-(2-hydroxyphenyl)cinnolin-6-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Intermediate 10).

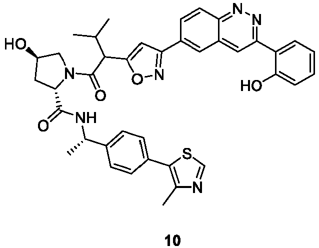

To a stirred solution of intermediate **9** (51 mg, 0.131 mmol, 1 equiv) and (2S,4R)-4-hydroxy-N-[(1S)-1-[4-(4-methyl-1,3-thiazol-5-yl) phenyl] ethyl]pyrrolidine-2-carboxamide (43.4 mg, 0.131 mmol, 1 equiv) in DMF (3 mL) were added PyBOP (136.3 mg, 0.262 mmol, 2 equiv) and DIEA (50.7 mg, 0.393 mmol, 3 equiv). The resulting mixture was stirred for 2 h at room temperature. The residue was purified by reverse phase flash with the following conditions (Mobile Phase A: Water (0.1% FA), Mobile Phase B: ACN; Flow rate: 35 mL/min; Gradient: 0% B to 100% B in 40 min; 254/220 nm) to afford intermediate **10** (40.0 mg, 39.9%) as a light yellow solid. LCMS (ESI) m/z: [M+H]⁺ =703.

### Step 10: Preparation of (2S,4R)-4-hydroxy-1-[(2R)-2-{3-[3-(2-hydroxyphenyl)cinnolin-6-yl]-1,2-oxazol-5-yl}-3-methylbutanoyl]-N-[(1S)-1-[4-(4-methyl-1,3-thiazol-5-yl)phenyl]ethyl]pyrrolidine-2-carboxamide (Compound 97-001).

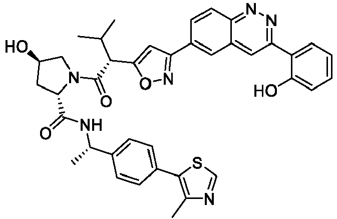

The intermediate **10** (40 mg) was separated by Chiral-HPLC with the following conditions (Column: CHIRALPAK IA-3, 4.6*50mm, 3µm; Mobile Phase A: MtBE(0.1%DEA): ETOH=70: 30; Flow rate: 1 mL/min; Gradient: 0% B to 0% B; Injection Volume: 5ul mL; Number of Runs: 0) to afford Compound **97-001** (13.4 mg, 35.2%) as a light yellow solid. LCMS (ESI) m/z: [M+H]⁺ =703.25. ¹H NMR (300 MHz, DMSO-d6) δ 11.42 (s, 1H), 8.97 (d, J = 13.9 Hz, 2H), 8.73 (d, J = 1.9 Hz, 1H), 8.58 (t, J = 8.1 Hz, 1H), 8.49 - 8.37 (m, 2H), 8.13 (dd, J = 7.8, 1.7 Hz, 1H), 7.52 - 7.31 (m, 5H), 7.23 (s, 1H), 7.13 - 7.01 (m, 2H), 5.12 (d, J = 3.7 Hz, 1H), 4.96 - 4.88 (m, 1H), 4.42 (t, J = 7.9 Hz, 1H), 4.32 (s, 1H), 3.97 (d, J = 9.6 Hz, 1H), 3.84 - 3.70 (m, 1H), 3.58 (t, J = 11.0 Hz, 1H), 2.46 (s, 3H), 2.42 (s, 1H), 2.05 (t, J = 10.3 Hz, 1H), 1.87 - 1.71 (m, 1H), 1.55 - 1.36 (m, 3H), 1.05 (d, J = 6.5 Hz, 3H), 0.92 - 0.84 (m, 3H).

Compounds in Table 19 were prepared using procedures similar to the one used above for the preparation of **Compound 97** using the appropriate chloro-cinnolines.

**Table 19.**

| **No.** | **Name** | **LCMS (ESI) m/z** | **¹H NMR** |
|---|---|---|---|
| 149 | (2S,4R)-4-hydroxy-1-((R)-2-(3-(3-(2-hydroxyphenyl)-7-methylcinnolin-6-yl)isoxazol-5-yl)-3-methylbutanoyl)-N-((S)-1-(4-(4-methylthiazol-5-yl)phenyl)ethyl)pyrrolidine-2-carboxamide | 717.25 | 1H NMR (400 MHz, DMSO-d6) δ 8.99 (s, 1H), 8.93 (m, 1H), 8.48 - 8.41 (m, 3H), 8.10 (d, J = 7.2 Hz, 1H), 7.47 - 7.34 (m, 5H), 7.11 - 7.03 (m, 2H), 6.99 (s, 1H), 5.14 - 4.85 (m, 2H), 4.49 - 4.29 (m, 2H), 3.96 (d, J = 9.4 Hz, 1H), 3.82-3.71 (m, 1H), 3.61 - 3.55 (m, 1H), 2.71 (s, 3H), 2.47 - 2.31 (m, 4H), 2.12 - 1.98 (m, 1H), 1.87 - 1.74 (m, 1H), 1.39 (d, J = 7.0 Hz, 3H), 1.06 (d, J = 6.5 Hz, 3H), 0.89 (d, J = 6.7 Hz, 3H). |
| 143 | (2S,4R)-1-((R)-2-(3-(3-(3-fluoro-2-hydroxyphenyl)cinnolin-6-yl)isoxazol-5-yl)-3-methylbutanoyl)-4-hydroxy-N-((S)-1-(4-(4-methylthiazol-5-yl)phenyl)ethyl)pyrrolidine-2-carboxamide | 721.25 | 1H NMR (400 MHz, DMSO-d6) δ 11.99 (s, 1H), 8.99 (s, 2H), 8.88 - 8.71 (m, 1H), 8.66 - 8.56 (m, 1H), 8.49 - 8.42 (m, 2H), 7.96 - 7.90 (m, 1H), 7.48 - 7.36 (m, 5H), 7.23 (s, 1H), 7.13 - 7.03 (m, 1H), 5.13 (d, J = 3.6 Hz, 1H), 4.98 - 4.90 (m, 1H), 4.45 - 4.37 (m, 1H), 4.34 - 4.29 (m, 1H), 3.97 (d, J = 9.5 Hz, 1H), 3.81 - 3.70 (m, 1H), 3.64 - 3.53 (m, 1H), 2.49 - 2.44 (m, 4H), 2.10 - 2.02 (m, 1H), 1.86 - 1.76 (m, 1H), 1.55 - 1.35 (m, 3H), 1.08 - 1.02 (m, 3H), 0.93 - 0.85 (m, 3H). |
| 135 | (2S,4R)-4-hydroxy-1-((R)-2-(3-(3-(2-hydroxyphenyl)cinnolin-6-yl)isoxazol-5-yl)-3-methylbutanoyl)-N-(4-(4-methylthiazol-5-yl)benzyl)pyrrolidine-2-carboxamide | 689.2 | 1H NMR (300 MHz, DMSO-d6) δ 11.43 (s, 1H), 9.02 - 8.83 (m, 2H), 8.76 - 8.66 (m, 1H), 8.62 - 8.50 (m, 2H), 8.46 - 8.29 (m, 1H), 8.13 (dd, J = 7.8, 1.7 Hz, 1H), 7.56 - 7.30 (m, 5H), 7.22 (s, 1H), 7.14 - 6.98 (m, 2H), 5.10 (d, J = 16.9 Hz, 1H), 4.52 - 4.28 (m, 4H), 3.98 (d, J = 9.5 Hz, 1H), 3.85 (dd, J = 10.6, 4.4 Hz, 1H), 3.60 (dd, J = 22.5, 7.9 Hz, 1H), 2.46 (s, 3H), 2.33 (s, 1H), 2.05 (d, J = 13.6 Hz, 1H), 1.98 - 1.86 (m, 1H), 1.04 (d, J = 6.6 Hz, 3H), 0.89 (d, J = 6.7 Hz, 3H). |
| 134 | (2S,4R)-N-((S)-1-(4-fluorophenyl)ethyl)-4-hydroxy-1-((R)-2-(3-(3-(2-hydroxyphenyl)cinnolin-6-yl)isoxazol-5-yl)-3-methylbutanoyl)pyrrolidine-2-carboxamide | 624.2 | 1H NMR (300 MHz, DMSO-d6) δ 11.48 - 11.37 (m, 1H), 8.98 - 8.91 (m, 1H), 8.73 (s, 1H), 8.63 - 8.54 (m, 1H), 8.46 - 8.34 (m, 2H), 8.17 - 8.08 (m, 1H), 7.44 - 7.26 (m, 3H), 7.22 (s, 1H), 7.19 - 7.01 (m, 4H), 5.10 (s, 1H), 4.96 - 4.84 (m, 1H), 4.42 (t, J = 7.9 Hz, 1H), 4.30 (s, 1H), 3.92 (d, J = 9.7 Hz, 1H), 3.83 - 3.66 (m, 1H), 3.60 - 3.50 (m, 1H), 2.45 - 2.34 (m, 1H), 2.08 - 1.95 (m, 1H), 1.84 - 1.69 (m, 1H), 1.53 - 1.31 (m, 3H), 1.09-0.99 (m, 3H), 0.93 - 0.84 (m, 3H). |
| 133 | (2S,4R)-4-hydroxy-1-((R)-2-(3-(3-(2-hydroxyphenyl)cinnolin-6-yl)isoxazol-5-yl)-3-methylbutanoyl)-N-((S)-1-phenylethyl)pyrrolidine-2-carboxamide | 606.2 | 1H NMR (400 MHz, DMSO-d6) δ 11.42 (s, 1H), 8.95 (d, J = 7.1 Hz, 1H), 8.87 - 8.67 (m, 1H), 8.59 (d, J = 8.9 Hz, 1H), 8.45 - 8.34 (m, 2H), 8.13 (ddd, J = 8.0, 4.4, 1.7 Hz, 1H), 7.39 (ddd, J = 8.5, 7.2, 1.7 Hz, 1H), 7.35 - 7.26 (m, 4H), 7.25 - 7.19 (m, 2H), 7.12 - 7.05 (m, 2H), 5.11 (s, 1H), 4.96 - 4.71 (m, 1H), 4.41 (t, J = 7.9 Hz, 1H), 4.31 (p, J = 5.5, 4.7 Hz, 1H), 3.96 (d, J = 9.7 Hz, 1H), 3.84 - 3.49 (m, 2H), 2.45 - 2.17 (m, 1H), 2.03 (ddd, J = 11.9, 8.1, 3.0 Hz, 1H), 1.95 - 1.70 (m, 1H), 1.55 - 1.32 (m, 3H), 1.05 (d, J = 6.7 Hz, 3H), 0.88 (d, J = 6.9 Hz, 3H). |

### Preparation of (2S,4R)-4-hydroxy-1-((S)-2-(4-(3-(2-hydroxyphenyl)cinnolin-6-yl)-1H-1,2,3-triazol-1-yl)-3-methylbutanoyl)-N-((S)-1-(4-(4-methylthiazol-5-yl)phenyl)ethyl)pyrrolidine-2-carboxamide (Compound 128)

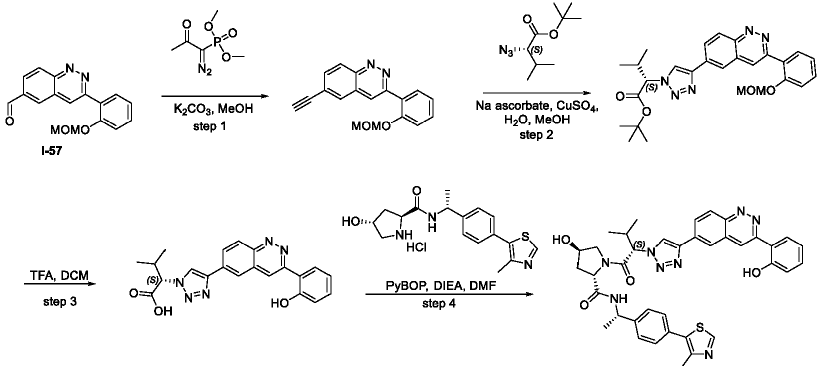

### Step 3: Preparation of 6-ethynyl-3-[2-(methoxymethoxy)phenyl]cinnoline

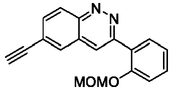

To a stirred mixture of **I-57** (400 mg, 1.359 mmol, 1 equiv) and K₂CO₃ (567.62 mg, 4.077 mmol, 3 equiv) in MeOH (2 mL) was added seyferth-gilbert homologation (261.10 mg, 1.359 mmol, 1 equiv) dropwise at room temperature. The resulting mixture was stirred for overnight at room temperature. Desired product could be detected by LCMS. The resulting mixture was extracted with EtOAc (3 x 25 mL). The combined organic layers were washed with brine (1 x 30 mL), dried over anhydrous Na₂SO₄. After filtration, the filtrate was concentrated under reduced pressure. The residue was purified by silica gel column chromatography, eluted with PE / EA (4:1) to afford the title compound (325 mg) as a light-yellow solid. LCMS (ESI) m/z [M+H]⁺ = 291.

### Step 4: Preparation of tert-butyl (S)-2-(4-(3-(2-(methoxymethoxy)phenyl)cinnolin-6-yl)-1H-1,2,3-triazol-1-yl)-3-methylbutanoate

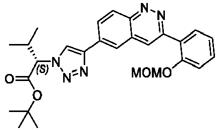

To a stirred mixture of 6-ethynyl-3-[2-(methoxymethoxy)phenyl]cinnoline (300 mg, 1.033 mmol, 1 equiv) and tert-butyl (S)-2-azido-3-methylbutanoate (10.29 mg, 0.051 mmol, 1.5 equiv) in MeOH (2 mL) and H₂O (1 mL) were added sodium (5R)-5-[(1S)-1,2-dihydroxyethyl]-3,4-dihydroxy-2,5-dihydrofuran-2-one (3.43 mg, 0.017 mmol, 0.5 equiv) and CuSO₄.5H₂O (129.00 mg, 0.516 mmol, 0.5 equiv) in portions at room temperature. The resulting mixture was stirred for 8 h at room temperature. The mixture was diluted with water (50 mL), extracted with EtOAc (3 x 50 mL), The combined organic layers were washed with brine (1 x 30 mL), dried over anhydrous Na₂SO₄. After filtration, the filtrate was concentrated under reduced pressure. The residue was purified by silica gel column chromatography, eluted with PE / EA (4:1) to afford the title compound (406 mg) as a light-yellow solid. LCMS (ESI) m/z [M+H]⁺ = 490.

### Step 5: Preparation of (S)-2-(4-(3-(2-hydroxyphenyl)cinnolin-6-yl)-1H-1,2,3-triazol-1-yl)-3-methylbutanoic acid

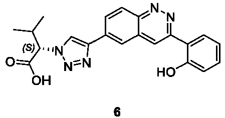

A mixture of intermediate **5** (406 mg, 0.829 mmol, 1 equiv) and TFA (1 mL) in DCM (3 mL) was stirred for 1.5 h at room temperature. Desired product could be detected by LCMS. The resulting mixture was concentrated under reduced pressure. The residue was purified by reversed-phase flash chromatography with the following conditions: column, C18 silica gel; mobile phase, MeCN in Water (10mmol/L NH₄HCO₃), 0% to 100% gradient in 30 min; detector, UV 254 nm. This resulted in intermediate **6** (223 mg) as a light-yellow solid. LCMS (ESI) m/z [M+H]⁺ = 390.

### Step 6: Preparation of (2S,4R)-4-hydroxy-1-((S)-2-(4-(3-(2-hydroxyphenyl)cinnolin-6-yl)-1H-1,2,3-triazol-1-yl)-3-methylbutanoyl)-N-((S)-1-(4-(4-methylthiazol-5-yl)phenyl)ethyl)pyrrolidine-2-carboxamide (Compound 128)

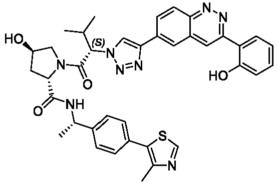

To a stirred mixture of intermediate **6** (213 mg, 0.547 mmol, 1 equiv) and DIEA (212.08 mg, 1.641 mmol, 3 equiv) in DMF (2 mL) were added Intermediate **10** (362.57 mg, 1.094 mmol, 2 equiv) and PyBOP (569.29 mg, 1.094 mmol, 2 equiv) in portions at room temperature. The resulting mixture was stirred for 2 h at room temperature. The crude product was purified by Prep-HPLC with the following conditions: Column, XBridge Shield RP18 OBD Column, 30*150 mm, 5 µm; mobile phase, water (10 mmol/L NH₄HCO₃+0.1%NH₃.H₂O) and ACN (45% ACN up to 50% in 10 min); Detector, UV 254 nm. This resulted in **128** (99.9 mg) as a light-yellow solid. ¹H NMR (400 MHz, DMSO-d₆) δ 11.84 (s, 1H), 9.09 (d, J = 26.3 Hz, 1H), 8.98 (d, J = 7.2 Hz, 1H), 8.93 (d, J = 0.9 Hz, 1H), 8.69 (d, J = 1.6 Hz, 1H), 8.59 - 8.48 (m, 3H), 8.14 (d, 1H), 7.50 - 7.35 (m, 5H), 7.12 - 7.02 (m, 2H), 5.47 (d, J = 10.1 Hz, 1H), 5.24 - 5.10 (m, 1H), 5.04 - 4.85 (m, 1H), 4.47 - 4.30 (m, 2H), 3.85 - 3.58 (m, 2H), 2.70 - 2.50 (m, 1H), 2.46 (d, J = 6.4 Hz, 3H), 2.14 - 2.04 (m, 1H), 1.86 - 1.75 (m, 1H), 1.61 - 1.36 (m, 3H), 1.13 (d, J = 6.7 Hz, 3H), 0.80 (d, J = 6.7 Hz, 3H). LCMS (ESI) m/z: [M+H]⁺ = 703.30.

Compounds in Table 20 were prepared using procedures similar to the one used above for the preparation of **Compound 128.**

**Table 20.**

| **No.** | **Name** | **LCMS (ESI) m/z** | **¹H NMR** |
|---|---|---|---|
| **150** | (2S,4R)-1-((S)-2-(4-(3-(3-fluoro-2-hydroxyphenyl)cinnolin-6-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide | 721.25 | 1H NMR (400 MHz, DMSO-d6) δ 12.50 (s, 1H), 9.14 (s, 1H), 9.04 - 8.91 (m, 2H), 8.71 (s, 1H), 8.62 - 8.52 (m, 3H), 7.95 (d, J = 8.1 Hz, 1H), 7.49 - 7.40 (m, 2H), 7.42 - 7.33 (m, 3H), 7.10 - 7.02 (m, 1H), 5.47 (d, J = 10.1 Hz, 1H), 5.24 - 5.18 (m, 1H), 4.94 (t, J = 7.1 Hz, 1H), 4.43 (t, J = 8.1 Hz, 1H), 4.35 (s, 1H), 3.81 - 3.73 (m, 2H), 2.61 - 2.55 (m, 1H), 2.46 (s, 3H), 2.09 (t, J = 10.5 Hz, 1H), 1.86 - 1.75 (m, 1H), 1.39 (dd, 3H), 1.13 (d, J = 6.7 Hz, 3H), 0.88 - 0.74 (m, 3H). |
| **171** | (2S,4R)-1-((S)-2-(4-(7-(difluoromethyl)-3-(2-hydroxyphenyl)cinnolin-6-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide | 753.2 | 1H NMR (300 MHz, DMSO-d6) δ 11.27 (s, 1H), 9.10 - 8.89 (m, 3H), 8.81 (s, 1H), 8.69 (s, 1H), 8.55 (d, J = 7.6 Hz, 1H), 8.17 (d, J = 7.5 Hz, 1H), 7.81 (d, J = 54.3 Hz, 1H), 7.64 - 7.24 (m, 5H), 7.08 (dd, J = 13.0, 7.5 Hz, 2H), 5.50 (d, J = 10.0 Hz, 1H), 5.21 (d, J = 3.5 Hz, 1H), 5.06 - 4.83 (m, 1H), 4.63 - 4.19 (m, 2H), 3.79 (s, 2H), 2.46 (d, J = 3.5 Hz, 4H), 2.10 (s, 1H), 1.81 (s, 1H), 1.39 (d, J = 7.0 Hz, 3H), 1.12 (d, J = 6.5 Hz, 3H), 0.79 (d, J = 6.5 Hz, 3H). |
| **141** | (2S,4R)-4-hydroxy-1-((S)-2-(4-(3-(2-hydroxyphenyl)-7-methylcinnolin-6-yl)-1H-1,2,3-triazol-1-yl)-3-methylbutanoyl)-N-((S)-1-(4-(4-methylthiazol-5-yl)phenyl)ethyl)pyrrolidine-2-carboxamide | 717.2 | 1H NMR (300 MHz, DMSO-d6) δ 12.02 (s, 1H), 8.97 (d, J = 13.7 Hz, 2H), 8.80 (s, 1H), 8.56 (d, J = 9.5 Hz, 2H), 8.44 (s, 1H), 8.12 (d, J = 7.4 Hz, 1H), 7.46 (d, J = 8.2 Hz, 2H), 7.38 (d, J = 8.4 Hz, 3H), 7.06 (t, J = 8.4 Hz, 2H), 5.48 (d, J = 10.2 Hz, 1H), 5.22 (d, J = 3.8 Hz, 1H), 4.94 (t, J = 7.1 Hz, 1H), 4.45 (t, J = 8.2 Hz, 1H), 4.37 (s, 1H), 3.79 (s, 2H), 2.79 (s, 3H), 2.46 (d, J = 4.7 Hz, 3H), 2.08 (s, 1H), 1.81 (s, 1H), 1.40 (d, J = 7.0 Hz, 3H), 1.14 (d, J = 6.5 Hz, 3H), 0.79 (d, J = 6.6 Hz, 3H). |
| **176** | (2S,4R)-1-((S)-2-(4-(7-fluoro-3-(2-hydroxyphenyl)cinnolin-6-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide | 721.2 | 1H NMR (300 MHz, DMSO-d6) δ 11.66 (s, 1H), 9.11 - 8.90 (m, 3H), 8.74 (d, J = 4.3 Hz, 1H), 8.56 (d, J = 7.7 Hz, 1H), 8.45 (d, J = 11.4 Hz, 1H), 8.09 (dd, J = 7.8, 1.7 Hz, 1H), 7.51 - 7.32 (m, 5H), 7.17 - 6.97 (m, 2H), 5.52 (d, J = 10.2 Hz, 1H), 5.20 (d, J = 3.9 Hz, 1H), 4.94 (s, 1H), 4.44 (t, J = 8.1 Hz, 1H), 4.35 (s, 1H), 3.78 (s, 2H), 2.70 - 2.56 (m, 1H), 2.45 (d, J = 7.6 Hz, 3H), 2.08 (s, 1H), 1.90 - 1.74 (m, 1H), 1.40 (d, J = 7.0 Hz, 3H), 1.12 (d, J = 6.6 Hz, 3H), 0.77 (d, J = 6.5 Hz, 3H). |
| **190** | (2S,4R)-1-((S)-2-(4-(5-chloro-3-(2-hydroxyphenyl)cinnolin-6-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide | 737.2 | 1H NMR (300 MHz, DMSO-d6) δ 9.10 - 8.97 (m, 2H), 8.92 (d, 1H), 8.77 - 8.49 (m, 2H), 8.27 - 8.13 (m, 2H), 7.64 - 7.29 (m, 5H), 7.25 - 6.89 (m, 2H), 5.60 - 5.24 (m, 1H), 5.07 - 4.80 (m, 1H), 4.53 (t, J = 7.8 Hz, 1H), 4.40 (s, 1H), 3.93 - 3.80 (m, 1H), 3.71 (d, J = 10.6 Hz, 1H), 2.75 - 2.56 (m, 1H), 2.48 - 2.45 (m, 3H), 2.17 - 2.00 (m, 1H), 1.99 - 1.83 (m, 1H), 1.69 - 1.37 (m, 3H), 1.14 (d, J = 6.4 Hz, 3H), 0.85 (d, 3H). |
| **178** | (2S,4R)-N-((S)-1-(4-bromophenyl)ethyl)-1-((S)-2-(4-(3-(3-fluoro-2-hydroxyphenyl)cinnolin-6-yl)-1 H-1 ,2,3-triazol-1-yl)-3-methylbutanoyl)-4-hydroxypyrrolidine-2-carboxamide | 702.15 | 1H NMR (400 MHz, DMSO-d6) δ 12.49 (s, 1H), 9.12 (s, 1H), 9.02 - 8.88 (m, 1H), 8.71 (s, 1H), 8.61 - 8.47 (m, 3H), 7.95 (dd, J = 8.1, 1.4 Hz, 1H), 7.56 - 7.47 (m, 2H), 7.43 - 7.33 (m, 1H), 7.31 - 7.20 (m, 2H), 7.11 - 7.01 (m, 1H), 5.46 (d, J = 10.1 Hz, 1H), 5.23 - 5.09 (m, 1H), 4.87 (p, J = 7.2 Hz, 1H), 4.44 - 4.26 (m, 2H), 3.84 - 3.59 (m, 2H), 2.61 - 2.51 (m, 1H), 2.08 - 1.91 (m, 1H), 1.81 - 1.70 (m, 1H), 1.34 (dd, 3H), 1.15 - 1.07 (m, 3H), 0.79 (d, J = 6.6 Hz, 3H). |
| **177** | (2S,4R)-1-((S)-2-(4-(3-(3-fluoro-2-hydroxyphenyl)cinnolin-6-yl)-1H-1,2,3-triazol-1-yl)-3-methylbutanoyl)-N-((S)-1-(4-fluorophenyl)ethyl)-4-hydroxypyrrolidine-2-carboxamide | 642.2 | 1H NMR (400 MHz, DMSO-d6) δ 12.50 (s, 1H), 9.18 - 8.83 (m, 2H), 8.71 (s, 1H), 8.61 - 8.43 (m, 3H), 7.95 (d, J = 8.1, 1.4 Hz, 1H), 7.42 - 7.27 (m, 3H), 7.21 - 7.01 (m, 3H), 5.46 (d, J = 10.1 Hz, 1H), 5.24 - 5.07 (m, 1H), 5.01 - 4.80 (m, 1H), 4.44 - 4.26 (m, 2H), 3.84 - 3.59 (m, 2H), 2.62 - 2.57 (m, 1H), 2.10 - 1.92 (m, 1H), 1.82 - 1.71 (m, 1H), 1.42 (dd, 3H), 1.15 - 1.07 (m, 3H), 0.79 (d, J = 6.6 Hz, 3H). |
| **203** | (2S,4R)-4-hydroxy-1-((S)-2-(4-(3-(2-hydroxyphenyl)-5-methylcinnolin-6-yl)-1H-1,2,3-triazol-1-yl)-3-methylbutanoyl)-N-((S)-1-(4-(4-methylthiazol-5-yl)phenyl)ethyl)pyrrolidine-2-carboxamide | 717.15 | 1H NMR (300 MHz, DMSO-d6) δ 12.00 (d, J = 12.6 Hz, 1H), 9.00 (d, J = 1.9 Hz, 2H), 8.76 (d, J = 25.0 Hz, 1H), 8.57 (d, J = 7.6 Hz, 1H), 8.41 (d, J = 9.0 Hz, 1H), 8.35 - 8.17 (m, 2H), 7.51 - 7.33 (m, 5H), 7.08 (d, J = 7.8 Hz, 2H), 5.48 (d, J = 10.3 Hz, 1H), 5.28 - 5.13 (m, 1H), 4.93 (q, J = 8.3, 7.7 Hz, 1H), 4.46 (t, J = 8.1 Hz, 1H), 4.37 (s, 1H), 3.81 (d, J = 3.6 Hz, 2H), 2.90 (s, 3H), 2.73 - 2.56 (m, 1H), 2.46 (d, J = 4.1 Hz, 3H), 2.11 (td, J = 8.5, 4.1 Hz, 1H), 1.87 - 1.76 (m, 1H), 1.40 (d, J = 7.0 Hz, 3H), 1.18 - 1.08 (m, 3H), 0.80 (d, J = 6.5 Hz, 3H). |

### Preparation of (2S,4R)-4-hydroxy- 1-[(2R)-2-{1-[3-(2-hydroxyphenyl)cinnolin-6-yl]- 1,2,3-triazol-4-yl}-3-methylbutanoyl]-N-[(1S)-1-[4-(4-methyl-1,3-thiazol-5-yl)phenyl]ethyl]pyrrolidine-2-carboxamide (Compound 161).

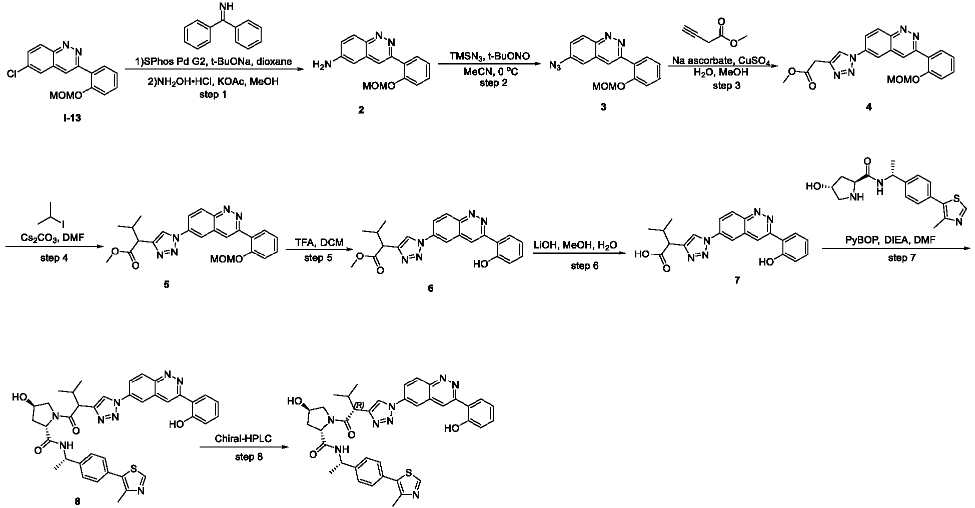

### Step 1: Preparation of 3-[2-(methoxymethoxy)phenyl]cinnolin-6-amine (Intermediate 2).

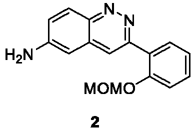

To a stirred solution of **I-13** (600 mg, 1.995 mmol, 1 equiv) and benzenemethanimine, α-phenyl- (433.90 mg, 2.394 mmol, 1.2 equiv) in dioxane (5 mL) were added t-BuONa (287.61 mg, 2.993 mmol, 1.5 equiv) and 2nd Generation SPhos precatalyst (143.77 mg, 0.200 mmol, 0.1 equiv) in portions at room temperature under nitrogen atmosphere. The resulting mixture was stirred overnight at 100 °C. To the above mixture was added NH₂OH.HCl (415.91 mg, 5.985 mmol, 3 equiv), KOAc (587.40 mg, 5.985 mmol, 3 equiv) and MeOH (5 mL) in portions over 5 min at room temperature. The resulting mixture was stirred for additional 16 h at room temperature. The mixture was diluted with EtOAc (250 mL) and washed with water (250 mL x 3). The organic layer was dried over anhydrous sodium sulfate, filtered and concentrated to give a crude product. The crude product was purified by reversed-phase flash chromatography with the following conditions: column, C18 silica gel; mobile phase, MeCN in Water (0.1% FA), 0% to 51 % gradient in 24 min; detector, UV 254 nm. This resulted in intermediate **2** (341 mg, 60.76%) as a yellow solid. LCMS (ESI) m/z: [M+H]⁺ = 282.

### Step 2: Preparation of 6-azido-3-[2-(methoxymethoxy)phenyl]cinnoline (Intermediate 3).

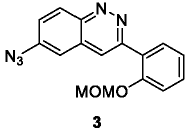

To a solution of intermediate **2** (341 mg, 1.212 mmol, 1 equiv) in acetonitrile (4 mL) were added tert-Butyl nitrite (250.00 mg, 2.424 mmol, 2 equiv) and trimethylsilyl azide (279.31 mg, 2.424 mmol, 2 equiv) at 0 degrees C. The resulting solution was stirred at 25 degrees C for 16 hours. The mixture was diluted with EtOAc (200 mL) and washed with water (200 mL x 3). The organic layer was dried over anhydrous sodium sulfate, filtered and concentrated to give intermediate **3** (277 mg, crude) as a brown solid that was used in the next step directly without further purification. LCMS (ESI) m/z: [M+H]⁺ = 308.

### Step 3: Preparation of 2-(1-{3-[2-(methoxymethoxy)phenyl]cinnolin-6-yl}-1,2,3-triazol-4-yl)acetate (Intermediate 4).

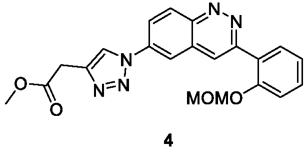

To a solution of intermediate **3** (272 mg, 0.885 mmol, 1 equiv) and sodium ascorbate (88.12 mg, 0.443 mmol, 0.5 equiv) in MeOH (4 mL) and H₂O (2 mL) were added methyl but-3-ynoate (130.24 mg, 1.328 mmol, 1.5 equiv) and CuSO₄ (70.63 mg, 0.443 mmol, 0.5 equiv). The resulting solution was stirred at 25 degrees C for 2 hours. The mixture was diluted with EtOAc (150 mL) and washed with water (150 mL x 3). The organic layer was dried over anhydrous sodium sulfate, filtered and concentrated to give a crude product. The crude product was purified by silica gel column chromatography, eluted with 0 to 50% EtOAc in petroleum ether to afford intermediate **4** (173 mg, 48.21%) as a yellow solid. LCMS (ESI) m/z: [M+H]⁺ = 406.

### Step 4: Preparation of methyl 2-(1-[3-[2-(methoxymethoxy)phenyl]cinnolin-6-yl)-1,2,3-triazol-4-yl)-3-methylbutanoate (Intermediate 5).

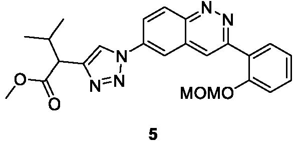

To a solution of intermediate **4** (164 mg, 0.405 mmol, 1 equiv) and Cs₂CO₃ (263.60 mg, 0.810 mmol, 2 equiv) in DMF (3 mL) was added 2-iodopropane (137.53 mg, 0.810 mmol, 2 equiv). The resulting solution was stirred at 60 degrees C for 2 hours (under N₂ atmosphere). The mixture was purified by reversed-phase flash chromatography with the following conditions: column, C18 silica gel; mobile phase, MeCN in Water (0.1% FA), 0% to 63% gradient in 27 min; detector, UV 254 nm. This resulted in intermediate **5** (129 mg, 71.26%) as a yellow solid. LCMS (ESI) m/z: [M+H]⁺ = 448.

### Step 5: Preparation of methyl 2-{1-[3-(2-hydroxyphenyl)cinnolin-6-yl]-1,2,3-triazol-4-yl}-3-methylbutanoate (Intermediate 6).

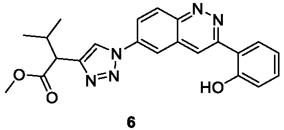

To a solution of intermediate **5** (124 mg, 0.277 mmol, 1 equiv) in DCM (3 mL) was added TFA (1 mL). The resulting solution was stirred at 25 degrees C for 2 hours. The resulting mixture was concentrated under reduced pressure. The residue was purified by reversed-phase flash chromatography with the following conditions: column, C18 silica gel; mobile phase, MeCN in Water (10mmol/L NH₄HCO₃), 0% to 42% gradient in 15 min; detector, UV 254 nm. This resulted in intermediate **6** (103 mg, 92.13%) as a yellow solid. LCMS (ESI) m/z: [M+H]⁺ = 404.

### Step 6: Preparation of 2-{1-[3-(2-hydroxyphenyl)cinnolin-6-yl]-1,2, 3-triazol-4-yl}-3-methylbutanoic acid (Intermediate 7).

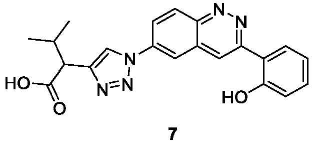

To a solution of intermediate **6** (98 mg, 0.243 mmol, 1 equiv) in MeOH (2 mL) and H₂O (0.5 mL) was added LiOH (29.09 mg, 1.215 mmol, 5 equiv). The resulting solution was stirred at 25 degrees C for 2 hours. The mixture was acidified to pH 5 with 1 M HCl (aq.). The mixture was diluted with EtOAc (100 mL) and washed with water (100 mL x 3). The organic layer was dried over anhydrous sodium sulfate, filtered, and concentrated to afford intermediate 7 (106 mg, crude) as a yellow solid. Intermediate 7 was used in the next step directly without further purification. LCMS (ESI) m/z: [M+H]⁺ = 390.

### Step 7: Preparation of (2S,4R)-4-hydroxy-1-(2-{1-[3-(2-hydroxyphenyl)cinnolin-6-yl]-1,2, 3-triazol-4-yl}-3-methylbutanoyl)-N-[(1S)-1-[4-(4-methyl-1,3-thiazol-5-yl)phenyl]ethyl]pyrrolidine-2-carboxamide (Intermediate 8).

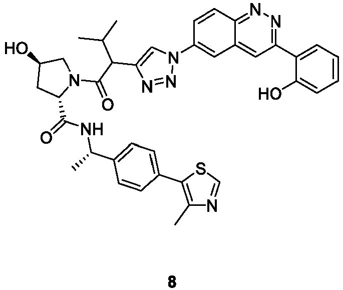

To a solution of intermediate 7 (98 mg, 0.252 mmol, 1 equiv) and (2S,4R)-4-hydroxy-N-[(1R)-1-[4-(4-methyl-1,3-thiazol-5-yl)phenyl]ethyl]pyrrolidine-2-carboxamide (100.09 mg, 0.302 mmol, 1.2 equiv) in DMF (3 mL) was added PyBOP (261.93 mg, 0.504 mmol, 2 equiv). The resulting solution was stirred at 25 degrees C for 10 minutes, then DIEA (162.63 mg, 1.260 mmol, 5 equiv) was added to the mixture. The resulting solution was stirred at 25 degrees C for 2 hours. The residue was purified by reversed-phase flash chromatography with the following conditions: column, C18 silica gel; mobile phase, MeCN in Water (10mmol/L NH₄HCO₃), 0% to 72% gradient in 27 min; detector, UV 254 nm. This resulted in intermediate **8** (92 mg, 52.01%) as a yellow solid. LCMS (ESI) m/z: [M+H]⁺ = 703.

### Step 9: Preparation of (2S,4R)-4-hydroxy-1-[(2R)-2-{1-[3-(2-hydroxyphenyl)cinnolin-6-yl]-1,2,3-triazol-4-yl}-3-methylbutanoyl]-N-[(1S)-1-[4-(4-methyl-1,3-thiazol-5-yl)phenyl]ethyl]pyrrolidine-2-carboxamide (Compound 161).

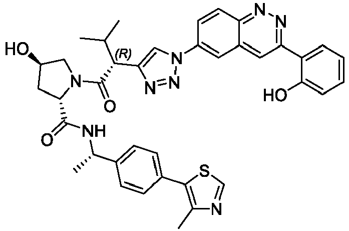

Intermediate **8** was purified by Prep-Chiral-HPLC with the following conditions: Column: CHIRALPAK IA 2*25 cm, 5 µm; Mobile Phase A: HEX: MtBE=1: 1(1: 1(0.5% 2M NH₃-MEOH), Mobile Phase B: MeOH--HPLC; Flow rate: 20 mL/min; Gradient: isocratic 30; Wave Length: 212/276 nm; RT1 (min): 5; RT2(min): 8.5; Sample Solvent: MEOH; Injection Volume: 0.4 mL; Number of Runs: 8. Compound **161** (second peak) (36.1 mg, 39.08%) was obtained as a yellow solid. ¹H NMR (400 MHz, DMSO-d6) δ 11.35 (s, 1H), 9.01 - 8.91 (m, 3H), 8.83 - 8.73 (m, 1H), 8.68 (t, J = 10.2 Hz, 1H), 8.59 (dd, J = 9.3, 2.3 Hz, 1H), 8.39 (d, J = 7.7 Hz, 1H), 8.14 (dd, J = 7.8, 1.7 Hz, 1H), 7.50 - 7.28 (m, 5H), 7.18 - 6.99 (m, 2H), 5.16 - 4.99 (m, 1H), 4.94 (t, J = 7.1 Hz, 1H), 4.43 - 4.20 (m, 2H), 3.89 - 3.73 (m, 2H), 3.62 (d, J = 10.4 Hz, 1H), 2.49 - 2.41 (m, 3H), 2.39 - 2.22 (m, 1H), 2.10 - 1.95 (m, 1H), 1.85 - 1.78 (m, 1H), 1.46 (dd, J = 7.0, 7.0 Hz, 3H), 1.03 (d, J = 6.5 Hz, 3H), 0.86 (dd, J = 14.5, 6.7 Hz, 3H). LCMS (ESI) m/z: [M+H]⁺ = 703.25.

### Preparation of (2S,4R)-1-[(2S)-2-{4-[7-cyano-3-(2-hydroxyphenyl) cinnolin-6-yl]-1,2,3-triazol-1-yl}-3-methylbutanoyl]-4-hydroxy-N-[(1S)-1-[4-(4-methyl-1,3-thiazol-5-yl)phenyl] ethyl] pyrrolidine-2-carboxamide (Compound 170).

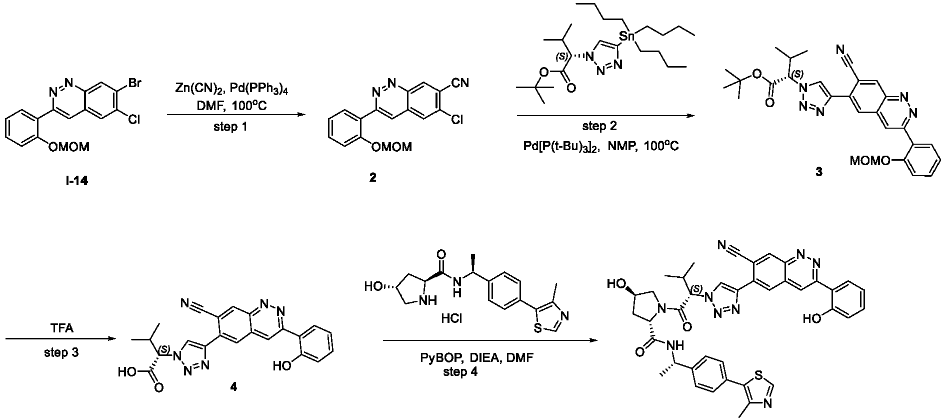

### Step 1: Preparation of 6-chloro-3-[2-(methoxymethoxy) phenyl] cinnoline-7-carbonitrile (Intermediate 2).

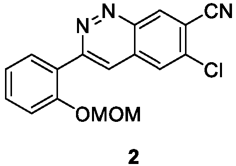

To a stirred solution of **I-14** (200 mg, 0.527 mmol, 1 equiv) in DMF (5 mL) were added Zn(CN)₂ (30.93 mg, 0.264 mmol, 0.5 equiv) and Pd(Pph₃)₄ (121.76 mg, 0.105 mmol, 0.2 equiv) in portions at room temperature under nitrogen atmosphere. The resulting mixture was stirred for additional overnight at 100°C. The residue was purified by reversed-phase flash chromatography with the following conditions: column, C18 silica gel; mobile phase, MeCN in Water (0.1% FA) to afford intermediate **2** (258 mg, 90.20%) as a yellow solid. LCMS (ESI) m/z: [M+H] ⁺ = 326.

### Step 2: Preparation of tert-butyl (2S)-2-(4-{7-cyano-3-[2-(methoxymethoxy) phenyl] cinnolin-6-yl}-1,2, 3-triazol-1-yl)-3-methylbutanoate (Intermediate 3).

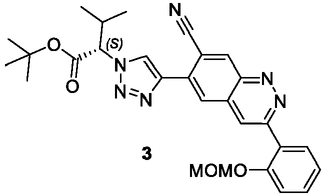

To a stirred solution of intermediate **2** (238 mg, 0.731 mmol, 1 equiv) and tert-butyl (2S)-3-methyl-2-[4-(tributylstannyl)-1,2,3-triazol-1-yl] butanoate (187.89 mg, 0.365 mmol, 0.5 equiv) in NMP (8 mL) was added Pd [(t-Bu) 3P] 2 (74.68 mg, 0.146 mmol, 0.2 equiv) in portions at room temperature under nitrogen atmosphere. The resulting mixture was stirred for additional 3 h at 100 °C. The residue was dissolved in water (5 mL). The resulting mixture was extracted with EtOAc (3 x 10mL). The combined organic layers were washed with brine (2x8 mL), dried over anhydrous Na₂SO₄. After filtration, the filtrate was concentrated under reduced pressure. The residue was purified by reverse-phase flash chromatography with the following conditions: column, C18 silica gel; mobile phase, MeCN in Water (0.1% TFA) to afford intermediate 3 (98 mg, 25.99%) as a yellow solid. LCMS (ESI) m/z: [M+H] ⁺ =515.

### Step 3: Preparation of (2S)-2-{4-[7-cyano-3-(2-hydroxyphenyl) cinnolin-6-yl]-1,2,3-triazol-1-yl}-3-methylbutanoic acid (intermediate 4).

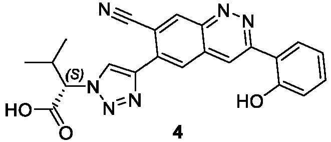

To a stirred solution of intermediate 3 (93 mg, 0.010 mmol, 1 equiv) in DCM (2 mL, 31.461 mmol, 174.08 equiv) was added TFA (2 mL, 26.926 mmol, 148.99 equiv) dropwise at room temperature under air atmosphere. The resulting mixture was stirred for an additional 2 h at room temperature. The resulting mixture was concentrated under reduced pressure. The resulting solid was dried by lyophilization. This resulted in intermediate **4** (54 mg, 67.56%) as a yellow solid. LCMS (ESI) m/z: [M+H] ⁺ = 415.

### Step 4: Preparation of (2S,4R)-1-[(2S)-2-{4-[7-cyano-3-(2-hydroxyphenyl) cinnolin-6-yl]-1,2,3-triazol-1-yl}-3-methylbutanoyl]-4-hydroxy-N-[(1S)-1-[4-(4-methyl-1,3-thiazol-5-yl) phenyl] ethyl] pyrrolidine-2-carboxamide (Compound 170).

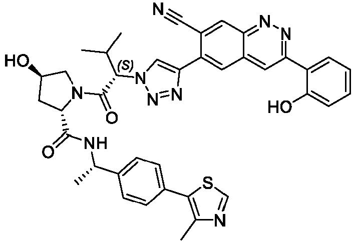

To a stirred solution of intermediate **4** (79.97 mg, 0.242 mmol, 2 equiv) in DMF (2 mL) were added DIEA (0.11 mL, 0.605 mmol, 5 equiv) and PyBOP (94.18 mg, 0.181 mmol, 1.5 equiv) in portions at room temperature under air atmosphere.To the above mixture was added (2S)-2-{4-[7-cyano-3-(2-hydroxyphenyl)cinnolin-6-yl]-1,2,3-triazol-1-yl}-3-methylbutanoic acid (50 mg, 0.121 mmol, 1 equiv) in portions over 10 min at room temperature. The resulting mixture was stirred for additional 1 h at room temperature. The residue was purified by reversed-phase flash chromatography with the following conditions: column, C18 silica gel; mobile phase, MeCN in Water (10mmol/L NH₄HCO₃) to afford **170** (3.6 mg, 4.00%) as a yellow solid. ¹H NMR (400 MHz, DMSO-d6) δ 11.18 (s, 1H), 9.28 (d, *J* = 2.9 Hz, 1H), 9.05 - 8.80 (m, 4H), 8.50 (d, *J* = 33.5 Hz, 1H), 8.17 (dd, *J* = 7.9, 1.8 Hz, 1H), 7.49 - 7.28 (m, 5H), 7.11 - 6.95 (m, 2H), 5.55 (d, *J =* 9.8 Hz, 1H), 5.31 - 5.08 (m, 1H), 5.04 - 4.82 (m, 1H), 4.46 (t, *J* = 8.1 Hz, 1H), 4.33 (d, *J* = 16.8 Hz, 1H), 3.91 - 3.64 (m, 2H), 2.54 (s, 1H), 2.45 (d, *J =* 7.4 Hz, 3H), 2.12 (d, *J* = 11.4 Hz, 1H), 1.81 (ddd, *J* = 12.9, 8.6, 4.6 Hz, 1H), 1.40 (d, *J =* 7.0 Hz, 3H), 1.11 (dd, *J* = 6.7, 4.1 Hz, 3H), 0.80 (d, *J =* 6.5 Hz, 3H). LCMS (ESI) m/z: [M+H] ⁺ = 728.

### Example 2. Degradation of BRM and BRG1 by Compounds of the Invention

This example demonstrates the ability of the compounds of the disclosure to degrade a HiBit-BRM or HiBit-BRG1 fusion protein in a cell-based degradation assay.

**Procedure:** A stable HeLa cell line expressing HiBiT-BRM was generated. On day 0, 5000 cells were seeded in 40 µL of media into each well of 384-well cell culture plates. On day 1, cells were treated with 120 nL DMSO or 120 nL of 3-fold serially DMSO-diluted compounds (10 points in duplicate with 30 µM as final top dose). Subsequently plates were incubated for 24 h in a standard tissue culture incubator and equilibrated at room temperature for 15 minutes. Nano-Glo HiBiT Lytic Detection System (Promega N3050) reagent was freshly prepared and 20 ul was added to each well. Upon addition of this LgBit-containing reagent, the HiBiT and LgBiT proteins associate to form the luminescent NanoBiT luciferase. The plates were shaken for 10 minutes at room temperature and the bioluminescence read using an EnVision plate reader (PerkinElmer).

For measurement of BRG1 degradation, a stable HeLa cell line expressing HiBit-BRG1 and LgBit was generated. The same protocol as above was then followed.

The degradation% was calculated using the following formula: % degradation = 100%-100% x (Lum_{Sample} - Lum_{LC}) / (Lum_{HC} -Lum_{LC}). DMSO treated cells are employed as High Control (HC) and 2 µM of a known BRM/BRG1 degrader standard treated cells are employed as Low Control (LC). The data was fit to a four parameter, non-linear curve fit to calculate IC₅₀ (µM) values as shown in Table 21.

**Results:** As shown in Table 21 below, the compounds of the invention degraded BRM and/or BRG1.

**Table 21.**

| **Compound No.** | **BRM HiBit Degradation IC50 (nM)** | **BRM HiBit Degradation Maximum (%)** | **BRMG1 HiBit Degradation IC50 (nM)** | **BRG1 HiBit Degradation Maximum (%)** |
|---|---|---|---|---|
| 1 | +++ | B | NC | C |
| 2 | +++ | A | NC | C |
| 3 | ++++ | A | ++++ | A |
| 4 | ++++ | A | +++ | A |
| 5 | ++ | B | NC | C |
| 6 | ++ | B | NC | C |
| 7 | +++ | A | ++ | B |
| 8 | ++++ | A | NC | B |
| 9 | +++ | A | NC | C |
| 10 | +++ | A | NC | C |
| 11 | ++++ | A | NC | C |
| 12 | ++ | C | NC | C |
| 13 | ++++ | A | ++++ | A |
| 14 | +++ | A | ++ | A |
| 15 | NT | | NT | |
| 16 | NT | | NT | |
| 17 | +++ | A | NC | C |
| 18 | ++++ | A | +++ | B |
| 19 | ++ | B | NC | C |
| 20 | ++++ | A | NC | C |
| 21 | NC | C | NC | C |
| 22 | ++++ | A | ++++ | A |
| 23 | +++ | A | NC | C |
| 24 | ++++ | A | ++++ | A |
| 25 | ++ | B | NC | C |
| 26 | ++++ | A | +++ | A |
| 27 | ++ | A | NC | C |
| 28 | +++ | A | NC | C |
| 29 | ++ | A | NC | C |
| 30 | ++++ | A | +++ | A |
| 31 | 243 | A | NC | C |
| 32 | +++ | B | NC | C |
| 33 | ++ | A | NC | C |
| 34 | ++++ | A | +++ | A |
| 35 | ++ | B | NC | C |
| 36 | +++ | B | NC | C |
| 37 | NC | C | NC | C |
| 38 | ++ | A | NC | C |
| 39 | NC | C | NC | C |
| 40 | ++++ | A | NC | C |
| 41 | NC | C | NC | C |
| 42 | +++ | A | NC | C |
| 43 | ++ | A | NC | C |
| 44 | +++ | A | NC | C |
| 45 | ++++ | A | +++ | A |
| 46 | ++ | A | NC | C |
| 47 | ++++ | A | NC | C |
| 48 | ++++ | A | ++++ | A |
| 49 | ++++ | A | ++++ | A |
| 50 | ++++ | A | NC | B |
| 51 | ++++ | A | NC | C |
| 52 | ++++ | A | +++ | A |
| 53 | ++++ | A | +++ | B |
| 54 | +++ | B | NC | C |
| 55 | +++ | B | NC | C |
| 56 | ++++ | A | ++++ | A |
| 57 | ++++ | A | +++ | A |
| 58 | ++++ | A | NC | C |
| 59 | ++++ | A | NC | C |
| 60 | ++++ | A | ++++ | A |
| 61 | +++ | A | NC | C |
| 62 | ++++ | A | +++ | A |
| 63 | ++++ | A | ++++ | A |
| 64 | ++++ | A | +++ | B |
| 65 | ++++ | A | +++ | A |
| 66 | ++++ | A | NC | C |
| 67 | ++++ | A | +++ | A |
| 68 | ++++ | A | +++ | A |
| 69 | ++++ | A | ++ | B |
| 70 | +++ | A | +++ | B |
| 71 | ++++ | A | ++++ | A |
| 72 | ++++ | A | +++ | A |
| 73 | ++++ | A | NC | C |
| 74 | ++++ | A | NC | C |
| 75 | ++++ | A | +++ | A |
| 76 | ++++ | A | +++ | A |
| 77 | ++++ | A | NC | C |
| 78 | +++ | A | NC | C |
| 79 | ++++ | A | ++++ | A |
| 80 | ++++ | A | +++ | A |
| 81 | ++++ | A | NC | C |
| 82 | ++++ | A | NC | C |
| 83 | ++++ | A | +++ | A |
| 84 | ++++ | A | +++ | B |
| 85 | ++++ | A | NC | C |
| 86 | ++++ | A | NC | C |
| 87 | ++++ | A | ++++ | A |
| 88 | ++++ | A | NC | C |
| 89 | ++++ | A | NC | C |
| 90 | ++++ | A | ++++ | A |
| 91 | ++++ | A | ++++ | A |
| 92 | ++++ | A | +++ | A |
| 93 | ++++ | A | NC | C |
| 94 | ++++ | A | NC | C |
| 95 | ++++ | A | ++++ | A |
| 96 | ++++ | A | +++ | A |
| 97 | ++++ | A | ++++ | A |
| 98 | ++++ | A | +++ | A |
| 99 | ++++ | A | ++++ | A |
| 100 | +++ | B | NC | C |
| 101 | +++ | A | NC | C |
| 102 | ++++ | A | NC | C |
| 103 | ++++ | A | NC | C |
| 104 | ++++ | A | NC | C |
| 105 | ++++ | A | ++++ | A |
| 106 | ++++ | A | ++++ | A |
| 107 | ++++ | A | ++++ | A |
| 108 | ++++ | A | +++ | A |
| 109 | ++++ | A | NC | C |
| 110 | ++++ | A | NC | C |
| 111 | ++++ | A | NC | C |
| 112 | ++++ | A | +++ | B |
| 113 | ++++ | A | NC | C |
| 114 | ++++ | A | NC | C |
| 115 | ++++ | A | ++++ | A |
| 116 | ++++ | A | NC | C |
| 117 | ++++ | A | NC | C |
| 118 | +++ | A | NC | C |
| 119 | +++ | A | NC | C |
| 120 | +++ | A | NC | C |
| 121 | ++++ | A | ++++ | A |
| 122 | ++++ | A | ++++ | A |
| 123 | ++++ | A | ++++ | A |
| 124 | ++++ | A | ++++ | A |
| 125 | ++++ | A | NC | C |
| 126 | ++++ | A | ++++ | A |
| 127 | ++++ | A | ++++ | A |
| 128 | ++++ | A | ++++ | A |
| 129 | ++++ | A | NC | C |
| 130 | ++++ | A | ++++ | A |
| 131 | ++++ | A | NC | B |
| 132 | ++++ | A | ++++ | A |
| 133 | ++++ | A | +++ | A |
| 134 | ++++ | A | +++ | A |
| 135 | ++++ | A | ++++ | A |
| 136 | ++++ | A | ++++ | A |
| 137 | ++++ | A | ++++ | A |
| 138 | ++++ | A | +++ | A |
| 139 | ++++ | A | NC | C |
| 140 | ++++ | A | NC | C |
| 141 | ++++ | A | ++++ | A |
| 142 | +++ | A | NC | C |
| 143 | ++++ | A | NC | C |
| 144 | ++++ | A | +++ | A |
| 145 | ++++ | A | NC | C |
| 146 | ++++ | A | +++ | A |
| 147 | ++++ | A | +++ | A |
| 148 | ++++ | A | ++++ | A |
| 149 | ++++ | A | +++ | B |
| 150 | ++++ | A | ++++ | A |
| 151 | ++++ | A | NC | C |
| 152 | ++++ | A | ++++ | A |
| 153 | ++++ | A | NC | C |
| 154 | ++++ | A | ++++ | A |
| 155 | ++++ | A | ++++ | A |
| 156 | ++++ | A | NC | C |
| 157 | ++++ | A | ++++ | B |
| 158 | ++++ | A | ++++ | A |
| 159 | ++++ | A | ++++ | A |
| 160 | NC | B | NC | C |
| 161 | ++++ | A | ++++ | A |
| 162 | ++++ | A | ++++ | A |
| 163 | ++++ | A | NC | C |
| 164 | ++++ | A | ++++ | A |
| 165 | ++++ | A | ++++ | A |
| 166 | ++++ | A | +++ | A |
| 167 | ++++ | A | NC | C |
| 168 | ++++ | A | ++++ | B |
| 169 | ++++ | A | ++++ | B |
| 170 | +++ | B | NC | C |
| 171 | ++++ | A | ++++ | A |
| 172 | ++++ | A | ++++ | B |
| 173 | +++ | B | NC | C |
| 174 | +++ | A | NC | C |
| 175 | +++ | A | NC | C |
| 176 | ++++ | A | ++++ | A |
| 177 | ++++ | A | +++ | A |
| 178 | ++++ | A | +++ | A |
| 179 | ++++ | A | NC | C |
| 180 | ++++ | A | NC | C |
| 181 | ++ | A | NC | C |
| 182 | ++++ | A | +++ | A |
| 183 | ++++ | A | +++ | B |
| 184 | ++++ | A | +++ | B |
| 185 | ++++ | A | ++++ | A |
| 186 | ++++ | A | ++++ | A |
| 187 | ++++ | A | +++ | A |
| 188 | +++ | A | +++ | A |
| 189 | ++++ | A | NC | C |
| 190 | ++++ | A | NC | C |
| 191 | +++ | A | NC | C |
| 192 | ++++ | A | ++++ | A |
| 193 | ++++ | A | +++ | A |
| 194 | ++ | A | NC | C |
| 195 | ++++ | A | NC | B |
| 196 | +++ | B | NC | C |
| 197 | ++++ | A | ++++ | A |
| 198 | ++++ | A | ++++ | A |
| 199 | ++++ | A | +++ | A |
| 200 | ++++ | A | ++++ | A |
| 201 | ++++ | A | +++ | B |
| 202 | ++++ | A | +++ | B |
| 203 | +++ | A | NC | C |
| 204 | ++++ | A | ++++ | A |
| 205 | ++++ | A | NC | C |
| 206 | ++++ | A | +++ | B |
| 207 | ++++ | A | ++++ | A |
| 208 | ++++ | A | +++ | A |
| 209 | ++++ | A | NC | C |
| 210 | ++++ | A | NC | C |
| 211 | ++++ | A | NC | C |
| 212 | ++++ | A | +++ | A |
| 213 | ++++ | A | NC | C |
| 214 | ++++ | A | NC | B |
| 215 | ++++ | A | NC | C |
| 216 | +++ | A | NC | C |
| 217 | ++++ | A | NC | C |
| 218 | ++++ | A | +++ | A |
| 219 | ++++ | A | +++ | A |
| 220 | ++++ | A | +++ | A |
| 221 | ++++ | A | +++ | B |
| 222 | ++++ | A | NC | C |
| 223 | ++++ | A | NC | B |
| 224 | ++++ | A | +++ | A |
| 225 | ++++ | A | +++ | A |
| 226 | +++ | A | NC | C |
| 227 | ++ | A | ++ | B |
| 228 | ++++ | A | ++++ | A |
| 229 | ++++ | A | ++++ | A |
| 230 | ++++ | A | +++ | A |
| 231 | ++++ | A | ++++ | A |
| 232 | +++ | A | NC | C |
| 233 | ++++ | A | ++++ | A |
| 234 | ++++ | A | +++ | B |
| 235 | ++++ | A | +++ | A |
| 236 | +++ | A | NC | C |
| 237 | ++++ | A | ++++ | A |
| 238 | ++++ | A | NC | C |
| 239 | ++++ | A | +++ | B |
| 240 | ++++ | A | NC | C |
| 241 | ++++ | A | ++++ | A |
| 242 | ++++ | A | ++++ | B |
| 243 | ++++ | A | ++++ | A |
| 244 | ++ | B | NC | C |
| 245 | +++ | B | NC | C |
| 246 | ++++ | A | NC | C |
| 247 | ++++ | A | ++++ | A |
| 248 | ++++ | A | +++ | A |
| 249 | +++ | A | ++ | A |
| 250 | ++++ | A | ++++ | A |
| 251 | ++++ | A | ++++ | A |
| 252 | ++++ | A | ++++ | A |
| 253 | ++++ | A | ++++ | A |
| 254 | ++++ | A | NC | C |
| 255 | ++++ | A | NC | C |
| 256 | ++ | B | +++ | A |
| 257 | ++++ | A | NC | C |
| 258 | +++ | A | +++ | B |
| 259 | ++++ | A | +++ | A |
| 260 | +++ | A | +++ | A |
| 261 | +++ | A | +++ | A |
| 262 | ++ | A | ++ | B |
| 263 | +++ | A | +++ | A |
| 264 | +++ | A | NC | C |
| 265 | ++ | A | ++ | A |
| 266 | ++++ | A | ++++ | A |
| 267 | ++ | B | NC | C |
| 268 | ++ | A | NC | C |
| 269 | ++++ | A | +++ | A |
| 270 | ++++ | A | +++ | A |
| 271 | +++ | A | NC | C |
| 272 | ++ | A | NC | C |
| 273 | ++++ | A | NC | C |
| 274 | ++++ | A | NC | C |
| 275 | ++++ | A | +++ | A |
| 276 | NC | C | +++ | A |
| 277 | ++++ | A | NC | C |
| 278 | ++++ | A | +++ | B |
| 279 | ++++ | A | +++ | A |
| 280 | ++++ | A | ++++ | A |
| 281 | ++++ | A | ++++ | A |
| 282 | ++++ | A | ++++ | A |
| 283 | ++++ | A | NC | C |
| 284 | ++++ | A | NC | C |
| 285 | ++++ | A | +++ | A |

| | | | | |
|---|---|---|---|---|
| "+" indicates inhibitory effect of ≥ 1000 nM; "++" indicates inhibitory effect of ≥ 100 nM; "+++" indicates inhibitory effect of ≥ 10 nM; "++++" indicates inhibitory effect of < 10 nM; "NT" indicates not tested; "NC" indicates not calculated; "A" indicates maximum degradation ≥ 75%; "B" indicates maximum degradation ≥ 50%; and "C" indicates maximum degradation < 50%. | | | | |

## Claims

1. A compound, or a pharmaceutically acceptable salt thereof, of **Formula I:** 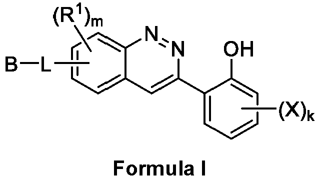 wherein
m is 0, 1, 2, or 3;
k is 0, 1, or 2;
each R¹ is, independently, halo, optionally substituted C₁-C₆ alkyl, optionally substituted C₁-C₆ heteroalkyl, optionally substituted C₂-C₉ heterocyclyl, optionally substituted C₃-C₈ cycloalkyl, optionally substituted C₃-C₈ cycloalkoxy, optionally substituted C₂-C₆ alkynyl, optionally substituted amino, or cyano;
each X is, independently, halo or optionally substituted C₁-C₆ heteroalkyl;
L is a linker
of **Formula II:**
A¹-(B¹)_{f}-(C¹)_{g}-(B²)ₕ-(D)-(B³)ᵢ-(C²)ⱼ-(B⁴)ₖ-A², **Formula II**
or a pharmaceutically acceptable salt thereof,
wherein
A¹ is a bond between the linker and the ring system A;
A² is a bond between the degradation moiety and the linker;
each of B¹, B², B³, and B⁴ is, independently, optionally substituted C₁-C₄ alkyl, optionally substituted C₆-C₁₀ aryl, optionally substituted C₆-C₁₀ aryl C₁-₄ alkyl, optionally substituted C₁-C₄ heteroalkyl, optionally substituted C₃-C₁₀ cycloalkyl, optionally substituted C₃-C₁₀ carbocyclyl, optionally substituted C₂-C₁₀ heterocyclyl, optionally substituted C₂-C₆ heteroaryl, optionally substituted C₆₋₁₂ aryl, O, S, S(O)₂, or NR^{N};
each R^{N} is, independently, H, optionally substituted C₁₋₄ alkyl, optionally substituted C₂₋₄ alkenyl, optionally substituted C₂₋₄ alkynyl, optionally substituted C₂₋₁₀ heterocyclyl, optionally substituted C₂₋₆ heteroaryl, or optionally substituted C₁₋₇ heteroalkyl;
each of C¹ and C² is, independently, carbonyl, thiocarbonyl, sulphonyl, or phosphoryl;
each of f, g, h, i, j, and k is, independently, 0 or 1; and
D is optionally substituted C₁₋₁₀ alkyl, optionally substituted C₂₋₁₀ alkenyl, optionally substituted C₂₋₁₀ alkynyl, optionally substituted C₂₋₁₀ heterocyclyl, optionally substituted C₂₋₆ heteroaryl, optionally substituted C₆₋₁₂ aryl, optionally substituted C₂-C₁₀ polyethylene glycol, optionally substituted C₃-C₁₀ cycloalkyl, optionally substituted C₃-C₁₀ carbocyclyl, or optionally substituted C₁₋₁₀ heteroalkyl; or D is absent, and the linker is A¹-(B¹)_{f}-(C¹)_{g}-(B²)ₕ-(B³)ᵢ-(C²)ⱼ-(B⁴)ₖ-A², and
B is a degradation moiety of **Formula C:** 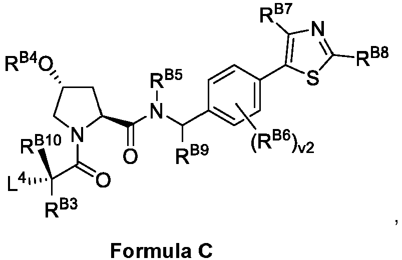
wherein
L⁴ is -N(R^{B1})(R^{B2}), 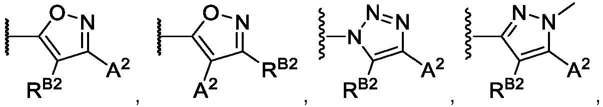 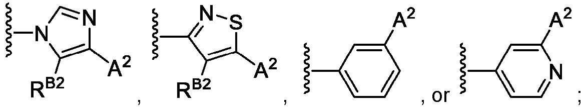
R^{B1} is H, A², optionally substituted C₁-C₆ alkyl, or optionally substituted C₁-C₆ heteroalkyl;
R^{B2} is H, optionally substituted C₁-C₆ alkyl, or optionally substituted C₁-C₆ heteroalkyl;
R^{B3} is A², optionally substituted C₁-C₆ alkyl, optionally substituted C₁-C₆ heteroalkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B4} is H, optionally substituted C₁-C₆ alkyl, optionally substituted C₃-C₁₀ carbocyclyl, optionally substituted C₆-C₁₀ aryl, optionally substituted C₁-C₆ alkyl C₃-C₁₀ carbocyclyl, or optionally substituted C₁-C₆ alkyl C₆-C₁₀ aryl;
R^{B5} is H, optionally substituted C₁-C₆ alkyl, or optionally substituted C₁-C₆ heteroalkyl;
v2 is 0, 1, 2, 3, or 4;
each R^{B6} is, independently, A², halogen, optionally substituted C₁-C₆ alkyl, optionally substituted C₂-C₆ alkynyl, optionally substituted C₁-C₆ heteroalkyl, optionally substituted C₃-C₁₀ carbocyclyl, optionally substituted C₂-C₉ heterocyclyl, optionally substituted C₆-C₁₀ aryl, optionally substituted C₂-C₉ heteroaryl, optionally substituted C₂-C₆ alkenyl, optionally substituted C₂-C₆ heteroalkenyl, hydroxy, thiol, cyano, or optionally substituted amino;
each of R^{B7} and R^{B8} is, independently, H, halogen, optionally substituted C₁-C₆ alkyl, or optionally substituted C₆-C₁₀ aryl;
R^{B9} is H or optionally substituted C₁-C₆ alkyl;
R^{B10} is H or F; and
A² is a bond between the degradation moiety and the linker;
wherein one and only one of R^{B1}, R^{B3}, and R^{B6} is A²,
or a pharmaceutically acceptable salt thereof.

2. A compound of claim 1, or a pharmaceutically acceptable salt thereof, wherein
each R¹ is, independently, halo, optionally substituted C₁-C₆ alkyl, optionally substituted C₁-C₆ heteroalkyl, optionally substituted C₂-C₉ heterocyclyl, or optionally substituted C₃-C₈ cycloalkyl; and
each X is, independently, halo.

3. The compound of claim 1 or 2, or a pharmaceutically acceptable salt thereof, wherein the compound has the structure of **Formula I-A:**

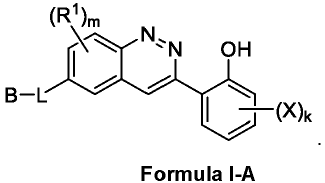

4. The compound of claim 1 or 2, or a pharmaceutically acceptable salt thereof, wherein the compound has the structure of **Formula I-B:**

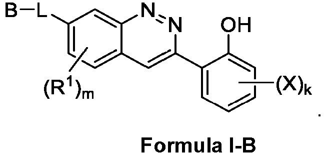

5. The compound of any one of claims 1 to 4, or a pharmaceutically acceptable salt thereof, wherein m is 1.

6. The compound of any one of claims 1 to 5, or a pharmaceutically acceptable salt thereof, wherein R¹ is optionally substituted C₁-C₆ alkoxy, or halo.

7. The compound of any one of claims 1-6, or a pharmaceutically acceptable salt thereof, wherein the degradation moiety has the structure of **Formula Ca2, Formula Cb2, Formula Cc2 Formula Cd2, Formula Ce2** or **Formula Cf2:**

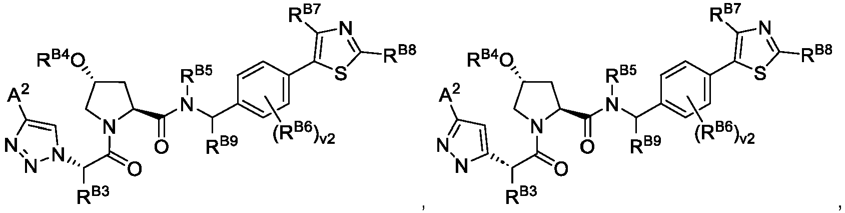

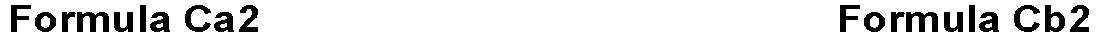

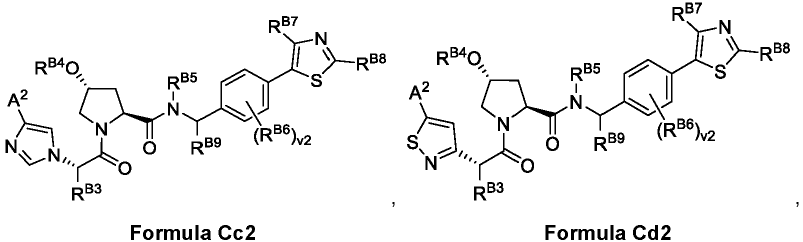

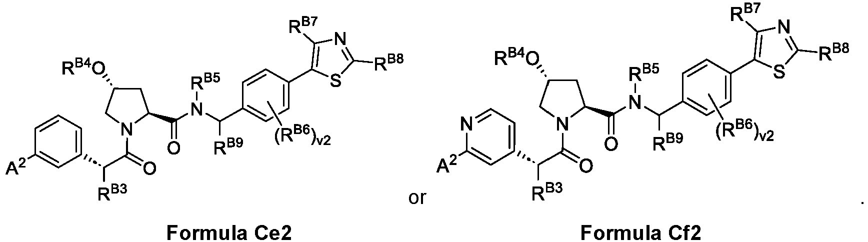

8. The compound of any one of claims 1-7, or a pharmaceutically acceptable salt thereof, wherein R^{B9} is methyl.

9. The compound of any one of claims 1-8, or a pharmaceutically acceptable salt thereof, wherein R^{B4} is H.

10. The compound of any one of claims 1-9, or a pharmaceutically acceptable salt thereof, wherein R^{B5} is H.

11. The compound of any one of claims 1-10, or a pharmaceutically acceptable salt thereof, wherein R^{B7} is methyl.

12. The compound of any one of claims 1-11, or a pharmaceutically acceptable salt thereof, wherein R^{B3} is optionally substituted C₁-C₆ alkyl.

13. The compound of any one of claims 1-6, or a pharmaceutically acceptable salt thereof, wherein the degradation moiety is

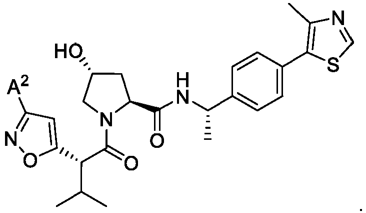

14. The compound of any one of claims 1-6, or a pharmaceutically acceptable salt thereof, wherein the degradation moiety is

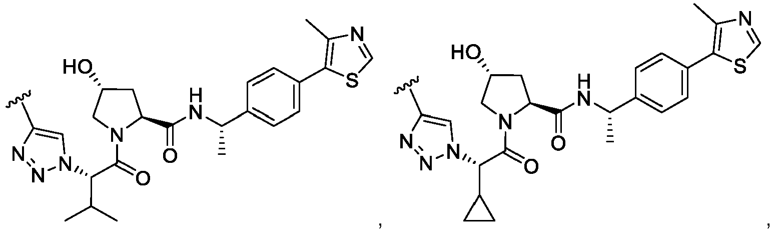

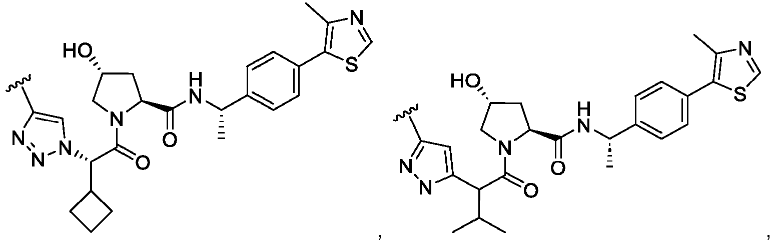

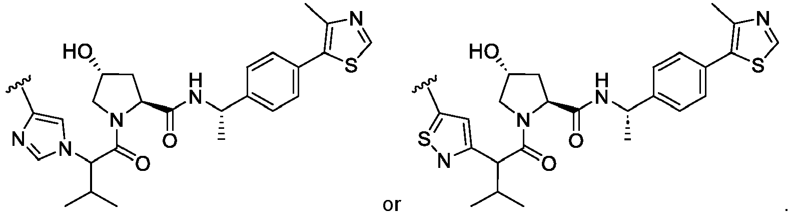

15. The compound of any one of claims 1-14, or a pharmaceutically acceptable salt thereof, wherein the linker has the structure of

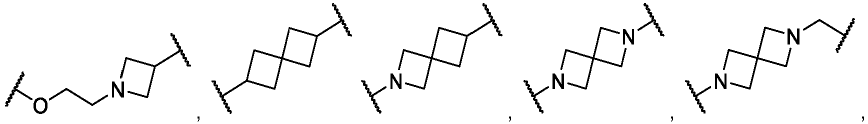

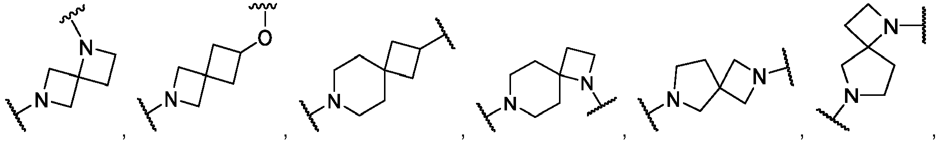

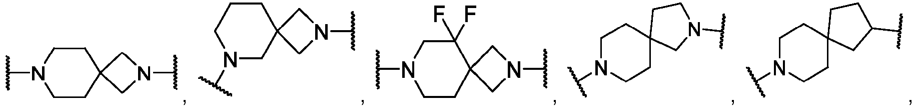

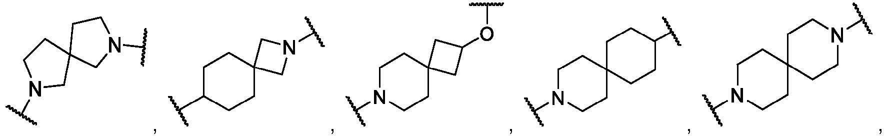

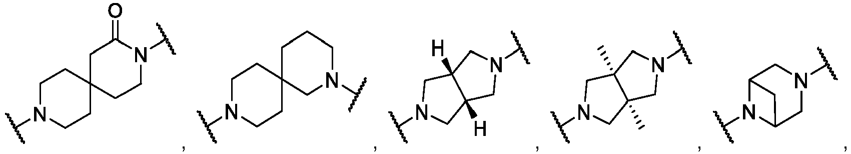

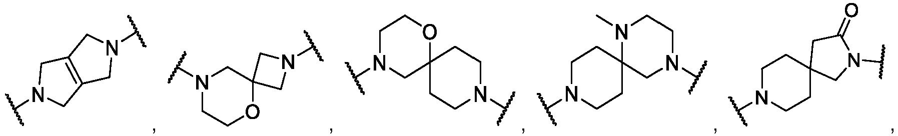

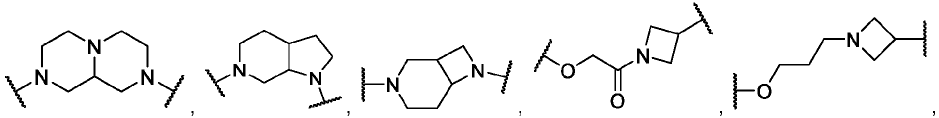

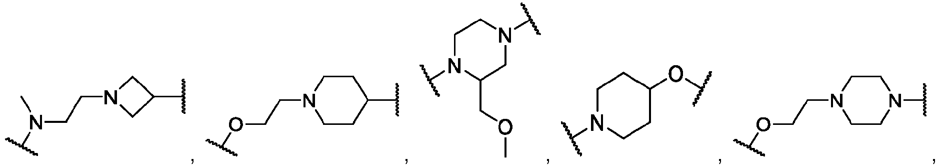

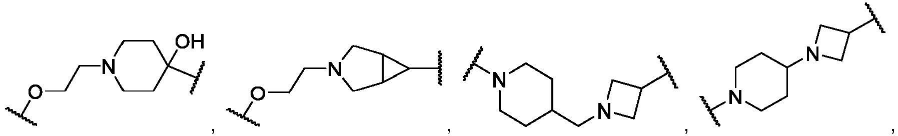

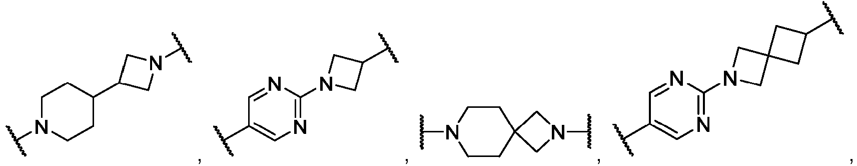

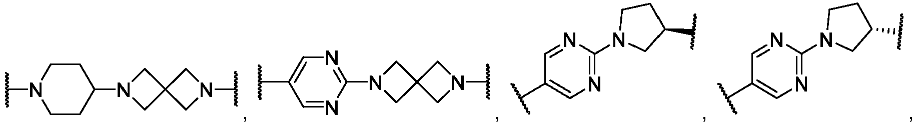

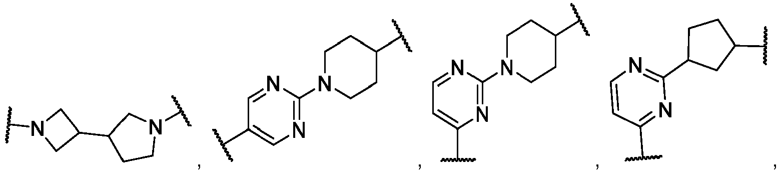

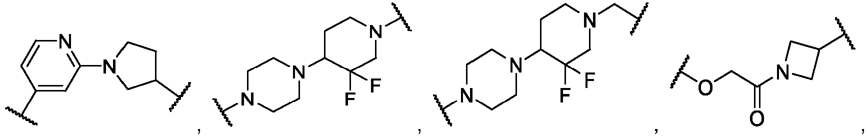

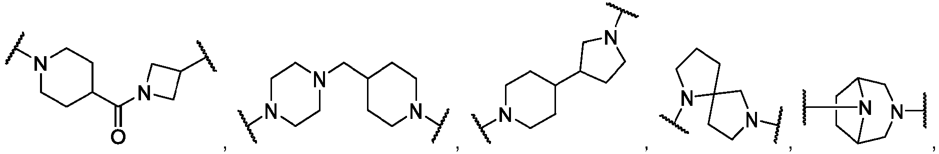

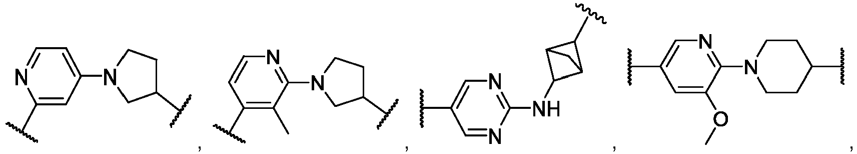

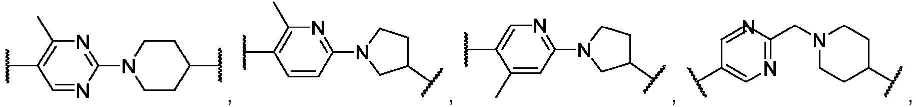

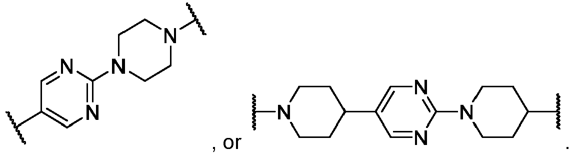

16. The compound of any one of claims 1-14, or a pharmaceutically acceptable salt thereof, wherein the linker has the structure of

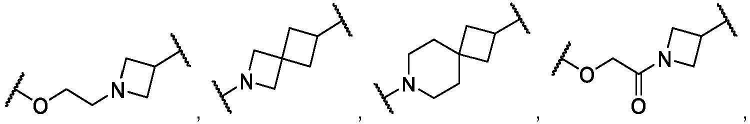

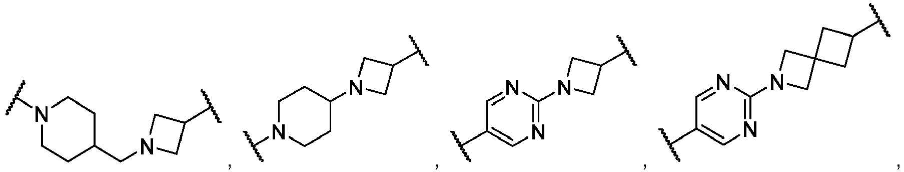

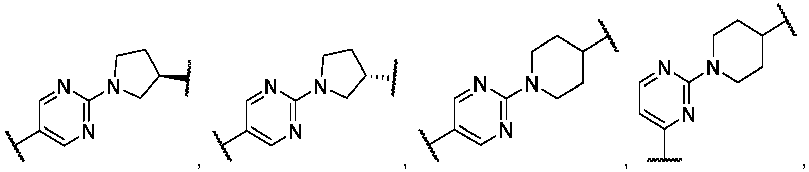

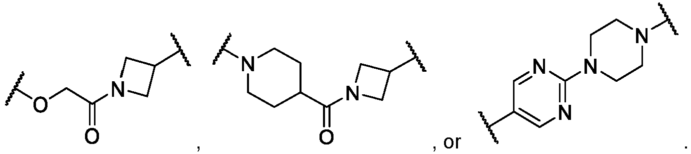

17. The compound of any one of claims 1-14, or a pharmaceutically acceptable salt thereof, wherein the linker has the structure of

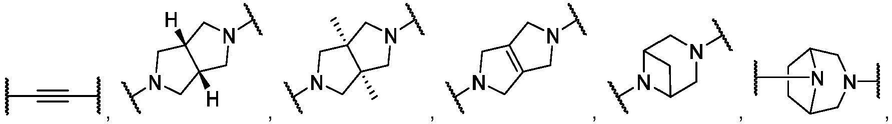

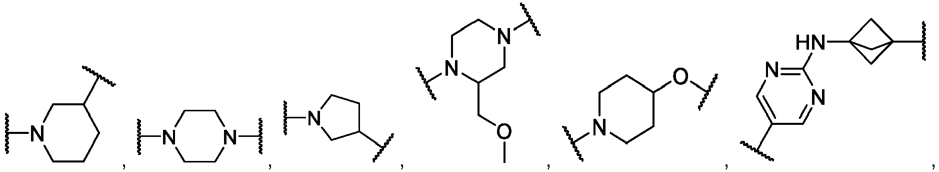

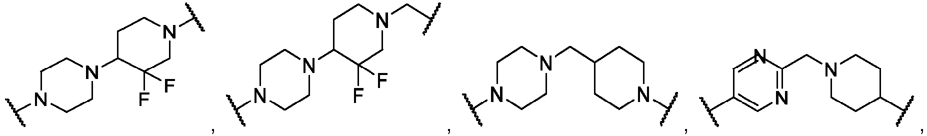

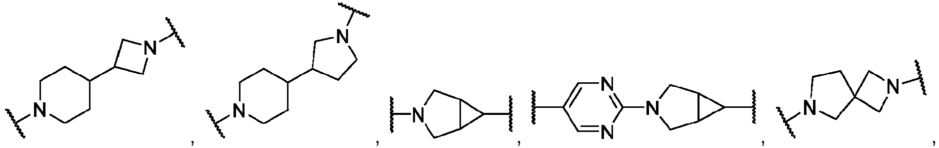

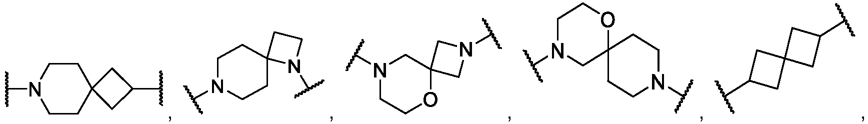

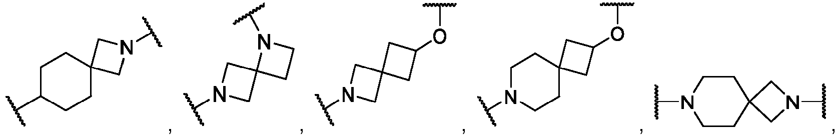

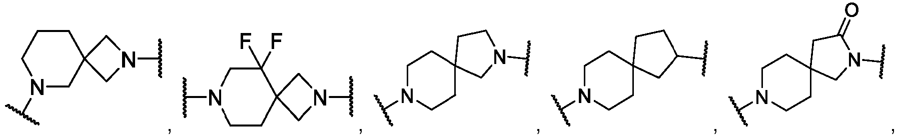

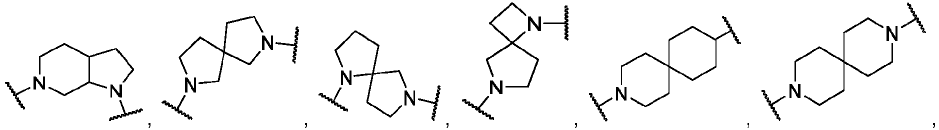

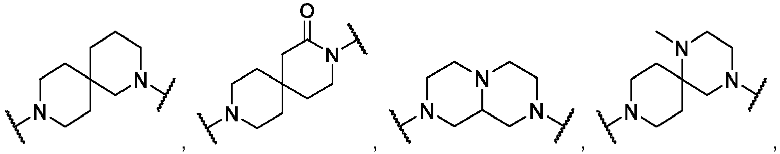

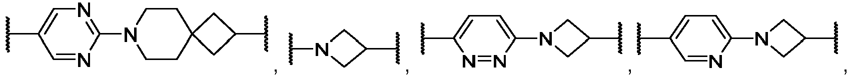

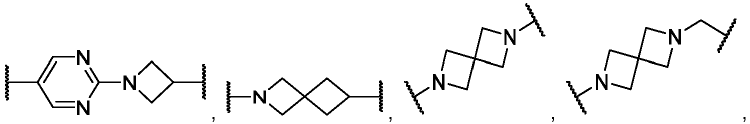

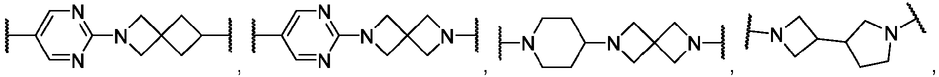

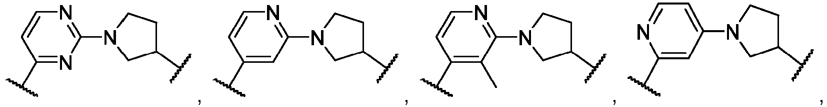

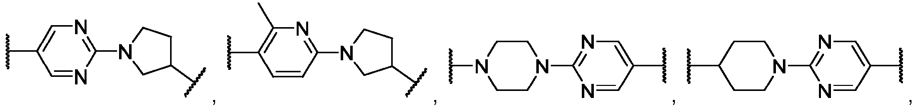

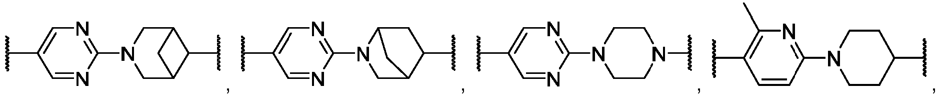

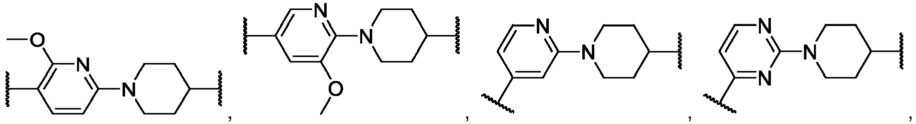

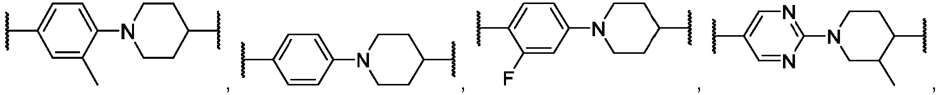

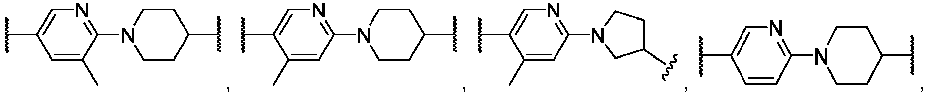

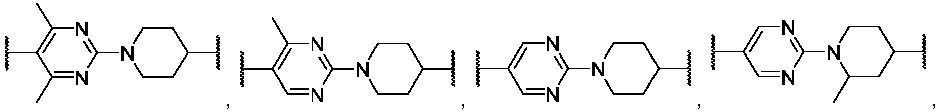

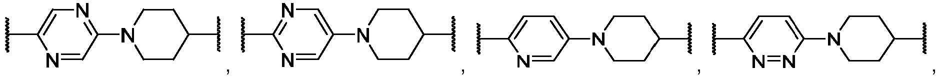

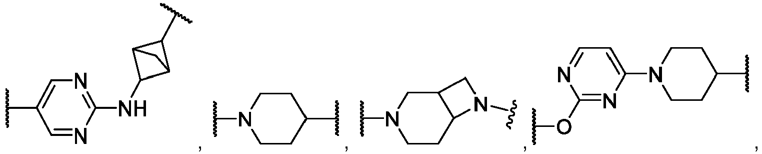

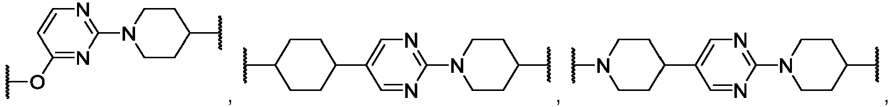

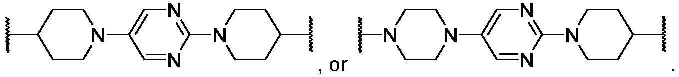

18. A compound selected from the group consisting of compounds 1-291 in the Table below and pharmaceutically acceptable salts thereof:
| # | **Compound** |
|---|---|
| 1 | 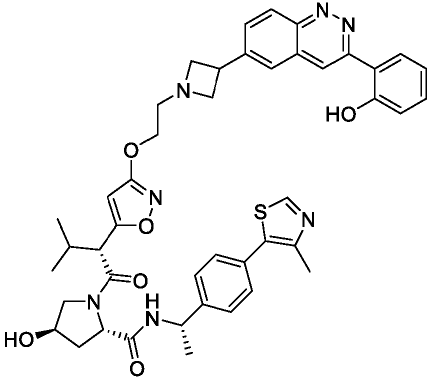 |
| 2 | 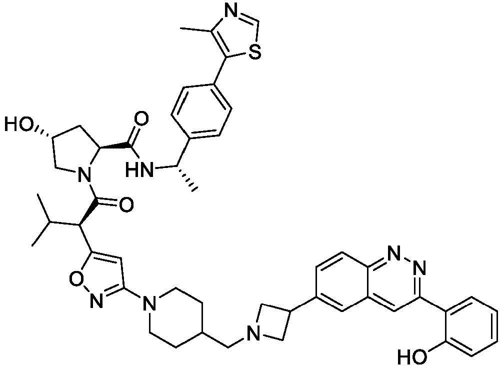 |
| 3 | 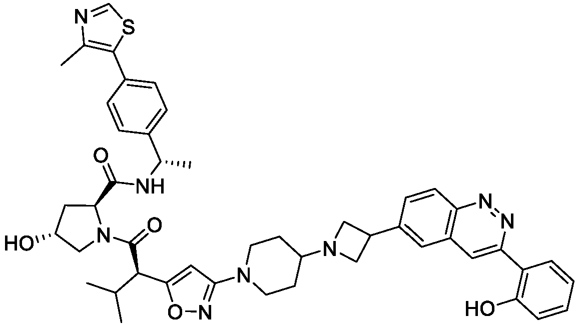 |
| 4 | 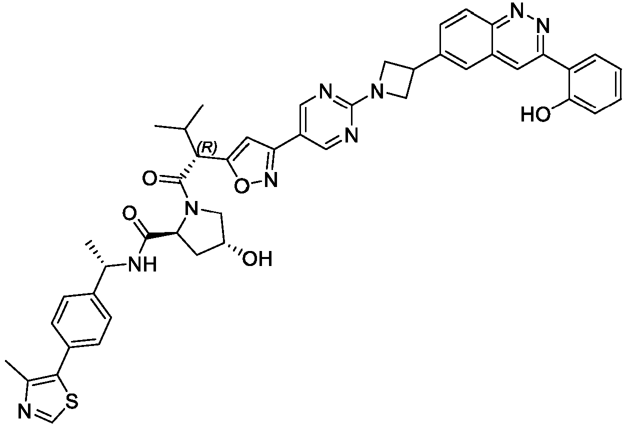 |
| 5 | 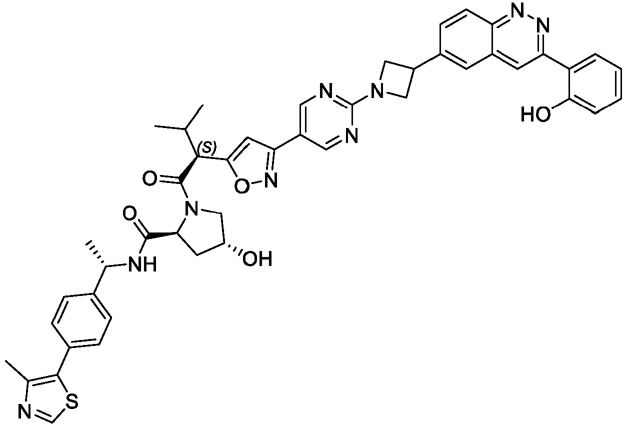 |
| 6 | 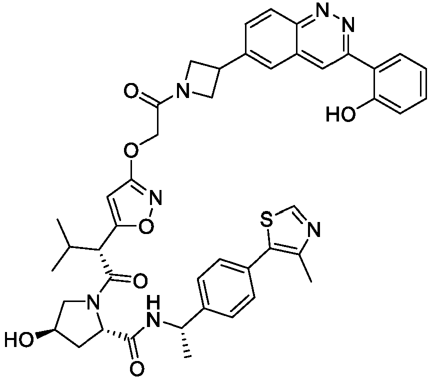 |
| 7 | 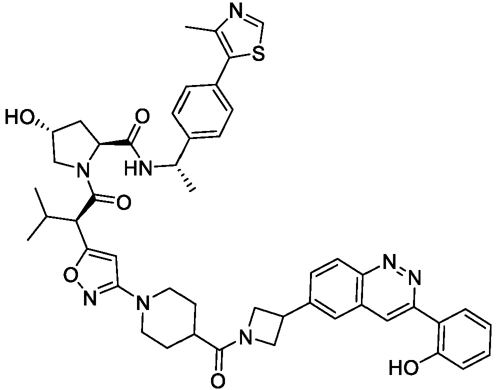 |
| 8 | 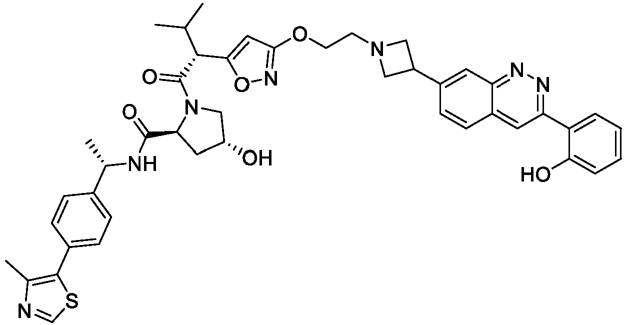 |
| 9 | 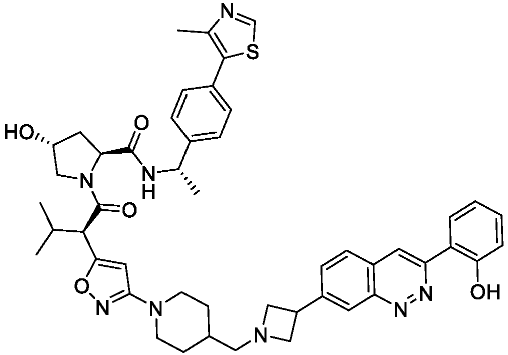 |
| 10 | 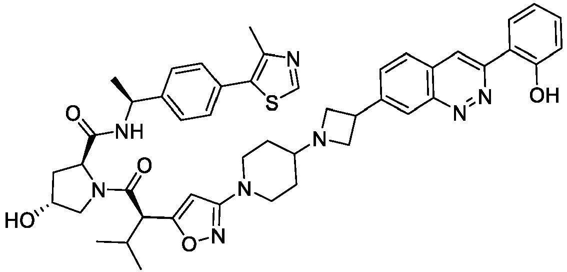 |
| 11 | 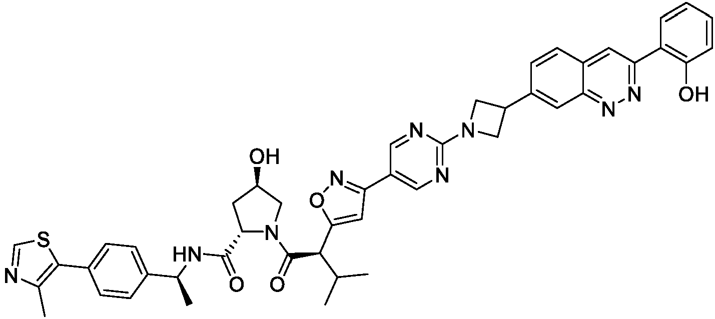 |
| 12 | 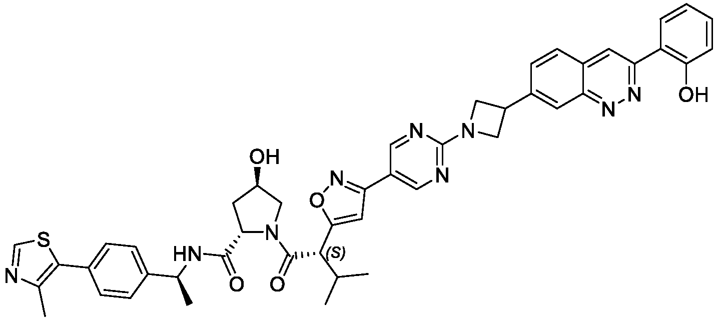 |
| 13 | 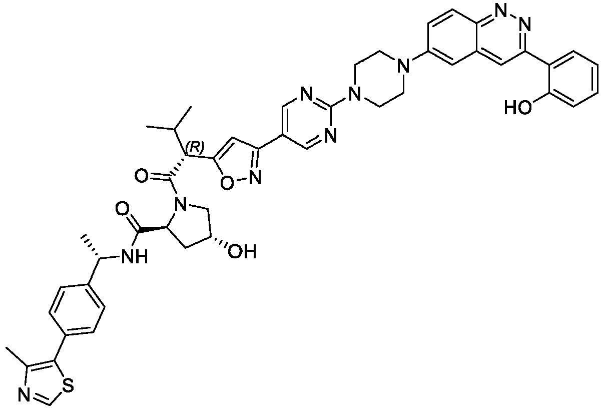 |
| 14 | 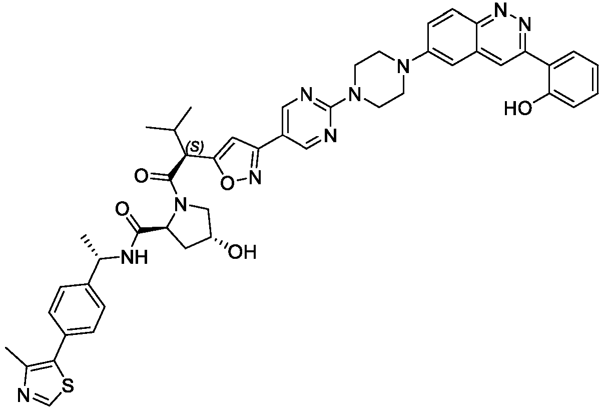 |
| 15 | 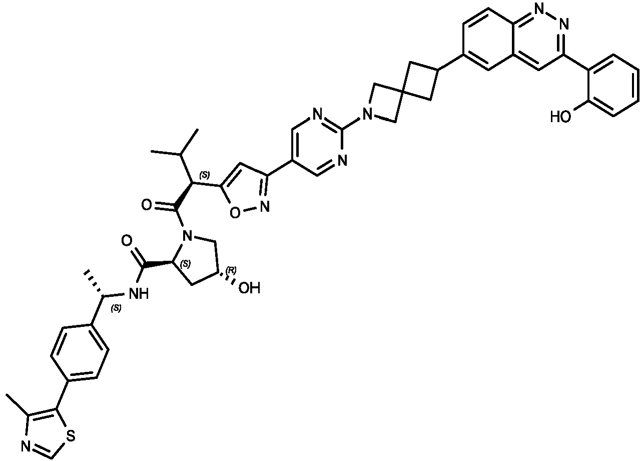 |
| 16 | 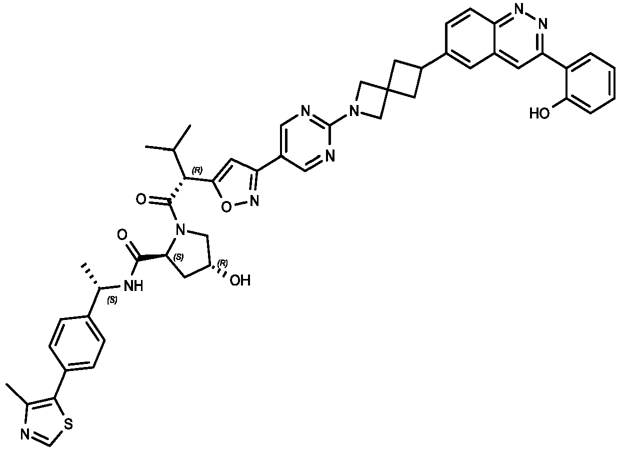 |
| 17 | 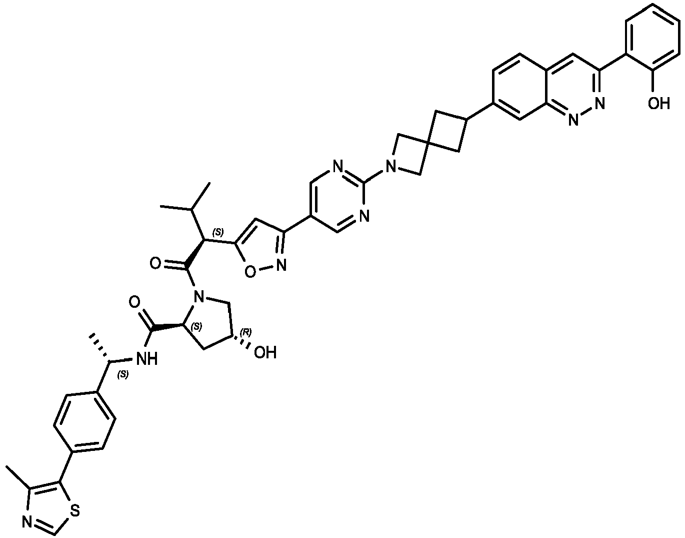 |
| 18 | 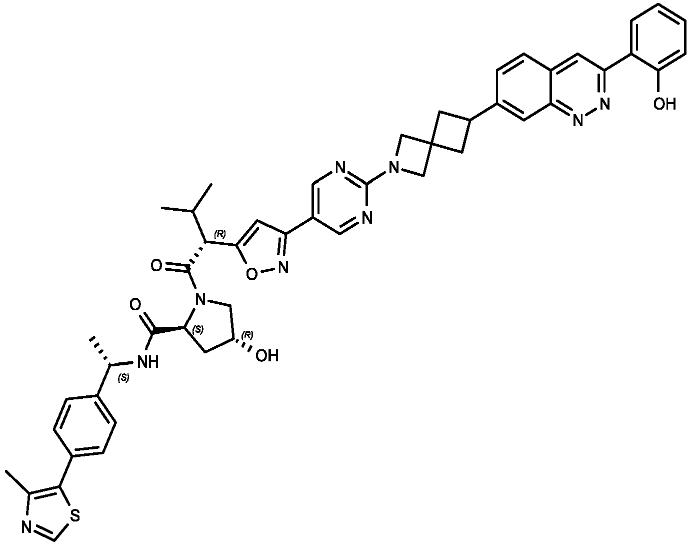 |
| 19 | 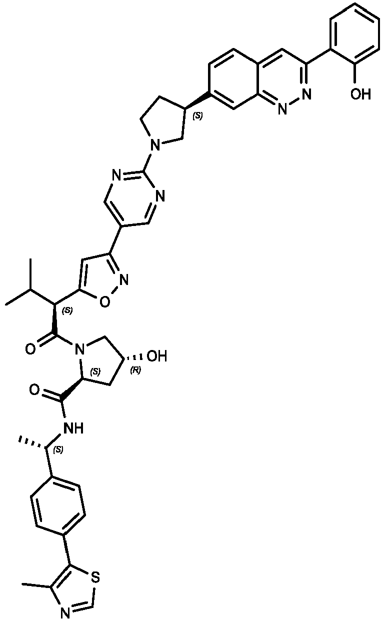 |
| 20 | 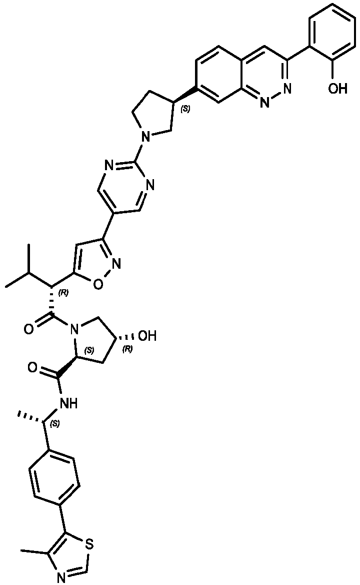 |
| 21 | 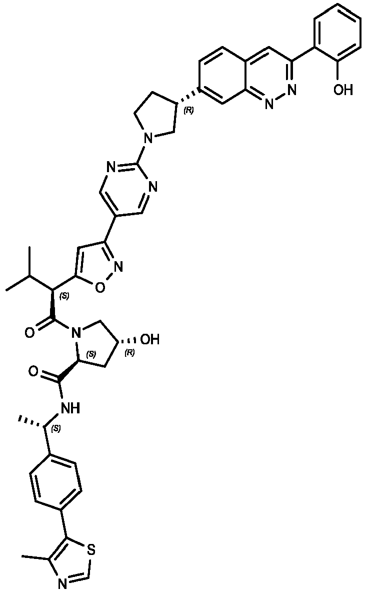 |
| 22 | 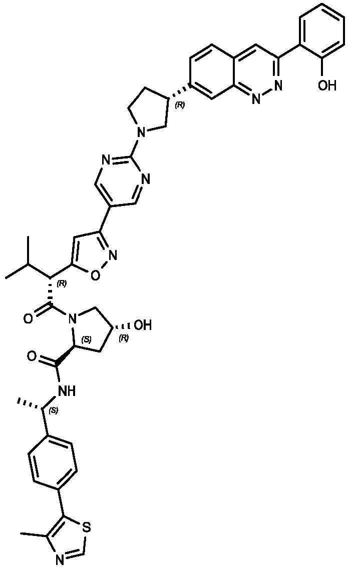 |
| 23 | 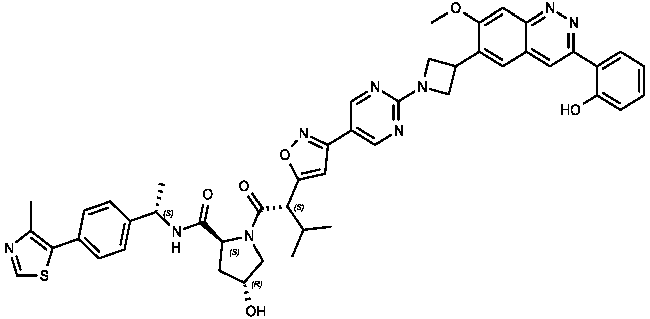 |
| 24 | 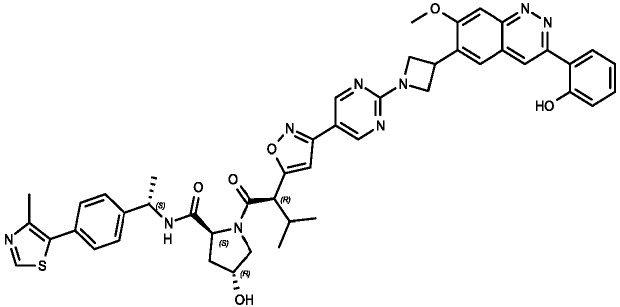 |
| 25 | 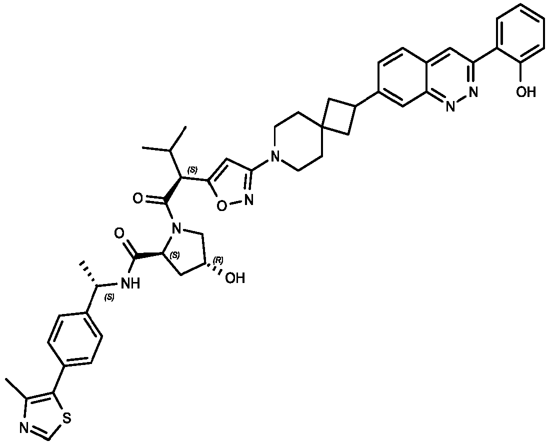 |
| 26 | 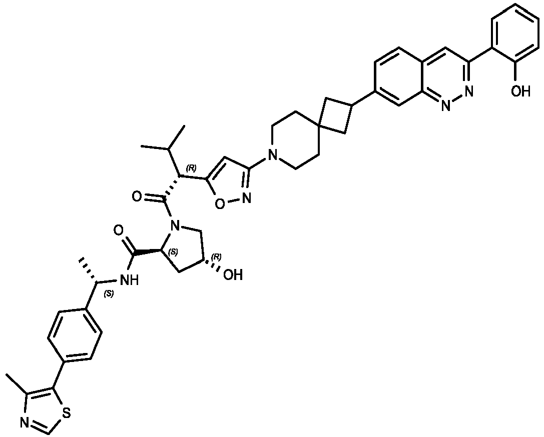 |
| 27 | 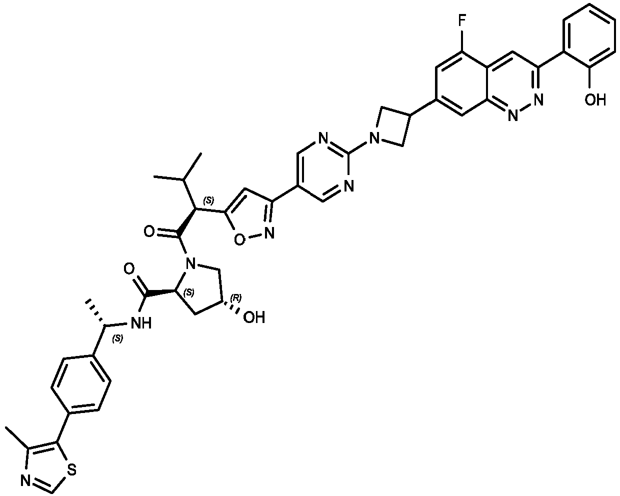 |
| 28 | 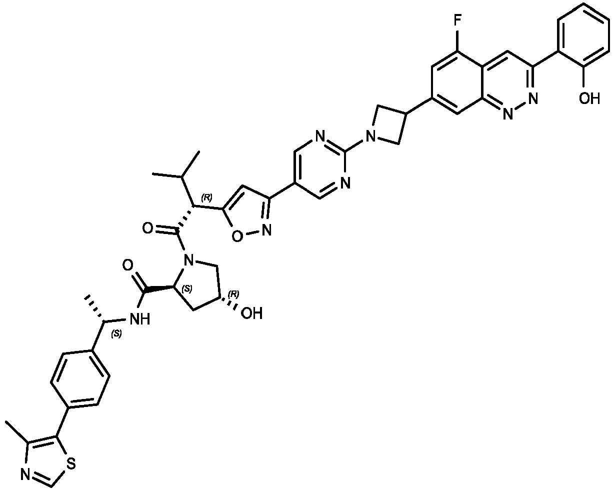 |
| 29 | 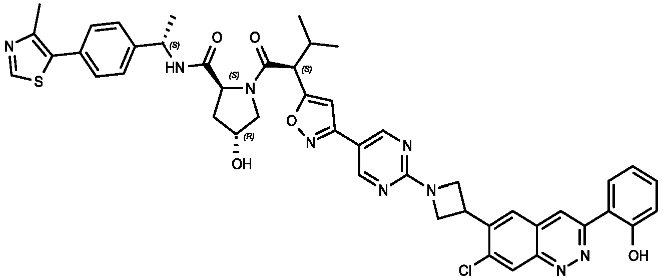 |
| 30 | 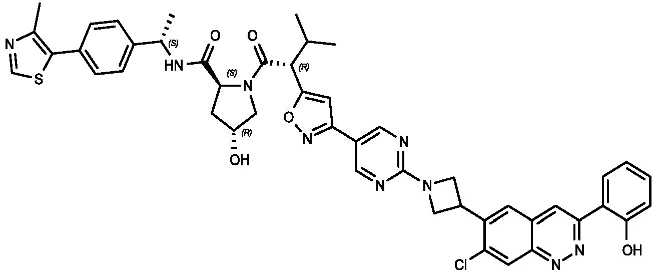 |
| 31 | 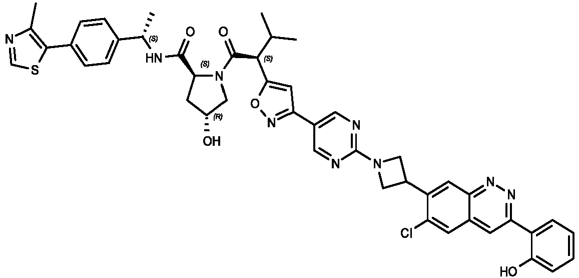 |
| 32 | 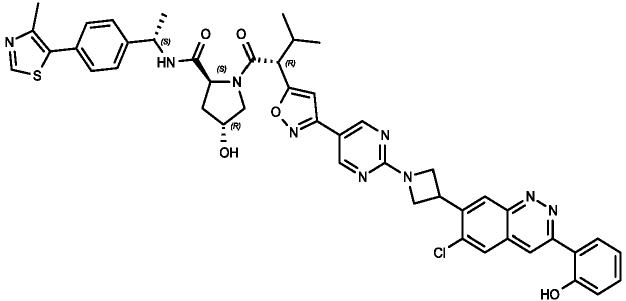 |
| 33 | 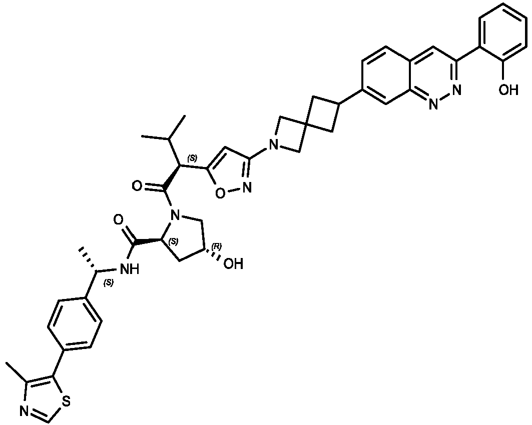 |
| 34 | 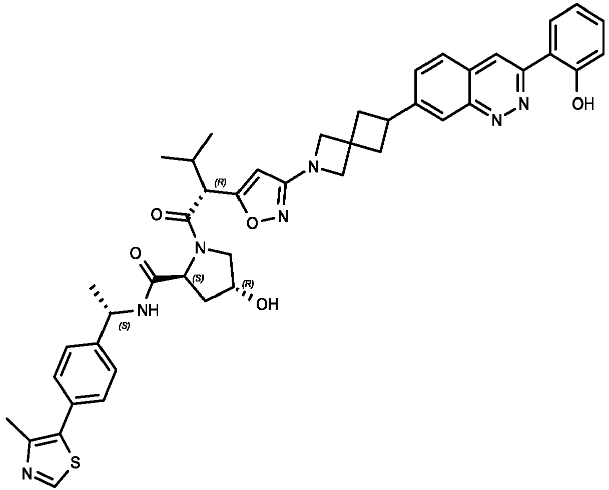 |
| 35 | 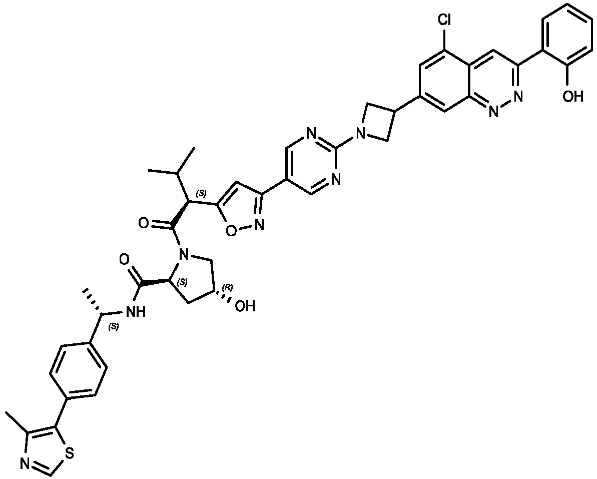 |
| 36 | 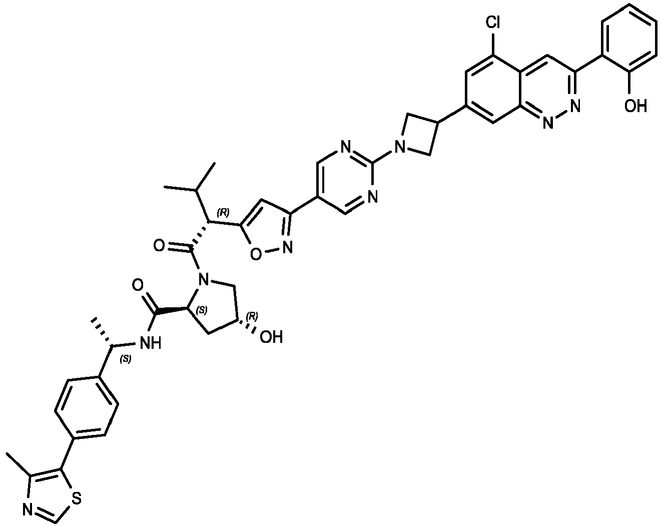 |
| 37 | 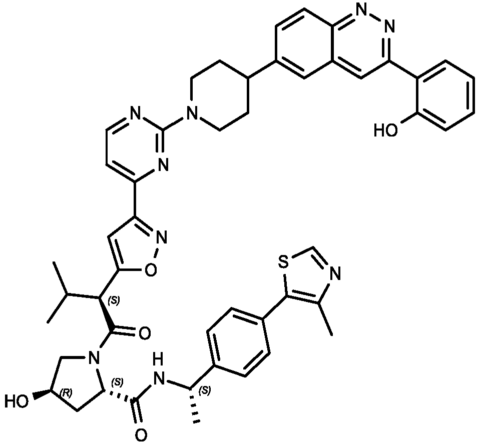 |
| 38 | 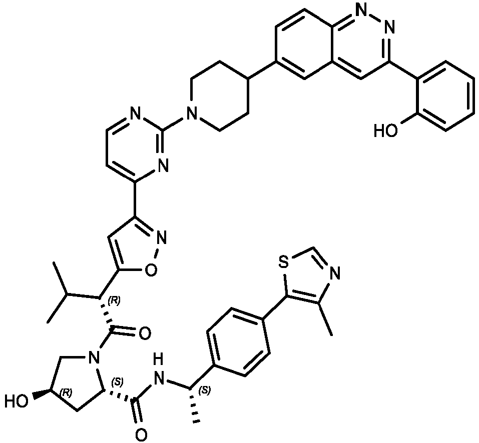 |
| 39 | 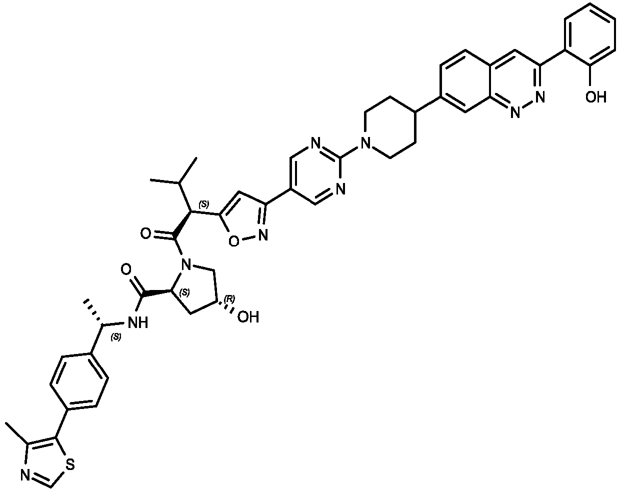 |
| 40 | 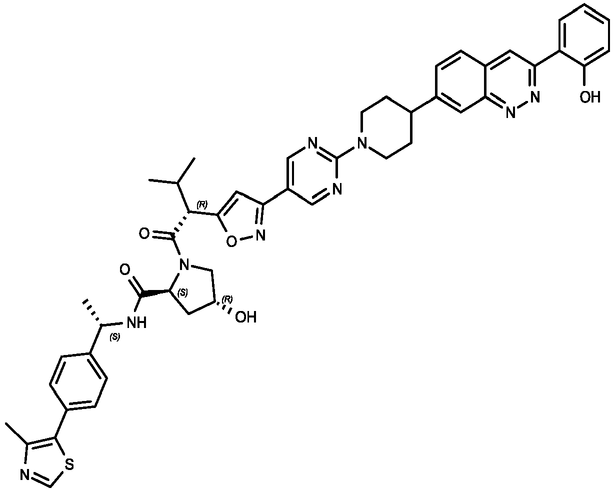 |
| 41 | 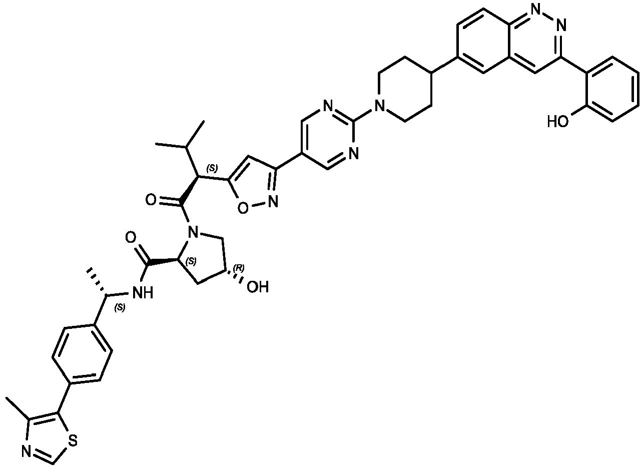 |
| 42 | 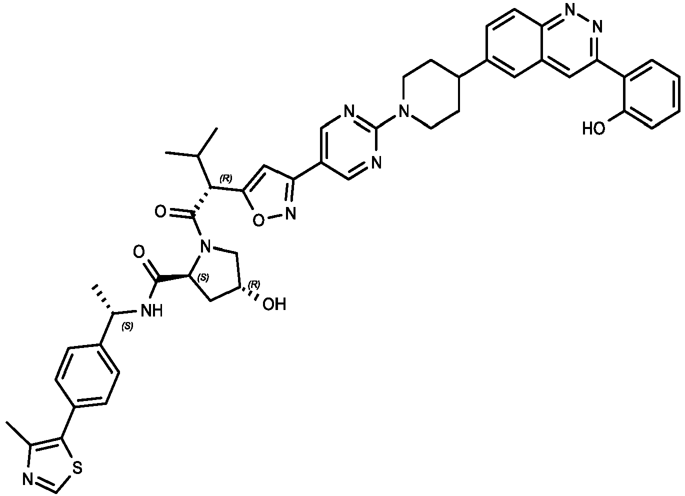 |
| 43 | 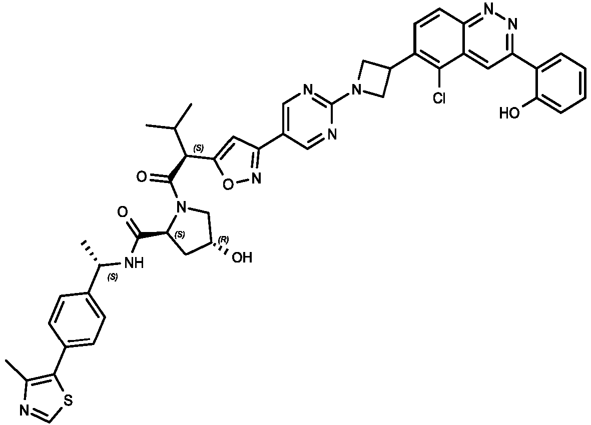 |
| 44 | 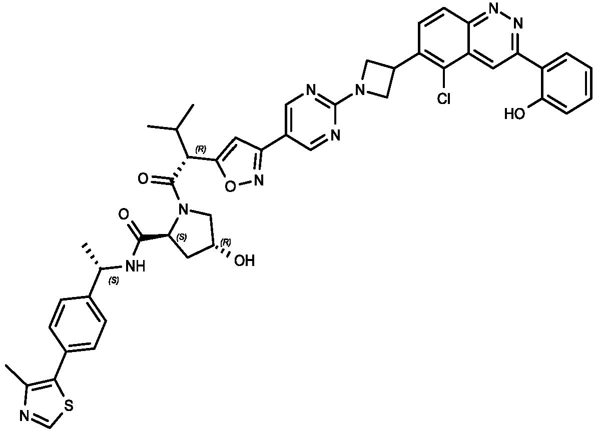 |
| 45 | 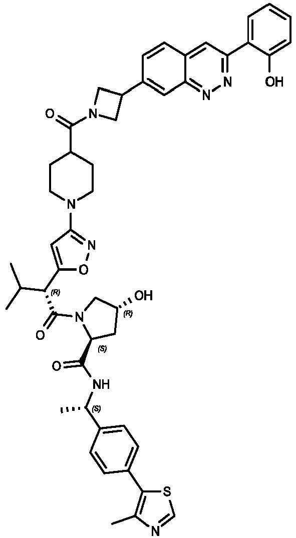 |
| 46 | 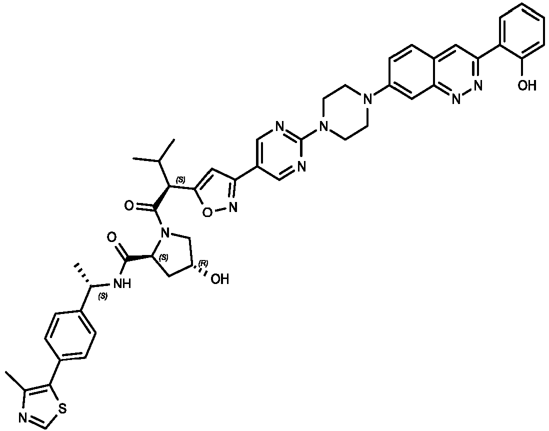 |
| 47 | 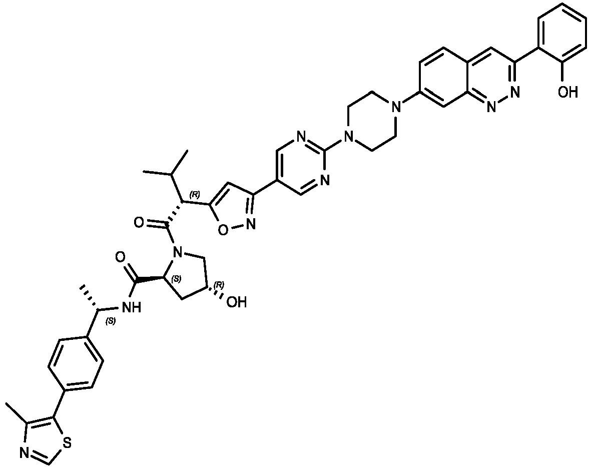 |
| 48 | 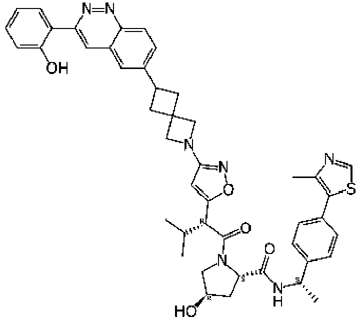 |
| 49 | 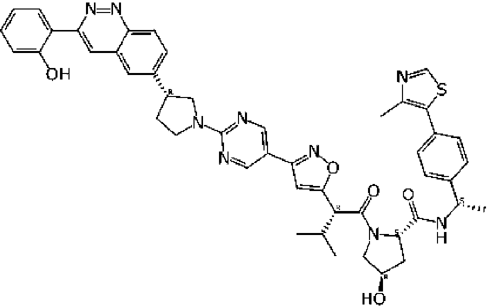 |
| 50 | 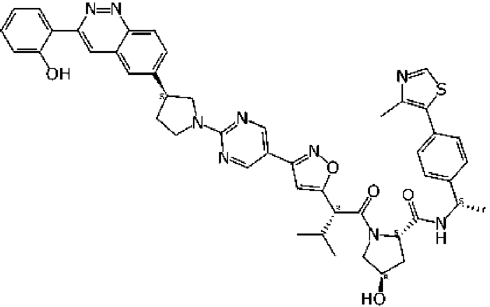 |
| 51 | 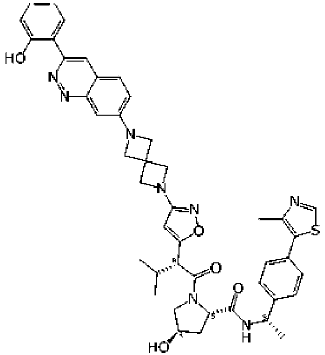 |
| 52 | 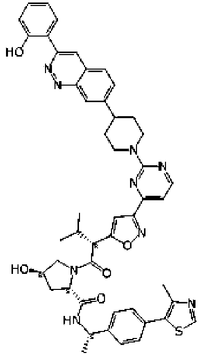 |
| 53 | 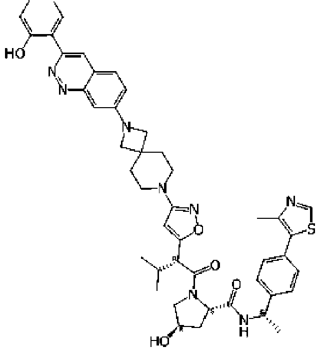 |
| 54 | 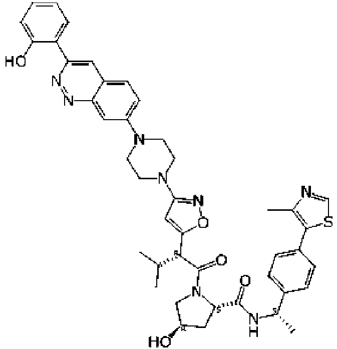 |
| 55 | 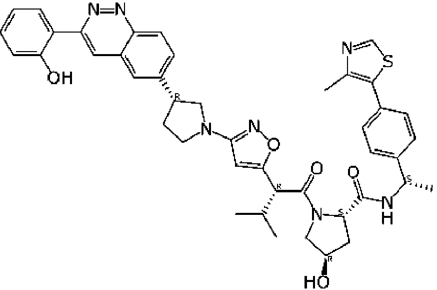 |
| 56 | 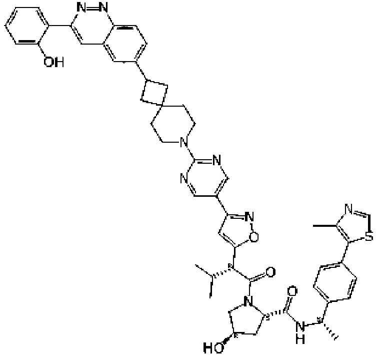 |
| 57 | 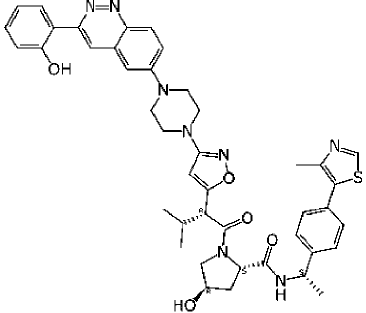 |
| 58 | 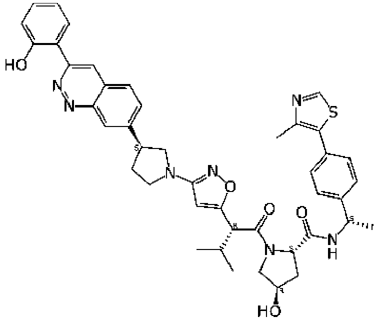 |
| 59 | 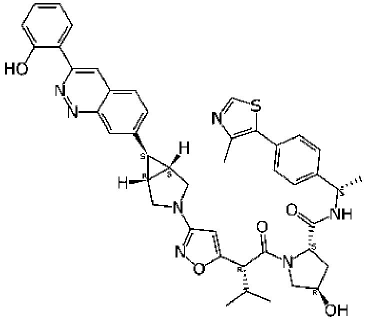 |
| 60 | 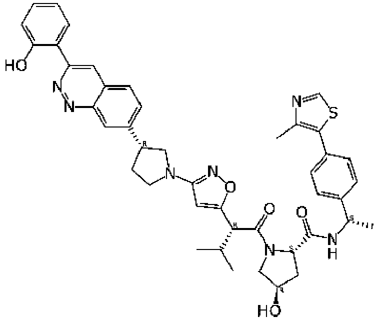 |
| 61 | 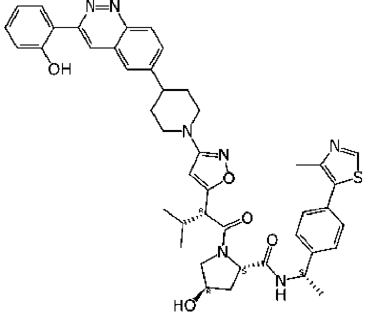 |
| 62 | 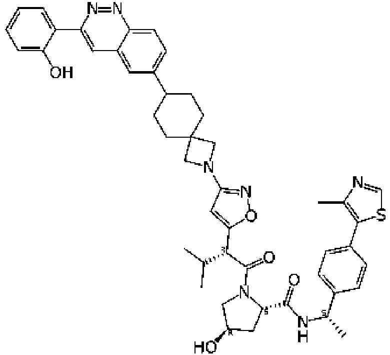 |
| 63 | 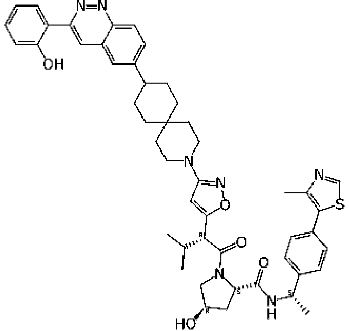 |
| 64 | 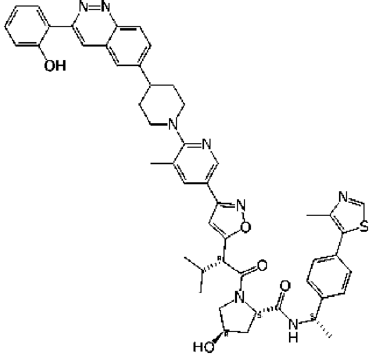 |
| 65 | 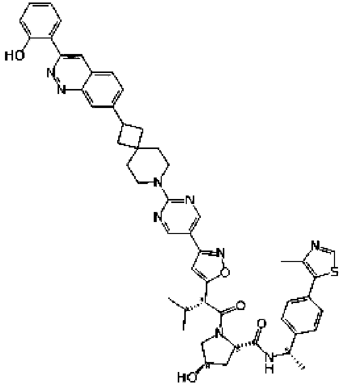 |
| 66 | 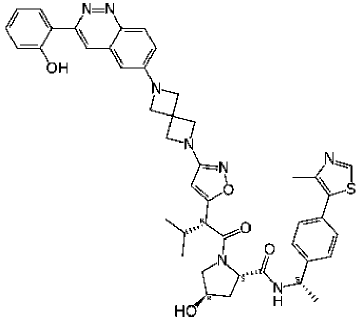 |
| 67 | 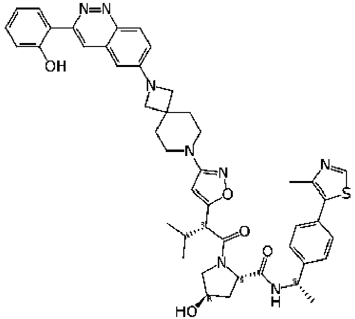 |
| 68 | 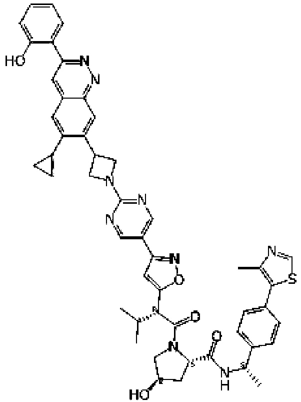 |
| 69 | 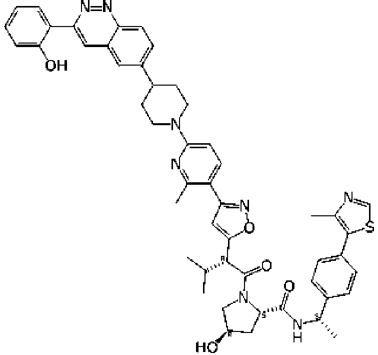 |
| 70 | 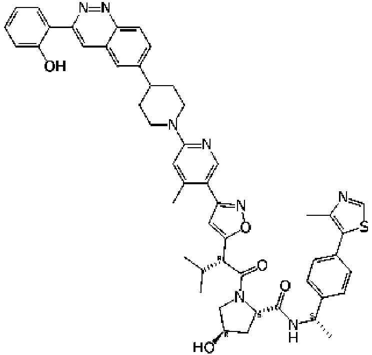 |
| 71 | 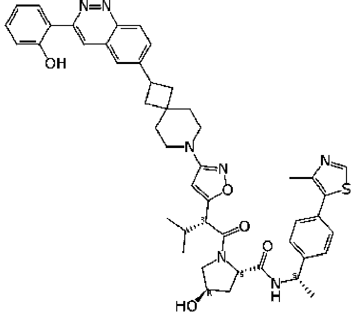 |
| 72 | 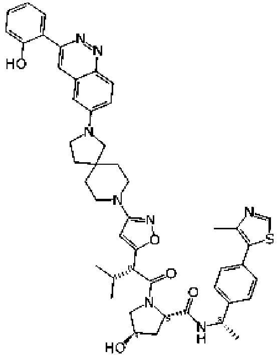 |
| 73 | 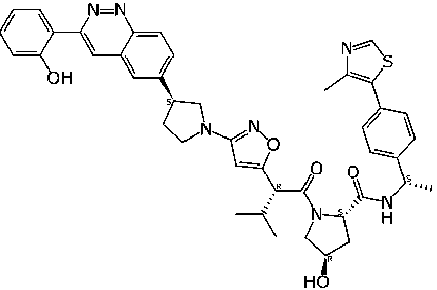 |
| 74 | 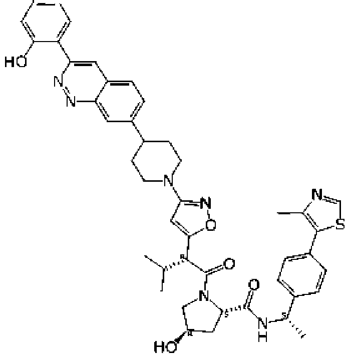 |
| 75 | 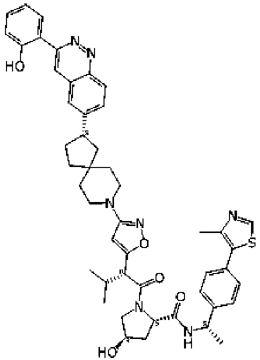 |
| 76 | 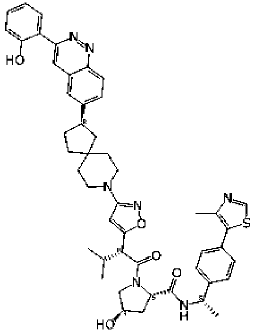 |
| 77 | 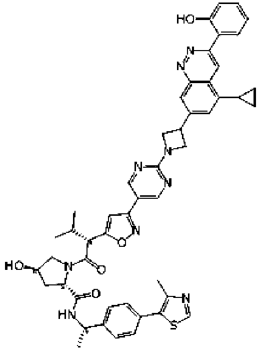 |
| 78 | 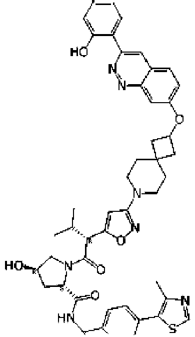 |
| | |
| 79 | 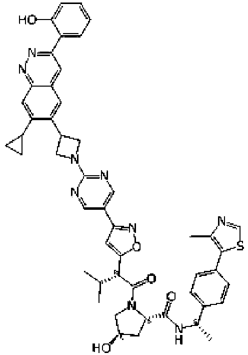 |
| 80 | 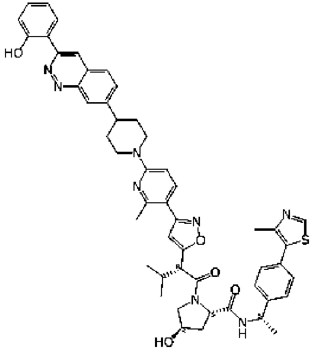 |
| 81 | 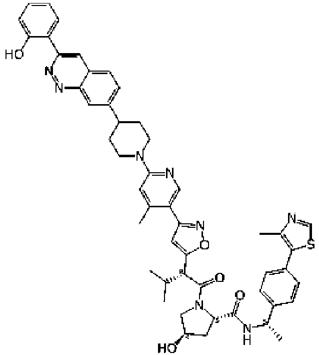 |
| 82 | 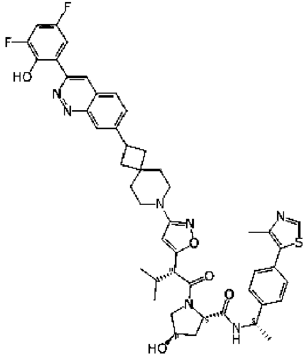 |
| 83 | 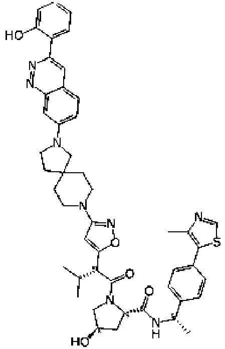 |
| 84 | 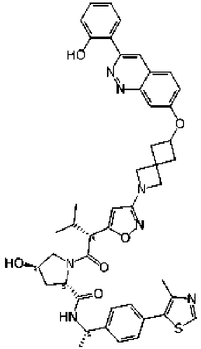 |
| 85 | 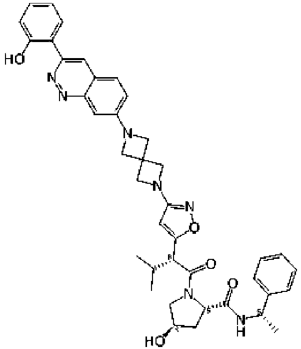 |
| 86 | 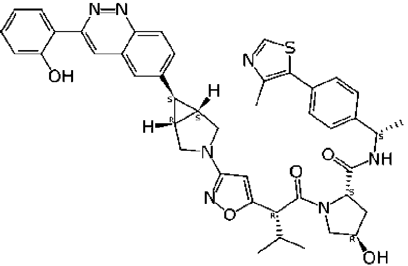 |
| 87 | 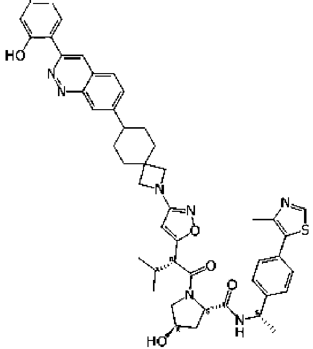 |
| 88 | 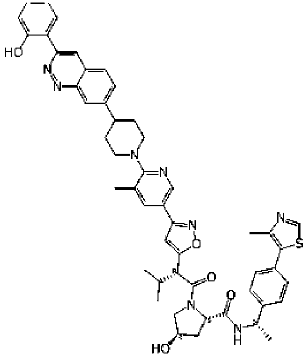 |
| 89 | 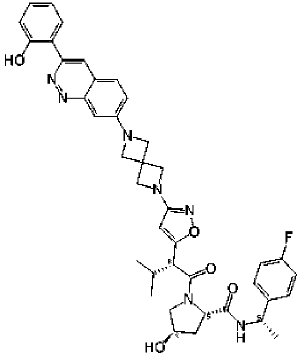 |
| 90 | 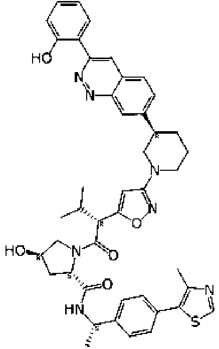 |
| 91 | 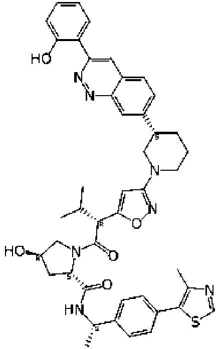 |
| 92 | 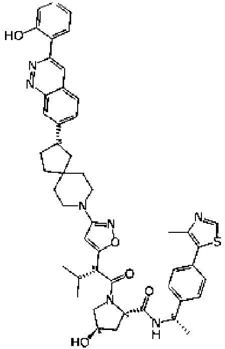 |
| 93 | 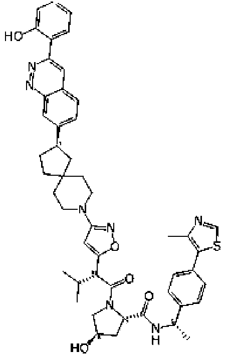 |
| 94 | 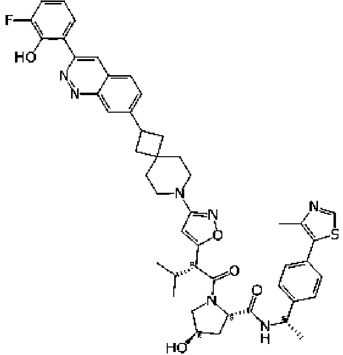 |
| 95 | 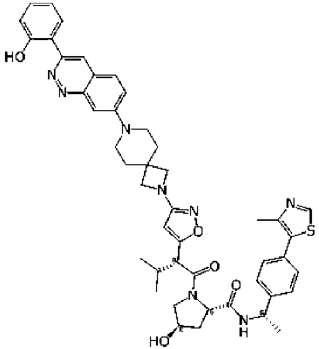 |
| 96 | 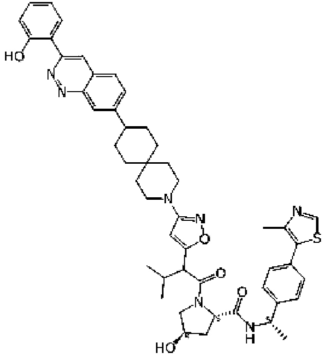 |
| 97 | 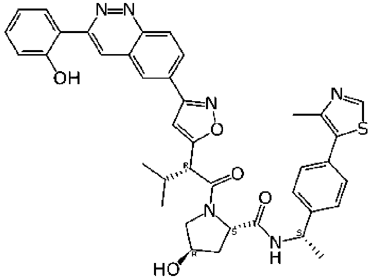 |
| 98 | 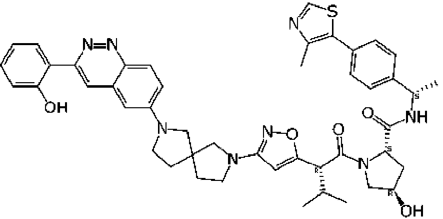 |
| 99 | 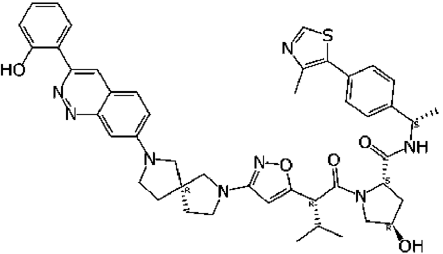 |
| 100 | 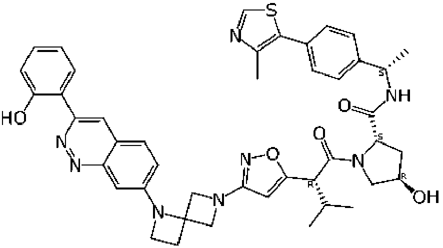 |
| 101 | 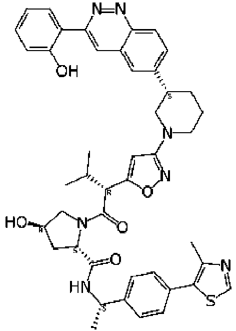 |
| 102 | 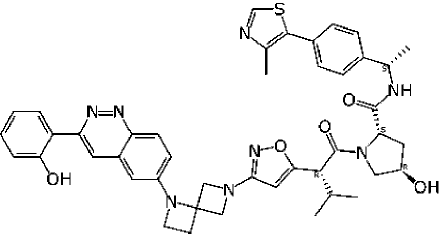 |
| 103 | 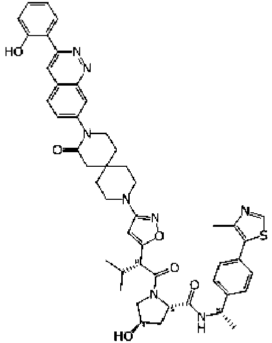 |
| 104 | 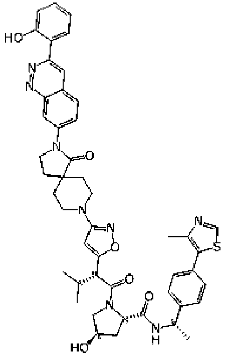 |
| 105 | 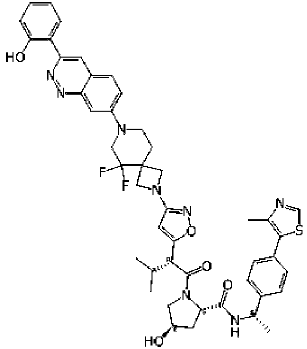 |
| 106 | 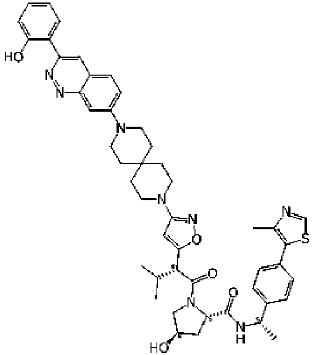 |
| 107 | 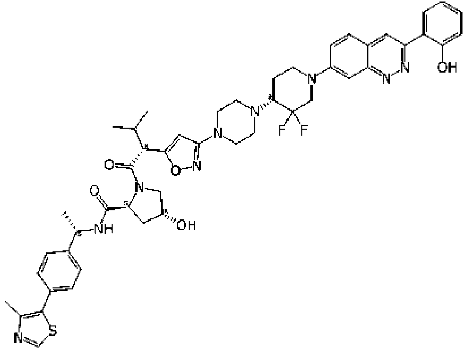 |
| 108 | 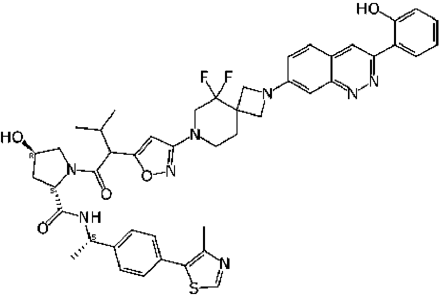 |
| 109 | 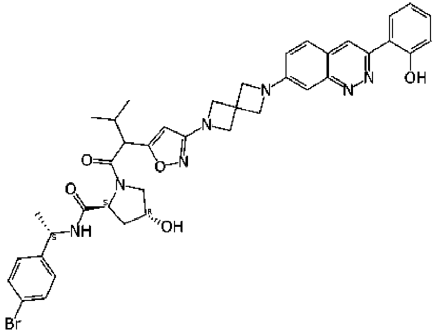 |
| 110 | 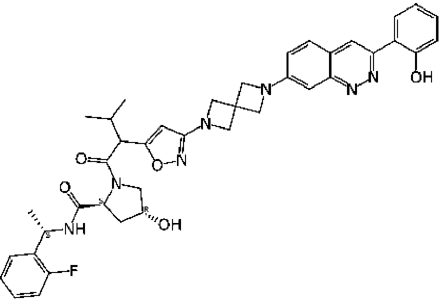 |
| 111 | 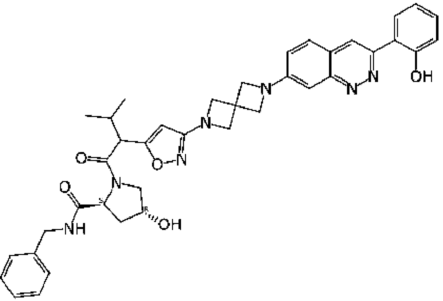 |
| 112 | 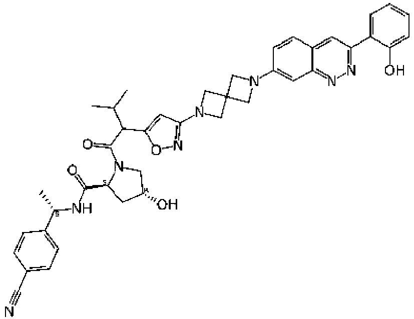 |
| 113 | 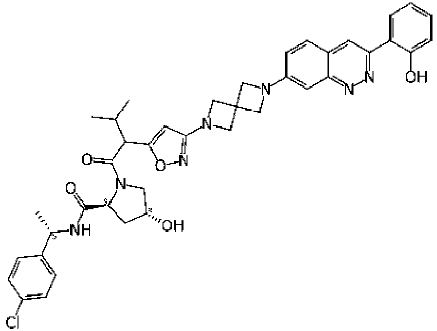 |
| 114 | 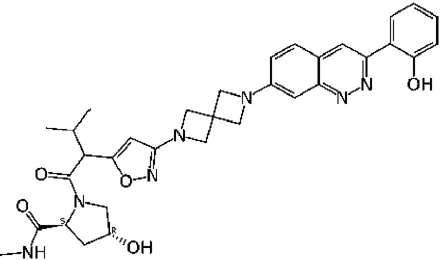 |
| 115 | 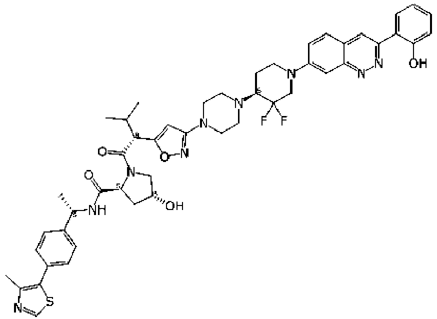 |
| 116 | 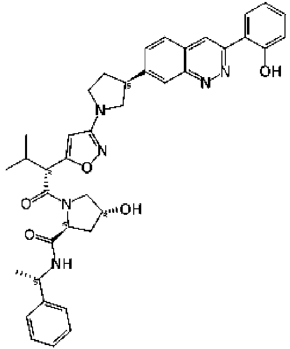 |
| 117 | 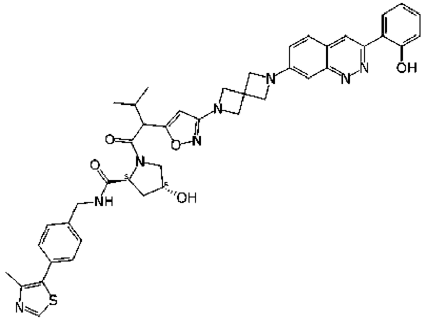 |
| 118 | 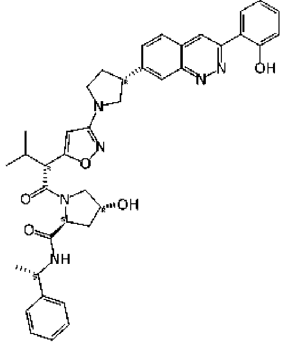 |
| 119 | 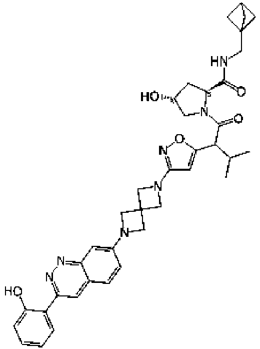 |
| 120 | 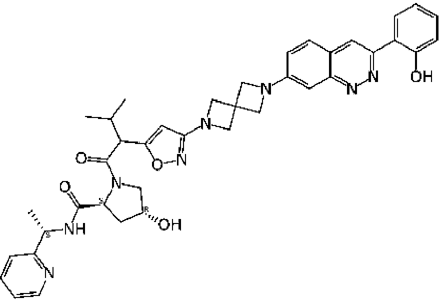 |
| 121 | 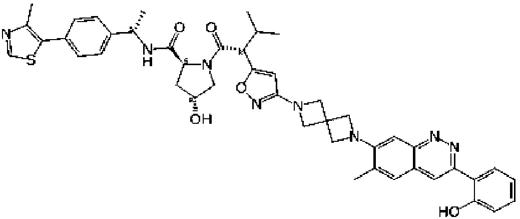 |
| 122 | 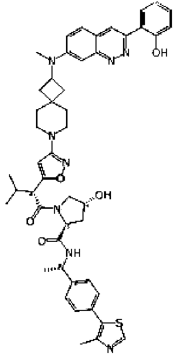 |
| 123 | 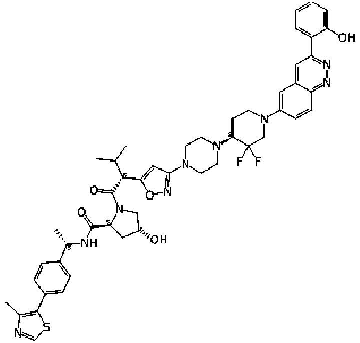 |
| 124 | 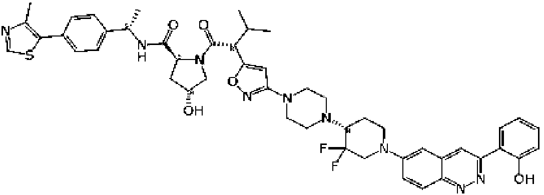 |
| 125 | 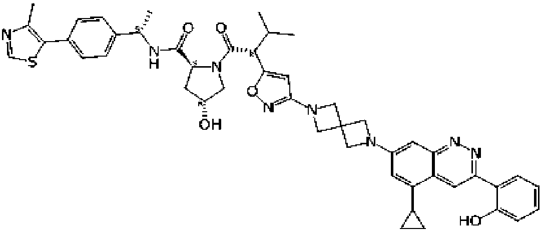 |
| 126 | 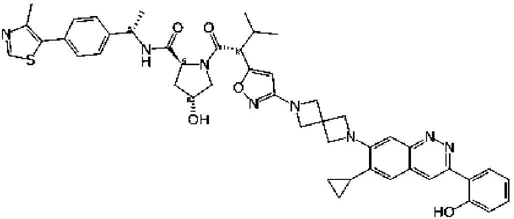 |
| 127 | 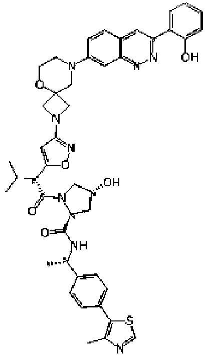 |
| 128 | 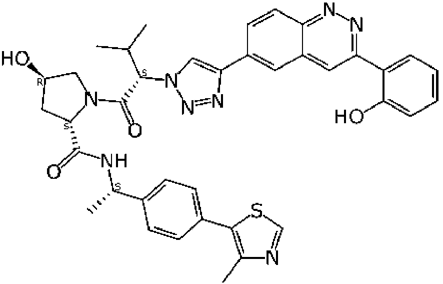 |
| 129 | 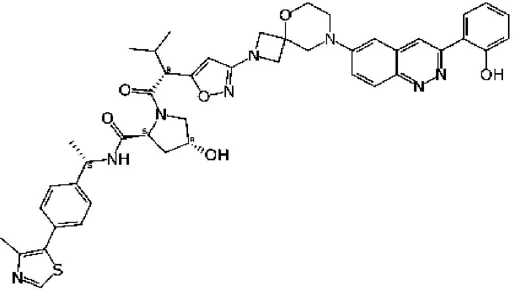 |
| 130 | 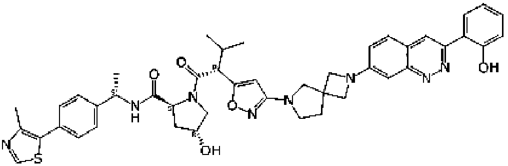 |
| 131 | 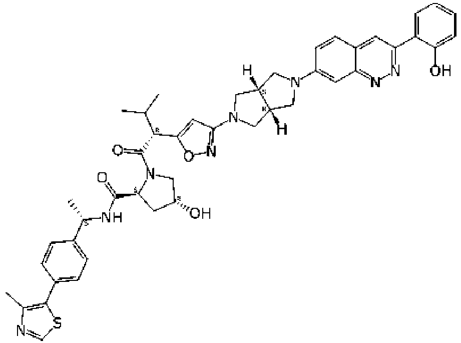 |
| 132 | 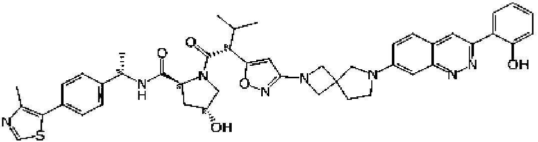 |
| 133 | 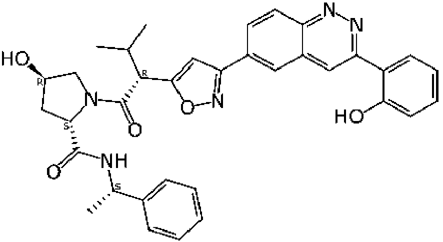 |
| 134 | 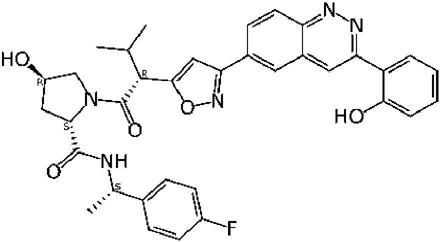 |
| 135 | 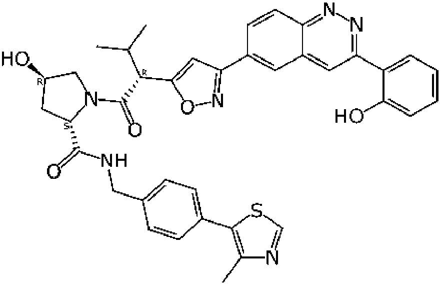 |
| 136 | 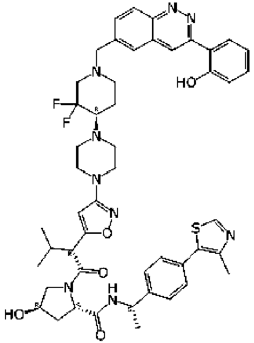 |
| 137 | 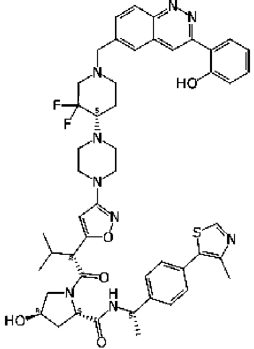 |
| 138 | 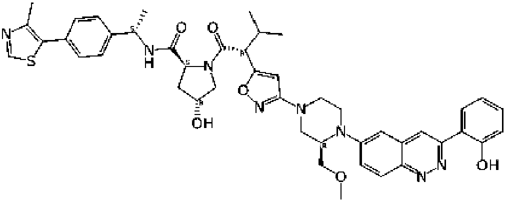 |
| 139 | 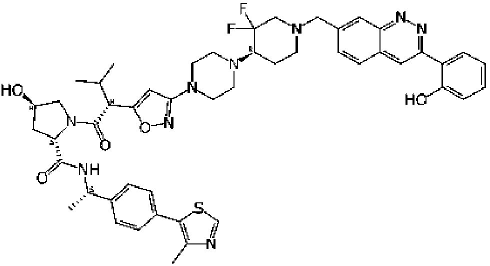 |
| 140 | 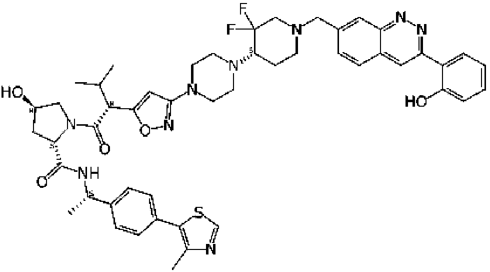 |
| 141 | 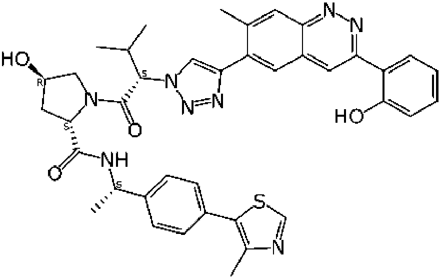 |
| 142 | 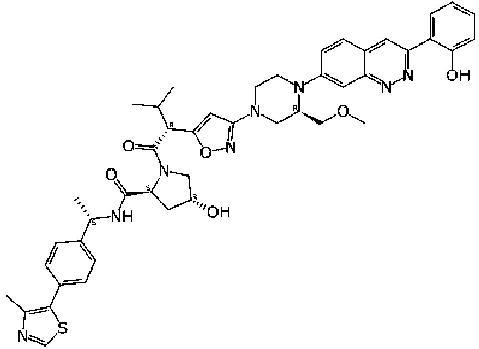 |
| 143 | 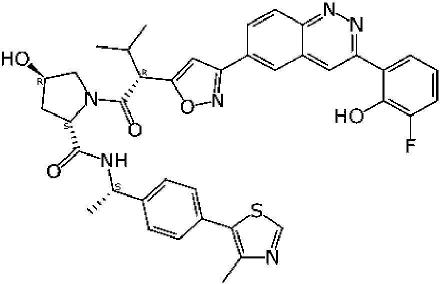 |
| 144 | 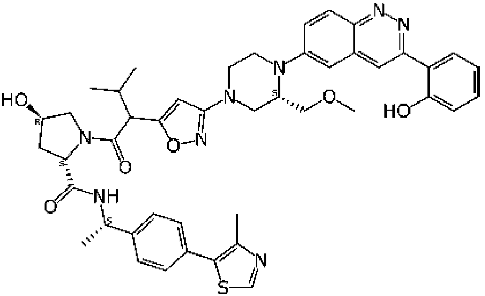 |
| 145 | 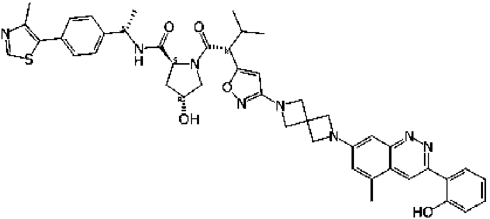 |
| 146 | 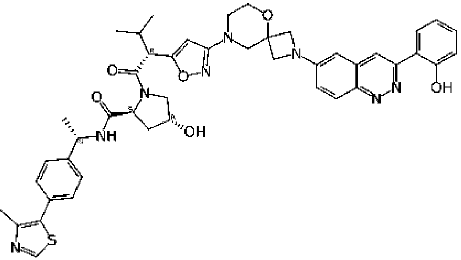 |
| 147 | 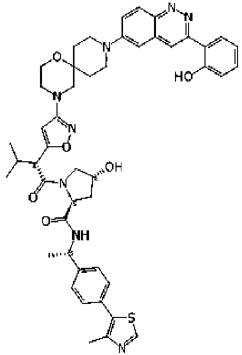 |
| 148 | 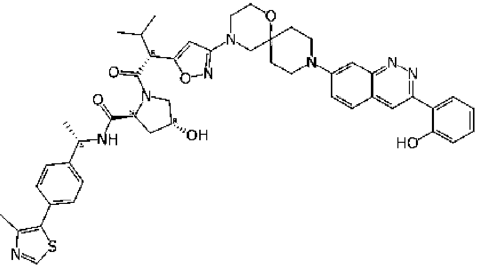 |
| 149 | 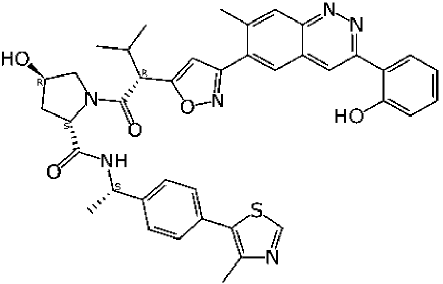 |
| 150 | 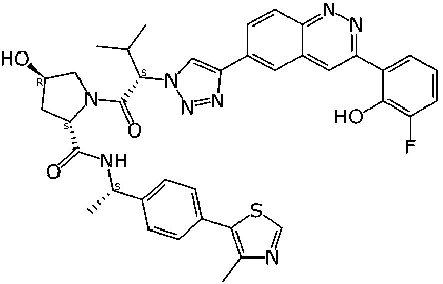 |
| 151 | 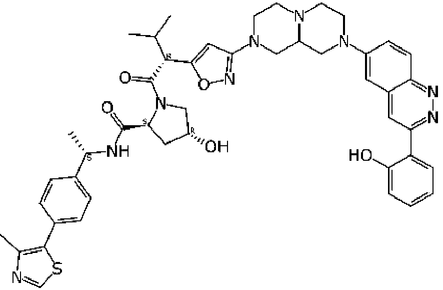 |
| 152 | 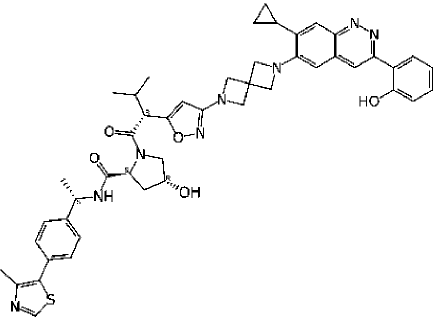 |
| 153 | 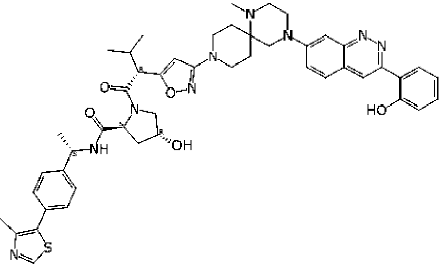 |
| 154 | 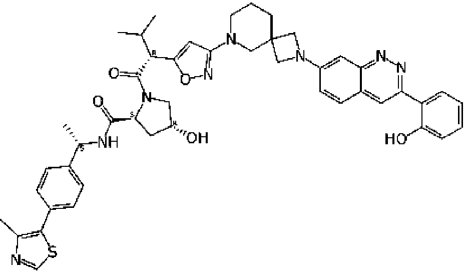 |
| 155 | 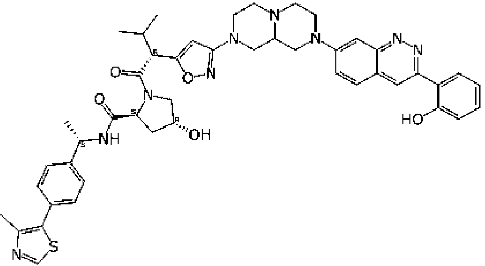 |
| 156 | 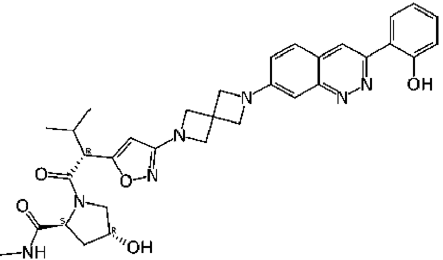 |
| 157 | 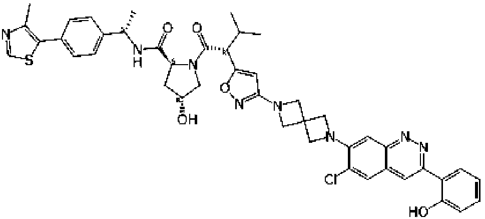 |
| 158 | 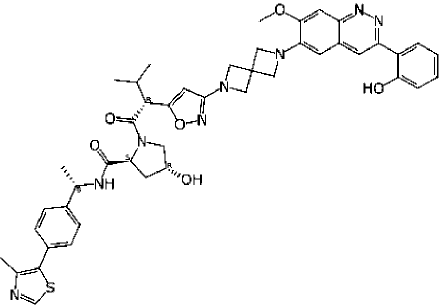 |
| 159 | 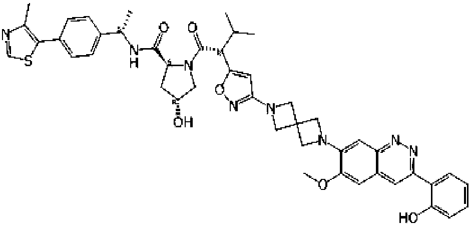 |
| 160 | 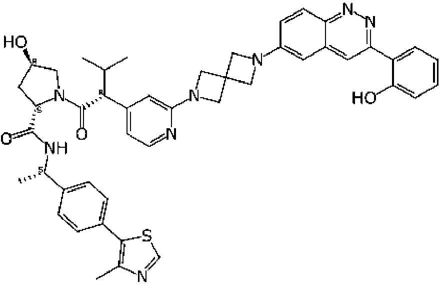 |
| 161 | 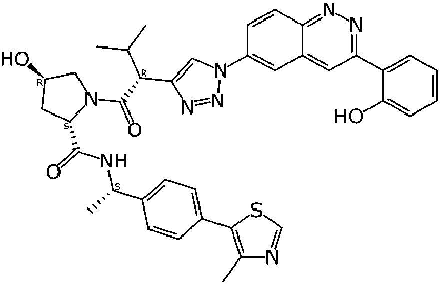 |
| 162 | 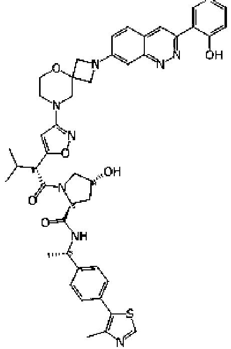 |
| 163 | 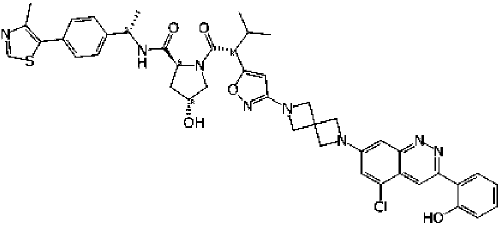 |
| 164 | 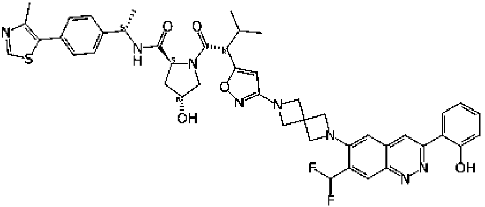 |
| 165 | 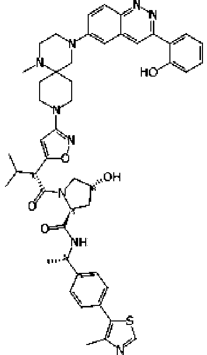 |
| 166 | 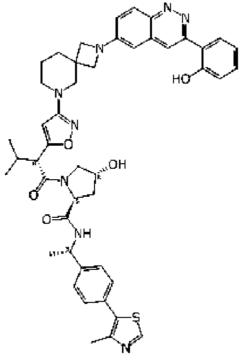 |
| 167 | 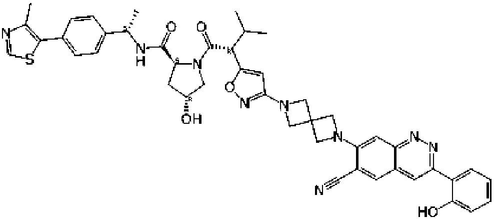 |
| 168 | 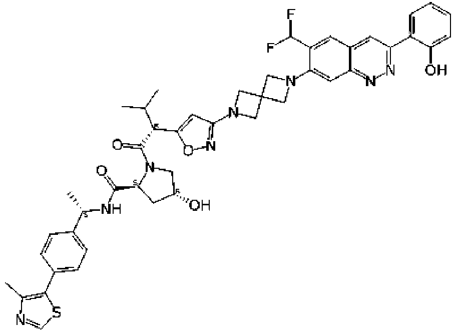 |
| 169 | 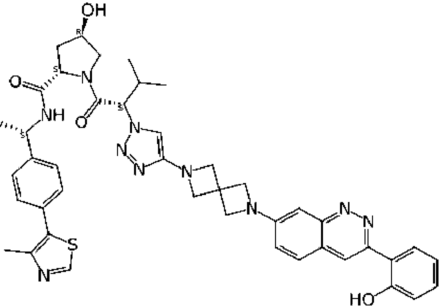 |
| 170 | 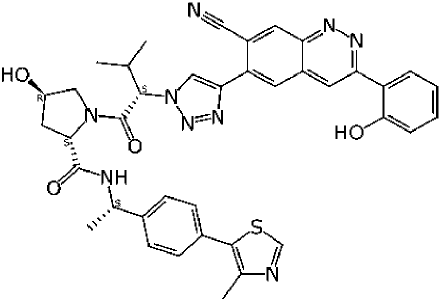 |
| 171 | 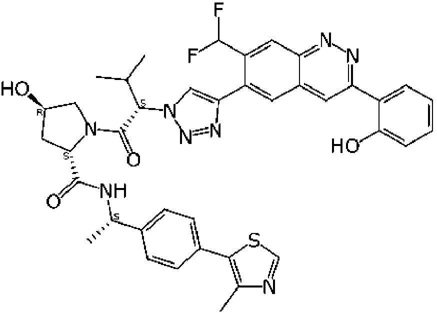 |
| 172 | 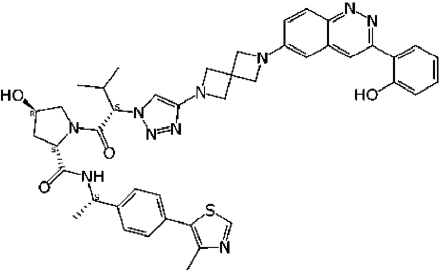 |
| 173 | 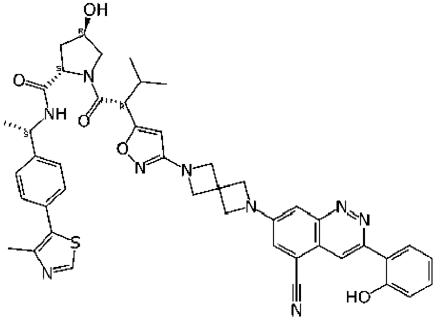 |
| 174 | 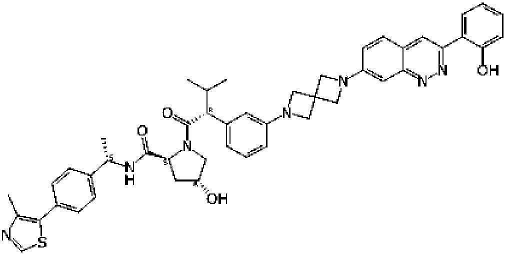 |
| 175 | 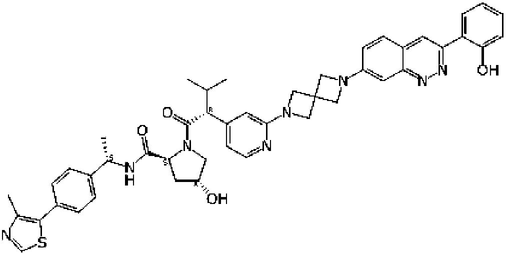 |
| 176 | 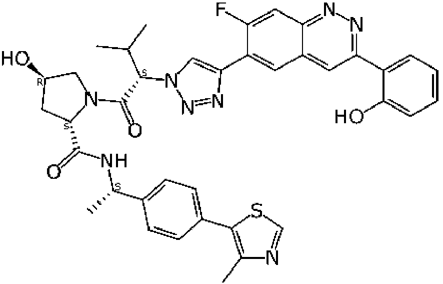 |
| 177 | 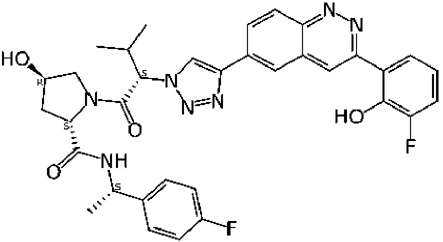 |
| 178 | 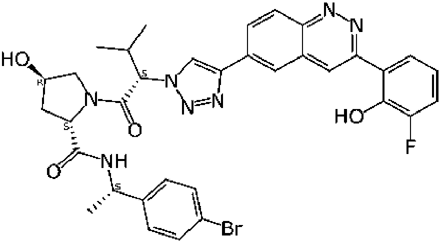 |
| 179 | 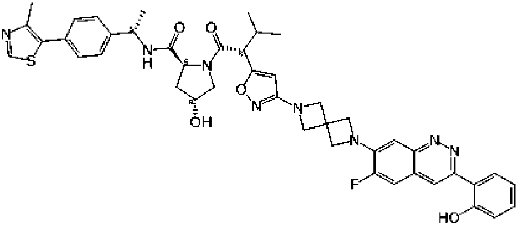 |
| 180 | 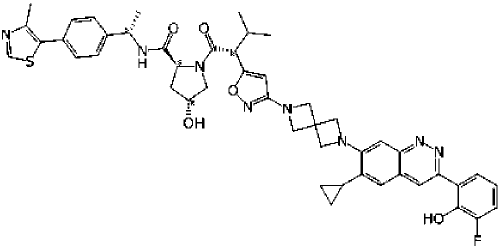 |
| 181 | 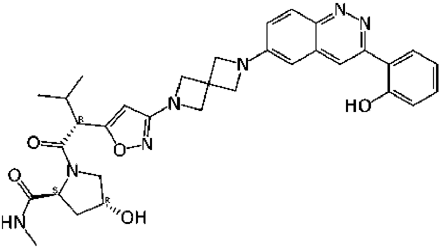 |
| 182 | 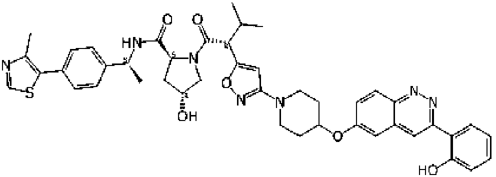 |
| 183 | 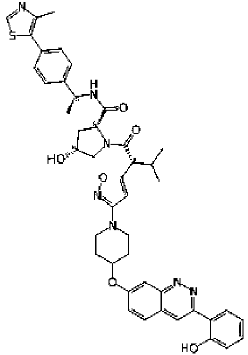 |
| 184 | 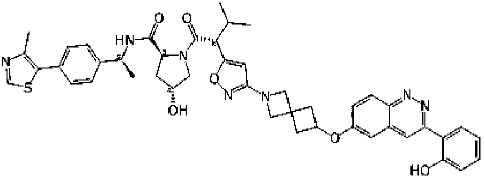 |
| 185 | 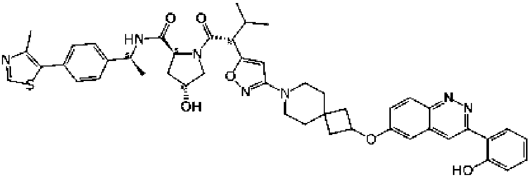 |
| 186 | 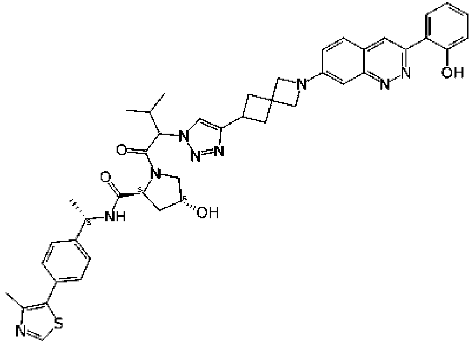 |
| 187 | 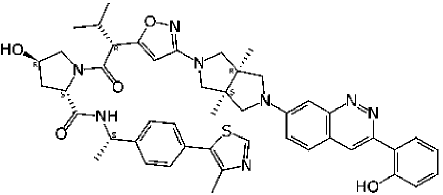 |
| 188 | 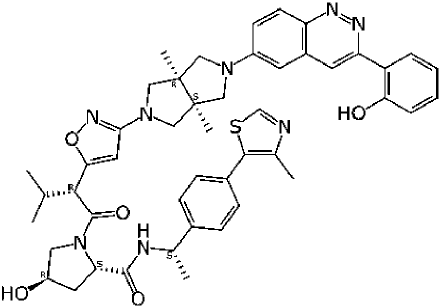 |
| 189 | 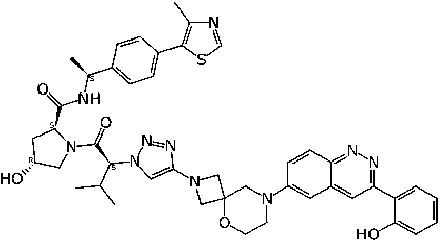 |
| 190 | 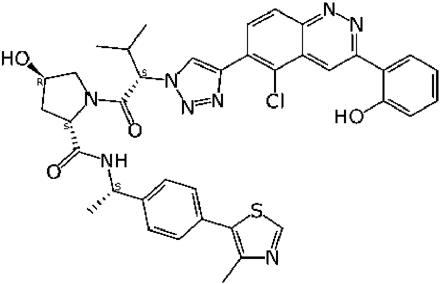 |
| 191 | 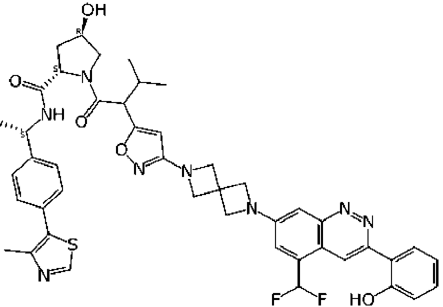 |
| 192 | 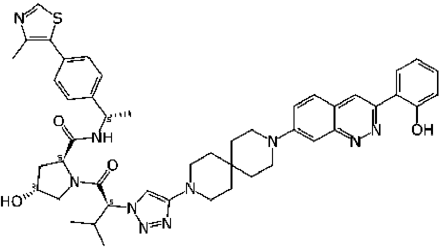 |
| 193 | 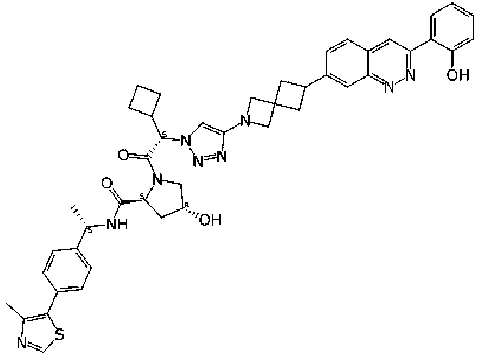 |
| 194 | 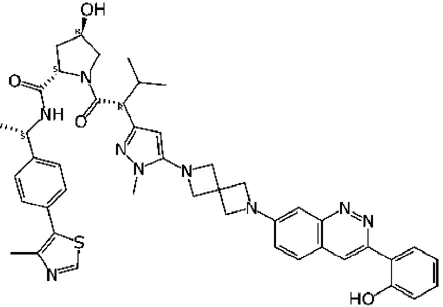 |
| 195 | 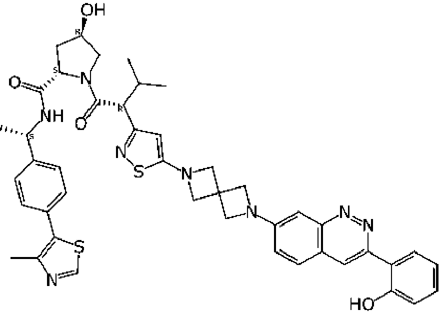 |
| 196 | 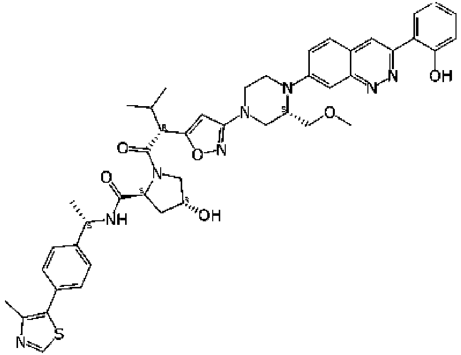 |
| 197 | 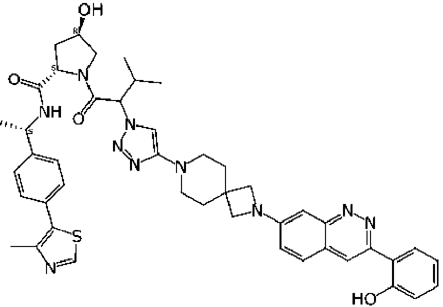 |
| 198 | 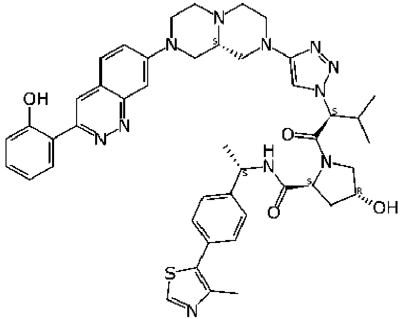 |
| 199 | 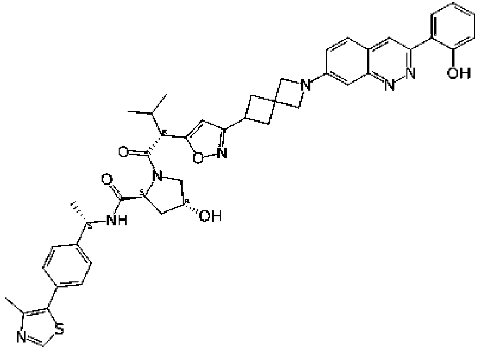 |
| 200 | 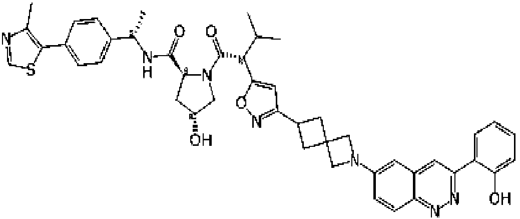 |
| 201 | 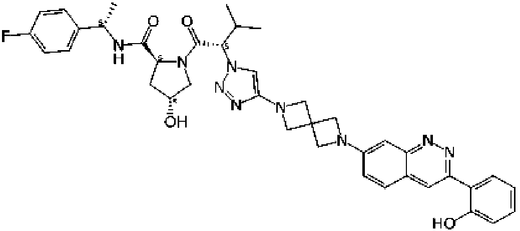 |
| 202 | 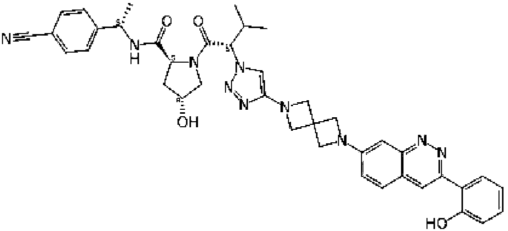 |
| 203 | 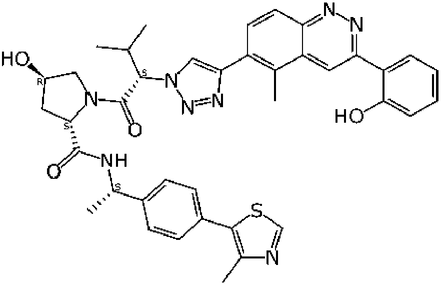 |
| 204 | 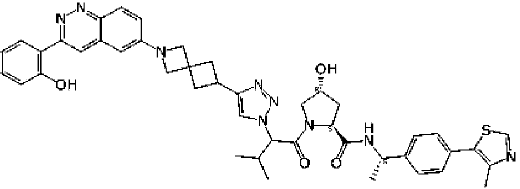 |
| 205 | 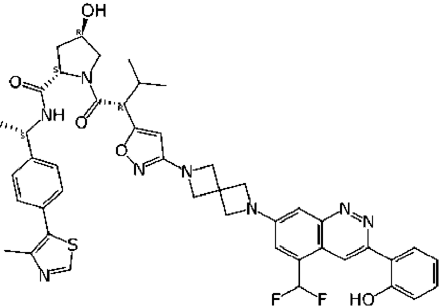 |
| 206 | 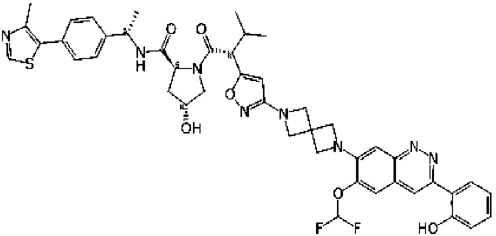 |
| 207 | 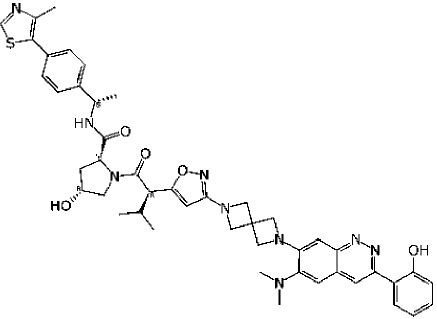 |
| 208 | 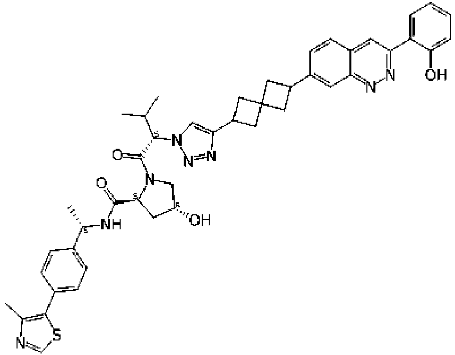 |
| 209 | 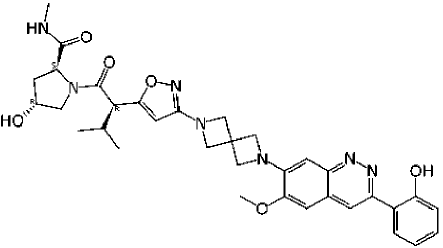 |
| 210 | 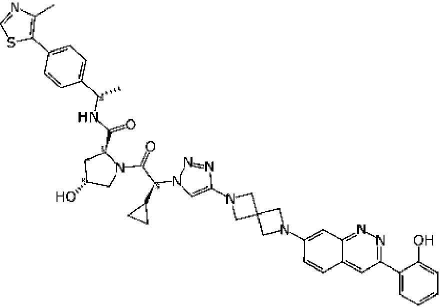 |
| 211 | 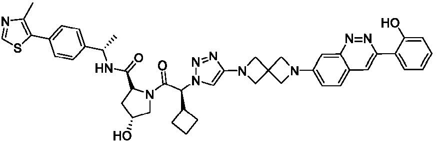 |
| 212 | 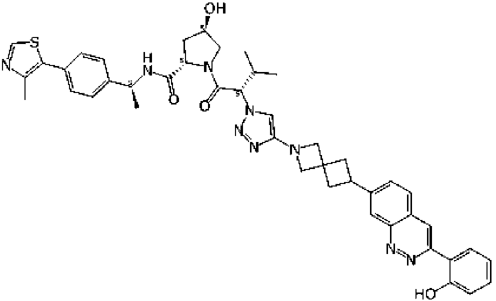 |
| 213 | 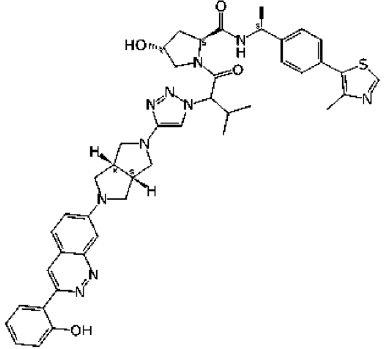 |
| 214 | 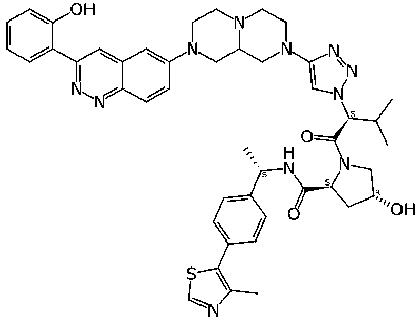 |
| 215 | 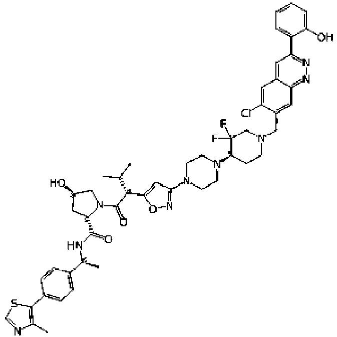 |
| 216 | 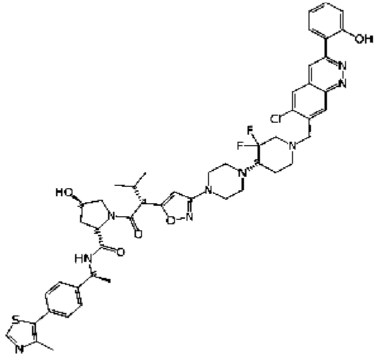 |
| 217 | 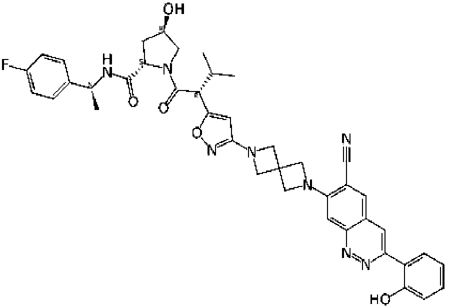 |
| 218 | 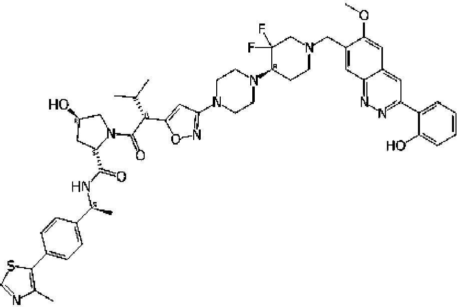 |
| 219 | 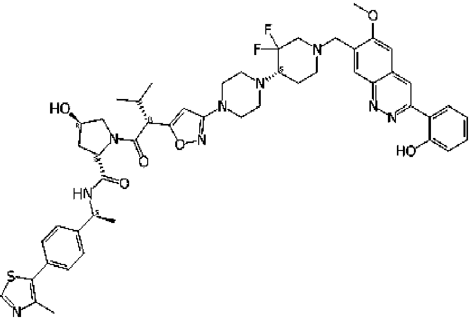 |
| 220 | 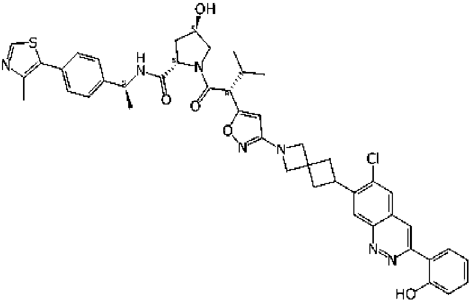 |
| 221 | 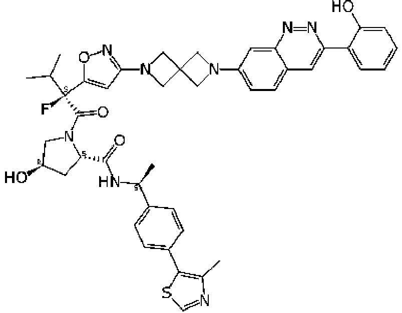 |
| 222 | 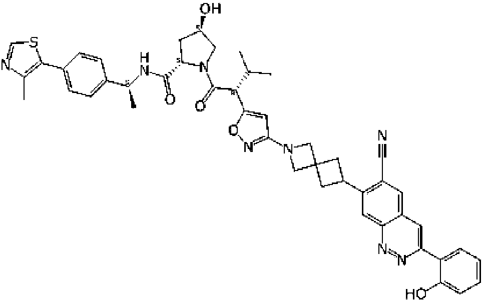 |
| 223 | 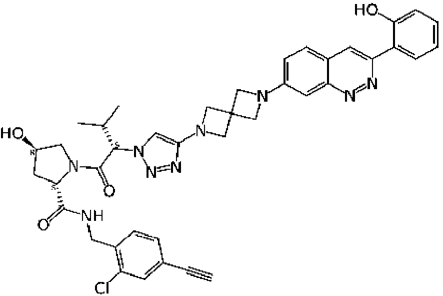 |
| 224 | 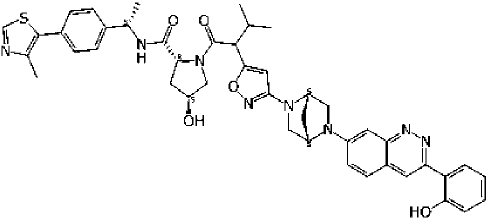 |
| 225 | 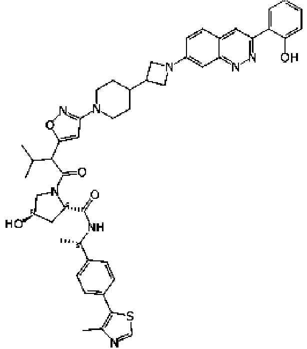 |
| 226 | 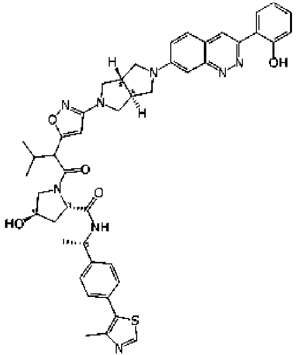 |
| 227 | 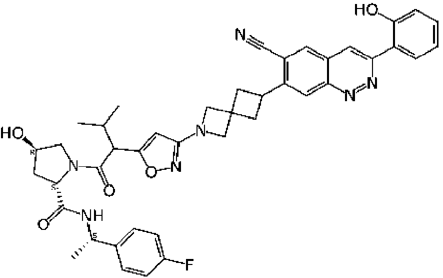 |
| 228 | 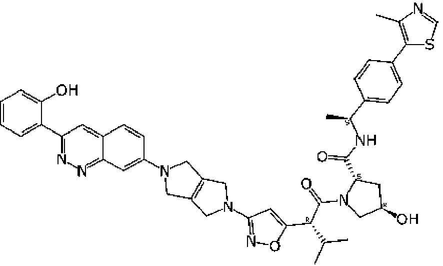 |
| 229 | 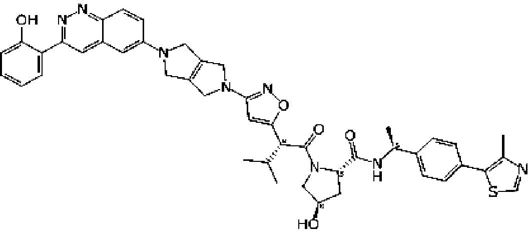 |
| 230 | 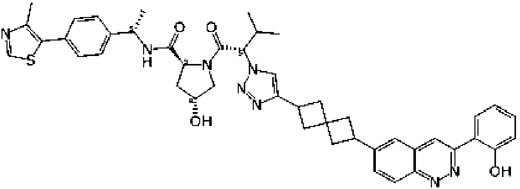 |
| 231 | 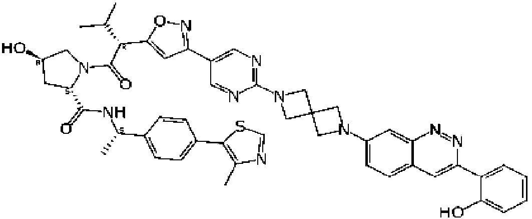 |
| 232 | 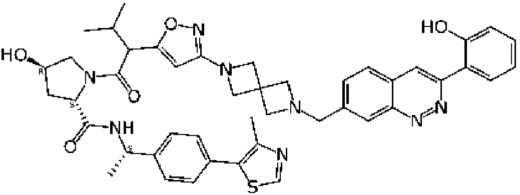 |
| 233 | 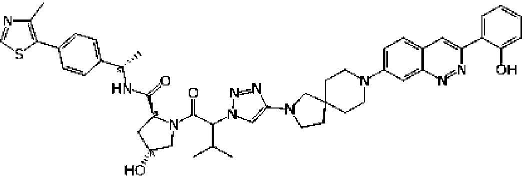 |
| 234 | 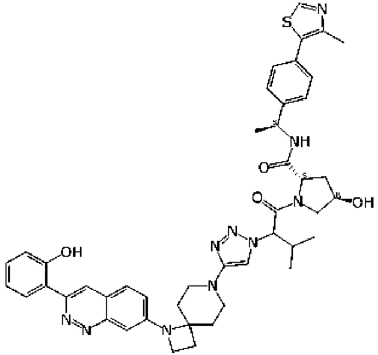 |
| 235 | 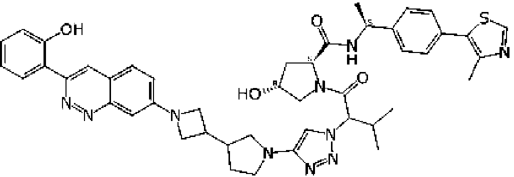 |
| 236 | 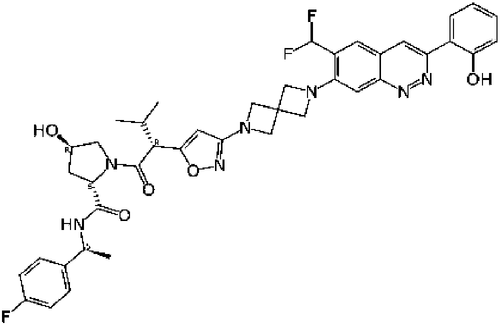 |
| 237 | 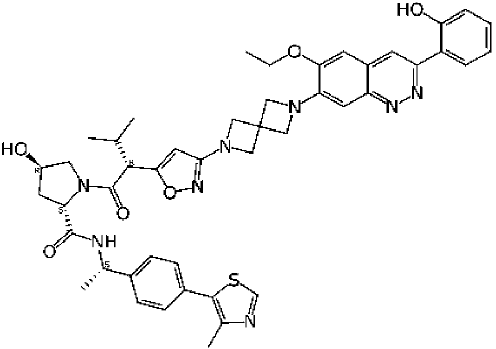 |
| 238 | 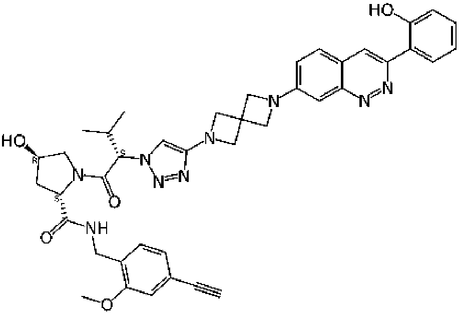 |
| 239 | 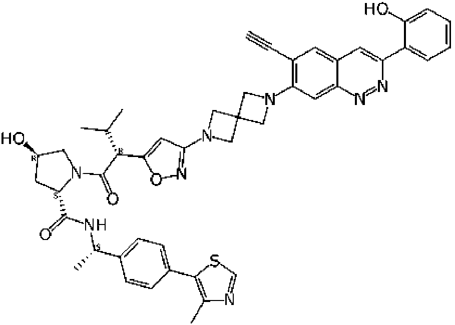 |
| 240 | 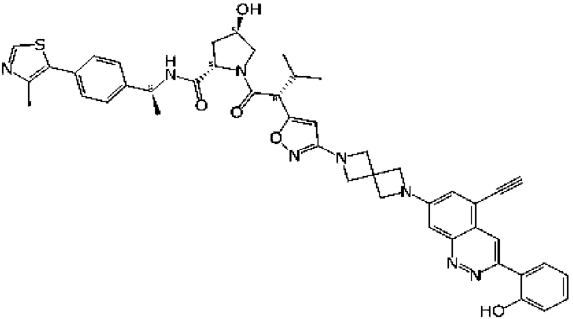 |
| 241 | 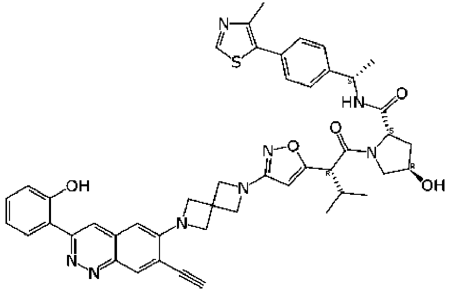 |
| 242 | 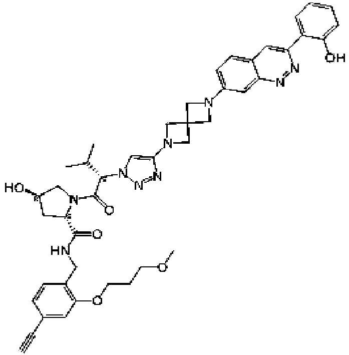 |
| 243 | 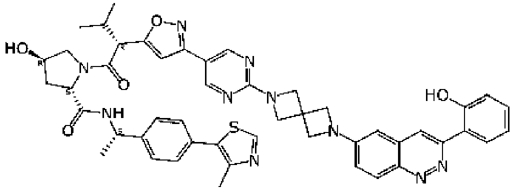 |
| 244 | 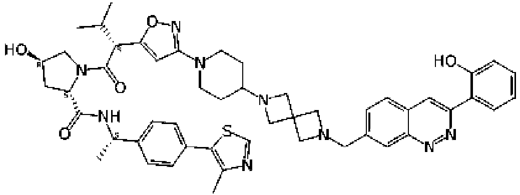 |
| 245 | 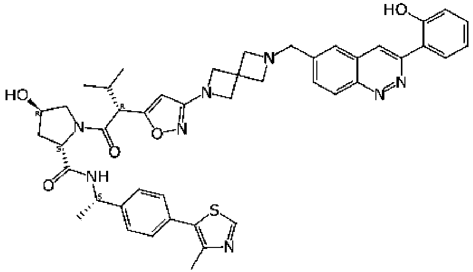 |
| 246 | 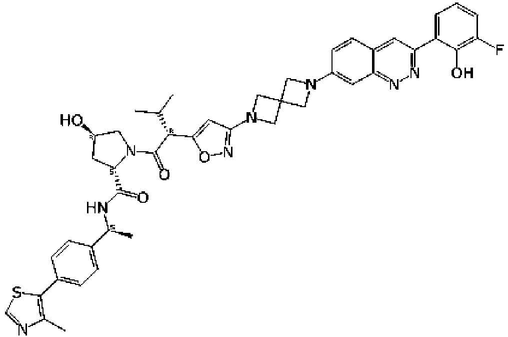 |
| 247 | 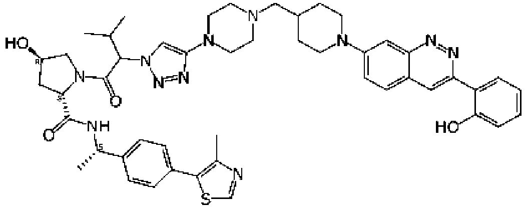 |
| 248 | 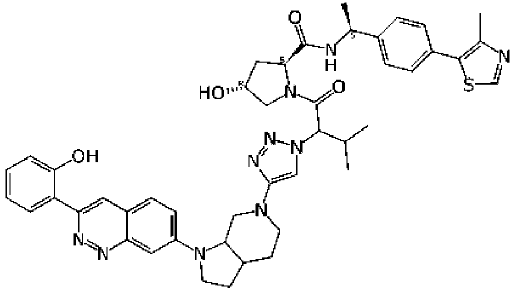 |
| 249 | 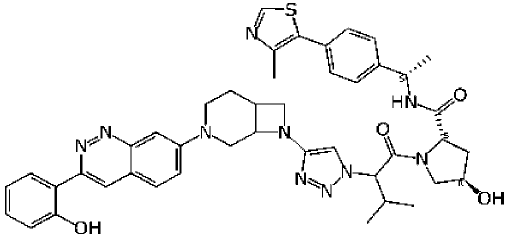 |
| 250 | 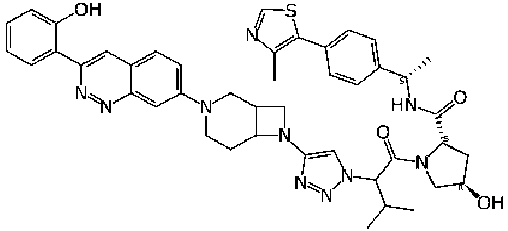 |
| 251 | 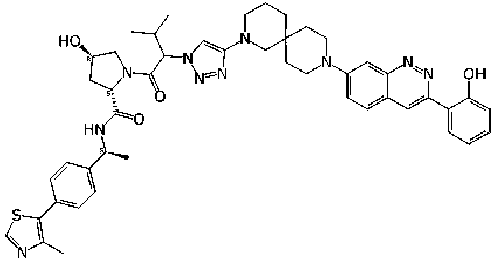 |
| 252 | 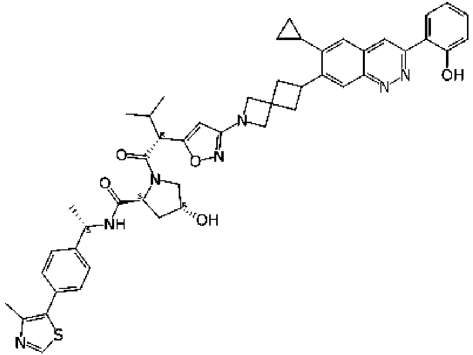 |
| 253 | 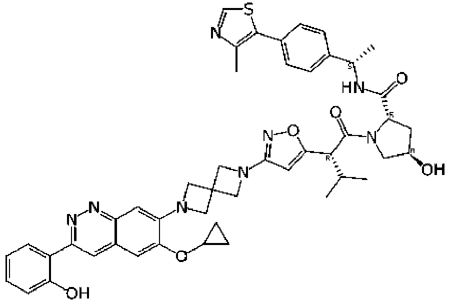 |
| 254 | 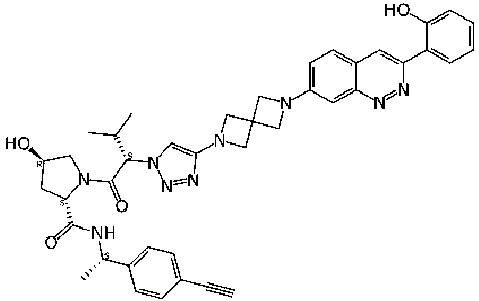 |
| 255 | 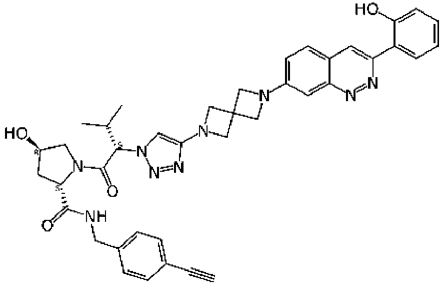 |
| 256 | 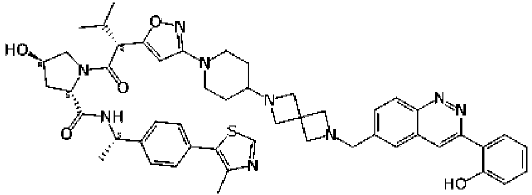 |
| 257 | 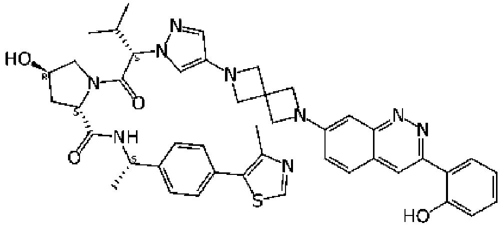 |
| 258 | 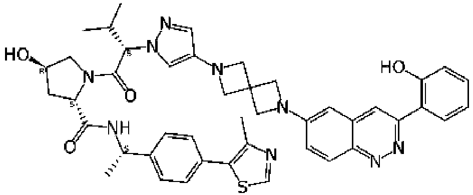 |
| 259 | 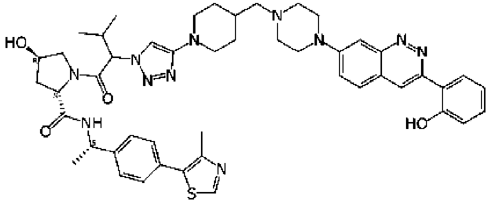 |
| 260 | 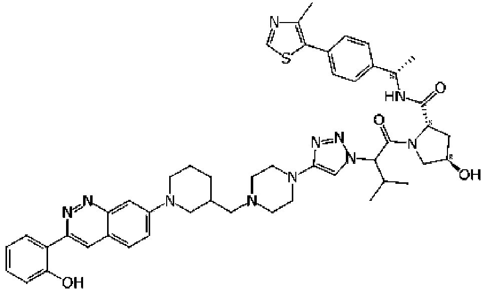 |
| 261 | 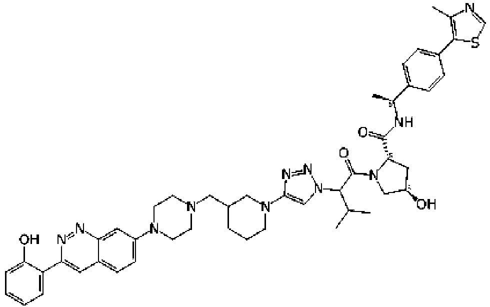 |
| 262 | 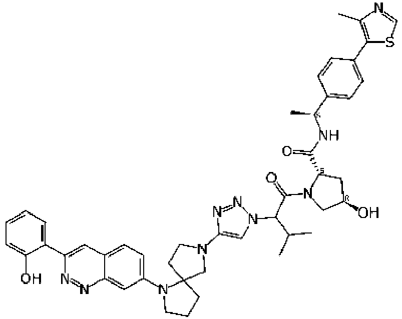 |
| 263 | 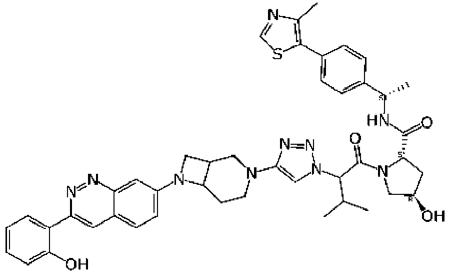 |
| 264 | 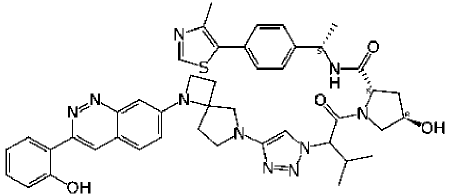 |
| 265 | 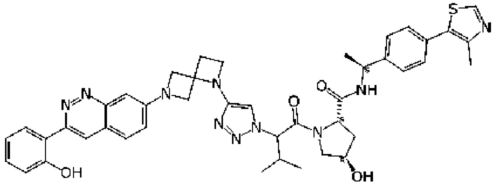 |
| 266 | 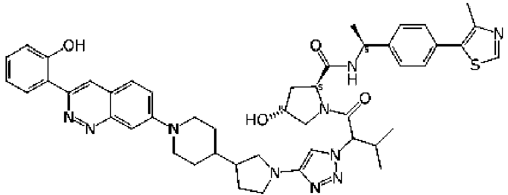 |
| 267 | 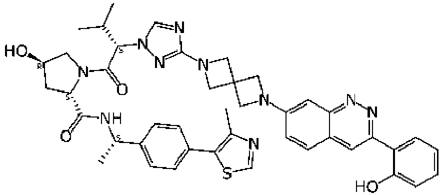 |
| 268 | 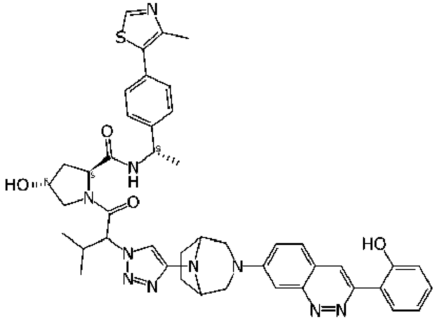 |
| 269 | 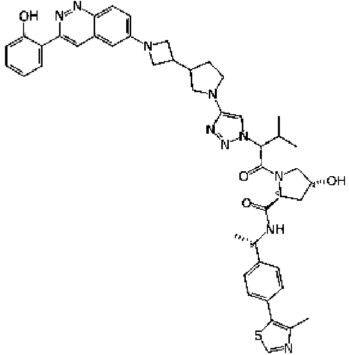 |
| 270 | 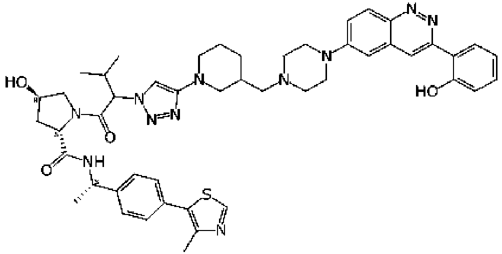 |
| 271 | 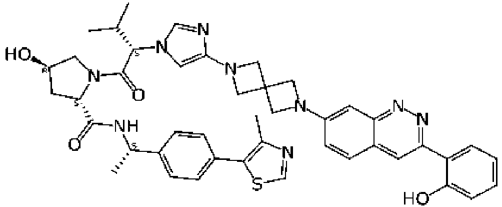 |
| 272 | 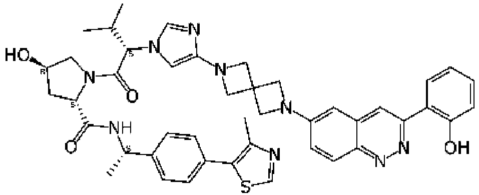 |
| 273 | 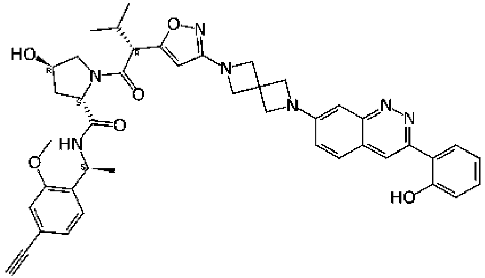 |
| 274 | 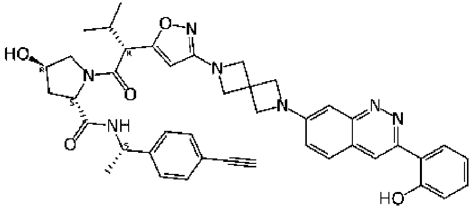 |
| 275 | 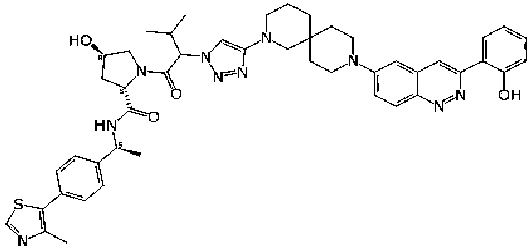 |
| 276 | 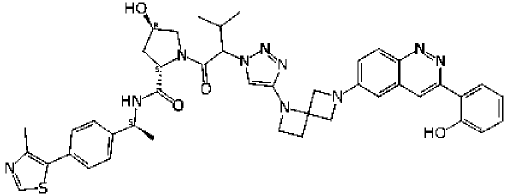 |
| 277 | 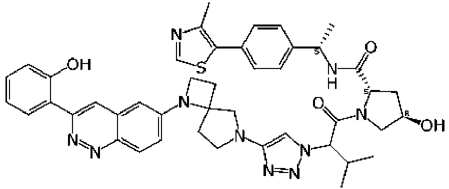 |
| 278 | 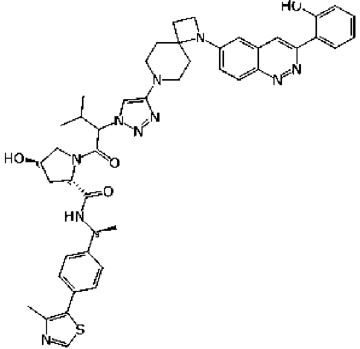 |
| 279 | 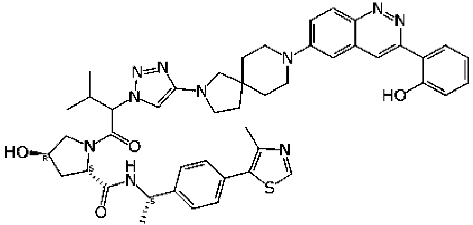 |
| 280 | 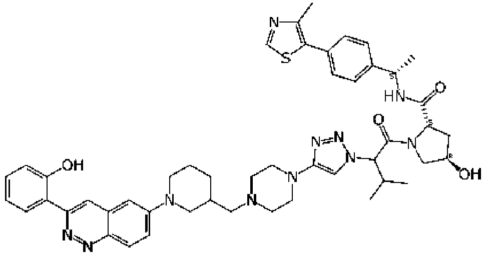 |
| 281 | 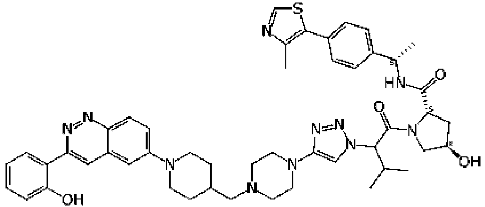 |
| 282 | 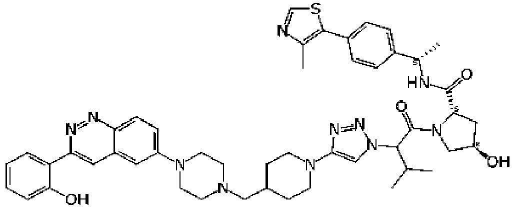 |
| 283 | 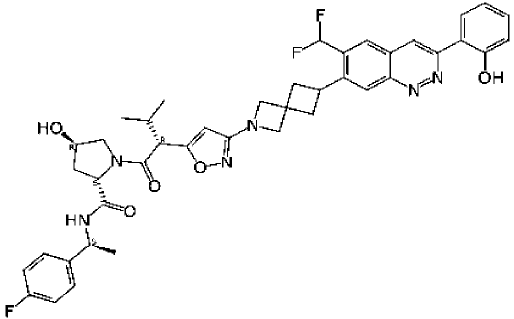 |
| 284 | 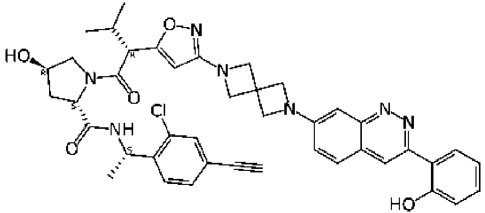 |
| 285 | 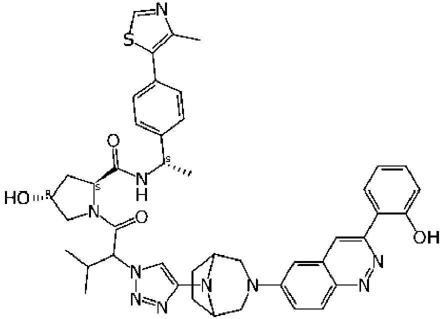 |
| 286 | 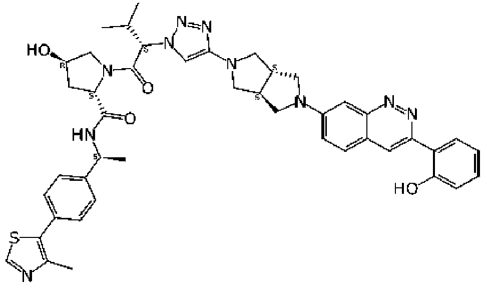 |
| 287 | 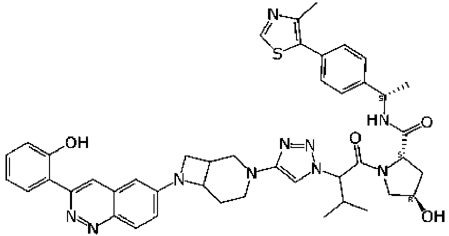 |
| 288 | 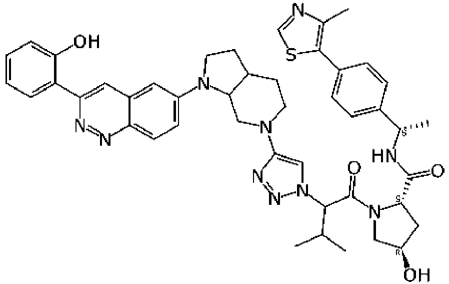 |
| 289 | 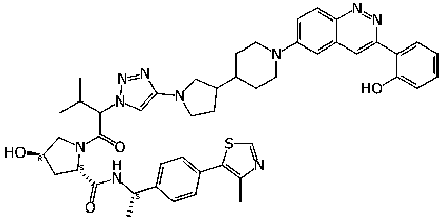 |
| 290 | 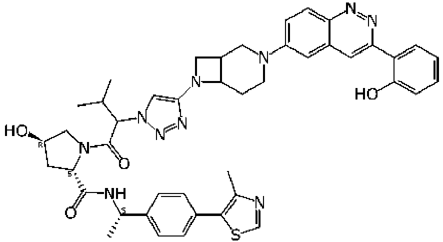 |
| 291 | 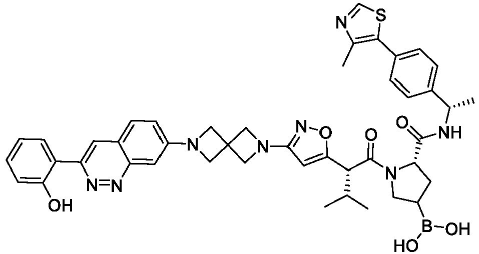 |

19. A pharmaceutical composition comprising a compound of any one of claims 1 to 18 and a pharmaceutically acceptable excipient.

20. A compound of any one of claims 1 to 18, or a pharmaceutically acceptable salt thereof, or a pharmaceutical composition of claim 19, for use in therapy.

21. A compound of any one of claims 1 to 18, or a pharmaceutically acceptable salt thereof, or a pharmaceutical composition of claim 19, for use in treating cancer.

22. The compound, or the pharmaceutically acceptable salt thereof, or the pharmaceutical composition for use according to claim 21, wherein the cancer is non-small cell lung cancer, colorectal cancer, bladder cancer, cancer of unknown primary, glioma, breast cancer, melanoma, non-melanoma skin cancer, endometrial cancer, esophagogastric cancer, pancreatic cancer, hepatobiliary cancer, soft tissue sarcoma, ovarian cancer, head and neck cancer, renal cell carcinoma, bone cancer, non-Hodgkin lymphoma, small-cell lung cancer, prostate cancer, embryonal tumor, germ cell tumor, cervical cancer, thyroid cancer, salivary gland cancer, gastrointestinal neuroendocrine tumor, uterine sarcoma, gastrointestinal stromal tumor, CNS cancer, thymic tumor, Adrenocortical carcinoma, appendiceal cancer, small bowel cancer, or penile cancer.

## Patentansprüche

1. Verbindung oder ein pharmazeutisch verträgliches Salz davon der Formel I: 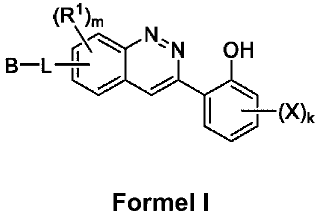 wobei
m 0, 1,2 oder 3 ist;
k 0, 1 oder 2 ist;
jedes R¹ unabhängig Halogen, gegebenenfalls substituiertes C₁-C₆-Alkyl, gegebenenfalls substituiertes C₁-C₆-Heteroalkyl, gegebenenfalls substituiertes C₂-C₉-Heterocyclyl, gegebenenfalls substituiertes C₃-C₈-Cycloalkyl, gegebenenfalls substituiertes C₃-C₈-Cycloalkoxy, gegebenenfalls substituiertes C₂-C₆-Alkinyl, gegebenenfalls substituiertes Amino oder Cyano ist;
jedes X unabhängig Halogen oder gegebenenfalls substituiertes C₁-C₆-Heteroalkyl ist;
L ein Linker der
**Formel II:**
A¹-(B¹)_{f}-(C¹)_{g}-(B²)ₕ-(D)-(B³)ᵢ-(C²)ⱼ-(B⁴)ₖ-A², **Formel II**
oder ein pharmazeutisch verträgliches Salz davon ist,
wobei
A¹ eine Bindung zwischen dem Linker und dem Ringsystem A ist;
A² eine Bindung zwischen dem Abbaurest und dem Linker ist;
jedes von B¹, B², B³ und B⁴ unabhängig voneinander gegebenenfalls substituiertes C₁-C₄-Alkyl, gegebenenfalls substituiertes C₆-C₁₀-Aryl, gegebenenfalls substituiertes C₆-C₁₀-Aryl-C₁₋₄-alkyl, gegebenenfalls substituiertes C₁-C₄-Heteroalkyl, gegebenenfalls substituiertes C₃-C₁₀-Cycloalkyl, gegebenenfalls substituiertes C₃-C₁₀-Carbocyclyl, gegebenenfalls substituiertes C₂-C₁₀-Heterocyclyl, gegebenenfalls substituiertes C₂-C₆-Heteroaryl, gegebenenfalls substituiertes C₆₋₁₂-Aryl, O, S, S(O)₂ oder NR^{N} ist;
jedes R^{N} unabhängig H, gegebenenfalls substituiertes C₁₋₄-Alkyl, gegebenenfalls substituiertes C₂₋₄-Alkenyl, gegebenenfalls substituiertes C₂₋₄-Alkinyl, gegebenenfalls substituiertes C₂₋₁₀-Heterocyclyl, gegebenenfalls substituiertes C₂₋₆-Heteroaryl oder gegebenenfalls substituiertes C₁₋₇-Heteroalkyl ist;
jedes von C¹ und C² unabhängig voneinander Carbonyl, Thiocarbonyl, Sulfonyl oder Phosphoryl ist;
jedes von f, g, h, i, j und k unabhängig voneinander 0 oder 1 ist; und
D gegebenenfalls substituiertes C₁₋₁₀-Alkyl, gegebenenfalls substituiertes C₂₋₁₀-Alkenyl, gegebenenfalls substituiertes C₂₋₁₀-Alkinyl, gegebenenfalls substituiertes C₂₋₁₀-Heterocyclyl, gegebenenfalls substituiertes C₂₋₆-Heteroaryl, gegebenenfalls substituiertes C₆₋₁₂-Aryl, gegebenenfalls substituiertes C₂-C₁₀-Polyethylenglykol, gegebenenfalls substituiertes C₃-C₁₀-Cycloalkyl, gegebenenfalls substituiertes C₃-C₁₀-Carbocyclyl oder gegebenenfalls substituiertes C₁₋₁₀-Heteroalkyl ist; oder D fehlt, und der Linker A¹-(B¹)_{f}-(C¹)_{g}-(B²)ₕ-(B³)ᵢ-(C²)ⱼ-(B⁴)ₖ-A² ist, und
B ein Abbaurest der **Formel C** ist: 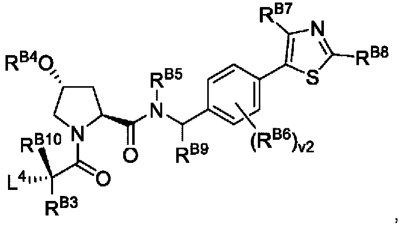 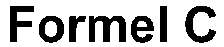
wobei
L⁴ -N(R^{B1})(R^{B2}) ist, 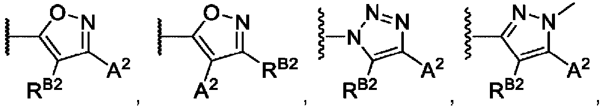 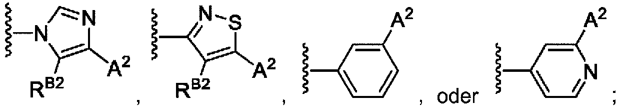
R^{B1} H, A², gegebenenfalls substituiertes C₁-C₆-Alkyl oder gegebenenfalls substituiertes C₁-C₆-Heteroalkyl ist;
R^{B2} H, gegebenenfalls substituiertes C₁-C₆-Alkyl oder gegebenenfalls substituiertes C₁-C₆-Heteroalkyl ist;
R^{B3} A², gegebenenfalls substituiertes C₁-C₆-Alkyl, gegebenenfalls substituiertes C₁-C₆-Heteroalkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B4} H, gegebenenfalls substituiertes C₁-C₆-Alkyl, gegebenenfalls substituiertes C₃-C₁₀-Carbocyclyl, gegebenenfalls substituiertes C₆-C₁₀-Aryl, gegebenenfalls substituiertes C₁-C₆-Alkyl-C₃-C₁₀-carbocyclyl oder gegebenenfalls substituiertes C₁-C₆-Alkyl-C₆-C₁₀-aryl ist;
R^{B5} H, gegebenenfalls substituiertes C₁-C₆-Alkyl oder gegebenenfalls substituiertes C₁-C₆-Heteroalkyl ist;
v2 0, 1, 2, 3 oder 4 ist;
jedes R^{B6} unabhängig A², Halogen, gegebenenfalls substituiertes C₁-C₆-Alkyl, gegebenenfalls substituiertes C₂-C₆-Alkinyl, gegebenenfalls substituiertes C₁-C₆-Heteroalkyl, gegebenenfalls substituiertes C₃-C₁₀-Carbocyclyl, gegebenenfalls substituiertes C₂-C₉-Heterocyclyl, gegebenenfalls substituiertes C₆-C₁₀-Aryl, gegebenenfalls substituiertes C₂-C₉-Heteroaryl, gegebenenfalls substituiertes C₂-C₆-Alkenyl, gegebenenfalls substituiertes C₂-C₆-Heteroalkenyl, Hydroxy, Thiol, Cyano oder gegebenenfalls substituiertes Amino ist;
R^{B7} und R^{B8} jeweils unabhängig voneinander H, Halogen, gegebenenfalls substituiertes C₁-C₆-Alkyl oder gegebenenfalls substituiertes C₆-C₁₀-Aryl sind;
R^{B9} H oder gegebenenfalls substituiertes C₁-C₆-Alkyl ist;
R^{B10} H oder F ist; und
A² eine Bindung zwischen dem Abbaurest und dem Linker ist;
wobei genau eines von R^{B1}, R^{B3} und R^{B6} A² ist,
oder ein pharmazeutisch verträgliches Salz davon.

2. Verbindung nach Anspruch 1 oder ein pharmazeutisch verträgliches Salz davon, wobei
jedes R¹ unabhängig Halogen, gegebenenfalls substituiertes C₁-C₆-Alkyl, gegebenenfalls substituiertes C₁-C₆-Heteroalkyl, gegebenenfalls substituiertes C₂-C₉-Heterocyclyl oder gegebenenfalls substituiertes C₃-C₈-Cycloalkyl ist; und
jedes X unabhängig Halogen ist.

3. Verbindung nach Anspruch 1 oder 2 oder ein pharmazeutisch verträgliches Salz davon, wobei die Verbindung die Struktur von **Formel I-A** aufweist:

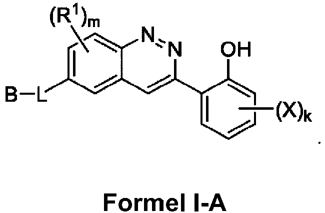

4. Verbindung nach Anspruch 1 oder 2 oder ein pharmazeutisch verträgliches Salz davon, wobei die Verbindung die Struktur von **Formel I-B** aufweist:

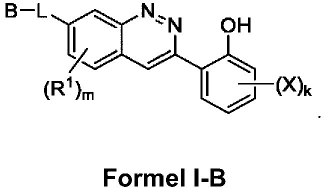

5. Verbindung nach einem der Ansprüche 1 bis 4 oder ein pharmazeutisch verträgliches Salz davon, wobei m 1 ist.

6. Verbindung nach einem der Ansprüche 1 bis 5 oder ein pharmazeutisch verträgliches Salz davon, wobei R¹ gegebenenfalls substituiertes C₁-C₆-Alkoxy oder Halogen ist.

7. Verbindung nach einem der Ansprüche 1-6 oder ein pharmazeutisch verträgliches Salz davon, wobei der Abbaurest die Struktur von **Formel Ca2, Formel Cb2, Formel Cc2, Formel Cd2, Formel Ce2** oder Formel Cf2 aufweist:

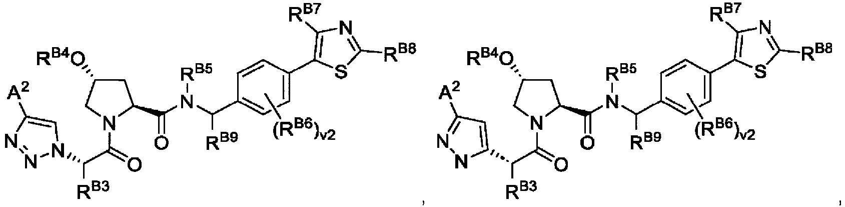

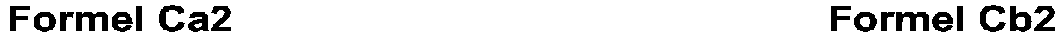

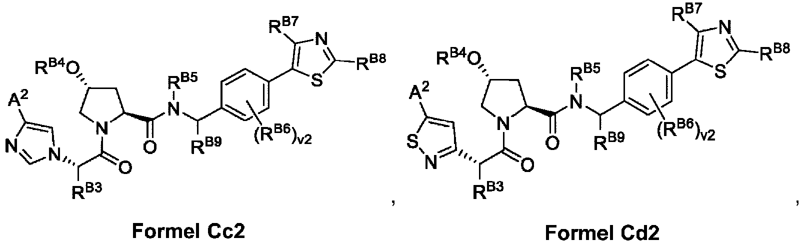

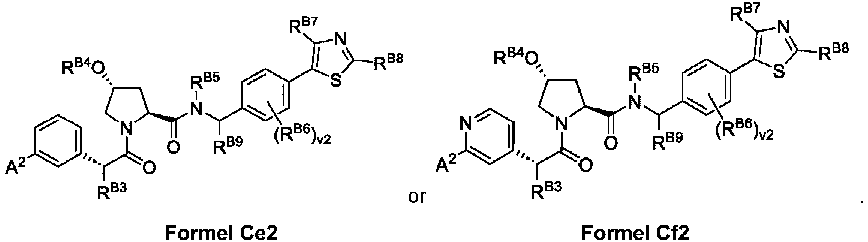

8. Verbindung nach einem der Ansprüche 1-7 oder ein pharmazeutisch verträgliches Salz davon, wobei R^{B9} Methyl ist.

9. Verbindung nach einem der Ansprüche 1-8 oder ein pharmazeutisch verträgliches Salz davon, wobei R^{B4} H ist.

10. Verbindung nach einem der Ansprüche 1-9 oder ein pharmazeutisch verträgliches Salz davon, wobei R^{B5} H ist.

11. Verbindung nach einem der Ansprüche 1-10 oder ein pharmazeutisch verträgliches Salz davon, wobei R^{B7} Methyl ist.

12. Verbindung nach einem der Ansprüche 1-11 oder ein pharmazeutisch verträgliches Salz davon, wobei R^{B3} gegebenenfalls substituiertes C₁-C₆-Alkyl ist.

13. Verbindung nach einem der Ansprüche 1-6 oder ein pharmazeutisch verträgliches Salz davon, wobei der Abbaurest Folgender ist

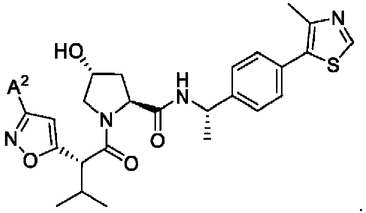

14. Verbindung nach einem der Ansprüche 1-6 oder ein pharmazeutisch verträgliches Salz davon, wobei der Abbaurest Folgender ist

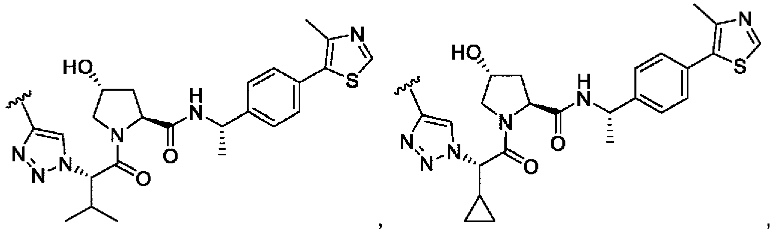

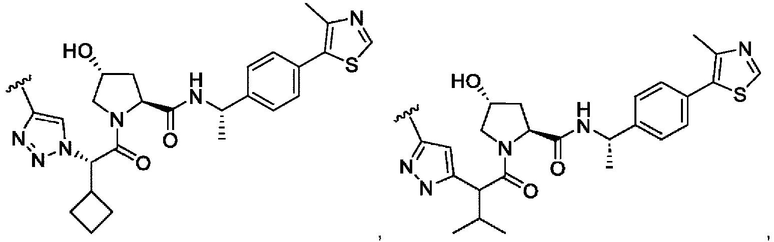

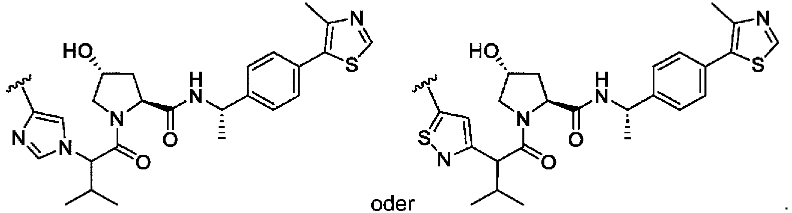

15. Verbindung nach einem der Ansprüche 1-14 oder ein pharmazeutisch verträgliches Salz davon, wobei der Linker die folgende Struktur aufweist

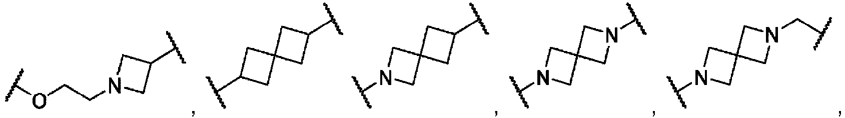

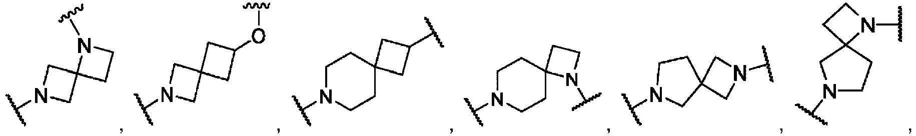

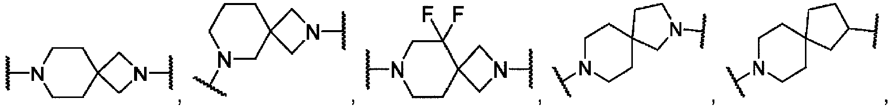

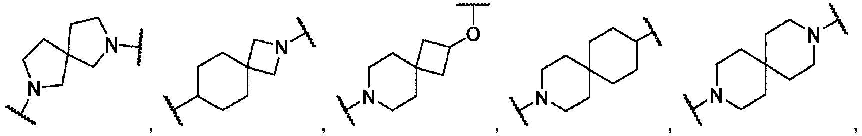

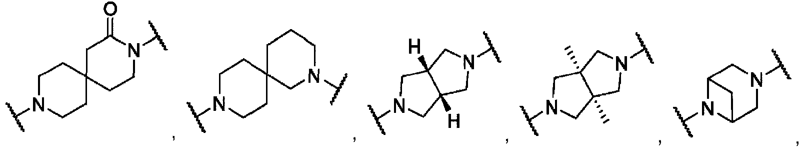

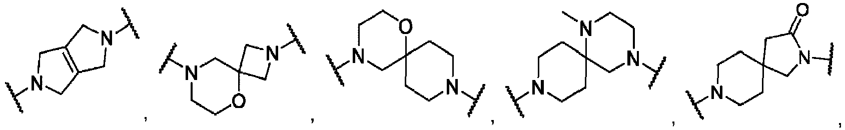

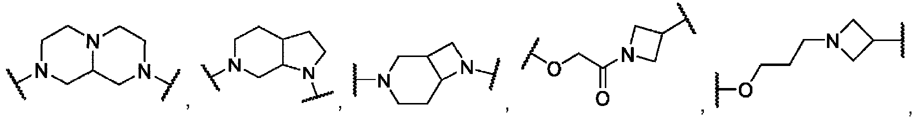

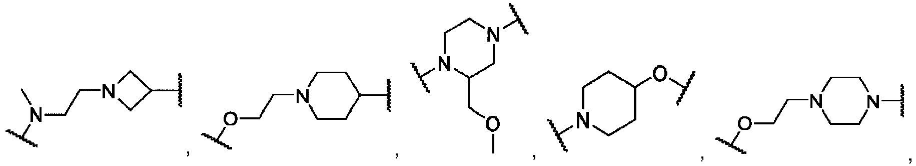

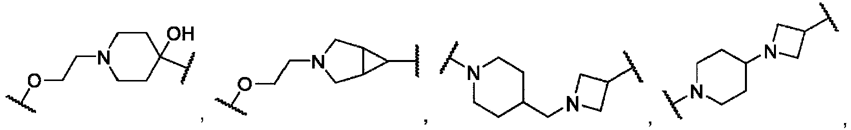

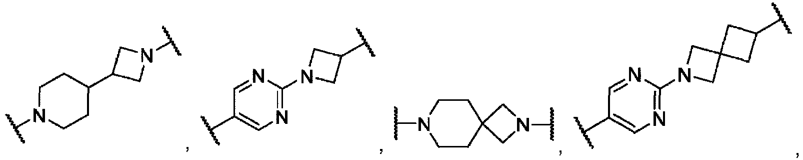

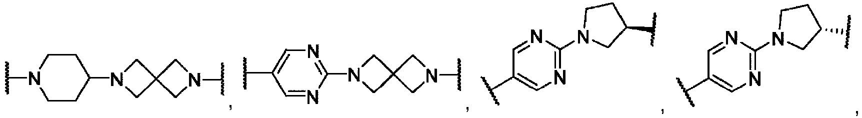

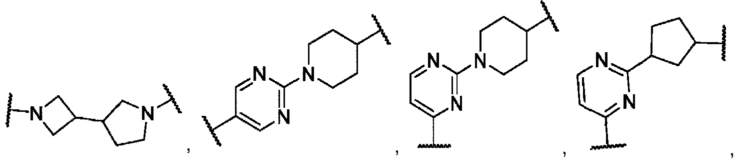

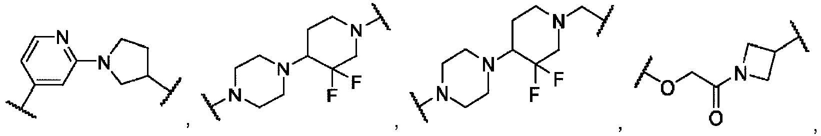

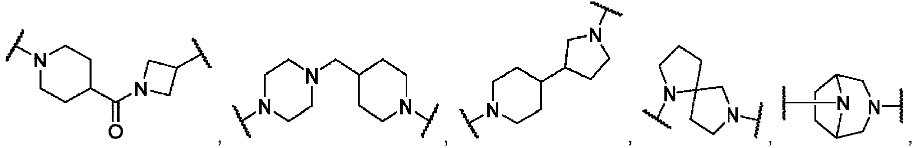

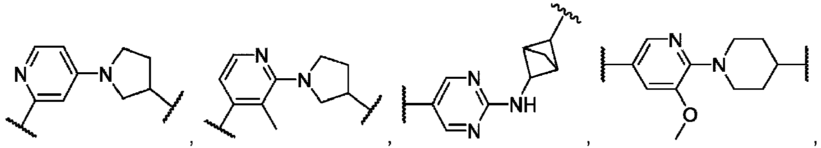

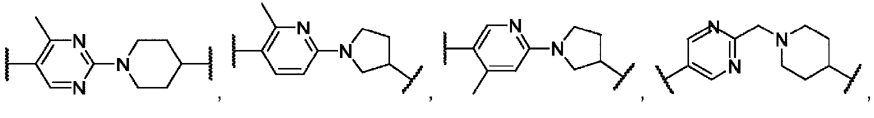

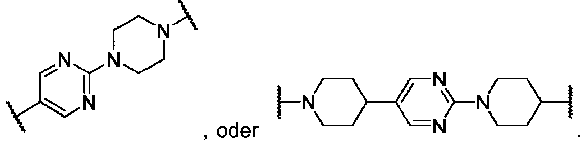

16. Verbindung nach einem der Ansprüche 1-14 oder ein pharmazeutisch verträgliches Salz davon, wobei der Linker die folgende Struktur aufweist

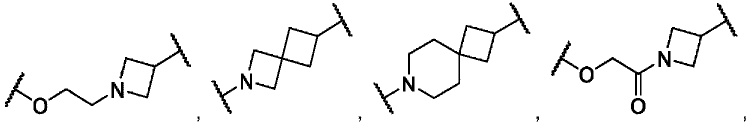

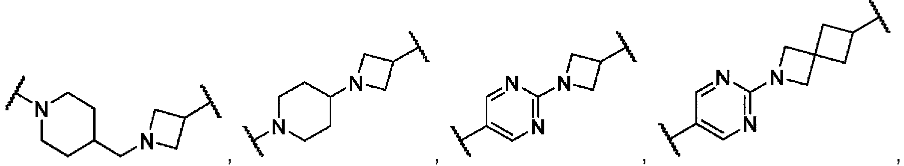

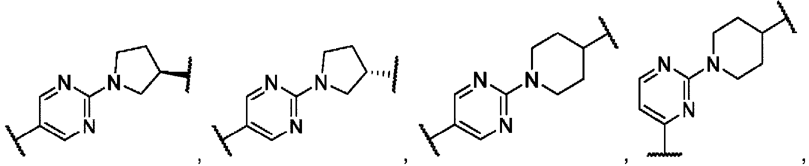

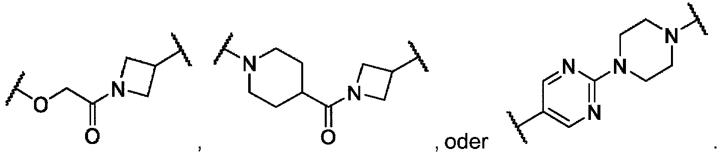

17. Verbindung nach einem der Ansprüche 1-14 oder ein pharmazeutisch verträgliches Salz davon, wobei der Linker die folgende Struktur aufweist

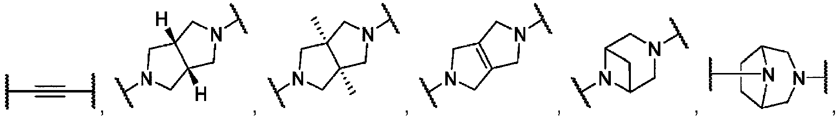

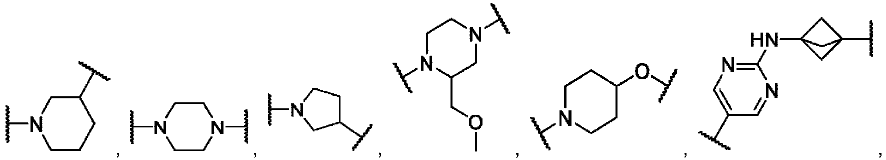

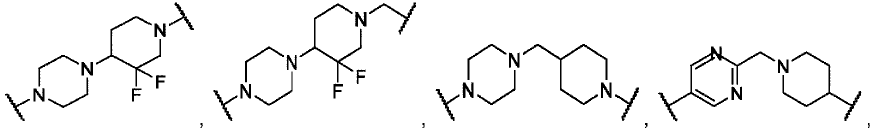

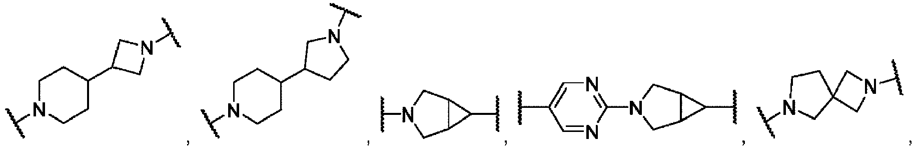

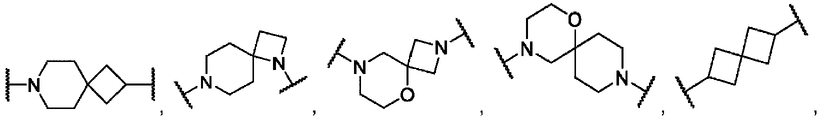

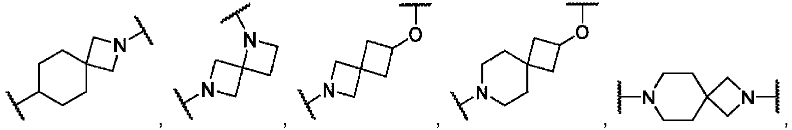

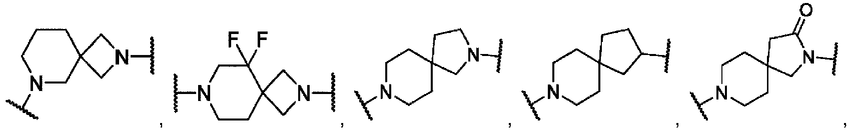

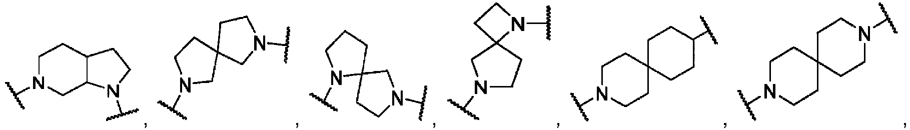

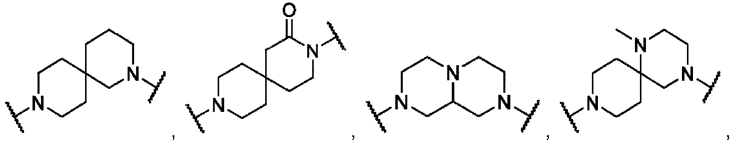

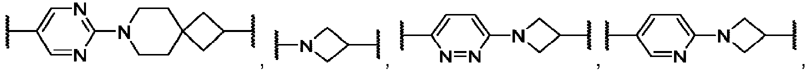

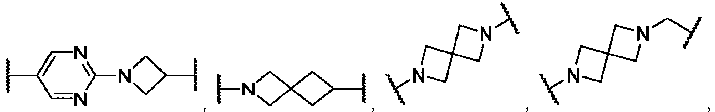

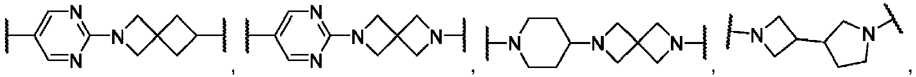

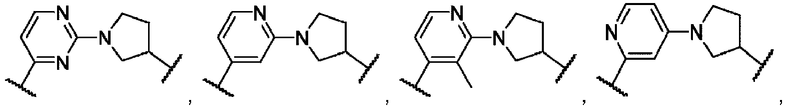

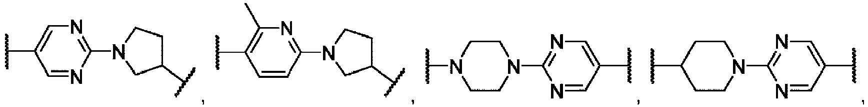

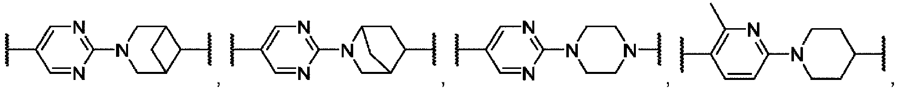

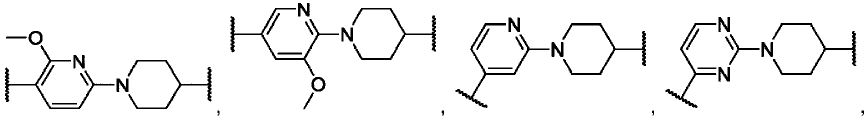

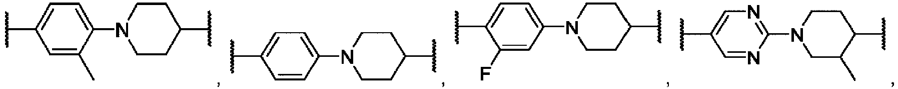

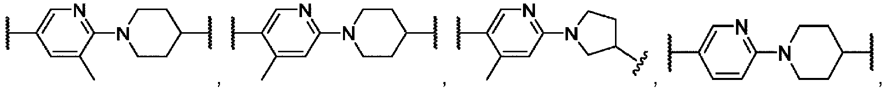

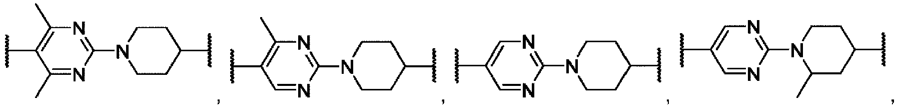

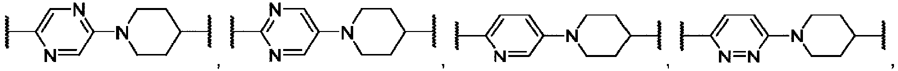

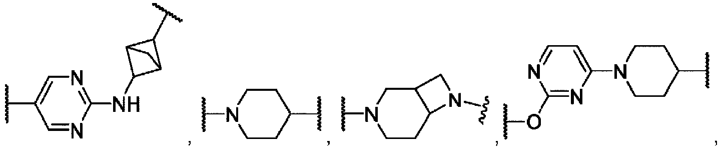

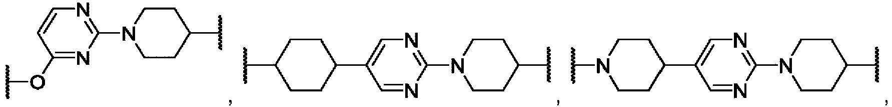

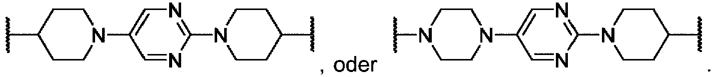

18. Verbindung, ausgewählt aus der Gruppe, die aus den Verbindungen 1-291 in der nachstehenden Tabelle und pharmazeutisch verträglichen Salzen davon besteht:
| **#** | **Verbindung** |
|---|---|
| 1 | 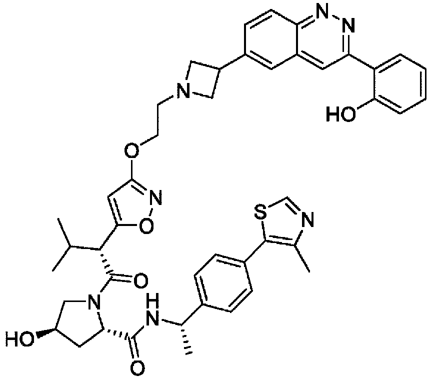 |
| 2 | 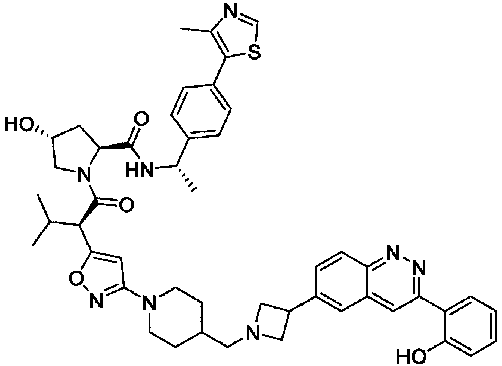 |
| 3 | 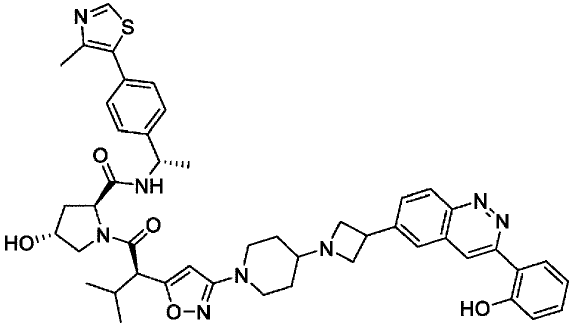 |
| 4 | 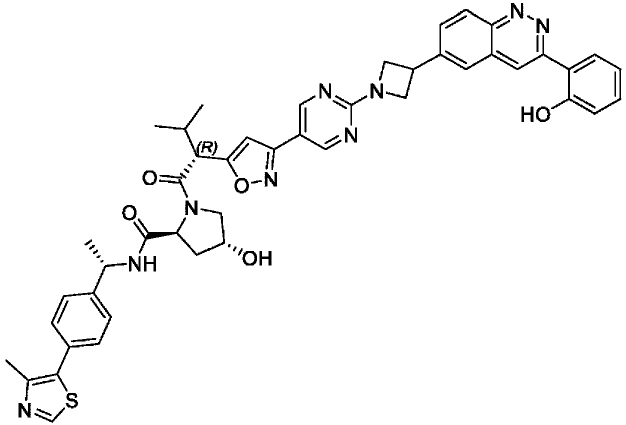 |
| 5 | 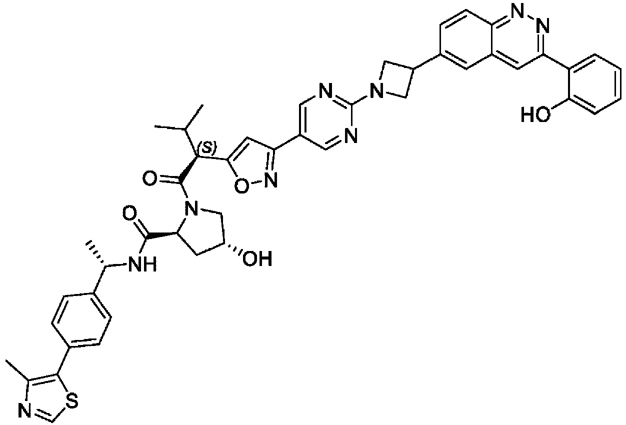 |
| 6 | 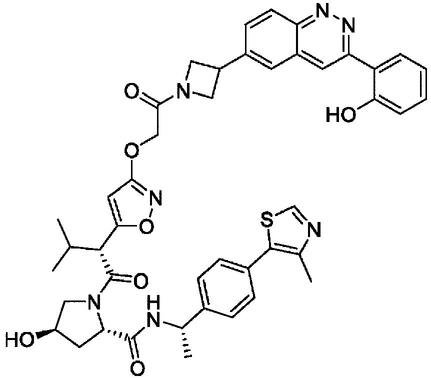 |
| 7 | 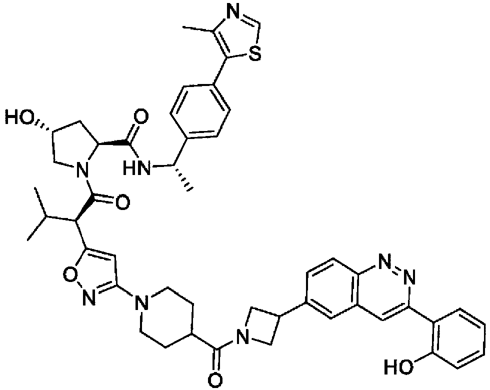 |
| 8 | 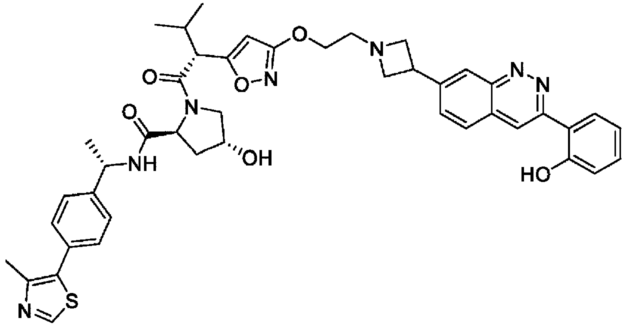 |
| 9 | 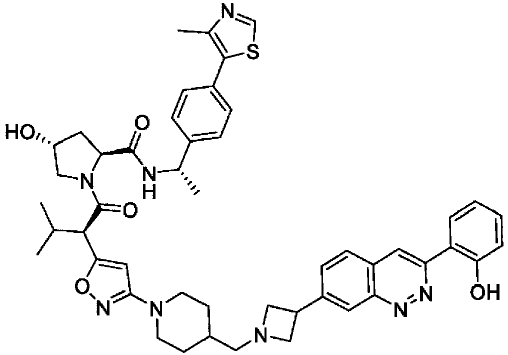 |
| 10 | 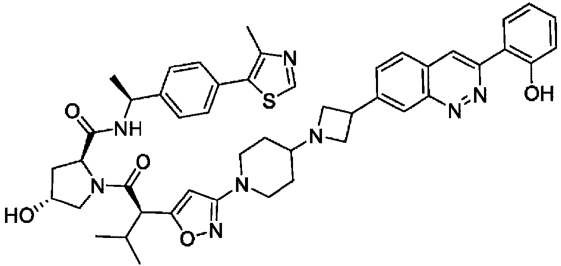 |
| 11 | 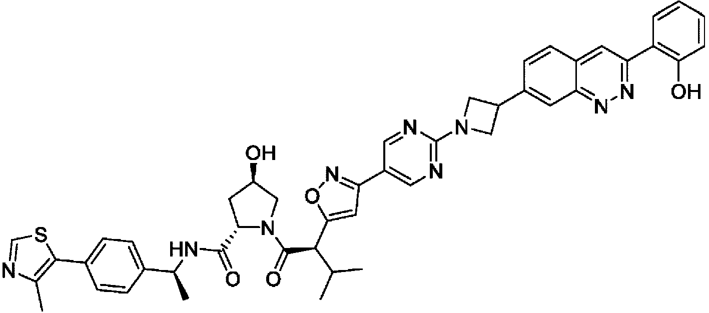 |
| 12 | 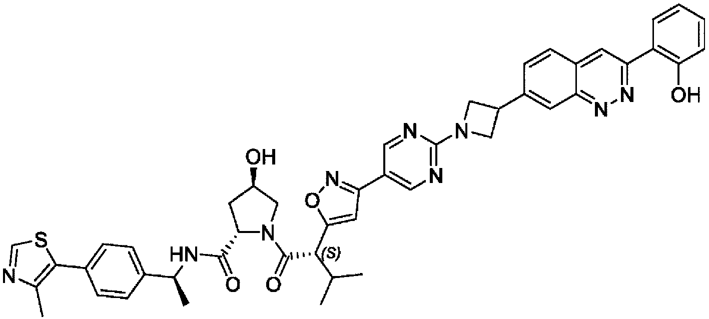 |
| 13 | 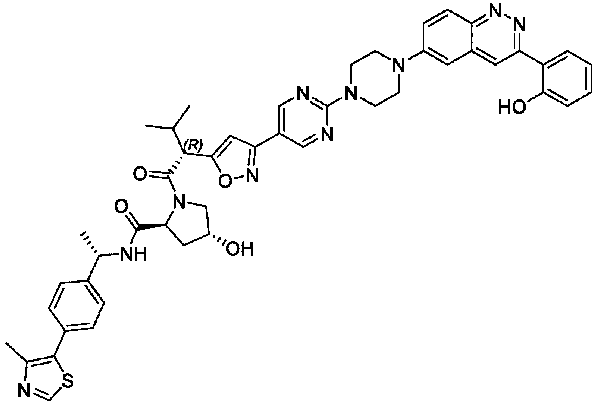 |
| 14 | 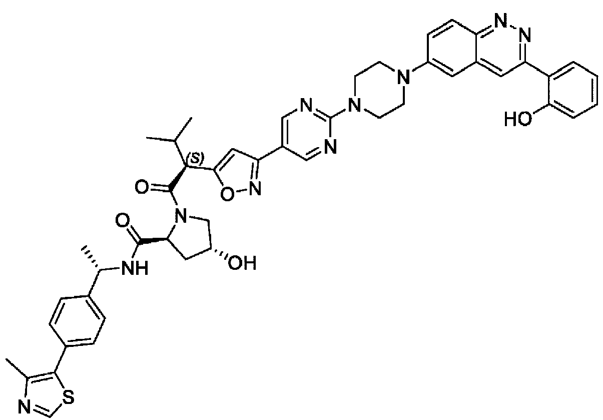 |
| 15 | 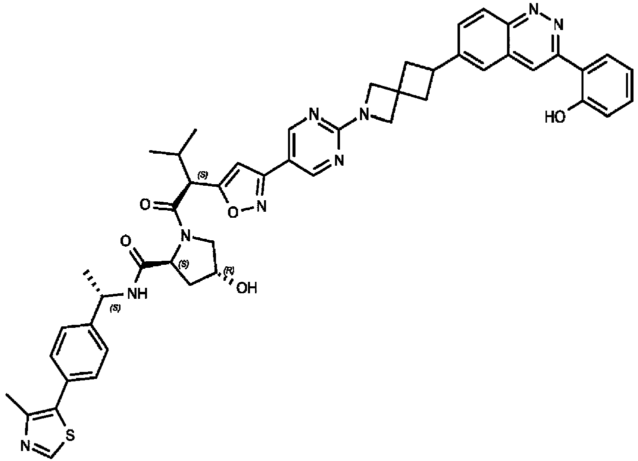 |
| 16 | 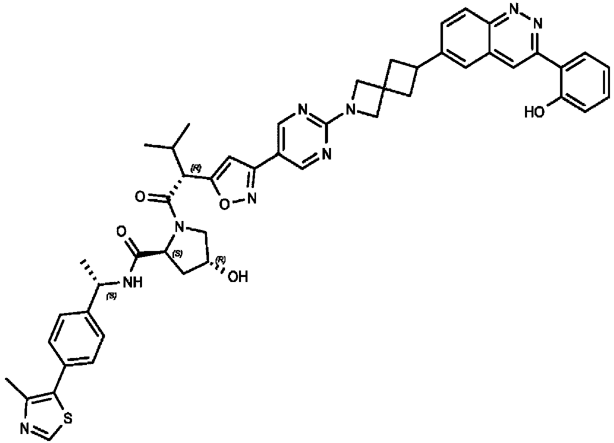 |
| 17 | 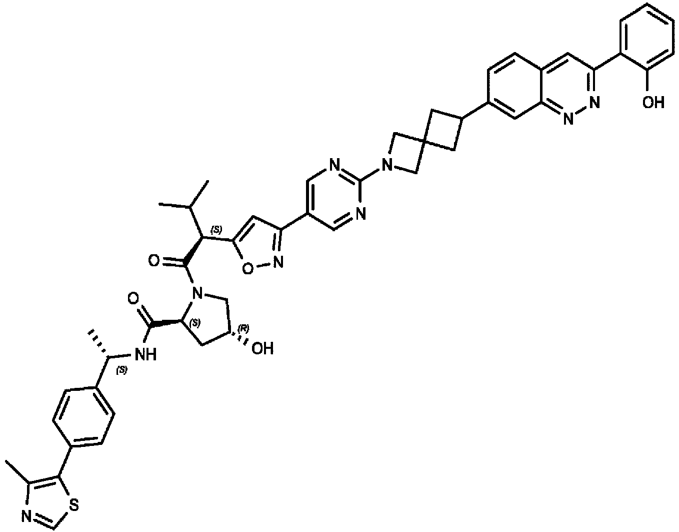 |
| 18 | 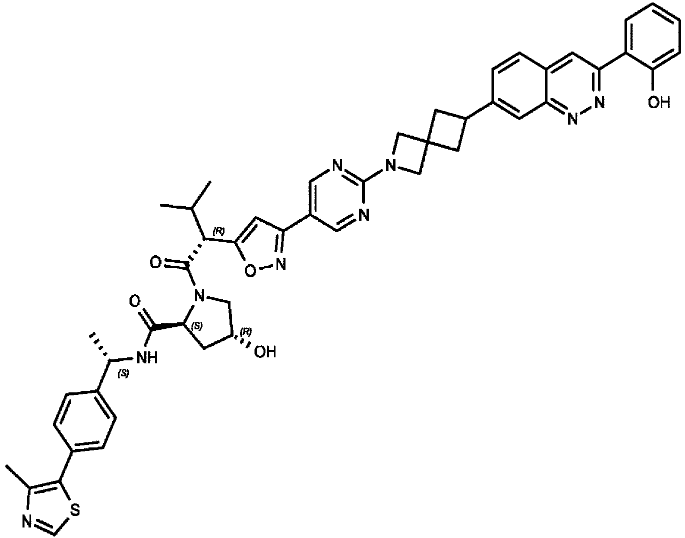 |
| 19 | 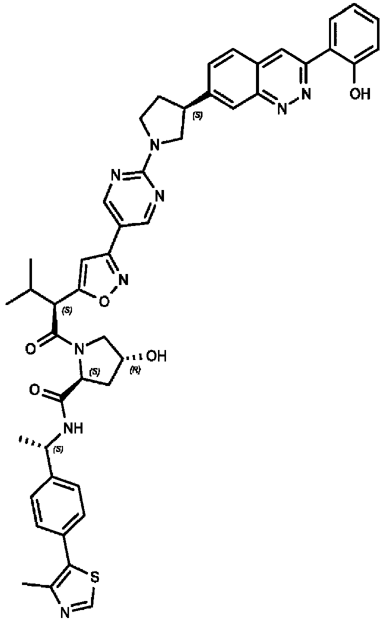 |
| 20 | 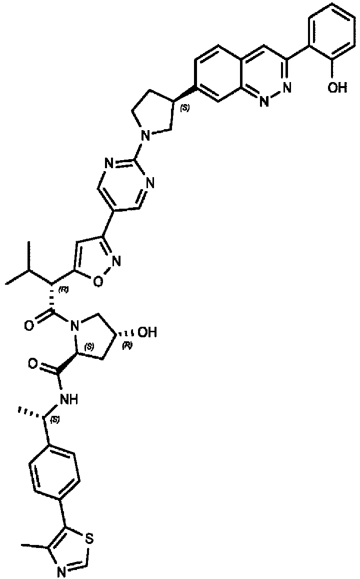 |
| 21 | 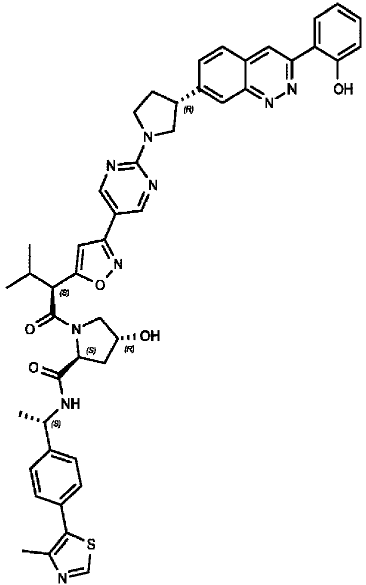 |
| 22 | 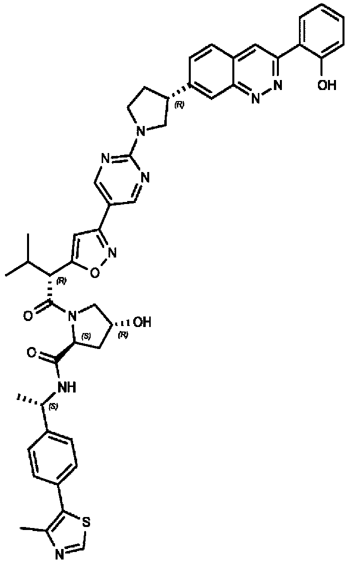 |
| 23 | 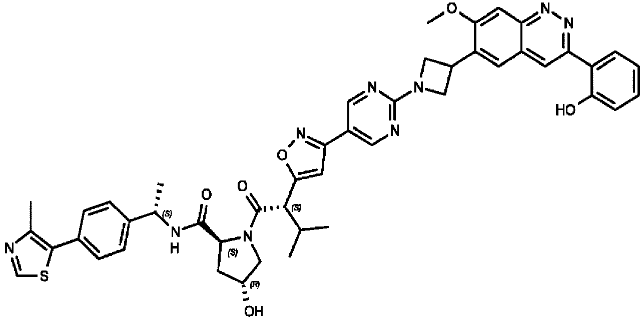 |
| 24 | 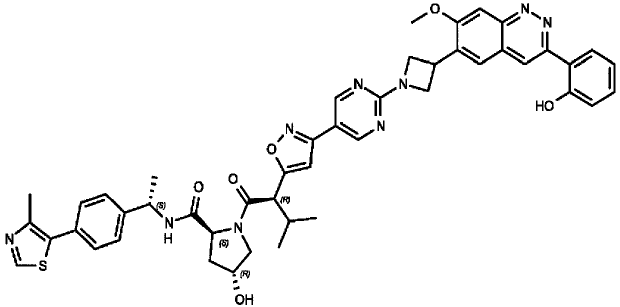 |
| 25 | 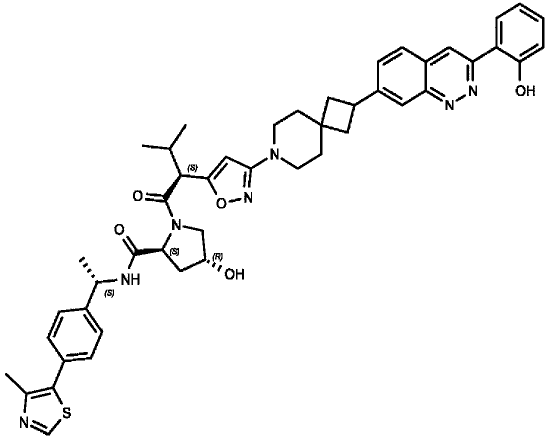 |
| 26 | 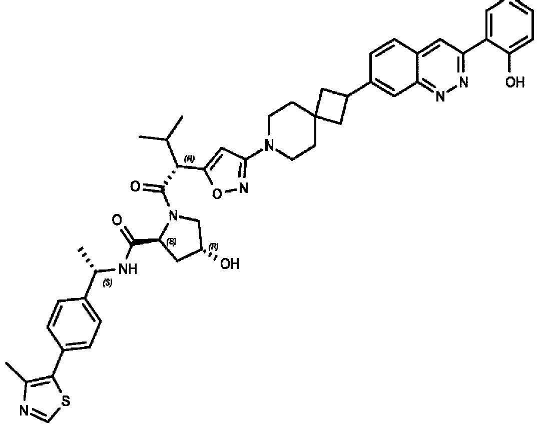 |
| 27 | 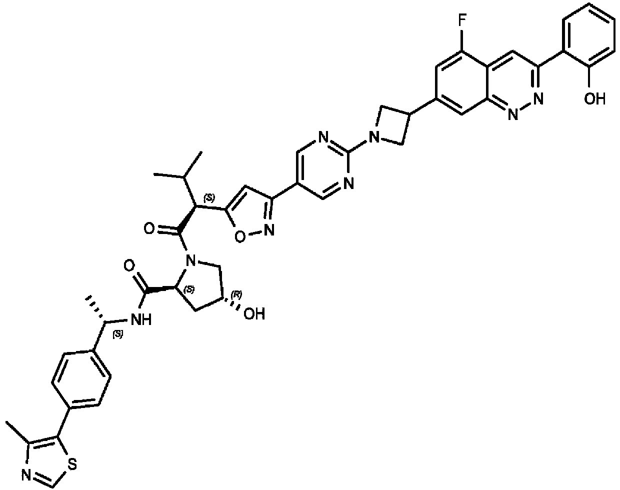 |
| 28 | 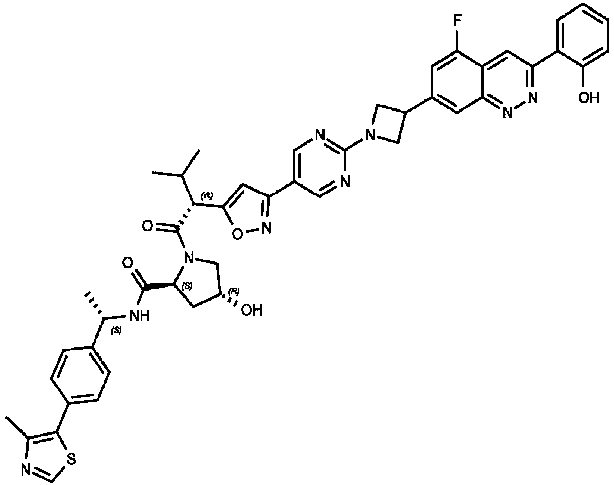 |
| 29 | 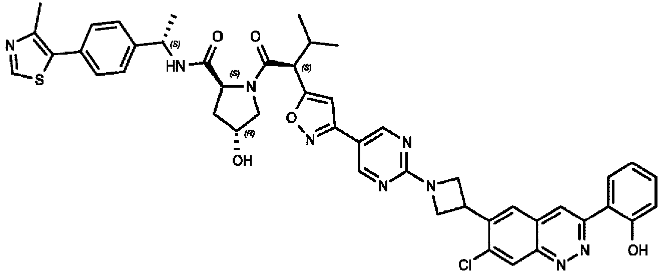 |
| 30 | 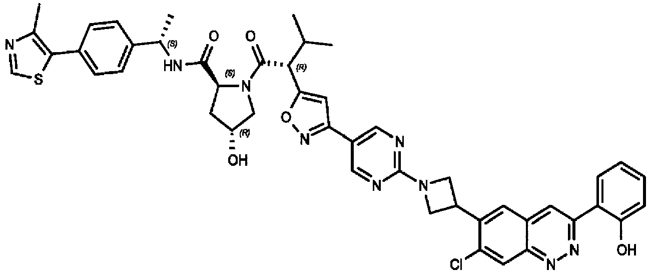 |
| 31 | 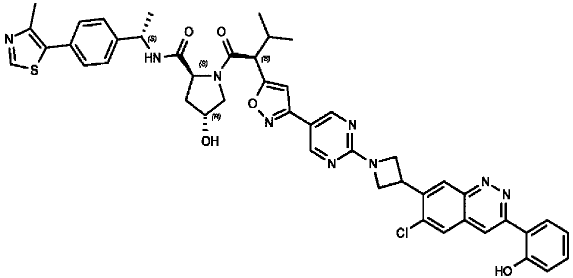 |
| 32 | 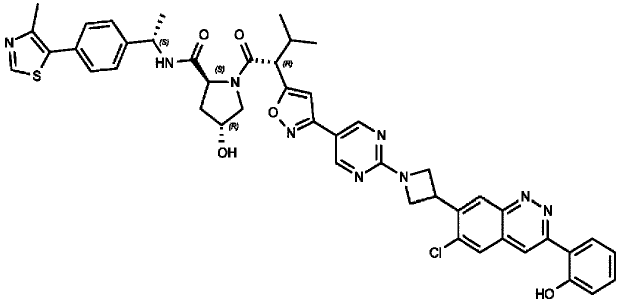 |
| 33 | 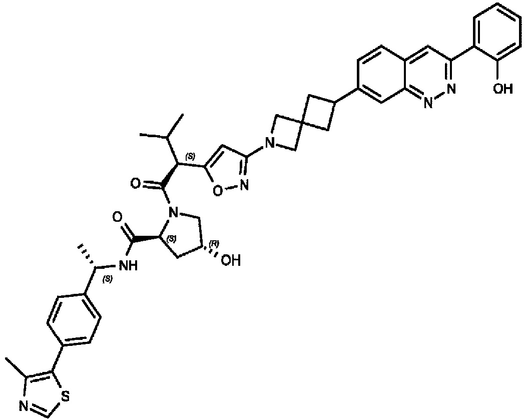 |
| 34 | 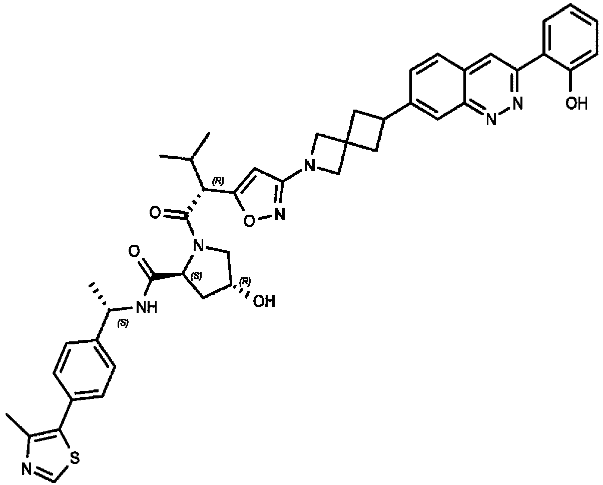 |
| 35 | 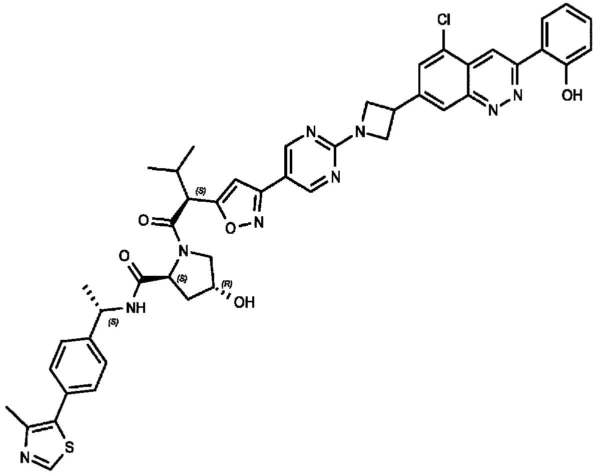 |
| 36 | 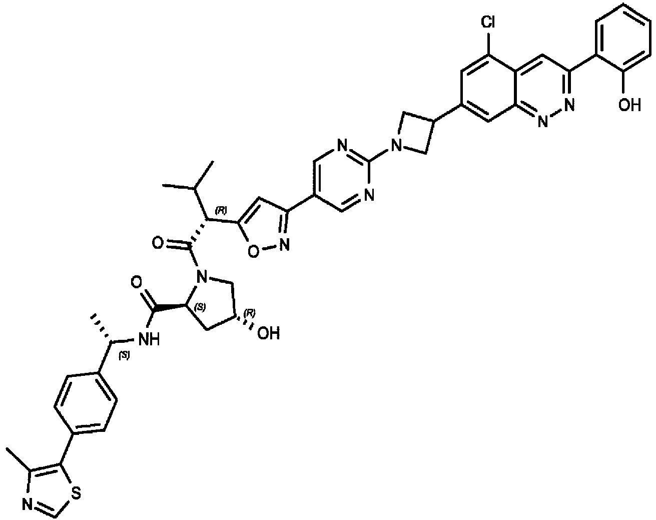 |
| 37 | 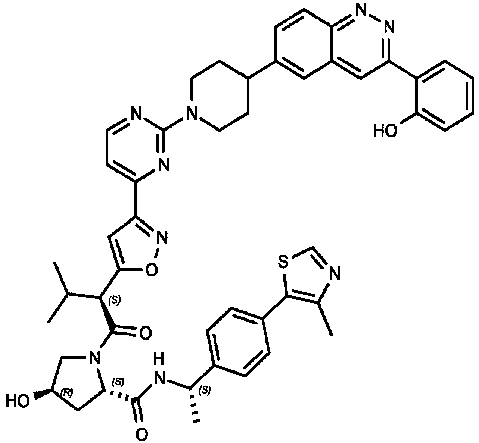 |
| 38 | 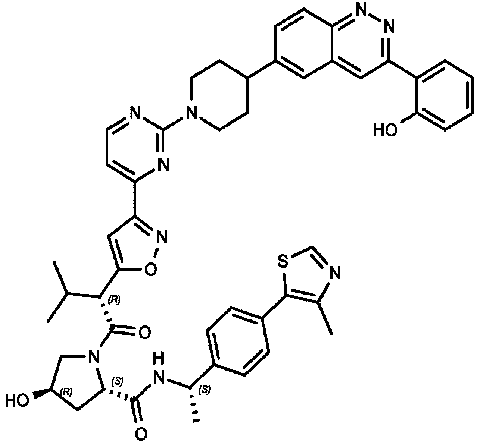 |
| 39 | 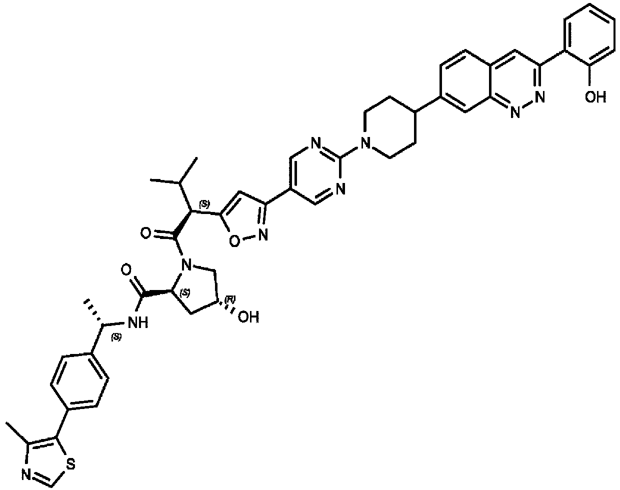 |
| 40 | 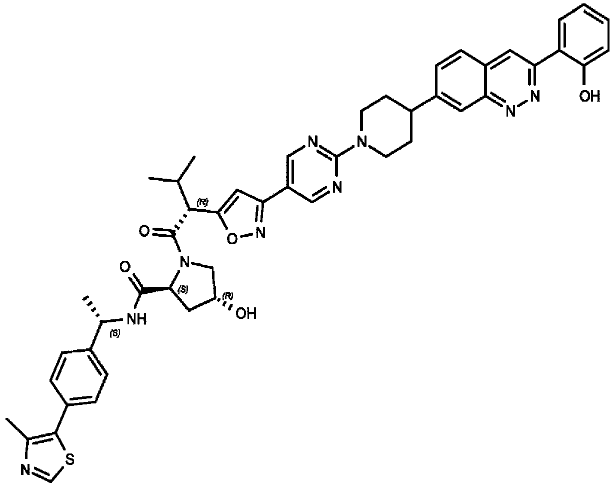 |
| 41 | 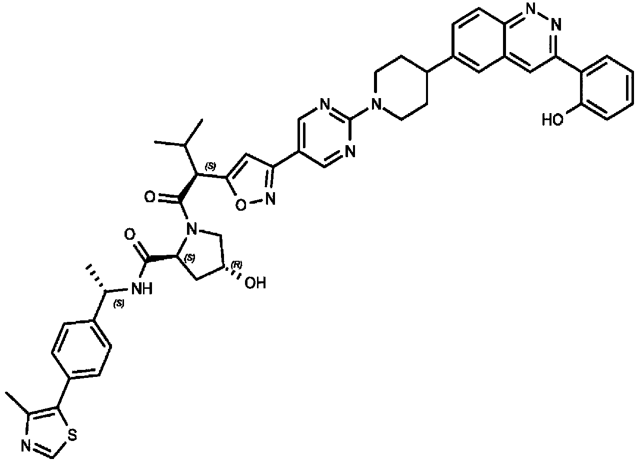 |
| 42 | 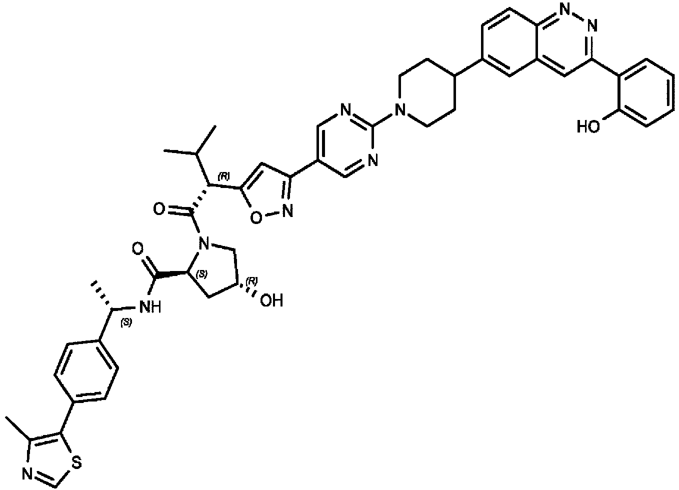 |
| 43 | 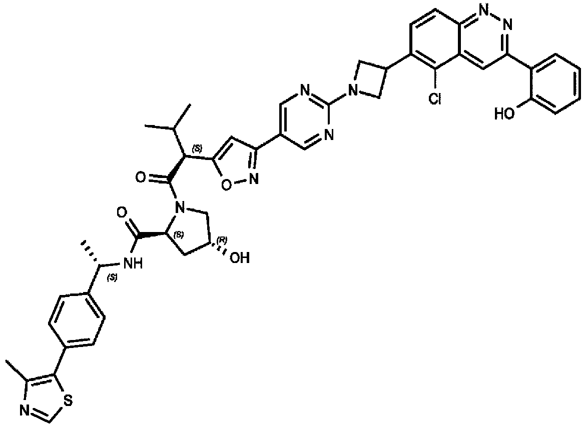 |
| 44 | 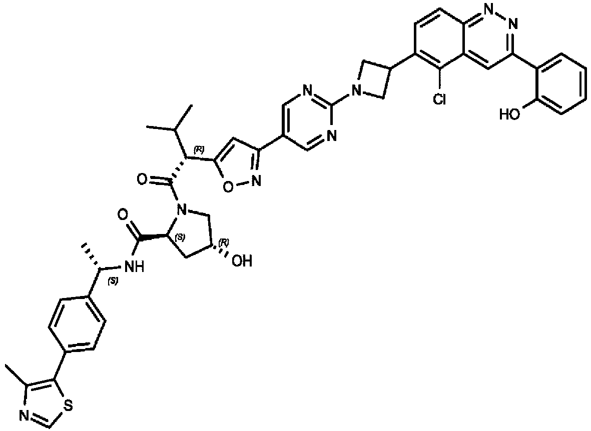 |
| 45 | 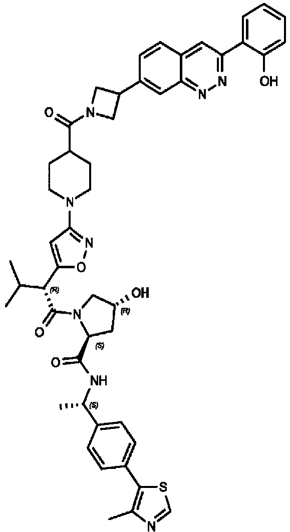 |
| 46 | 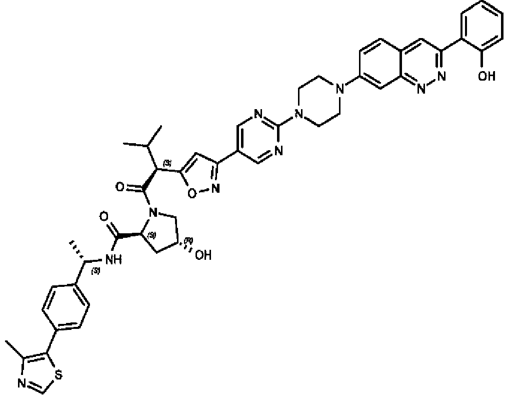 |
| 47 | 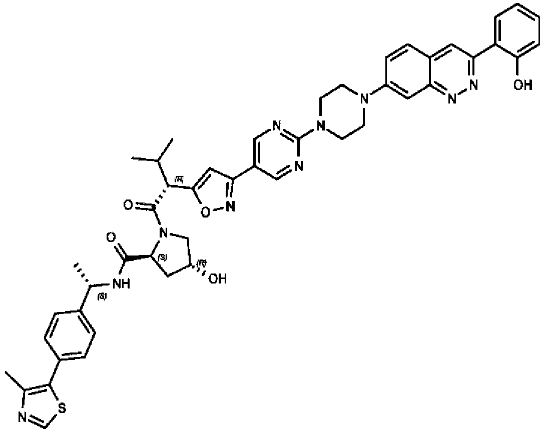 |
| 48 | 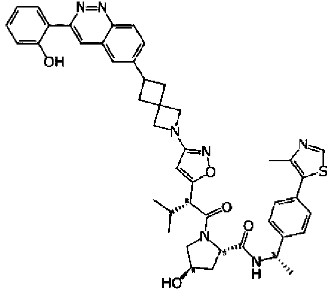 |
| 49 | 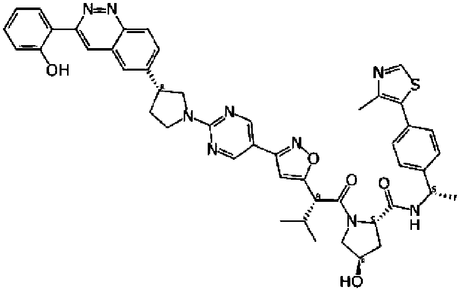 |
| 50 | 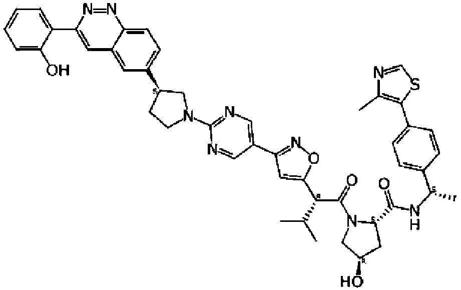 |
| 51 | 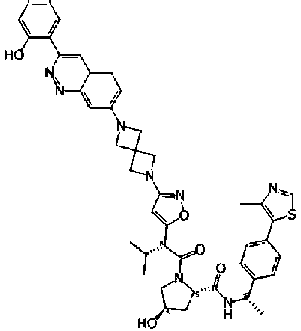 |
| 52 | 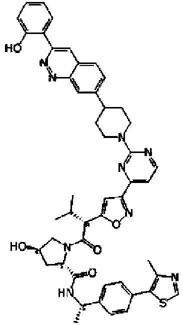 |
| 53 | 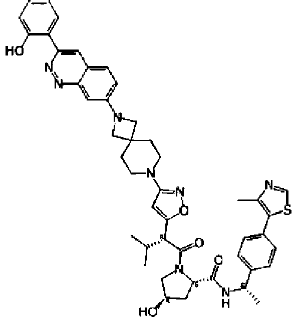 |
| 54 | 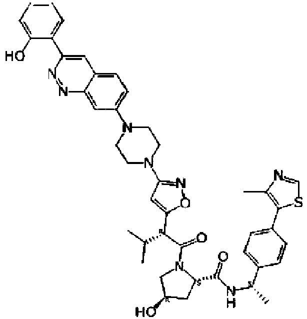 |
| 55 | 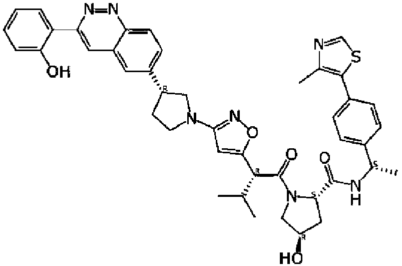 |
| 56 | 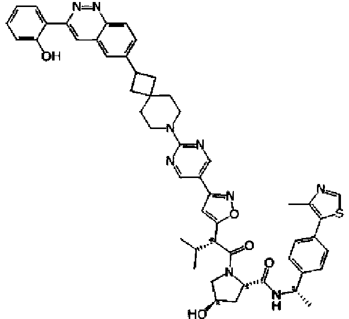 |
| 57 | 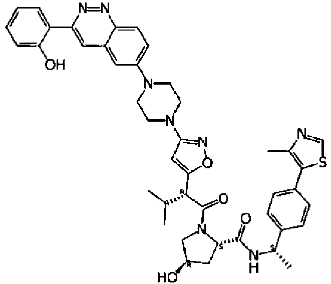 |
| 58 | 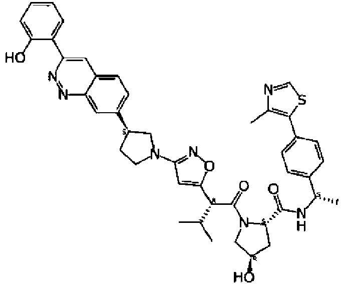 |
| 59 | 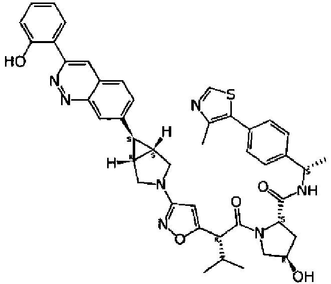 |
| 60 | 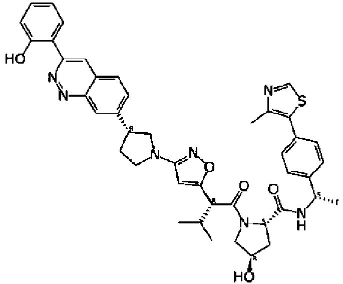 |
| 61 | 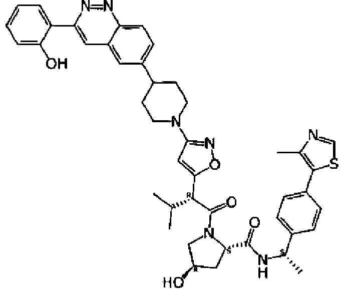 |
| 62 | 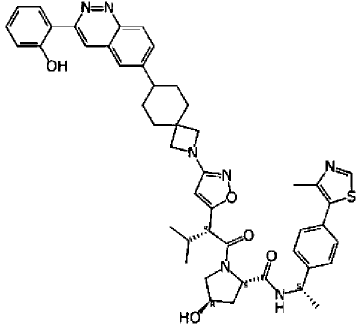 |
| 63 | 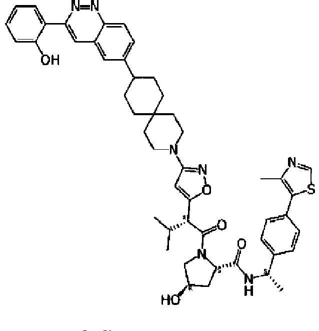 |
| 64 | 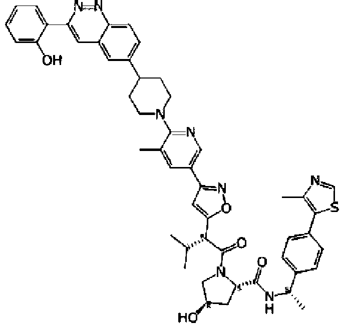 |
| 65 | 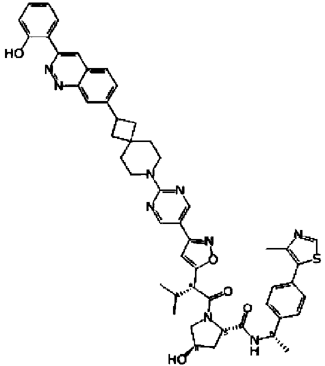 |
| 66 | 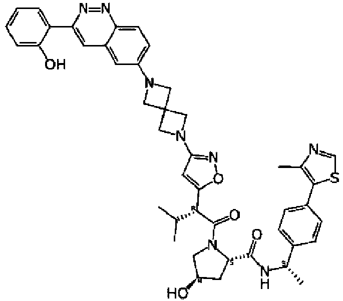 |
| 67 | 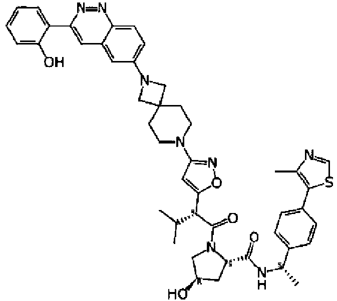 |
| 68 | 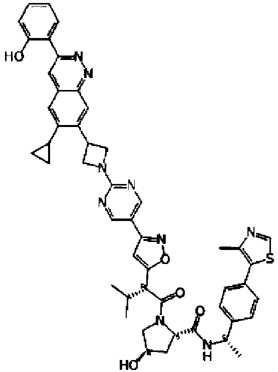 |
| 69 | 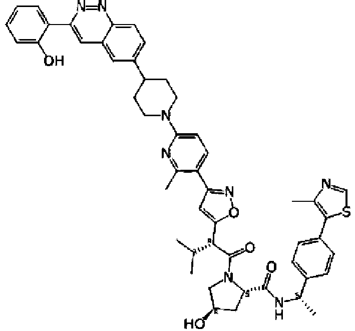 |
| 70 | 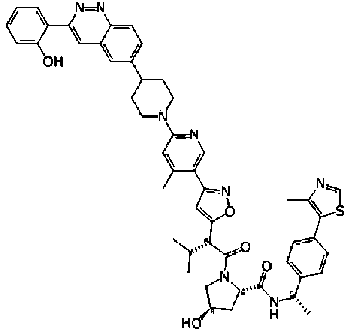 |
| 71 | 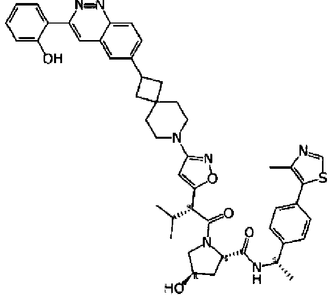 |
| 72 | 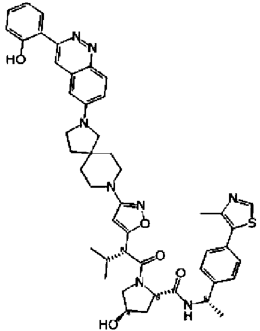 |
| 73 | 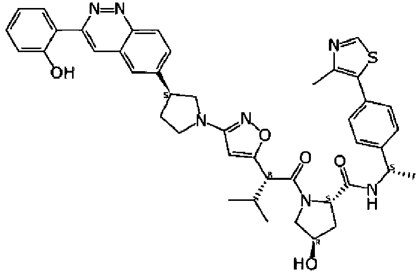 |
| 74 | 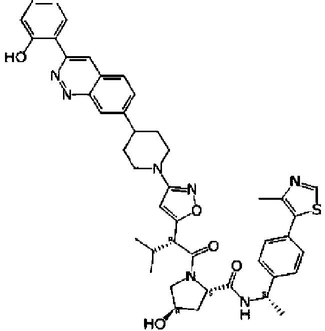 |
| 75 | 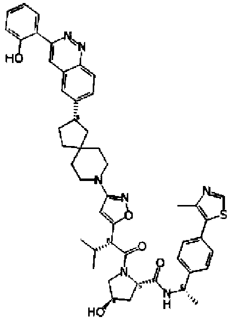 |
| 76 | 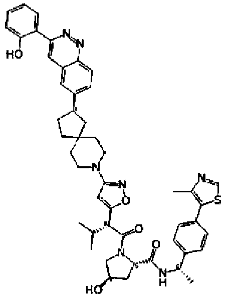 |
| 77 | 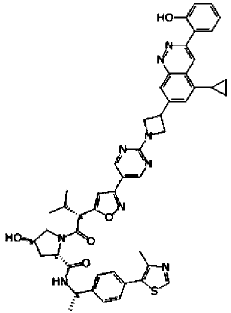 |
| 78 | 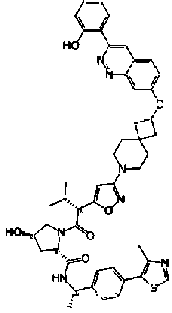 |
| | |
| 79 | 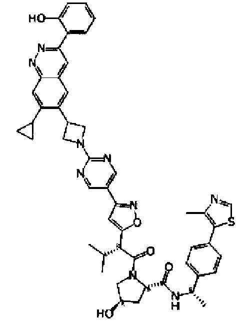 |
| 80 | 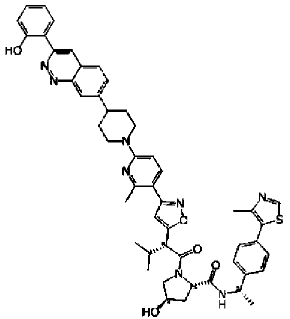 |
| 81 | 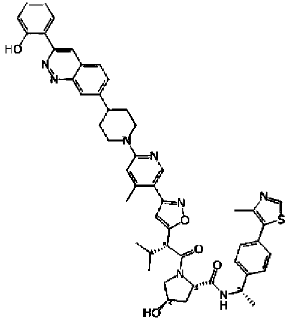 |
| 82 | 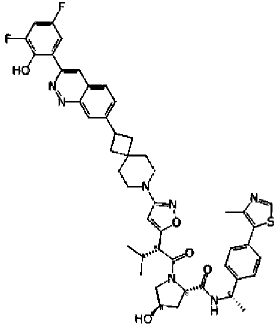 |
| 83 | 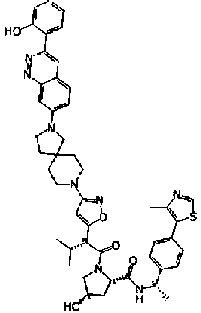 |
| 84 | 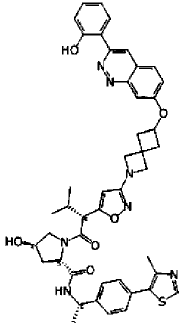 |
| 85 | 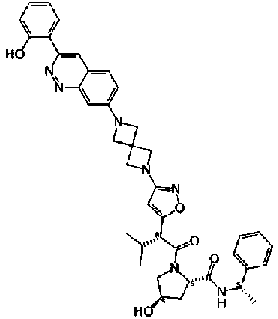 |
| 86 | 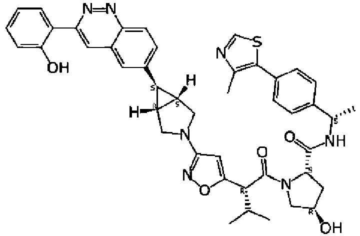 |
| 87 | 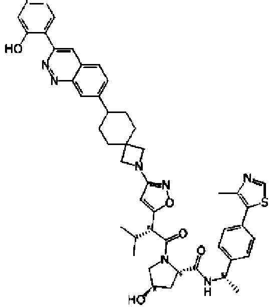 |
| 88 | 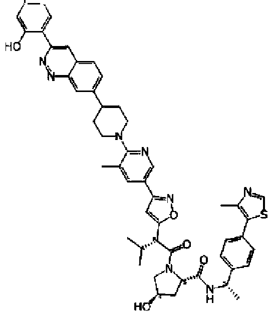 |
| 89 | 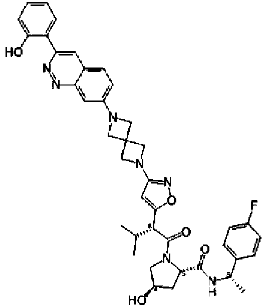 |
| 90 | 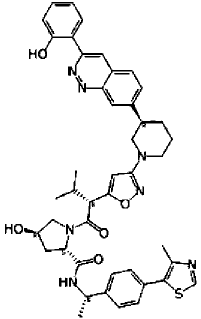 |
| 91 | 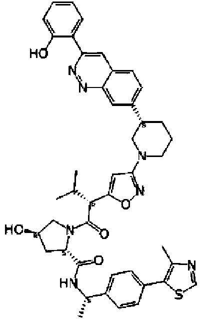 |
| 92 | 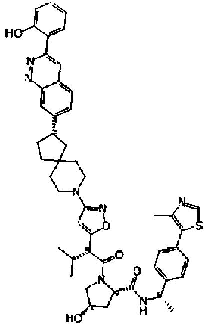 |
| 93 | 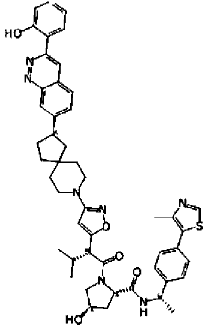 |
| 94 | 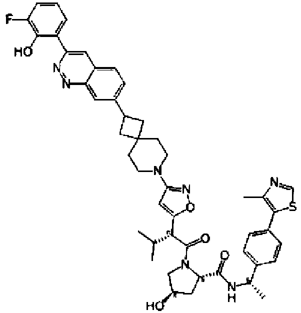 |
| 95 | 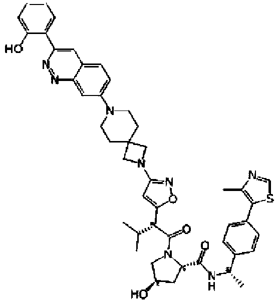 |
| 96 | 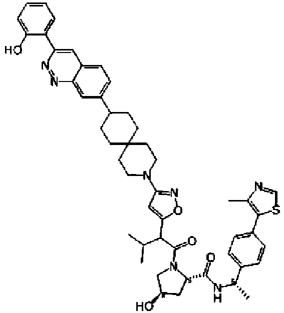 |
| 97 | 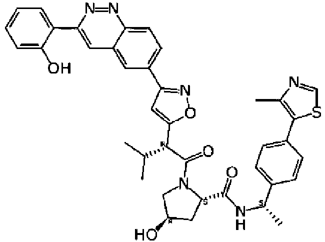 |
| 98 | 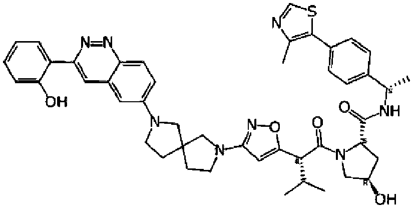 |
| 99 | 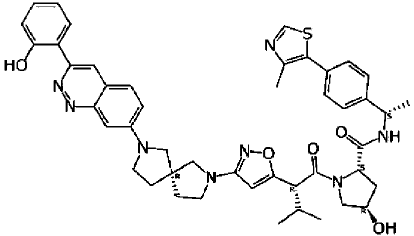 |
| 100 | 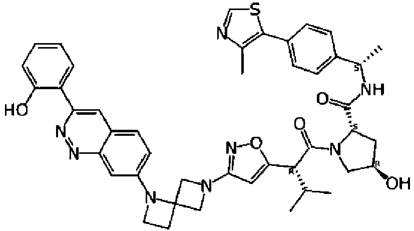 |
| 101 | 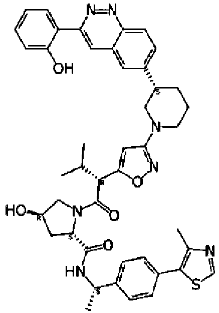 |
| 102 | 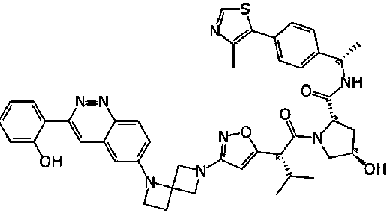 |
| 103 | 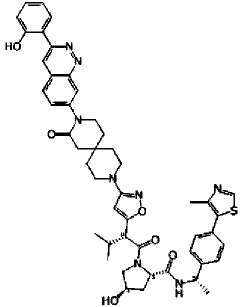 |
| 104 | 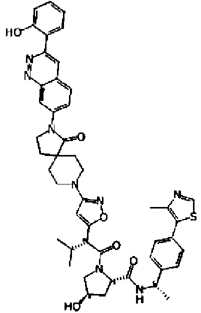 |
| 105 | 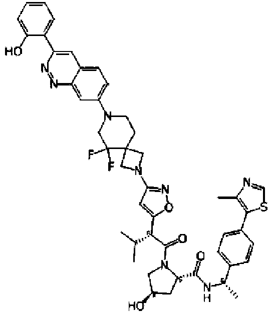 |
| 106 | 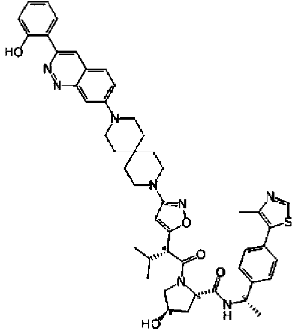 |
| 107 | 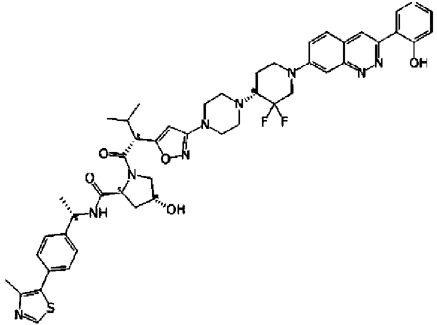 |
| 108 | 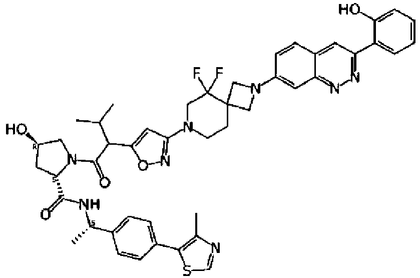 |
| 109 | 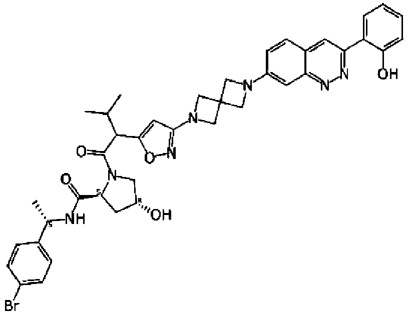 |
| 110 | 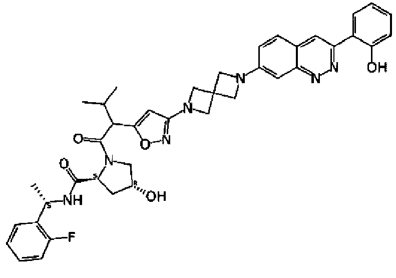 |
| 111 | 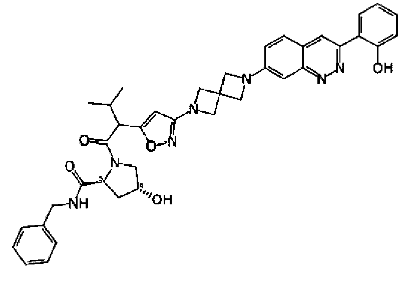 |
| 112 | 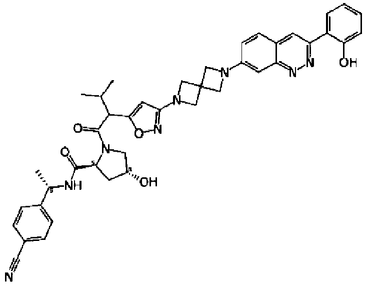 |
| 113 | 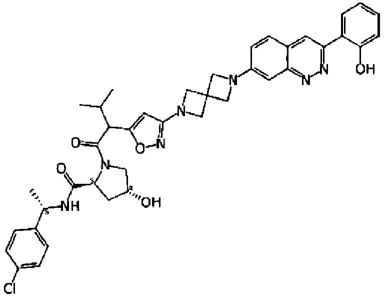 |
| 114 | 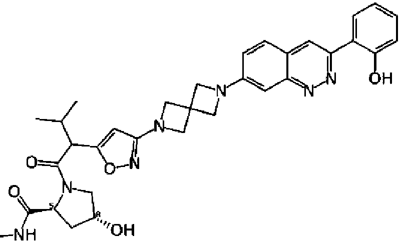 |
| 115 | 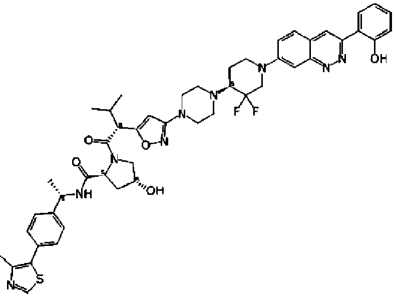 |
| 116 | 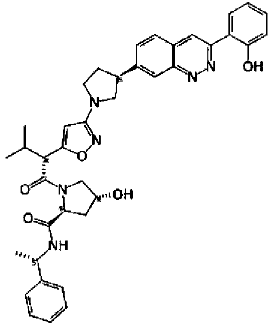 |
| 117 | 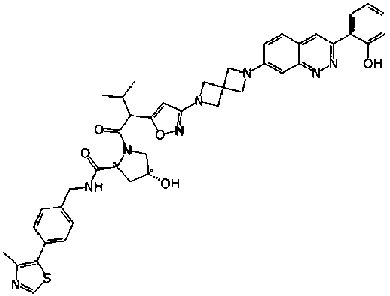 |
| 118 | 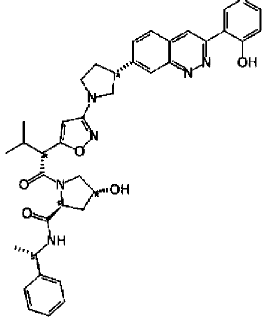 |
| 119 | 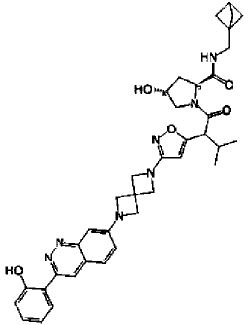 |
| 120 | 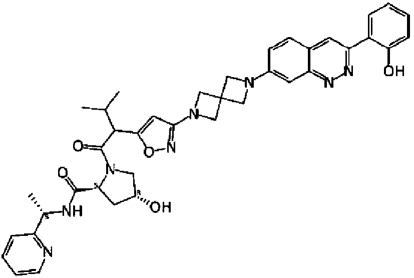 |
| 121 | 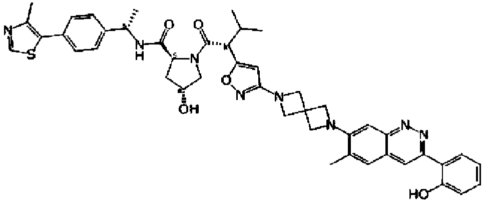 |
| 122 | 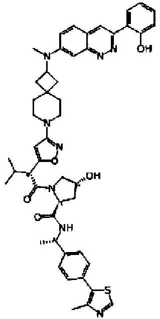 |
| 123 | 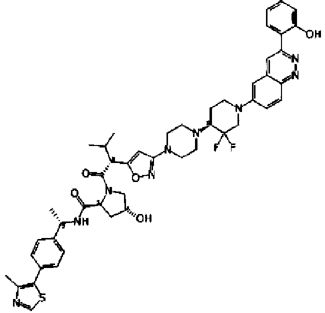 |
| 124 | 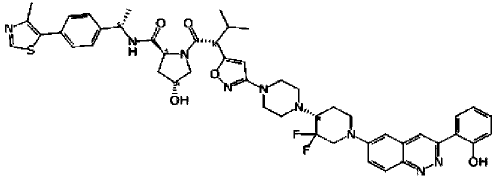 |
| 125 | 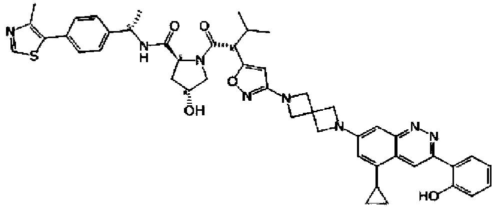 |
| 126 | 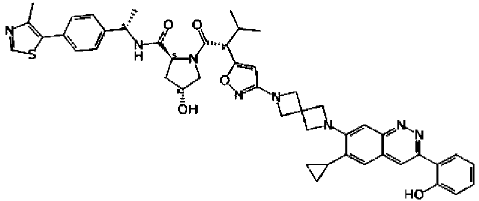 |
| 127 | 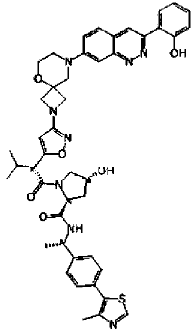 |
| 128 | 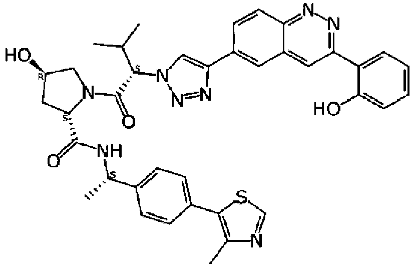 |
| 129 | 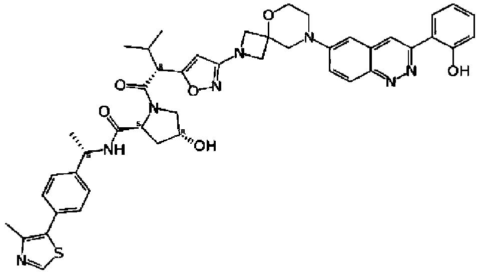 |
| 130 | 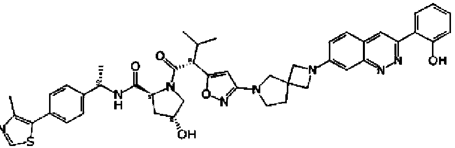 |
| 131 | 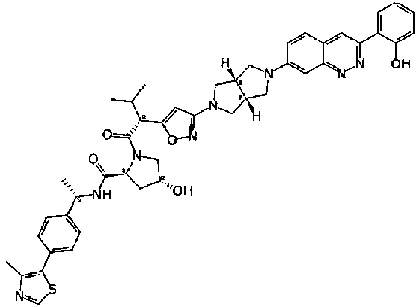 |
| 132 | 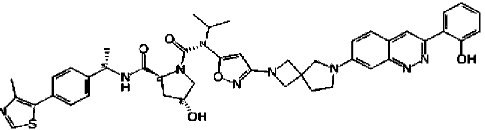 |
| 133 | 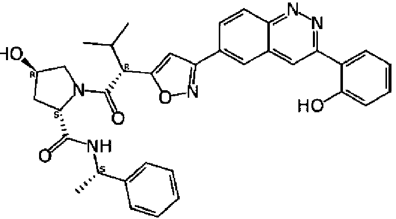 |
| 134 | 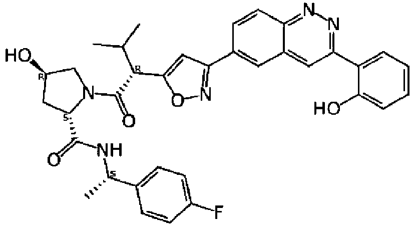 |
| 135 | 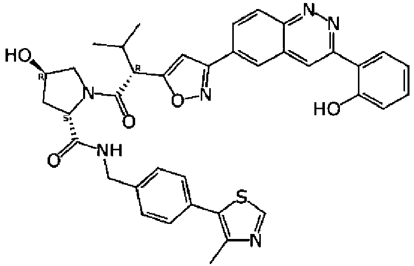 |
| 136 | 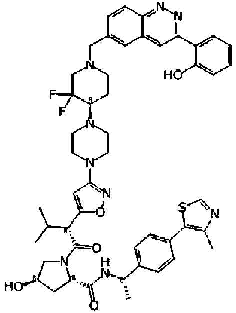 |
| 137 | 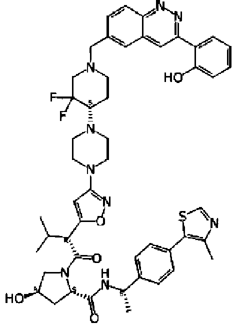 |
| 138 | 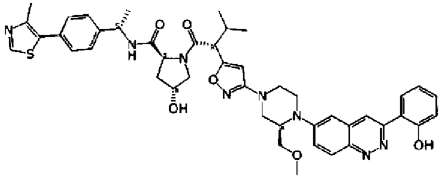 |
| 139 | 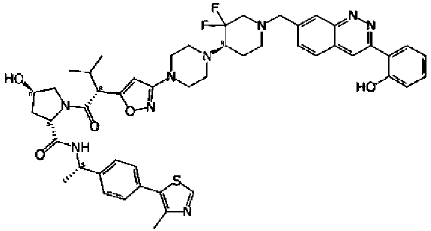 |
| 140 | 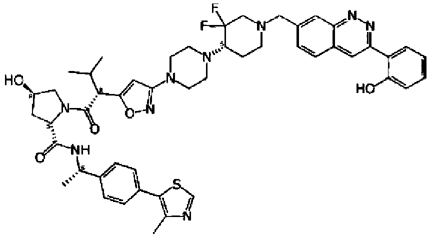 |
| 141 | 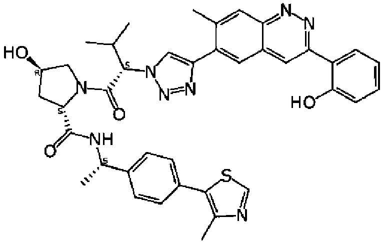 |
| 142 | 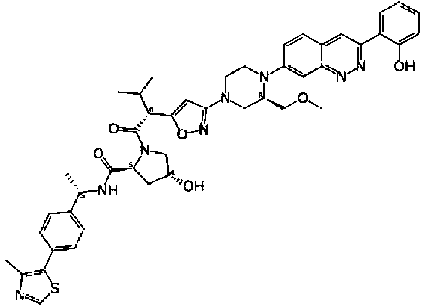 |
| 143 | 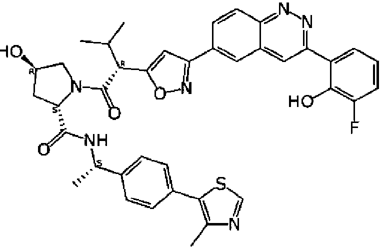 |
| 144 | 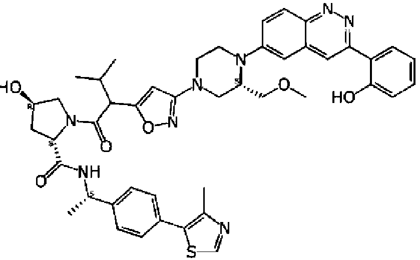 |
| 145 | 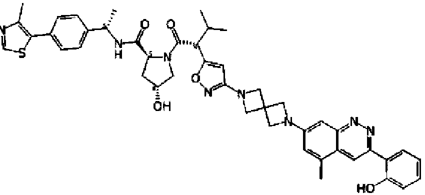 |
| 146 | 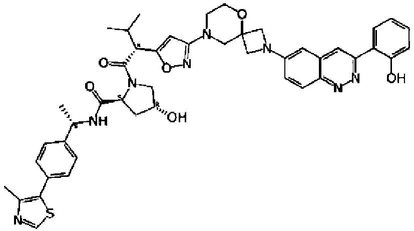 |
| 147 | 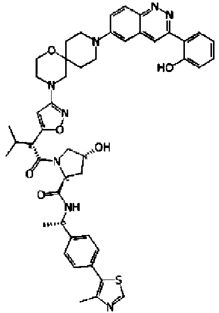 |
| 148 | 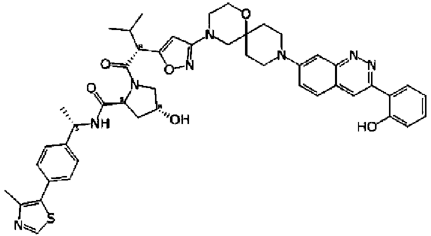 |
| 149 | 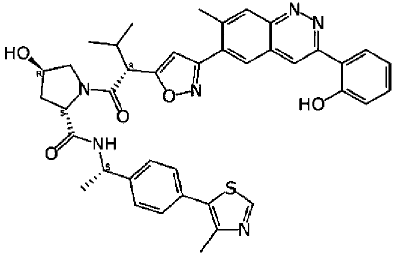 |
| 150 | 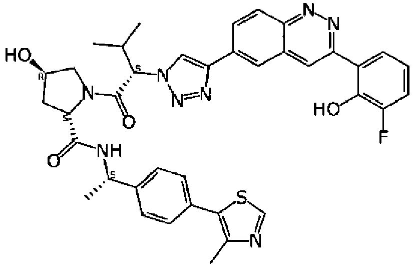 |
| 151 | 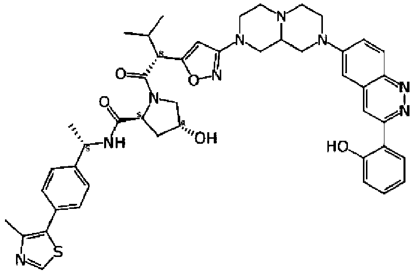 |
| 152 | 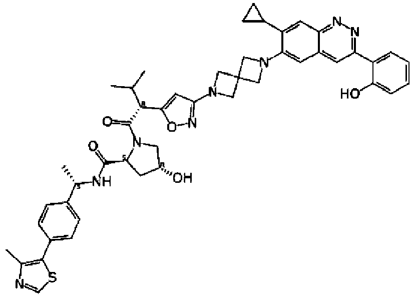 |
| 153 | 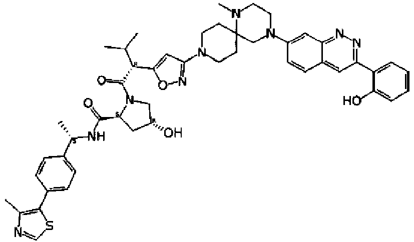 |
| 154 | 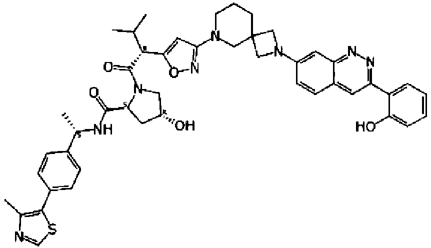 |
| 155 | 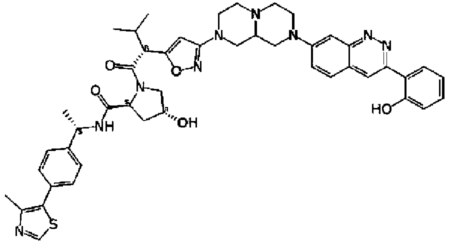 |
| 156 | 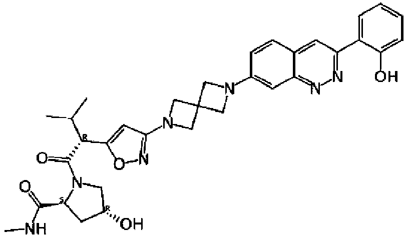 |
| 157 | 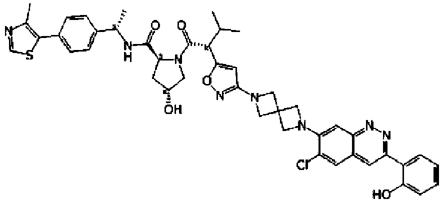 |
| 158 | 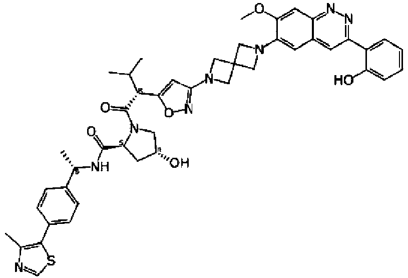 |
| 159 | 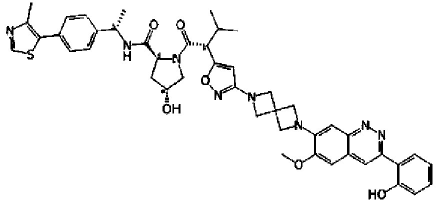 |
| 160 | 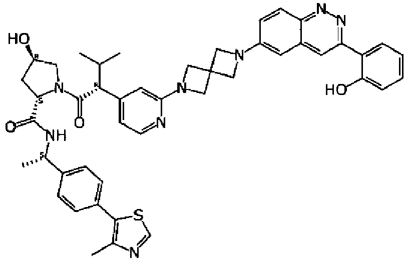 |
| 161 | 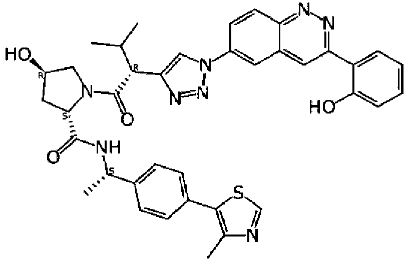 |
| 162 | 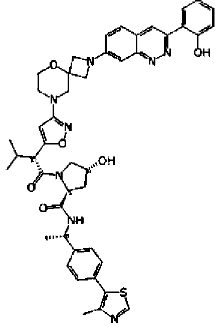 |
| 163 | 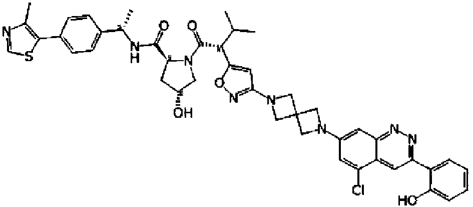 |
| 164 | 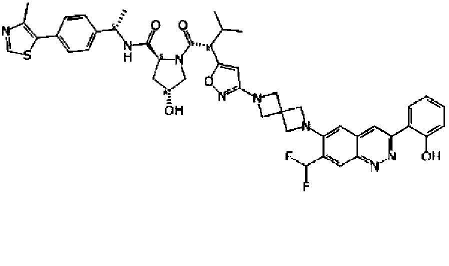 |
| 165 | 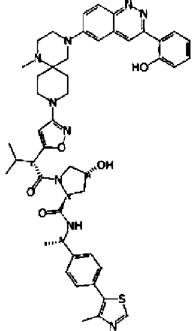 |
| 166 | 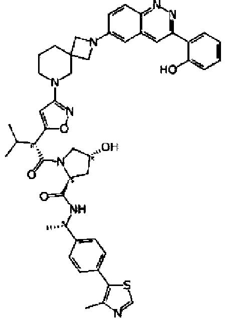 |
| 167 | 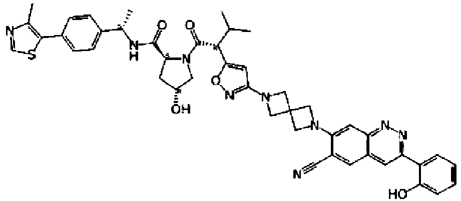 |
| 168 | 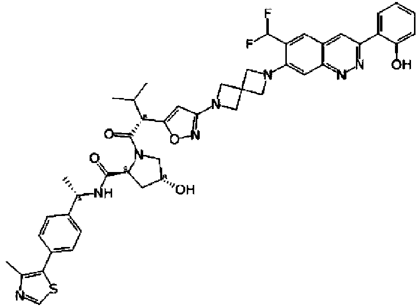 |
| 169 | 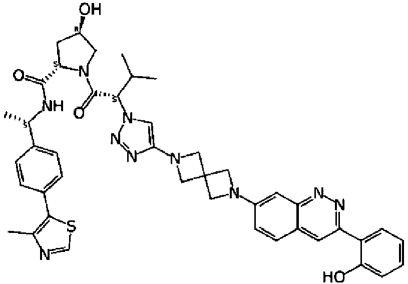 |
| 170 | 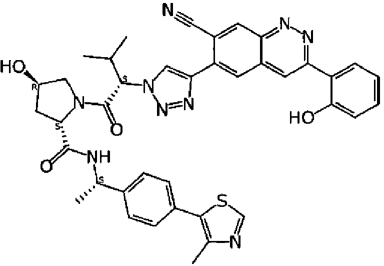 |
| 171 | 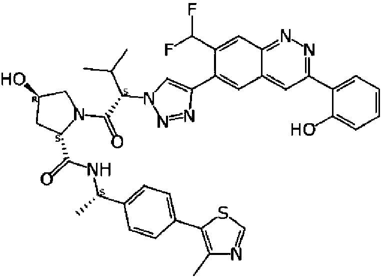 |
| 172 | 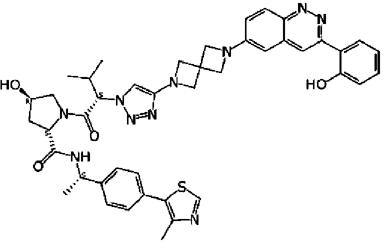 |
| 173 | 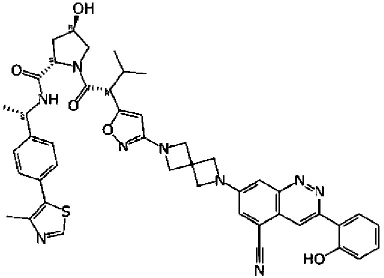 |
| 174 | 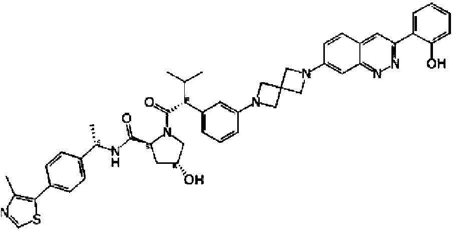 |
| 175 | 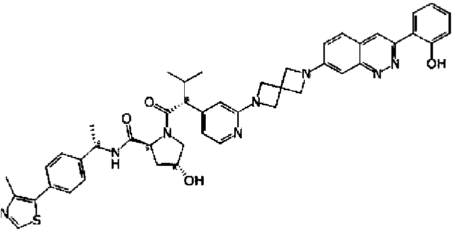 |
| 176 | 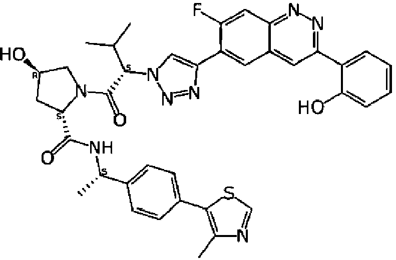 |
| 177 | 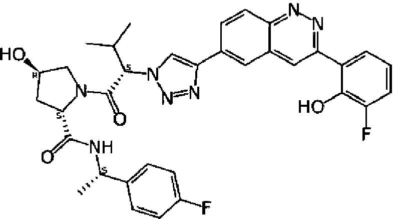 |
| 178 | 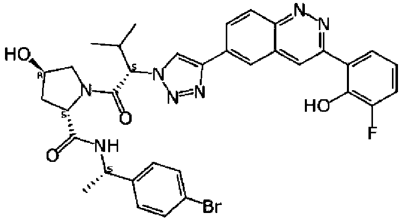 |
| 179 | 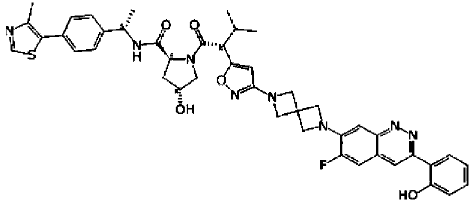 |
| 180 | 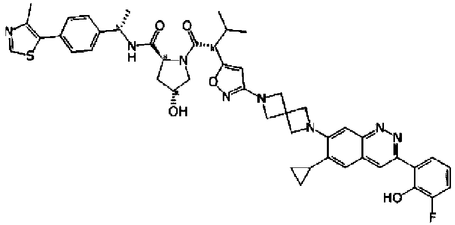 |
| 181 | 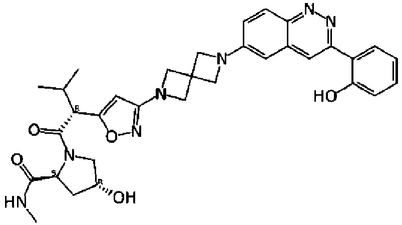 |
| 182 | 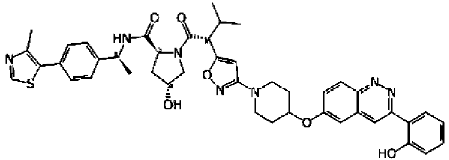 |
| 183 | 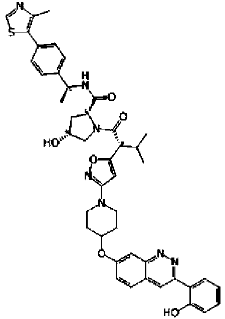 |
| 184 | 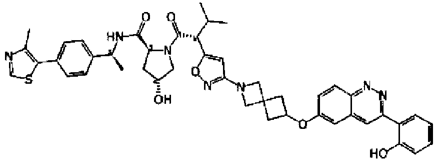 |
| 185 | 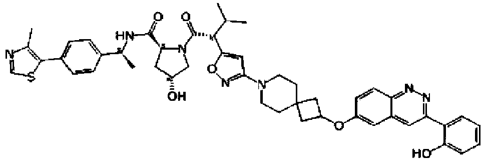 |
| 186 | 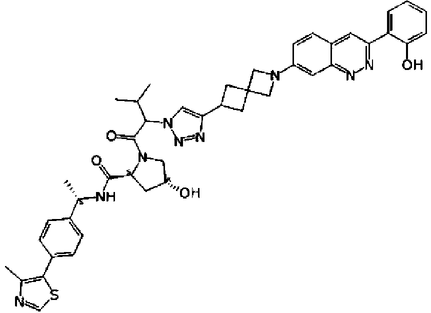 |
| 187 | 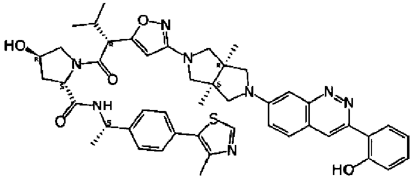 |
| 188 | 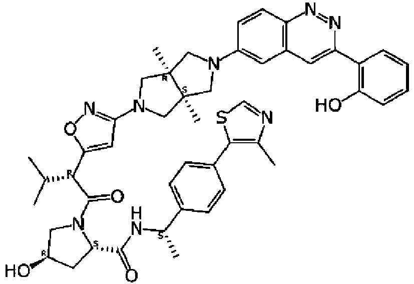 |
| 189 | 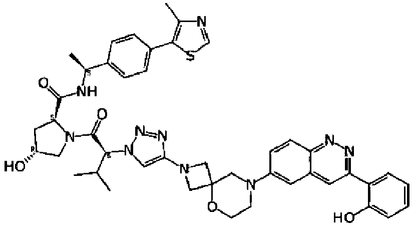 |
| 190 | 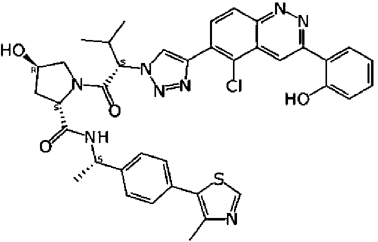 |
| 191 | 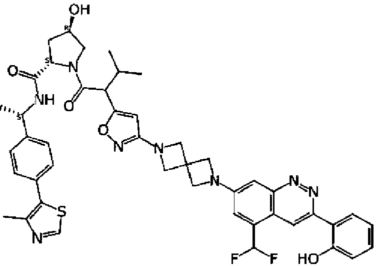 |
| 192 | 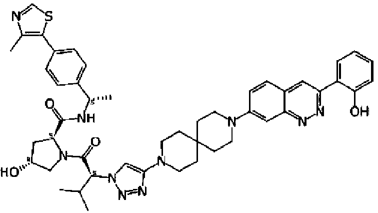 |
| 193 | 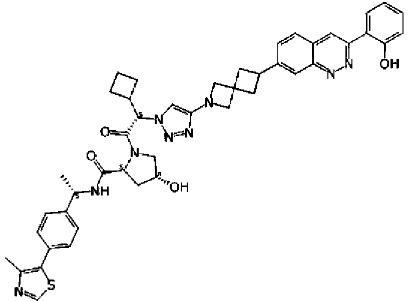 |
| 194 | 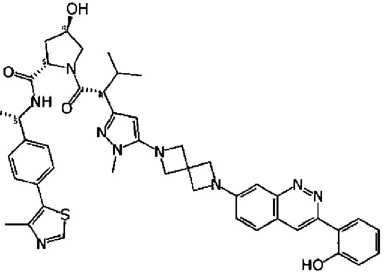 |
| 195 | 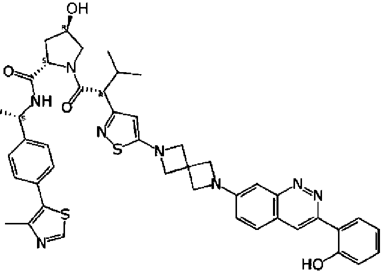 |
| 196 | 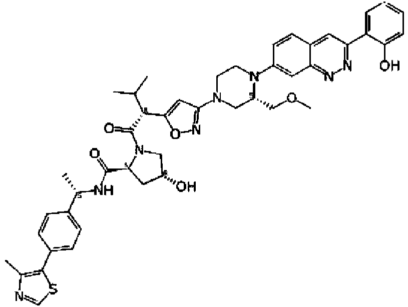 |
| 197 | 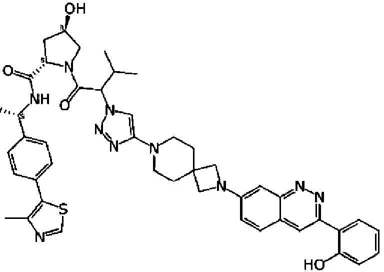 |
| 198 | 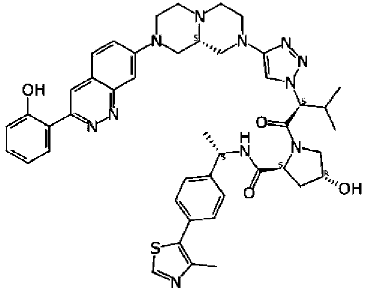 |
| 199 | 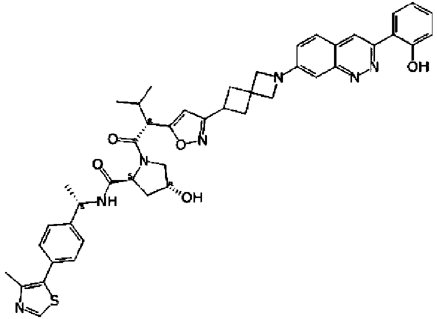 |
| 200 | 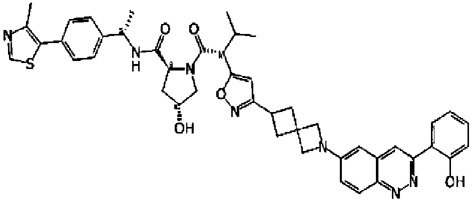 |
| 201 | 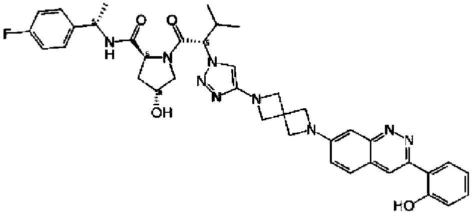 |
| 202 | 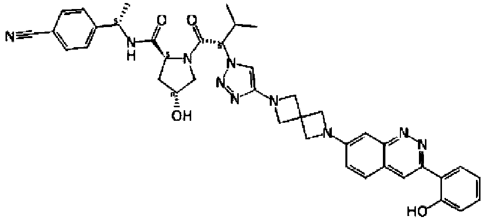 |
| 203 | 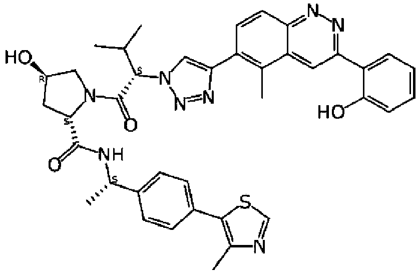 |
| 204 | 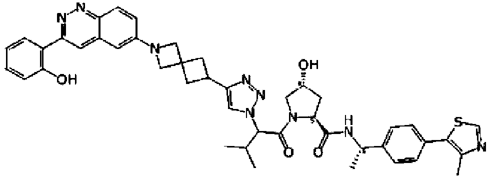 |
| 205 | 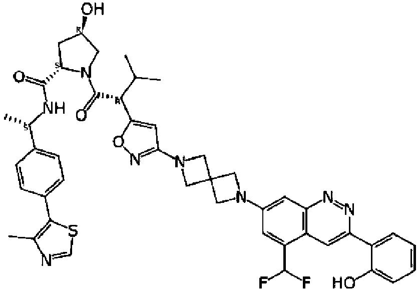 |
| 206 | 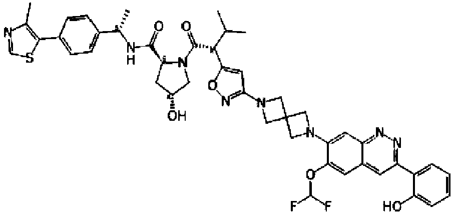 |
| 207 | 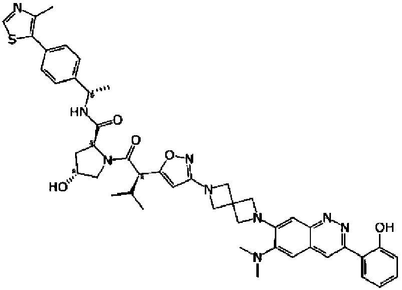 |
| 208 | 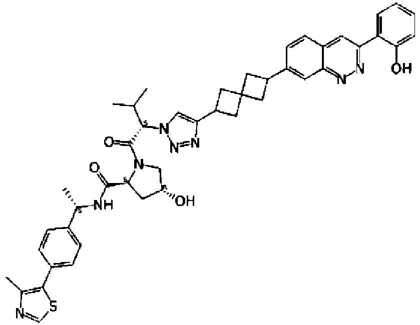 |
| 209 | 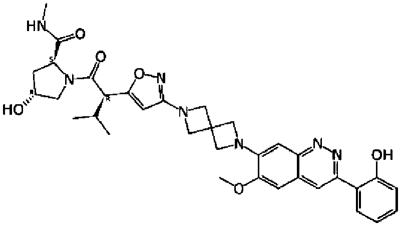 |
| 210 | 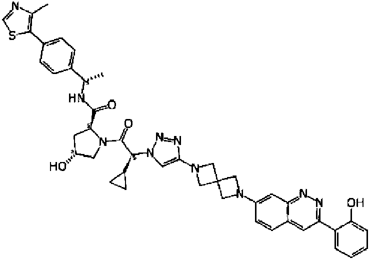 |
| 211 | 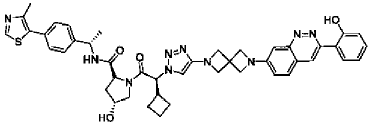 |
| 212 | 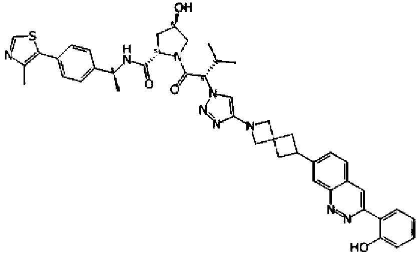 |
| 213 | 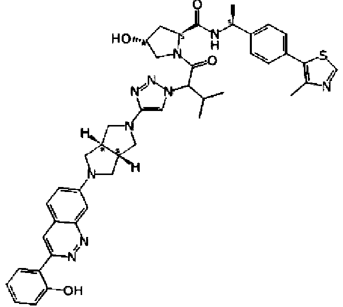 |
| 214 | 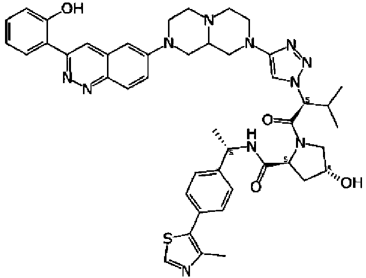 |
| 215 | 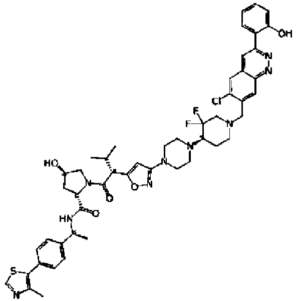 |
| 216 | 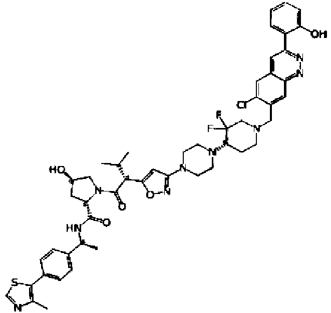 |
| 217 | 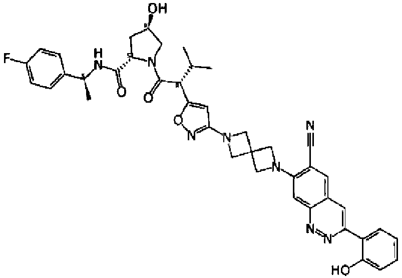 |
| 218 | 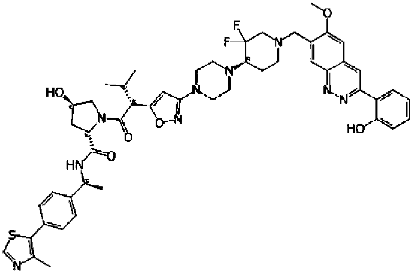 |
| 219 | 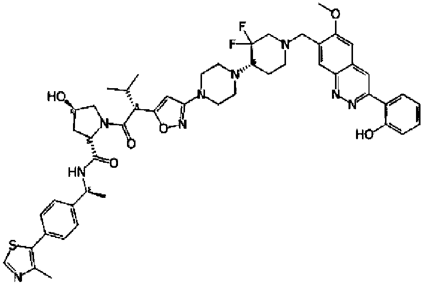 |
| 220 | 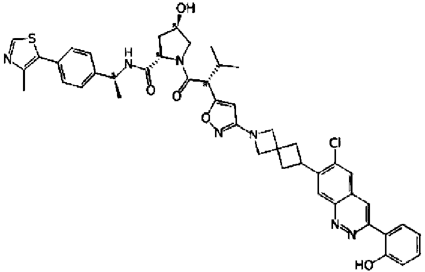 |
| 221 | 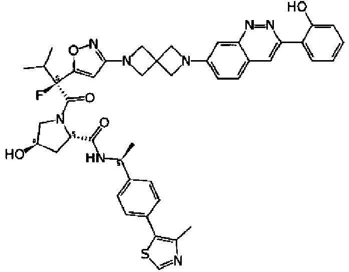 |
| 222 | 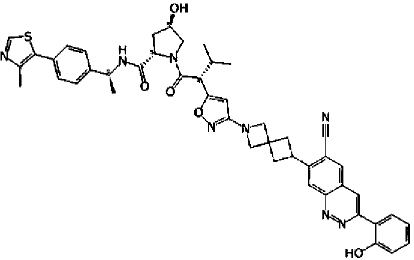 |
| 223 | 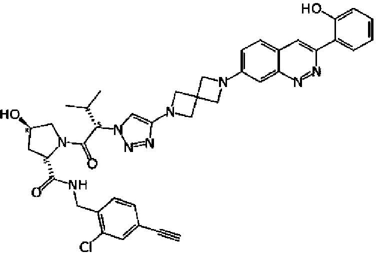 |
| 224 | 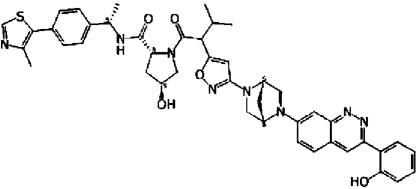 |
| 225 | 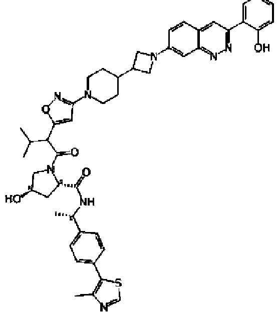 |
| 226 | 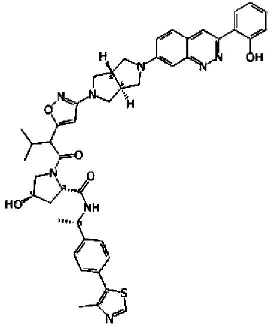 |
| 227 | 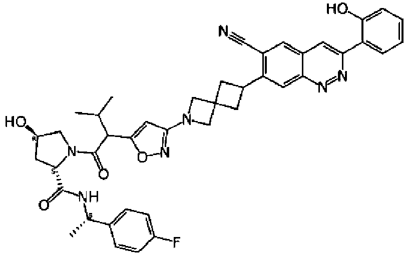 |
| 228 | 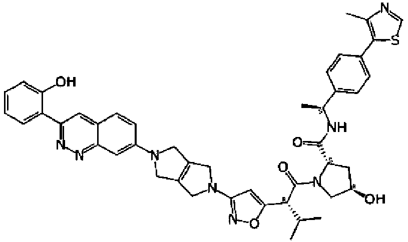 |
| 229 | 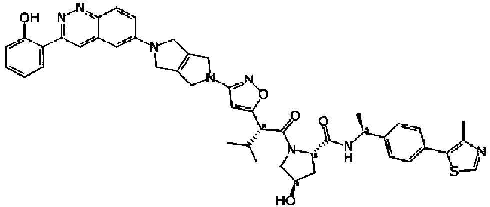 |
| 230 | 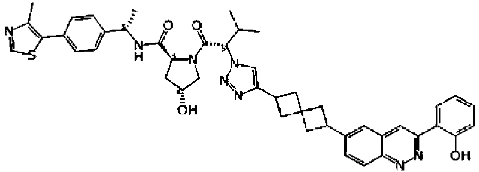 |
| 231 | 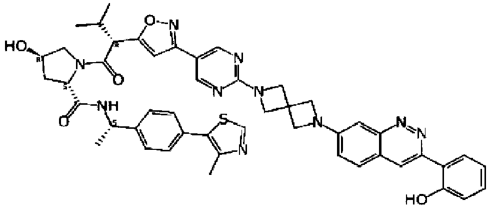 |
| 232 | 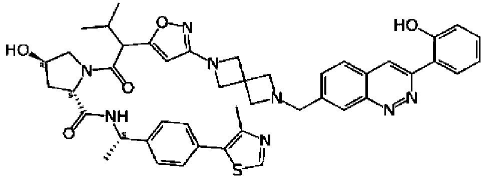 |
| 233 | 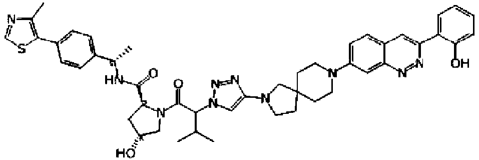 |
| 234 | 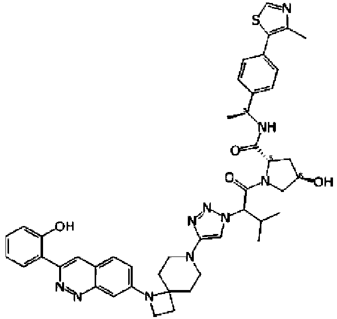 |
| 235 | 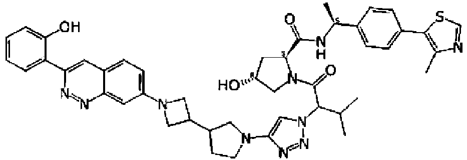 |
| 236 | 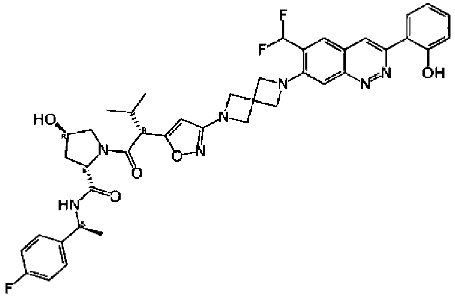 |
| 237 | 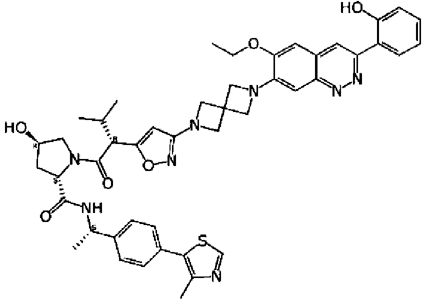 |
| 238 | 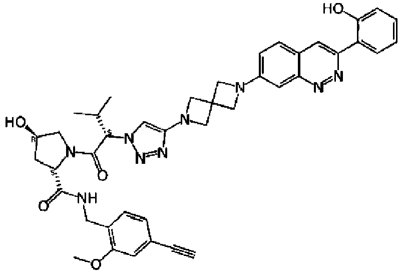 |
| 239 | 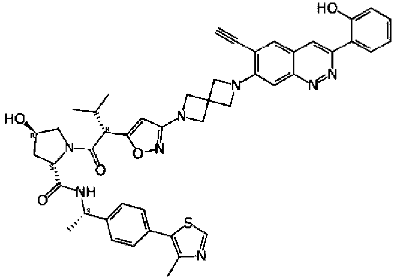 |
| 240 | 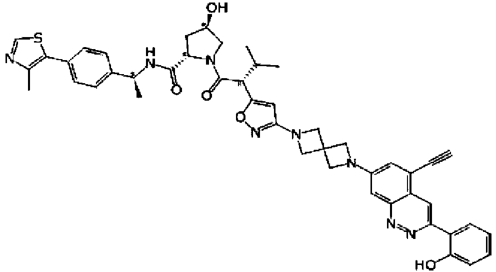 |
| 241 | 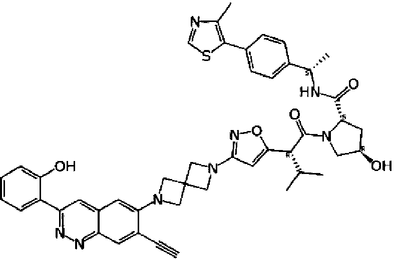 |
| 242 | 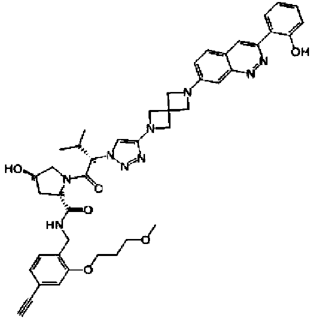 |
| 243 | 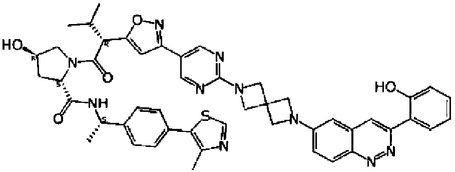 |
| 244 | 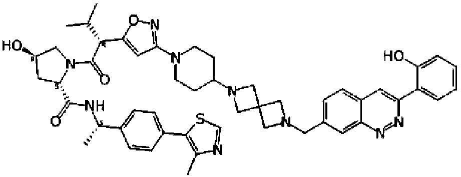 |
| 245 | 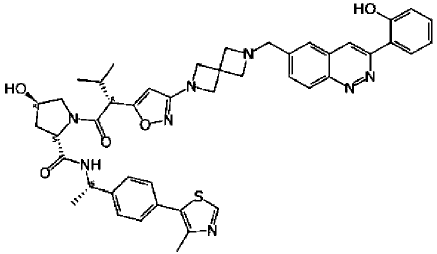 |
| 246 | 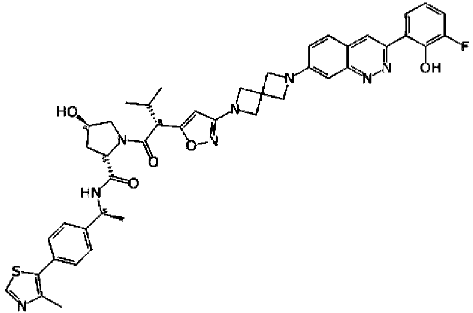 |
| 247 | 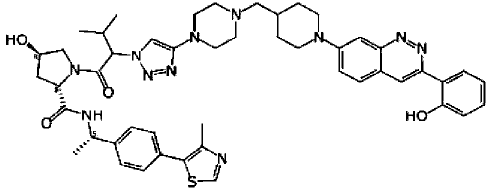 |
| 248 | 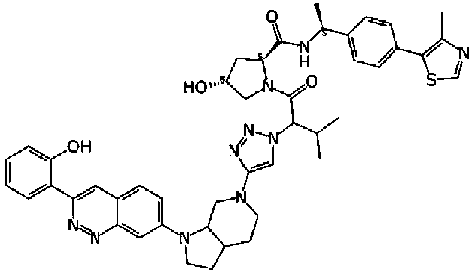 |
| 249 | 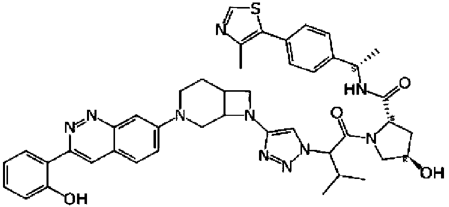 |
| 250 | 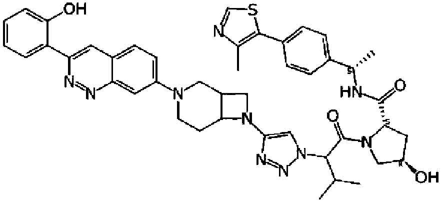 |
| 251 | 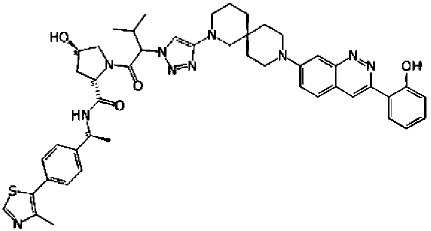 |
| 252 | 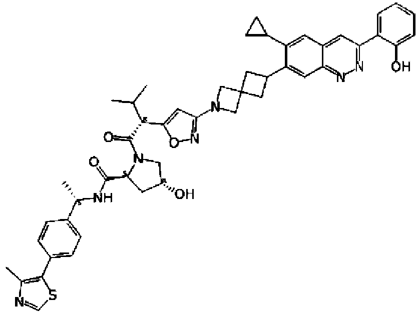 |
| 253 | 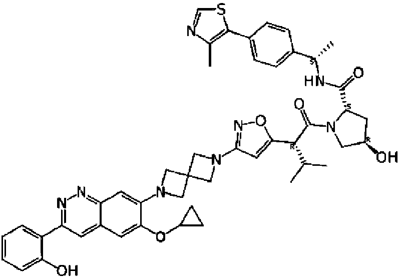 |
| 254 | 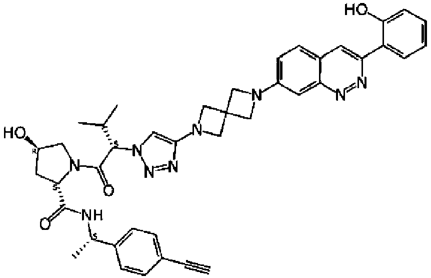 |
| 255 | 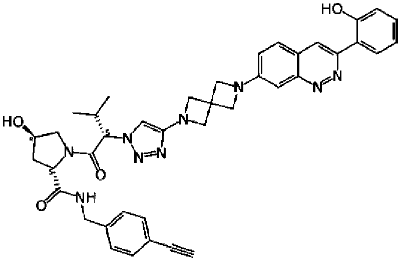 |
| 256 | 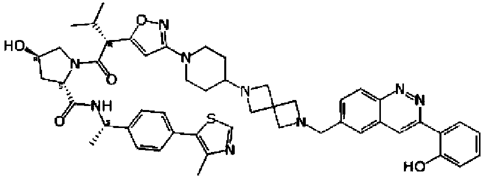 |
| 257 | 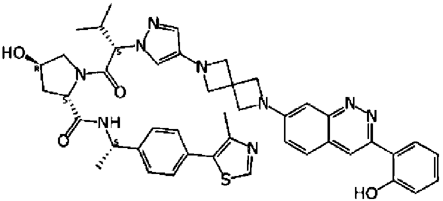 |
| 258 | 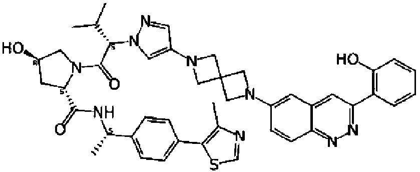 |
| 259 | 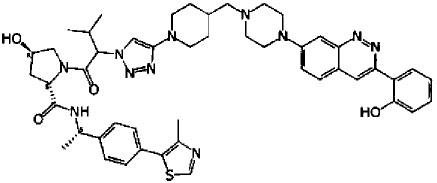 |
| 260 | 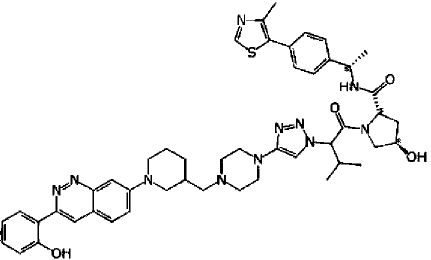 |
| 261 | 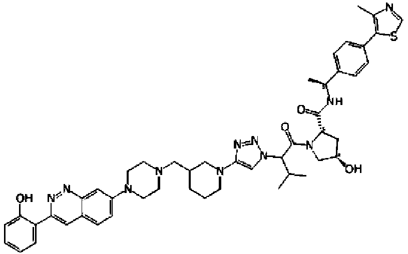 |
| 262 | 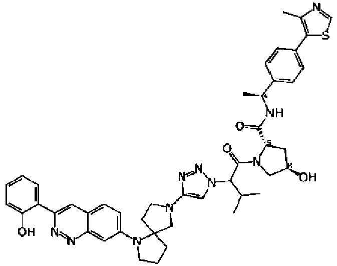 |
| 263 | 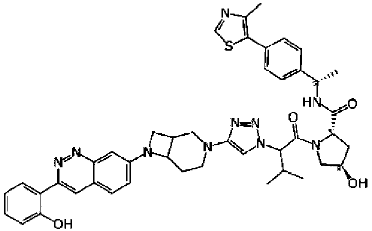 |
| 264 | 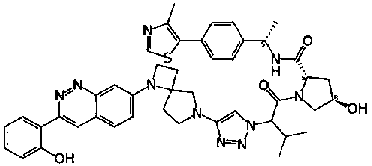 |
| 265 | 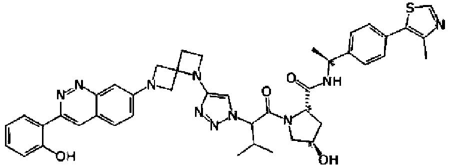 |
| 266 | 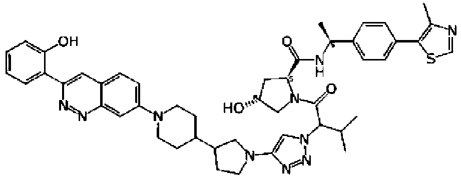 |
| 267 | 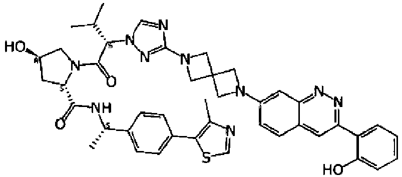 |
| 268 | 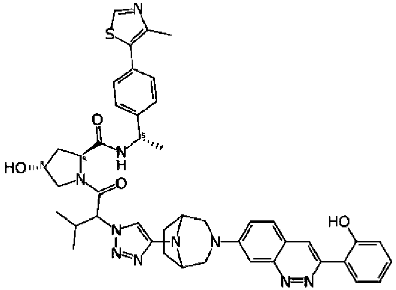 |
| 269 | 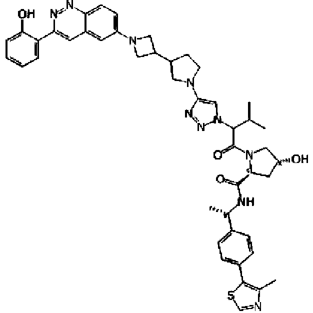 |
| 270 | 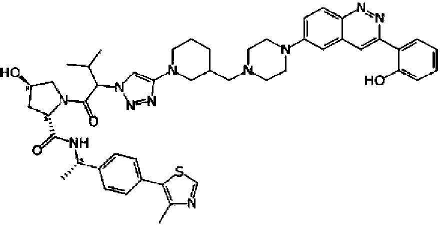 |
| 271 | 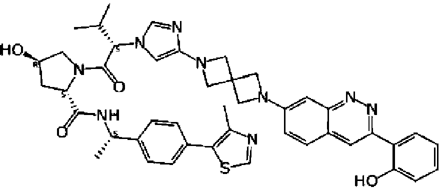 |
| 272 | 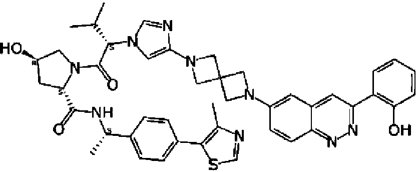 |
| 273 | 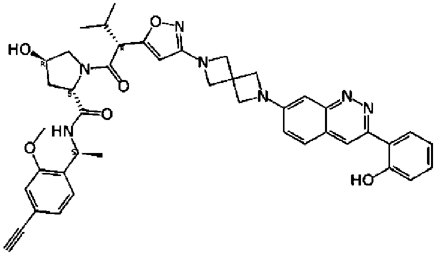 |
| 274 | 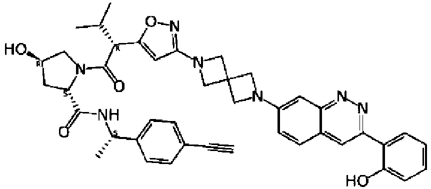 |
| 275 | 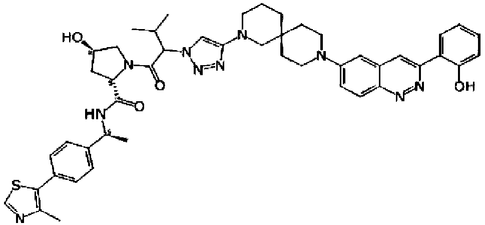 |
| 276 | 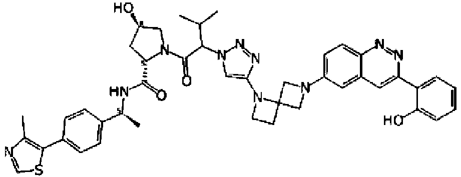 |
| 277 | 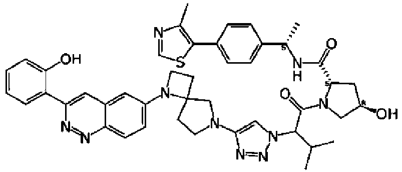 |
| 278 | 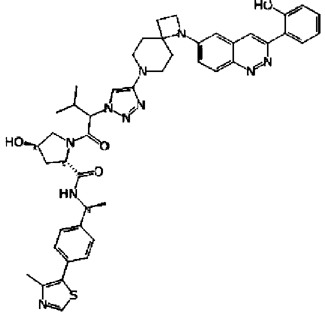 |
| 279 | 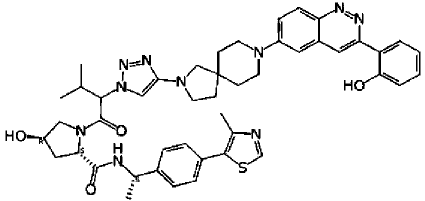 |
| 280 | 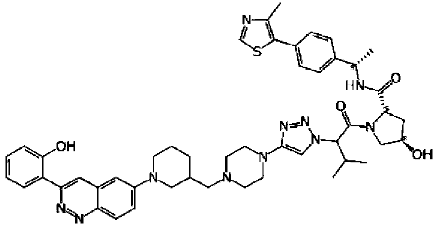 |
| 281 | 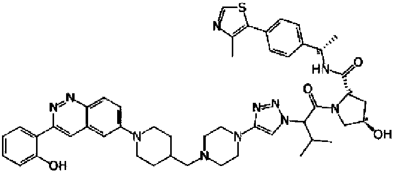 |
| 282 | 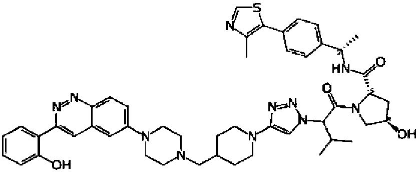 |
| 283 | 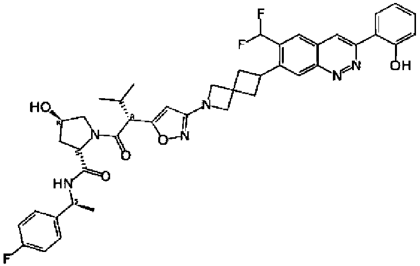 |
| 284 | 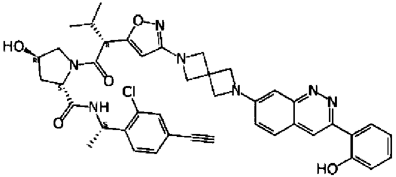 |
| 285 | 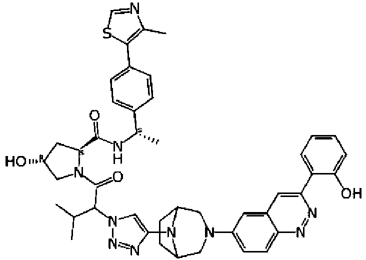 |
| 286 | 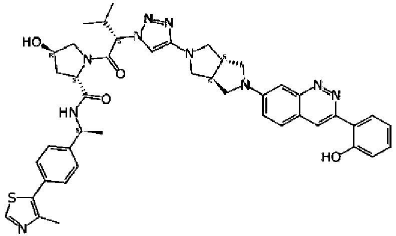 |
| 287 | 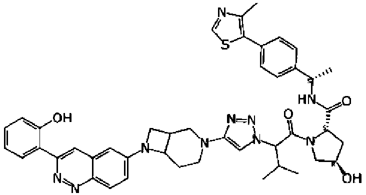 |
| 288 | 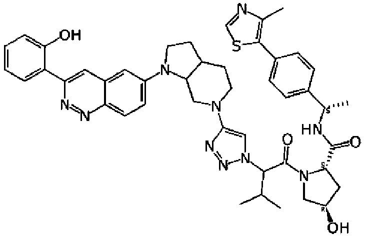 |
| 289 | 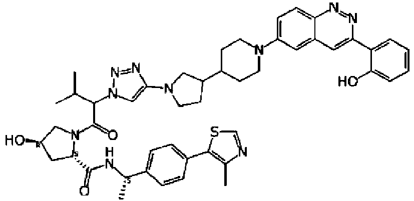 |
| 290 | 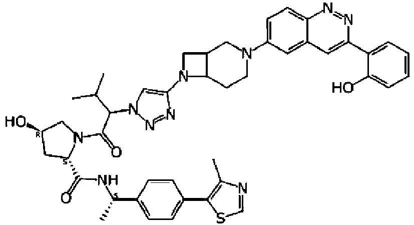 |
| 291 | 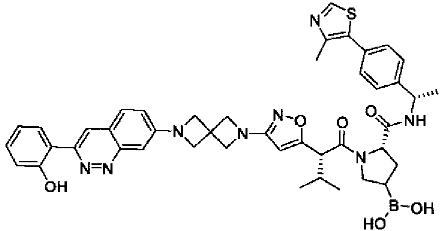 |

19. Pharmazeutische Zusammensetzung, umfassend eine Verbindung nach einem der Ansprüche 1 bis 18 und einen pharmazeutisch verträglichen Trägerstoff.

20. Verbindung nach einem der Ansprüche 1 bis 18 oder ein pharmazeutisch verträgliches Salz davon oder eine pharmazeutische Zusammensetzung nach Anspruch 19 für eine Verwendung bei einer Therapie.

21. Verbindung nach einem der Ansprüche 1 bis 18 oder ein pharmazeutisch verträgliches Salz davon oder eine pharmazeutische Zusammensetzung nach Anspruch 19 für eine Verwendung beim Behandeln von Krebs.

22. Verbindung oder pharmazeutisch verträgliches Salz davon oder die pharmazeutische Zusammensetzung zur Verwendung nach Anspruch 21, wobei der Krebs nichtkleinzelliger Lungenkrebs, kolorektaler Krebs, Blasenkrebs, Krebs unbekannten Primärtumors, Gliom, Brustkrebs, Melanom, Nicht-Melanom-Hautkrebs, Endometriumkrebs, ösophagogastrischer Krebs, Bauchspeicheldrüsenkrebs, hepatobiliärer Krebs, Weichgewebesarkom, Eierstockkrebs, Kopf- und Halskrebs, Nierenzellkarzinom, Knochenkrebs, Non-Hodgkin-Lymphom, kleinzelliger Lungenkrebs, Prostatakrebs, embryonaler Tumor, Keimzelltumor, Gebärmutterhalskrebs, Schilddrüsenkrebs, Speicheldrüsenkrebs, gastrointestinaler neuroendokriner Tumor, Uterussarkom, gastrointestinaler Stromatumor, ZNS-Krebs, Thymustumor, Nebennierenrindenkarzinom, Appendixkrebs, Dünndarmkrebs oder Peniskrebs ist.

## Revendications

1. Composé, ou sel pharmaceutiquement acceptable de celui-ci, de **Formule I :** 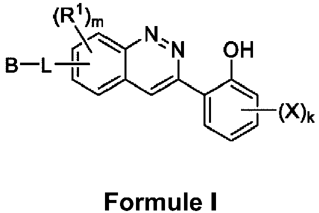 dans lequel
m vaut 0, 1,2, ou 3 ;
k vaut 0, 1 ou 2 ;
chaque R¹ est, indépendamment, halo, alkyle en C₁ à C₆ facultativement substitué, hétéroalkyle en C₁ à C₆ facultativement substitué, hétérocyclyle en C₂ à C₉ facultativement substitué, cycloalkyle en C₃ à C₈ facultativement substitué, cycloalcoxy en C₃ à C₈ facultativement substitué, alcynyle en C₂ à C₆ facultativement substitué, amino facultativement substitué, ou cyano ;
chaque X est, indépendamment, halo ou hétéroalkyle en C₁ à C₆ facultativement substitué ;
L est un lieur
de **Formule II** :
A¹-(B¹)_{f}-(C¹)_{g}-(B²)ₕ-(D)-(B³)ᵢ-(C²)ⱼ-(B⁴)ₖ-A², **Formule II**
ou un sel pharmaceutiquement acceptable de celui-ci,
dans lequel
A¹ est une liaison entre le lieur et le système cyclique A ;
A² est une liaison entre le fragment de dégradation et le lieur ;
chacun de B¹, B², B³ et B⁴ est, indépendamment, alkyle en C₁ à C₄ facultativement substitué, aryle en C₆ à C₁₀ facultativement substitué, aryl en C₆ à C₁₀-alkyle en C₁ à ₄ facultativement substitué, hétéroalkyle en C₁ à C₄ facultativement substitué, cycloalkyle en C₃ à C₁₀ facultativement substitué, carbocyclyle en C₃ à C₁₀ facultativement substitué, hétérocyclyle en C₂ à C₁₀ facultativement substitué, hétéroaryle en C₂ à C₆ facultativement substitué, aryle en C₆ à C₁₂ facultativement substitué, O, S, S(O)₂ ou NR^{N} ;
chaque R^{N} est, indépendamment, H, alkyle en C_{1 à 4} facultativement substitué, alcényle en C_{2 à 4} facultativement substitué, alcynyle en C_{2 à 4} facultativement substitué, hétérocyclyle en C_{2 à 10} facultativement substitué, hétéroaryle en C_{2 à 6} facultativement substitué ou hétéroalkyle en C_{1 à 7} facultativement substitué ;
chacun de C¹ et de C² est, indépendamment, carbonyle, thiocarbonyle, sulfonyle ou phosphoryle ;
chacun de f, g, h, i, j et k vaut, indépendamment, 0 ou 1 ; et
D est alkyle en C_{1 à 10} facultativement substitué, alcynyle en C_{2 à 10} facultativement substitué, alcynyle en C_{2 à 10} facultativement substitué, hétérocyclyle en C_{2 à 10} facultativement substitué, hétéroaryle en C_{2 à 6} facultativement substitué, aryle en C_{6 à 12} facultativement substitué, polyéthylène glycol en C₂ à C₁₀ facultativement substitué, cycloalkyle en C₃ à C₁₀ facultativement substitué, carbocyclyle en C₃ à C₁₀ facultativement substitué ou hétéroalkyle en C₁ à ₁₀ facultativement substitué ; ou D est absent, et le lieur est A¹-(B¹)f-(C¹)_{g}-(B²)ₕ-(B³)ᵢ-(C²)ⱼ-(B⁴)ₖ-A², et
B est un fragment de dégradation de **Formule C :** 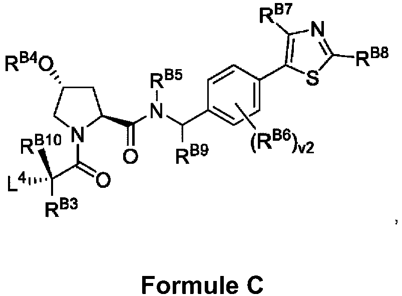
dans lequel
L⁴ est -N(R^{B1})(R^{B2}), 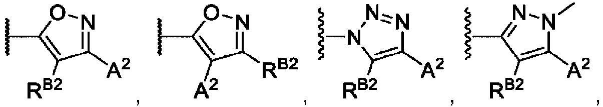 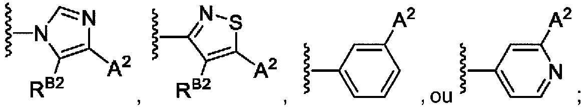
R^{B1} est H, A², alkyle en C₁ à C₆ facultativement substitué, ou hétéroalkyle en C₁ à C₆ facultativement substitué ;
R^{B2} est H, alkyle en C₁ à C₆ facultativement substitué, ou hétéroalkyle en C₁ à C₆ facultativement substitué ;
R^{B3} est A², alkyle en C₁ à C₆ facultativement substitué, hétéroalkyle en C₁ à C₆ facultativement substitué, carbocyclyle en C₃ à C₁₀ facultativement substitué, aryle en C₆ à C₁₀ facultativement substitué, alkyl en C₁ à C₆-carbocyclyle en C₃ à C₁₀ facultativement substitué, ou alkyl en C₁ à C₆-aryle en C₆ à C₁₀ facultativement substitué ;
R^{B4} est H, alkyle en C₁ à C₆ facultativement substitué, carbocyclyle en C₃ à C₁₀ facultativement substitué, aryle en C₆ à ₁₀ facultativement substitué, alkyl en C₁ à C₆-carbocyclyle en C₃ à C₁₀ facultativement substitué ou alkyl en C₁ à C₆-aryle en C₆ à C₁₀ facultativement substitué ;
R^{B5} est H, alkyle en C₁ à C₆ facultativement substitué, ou hétéroalkyle en C₁ à C₆ facultativement substitué ;
v2 vaut 0, 1, 2, 3 ou 4 ;
chaque R^{B6} est, indépendamment, A², halogène, alkyle en C₁ à C₆ facultativement substitué, alcynyle en C₂ à C₆ facultativement substitué, hétéroalkyle en C₁ à C₆ facultativement substitué, carbocyclyle en C₃ à C₁₀ facultativement substitué, hétérocyclyle en C₂ à C₉ facultativement substitué, aryle en C₆ à C₁₀ facultativement substitué, hétéroaryle en C₂ à C₉ facultativement substitué, alcényle en C₂ à C₆ facultativement substitué, hétéroalcényle en C₂ à C₆ facultativement substitué, hydroxy, thiol, cyano, ou amino facultativement substitué ;
chacun de R^{B7} et R^{B8} est, indépendamment, H, halogène, alkyle en C₁ à C₆ facultativement substitué, ou aryle en C₆ à C₁₀ facultativement substitué ;
R^{B9} est H ou alkyle en C₁ à C₆ facultativement substitué ;
R^{B10} est H ou F ; et
A² est une liaison entre le fragment de dégradation et le lieur ;
dans lequel un et seulement un parmi R^{B1}, R^{B3} et R^{B6} est A²,
ou sel pharmaceutiquement acceptable de celui-ci.

2. Composé selon la revendication 1 ou sel pharmaceutiquement acceptable de celui-ci, dans lequel
chaque R¹ est, indépendamment, halo, alkyle en C₁ à C₆ facultativement substitué, hétéroalkyle en C₁ à C₆ facultativement substitué, hétérocyclyle en C₂ à C₉ facultativement substitué ou cycloalkyle en C₃ à C₈ facultativement substitué ; et
chaque X est, indépendamment, halo.

3. Composé selon la revendication 1 ou 2, ou sel pharmaceutiquement acceptable de celui-ci, dans lequel le composé a la structure de **Formule I-A** :

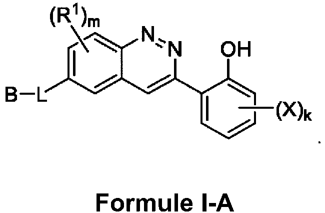

4. Composé selon la revendication 1 ou 2, ou sel pharmaceutiquement acceptable de celui-ci, dans lequel le composé a la structure de **Formule I-B** :

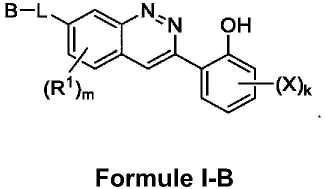

5. Composé selon l'une quelconque des revendications 1 à 4, ou sel pharmaceutiquement acceptable de celui-ci, dans lequel m vaut 1.

6. Composé selon l'une quelconque des revendications 1 à 5, ou sel pharmaceutiquement acceptable de celui-ci, dans lequel R¹ est alcoxy en C₁ à C₆ facultativement substitué, ou halo.

7. Composé selon l'une quelconque des revendications 1 à 6, ou sel pharmaceutiquement acceptable de celui-ci, dans lequel le fragment de dégradation a la structure de **Formule Ca2, Formule Cb2, Formule Cc2 Formule Cd2, Formule Ce2** ou **Formule Cf2 :**

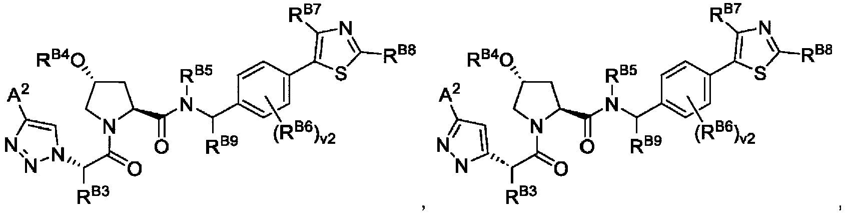

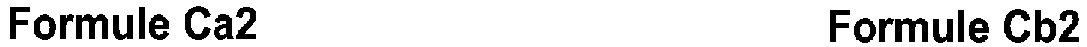

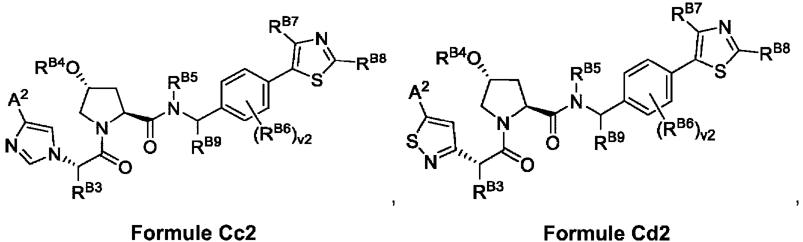

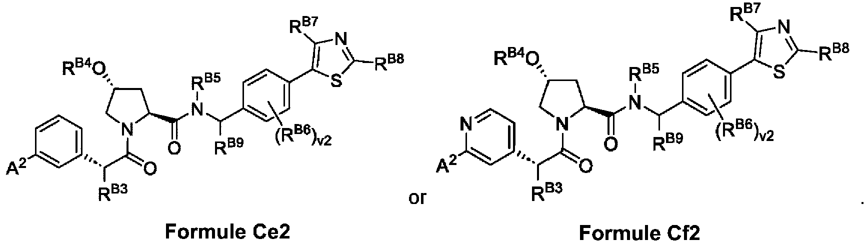

8. Composé selon l'une quelconque des revendications 1 à 7, ou sel pharmaceutiquement acceptable de celui-ci, dans lequel R^{B9} est méthyle.

9. Composé selon l'une quelconque des revendications 1 à 8, ou sel pharmaceutiquement acceptable de celui-ci, dans lequel R^{B4} est H.

10. Composé selon l'une quelconque des revendications 1 à 9, ou sel pharmaceutiquement acceptable de celui-ci, dans lequel R^{B5} est H.

11. Composé selon l'une quelconque des revendications 1 à 10, ou sel pharmaceutiquement acceptable de celui-ci, dans lequel R^{B7} est méthyle.

12. Composé selon l'une quelconque des revendications 1 à 11, ou sel pharmaceutiquement acceptable de celui-ci, dans lequel R^{B3} st alkyle en C₁ à C₆ facultativement substitué.

13. Composé selon l'une quelconque des revendications 1 à 6, ou sel pharmaceutiquement acceptable de celui-ci, dans lequel le fragment de dégradation est

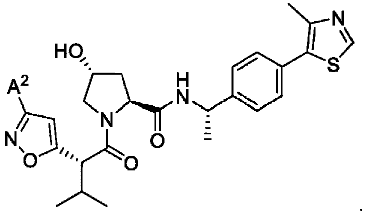

14. Composé selon l'une quelconque des revendications 1 à 6, ou sel pharmaceutiquement acceptable de celui-ci, dans lequel le fragment de dégradation est

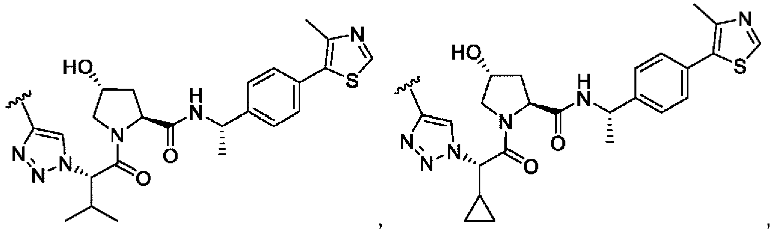

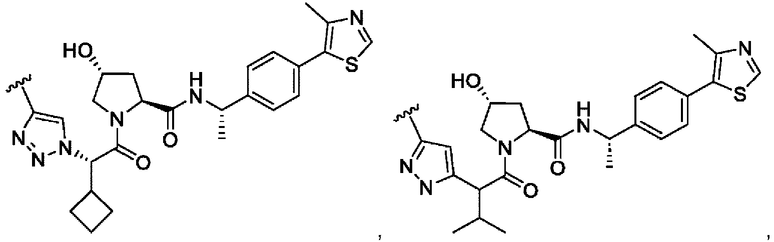

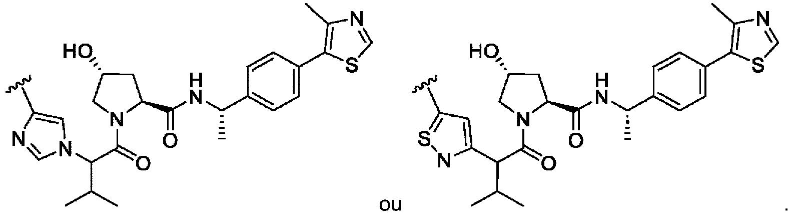

15. Composé selon l'une quelconque des revendications 1 à 14, ou sel pharmaceutiquement acceptable de celui-ci, dans lequel le lieur a la structure de

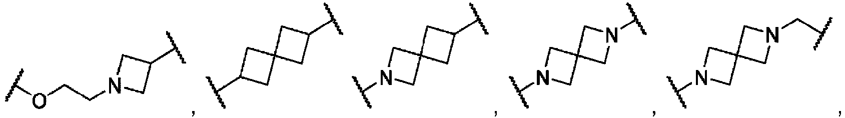

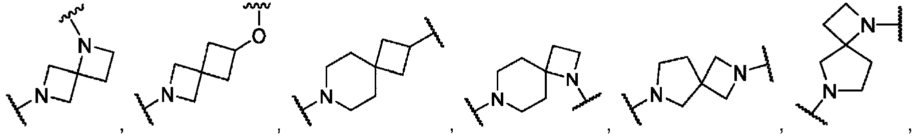

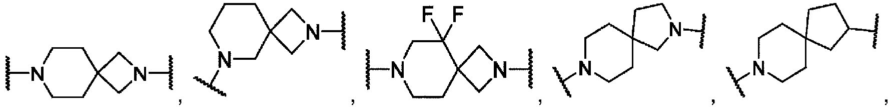

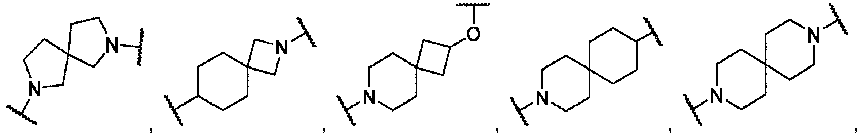

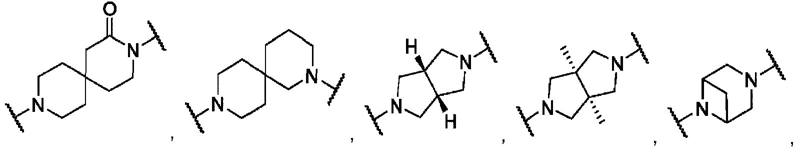

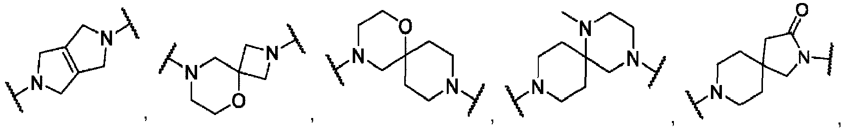

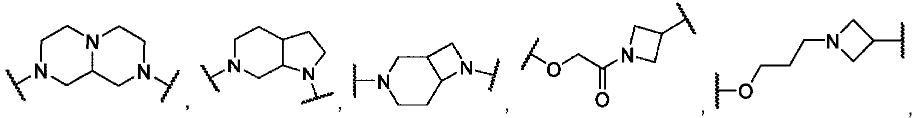

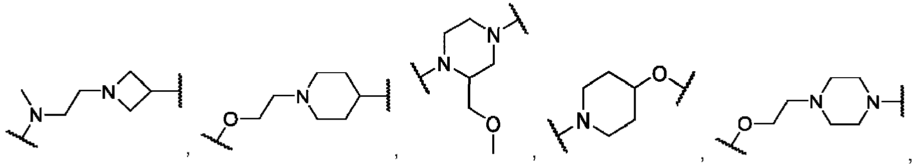

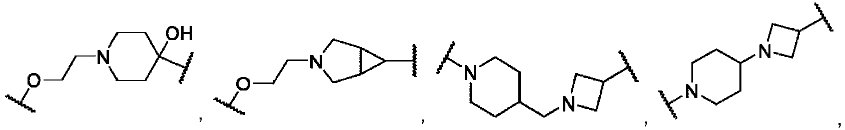

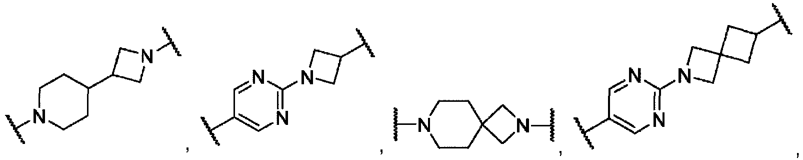

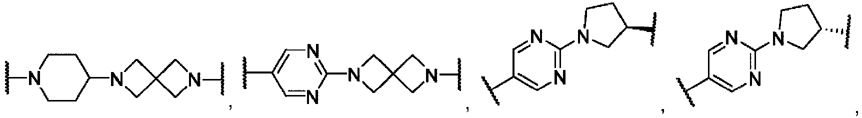

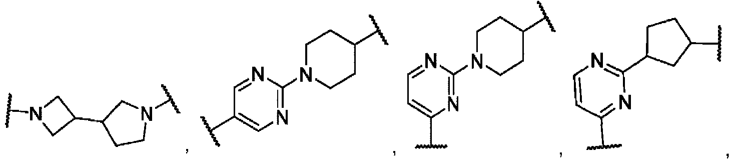

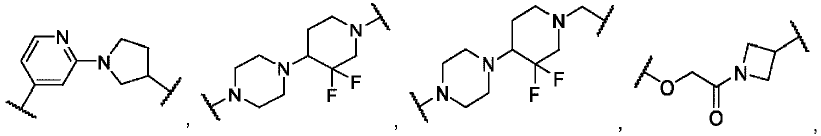

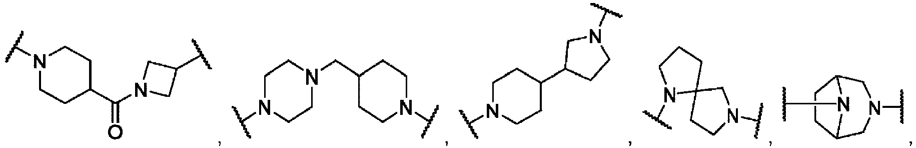

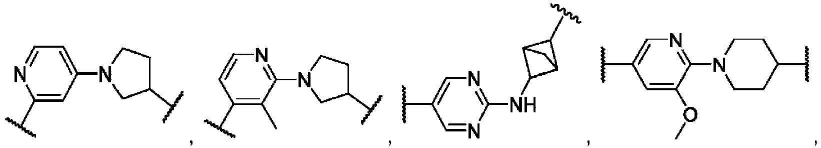

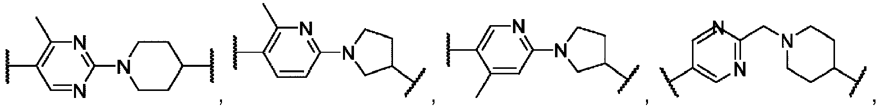

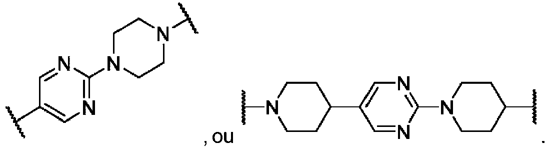

16. Composé selon l'une quelconque des revendications 1 à 14, ou sel pharmaceutiquement acceptable de celui-ci, dans lequel le lieur a la structure de

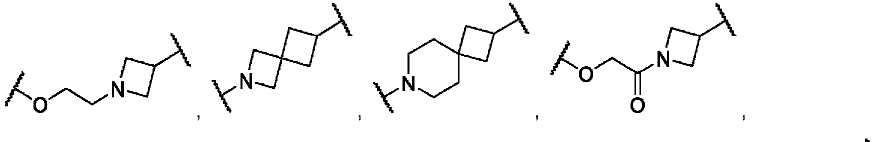

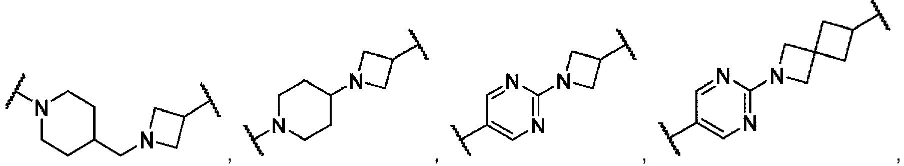

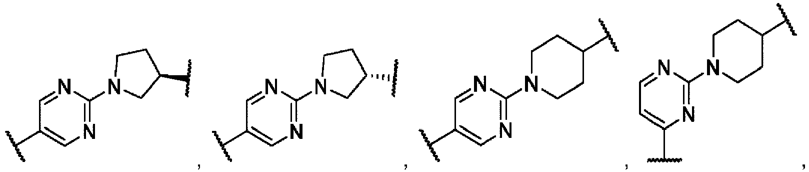

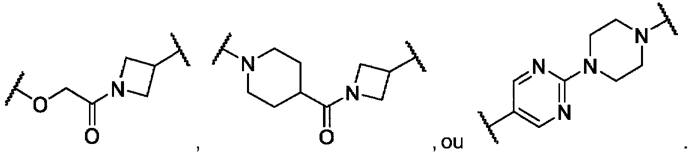

17. Composé selon l'une quelconque des revendications 1 à 14, ou sel pharmaceutiquement acceptable de celui-ci, dans lequel le lieur a la structure de

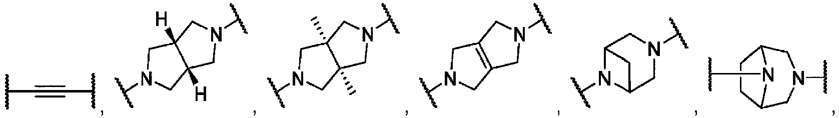

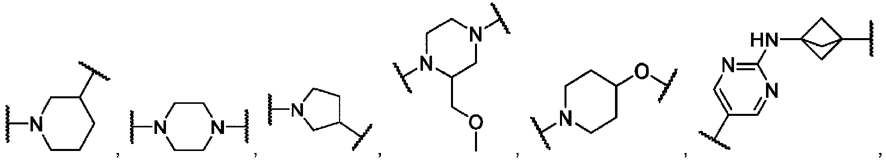

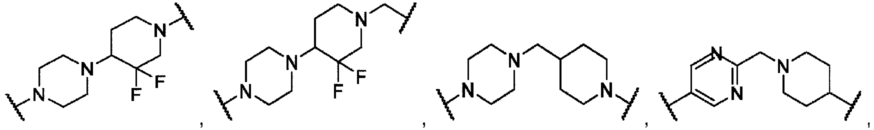

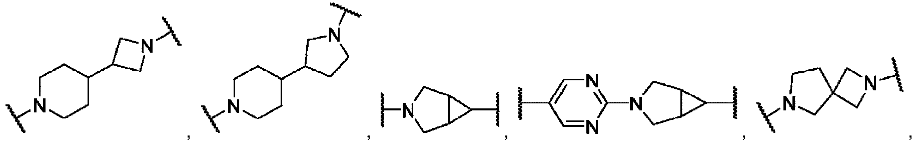

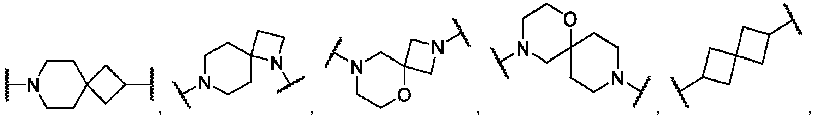

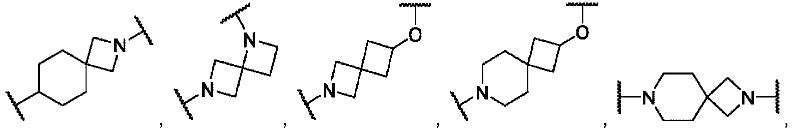

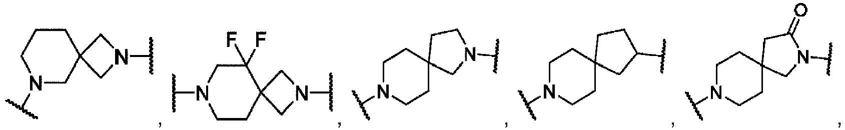

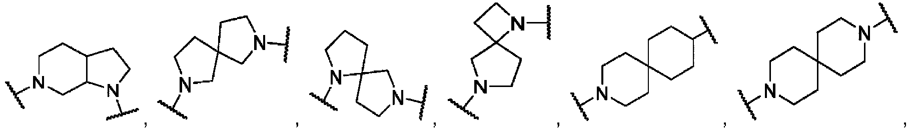

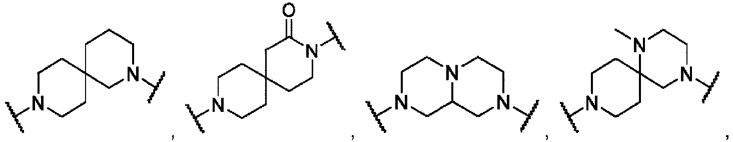

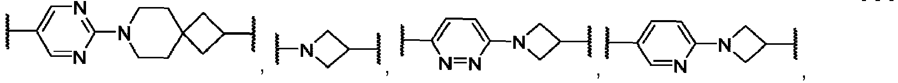

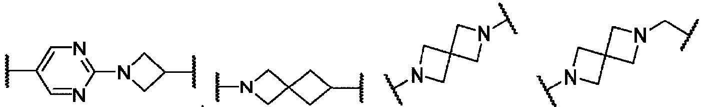

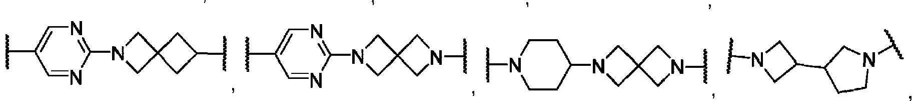

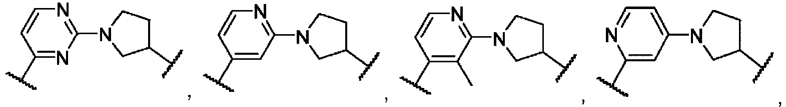

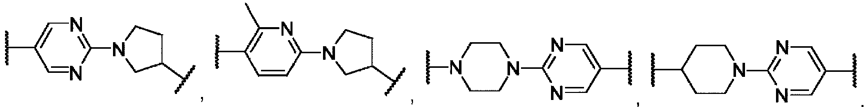

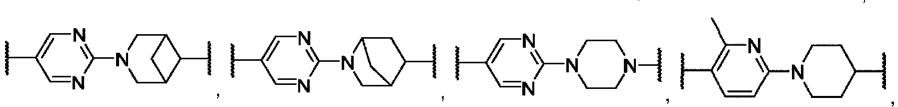

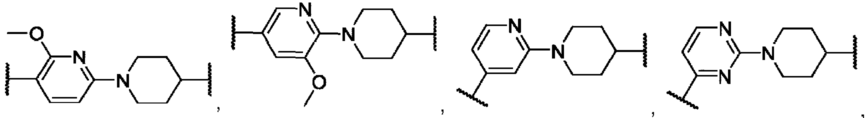

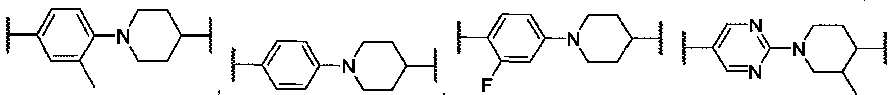

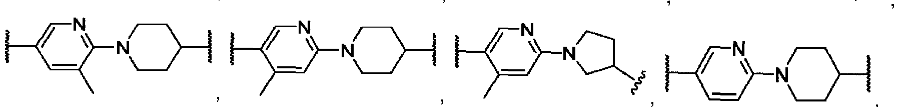

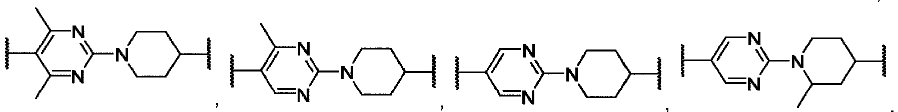

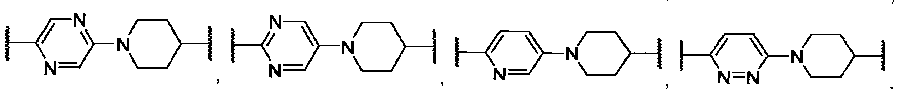

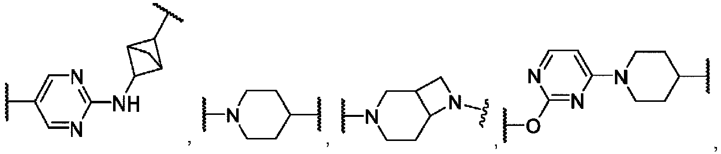

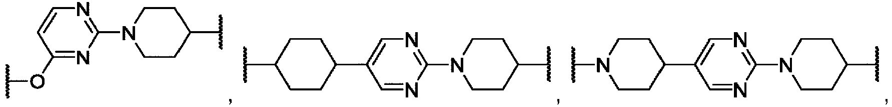

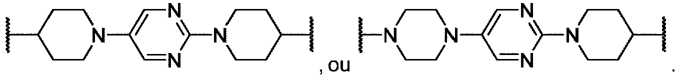

18. Composé choisi dans le groupe constitué des composés 1 à 291 dans le Tableau ci-dessous et de sels pharmaceutiquement acceptables de ceux-ci :
| **#** | **Composé** |
|---|---|
| 1 | 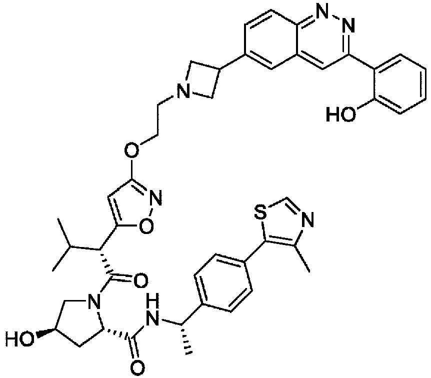 |
| 2 | 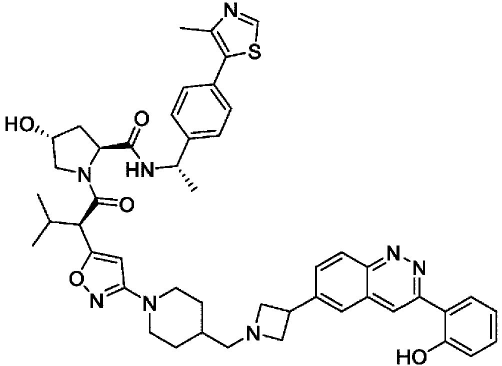 |
| 3 | 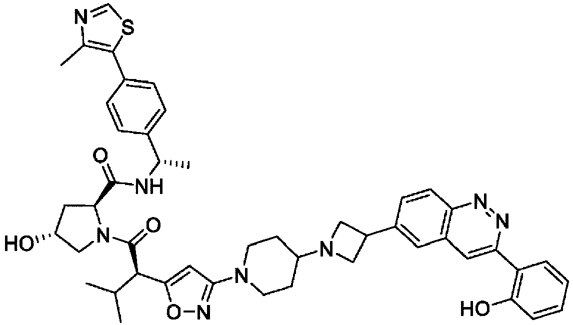 |
| 4 | 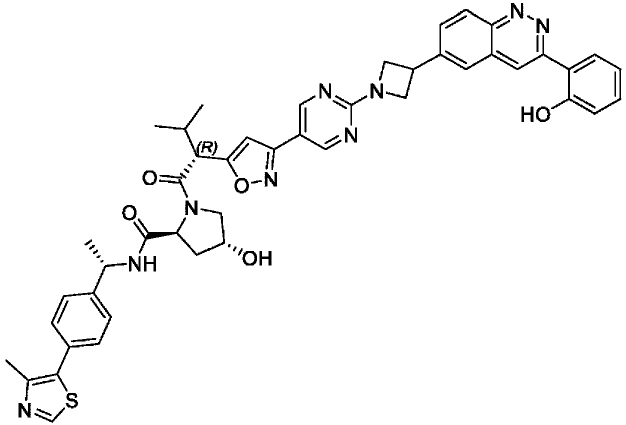 |
| 5 | 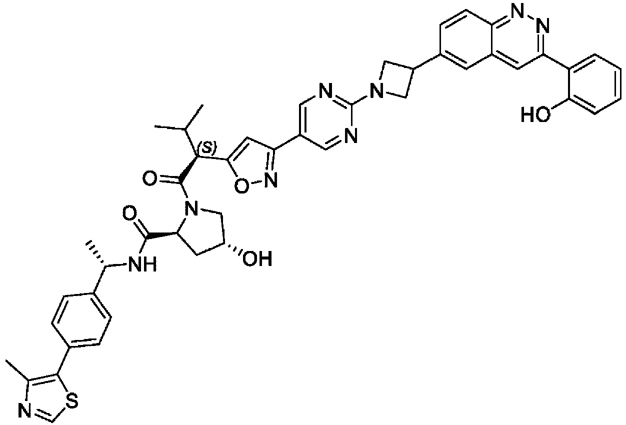 |
| 6 | 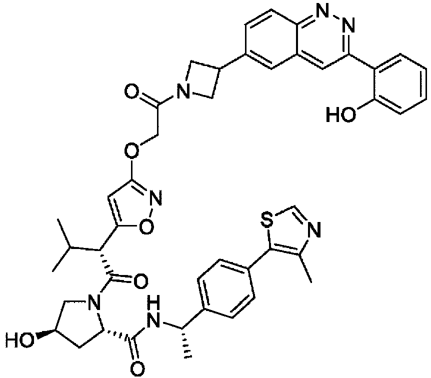 |
| 7 | 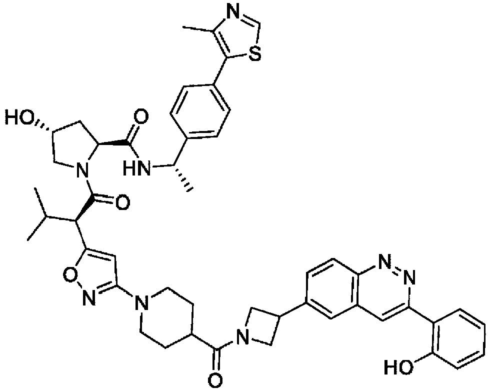 |
| 8 | 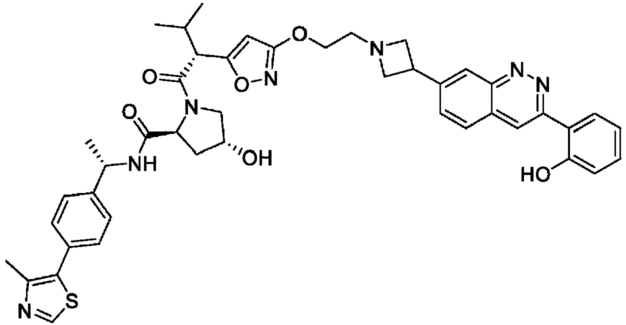 |
| 9 | 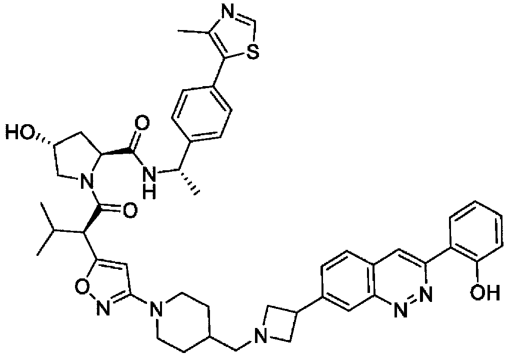 |
| 10 | 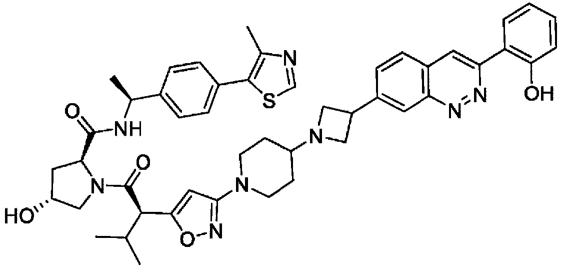 |
| 11 | 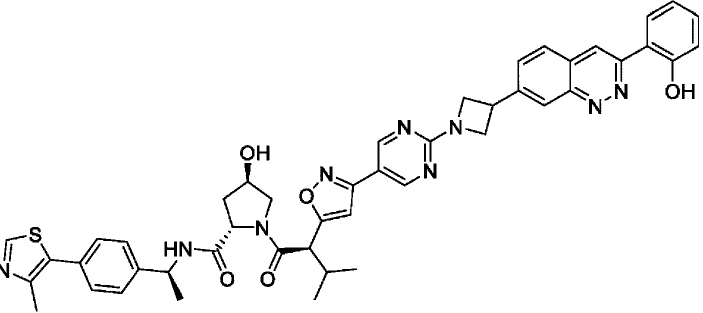 |
| 12 | 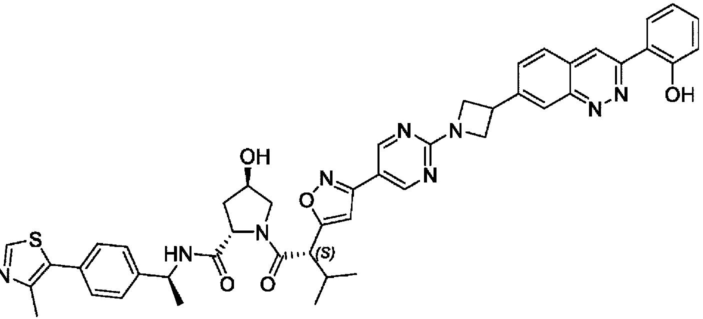 |
| 13 | 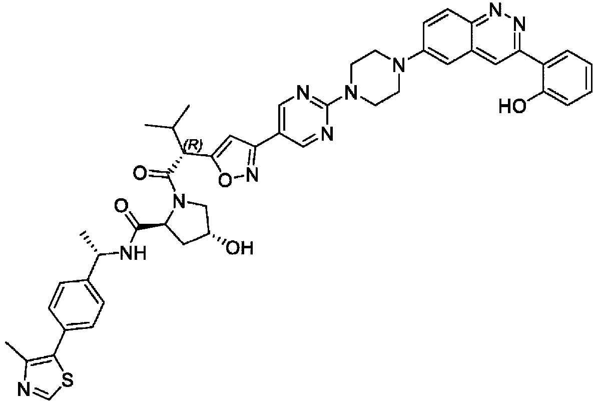 |
| 14 | 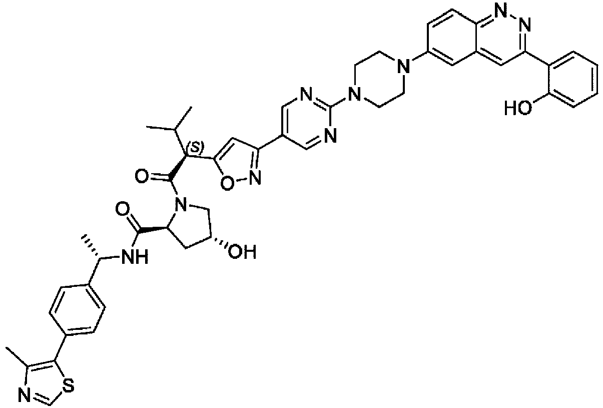 |
| 15 | 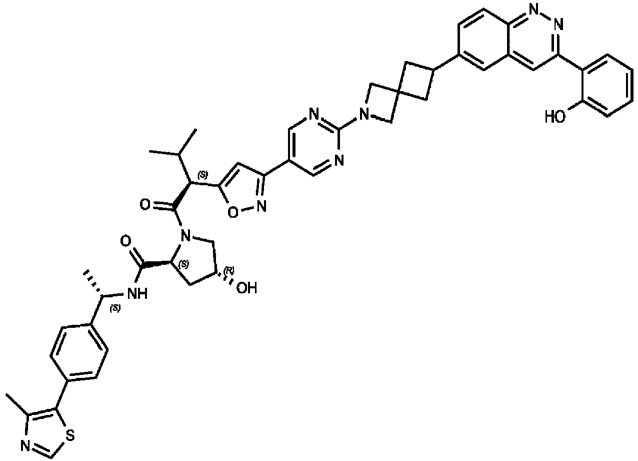 |
| 16 | 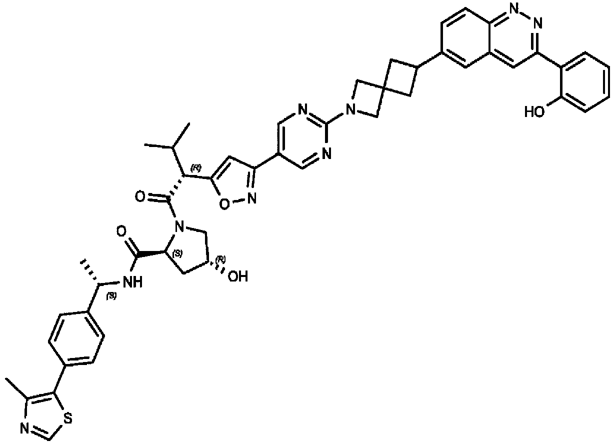 |
| 17 | 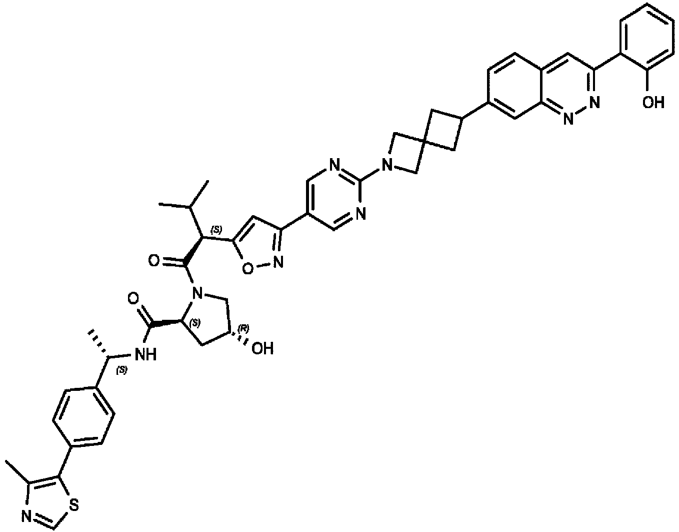 |
| 18 | 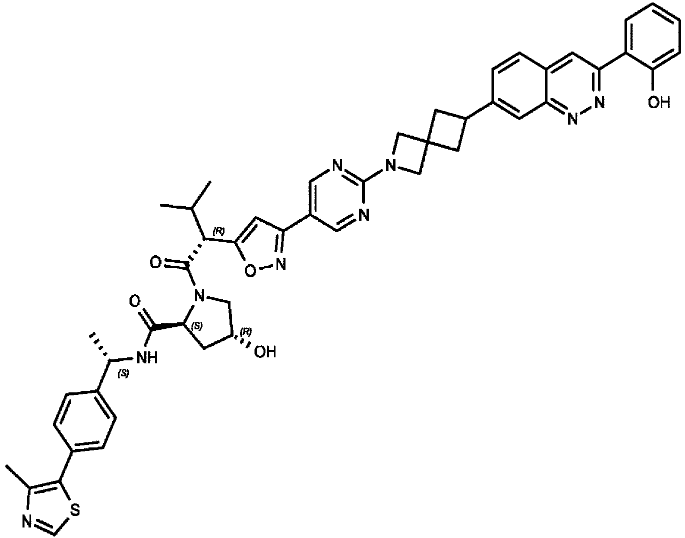 |
| 19 | 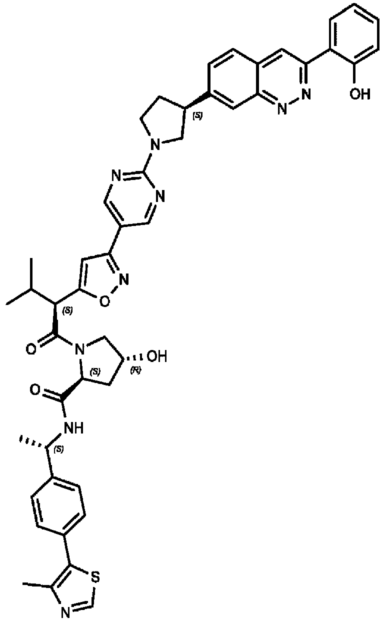 |
| 20 | 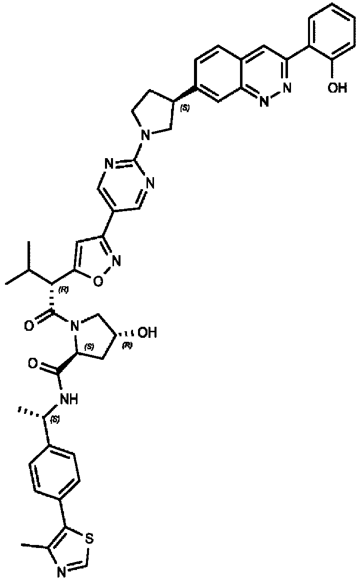 |
| 21 | 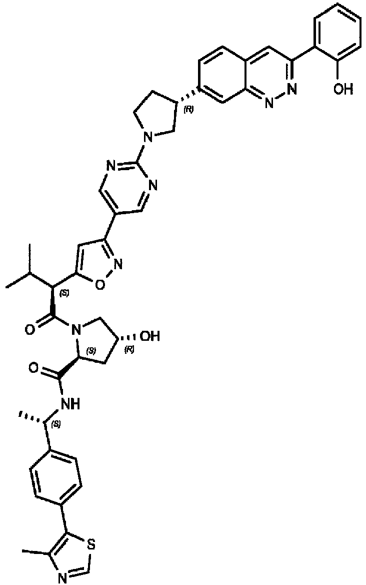 |
| 22 | 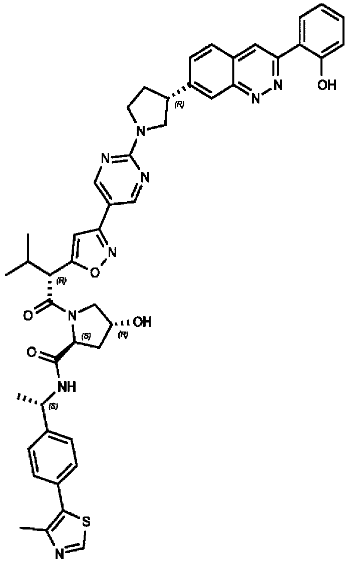 |
| 23 | 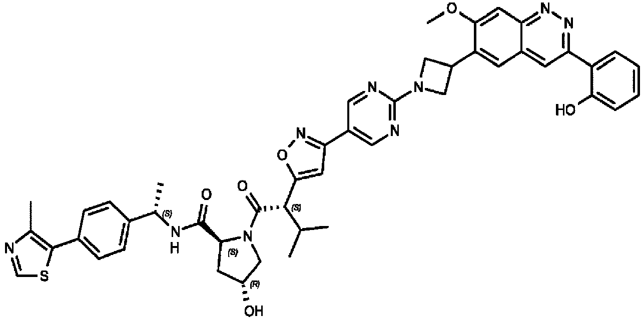 |
| 24 | 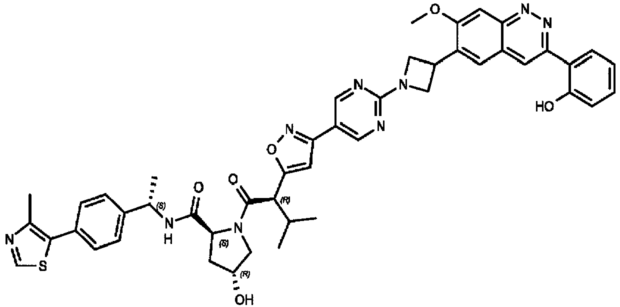 |
| 25 | 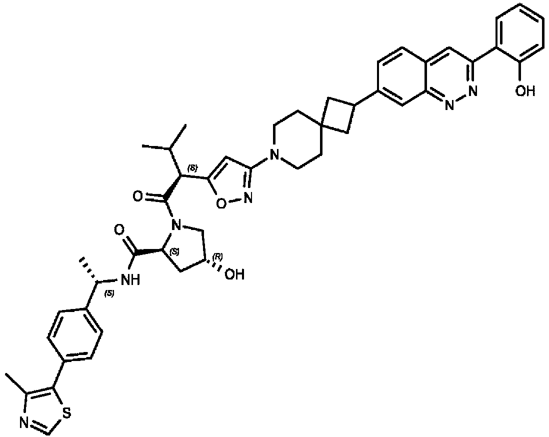 |
| 26 | 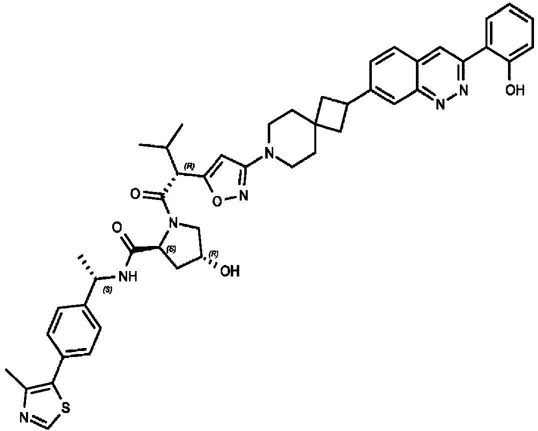 |
| 27 | 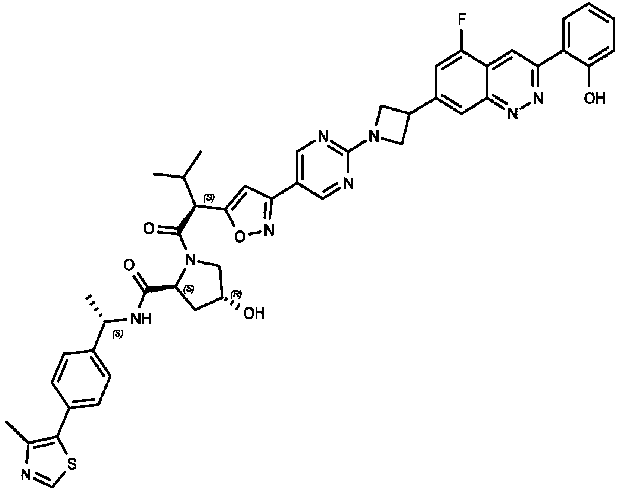 |
| 28 | 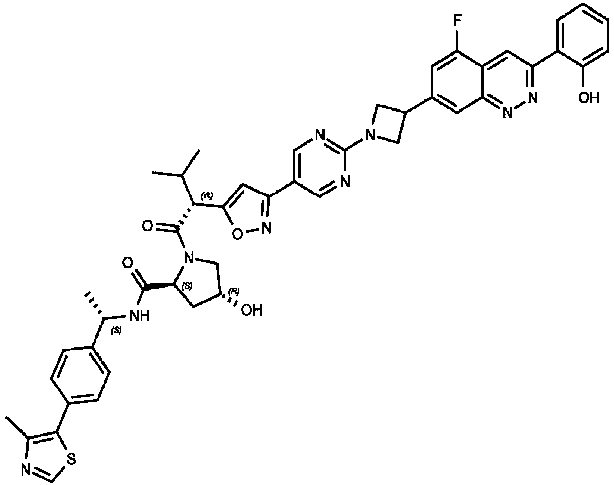 |
| 29 | 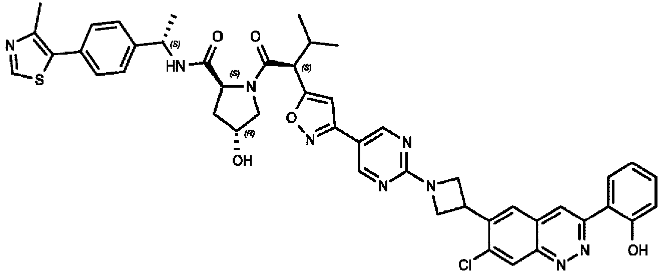 |
| 30 | 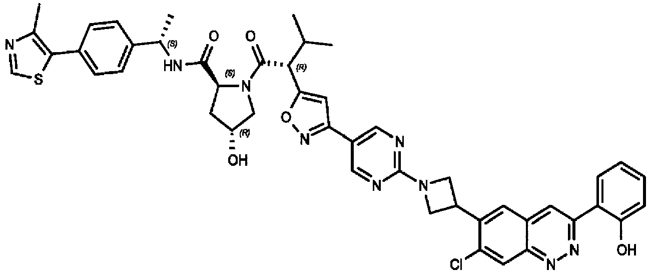 |
| 31 | 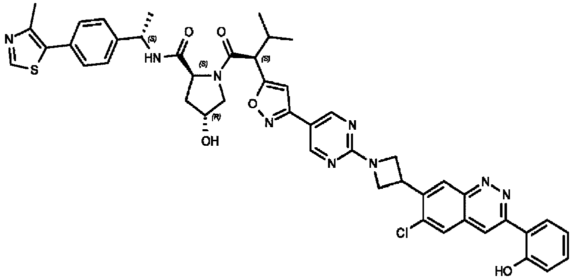 |
| 32 | 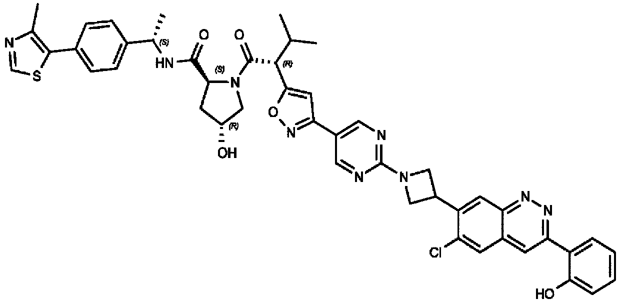 |
| 33 | 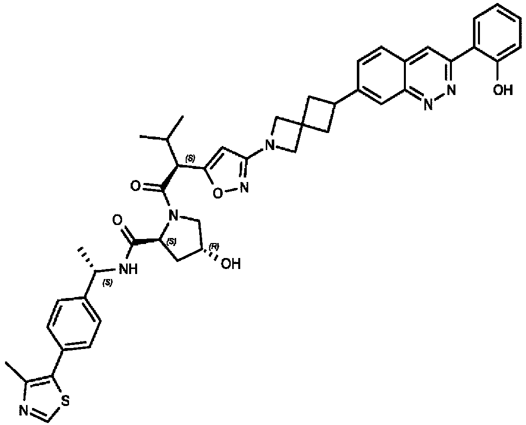 |
| 34 | 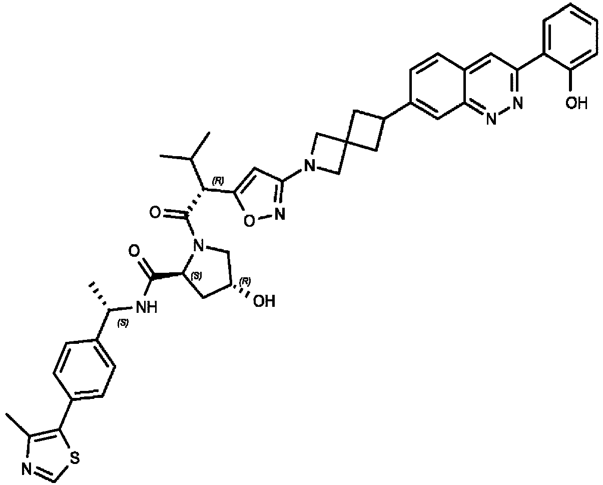 |
| 35 | 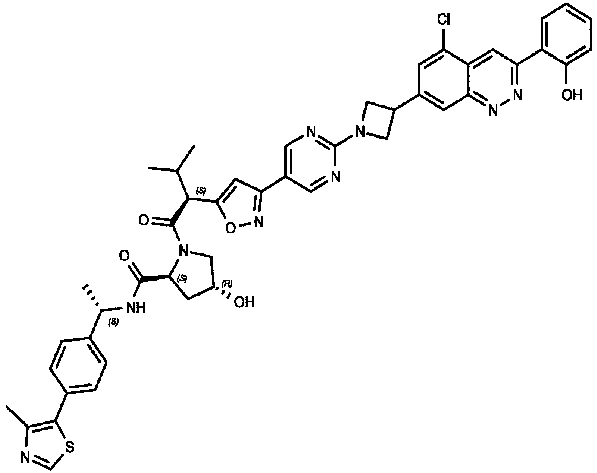 |
| 36 | 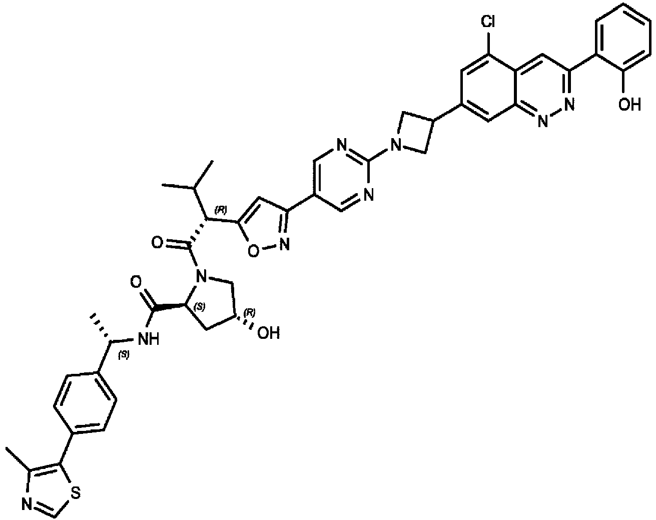 |
| 37 | 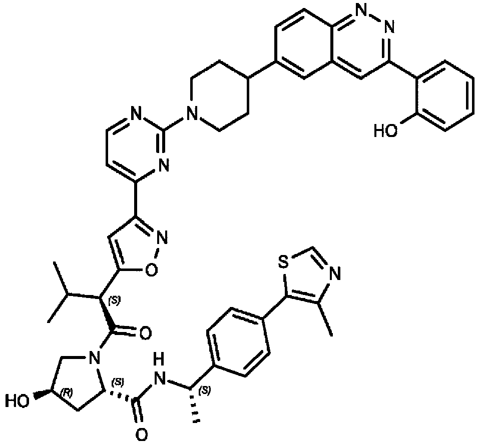 |
| 38 | 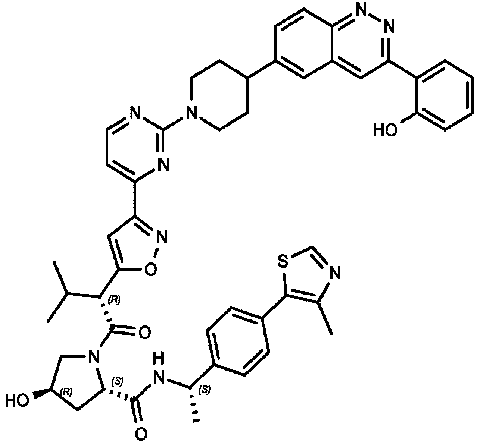 |
| 39 | 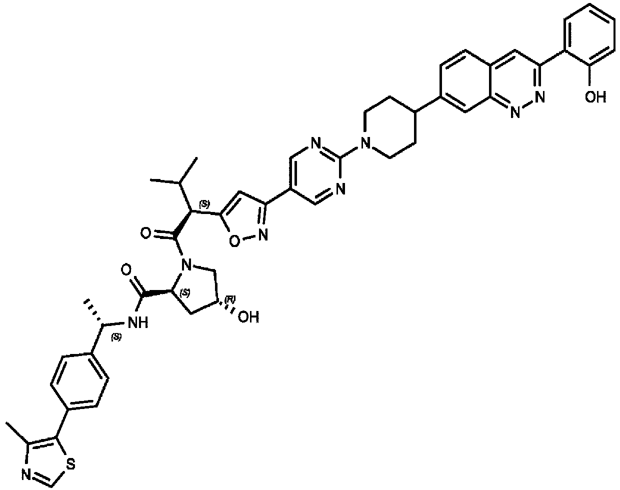 |
| 40 | 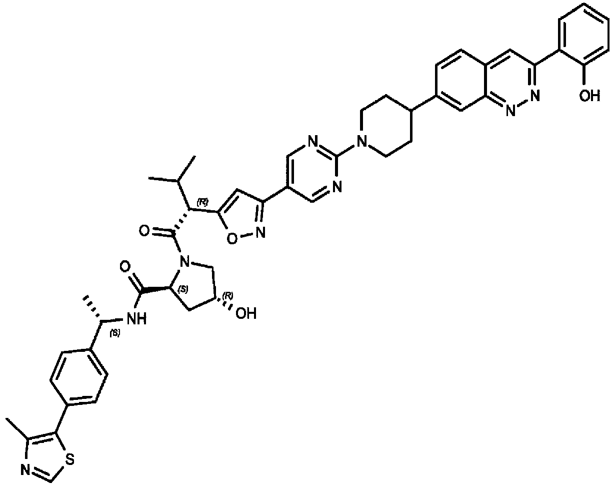 |
| 41 | 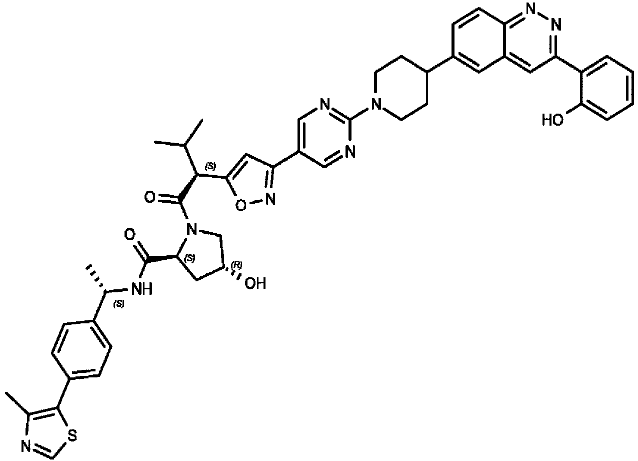 |
| 42 | 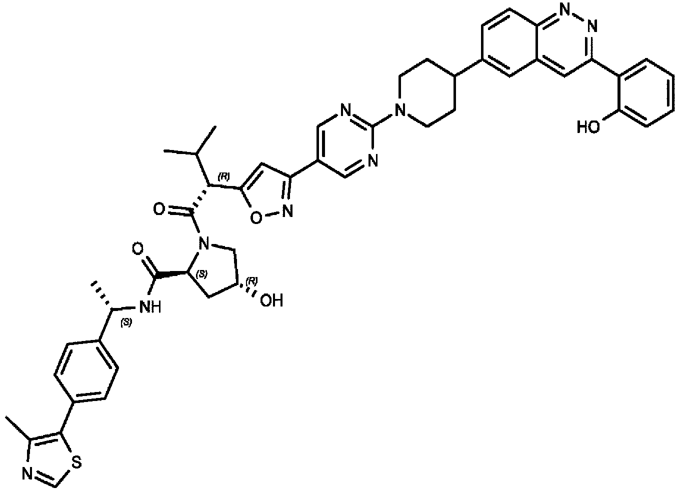 |
| 43 | 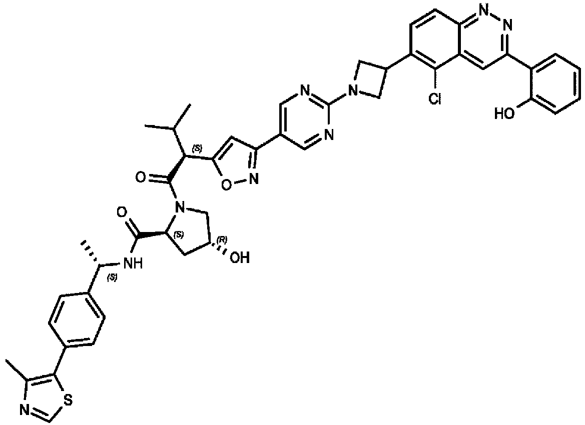 |
| 44 | 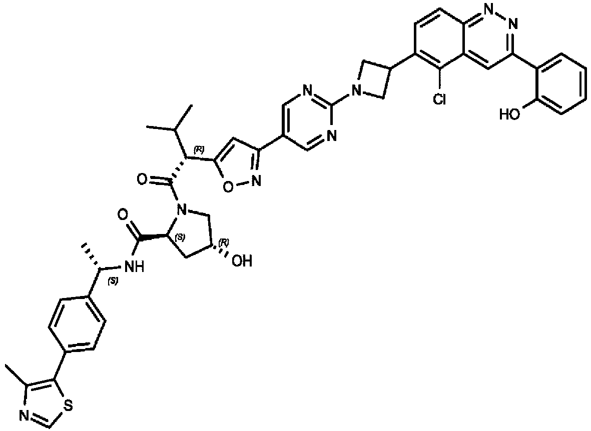 |
| 45 | 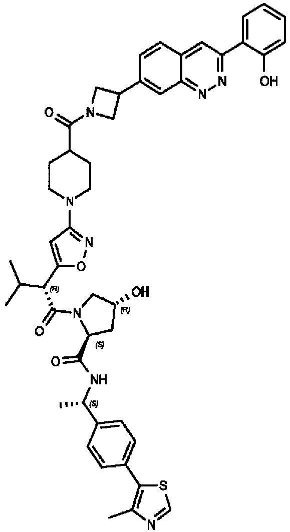 |
| 46 | 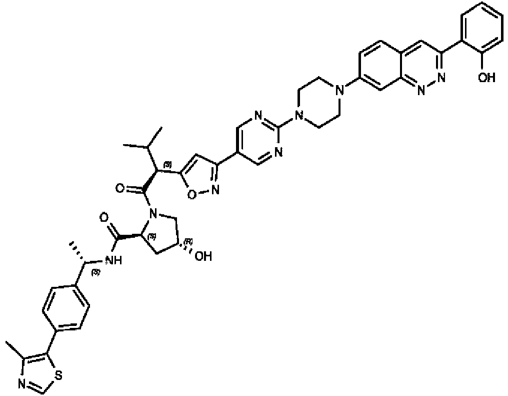 |
| 47 | 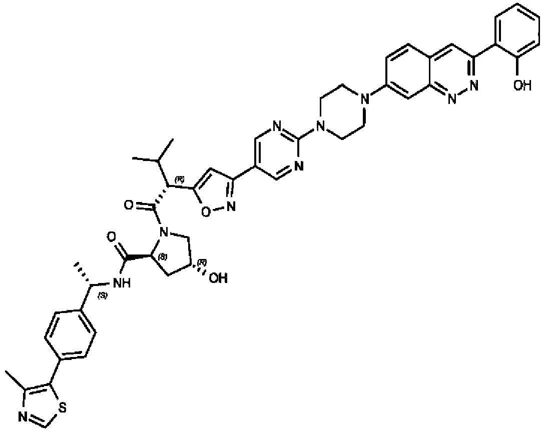 |
| 48 | 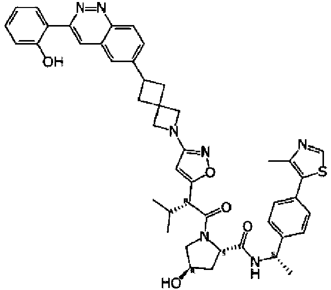 |
| 49 | 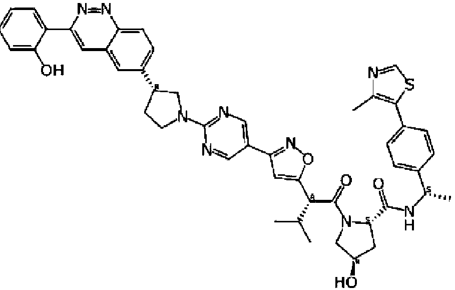 |
| 50 | 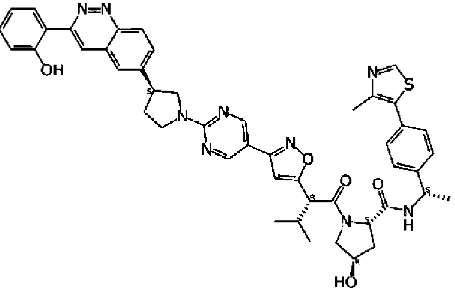 |
| 51 | 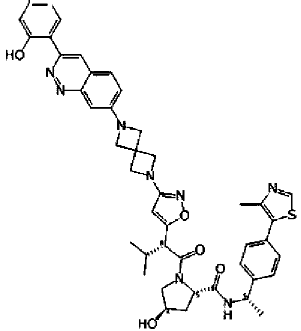 |
| 52 | 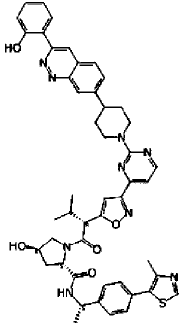 |
| 53 | 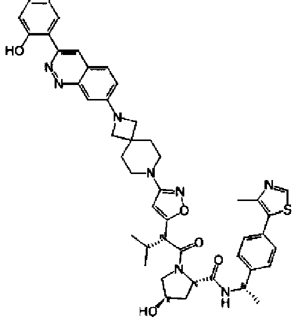 |
| 54 | 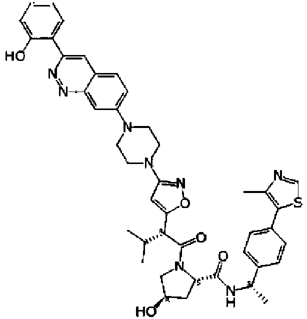 |
| 55 | 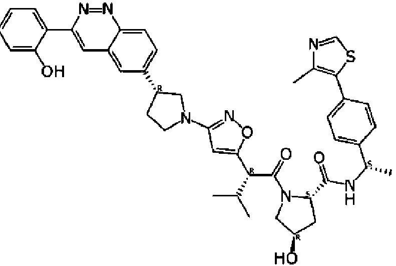 |
| 56 | 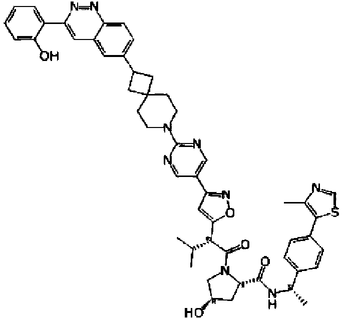 |
| 57 | 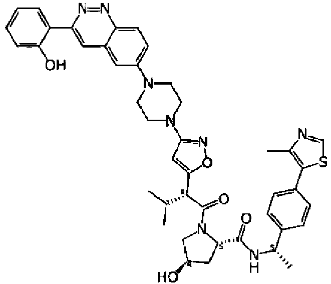 |
| 58 | 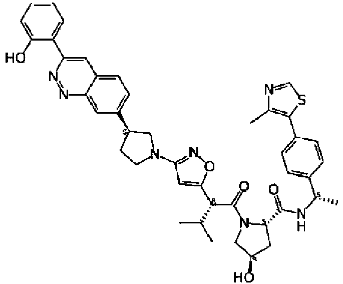 |
| 59 | 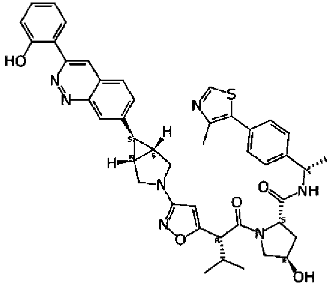 |
| 60 | 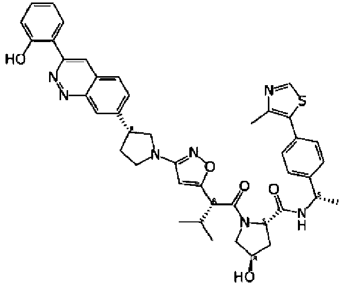 |
| 61 | 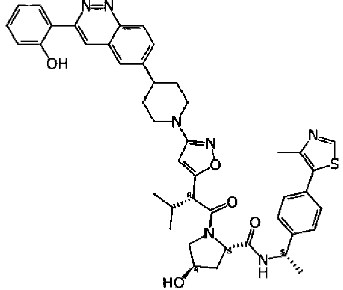 |
| 62 | 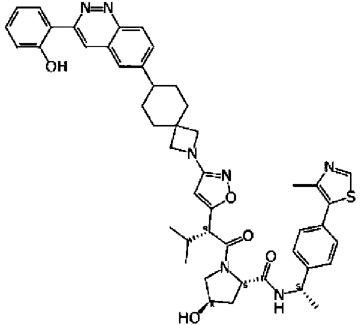 |
| 63 | 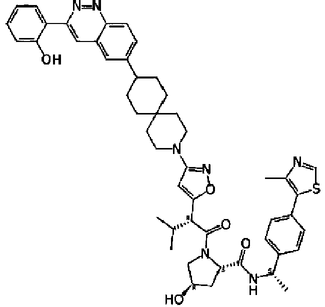 |
| 64 | 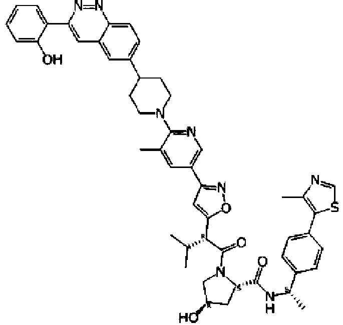 |
| 65 | 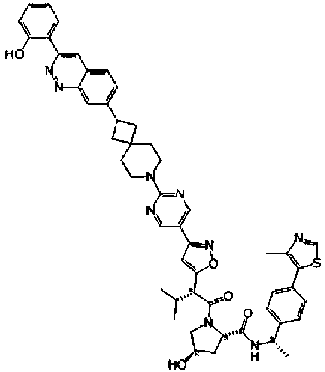 |
| 66 | 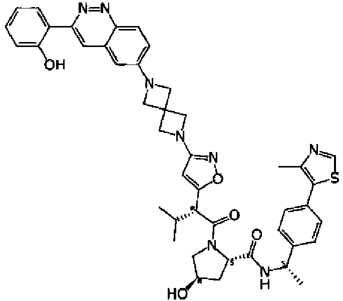 |
| 67 | 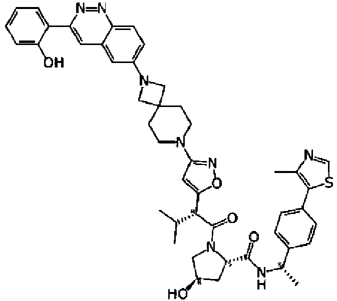 |
| 68 | 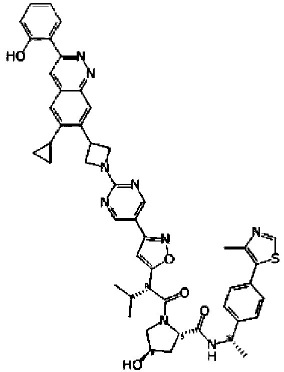 |
| 69 | 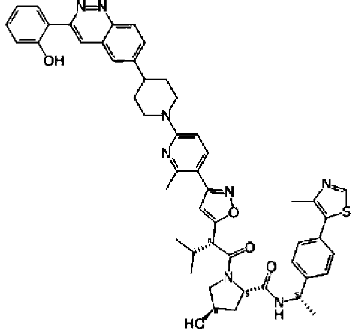 |
| 70 | 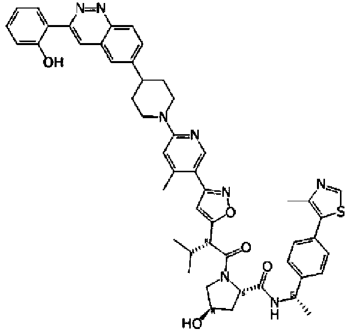 |
| 71 | 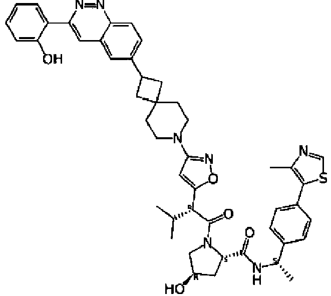 |
| 72 | 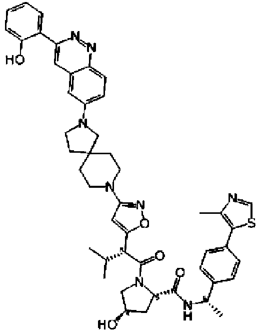 |
| 73 | 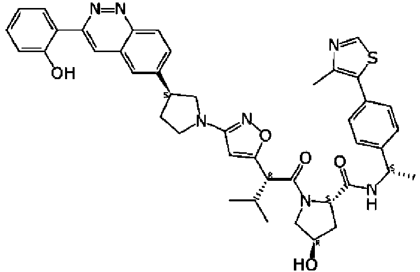 |
| 74 | 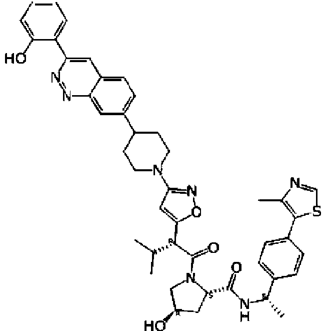 |
| 75 | 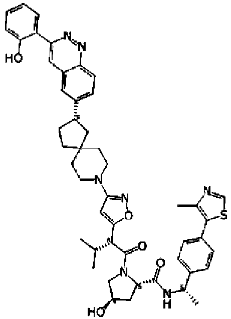 |
| 76 | 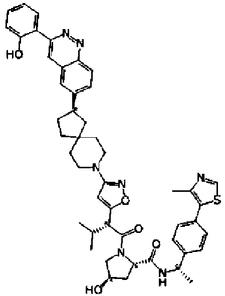 |
| 77 | 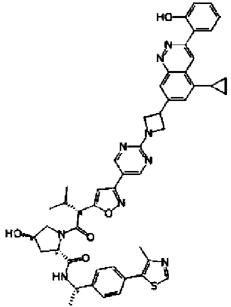 |
| 78 | 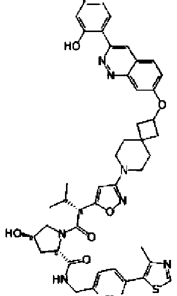 |
| | |
| 79 | 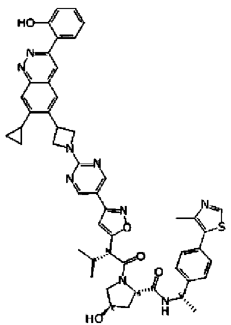 |
| 80 | 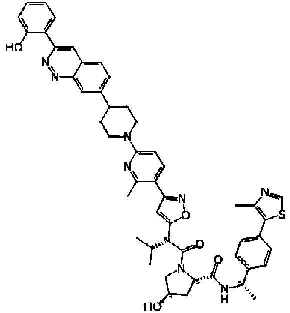 |
| 81 | 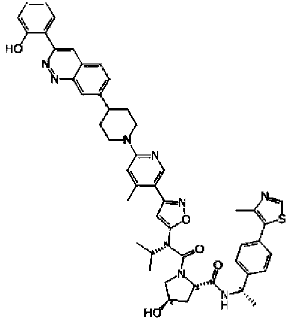 |
| 82 | 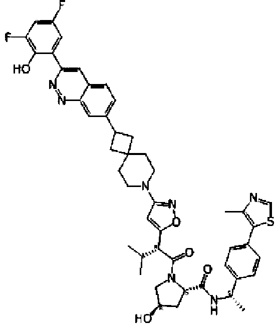 |
| 83 | 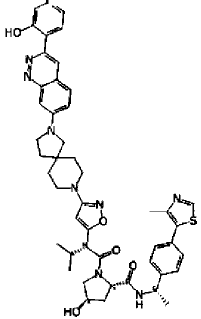 |
| 84 | 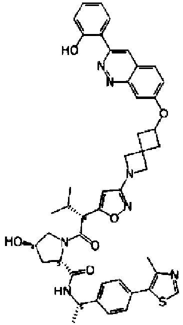 |
| 85 | 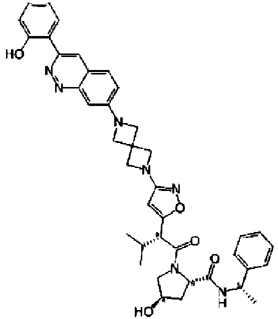 |
| 86 | 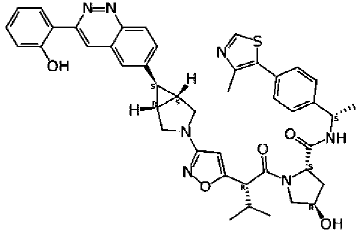 |
| 87 | 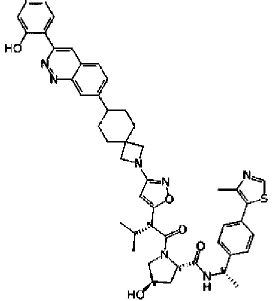 |
| 88 | 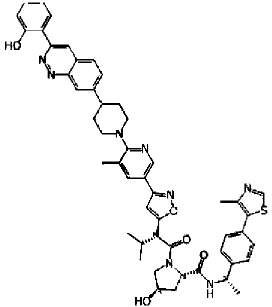 |
| 89 | 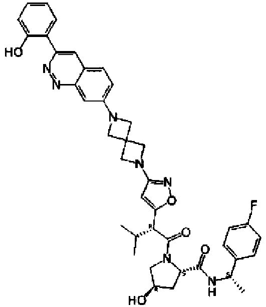 |
| 90 | 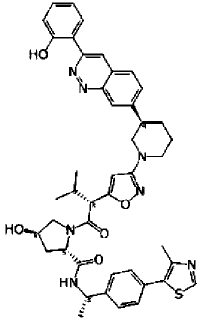 |
| 91 | 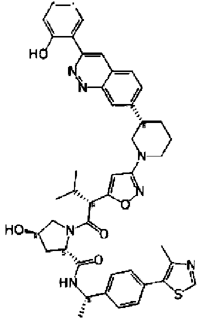 |
| 92 | 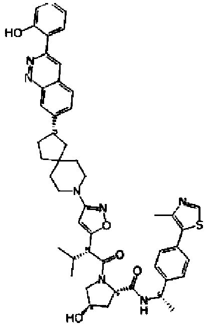 |
| 93 | 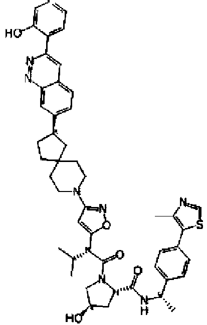 |
| 94 | 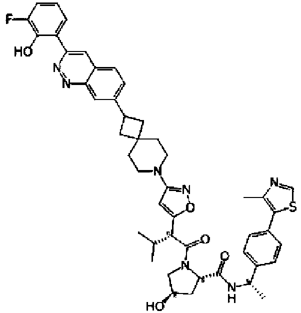 |
| 95 | 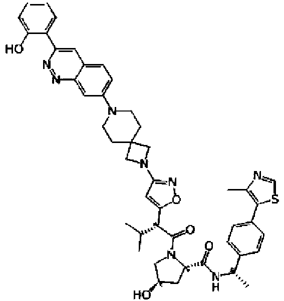 |
| 96 | 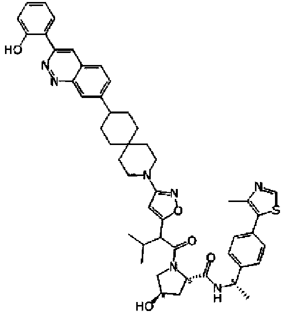 |
| 97 | 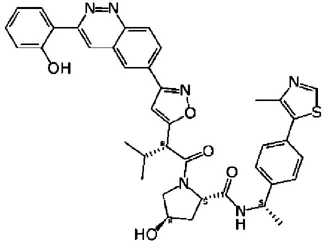 |
| 98 | 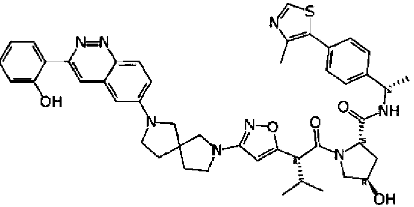 |
| 99 | 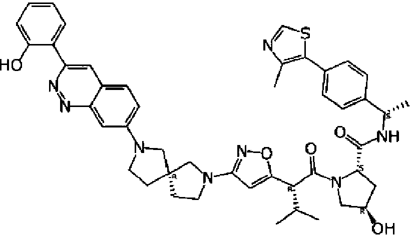 |
| 100 | 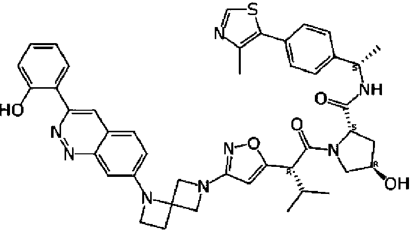 |
| 101 | 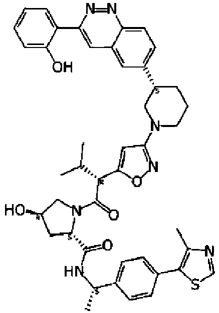 |
| 102 | 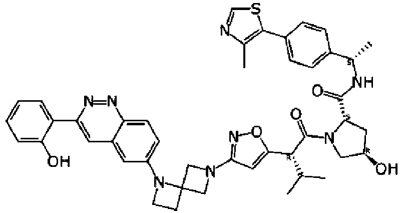 |
| 103 | 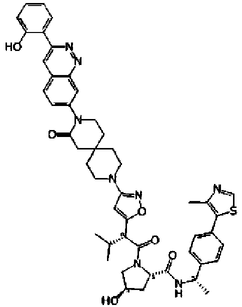 |
| 104 | 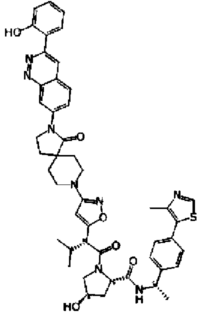 |
| 105 | 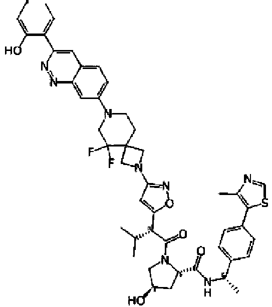 |
| 106 | 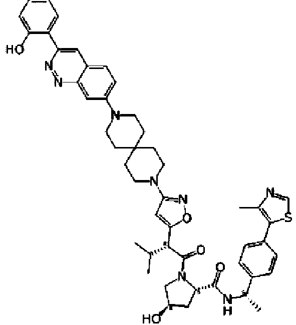 |
| 107 | 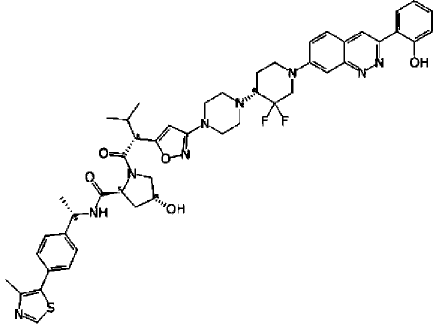 |
| 108 | 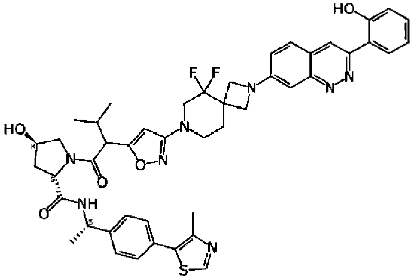 |
| 109 | 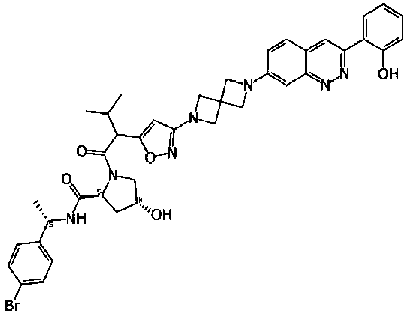 |
| 110 | 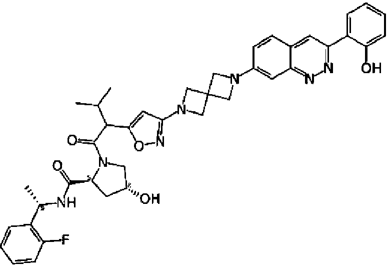 |
| 111 | 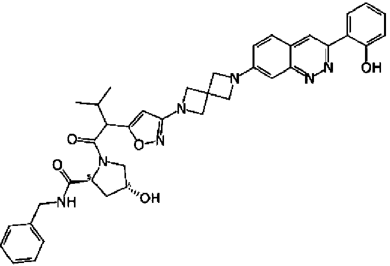 |
| 112 | 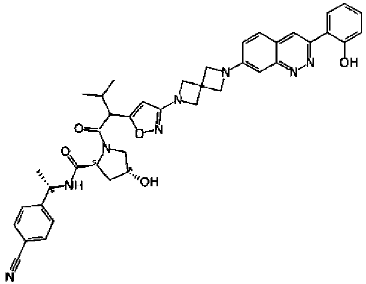 |
| 113 | 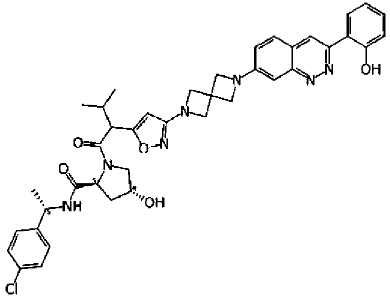 |
| 114 | 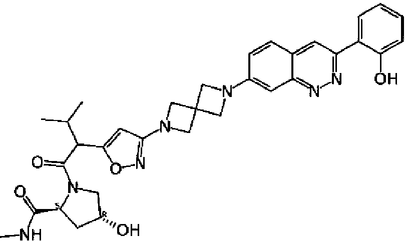 |
| 115 | 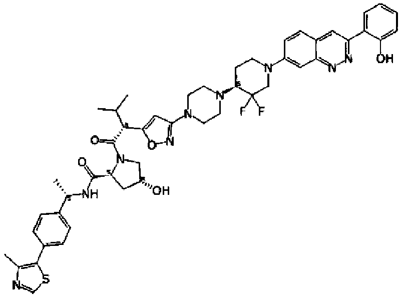 |
| 116 | 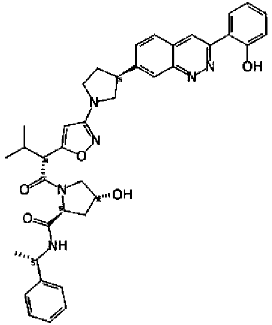 |
| 117 | 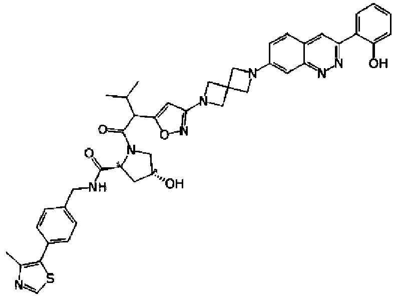 |
| 118 | 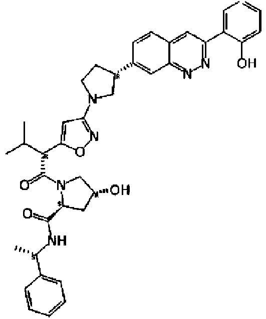 |
| 119 | 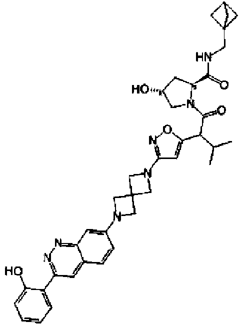 |
| 120 | 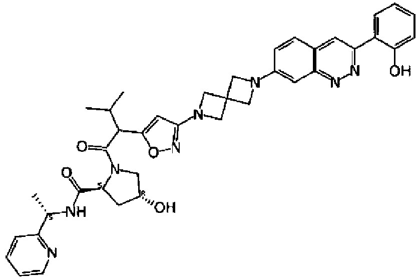 |
| 121 | 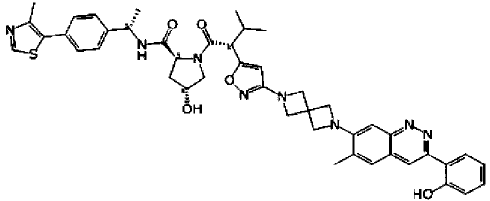 |
| 122 | 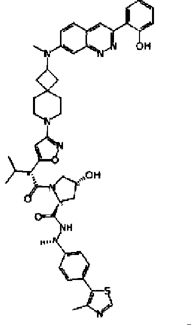 |
| 123 | 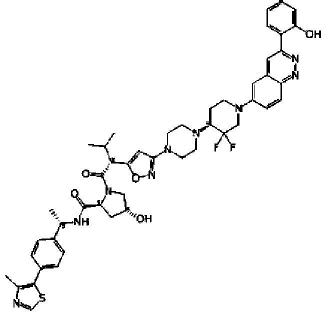 |
| 124 | 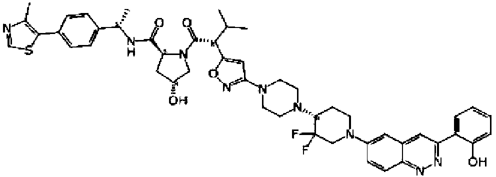 |
| 125 | 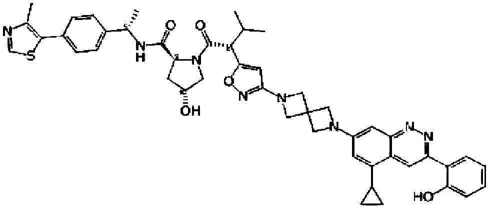 |
| 126 | 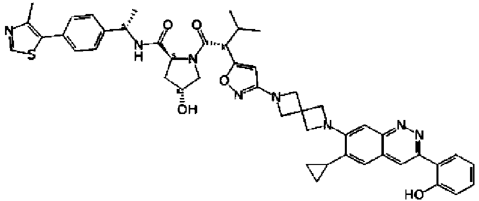 |
| 127 | 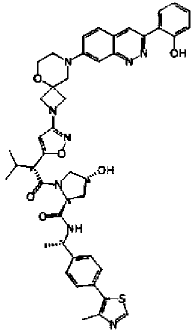 |
| 128 | 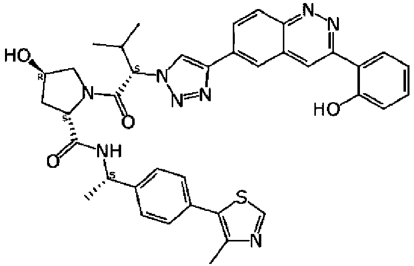 |
| 129 | 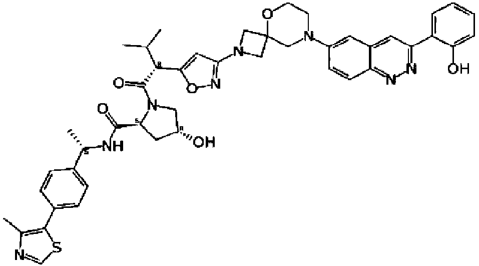 |
| 130 | 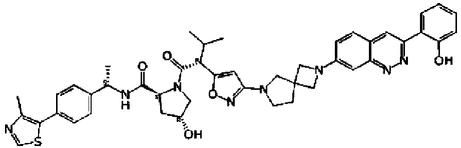 |
| 131 | 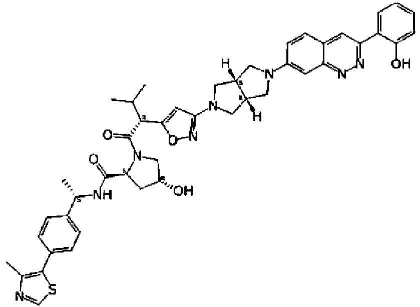 |
| 132 | 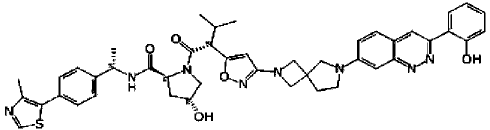 |
| 133 | 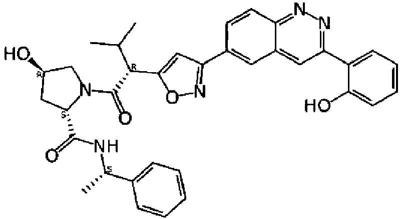 |
| 134 | 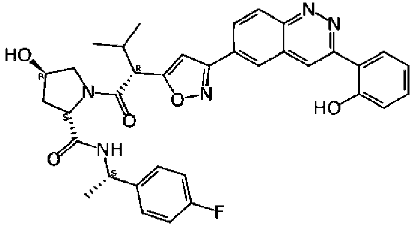 |
| 135 | 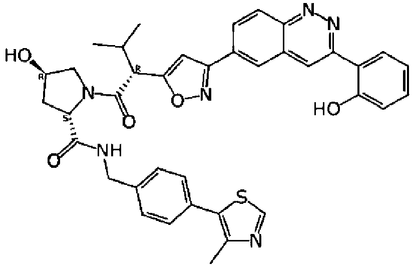 |
| 136 | 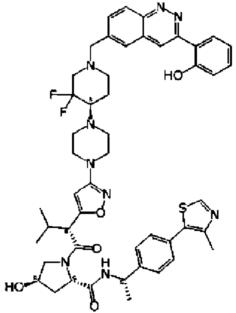 |
| 137 | 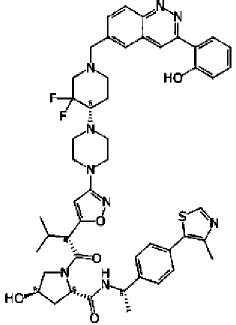 |
| 138 | 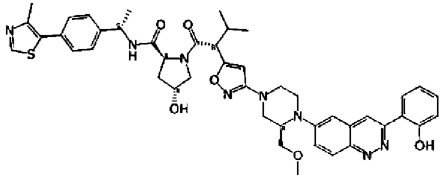 |
| 139 | 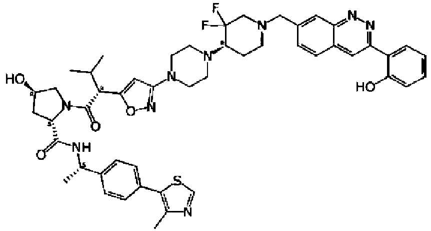 |
| 140 | 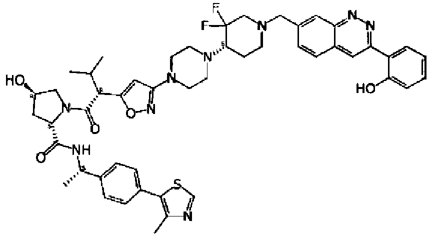 |
| 141 | 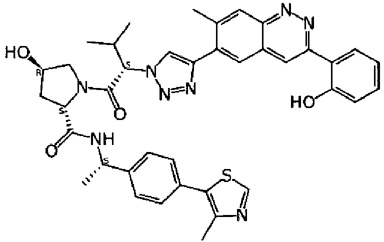 |
| 142 | 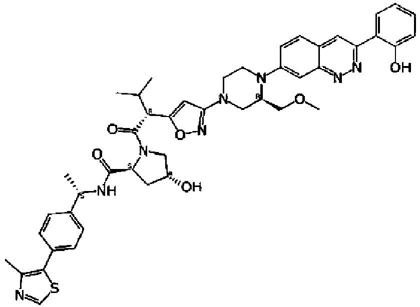 |
| 143 | 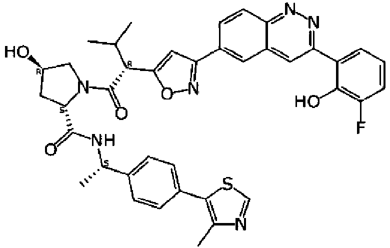 |
| 144 | 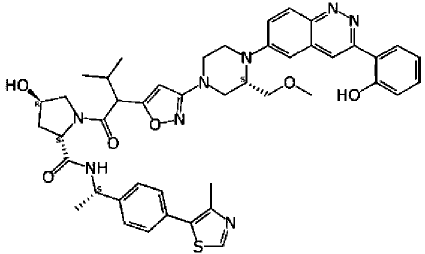 |
| 145 | 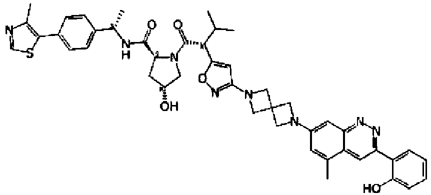 |
| 146 | 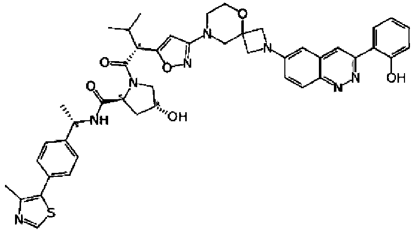 |
| 147 | 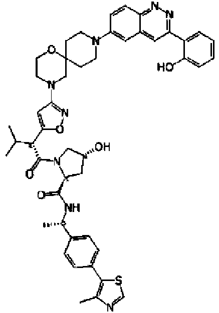 |
| 148 | 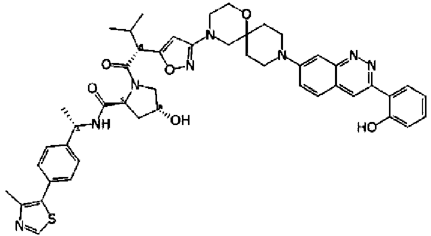 |
| 149 | 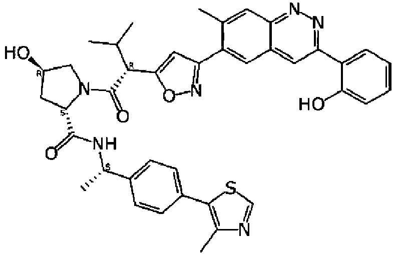 |
| 150 | 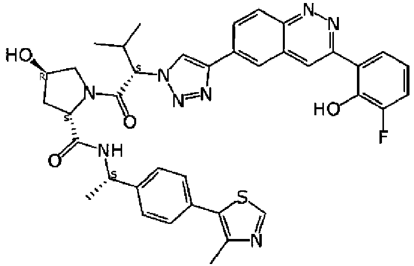 |
| 151 | 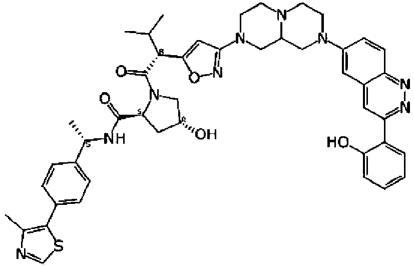 |
| 152 | 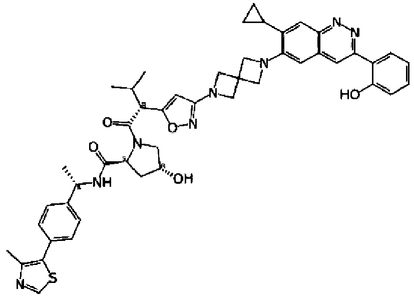 |
| 153 | 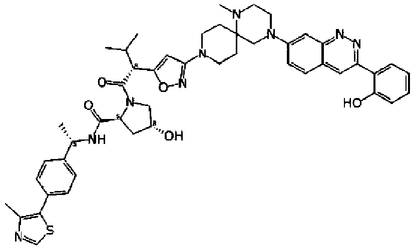 |
| 154 | 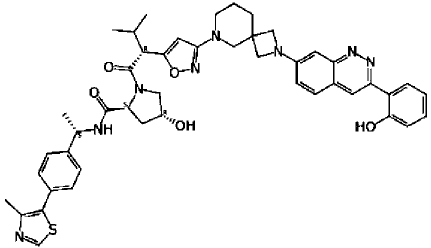 |
| 155 | 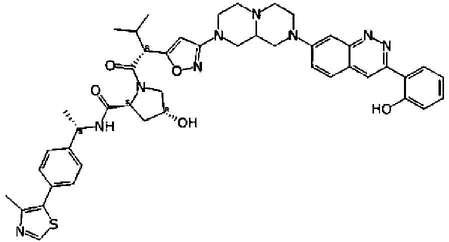 |
| 156 | 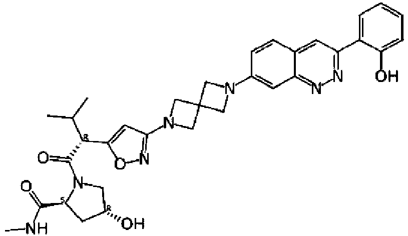 |
| 157 | 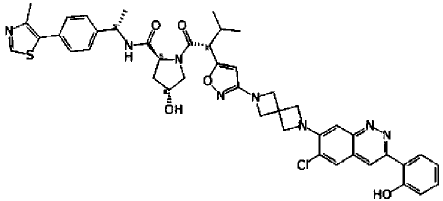 |
| 158 | 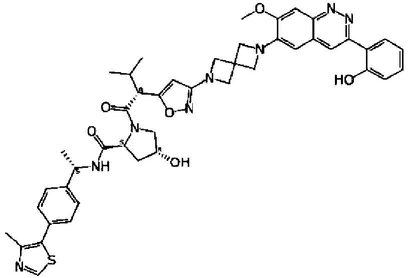 |
| 159 | 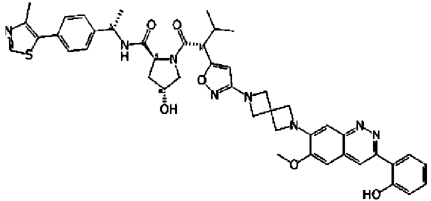 |
| 160 | 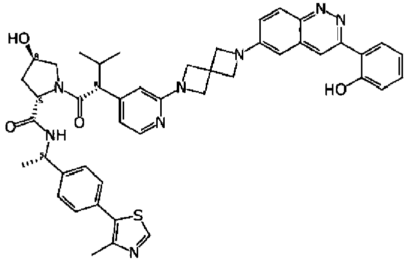 |
| 161 | 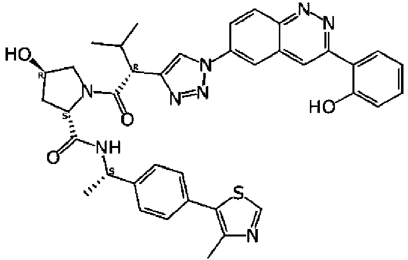 |
| 162 | 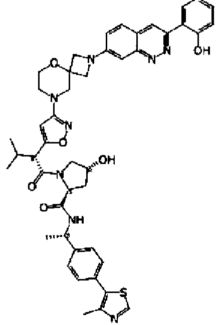 |
| 163 | 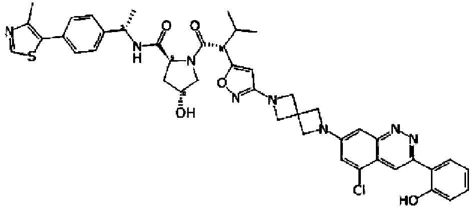 |
| 164 | 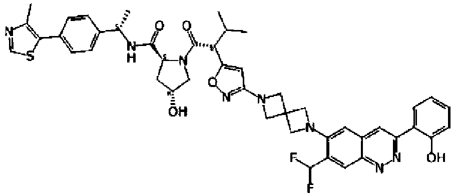 |
| 165 | 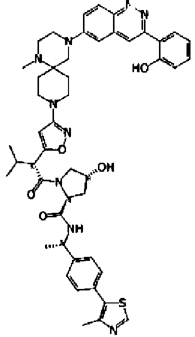 |
| 166 | 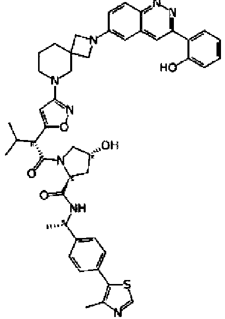 |
| 167 | 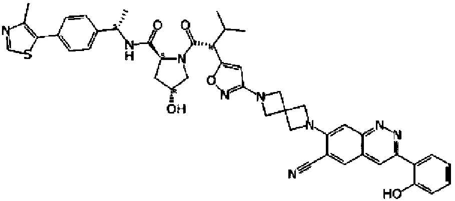 |
| 168 | 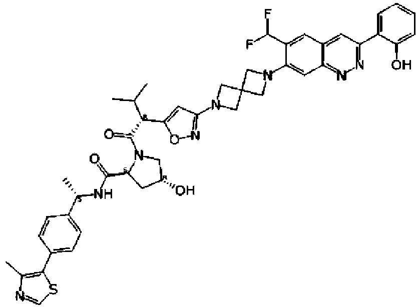 |
| 169 | 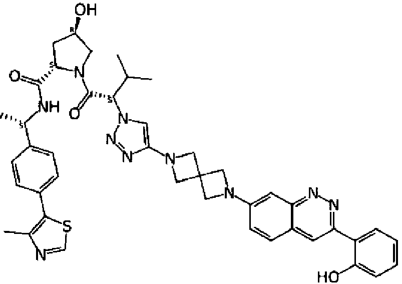 |
| 170 | 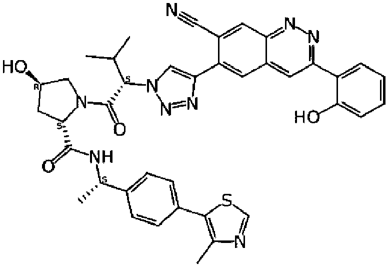 |
| 171 | 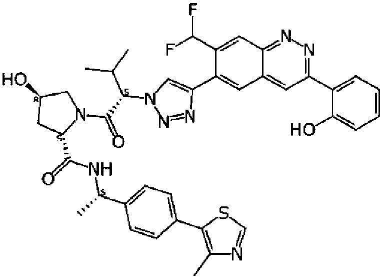 |
| 172 | 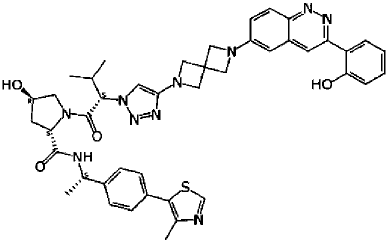 |
| 173 | 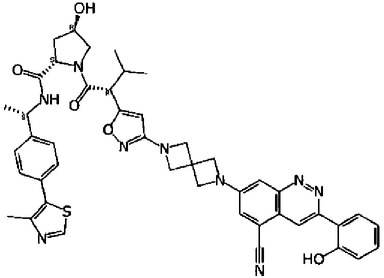 |
| 174 | 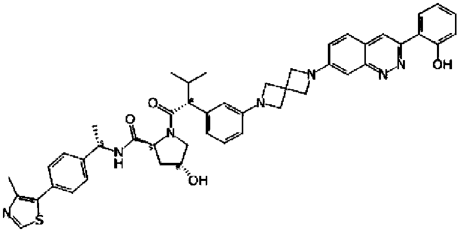 |
| 175 | 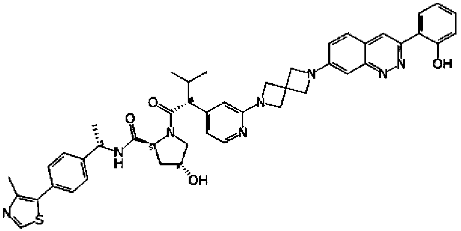 |
| 176 | 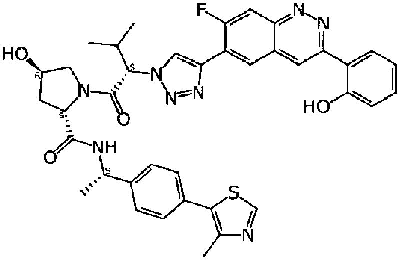 |
| 177 | 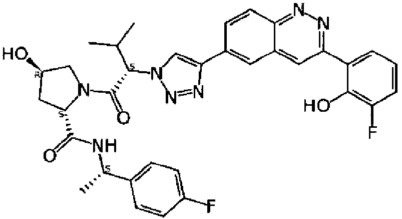 |
| 178 | 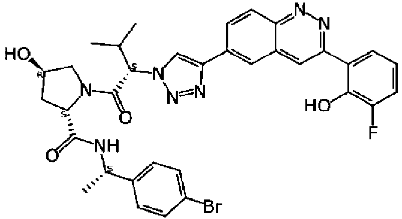 |
| 179 | 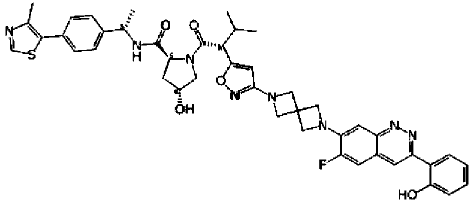 |
| 180 | 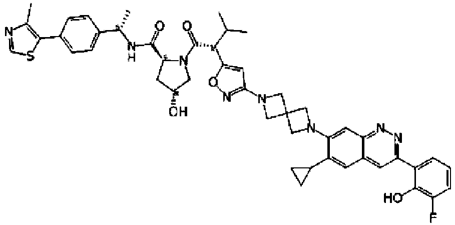 |
| 181 | 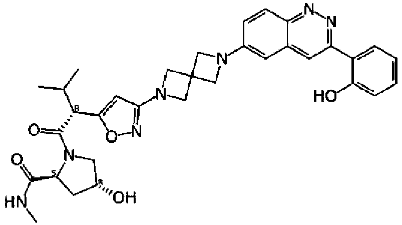 |
| 182 | 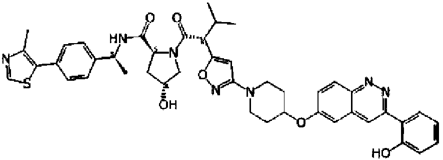 |
| 183 | 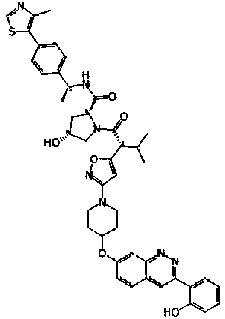 |
| 184 | 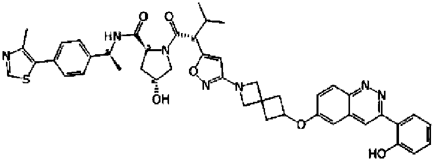 |
| 185 | 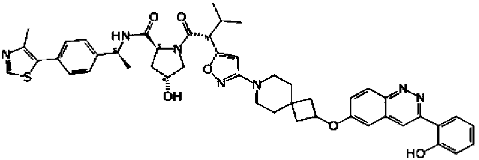 |
| 186 | 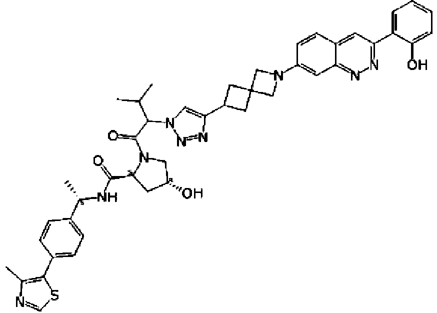 |
| 187 | 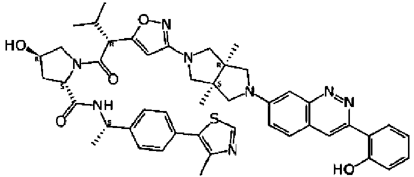 |
| 188 | 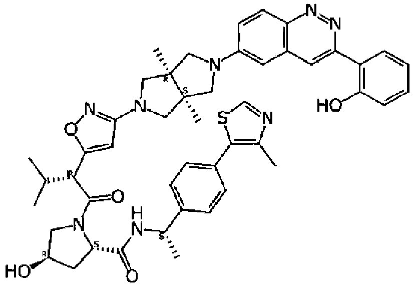 |
| 189 | 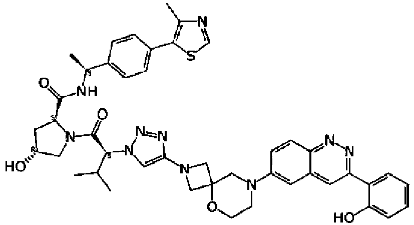 |
| 190 | 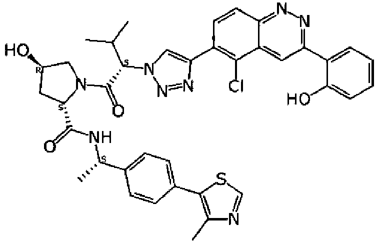 |
| 191 | 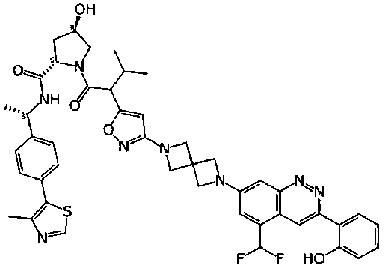 |
| 192 | 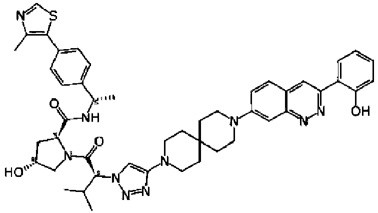 |
| 193 | 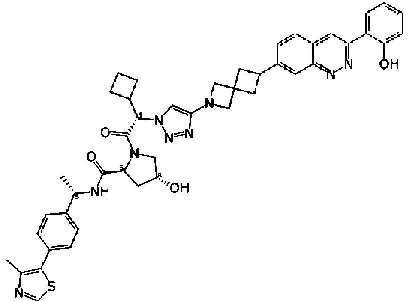 |
| 194 | 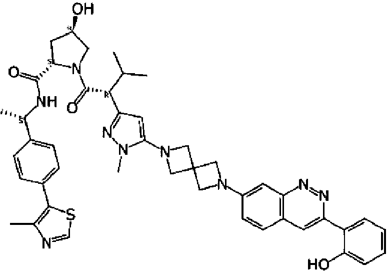 |
| 195 | 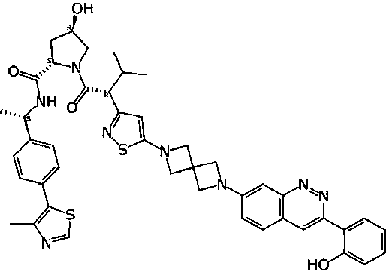 |
| 196 | 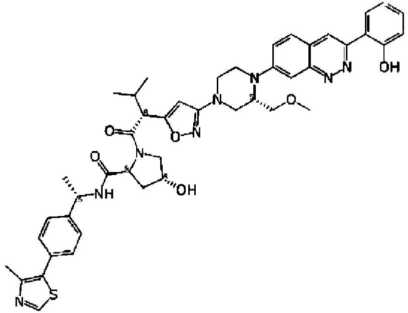 |
| 197 | 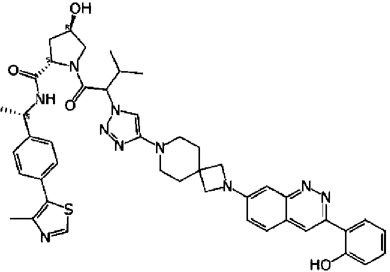 |
| 198 | 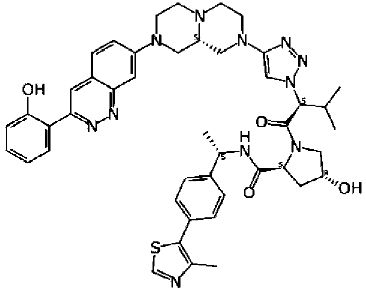 |
| 199 | 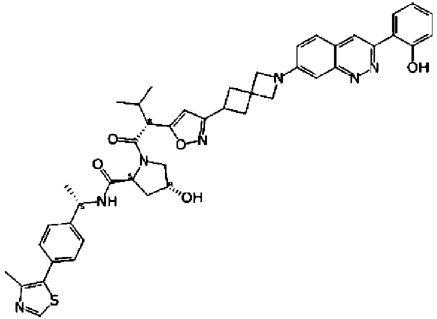 |
| 200 | 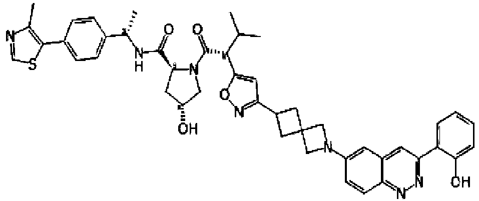 |
| 201 | 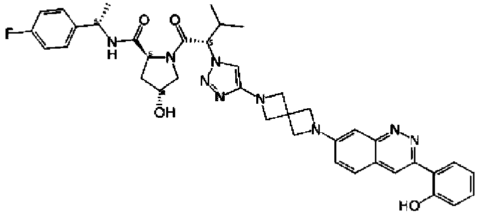 |
| 202 | 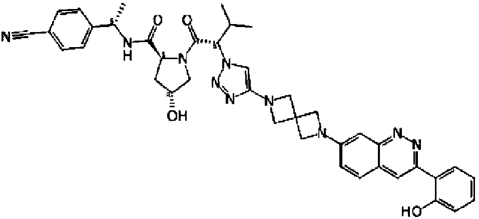 |
| 203 | 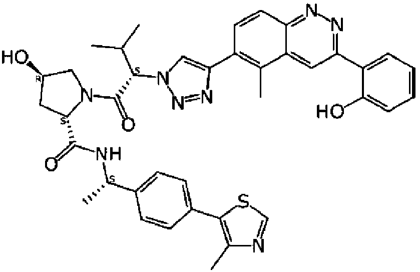 |
| 204 | 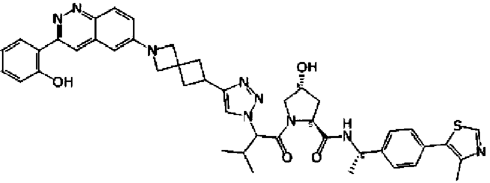 |
| 205 | 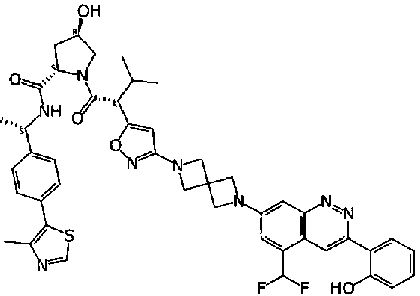 |
| 206 | 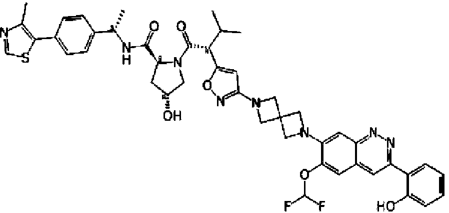 |
| 207 | 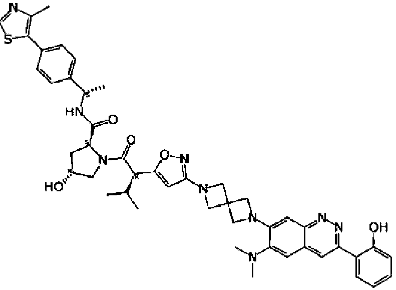 |
| 208 | 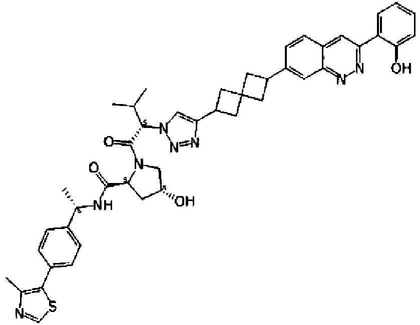 |
| 209 | 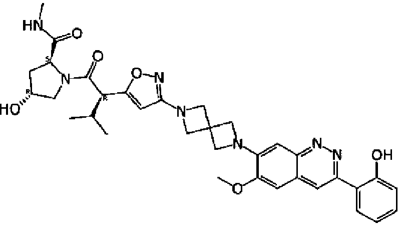 |
| **#** | **Compound** |
|---|---|
| 210 | 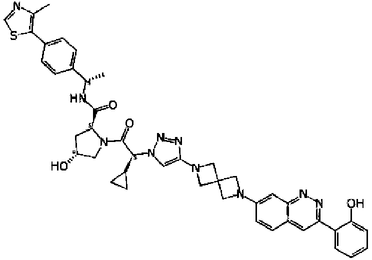 |
| 211 | 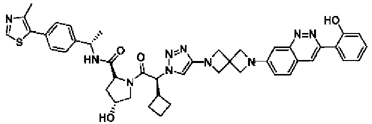 |
| 212 | 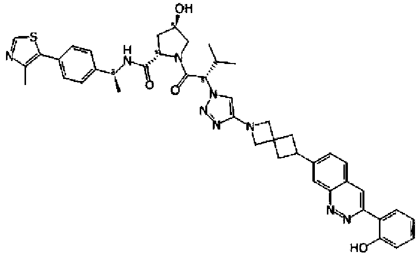 |
| 213 | 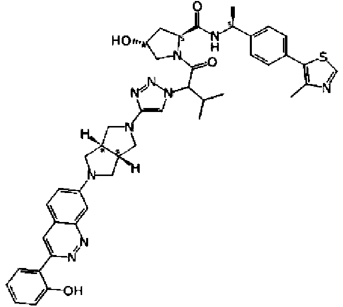 |
| 214 | 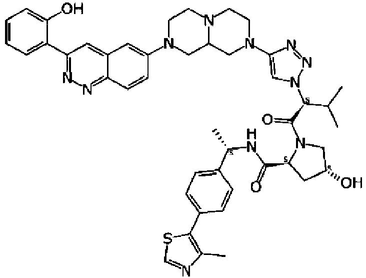 |
| 215 | 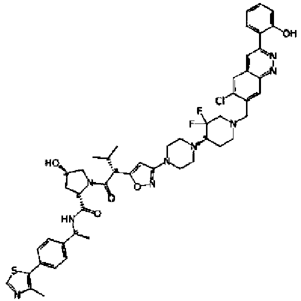 |
| 216 | 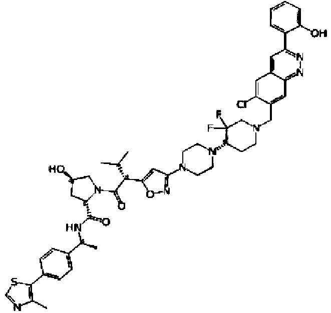 |
| 217 | 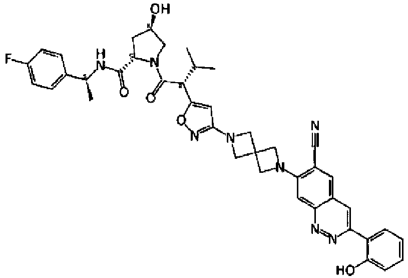 |
| 218 | 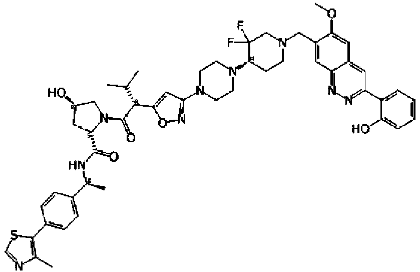 |
| 219 | 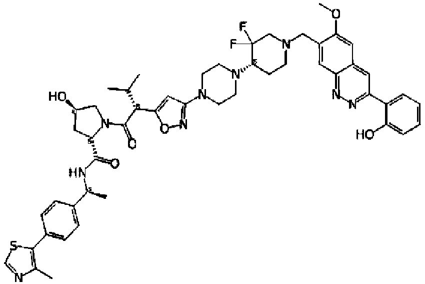 |
| 220 | 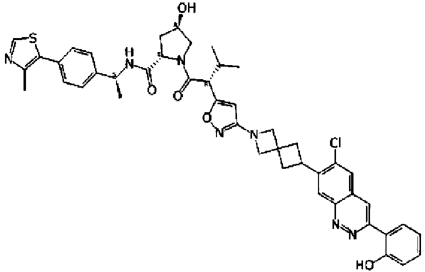 |
| **#** | **Composé** |
|---|---|
| 221 | 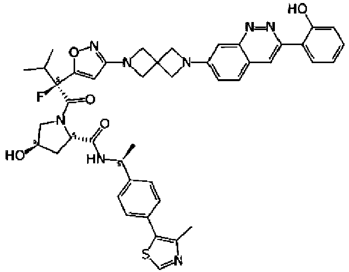 |
| 222 | 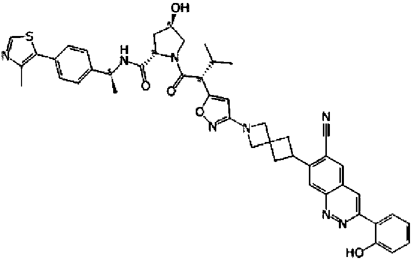 |
| 223 | 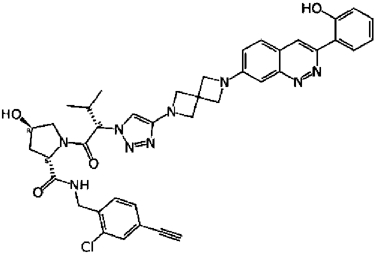 |
| 224 | 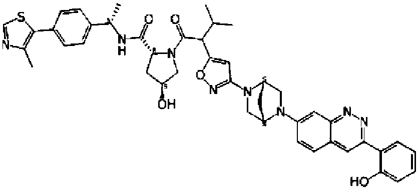 |
| 225 | 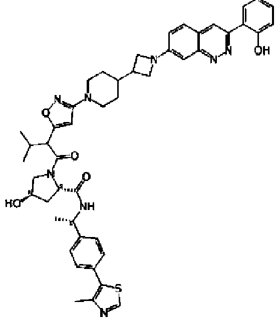 |
| 226 | 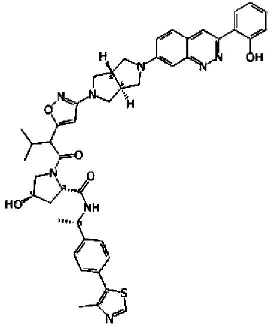 |
| 227 | 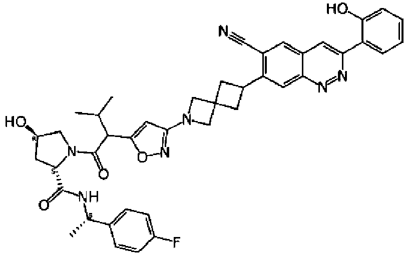 |
| 228 | 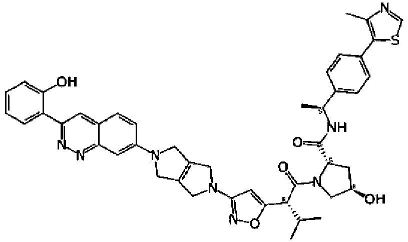 |
| 229 | 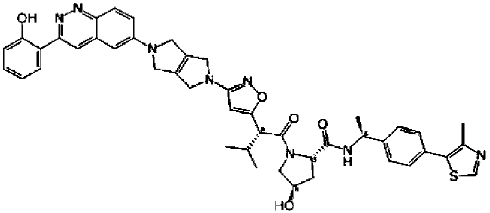 |
| 230 | 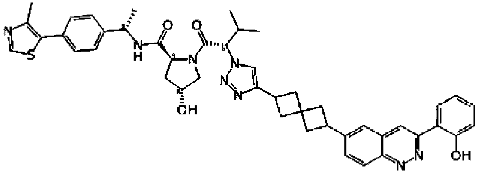 |
| 231 | 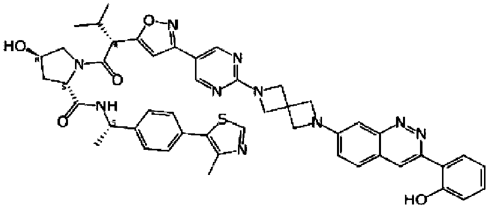 |
| 232 | 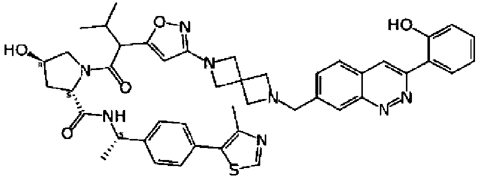 |
| 233 | 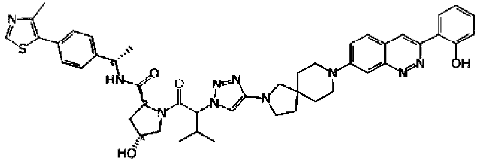 |
| 234 | 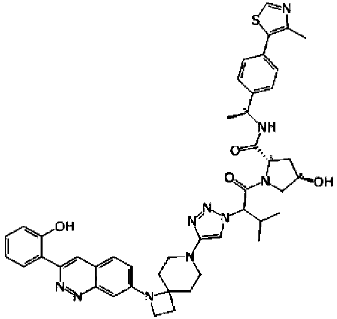 |
| 235 | 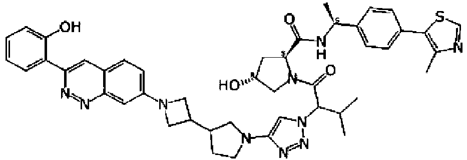 |
| 236 | 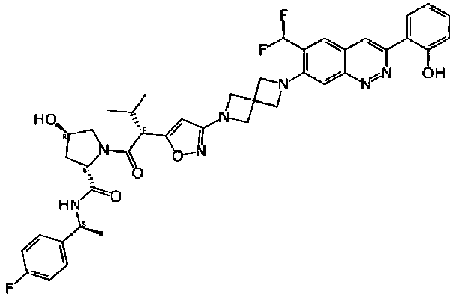 |
| 237 | 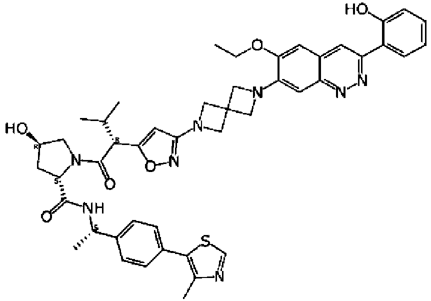 |
| 238 | 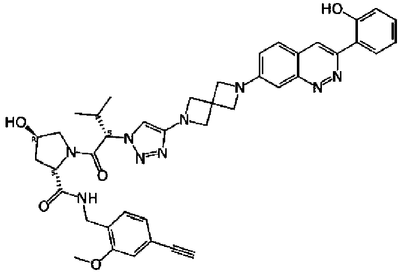 |
| 239 | 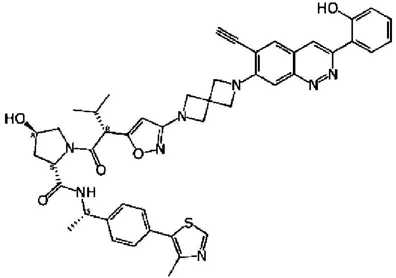 |
| 240 | 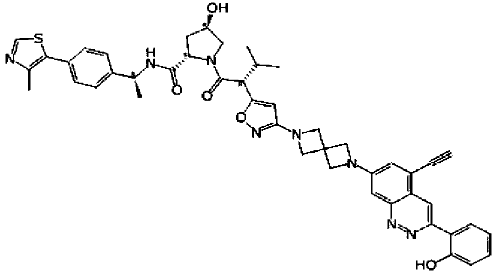 |
| 241 | 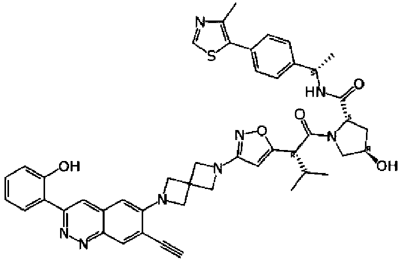 |
| 242 | 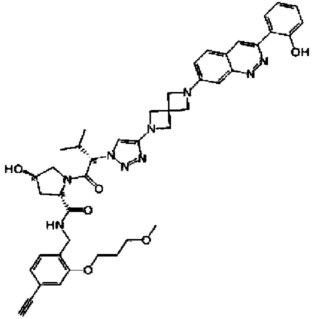 |
| 243 | 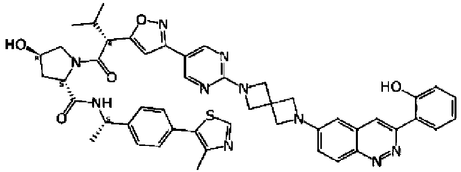 |
| 244 | 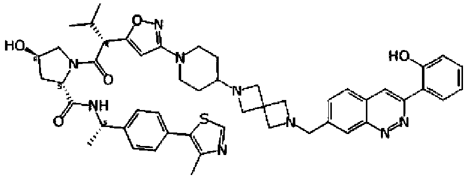 |
| 245 | 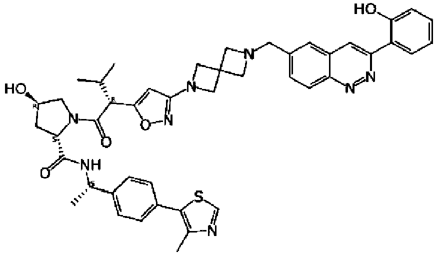 |
| 246 | 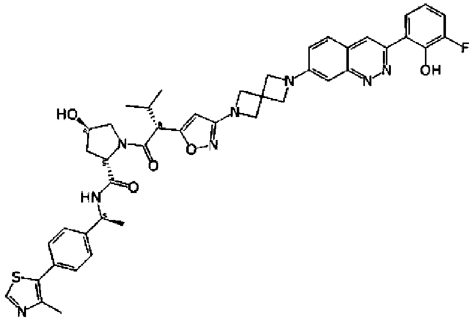 |
| 247 | 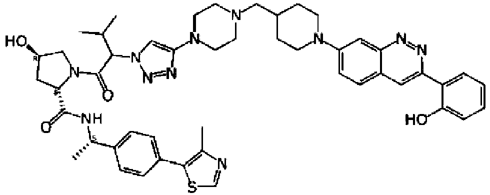 |
| 248 | 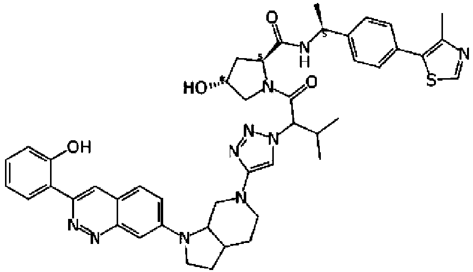 |
| 249 | 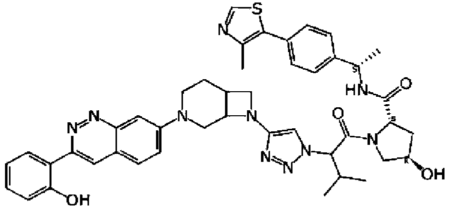 |
| 250 | 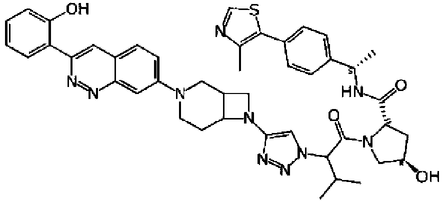 |
| 251 | 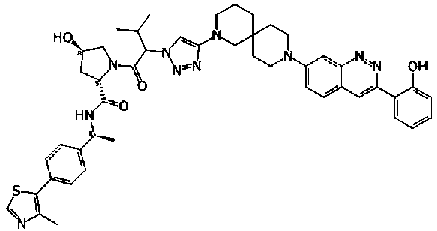 |
| 252 | 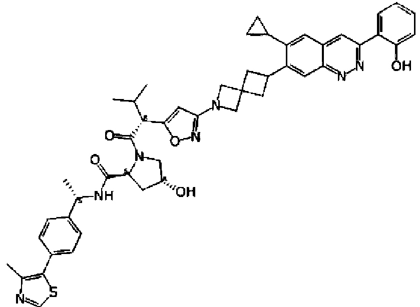 |
| 253 | 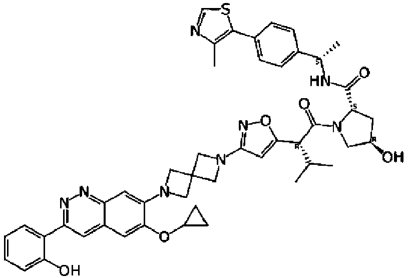 |
| 254 | 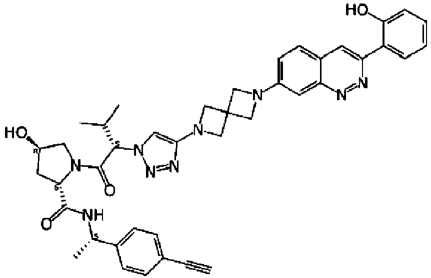 |
| 255 | 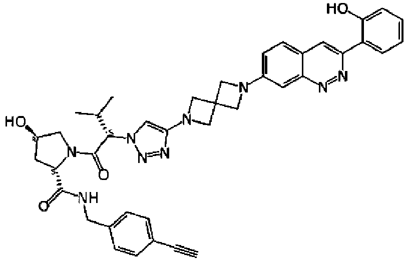 |
| 256 | 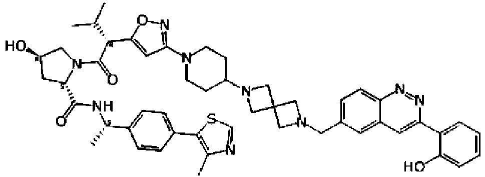 |
| 257 | 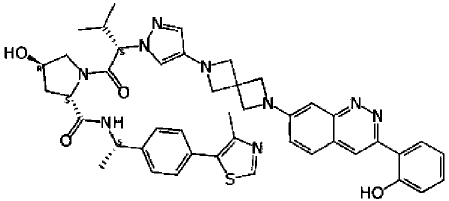 |
| 258 | 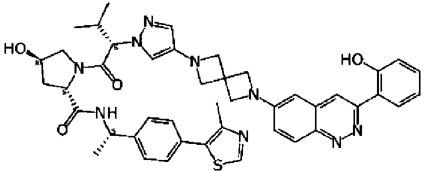 |
| 259 | 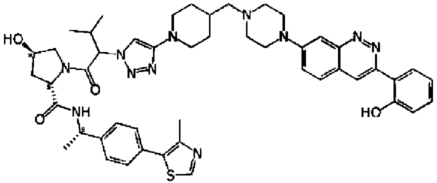 |
| 260 | 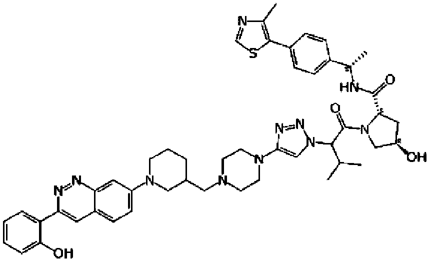 |
| 261 | 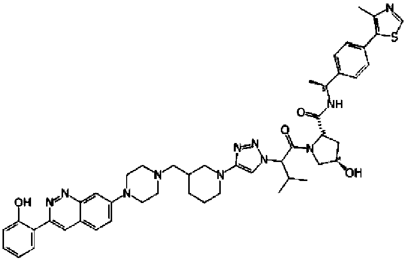 |
| 262 | 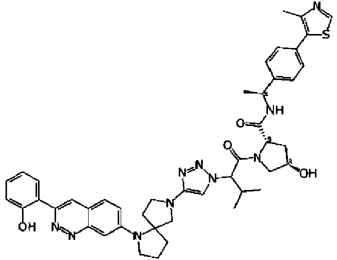 |
| 263 | 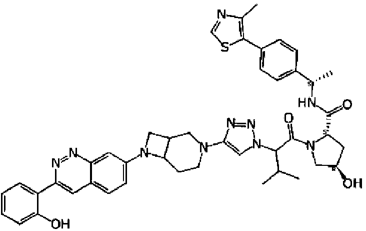 |
| 264 | 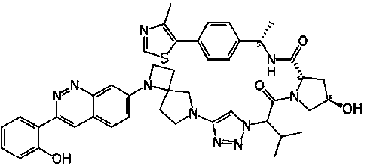 |
| 265 | 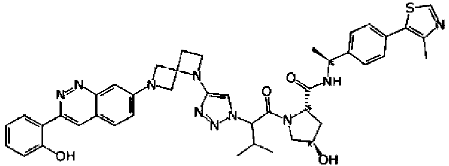 |
| 266 | 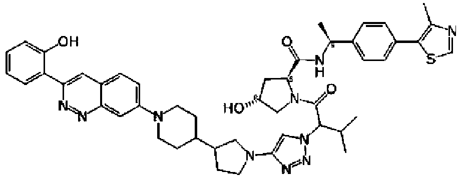 |
| 267 | 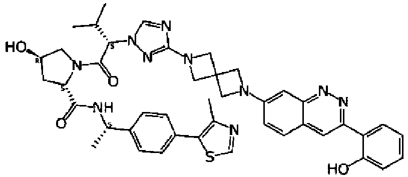 |
| 268 | 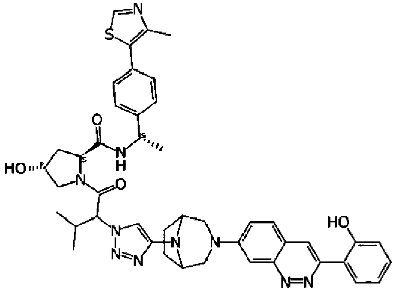 |
| 269 | 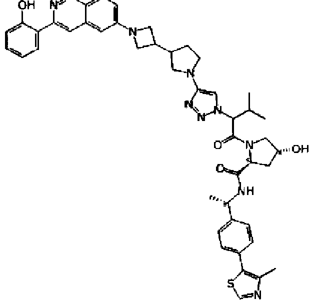 |
| 270 | 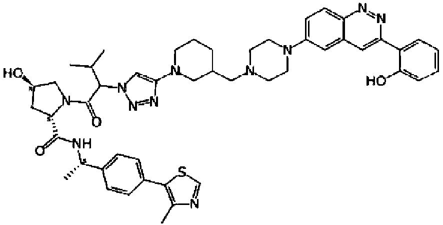 |
| 271 | 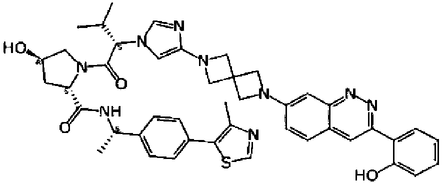 |
| 272 | 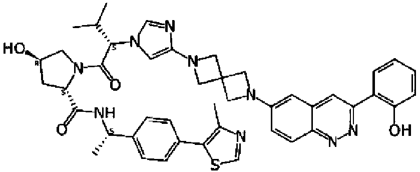 |
| 273 | 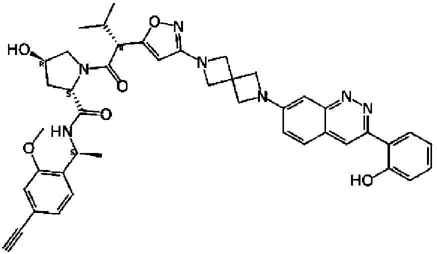 |
| 274 | 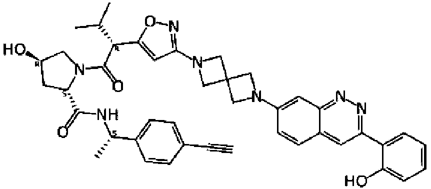 |
| 275 | 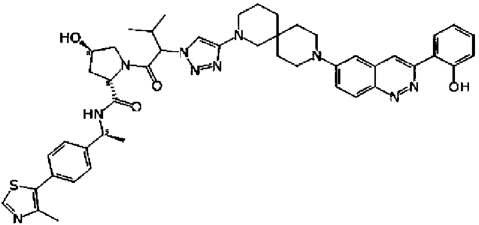 |
| 276 | 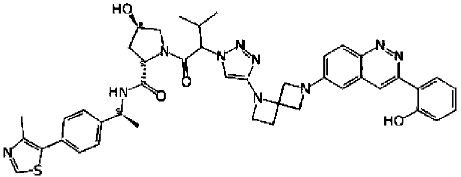 |
| 277 | 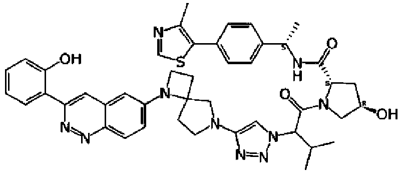 |
| 278 | 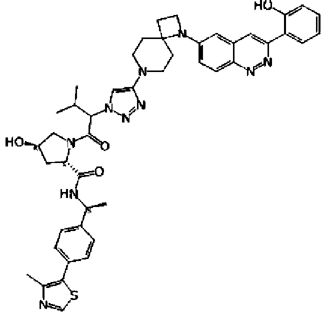 |
| 279 | 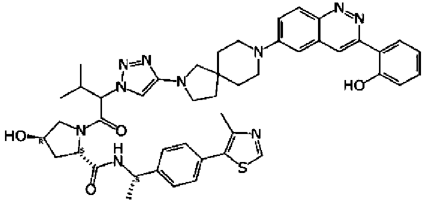 |
| 280 | 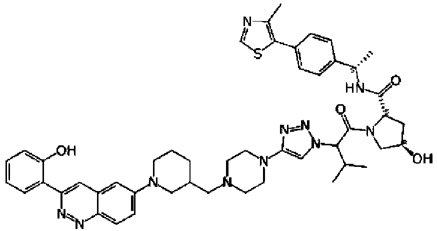 |
| 281 | 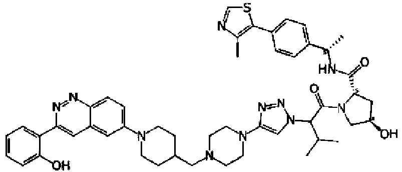 |
| 282 | 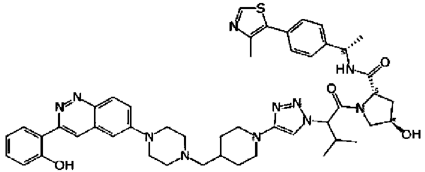 |
| 283 | 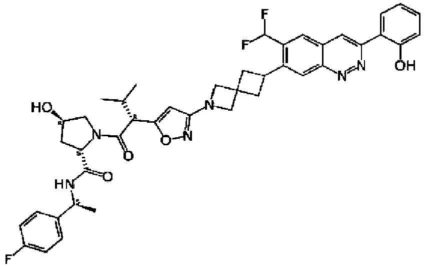 |
| 284 | 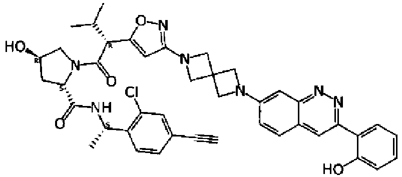 |
| 285 | 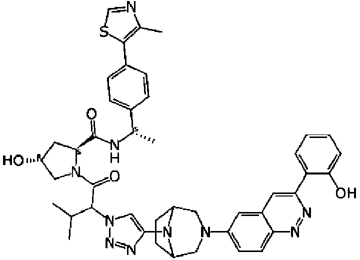 |
| 286 | 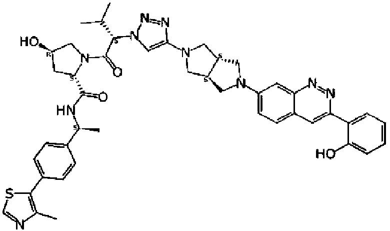 |
| 287 | 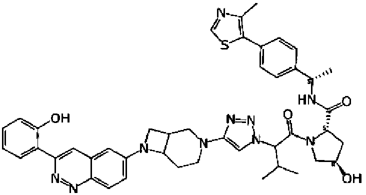 |
| 288 | 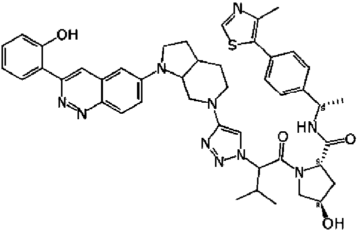 |
| 289 | 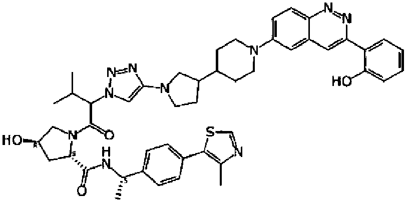 |
| 290 | 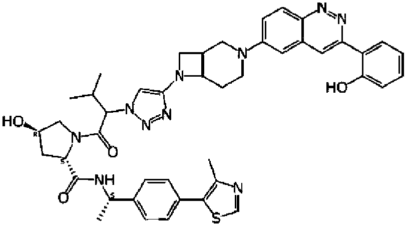 |
| 291 | 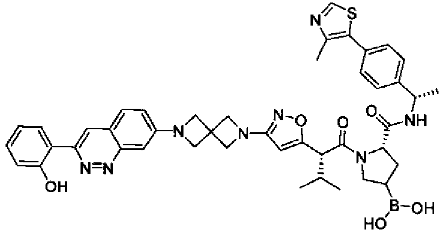 |

19. Composition pharmaceutique comprenant un composé selon l'une quelconque des revendications 1 à 18 et un excipient pharmaceutiquement acceptable.

20. Composé selon l'une quelconque des revendications 1 à 18, ou sel pharmaceutiquement acceptable de celui-ci, ou composition pharmaceutique selon la revendication 19, pour une utilisation en thérapie.

21. Composé selon l'une quelconque des revendications 1 à 18, ou sel pharmaceutiquement acceptable de celui-ci, ou composition pharmaceutique selon la revendication 19, pour utilisation dans le traitement d'un cancer.

22. Composé, ou sel pharmaceutiquement acceptable de celui-ci, ou composition pharmaceutique pour utilisation selon la revendication 21, dans lequel le cancer est un cancer du poumon non à petites cellules, un cancer colorectal, un cancer de la vessie, un cancer d'origine inconnue, un gliome, un cancer du sein, un mélanome, un cancer de la peau autre que mélanome, un cancer de l'endomètre, un cancer œsophagien et gastrique, un cancer du pancréas, un cancer hépatobiliaire, un sarcome des tissus mous, un cancer de l'ovaire, un cancer de la tête et du cou, un carcinome rénal, un cancer des os, un lymphome non hodgkinien, un cancer du poumon à petites cellules, un cancer de la prostate, une tumeur embryonnaire, une tumeur germinale, un cancer du col de l'utérus, un cancer de la thyroïde, un cancer des glandes salivaires, une tumeur neuroendocrine gastro-intestinale, un sarcome utérin, une tumeur stromale gastro-intestinale, un cancer du système nerveux central, une tumeur thymique, un carcinome corticosurrénalien, un cancer de l'appendice, un cancer de l'intestin grêle ou un cancer du pénis.
